# EUROPEAN PATENT APPLICATION

(11) **EP 1 801 118 A1**
(43) Date of publication of application: **27.06.2007**
(21) Application number: 05783456.6
(22) Date of filing: 14.09.2005
(51) Int. Cl.: C07K 14/435, C07K 7/06, C07K 7/08, C12P 21/02

(54) **PROCESS FOR SYNTHESIS OF MUCIN-TYPE PEPTIDES AND MUC1-RELATED GLYCOPEPTIDES**

(30) Priority: 14.09.2004 JP 2004267521; 25.03.2005 JP 2005090182
(71) Applicant: SHIONOGI & CO., LTD., Osaka-shi, Osaka 541-0045 (JP); National Institute of Advanced Industrial Science and Technology, Tokyo 100-8921 (JP)
(72) Inventor: NISHIMURA, Shinichiro, Sapporo-shi, Hokkaido 0628517 (JP); HINOU, Hiroshi, Sapporo-shi, Hokkaido 0628517 (JP); FUMOTO, Masataka, Room 403, Flat Sapporo, Sapporo-shi, Hokkaido 0640805 (JP)
(74) Representative: UEXKÜLL & STOLBERG
(86) International application number: PCT/JP2005/016975
(87) International publication number: WO 2006/030840

(57) **Abstract**

The invention aims at providing novel compounds useful as primer in producing mucin-type glycopeptides which are useful in a wide field including materials for biochemical research, drugs, and food and the production of which was difficult in the prior art; and a process for the production of glycopeptides by using the primers. The aim is attained by providing novel glycopeptide derivatives (represented by the general formula (I)) which each bear an aldehyde or ketone group at the end and each contain an amino acid residue cleavable with a protease; and an easy and simple process for the production of glycopeptides by using the derivative as the primer.

## Description

The present invention relates to novel compounds useful as primers in producing a glycopeptide, and a method for producing a glycopeptide using such primers. The present invention also relates to glycopeptides obtained by such production methods.

### BACKGROUND ART

Sugar chains are one of the main components composing an organism, as well as nucleic acids and proteins, and are well known as an energy source of an organism. In recent years, it has been clarified that sugar chains have various higher-order functions such as signal transduction, quality control of proteins, stabilization of structures, labeling for protein transport and the like in an organism. However, in comparison with the case of nucleic acids and proteins, no general method for the preparation of sugar chains has been established. Further, since sugar chains often function as glycoconjugates as a result of binding to lipids, proteins or the like, an extremely large part of the study of the functions of sugar chains including structural information thereof remains unexplained. Although in the field of study of proteins a number of proteins have been found which are regarded to achieve their functions together with sugar chains, studies on the detailed mechanisms thereof are extremely difficult in actual circumstance.

In order to proceed these studies and to further utilize them for medicaments or the like, it is required to prepare a homogeneous sample in a state of not an independent sugar chain but a glycoconjugate. Regarding glycopeptides, in particular, since both sugar chains and peptides have great variability, it is practically impossible to obtain the required structure each time from native glycopeptides. The development of a method for rapidly producing a glycopeptide has been expected. As represented by combinatorial chemistry developed in recent years, chemical synthesis methods are good at producing various structures through common procedures. Based on these backgrounds, various methods for producing a glycopeptide have been studied so far, but no practical production method has been reported. Principal reasons for this include: difficulty in preparing glycoamino acids having various sugar chain structures due to complicated preparation of glycoamino acids as raw materials; low yield and reaction rates due to large steric hindrance caused by glycoamino acids having a large sugar chain structure; and difficulty in the elongation of a sugar chain by chemical synthesis methods after construction of glycopeptides in terms of reactivity and control of position/configuration. In other words, due to low reaction yields and the long times required for preparation in current techniques, and difficulties in the preparation of raw materials *per se* for synthesis of glycopeptides, it is extremely difficult to produce a sugar chain in a tailor-made manner so as to rapidly prepare the required sugar chain structures and to construct a glycopeptide library including a complicated sugar chain structure required for exhaustive analysis of the functions of glycopeptides and glycoproteins.

In the synthesis of glycopeptides, generally, a method is used in which an Fmoc glycosylamino acid together with an Fmoc-amino acid (amino acid in which an amino group is protected with 9-fluorenylmethyloxycarbonyl group; hereinafter, 9-fluorenylmethyloxycarbonyl is abbreviated as Fmoc) to synthesize on a solid-phase support a peptide moiety as a base for the automatic peptide synthesis apparatus, the peptide moiety is released from the solid-phase support and is purified, and thereafter sugar chains are elongated one by one by organic chemistry or enzymatic synthesis method. As such, elongation of a sugar chain requires complicated manipulations and long times. Accordingly, the automatic synthesis of an oligosaccharide chain moiety as well as peptide moiety would be very useful for more rapid synthesis of glycopeptides and construction of a library. Automatic synthesis techniques have been established with regard to nucleic acids and proteins. It is appreciated by anyone that such techniques have contributed to significant advances in the studies of these fields, and the establishment of such automatic synthesis techniques are also desired eagerly with regard to sugar chains.

So far, some reports have been made with regard to studies oriented to the library synthesis of glycopeptides. In all of those reports, synthesis of the peptide portion is performed by solid-phase chemical synthesis methods based on the method of R. B. Merrifield. On the other hand, methods for synthesis of oligosaccharide chains are broadly classified into two methods. One is a chemical synthesis method, which has problems in that a method for stereoselectively binding sugar residues has not been sufficiently established, and that the steps such as binding or elimination of protective groups are complicated. The other is an enzyme synthesis method, which does not require protective groups and can stereoselectively bind sugar residues. Thus, this method is very advantageous in comparison with chemical synthesis. In recent years, some methods which enable automatic synthesis using such a method in combination with polymeric supports have been proposed. The background behind this is that genes of various glycosyltransferases have been recently isolated, which has enabled mass production of glycosyltransferases by genetic recombination techniques.

As an example of such a technique, U. Zehavi et al. have reported solid-phase synthesis by glycosyltransf erase using polyacrylamide gel having an aminoethyl group or aminohexyl group bound thereto as a solid-phase support (see Non-Patent Document 1-4). In this method, an appropriate monosaccharide is formed into 4-carboxy-2-nitrobenzylglycoside and isbound to an amino group of the above support directly or through a spacer. Using this as a primer, a sugar chain elongation reaction by glycosyltransferase is subsequently performed, and the elongated sugar chain is subsequently released by photolysis. However, the glycosyl transfer yield is approximately 50% and is insufficient. Further, this method provides not glycopeptides but oligosaccharides.

As another example, C.-H. Wong et al. have reported a method using aminated silica with glycopeptides bound thereto used as primers. In this method, after the sugar chain is elongated with glycosyltransferase, the elongated sugar chain is cleaved in the form of a glycopeptide utilizing the hydrolytic action of α-chymotrypsin (see Non-Patent Document 5). The peptide chain of the obtained glycopeptide is as short as Asn (asparagine)-Gly (glycine)-Phe (phenylalanine). Further, the yield of the sugar chain elongation reaction by glycosyltransferase is 55% to 65%, which is not sufficient at all.

Further, C.-H. Wong *et al.* have improved the group to be bound to aminated silica which is a solid-phase support, and have reported a method in which a sugar chain is elongated by glycosyltransferase and is subsequently released by hydrazinolysis. They have also reported that a glycosyl transfer reaction with the enzyme could be performed almost quantitatively (see Non-Patent Document 6). However, sugar chain compounds obtained from this method are not glycopeptides.

Further, C.-H. Wong *et al*. have reported a method in which an Fmoc-amino acid and an Fmoc-Thr (βGlcNAc) -OH are applied to the primer in the Non-Patent Document 7, which uses aminated silica as a solid-phase support, to elongate the peptide chain, a protective group on the peptide chain is eliminated, a glycosyltransferase is subsequently applied to the above N-GlcNAc residue to elongate the sugar chain, and glycopeptides synthesized on the solid-phase support are released by treatment with palladium tetrakistriphenylphosphine (see Non-Patent Document 7). The glycopeptide chain obtained from this method consists of eight amino acid residues and has a sufficient length for a peptide chain. However, the yield of the obtained glycopeptides is 10% or less with respect to the amino acids which were first introduced on the solid-phase support, and is insufficient. Further, since impurities such as unreacted substances accumulate through peptide synthesis and sugar chain synthesis, when each peptide chain structure and sugar chain structure are complicated, it becomes difficult to isolate and purify the substance of interest. Further, since the automatic synthesis of peptides is typically performed in an organic solvent and sugar chain synthesis by glycosyltransferase is typically performed in an aqueous solution, the desired properties of the supports in the respective reactions may vary, it is difficult to automatically synthesize both a peptide and a sugar chain on a single support.

Further, M. Meldal et al. have reported a method using a primer obtained by binding a glycopeptide derivative to a polymer of monoacryloylated and diacryloylated derivatives of diaminated polyethylene glycol. In this method, a sugar chain is elongated using a glycosyltransferase and is subsequently released by trifluoroacetic acid (see Non-Patent Document 8). However, the peptide chain of the glycopeptide obtained from this method is Asn (asparagine)-Gly (glycine) and is too short to be referred to as a glycopeptide. Further, the glycine residue at the C-terminus is glycinamide residue, and it is required to substitute the glycinamide residue with a glycine residue in some cases.

S. Roth *et al.* have disclosed the followingmethod in Patent Document 1. First, a sugar acceptor of a glycosyltransferase is bound to a solid-phase support to be used as an affinity adsorbent. By contacting tissue extract containing a glycosyltransferase which is capable of binding to the sugar acceptor with the affinity adsorbent, the glycosyltransf erase is bound to the affinity adsorbent. Subsequently, by contacting this affinity adsorbent to which glycosyltransferase has beenboundwith a solution containing a sugar nucleotide which can be used by the glycosyltransferase as a sugar donor, the glycosyltransferase is released from the affinity adsorbent, and concurrently a sugar chain of the sugar acceptor is elongated by one sugar residue. Further, by contacting a tissue extract containing glycosyltransferase which is capable of binding to the sugar acceptor having the sugar chain elongated by one sugar residue, the same procedure is repeated, thereby synthesizing a sugar chain of interest on a solid-phase support. However, no specific data which shows utility of this method or application of this method to the synthesis of non-native glycopeptides is shown therein, and no method for separating obtained sugar chains from a solid-phase support is disclosed therein.

Nishimura et al. has disclosed a protease-cleavable primer which can be used for the synthesis of glycopeptides or neo-glycopeptides (glycopeptides of non-native type), a method for producing a glycopeptide using such a primer, and polymeric aromatic amino acid derivatives useful for synthesis of such a primer (see Patent Document 2) . However, this method has the following problems remaining. Since peptides having a sugar residue are radically polymerized in this method, it is difficult to prepare glycopeptides including a radically sensitive sulfur atom. Further, the method involves complicated manipulations such as column purification, polymerization and the like after peptide synthesis, and thus it takes a long time to switch from solid-phase chemical peptide synthesis to sugar chain elongation reaction by enzymes.

Thus, there has not yet been a primer which can be readily instrumentated and purified for rapidly producing a glycopeptide with a high yield. A novel technique which can efficiently associate automatic peptide synthesis by a chemical method and automatic sugar chain synthesis by an enzymatic method is very important in the age of glycomics and glycoproteomics that supports post-genome and post-proteomics, and the development of such a technique is eagerly desired. Among methods for glycopeptide synthesis methods as actually described herein as examples which are oriented to instrumentation, there is no example of synthesis of multiple types or synthesis of glycopeptides including a complicated native sugar chain or a plurality of sugar chains, which might be referred to as glycopeptide library.

Mucin is a main glycoprotein of mucilage which covers digestive canals, such as the trachea and gastrointestine, and lumens, such as the genital glands. MUC1 is a membrane-bound glycoprotein of epithelial cells, and is the first mucin that was studied in detail. MUC1 is a gigantic cell surface molecule having a characteristic structure referred to as tandem repeat (HGVTSAPDTRPAPGSTAPPA) which is a repetition of an amino acid sequence including serine and threonine to which O-linked sugar chains may be added. Since not all additions of sugar chains occur in serine and threonine, and the degree of sugar chain elongation is also variable, there may be a number of glycoproteins which have different functions while having the same amino acid sequence.

It has been reported that the expression level of MUC1 varies with progress of canceration (Non-Patent Document 9: Nakamori, S.; Ota, D. M.; Karen, R.; Shirotani, K.; Irimura, T. Gastroenterology, 1994, 106, 353-361.). For example, in the case of colorectal cancer, increases in expression of MUC1 has been observed in a primary tumor at a progressed stage or metastatic focus. Further, there have been a number of reports that the glycosylation degree (site of introduction of the sugar chain) and the sugar chain structure of MUC1 are different between MUC1 derived from a normal epithelium and MUC1 derived from cancer cells (Non-Patent Document 10: Llod, K. O.; Burchell, J.; Kudryashov, V.; Yin, B. W. T.; Taylor-Papadimitriou, J. J. Biol. Chem., 1996, 271, 33325-33334,; Non-Patent Document 11: Hanisch, F.-G.; Muller, S. Glycobiology, 2000, 10, 439-449.). For example, in some cases, even peptides which are glycosylated in a normal cell are not glycosylated in a cancer cell and are exposed on the cell surface. In such a case, the exposed peptide portion is an epitope. Such exposed epitopes have been found in cell membranes of epithelial cell lines derived from lung cancer, breast cancer, colonic cancer and pancreatic cancer. Specifically, cytotoxic T lymphocyte isolated from a patient with breast cancer recognizes a peptide which has not accepted glycosylation of a MUC1 protein. On the other hand, core structures such as Tn and T which are cancer-associated sugar chain antigens and sialyl Tn and sialyl T with sialic acid bound thereto, and further, sialyl Lewis A antigen and sialyl Lewis X antigen have been found in mucin on cancer cell membranes and mucin in serum from patients with cancer.

In recent years, the application of MUC1 to drug design and diagnostic drugs targeting such specific changes of MUC1 associated with canceration has attracted attention (Non-Patent Document 12: Koganty, R. R.; Reddish, M. R.; Longenecker, B. M. DrugDiscov. Today, 1996, 1, 190-198.). For example, Biomira-Merck is developing a synthetic MUC1 peptide vaccine: "L-BLP25", in which a sequence of 25 amino acids of MUC1 cancer mucin is incorporated into a liposomal formulation, and is carrying out Phase II clinical tests targeting lung cancer and prostatic cancer. Further, a synthetic vaccine: "Theratope" obtained by binding KLH (Keyhole limpet hemocyanin) which stimulates the production of antibodies and T-cell reactions as a carrier protein to synthetic STn targetting STn (disaccharide) which shows expression specific to mucin on cancer cells, is under Phase III clinical development by Biomira-Merck, targeting breast cancer and rectal cancer.
Patent Document 1: Japanese National Phase PCT Laid-Open Patent Publication No. 5-500905
Patent Document 2: Japanese Laid-Open Patent Publication No. 2001-220399
Non-Patent Document 1: Carbohydr. Res., 124, 23 (1983)
Non-Patent Document 2: Carbohydr. Res., 228, 255 (1992)
Non-Patent Document 3: React.Polym., 22, 171 (1994) Non-Patent Document 4: Carbohydr. Res., 265, 161 (1994)
Non-Patent Document 5: J. Am. Chem. Soc., 116, 1136 (1994)
Non-Patent Document 6: J. Am. Chem. Soc., 116, 11315 (1994)
Non-Patent Document 7: J. Am. Chem. Soc., 119, 8766 (1997)
Non-Patent Document 8: J. Chem. Soc. Chem. Commun., 1849 (1994)
Non-Patent Document 9: Gastroenterology, 106, 353-361 (1994)
Non-Patent Document 10: J. Biol. Chem., 271, 33325-33334 (1996)
Non-Patent Document 11: Glycobiology, 10, 439-449(2000)
Non-Patent Document 12: Drug Discov. Today, 1, 190-198 (1996)

### DISCLOSURE OF THE INVENTION

### PROBLEMS TO BE SOLVED BY THE INVENTION

It is an object of the present invention to provide novel compounds which are useful as primers in producing glycopeptides and a method for producing a glycopeptide using such primers, and to produce mucin-type peptides which are useful in a wide range of fields including materials for biochemical research, drugs and food and which have been conventionally difficult to produce.

### MEANS FOR SOLVING THE PROBLEMS

The present inventors have found as a result of their wholehearted studies that novel glycopeptide derivatives which have an aldehyde group or a ketone group at the end and contain an amino acid residue which can be cleaved by a protease can be firmly bound to a certain support through the aldehyde group or ketone group, and serves as a primer suitable for the production of glycopeptides since this bond is not decomposed under hydrolytic conditions by a protease, and that use of this primer facilitates purification of glycopeptides which have conventionally required multi-step purification and enables rapid production of glycopeptides with a high yield, thereby achieving the above objective.

Further, the present inventors have found that the method for producing a glycopeptide using the above primer enables synthesis of mucin-type glycopeptides which are useful in a wide range of field including materials for biochemical research, drugs, and food and which have been conventionally difficult in the prior art, thereby completing the present invention.

The MUC1 and MUC1 peptide library of the present invention are effective for elucidation of the functions of MUC1, and the possibility of novel drug design based on the knowledge obtained therefrom is considered. As studies using glycopeptides, for example, developments to immobilization of glycopeptide library/construction of on-chip glycopeptide library, antibody reactive screening, search for specific antibodies, investigation of structure-activity relationship in antigen-antibody reaction, production of monoclonal antibodies with a high specificity/selectivity, and further, antibody drugs, vaccine therapy using glycopeptides and the like are considered.

Accordingly, the present invention provides the following.
(1) A compound represented by the following formula:

   X-C (=O) - (CH₂)ₙ-A₁-A₂-A₃ (I)

   wherein X represents a hydrogen atom, C₁-C₃₀ alkyl, C₆-C₃₀ aryl or a chromophore;
   n represents an integer from 0 to 20;
   A₁ represents -(CH₂)₀₋₂₀-C(=O)-, -(CH₂CH₂O)₁₋₁₀-, oligoacrylamide or polyacrylamide having a degree of polymerization of 1 to 10, oligopeptide or polypeptide having a degree of polymerization of 1 to 10, an oxygen atom or NH;
   A₂ represents an amino acid residue which can be cleaved by a protease; and
   A₃ represents a glycoamino acid residue substantially free of a site which can be cleaved by a protease, or a glycopeptide residue free of a site which can be cleaved by a protease and including a glycoamino acid.
(2) The compound according to (1), wherein A₂ is a glutamic acid residue or cysteine residue which can be cleaved by a protease derived from *Bacillus Licheniformis.*
(3) The compound according to (1), wherein at least a part of A₃ has an amino acid sequence selected from the group consisting of the amino acid sequences as set forth in SEQ ID NOS: 1-60 derived from mucin-type glycoprotein MUC1.
(4) A compound which is obtained by reacting the compound according to (1) and a support including a functional group selected from the group consisting of: a protected or unprotected aminooxy group; a protected or unprotected N-alkylaminooxy group; a protected or unprotected hydrazid group; a protected or unprotected azide group; a protected or unprotected thiosemicarbazide group; a protected or unprotected 1,2-dithiolgroup; and a protected or unprotected cysteine residue.
(5) The compound according to (4), wherein the support is selected from the group consisting of:
   a) a polymer or copolymer of a vinyl-type monomer having a protected or unprotected amiooxy group or a protected or unprotected hydrazide group, or polyethers having a protected or unprotected aminooxy group or a protected or unprotected hydrazide group;
   b) a silica support, a resin support, magnetic beads or a metalic support, having a protected or unprotected aminooxy group or a protected or unprotected hydrazide group; and
   c) a compound represented by the following formula:
      [(NH₂OCH₂C(=O))₂-Lys]₂-Lys-NHCH₂CH₂C(=O)-R³,
      [(NH₂OCH₂C(=O))₂-Lys]₂-Lys-NHCH(CH₂SH)C(=O)-R³,
      [(NH₂OCH₂C(=O))₂-Lys]₂-Lys-Cys-NHCH₂CH₂C(=O)-R³ (SEQ ID NO: 61);
      {[(NH₂OCH₂C(=O))₂-Lys]₂-Lys-NHCH[C(=O)-R³]CH₂-S}₂,
      {[(NH₂OCH₂C(=O))₂-Lys]₂-Lys-NHCH[C(=O)NHCH₂CH₂C(=O)-R³ ]CH₂-S}₂,
      {[(NH₂OCH₂C(=O))₂-Lys]₂-Lys}₂-Lys-NHCH₂CH₂C(=O)-R³(SEQ ID NO: 62);,
      {[(NH₂OCH₂C (=O))₂-Lys]₂-Lys}₂-Lys-NHCH(CH₂SH)C(=O)-R³ (SEQ ID NO: 63);,
      {[(NH₂OCH₂C(=O))₂-Lys]₂-Lys}₂-Lys-Cys-N
      HCH₂CH₂C(=O)-R³ (SEQ ID NO: 64);,
      [[[(NH₂OCH₂C(=O))₂-Lys]₂-Lys)₂-Lys-NHCH[C(=O)-R³]CH₂-S ]₂ (SEQ ID NO: 65);
      [[[(NH₂OCH₂C(=O))₂-Lys]₂-Lys]₂-Lys-NHCH[C(=O)NHCH₂CH₂C (=O)-R³]CH₂-S]₂(SEQ ID NO: 66);

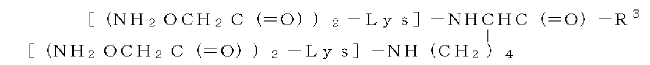

or

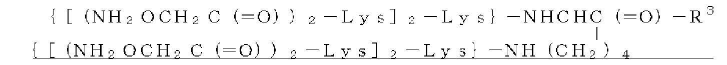

wherein R₃ represents a hydroxyl group or amino group, Lys represents lysine and Cys represents cysteine;

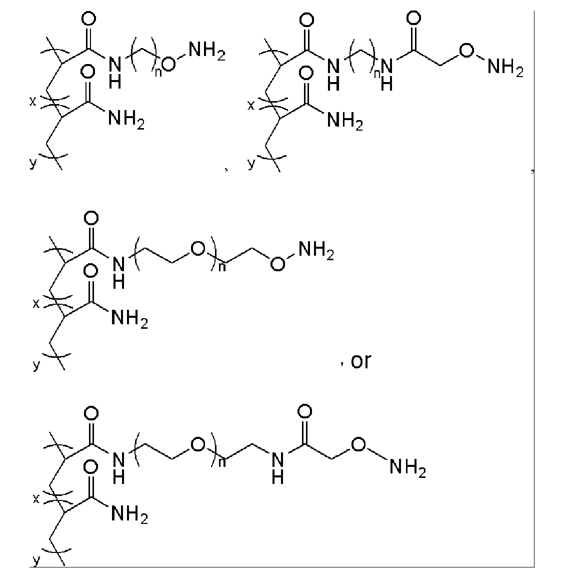

wherein n is an integer from 1 to 15 and x:y is 1:0 to 1:1000.
(6) A compound represented by the following formula:

   A₄-N=C (-X) - (CH₂)ₙ-A₁-A₂-A₃ (II)

   wherein X represents a hydrogen atom, C₁-C₃₀ alkyl, C₆-C₃₀ aryl or a chromophore;
   n represents an integer from 0 to 20;
   A₁ represents -(CH₂)₀₋₂₀-C(=O)-, -(CH₂CH₂O)₁₋₁₀-, oligoacrylamide or polyacrylamide having a degree of polymerization of 1 to 10, oligopeptide or polypeptide having a degree of polymerization of 1 to 10, an oxygen atom or NH;
   A₂ represents a glutamic acid residue or cysteine residue which can be cleaved by a protease derived from *Bacillus Licheniformis;*
   A₃ represents a glycoamino acid residue substantially free of a site which can be cleaved by a protease, or a glycopeptide residue free of a site which can be cleaved by a protease and including a glycoamino acid;
   A₄ is a group represented by the following formula:

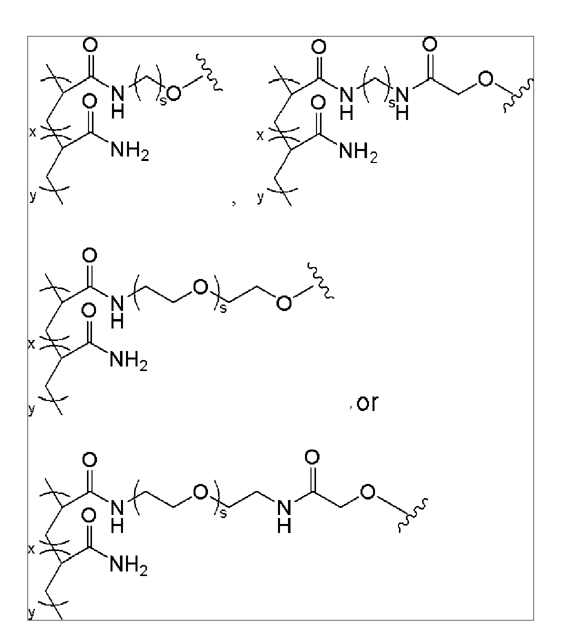

wherein s is an integer of 1 to 15 and x:y is 1:0 to 1:1000.
(7) The compound according to (6), wherein at least a part of A₃ has an amino acid sequence selected from the group consisting of the amino acid sequences as set forth in SEQ ID NOS: 1-60 derived from mucin-type glycoprotein MUC1.
(8) A method for producing a glycopeptide, the method including the steps of:
   (A) reacting the compound according to any one of claims 1 to 3 with a support including a functional group selected from the group consisting of: a protected or unprotected aminooxy group; a protected or unprotected N-alkylaminooxy group; a protected or unprotected hydrazid group; a protected or unprotected azide group; a protected or unprotected thiosemicarbazide group; a protected or unprotected 1,2-dithiol group; and a protected or unprotected cysteine residue, the functional group being capable of specifically reacting with a ketone residue or aldehyde residue;
   (B) allowing glycosyltransferase to act on the compound obtainedfromthestep (A) in the presence of a sugar nucleotide so as to cause a sugar residue to transfer from the sugar nucleotide to the compound, thereby obtaining a compound having an elongated sugar chain;
   (C) optionally removing unreacted sugar nucleotides and by-product nucleotides; and
   (D) allowing a protease to act on the compound having an elongated sugar chain as a result of transfer of the sugar residue.
(9) A method for producing a glycopeptide, the method including the steps of:
   (A) allowing glycosyltransferase to act on the compound according to any one of (4) to (7) in the presence of a sugar nucleotide so as to cause a sugar residue to transfer from the sugar nucleotide to the compound, thereby obtaining a compound having an elongated sugar chain;
   (B) optionally removing unreacted sugar nucleotides and by-product nucleotides; and
   (C) allowing a protease to act on the compound having an elongated sugar chain as a result of transfer of the sugar residue.
(10) A method for producing a glycopeptide, the method including the steps of:
   (A) allowing glycosyltransferase to act on the compound according to any one of (4) to (7) in the presence of a sugar nucleotide so as to cause a sugar residue to transfer from the sugar nucleotide to the compound, thereby obtaining a compound having an elongated sugar chain;
   (B) repeating the step (A) for one or more times to elongate a sugar chain;
   (C) optionally removing unreacted sugar nucleotides and by-product nucleotides; and
   (D) allowing a protease to act on the compound having an elongated sugar chain as a result of transfer of a plurality of sugar residues.
(11) A method for producing a glycopeptide, the method including the steps of:
   (A) performing solid phase peptide synthesis using an amino acid, glycoamino acid and keto acid or aldehydic acid, which can be cleaved by a protease, as raw materials, thereby obtaining the compound according to any one of (1) to (3);
   (B) reacting the compound obtained from the step (A) with a support including a functional group selected from the group consisting of: a protected or unprotected aminooxy group; a protected or unprotected N-alkylaminooxy group; a protected or unprotected hydrazid group; a protected or unprotected azide group; a protected or unprotected thiosemicarbazide group; a protected or unprotected 1,2-dithiol group; and a protected or unprotected cysteine residue, the functional group being capable of specifically reacting with a ketone residue or aldehyde residue;
   (C) allowing glycosyltransferase to act on the compound obtainedfromthestep (B) in the presence of a sugar nucleotide so as to cause a sugar residue to transfer from the sugar nucleotide to the compound, thereby obtaining a compound having an elongated sugar chain;
   (D) optionally removing unreacted sugar nucleotides and by-product nucleotides; and
   (E) allowing a protease to act on the compound having an elongated sugar chain as a result of transfer of the sugar residue.
(12) A method for producing a glycopeptide, the method including the steps of:
   (A) performing solid phase peptide synthesis using an amino acid, glycoamino acid and keto acid or aldehydic acid, which can be cleaved by a protease, as raw materials, thereby obtaining the compound according to any one of (1) to (3);
   (B) reacting the compound obtained from the step (A) with a support including a functional group selected from the group consisting of: a protected or unprotected aminooxy group; a protected or unprotected N-alkylaminooxy group; a protected or unprotected hydrazid group; a protected or unprotected azide group; a protected or unprotected thiosemicarbazide group; a protected or unprotected 1,2-dithiol group; and a protected or unprotected cysteine residue, the functional group being capable of specifically reacting with a ketone residue or aldehyde residue;
   (C) allowing glycosyltransferase to act on the compound bound to the support, which has been obtained from the step (B), in the presence of a sugar nucleotide so as to cause a sugar residue to transfer from the sugar nucleotide to the compound, thereby obtaining a compound having an elongated sugar chain;
   (D) repeating the step (C) for one or more times to elongate a sugar chain;
   (E) optionally removing unreacted sugar nucleotides and by-product nucleotides; and
   (F) allowing a protease to act on the compound having an elongated sugar chain as a result of transfer of a plurality of sugar residues.
(13) A method for producing a glycopeptide, the method including the steps of:
   (A) performing solid phase peptide synthesis using an amino acid, glycoamino acid and keto acid or aldehydic acid, which can be cleaved by a protease, as raw materials, thereby obtaining the compound according to any one of (1) to (3);
   (B) reacting the compound obtained from the step (A) with a support including a functional group selected from the group consisting of: a protected or unprotected aminooxy group; a protected or unprotected N-alkylaminooxy group; a protected or unprotected hydrazid group; a protected or unprotected azide group; a protected or unprotected thiosemicarbazide group; a protected or unprotected 1,2-dithiol group; and a protected or unprotected cysteine residue, the functional group being capable of specifically reacting with a ketone residue or aldehyde residue, and simultaneously removing unreacted substances in the step (A);
   (C) allowing glycosyltransferase to act on the compound bound to the support, which has been obtained from the step (B), in the presence of a sugar nucleotide so as to cause a sugar residue to transfer from the sugar nucleotide to the compound, thereby obtaining a compound having an elongated sugar chain; and
   (D) allowing a protease to act on the compound having an elongated sugar chain obtained from the step (C).
(14) A method for producing a glycopeptide, the method including the steps of:
   (A) performing solid phase peptide synthesis using an amino acid, glycoamino acid and keto acid or aldehydic acid, which can be cleaved by a protease, as raw materials, thereby obtaining the compound according to any one of (1) to (3);
   (B) reacting the compound obtained from the step (A) with a support including a functional group selected from the group consisting of: a protected or unprotected aminooxy group; a protected or unprotected N-alkylaminooxy group; a protected or unprotected hydrazid group; a protected or unprotected azide group; a protected or unprotected thiosemicarbazide group; a protected or unprotected 1,2-dithiol group; and a protected or unprotected cysteine residue, the functional group being capable of specifically reacting with a ketone residue or aldehyde residue, and simultaneously removing unreacted substances in the step (A);
   (C) allowing glycosyltransferase to act on the compound bound to the support, which has been obtained from the step (B), in the presence of a sugar nucleotide so as to cause a sugar residue to transfer from the sugar nucleotide to the compound, thereby obtaining a compound having an elongated sugar chain;
   (D) repeating the step (C) for one or more times to elongate a sugar chain;
   (E) optionally removing unreacted sugar nucleotides and by-product nucleotides; and
   (F) allowing a protease to act on the compound having an elongated sugar chain as a result of transfer of a plurality of sugar residues.
(15) The method according to (11) or (12), wherein the keto acid or aldehydic acid in the step (A) is a compound represented by the following formula:

   X-C (=O) - (CH₂) n-A₁-COOH (III)

   wherein X represents a hydrogen atom, C₁-C₃₀ alkyl, C₆-C₃₀ aryl or a chromophore;
   n represents an integer from 0 to 20; and
   A₁ represents a linker having a length of 1 to 20 methylene groups.
(16) A method for producing a glycopeptide, the method including the steps of:
   (A) allowing glycosyltransferase to act on the compound according to any one of (1) to (3) in the presence of a sugar nucleotide so as to cause a sugar residue to transfer from the sugar nucleotide to the compound, thereby obtaining a compound having an elongated sugar chain;
   (B) optionally repeating the step (A) for one or more times to elongate a sugar chain;
   (C) reacting the compound having an elongated sugar chain as a result of transfer of the sugar residue with a support including a functional group selected from the group consisting of: a protected or unprotected aminooxy group; a protected or unprotected N-alkylaminooxygroup; a protected or unprotected hydrazid group; a protected or unprotected azide group; a protected or unprotected thiosemicarbazide group; a protected or unprotected 1,2-dithiol group; and a protected or unprotected cysteine residue, the functional group being capable of specifically reacting with a ketone residue or aldehyde residue; and
   (D) optionally removing unreacted sugar nucleotides and by-product nucleotides.
(17) A method for producing a glycopeptide, the method including the steps of:
   (A) allowing glycosyltransferase to act on the compound according to any one of (1) to (3) in the presence of a sugar nucleotide so as to cause a sugar residue to transfer from the sugar nucleotide to the compound, thereby obtaining a compound having an elongated sugar chain;
   (B) optionally repeating the step (A) for one or more times to elongate a sugar chain;
   (C) reacting the compound having an elongated sugar chain as a result of transfer of the sugar residue with a support including a functional group selected from the group consisting of: a protected or unprotected aminooxy group; a protected or unprotected N-alkylaminooxy group; a protected or unprotected hydrazid group; a protected or unprotected azide group; a protected or unprotected thiosemicarbazide group; a protected or unprotected 1,2-dithiol group; and a protected or unprotected cysteine residue, the functional group being capable of specifically reacting with a ketone residue or aldehyde residue; and
   (D) optionally removing unreacted sugar nucleotides and by-product nucleotides; and
   (E) allowing a protease to act on the compound having an elongated sugar chain as a result of transfer of the sugar residue.
(18) A glycopeptide represented by the following formula:

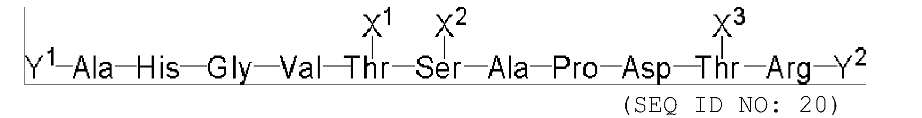

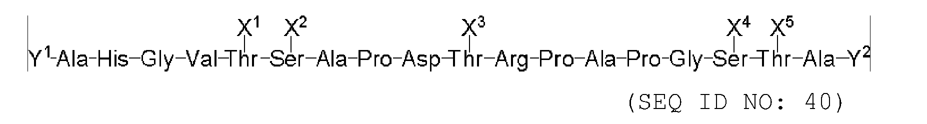

or

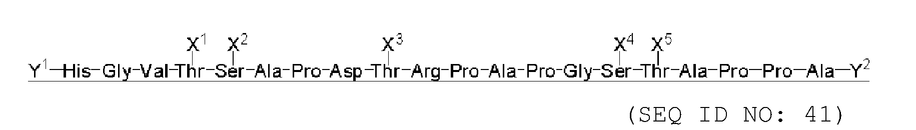

wherein X¹-X³ independently represent a hydrogen atom or a group represented by the following formula:

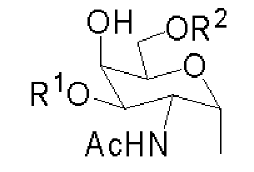

wherein R¹ and R² independently represent a hydrogen atom, monosaccharide or sugar chain, and Ac represents acetyl;
Y¹ represents a hydrogen atom, acetyl, acyl, alkyl or aryl;
Y² represents a hydroxyl group, NH₂, alkyl or aryl. Here, groups represented by the formulae:

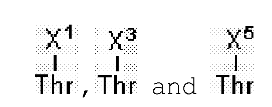

mean a group represented by:

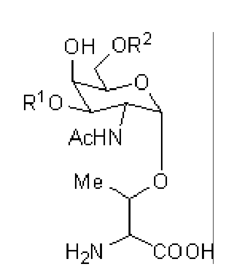

and groups represented by the formulae:

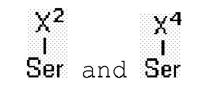

mean a group represented by:

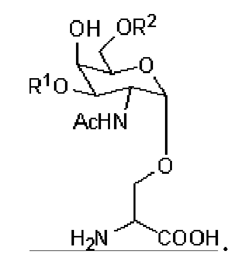

(19) A method for producing a glycopeptide, the method including the steps of:
(A) performing solid phase peptide synthesis using an amino acid, glycoamino acid and keto acid or aldehydic acid, which can be cleaved by a protease, as raw materials, thereby obtaining the compound according to any one of (1) to (3);
(B) reacting the compound obtained from the step (A) with a soluble support including a functional group selected from the group consisting of: a protected or unprotected aminooxy group; a protected or unprotected N-alkylaminooxy group; a protected or unprotected hydrazid group; a protected or unprotected azide group; a protected or unprotected thiosemicarbazide group; a protected or unprotected 1,2-dithiol group; and a protected or unprotected cysteine residue, the functional group being capable of specifically reacting with a ketone residue or aldehyde residue, and removing unreacted substances in the step (A) by reprecipitation, gel filtration, ultrafiltration or the like;
(C) allowing glycosyltransferase to act on the compound solubly bound to the support, which has been obtained from the step (B), in the presence of a sugar nucleotide so as to cause a sugar residue to transfer from the sugar nucleotide to the compound, thereby obtaining a compound having an elongated sugar chain;
(D) repeating the step (C) for one or more times to elongate a sugar chain;
(E) optionally removing unreacted sugar nucleotides and by-product nucleotides;
(F) reacting the compound having elongated a sugar chain as a result of transfer of the sugar residue with a non-soluble support having keto acid or aldehydic acid bound to a surface thereof, thereby immobilizing the compound on the surface thereof; and
(G) optionally removing reagents and enzymes used for sugar chain elongation reaction.

(20) A method for producing a glycopeptide, the method including the steps of:
   (A) performing solid phase peptide synthesis using an amino acid, glycoamino acid and keto acid or aldehydic acid, which can be cleaved by a protease, as raw materials, thereby obtaining the compound according to any one of (1) to (3);
   (B) reacting the compound obtained from the step (A) with a soluble support including a functional group selected from the group consisting of: a protected or unprotected aminooxy group; a protected or unprotected N-alkylaminooxy group; a protected or unprotected hydrazid group; a protected or unprotected azide group; a protected or unprotected thiosemicarbazide group; a protected or unprotected 1,2-dithiol group; and a protected or unprotected cysteine residue, the functional group being capable of specifically reacting with a ketone residue or aldehyde residue, and removing unreacted substances in the step (A) by reprecipitation, gel filtration, ultrafiltration or the like;
   (C) allowing glycosyltransferase to act on the compound solubly bound to the support, which has been obtained from the step (B), in the presence of a sugar nucleotide so as to cause a sugar residue to transfer from the sugar nucleotide to the compound, thereby obtaining a compound having an elongated sugar chain;
   (D) repeating the step (C) for one or more times to elongate a sugar chain;
   (E) optionally removing unreacted sugar nucleotides and by-product nucleotides;
   (F) reacting the compound having elongated a sugar chain as a result of transfer of the sugar residue with a non-soluble support having keto acid or aldehydic acid bound to a surface thereof, thereby immobilizing the compound on the surface thereof;
   (G) optionally removing reagents and enzymes used for sugar chain elongation reaction; and
   (H) allowing a protease to act on the compound having an elongated sugar chain, which has been immobilized in the step (F).
(21) The method according to (19) or (20), wherein the keto acid or aldehydic acid in the step (A) and (F) is respectively a compound represented by the following formula:

   X-C-(=O)-(CH₂)ₙ-A₁-COOH (III)

   wherein X represents a hydrogen atom, C₁-C₃₀ alkyl, C₆-C₃₀ aryl or chromophore; n represents an integer from 0 to 20; A₁ represents a linker having a length of 1 to 20 methylene groups.
(21) The method according to any one of (8) to (17), (19) or (20), wherein the glycopeptide is represented by the following formula:

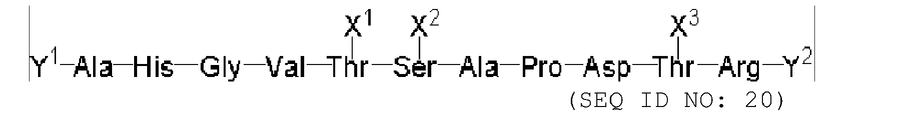

wherein X¹-X³ independently represent a hydrogen atom or a group represented by the following formula:

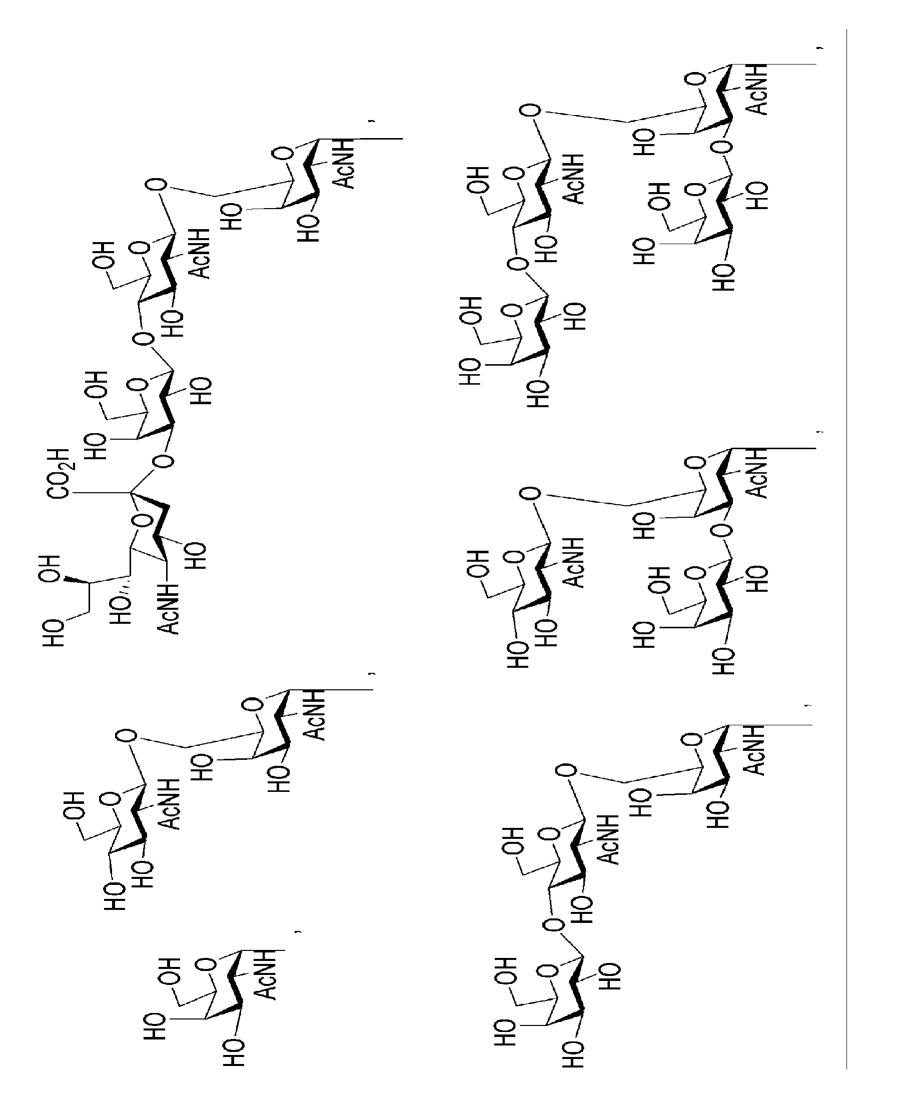

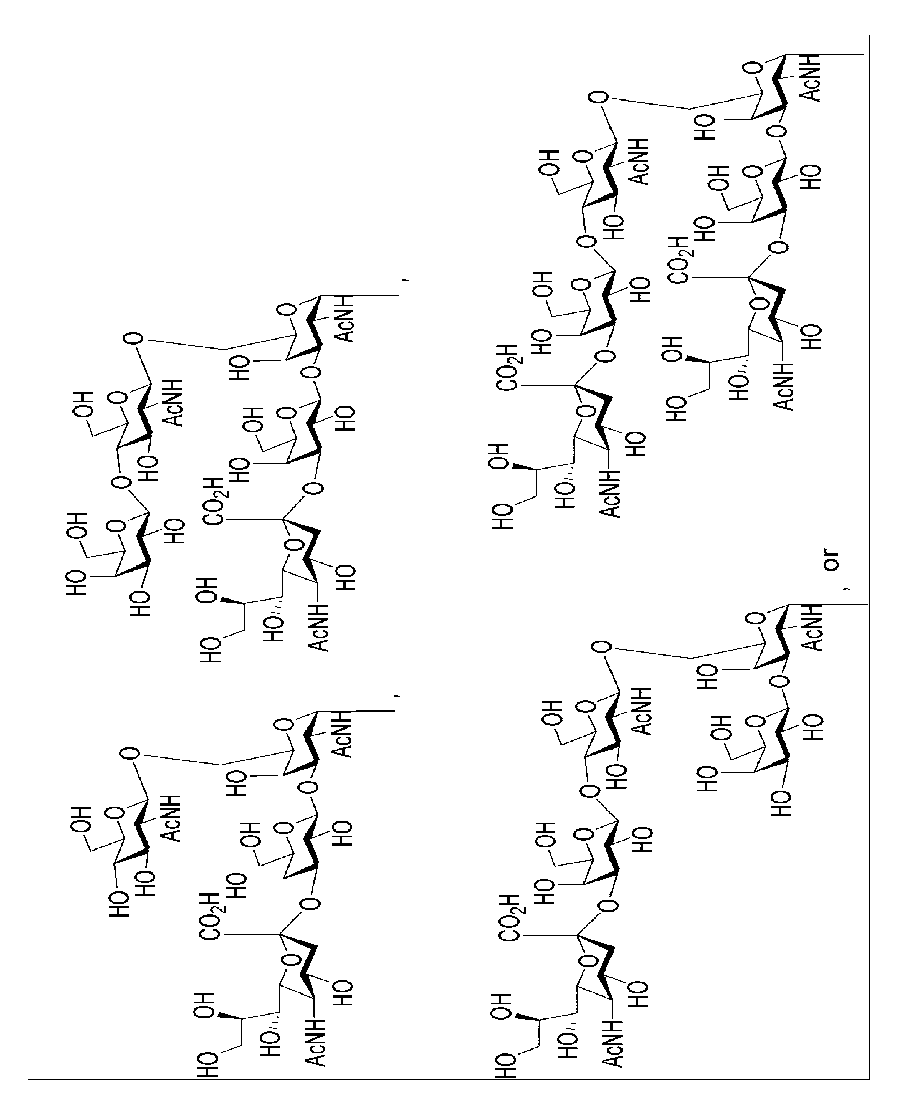

wherein Ac represents acetyl;
Y¹ represents a hydrogen atom, acetyl, acyl, alkyl or aryl; and
Y² represents hydroxyl group, NH₂, alkyl or aryl, except the case where all of X¹-X³ are hydrogen atoms.

(22) The method according to any one of (8) to (17), (19) and (20), wherein the glycopeptide is represented by the following formula:

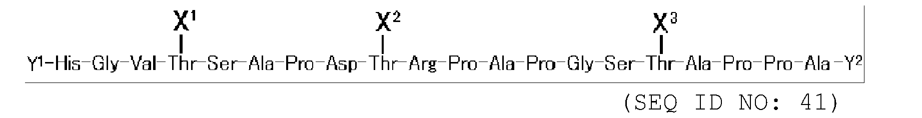

wherein X¹-X³ independently represent a hydrogen atom or a group represented by the following formula:

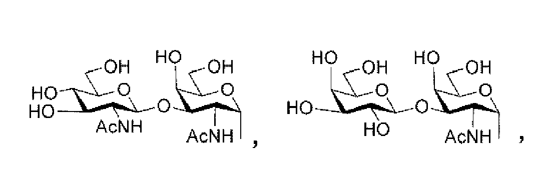

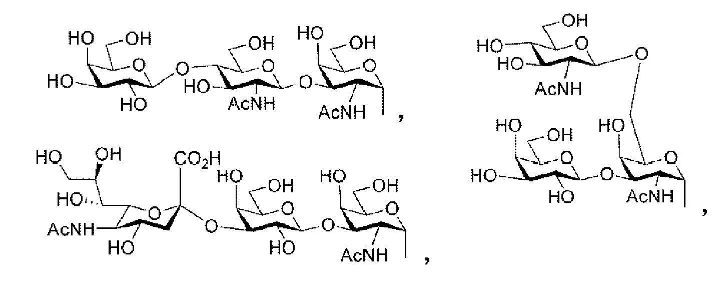

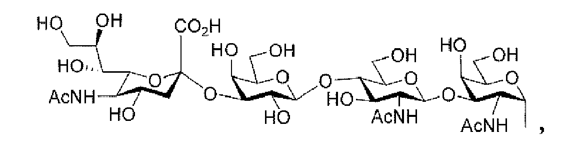

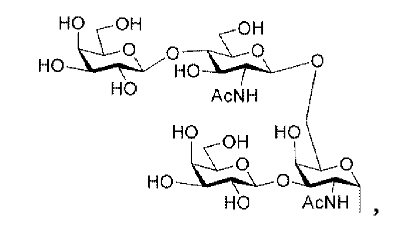

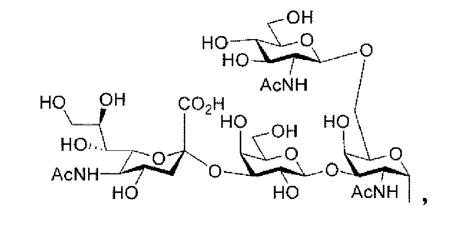

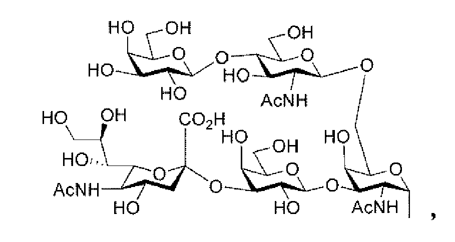

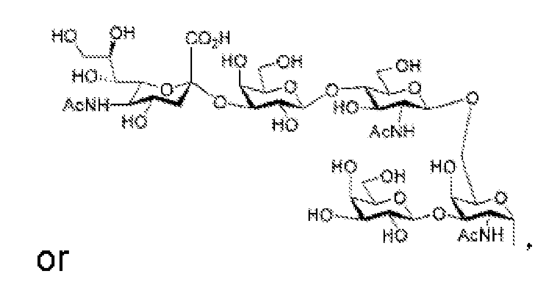

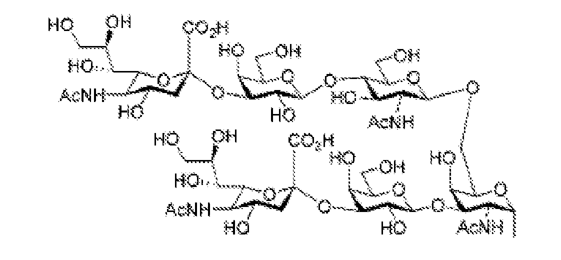

wherein Ac represents acetyl;
Y¹ represents a hydrogen atom, acetyl, acyl, alkyl or aryl;
Y² represents hydroxyl group, NH₂, alkyl or aryl, except the case where all of X¹-X³ are hydrogen atoms.

(23) The glycopeptide according to (18), represented by the formula:

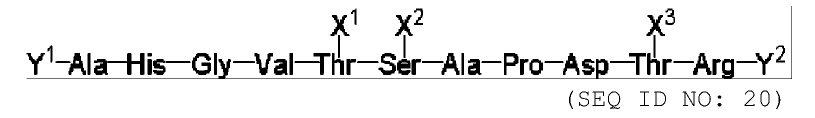

wherein X¹-X³ independently represent a hydrogen atom or a group represented by the following formula:

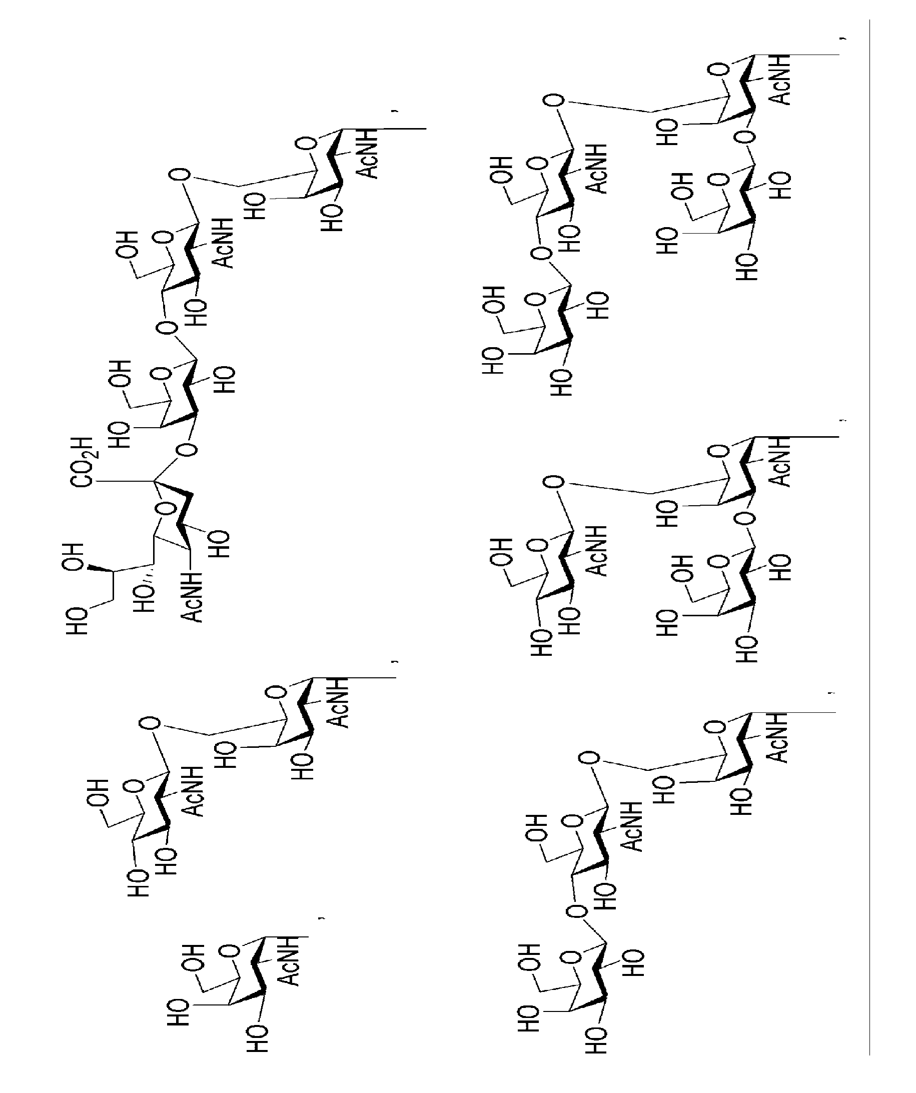

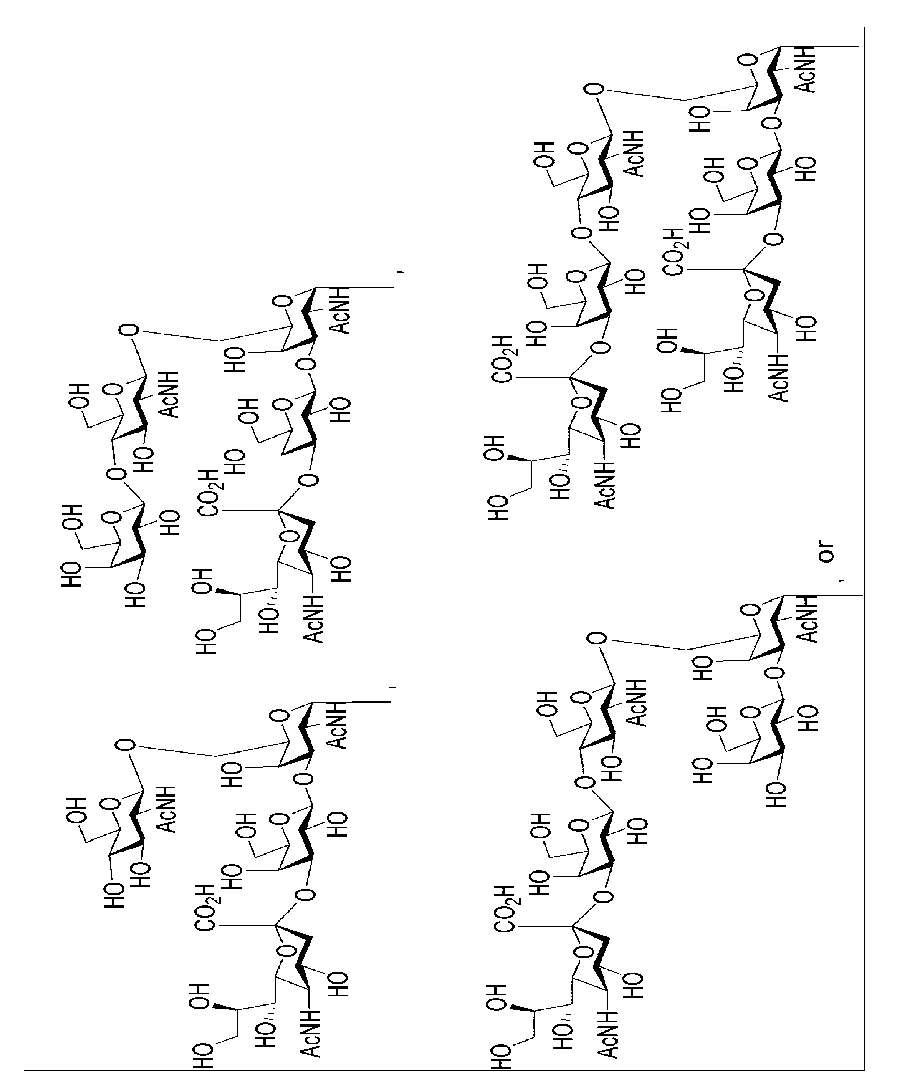

wherein Ac represents acetyl;
Y¹ represents a hydrogen atom, acetyl, acyl, alkyl or aryl;
Y² represents hydroxyl group, NH₂, alkyl or aryl, except the case where all of X¹-X³ are hydrogen atoms.

(24) The glycopeptide according to (18), represented by the formula:

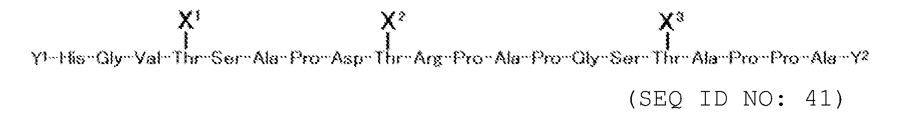

wherein X¹-X³ independently represent a hydrogen atom or a group represented by the following formula:

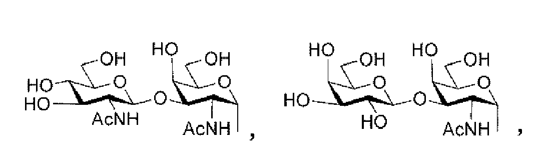

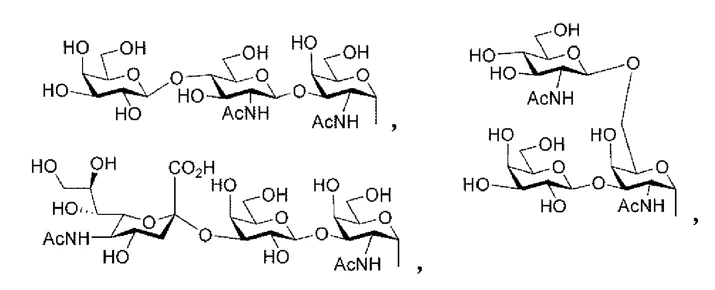

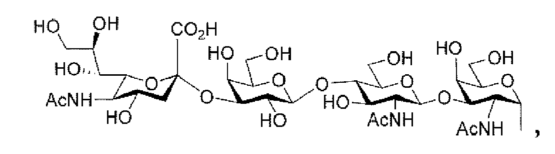

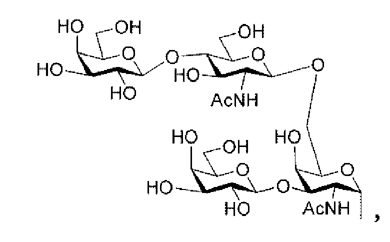

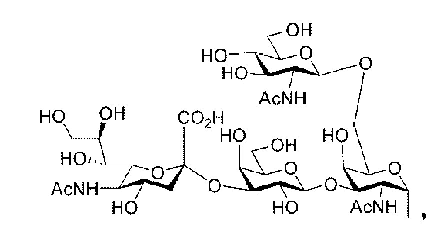

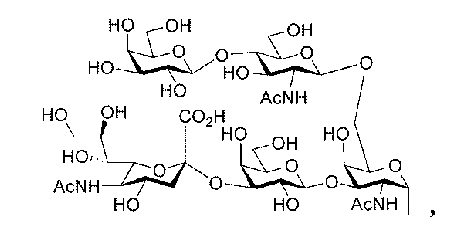

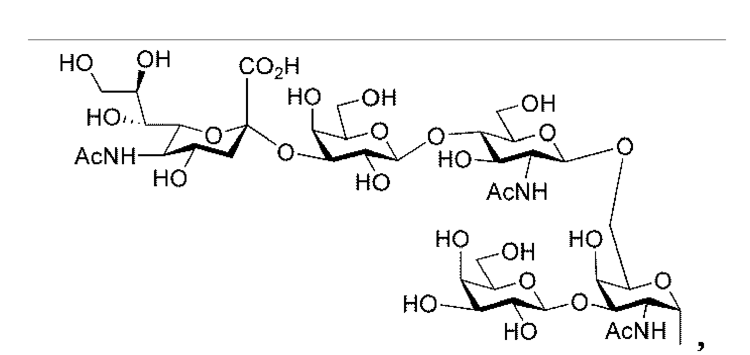

or 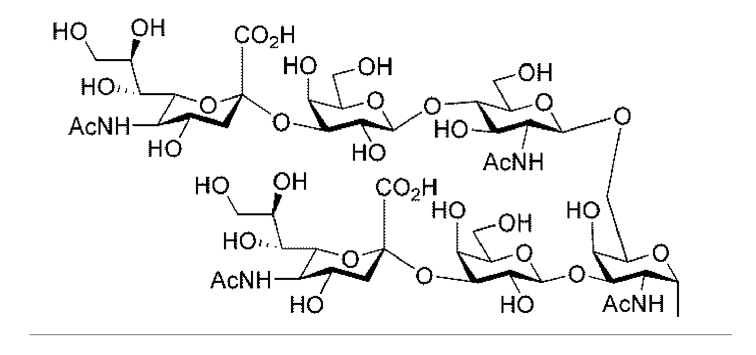 wherein Ac represents acetyl;
Y¹ represents a hydrogen atom, acetyl, acyl, alkyl or aryl;
Y² represents hydroxyl group, NH₂, alkyl or aryl, except the case where all of X¹-X³ are hydrogen atoms.

### EFFECTS OF THE INVENTION

According to the present invention, by performing sugar chain elongation after peptide synthesis using glycoamino acids which are relatively easy to prepare in glycopeptide synthesis, including monosaccharides to trisaccharides, synthesis of glycopeptides having complicated sugar chains is enabled, and further, library preparation of respective sugar chain structures as intermediates of a sugar chain elongation reaction is also enabled. Further, since sugar chain elongation reactions are performed while glycopeptides are supported on a water-soluble polymer, effects of accelerating the reaction and simplification of the manipulation of molecules are enabled, and thus automatization of a sugar chain elongation reaction is enabled. This enables preparation of glycopeptide library exhaustively having simple sugar chain structures to complicated sugar chain structures, which has been extremely difficult in conventional techniques. For example, the present invention enables synthesis of mucin-type glycopeptides which are useful in a wide range of field including materials for biochemical research, drugs and food and which has been difficult to produce in the prior art.

The obtained glycopeptide library can be used as a standard sample for structural analysis and biochemical tests. Further, it is enabled to arrange this glycopeptide library on a chip to exhaustively perform detection of glycopeptide-recognizingproteins, pathological diagnosis, search for a cell adhesion sequence, sequence analysis related to cellular growth, apotopsis and the like.

### BRIEF DESCRIPTION OF THE DRAWINGS

Figure 1 Figures 1A-D show examples of a sugar chain elongation reaction of glycopeptides and sugar chain cleavage reaction in the present invention.
Figure 2 shows a conceptual diagram of the combinatorial synthesis of compounds (97) to (162) using a distribution apparatus.

### (Explanation of Sequence Listing)

SEQ ID NOS: 1-20: partial amino acid sequence of 11 residues of mucin-type glycoprotein MUC1
SEQ ID NOS: 21-40: partial amino acid sequence of 18 residues of mucin-type glycoprotein MUC1
SEQ ID NOS: 41-60: partial amino acid sequence of 20 residues mucin-type glycoprotein MUC1
SEQ ID NOS: 61-66: examples of an amino acid sequence included in a support in a compound

### BEST MODE FOR CARRYING OUT THE INVENTION

Hereinafter, the present invention will be described. It should be understood that, throughout the specification, expressions in singular forms also include concepts of plural forms unless otherwise noted. Furthermore, it should be understood that the terms as used herein have the meanings which are generally referred to in the field unless otherwise noted.

### (Terms)

Hereinafter, definitions of the terms used herein will be listed.

As used herein, "glycoamino acid" refers to a conjugate of a sugar residue and amino acid residue, and is used interchangeably with "glycoamino acid residue".

As used herein, "glycoamino acid residue substantially free of a site which can be cleaved by a protease" refers to a glycoamino acid residue in which glycoamino acid portion is cleaved less than 50%, preferably 20%, by a protease, when treating a compound such as that represented by the above item (4) with a protease.

As used herein, "glycopeptide residue" refers to a peptide residue including at least one glycoamino acid, and is used interchangeably with "glycopeptide".

As a sugar residue forming a glycoamino acid included in the above glycopeptide residue, from a monosaccharide to trisaccharide or a derivative of a monosaccharide to trisaccharide is preferred, and a monosaccharide or a derivative of a monosaccharide is used more preferably, although not particularly limited to.

As used herein, "sugar chain" refers to a compound formed by one or more unit sugars (monosaccharide and/or derivatives thereof) in series. When there are two or more unit sugars in series, each of the unit sugars are bound by dehydrocondensation by a glycosidic bond. Such sugar chains include a wide variety of sugar chains, for example, polysaccharides (glucose, galactose, mannose, fucose, xylose, N-acetylglucosamine, N-acetylgalactosamine, sialic acid and, complexes and derivatives thereof) included in living bodies, decomposed polysaccharides, sugar chains decomposed or derived from complex living body molecules such as glycoproteins, proteoglycans, glycosaminoglycans, glycolipids, and the like, but not limited to these. Thus, as used herein, the term sugar chain is used interchangeably with "polysaccharide", "glucid", and "carbohydrate". Furthermore, unless specifically referred to, "sugar chains" as used herein may include both sugar chains and a sugar chain-containing substance.

As used herein, "monosaccharide" refers to a polyhydroxy aldehyde or polyhydroxy ketone which cannot be hydrolyzed into simpler molecules, and contains at least one hydroxyl group and at least one aldehyde group or ketone group, and derivatives thereof. Normally, monosaccharides are represented by the formula CₙH₂ₙOₙ, but is not limited thereto. Fucose (deoxyhexose),N-acetylglucosamine and the like are also included. The compounds in which, n=2, 3, 4, 5, 6, 7, 8, 9 and 10 in the above formula, are respectively referred to as diose, triose, tetrose, pentose, hexose, heptose, octose, nonose and decose. In general, the compounds correspond to aldehydes or ketones of linear polyvalent alcohols. The former are called aldoses, and the latter is called ketoses.

As used herein, particularly, "derivative of monosaccharide" refers to a substance produced as a result of substitution of one or more hydroxyl group on an unsubstituted monosaccharide by another substituent, which does not fall in the range of a monosaccharide. Such derivatives of monosaccharides include sugars having a carboxyl group (for example, aldonic acid which has the C-1 site oxidized and has became carboxylic acid (for example, D-gluconic acid having D-glucose oxidized), uronic acid having a C atom at the terminal which has become a carboxylic acid (D-glucuronic acid having D-glucose oxidized), sugar having an amino group or a derivative of an amino group (for example, acetylated amino group) (for example, N-acetyl-D-glucosamine, N-acetyl-D-galactosamine and the like), sugar having both an amino group and a carboxyl group (for example, N-acetyl neuraminic acid (sialic acid), N-acetyl muramic acid and the like), deoxylated sugar (for example, 2-deoxy-D-ribose), sulfated sugar including a sulfuric acid group, phosphorylated sugar including a phosphate group, and the like, but not limited to these. In the present specification, monosaccharides also encompass the above derivatives. Glycosides having an acetal structure formed by reacting an alcohol with a sugar forming a hemiacetal structure are also within the range of monosaccharide.

"Amino acid residue" forming a glycopeptide residue of the present invention is not particularly limited, as long as it has an amino group and a carboxyl group in the molecule . Examples of such an amino acid residues include α-amino acid residues such as Gly (glycine), Ala (alanine), Val (valine), Leu (leucine), Ile (isoleucine), Tyr (tyrosine), Trp (tryptophan), Glu (glutamic acid), Asp (aspartic acid), Lys (lysine), Arg (arginine), His (histidine), Cys (cysteine), Met (methionine), Ser (serine), Thr (threonine), Asn (asparagine), Gln (glutamine) or Pro (proline) residue, or β-amino acid residues such as β-Ala residue. An amino acid residue may be either of D-type or L-type, but L-type is preferred. As a glycopeptide residue, amino acids residues as described above or glycopeptide residue consisting of 2-30 residues are preferred. Glycopeptide residues consisting of 4-20 residues are more preferred.

The combination of glycoamino acids of the present invention as defined above is not particularly limited, as long as the amino acid residues listed above and a sugar residue can be theoretically bound. Examples of preferred combinations include Asn-(CH₂)ₙ-1αGlcNAc, Asn-(CH₂)ₙ-1βGlcNAc, Gln-(CH₂)ₙ-1αGlcNAc, Gln-(CH₂)ₙ-1βGlcNAc, Ser-1αGlcNAc, Ser-1βGlcNAc, Thr-1αGlcNAc, Thr-1βGlcNAc, Asn-1αGlcNAc, Asn-1βGlcNAc, Ser-1αGalNAc, Ser-1βGalNAc, Thr-1αGalNAc, Thr-1βGalNAc, Asn-1αGalNAc, Asn-1βGalNAc, Ser-1αGlc, Ser-1βGlc, Thr-1αGlc, Thr-1βGlc, Asn-1αGlc, Asn-1βGlc, Ser-1αGal, Ser-1βGal, Thr-1αGal, Thr-1βGal, Asn-1αGal, Asn-1βGal,Ser-1αMan, Ser-1βMan, Thr-1αMan, Thr-1βMan, Asn-1αMan, Asn-1βMan, Ser-1αGalNAc3-1βGal, Ser-1βGalNAC3-1βGal, Thr-1αGalNAc3-1βGal, Thr-1βGalNAc3-1βGal, Ser-1αGalNAc(3-1βGal)6-1βGlcNAc, Ser-1βGalNAc(3-1βGal)6-1βGlcNAc, Thr-1αGalNAc(3-1βGal)6-1βGlcNAc, Thr-1βGalNAc(3-1βGal)6-1βGlcNAc, Ser-1αGalNAc3-1βGlcNAc, Ser-1βGalNAc3-1βGlcNAc, Thr-1αGalNAc3-1βGlcNAc, Thr-1βGalNAc3-1βGlcNAc, Ser-1αGalNAc(3-1βGlcNAc)6-1βGlcNAc, Ser-1βGalNAc(3-1βGlcNAc)6-1βGlcNAc, Thr-1αGalNAc(3-1βGlcNAc)6-1βGlcNAc, Thr-1βGalNAc(3-1βGlcNAc)6-1βGlcNAc, Ser-1αGalNAc3-1αGalNAc, Ser-1βGalNAc3-1αGalNAc, Thr-1αGalNAc3-1αGalNAc, Thr-1βGalNAc3-1αGalNAc, Ser-1αGalNAc6-1βGlcNAc, Ser-1βGalNAc6-1βGlcNAc, Thr-1αGalNAc6-1βGlcNAc, Thr-1βGalNAc6-1βGlcNAc, Ser-1αGalNAc6-1αGalNAc, Ser-1βGalNAc6-1αGalNAc, Thr-1αGalNAc6-1αGalNAc, Thr-1βGalNAc6-1αGalNAc, Ser-1αGalNAc3-1αGal, Ser-1βGalNAc3-1αGal, Thr-1αGalNAc3-1αGal, Thr-1βGalNAc3-1αGal, Asn-1αGlcNAc4-1βGlcNAc, Asn-1βGlcNAc4-1βGlcNAc, Asn-1αGlcNAc4-1βGlcNAc4-1βMan, Asn-1βGlcNAc4-1βGlcNAc4-1βMan, Asn-1αGlcNAc4-1βGlcNAc4-1βMan6-1αMan, Asn-1βGlcNAc4-1βGlcNAc4-1βMan6-1αMan, Asn-1αGlcNAc4-1βGlcNAc4-1βMan3-1αMan, Asn-1βGlcNAc4-1βGlcNAc4-1βMan3-1αMan, Asn-1αGlcNAc4-1βGlcNAc4-1βMan(3-1αMan)6-1αMan, Asn-1βGlcNAc4-1βGlcNAc4-1βMan(3-1αMan)6-1αMan, Ser-1αXyl, Ser-1βXyl, Thr-1αXyl, Thr-1βXyl, Ser-1αXyl4-1βGal, Ser-1βXyl4-1βGal, Thr-1αXyl4-1βGal, Thr-1βXyl4-1βGal, Ser-1αXyl4-1βGal3-1βGal, Ser-1βXyl4-1βGal3-1βGal, Thr-1αXyl4-1βGal3-1βGal, Thr-1βXyl4-1βGal3-1βGal, Ser-1αXyl4-1βGal3-1βGal3-1βGlcA, Ser-1βXyl4-1βGal3-1βGal3-1βGlcA, Thr-1αXyl4-1βGal3-1βGal3-1βGlcA, Thr-1βXyl4-1βGal3-1βGal3-1βGlcA, Ser-1αXyl4-1βGal3-1βGal3-1βGlcA4-1αGlcNAc, Ser-1βXyl4-1βGa13-1βGa13-1βGlcA4-1αGlcNAc, Thr-1αXyl4-1βGal3-1βGal3-1βGlcA4-1αGlcNAc, Thr-1βXy14-1βGal3-1βGal3-1βGlcA4-1αGlcNAc, Ser-1αXyl4-1βGal3-1βGal3-1βGlcA3-1αGalNAc, Ser-1βXyl4-1βGal3-1βGal3-1βGlcA3-1αGalNAc, Thr-1αXyl4-1βGal3-1βGal3-1βGlcA3-1αGalNAc, Thr-1βXyl4-1βGal3-1βGal3-1βGlcA3-1αGalNAc, Ser-1αXyl4-1βGal3-1βGal3-1βGlcA4-1βGalNAc, Ser-1βXyl4-1βGal3-1βGal3-1βGlcA4-1βGalNAc, Thr-1αXyl4-1βGal3-1βGal3-1βGlcA4-1βGalNAc, Thr-1βXyl4-1βGal3-1βGal3-1βGlcA4-1βGalNAc and the like. Here, n represents an integer from 1 to 10, Gal represents galactose, Glc represents glucose, Man represents mannose, Xyl represents xylose, GlcNAc represents N-acetyl-D-glucosamine, GalNAc represents, N-acetyl-D-galactosamine and the like.

As used herein, "N-terminus" refers to a substituted or unsubstituted amino group which is located at the end of a peptide principal chain.

As used herein, "C-terminus" refers to a substituted or unsubstituted carboxyl group which is located at the end of a peptide principal chain.

As used herein, "side chain" refers to a functional group which extends from a peptide principal chain to a direction perpendicular to a direction in which the peptide principal chain extends, or a portion including the functional group.

As used herein, "primer" refers to a substance having an action which causes the initiation of an enzymatic reaction.

As used herein, "transferring enzyme" is a generic term referring to enzymes which catalyze group transferring reactions. Herein, "transferring enzyme" may be used interchangeably with "transferase". The group transfer reaction occurs so that a group Y is transferred from a compound (donor) to another compound (receptor), as shown in the following formula (1) :

X-Y+Z-H⇔X-H+Z-Y (1)

As used herein, "glycosyltransferase" refers to an enzyme which catalyzes the transfer of a sugar (corresponding to group Y in the above formula (1); a saccharide unit or a sugar chain) from one site to another site, which correspond to the compounds X-Y and Z-H, respectively in the formula (1). Examples of glycosyl transferase include, but not limited to, for example, galactosyltransferase, glucosyltransferase, sialyltransferase, mannosyltransferase, fucosyltransferase, xylosyltransferase, N-acetylglucosaminyltransferase, N-acetylgalactosaminyltransferase and the like.

As used herein, "sugar chain elongation reaction" refers to a reaction where a chain length of a sugar chain elongates in the presence of a glycotransferase as defined above.

As used herein, the term "biomolecule" refers to a molecule related to living bodies. A sample including such biomolecules may be referred to as a biological sample in the present specification. As used herein, "living body" refers to a biological organic body, and includes animals, plants, fungi, virus and the like, but are not limited to these. Therefore, a biomolecule includes molecules extracted from living bodies. However, it is not limited to this, and any molecule may fall within the definition of biomolecule as long as it can affect living bodies. Such biomolecules includes proteins, polypeptides, oligopeptides, peptides, glycopeptides, polynucleotides oligonucleotides, nucleotides, sugar nucleotides, nucleic acids (including, for example, DNA such as cDNA and genomic DNA, and RNA such as mRNA), polysaccharides, oligosaccharides, lipid, small molecules (for example, hormones, ligands, signaling substances, organic small molecules and the like), complex molecules thereof, and the like, but not limited to these. As used herein, the biomolecules maybe, preferably, complex molecules including sugar chains, or sugar chains (for example, glycoproteins, glycolipids and the like).

The source of such a biomolecule is not particularly limited as long as it is a material to which sugar chains derived from living organisms are bound or attached. It may be animal, plant, bacterial, or viral. A biological sample derived from an animal is preferable. For example, whole blood, blood plasma, blood serum, sweat, saliva, urine, pancreatic fluid, amnionic fluid, cerebrospinal fluid and the like are preferable. More preferably, it may be blood plasma, blood serum, or urine. The biological sample includes a biological sample which has not previously been isolated from a subject. The biological sample may include, for example, mucosal tissue or glandular tissue to which a sample can be attached from the outside, and preferably, the epithelium of ductal tissue attached to the mammary glands, prostate, or pancreas.

As used herein, the terms "protein", "polypeptide", "oligopeptide" and "peptide" have the same meaning in the present specification and refer to a polymer of an amino acid having any length. This polymer may be straight, branched or cyclic. An amino acid may be natural or unnatural, and may be a modified amino acid. These terms may encompass those assembled with a complex of a plurality of polypeptide chains. These terms further encompass a naturally occurring or artificially modified amino acid polymer. Examples of such a modification include, for example, formation of a disulfide bond, glycosylation, lipidation, acetylation, phpophorylation, or any other manipulation or modification (for example, conjugation with a label component). The definition also encompasses, for example, a polypeptide including one or two or more analog(s) of (including, for example, an unnatural amino acid and the like) peptide-like compounds (for example, peptoid) and other modifications known in the art.

As used herein, "sugar nucleotide" refers to a nucleotide to which a sugar residue as defined above is bound. A sugar nucleotide used in the present invention is not particularly limited, as long as it can be used by the above enzyme. Examples of such a sugar nucleotide include uridine-5'-diphosphate galactose, uridine-5'-diphosphate-N-acetylglucosamine, uridine-5'-diphosphate-N-acetylgalactosamine, uridine-5'-diphosphate glucuronic acid, uridine-5'-diphosphate xylose, guanosine-5'-diphosphate fucose, guanosine-5'-diphosphate mannose, cytidine-5'-monophosphate-N-acetylneuraminic acid and sodium salts thereof and the like.

### (Organic chemistry)

Organic chemistry is described in, for example, Organic Chemistry, R. T. Morrison, R. N. Boyd 5th ed. (1987) and the like, relevant portions of which are incorporated herein as a reference.

As used herein, "substitution" refers to substituting one or two or more hydrogen atom (s) in an organic compound or a substituent with another atom or atom group, if not particularly mentioned. It is possible to substitute one hydrogen atom with a monovalent substituent, and to substitute two hydrogen atoms with a bivalent substituent.

As used herein, "alkyl" refers to a monovalent group generated when one hydrogen atom is lost from aliphatic hydrocarbon (alkane) such as methane, ethane, propane, and the like, and is represented by CₙH₂ₙ₊₁- in general (herein, n is a positive integer). Alkyl may be a straight chain or abranchedchain. As used herein, "substituted alkyl" refers to an alkyl having one or more hydrogen atoms independently substituted with a substituent as defined below. Specific examples of such alkyls may be, C1-C2 alkyl, C1-C3 alkyl, C1-C4 alkyl, C1-C5 alkyl, C1-C6 alkyl, C1-C7 alkyl, C1-C8 alkyl, C1-C9 alkyl, C1-C10 alkyl, C1-C11 alkyl or C1-C12 alkyl, C1-C15 alkyl, C1-C20 alkyl, C1-C25 alkyl or C1-C30 alkyl. Herein, for example, C1-C10 alkyl denotes straight chain or branched alkyl having 1-10 carbon atoms, and examples may be methyl (CH₃-), ethyl (C₂H₅-), n-propyl (CH₃CH₂CH₂-), isopropyl ((CH₃)₂CH-), n-butyl (CH₃CH₂CH₂CH₂-), n-pentyl (CH₃CH₂CH₂CH₂CH₂-), n-hexyl (CH₃CH₂CH₂CH₂CH₂CH₂-), n-heptyl (CH₃CH₂CH₂CH₂CH₂CH₂CH₂-), n-octyl (CH₃CH₂CH₂CH₂CH₂CH₂CH₂CH₂-), n-nonyl (CH₃CH₂CH₂CH₂CH₂CH₂CH₂CH₂CH₂-), n-decyl (CH₃CH₂CH₂CH₂CH₂CH₂CH₂CH₂CH₂CH₂-), -C(CH₃)₂CH₂CH₂CH(CH₃)₂, -CH₂CH(CH₃)₂ or the like.

As used herein, "aryl" refers to a monovalent aromatic hydrocarbon radical having 6 to 30 carbon atoms, which is derivated by removing one hydrogen atom from one carbon atom of a parent aromatic ring system. Representative aryl groups include, but not limited to, benzene, naphthalene, anthracene, biphenyl and the like.

As used herein, "chromophore" refers to a functional group having an absorption band in the ultraviolet light or visible light range, or a functional group which is excited by an electromagnetic wave in the ultraviolet light or visible light range to emit radiated light in a visible light range. Example of chromophores include, but not limited to, nitro groups, benzyl groups, thiophenyl groups, paranitrophenyl groups, 2,4-dinitrophenyl, dansyl groups, 2-aminobenzyl groups, fluorescein isothiocyanate (FITC) groups, 4-methoxy-β-naphthylamide groups and the like.

As used herein, "keto acid" generically refers to compounds having a carboxyl group and a carbonyl group of ketone.

As used herein, "aldehydic acid" generically refers to compounds having a carboxyl group and carbonyl group of aldehyde.

Such keto acid or aldehydic acid is, for example, a compound represented by X-C-(=O)-(CH₂)ₙ-A₁-COOH (III) wherein X represents a hydrogen atom, C₁-C₃₀ alkyl, C₆-C₃₀ aryl or chromophore; n represents an integer from 0 to 20; A₁ represents a linker having a length of 1 to 20 methylene groups.

As used herein, "protection reaction" refers to a reaction to add a protecting group such as Boc (t-buthoxycarbonyl group) to a functional group which is desired to be protected. By protecting a functional group with a protecting group, the reaction of a functional group having high reactivity can be suppressed, and only a functional group having lower reactivity reacts.

As used herein, "deprotection reaction" refers to a reaction to disengage a protecting group such as Boc. The deprotection reaction may be a reaction such as a reaction using trifluoroacetic acid (TFA) or a reduction reaction using Pd/C.

Typical examples of "protecting group" used herein include, for example, fluorenyl methoxy carbonyl group (Fmoc), acetyl group, benzyl group, benzoyl group, t-buthoxycarbonyl group, t-butyldimethyl group, silyl group, trimethylsilylethyl ethyl group, N-phthalimidyl group, trimethylsilylethyl oxycarbonyl group, 2-nitro-4,5-dimethoxy benzyl group, 2-nitro-4,5-dimethoxy benzyloxycarbonyl group, carbamate group and the like. A protecting group can be used for protecting a reactive functional group such as, for example, amino group, carboxyl group and the like. Various protecting groups can be used properly depending on conditions or purposes of the reaction. As a protecting group for an aminooxy group andN-alkylaminoxy group, a trimethylsilylethyl oxycarbonyl group, 2-nitro-4,5-dimethoxy benzyloxycarbonyl group or derivatives thereof are preferable.

In the methods of the present invention, intended products may be isolated by removing foreign substances (unreacted raw material, by-product, solvent and the like) from a reaction solution using a method commonly used in the field of art (for example, extraction, distillation, washing, concentration, precipitation, filtration, drying or the like), and then combining after treatment methods commonly used in the field of art (for example, adsorption, dissolution, elution, distillation, precipitation, deposition, chromatography, or the like).

### (General techniques used in the present specification)

The techniques used in the present specification are, unless otherwise noted specifically, well-known commonly used techniques in organic chemistry, biochemistry, genetic engineering, molecular biology, microbiology, genetics and related fields within the technical range of the field of art. Such techniques are sufficiently disclosed in, for example, documents which will be listed below and documents cited in other parts of the present specification.

Molecular biological methods, biochemical methods, microbiological methods used in the present specification are those well-known and commonly used in the art, and are disclosed in, for example: Maniatis, T. et al. (1989) Molecular Cloning: A Laboratory Manual, Cold Spring Harbor and 3rd Ed. Thereof (2001); Ausubel, F.M., et al. eds, Current Protocols in Molecular Biology, John Wiley & Sons Inc., NY, 10158 (2000); Innis, M.A. (1990) .PCR Protocols: A Guide to Methods and Applications, Academic Press; Innis, M.A. et al. (1995) PCR Strategies, Academic Press; Sninsky, J.J. et al. (1999) PCR Applications: Protocols for Functional Genomics, Academic Press; Gait, M.J. (1985) Oligonucleotide Synthesis: A Practical Approach, IRL Press; Gait, M. J. (1990) Oligonucleotide Synthesis: A Practical Approach, IRL Press; Eckstein, F. (1991) Oligonucleotides and Analogues: A Practical Approach, IRL Press; Adams, R.L. et al. (1992) The Biochemistry of the Nucleic Acids, Chapman & Hall; Shabarova, Z. et al. (1994) Advanced Organic Chemistry of Nucleic Acids, Weinheim; Blackburn, G.M. et al. (1996) Nucleic Acids in Chemistry and Biology, Oxford University Press; Hermanson, G.T. (1996) Bioconjugate Techniques, Academic Press; Method in Enzymology 230, 242, 247, Academic Press, 1994;Bessatsu JikkennIgaku(Separate volume of Experimental Medicine) "Idenshidonyu & Hatsugen Kaiseki Jikkenho (Gene Introduction & Expression Analysis Experimental Method)", Yodosha Co. Ltd., 1997; Hatanaka, Nishimura, et.al., "Toshitsu no Kagaku to Kogaku (Science and engineering of Glucids)", Kodansha ScientificKK, 1997; Tosabunshi no Sekkei to Seirikino (Design and Physiology of Sugar Chain Molecules), Chemical Society of Japan ed., Japan Scientific Societies Press, 2001; and the like. The relevant portions (these may be the entirety) of these documents are herein incorporated.

### (Description of preferable embodiments)

Hereinafter, preferable embodiments of the present invention will be described. It is appreciated that the embodiments as described below are provided only for better understanding of the present invention, and that the scope of the present invention should not be limited to the following description. Therefore, it is apparent that those skilled in the art can properly modify the invention within the scope of the present invention by considering the description in the present specification.

According to one aspect, the present invention provides a compound represented by the following formula:

X-C(=O)-(CH₂)ₙ-A₁-A₂-A₃ (I)

wherein X represents a hydrogen atom, C₁-C₃₀ alkyl, C₆-C₃₀ aryl or a chromophore; n represents an integer from 0 to 20; A₁ represents -(CH₂)₀₋₂₀-C(=O)-, -(CH₂CH₂O)₁₋₁₀-, oligoacrylamide or polyacrylamide having a degree of polymerization of 1 to 10, oligopeptide or polypeptide having a degree of polymerization of 1 to 10, an oxygen atom or NH; A₂ represents an amino acid residue which can be cleaved by a protease; and A₃ represents a glycoamino acid residue substantially free of a site which can be cleaved by a protease, or a glycopeptide residue free of a site which can be cleaved by a protease and including a glycoamino acid. By using such a compound as a primer, purification of glycopeptides which has conventionally required multi-step purification, and glycopeptides can be rapidly produced with a high yield. The compound of the above formula (I) of the present invention necessarily has an aldehyde group or ketone group at the end. Therefore, by reacting the compound with a support including a functional group selected from the group consisting of: a protected or unprotected aminooxy group; a protected or unprotected N-alkylaminooxy group; a protected or unprotected hydrazid group; a protected or unprotected azide group; a protected or unprotected thiosemicarbazide group; a protected or unprotected 1,2-dithiol group; and a protected or unprotected cysteine residue, the compound of the above formula (I) can be supported on the support and can be used as a polymeric primer. Since bonding obtained from this reaction is a strong bonding which is not decomposed under subsequent hydrolytic conditions (such as pH conditions) by a protease, there is an advantage that purification by the hydrolozate is quite simple.

As combination of protease used in hydrolysis in the present invention and an amino acid residue (A₂) which can be cleaved by this protease, any combination may be used, as long as bonding which is caused by reaction between at least the above aldehyde group or ketone group at the end of the compound of the above formula (I) and the above support is not decomposed within a pH range where hydrolysis by a protease may occur. Proteases which recognize a part or all of a polypeptide of A₁ and a peptide consisting of an amino acid residue of A₂ may also be used. Examples of such a combination include: combination of a protease derived from *Bacillus Licheniformis* (glutaminidase) and a glutamic acid residue or cysteine residue which can be cleaved by such a protease; combination of an asparaginyl endopeptidase and Asn (recognition site (A₂)) (the C-terminus of asparagine (Asn) is cleaved); combination of an arginyl endopeptidase and Arg (recognition site (A₂)) (the C-terminus of arginine (Arg) is cleaved) ; combination of an *Achromobacter* protease I and lysine (Lys) (recognition site (A₂)) (the C-terminus of lysine (Lys) is cleaved); combination of a trypsin and arginine (Arg) or lysine (Lys) (recognition site (A₂)) (when Arg is recognized, the C-terminus of arginine (Arg) is cleaved, and when lysine (Lys) is recognized, the C-terminus of lysine (Lys) is cleaved; combination of a chymotrypsin and Phe, Tyr or Trp (recognition site (A₂)) (when Phe is recognized, the C-terminus of phenylalanine (Phe) is cleaved, when Tyr is recognized, the C-terminus of tyrosine is cleaved, and when Trp is recognized, the C-terminus of tryptophan (Trp) is cleaved) ; combination of a V8 protease and Glu (recognition site (A₂)) (the C-terminus of glutamic acid (Glu) is cleaved) ; combination of factor Xa and -Ile-Glu-Gly-Arg- (recognition site: according to the definition described herein, recognition site (A₂) is arginine (Arg), and -Ile-Glu-Gly-is the end of A₁; and the C-terminus of arginine (Arg) is cleaved); and combination of an enterokinase and -Asp-Asp-Asp-Asp-Lys- (recognition site: according to the definition described herein, recognition site (A₂) is lysine (Lys), and -Asp-Asp-Asp-Asp- is the end of A₁; and the C-terminus of lysine (Lys) is cleaved). As such combination, combination of a protease derived from *Bacillus Licheniformis* (glutaminidase (for example, glutamic acid residue-specific protease derived from *Bacillus Licherformis* (BLase: available from Shionogi & Co., Ltd.))) and a glutamic acid residue or cysteine residue which can be cleaved by such protease is preferred. BLase can be produced in accordance with a method described in Japanese Laid-Open Patent Publication No. 4-166085 (Japanese Patent No. 3046344). BLase is produced from *Bacillus* bacteria, in particular, *Bacillus Licherformis* ATCC 14580 strain. This bacterial strain can be obtained from the American Type Culture Collection (ATCC). If necessary, a genomic DNA of *Bacillus Licherformis* ATCC 14580 strain can be prepared from cultured cells of this bacterial strain in accordance with known methods (M. Stahl et al., Journal of Bacteriology, 154, 406-412 (1983)).

In a preferred embodiment of the present invention, at least a part of A₃ included in the compound of the above formula (I) has an amino acid sequence selected from the group consisting of the amino acid sequences as set forth in the following SEQ ID NOS: 1-60 derived from mucin-type glycoprotein MUC1:
HGVTSAPDTRP (SEQ ID NO: 1),
GVTSAPDTRPA (SEQ ID NO: 2),
VTSAPDTRPAP (SEQ ID NO: 3);
TSAPDTRPAPG (SEQ ID NO: 4);
SAPDTRPAPGS (SEQ ID NO: 5);
APDTRPAPGST (SEQ ID NO: 6);
PDTRPAPGSTA (SEQ ID NO: 7);
DTRPAPGSTAP (SEQ ID NO: 8);
TRPAPGSTAPP (SEQ ID NO: 9);
RPAPGSTAPPA (SEQ ID NO: 10);
PAPGSTAPPAH (SEQ ID NO: 11);
APGSTAPPAHG (SEQ ID NO: 12);
PGSTAPPAHGV (SEQ ID NO: 13);
GSTAPPAHGVT (SEQ ID NO: 14);
STAPPAHGVTS (SEQ ID NO: 15);
TAPPAHGVTSA (SEQ ID NO: 16);
APPAHGVTSAP (SEQ ID NO: 17);
PPAHGVTSAPD (SEQ ID NO: 18);
PAHGVTSAPDT (SEQ ID NO: 19);
AHGVTSAPDTR (SEQ ID NO: 20);
HGVTSAPDTRPAPGSTAP (SEQ ID NO: 21);
GVTSAPDTRPAPGSTAPP (SEQ ID NO: 22);
VTSAPDTRPAPGSTAPPA (SEQ ID NO: 23);
TSAPDTRPAPGSTAPPAH (SEQ ID NO: 24);
SAPDTRPAPGSTAPPAHG (SEQ ID NO: 25);
APDTRPAPGSTAPPAHGV (SEQ ID NO: 26);
PDTRPAPGSTAPPAHGVT (SEQ ID NO: 27);
DTRPAPGSTAPPAHGVTS (SEQ ID NO: 28);
TRPAPGSTAPPAHGVTSA (SEQ ID NO: 29);
RPAPGSTAPPAHGVTSAP (SEQ ID NO: 30);
PAPGSTAPPAHGVTSAPD (SEQ ID NO: 31);
APGSTAPPAHGVTSAPDT (SEQ ID NO: 32);
PGSTAPPAHGVTSAPDTR (SEQ ID NO: 33);
GSTAPPAHGVTSAPDTRP (SEQ ID NO: 34);
STAPPAHGVTSAPDTRPA (SEQ ID NO: 35);
TAPPAHGVTSAPDTRPAP (SEQ ID NO: 36);
APPAHGVTSAPDTRPAPG (SEQ ID NO: 37);
PPAHGVTSAPDTRPAPGS (SEQ ID NO: 38);
PAHGVTSAPDTRPAPGST (SEQ ID NO: 39);
AHGVTSAPDTRPAPGSTA (SEQ ID NO 40);
HGVTSAPDTRPAPGSTAPPA (SEQ ID NO: 41);
GVTSAPDTRPAPGSTAPPAH (SEQ ID NO 42);
VTSAPDTRPAPGSTAPPAHG (SEQ ID NO 43);
TSAPDTRPAPGSTAPPAHGV (SEQ ID NO 44);
SAPDTRPAPGSTAPPAHGVT (SEQ ID NO 45);
APDTRPAPGSTAPPAHGVTS (SEQ ID NO: 46);
PDTRPAPGSTAPPAHGVTSA (SEQ ID NO 47);
DTRPAPGSTAPPAHGVTSAP (SEQ ID NO 48);
TRPAPGSTAPPAHGVTSAPD (SEQ ID NO 49);
RPAPGSTAPPAHGVTSAPDT (SEQ ID NO: 50);
PAPGSTAPPAHGVTSAPDTR (SEQ ID NO: 51);
APGSTAPPAHGVTSAPDTRP (SEQ ID NO: 52);
PGSTAPPAHGVTSAPDTRPA (SEQ ID NO: 53);
GSTAPPAHGVTSAPDTRPAP (SEQ ID NO 54);
STAPPAHGVTSAPDTRPAPG (SEQ ID NO 55);
TAPPAHGVTSAPDTRPAPGS (SEQ ID NO 56);
APPAHGVTSAPDTRPAPGST (SEQ ID NO 57);
PPAHGVTSAPDTRPAPGSTA (SEQ ID NO 58);
PAHGVTSAPDTRPAPGSTAP (SEQ ID NO 59); and
AHGVTSAPDTRPAPGSTAPP (SEQ ID NO: 60).
Further, the amino acid sequence may be an amino acid sequence derived from a mucin-type protein which includes two or three repetitions of any one of the amino sequences set forth in SEQ ID NOS: 41-60.

A polymeric support which can be used in the present invention is not particularly limited, as long as it is capable of binding to a group represented by the formula (I) and the action of glycosyl transferase as will be described below can cause a further sugar residue to transfer to a sugar residue of the group represented by the formula (I) after binding. Examples of such a polymeric support include: a polymer or copolymer of a vinyl-type monomer having a a protected or unprotected amiooxy group or a protected or unprotected hydrazide group (the above vinyl-type monomer includes acrylamides, methacrylamides, acrylic acids, methacrylic acids, styrenes, fatty acid vinylesters and the like) or polyethers which may have a protected or unprotected aminooxy group or a protected or unprotected hydrazide group; a silica support, a resin support, magnetic beads or a metallic support, having a protected or unprotected aminooxy group or a protected or unprotected hydrazide group (examples thereof include a silica support, a resin support, magnetic beads or metallic support represented by the following formula:

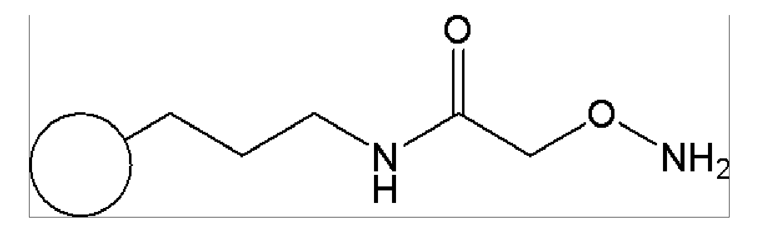

wherein ○ represents a silica, resin, magnetic beads or metal) ; a support similar to a support in Maps (Multiple Antigen peptide systems) method used in peptide synthesis; and a compound represented by the following formula:
[(NH₂OCH₂C(=O))₂-Lys]₂-Lys-NHCH₂CH₂C(=O)-R³,
[(NH₂OCH₂C(=O))₂-Lys]₂-Lys-NHCH(CH₂SH)C(=O)-R³,
[(NH₂OCH₂C(=O))₂-Lys]₂-Lys-Cys-NHCH₂CH₂C(=O)-R³ (SEQ ID NO: 61);
{[(NH₂OCH₂C(=O))₂-Lys]₂-Lys-NHCH[C(=O)-R³]CH₂-S}₂,
{[(NH₂OCH₂C(=O))₂-Lys₂-Lys-NHCH[C(=O)NHCH₂CH₂C(=O)-R³ ]CH₂-S}₂,
{[(NH₂OCH₂C(=O))₂-Lys]₂-Lys}₂-Lys-NHCH₂CH₂C(=O)-R³ (SEQ ID NO: 62);,
{[(NH₂OCH₂C(=O))₂-Lys]₂-Lys}₂-Lys-NHCH(CH₂SH)C(=O)-R³ (SEQ ID NO: 63);,
{[(NH₂OCH₂C(=O))₂-Lys]₂-Lys}₂-Lys-Cys-N
HCH₂CH₂C(=O)-R³ (SEQ ID NO: 64);,
[[[(NH₂OCH₂C(=O))₂-Lys]₂-Lys)₂-Lys-NHCH[C(=O)-R³]CH₂-S ]₂ (SEQ ID NO: 65);
[[[(NH₂OCH₂C(=O))₂-Lys]₂-Lys]₂-Lys-NHCH[C(=O)NHCH₂CH₂C (=O)-R³]CH₂-S]₂ (SEQ ID NO: 66);

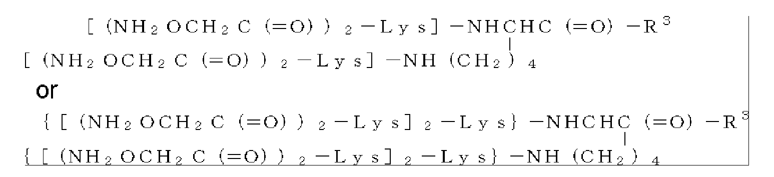

wherein R₃ represents a hydroxyl group or amino group, Lys represents lysine and Cys represents cysteine, and the like.

The above polymer or copolymer of a vinyl-type monomer having a protected or unprotected amiooxy group or a protected or unprotected hydrazide group is prepared by a method in which at least a part of a polymer or copolymer of a vinyl-type monomer without substitution is substituted with a protected or unprotected aminooxy group or a protected or unprotected hydrazide group, or a method in which a vinyl-type monomer having a protectedor unprotected aminooxy group or a protected or unprotected hydrazide group is polymerized or copolymerized.

Examples of an acrylamide as described above include acrylamides and N-alkylacrylamides such as N-ethylacrylamide, N-isopropylacrylamide and the like, which may have a protected or unprotected aminooxy group or a protected or unprotected hydrazide group.

Examples of an methacrylamide as described above include methacrylamides and N-alkylmethacrylamides such as N-ethylmethacrylamide, N-isopropylmethacrylamide and the like, which may have a protected or unprotected aminooxy group or a protected or unprotected hydrazide group.

Examples of acrylic acids as described above include acrylic acid and acrylic acid esters such as methyl acrylate, ethyl acrylate, hydroxyethyl acrylate, dimethylaminoethyl acrylate and the like, which may have a protected or unprotected aminooxy group or a protected or unprotected hydrazide group.

Examples of methacrylic acids as described above include methacrylic acid and methacryl acid esters such as methyl methacrylate, ethyl methacrylate, hydroxyethyl methacrylate, dimethylaminoethyl methacrylate and the like, which may have a protected or unprotected aminooxy group or a protected or unprotected hydrazide group.

Examples of styrenes as described above include styrene, p-hydroxystyrene, p-hydroxymethylstyrene and the like, which may have a protected or unprotected aminooxy group or a protected or unprotected hydrazide group.

Examples of fatty acid vinylesters as described above include vinyl acetate, vinyl butyrate and the like, which may have a protected or unprotected aminooxy group or a protected or unprotected hydrazide group. Further, a polymer or copolymer of fatty acid vinyl ester in the present invention also includes those which are obtained by hydrolyzing all or a part of ester bond by alkali or the like after polymerization reaction.

Examples of polyethers as described above include polyethylene glycol which may have a protected or unprotected aminooxy group or a protected or unprotected hydrazide group, polyethylene glycol having a substitution with alkyl or aryl group, which may have a protected or unprotected aminooxy group or a protected or unprotected hydrazide group, and the like.

A polymeric support herein may be either water-insoluble or water-soluble, but is preferably water-soluble. The general molecular weight is approximately 10000 to approximately 5000000, preferably 20000 to 2000000, and more preferably 50000 to 1000000. In the case of a water-insoluble support, a form thereof may be, but not particularly limited to, beads-shape, fiber-shape, membrane-shape, film-shape or the like.

Examples of a more preferable support include polymeric supports represented by the following formula:

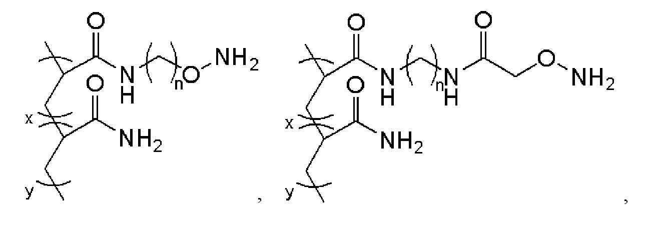

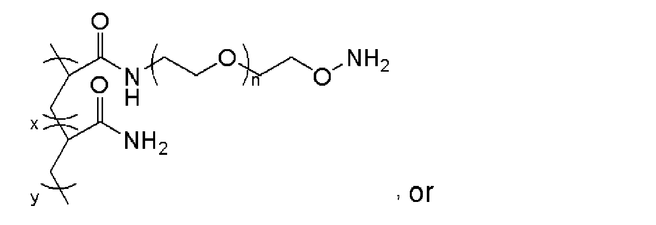

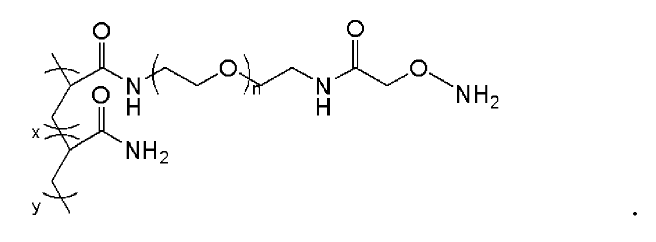

Here, n is an integer from 1 to 15, preferably 1 to 10, and more preferably 1 to 5. The ratio x: y is 1:0 to 1:1000, preferably 1:0 to 1:100. The molecular weight of a polymeric support is approximately 10000 to approximately 5000000, preferably 20000 to 2000000, and more preferably 50000 to 1000000.

In another preferred embodiment, the present invention provides a compound represented by the following formula:

A₄-N=C (-X) - (CH₂)ₙ-A₁-A₂-A₃ (II)

wherein X represents a hydrogen atom, C₁-C₃₀ alkyl, C₆-C₃₀ aryl or a chromophore;
n represents an integer from 0 to 20;
A₁ represents -(CH₂)₀₋₂₀-C(=O)-, -(CH₂CH₂O)₁₋₁₀-, oligoacrylamide or polyacrylamide having a degree of polymerization of 1 to 10, oligopeptide or polypeptide having a degree of polymerization of 1 to 10, an oxygen atom or NH;
A₂ represents a glutamic acid residue or cysteine residue which can be cleaved by a protease derived from *Bacillus Licheniformis;*
A₃ represents a glycoamino acid residue substantially free of a site which can be cleaved by a protease, or a glycopeptide residue free of a site which can be cleaved by a protease and including a glycoamino acid;
A₄ is a group represented by the following formula:

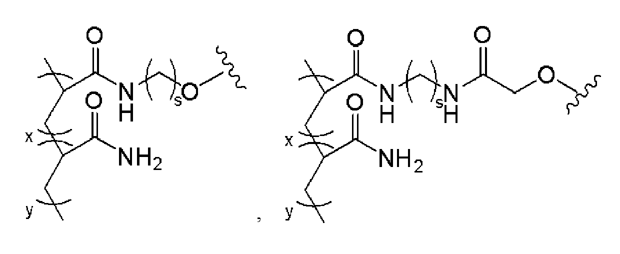

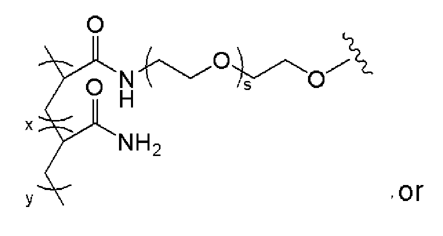

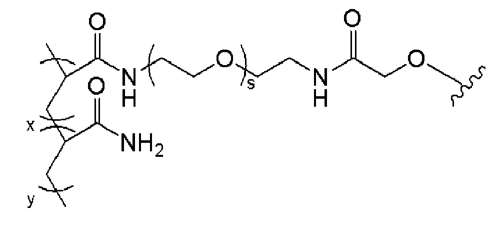

wherein s is an integer of 1 to 15 and x:y is 1:0 to 1:1000.

According to another aspect, the present invention provides a composition including a compound as set forth in the above formula (I) or (II), for a primer used for producing glycoamino acid or glycopeptide.

Synthesis and purification of a compound of the present invention which is useful as a primer for producing glycopeptides is performed in accordance with the following procedure:
1) performing solid phase peptide synthesis using a protected amino acid (including an amino acid residue which can be cleaved by a protease), previously synthesized glycoamino acid having a protective group, and keto acid or aldehydic acid, as raw materials, thereby synthesizing a glycopeptide (with a sugar chain and amino acid protected) having a ketone residue or aldehyde residue at the end and including an amino acid residue which can be cleaved by a protease (and optionally performing a capping reaction after each step of the amino acid coupling reaction, that is, a reaction for inactivating unreacted substances in amino acid coupling);
2) separating the glycopeptide having a ketone residue or aldehyde residue at the end and including an amino acid residue which can be cleaved by a protease from the solid-phase support, and simultaneously deprotecting a protective group of an amino acid side chain by acid treatment (if the protective group of the amino acid side chain is not eliminated by acid treatment, the protective group may be deprotected by a separate deprotection reaction);
3) purifying a reaction solution or a mixture obtained by ether precipitation method by HPLC, thereby isolating the glycopeptide (with a sugar chain protected) having a ketone residue or aldehyde residue at the end and including an amino acid residue which can be cleaved by a protease;
4) deprotecting the protective group of the sugar chain; and
5) performing purification by HPLC, thereby isolating the glycopeptide (with a sugar chain protected) having a ketone residue or aldehyde residue at the end and including an amino acid residue which can be cleaved by a protease. This method can be applied to synthesis of peptides free of glycoamino acid. In such a case, the step 4) is omitted.

Synthesis and purification of apolymericprimer from thus obtained glycopeptide having a ketone residue or aldehyde residue at the end and including an amino acid residue which can be cleaved by a protease is performed in accordance with the following procedure:
6) reacting the glycopeptide obtained in the above procedure with a polymeric support; and
7) performing purification by gel filtration column chromatography, thereby obtaining a polymeric primer.

Another general synthesis and purification method of a compound of the present invention is performed in accordance with the following procedure:
1) performing solid phase peptide synthesis using a protected amino acid (including an amino acid residue which can be cleaved by a protease), previously synthesized glycoamino acid having a protective group, and keto acid or aldehydic acid, as raw materials, thereby synthesizing a glycopeptide (with a sugar chain and amino acid protected) having a ketone residue or aldehyde residue at the end and including an amino acid residue which can be cleaved by a protease (and optionally performing a capping reaction after each step of the amino acid coupling reaction, that is, reaction for inactivating unreacted substances in amino acid coupling);
2) separating the glycopeptide having a ketone residue or aldehyde residue at the end and including an amino acid residue which can be cleaved by a protease from the solid-phase support, and simultaneously deprotecting a protective group of an amino acid side chain by acid treatment (if the protective group of the amino acid side chain is not eliminated by acid treatment, the protective group may be deprotected by a separate deprotection reaction);
3) deprotecting the protective group of the sugar chain;
4) introducing a polymeric support into a reaction solution containing the glycopeptide of 3), thereby selectively reacting the polymeric support with the glycopeptide;
5) purifying the glycopeptide bound to the support by gel filtration, dialysis, ultrafiltration or the like; and
6) hydrolyzing the glycopeptide bound to the support by a protease to separate the glycopeptide and removing the support, thereby isolating the glycopeptide of interest.

According to this procedure, a polymeric primer can be lead in one pot without performing the respective steps separate. Synthesis and purification of a polymeric primer from thus obtained glycopeptide having a ketone residue or aldehyde residue at the end and including an amino acid residue which can be cleaved by a protease is performed in accordance with the following procedure:
1) performing solid phase peptide synthesis using a protected amino acid (including an amino acid residue which can be cleaved by a protease), previously synthesized glycoamino acid having a protective group, and keto acid or aldehydic acid, as raw materials, thereby synthesizing a glycopeptide (with a sugar chain and amino acid protected) having a ketone residue or aldehyde residue at the end and including an amino acid residue which can be cleaved by a protease (and optionally performing a capping reaction after each step of the amino acid coupling reaction, that is, reaction for inactivating unreacted substances in amino acid coupling);
2) separating the glycopeptide having a ketone residue or aldehyde residue at the end and including an amino acid residue which can be cleaved by a protease from the solid-phase support, and simultaneously deprotecting a protective group of an amino acid side chain by acid treatment (if the protective group of the amino acid side chain is not eliminated by acid treatment, the protective group may be deprotected by a separate deprotection reaction);
3) deprotecting the protective group of the sugar chain;
4) introducing a polymeric support into a reaction solution containing the glycopeptide of 3), thereby selectively reacting the polymeric support with the glycopeptide; and
5) purifying the glycopeptide bound to the support by gel filtration, dialysis, ultrafiltration or the like, thereby obtaining a primer.

In a preferred embodiment, the keto acid or aldehydic acid used in the above procedure 1) is a compound represented by the following formula:

X-C(=O)-(CH₂)ₙ-A₁-COOH (III)

wherein X represents a hydrogen atom, C₁-C₃₀ alkyl, C₆-C₃₀ aryl or a chromophore;
n represents an integer from 0 to 20; and
A₁ represents a linker having a length of 1 to 20 methylene groups.

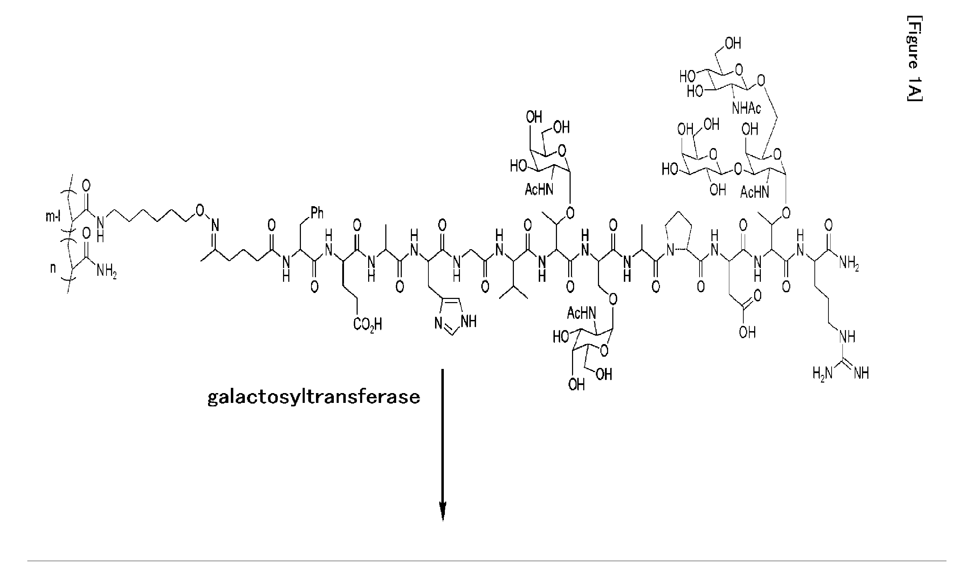

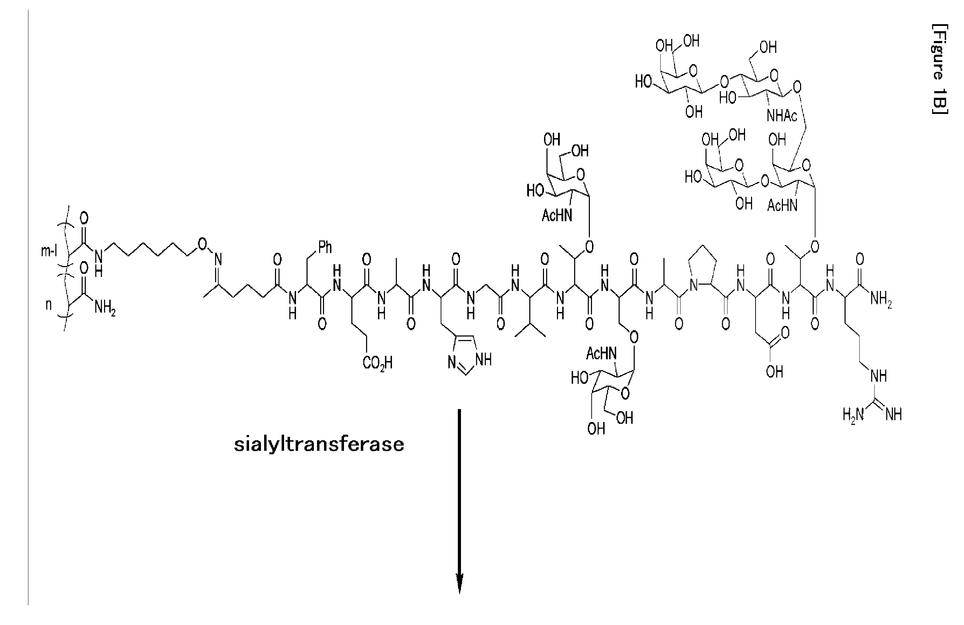

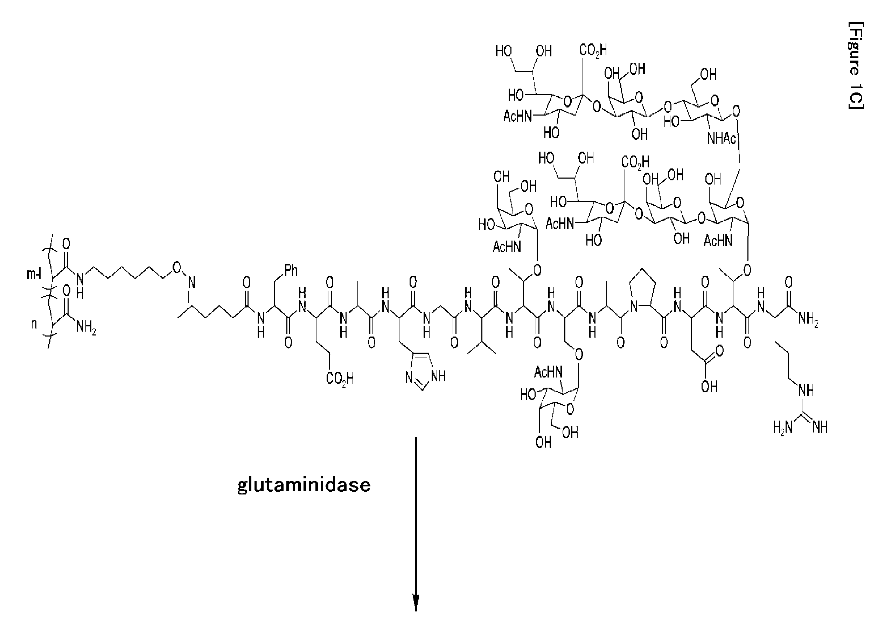

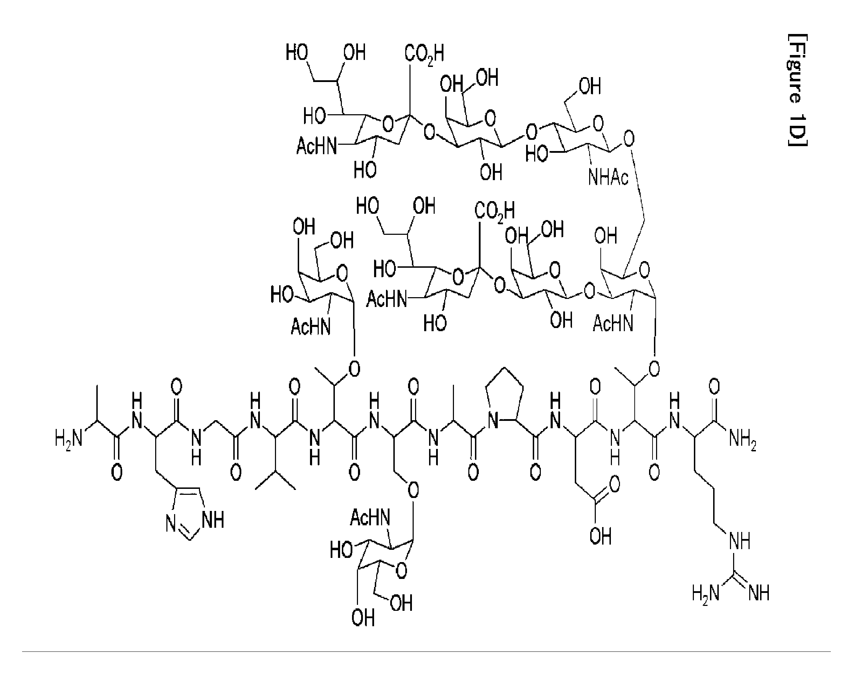

In a preferred embodiment, the method for producing a glycopeptide of the present invention includes the steps of:
(A) reacting the compound according to any one of claims 1 to 3 with a support including a functional group selected from the group consisting of: a protected or unprotected aminooxy group; a protected or unprotected N-alkylaminooxy group; a protected or unprotected hydrazid group; a protected or unprotected azide group; a protected or unprotected thiosemicarbazide group; a protected or unprotected 1,2-dithiol group; and a protected or unprotected cysteine residue, the functional group being capable of specifically reacting with a ketone residue or aldehyde residue;
(B) allowing glycosyltransferase to act on the compound obtainedfromthestep (A) in the presence of a sugar nucleotide so as to cause a sugar residue to transfer from the sugar nucleotide to the compound, thereby obtaining a compound having an elongated sugar chain;
(C) optionally removing unreacted sugar nucleotides and by-product nucleotides; and
(D) allowing a protease to act on the compound having an elongated sugar chain as a result of transfer of the sugar residue (see, for example, Figure 1).

Any glycosyltransferase can be used in the present invention, as long as it can use sugar nucleotides can be used as a sugar donor. Examples of preferred glycosyltransferases include β1,4-galactosyltransferase, α-1,3-galactosyltransferase, β1,4-galactosyltransferase, β1,3-galactosyltransferase, β1,6-galactosyltransferase, α2,6-sialyltransferase, α1,4-galactosyltransferase, ceramide galactosyltransferase, α1,2-fucosyltransferase, α1,3-fucosyltransferase, α1,4-fucosyltransferase, α1,6-fucosyltransferase, α1,3-N-acetylgalactosaminyltransferase, α1,6-N-acetylgalactosaminyltransferase, β1,4-N-acetylgalactosaminyltransferase, polypeptide N-acetylgalactosaminyltransferase, β1,4-N-acetylglucosaminyltransferase, β1,2-N-acetylglucosaminyltransferase, β1,3-N-acetylglucosaminyltransferase, β1,6-N-acetylglucosaminyltransferase, α1,4-N-acetylglucosaminyltransferase, β1,4-mannosyltransferase, α1,2-mannosyltransferase, α1,3-mannosyltransferase, α1,4-mannosyltransferase, α1,6-mannosyltransferase, α1,2-glucosyltransferase, α1,3-glucosyltransferase, α2,3-sialyltransferase, α2,8-sialyltransferase, α1,6-glucosaminyltransferase, α1,6-xylosyltransferase, β-xylosyltransferase (proteoglycan core structure synthesizing enzyme), β1,3-glucuronosyltransferase, hyaluronic acid synthesizing enzyme, glycosyltransferases using other nucleotide sugar as a sugar donor, glycosyl transferases using dolichol-phosphate-type sugar donor, and the like.

In another preferred embodiment, the method for producing a glycopeptide of the present invention includes the steps of:
(A) allowing glycosyltransferase to act on the compound according to any one of (4) to (7) in the presence of a sugar nucleotide so as to cause a sugar residue to transfer from the sugar nucleotide to the compound, thereby obtaining a compound having an elongated sugar chain;
(B) optionally removing unreacted sugar nucleotides and by-product nucleotides; and
(C) allowing a protease to act on the compound having an elongated sugar chain as a result of transfer of the sugar residue. Further, the method may include the step of isolating the glycopeptide. In the present production method, glycopeptide of interest can be readily separated from by-products other than the glycopeptide, including the support.

In a yet another preferred embodiment, the method for producing a glycopeptide of the present invention includes the steps of:
(A) allowing glycosyltransferase to act on the compound according to any one of (4) to (7) in the presence of a sugar nucleotide so as to cause a sugar residue to transfer from the sugar nucleotide to the compound, thereby obtaining a compound having an elongated sugar chain;
(B) repeating the step (A) for one or more times to elongate a sugar chain;
(C) optionally removing unreacted sugar nucleotides and by-product nucleotides; and
(D) allowing a protease to act on the compound having an elongated sugar chain as a result of transfer of a plurality of sugar residues.

In a yet another preferred embodiment, the method for producing a glycopeptide of the present invention includes the steps of:
(A) performing solid phase peptide synthesis using an amino acid, glycoamino acid and keto acid or aldehydic acid, which can be cleaved by a protease, as raw materials, thereby obtaining the compound according to any one of (1) to (3);
(B) reacting the compound obtained from the step (A) with a support including a functional group selected from the group consisting of: a protected or unprotected aminooxy group; a protected or unprotected N-alkylaminooxy group; a protected or unprotected hydrazid group; a protected or a unprotected azide group; a protected or unprotected thiosemicarbazide group; a protected or unprotected 1,2-dithiol group; and a protected or unprotected cysteine residue, the functional group being capable of specifically reacting with a ketone residue or aldehyde residue;
(C) allowing glycosyltransferase to act on the compound obtainedfromthestep (B) in the presence of a sugar nucleotide so as to cause a sugar residue to transfer from the sugar nucleotide to the compound, thereby obtaining a compound having an elongated sugar chain;
(D) optionally removing unreacted sugar nucleotides and by-product nucleotides; and
(E) allowing a protease to act on the compound having an elongated sugar chain as a result of transfer of the sugar residue.

In another preferred embodiment, the method for producing a glycopeptide of the present invention includes the steps of:
(A) performing solid phase peptide synthesis using an amino acid, glycoamino acid and keto acid or aldehydic acid, which can be cleaved by a protease, as raw materials, thereby obtaining the compound according to any one of (1) to (3);
(B) reacting the compound obtained from the step (A) with a support including a functional group selected from the group consisting of: a protected or unprotected aminooxy group; a protected or unprotected N-alkylaminooxy group; a protected or unprotected hydrazid group; a protected or unprotected azide group; a protected or unprotected thiosemicarbazide group; a protected or unprotected 1,2-dithiol group; and a protected or unprotected cysteine residue, the functional group being capable of specifically reacting with a ketone residue or aldehyde residue, and simultaneously removing unreacted substances in the step (A);
(C) allowing glycosyltransferase to act on the compound, which has been obtained from the step (B), in the presence of a sugar nucleotide so as to cause a sugar residue to transfer from the sugar nucleotide to the compound, thereby obtaining a compound having an elongated sugar chain;
(D) repeating the step (C) for one or more times to elongate a sugar chain;
(E) optionally removing unreacted sugar nucleotides and by-product nucleotides; and
(F) allowing a protease to act on the compound having an elongated sugar chain as a result of transfer of a plurality of sugar residues.

In yet another preferred embodiment, the method for producing a glycopeptide of the present invention includes the steps of:
(A) performing solid phase peptide synthesis using an amino acid, glycoamino acid and keto acid or aldehydic acid, which can be cleaved by a protease, as raw materials, thereby obtaining the compound according to any one of (1) to (3);
(B) reacting the compound obtained from the step (A) with a support including a functional group selected from the group consisting of: a protected or unprotected aminooxy group; a protected or unprotected N-alkylaminooxy group; a protected or unprotected hydrazid group; a protected or unprotected azide group; a protected or unprotected thiosemicarbazide group; a protected or unprotected 1,2-dithiol group; and a protected or unprotected cysteine residue, the functional group being capable of specifically reacting with a ketone residue or aldehyde residue, and simultaneously removing unreacted substances in the step (A);
(C) allowing glycosyltransferase to act on the compound bound to the support, which has been obtained from the step (B), in the presence of a sugar nucleotide so as to cause a sugar residue to transfer from the sugar nucleotide to the compound, thereby obtaining a compound having an elongated sugar chain; and
(D) allowing a protease to act on the compound having an elongated sugar chain obtained from the step (C).

In a yet another preferred embodiment, the method for producing a glycopeptide of the present invention includes the steps of:
(A) performing solid phase peptide synthesis using an amino acid, glycoamino acid and keto acid or aldehydic acid, which can be cleaved by a protease, as raw materials, thereby obtaining the compound according to any one of (1) to (3);
(B) reacting the compound obtained from the step (A) with a support including a functional group selected from the group consisting of: a protected or unprotected aminooxy group; a protected or unprotected N-alkylaminooxy group; a protected or unprotected hydrazid group; a protected or unprotected azide group; a protected or unprotected thiosemicarbazide group; a protected or unprotected 1,2-dithiol group; and a protected or unprotected cysteine residue, the functional group being capable of specifically reacting with a ketone residue or aldehyde residue, and simultaneously removing unreacted substances in the step (A);
(C) allowing glycosyltransferase to act on the compound bound to the support, which has been obtained from the step (B), in the presence of a sugar nucleotide so as to cause a sugar residue to transfer from the sugar nucleotide to the compound, thereby obtaining a compound having an elongated sugar chain;
(D) repeating the step (C) for one or more times to elongate a sugar chain;
(E) optionally removing unreacted sugar nucleotides and by-product nucleotides; and
(F) allowing a protease to act on the compound having an elongated sugar chain as a result of transfer of a plurality of sugar residues.

In a yet another preferred embodiment, the method for producing a glycopeptide of the present invention includes the steps of:
(A) allowing glycosyltransferase to act on the compound according to any one of (1) to (3) in the presence of a sugar nucleotide so as to cause a sugar residue to transfer from the sugar nucleotide to the compound, thereby obtaining a compound having an elongated sugar chain;
(B) optionally repeating the step (A) for one or more times to elongate a sugar chain;
(C) reacting the compound having an elongated sugar chain as a result of transfer of the sugar residue with a support including a functional group selected from the group consisting of: a protected or unprotected aminooxy group; a protected or unprotected N-alkylaminooxy group; a protected or unprotected hydrazid group; a protected or unprotected azide group; a protected or unprotected thiosemicarbazide group; a protected or unprotected 1,2-dithiol group; and a protected or unprotected cysteine residue, the functional group being capable of specifically reacting with a ketone residue or aldehyde residue; and
(D) optionally removing unreacted sugar nucleotides and by-product nucleotides.

In a yet another preferred embodiment, the method for producing a glycopeptide of the present invention includes the steps of:
(A) allowing glycosyltransferase to act on the compound according to any one of (1) to (3) in the presence of a sugar nucleotide so as to cause a sugar residue to transfer from the sugar nucleotide to the compound, thereby obtaining a compound having an elongated sugar chain;
(B) optionally repeating the step (A) for one or more times to elongate a sugar chain;
(C) reacting the compound having an elongated sugar chain as a result of transfer of the sugar residue with a support including a functional group selected from the group consisting of: a protected or unprotected aminooxy group; a protected or unprotected N-alkylaminooxy group; a protected or unprotected hydrazid group; a protected or unprotected azide group; a protected or unprotected thiosemicarbazide group; a protected or unprotected 1,2-dithiol group; and a protected or unprotected cysteine residue, the functional group being capable of specifically reacting with a ketone residue or aldehyde residue; and
(D) optionally removing unreacted sugar nucleotides and by-product nucleotides; and
(E) allowing a protease to act on the compound having an elongated sugar chain as a result of transfer of the sugar residue.

In a yet another preferred embodiment, the method for producing a glycopeptide of the present invention includes the steps of:
(A) performing solid phase peptide synthesis using an amino acid, glycoamino acid and keto acid or aldehydic acid, which can be cleaved by a protease, as raw materials, thereby obtaining the compound according to any one of (1) to (3);
(B) reacting the compound obtained from the step (A) with a support including a functional group selected from the group consisting of: a protected or unprotected aminooxy group; a protected or unprotected N-alkylaminooxy group; a protected or unprotected hydrazid group; a protected or unprotected azide group; a protected or unprotected thiosemicarbazide group; a protected or unprotected 1,2-dithiol group; and a protected or unprotected cysteine residue, the functional group being capable of specifically reacting with a ketone residue or aldehyde residue, and removing unreacted substances in the step (A) by reprecipitation, gel filtration, ultrafiltration or the like;
(C) allowing glycosyltransferase to act on the compound solubly bound to the support, which has been obtained from the step (B), in the presence of a sugar nucleotide so as to cause a sugar residue to transfer from the sugar nucleotide to the compound, thereby obtaining a compound having an elongated sugar chain;
(D) repeating the step (C) for one or more times to elongate a sugar chain;
(E) optionally removing unreacted sugar nucleotides and by-product nucleotides;
(F) reacting the compound having elongated a sugar chain as a result of transfer of the sugar residue with a non-soluble support having keto acid or aldehydic acid bound to a surface thereof, thereby immobilizing the compound on the surface thereof; and
(G) optionally removing reagents and enzymes used for sugar chain elongation reaction.

In yet another preferred embodiment, the method for producing a glycopeptide of the present invention includes the steps of:
(A) performing solid phase peptide synthesis using an amino acid, glycoamino acid and keto acid or aldehydic acid, which can be cleaved by a protease, as raw materials, thereby obtaining the compound according to any one of (1) to (3);
(B) reacting the compound obtained from the step (A) with a support including a functional group selected from the group consisting of: a protected or unprotected aminooxy group; a protected or unprotected N-alkylaminooxy group; a protected or unprotected hydrazid group; a protected or unprotected azide group; a protected or unprotected thiosemicarbazide group; a protected or unprotected 1,2-dithiol group; and a protected or unprotected cysteine residue, the functional group being capable of specifically reacting with a ketone residue or aldehyde residue, and removing unreacted substances in the step (A) by reprecipitation, gel filtration, ultrafiltration or the like;
(C) allowing glycosyltransferase to act on the compound solubly bound to the support, which has been obtained from the step (B), in the presence of a sugar nucleotide so as to cause a sugar residue to transfer from the sugar nucleotide to the compound, thereby obtaining a compound having an elongated sugar chain;
(D) repeating the step (C) for one ormore times to elongate a sugar chain;
(E) optionally removing unreacted sugar nucleotides and by-product nucleotides;
(F) reacting the compound having elongated a sugar chain as a result of transfer of the sugar residue with a non-soluble support having keto acid or aldehydic acid bound to a surface thereof, thereby immobilizing the compound on the surface thereof;
(G) optionally removing reagents and enzymes used for sugar chain elongation reaction; and
(H) allowing a protease to act on the compound having an elongated sugar chain, which has been immobilized in the step (F).

In the method for producing a glycopeptide of the present invention, a series of reactions using glycosyltransferase as described above can be optionally performed in automatized manner using a distribution apparatus (distributor) or the like which is capable of controlling the temperature of a reaction part.

### (Mucin-type glycopeptide)

According to the present invention, a novel primer as explained above and a method for producing a glycopeptide using such a primer allows synthesis of mucin-type glycopeptides which are useful in a wide range of field including materials for biochemical research, drugs, and foods and which have been conventionally difficult to produce. Examples of mucin-type glycopeptide include a glycopeptide represented by the following formula:

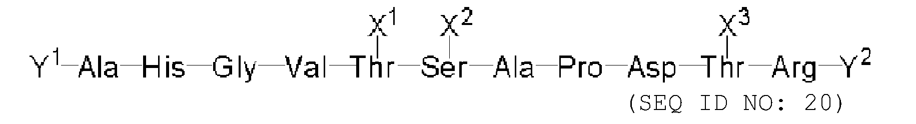

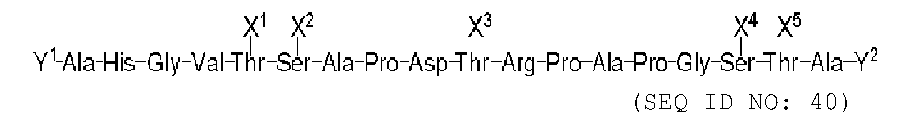

or

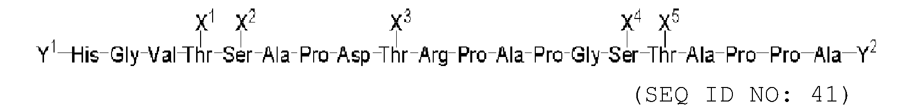

wherein X¹-X⁵ independently represent a hydrogen atom or a group represented by the following formula:

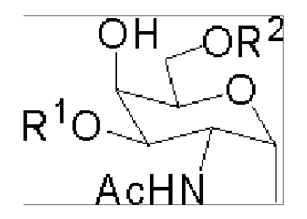

wherein R¹ and R² independently represent a hydrogen atom, monosaccharide or sugar chain, and Ac represents acetyl;
Y¹ represents a hydrogen atom, acetyl, acyl, alkyl or aryl;
Y² represents a hydroxyl group, NH₂, alkyl or aryl.

When the above R¹ and R² represent a sugar chain, R¹ and R² are independently selected from the group consisting of:

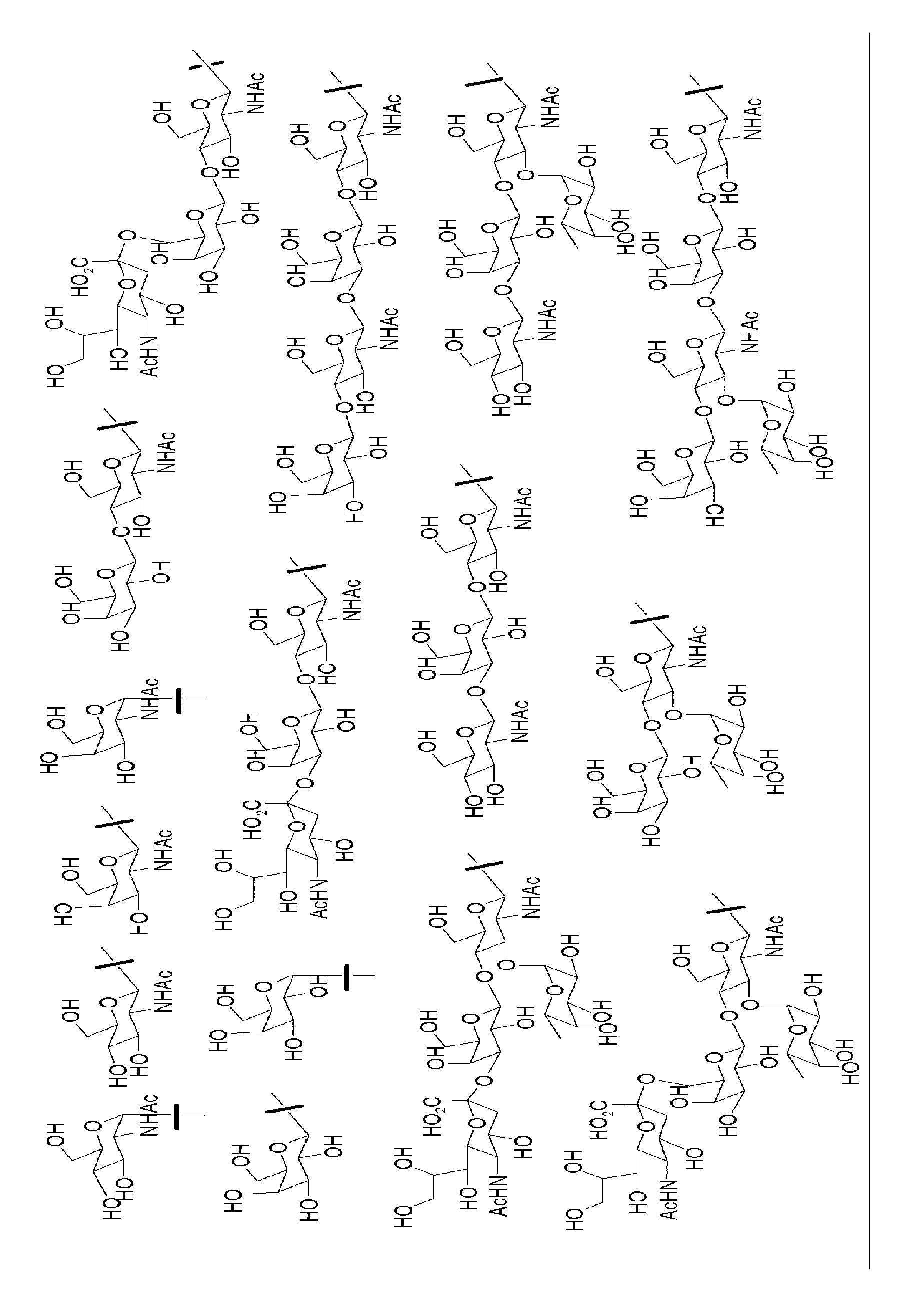

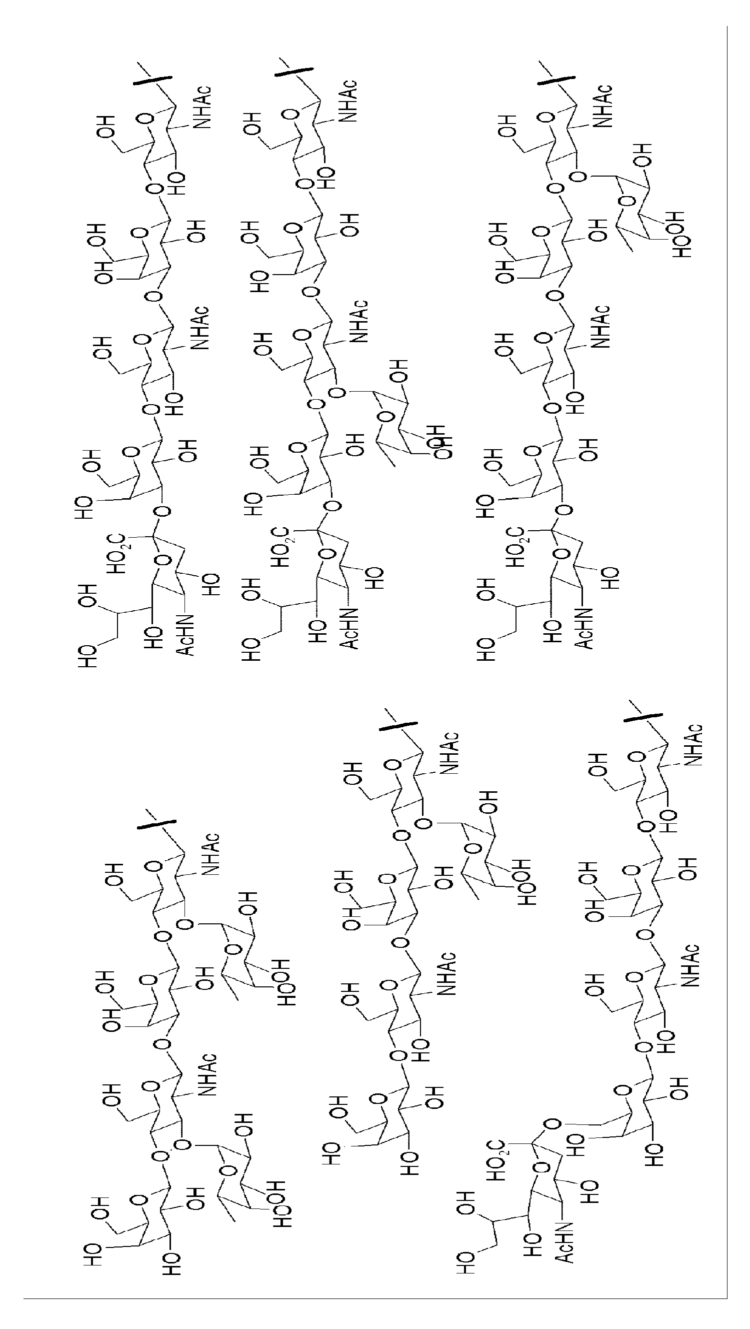

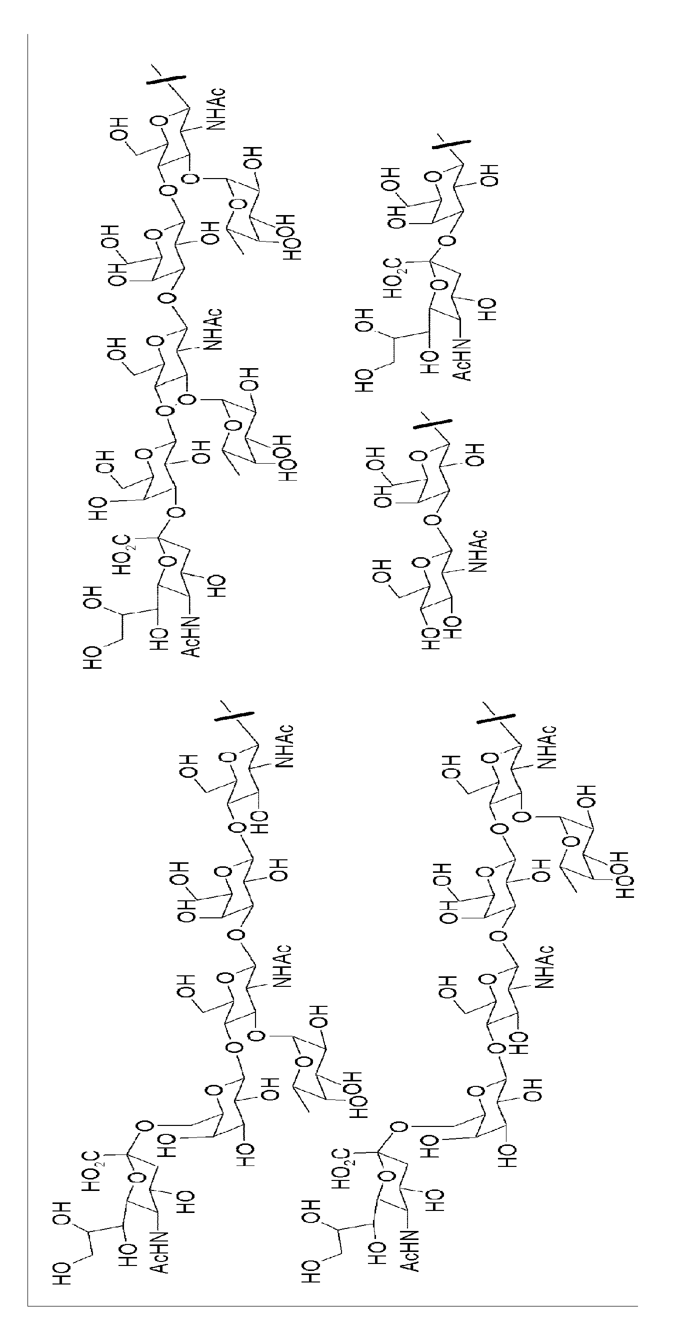

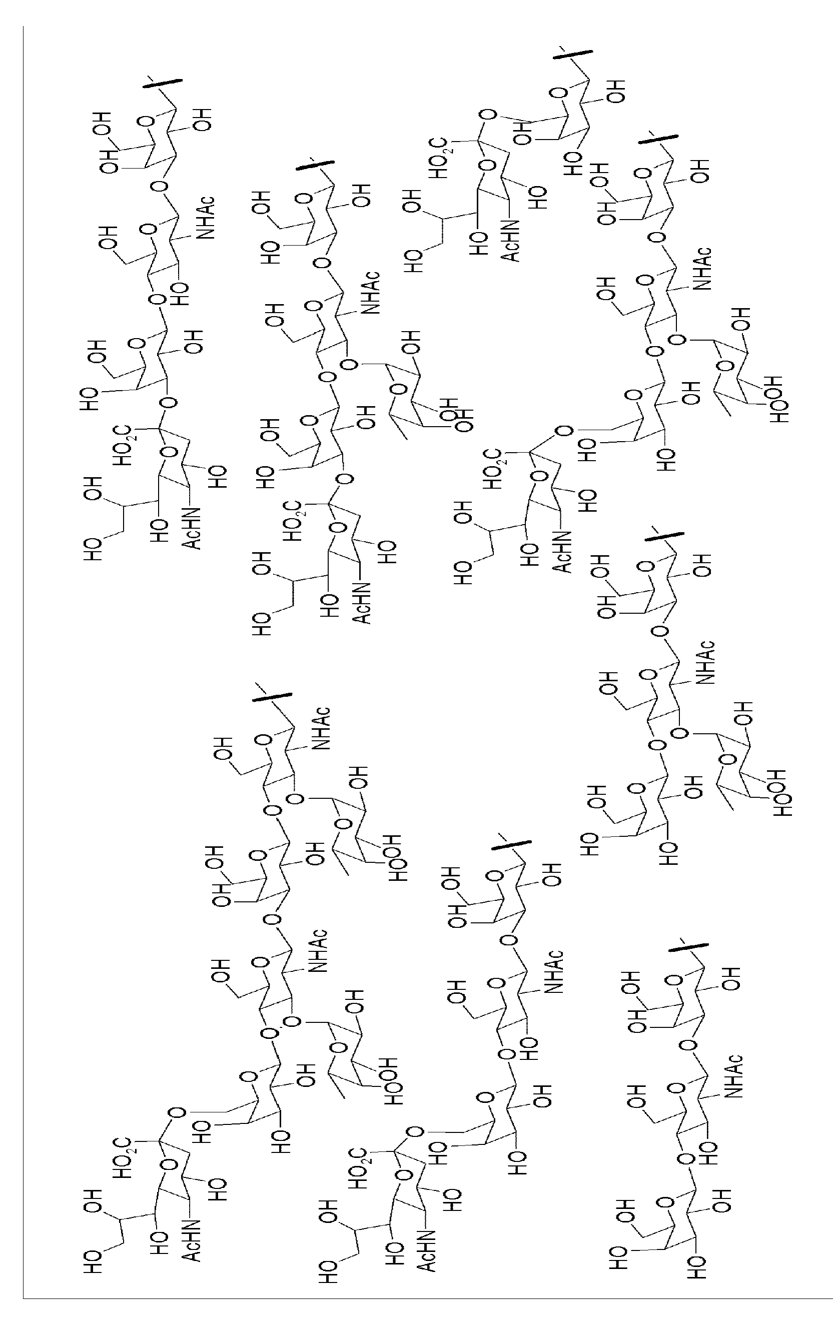

### (Medicaments and therapy, prophylaxis and the like using such medicaments)

According to another aspect, the present invention relates to a medicament (for example, medicaments such as vaccines, health foods, medicaments of residue proteins or residue lipids with reduced antigenicity) containing glycopeptides (for example, mucin-type glycopeptides) obtained by the production method of the present invention. Such medicaments may further contain pharmaceutically acceptable carriers or the like. Examples of pharmaceutically acceptable carriers included in a medicament of the present invention include any substance known in the art.

Examples of such appropriate materials to be formulated or pharmaceutically acceptable carriers include, but not limited to, antioxidants, preservative agents, coloring agents, flavoring agents, diluents, emulsifying agents, suspending agents, solvents, fillers, extending agents, buffering agents, delivery vehicles, diluents, excipients and/or pharmaceutical adjuvants. Typically, a medicament of the present invention is administered in the form of a composition including an isolated multi-function stem cell or a variant or derivative thereof in combination with one or more physiologically acceptable carriers, excipients or diluents. For example, an appropriate vehicle may be water for injection, physiological solution or artificial cerebrospinal solution. Other substances common in a composition for parenteral delivery can be supplied to these vehicles.

An acceptable carrier, excipient or stabilizing agent used herein is nontoxic to a recipient, and is preferably inactive at a dosage and concentration to be used. Examples thereof include: phosphates, citrates or other organic salts; ascorbic acid or α-tocopherol; polypeptides with a low molecular weight; proteins (for example, serum albumin, geratin or immunoglobulin); hydrophilic polymers (for example, polyvinyl pyrrolidone); amino acids (for example, glycine, glutamine, asparagine, arginine or lysine); monosaccharides, disaccharides and other carbohydrates (including glucose, mannose or dextrin); chelating agents (for example, EDTA); sugar alcohols (for example, mannitol or sorbitol) ; salt-forming ions (for example, sodium) ; and/or nonionic surfactants (for example, Tween, Pluronic or polyethylene glycol (PEG)).

Exemplary appropriate carriers include neutral buffered saline solution, or saline solution mixed with serum albumin. Preferably, products are formulated as a lyophilized agent using an appropriate excipient (for example, sucrose). Other standard carriers, diluents and excipients may be contained as desired. Other exemplary composition includes Tris buffer with pH of 7.0 to 8.5 or acetic acid buffer with pH of 4.0 to 5.5, and may further include sorbitol or an appropriate substitute therewith.

A medicament of the present invention may be orally or parenterally administered. Alternatively, a medicament of the present invention may be intravenously or subcutaneously administered. In the case of systemic administration, a medicament used in the present invention may take the form of pharmaceutically acceptable aqueous solution which is free of pyrogenic substance. Such a pharmaceutically acceptable composition can be readily prepared by those skilled in the art by taking pH, isotonicity, stability and the like into consideration. Herein, a method for administration may be oral administration or parenteral administration (for example, intravenous administration, intramuscular administration, subcutaneous administration, intracutaneous administration, mucosal administration, intrarectal administration, intravaginal administration, local administration to a diseased part, cutaneous administration and the like). A formulation for such administration may be provided in any preparation form. Such a preparation form includes, for example, liquid preparation, injection, release agent and the like.

A medicament of the present invention may be prepared and preserved in the form of a lyophilized cake or aqueous solution by optionally mixing a physiologically acceptable carrier, excipient or stabilizing agent (see Japanese Pharmacopoeia, 14th edition or the latest edition, Remington's Pharmaceutical Sciences, 18th Edition, A. R. Gennaro, ed., Mack Publishing Company, 1990 or the like) and a composition including a glycopeptide (for example, a mucine-type glycopeptide) obtained by the production method of the present invention, which has a desired degree of purity.

An amount of a composition including a glycopeptide (for example, a mucin-type glycopeptide) used in a treatment method of the present invention can be readily determined by those skilled in the art by taking the purpose of use, disease of interest (type, gravity or the like), age, body weight, sex and anamnesis of a patient, form and type of a cell, and the like into consideration. Frequency of application of a treatment method of the present invention to a subject (or a patient) can also be readily determined by those skilled in the art by taking the purpose of use, disease of interest (type, gravity or the like), age, body weight, sex and anamnesis of a patient, progress of therapy and the like into consideration. Frequency includes, for example, administration of from one time per day to one time per several months (for example, from one time per week to one time per month). It is preferred to apply administration of from one time per week to one time per month while observing progress.

The present invention has been illustrated with reference to the preferred embodiments of the present invention. However, the present invention should not be construed to be limited to such embodiments. It is noted that the scope of the present invention should be construed only by the scope of the claims. It is understood that those skilled in the art can carry out equivalent scope from the disclosure of the specific preferred embodiments of the present invention and based on the common technical knowledge. It is noted that patents, patent applications and documents cited in the present specification should be herein incorporated by reference as if the contents themselves are specifically described herein.

### EXAMPLES

Abbreviations as used herein have the meaning as will be described below. Although the present studies will be described in more detail by way of the following Examples, the present studies will not be limited to these Examples.
Abbreviations as used in the present Examples have the meaning as will be described below.

DMF=N,N-dimethylformamide;
DCM=dichloromethane;
HOBT=N-hydroxybenzotriazole;
HBTU=1-(bis(dimethylamino)methylene)-benzotriazolium
3-oxide hexafluorophosphate;
DIEA=diisopropylethylenamine;
Boc group=tert-butoxycarbonyl group
Fmoc group=9-fluorenylmetoxycarbonyl group
Pbf group=2,2,4,6,7-pentamethyl-dihydrobenzofuran-5-sulfonyl group;
Fmoc-Ala-OH=N-α-Fmoc-L-alanine;
Fmoc-Gly-OH=N-α-Fmoc-L-glycine;
Fmoc-Pro-OH=N-α-Fmoc-L-proline;
Fmoc-Arg(Pbf)-OH=N-α-Fmoc-Nγ-(2,2,4,6,7-pentamethyl-dihydrobenzofuran-5-sulfonyl)-L-arginine;
Fmoc-Asp(OtBu)-OH=N-α-Fmoc-L-aspartic acid β-t-butyl ester;
Fmoc-Gln(OtBu)-OH=N-α-Fmoc-L-glutamic acid β-t-butyl ester;
Fmoc-Phe-OH=N-α-Fmoc-L-phenylalanine;
Fmoc-Val-OH=N-α-Fmoc-L-valine;
Fmoc-His(Trt)-OH=N-α-Fmoc-N-im-trityl-L-histidine;
Fmoc-Thr-OH=N-α-Fmoc-L-threonine;
Fmoc-Ser-OH=N-α-Fmoc-L-serine;
Fmoc-Thr(Ac3GalNAc)-OH=N-α-Fmoc-O-(2-acetamide-3,4,6-tri-O-acetyl-2-deoxy-α-D-galactopyranosyl)-L-threonine;
Fmoc-Ser(Ac3GalNAc)-OH=N-α-Fmoc-O-(2-acetamide-3,4,6-tri-O-acetyl-2-deoxy-α-D-galactopyranosyl)-L-serine;
Fmoc-Thr(Ac7core2)-OH=N-α-Fmoc-O-{O-(2',3',4',6'-tetra-O-acetyl-β-D-galactopyranosyl)-(1'→3)-O-[2"-acetamide-3 ",4",6"-tri-O-acetyl-2"-deoxy-β-D-glucopyranosyl(1"→6)] -2-acetamide-2-deoxy-α-D-galactopyranosyl}-L-threonine;
Fmoc-Ser(Ac7core2)-OH=N-α-Fmoc-O-{O-(2',3',4',6'-tetra-O-acetyl-β-D-galactopyranosyl)-(1'→3)-O-[2"-acetamide-3",4",6"-tri-O-acetyl-2"-deoxy-β-D-glucopyranosyl-(1"→6 )]-2-acetamide-2-deoxy-α-D-galactopyranosyl}-L-serine;
Fmoc-Thr(Ac5core6)-OH=N-α-Fmoc-O-[O-(2"-acetamide-3",4" ,6"-tri-O-acetyl-2"-deoxy-β-D-glucopyranosyl)-(1"→6)-2-acetamide-4,6-di-O-acetyl-2-deoxy-α-D-galactopyranosyl] -L-threonine; and
Fmoc-Ser(Ac5core6)-OH=N-α-Fmoc-O-[O-(2"-acetamide-3",4" ,6"-tri-O-acetyl-2"-deoxy-β-D-glucopyranosyl)-(1"→6)-2-acetamide-4,6-di-O-acetyl-2-deoxy-α-D-galactopyranosyl] -L-serine

(Example 1: Synthesis of MUC1-related glycopeptide derivatives (1) to (12) having a ketone derivative at the N-terminus)

### (1.1 Synthesis of compound (1))

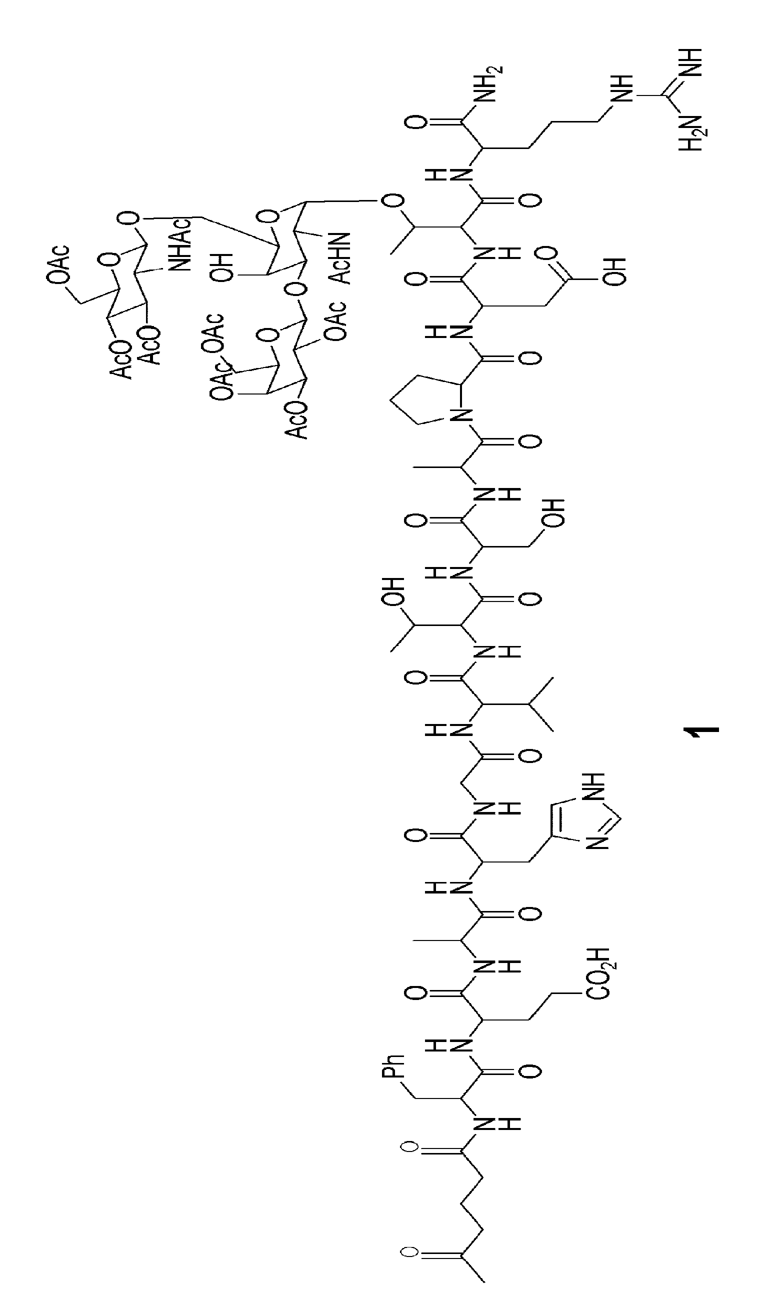

Using 0.12g (0.03mmol) of Tentagel® (Hipep Laboratories, 0.25mmol/g) as a support, N-protected amino acids and keto acid as described below were sequentially condensed by Fmoc/HBTU/HOBt method, thereby synthesizing a glycopeptide derivative of interest: Fmoc-Arg(Pbf)-OH; Fmoc-Thr(Ac7core2)-OH; Fmoc-Asp(OtBu)-OH; Fmoc-Pro-OH; Fmoc-Ala-OH; Fmoc-Ser-OH; Fmoc-Thr-OH; Fmoc-Val-OH; Fmoc-Gly-OH; Fmoc-His(Trt)-OH; Fmoc-Ala-OH; Fmoc-Gln(OtBu)-OH; Fmoc-Phe-OH; and 5-ketohexane acid. After the peptide elongation reaction, the glycopeptide derivative was allowed to react in 90% TFA aqueous solution for two hours at room temperature to eliminate the protective group on the peptide residue and concurrently separate the compound (1) from the solid-phase support. The resin was separated by filtration, and TFA was removed by volatilization. Thereafter, the resin was dissolved in 10% acetonitrile aqueous solution and was purified by reverse phase HPLC (Inertsil®, ODS-320×250mm column, Mobile phase: A: 0.1% TFA aqueous solution; B: acetonitrile containing 0.1% TFA, Gradient: 5% to 60%B with respect to A), thereby obtaining 8.5mg of compound (1) (yield: 28%). MALDI-TOF/MS:[M(average)+H]⁺ =2360.5 (theoretical value: [M(average)+H]⁺=2362.4)

(1.2 Synthesis of compound (2))

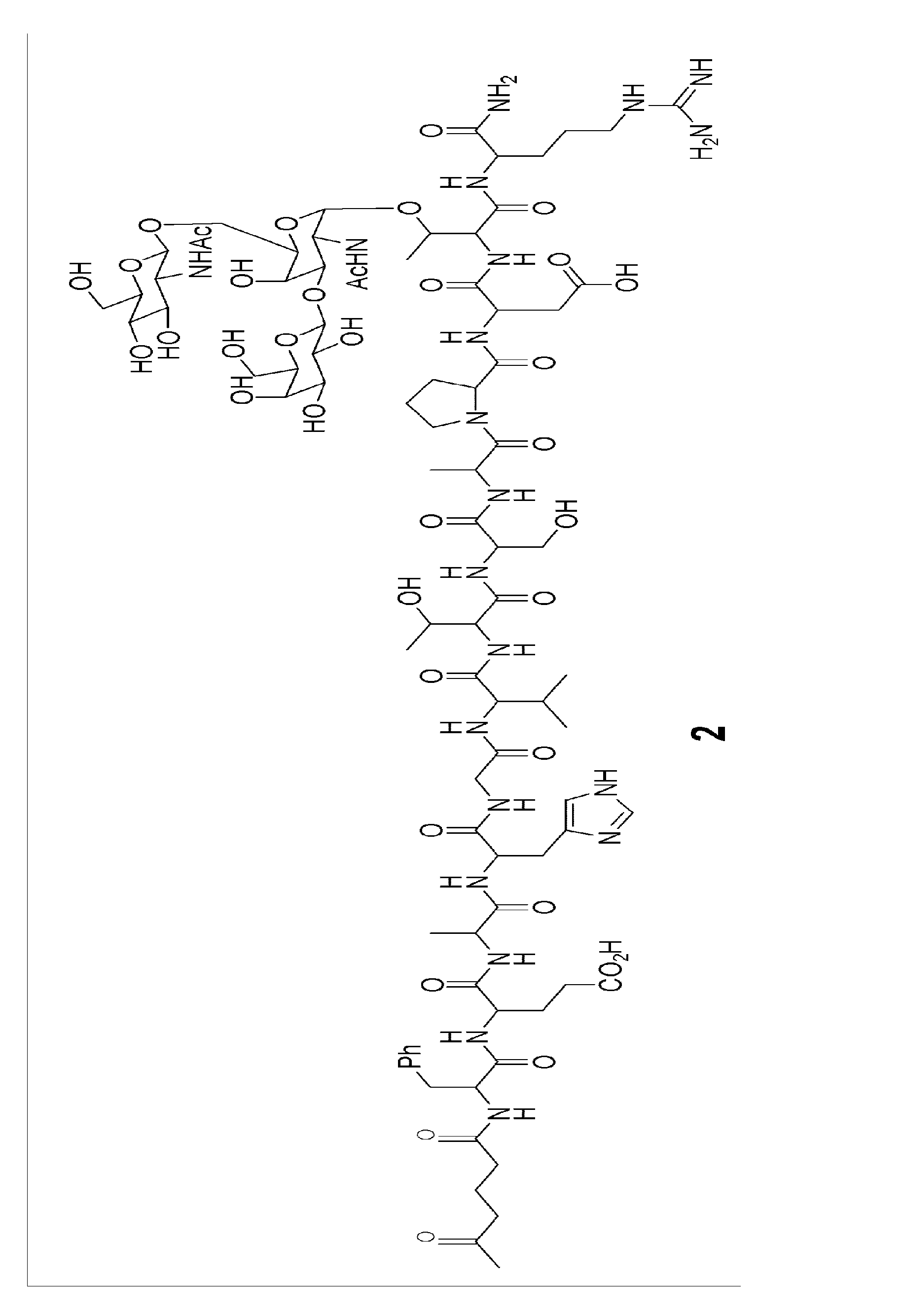

Compound (1) was dissolved in 7ml of methanol, and pH was adjusted to be 12.0 with 0.1N sodium hydroxide aqueous solution. While adjusting pH with 0.1N sodium hydroxide aqueous solution at any time, the solution was stirred for two hours until completion of the reaction. After completion of the reaction, H⁺-type cation exchange resin, Dowex50WX8 (available from Dow Chemical), was added and the solution was neutralized. Thereafter, the resin was separated by filtration. The solvent in the filtrate was removed, and the residue was purified by reverse phase HPLC (Inertsil®, ODS-3 20×250mm column, Mobile phase: A: 0.1% TFA aqueous solution; B: acetonitrile containing 0.1%TFA, Gradient: 0% to 60%B with respect to A), thereby obtaining 2.2mg of compound (2) (yield: 81%). MALDI-TOF/MS:[M(average)+H]⁺ =2066.2 (theoretical value: [M(average)+H]⁺=2068.1)

(1.3 Synthesis of compound (3))

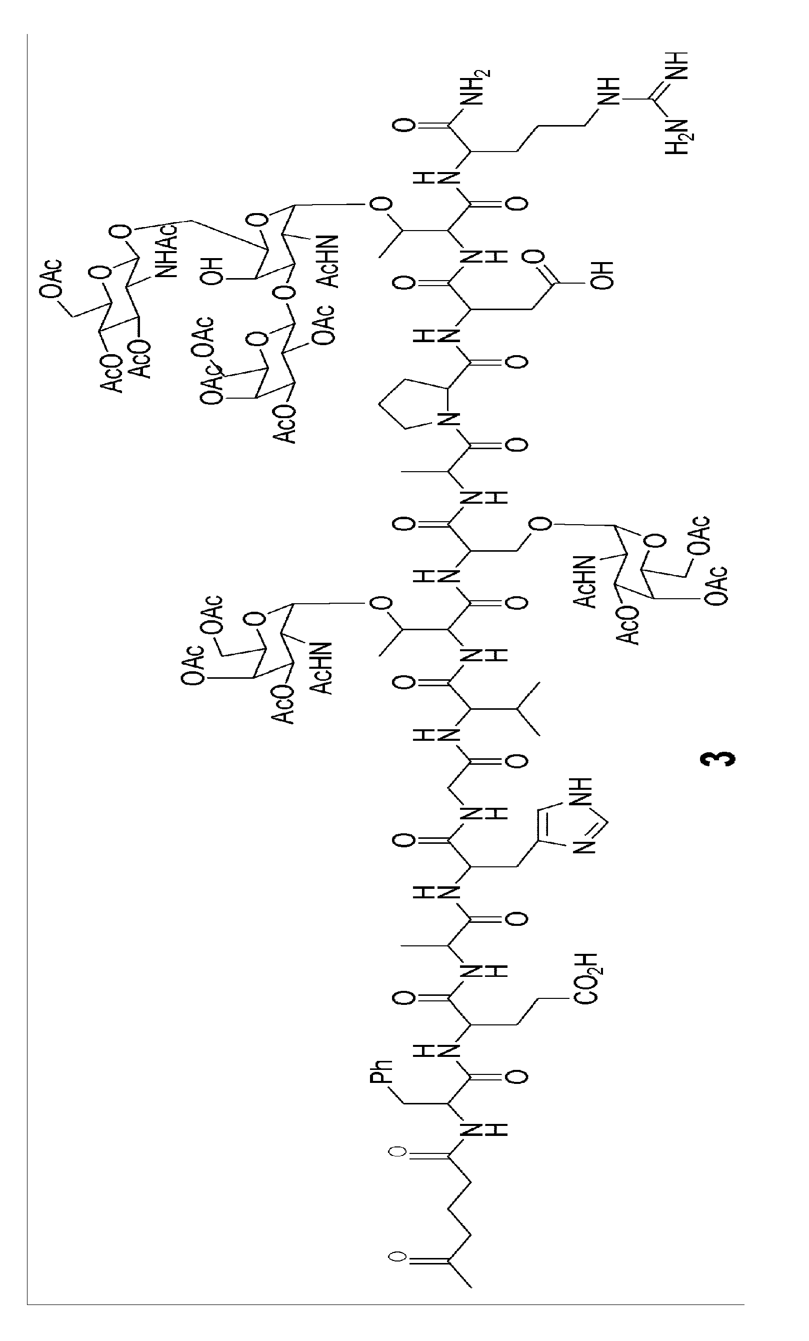

Using 0.12g (0.03mmol) of Tentagel® (Hipep Laboratories, 0.25mmol/g) as a support, N-protected amino acids and keto acid as described below were sequentially condensed by Fmoc/HBTU/HOBt method, thereby synthesizing a glycopeptide derivative of interest: Fmoc-Arg(Pbf)-OH; Fmoc-Thr(Ac7core2)-OH; Fmoc-Asp(OtBu)-OH; Fmoc-Pro-OH; Fmoc-Ala-OH; Fmoc-Ser(Ac3GalNAc)-OH; Fmoc-Thr(Ac3GalNAc)-OH; Fmoc-Val-OH; Fmoc-Gly-OH; Fmoc-His(Trt)-OH; Fmoc-Ala-OH; Fmoc-Gln(OtBu)-OH; Fmoc-Phe-OH; and 5-ketohexane acid. After the peptide elongation reaction, the glycopeptide derivative was allowed to react in 90% TFA aqueous solution for two hours at room temperature to eliminate the protective group on the peptide residue and concurrently release compound (3) from the solid-phase support. The resin was separated by filtration, and TFA wasremoved by volatilization. Thereafter, the resin was dissolved in 10% acetonitrile aqueous solution and, and a solid body was purified by reverse phase HPLC (Inertsil®, ODS-3 20×250mm column, Mobile phase: A: 0.1% TFA aqueous solution; B: acetonitrile containing 0.1% TFA, Gradient: 5% to 60%B with respect to A), thereby obtaining 16mg of compound (3) (yield: 18%). MALDI-TOF/MS:[M(average)+H]⁺=3018.340 (theoretical value: [M(average)+H]⁺=3021.0)

(1.4 Synthesis of compound (4))

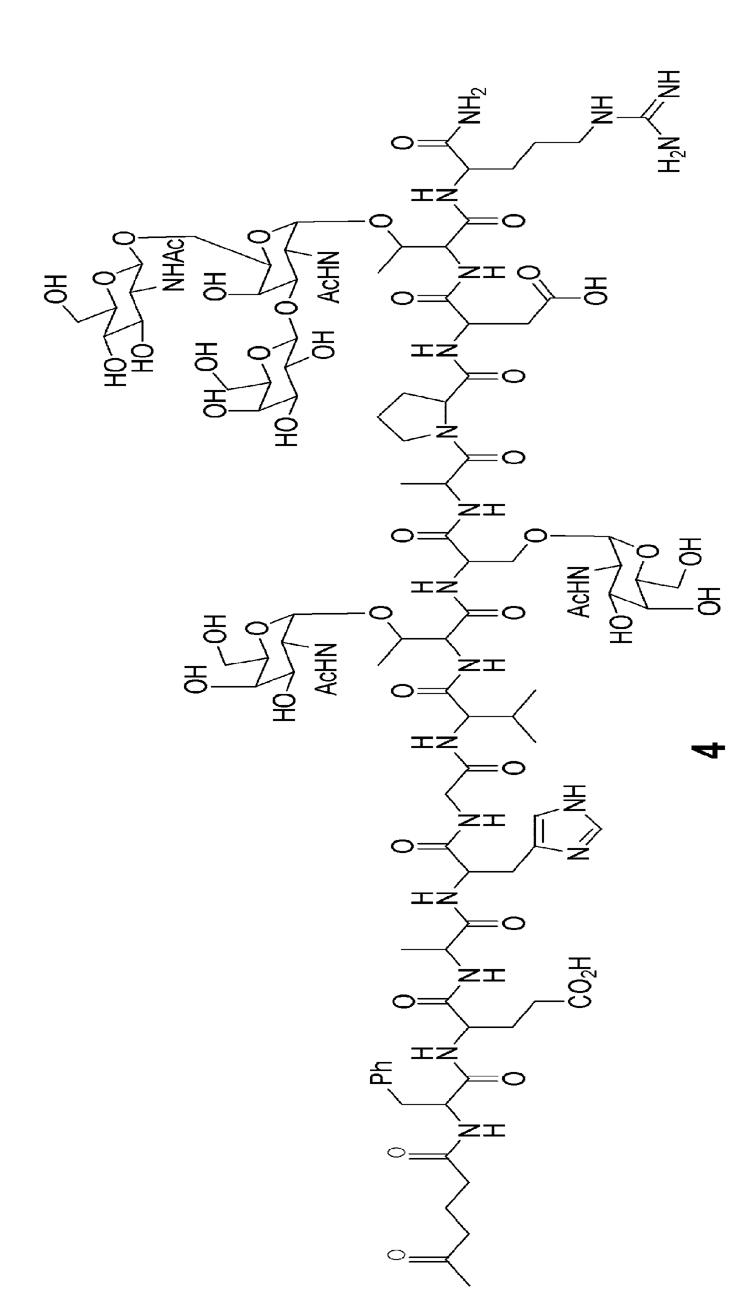

Compound (3) was dissolved in 5ml of methanol, and pH was adjusted to be 12.0 with 0.1N sodium hydroxide aqueous solution. While adjusting pH with 0.1N sodium hydroxide aqueous solution at any time, the solution was stirred for two hours until completion of the reaction. After completion of the reaction, H⁺-type cation exchange resin, Dowex50WX8 (available from Dow Chemical), was added and the solution was neutralized. Thereafter, the resin was separated by filtration. The solvent in the filtrate was removed, and the residue was purified by reverse phase HPLC (Inertsil®, ODS-3 20×250mm column, Mobile phase: A: 0.1% TFA aqueous solution; B: acetonitrile containing 0.1%TFA, Gradient: 0% to 60%B with respect to A), thereby obtaining 6.8mg of compound (4) (yield: 62%). MALDI-TOF/MS:[M(average)+H]⁺=2472.952 (theoretical value: [M(average)+H]⁺=2474.5)

(1.5 Synthesis of compound (5))

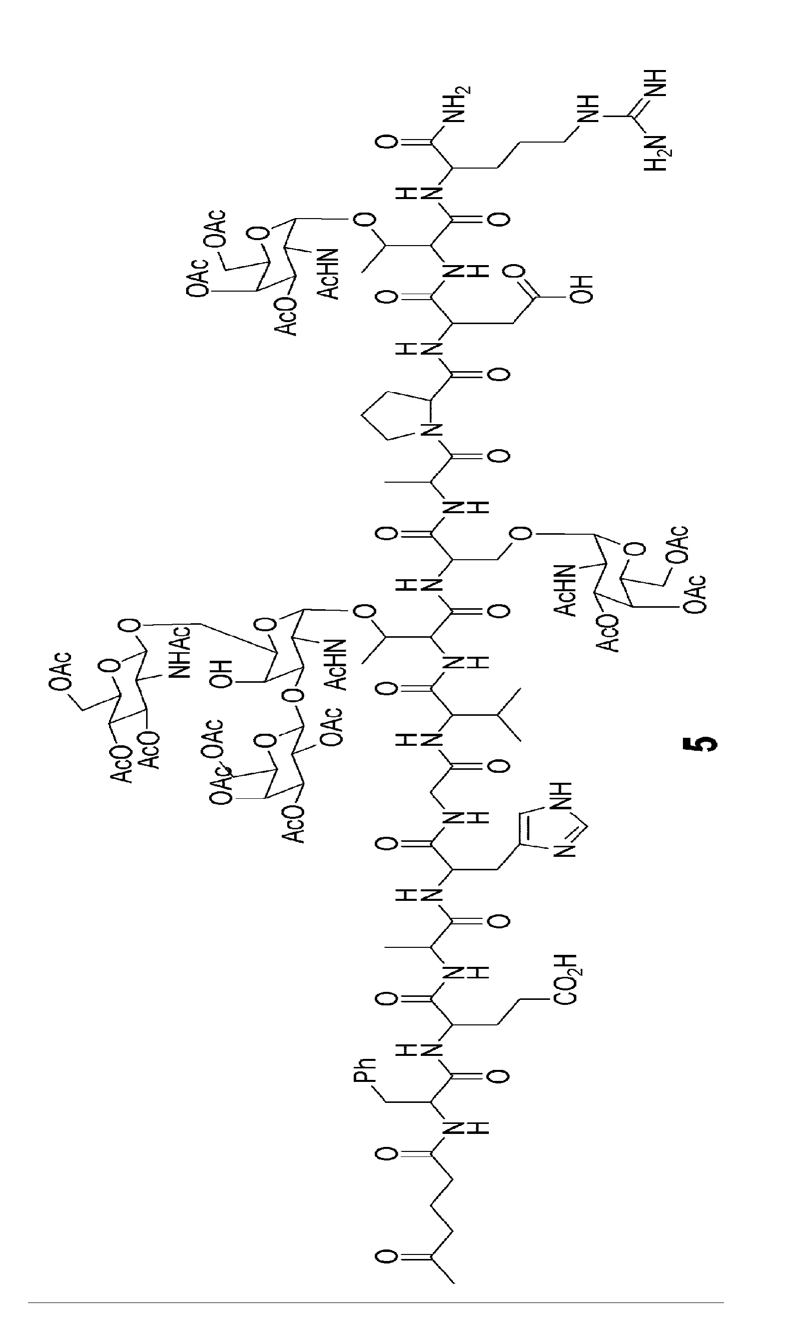

Using 0.12g (0.03mmol) of Tentagel® (Hipep Laboratories, 0.25mmol/g) as a support, N-protected amino acids and keto acid as described below were sequentially condensed by Fmoc/HBTU/HOBt method, thereby synthesizing a glycopeptide derivative of interest: Fmoc-Arg(Pbf)-OH; Fmoc-Thr(Ac7core2)-OH; Fmoc-Asp(OtBu)-OH; Fmoc-Pro-OH; Fmoc-Ala-OH; Fmoc-Ser(Ac3GalNAc)-OH; Fmoc-Thr(Ac3GalNAc)-OH; Fmoc-Val-OH; Fmoc-Gly-OH; Fmoc-His(Trt)-OH; Fmoc-Ala-OH; Fmoc-Gln(OtBu)-OH; Fmoc-Phe-OH; and 5-ketohexane acid. After the peptide elongation reaction, the glycopeptide derivative was allowed to react in 90% TFA aqueous solution for two hours at room temperature to eliminate the protective group on the peptide residue and concurrently release compound (5) from the solid-phase support. The resin was separated by filtration, and TFA wasremoved by volatilization. Thereafter, the resin was dissolved in 10% acetonitrile aqueous solution and a solid body was purified by reverse phase HPLC (Inertsil®, ODS-3 20×250mm column, Mobile phase: A: 0.1% TFA aqueous solution; B: acetonitrile containing 0.1% TFA, Gradient: 10% to 70%B with respect to A), thereby obtaining 9.8mg of compound (5) (yield: 7%). MALDI-TOF/MS:[M(average)+H]⁺ =3018.4 (theoretical value: [M(average)+H]⁺=3021.0)

(1.6 Synthesis of compound (6))

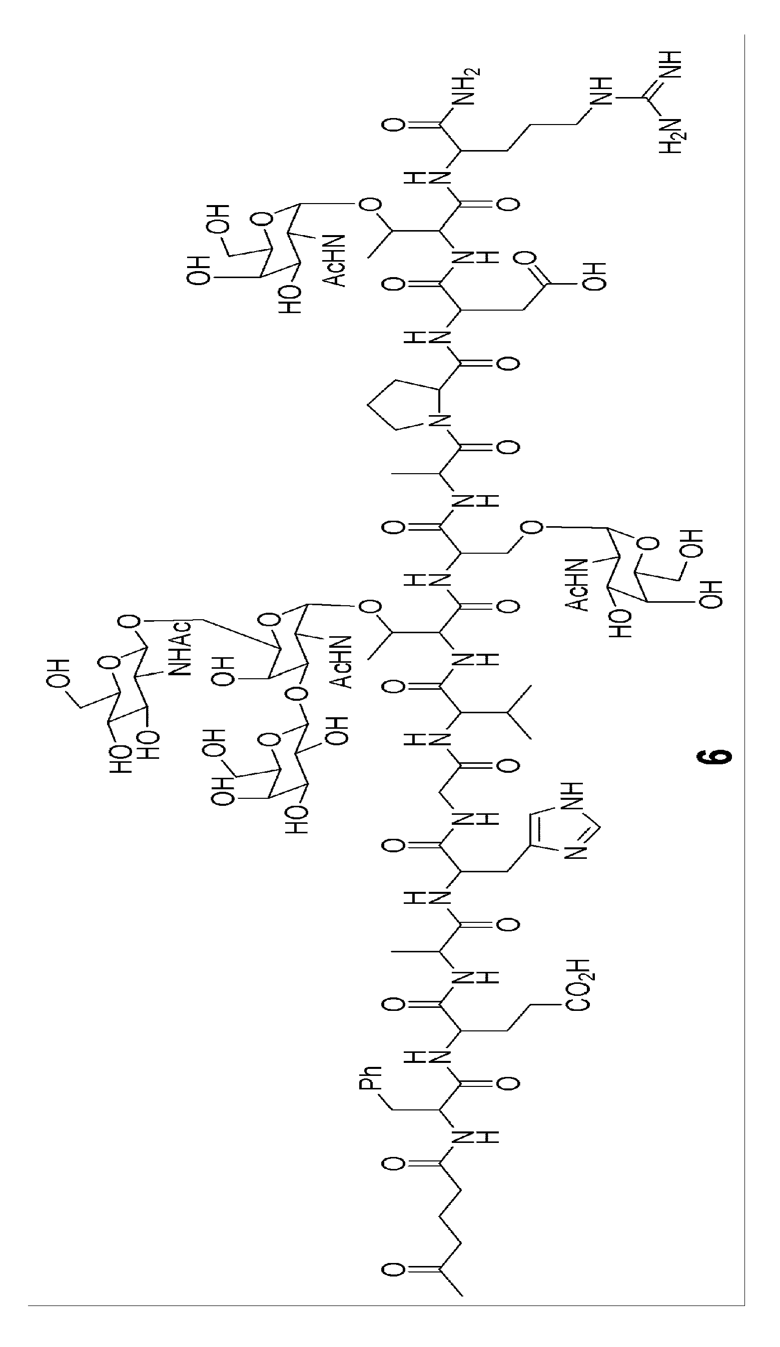

Compound (5) was dissolved in 5ml of methanol, and pH was adjusted to be 12.0 with 0.1N sodium hydroxide aqueous solution. While adjusting pH with 0.1N sodium hydroxide aqueous solution at any time, the solution was stirred for two hours until completion of the reaction. After completion of the reaction, H⁺-type cation exchange resin, Dowex50WX8 (available from Dow Chemical), was added and the solution was neutralized. Thereafter, the resin was separated by filtration. The solvent in the filtrate was removed, and the residue was purified by reverse phase HPLC (Inertsil®, ODS-3 20×250mm column, Mobile phase: A: 0.1% TFA aqueous solution; B: acetonitrile containing 0.1%TFA, Gradient: 0% to 60%B with respect to A), thereby obtaining 4.4mg of compound (6) (yield: 56%). MALDI-TOF/MS:[M(average)+H]⁺=2472.952 (theoretical value: [M(average)+H]⁺=2474.5)

### (1.7 Synthesis of compound (7))

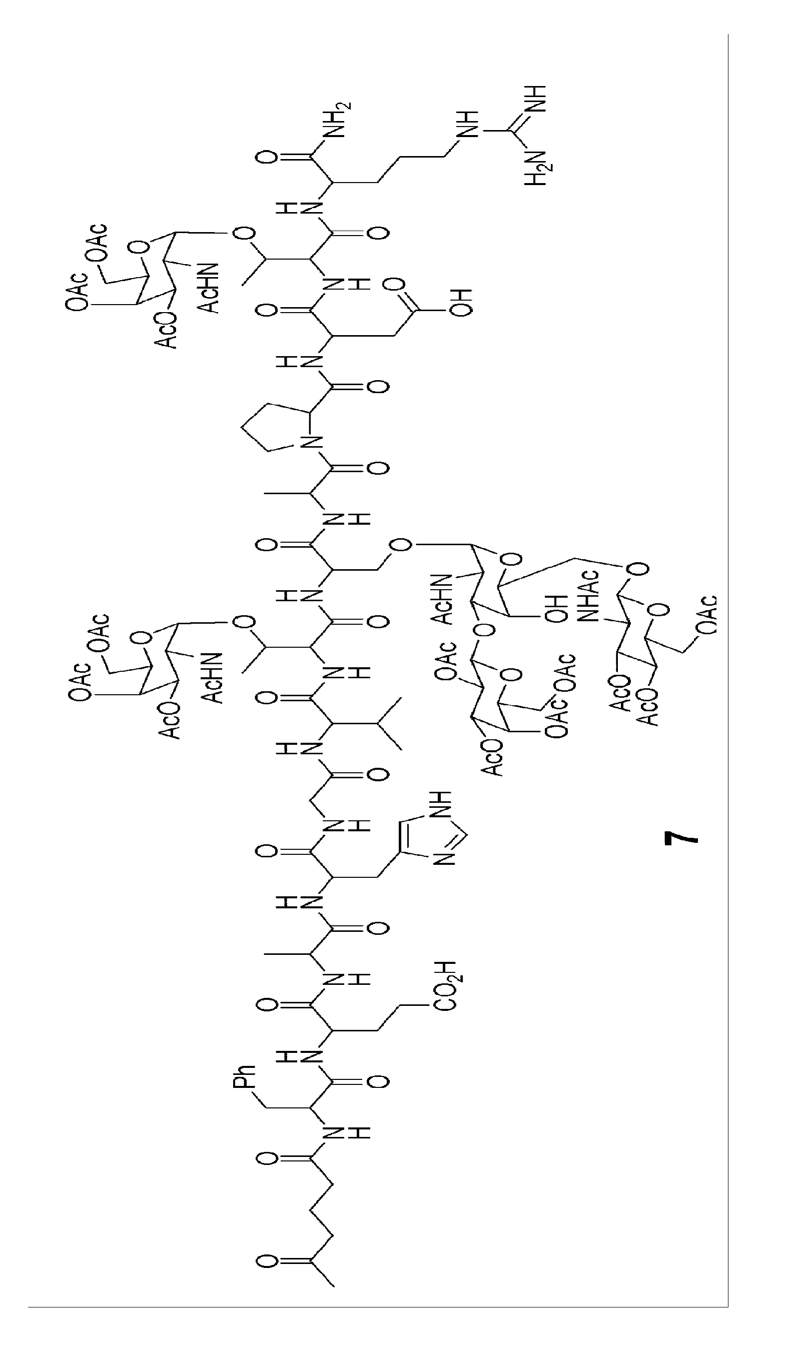

Using 0.12g (0.03mmol) of Tentagel® (Hipep Laboratories, 0.25mmol/g) as a support, N-protected amino acids and keto acid as described below were sequentially condensed by Fmoc/HBTU/HOBt method, thereby synthesizing a glycopeptide derivative of interest: Fmoc-Arg(Pbf)-OH; Fmoc-Ser(Ac7core2)-OH; Fmoc-Asp(OtBu)-OH; Fmoc-Pro-OH; Fmoc-Ala-OH; Fmoc-Thr(Ac3GalNAc)-OH; Fmoc-Val-OH; Fmoc-Gly-OH; Fmoc-His(Trt)-OH; Fmoc-Ala-OH; Fmoc-Gln(OtBu)-OH; Fmoc-Phe-OH; and 5-ketohexane acid. After the peptide elongation reaction, the glycopeptide derivative was allowed to react in 90% TFA aqueous solution for two hours at room temperature to eliminate the protective group on the peptide residue and concurrently release compound (7) from the solid-phase support. The resin was separated by filtration, and TFA was removed by volatilization. Thereafter, the resin was dissolved in 10% acetonitrile aqueous solution and a solid body was purified by reverse phase HPLC (Inertsil®, ODS-3 20×250mm column, Mobile phase: A: 0.1% TFA aqueous solution; B: acetonitrile containing 0.1% TFA, Gradient: 10% to 70%B with respect to A), thereby obtaining 11.3mg of compound (7) (yield: 12%). MALDI-TOF/MS:[M(average)+H]⁺=3018.602 (theoretical value: [M(average)+H]⁺=3021.0)

(1.8 Synthesis of compound (8))

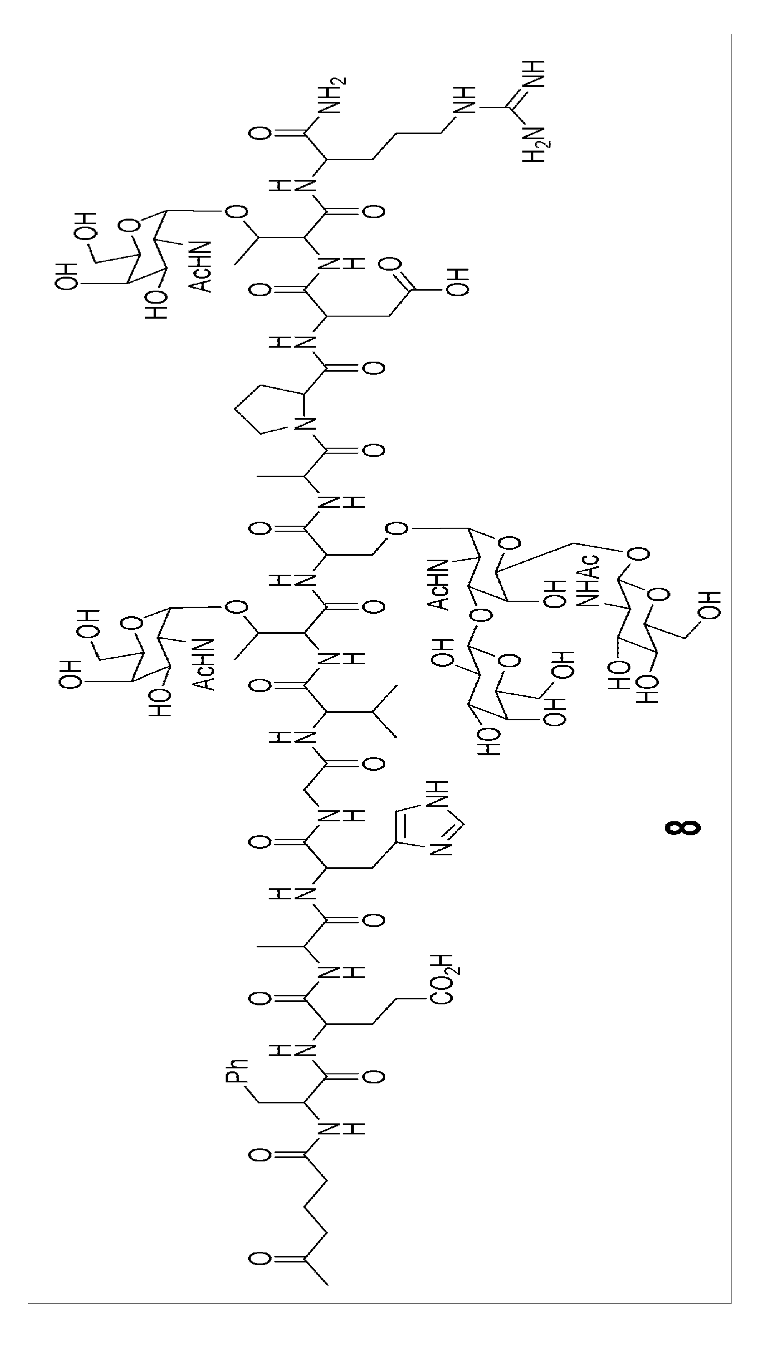

Compound (7) was dissolved in 5ml of methanol, and pH was adjusted to be 12.0 with 0.1N sodium hydroxide aqueous solution. While adjusting pH with 0.1N sodium hydroxide aqueous solution at any time, the solution was stirred for two hours until completion of the reaction. After completion of the reaction, H⁺-type cation exchange resin, Dowex50WX8 (available from Dow Chemical), was added and the solution was neutralized. Thereafter, the resin was separated by filtration. The solvent in the filtrate was removed, and the residue was purified by reverse phase HPLC (Inertsil®, ODS-3 20×250mm column, Mobile phase: A: 0.1% TFA aqueous solution; B: acetonitrile containing 0.1%TFA, Gradient: 0% to 60%B with respect to A), thereby obtaining 5.6mg of compound (8) (yield: 620). MALDI-TOF/MS:[M(average)+H]⁺=2473.328 (theoretical value: [M(average)+H]⁺=2474.5)

(1.9 Synthesis of compound (9))

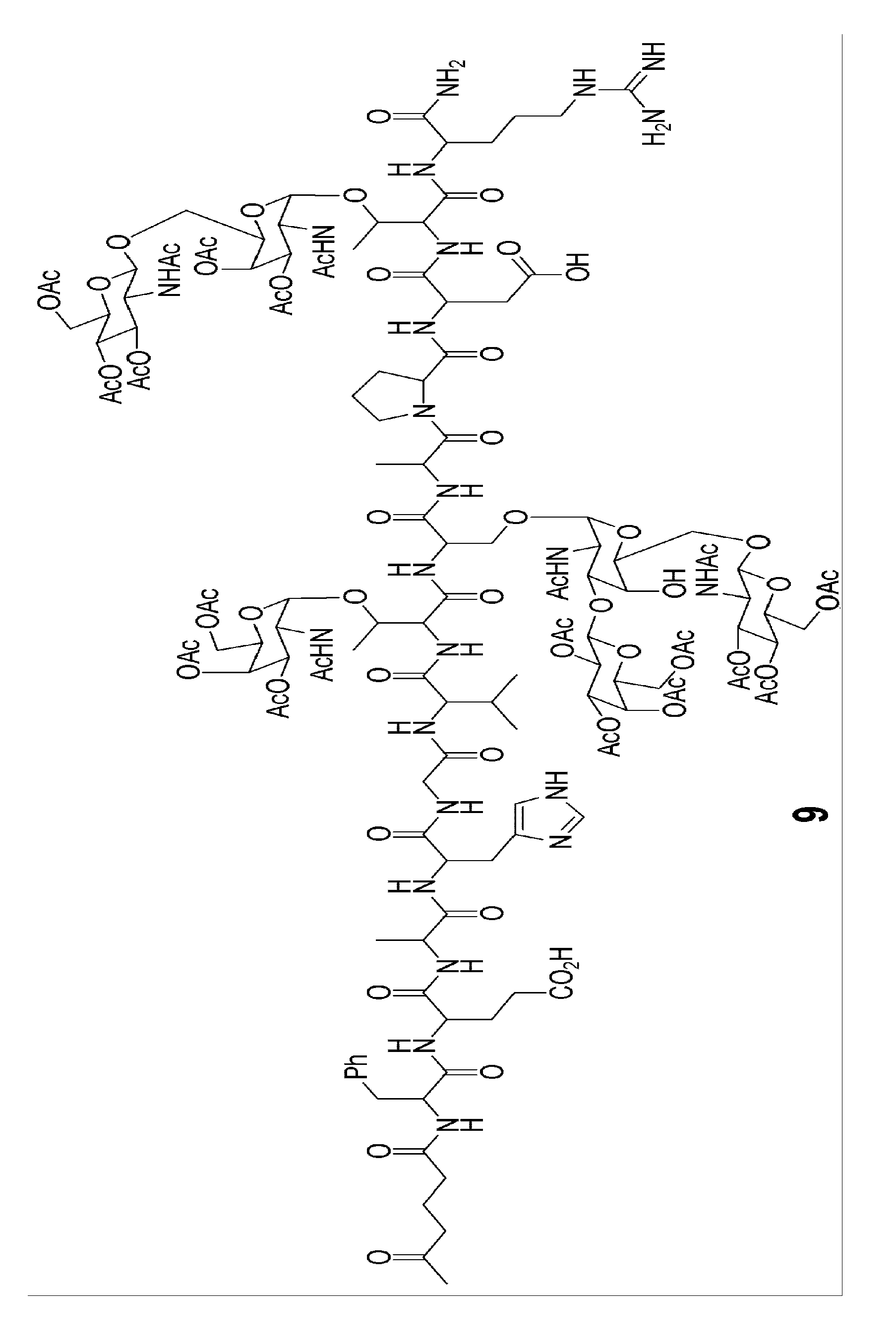

Using 0.12g (0.03mmol) of Tentagel® (Hipep Laboratories, 0.25mmol/g) as a support, N-protected amino acids and keto acid as described below were sequentially condensed by Fmoc/HBTU/HOBt method, thereby synthesizing a glycopeptide derivative of interest: Fmoc-Arg(Pbf)-OH; Fmoc-Thr(Ac5core6)-OH; Fmoc-Asp(OtBu)-OH; Fmoc-Pro-OH; Fmoc-Ala-OH; Fmoc-Ser(Ac7core2)-OH; Fmoc-Thr(Ac3GalNAc)-OH; Fmoc-Val-OH; Fmoc-Gly-OH; Fmoc-His(Trt)-OH; Fmoc-Ala-OH; Fmoc-Gln(OtBu)-OH; Fmoc-Phe-OH; and 5-ketohexane acid. After the peptide elongation reaction, the glycopeptide derivative was allowed to react in 90% TFA aqueous solution for two hours at room temperature to eliminate the protective group on the peptide residue and concurrently release compound (9) from the solid-phase support. The resin was separated by filtration, and TFA wasremoved by volatilization. Thereafter, the resin was dissolved in 10% acetonitrile aqueous solution and a solid body was purified by reverse phase HPLC (Inertsil®, ODS-3 20x250mm column, Mobile phase: A: 0.1% TFA aqueous solution; B: acetonitrile containing 0.1% TFA, Gradient: 10% to 70%B with respect to A), thereby obtaining 17mg of compound (9) (yield: 17%). MALDI-TOF/MS: [M(average)+H]⁺ =3306.3 (theoretical value: [M(average)+H]⁺=3308.3)

(1.10 Synthesis of compound (10))

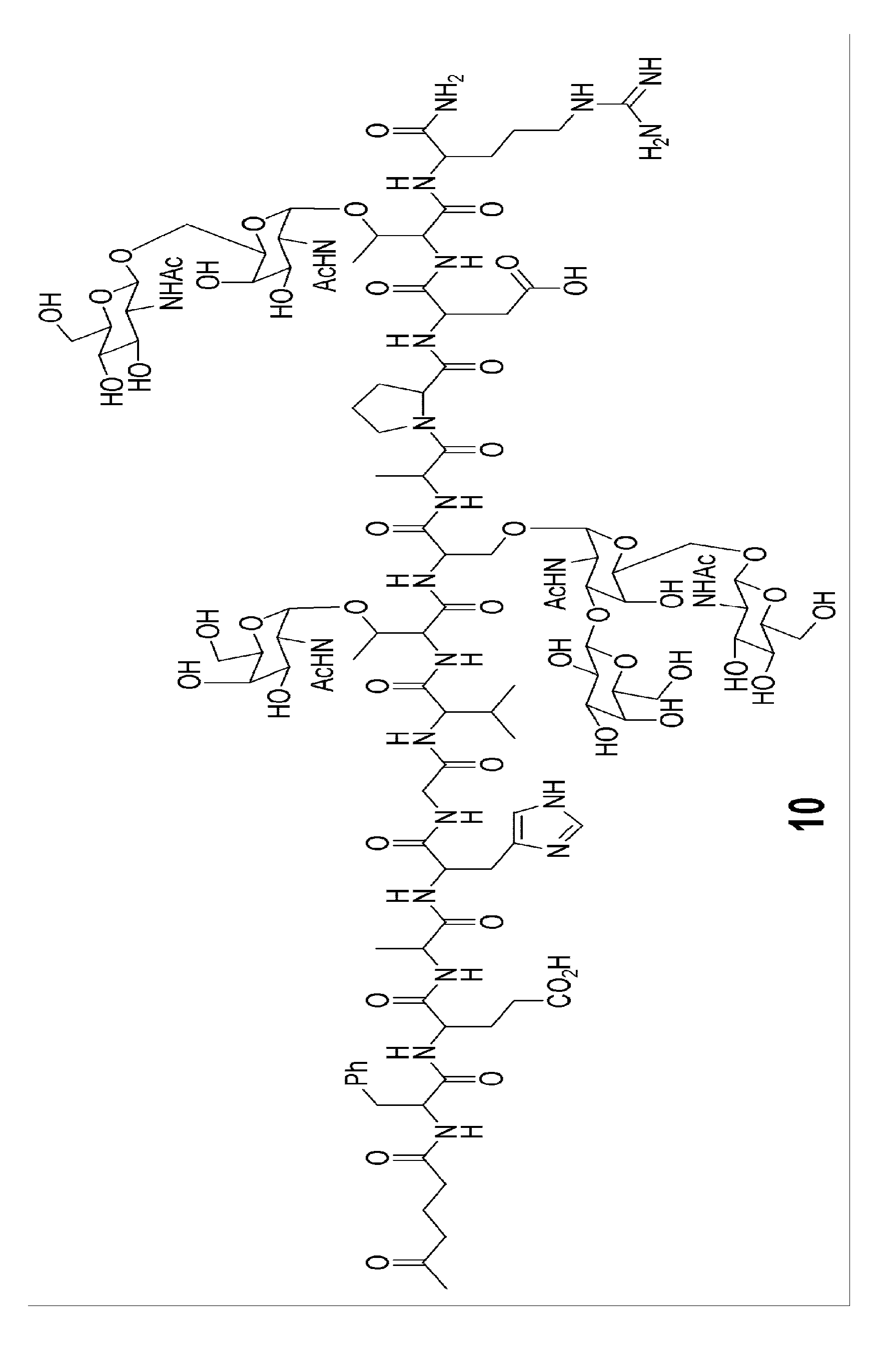

Compound (9) was dissolved in 5ml of methanol, and pH was adjusted to be 12.0 with 0.1N sodium hydroxide aqueous solution. While adjusting pH with 0.1N sodium hydroxide aqueous solution at any time, the solution was stirred for two hours until completion of the reaction. After completion of the reaction, H⁺-type cation exchange resin, Dowex50WX8 (available from Dow Chemical), was added and the solution was neutralized. Thereafter, the resin was separated by filtration. The solvent in the filtrate was removed, and the residue was purified by reverse phase HPLC (Inertsil®, ODS-3 20x250mm column, Mobile phase: A: 0.1% TFA aqueous solution; B: acetonitrile containing 0.1% TFA, Gradient: 0% to 60%B with respect to A), thereby obtaining 5.6mg of compound (10) (yield: 40%). MALDI-TOF/MS:[M(average)+H]⁺ =2675.5 (theoretical value: [M(average)+H]⁺=2677.7)

(1.11 Synthesis of compound (11))

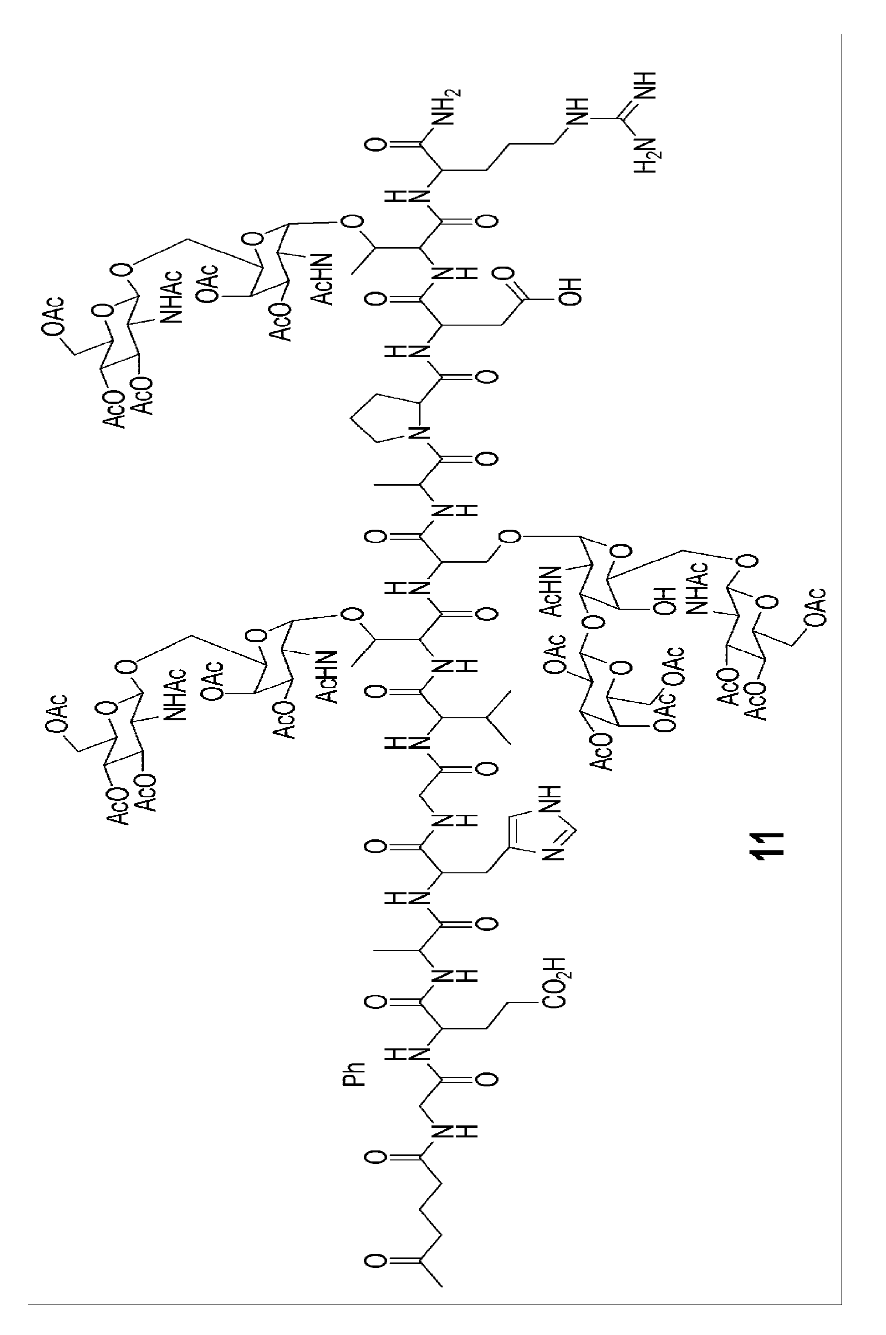

Using 0.12g (0.03mmol) of Tentagel® (Hipep Laboratories, 0.25mmol/g) as a support, N-protected amino acids and keto acid as described below were sequentially condensed by Fmoc/HBTU/HOBt method, thereby synthesizing a glycopeptide derivative of interest: Fmoc-Arg(Pbf)-OH; Fmoc-Thr(Ac5core6)-OH; Fmoc-Asp(OtBu)-OH; Fmoc-Pro-OH; Fmoc-Ala-OH; Fmoc-Ser(Ac7core2)-OH; Fmoc-Thr(Ac5core6)-OH; Fmoc-Val-OH; Fmoc-Gly-OH; Fmoc-His(Trt)-OH; Fmoc-Ala-OH; Fmoc-Gln(OtBu)-OH; Fmoc-Phe-OH; and 5-ketohexane acid. After the peptide elongation reaction, the glycopeptide derivative was allowed to react in 90% TFA aqueous solution for two hours at room temperature to eliminate the protective group on the peptide residue and concurrently release compound (11) from the solid-phase support. The resin was separated by filtration, and TFA wasremoved by volatilization. Thereafter, the resin was dissolved in 10% acetonitrile aqueous solution and a solid body was purified by reverse phase HPLC (Inertsil®, ODS-3 20×250mm column, Mobile phase: A: 0.1% TFA aqueous solution; B: acetonitrile containing 0.1% TFA, Gradient: 10% to 70%B with respect to A), thereby obtaining 24mg of compound (11) (yield: 22%). MALDI-TOF/MS:[M(average)+H]⁺ =3593.4 (theoretical value: [M(average)+H]⁺=3594.5)

(1.12 Synthesis of compound (12))

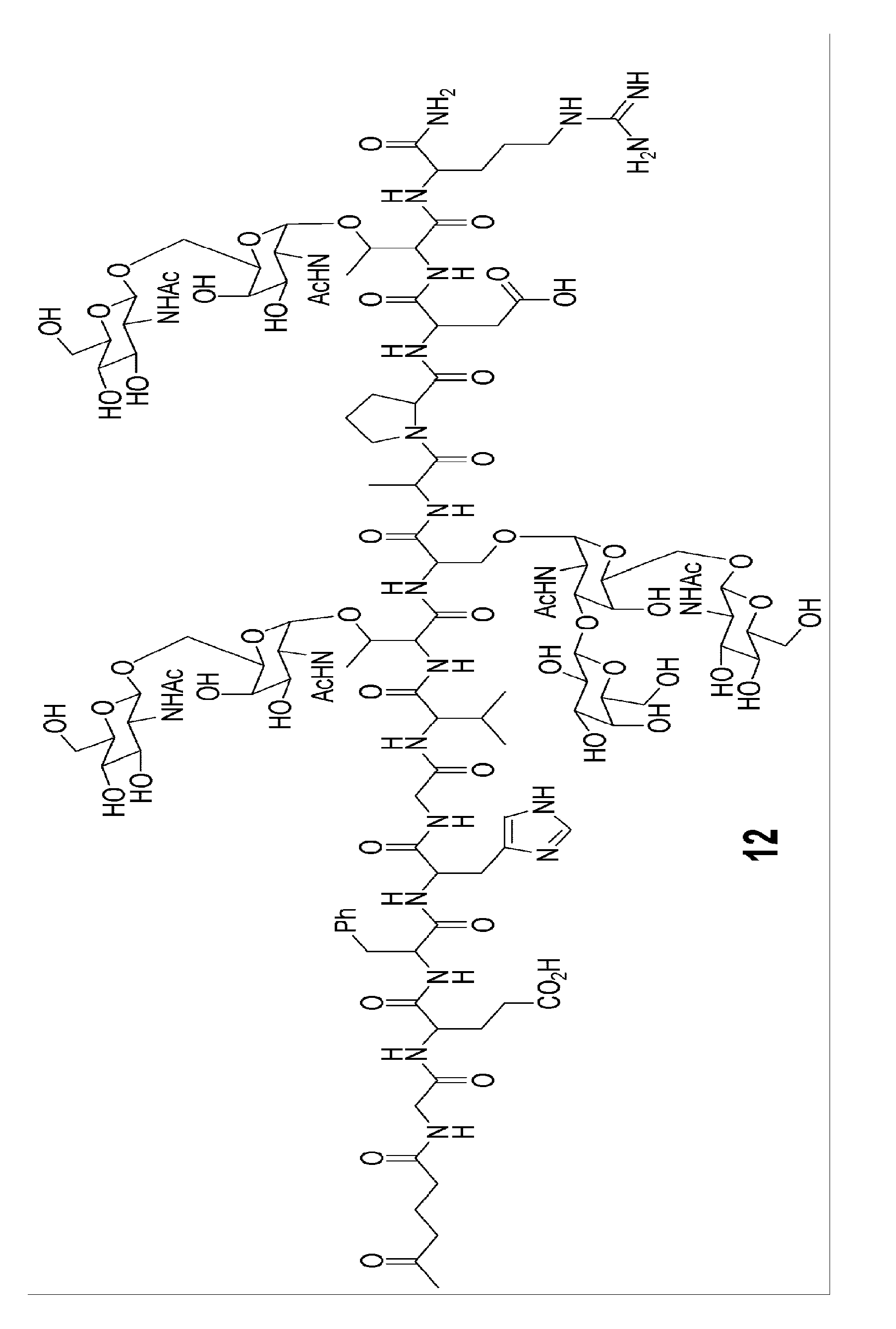

Compound (3) was dissolved in 5ml of methanol, and pH was adjusted to be 12.0 with 0.1N sodium hydroxide aqueous solution. While adjusting pH with 0.1N sodium hydroxide aqueous solution at any time, the solution was stirred for two hours until completion of the reaction. After completion of the reaction, H⁺-type cation exchange resin, Dowex50WX8 (available from Dow Chemical), was added and the solution was neutralized. Thereafter, the resin was separated by filtration. The solvent in the filtrate was removed, and the residue was purified by reverse phase HPLC (Inertsil®, ODS-3 20×250mm column, Mobile phase: A: 0.1% TFA aqueous solution; B: acetonitrile containing 0.1%TFA, Gradient: 0% to 60%B with respect to A), thereby obtaining 6.2mg of compound (12) (yield: 31%). MALDI-TOF/MS:[M(average)+H]⁺=2879.597 (theoretical value: [M(average)+H]⁺ =2880.9)

(Example 2: Glycosyl transfer reaction and selective cleavage reaction by a protease to MUC1-related peptide derivative having a ketone derivative at the N-terminus)

### (2.1 Synthesis of compounds (13) and (14))

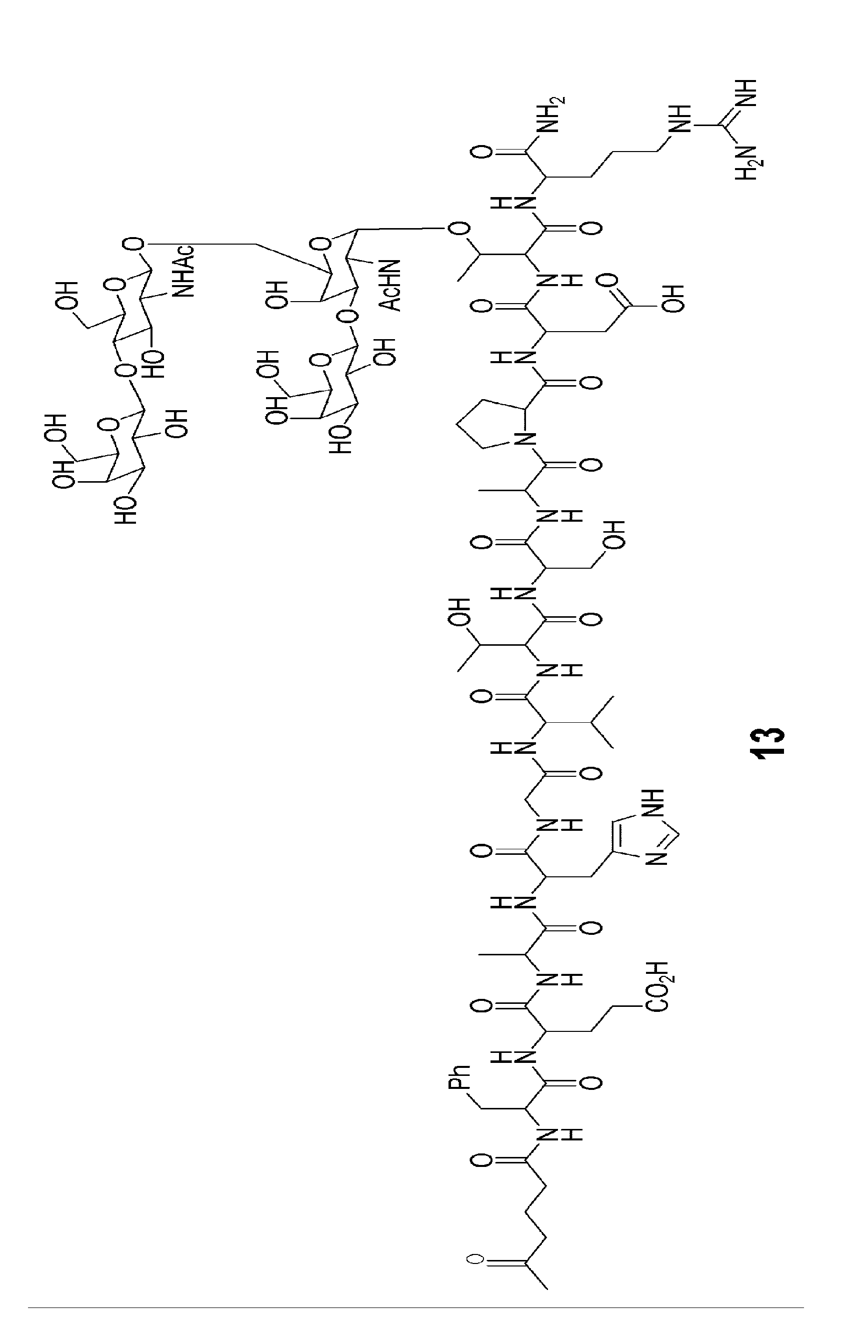

50µl of reaction solution containing 25mM HEPES buffer solution (pH 7.6), 0.20U/ml human-derived β1,4-galactosyltransferase (available from TOYOBO CO., LTD.), 10mM manganese chloride, 5mM uridine-5'-disodium diphosphogalactose (UDP-Gal) and 1mM glycopeptide derivative (2) was stirred for 45 minutes at 25°C. A part of the reaction solution was purified by reverse phase HPLC (Inertsil®, ODS-3 4.6×250mm column, Mobile phase: A: 0.1% TFA aqueous solution; B: acetonitrile containing 0.1% TFA, Gradient: 5% to 40%B with respect to A), thereby obtaining compound (13) [ratio of transfer: 95% or higher (HPLC)]. Identification of the transferred compound was performed by confirming [M(average)+H]⁺ =2228.6 (theoretical value: [M(average)+H]⁺ =2230.3) derived from compound (13) by MALDI-TOF/MS.

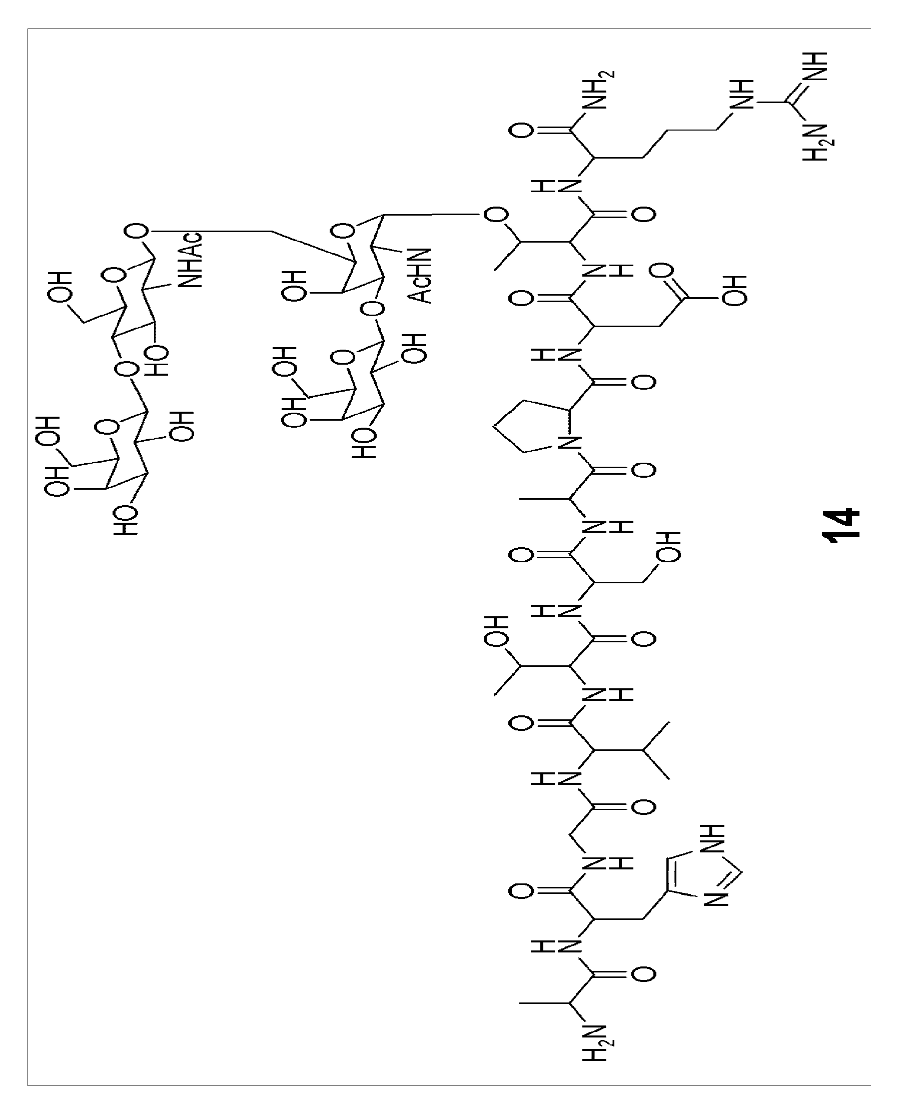

To 10µl of the above reaction solution, 2µl of 1.74mg/ml solution of protease (BLase: available from Shionogi & Co., Ltd.) derived from *Bacillus Licheniformis,* specific for glutamic acid residue was added, and the solution was stirred for 45 minutes at 25°C. Identification of the transferred compound was performed by analyzing the reaction solution by MALDI-TOF/MS to confirm [M(average)+H]⁺=1840.7 (theoretical value: [M(average)+H]⁺ =1841.9) derived from compound (14).

(2.2 Synthesis of compounds (15) and (16))

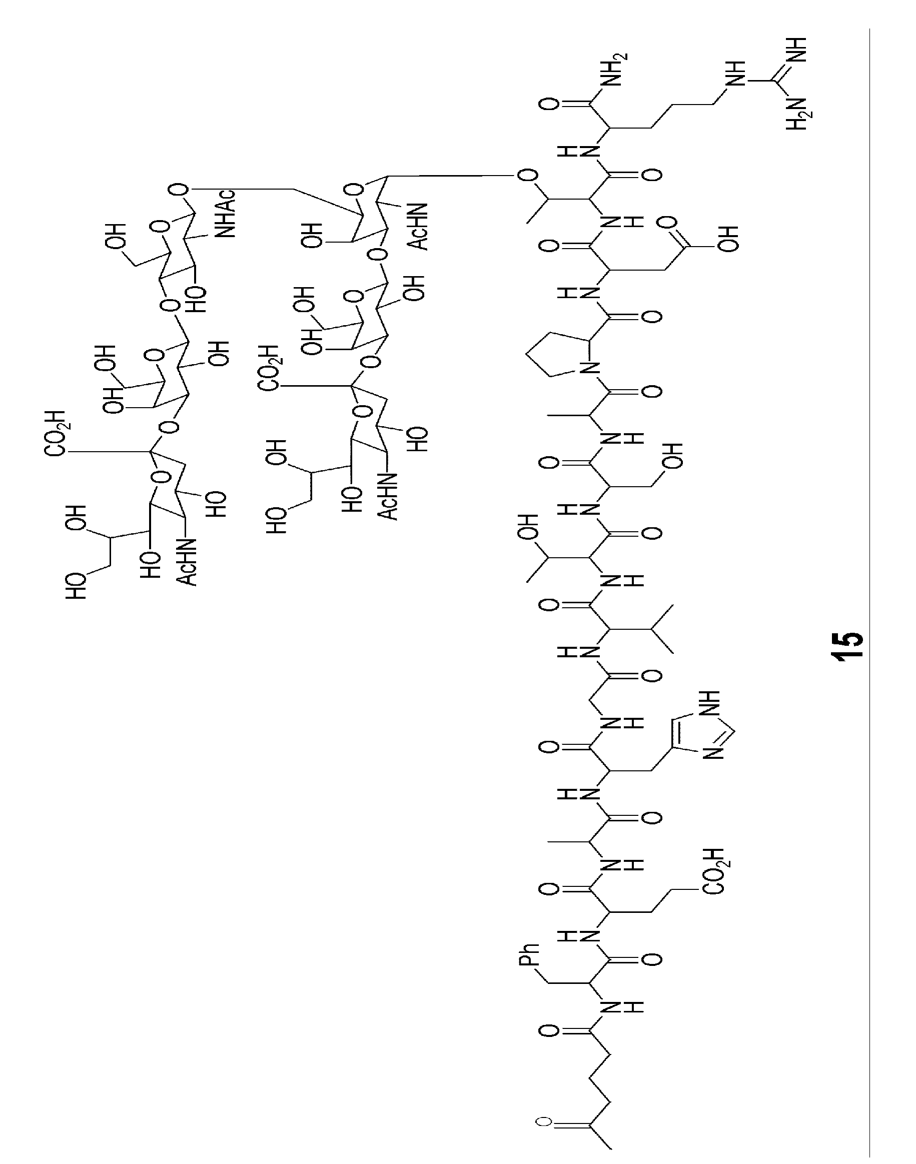

50µl of reaction solution containing 25mM HEPES buffer solution (pH 7.0), 0.1% Triton X-100, 74mU/ml recombinant rat α2, 3- (N) -sialyltransferase (available from Calbiochem), 17.5mU/ml recombinant rat α2,3-(O)-sialyltransferase (available from Calbiochem), 5mM cytidine-5'-sodium monophosphosialate (CMP-NANA), 1mM glycopeptide derivative (13) was stirred for 4 hours at 25°C. A part of the reaction solution was purified by reverse phase HPLC (Inertsil®, ODS-3 4.6×250mm column, Mobile phase: A: 0.1% TFA aqueous solution; B: acetonitrile containing 0.1% TFA, Gradient: 5% to 40%B with respect to A), thereby obtaining compound (15) [ratio of transfer: 95% or higher (HPLC)]. Identification of the transferred compound was performed by confirming [M(average)+H]⁺ =2811.8 (theoretical value: [M(average)+H]⁺ =2812.8) derived from compound (15) by MALDI-TOF/MS.

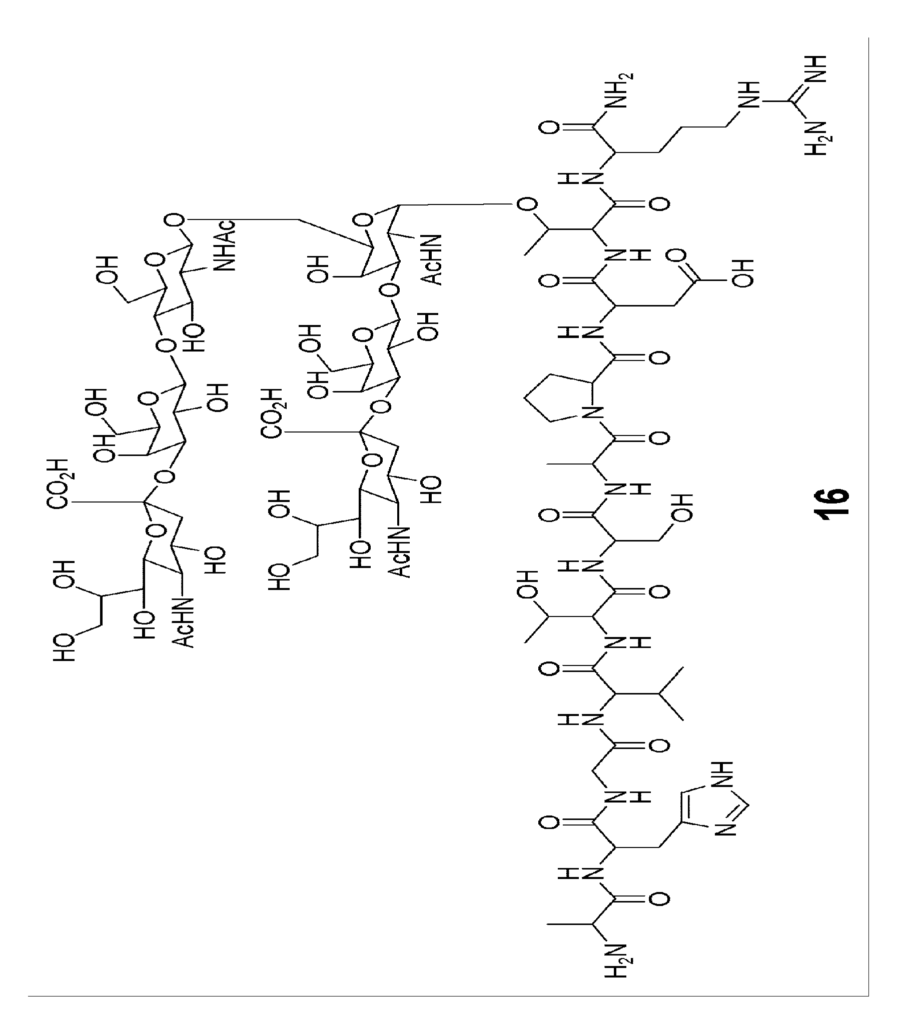

To 5µl of the above reaction solution, 4µl of Milli Q water and 1µl of 1. 74mg/ml solution of BLase (available from Shionogi & Co., Ltd.) were added, and the solution was stirred for 14 hours at 25°C. The reaction solution was purified by reverse phase HPLC (Inertsil®, ODS-3 4.6×250mm column, Mobile phase: A: 0.1% TFA aqueous solution; B: acetonitrile containing 0.1% TFA, Gradient: 5% to 40%B with respect to A), thereby obtaining compound (16) [yield: 90% or higher (HPLC)]. Identification of the transferred compound was performed by confirming [M(average)+H]⁺=2423.8 (theoretical value: [M(average)+H]⁺ =2424.4) derived from compound (16) by MALDI-TOF/MS.

(Example 3: Synthesis and sugar chain elongation reaction of a polymeric primer for glycopeptide synthesis)

### (3.1 Synthesis of compounds (18), (A))

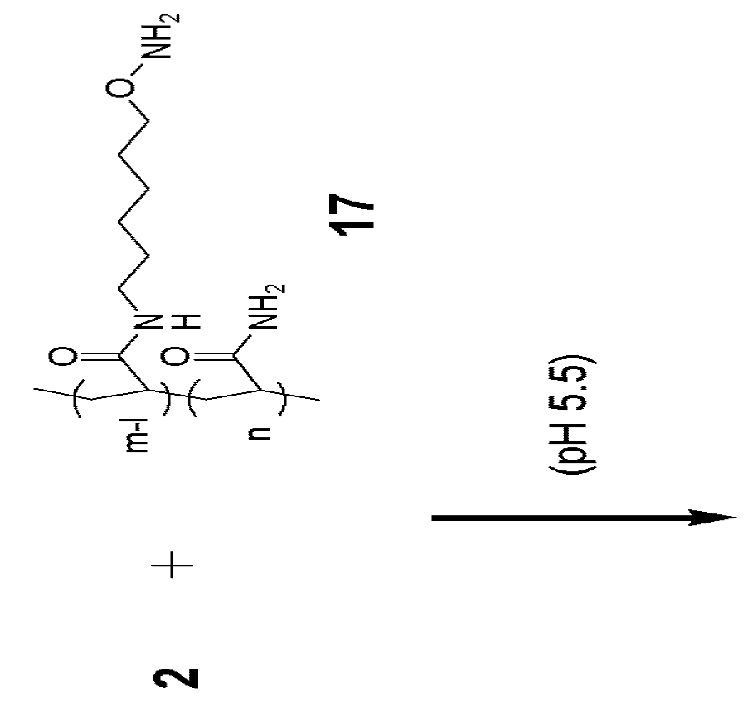

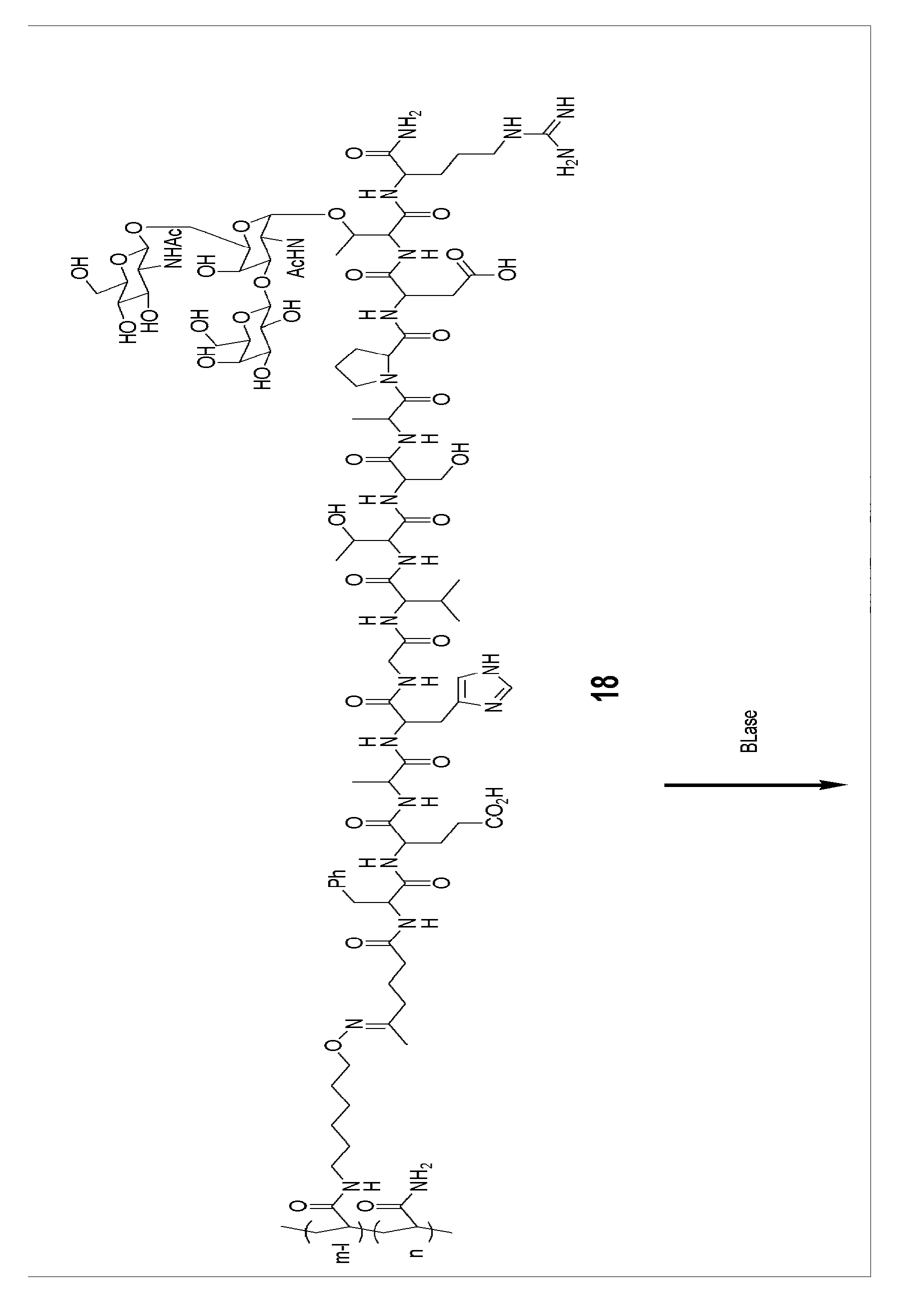

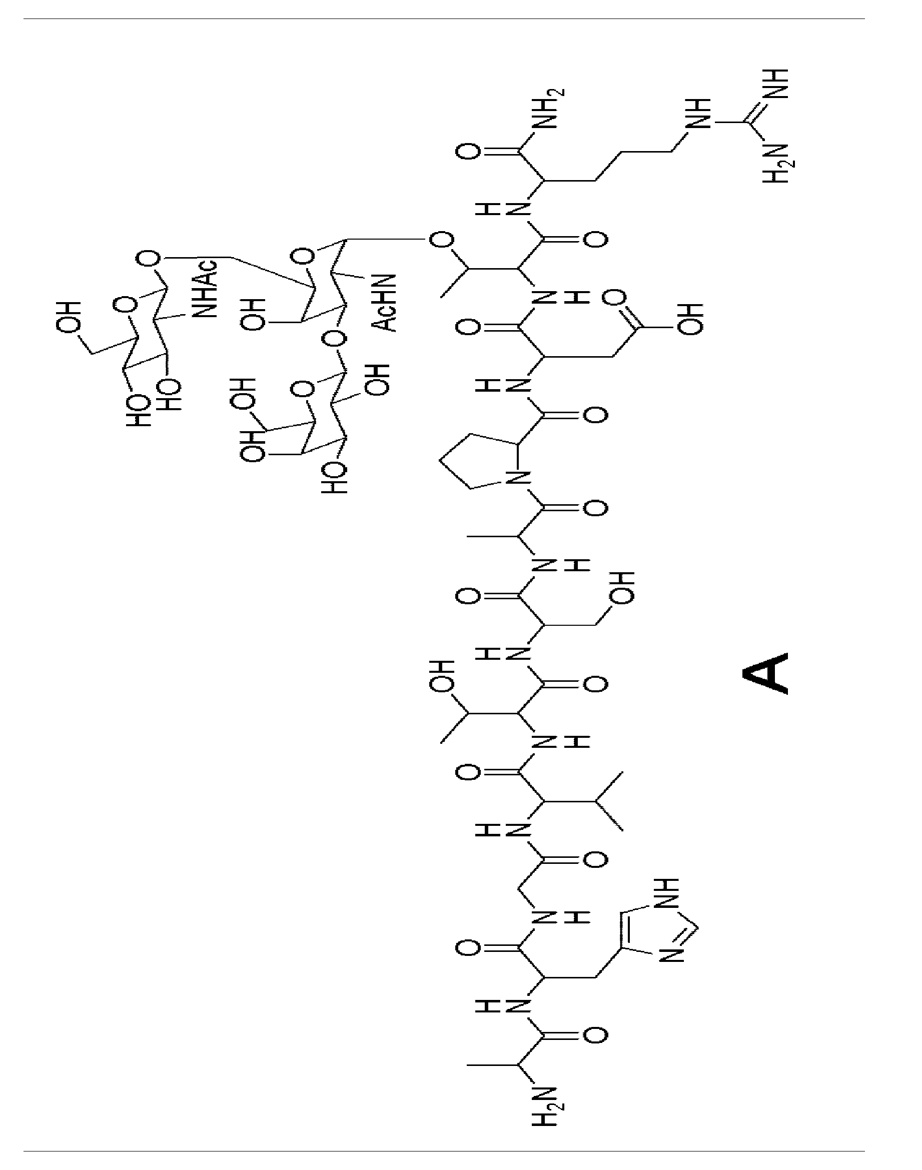

360µl of reaction solution containing 2.5mM glycopeptide derivative (2), 5mM (oxyamine residue calculation) water-soluble polymer (17) and 12.5mM sodium acetate buffer solution (pH 5.5) was stirred for eight hours at room temperature. The reaction solution was purified by gel filtration [Biogel P-4: eluate: 25mM ammonium acetate buffer solution (pH 6.5), thereby obtaining 4.2mg of lyophilized compound (18) [ratio of trapping compound (2): 95% or higher (GPC-HPLC)].

5µl of 5mM aqueous solution of the obtained glycopeptide derivative compound (18) was sorted, and 19µl of Milli Q water and 1µl of 1.74mg/ml solution of BLase (available from Shionogi & Co., Ltd.) was added thereto. The reaction solution was purified by reverse phase HPLC (Inertsil®, ODS-3 4.6×250mm column, Mobile phase: A: 25mM ammonium acetate buffer solution (pH 6.5) ; B: acetonitrile, Gradient: 2% to 20%B with respect to A), thereby obtaining compound (177) [yield: 90% or higher (HPLC)]. MALDI-TOF/MS: [M(average)+H]⁺ of compound (A)=1678.0 (theoretical value: [M(average)+H]⁺=1679.7)

(3.2 Synthesis of compounds (19), (14), (B) and (16))

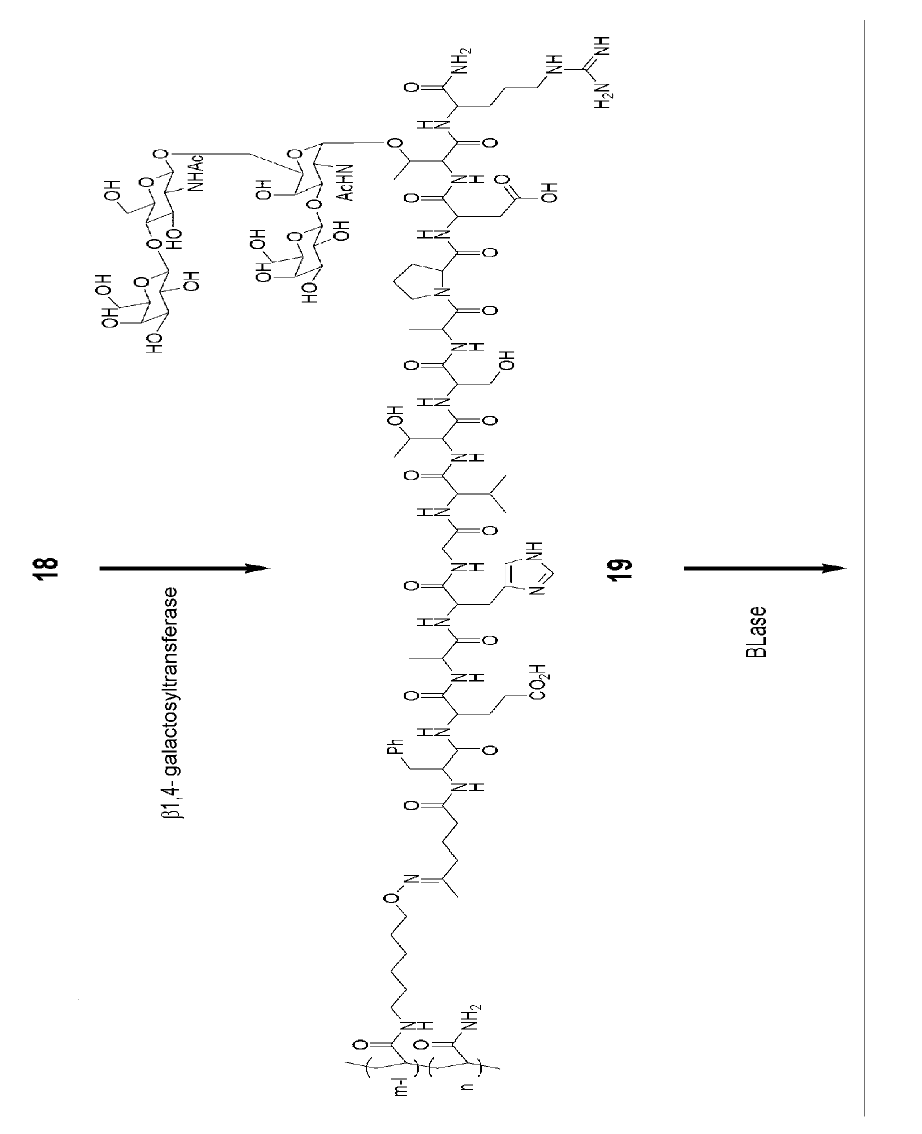

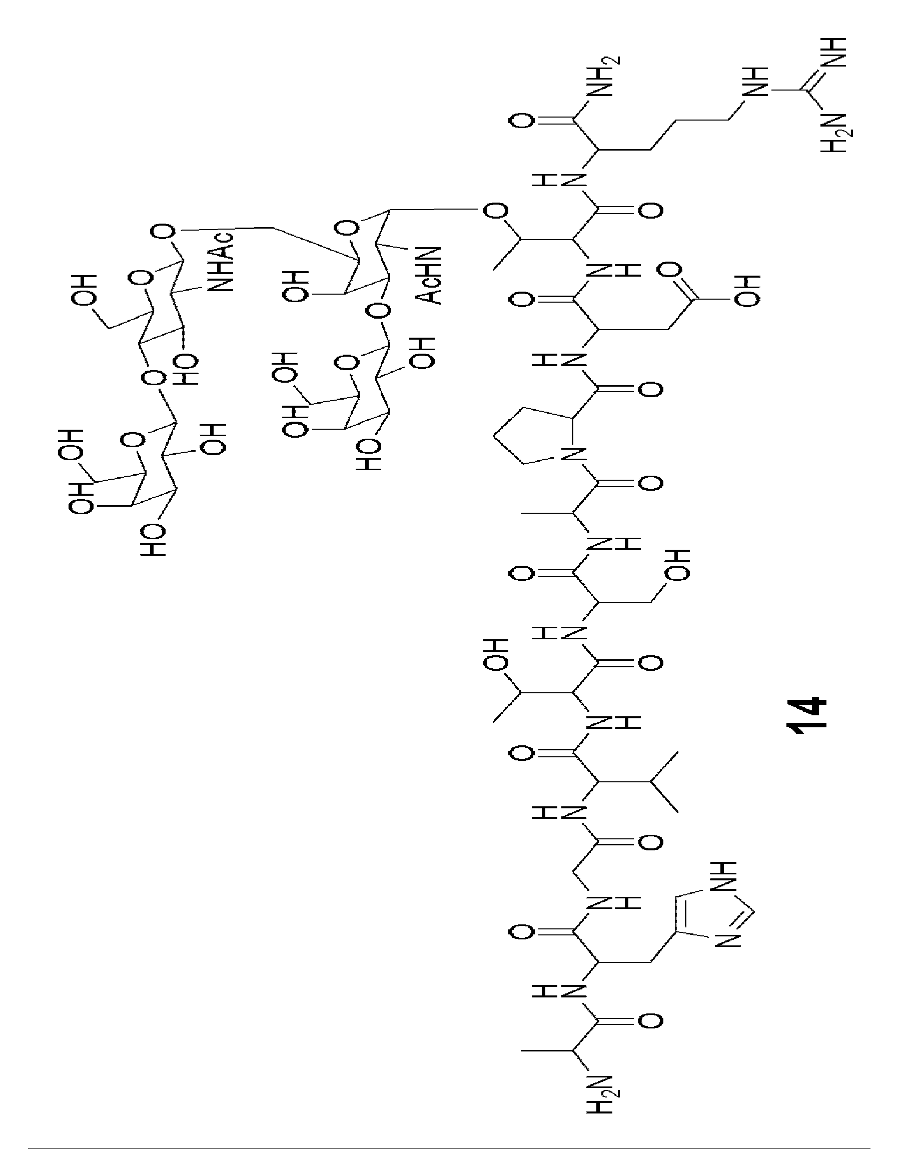

50µl of reaction solution containing 25mM HEPES buffer solution (pH 7.6), 0.20U/ml human-derived β1,4-galactosyltransferase (available from TOYOBO CO., LTD.), 10mM manganese chloride, 5mM uridine-5'-disodium diphosphogalactose (UDP-Gal) and 1mM glycopeptide derivative (18) was stirred for 30 minutes at 25°C, thereby obtaining a reaction solution containing compound (19). 20µl of the reaction solution was sorted, and 4µl of Milli Q water and 1µl of 1.74mg/ml solution of BLase (available from Shionogi & Co., Ltd.) were added, and the solution was stirred at 25°C. The reaction solution was analyzed by MALDI-TOF/MS to confirm [M(average)+H]⁺=1841.6 (theoretical value: [M (average) +H]⁺ =1841.9) derived from compound (14).

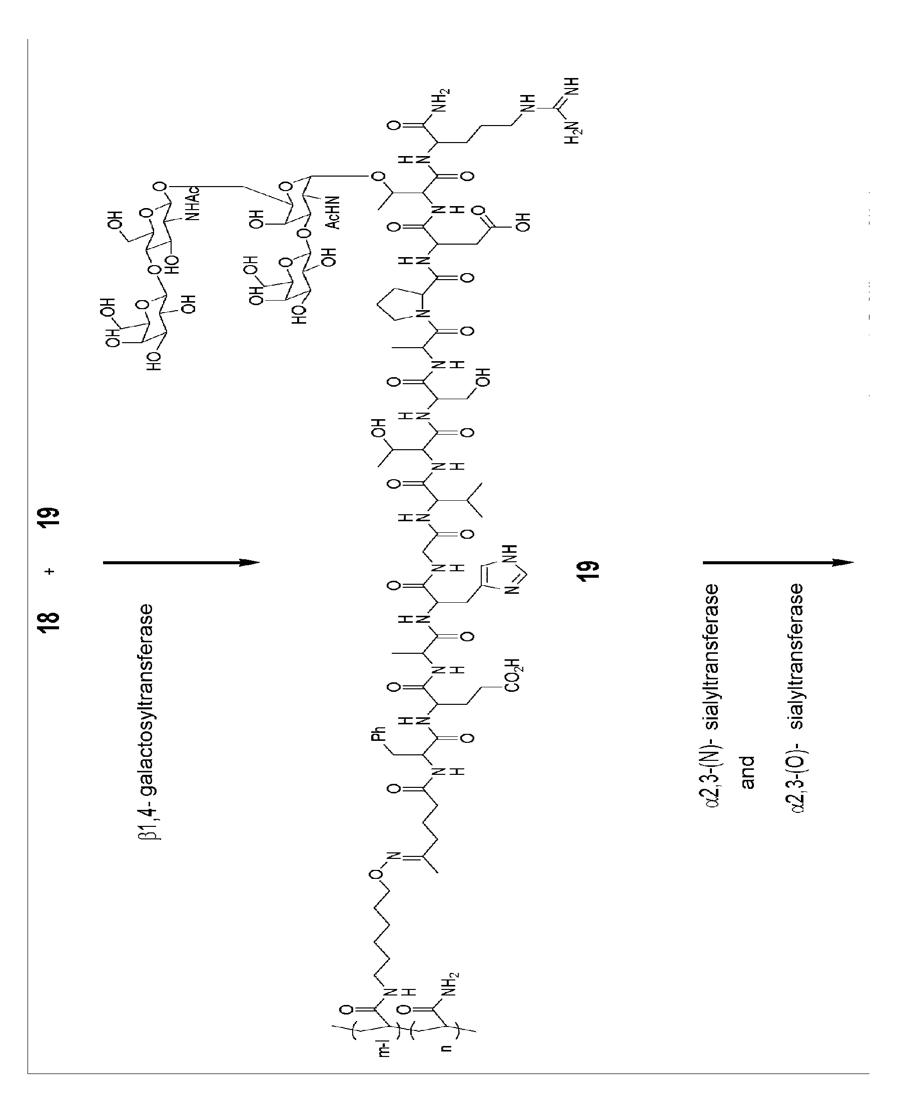

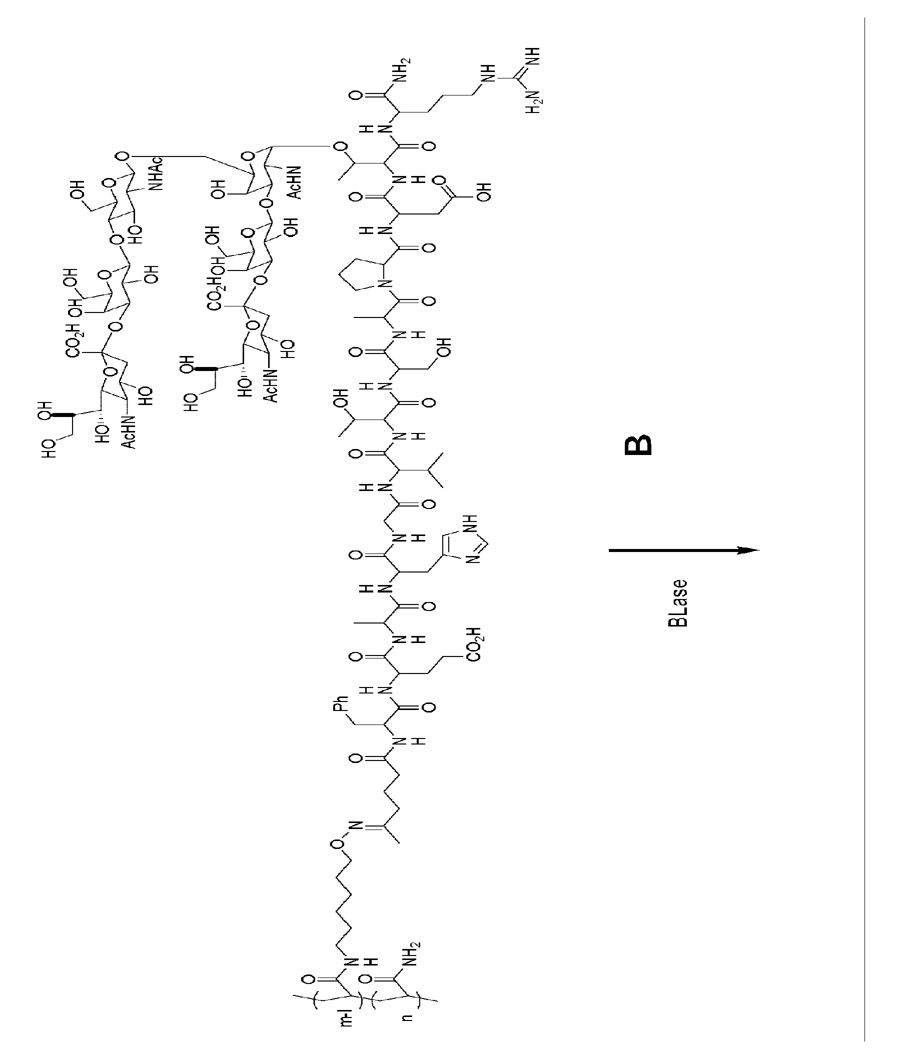

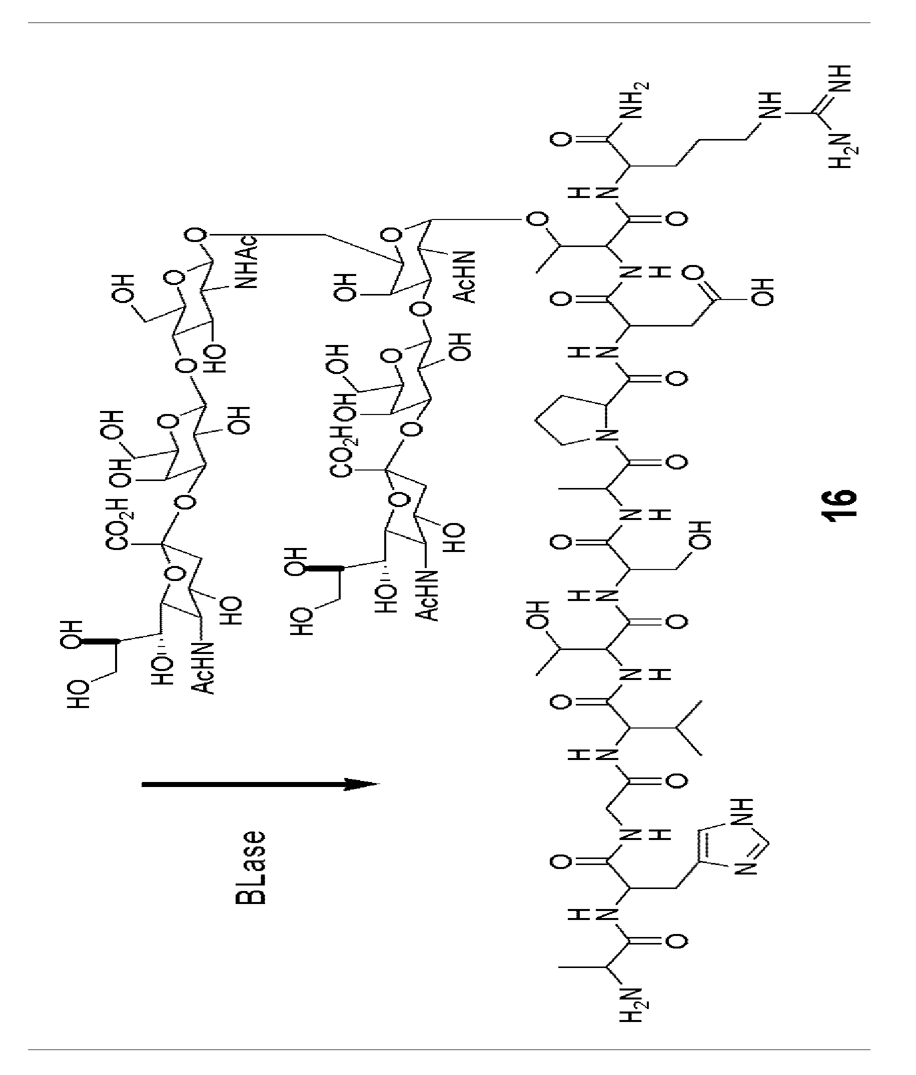

200µl of mixture solution containing 50mM HEPES buffer solution (pH 7.0), 10mM manganese chloride, 1mM (18+19; estimated) of a mixture of compound (18) and compound (19) was added to 300µl of mixture solution containing 50mM HEPES buffer solution (pH 7.0), 0.2U/ml human-derived β1,4-galactosyltransferase (available from TOYOBO CO., LTD.) and 5mM uridine-5'-disodium diphosphogalactose (UDP-Gal) (total amount: 500µl), and the solution was stirred for two hours at 25°C. 400µl of this reaction solution was added to 200µl of mixture solution containing 50mM HEPES buffer solution (pH 7.0), 0.1% Triton X-100 aqueous solution, 5mM cytidine-5'-sodium monophosphosialate (CMP-NANA), 74mU/ml recombinant rat α2,3-(N)-sialyltransferase (available from Calbiochem) and 17.5mU/ml recombinant rat α2,3-(O)-sialyltransferase (available from Calbiochem) (total amount: 600µl), and the solution was stirred for over six hours at 25°C, thereby obtaining a reaction solution containing compound (B). Identification of the transferred compound and the ratio of transfer was performed by sorting 20µl of the reaction solution after glycosyl transfer, adding 4µl of Milli Q water and 1µl of 1.74mg/ml solution of BLase (available from Shionogi & Co., Ltd.) thereto, and purifying the reaction solution by reverse phase HPLC (Inertsil®, ODS-3 4.6×250mm column, Mobile phase: A: 25mM ammonium acetate buffer solution (pH 6.5); B: acetonitrile, Gradient: 2% to 20%B with respect to A), thereby obtaining compound (16) . MALDI-TOF/MS:[M(average)+H]⁺ of compound (25)=2423.9 (theoretical value: [M(average)+H]⁺=2424.4)

(3.3 Synthesis of compounds (20) and (21))

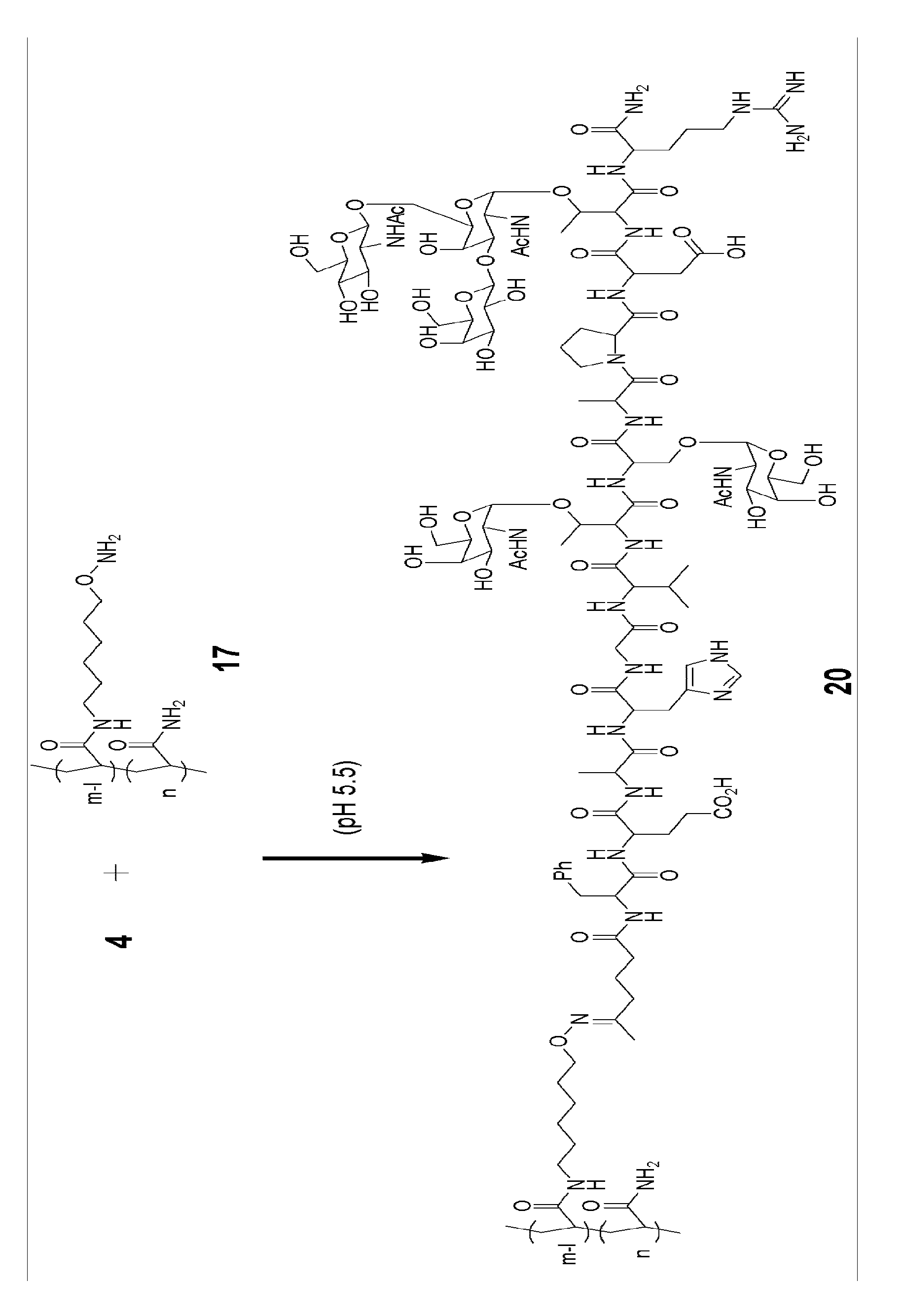

800µl of reaction solution containing 2.5mM glycopeptide derivative (4) and 5mM (oxyamine residue calculation) water-soluble polymer (17) was adjusted to have pH of 5.1 with 1N sodium hydroxide aqueous solution, and was stirred for 18 hours at room temperature. The reaction solution was purified by gel filtration [Biogel P-4: eluate: 25mM ammonium acetate buffer solution (pH 6.5), thereby obtaining 4.2mg of lyophilized compound (20) [ratio of trapping compound (4): 95% or higher (GPC-HPLC)].

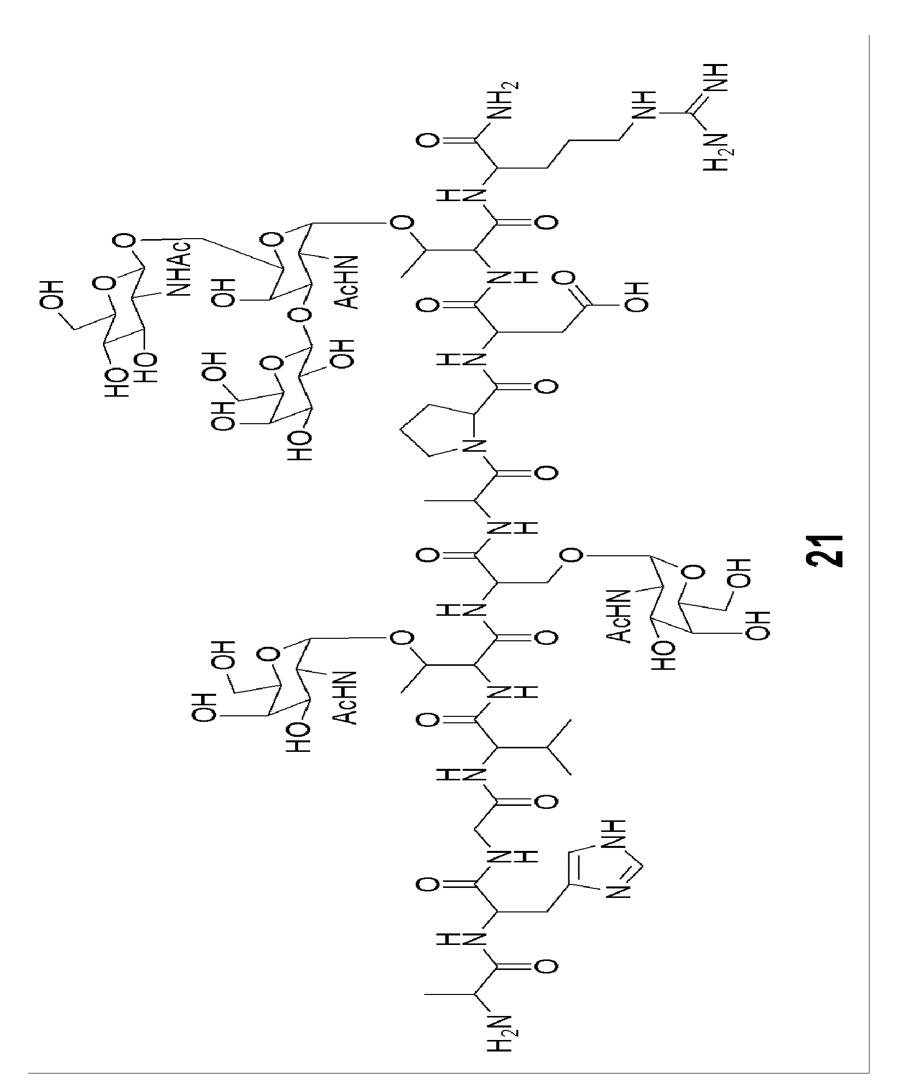

5µl of 5mM aqueous solution of the obtained glycopeptide derivative compound (20) was sorted, and 19µl of Milli Q water and 1µl of 1.74mg/ml solution of BLase (available from Shionogi & Co., Ltd.) was added thereto. The reaction solution was purified by reverse phase HPLC (Inertsil®, ODS-3 4.6×250mm column, Mobile phase: A: 25mM ammonium acetate buffer solution (pH 6.5) ; B: acetonitrile, Gradient: 2% to 20%B with respect to A), thereby obtaining compound (21) [yield: 90% or higher (HPLC)]. MALDI-TOF/MS: [M(average)+H]⁺ of compound (21)=2085.6 (theoretical value: [M(average)+H]⁺=2086.1)

(3.4 Synthesis of compounds (22) to (25))

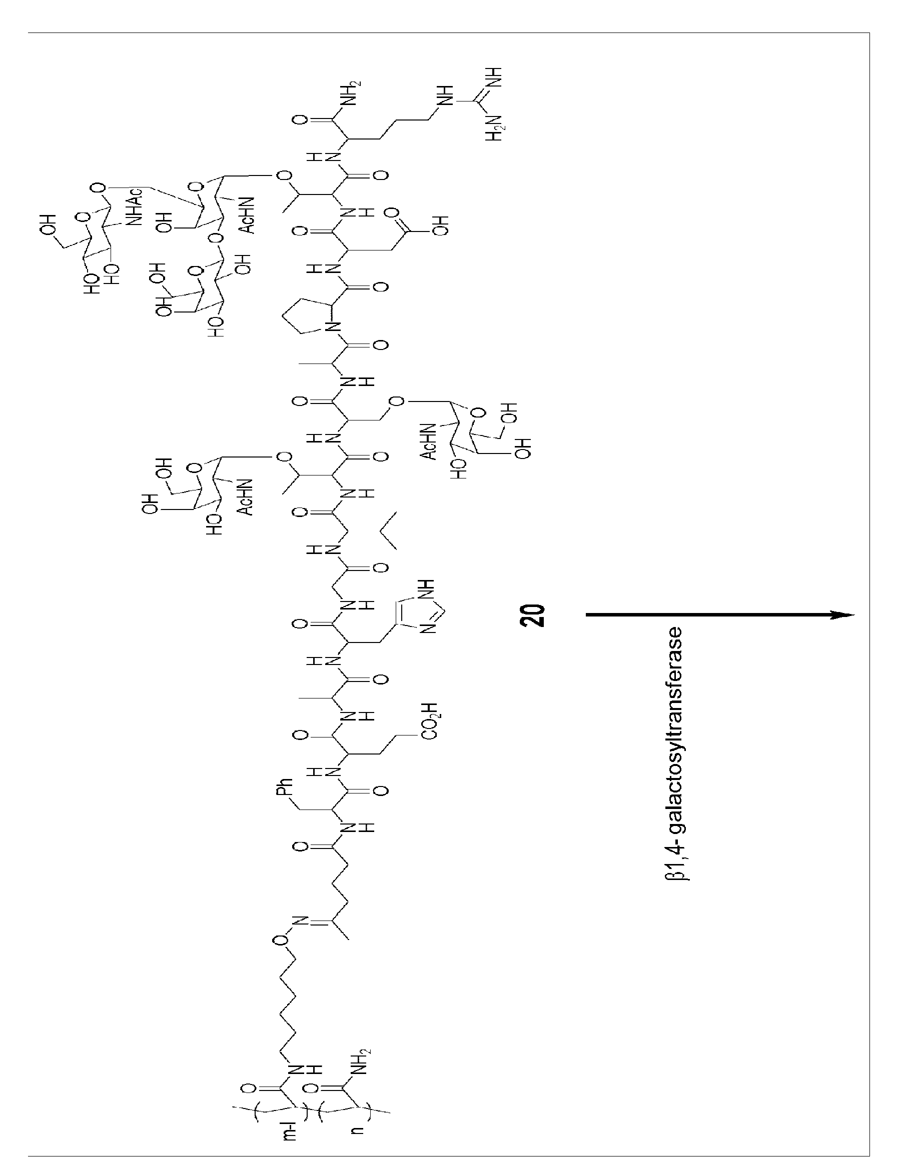

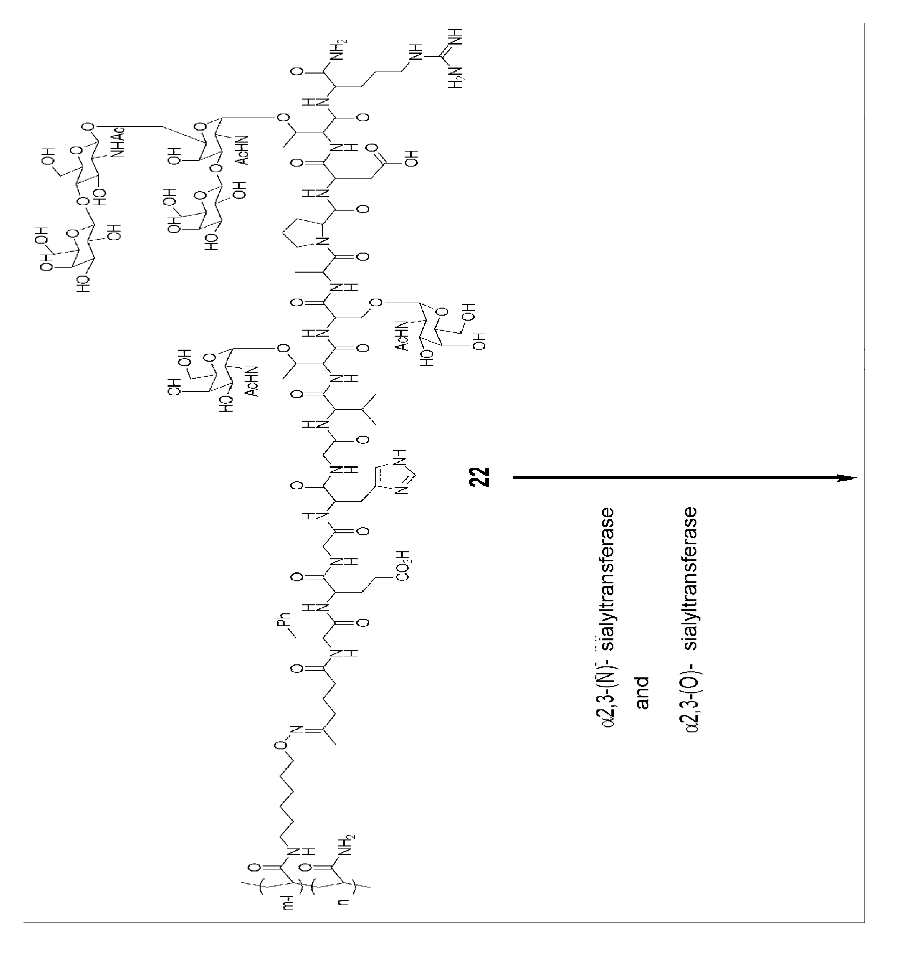

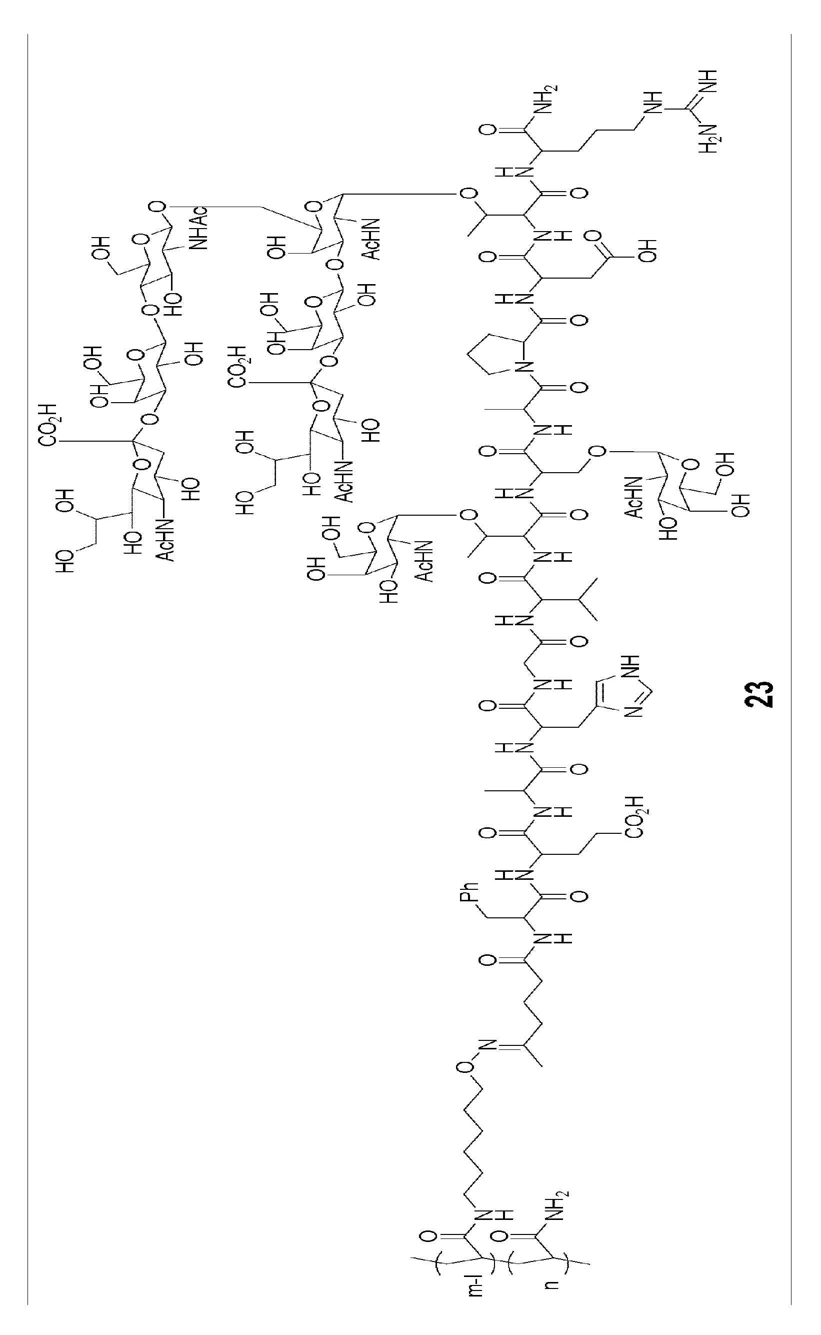

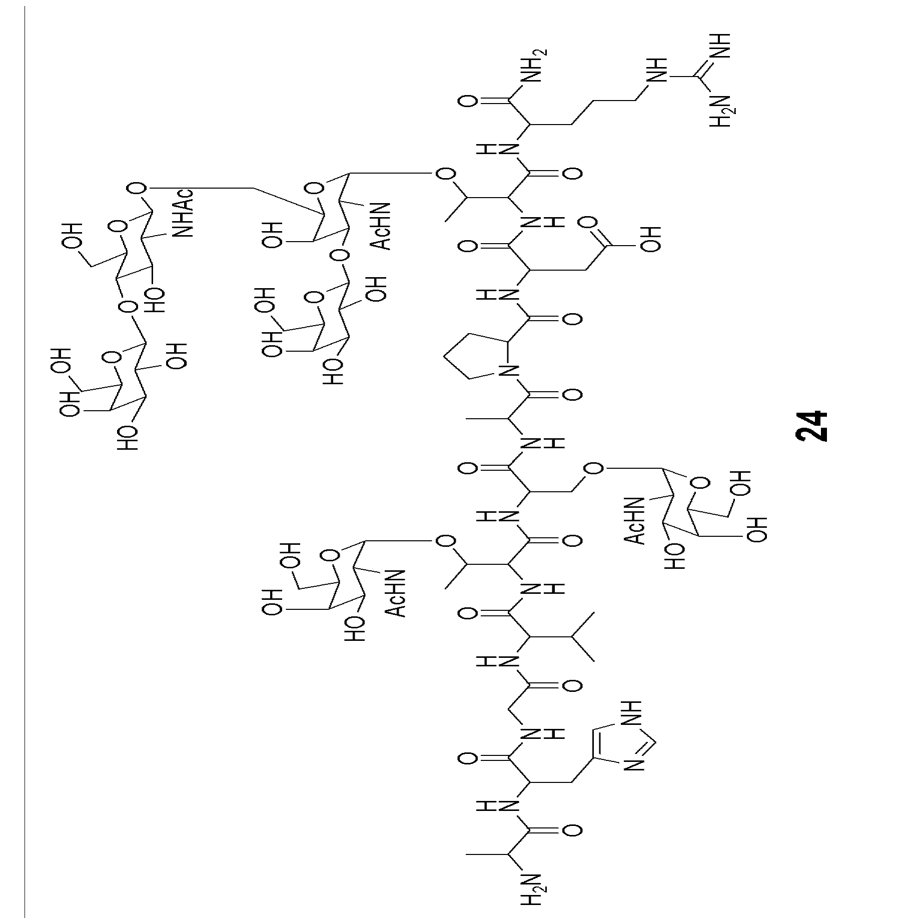

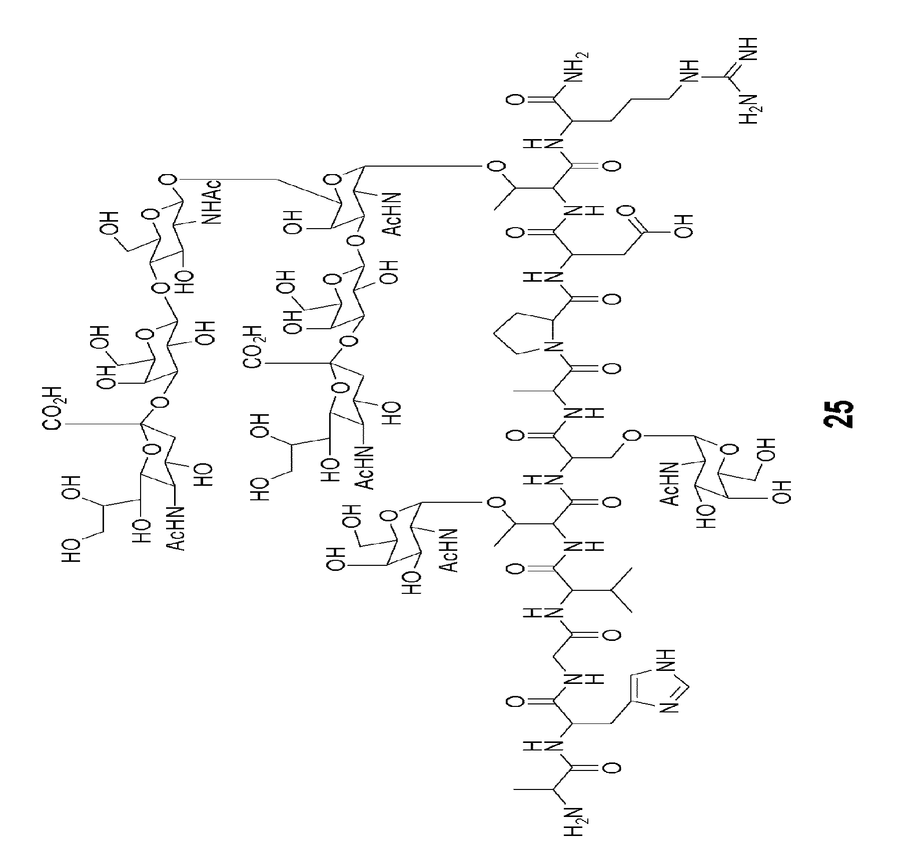

Using a distributing apparatus which is capable of controlling the temperature of a reaction part, a series of reactions using the following glycosyltransferases were performed in an automatized manner.

500µl of reaction solution containing 50mM HEPES buffer solution (pH 7.0), 0.20U/ml human-derived β1,4-galactosyltransferase (available from TOYOBO CO., LTD.), 10mM manganese chloride, 5mM uridine-5'-disodium diphosphogalactose (UDP-Gal) and 1mM glycopeptide derivative (18) was allowed to react for two hours at 25°C, thereby obtaining a reaction solution containing compound (22) [ratio of transfer: 95% or higher (HPLC)]. Identification of the transferred compound and the ratio of transfer was performed by sorting 20µl of the reaction solution after glycosyl transfer, adding 4µl of Milli Q water and 1µl of 1.74mg/ml solution of BLase (available from Shionogi & Co., Ltd.) thereto, and purifying the reaction solution by reverse phase HPLC (Inertsil®, ODS-3 4.6×250mm column, Mobile phase: A: 25mM ammonium acetate buffer solution (pH 6.5); B: acetonitrile, Gradient: 2% to 20%B with respect to A), thereby obtaining compound (24) [yield: 90% or higher (HPLC)]. MALDI-TOF/MS:[M(average)+H]⁺ of compound (24)=2248.1 (theoretical value: [M(average)+H]⁺ =2248.2) Meanwhile, 400µl of the reaction solution after galactose transfer reaction was sorted, and a mixture solution (total amount: 200µl) containing 20µl of 500mM HEPES buffer solution (pH 7.0), 60µl of 1% Triton X-100 aqueous solution, 12µl of 3.7U/ml recombinant rat α2,3-(N)-sialyltransferase (available from Calbiochem), 12µl of 0.88U/ml recombinant rat α2, 3-(O)-sialyltransferase (available from Calbiochem), 60µl of 50mM cytidine-5'-sodium monophosphosialate (CMP-NANA) and 36µl of Milli Q water was added thereto. The solution was allowed to react for four hours at 25°C, thereby obtaining a reaction solution containing compound (23) [ratio of transfer: 95% or higher (HPLC)]. Identification of the transferred compound and the ratio of transfer was performed by sorting 20µl of the reaction solution after glycosyl transfer, adding 4µl of Milli Q water and 1µl of 1.74mg/ml solution of BLase (available from Shionogi & Co., Ltd.) thereto, and purifying the reaction solution by reverse phase HPLC (Inertsil®, ODS-3 4.6x250mm column, Mobile phase: A: 25mM ammonium acetate buffer solution (pH 6.5); B: acetonitrile, Gradient: 2% to 20%B with respect to A), thereby obtaining compound (25) [yield: 90% or higher (HPLC)]. MALDI-TOF/MS:[M(average)+H]⁺ of compound (25)=2829.8 (theoretical value: [M(average)+H]⁺=2830.8)

(3.5 Synthesis of compounds (26) and (27))

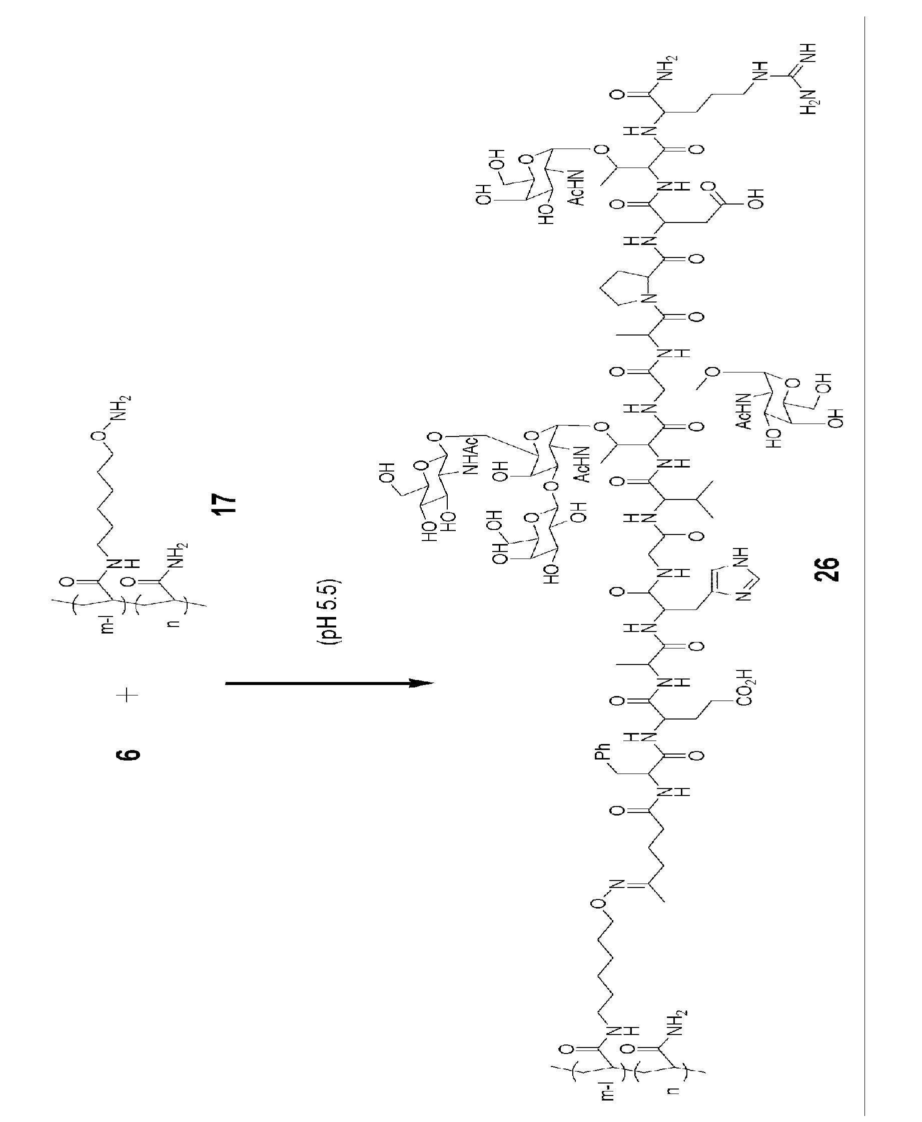

324µl of reaction solution containing 2.5mM glycopeptide derivative (6) and 5mM (oxyamine residue calculation) water-soluble polymer (17) was adjusted to have pH of 5.3 with 1N sodium hydroxide aqueous solution, and was stirred for five hours at room temperature. The reaction solution was purified by gel filtration [Biogel P-4: eluate: 25mM ammonium acetate buffer solution (pH 6.5), thereby obtaining 3.7mg of lyophilized compound (26) [ratio of trapping compound (25): 95% or higher (GPC-HPLC)].

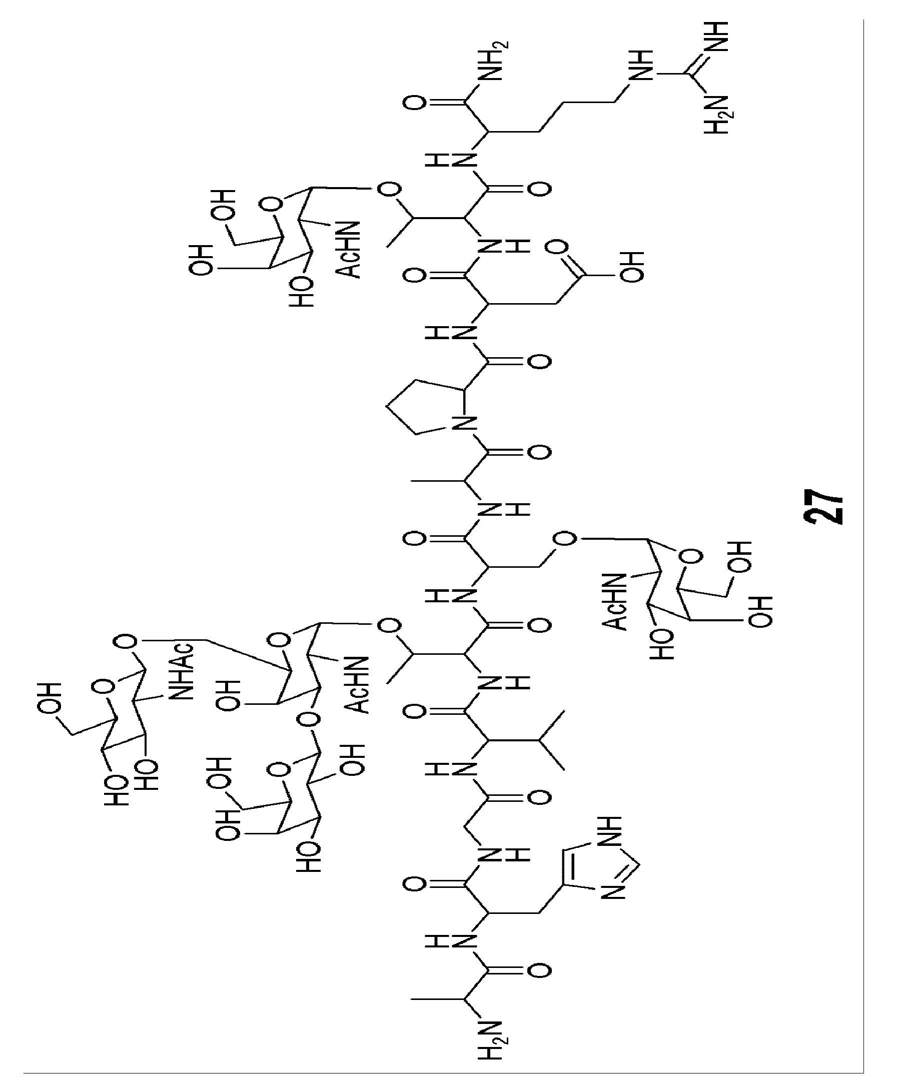

5µl of 5mM aqueous solution of glycopeptide derivative compound (26) was sorted, and 19µl of Milli Q water and 1µl of 1.74mg/ml solution of BLase (available from Shionogi & Co., Ltd.) was added thereto. The reaction solution was purified by reverse phase HPLC (Inertsil®, ODS-3 4.6x250mm column, Mobile phase: A: 25mM ammonium acetate buffer solution (pH 6.5); B: acetonitrile, Gradient: 2% to 20%B with respect to A), thereby obtaining compound (27) [yield: 90% or higher (HPLC)]. MALDI-TOF/MS: [M(average)+H]⁺ of compound (27)=2084.4 (theoretical value: [M(average)+H]⁺=2086.1)

(3.6 Synthesis of compounds (28) to (31))

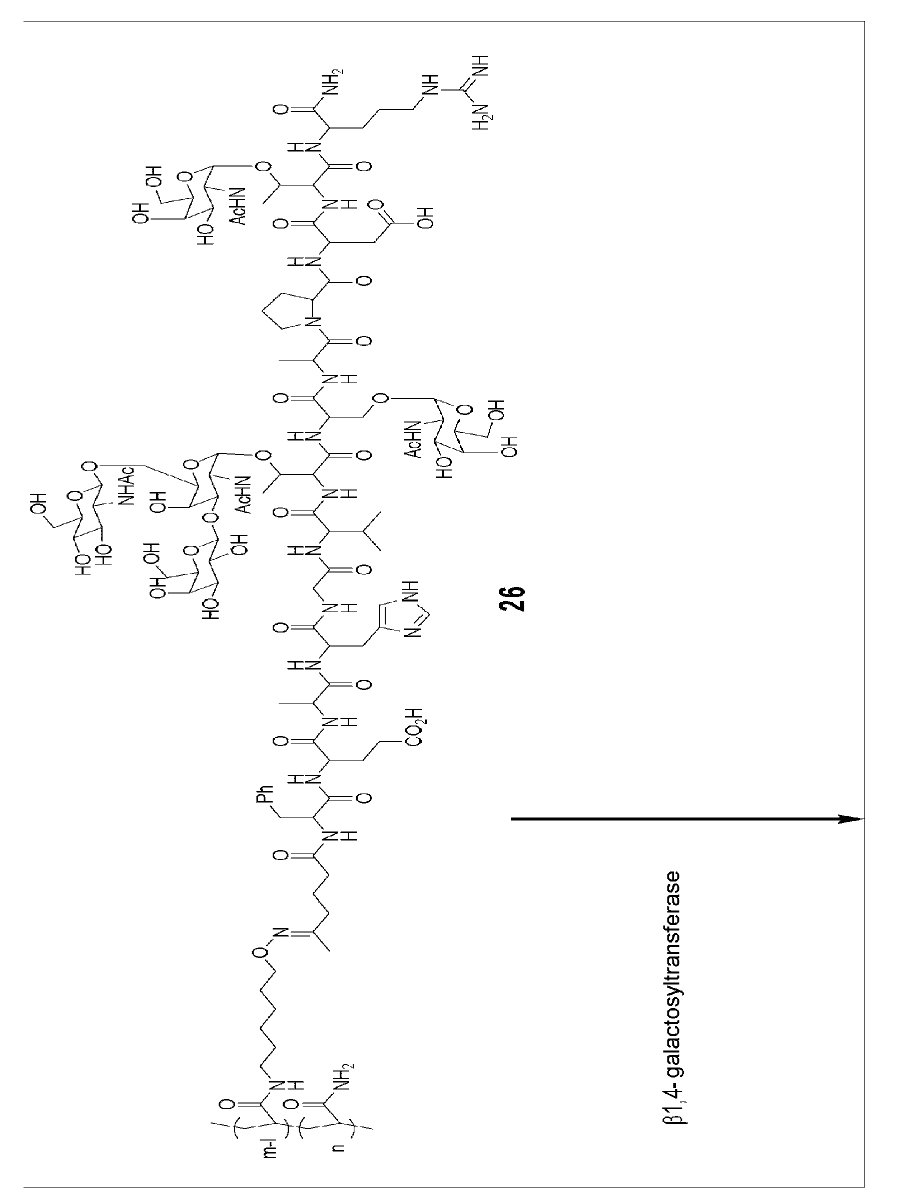

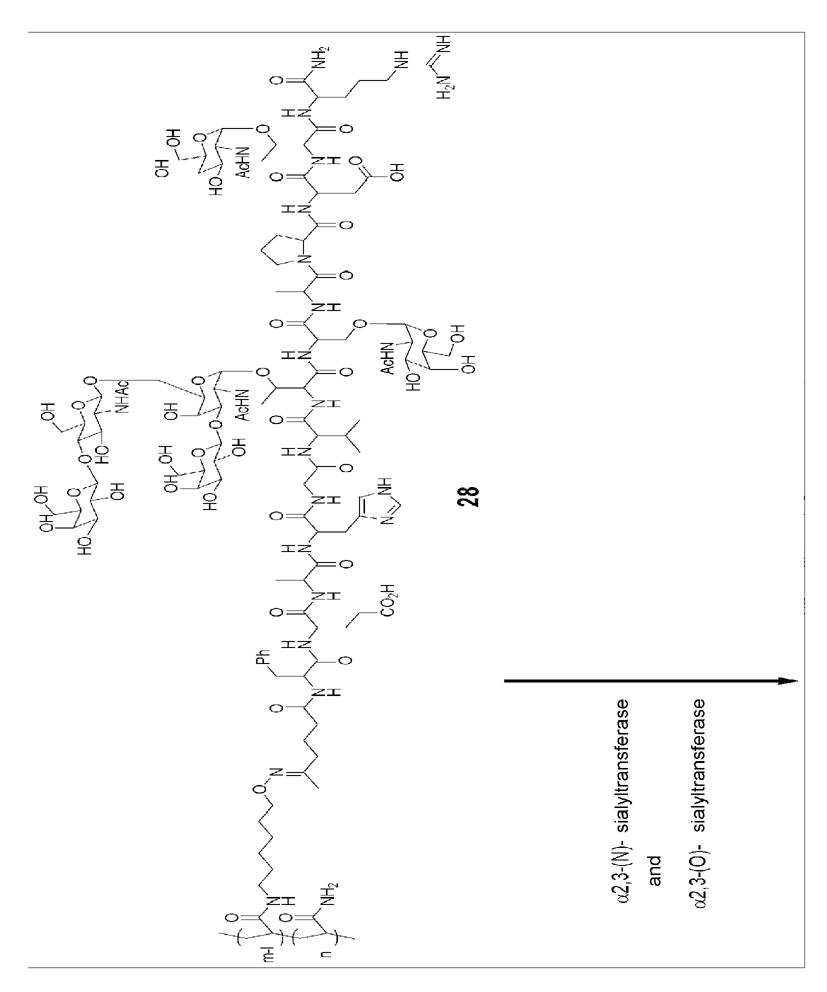

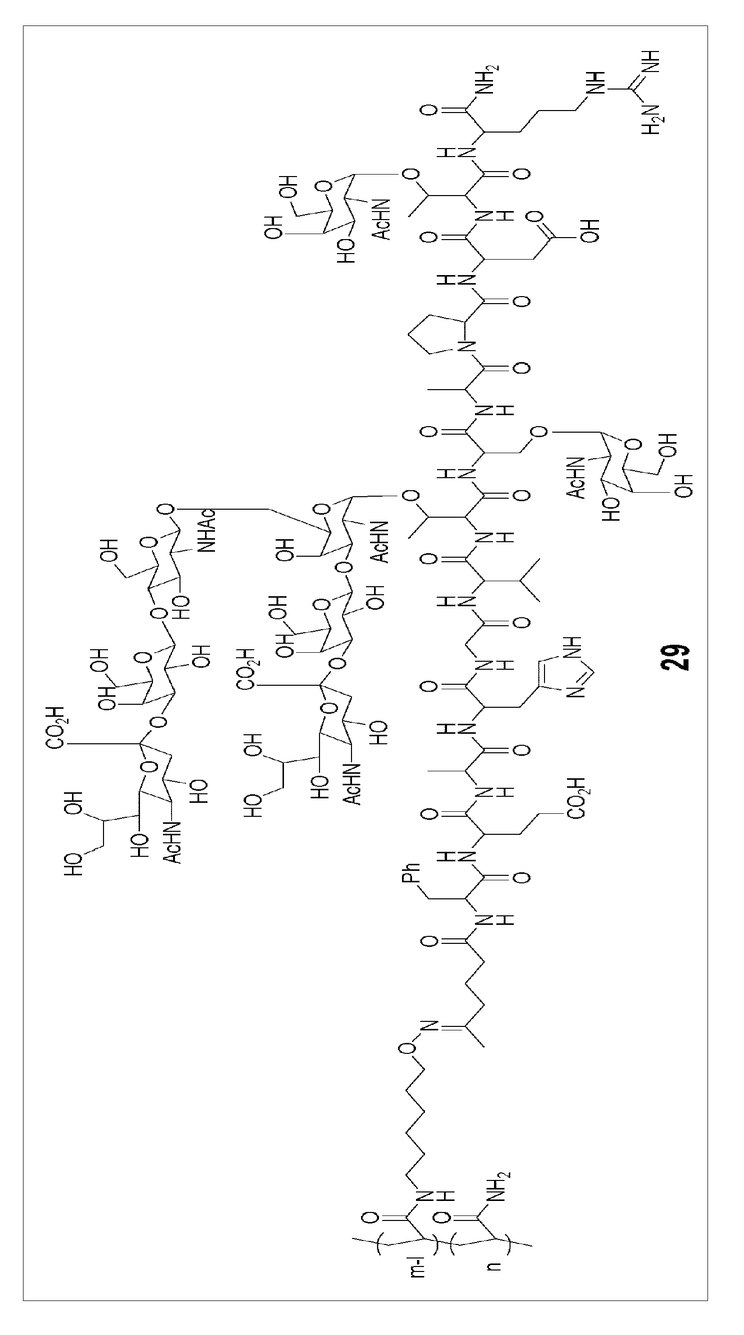

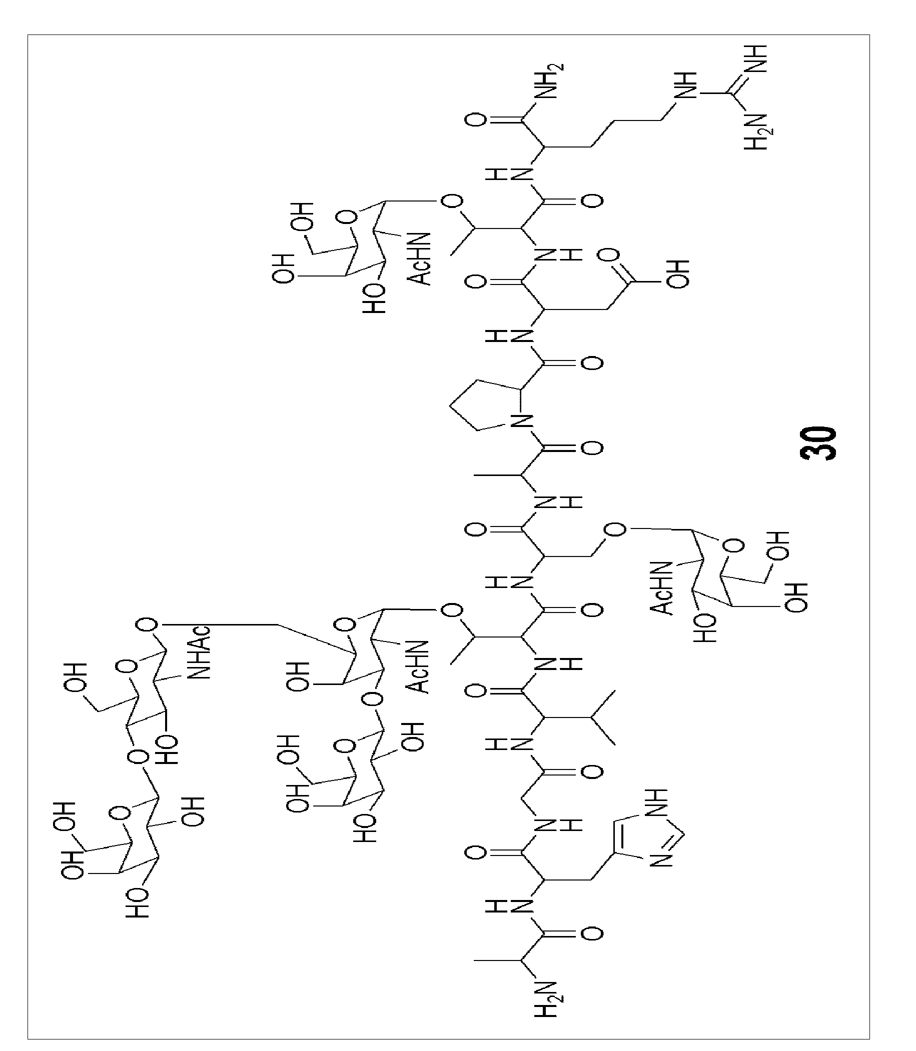

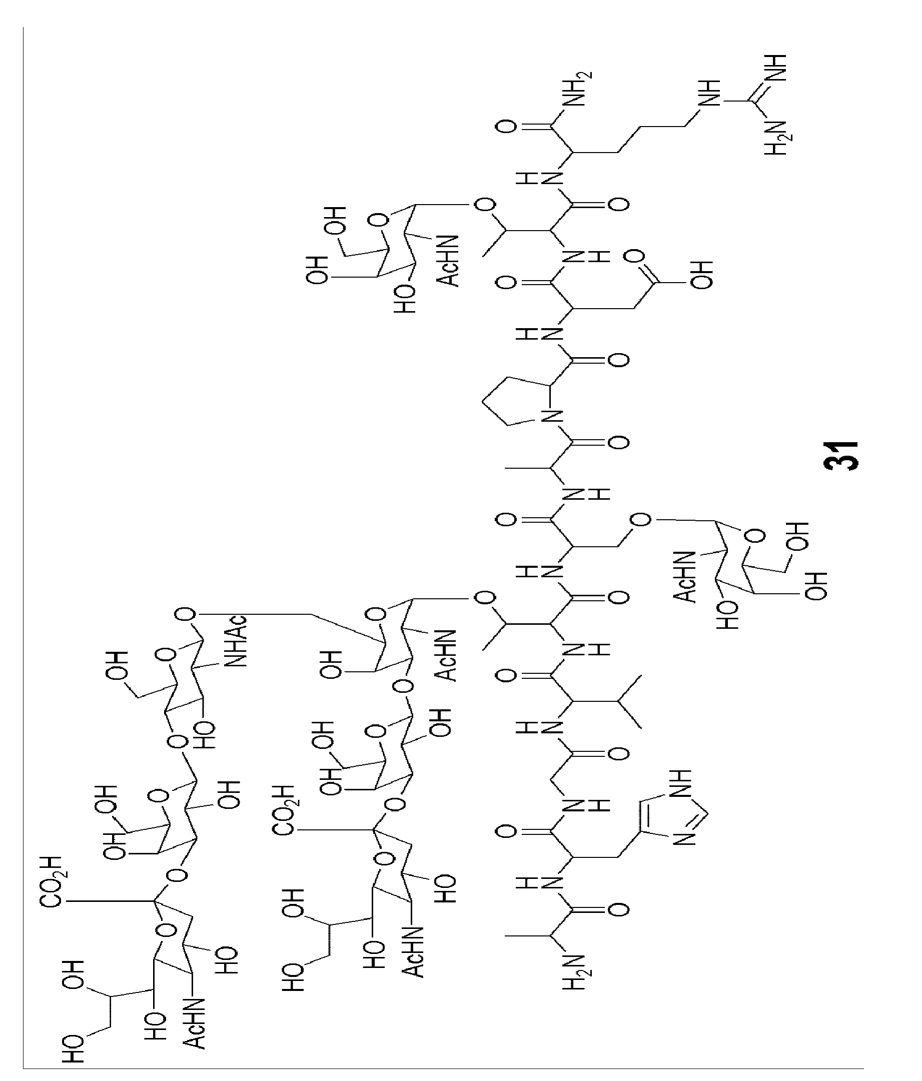

Using a distributing apparatus which is capable of controlling the temperature of a reaction part, a series of reactions using the following glycosyltransferases were performed in an automatized manner.

500µl of reaction solution containing 50mM HEPES buffer solution (pH 7.0), 0.20U/ml human-derived β1,4-galactosyltransferase (available from TOYOBO CO., LTD.), 10mM manganese chloride, 5mM uridine-5'-disodium diphosphogalactose (UDP-Gal) and 1mM glycopeptide derivative (18) was allowed to react for two hours at 25°C, thereby obtaining a reaction solution containing compound (28) [ratio of transfer: 95% or higher (HPLC)]. Identification of the transferred compound (28) and the ratio of transfer was performed by sorting 20µl of the reaction solution after glycosyl transfer, adding 4µl of Milli Q water and 1µl of 1.74mg/ml solution of BLase (available from Shionogi & Co., Ltd.) thereto, and purifying the reaction solution by reverse phase HPLC (Inertsil®, ODS-3 4.6×250mm column, Mobile phase: A: 25mM ammonium acetate buffer solution (pH 6.5); B: acetonitrile, Gradient: 2% to 20%B with respect to A), thereby obtaining compound (30) [yield: 90% or higher (HPLC)]. MALDI-TOF/MS:[M(average)+H]⁺ of compound (30)=2247.9 (theoretical value: [M(average)+H]⁺=2248.2) Meanwhile, 400µl of the reaction solution after galactose transfer reaction was sorted, and a mixture solution (total amount: 200µl) containing 20µl of 500mM HEPES buffer solution (pH 7.0), 60µl of 1% Triton X-100 aqueous solution, 12µl of 3.7U/ml recombinant rat α2,3-(N)-sialyltransferase (available from Calbiochem), 12µl of 0.88U/ml recombinant rat α2, 3- (O) -sialyltransferase (available fromCalbiochem), 60µl of 50mM cytidine-5'-sodium monophosphosialate (CMP-NANA) and 36µl of Milli Q water was added thereto. The solution was allowed to react for four hours at 25°C, thereby obtaining a reaction solution containing compound (29) [ratio of transfer: 95% or higher (HPLC)]. Identification of the transferred compound (29) and the ratio of transfer was performed by sorting 20µl of the reaction solution after glycosyl transfer, adding 4µl of Milli Q water and 1µl of 1.74mg/ml solution of BLase (available from Shionogi & Co., Ltd.) thereto, and purifying the reaction solution by reverse phase HPLC (Inertsil®, ODS-3 4.6×250mm column, Mobile phase: A: 25mM ammonium acetate buffer solution (pH 6.5); B: acetonitrile, Gradient: 2% to 20%B with respect to A), thereby obtaining compound (31) [yield: 90% or higher (HPLC)]. MALDI-TOF/MS:[M(average)+H]⁺ of compound (31)=2827.2 (theoretical value: [M(average)+H]⁺=2830.8)

(3.7 Synthesis of compounds (32) and (33))

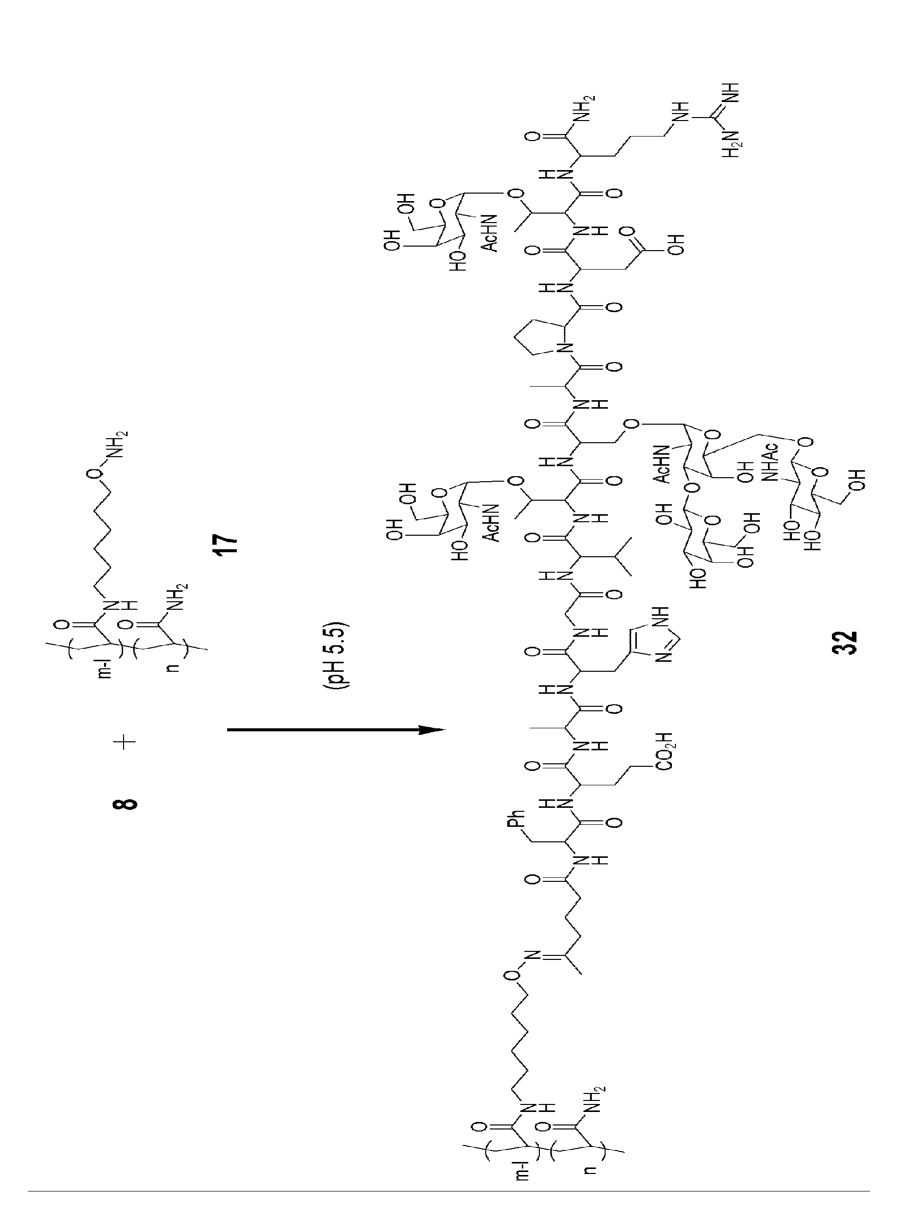

324µl of reaction solution containing 2.5mM glycopeptide derivative (8) and 6.7mM (oxyamine residue calculation) water-soluble polymer (17) was adjusted to have pH of 5.0 with 1N sodium hydroxide aqueous solution, and was stirred for six hours at room temperature. The reaction solution was purified by gel filtration [Biogel P-4: eluate: 25mM ammonium acetate buffer solution (pH 6.5), thereby obtaining 5.6mg of lyophilized compound (32) [ratio of trapping compound (8): 95% or higher (GPC-HPLC)].

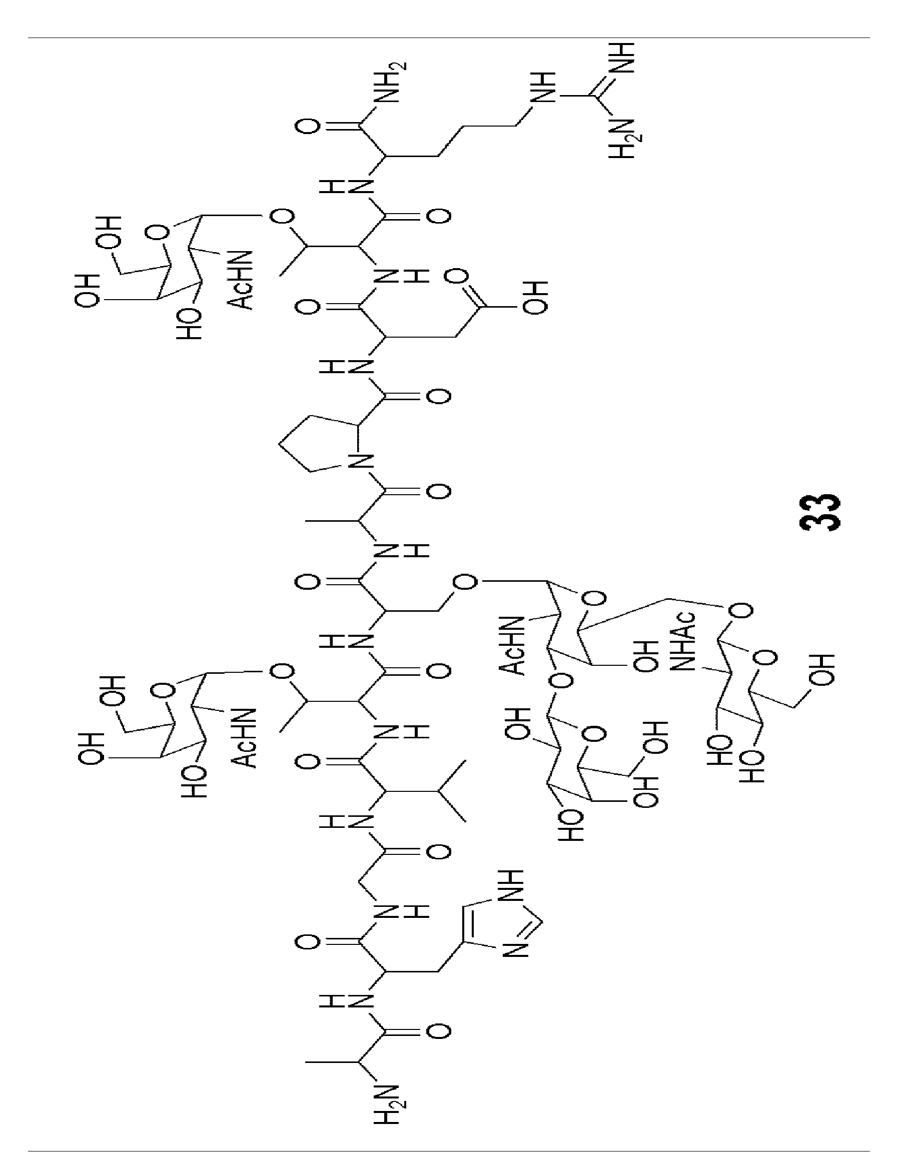

5µl of 5mM aqueous solution of glycopeptide derivative compound (32) was sorted, and 19µl of Milli Q water and 1µl of 1.74mg/ml solution of BLase (available from Shionogi & Co., Ltd.) was added thereto. The reaction solution was purified by reverse phase HPLC (Inertsil®, ODS-3 4.6×250mm column, Mobile phase: A: 25mM ammonium acetate buffer solution (pH 6.5); B: acetonitrile, Gradient: 2% to 20%B with respect to A), thereby obtaining compound (33) [yield: 90% or higher (HPLC)]. MALDI-TOF/MS: [M(average)+H]⁺ of compound (33) =2085. 3 (theoretical value: [M(average)+H]⁺=2086.1)

(3.8 Synthesis of compounds (34) to (41))

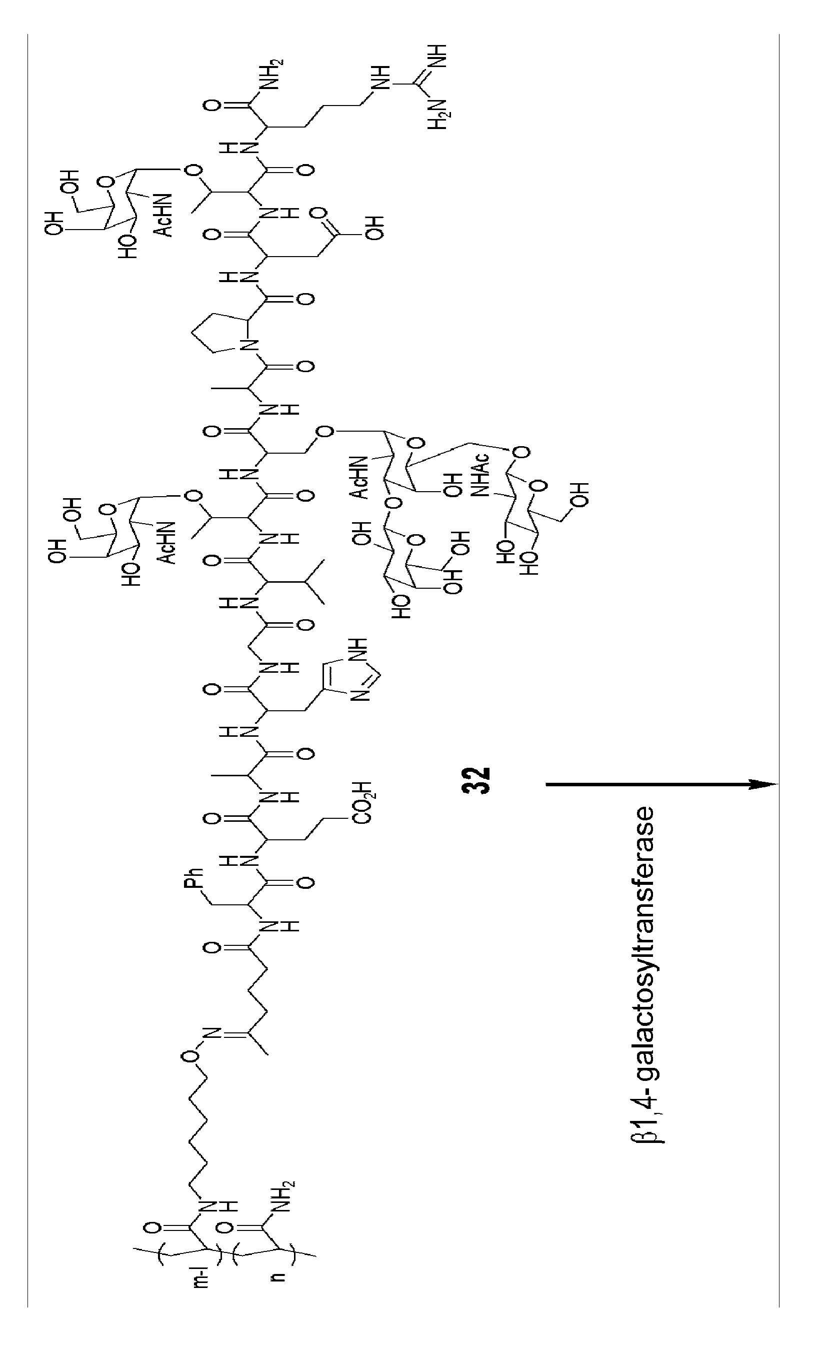

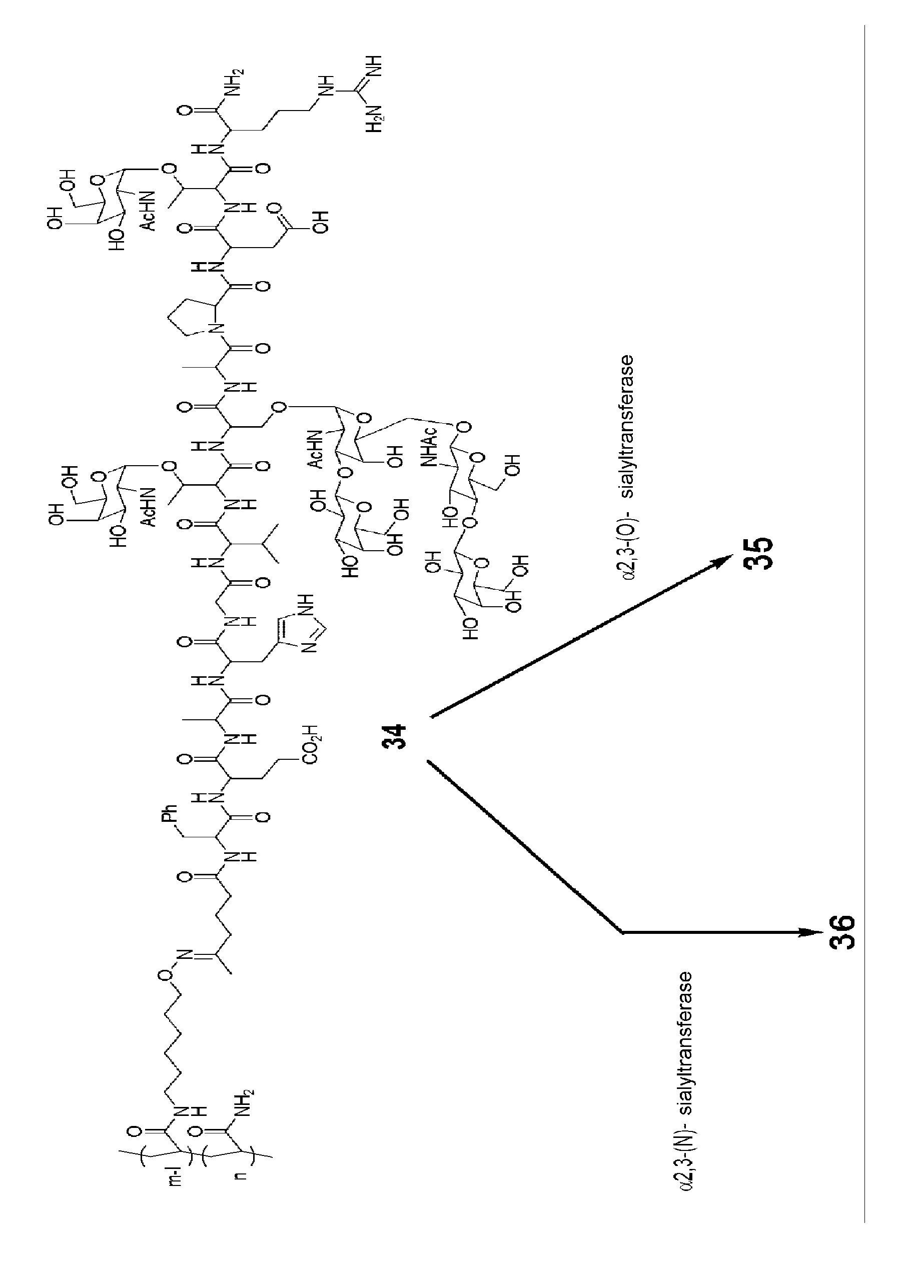

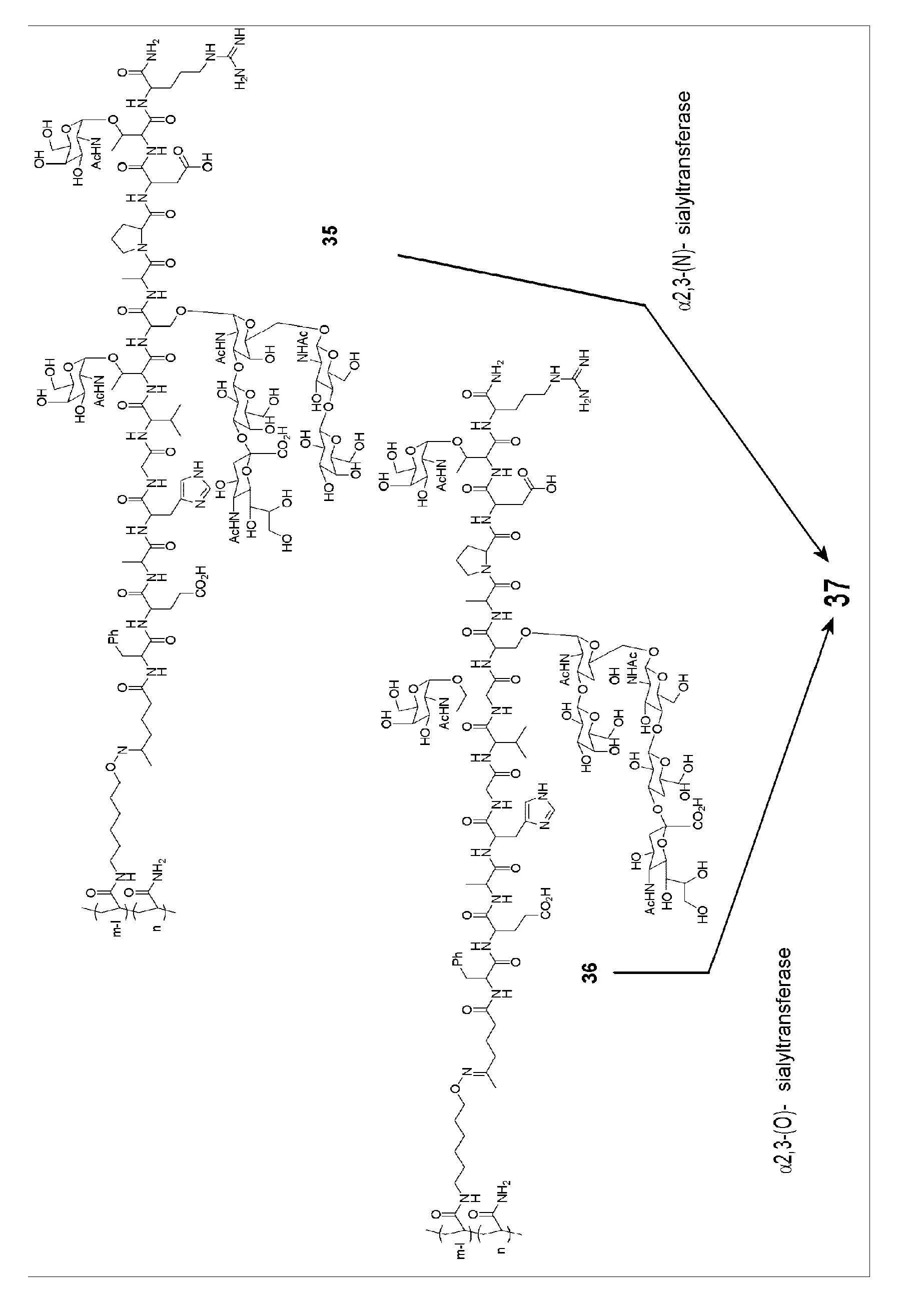

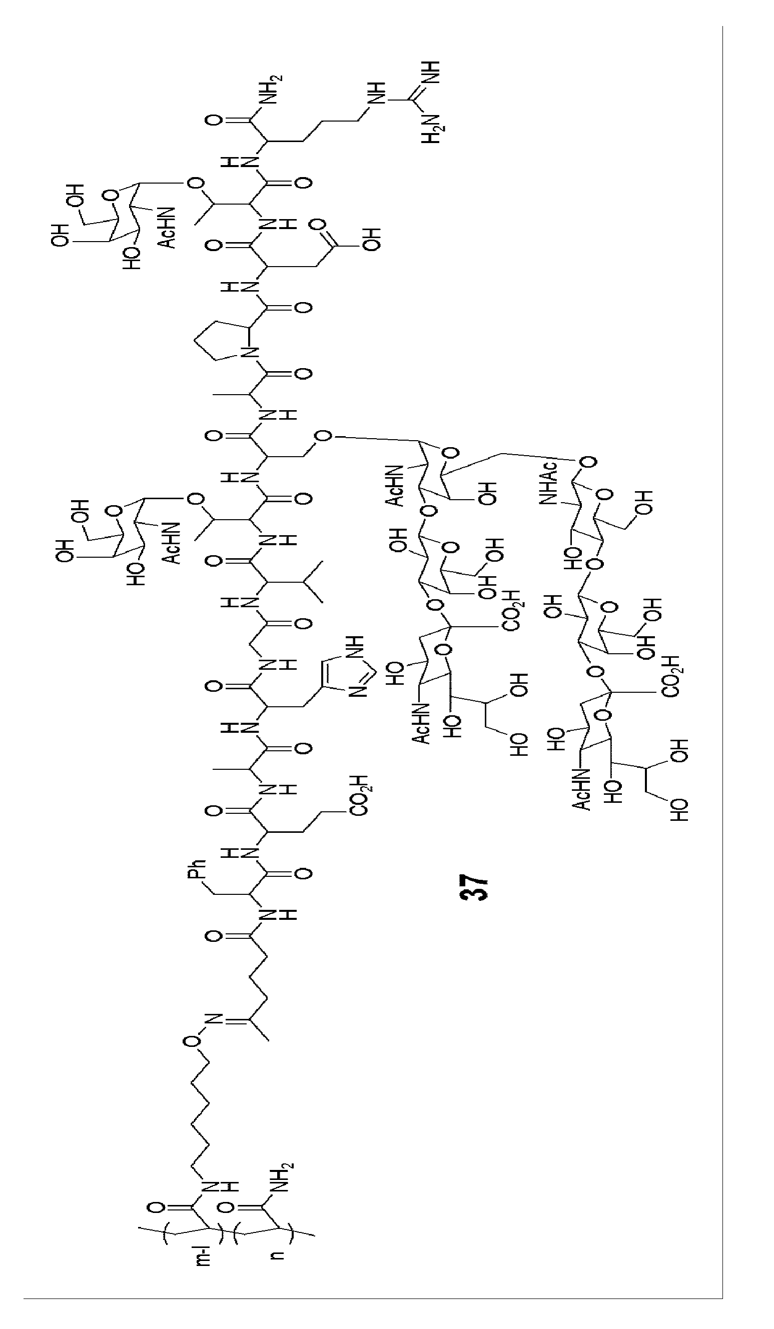

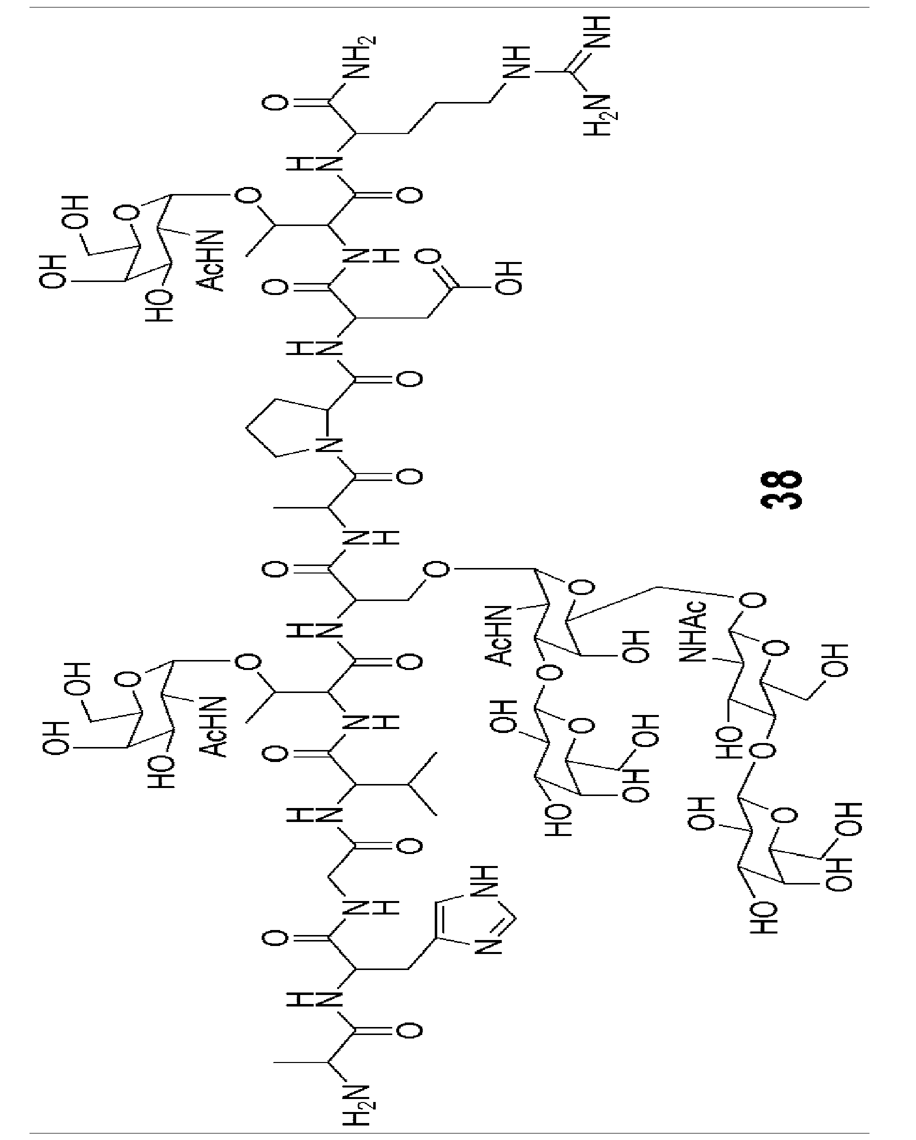

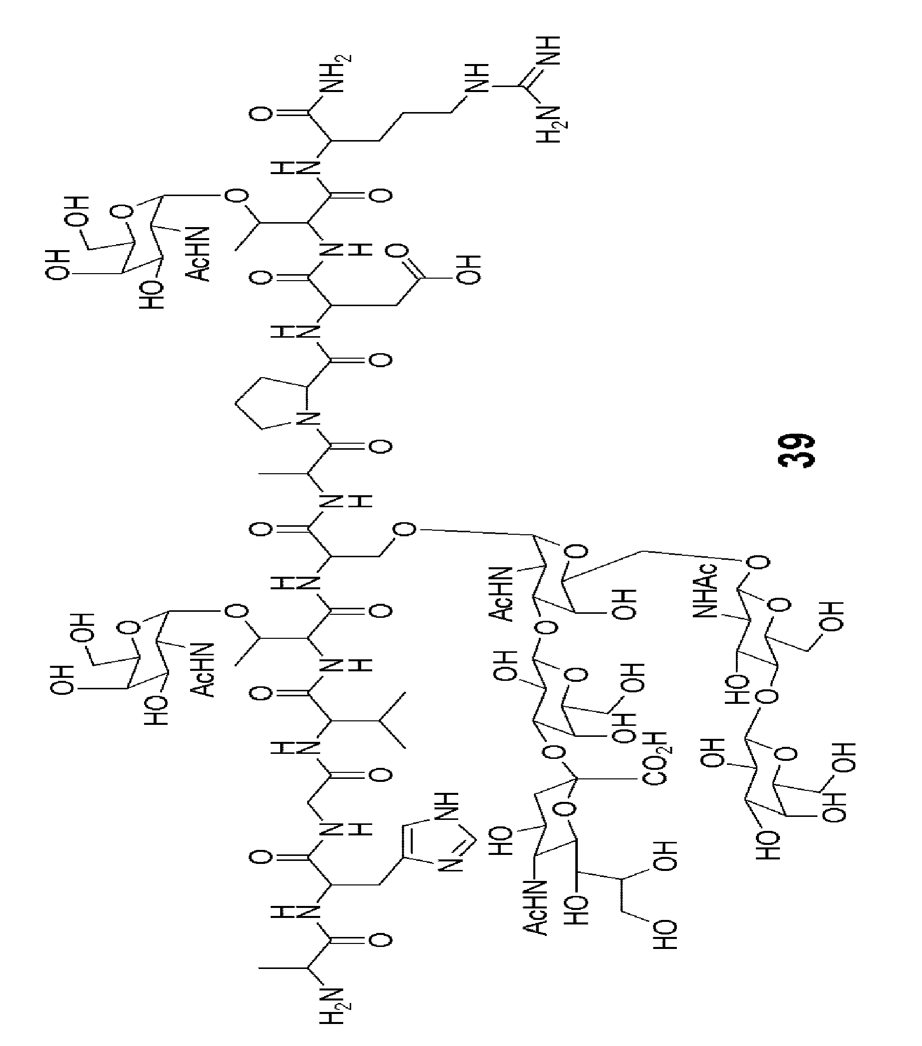

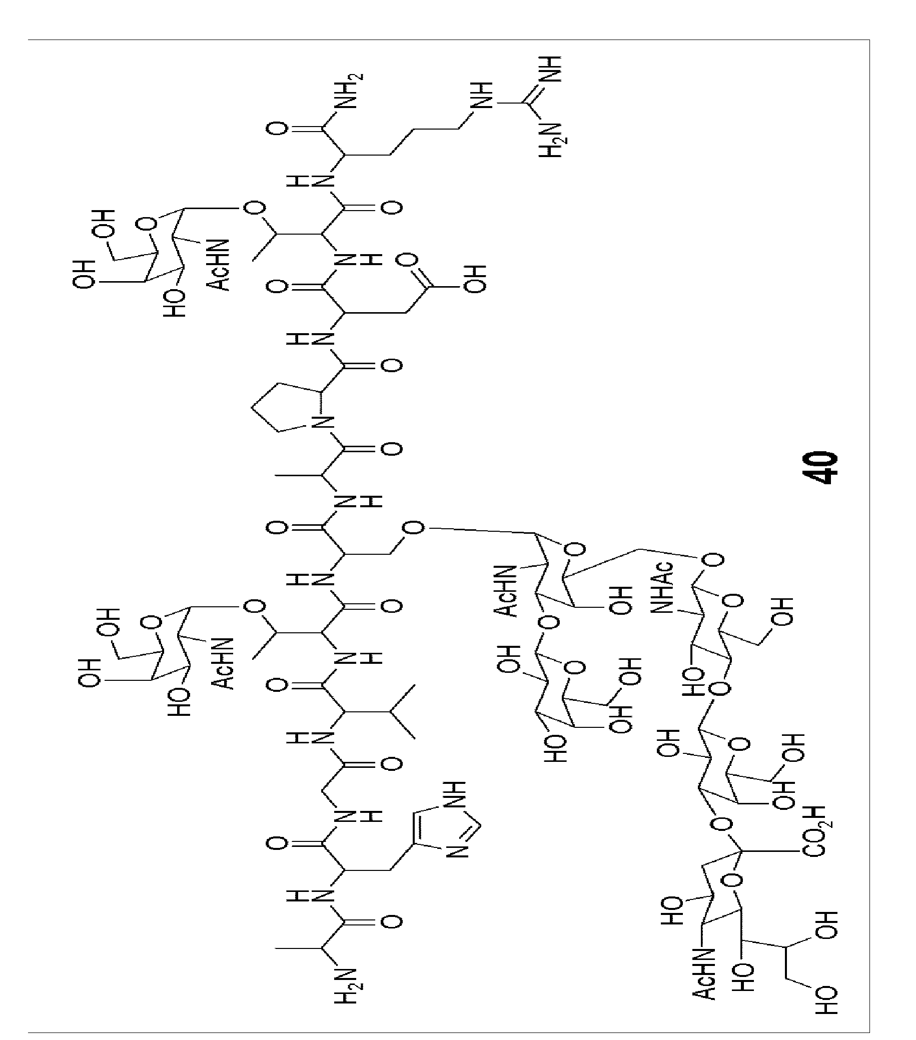

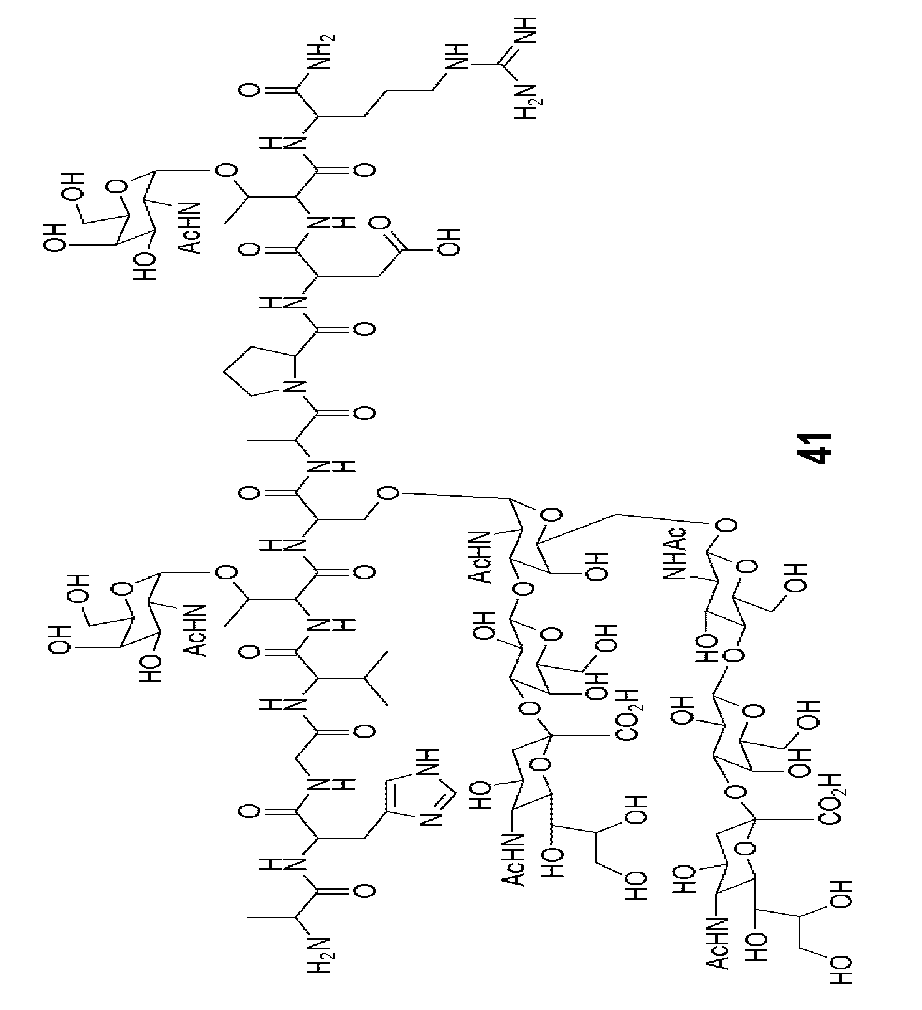

Using a distributing apparatus which is capable of controlling the temperature of a reaction part, a series of reactions using the following glycosyltransferases were performed in an automatized manner.

700µl of reaction solution containing 50mM HEPES buffer solution (pH 7.0), 0.20U/ml human-derived β1,4-galactosyltransferase (available from TOYOBO CO., LTD.), 10mM manganese chloride, 5mM uridine-5'-disodium diphosphogalactose (UDP-Gal) and 1mM glycopeptide derivative (32) was allowed to react for two hours at 25°C. Identification of the transferred compound (34) and the ratio of transfer was performed by sorting 20µl of the reaction solution after glycosyl transfer, adding 4µl of Milli Q water and 1µl of 1.74mg/ml solution of BLase (available from Shionogi & Co., Ltd.) thereto, and purifying the reaction solution by reverse phase HPLC (Inertsil®, ODS-3 4.6×250mm column, Mobile phase: A: 25mM ammonium acetate buffer solution (pH 6.5); B: acetonitrile, Gradient: 2% to 15%B with respect to A), thereby obtaining compound (38) [yield: 90% or higher (HPLC), ratio of transfer: 95% or higher (HPLC)]. MALDI-TOF/MS:[M(average)+H]⁺ of compound (38)=2246.8 (theoretical value: [M(average)+H]⁺=2248.2) Meanwhile, 300µl of the reaction solution after galactose transfer reaction was sorted, and a mixture solution (total amount: 150µl) containing 15µl of 500mM HEPES buffer solution (pH 7.0), 45µl of 1% TritonX-100 aqueous solution, 9µl of 0.88U/ml recombinant rat α2, 3- (O) -sialyltransferase (available from Calbiochem), 45µl of 50mM cytidine-5'-sodium monophosphosialate (CMP-NANA) and 36µl of Milli Q water was added thereto. The solution was allowed to react for four hours at 25°C. Identification of the transferred compound (35) and the ratio of transfer was performed by sorting 20µl of the reaction solution after glycosyl transfer, adding 4µl of Milli Q water and 1µl of 1.74mg/ml solution of BLase (available from Shionogi & Co. , Ltd.) thereto, and purifying the reaction solution by reverse phase HPLC (Inertsil®, ODS-3 4.6×250mm column, Mobile phase: A: 25mM ammonium acetate buffer solution (pH 6.5); B: acetonitrile, Gradient: 2% to 15%B with respect to A), thereby obtaining compound (39) [yield: 90% or higher (HPLC), ratio of transfer: 85% or higher (HPLC)]. Further, approximately 10% of compound (38) derived from unreacted compound (34) was obtained. MALDI-TOF/MS:[M(average)+H]⁺ of compound (39)=2537.4 (theoretical value: [M(average)+H]⁺=2539.5) Subsequently, 200µl of the reaction solution after α2,3-(O)-sialyltransferase reaction was sorted, and a mixture solution (total amount: 100µl) containing 10µl of 500mM HEPES buffer solution (pH 7.0), 10µl of 1% Triton X-100 aqueous solution, 6µl of 3.7U/ml recombinant rat α2,3-(N)-sialyltransferase (available from Calbiochem), 30µl of 50mM cytidine-5'-sodium monophosphosialate (CMP-NANA) and 44µl of Milli Q water was added thereto. The solution was allowed to react for six hours at 25°C. Identification of the transferred compound (37) and the ratio of transfer was performed by sorting 20µl of the reaction solution after glycosyl transfer, adding 4µl of Milli Q water and 1µl of 1.74mg/ml solution of BLase (available from Shionogi & Co., Ltd.) thereto, and purifying the reaction solution by reverse phase HPLC (Inertsil®, ODS-3 4.6×250mm column, Mobile phase: A: 25mM ammonium acetate buffer solution (pH 6.5); B: acetonitrile, Gradient: 2% to 15%B with respect to A), thereby obtaining compound (41) [yield: 90% or higher (HPLC), ratio of transfer: 95% or higher (HPLC)]. MALDI-TOF/MS:[M(average)+H]⁺ of compound (41)=2828.9 (theoretical value: [M(average)+H]⁺=2830.8)

Meanwhile, 300µl of the reaction solution after galactose transfer reaction was sorted, and a mixture solution (total amount: 150µl) containing 15µl of 500mM HEPES buffer solution (pH 7.0), 45µl of 1% Triton X-100 aqueous solution, 9µl of 3.7U/ml recombinant rat α2,3-(N)-sialyltransferase (available from Calbiochem), 45µl of 50mM cytidine-5'-sodium monophosphosialate (CMP-NANA) and 36µl of Milli Q water was added thereto. The solution was allowed to react for four hours at 25°C. Identification of the transferred compound (36) and the ratio of transfer was performed by sorting 20µl of the reaction solution after glycosyl transfer, adding 4µl of Milli Q water and 1µl of 1.74mg/ml solution of BLase (available from Shionogi & Co., Ltd.) thereto, and purifying the reaction solution by reverse phase HPLC (Inertsil®, ODS-3 4.6×250mm column, Mobile phase: A: 25mM ammonium acetate buffer solution (pH 6.5); B: acetonitrile, Gradient: 2% to 15%B with respect to A), thereby obtaining compound (40) [yield: 90% or higher (HPLC), ratio of transfer: 85% or higher (HPLC)]. Since approximately 10% of compound (41) was obtained, it was confirmed that monosialylated compound (36) and approximately 10% of disialylated compound (41) were produced at the same time in sialic acid transfer reaction. MALDI-TOF/MS:[M(average)+H]⁺ of compound (40)=2538.2 (theoretical value: [M(average)+H]⁺=2539.5) Subsequently, 200µl of the reaction solution after α2,3-(N)-sialyltransferase reaction was sorted, and a mixture solution (total amount: 100µl) containing 10µl of 500mM HEPES buffer solution (pH 7.0), 10µl of 1% Triton X-100 aqueous solution, 6µl of 0.88U/ml recombinant rat α2,3-(O)-sialyltransferase (available from Calbiochem), 30µl of 50mM cytidine-5'-sodium monophosphosialate (CMP-NANA) and 44µl of Milli Q water was added thereto. The solution was stirred for six hours at 25°C. Identification of the transferred compound (37) and the ratio of transfer was performed by confirmation of BLase-treated compound (41) in accordance with a method described in a summarized manner [yield: 90% or higher (HPLC), ratio of transfer: 95% or higher (HPLC)]. MALDI-TOF/MS:[M(average)+H]⁺ of compound (41)=2828.9 (theoretical value: [M(average)+H]⁺=2830.8)

### (3.9 Synthesis of compounds (42) and (43))

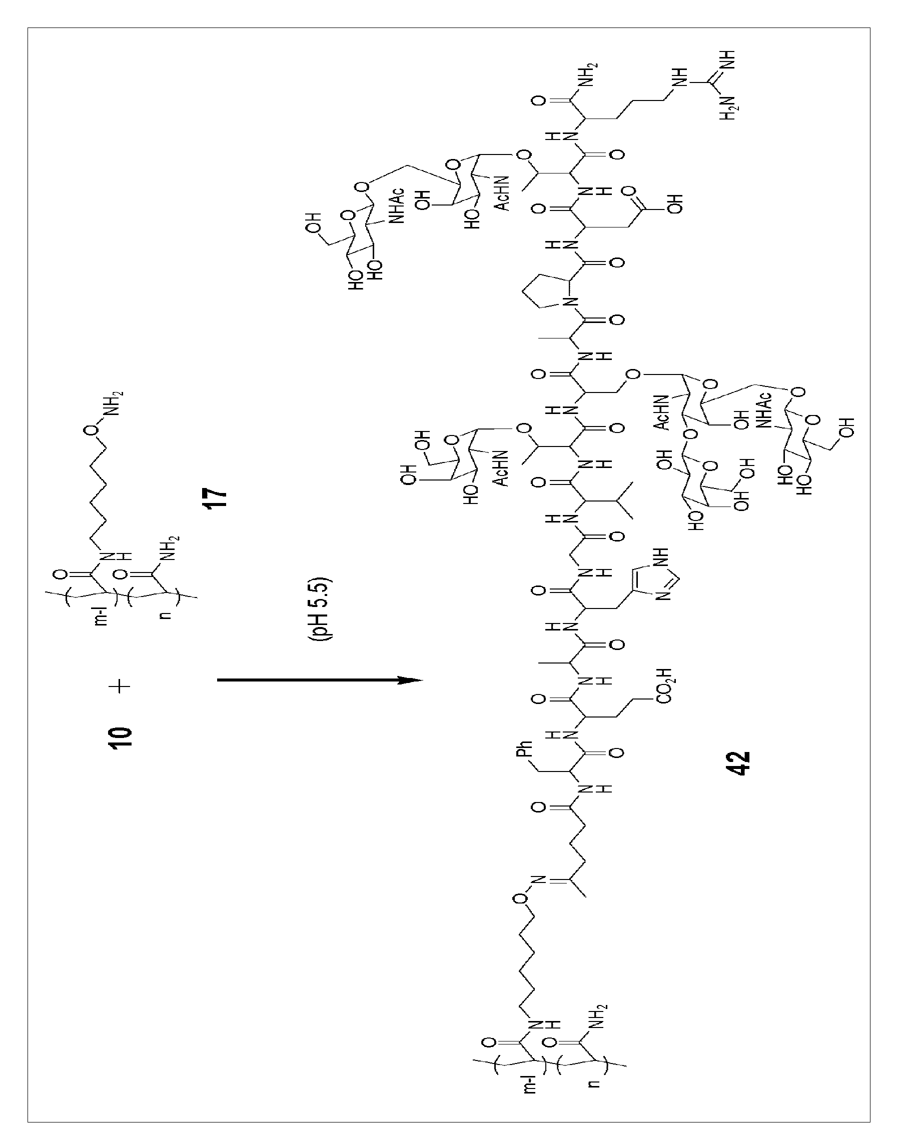

360µl of reaction solution containing 3.3mM glycopeptide derivative (10) and 6.7mM (oxyamine residue calculation) water-soluble polymer (17) was adjusted to have pH of 5.3 with 1N sodium hydroxide, and was stirred for six hours at room temperature. The reaction solution was purified by gel filtration [Biogel P-4: eluate: 25mM ammonium acetate buffer solution (pH 6.5), thereby obtaining 7.0mg of lyophilized compound (42) [ratio of trapping compound (10): 95% or higher (GPC-HPLC)].

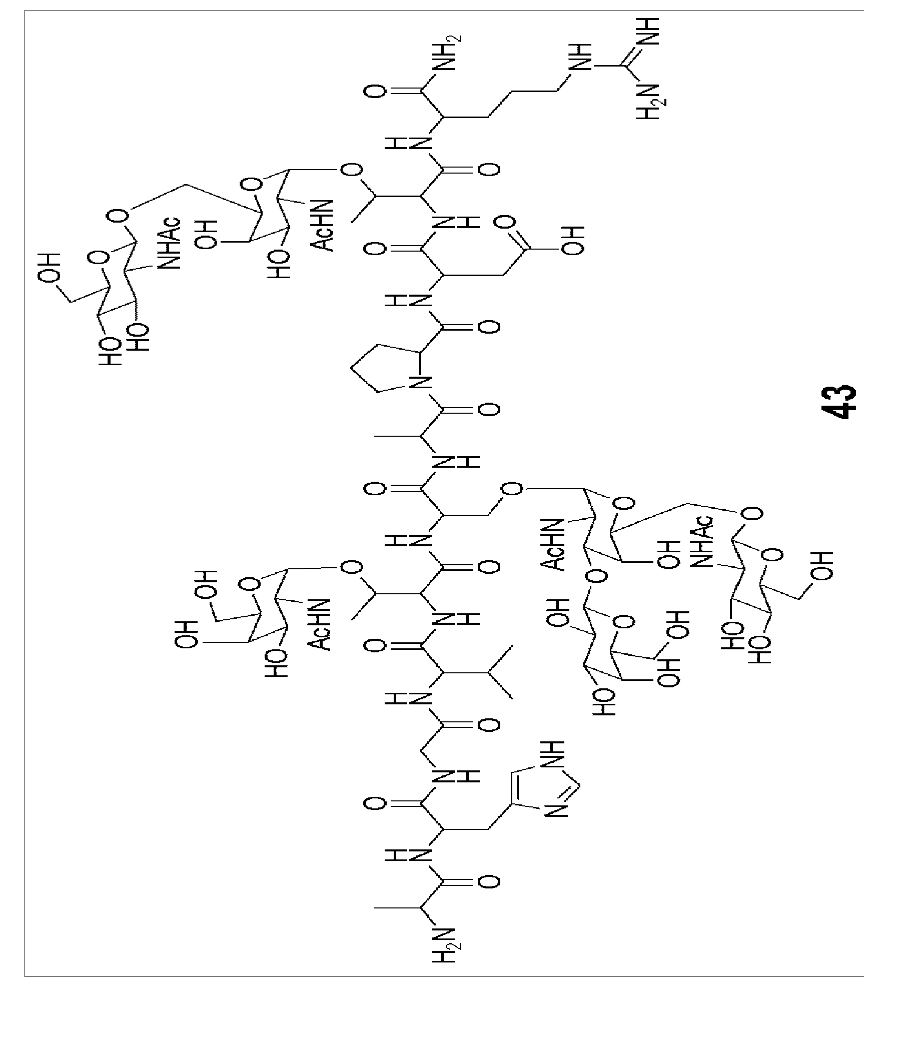

BLase reaction to compound (42) was performed in the same manner as in Example (3.7), thereby obtaining compound (43) [ratio of reaction: 90% or higher (GPC-HPLC)]. Identification of the transferred compound was performed by confirming [M(average)+H]⁺=2287.8 (theoretical value: [M(average)+H]⁺=2289.3) derived from compound (43) by MALDI-TOF/MS.

(3.10 Synthesis of compounds (44) to (51))

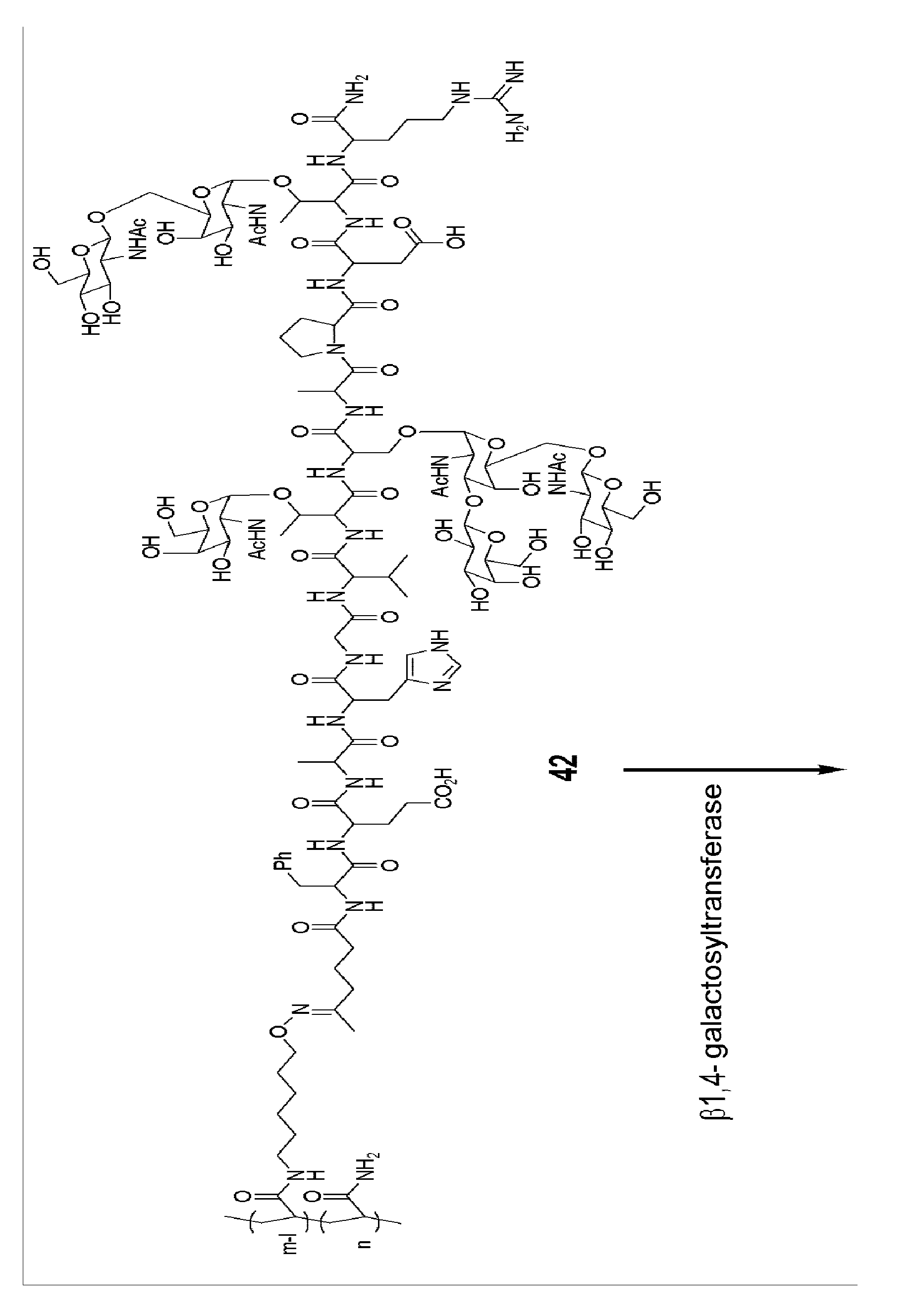

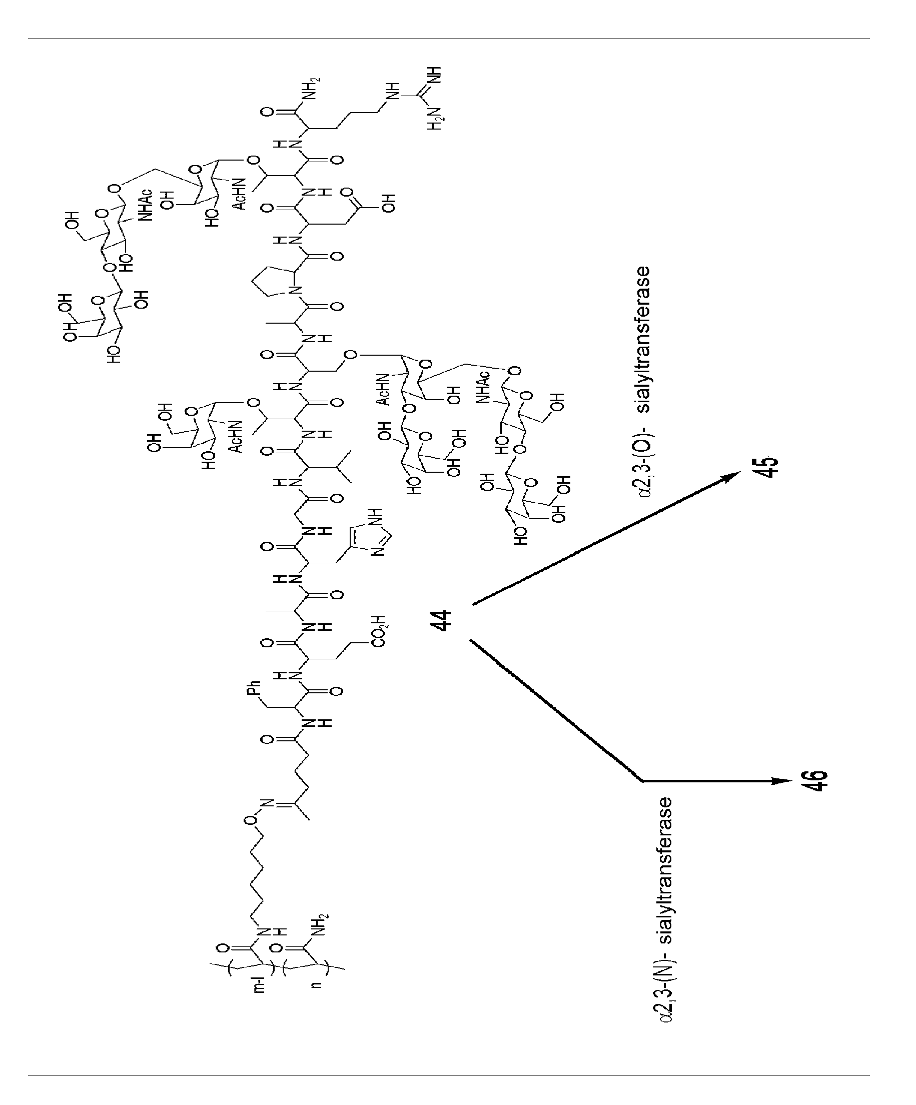

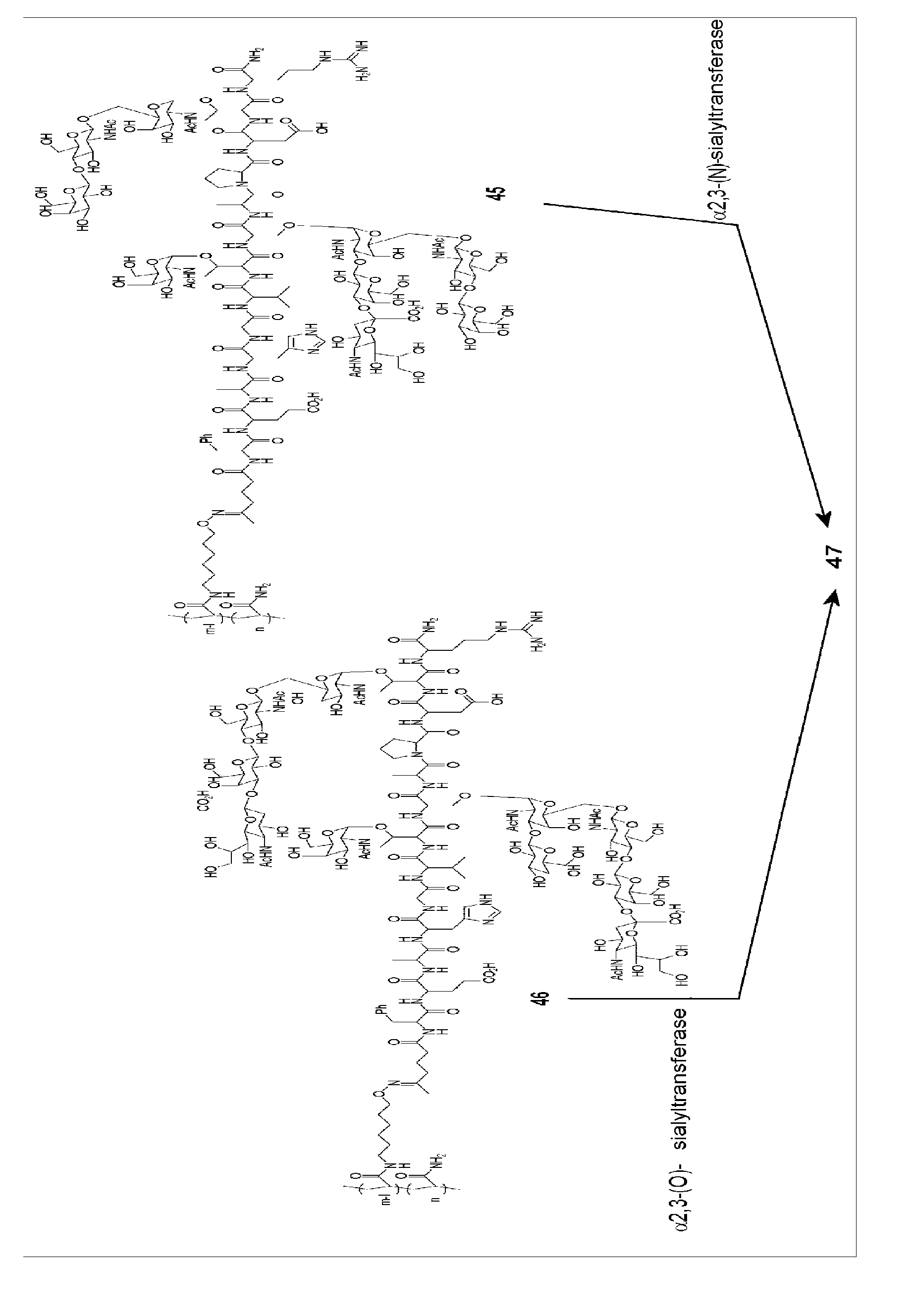

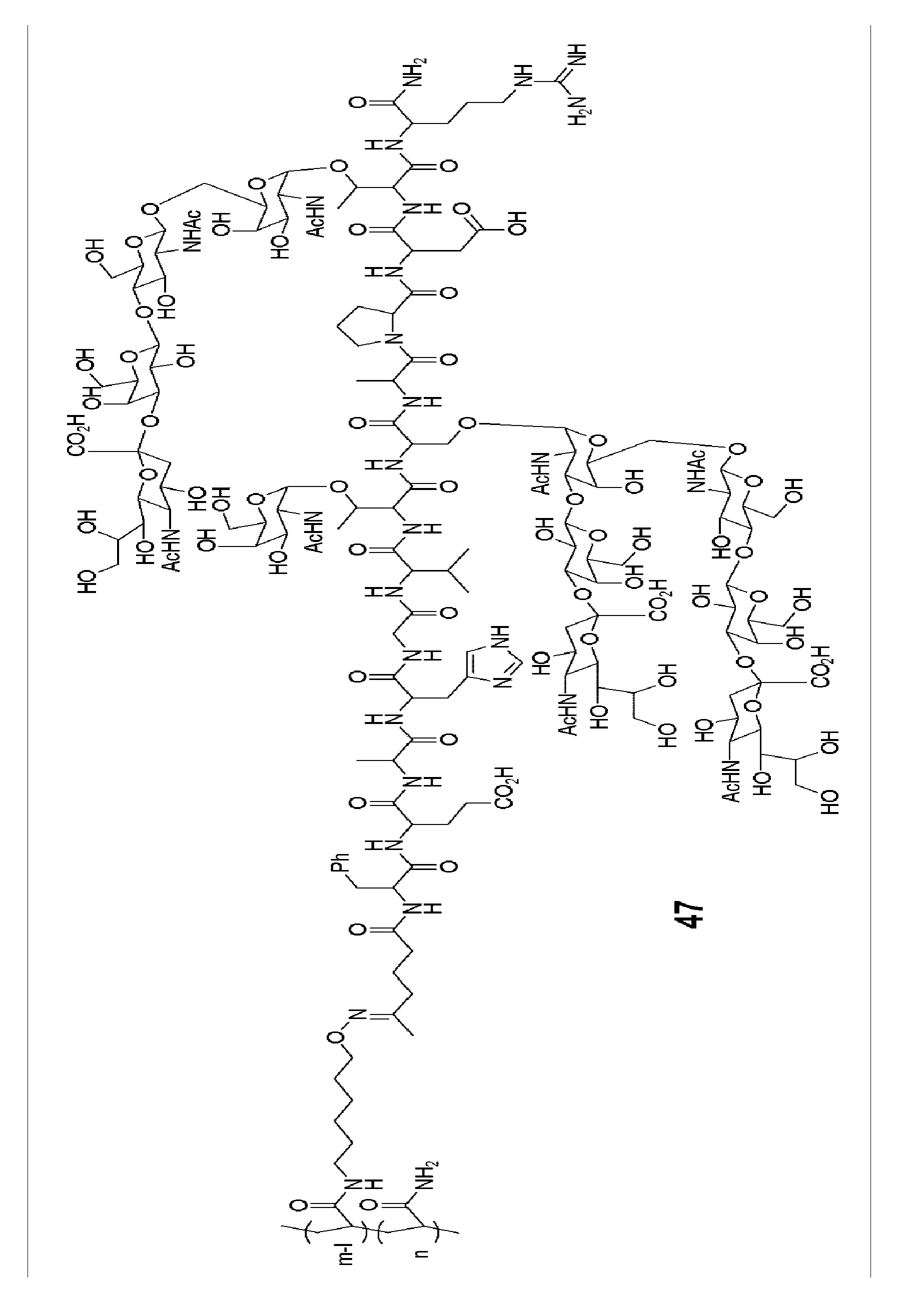

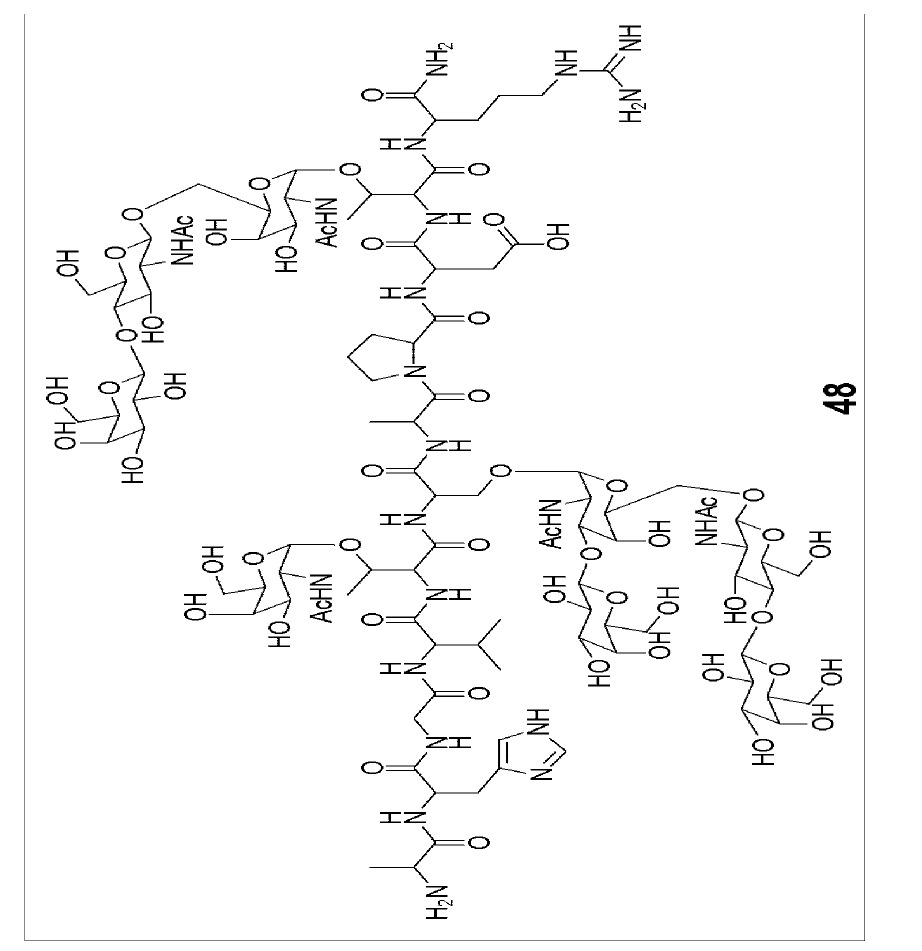

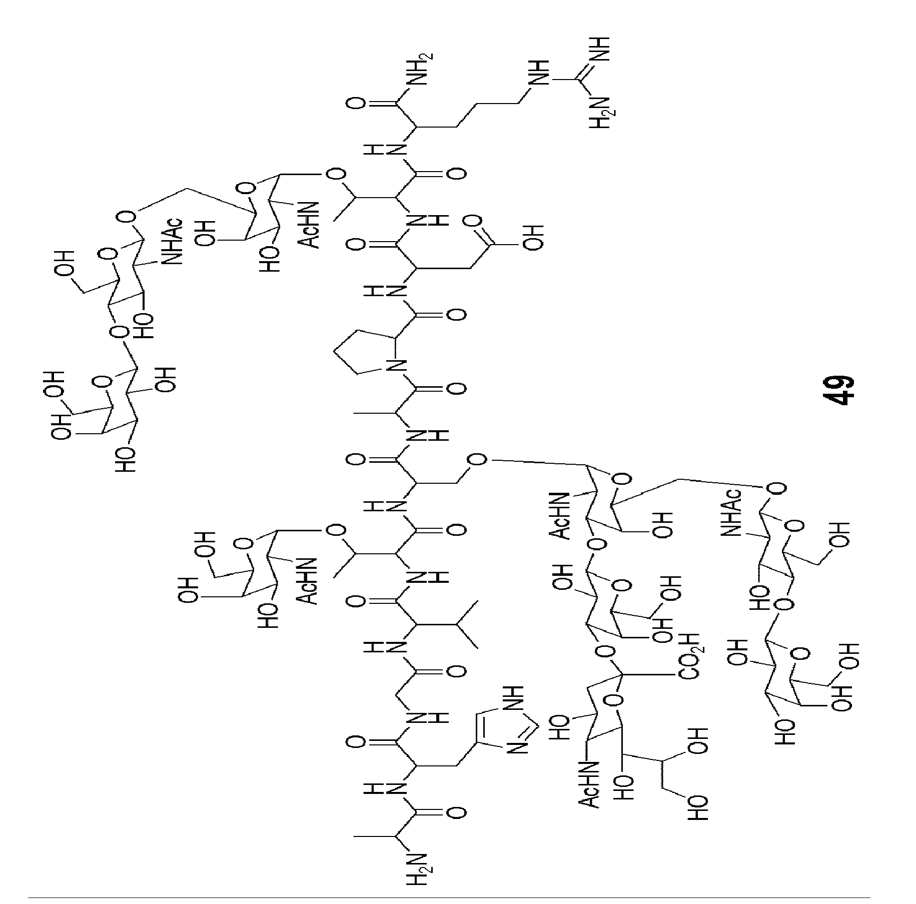

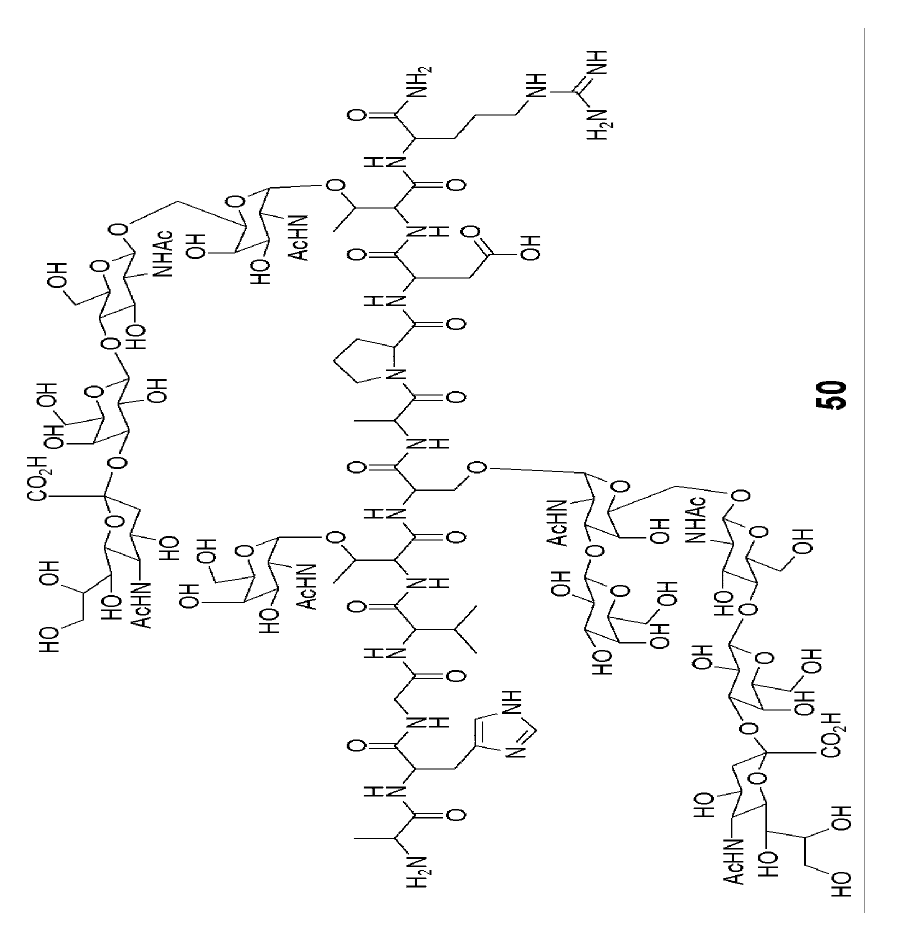

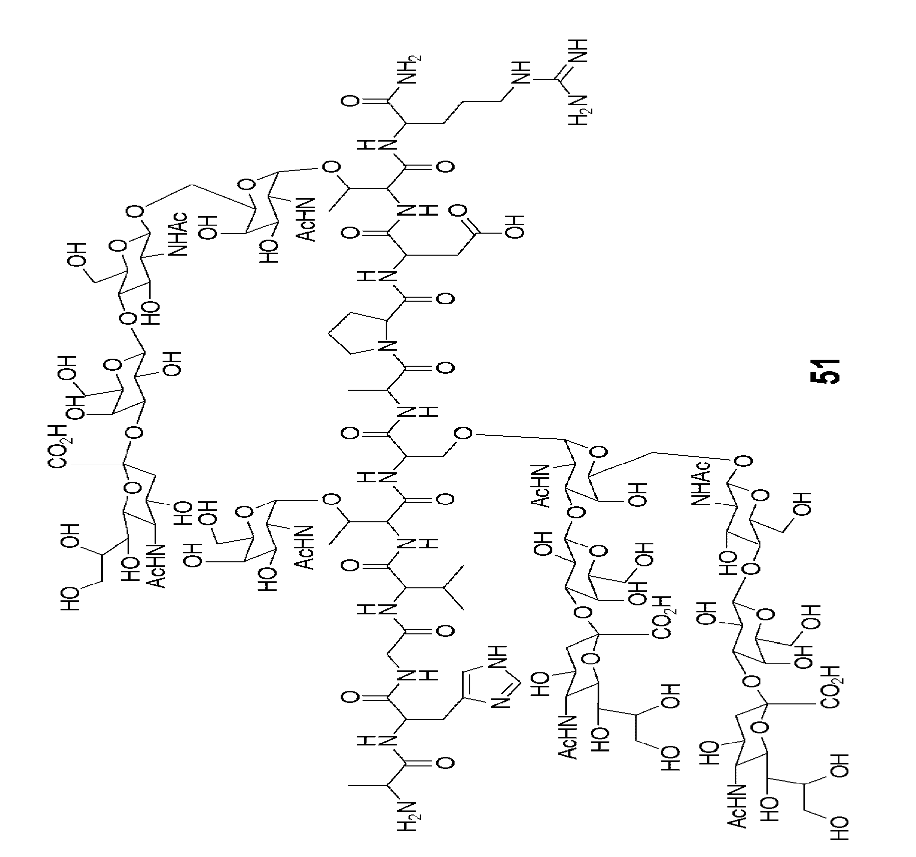

In accordance with the manipulation in Example (3.8), compounds (44), (45), (46) and (47) were prepared by consecutive reaction described in (3.10 Synthesis of compounds (44) to (51)). Confirmation of products and the ratio of glycosyl transfer reaction was performed by reverse phase HPLC and MALDI-TOF/MS analysis as will be described below for each of compounds (48), (49), (50) and (51) which were obtained from specific cleavage reaction of a peptide chain by BLase.

Compound (48): Yield: 90% or higher; ratio of transfer: 95% or higher; MALDI-TOF/MS:[M(average)+H]⁺ =2612.2 (theoretical value: [M(average)+H]⁺=2613.6)

Compound (49): Yield: 90% or higher; ratio of transfer: 90% or higher [approximately 5% of compound (48) derived from unreacted compound (44) was confirmed); MALDI-TOF/MS:[M(average)+H]⁺ =2903.3 (theoretical value: [M(average)+H]⁺=2904.8)

Compound (50): Yield: 90% or higher; ratio of transfer: 95% or higher; MALDI-TOF/MS:[M(average)+H]⁺ =3194.4 (theoretical value: [M(average)+H]⁺=3196.1)

Compound (51): Yield: 90% or higher; ratio of transfer: 95% or higher; MALDI-TOF/MS:[M(average)+H]⁺ =3493.0 (theoretical value: [M(average)+H]⁺=3487.3)

(3.11 Synthesis of compounds (52) and (53))

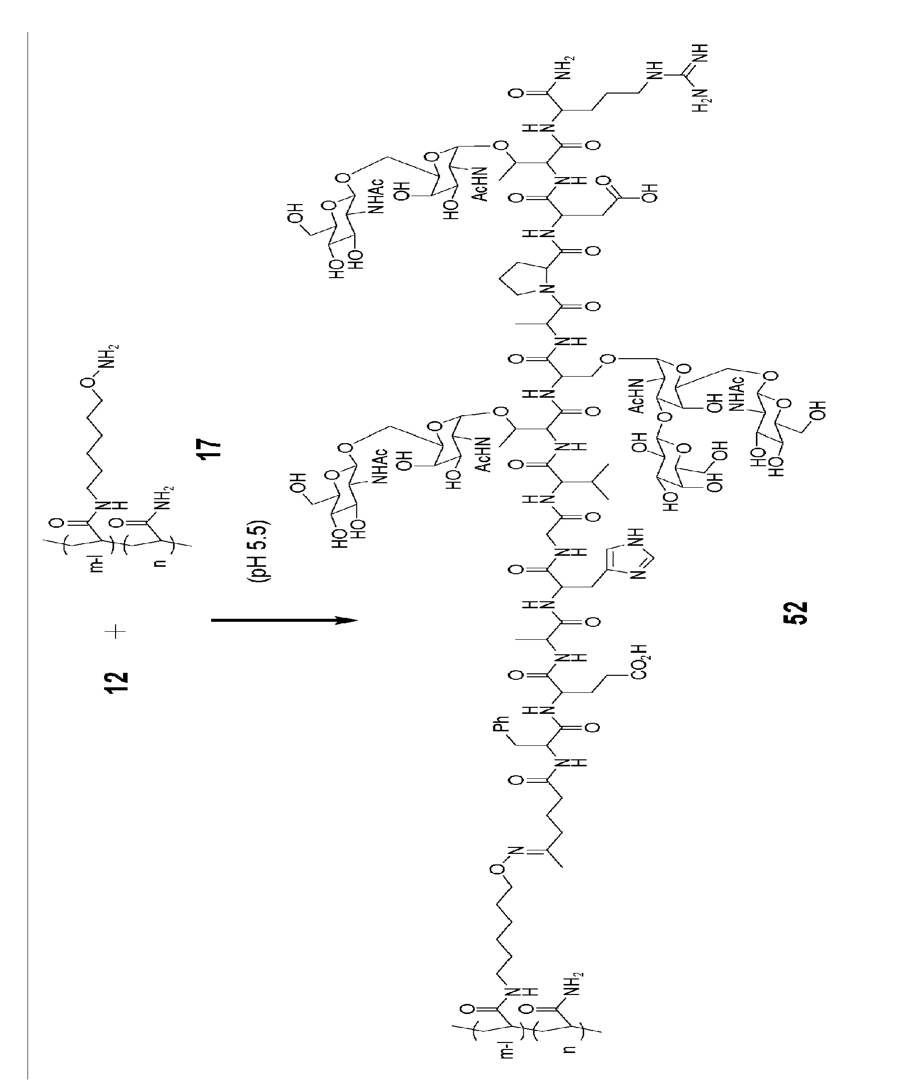

400µl of reaction solution containing 2.5mM glycopeptide derivative (12) and 5.0mM (oxyamine residue calculation) water-soluble polymer (17) was adjusted to have pH of 5.3 with 1N sodium hydroxide, and was stirred for six hours at room temperature. The reaction solution was purified by gel filtration [Biogel P-4: eluate: 25mM ammonium acetate buffer solution (pH 6.5)], thereby obtaining 6.3mg of lyophilized compound (52) [ratio of trapping compound (12): 95% or higher (GPC-HPLC)].

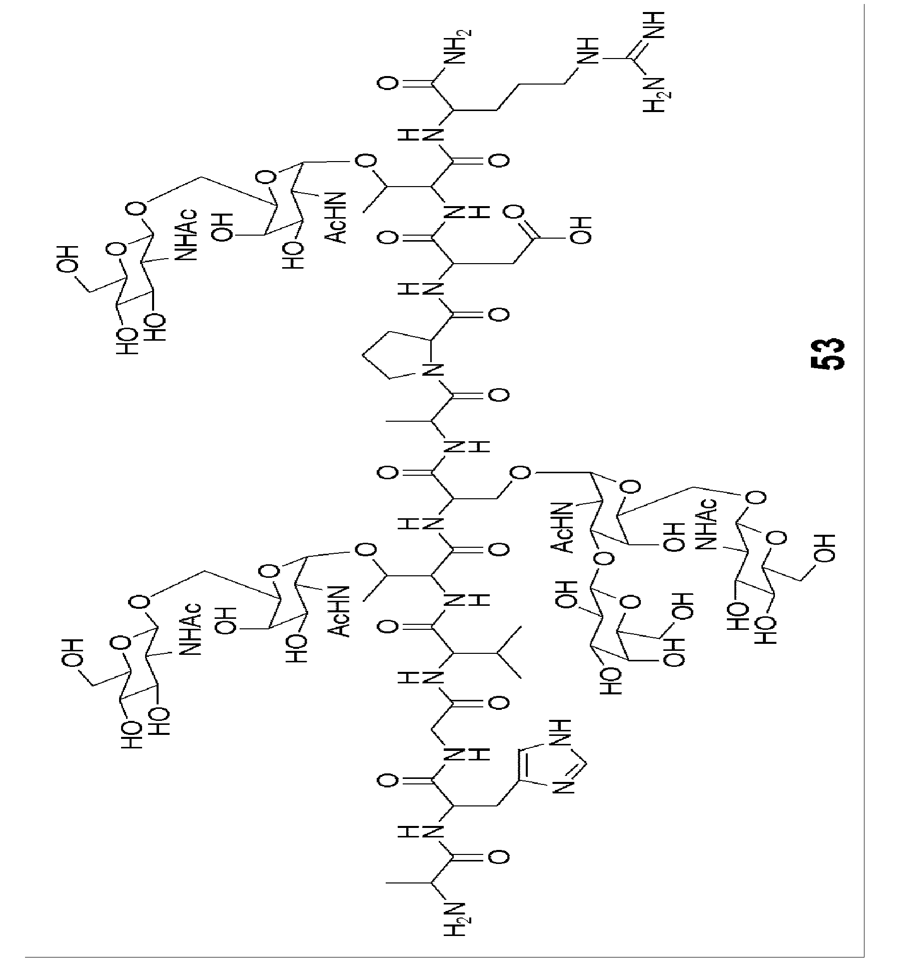

BLase reaction to compound (52) was performed in the same manner as in Example (3.7), thereby obtaining compound (53) [ratio of reaction: 90% or higher (GPC-HPLC)]. Identification of the transferred compound was performed by confirming [M(average)+H]⁺=2490.1 (theoretical value: [M(average)+H]⁺=2492.5) derived from compound (53) by MALDI-TOF/MS.

(3.12 Synthesis of compounds (54) to (61))

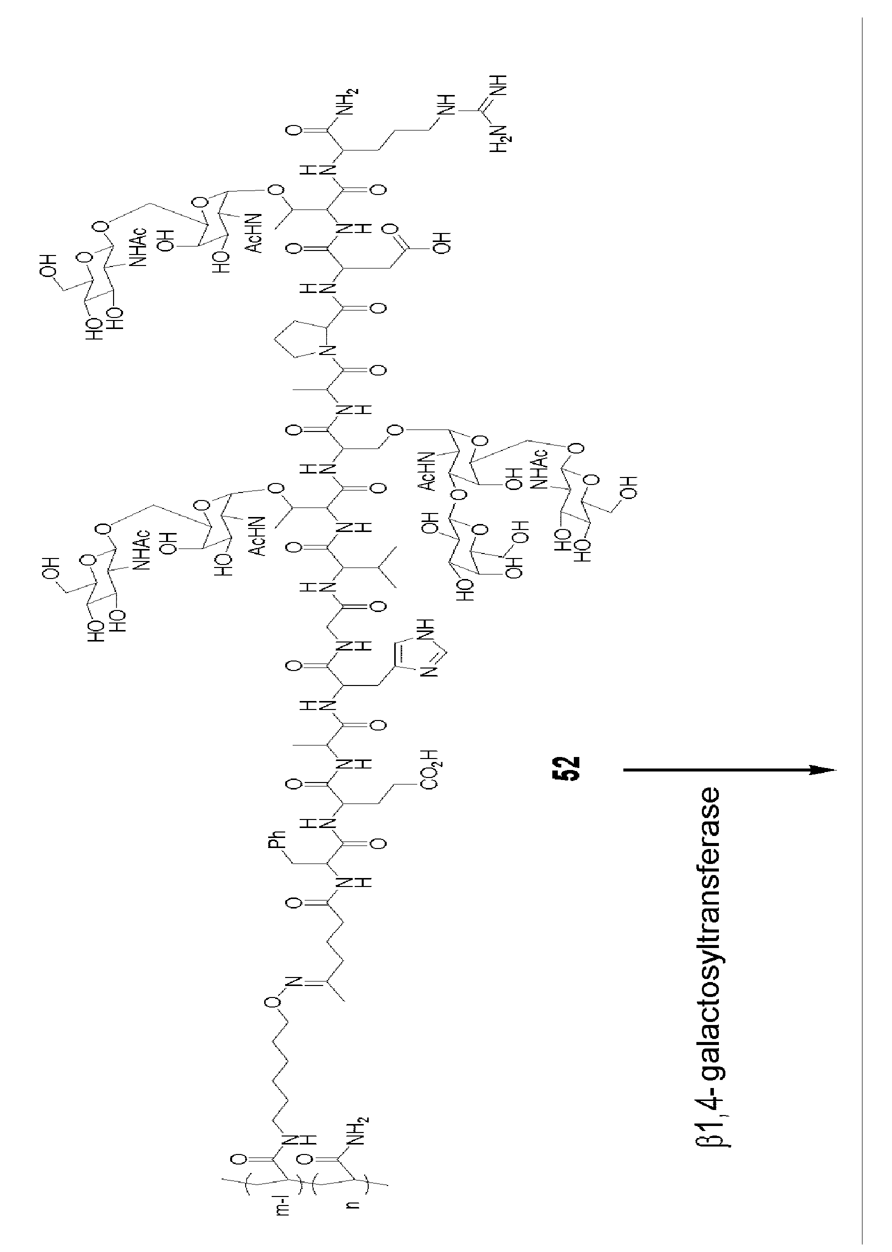

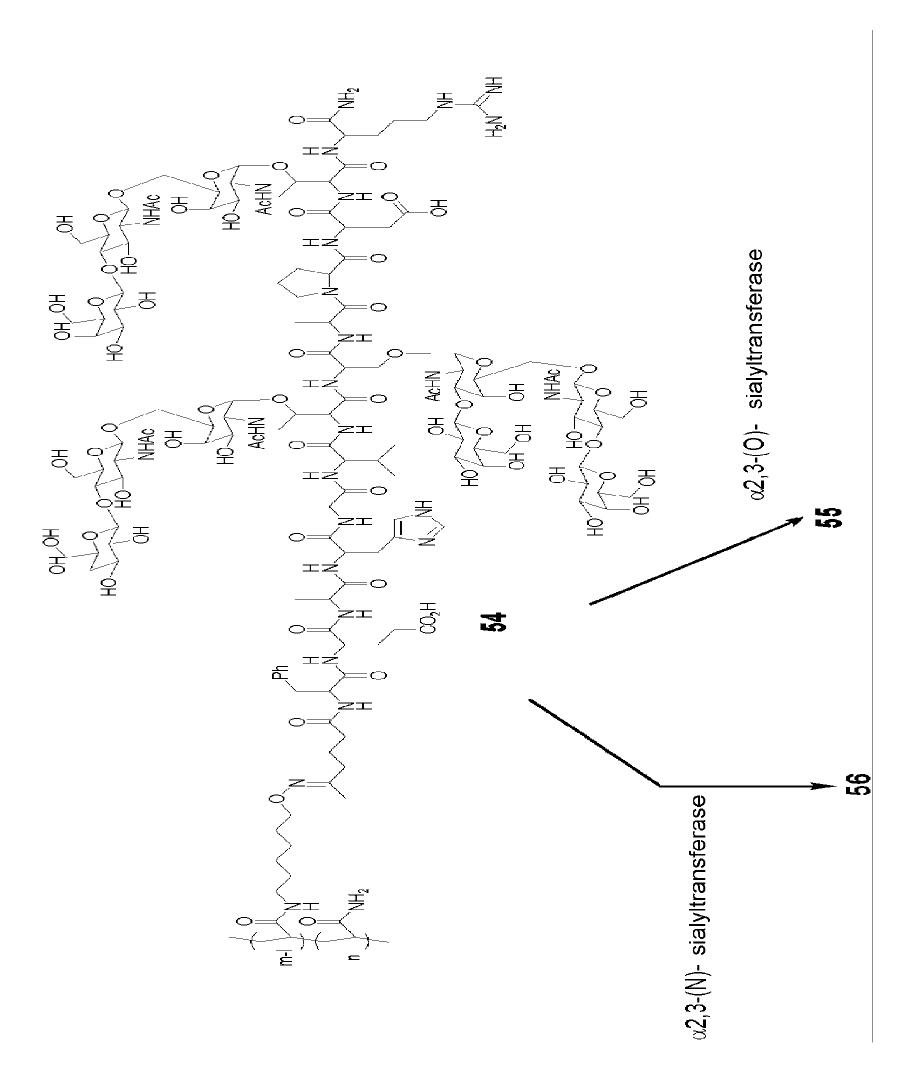

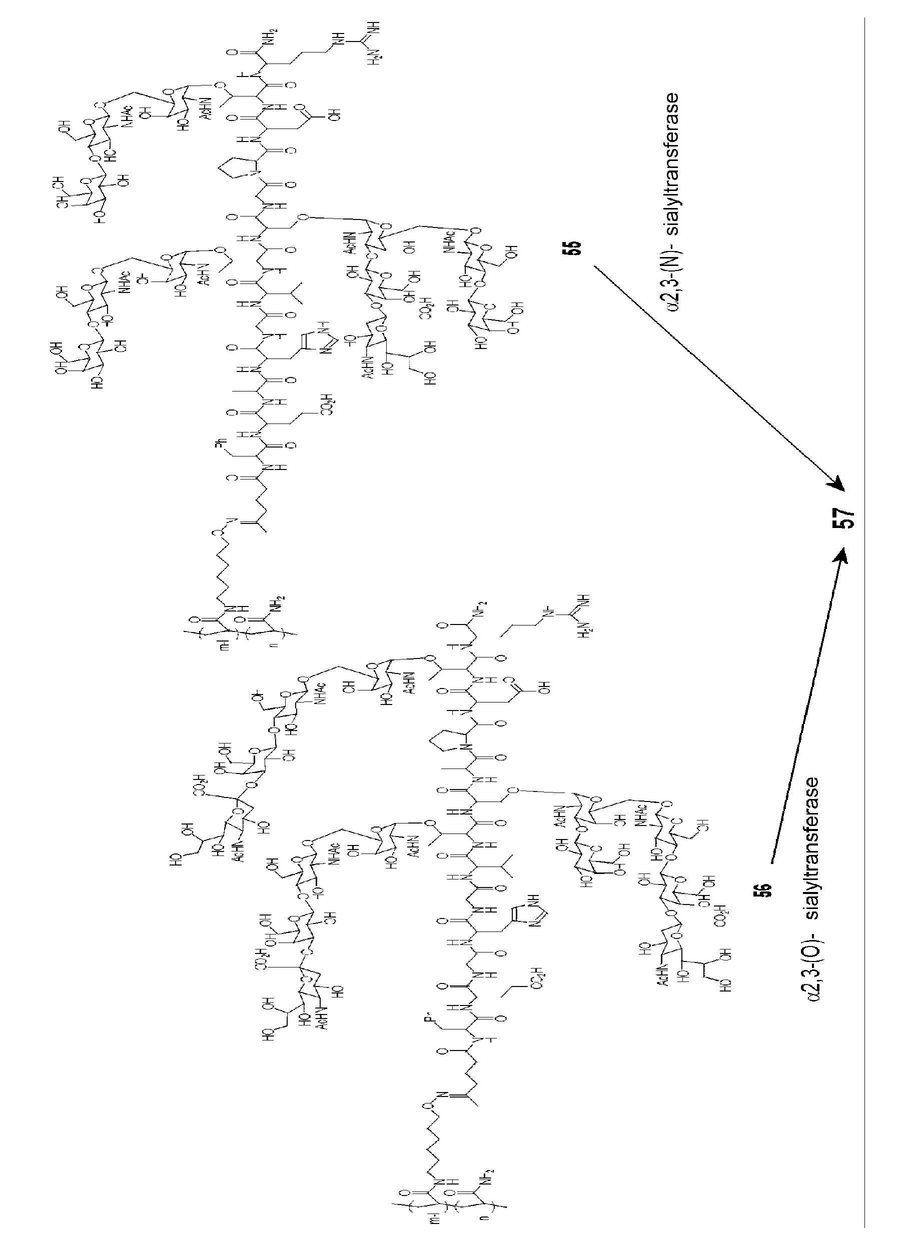

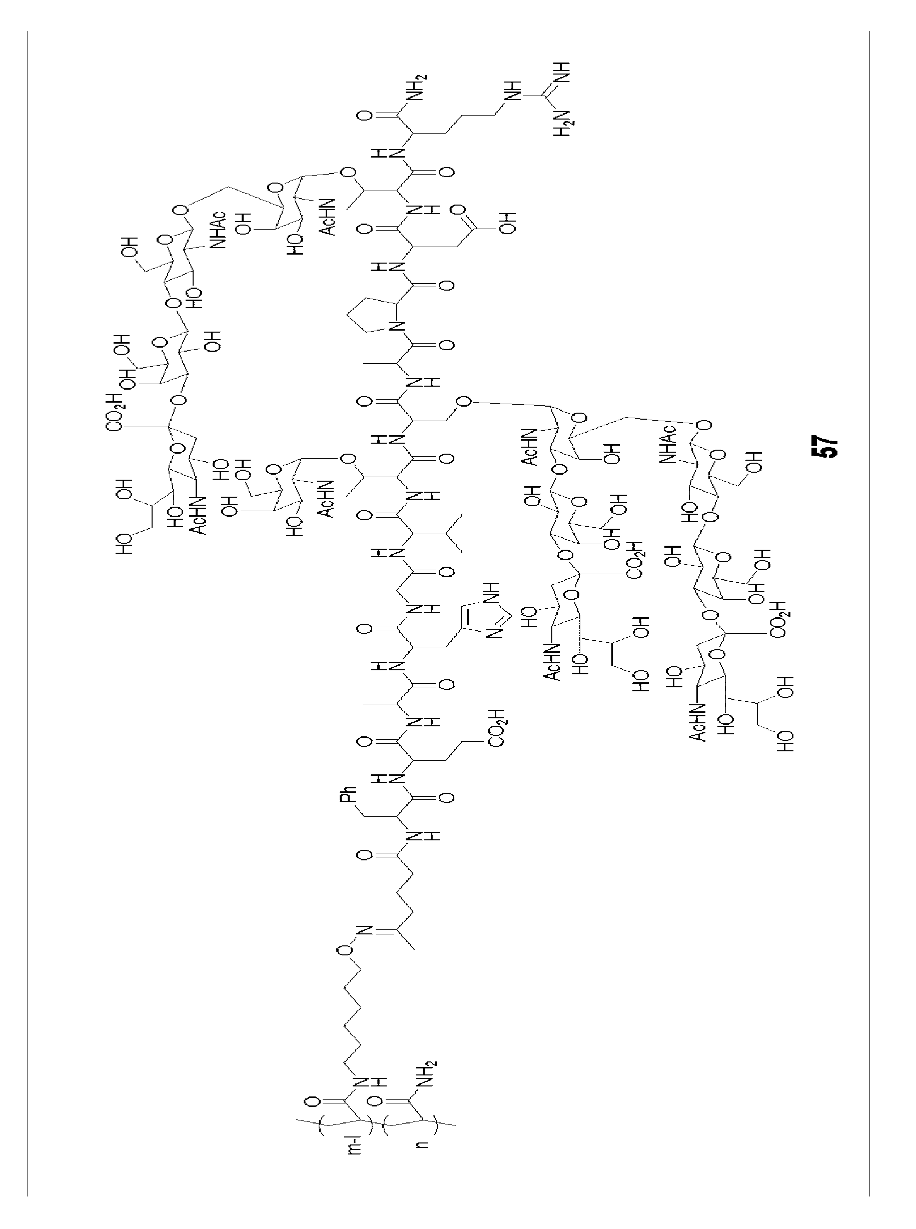

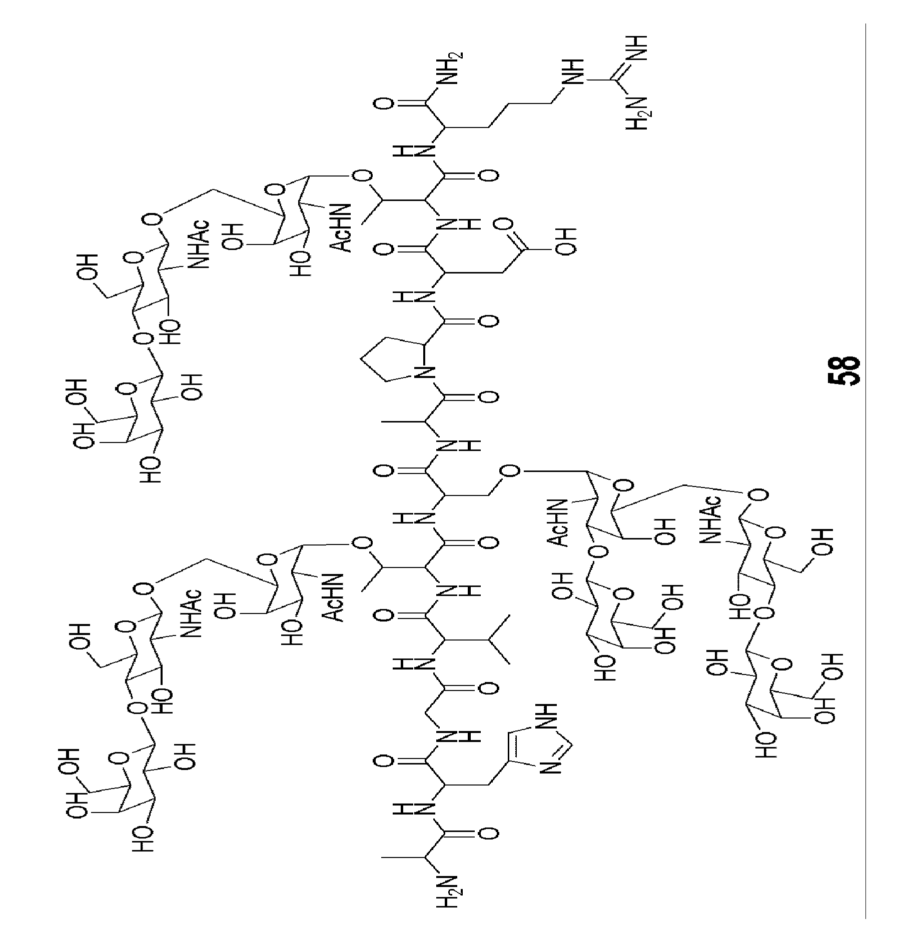

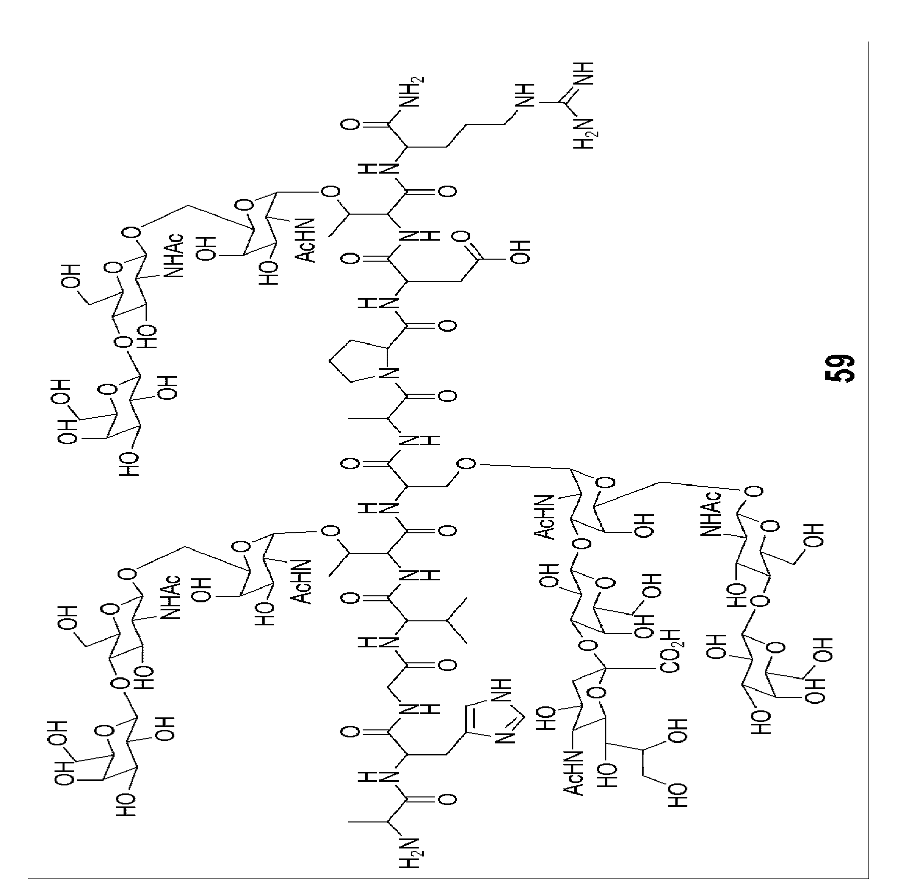

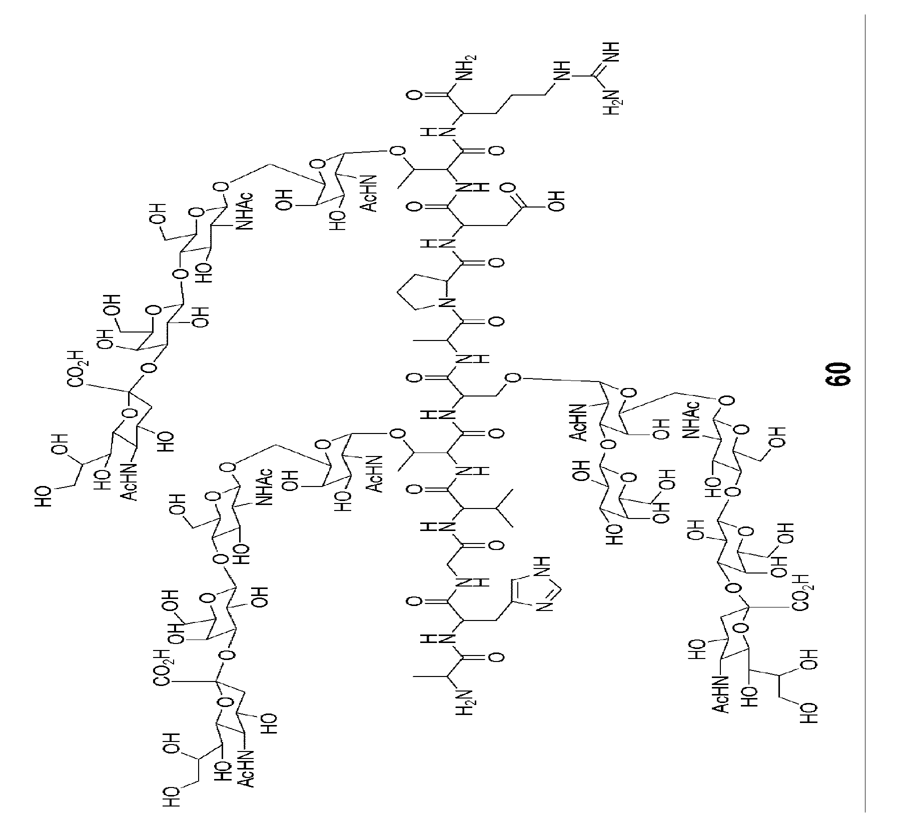

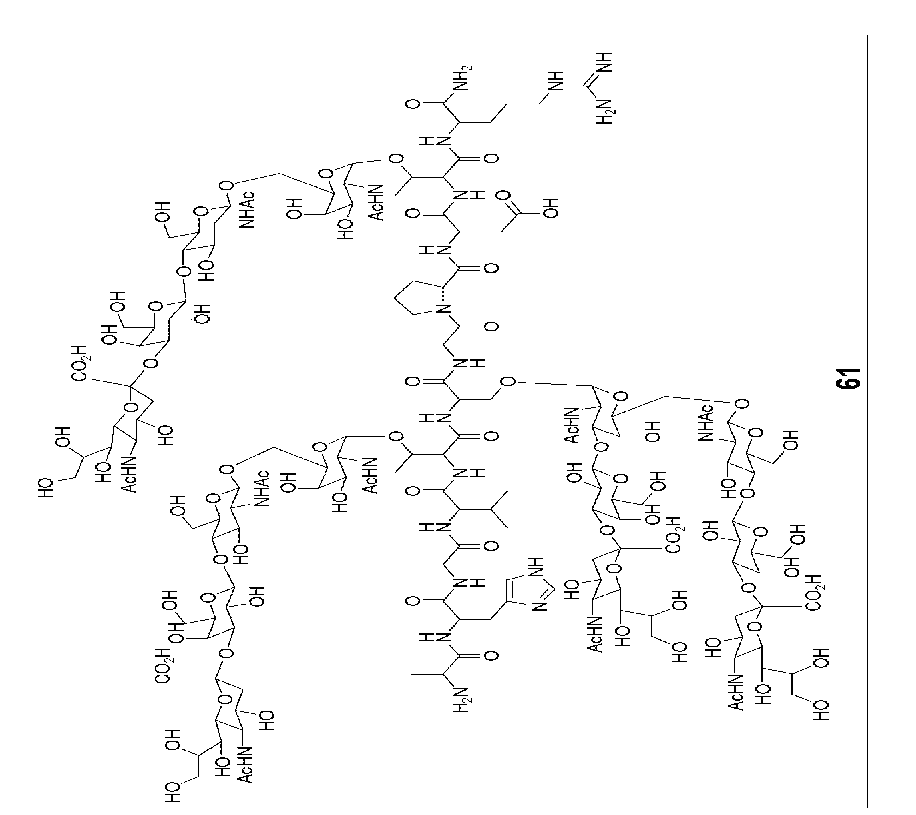

In accordance with the manipulation in Example (3.8), compounds (54), (55), (66) and (57) were prepared by consecutive reaction described in (3.10 Synthesis of compounds (44) to (51)). Confirmation of products and the ratio of glycosyl transfer was performed by reverse phase HPLC and MALDI-TOF/MS analysis as described below for each of compounds (58), (59), (60) and (61) which were obtained from specific cleavage reaction of a peptide chain by BLase.

Compound (58): Yield: 90% or higher; ratio of transfer: 95% or higher; MALDI-TOF/MS:[M(average)+H]⁺ =2976.9 (theoretical value: [M(average)+H]⁺=2978.9)

Compound (59): Yield: 90% or higher; ratio of transfer: 95% or higher; MALDI-TOF/MS:[M(average)+H]⁺ =3268.4 (theoretical value: [M(average)+H]⁺=3270.2)

Compound (60): Yield: 90% or higher; ratio of transfer: 95% or higher; MALDI-TOF/MS:[M(average)+H]⁺ =3857.1 (theoretical value: [M(average)+H]⁺=3852.7)

Compound (61): Yield: 90% or higher; ratio of transfer: 95% or higher; MALDI-TOF/MS:[M(average)+H]⁺ =4147.8 (theoretical value: [M(average)+H]⁺=4143.9)

### (3.13 Synthesis of compounds (62) to (69) using a one-pot reaction)

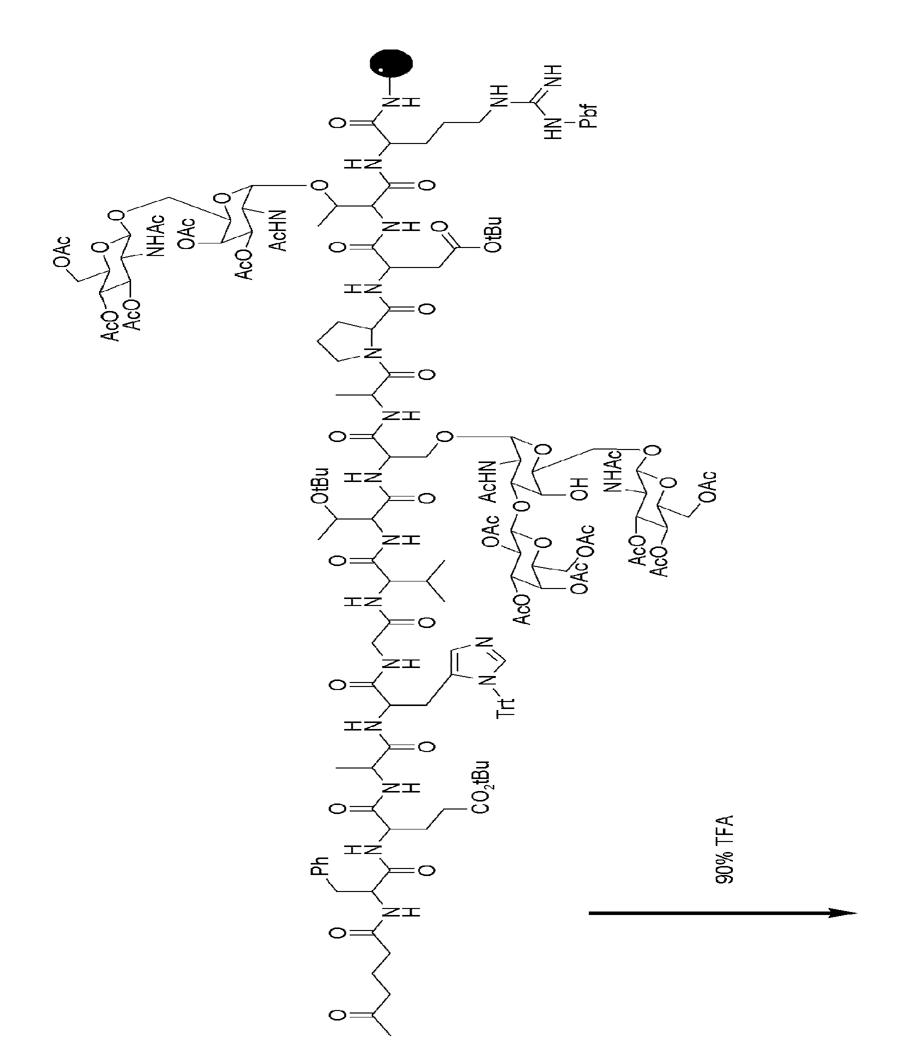

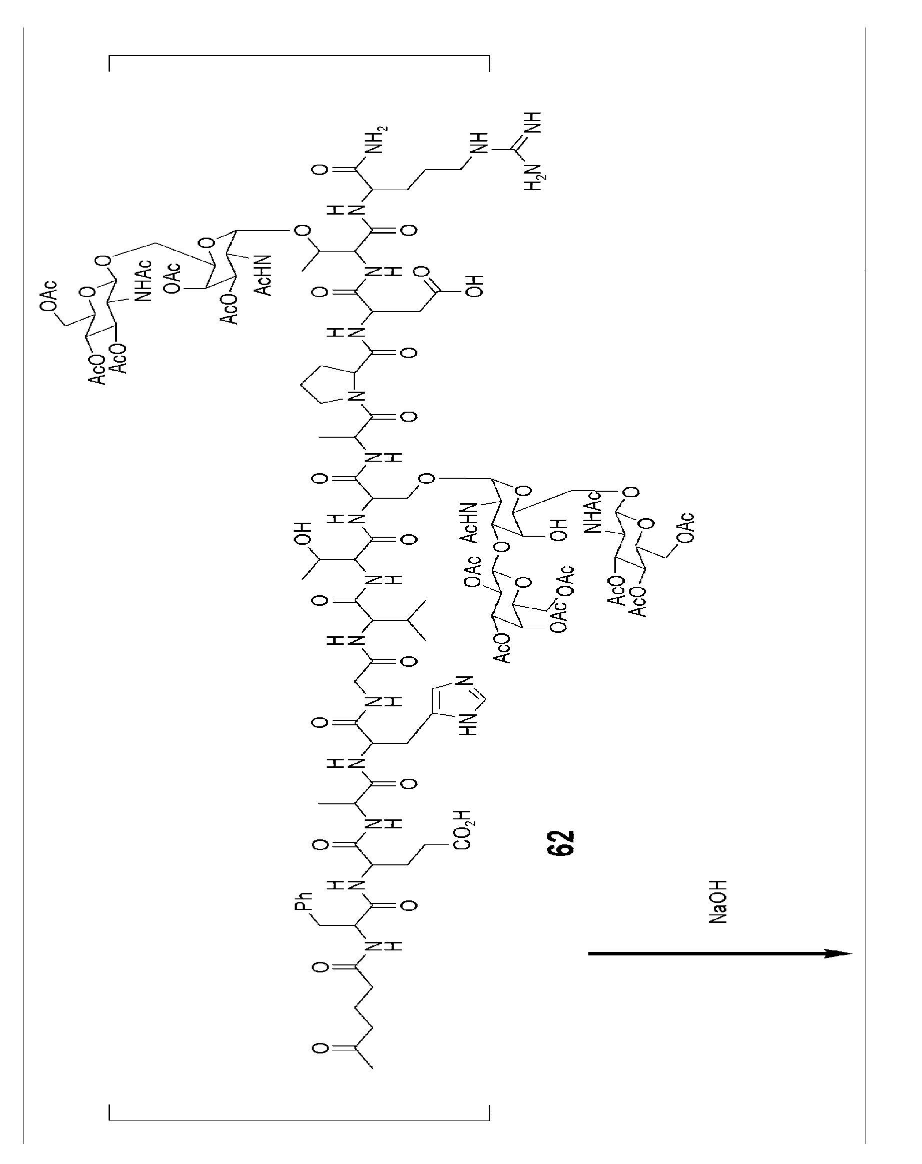

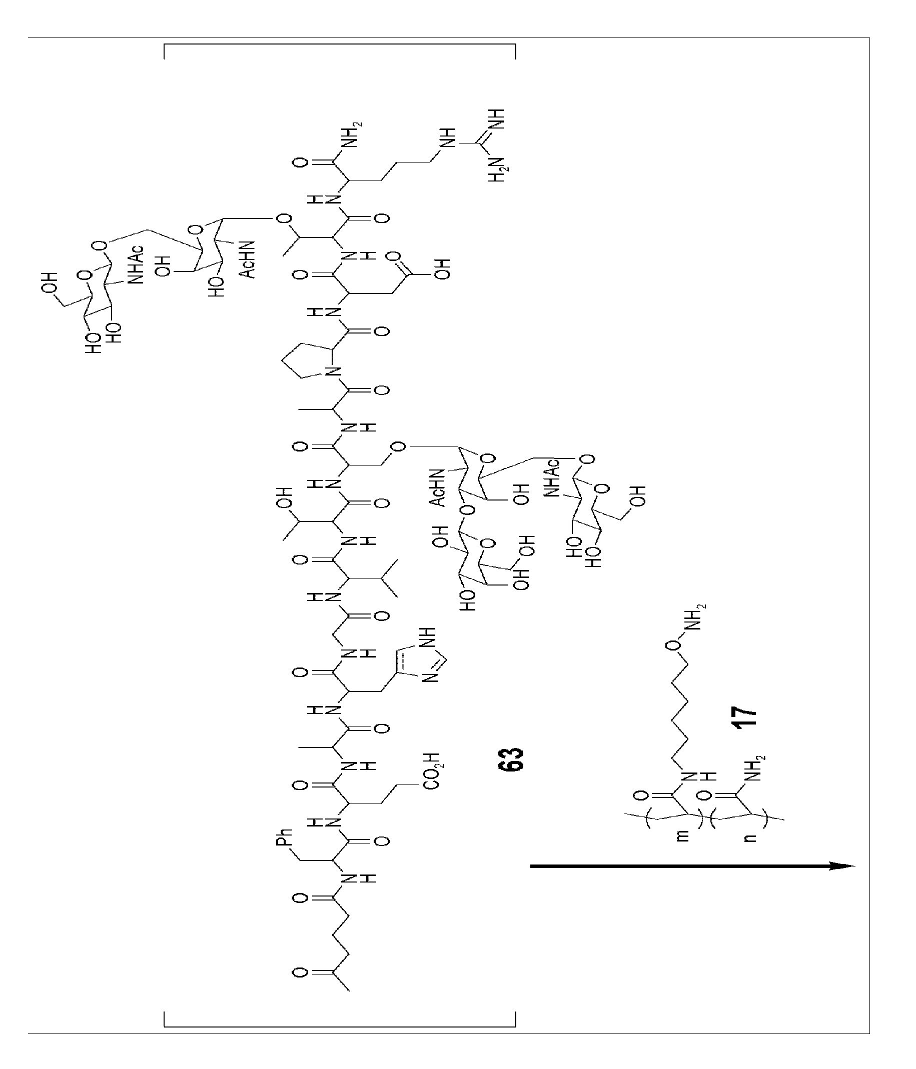

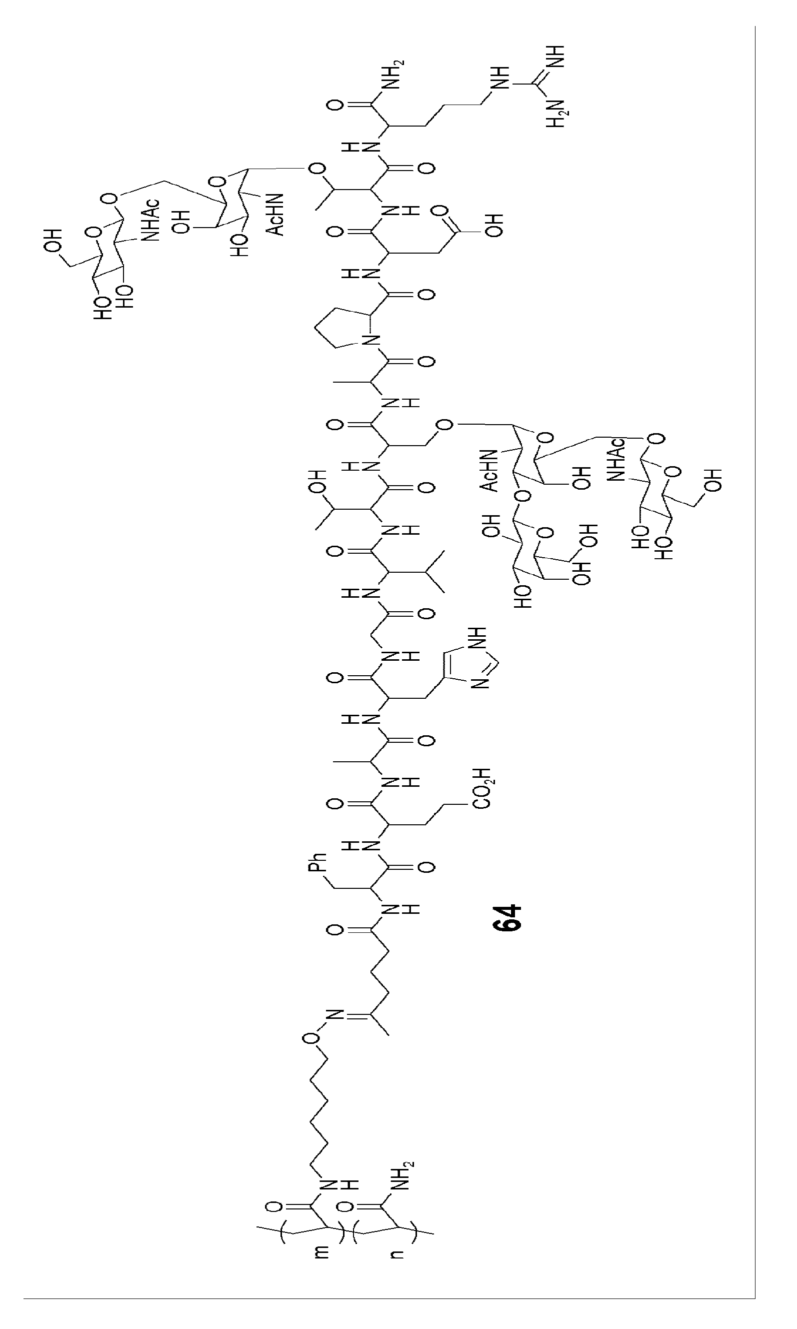

Using 0.12g (0.03mmol) of Tentagel® (Hipep Laboratories, 0.25mmol/g) as a support, N-protected amino acids and keto acid as described below were sequentially condensed by Fmoc/HBTU/HOBt method, thereby synthesizing a glycopeptide derivative of interest: Fmoc-Arg(Pbf)-OH; Fmoc-Thr(Ac5core6)-OH; Fmoc-Asp(OtBu)-OH; Fmoc-Pro-OH; Fmoc-Ala-OH; Fmoc-Ser(Ac7core2)-OH; Fmoc-Thr(OtBu)-OH; Fmoc-Val-OH; Fmoc-Gly-OH; Fmoc-His(Trt)-OH; Fmoc-Ala-OH; Fmoc-Gln(OtBu)-OH; Fmoc-Phe-OH; and 5-ketohexane acid. After the peptide elongation reaction, 61.3mg (equivalent to 0.01mmol) of the obtained resin was allowed to react in 90% TFA aqueous solution for two hours at room temperature to eliminate the protective group on the peptide residue and concurrently release compound (62) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 2.5ml of methanol. 40µl of 1N sodium hydroxide aqueous solution was added to this solution, and the solution was stirred for 1.5 hour at room temperature, thereby performing Ac deprotection reaction. After the reaction, H⁺-type cation exchange resin, Dowex50WX8 (available from Dow Chemical), was added and the solution was neutralized. Thereafter, the resin was separated by filtration. The solvent in the filtrate was removed, and the residue was dissolved in 2ml of 50mM acetic acid/sodium acetate buffer solution (pH 5.5). 10mM (oxyamine residue calculation) water-soluble polymer (17) aqueous solution was added to the solution, and the solution was stirred for 10 hours at room temperature, thereby reacting compound (63) with compound (17). After completion of the reaction, 2.5ml of the reaction solution (equivalent to 6.25µmol) was subjected to centrifugal concentration with ultrafiltration filter 30K Apollp® 20ml (Orbital Biosciences, available from LIC). 25mM HEPES buffer solution (pH 7.0) was added thereto and the solution was again subjected to concentration, thereby washing. The solution was concentrated so that the final amount was approximately 400µl. By adding 625µl of water, 10mM (theoretical content of glycopeptide) polymer (64) was obtained.

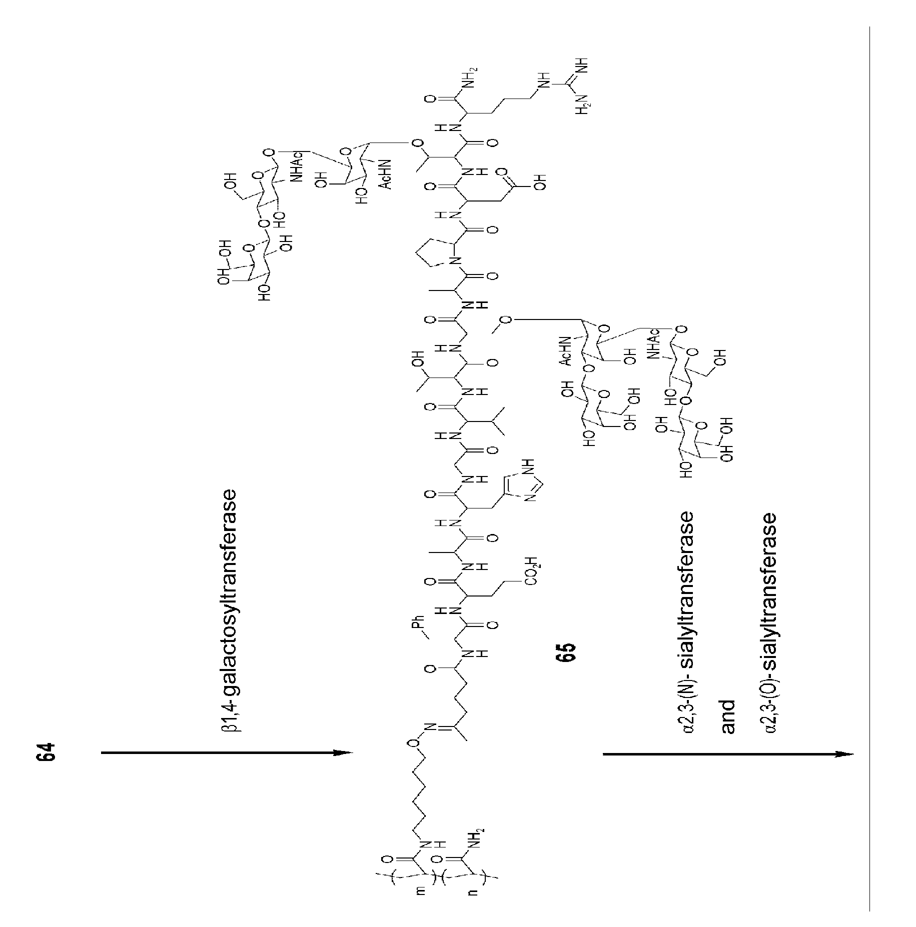

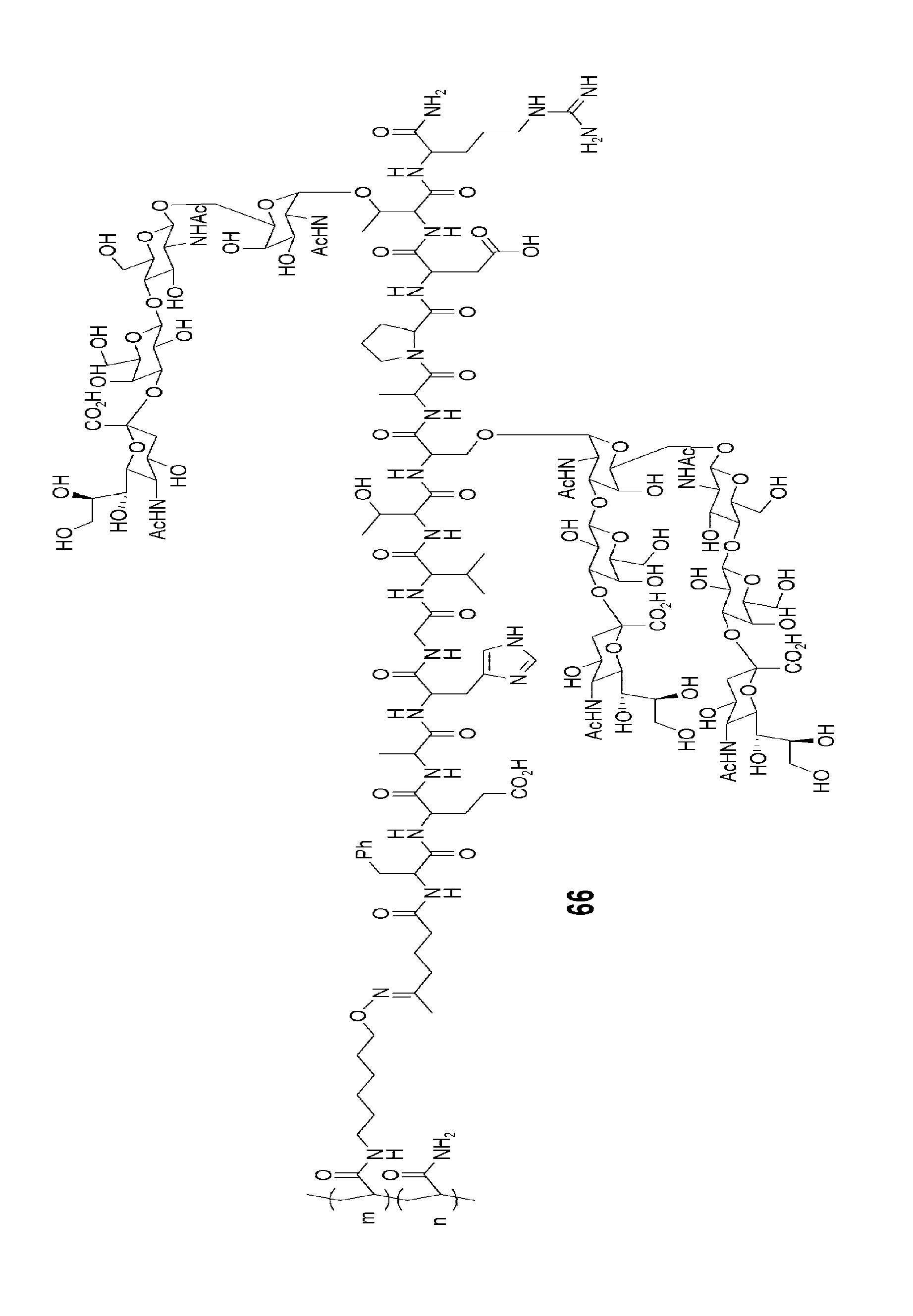

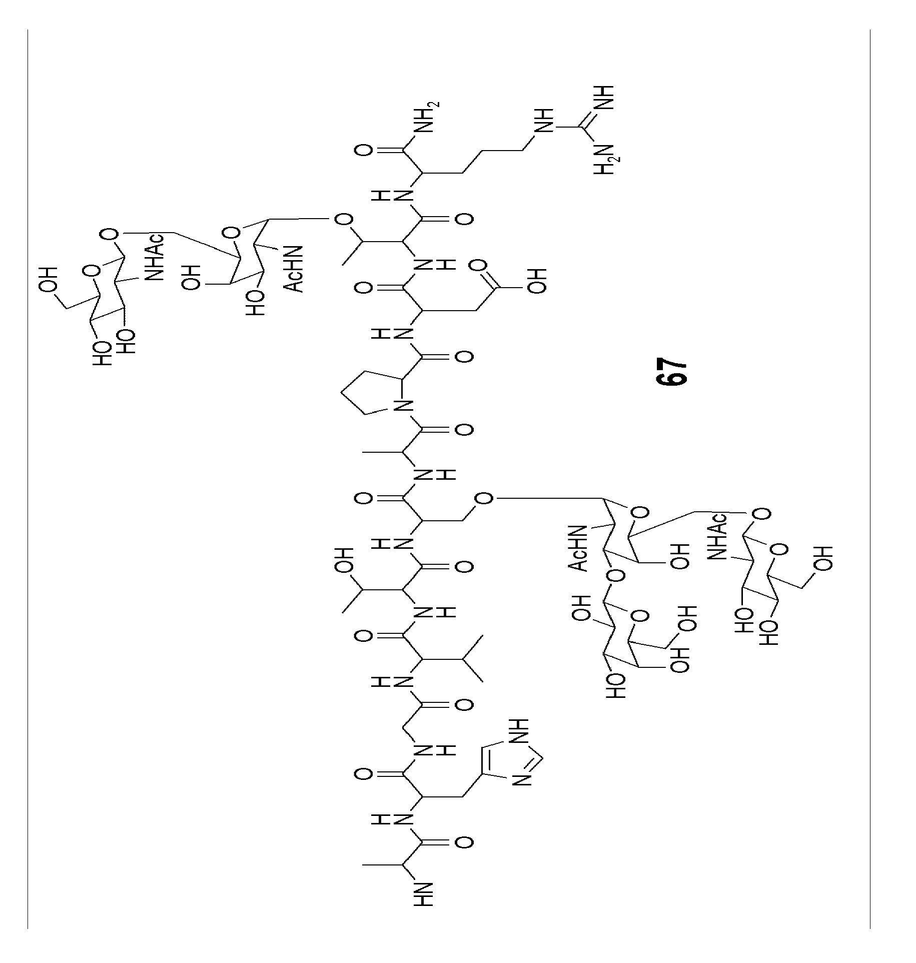

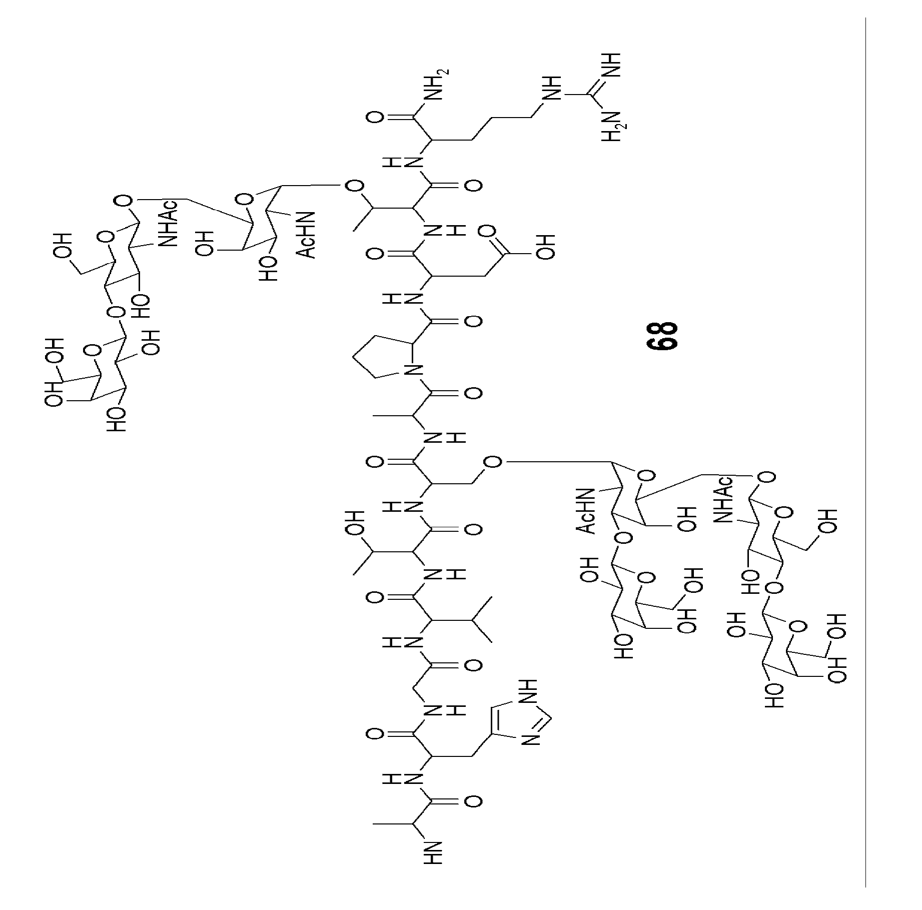

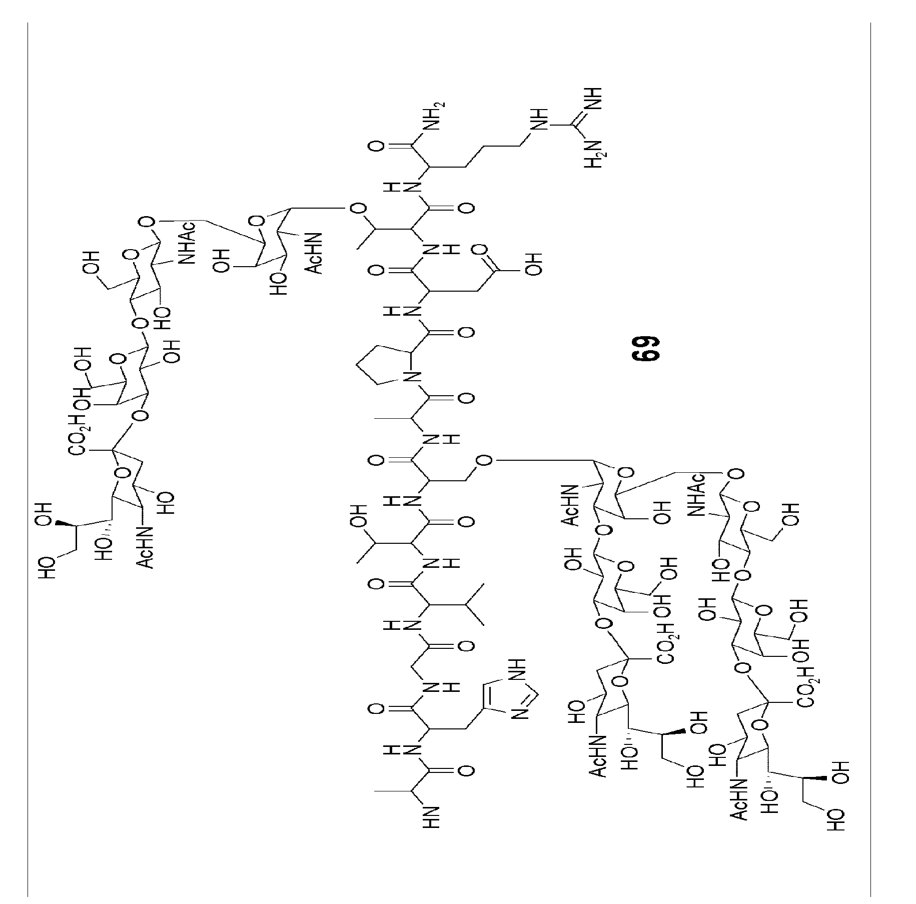

3µl of the above 10mM (theoretical content of glycopeptide) polymer (64) was sorted. 11µl of ammonium acetate buffer solution and 1µl of 1.74mg/ml solution of BLase (available from Shionogi & Co., Ltd.) were added thereto, and the solution was stirred for 10 minutes at 25°C. The reaction solution was analyzed by MALDI-TOF/MS to confirm [M(average)+H]⁺= (theoretical value: [M(average)+H]⁺=2085.1), thereby confirming production of compound (67).

100µl of reaction solution containing 50mM HEPES buffer solution (pH 7.0), 0.20U/ml human-derived β1,4-galactosyltransferase (available from TOYOBO CO., LTD.), 10mM manganese chloride, 5mM uridine-5'-disodium diphosphogalactose (UDP-Gal) and 1mM glycopeptide derivative (64) was allowed to react for two hours at 25°C. A part of the reaction solution was analyzed by MALDI-TOF/MS to confirm [M(average)+H]⁺=2408.6 (theoretical value: [M(average)+H]⁺=2409.4), thereby confirming production of compound (68). Meanwhile, 60µl of the reaction solution after galactose transfer reaction was sorted, and a mixture solution (total amount: 20µl) containing 2µl of 500mM HEPES buffer solution (pH 7.0), 2µl of 1% Triton X-100 aqueous solution, 1.2µl of 3.7U/ml recombinant rat α2,3-(N)-sialyltransferase (available from Calbiochem), 1.2µl of 0.88U/ml recombinant rat α2,3-(O)-sialyltransferase (available from Calbiochem), 6µl of 50mM cytidine-5'-sodium monophosphosialate (CMP-NANA) and 7.6µl of Milli Q water was added thereto. The solution was allowed to react at 25°C. The above reaction from the galactose transfer reaction was performed for two batches, and the solutions were mixed after the sialic acid transfer reaction. The reaction solution was transferred to an ultrafiltration filter, ULTRAFRE-MC 30, 000NMWL Filter Unit (available from Millipore, UFC3LTKOO) and was subjected to centrifugal concentration. Thereafter, 25mM ammonium acetate buffer solution (pH 6.5) was added thereto, and the solution was again concentrated with a centrifugal separator, thereby washing polymer (66). This manipulation was repeated for three times, thereby obtaining aqueous solution of compound (66). Thereafter, 2µl of 1.74mg/ml solution of BLase (available from Shionogi & Co., Ltd.) was added to the solution retained in the filter, which contains compound (66). The solution was again subjected to centrifugal filtration with the ultrafiltration filter, ULTRAFRE-MC 30,000NMWL Filter Unit (available fromMillipore,UFC3LTKOO). The obtained filtrate was purified by reverse phase HPLC (Inertsil®, ODS-3 4.6×250mm column, Mobile phase: A: 25mM ammonium acetate buffer solution (pH 6.5); B: acetonitrile, Gradient: 2% to 40%B with respect to A), thereby obtaining compound (69) [ratio of transfer: 95% or higher (HPLC)]. MALDI-TOF/MS:[M(average)+H]⁺ of compound (69)=3289.0 (theoretical value: [M(average)+H]⁺=3283.1)

(3.14 Synthesis of compounds (70) to (78), (21), (24) and (25) using a resin support)

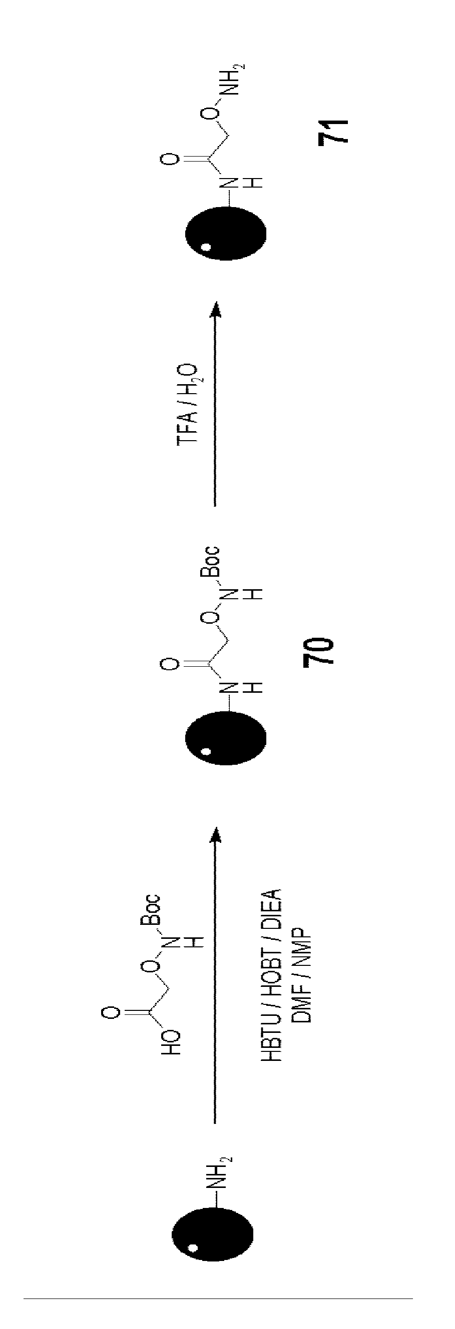

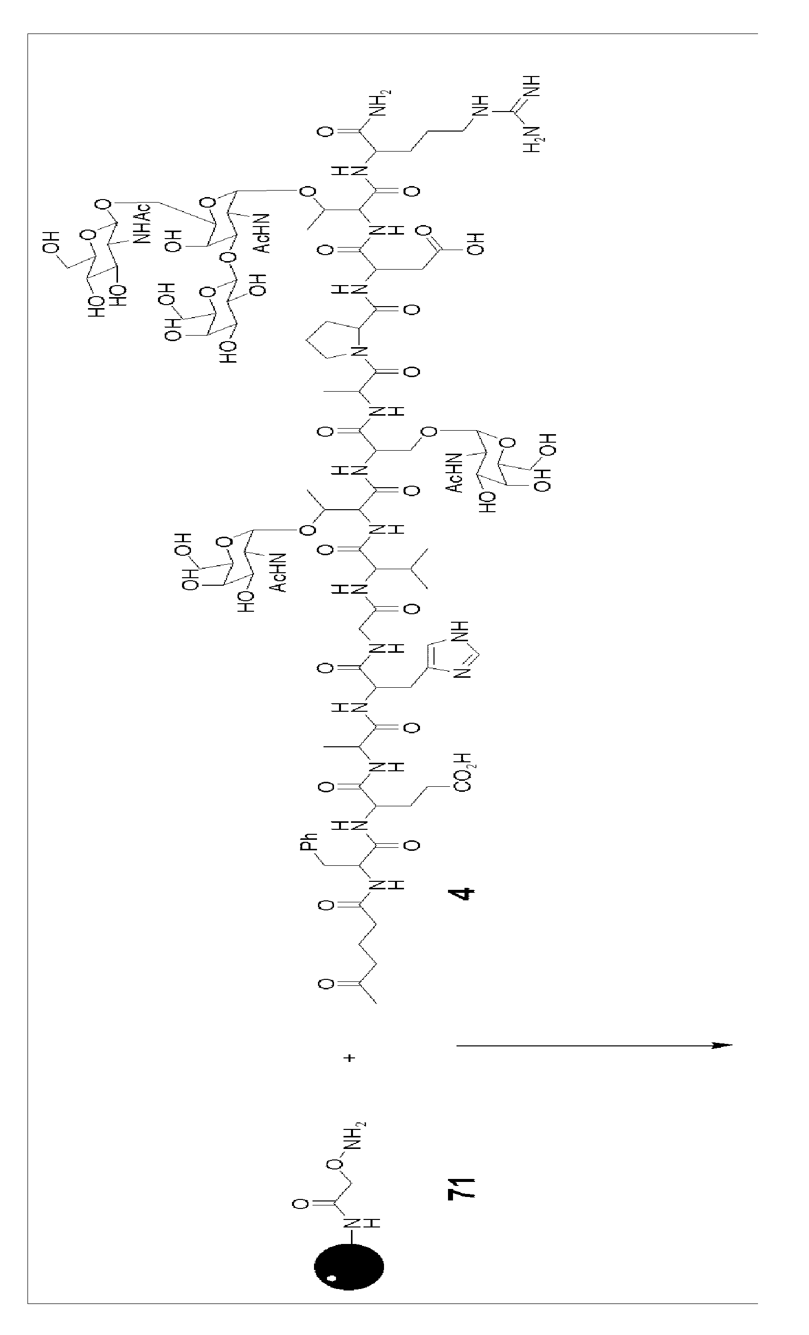

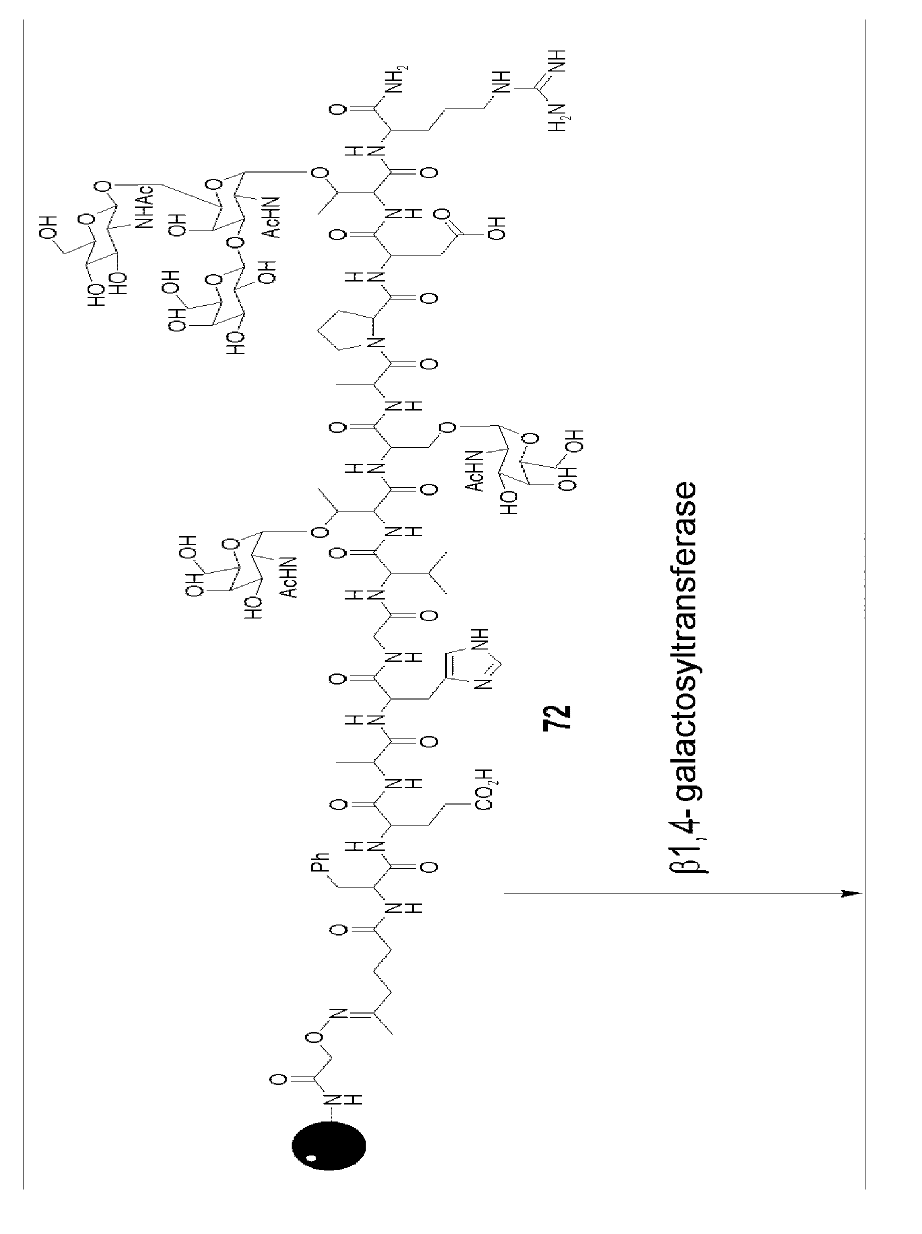

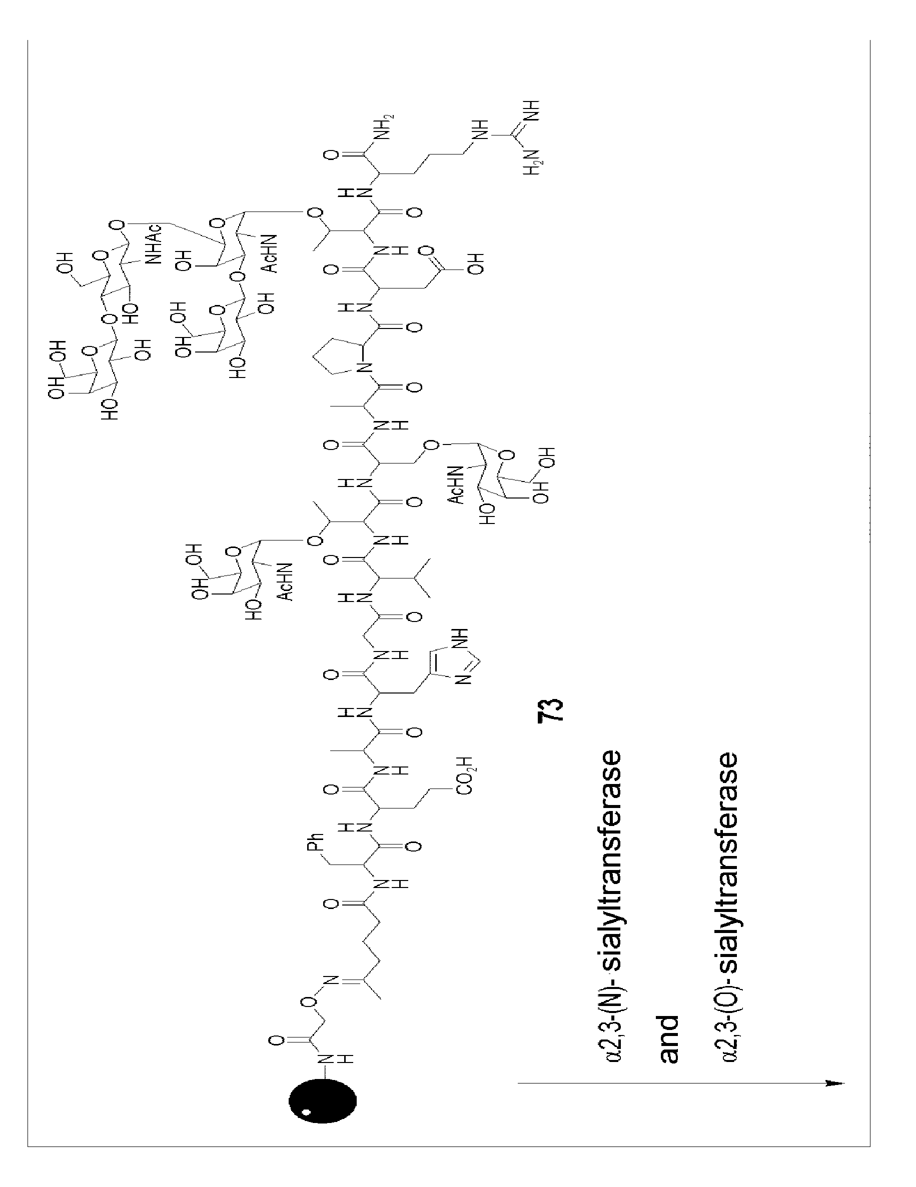

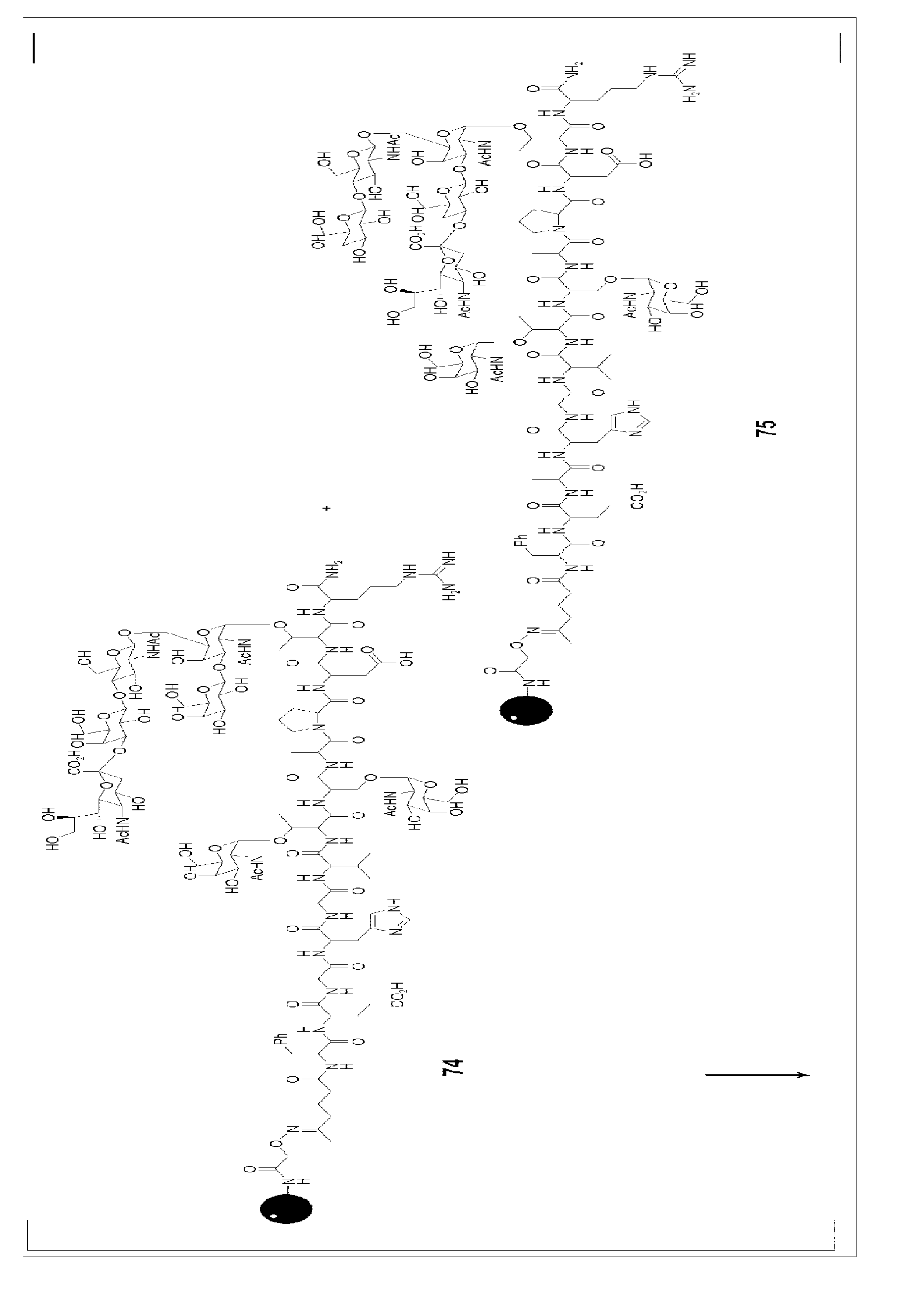

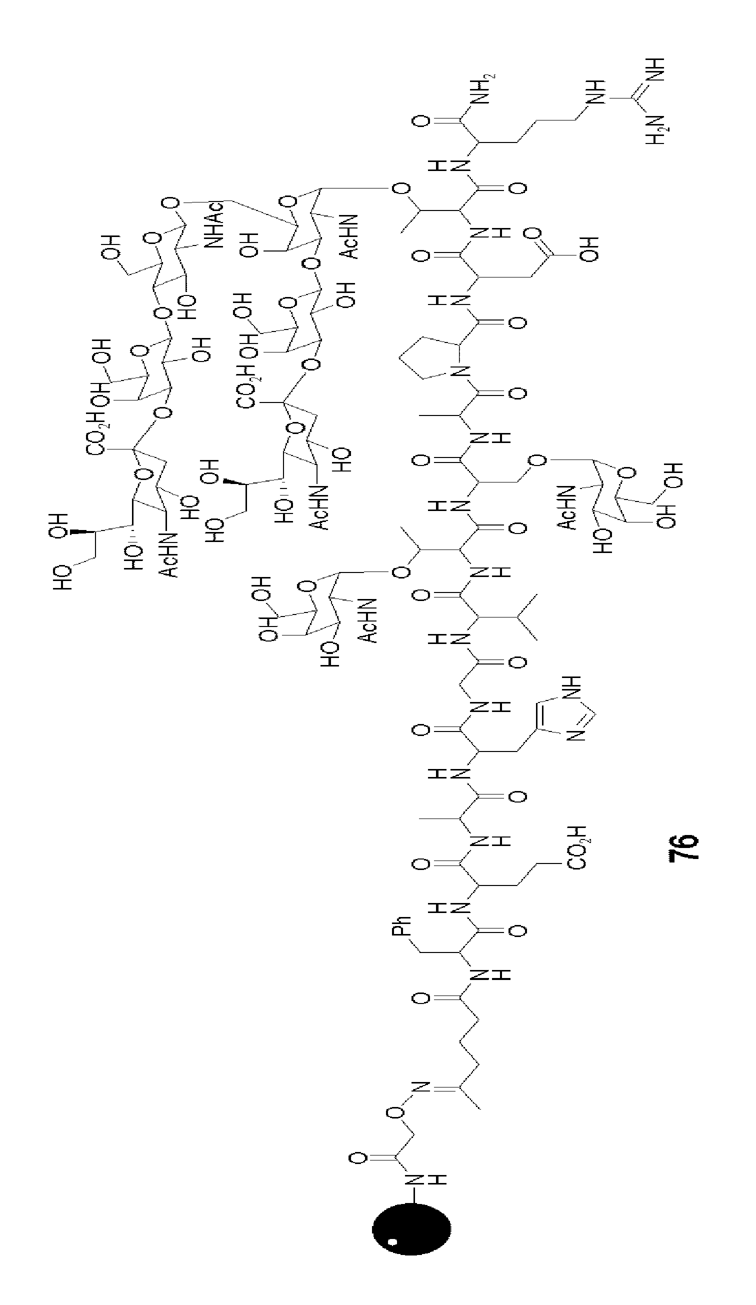

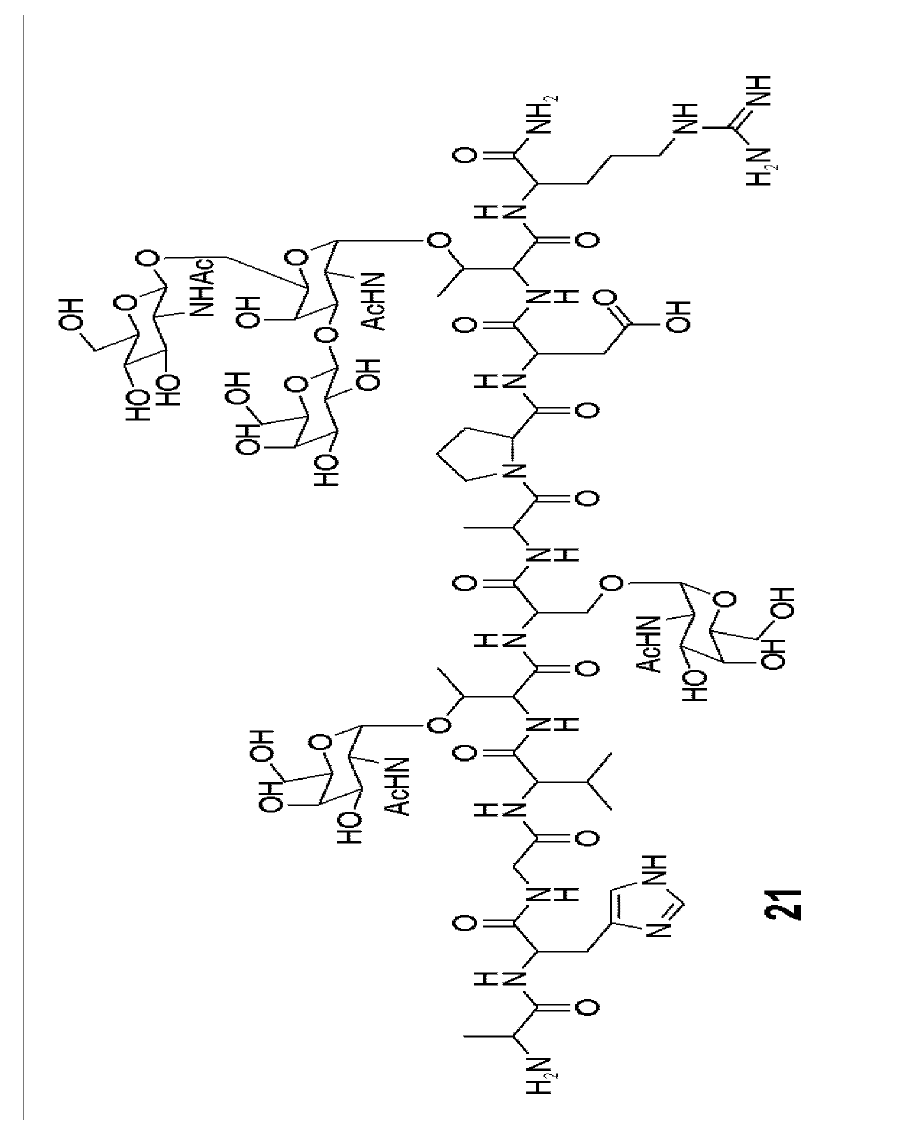

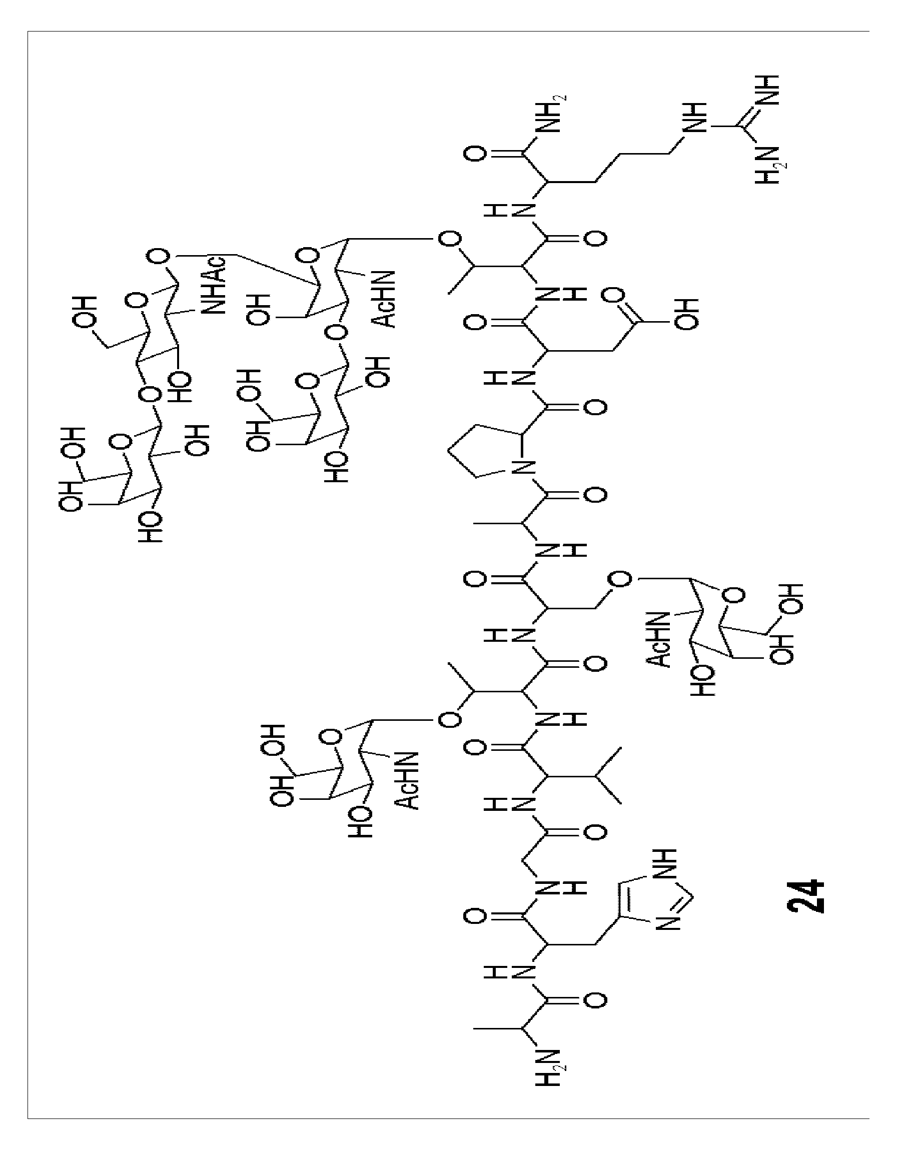

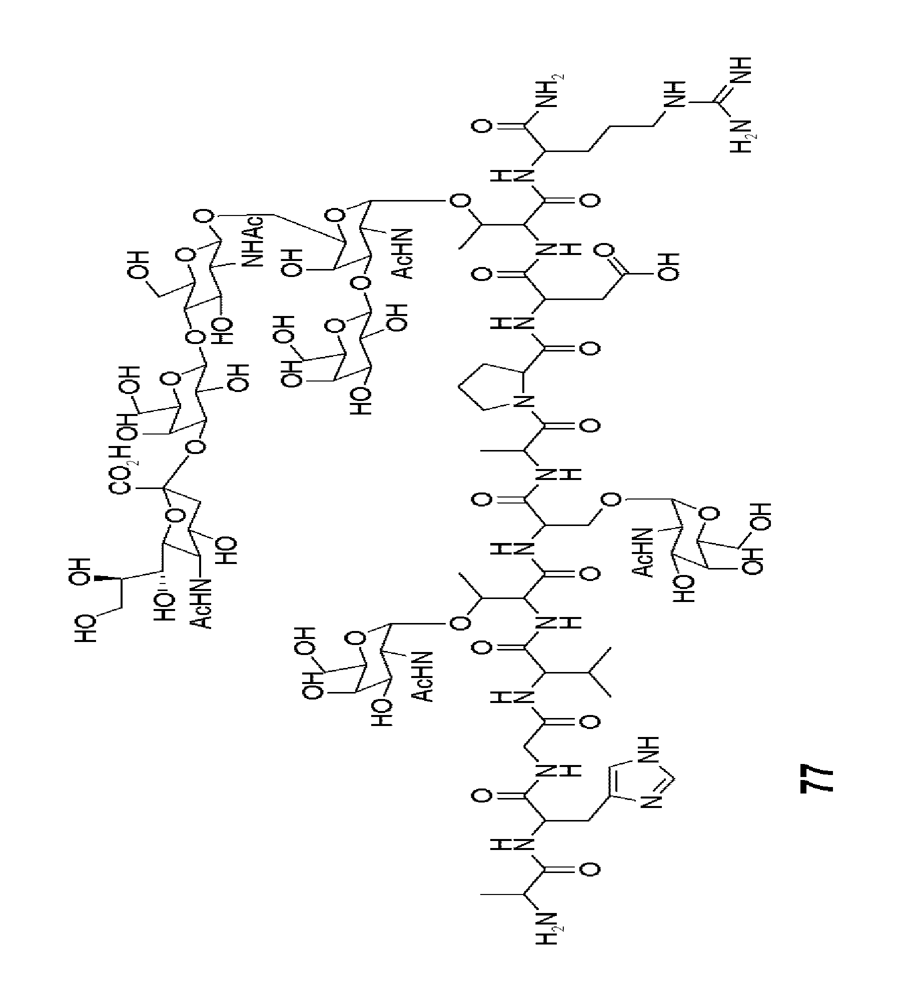

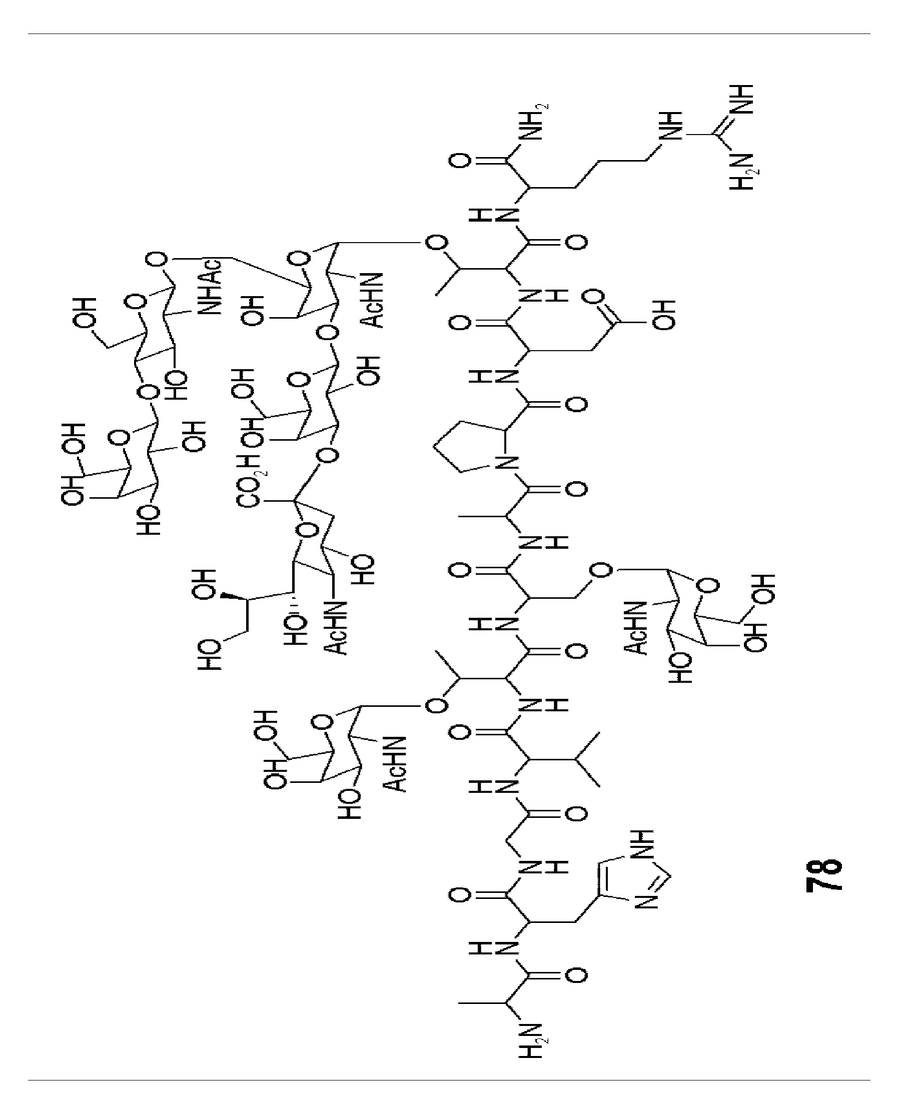

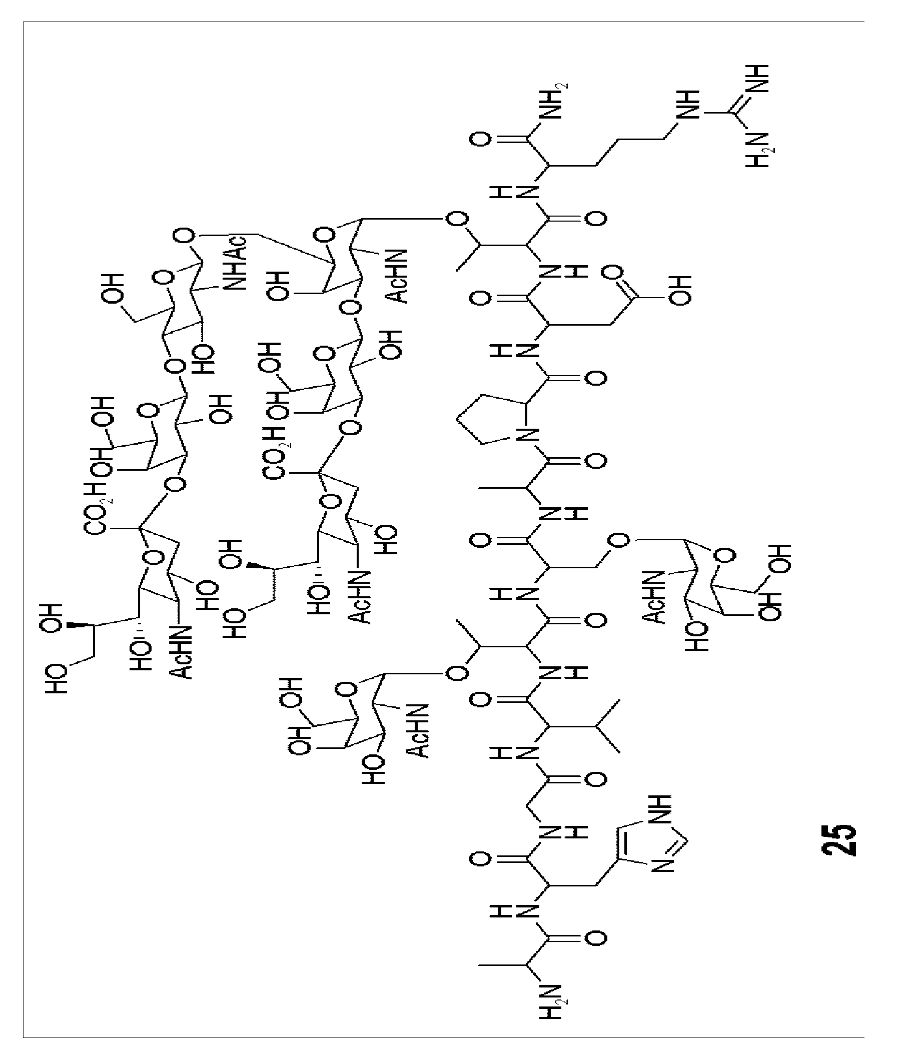

50mg of Amino PEGA resin (available from Novabiochem) (equivalent to 3µmol amino group) was coupled with Boc-amino-acetic acid by HBTU/HOBt method as a support. The obtained resin was stirred for one hour at room temperature in 50% TFA aqueous solution to eliminate Boc protective group, and the resin was washed in water. The resin was further washed in 50mM acetic acid/sodium acetate buffer solution (pH 5.5), thereby obtaining 85mg of undried compound (70). 62mg (2.2µl) of this undried resin (70) was slurried in 40µl of 50mM acetic acid/sodium acetate buffer solution (pH 5.5), and 40µl of aqueous solution of 5mM compound (4) (see the above synthesis method) was added thereto. After the solution was stirred for 19 hours at room temperature, the resin was separated by filtration, and the obtained resin was washed in 25mM HEPES buffer solution (pH 7.0), thereby obtaining 77mg of undried compound (71). Identification of the compound was performed by slurring a part of resin (71) in 25mMHEPES buffer solution (pH 7.0), adding 2µl of 1.74mg/ml solution of BLase (available from Shionogi & Co., Ltd.) thereto, stirring the solution for one hour and analyzing the supernatant liquid of the reaction solution by MALDI-TOF/MS to confirm [M(average)+H]⁺=2085.7 (theoretical value: [M(average)+H]⁺=2086.1) derived from compound (21) .

To the obtained undried compound (71), a mixture solution (total amount: 500µl) containing 50µl of 500mMHEPES buffer solution (pH 7.0), 25µl of 4U/ml human-derived β1,4-galactosyltransferase (available from TOYOBO CO., LTD.), 50µl of 1% bovine serum albumin (BSA, available from SIGMA) aqueous solution, 50µl of 100mM manganese chloride, 50µl of 50mM uridine-5'-disodium diphosphogalactose (UDP-Gal) and 275µl of Milli Qwaterwas added, and the solution was stirred for two hours at 25°C. Thereafter, a mixture solution (total amount: 100µl) containing 10µl of 500mMHEPES buffer solution (pH 7.0), 12µl of 3.7U/ml recombinant rat α2,3-(N)-sialyltransferase (available from Calbiochem), 12µl of 0.88U/ml recombinant rat α2, 3- (O)-sialyltransferase (available fromCalbiochem), 60µl of 50mM cytidine-5'-sodium monophosphosialate (CMP-NANA) and 6µl of Milli Q water was added to the reaction solution and the solution was stirred for 18 hours. Thereafter, 12µl of 0.88U/ml recombinant rat α2, 3- (O) -sialyltransferase (available from Calbiochem) was further added, and the solution was stirred for three hours. Thereafter, 150µl of slurry of the reaction solution was separated by filtration with a filter. The obtained resin was washed in 25mM HEPES buffer solution (pH 7.0), 50% acetonitrile aqueous solution, and further in 25mM HEPES buffer solution (pH 7.0). A part of the resin was slurried in 50µl of 25mM HEPES buffer solution (pH 7.0). 3µl of 1.74mg/ml solution of BLase (available from Shionogi & Co., Ltd.) was added thereto, and the solution was stirred. The supernatant liquid of the reaction solution was analyzed by MALDI-TOF/MS to confirm [M(average)+H]⁺=2247.6 (theoretical value: [M(average)+H]⁺=2248.2) derived from compound (24), [M(average)+H]⁺=2538.8 (theoretical value: [M(average)+H]⁺=2539.5) derived from compound (77) or (78), and [M(average)+H]⁺=2830.1 (theoretical value: [M(average)+H]⁺=2830.8) derived from compound (25).

(3.15 Synthesis of compounds (79) to (81) using a one-pot reaction)

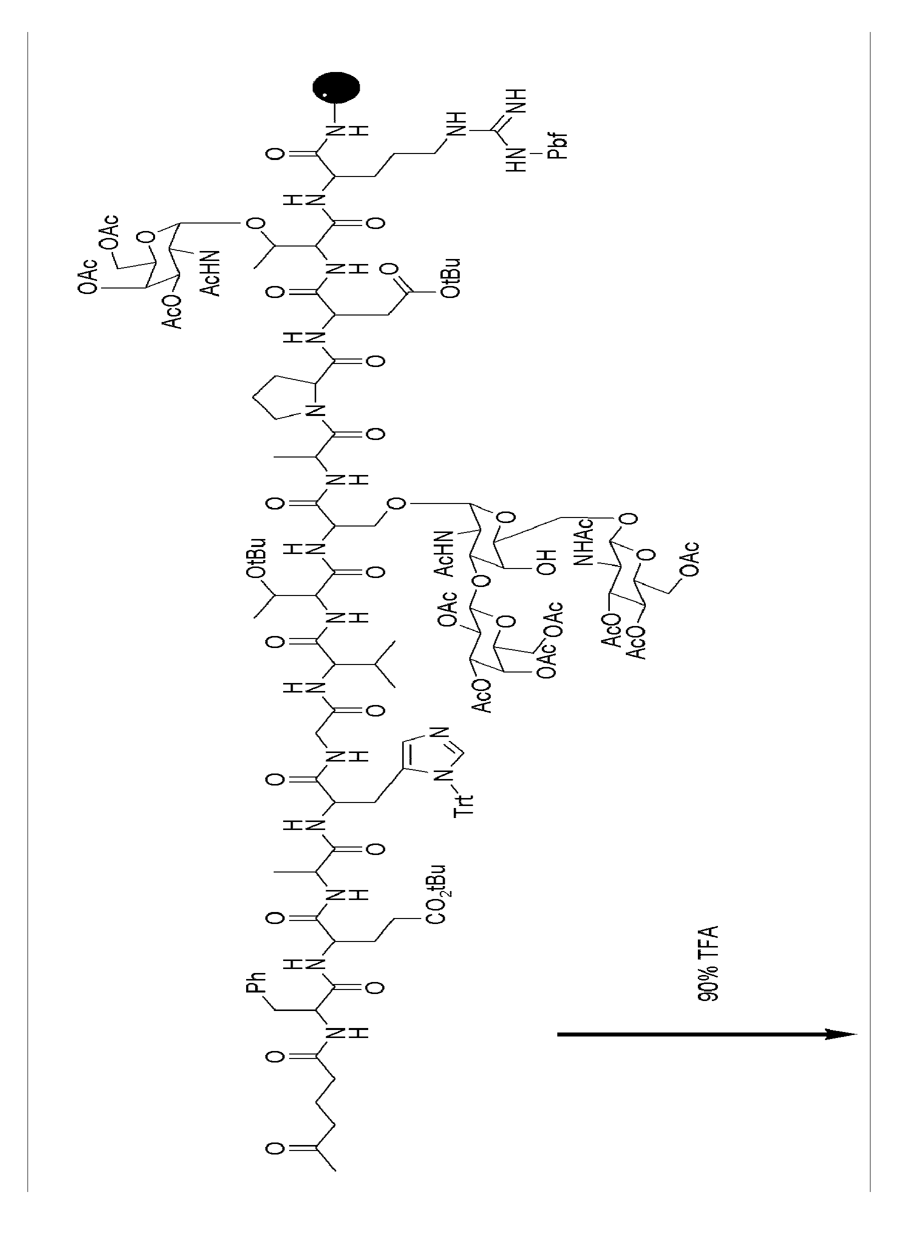

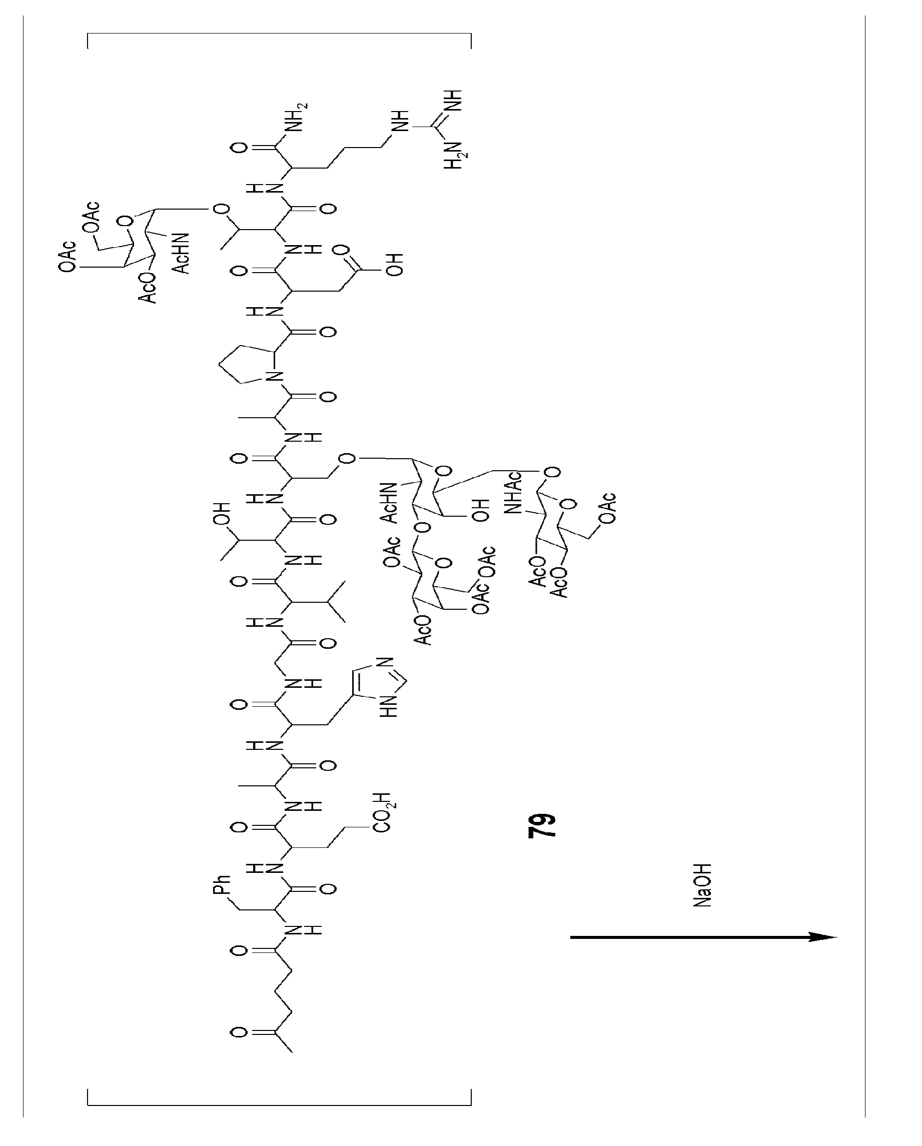

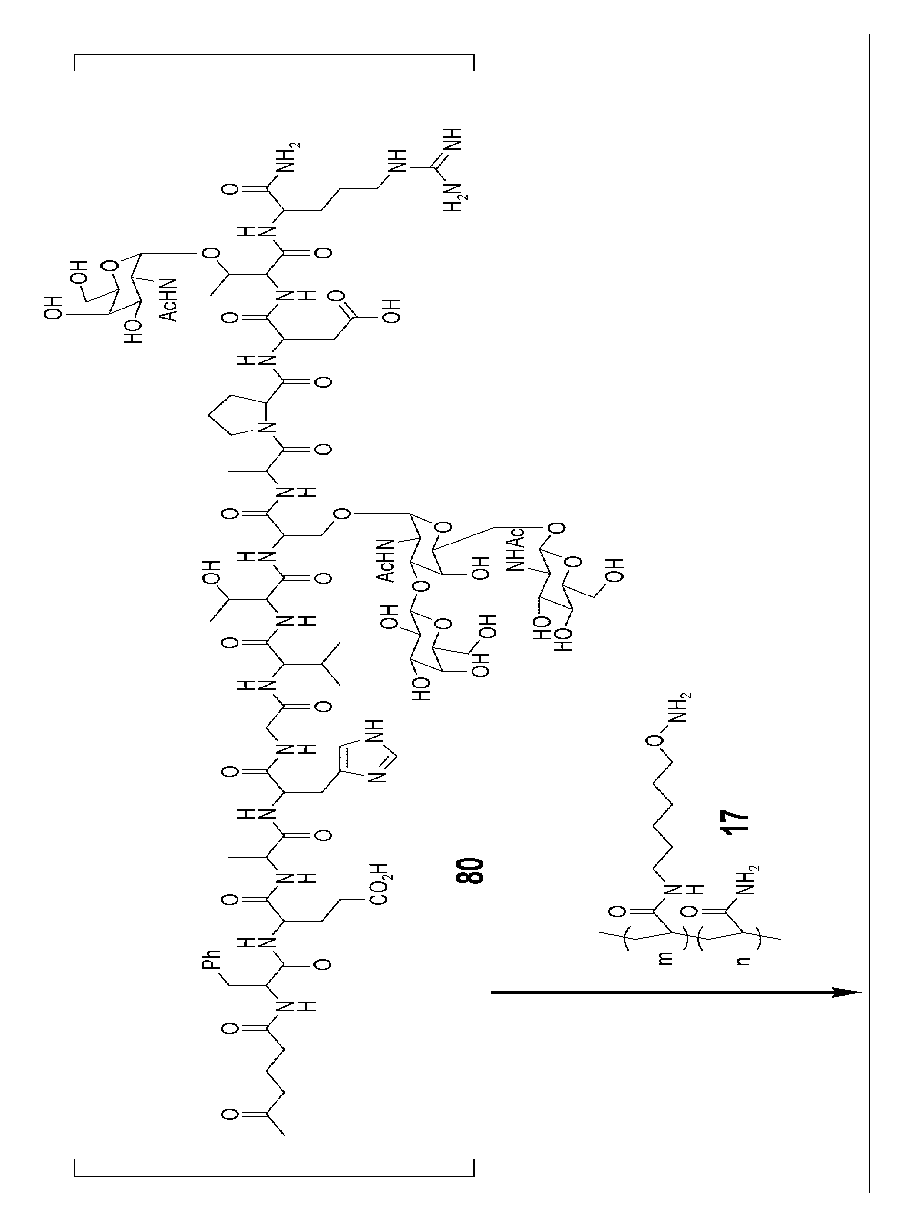

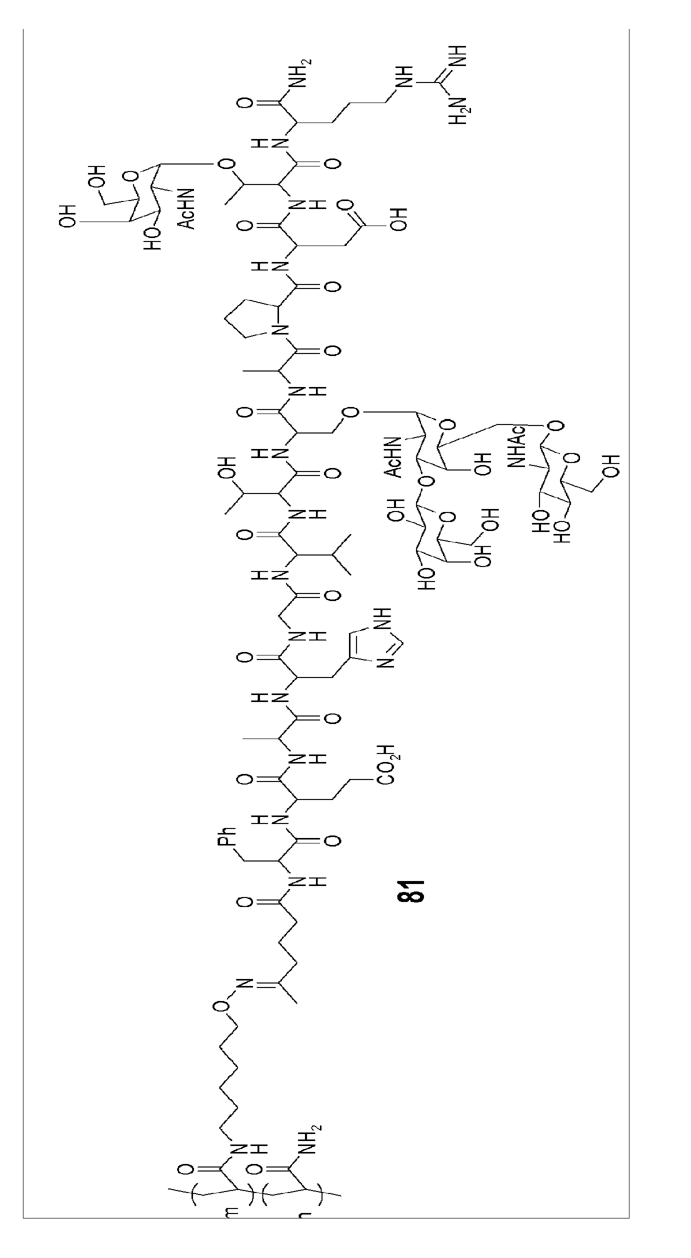

Using 0.12g (0.03mmol) of Tentagel® S RAM resin (Hipep Laboratories, 0.25mmol/g) as a support, N-protected amino acids and keto acid as describedbelowwere sequentially condensed by Fmoc/HBTU/HOBt method, thereby synthesizing a glycopeptide derivative of interest: Fmoc-Arg(Pbf)-OH; Fmoc-Thr(Ac3GalNAc)-OH; Fmoc-Asp(OtBu)-OH; Fmoc-Pro-OH; Fmoc-Ala-OH; Fmoc-Ser(Ac7core2)-OH; Fmoc-Thr(OtBu)-OH; Fmoc-Val-OH; Fmoc-Gly-OH; Fmoc-His(Trt)-OH; Fmoc-Ala-OH; Fmoc-Glu(OtBu)-OH; Fmoc-Phe-OH; and 5-ketohexane acid. After the peptide elongation reaction, the obtained resin equivalent to 0.01mmol was allowed to react in 90% TFA aqueous solution for 2.5 hours at room temperature to eliminate the protective group on the peptide residue and concurrently release compound (79) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 3.0ml of methanol. 1N sodium hydroxide aqueous solution was added to this solution to adjust so that the pH was 12 to 12.5. The solution was stirred for three hours at room temperature, thereby performing Ac deprotection reaction. After the reaction, 1N aqueous solution of acetic acid was added and the solution was neutralized. The solvent was removed and the residue was dissolved in 1ml of 50mM acetic acid/sodium acetate buffer solution (pH 5.5). 1ml of 10mM (oxyamine residue calculation) water-soluble polymer (17) aqueous solution was added to the solution, and the solution was stirred for 24 hours at room temperature, thereby reacting compound (80) with compound (17). After completion of the reaction, the reaction solution was subjected to centrifugal concentration with ultrafiltration filter 10K Apollp® 20ml (Orbital Biosciences, available from LIC). 25mM HEPES buffer solution (pH 7.0) was added thereto and the solution was again subjected to concentration, thereby washing. By adding water so that the final amount is 1.0ml, 10mM (theoretical content of glycopeptide) polymer (81) was obtained. Identification of polymer (81) was performed based on that a product (102) was obtained in the following (3.21) .

(3.16 Synthesis of compounds (82) to (84) using a one-pot reaction)

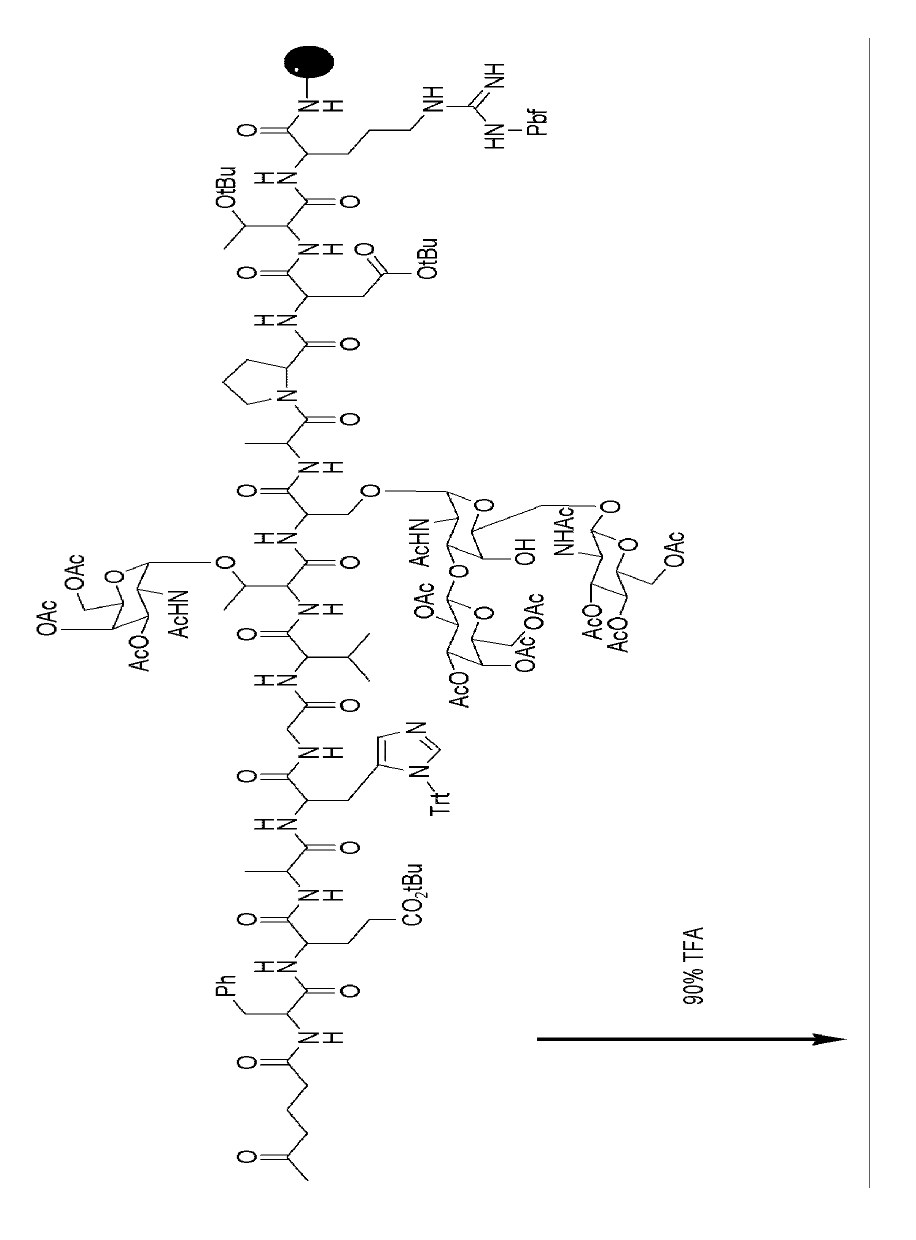

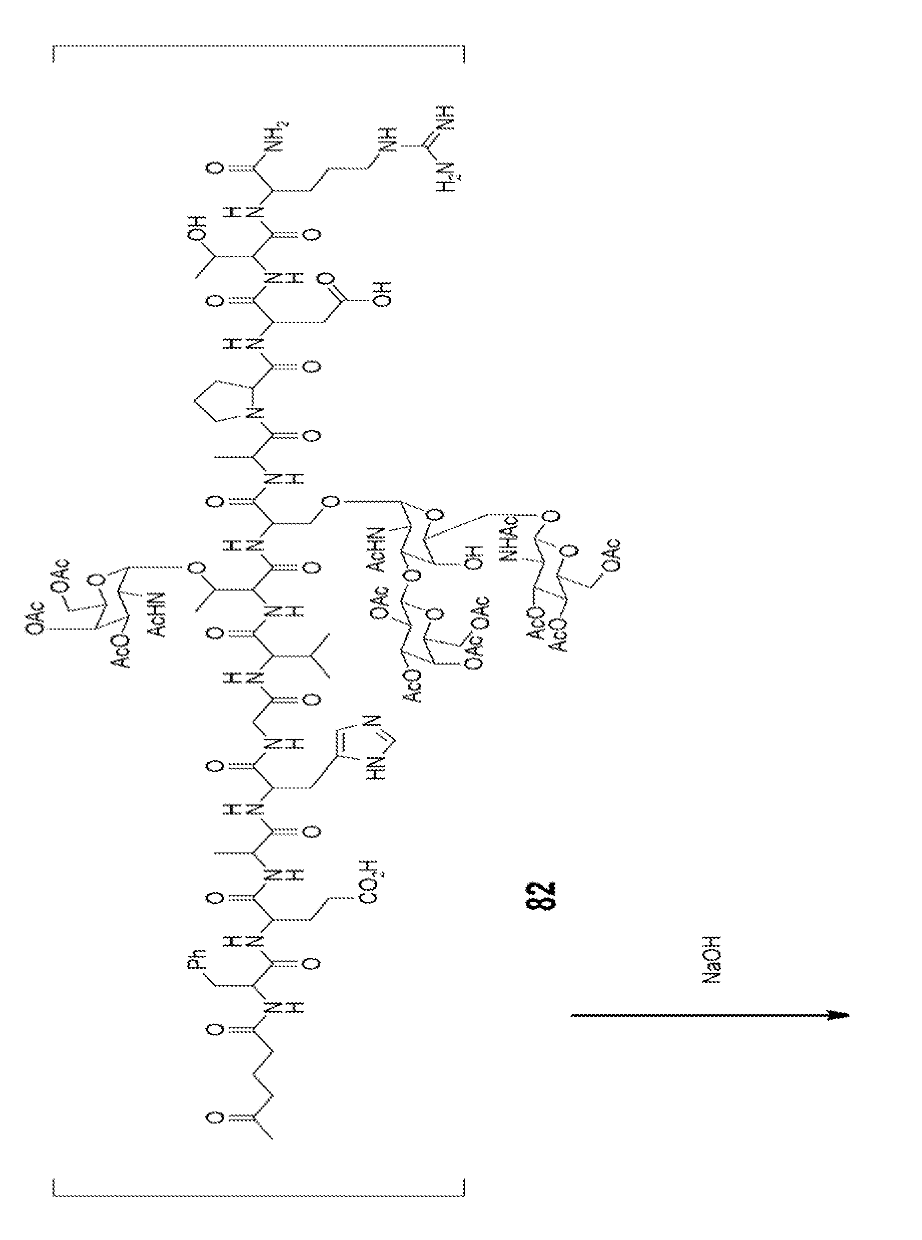

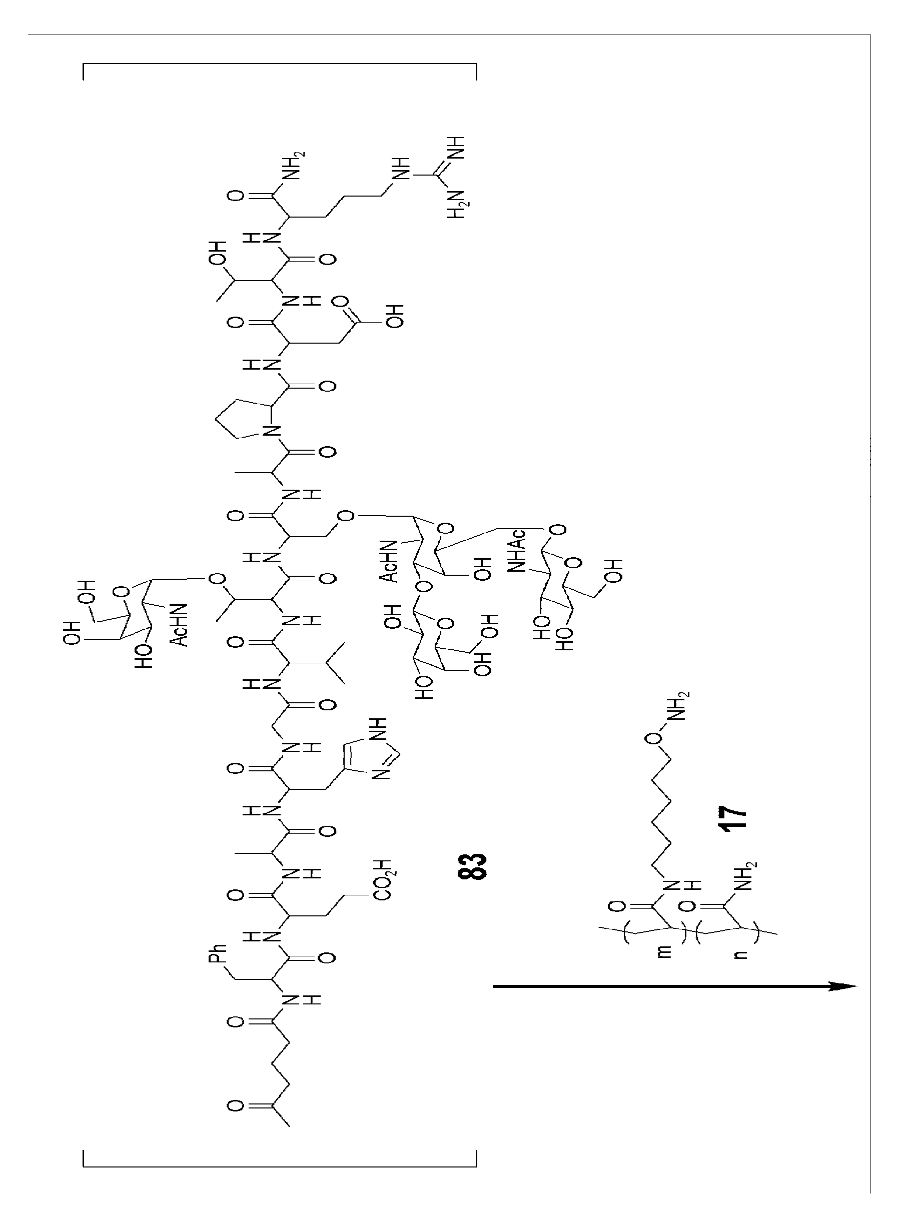

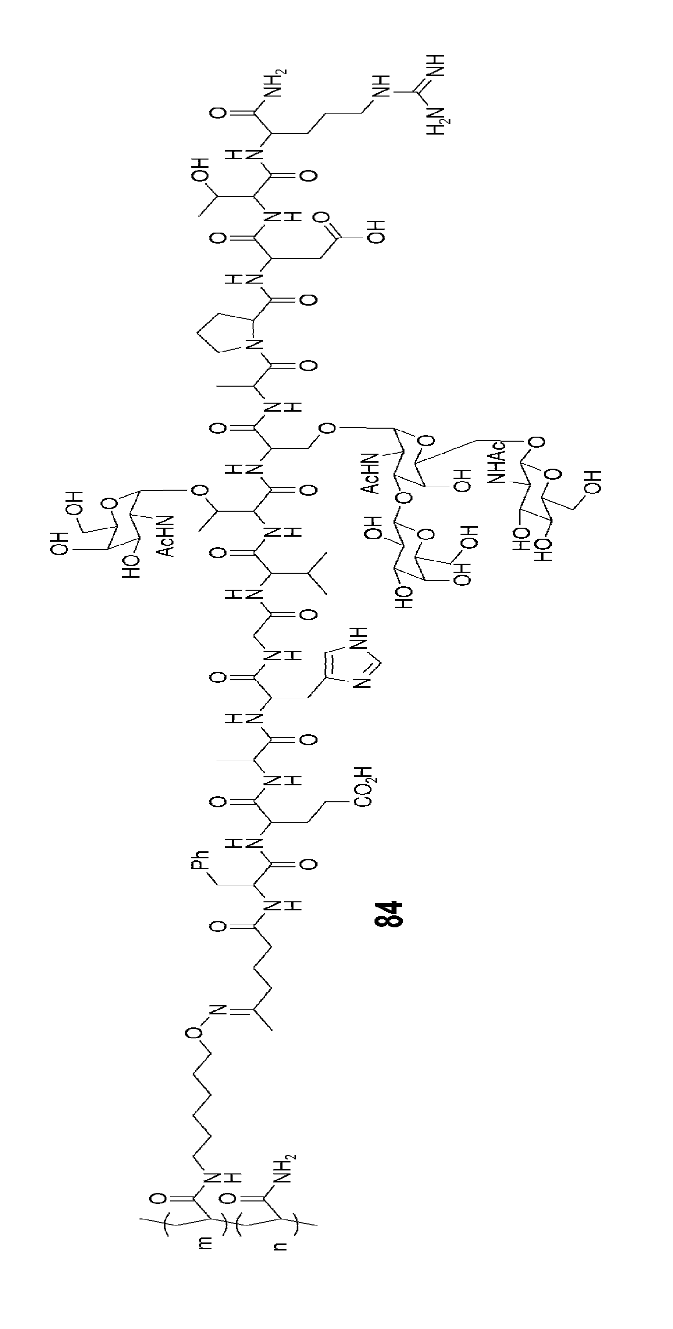

Using 0.12g (0.03mmol) of Tentagel® S RAM resin (Hipep Laboratories, 0.25mmol/g) as a support, N-protected amino acids and keto acid as describedbelowwere sequentially condensed by Fmoc/HBTU/HOBt method, thereby synthesizing a glycopeptide derivative of interest: Fmoc-Arg(Pbf)-OH; Fmoc-Thr(OtBu)-OH; Fmoc-Asp(OtBu)-OH; Fmoc-Pro-OH; Fmoc-Ala-OH; Fmoc-Ser(Ac7core2)-OH; Fmoc-Thr(Ac3GalNAc)-OH; Fmoc-Val-OH; Fmoc-Gly-OH; Fmoc-His(Trt)-OH; Fmoc-Ala-OH; Fmoc-Glu(OtBu)-OH; Fmoc-Phe-OH; and 5-ketohexane acid. After the peptide elongation reaction, the obtained resin equivalent to 0.01mmol was allowed to react in 90% TFA aqueous solution for 2.5 hours at room temperature to eliminate the protective group on the peptide residue and concurrently release compound (82) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether was again added, and the precipitate was washed. Centrifugal separation was again performed and the supernatant was removed. The obtained precipitant was dissolved in 3.0ml of methanol. 1N sodium hydroxide aqueous solution was added to this solution to adjust so that the pH was 12 to 12.5. The solution was stirred for three hours at room temperature, thereby performing Ac deprotection reaction. After the reaction, 1N aqueous solution of acetic acid was added and the solution was neutralized. The solvent was removed and the residue was dissolved in 1ml of 50mM acetic acid/sodium acetate buffer solution (pH 5.5). 1ml of 10mM (oxyamine residue calculation) water-soluble polymer (17) aqueous solution was added to the solution, and the solution was stirred for 24 hours at room temperature, thereby reacting compound (83) with compound (17) . After completion of the reaction, the reaction solution was subjected to centrifugal concentration with ultrafiltration filter 10K Apollp® 20ml (Orbital Biosciences, available from LIC). 25mM HEPES buffer solution (pH 7.0) was added thereto and the solution was again subjected to concentration, thereby washing. By adding water so that the final amount is 1.0ml, 10mM (theoretical content of glycopeptide) polymer (84) was obtained. Identification of polymer (84) was performed based on that a product (113) was obtained in the following (3.22).

(3.17 Synthesis of compounds (85) to (87) using a one-pot reaction)

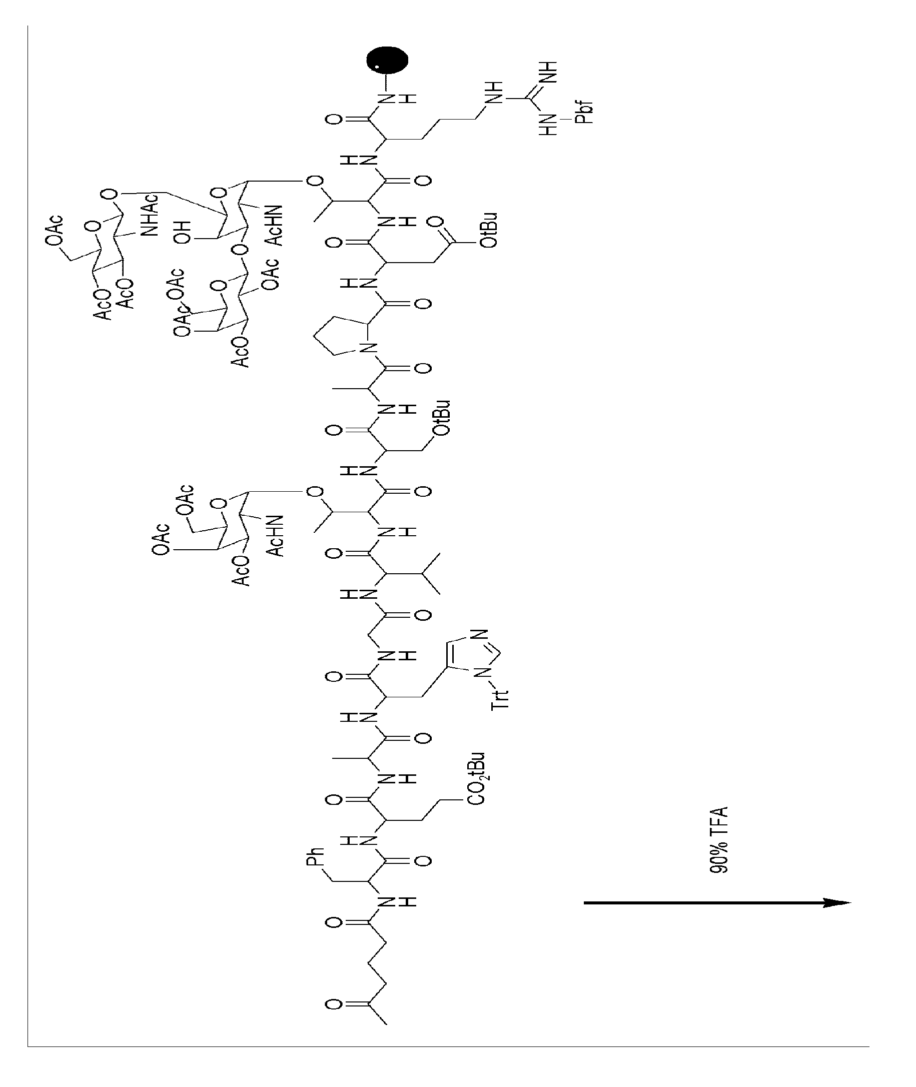

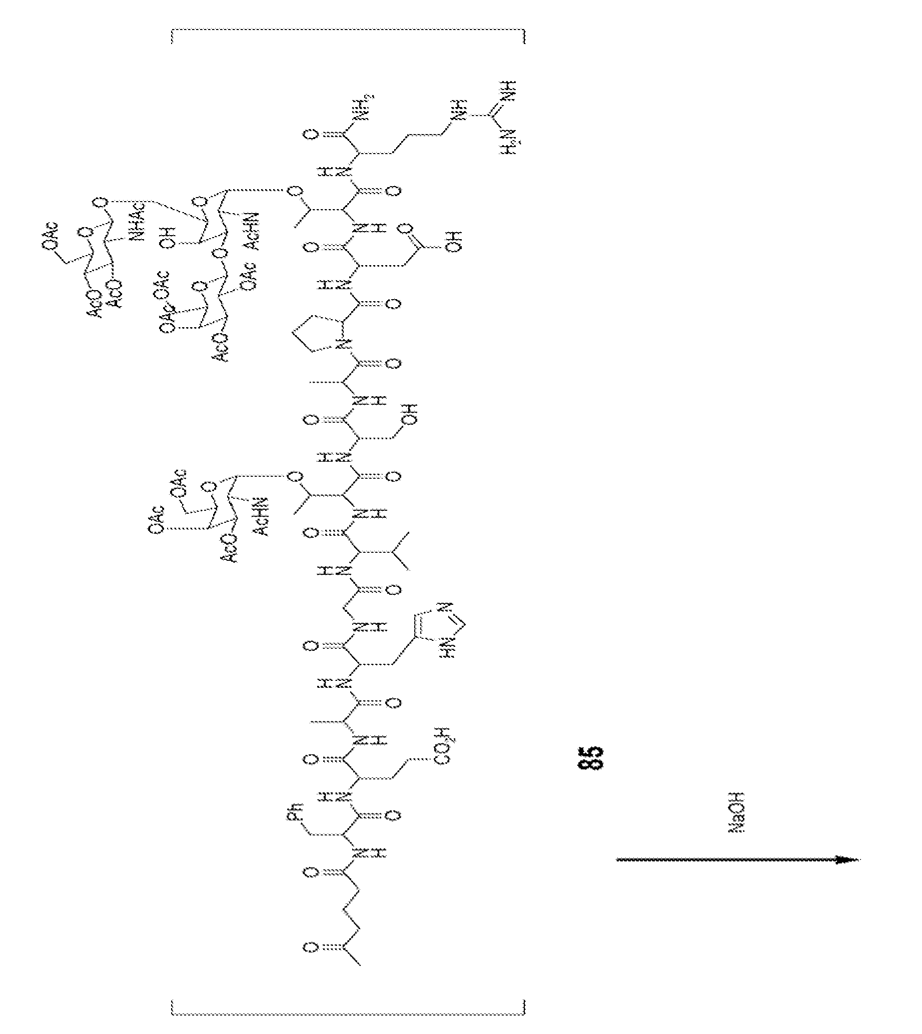

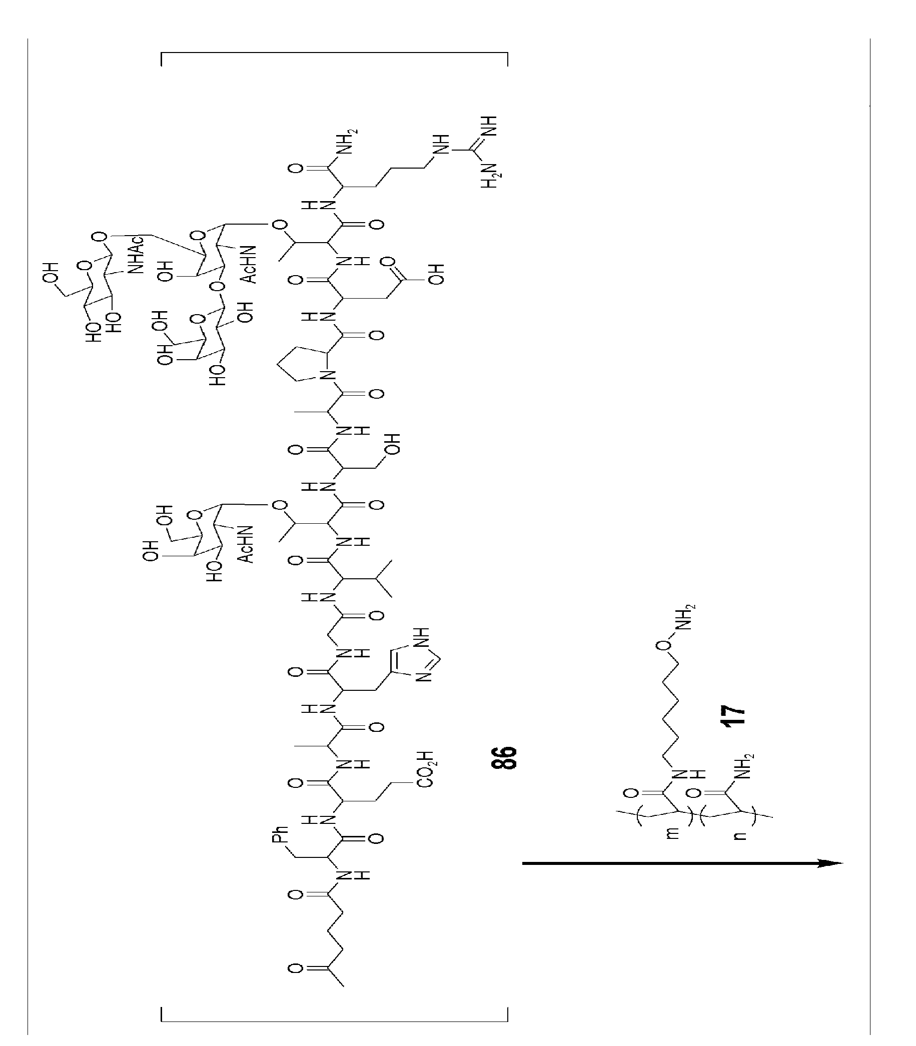

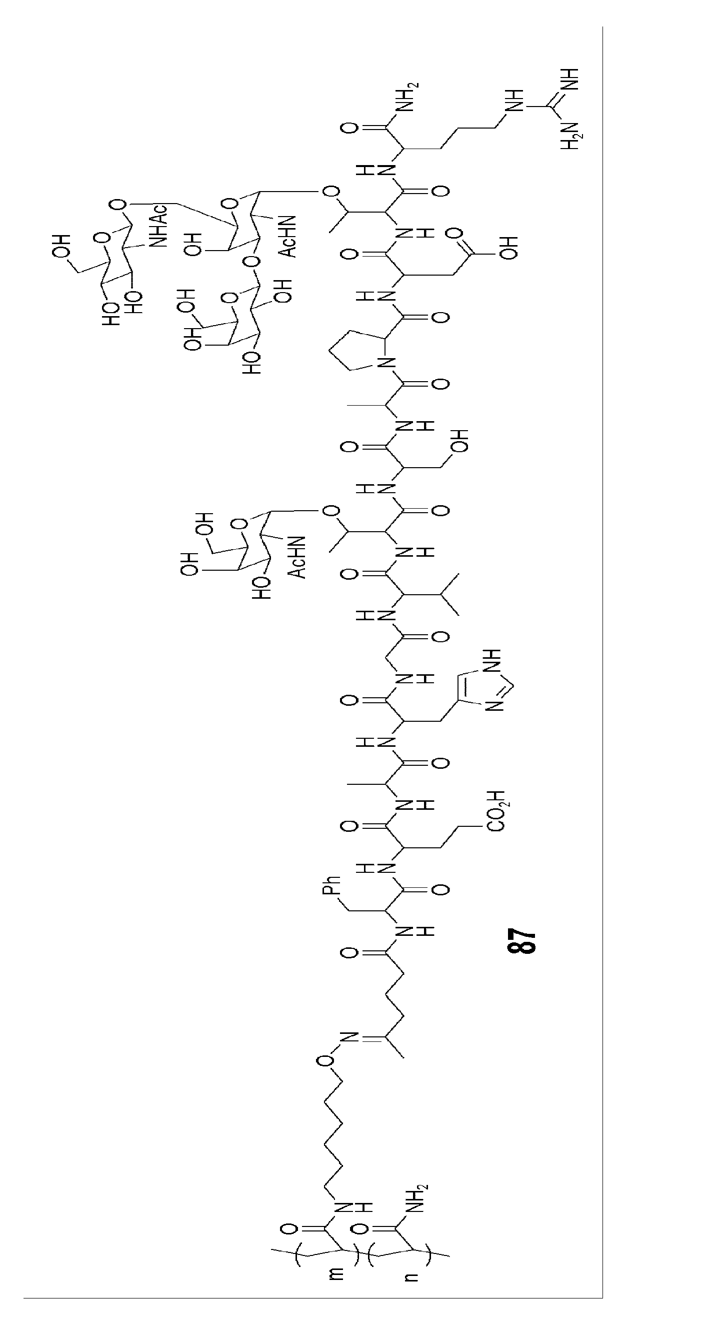

Using 0.12g (0.03mmol) of Tentagel® S RAM resin (Hipep Laboratories, 0.25mmol/g) as a support, N-protected amino acids and keto acid as describedbelowwere sequentially condensed by Fmoc/HBTU/HOBt method, thereby synthesizing a glycopeptide derivative of interest: Fmoc-Arg(Pbf)-OH; Fmoc-Thr(Ac7core2)-OH; Fmoc-Asp(OtBu)-OH; Fmoc-Pro-OH; Fmoc-Ala-OH; Fmoc-Ser(tBu)-OH; Fmoc-Thr(Ac3GalNAc)-OH; Fmoc-Val-OH; Fmoc-Gly-OH; Fmoc-His(Trt)-OH; Fmoc-Ala-OH; Fmoc-Glu(OtBu)-OH; Fmoc-Phe-OH; and 5-ketohexane acid. After the peptide elongation reaction, the obtained resin equivalent to 0.01mmol was allowed to react in 90% TFA aqueous solution for 2.5 hours at room temperature to eliminate the protective group on the peptide residue and concurrently release compound (85) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 3.0ml of methanol. 1N sodium hydroxide aqueous solution was added to this solution to adjust so that the pH was 12 to 12.5. The solution was stirred for three hours at room temperature, thereby performing Ac deprotection reaction. After the reaction, 1N aqueous solution of acetic acid was added and the solution was neutralized. The solvent was removed and the residue was dissolved in 1ml of 50mM acetic acid/sodium acetate buffer solution (pH 5.5). 1ml of 10mM (oxyamine residue calculation) water-soluble polymer (17) aqueous solution was added to the solution, and the solution was stirred for 24 hours at room temperature, thereby reacting compound (86) with compound (17). After completion of the reaction, the reaction solution was subjected to centrifugal concentration with ultrafiltration filter 10K Apollp® 20ml (Orbital Biosciences, available from LIC). 25mM HEPES buffer solution (pH 7.0) was added thereto and the solution was again subjected to concentration, thereby washing. By adding water so that the final amount is 1.0ml, 10mM (theoretical content of glycopeptide) polymer (87) was obtained. Identification of polymer (87) was performed based on that a product (124) was obtained in the following (3.23).

(3.18 Synthesis of compounds (88) to (90) using a one-pot reaction)

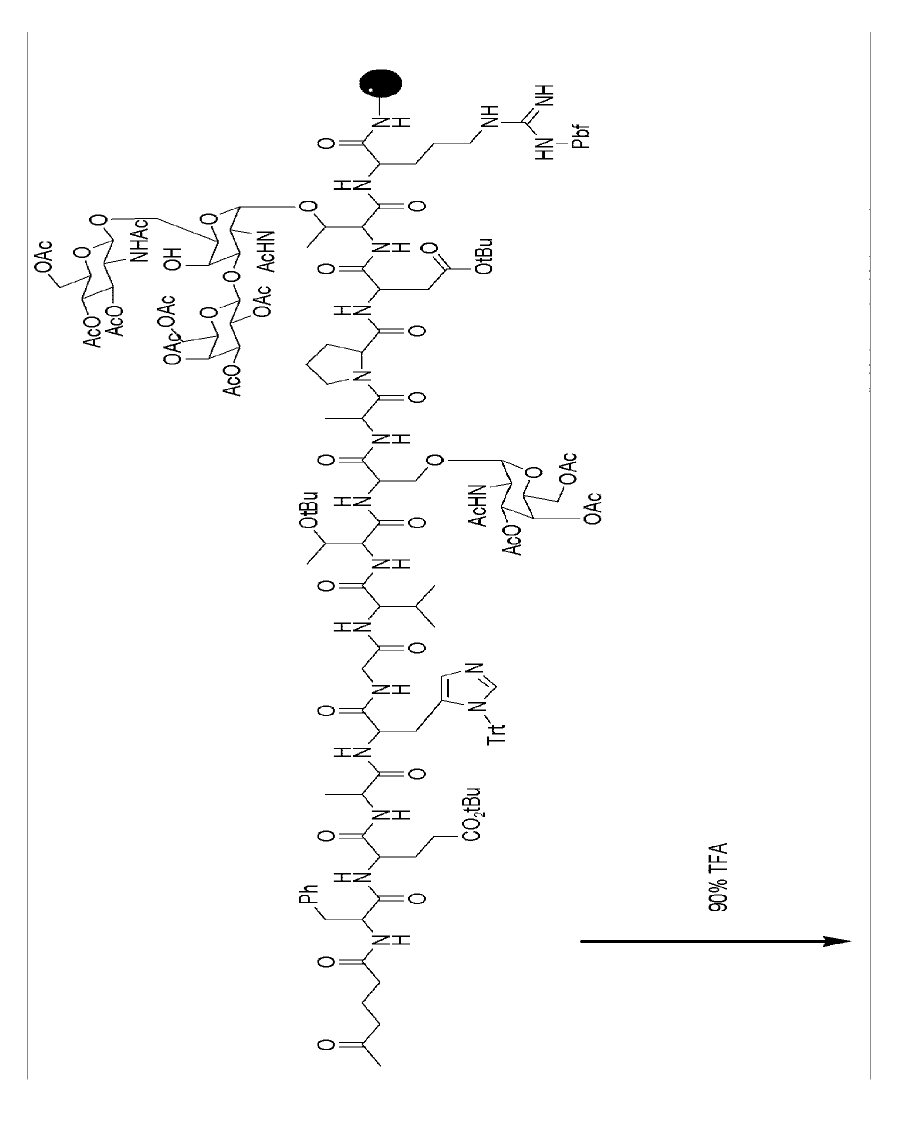

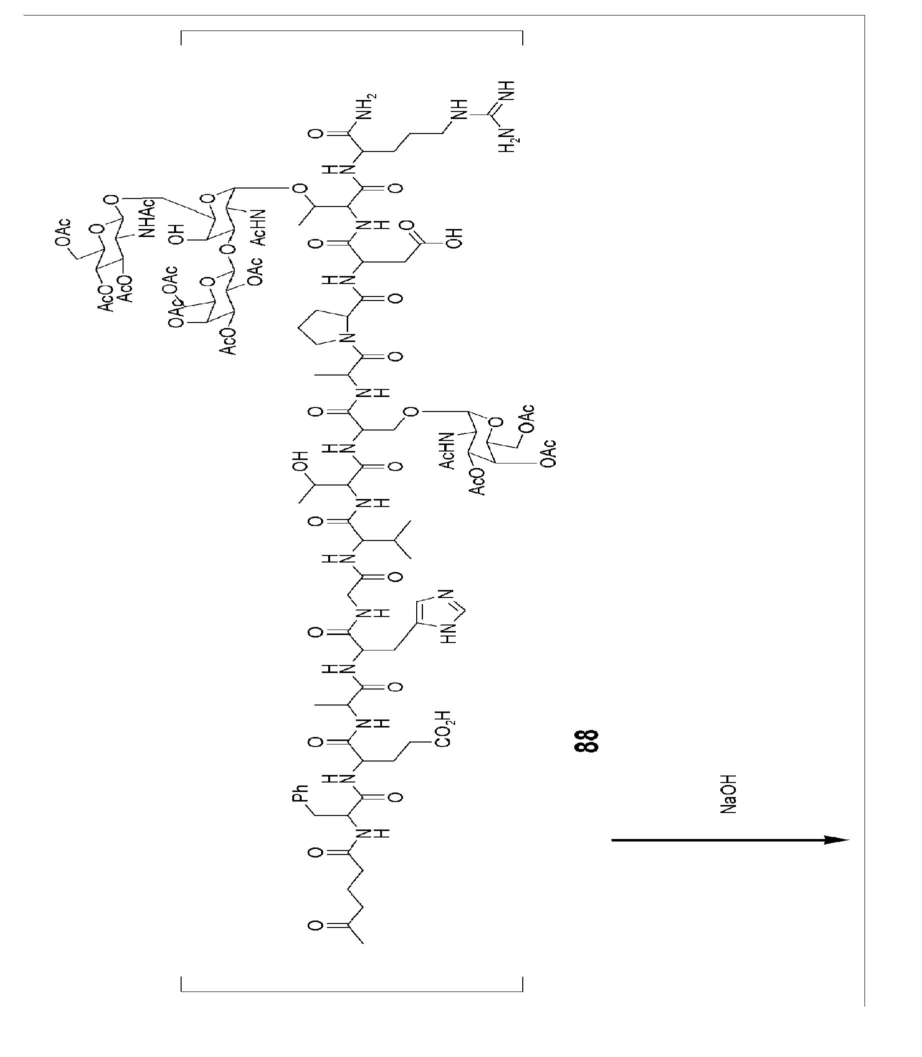

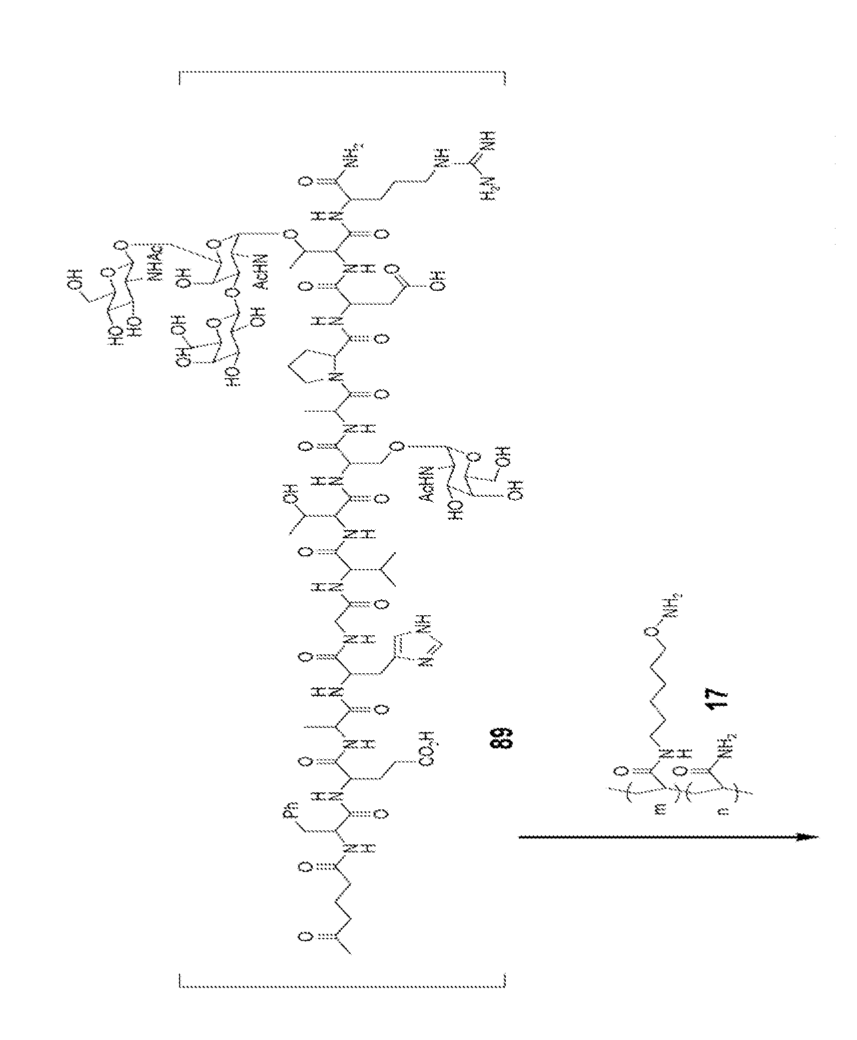

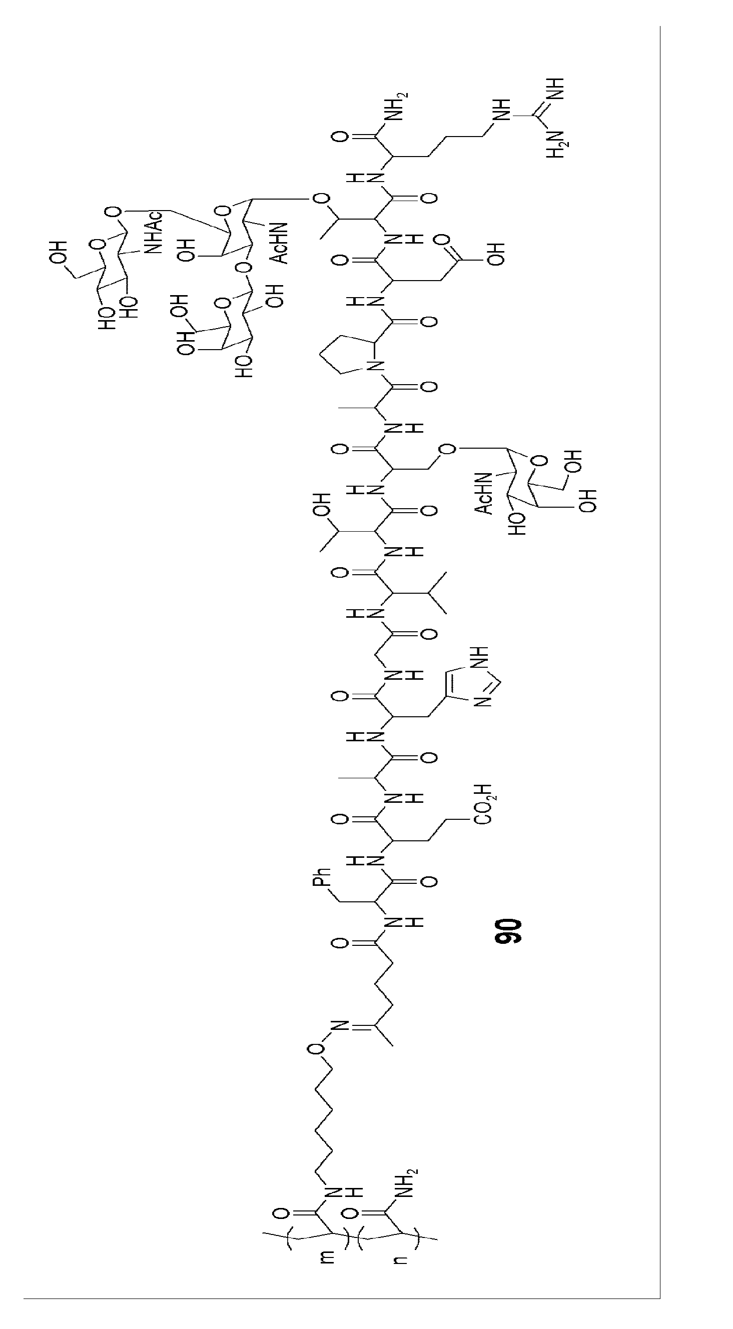

Using 0.12g (0.03mmol) of Tentagel® S RAM resin (Hipep Laboratories, 0.25mmol/g) as a support, N-protected amino acids and keto acid as describedbelowwere sequentially condensed by Fmoc/HBTU/HOBt method, thereby synthesizing a glycopeptide derivative of interest: Fmoc-Arg(Pbf)-OH; Fmoc-Thr(Ac7core2)-OH; Fmoc-Asp(OtBu)-OH; Fmoc-Pro-OH; Fmoc-Ala-OH; Fmoc-Ser(Ac3GalNAc)-OH; Fmoc-Thr(tBu)-OH; Fmoc-Val-OH; Fmoc-Gly-OH; Fmoc-His(Trt)-OH; Fmoc-Ala-OH; Fmoc-Glu(OtBu)-OH; Fmoc-Phe-OH; and 5-ketohexane acid. After the peptide elongation reaction, the obtained resin equivalent to 0.01mmol was allowed to react in 90% TFA aqueous solution for 2.5 hours at room temperature to eliminate the protective group on the peptide residue and concurrently release compound (88) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 3.0ml of methanol. 1N sodium hydroxide aqueous solution was added to this solution to adjust so that the pH was 12 to 12.5. The solution was stirred for two hours at room temperature, thereby performing Ac deprotection reaction. After the reaction, 1N aqueous solution of acetic acid was added and the solution was neutralized. The solvent was removed and the residue was dissolved in 1ml of 50mM acetic acid/sodium acetate buffer solution (pH 5.5). 1ml of 10mM (oxyamine residue calculation) water-soluble polymer (17) aqueous solution was added to the solution, and the solution was stirred for 24 hours at room temperature, thereby reacting compound (89) with compound (17). After completion of the reaction, the reaction solution was subjected to centrifugal concentration with ultrafiltration filter 10K Apollp® 20ml (Orbital Biosciences, available from LIC). 25mM HEPES buffer solution (pH 7.0) was added thereto and the solution was again subjected to concentration, thereby washing. By adding water so that the final amount is 1.0ml, 10mM (theoretical content of glycopeptide) polymer (90) was obtained. Identification of polymer (90) was performed based on that a product (135) was obtained in the following (3.24).

(3.19 Synthesis of compounds (91) to (93) using a one-pot reaction)

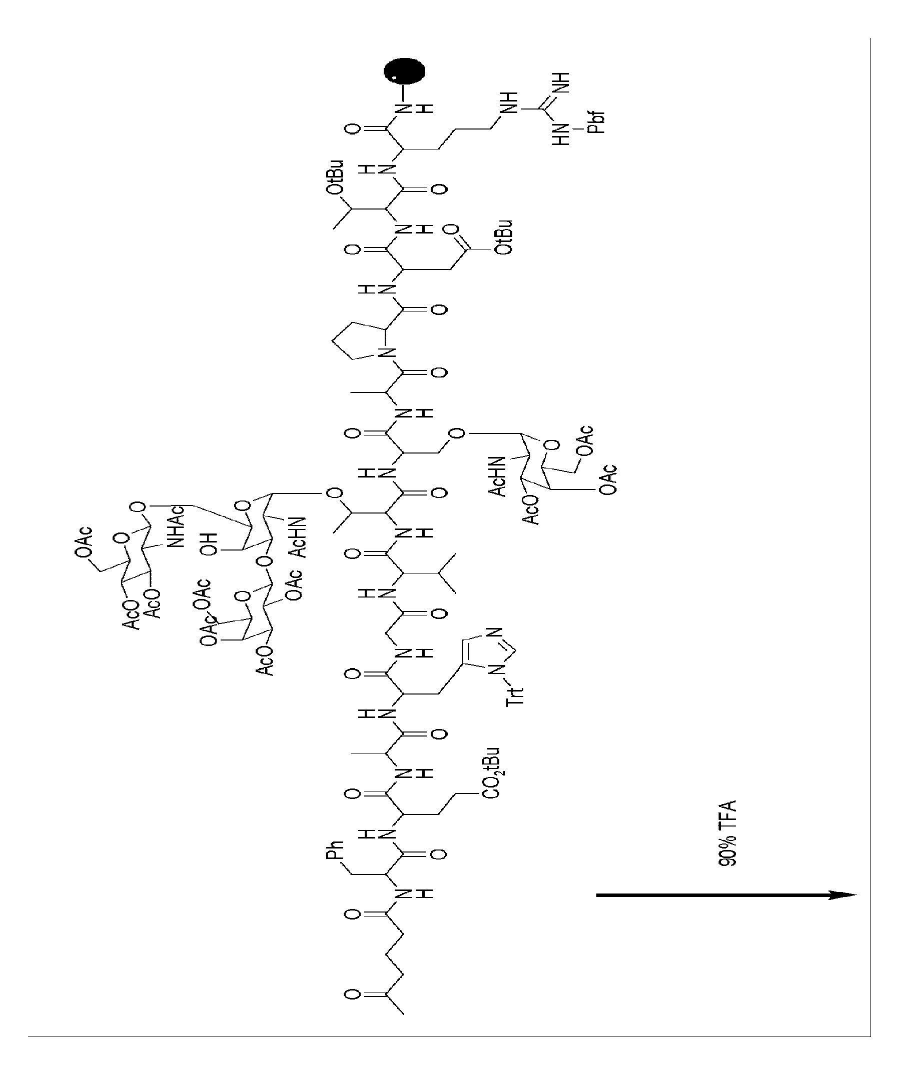

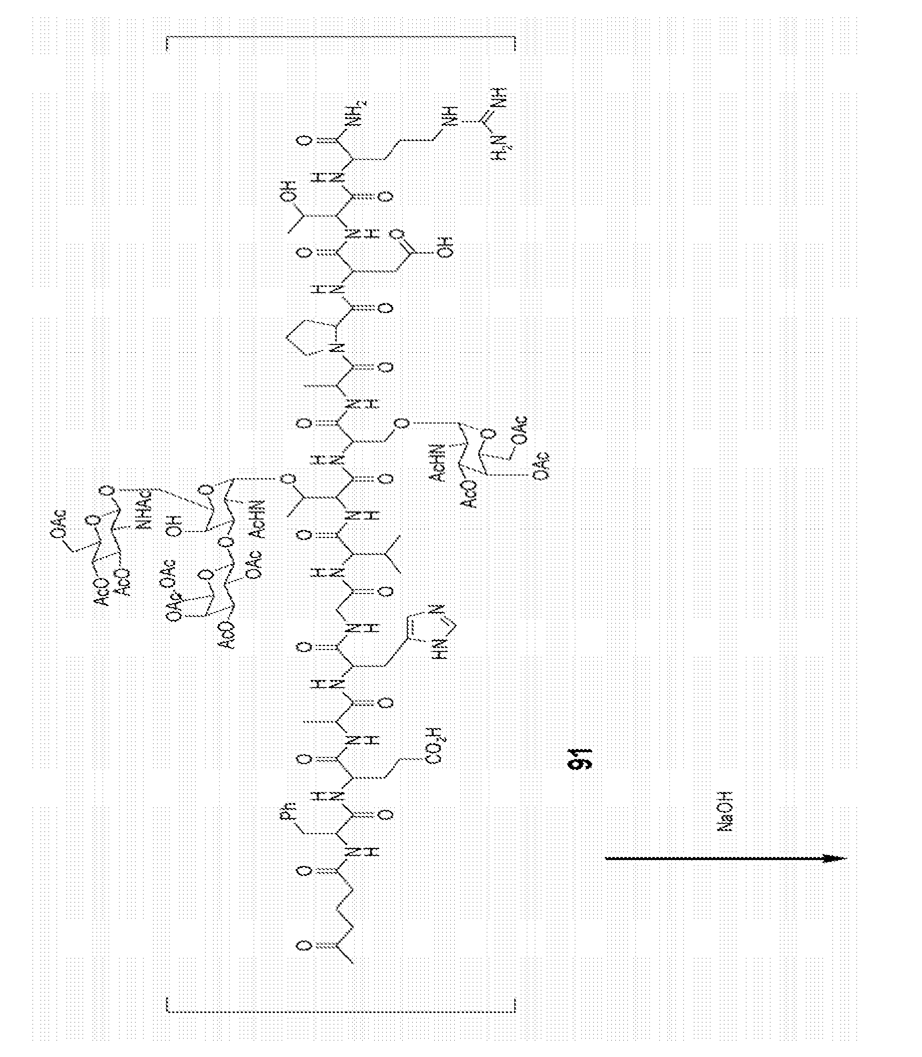

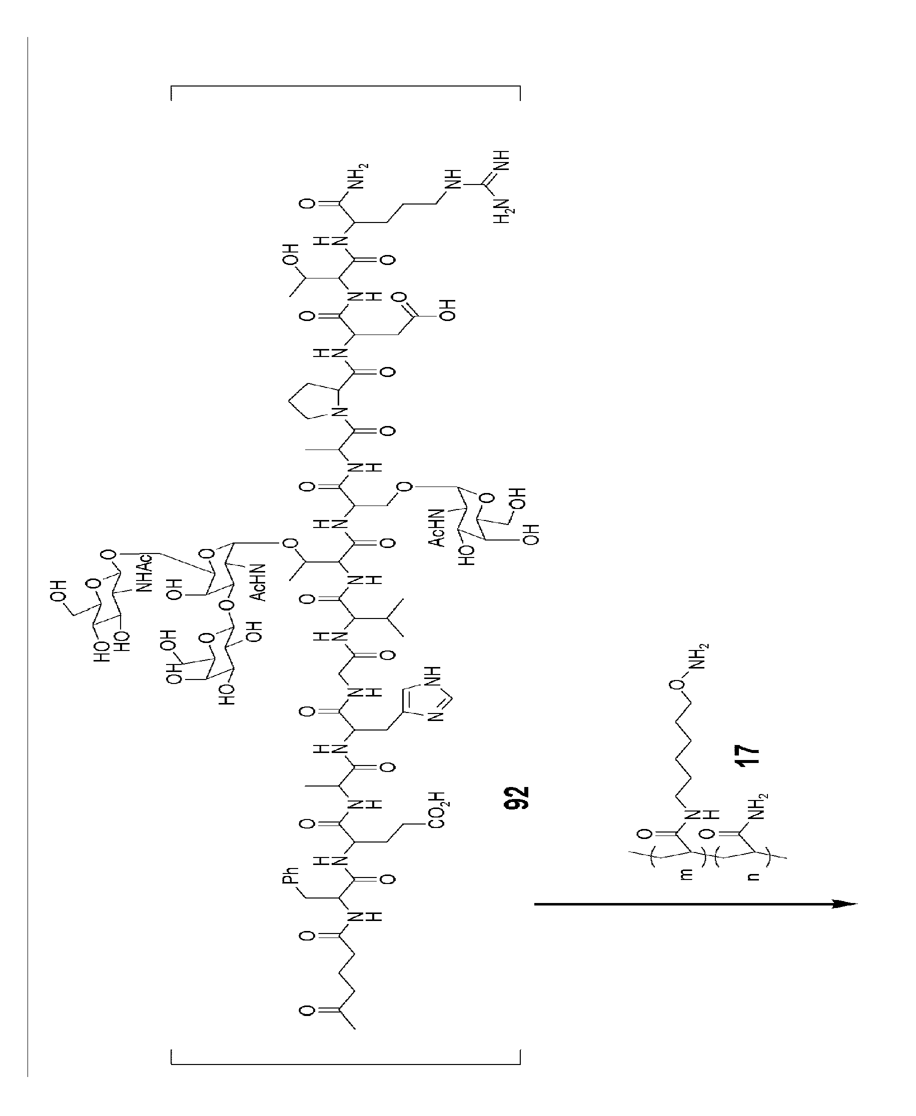

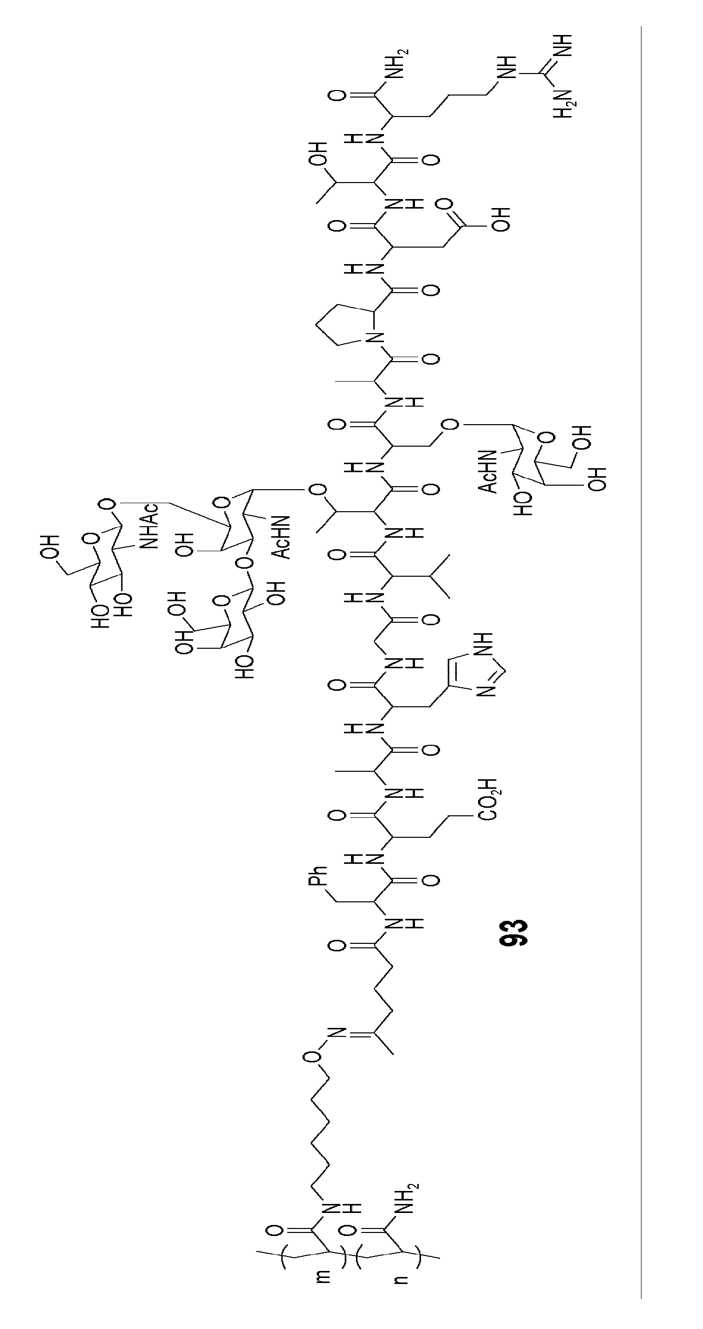

Using 0.12g (0.03mmol) of Tentagel® S RAM resin (Hipep Laboratories, 0.25mmol/g) as a support, N-protected amino acids and keto acid as describedbelowwere sequentially condensed by Fmoc/HBTU/HOBt method, thereby synthesizing a glycopeptide derivative of interest: Fmoc-Arg(Pbf)-OH; Fmoc-Thr(tBu)-OH; Fmoc-Asp(OtBu)-OH; Fmoc-Pro-OH; Fmoc-Ala-OH; Fmoc-Ser(Ac3GalNAc)-OH; Fmoc-Thr(Ac7core2)-OH; Fmoc-Val-OH; Fmoc-Gly-OH; Fmoc-His(Trt)-OH; Fmoc-Ala-OH; Fmoc-Glu(OtBu)-OH; Fmoc-Phe-OH; and 5-ketohexane acid. After the peptide elongation reaction, the obtained resin equivalent to 0.01mmol was allowed to react in 90% TFA aqueous solution for 2.5 hours at room temperature to eliminate the protective group on the peptide residue and concurrently release compound (91) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether was again added, and the precipitate was washed. Centrifugal separation was again performed and the supernatant was removed. The obtained precipitant was dissolved in 3.0ml of methanol. 1N sodium hydroxide aqueous solution was added to this solution to adjust so that the pH was 12 to 12.5. The solution was stirred for three hours at room temperature, thereby performing Ac deprotection reaction. After the reaction, 1N aqueous solution of acetic acid was added and the solution was neutralized. The solvent was removed and the residue was dissolved in 1ml of 50mM acetic acid/sodium acetate buffer solution (pH 5.5). 1ml of 10mM (oxyamine residue calculation) water-soluble polymer (17) aqueous solution was added to the solution, and the solution was stirred for 24 hours at room temperature, thereby reacting compound (92) with compound (17). After completion of the reaction, the reaction solution was subjected to centrifugal concentration with ultrafiltration filter 10K Apollp® 20ml (Orbital Biosciences, available from LIC). 25mM HEPES buffer solution (pH 7.0) was added thereto and the solution was again subjected to concentration, thereby washing. By adding water so that the final amount is 1.0ml, 10mM (theoretical content of glycopeptide) polymer (93) was obtained. Identification of polymer (93) was performed based on that a product (146) was obtained in the following (3.25) .

(3.20 Synthesis of compounds (94) to (96) using a one-pot reaction)

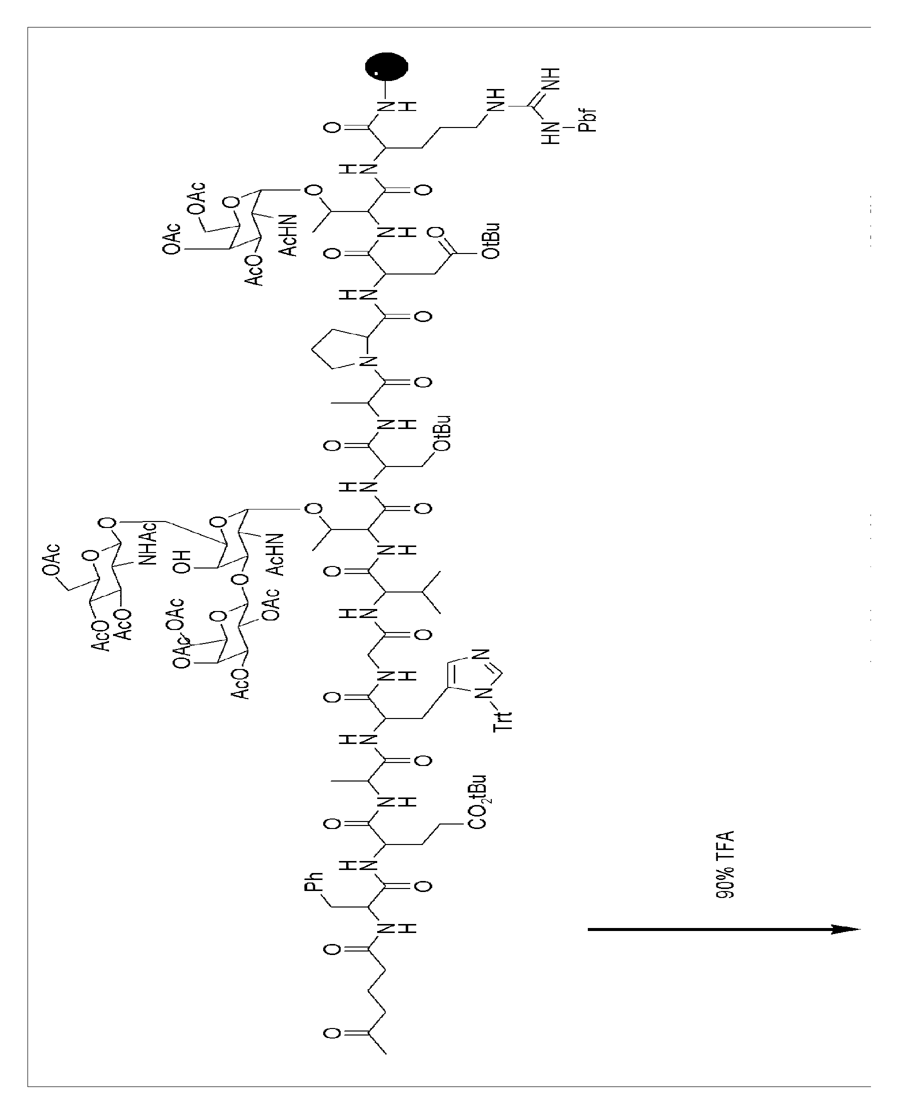

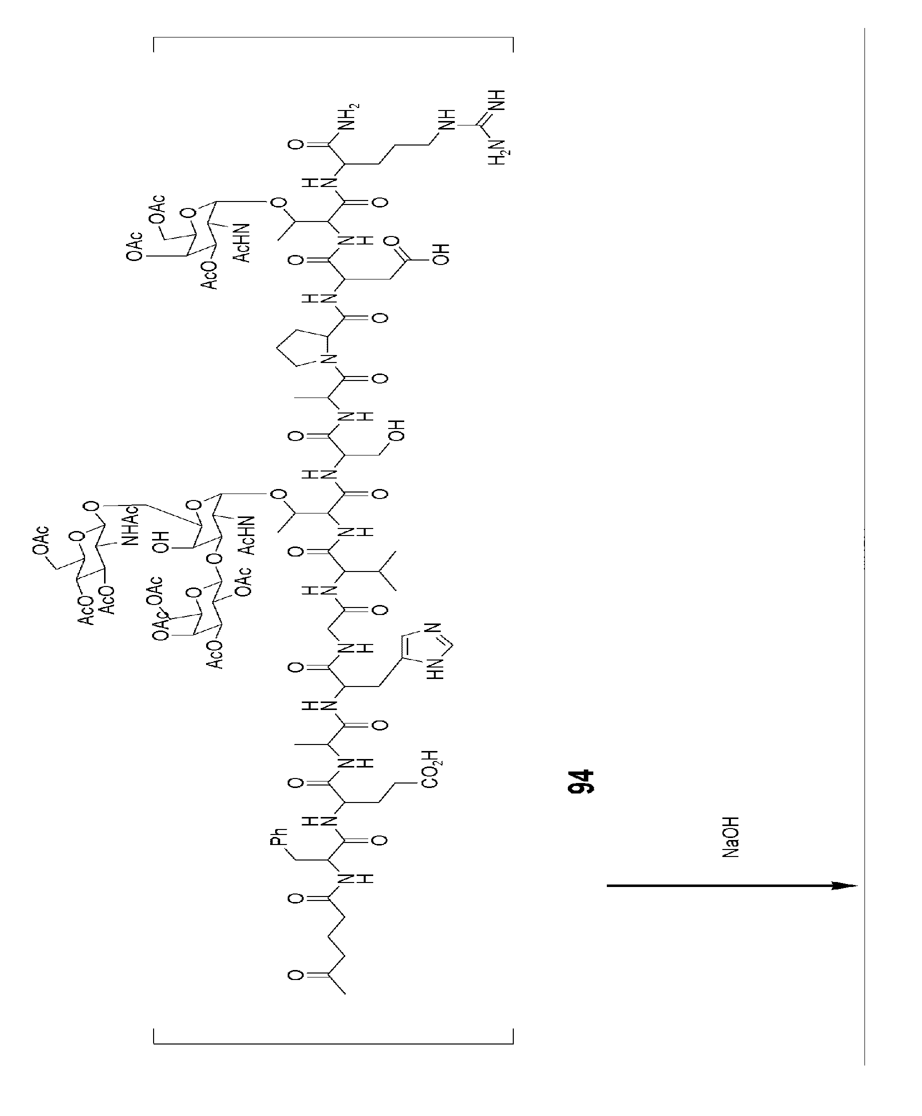

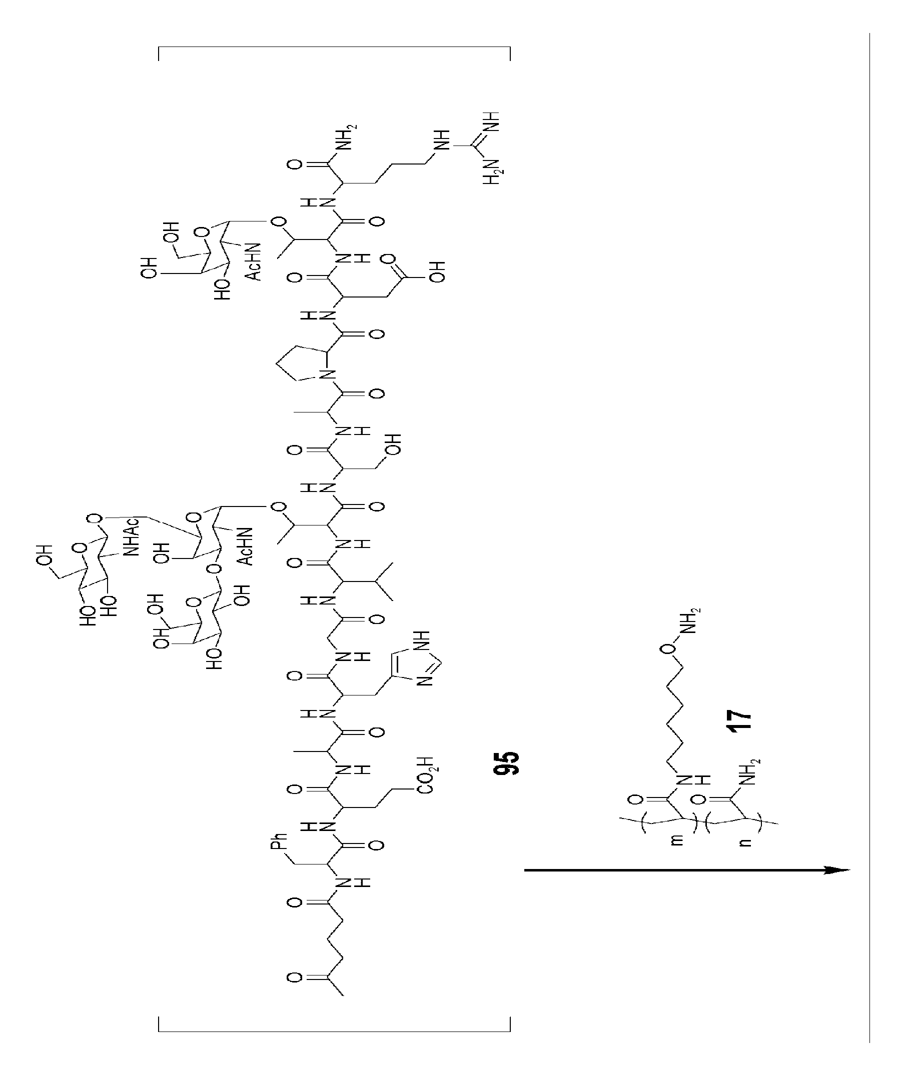

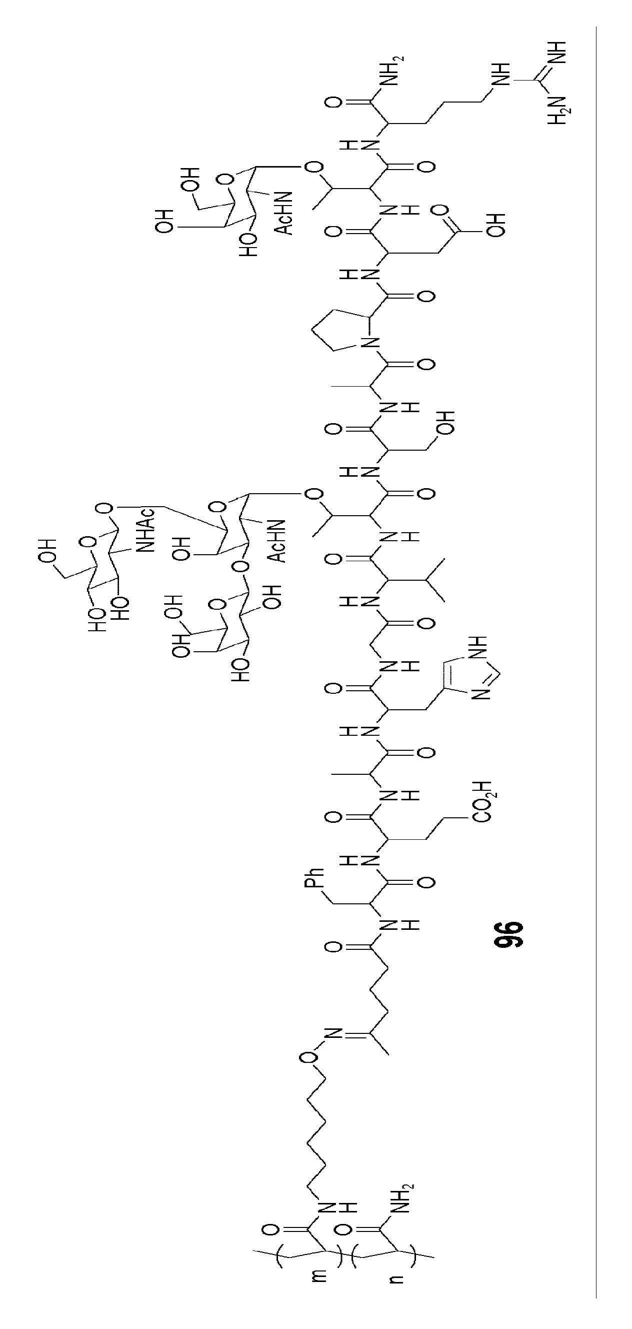

Using 0.12g (0.03mmol) of Tentagel® S RAM resin (Hipep Laboratories, 0.25mmol/g) as a support, N-protected amino acids and keto acid as describedbelowwere sequentially condensed by Fmoc/HBTU/HOBt method, thereby synthesizing a glycopeptide derivative of interest: Fmoc-Arg(Pbf)-OH; Fmoc-Thr(Ac3GalNAc)-OH; Fmoc-Asp(OtBu)-OH; Fmoc-Pro-OH; Fmoc-Ala-OH; Fmoc-Ser(tBu)-OH; Fmoc-Thr(Ac7core2)-OH; Fmoc-Val-OH; Fmoc-Gly-OH; Fmoc-His(Trt)-OH; Fmoc-Ala-OH; Fmoc-Glu(OtBu)-OH; Fmoc-Phe-OH; and 5-ketohexane acid. After the peptide elongation reaction, the obtained resin equivalent to 0.01mmol was allowed to react in 90% TFA aqueous solution for 2.5 hours at room temperature to eliminate the protective group on the peptide residue and concurrently release compound (94) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 3.0ml of methanol. 1N sodium hydroxide aqueous solution was added to this solution to adjust so that the pH was 12 to 12.5. The solution was stirred for three hours at room temperature, thereby performing Ac deprotection reaction. After the reaction, 1N aqueous solution of acetic acid was added and the solution was neutralized. The solvent was removed and the residue was dissolved in 1ml of 50mM acetic acid/sodium acetate buffer solution (pH 5.5). 1ml of 10mM (oxyamine residue calculation) water-soluble polymer (17) aqueous solution was added to the solution, and the solution was stirred for 24 hours at room temperature, thereby reacting compound (95) with compound (17). After completion of the reaction, the reaction solution was subjected to centrifugal concentration with ultrafiltration filter 10K Apollp® 20ml (Orbital Biosciences, available from LIC). 25mM HEPES buffer solution (pH 7.0) was added thereto and the solution was again subjected to concentration, thereby washing. By adding water so that the final amount is 1.0ml, 10mM (theoretical content of glycopeptide) polymer (96) was obtained. Identification of polymer (96) was performed based on that a product (157) was obtained in the following (3.26).

(3.21 Synthesis of (97) to (107))

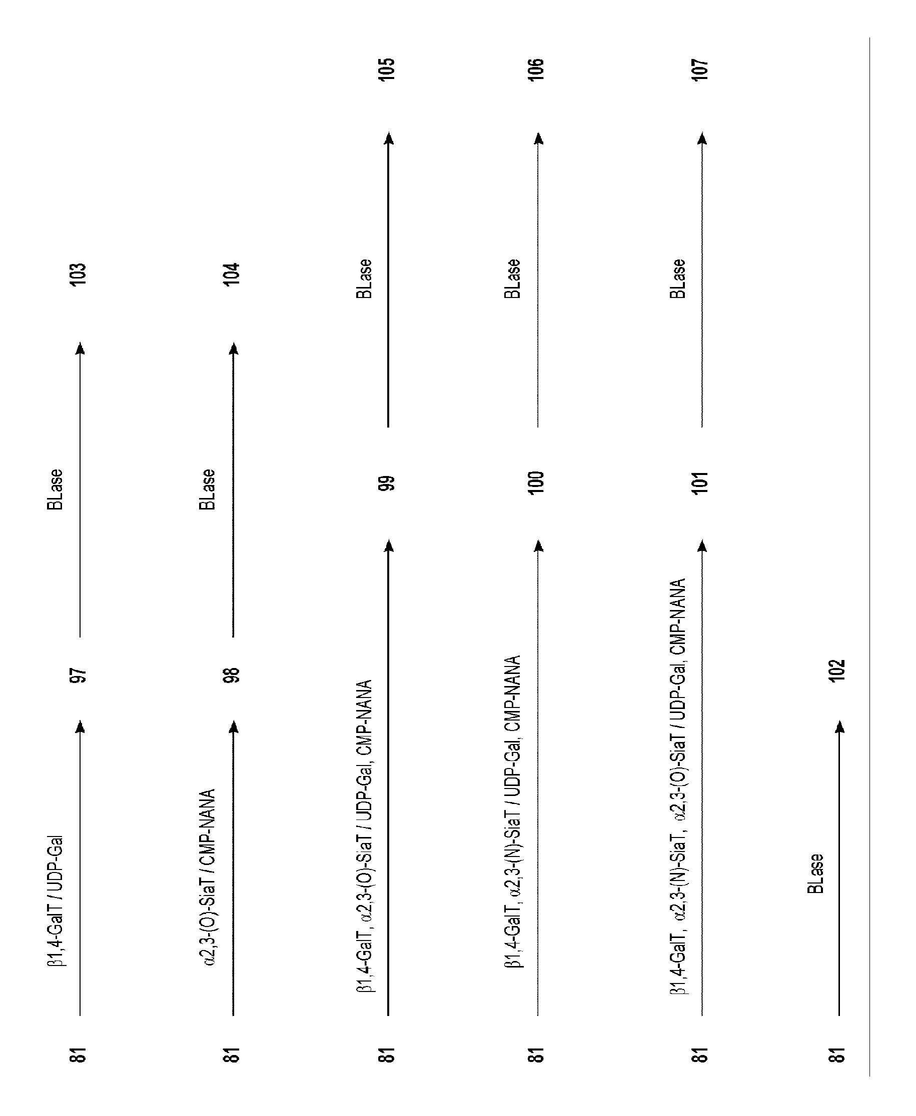

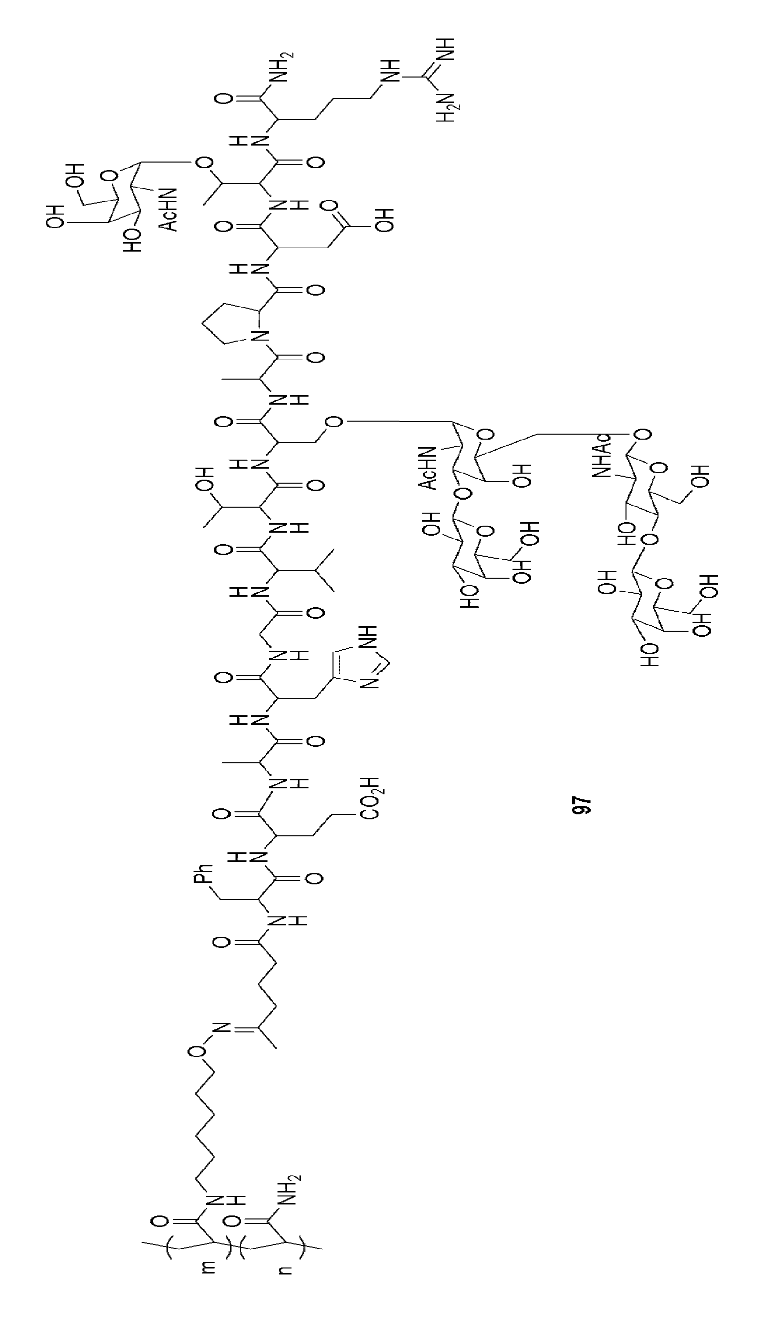

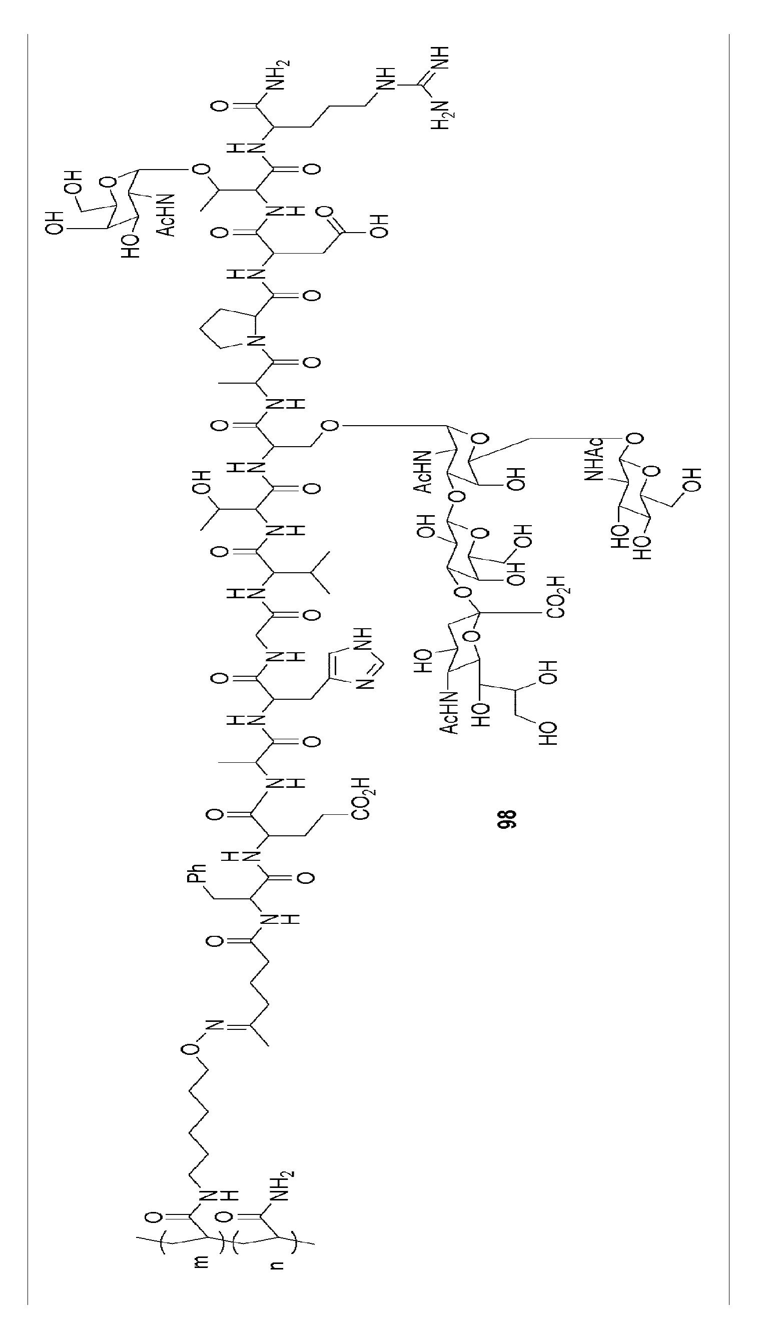

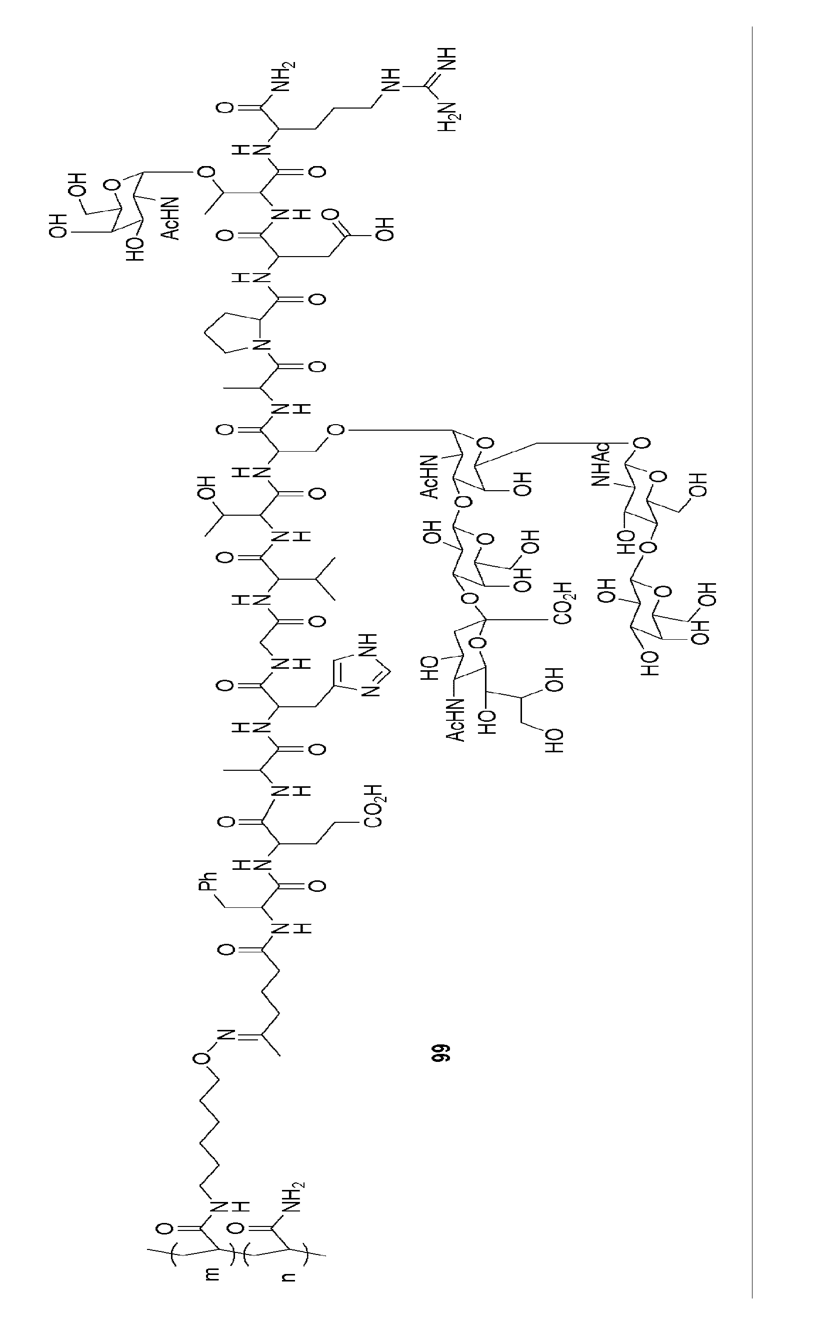

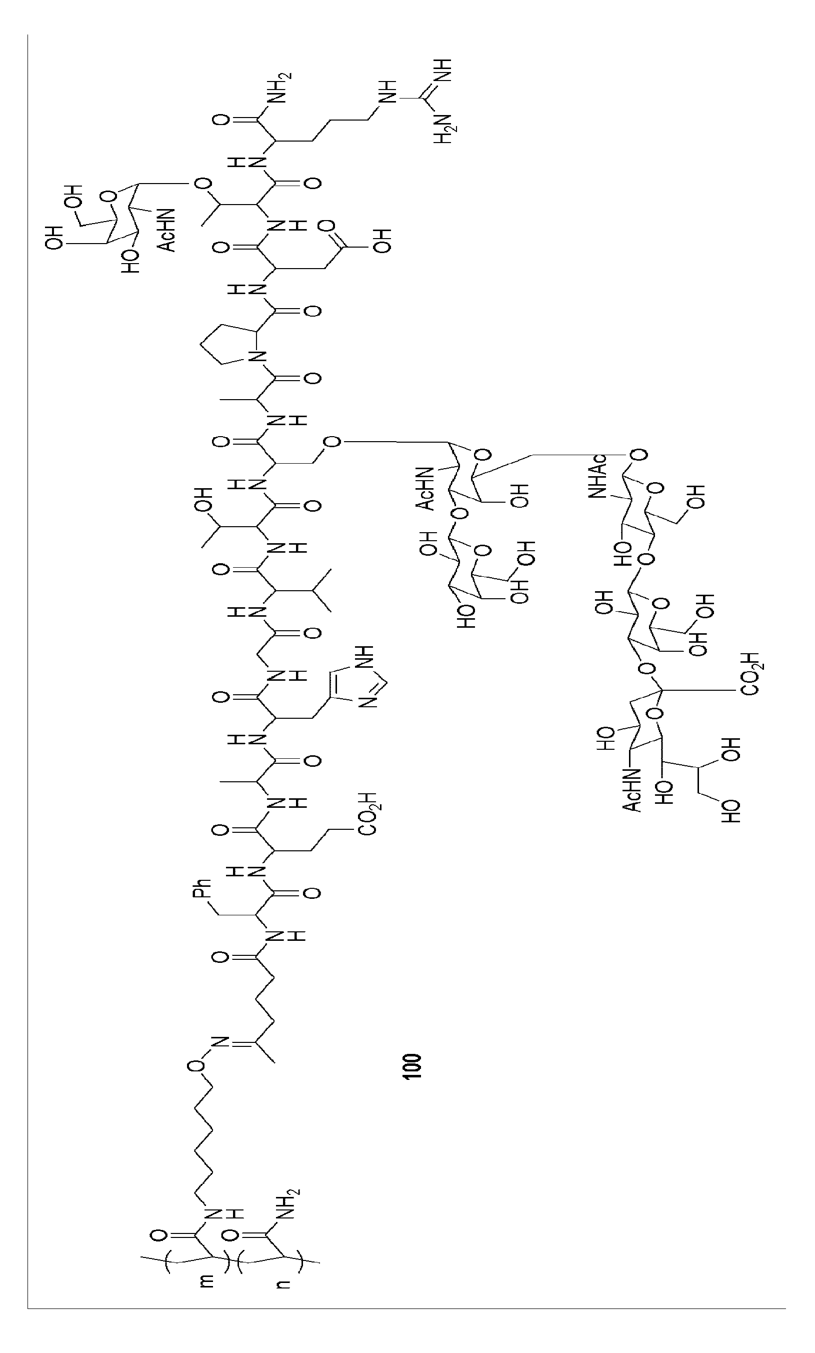

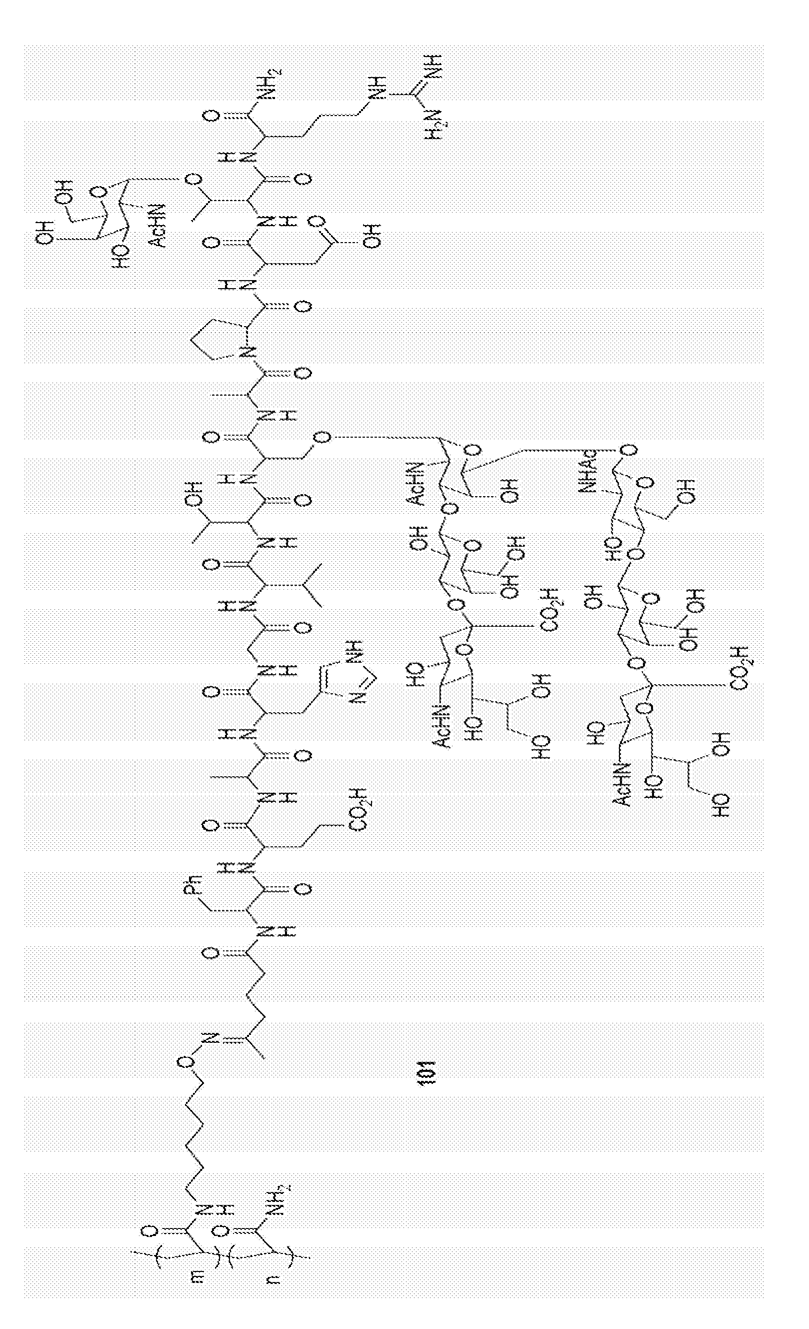

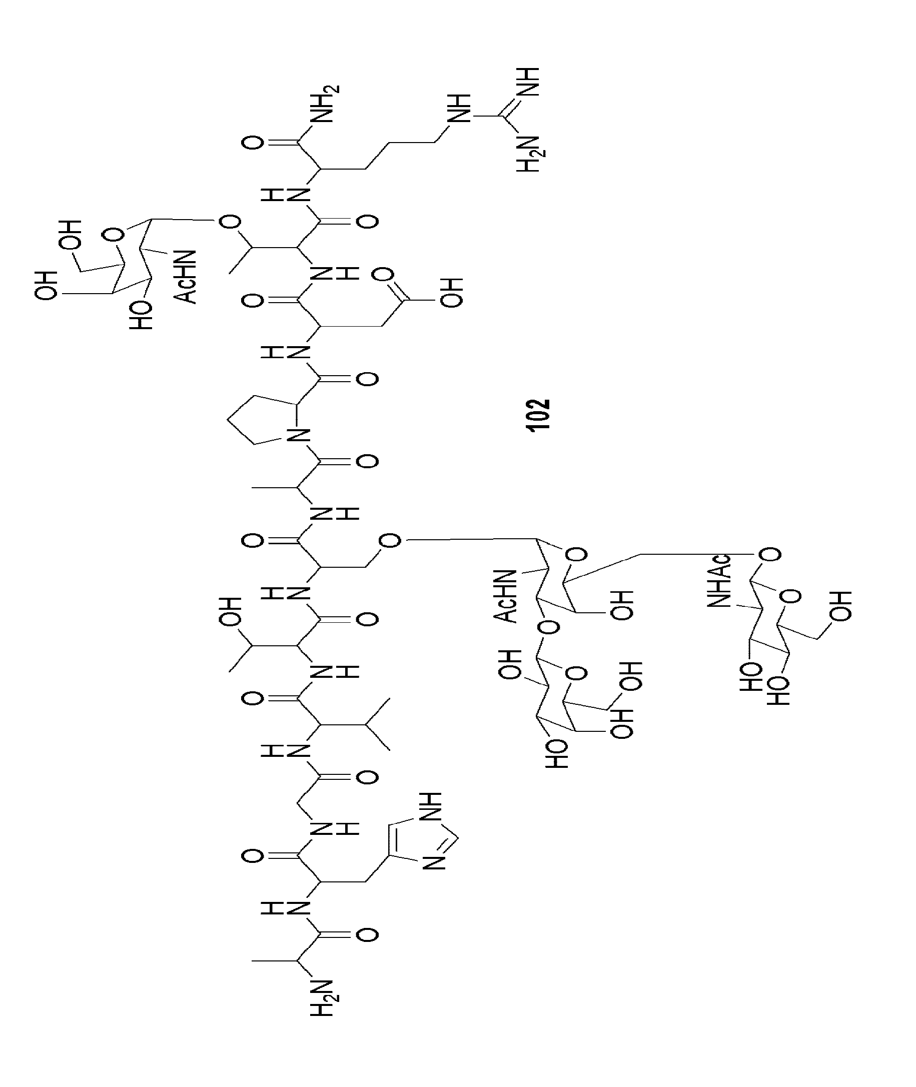

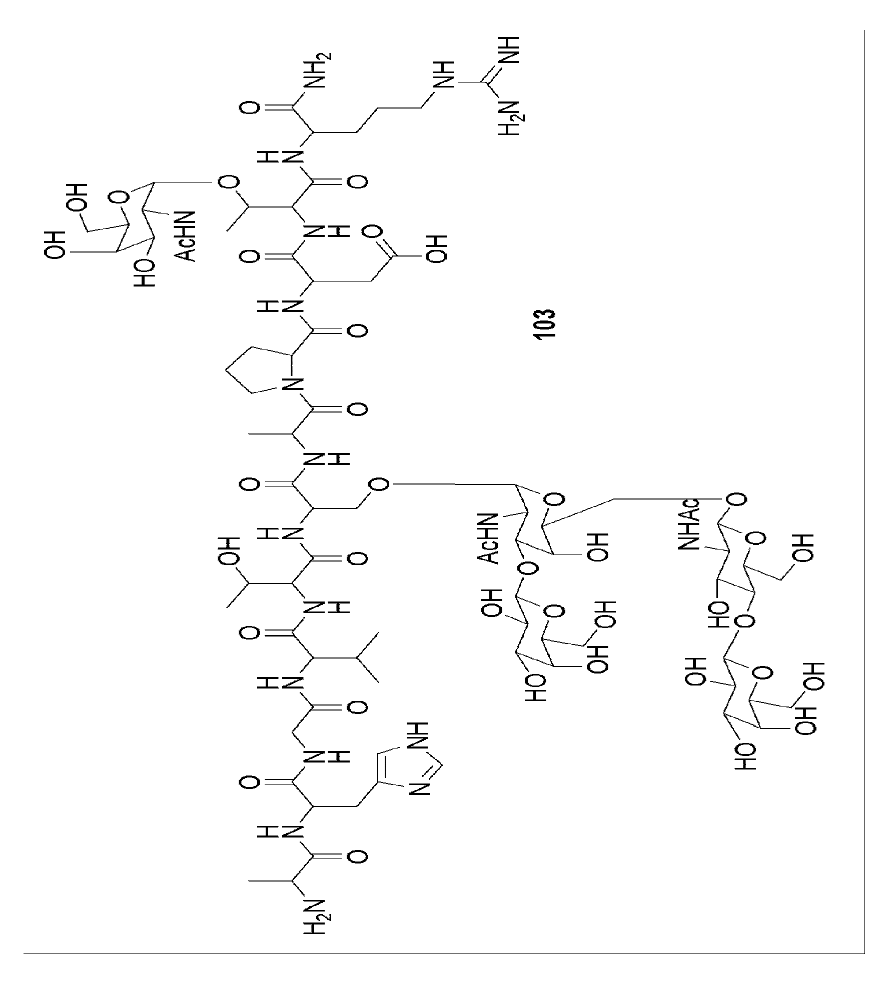

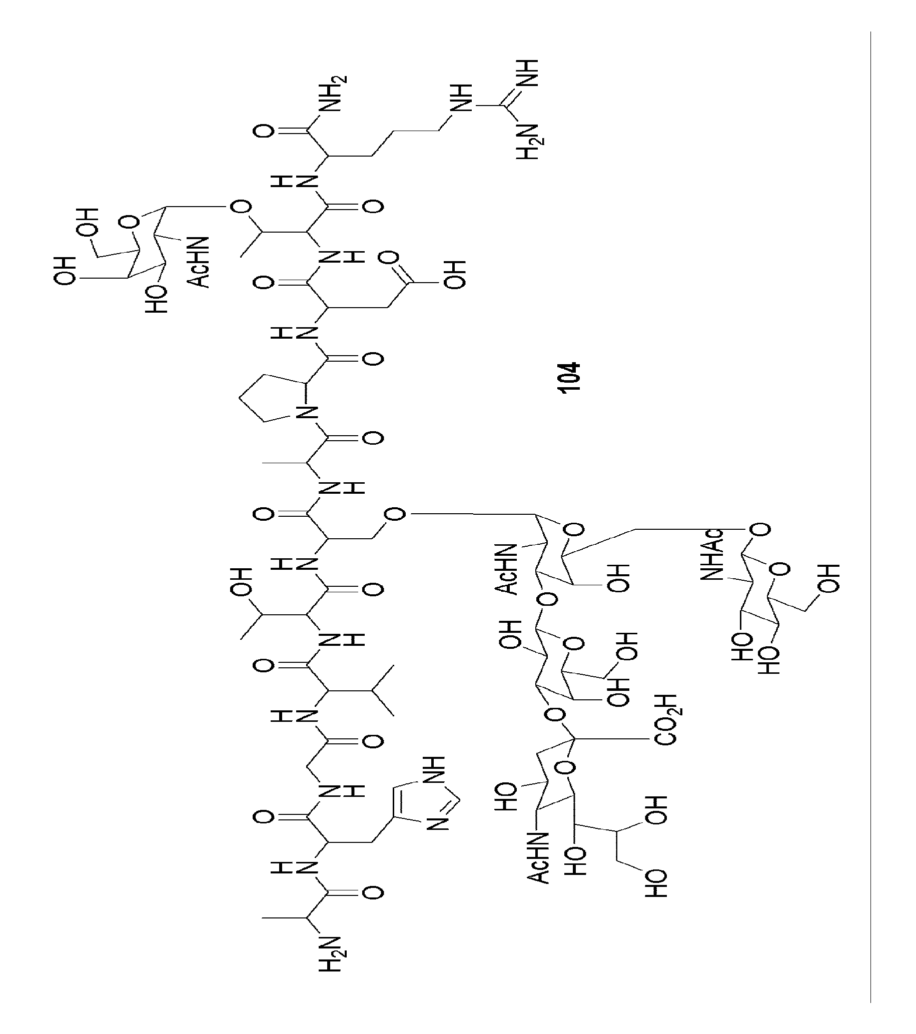

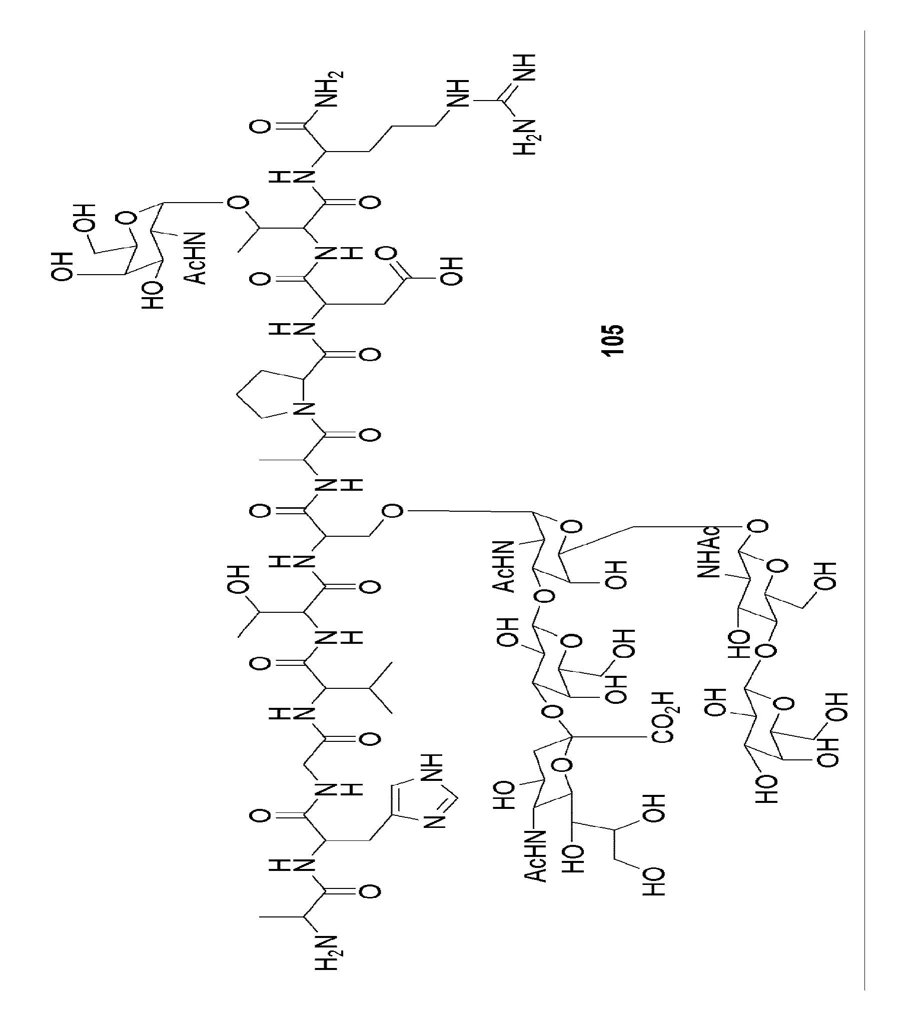

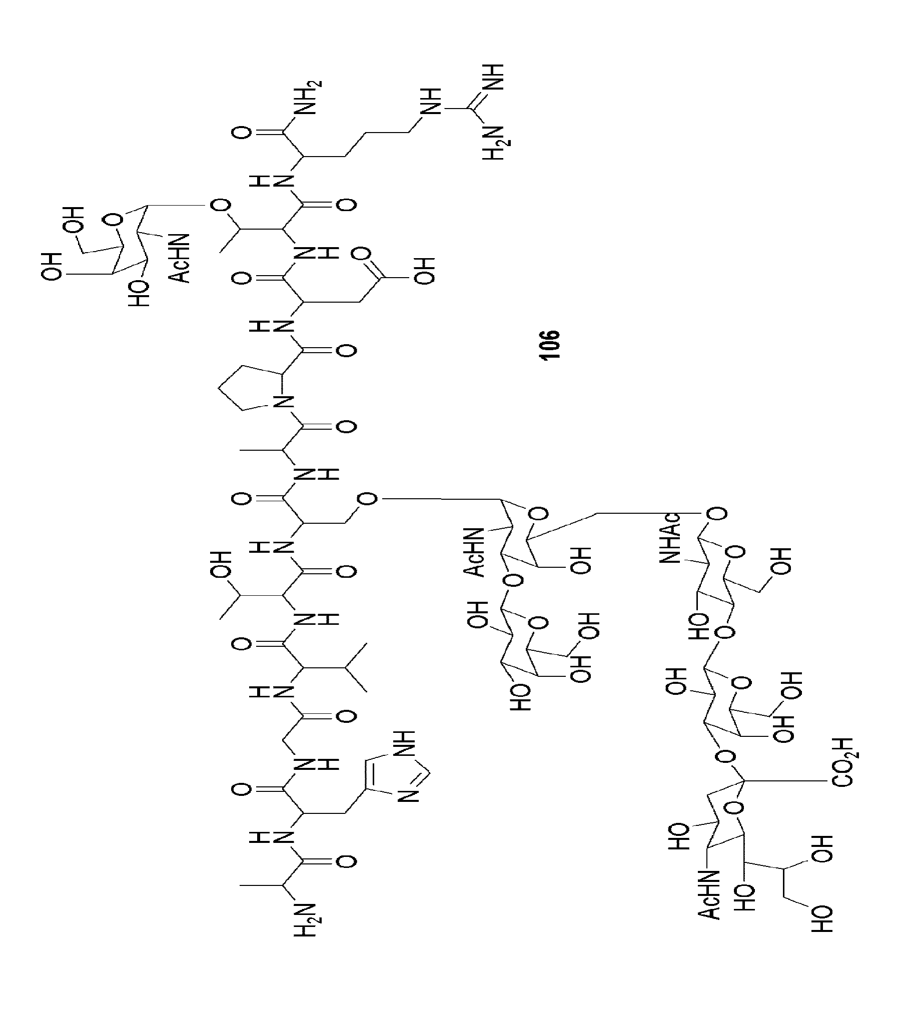

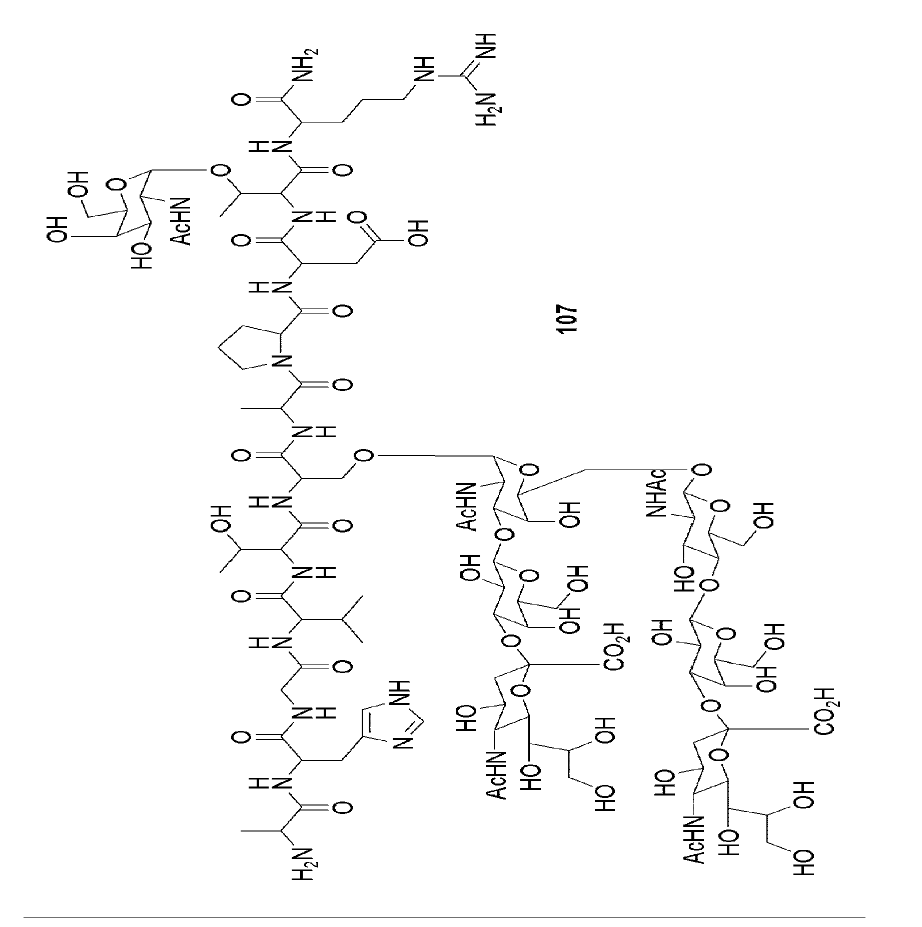

The following reaction solutions A) to E) were allowed to react for 24 hours at 25°C:
A) 250µl of reaction solution containing 50mM HEPES buffer solution (pH 7.0), 0.1U/ml human-derived β1,4-galactosyltransferase (available from TOYOBO CO., LTD.), 10mM manganese chloride, 0.1% BSA, 2mM uridine-5'-disodium diphosphogalactose (UDP-Gal) and glycopeptide derivative (81) (4mM at a theoretical content from solid-phase synthesis);
B) 250µl of reaction solution containing 50mM HEPES buffer solution (pH 7.0), 0.0175U/ml recombinant rat α2,3-(O)-sialyltransferase (available from Calbiochem), 10mM manganese chloride, 0.1% BSA, 2mM cytidine-5'-sodium monophosphosialate (CMP-NANA) and glycopeptide derivative (81) (4mM at a theoretical content from solid-phase synthesis);
C) 250µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (81) (4mM at a theoretical content from solid-phase synthesis);
D) 250µl of reaction solution containing 50mM HEPES buffer solution (pH 7.0), 0.1U/ml human-derived β1,4-galactosyltransferase (available from TOYOBO CO., LTD.), 0.0185U/ml recombinant rat α2,3-(N)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (81) (4mM at a theoretical content from solid-phase synthesis);
E) 250 µl of reaction solution containing 50 mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 0.0185U/ml recombinant rat α2,3-(N)-sialyltransferase (available from Calbiochem), 10mM manganese chloride, 0.1% BSA, 4mM uridine-5'-disodium diphosphogalactose (UDP-Gal), 2mM cytidine-5'-sodium monophosphosialate (CMP-NANA) and glycopeptide derivative (81) (4mM at a theoretical content from solid-phase synthesis).

After completion of the reaction, the respective reaction solutions were transferred to ultrafiltration filter, ULTRAFRE-MC 10,000NMWL Filter Unit (available from Millipore) and were subjected to centrifugal concentration. Thereafter, 25mM ammonium acetate buffer solution (pH 6.5) was added thereto, and the solution was again subjected to concentration with a centrifugal separator, thereby washing the polymer. This manipulation was repeated for three times, thereby respectively obtaining aqueous solutions of compounds (97) to (101) . Thereafter, to 150µl of the solution retained at the filter containing compounds (97) to (101) and 150µl solution which was obtained by diluting 100µl of aqueous solution of (81) with 25mM ammonium acetate buffer solution (pH 6.5), 1µl of 1.74mg/ml solution of BLase (available from Shionogi & Co., Ltd.) was added, and the solution was allowed to react for two hours at room temperature. Thereafter, the solution was subjected to centrifugal filtration with ultrafiltration filter, ULTRAFRE-MC 10,000NMWL Filter Unit (available from Millipore), thereby separating the glycopeptide of interest from the polymer. The obtained aqueous solution (filtrate) was lyophilized, thereby obtaining compounds (102) to (107). MALDI-TOF/MS:[M(average)+H]⁺ of compound (102)=1882.3 (theoretical value:[M(average)+H]⁺=1881.9); MALDI-TOF/MS:[M(average)+H]⁺ of compound (103)=2044.7 (theoretical value:[M(average)+H]⁺=2043.9); MALDI-TOF/MS:[M(average)+H]⁺ of compound (104)=2173.7 (theoretical value: [M(average)+H]⁺=2173.0); MALDI-TOF/MS:[M(average)+H]⁺ of compound (105)=2335.6 (theoretical value:[M(average)+H]⁺=2335.0); MALDI-TOF/MS:[M(average)+H]⁺ of compound (106)=2335.5 (theoretical value:[M(average)+H]⁺=2335.0); and MALDI-TOF/MS:[M(average)+H]⁺ of compound (107)=2626.3 (theoretical value:[M(average)+H]⁺=2626.1).

(3.22 Synthesis of (108) to (118))

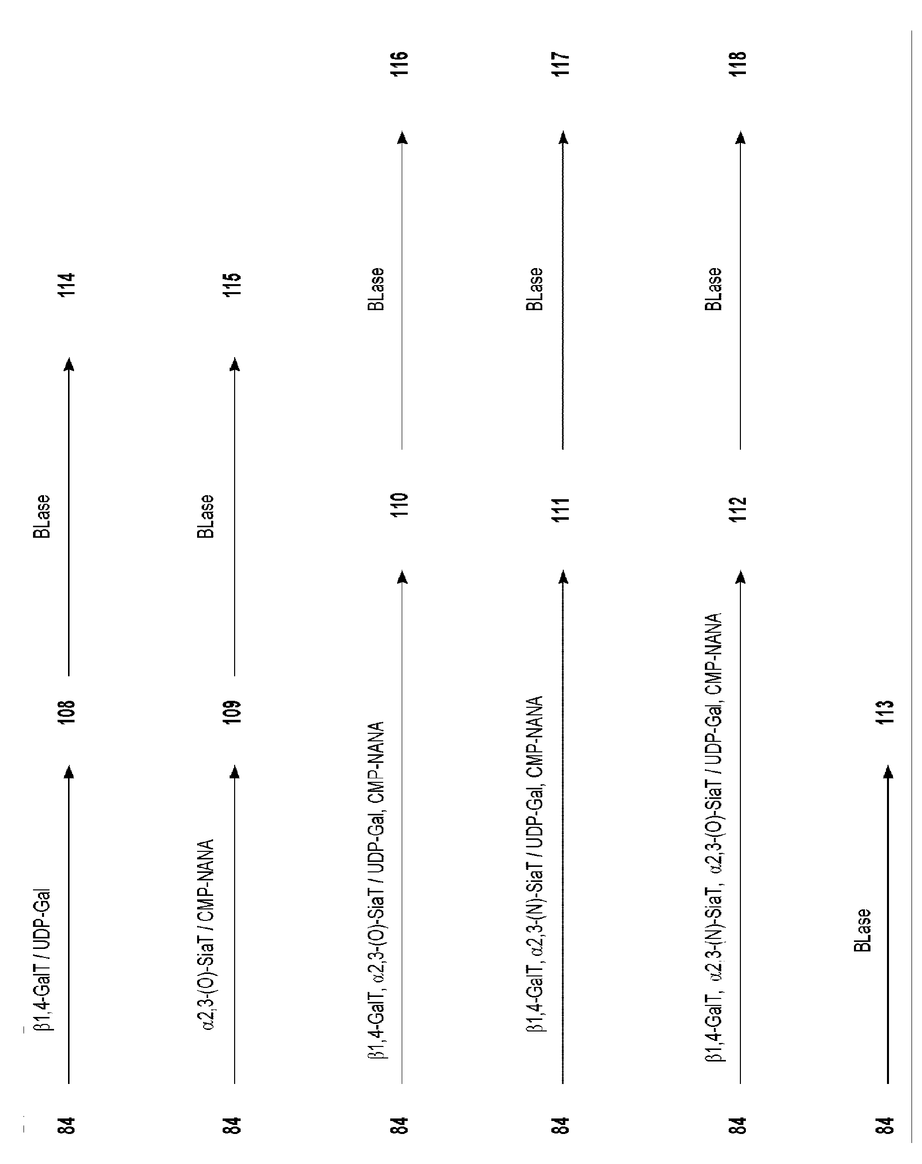

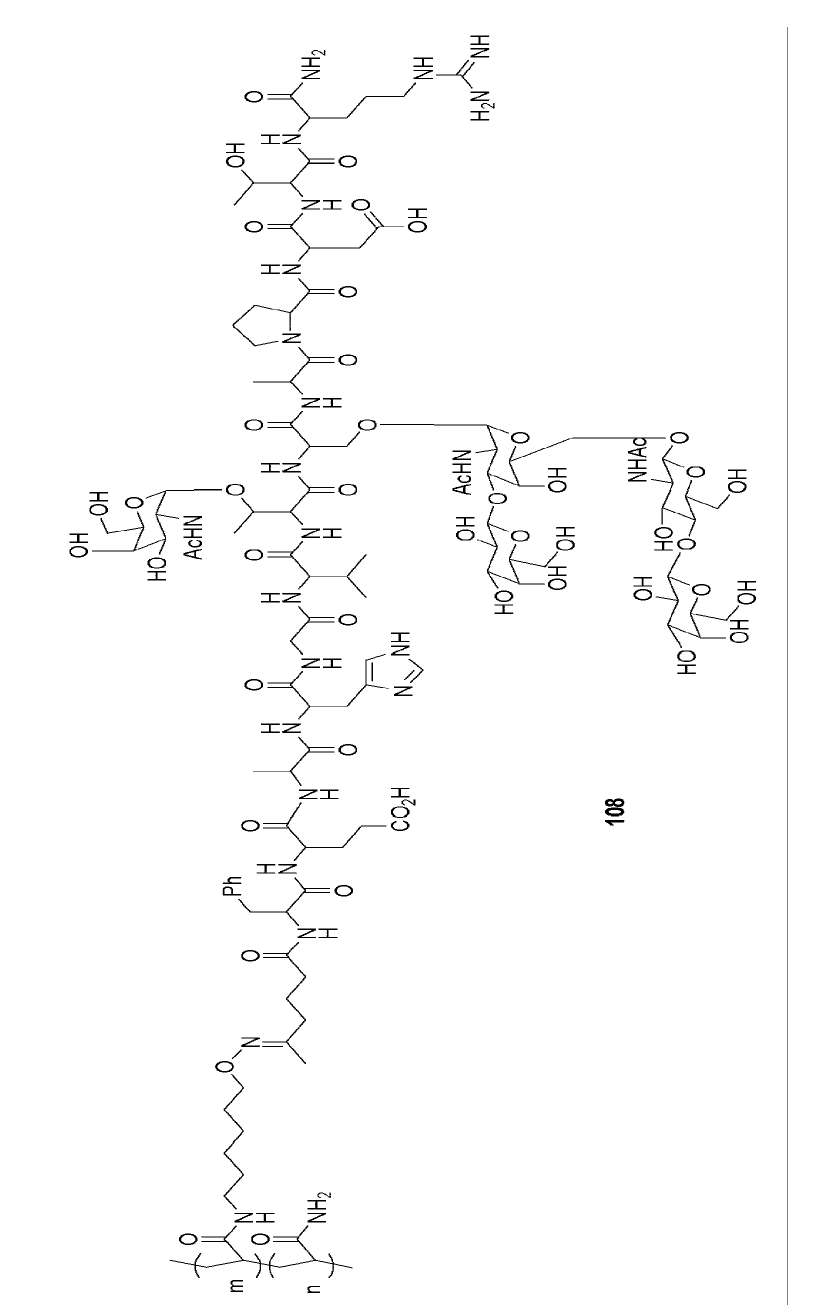

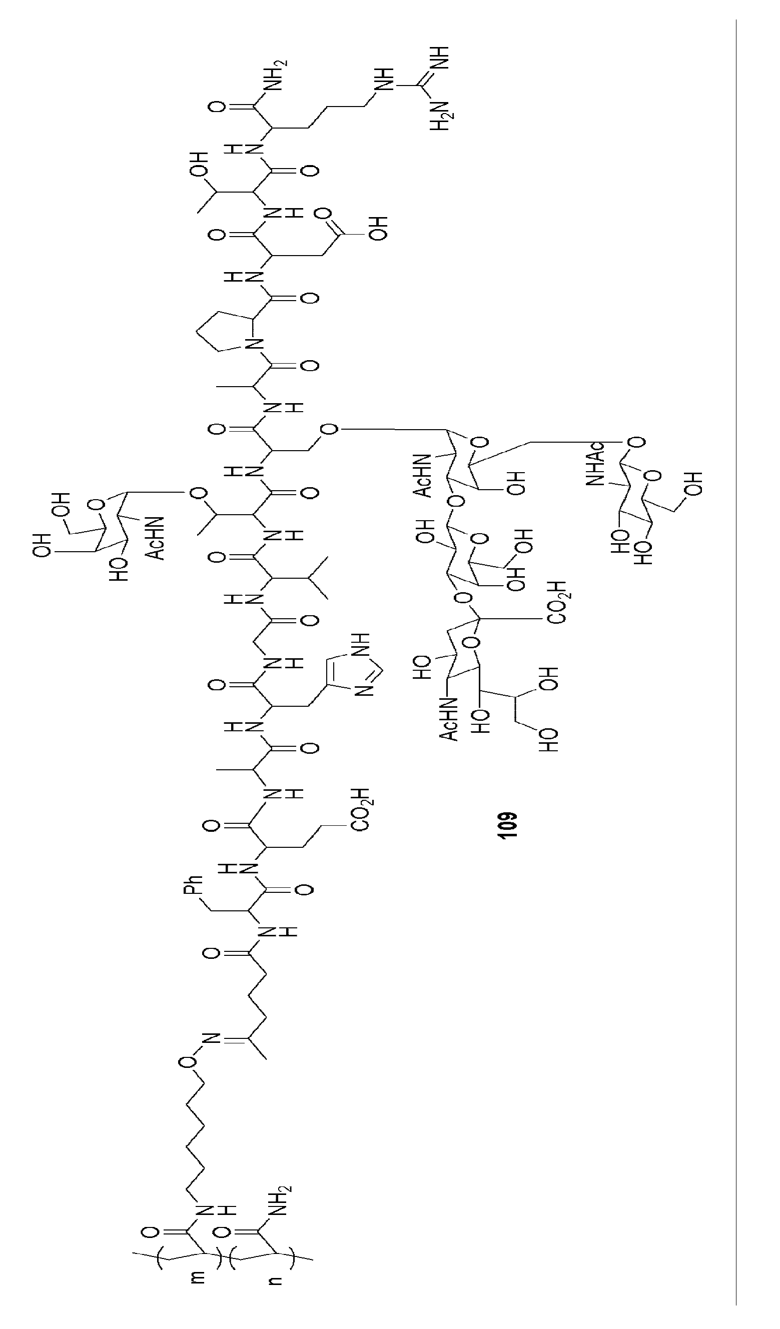

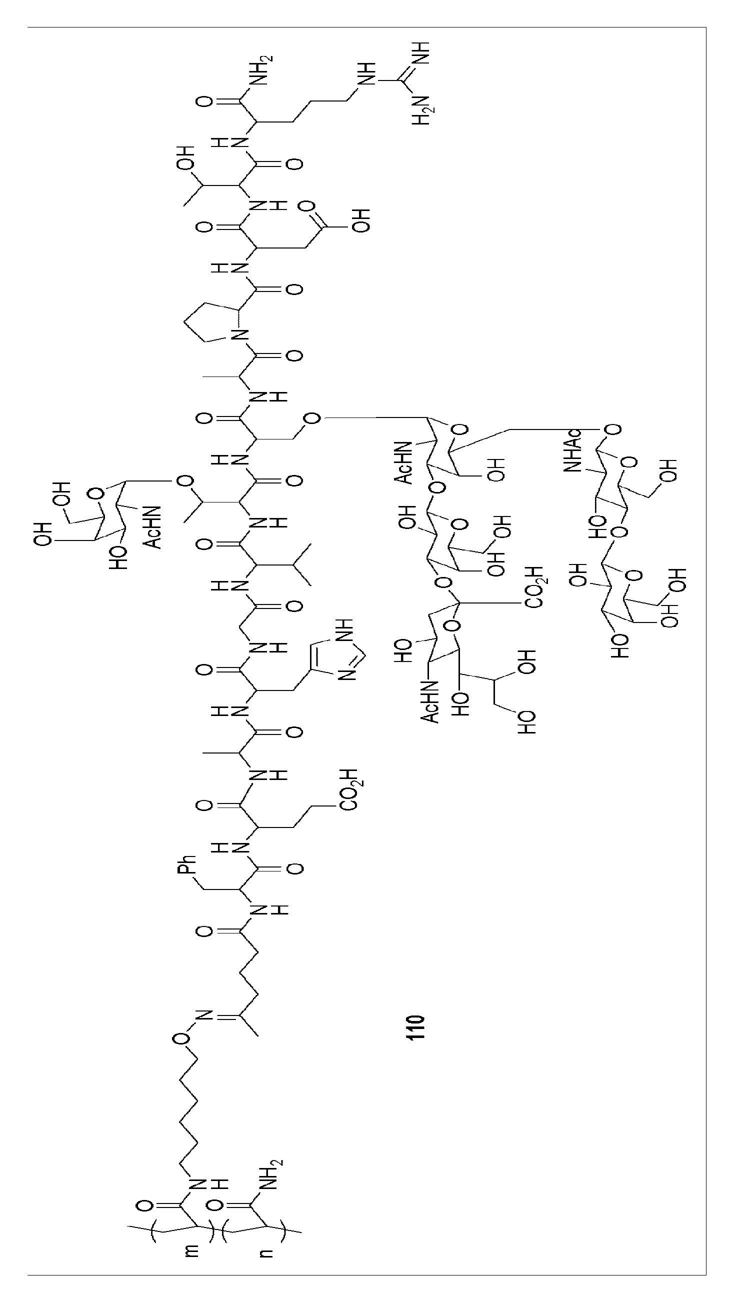

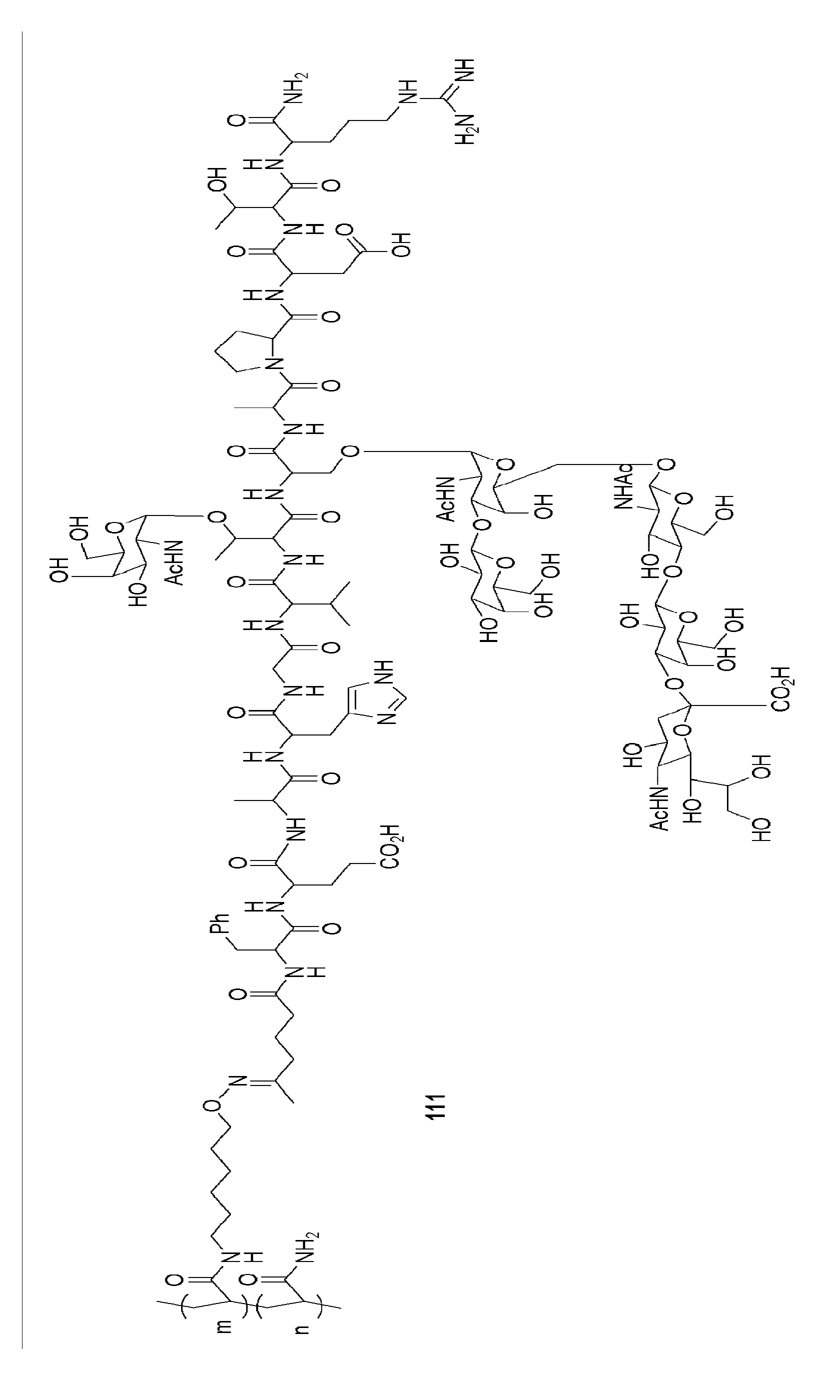

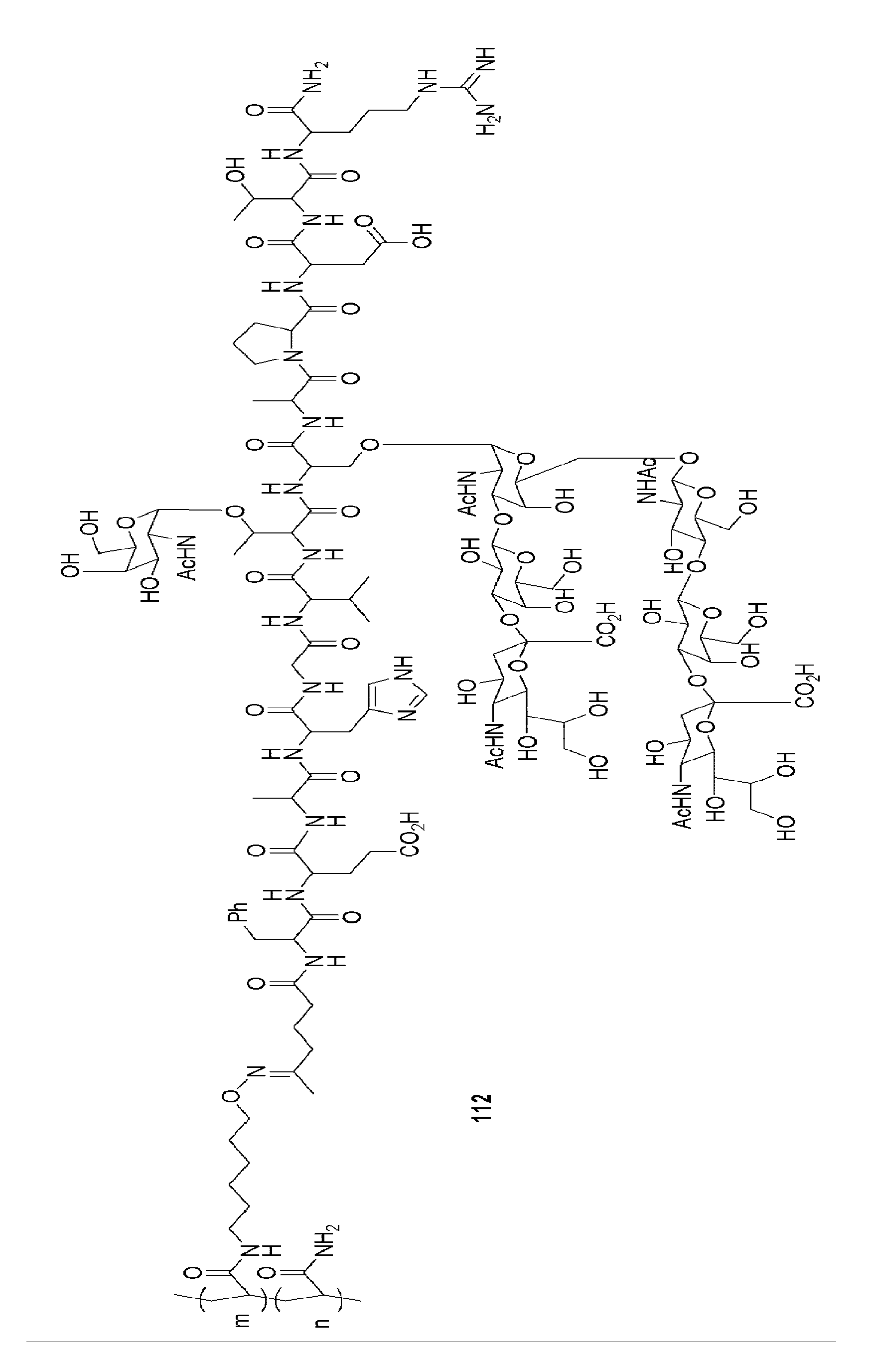

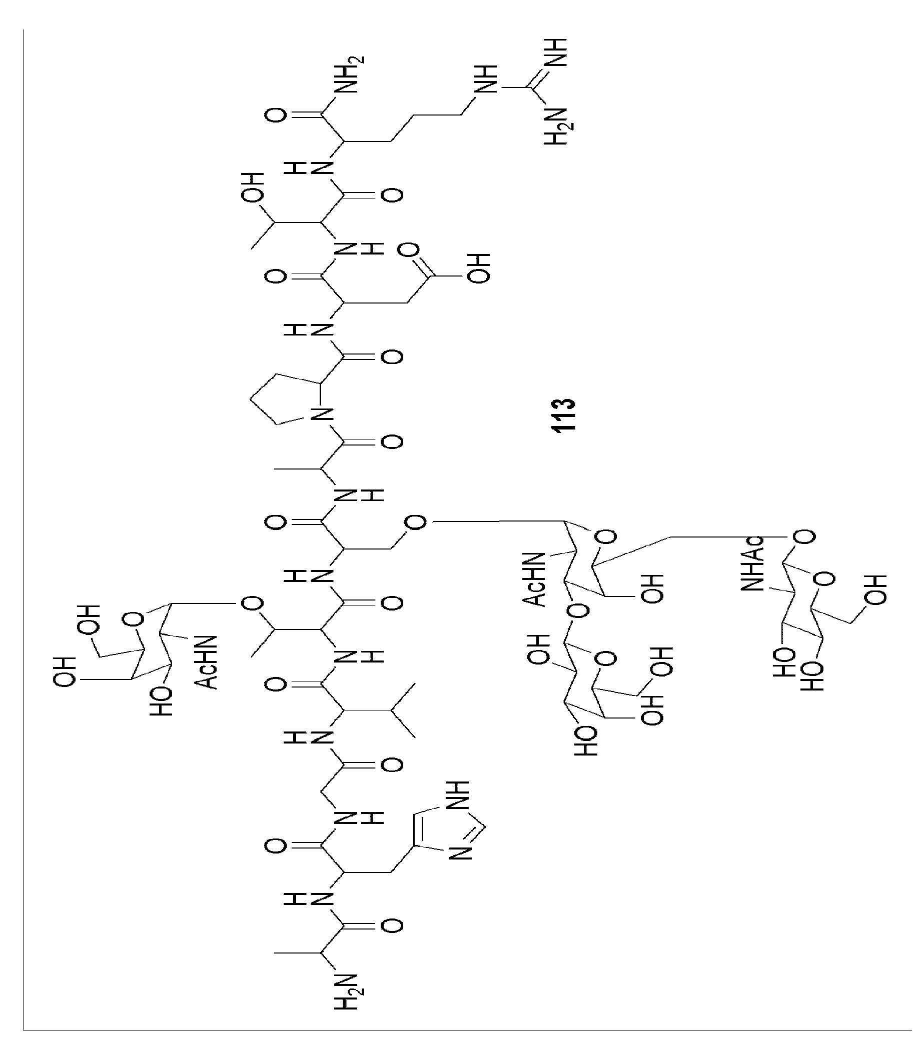

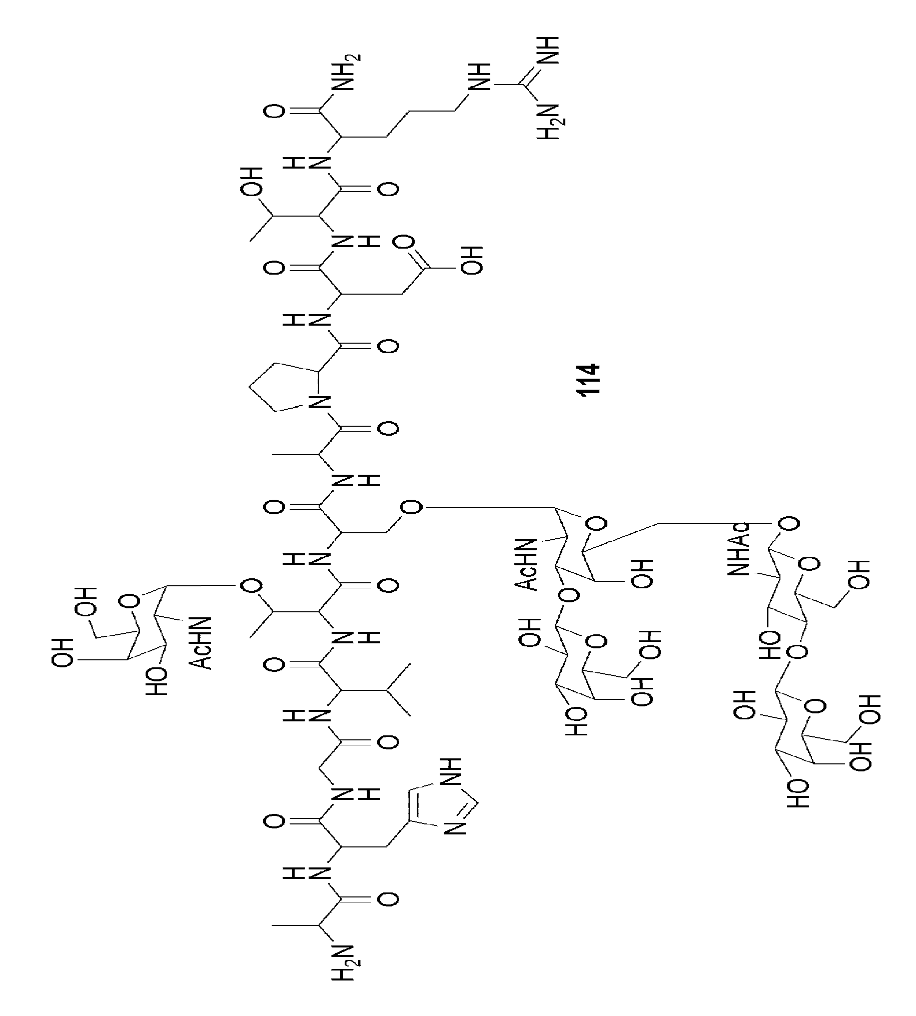

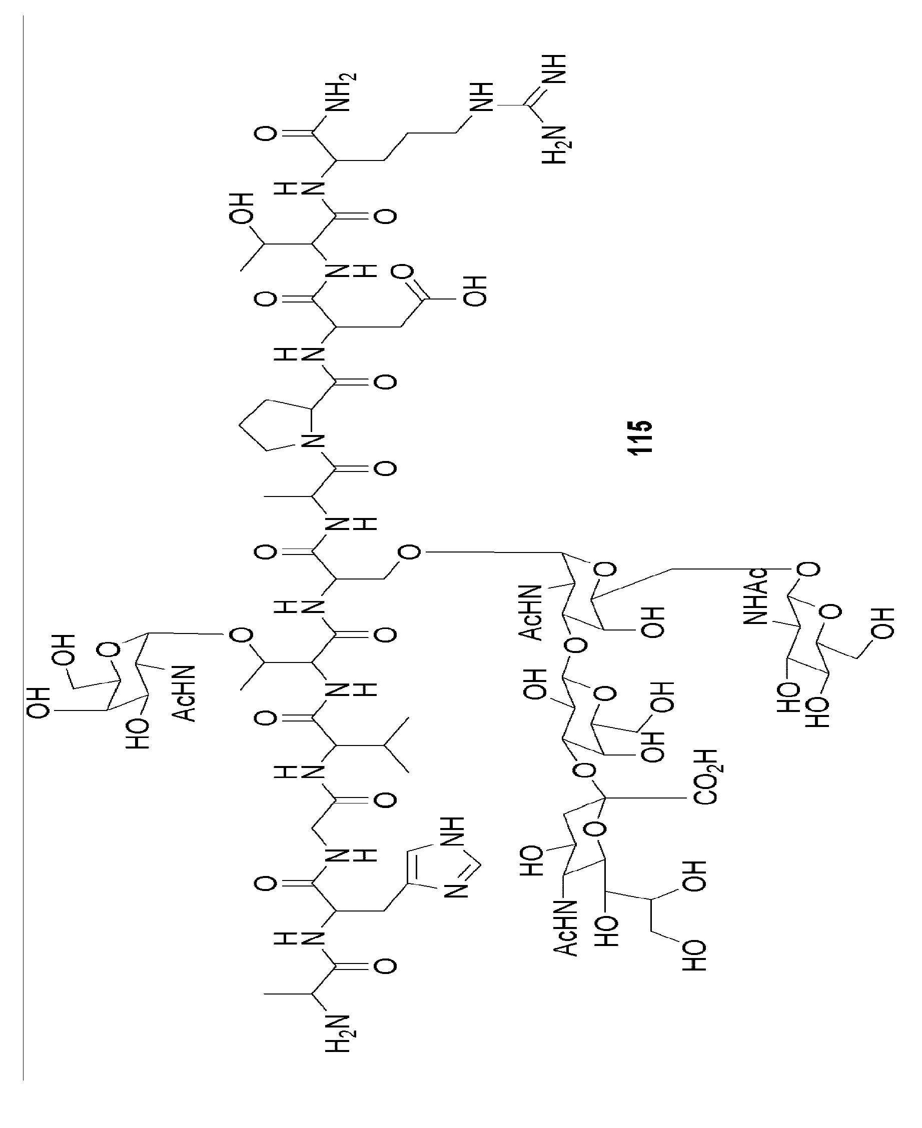

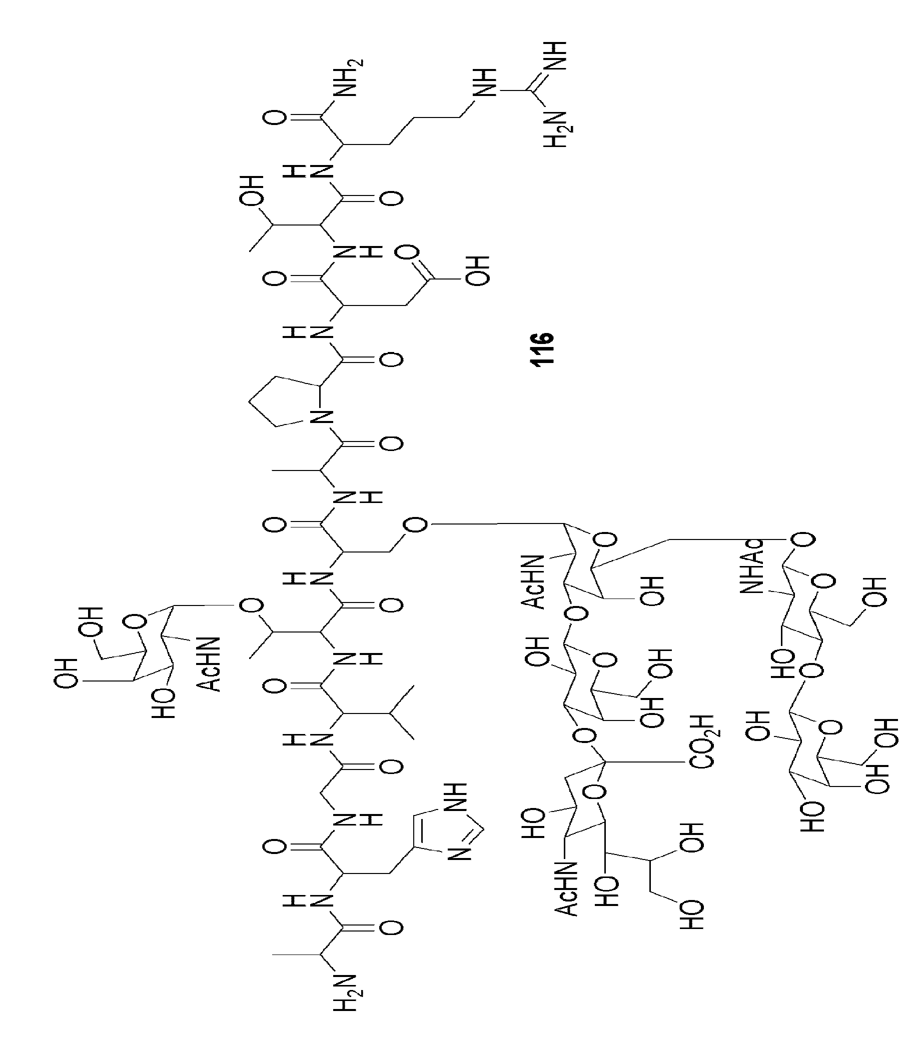

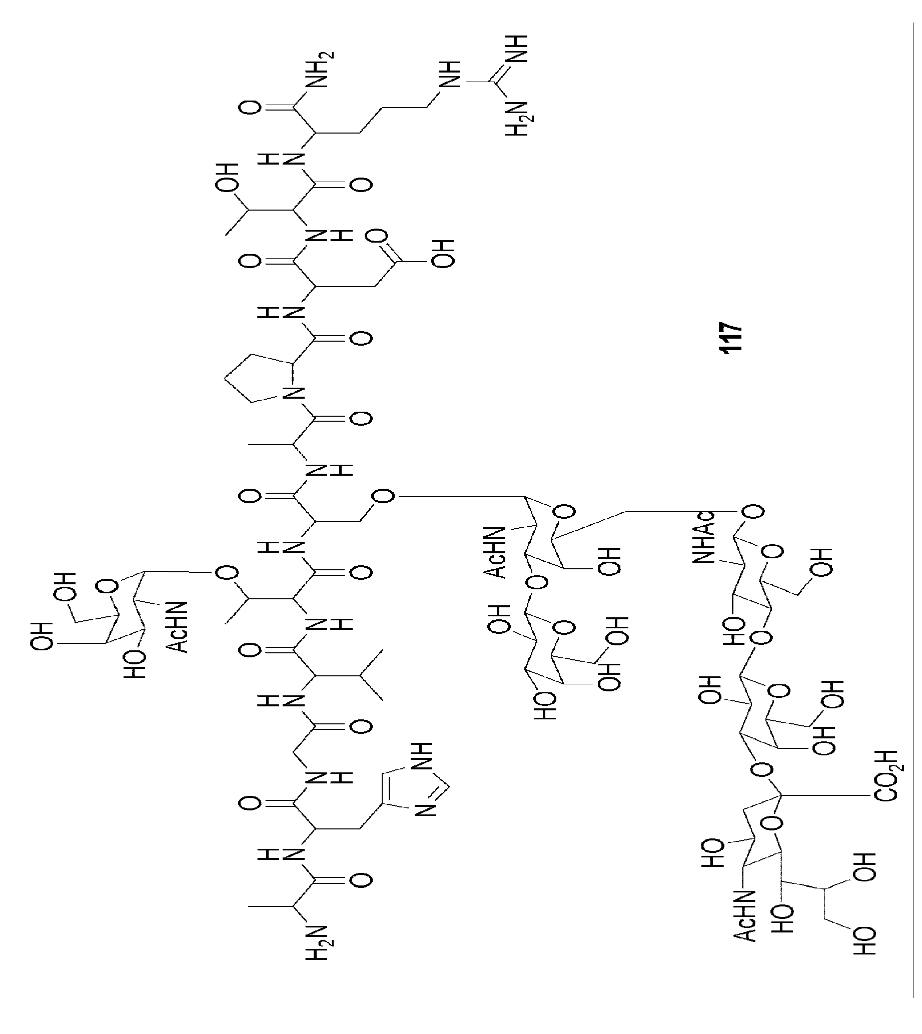

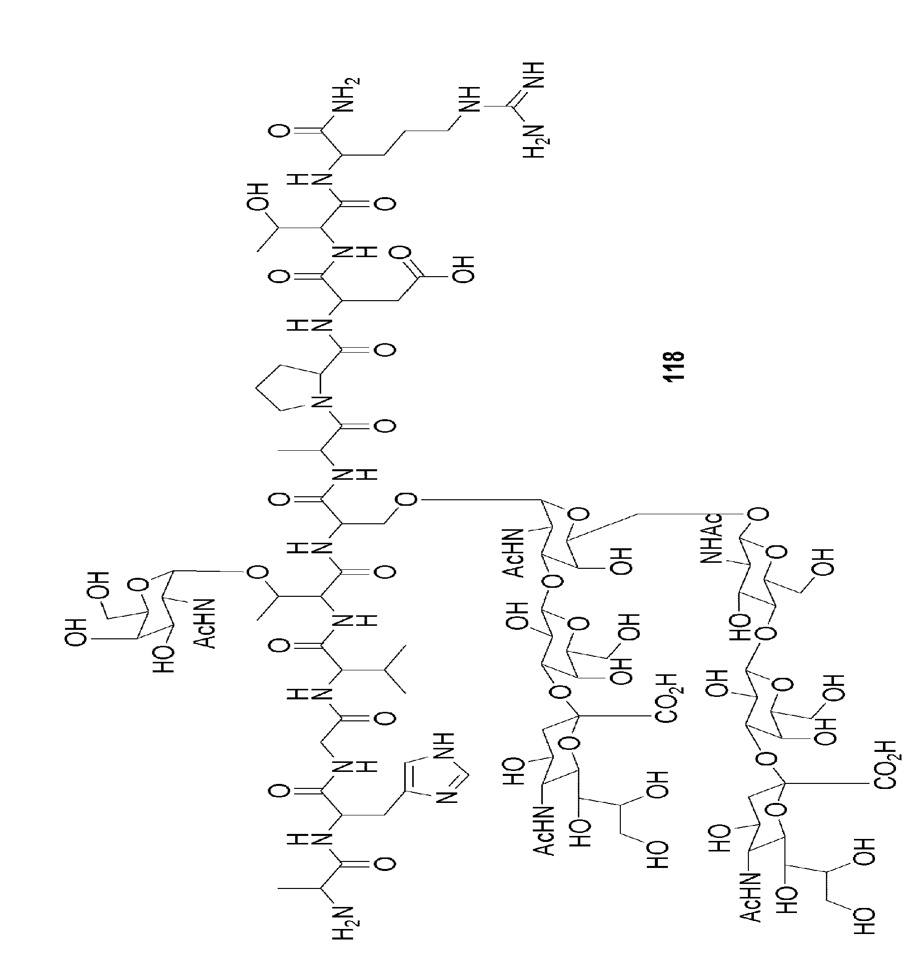

The following reaction solutions A) to E) were allowed to react for 24 hours at 25°C:
A) 250µl of reaction solution containing 50mM HEPES buffer solution (pH 7.0), 0.1U/ml human-derived β1,4-galactosyltransferase (available from TOYOBO CO., LTD.), 10mM manganese chloride, 0.1% BSA, 2mM uridine-5'-disodium diphosphogalactose (UDP-Gal) and glycopeptide derivative (84) (4mM at a theoretical content from solid-phase synthesis);
B) 250µl of reaction solution containing 50mM HEPES buffer solution (pH 7.0), 0.0175U/ml recombinant rat α2,3-(O)-sialyltransferase (available from Calbiochem), 10mM manganese chloride, 0.1% BSA, 2mM cytidine-5'-sodium monophosphosialate (CMP-NANA) and glycopeptide derivative (84) (4mM at a theoretical content from solid-phase synthesis);
C) 250µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (84) (4mM at a theoretical content from solid-phase synthesis);
D) 250µl of reaction solution containing 50mM HEPES buffer solution (pH 7.0), 0.1U/ml human-derived β1,4-galactosyltransferase (available from TOYOBO CO., LTD.), 0.0185U/ml recombinant rat α2,3-(N)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (84) (4mM at a theoretical content from solid-phase synthesis);
E) 250µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 0.0185U/ml recombinant rat α2,3-(N)-sialyltransferase (available from Calbiochem), 10mM manganese chloride, 0.1% BSA, 4mM uridine-5'-disodium diphosphogalactose (UDP-Gal), 2mM cytidine-5'-sodium monophosphosialate (CMP-NANA) and glycopeptide derivative (84) (4mM at a theoretical content from solid-phase synthesis).

After completion of the reaction, the respective reaction solutions were transferred to an ultrafiltration filter, ULTRAFRE-MC 10,000NMWL Filter Unit (available from Millipore) and were subjected to centrifugal concentration. Thereafter, 25mM ammonium acetate buffer solution (pH 6.5) was added thereto, and the solution was again subjected to concentration with a centrifugal separator, thereby washing the polymer. This manipulation was repeated three times, thereby respectively obtaining aqueous solutions of compounds (108) to (112). Thereafter, to 150µl of the solution retained at the filter containing compounds (108) to (112) and 150µl solution which was obtained by diluting 100µl of aqueous solution of (84) with 25mM ammonium acetate buffer solution (pH 6.5), 1µl of 1.74mg/ml solution of BLase (available from Shionogi & Co., Ltd.) was added, and the solution was allowedtoreact for two hours at room temperature. Thereafter, the solution was subjected to centrifugal filtration with an ultrafiltration filter, ULTRAFRE-MC 10,000NMWL Filter Unit (available from Millipore), thereby separating the glycopeptide of interest from the polymer. The obtained aqueous solution (filtrate) was lyophilized, thereby obtaining compounds (113) to (118). MALDI TOF/MS:[M(average)+H]⁺ of compound (113)=1882.3 (theoretical value:[M(average)+H]⁺=1881.9; MALDI TOF/MS:[M(average)+H]⁺ of compound (114)=2044.7 (theoretical value:[M(average)+H]⁺=2043.9); MALDI TOF/MS:[M(average)+H]⁺ of compound (115)=2173.6 (theoretical value:[M(average)+H]⁺=2173.0); MALDI TOF/MS:[M(average)+H]⁺ of compound (116)=2335.5 (theoretical value:[M(average)+H]⁺=2335.0); MALDI TOF/MS:[M(average)+H]⁺ of compound (117)=2335.5 (theoretical value: [M(average)+H]⁺=2335.0); and MALDI TOF/MS:[M(average)+H]⁺ of compound (118)=2626.3 (theoretical value:[M(average)+H]⁺=2626.1).

(3.23 Synthesis of (119) to (129))

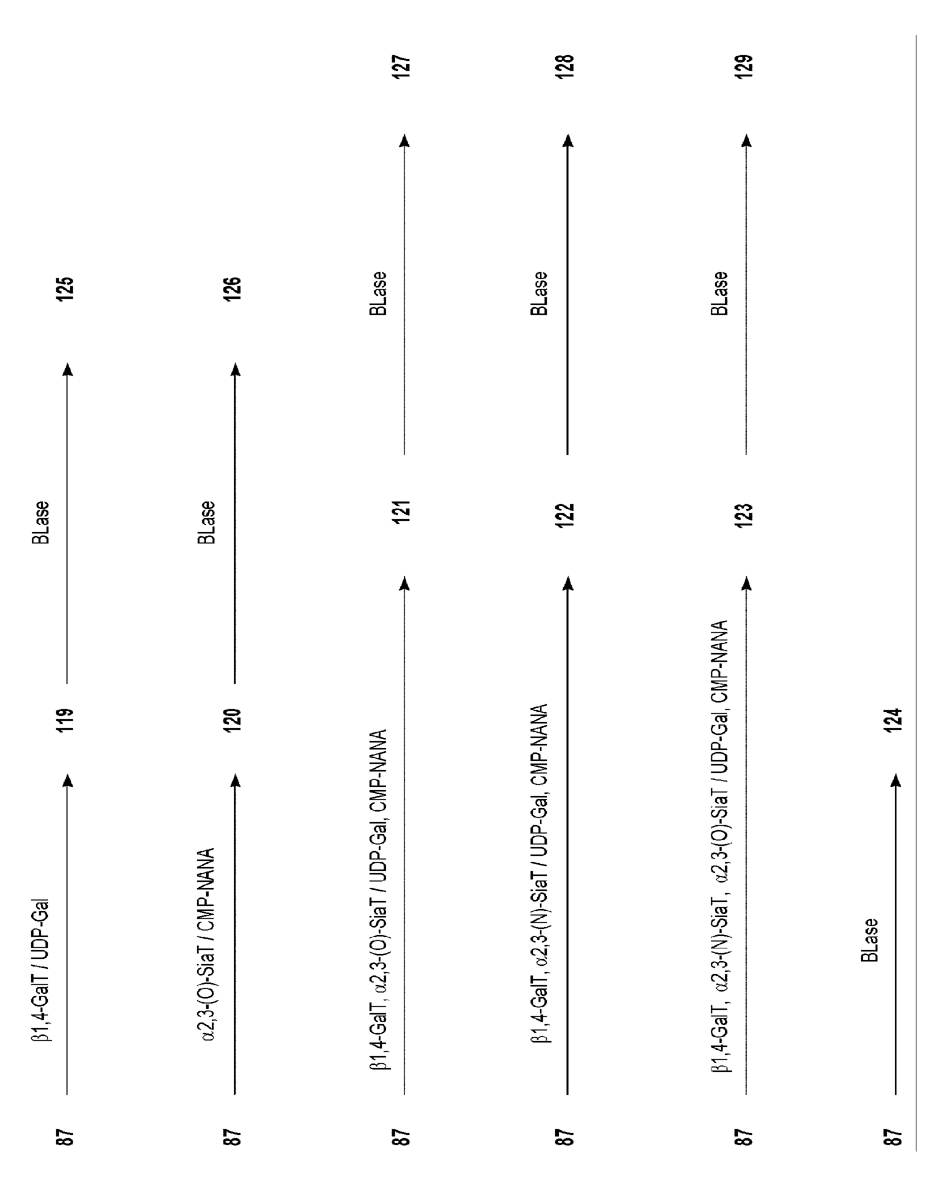

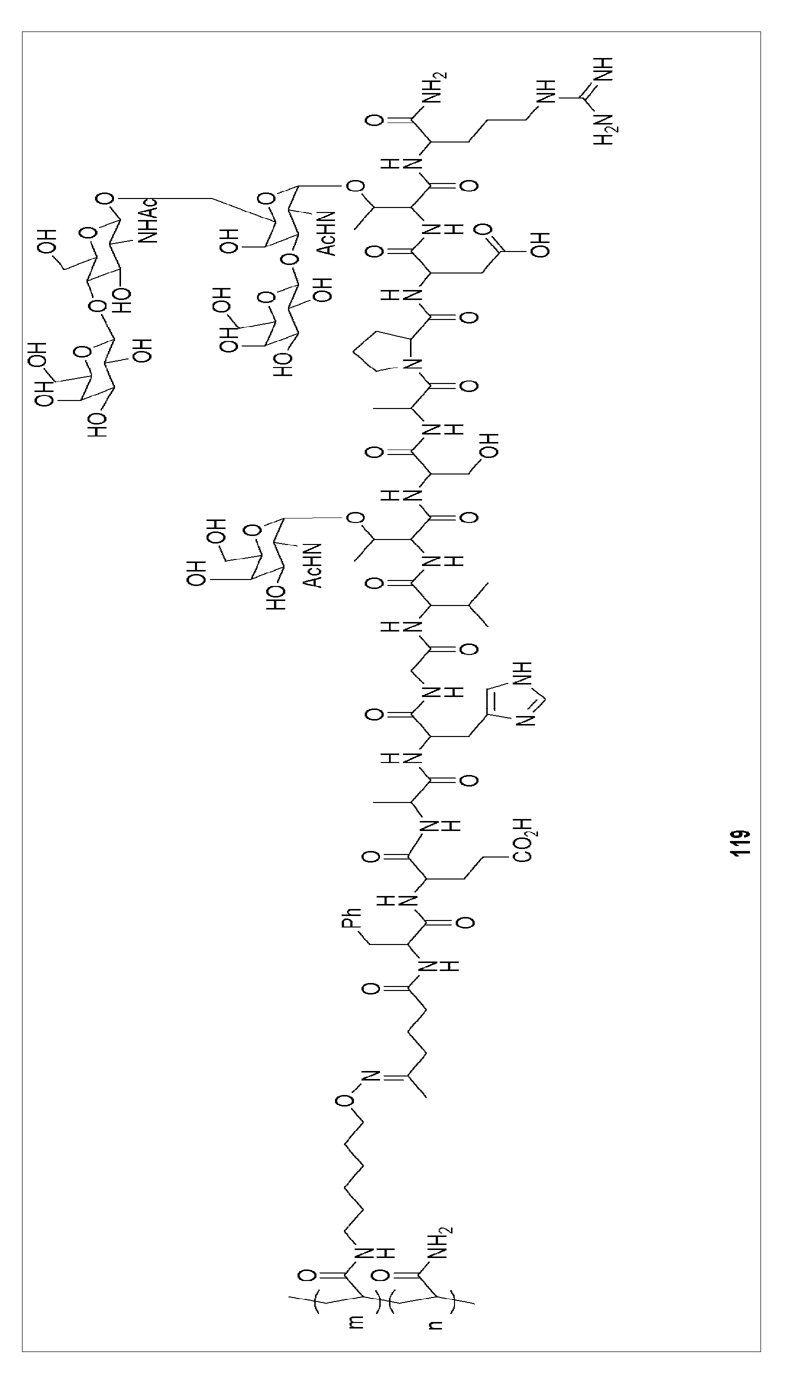

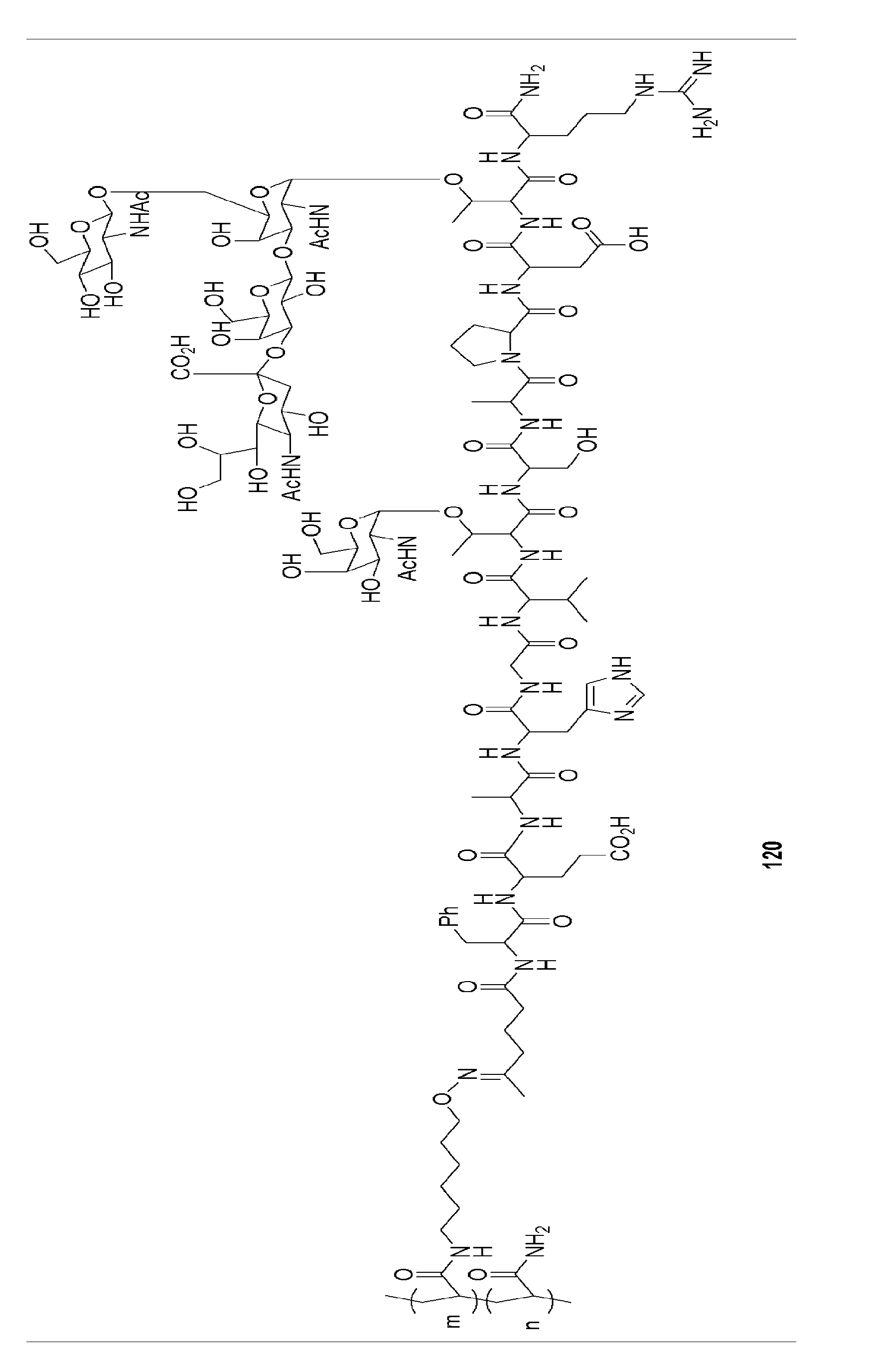

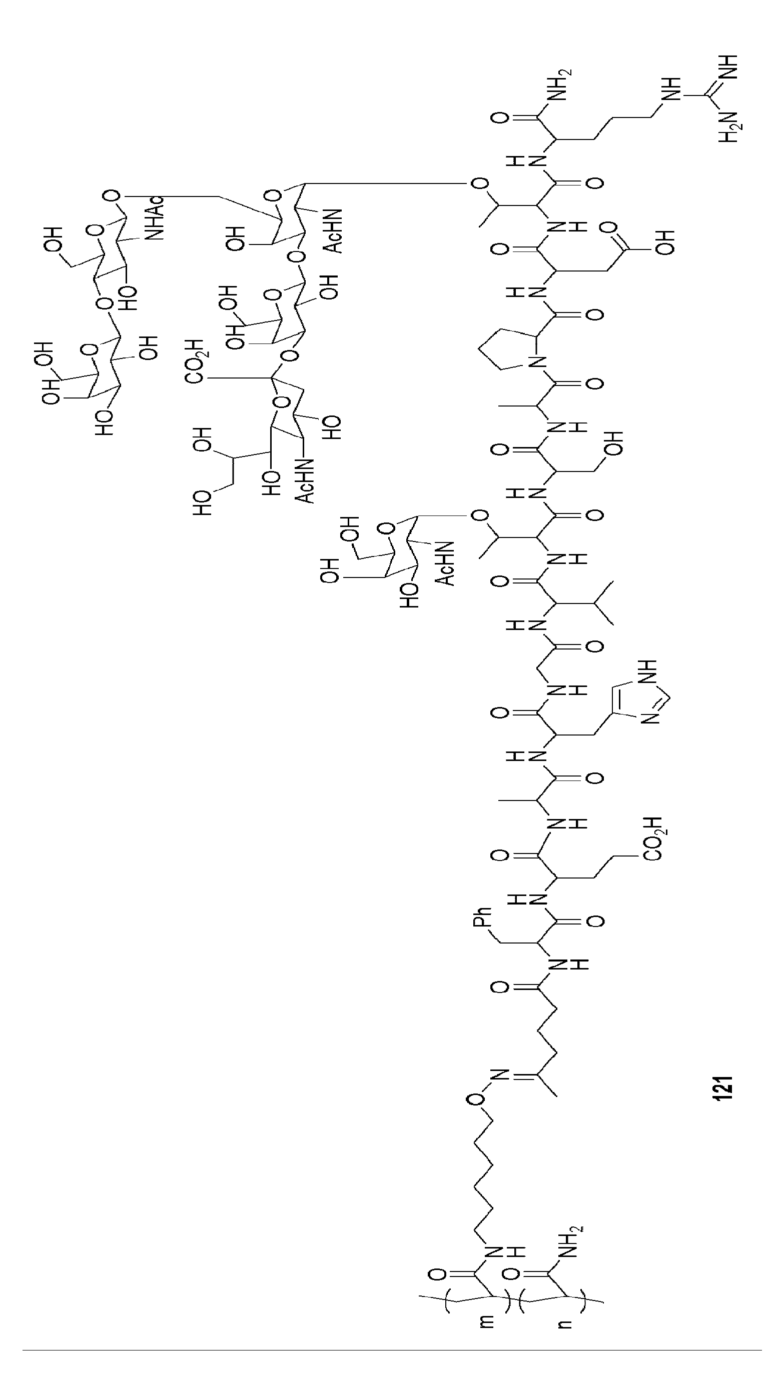

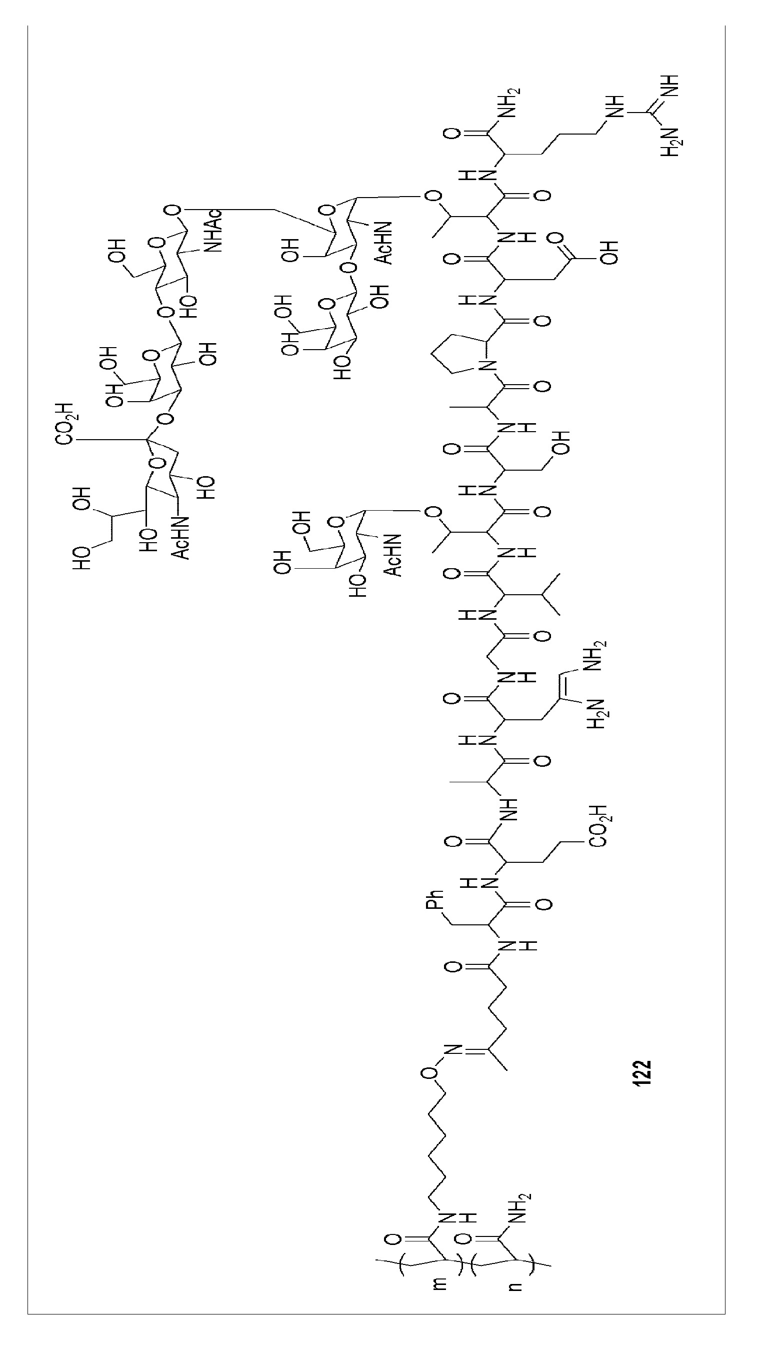

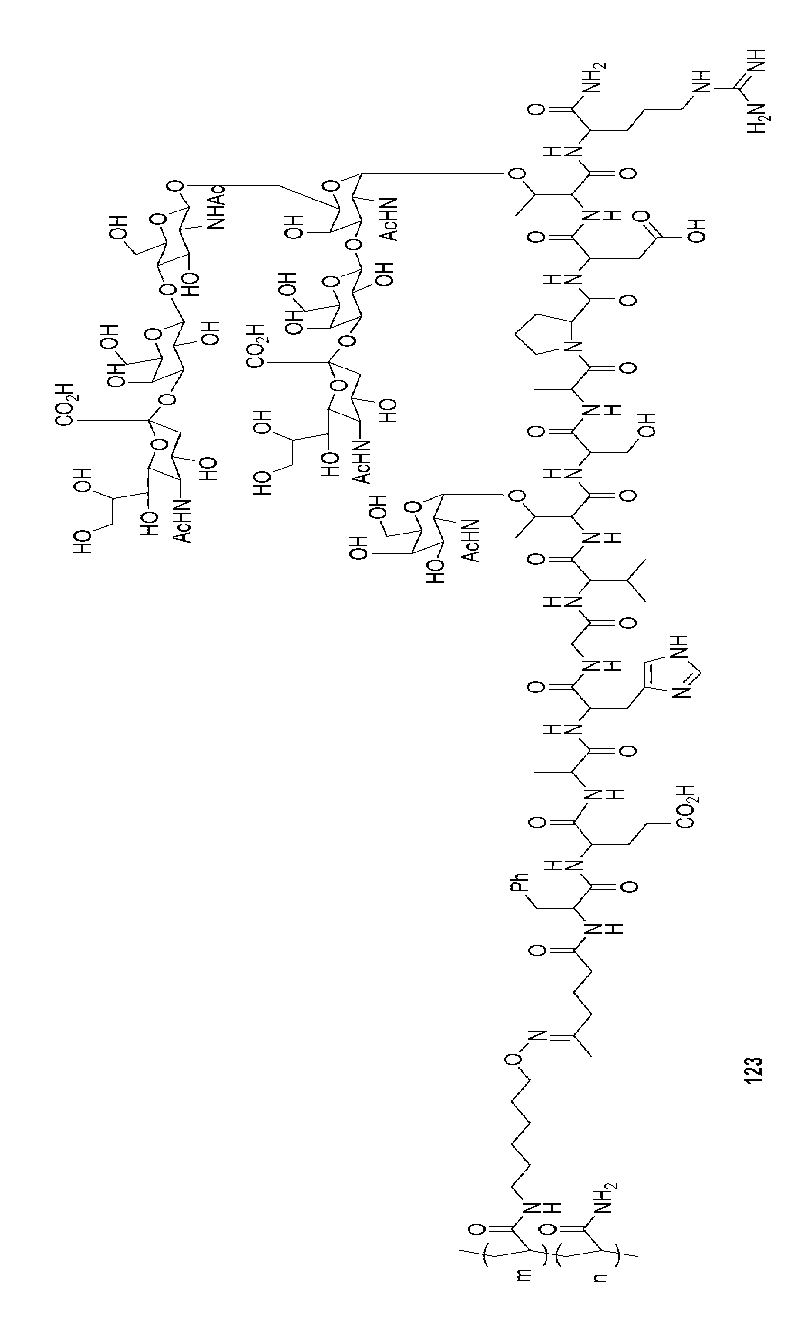

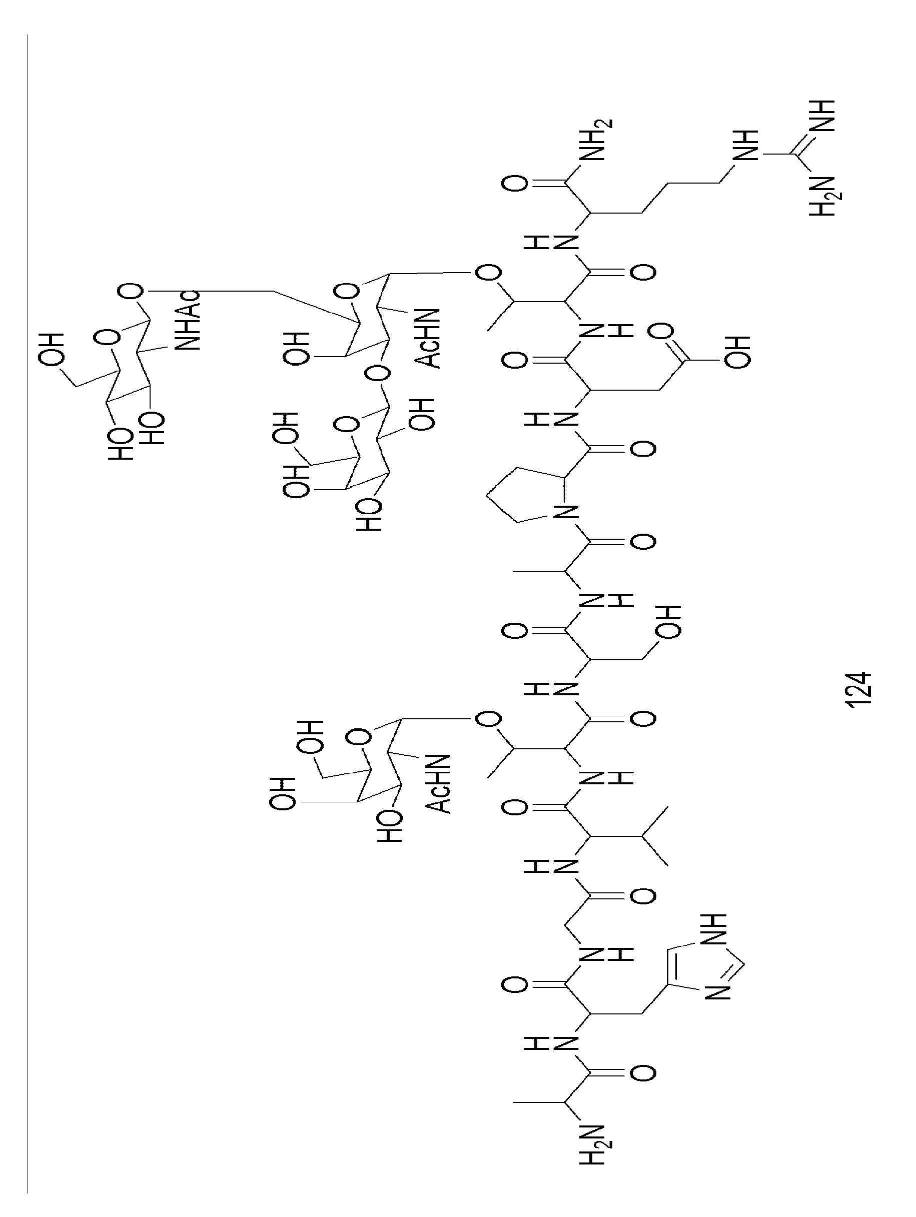

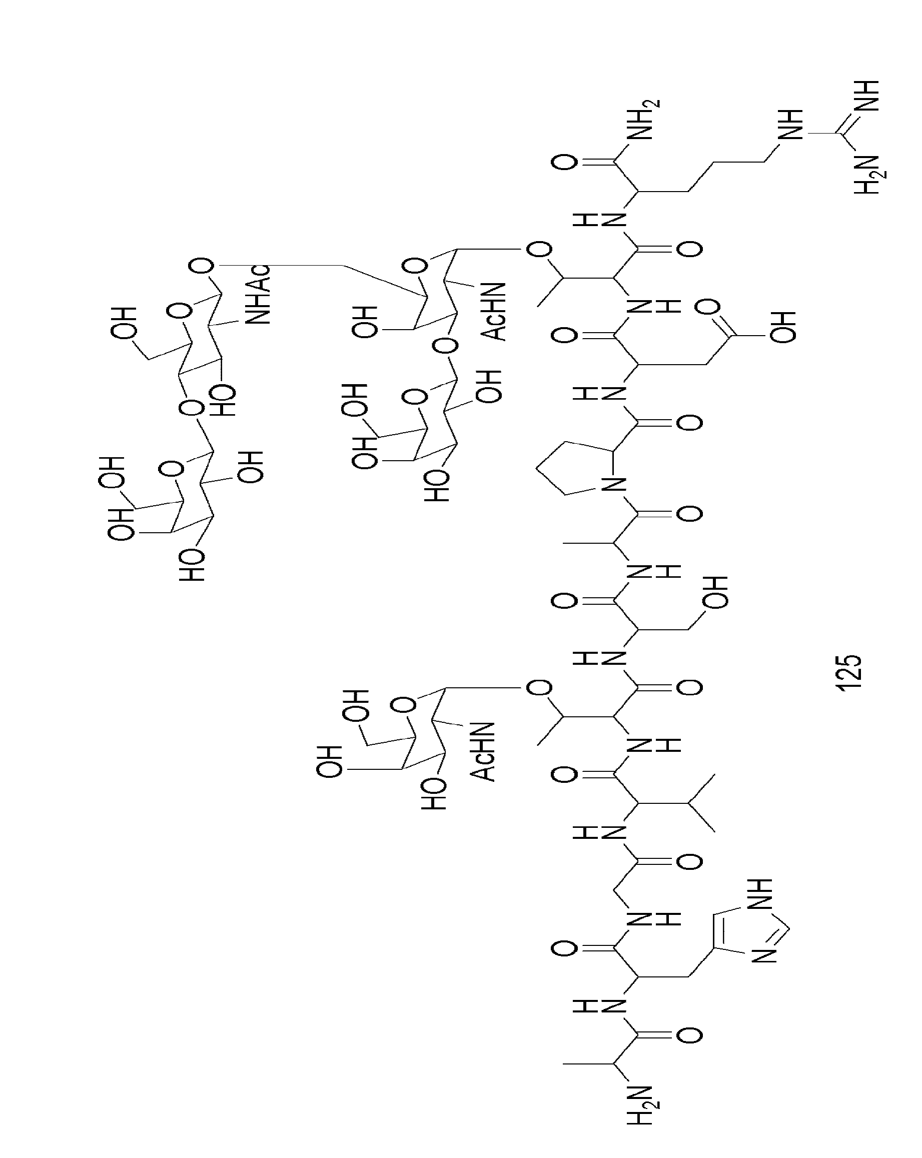

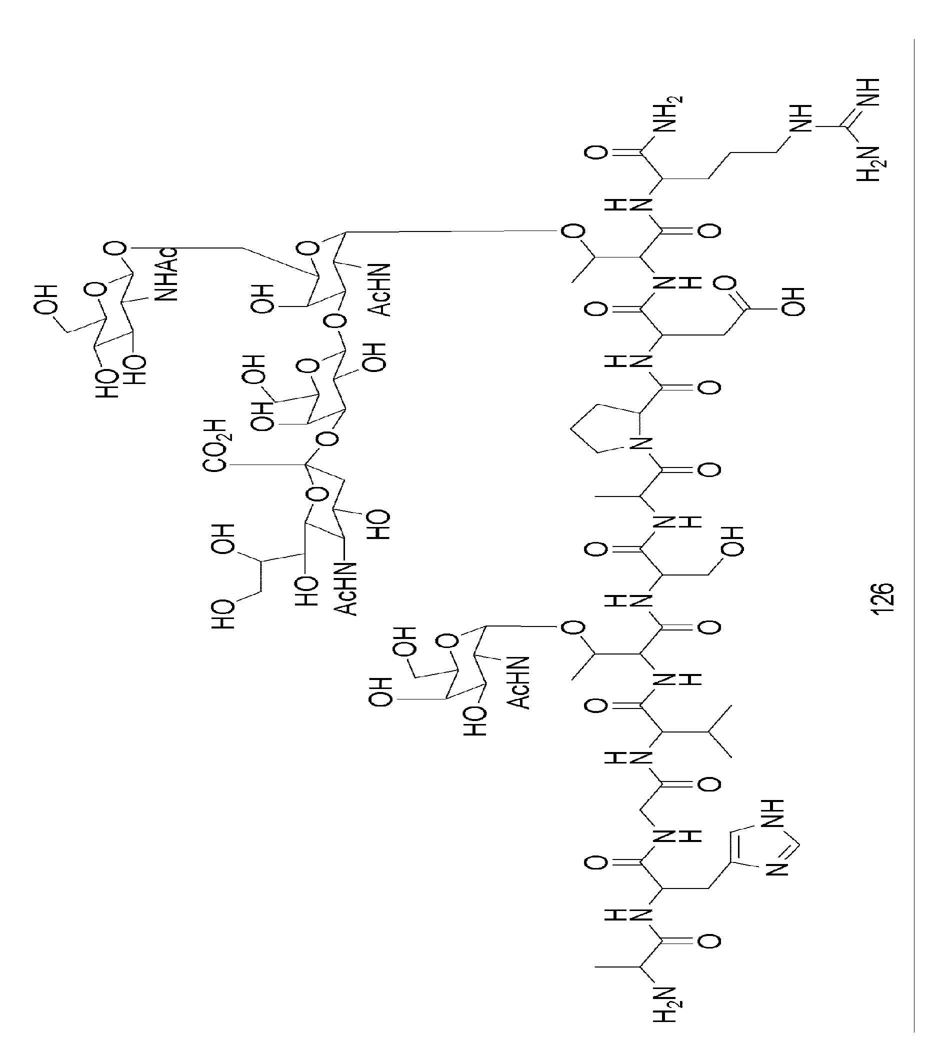

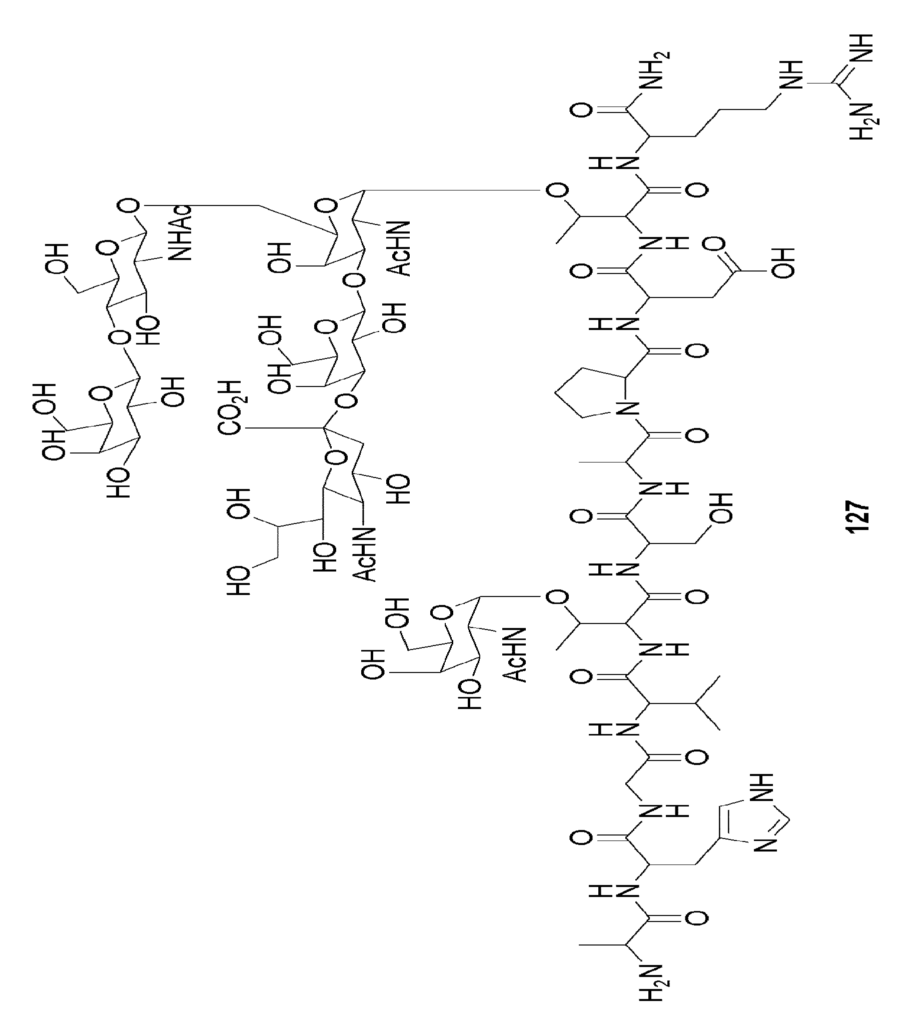

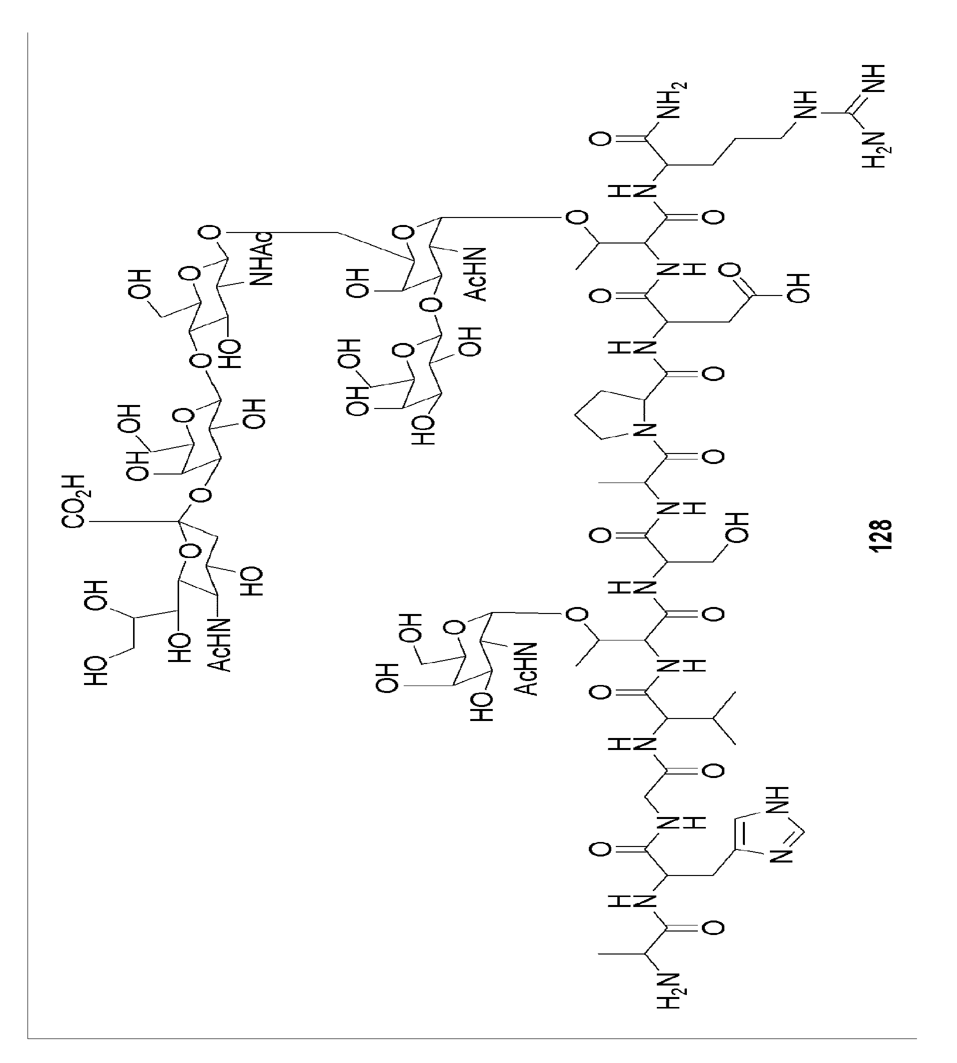

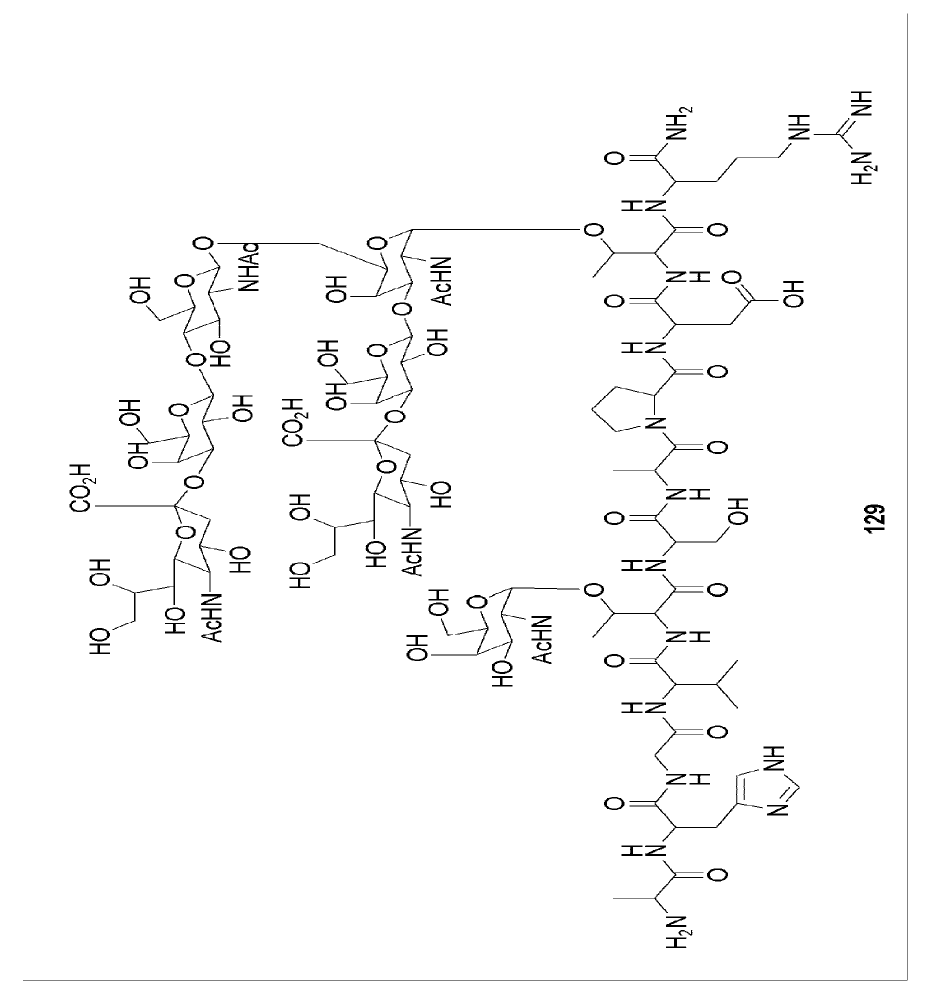

The following reaction solutions A) to E) were allowed to react for 24 hours at 25°C:
A) 250 µl of reaction solution containing 50 mM HEPES buffer solution (pH 7.0), 0.1U/ml human-derived β1,4-galactosyltransferase (available from TOYOBO CO., LTD.), 10mM manganese chloride, 0.1% BSA, 2mM uridine-5'-disodium diphosphogalactose (UDP-Gal) and glycopeptide derivative (87) (4mM at a theoretical content from solid-phase synthesis);
B) 250µl of reaction solution containing 50mM HEPES buffer solution (pH 7.0), 0.0175U/ml recombinant rat α2,3-(O)-sialyltransferase (available from Calbiochem), 10mM manganese chloride, 0.1% BSA, 2mM cytidine-5'-sodium monophosphosialate (CMP-NANA) and glycopeptide derivative (87) (4mM at a theoretical content from solid-phase synthesis);
C) 250µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (87) (4mM at a theoretical content from solid-phase synthesis);
D) 250µl of reaction solution containing 50mM HEPES buffer solution (pH 7.0), 0.1U/ml human-derived β1,4-galactosyltransferase (available from TOYOBO CO., LTD.), 0.0185U/ml recombinant rat α2,3-(N)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (87) (4mM at a theoretical content from solid-phase synthesis);
E) 250µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 0.0185U/ml recombinant rat α2,3-(N)-sialyltransferase (available from Calbiochem), 10mM manganese chloride, 0.1% BSA, 4mM uridine-5'-disodium diphosphogalactose (UDP-Gal), 2mM cytidine-5'-sodium monophosphosialate (CMP-NANA) and glycopeptide derivative (87) (4mM at a theoretical content from solid-phase synthesis).

After completion of the reaction, the respective reaction solutions were transferred to an ultrafiltration filter, ULTRAFRE-MC 10,000NMWL Filter Unit (available from Millipore) and were subjected to centrifugal concentration. Thereafter, 25mM ammonium acetate buffer solution (pH 6.5) was added thereto, and the solution was again subjected to concentration with a centrifugal separator, thereby washing the polymer. This manipulation was repeated three times, thereby respectively obtaining aqueous solutions of compounds (119) to (123). Thereafter, to 150µl of the solution retained at the filter containing compounds (119) to (123) and 150µl solution which was obtained by diluting 100µl of aqueous solution of (87) with 25mM ammonium acetate buffer solution (pH 6.5), 1µl of 1.74mg/ml solution of BLase (available from Shionogi & Co., Ltd.) was added, and the solution was allowed to react for two hours at room temperature. Thereafter, the solution was subjected to centrifugal filtration with an ultrafiltration filter, ULTRAFRE-MC 10,000NMWL Filter Unit (available from Millipore), thereby separating the glycopeptide of interest from the polymer. The obtained aqueous solution (filtrate) was lyophilized, thereby obtaining compounds (124) to (129). MALDI TOF/MS:[M(average)+H]⁺ of compound (124)=1882.2 (theoretical value:[M(average)+H]⁺=1881.9; MALDI TOF/MS:[M(average)+H]⁺ of compound (125)=2044.5 (theoretical value:[M(average)+H]⁺=2043.9); MALDI TOF/MS:[M(average)+H]⁺ of compound (126)=2173.4 (theoretical value:[M(average)+H]⁺=2173.0); MALDI TOF/MS:[M(average)+H]⁺ of compound (127)=2335.4 (theoretical value:[M(average)+H]⁺=2335.0); MALDI TOF/MS:[M(average)+H]⁺ of compound (128)=2335.3 (theoretical value:[M(average)+H]⁺=2335.0); and MALDI TOF/MS:[M(average)+H]⁺ of compound (129)=2626.1 (theoretical value:[M(average)+H]⁺=2626.0).

(3.24 Synthesis of (130) to (140)

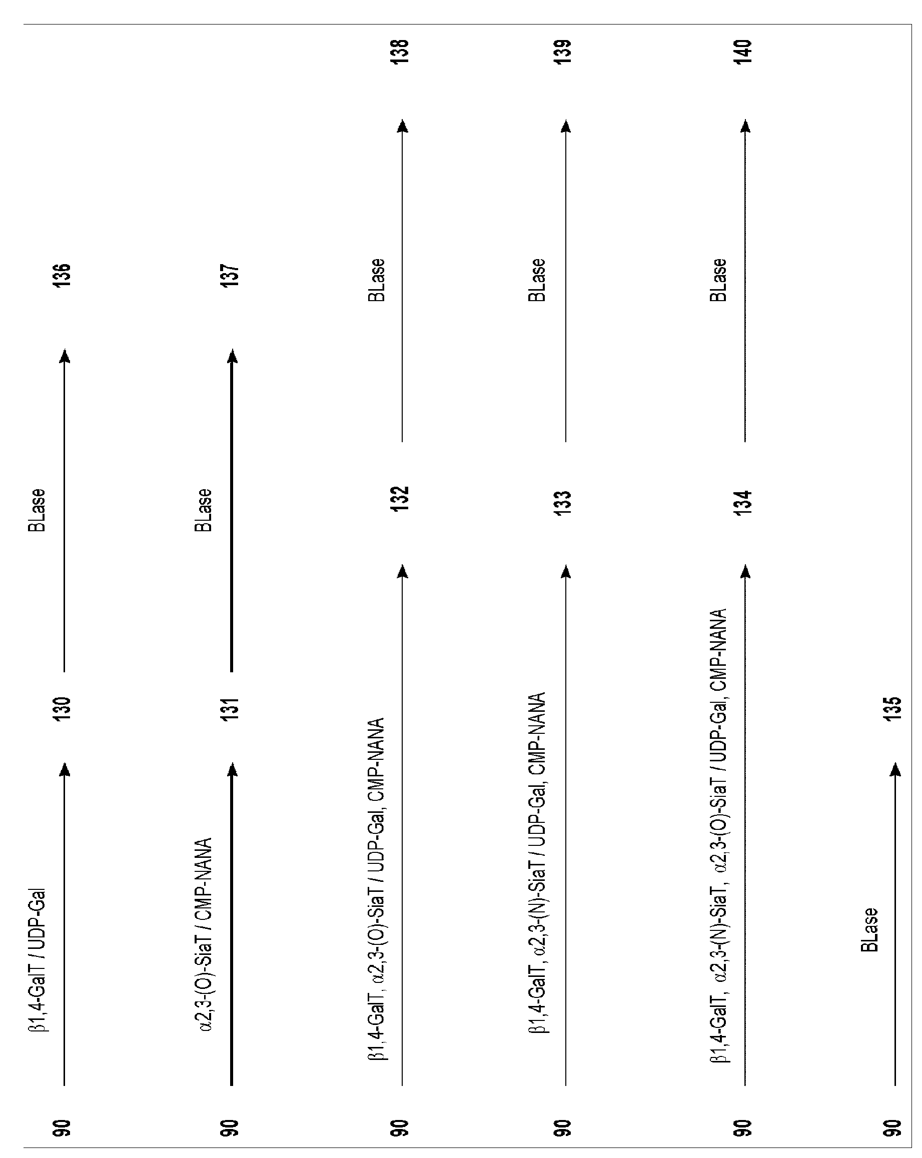

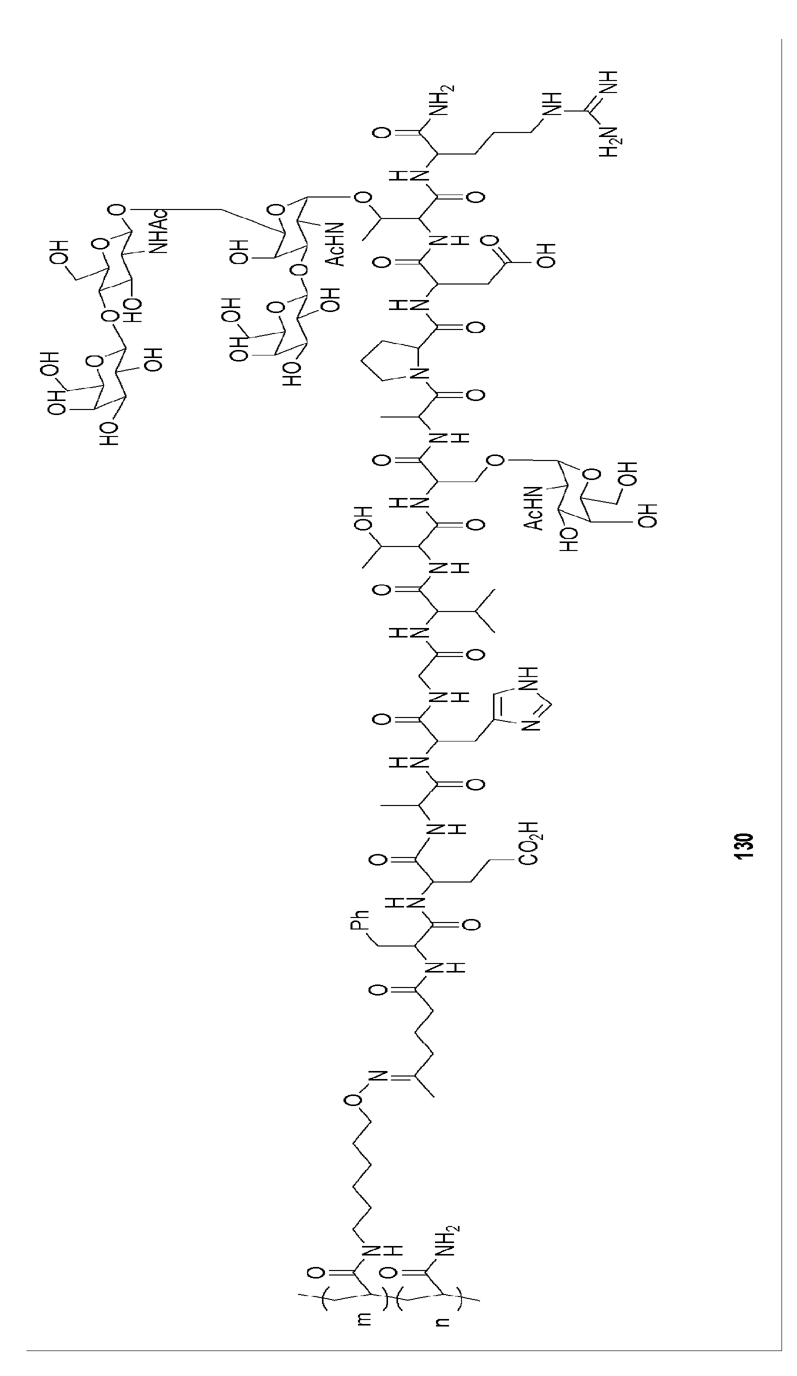

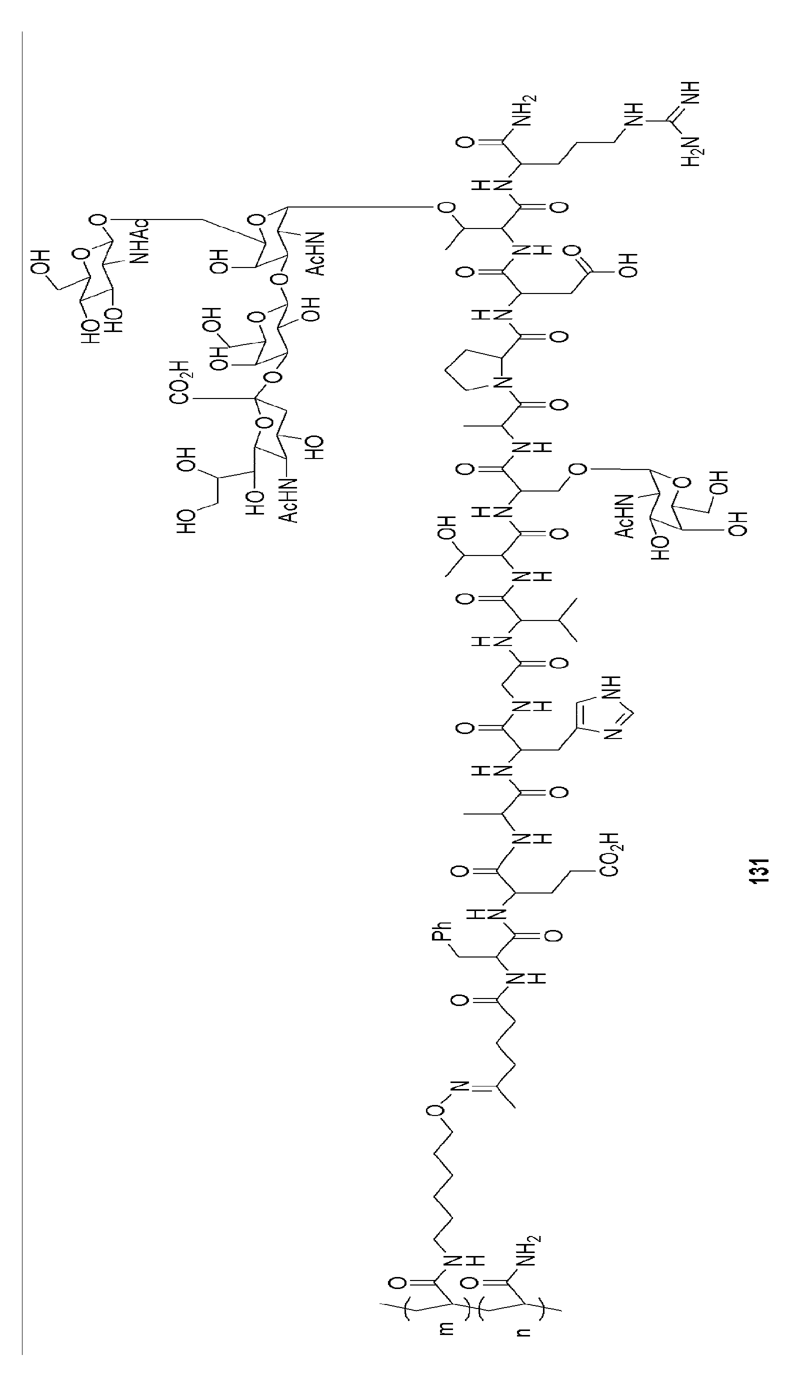

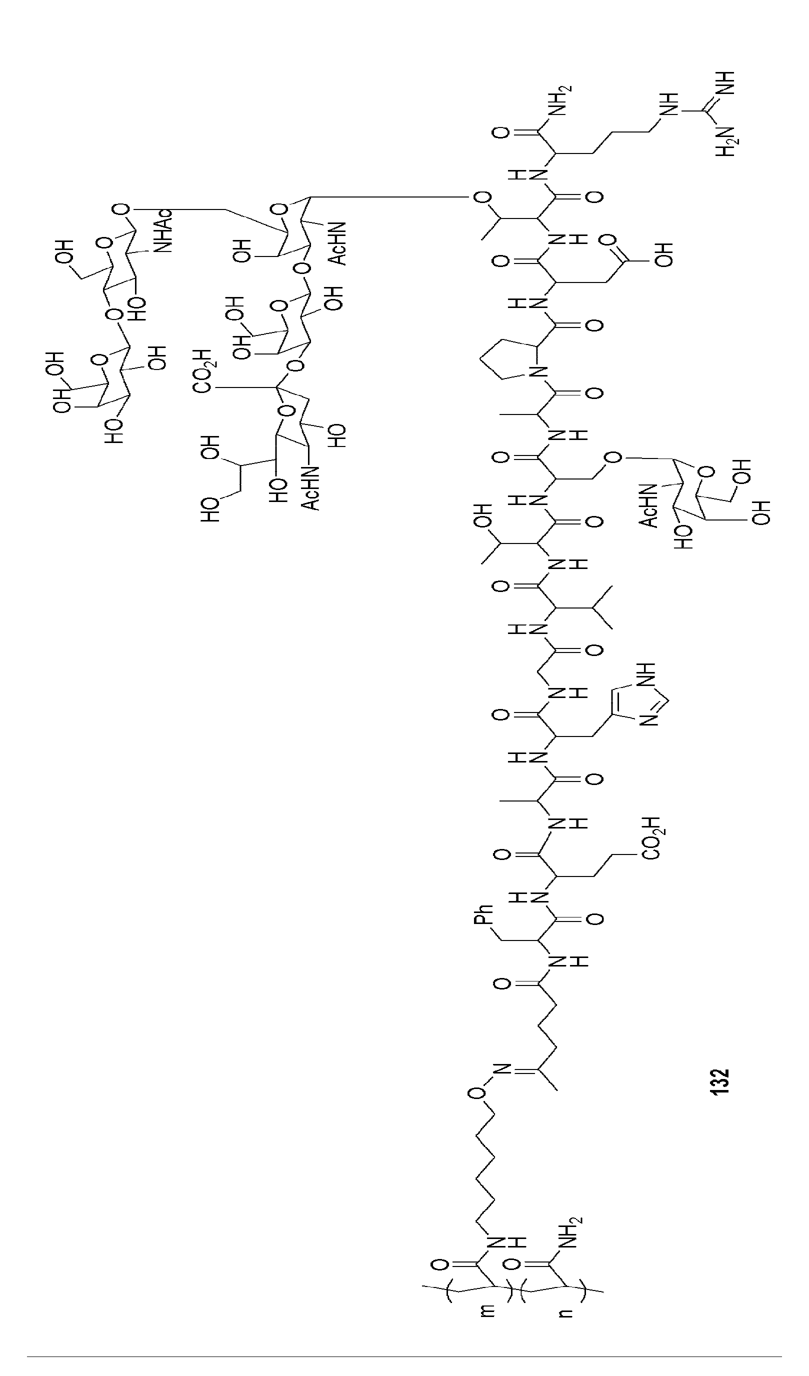

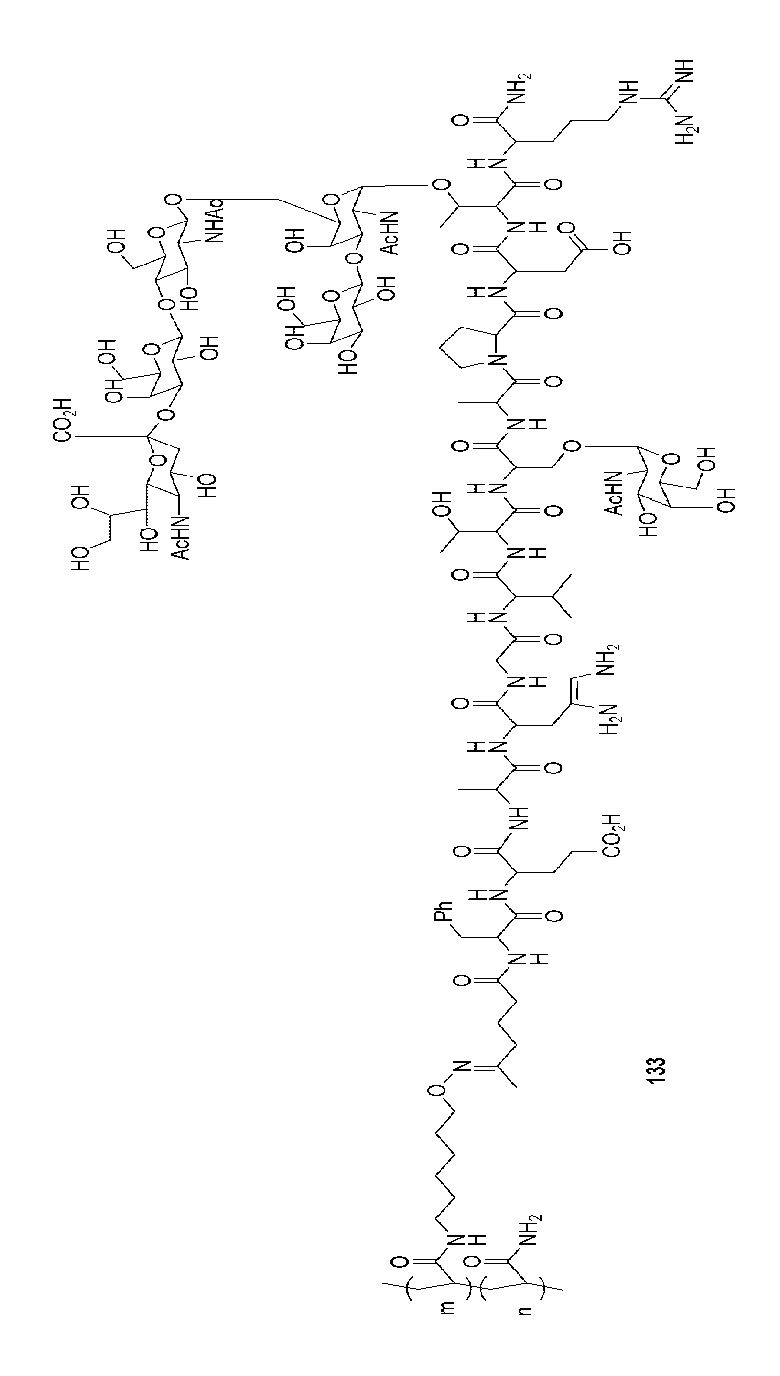

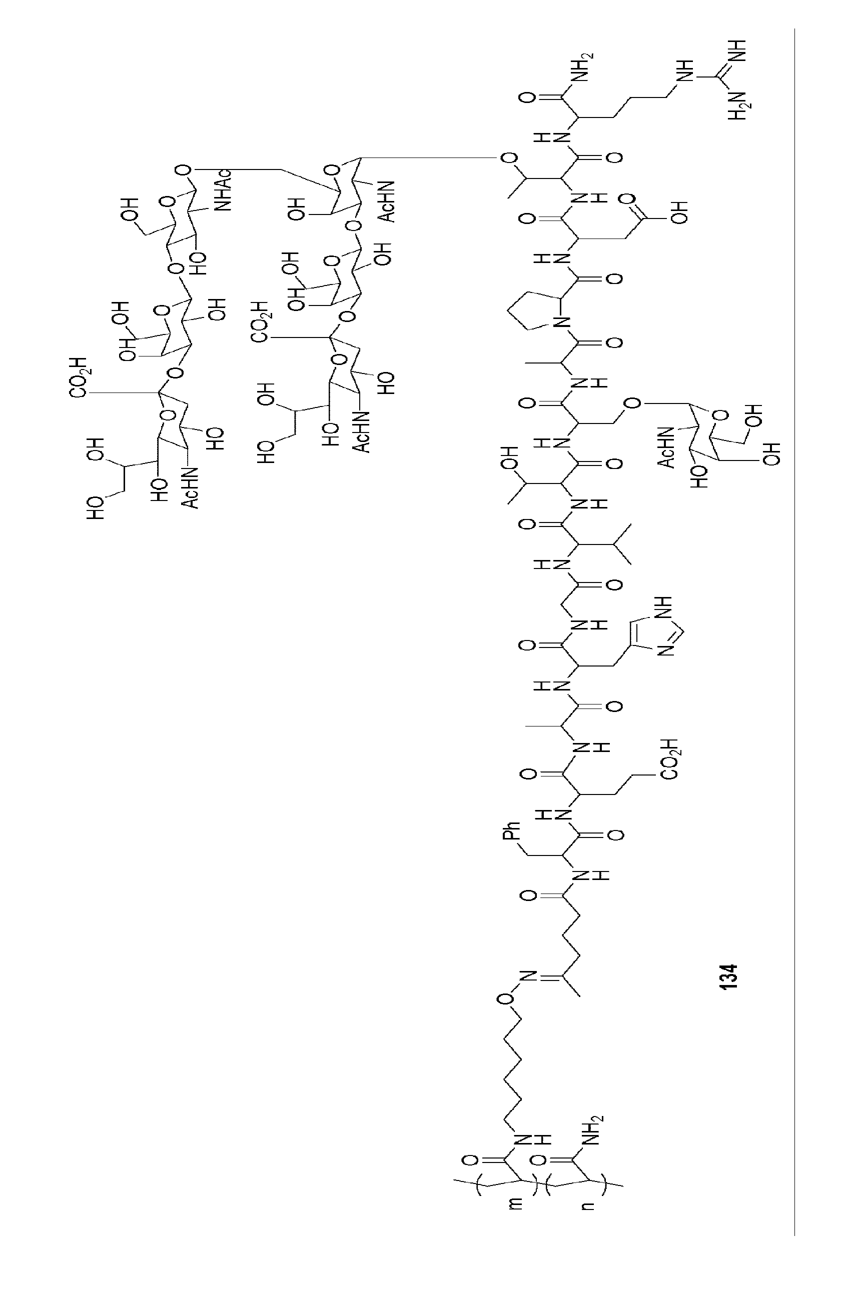

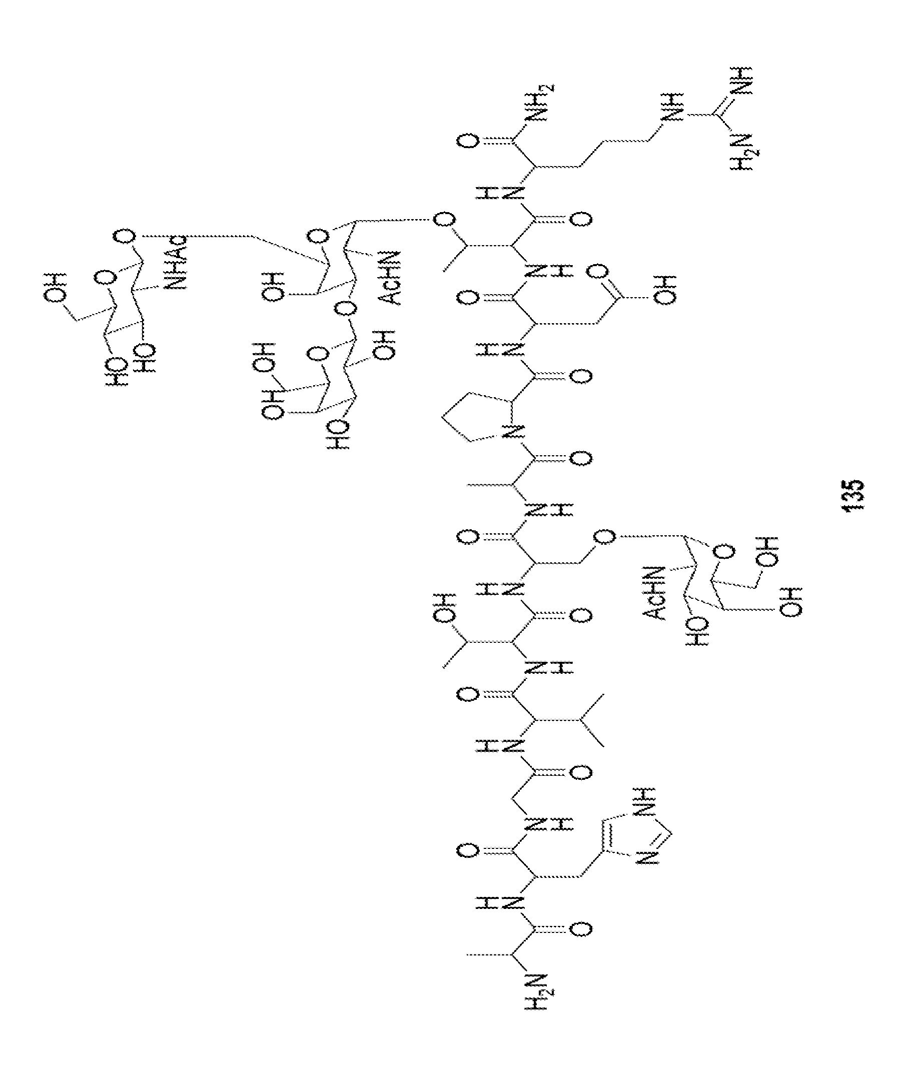

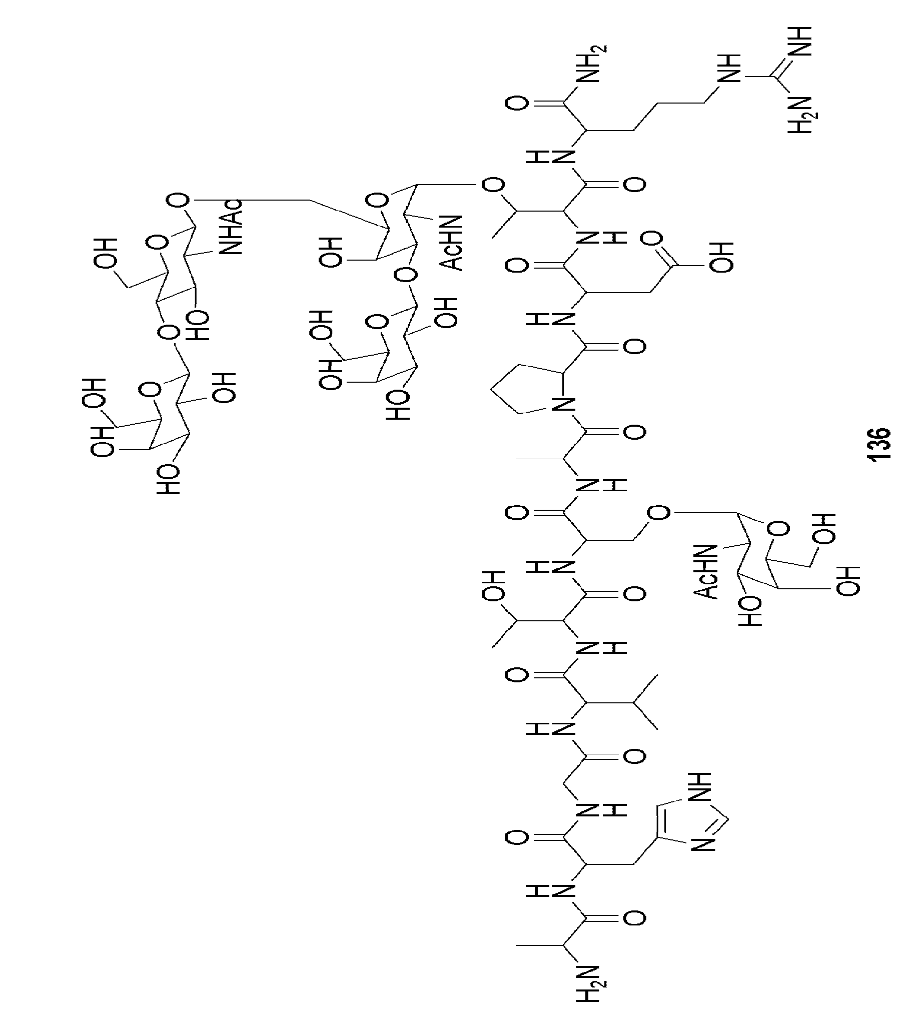

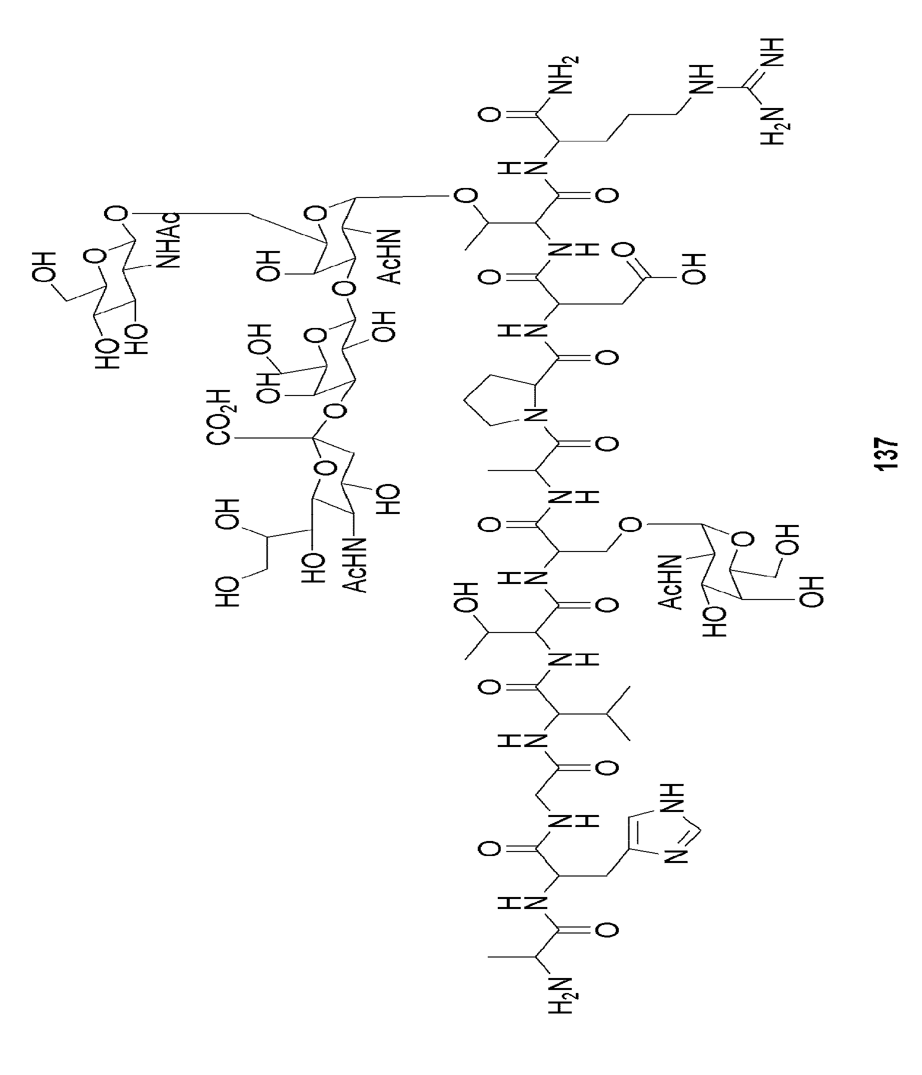

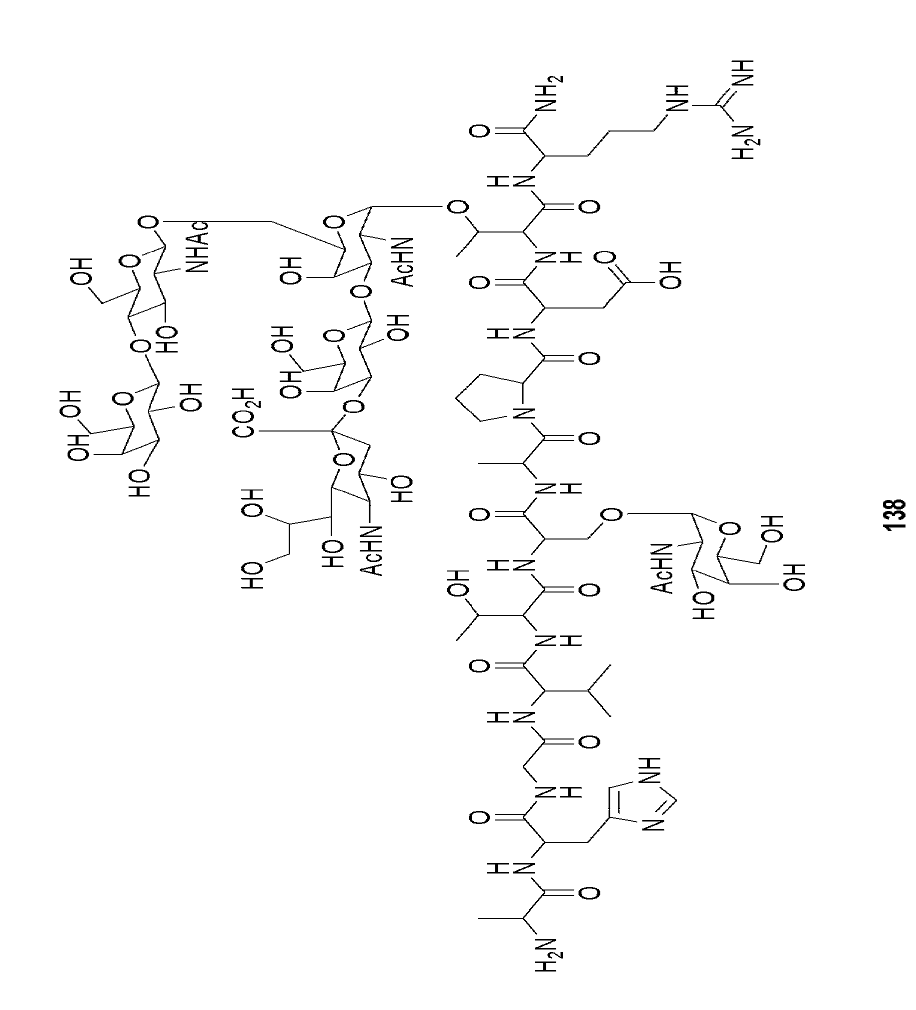

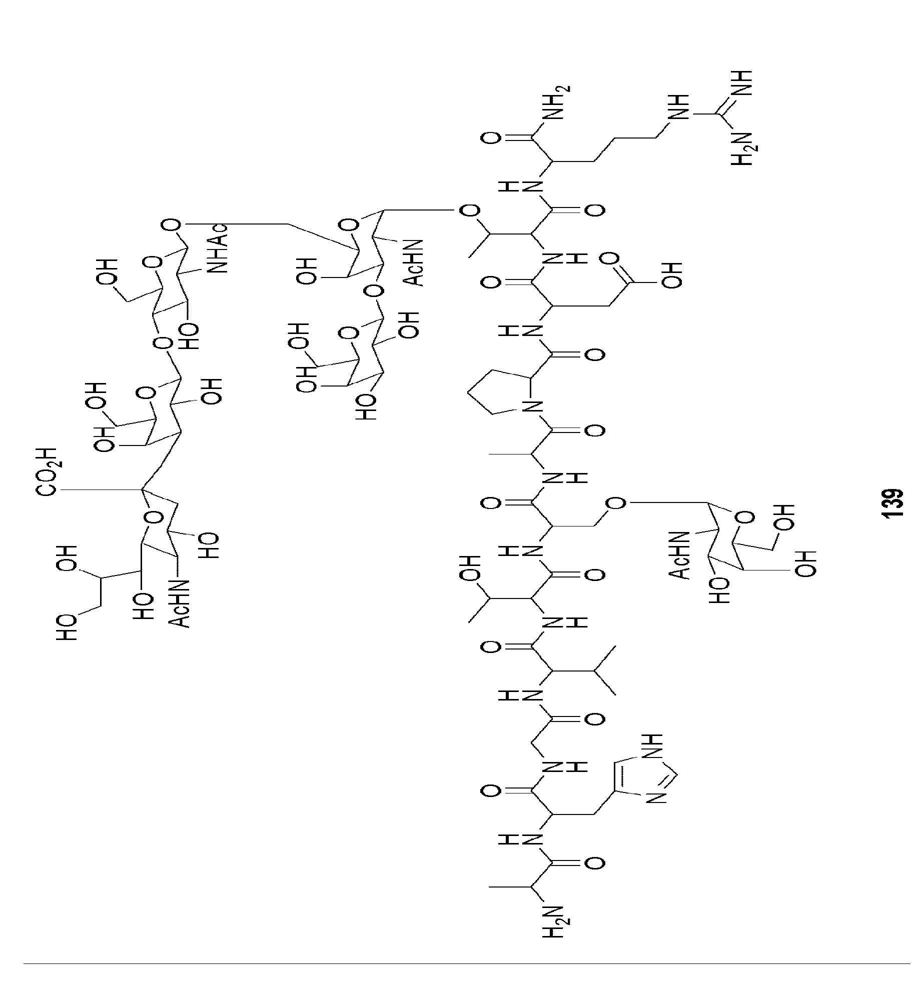

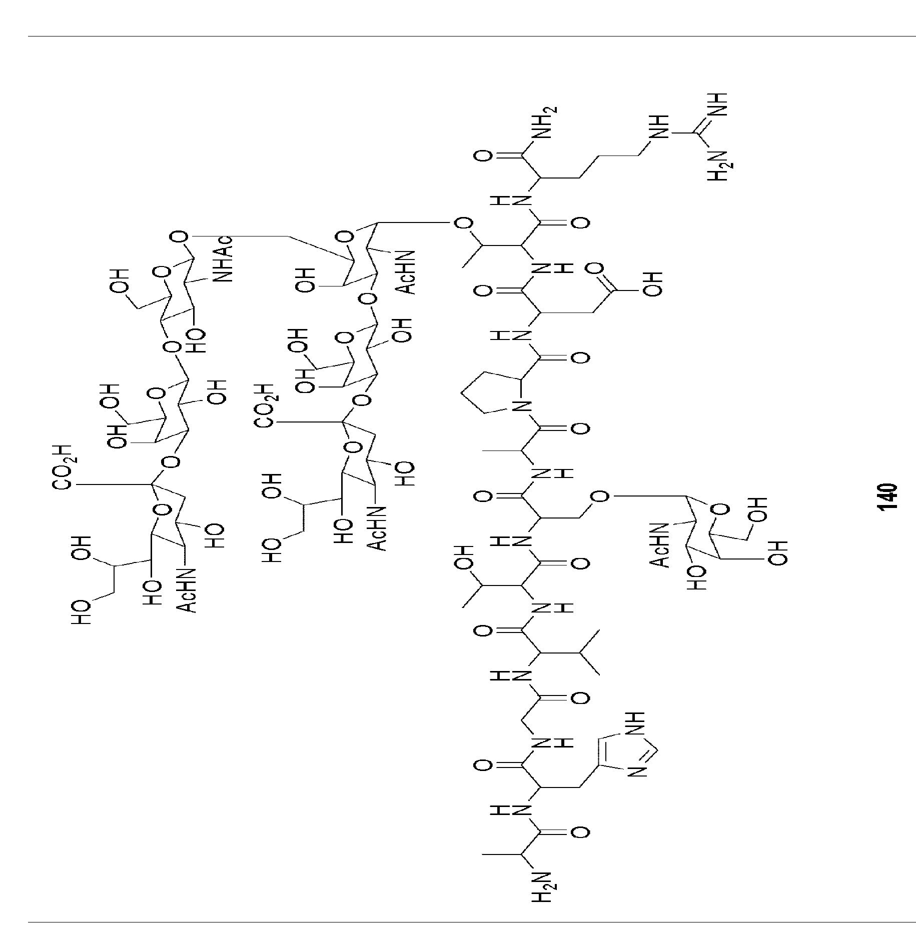

The following reaction solutions A) to E) were allowed to react for 24 hours at 25°C:
A) 250µl of reaction solution containing 50mM HEPES buffer solution (pH 7.0), 0.1U/ml human-derived β1,4-galactosyltransferase (available from TOYOBO CO., LTD.), 10mM manganese chloride, 0.1% BSA, 2mM uridine-5'-disodium diphosphogalactose (UDP-Gal) and glycopeptide derivative (90) (4mM at a theoretical content from solid-phase synthesis);
B) 250µl of reaction solution containing 50mM HEPES buffer solution (pH 7.0), 0.0175U/ml recombinant rat α2,3-(O)-sialyltransferase (available from Calbiochem), 10mM manganese chloride, 0.1% BSA, 2mM cytidine-5'-sodium monophosphosialate (CMP-NANA) and glycopeptide derivative (90) (4mM at a theoretical content from solid-phase synthesis);
C) 250µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (90) (4mM at a theoretical content from solid-phase synthesis);
D) 250µl of reaction solution containing 50mM HEPES buffer solution (pH 7.0), 0.1U/ml human-derived β1,4-galactosyltransferase (available from TOYOBO CO., LTD.), 0.0185U/ml recombinant rat α2,3-(N)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (90) (4mM at a theoretical content from solid-phase synthesis);
E) 250µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 0.0185U/ml recombinant rat α2,3-(N)-sialyltransferase (available from Calbiochem), 10mM manganese chloride, 0.1% BSA, 4mM uridine-5'-disodium diphosphogalactose (UDP-Gal), 2mM cytidine-5'-sodium monophosphosialate (CMP-NANA) and glycopeptide derivative (90) (4mM at a theoretical content from solid-phase synthesis).

After completion of the reaction, the respective reaction solutions were transferred to an ultrafiltration filter, ULTRAFRE-MC 10,000NMWL Filter Unit (available from Millipore) and were subjected to centrifugal concentration. Thereafter, 25mM ammonium acetate buffer solution (pH 6.5) was added thereto, and the solution was again subjected to concentration with a centrifugal separator, thereby washing the polymer. This manipulation was repeated three times, thereby respectively obtaining aqueous solutions of compounds (130) to (134). Thereafter, to 150µl of the solution retained at the filter containing compounds (130) to (134) and 150µl solution which was obtained by diluting 100µl of aqueous solution of (90) with 25mM ammonium acetate buffer solution (pH 6.5), 1µl of 1.74mg/ml solution of BLase (available from Shionogi & Co., Ltd.) was added, and the solution was allowed to react for two hours at room temperature. Thereafter, the solution was subjected to centrifugal filtration with an ultrafiltration filter, ULTRAFRE-MC 10,000NMWL Filter Unit (available from Millipore), thereby separating the glycopeptide of interest from the polymer. The obtained aqueous solution (filtrate) was lyophilized, thereby obtaining compounds (130) to (134). MALDI TOF/MS:[M(average)+H]⁺ of compound (135)=1882.3 (theoretical value:[M(average)+H]⁺=1881.9); MALDI TOF/MS:[M(average)+H]⁺ of compound (136)=2044.6 (theoretical value:[M(average)+H]⁺=2043.9); MALDI TOF/MS:[M(average)+H]⁺ of compound (137)=2173.6 (theoretical value:[M(average)+H]⁺=2173.0); MALDI TOF/MS:[M(average)+H]⁺ of compound (138)=2335.6 (theoretical value:[M(average)+H]⁺=2335.0); MALDI TOF/MS:[M(average)+H]⁺ of compound (139)=2335.4 (theoretical value: [M(average)+H]⁺=2335.0); and MALDI TOF/MS:[M(average)+H]⁺ of compound (140)=2626.3 (theoretical value:[M(average)+H]⁺=2626.1).

(3.25 Synthesis of (141) to (151)

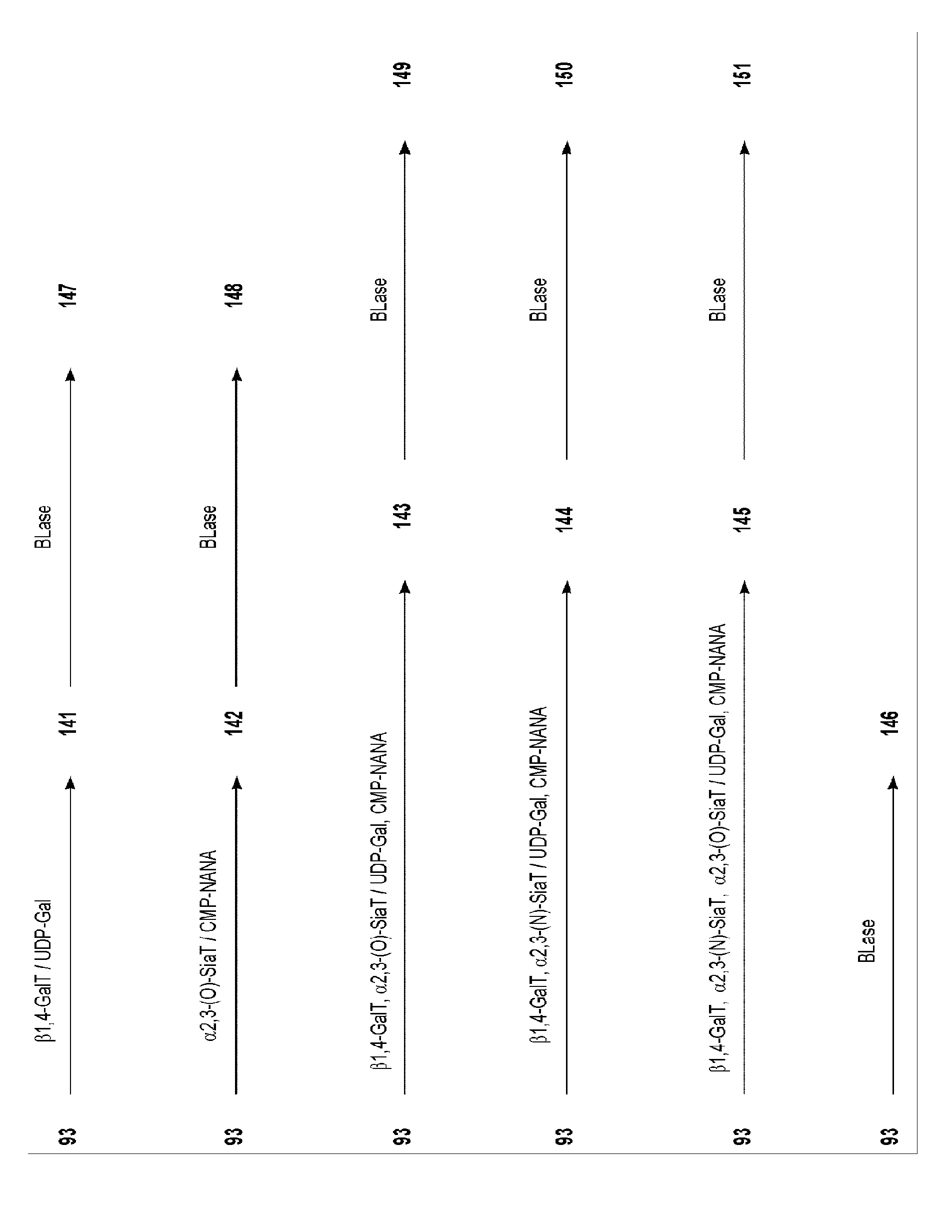

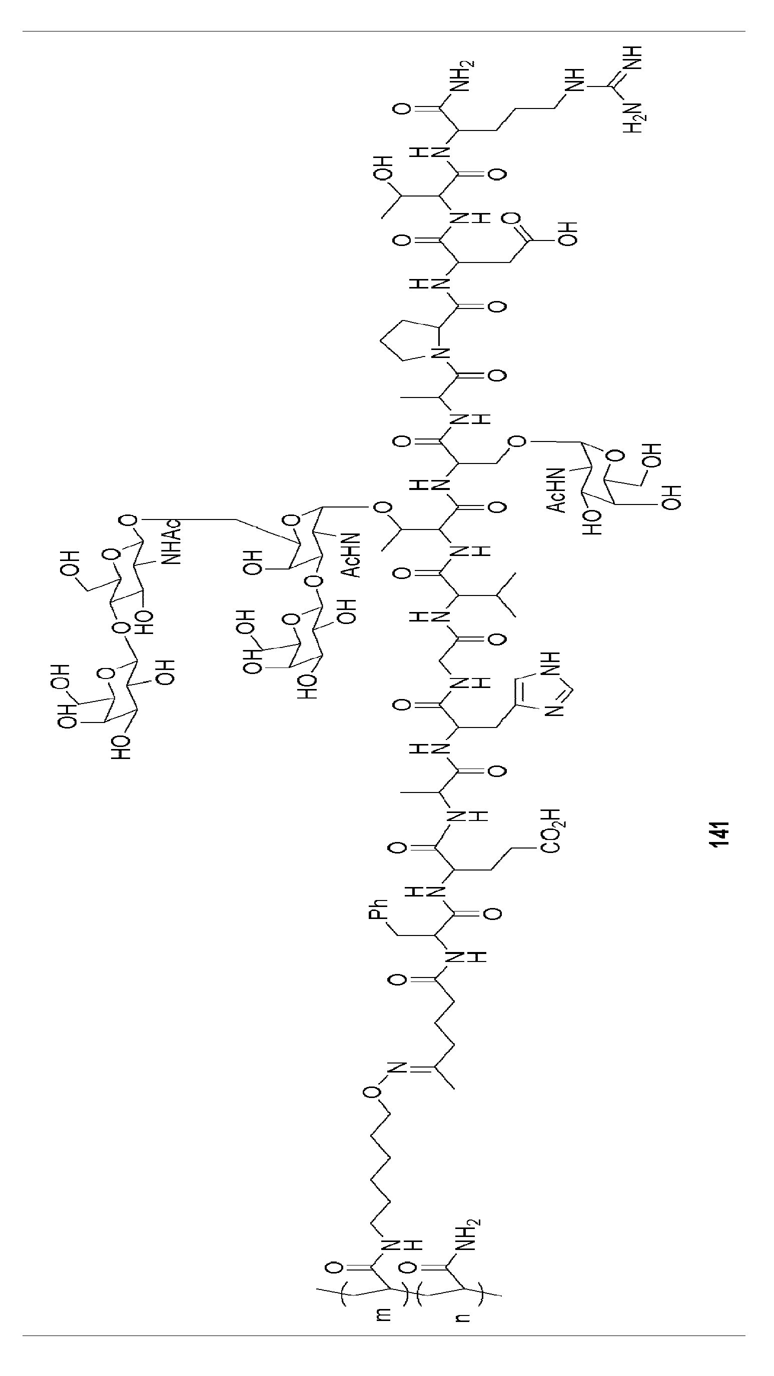

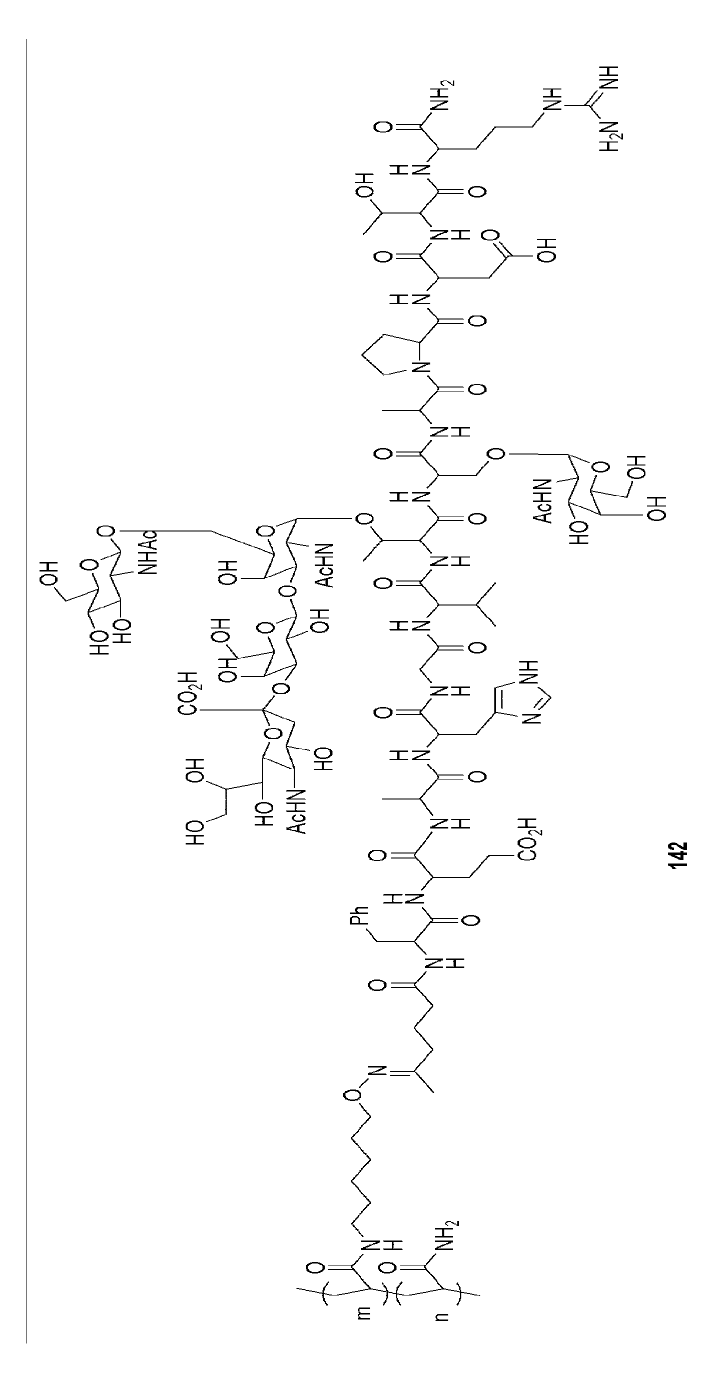

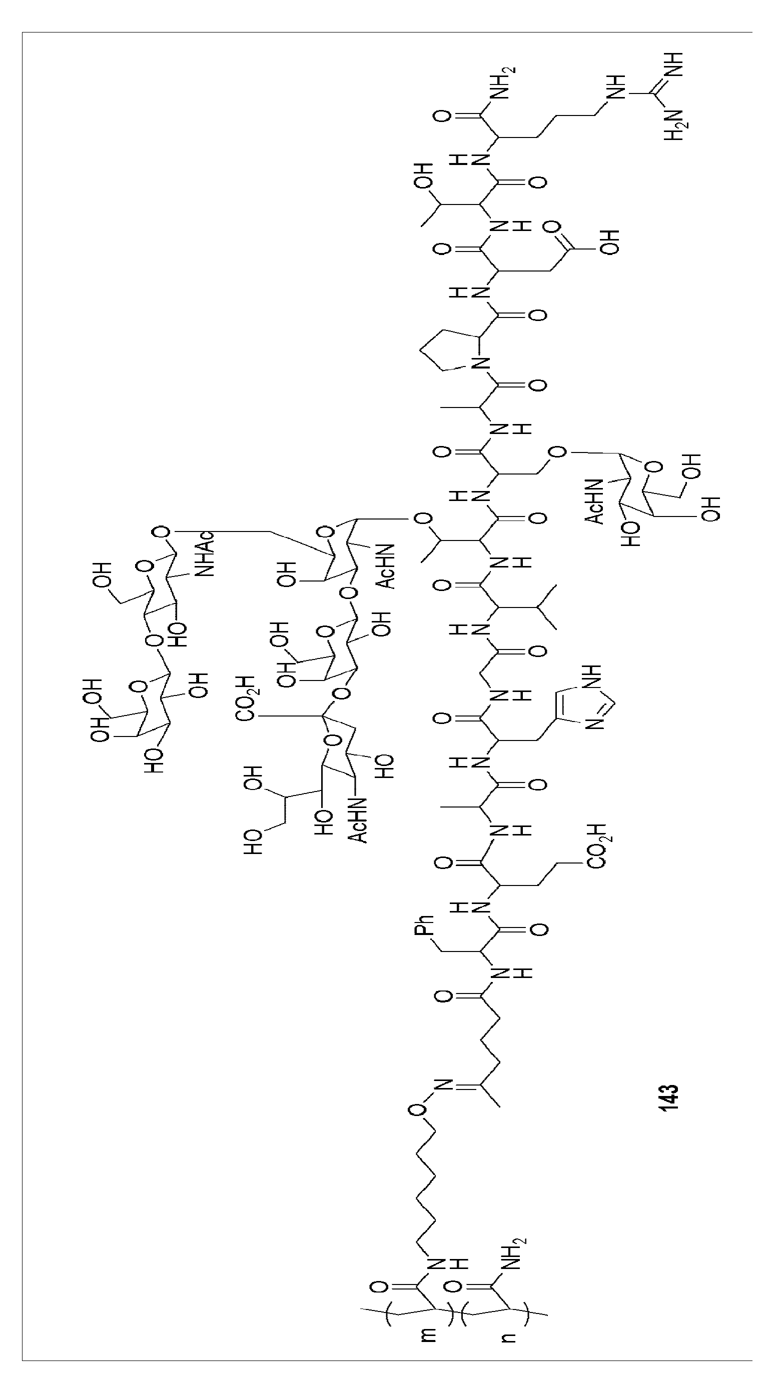

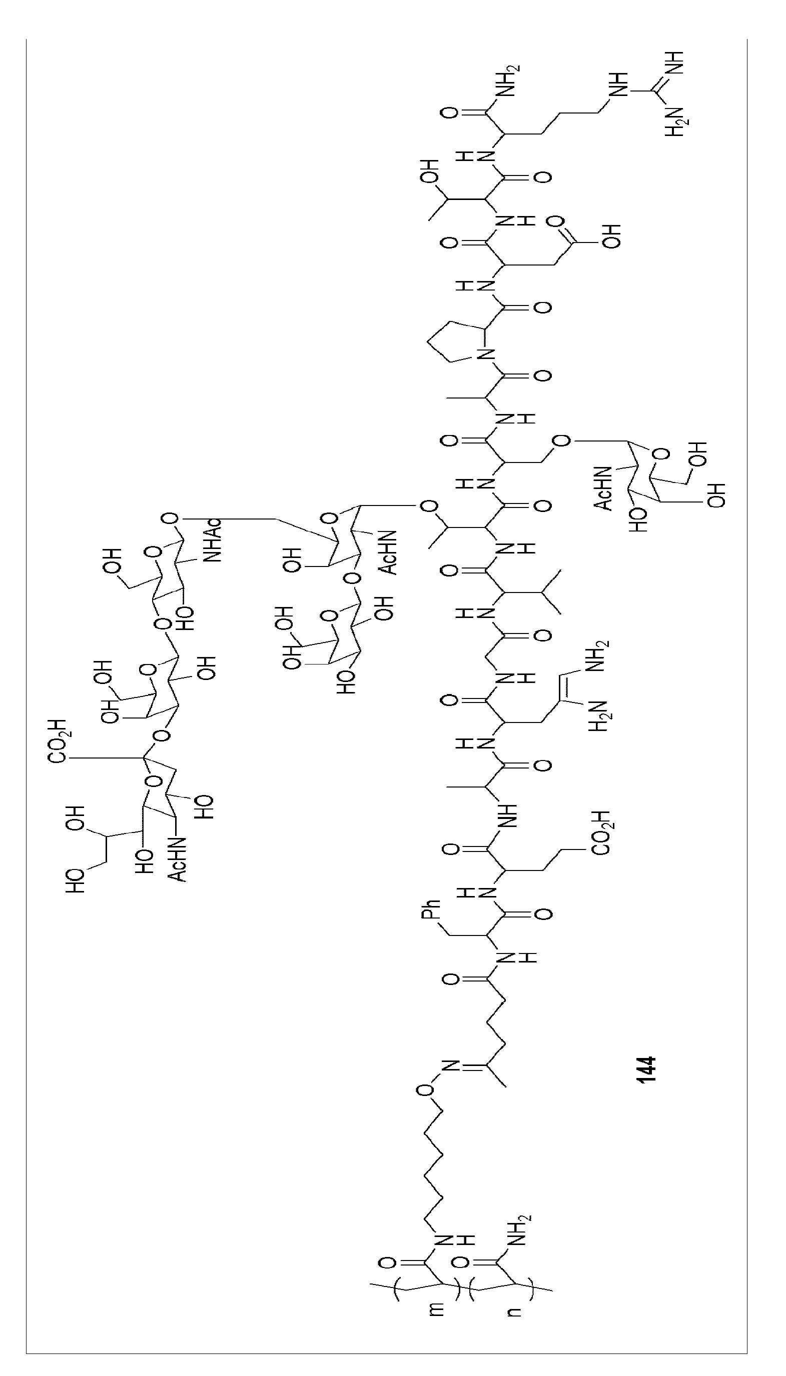

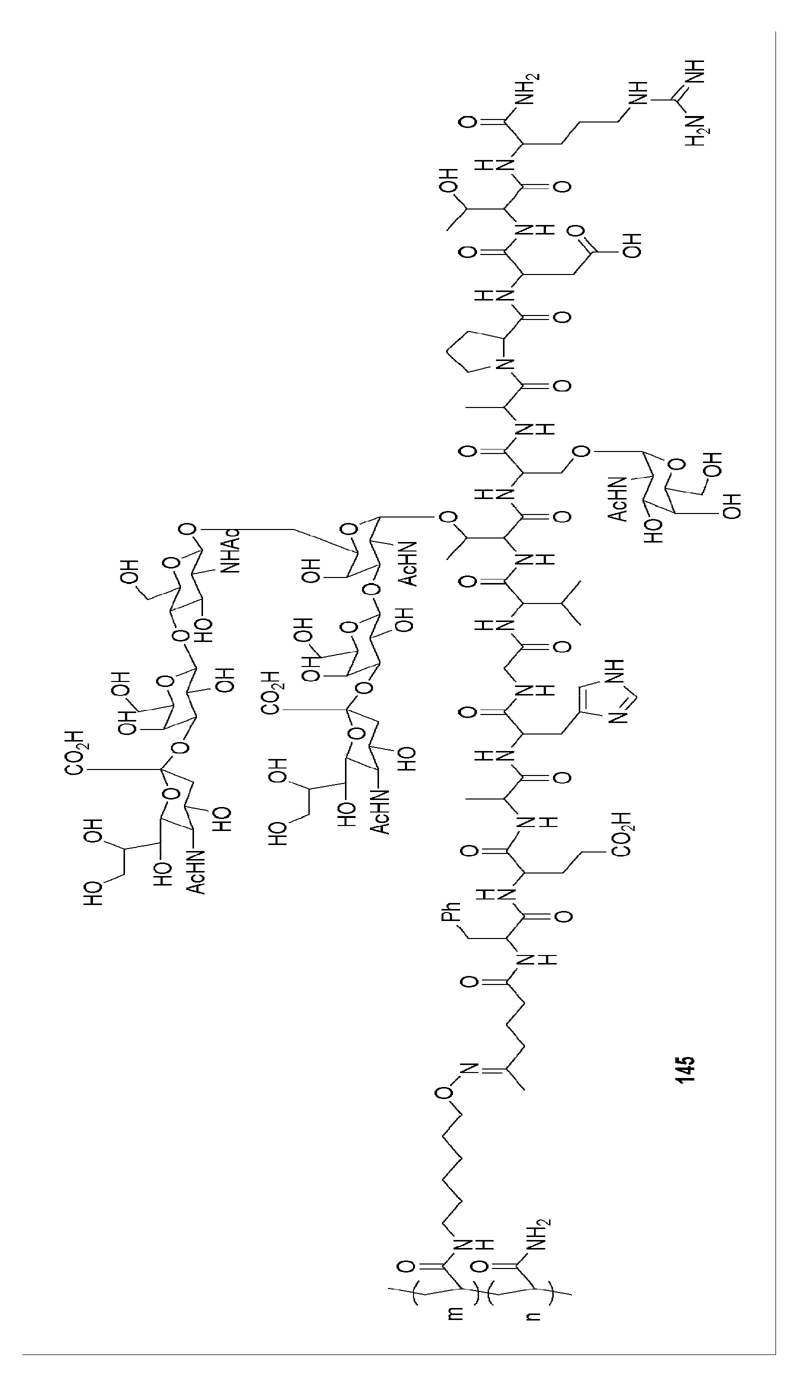

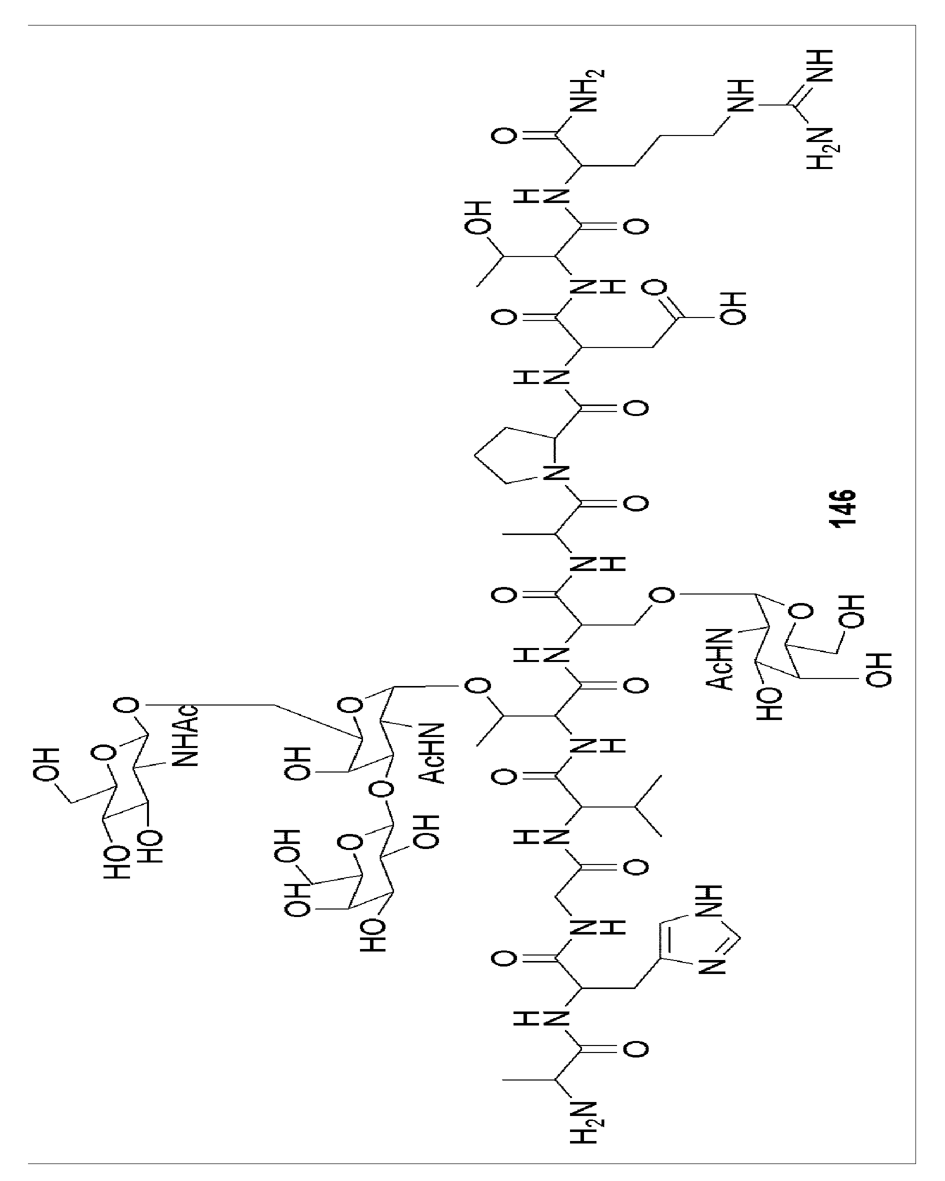

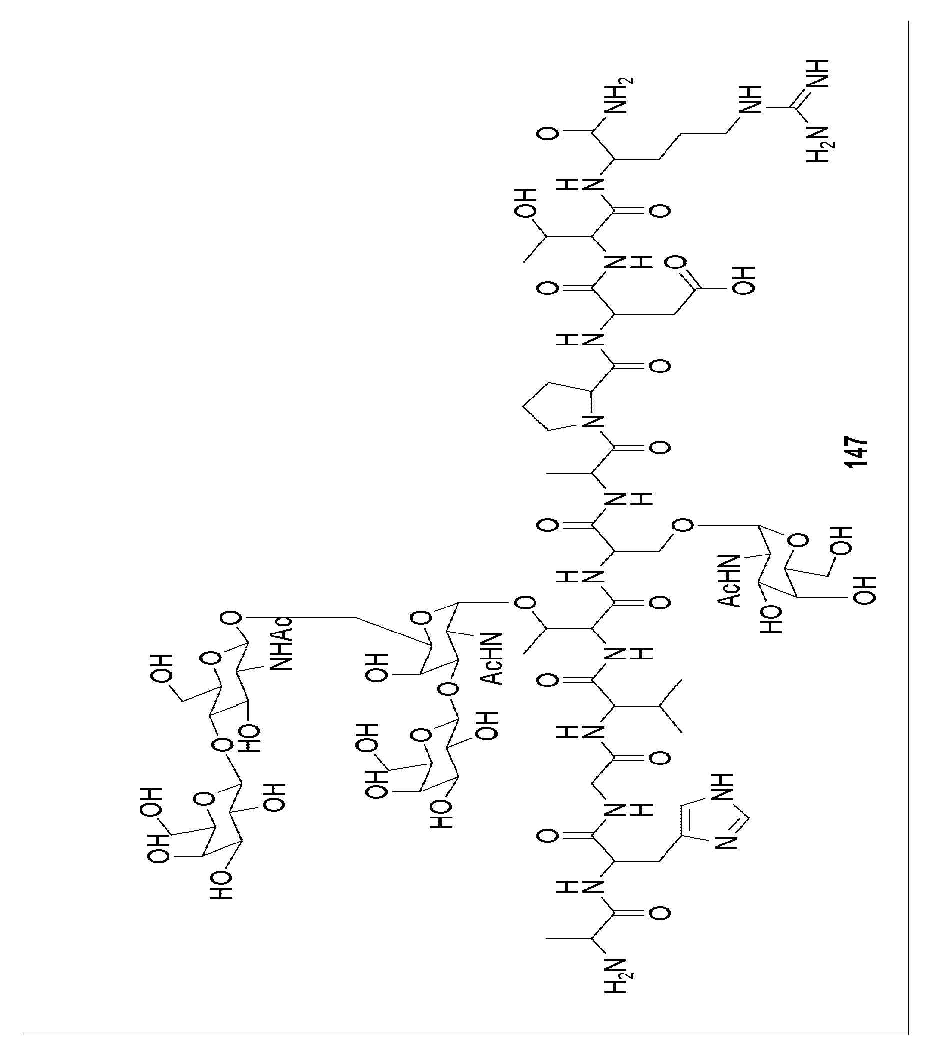

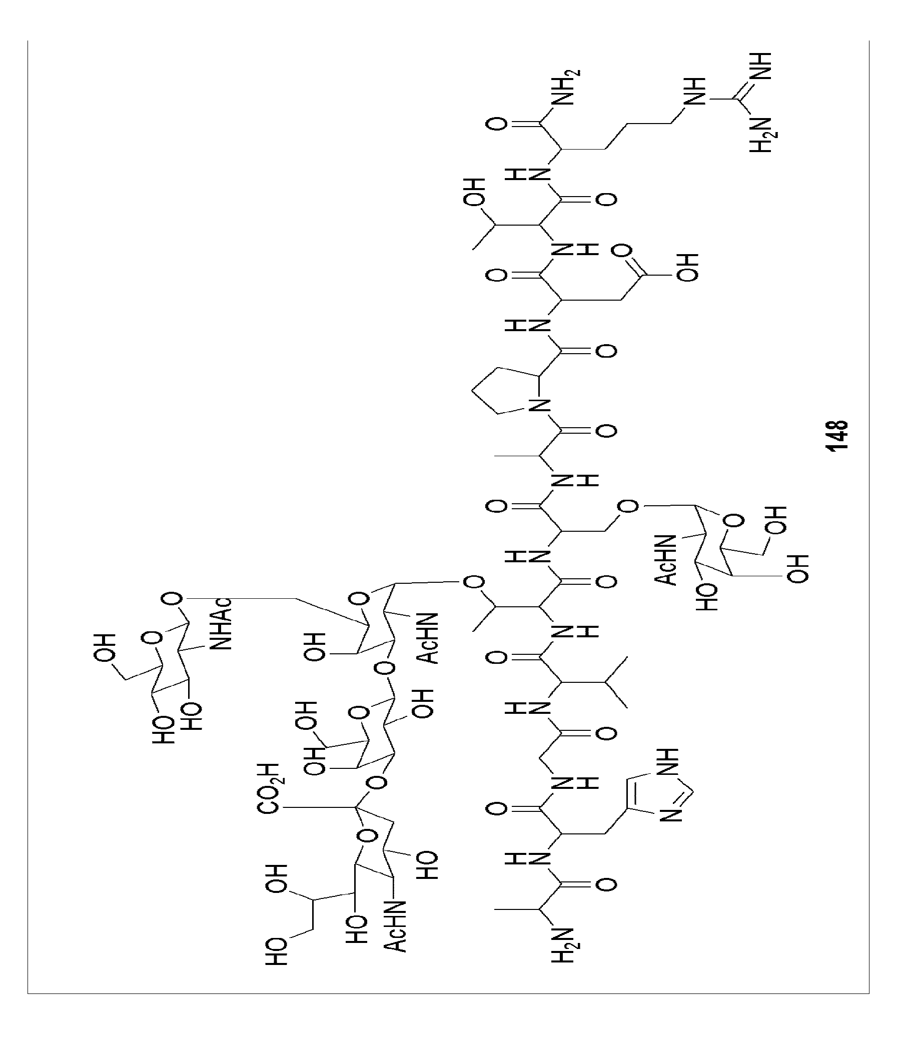

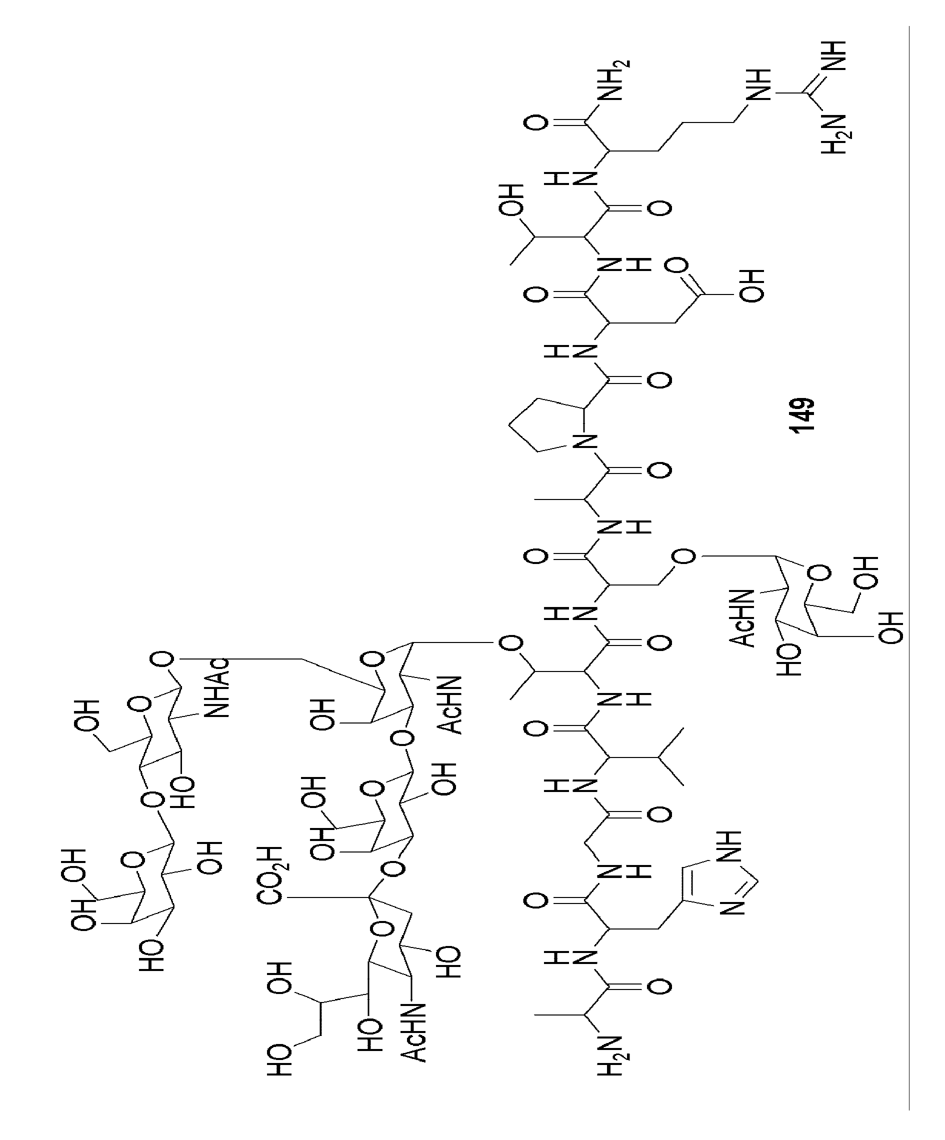

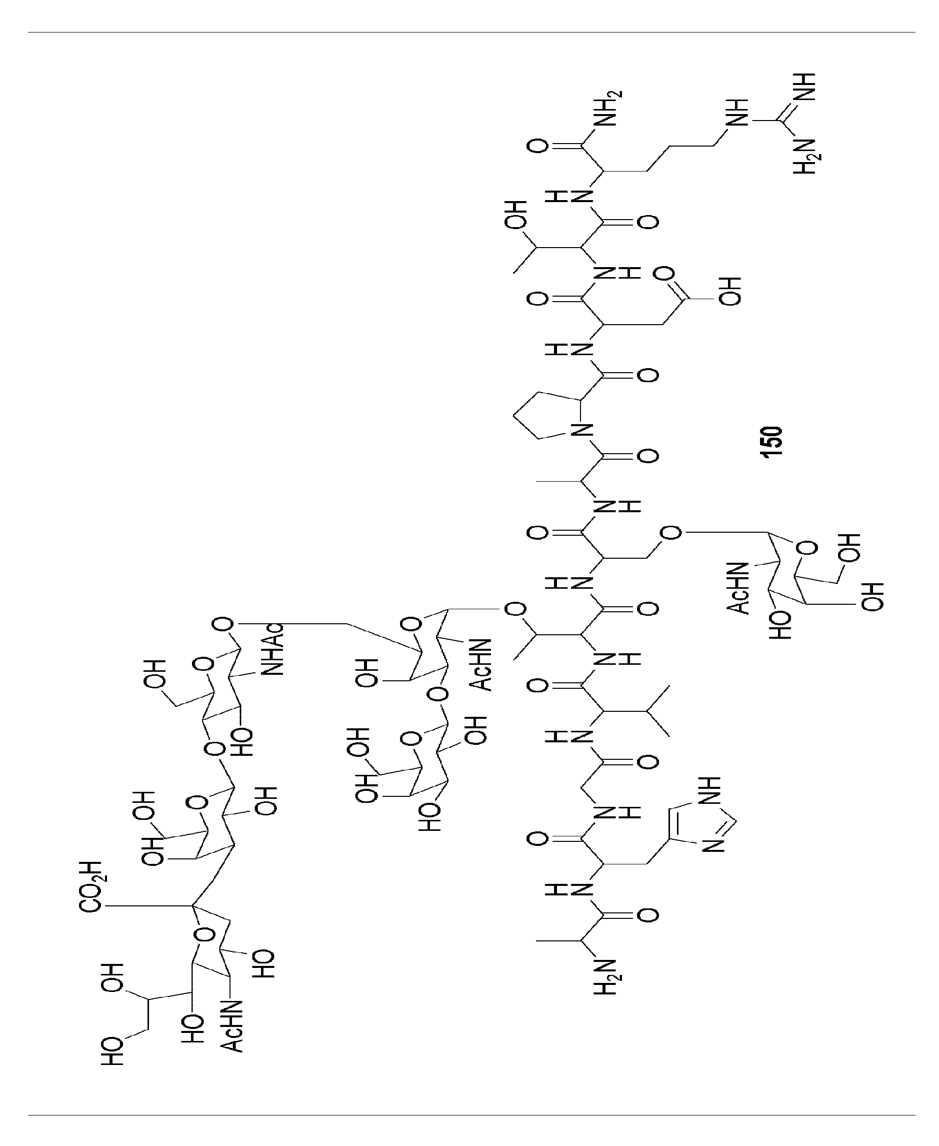

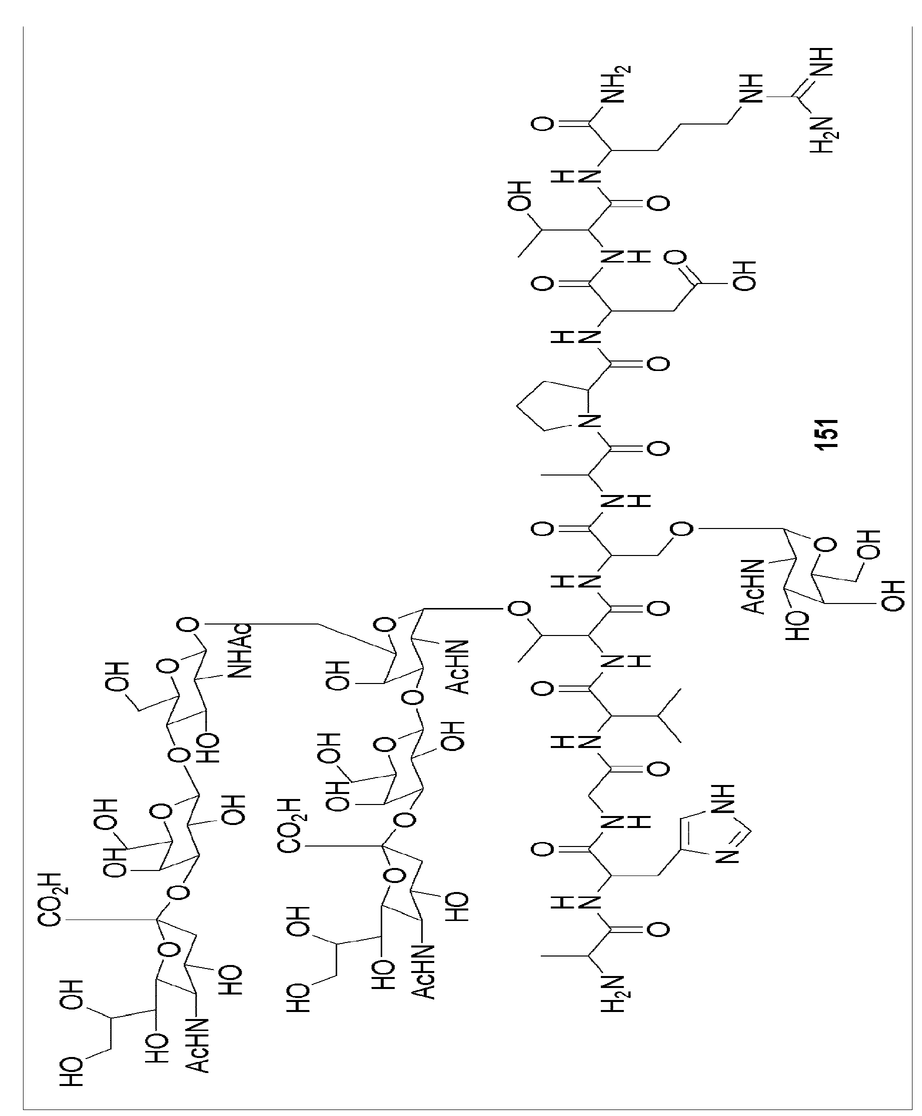

The following reaction solutions A) to E) were allowed to react for 24 hours at 25°C:
A) 250µl of reaction solution containing 50mM HEPES buffer solution (pH 7.0), 0.1U/ml human-derived β1,4-galactosyltransferase (available from TOYOBO CO., LTD.), 10mM manganese chloride, 0.1% BSA, 2mM uridine-5'-disodium diphosphogalactose (UDP-Gal) and glycopeptide derivative (93) (4mM at a theoretical content from solid-phase synthesis);
B) 250µl of reaction solution containing 50mM HEPES buffer solution (pH 7.0), 0.0175U/ml recombinant rat α2,3-(O)-sialyltransferase (available from Calbiochem), 10mM manganese chloride, 0.1% BSA, 2mM cytidine-5'-sodium monophosphosialate (CMP-NANA) and glycopeptide derivative (93) (4mM at a theoretical content from solid-phase synthesis);
C) 250µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (93) (4mM at a theoretical content from solid-phase synthesis);
D) 250µl of reaction solution containing 50mM HEPES buffer solution (pH 7.0), 0.1U/ml human-derived β1,4-galactosyltransferase (available from TOYOBO CO., LTD.), 0.0185U/ml recombinant rat α2,3-(N)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (93) (4mM at a theoretical content from solid-phase synthesis);
E) 250µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 0.0185U/ml recombinant rat α2,3-(N)-sialyltransferase (available from Calbiochem), 10mM manganese chloride, 0.1% BSA, 4mM uridine-5'-disodium diphosphogalactose (UDP-Gal), 2mM cytidine-5'-sodium monophosphosialate (CMP-NANA) and glycopeptide derivative (93) (4mM at a theoretical content from solid-phase synthesis).

After completion of the reaction, the respective reaction solutions were transferred to an ultrafiltration filter, ULTRAFRE-MC 10,000NMWL Filter Unit (available from Millipore) and were subjected to centrifugal concentration. Thereafter, 25mM ammonium acetate buffer solution (pH 6.5) was added thereto, and the solution was again subjected to concentration with a centrifugal separator, thereby washing the polymer. This manipulation was repeated three times, thereby respectively obtaining aqueous solutions of compounds (141) to (145). Thereafter, to 150µl of the solution retained at the filter containing compounds (141) to (145) and 150µl solution which was obtained by diluting 100µl of aqueous solution of (93) with 25mM ammonium acetate buffer solution (pH 6.5), 1µl of 1.74mg/ml solution of BLase (available from Shionogi & Co., Ltd.) was added, and the solution was allowed to react for two hours at room temperature. Thereafter, the solution was subjected to centrifugal filtration with an ultrafiltration filter, ULTRAFRE-MC 10,000NMWL Filter Unit (available from Millipore), thereby separating the glycopeptide of interest from the polymer. The obtained aqueous solution (filtrate) was lyophilized, thereby obtaining compounds (146) to (151). MALDI TOF/MS:[M(average)+H]⁺ of compound (146)=1882.3 (theoretical value:[M(average)+H]⁺=1881.9); MALDI TOF/MS:[M(average)+H]⁺ of compound (147)=2044.6 (theoretical value:[M(average)+H]⁺=2043.9); MALDI TOF/MS:[M(average)+H]⁺ of compound (148)=2173.6 (theoretical value:[M(average)+H]⁺=2173.0); MALDI TOF/MS:[M(average)+H]⁺ of compound (149)=2335.6 (theoretical value:[M(average)+H]⁺=2335.0); MALDI TOF/MS:[M(average)+H]⁺ of compound (150)=2335.4 (theoretical value:[M(average)+H]⁺=2335.0); and MALDI TOF/MS:[M(average)+H]⁺ of compound (151)=2626.3 (theoretical value:[M(average)+H]⁺=2626.1).

(3.26 Synthesis of (152) to (162))

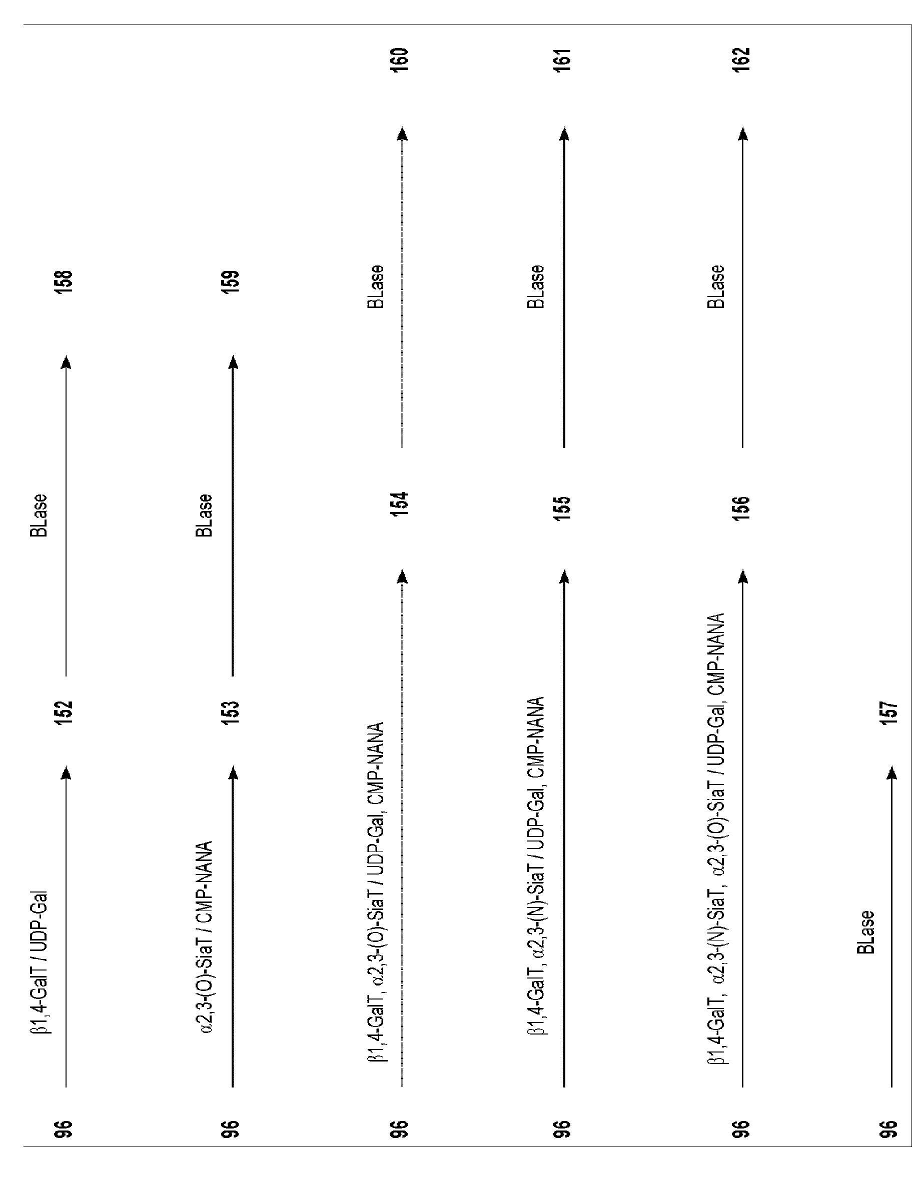

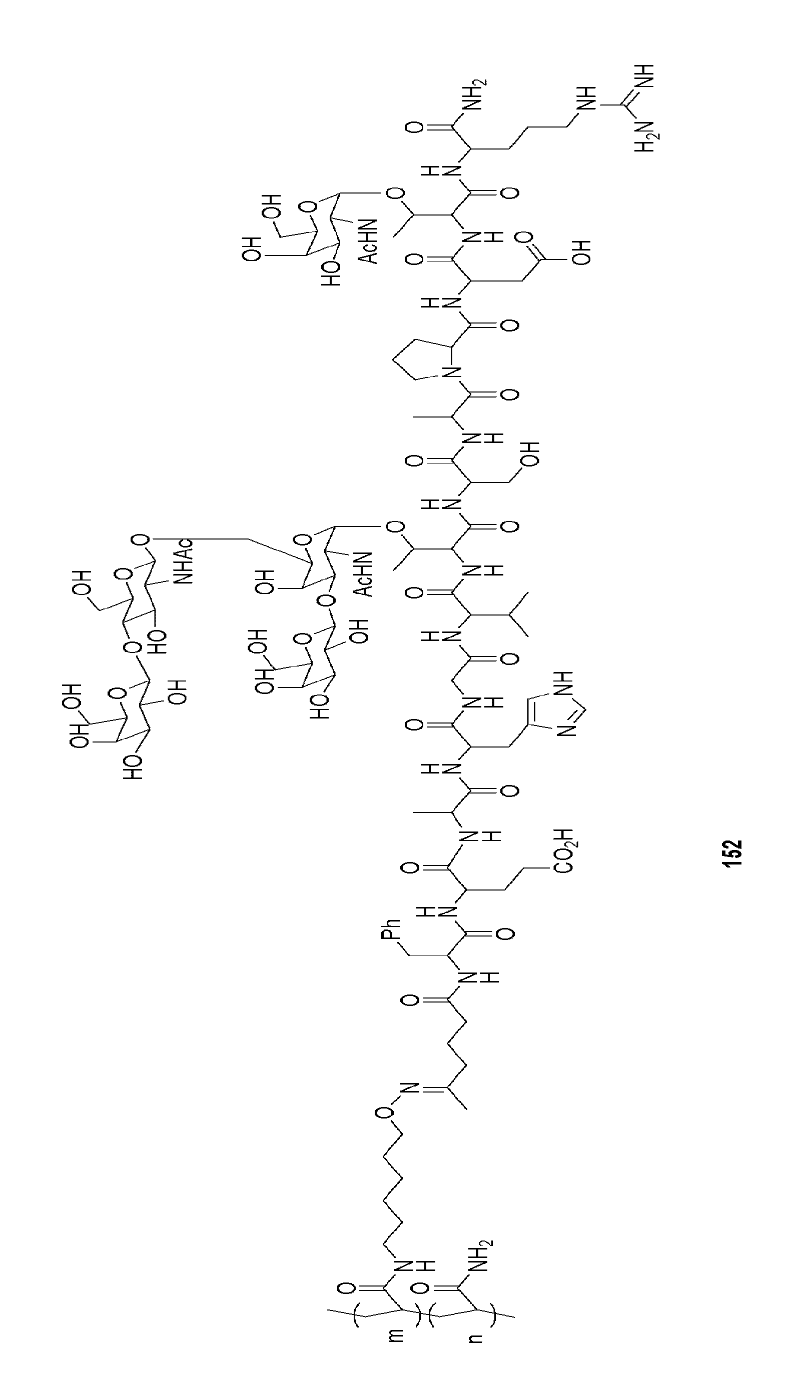

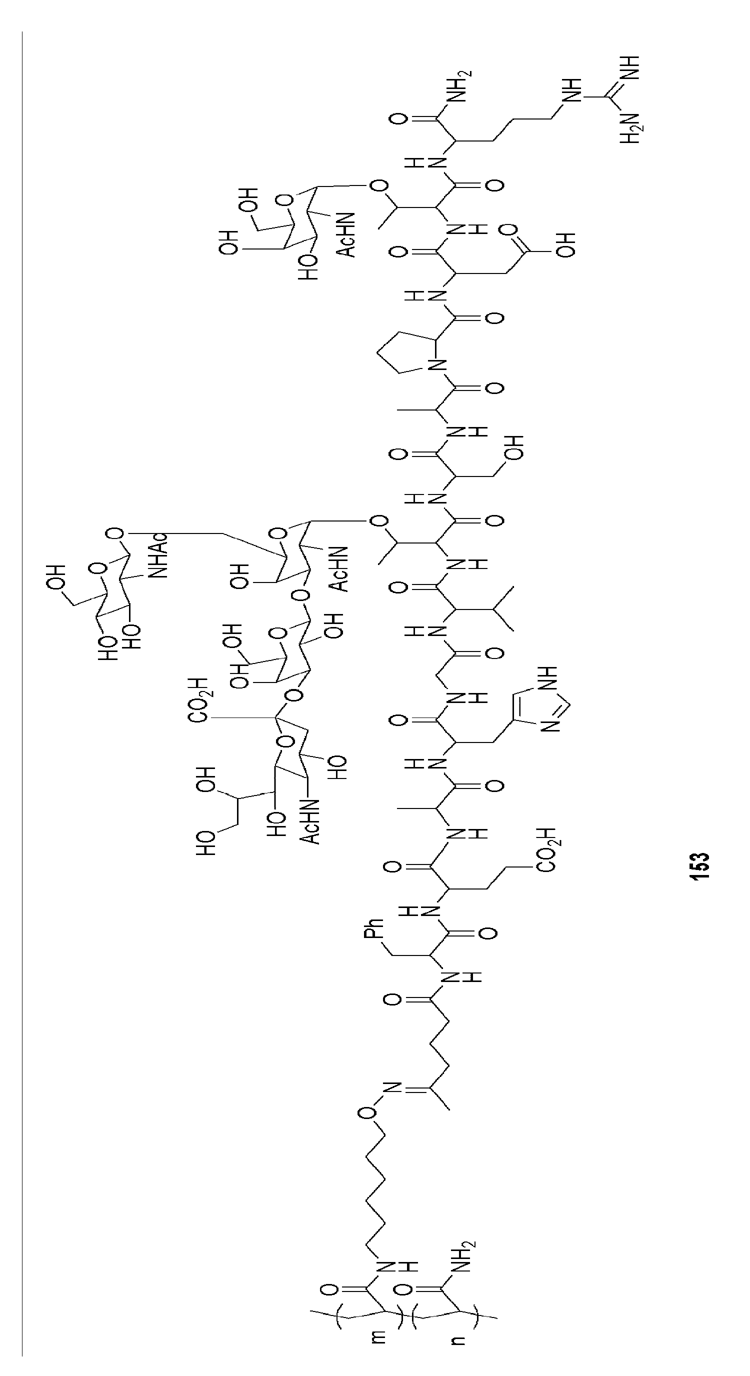

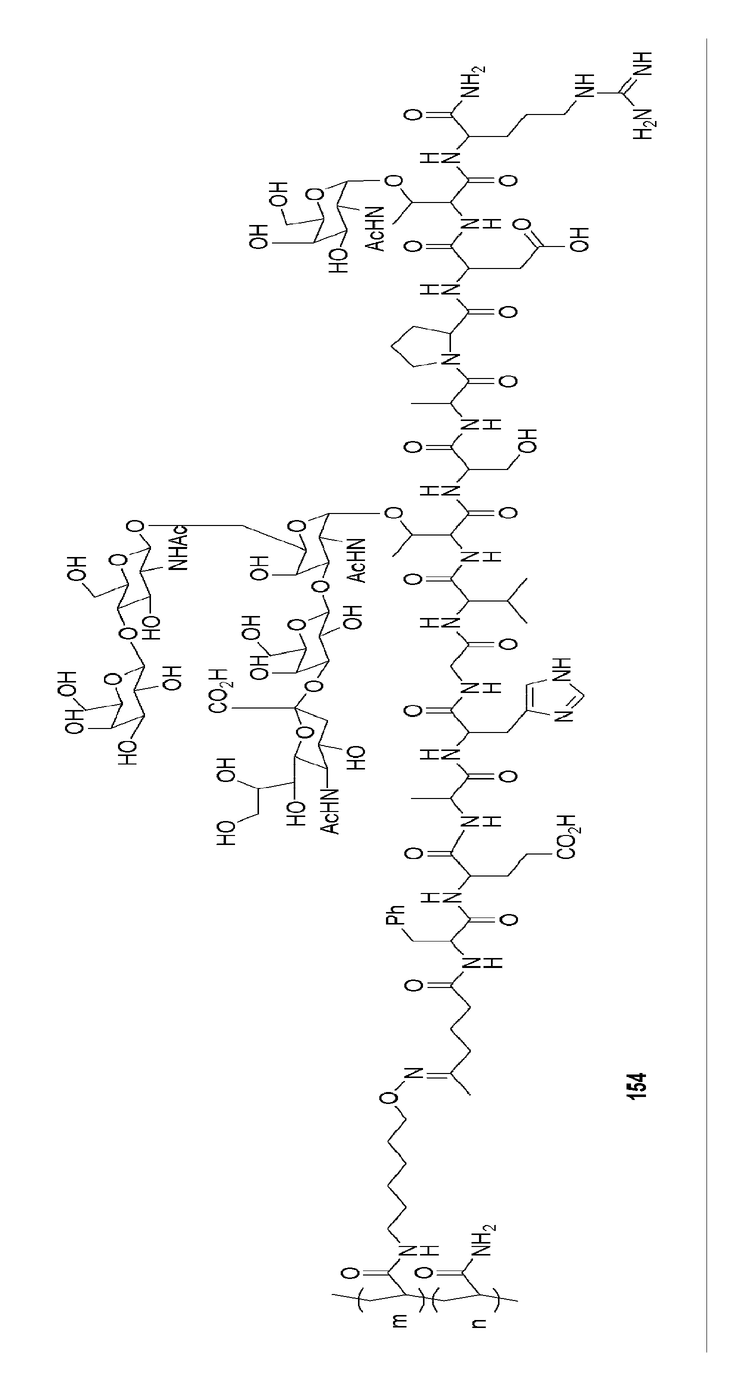

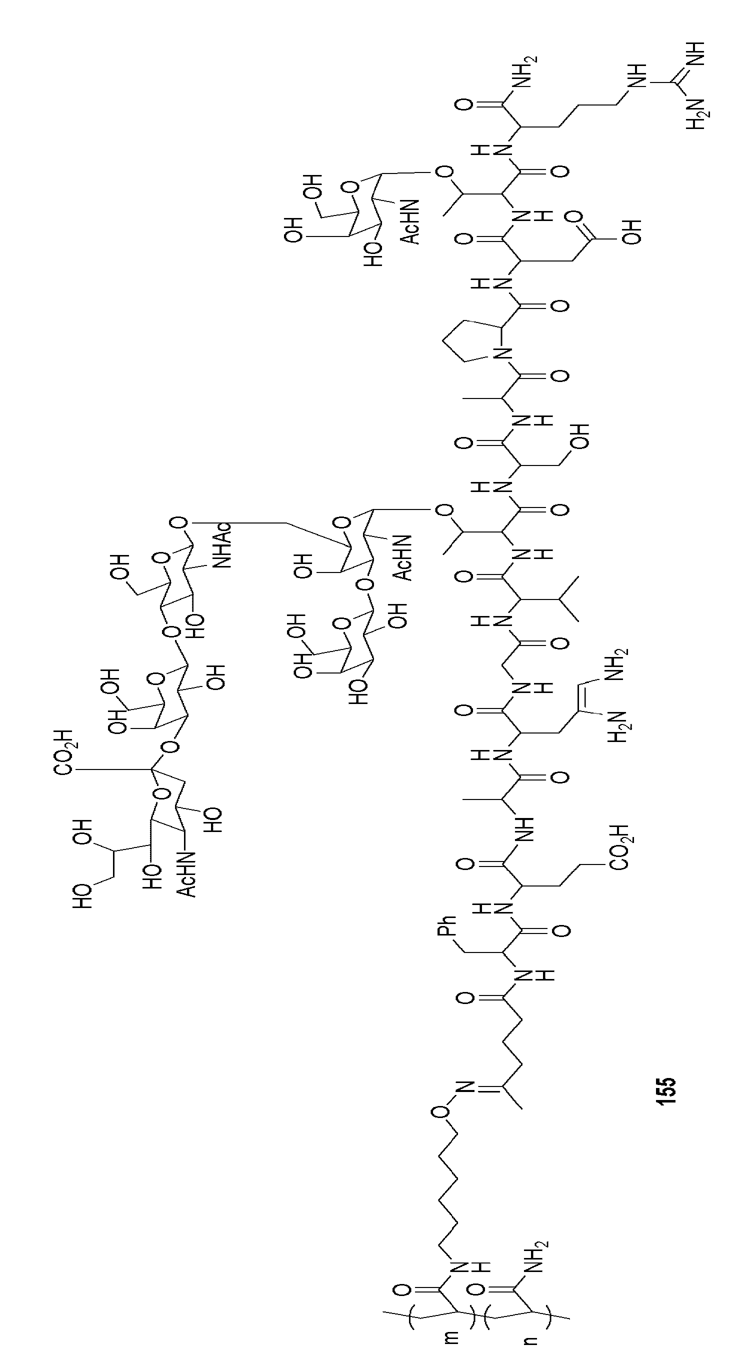

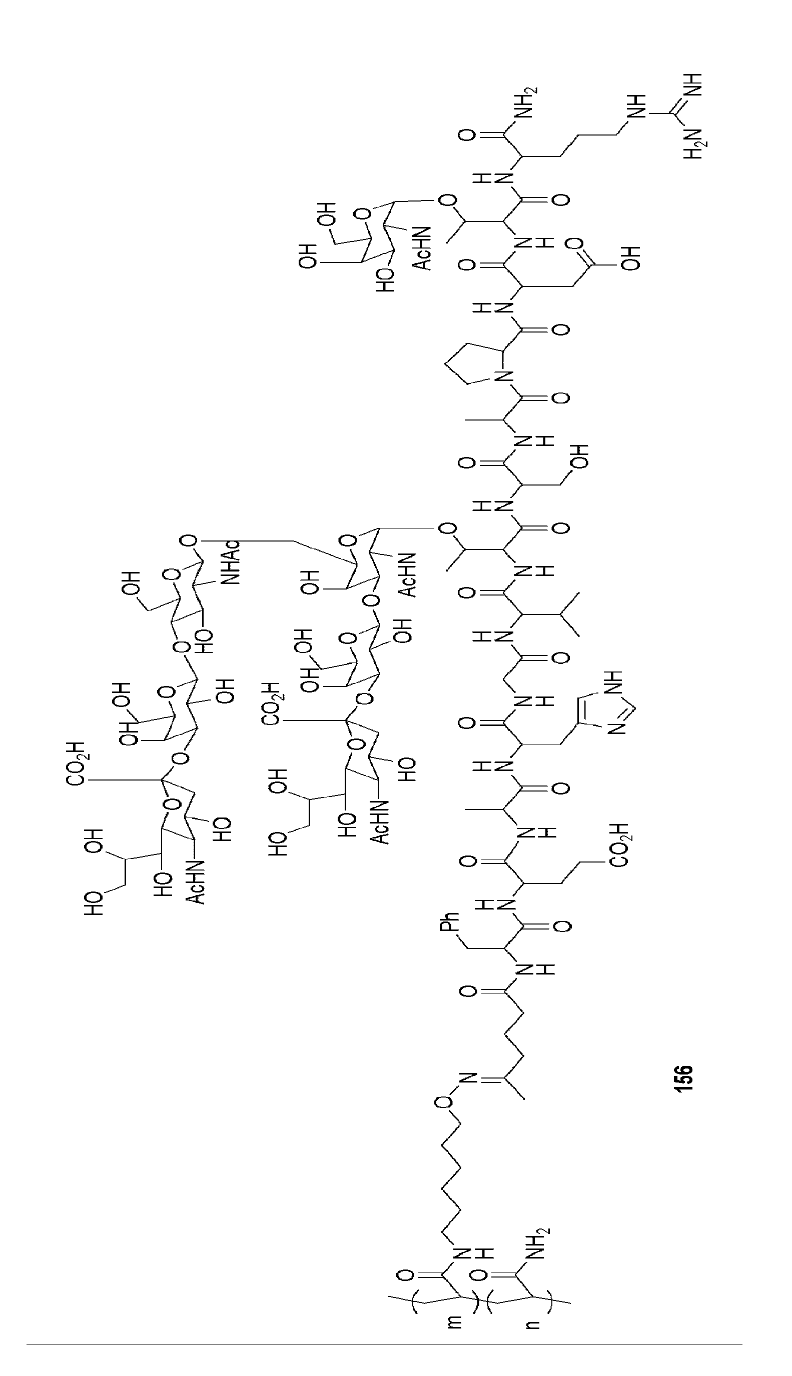

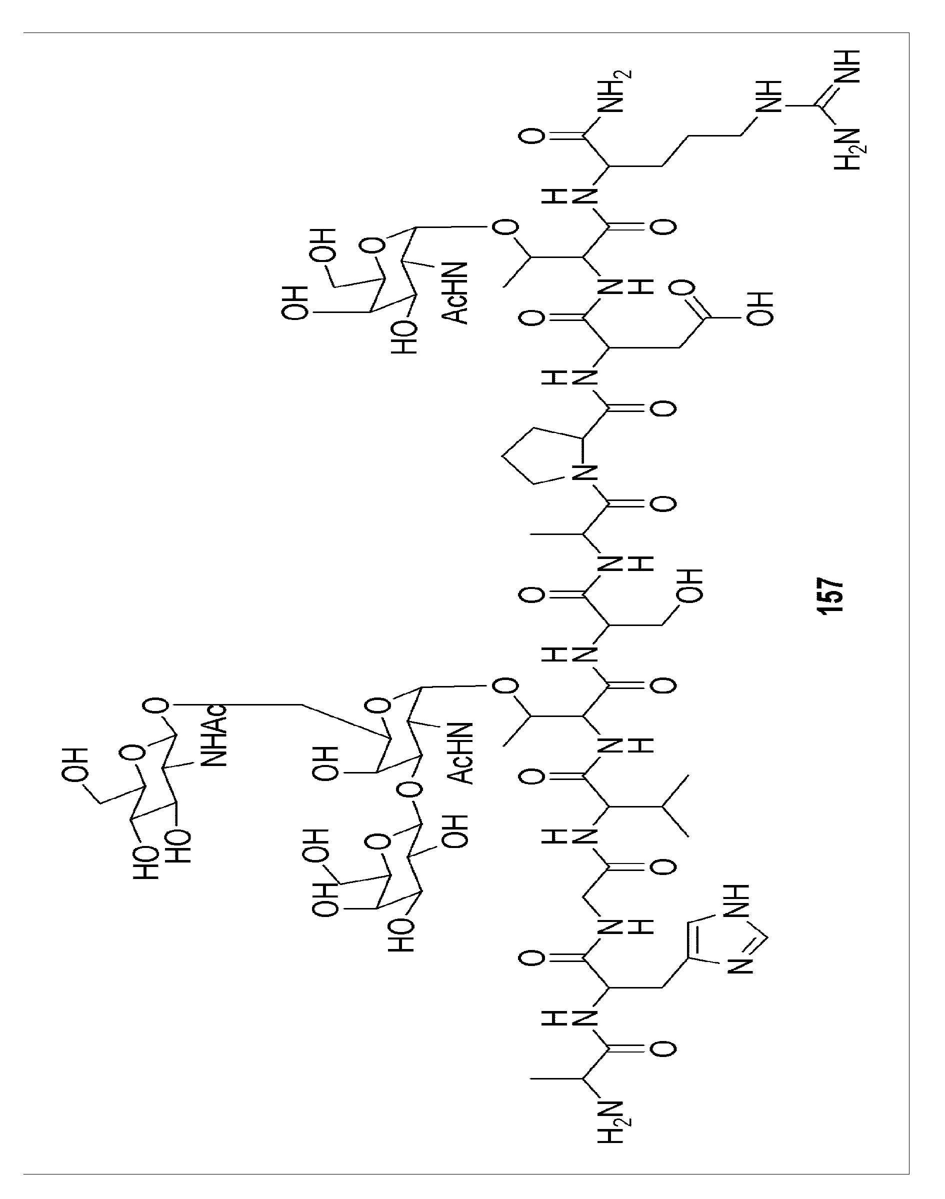

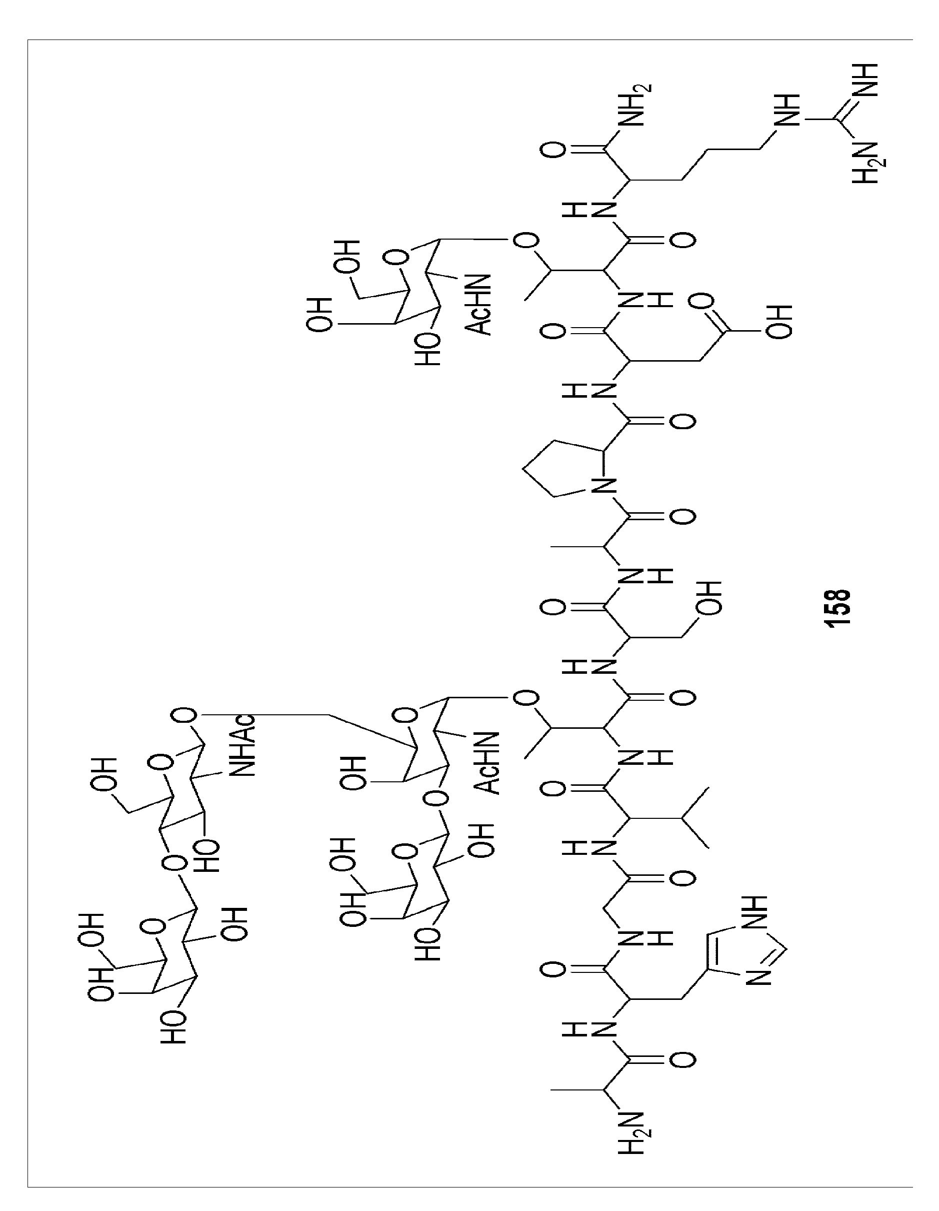

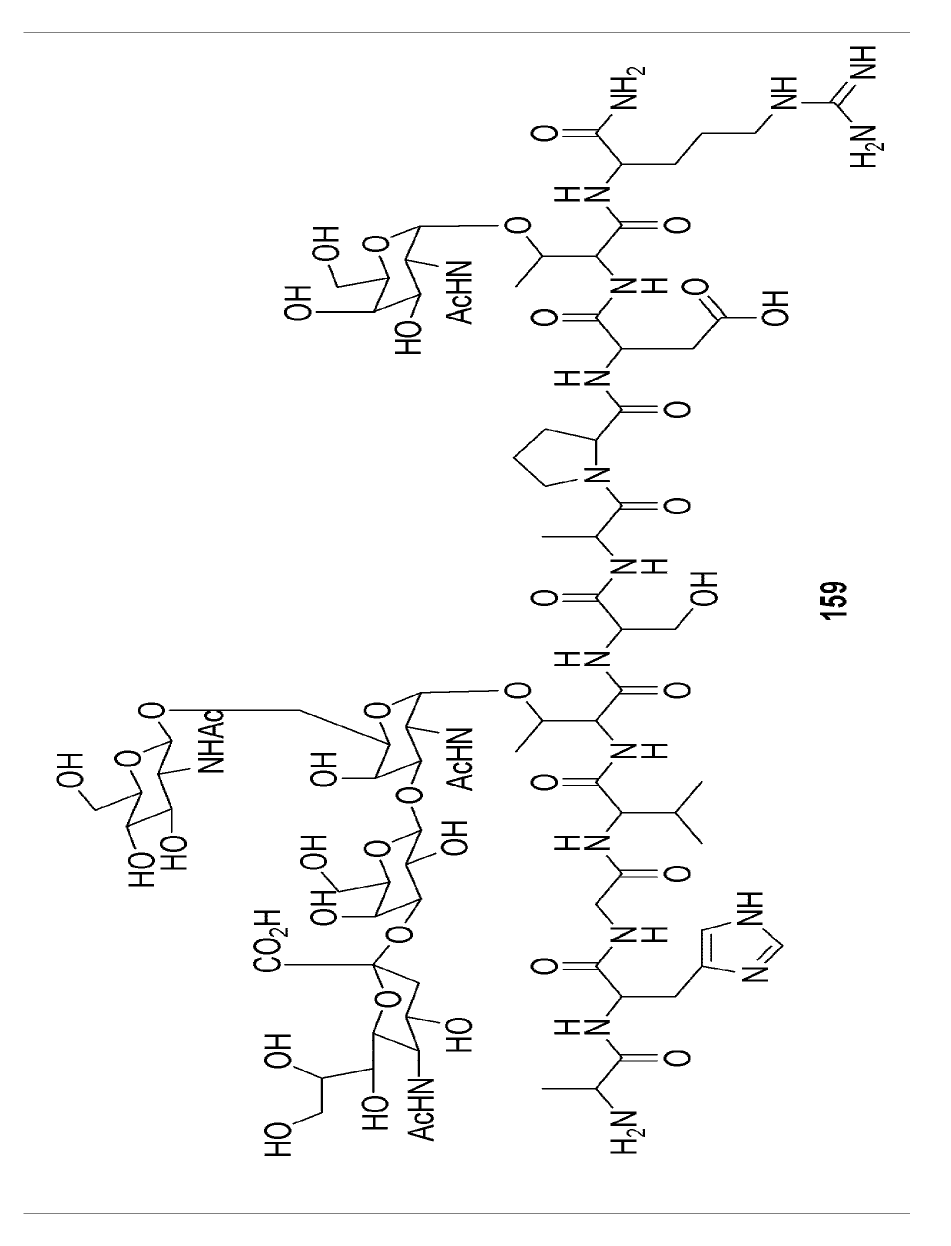

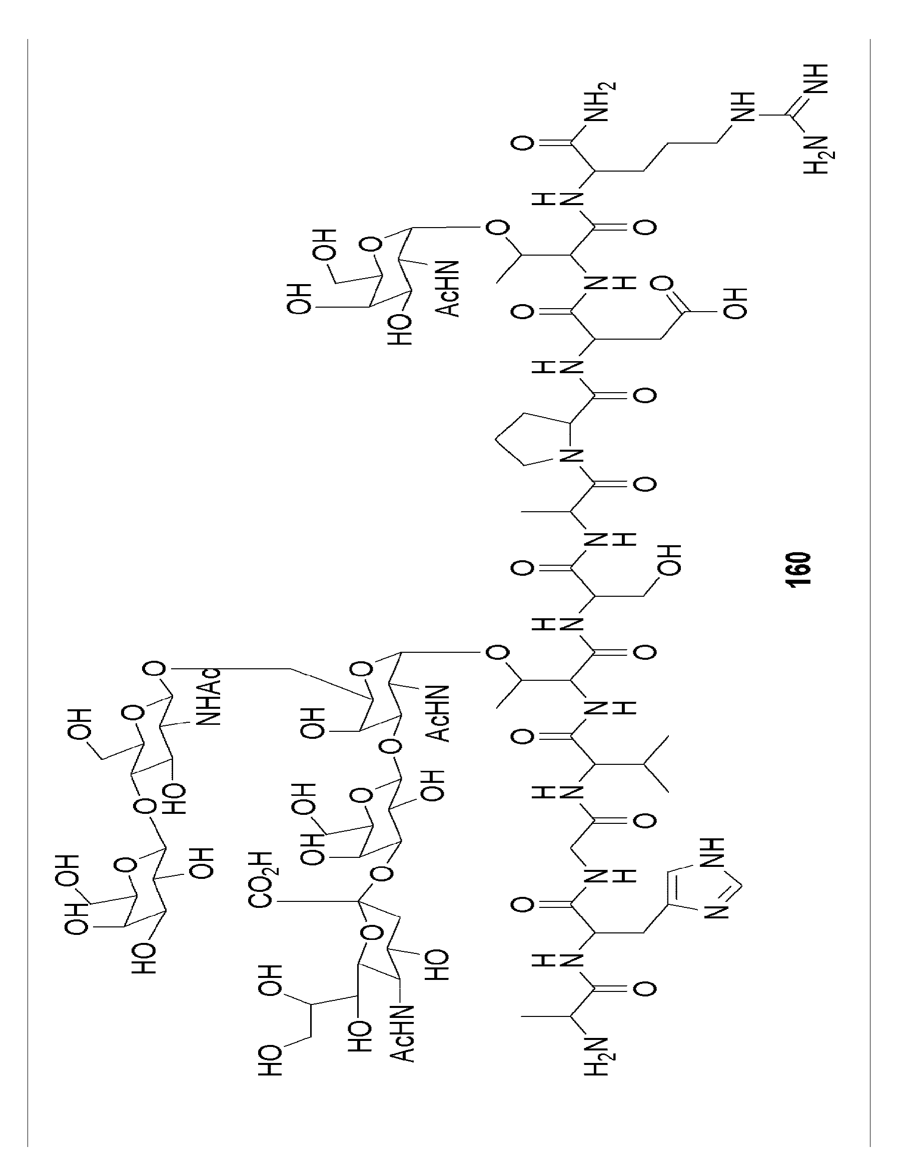

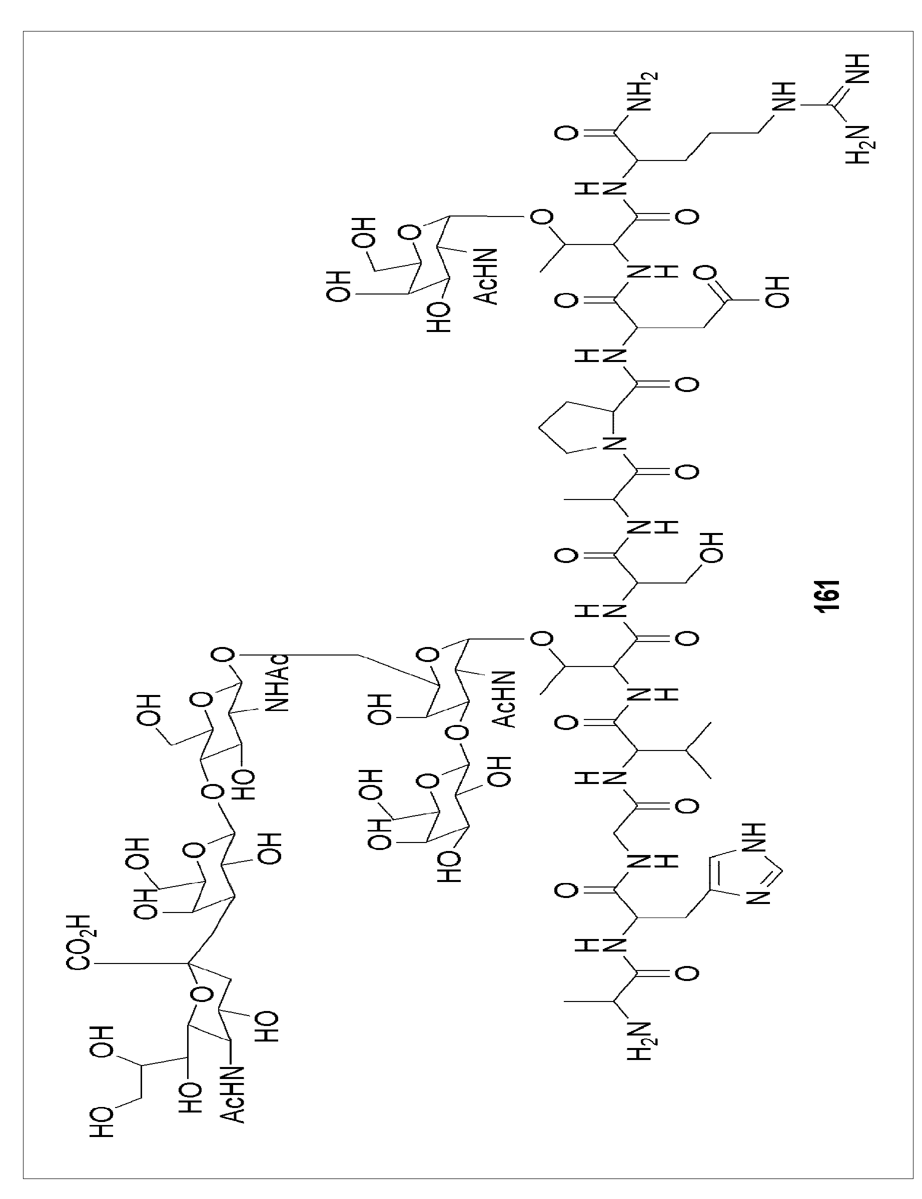

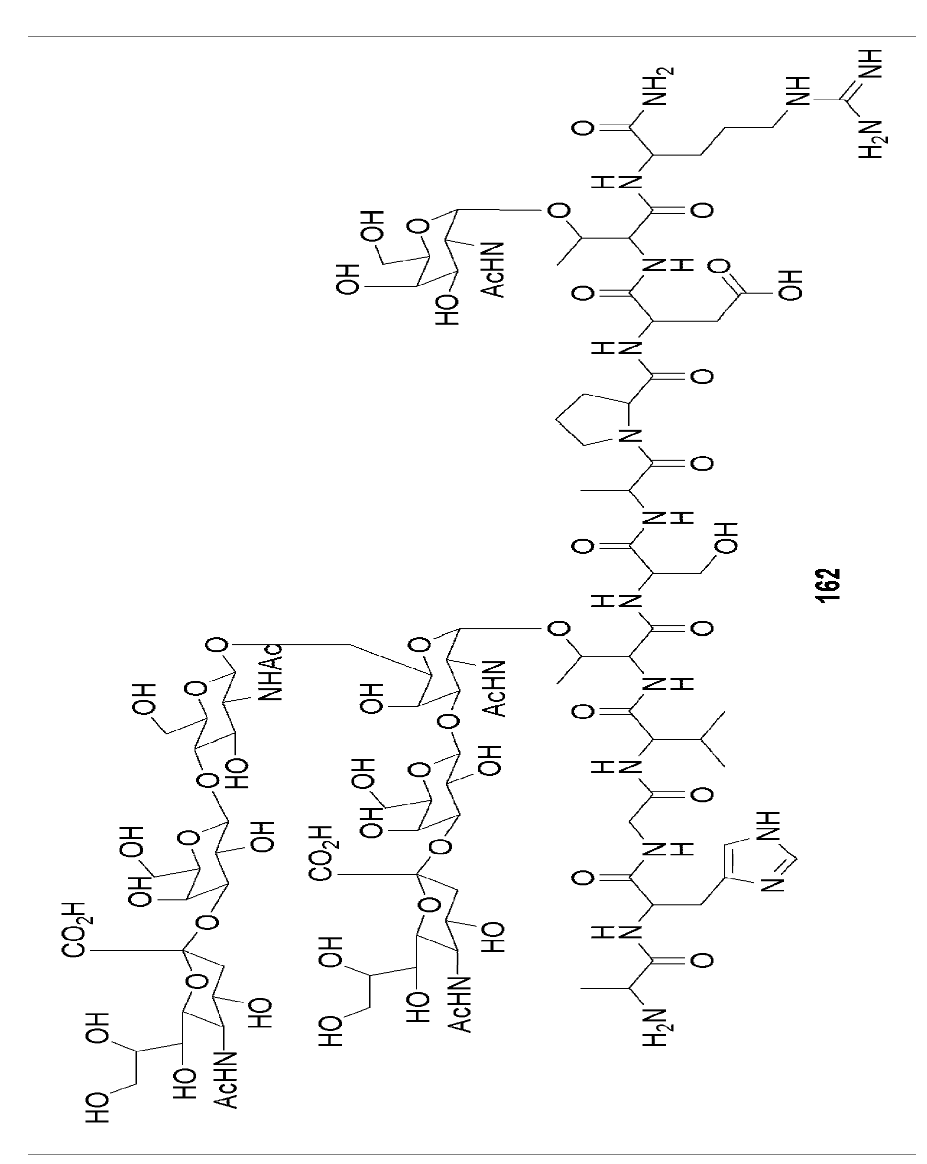

The following reaction solutions A) to E) were allowed to react for 24 hours at 25°C:
A) 250µl of reaction solution containing 50mM HEPES buffer solution (pH 7.0), 0.1U/ml human-derived β1,4-galactosyltransferase (available from TOYOBO CO., LTD.), 10mM manganese chloride, 0.1% BSA, 2mM uridine-5'-disodium diphosphogalactose (UDP-Gal) and glycopeptide derivative (96) (4mM at a theoretical content from solid-phase synthesis);
B) 250µl of reaction solution containing 50mM HEPES buffer solution (pH 7.0), 0.0175U/ml recombinant rat α2,3-(O)-sialyltransferase (available from Calbiochem), 10mM manganese chloride, 0.1% BSA, 2mM cytidine-5'-sodium monophosphosialate (CMP-NANA) and glycopeptide derivative (96) (4mM at a theoretical content from solid-phase synthesis);
C) 250µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (96) (4mM at a theoretical content from solid-phase synthesis);
D) 250µl of reaction solution containing 50mM HEPES buffer solution (pH 7.0), 0.1U/ml human-derived β1,4-galactosyltransferase (available from TOYOBO CO., LTD.), 0.0185U/ml recombinant rat α2,3-(N)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (96) (4mM at a theoretical content from solid-phase synthesis);
E) 250µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 0.0185U/ml recombinant rat α2,3-(N)-sialyltransferase (available from Calbiochem), 10mM manganese chloride, 0.1% BSA, 4mM uridine-5'-disodium diphosphogalactose (UDP-Gal), 2mM cytidine-5'-sodium monophosphosialate (CMP-NANA) and glycopeptide derivative (96) (4mM at a theoretical content from solid-phase synthesis).

After completion of the reaction, the respective reaction solutions were transferred to an ultrafiltration filter, ULTRAFRE-MC 10,000NMWL Filter Unit (available from Millipore) and were subjected to centrifugal concentration. Thereafter, 25mM ammonium acetate buffer solution (pH 6.5) was added thereto, and the solution was again subjected to concentration with a centrifugal separator, thereby washing the polymer. This manipulation was repeated three times, thereby respectively obtaining aqueous solutions of compounds (152) to (156). Thereafter, to 150µl of the solution retained at the filter containing compounds (152) to (156) and 150µl solution which was obtained by diluting 100µl of aqueous solution of (96) with 25mM ammonium acetate buffer solution (pH 6.5), 1µl of 1.74mg/ml solution of BLase (available from Shionogi & Co., Ltd.) was added, and the solution was allowed to re act for two hours at room temperature . Thereafter, the solution was subjected to centrifugal filtration with an ultrafiltration filter, ULTRAFRE-MC 10,000NMWL Filter Unit (available from Millipore), thereby separating the glycopeptide of interest from the polymer. The obtained aqueous solution (filtrate) was lyophilized, thereby obtaining compounds (157) to (162). MALDI TOF/MS:[M(average)+H]⁺ of compound (157)=1882.3 (theoretical value:[M(average)+H]⁺=1881.9); MALDI TOF/MS:[M(average)+H]⁺ of compound (158)=2044.6 (theoretical value:[M(average)+H]⁺=2043.9); MALDI TOF/MS:[M(average)+H]⁺ of compound (159)=2173.6 (theoretical value:[M(average)+H]⁺=2173.0); MALDI TOF/MS:[M(average)+H]⁺ of compound (160)=2335.5 (theoretical value:[M(average)+H]⁺=2335.0); MALDI TOF/MS:[M(average)+H]⁺ of compound (161)=2335.5 (theoretical value: [M(average)+H]⁺=2335.0); and MALDI TOF/MS:[M(average)+H]⁺ of compound (162)=2626.2 (theoretical value:[M(average)+H]⁺=2626.1).

(3.27 Synthesis of compounds (163) to (165) using a one-pot reaction))

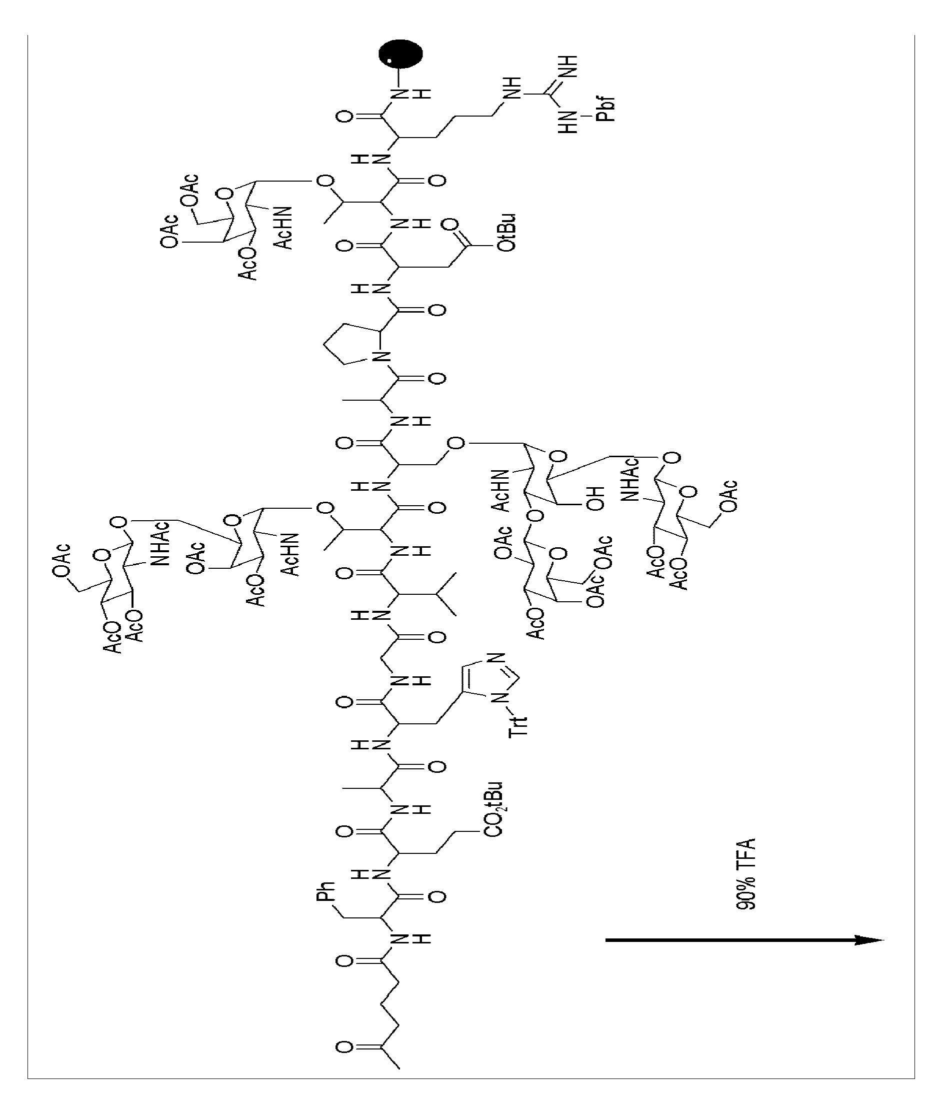

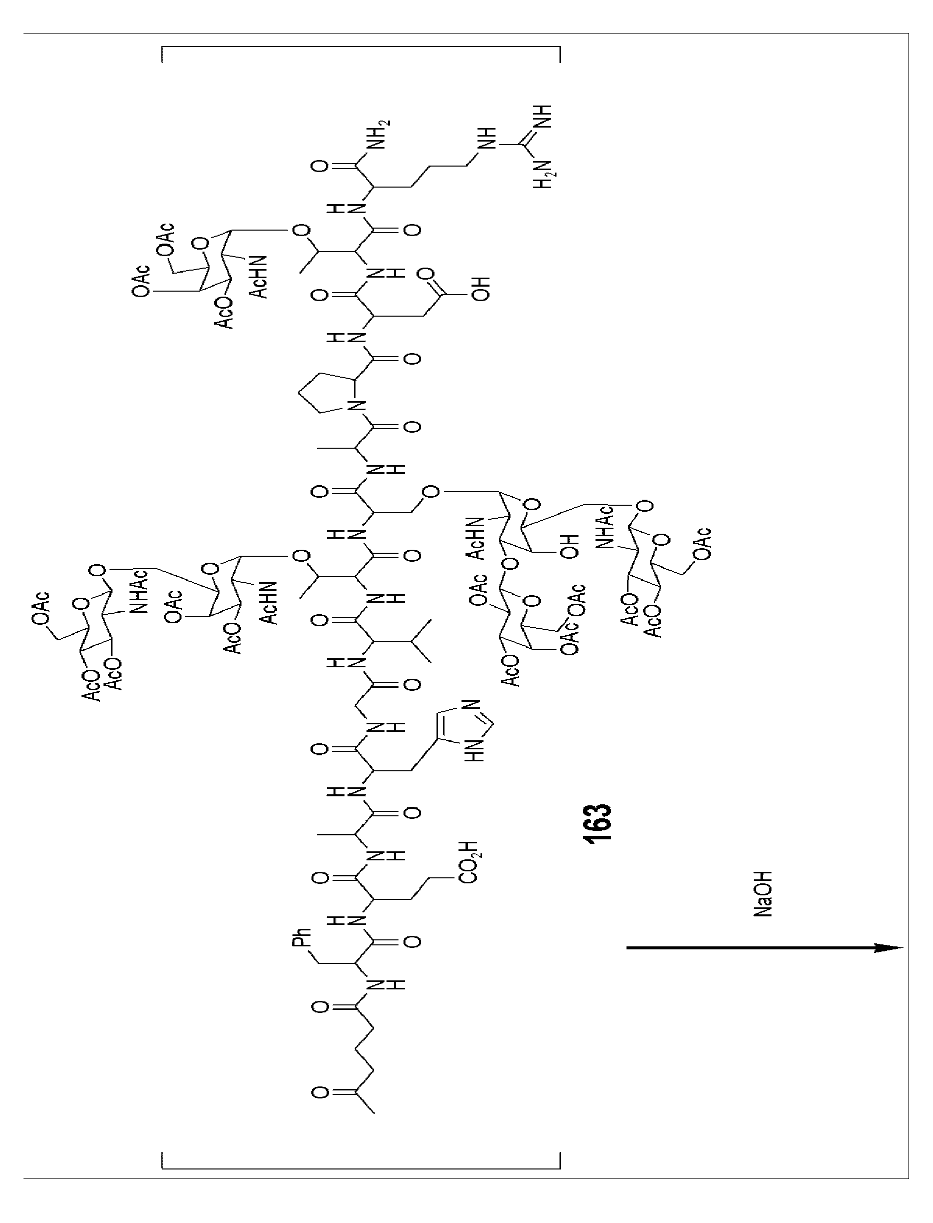

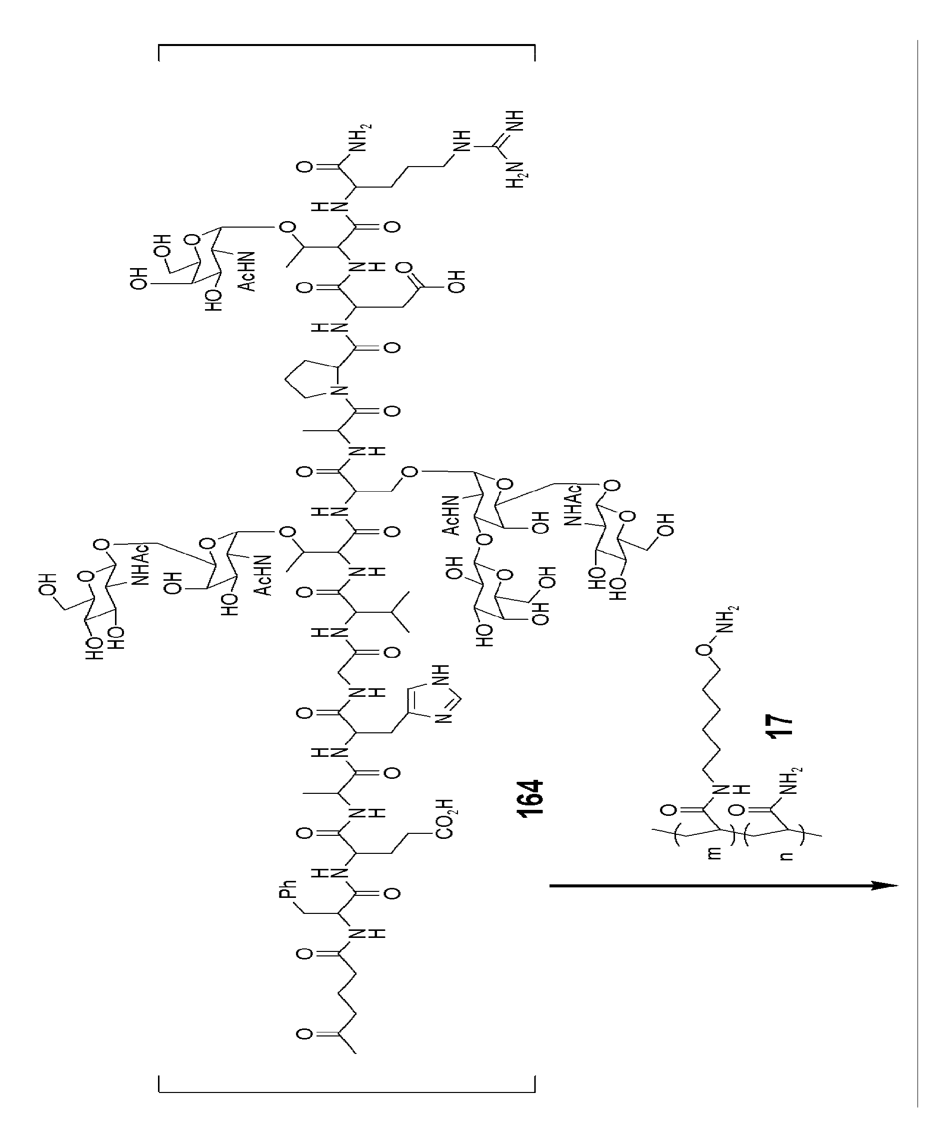

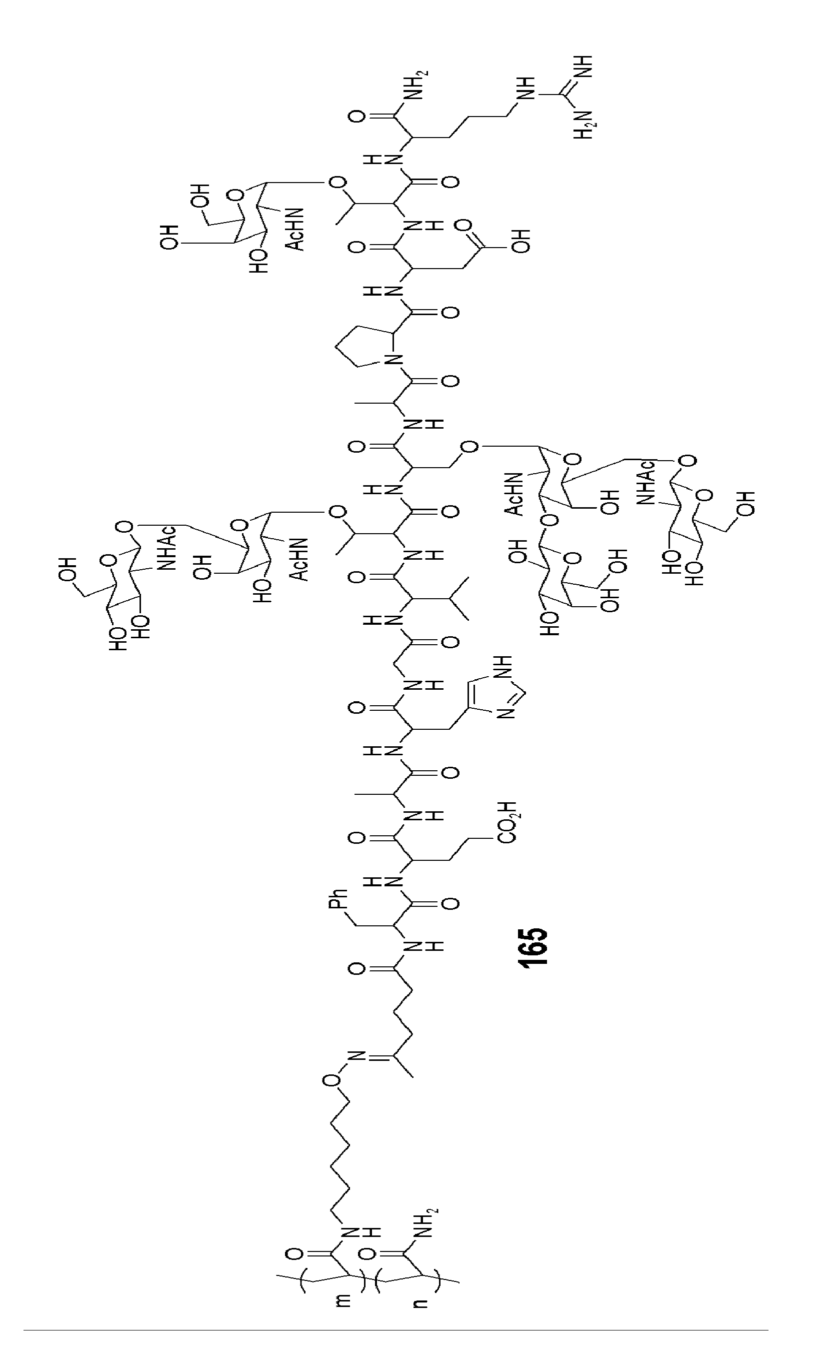

Using 0.12g (0.03mmol) of Tentagel® S RAM resin (Hipep Laboratories, 0.25mmol/g) as a support, N-protected amino acids and keto acids as described below were sequentially condensed by Fmoc/HBTU/HOBt method, thereby synthesizing a glycopeptide derivative of interest: Fmoc-Arg(Pbf)-OH; Fmoc-Thr(Ac3GalNAc)-OH; Fmoc-Asp(OtBu)-OH; Fmoc-Pro-OH; Fmoc-Ala-OH; Fmoc-Ser(Ac7core2)-OH; Fmoc-Thr(Ac5core6)-OH; Fmoc-Val-OH; Fmoc-Gly-OH; Fmoc-His(Trt)-OH; Fmoc-Ala-OH; Fmoc-Gln(OtBu)-OH; Fmoc-Phe-OH; and 5-ketohexane acid. After peptide elongation reaction, the resin was allowed to react in 90% TFA aqueous solution for two hours at room temperature to eliminate a protective group on a peptide residue and concurrently release compound (163) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether was again added, and the precipitate was washed. Centrifugal separation was again performed and the supernatant was removed. The obtained precipitant was dissolved in 6.0ml of methanol. 1N sodium hydroxide aqueous solution was added to this solution, and was adjusted to have a pH of 12-12.5. The solution was then stirred for three hours at room temperature, thereby performing Ac deprotection reaction. After the reaction, H⁺-type cation exchange resin, Dowex50WX8 (available from Dow Chemical), was added and the solution was neutralized. Thereafter, the resin was separated by filtration. The solvent in the filtrate was removed, and the residue was dissolved in 3.0ml of 50mM acetic acid/sodium acetate buffer solution (pH 5.5). 30ml of 10mM (oxyamine residue calculation) water-soluble polymer (17) aqueous solution was added to the solution, and the solution was stirred for 14 hours at room temperature, thereby reacting compound (164) with compound (17). After completion of the reaction, the reaction solution was subjected to centrifugal concentration with an ultrafiltration filter 10K Apollp ® 20ml (Orbital Biosciences, available from LIC). 25mM HEPES buffer solution (pH 7.0) was added thereto and the solution was again subjected to concentration, thereby washing the polymer. By adding water so that the final amount of the solution was 1.5ml, 20mM (theoretical content of glycopeptide from the solid-phase synthesis) polymer (165) was obtained. Identification of the polymer (165) was performed based on that product (171) was obtained in the following section (3.28).

(3.27.2 Synthesis of (166) to (176))

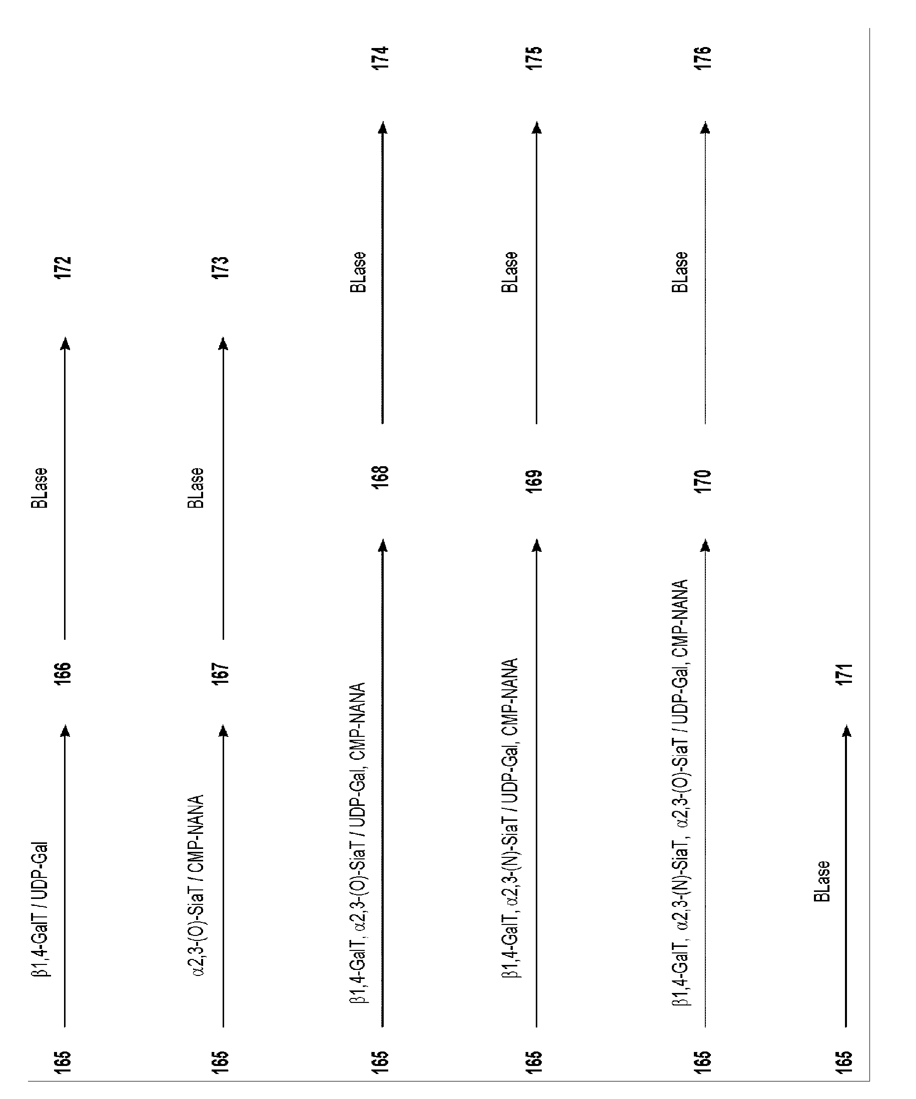

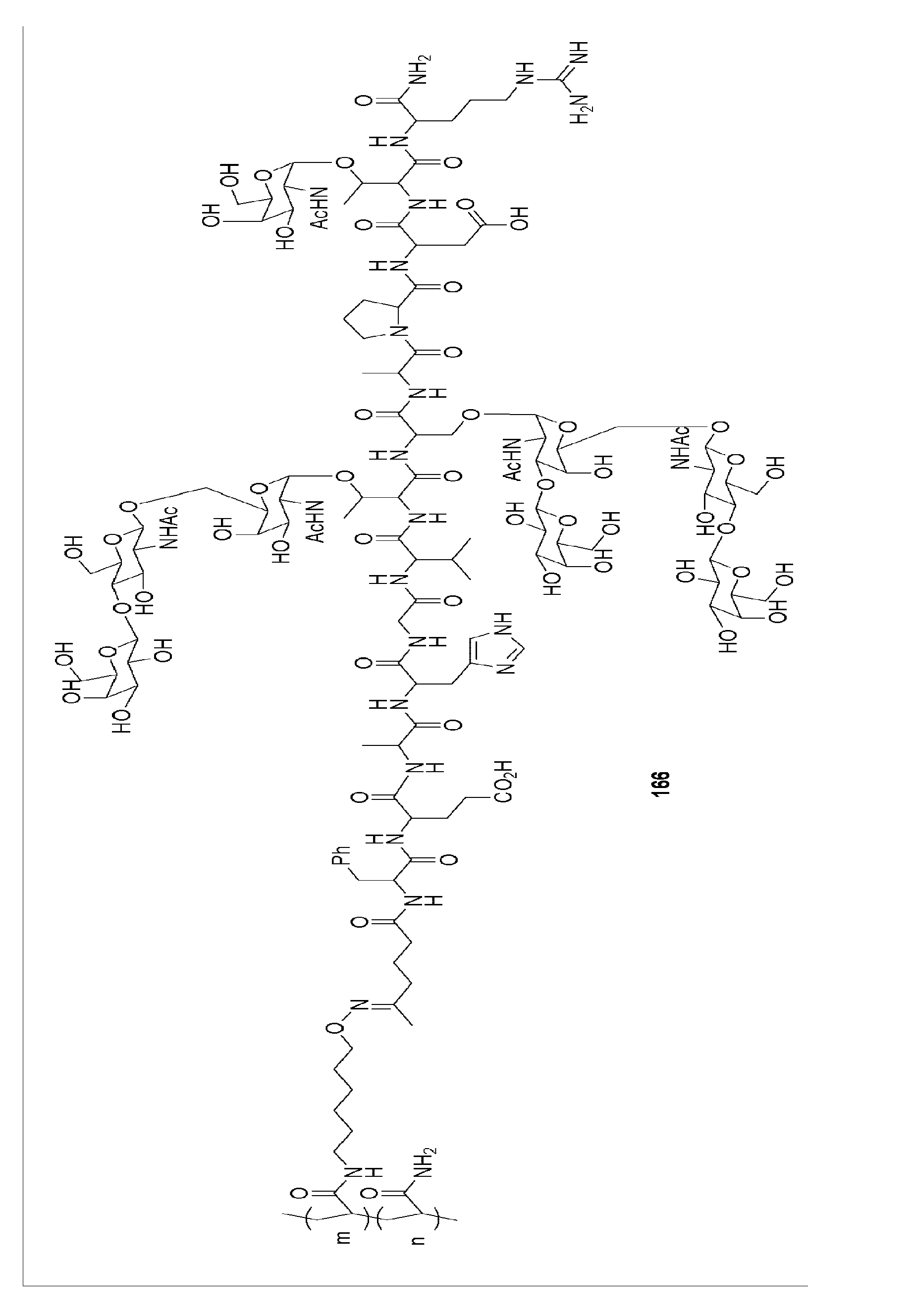

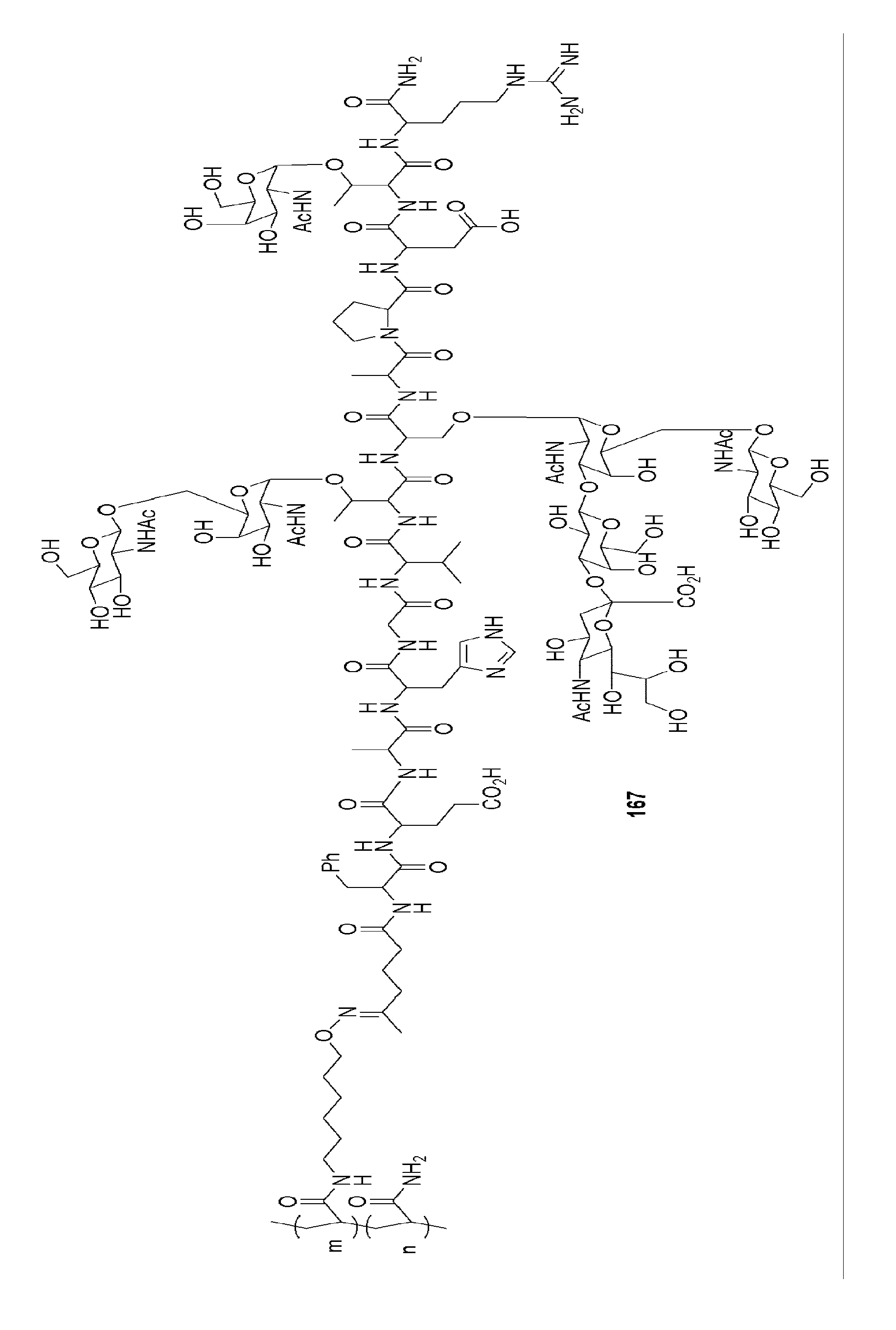

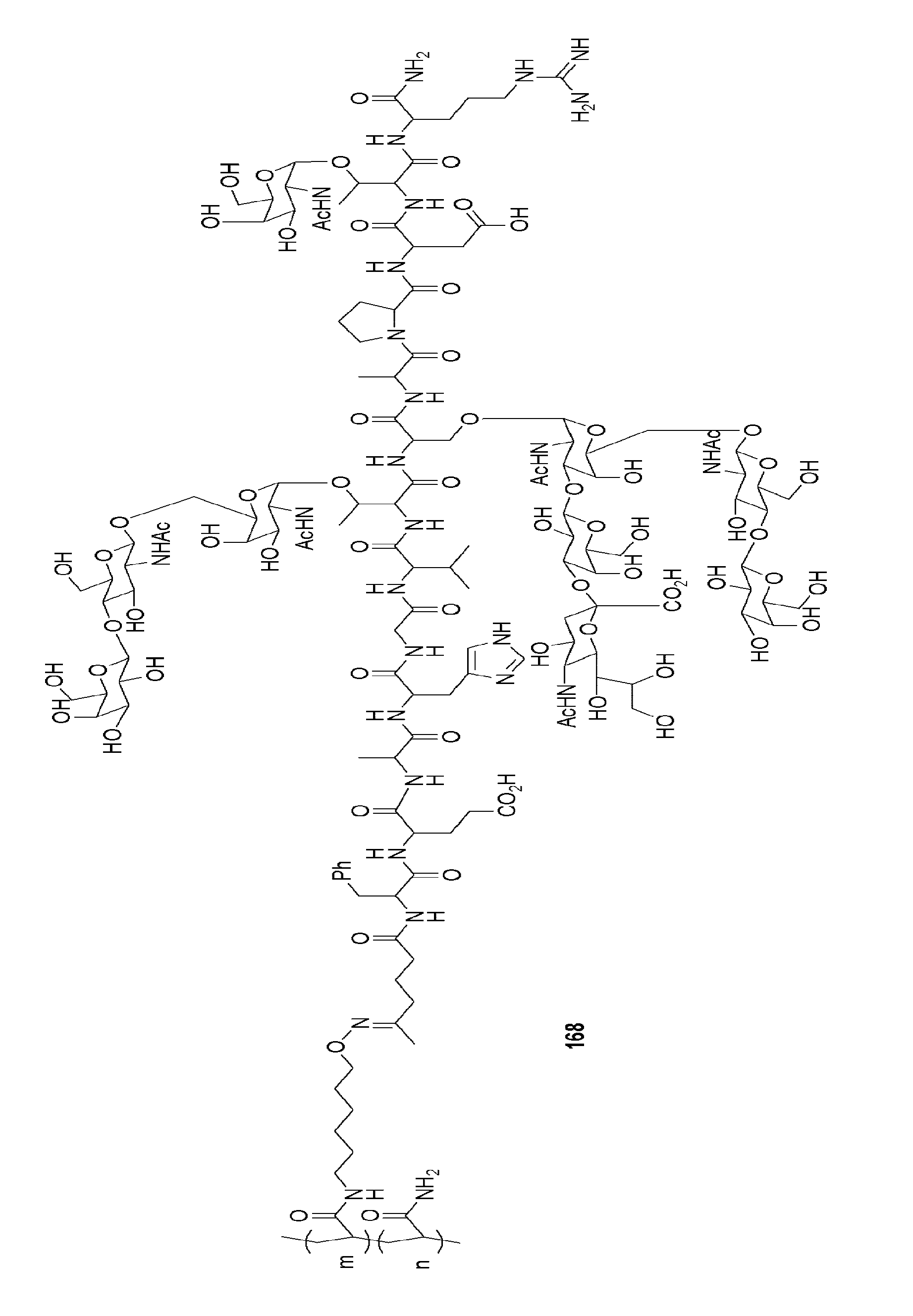

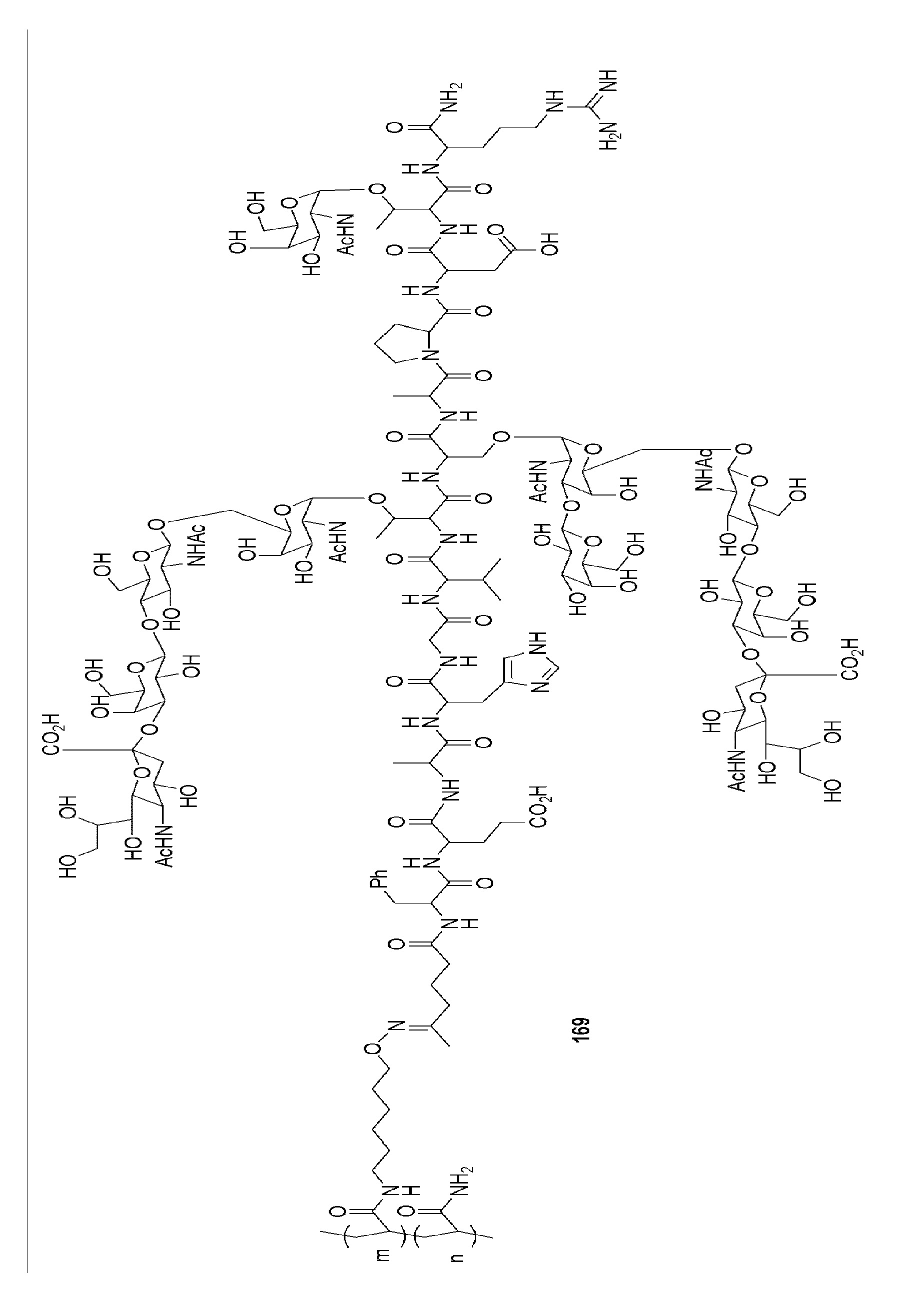

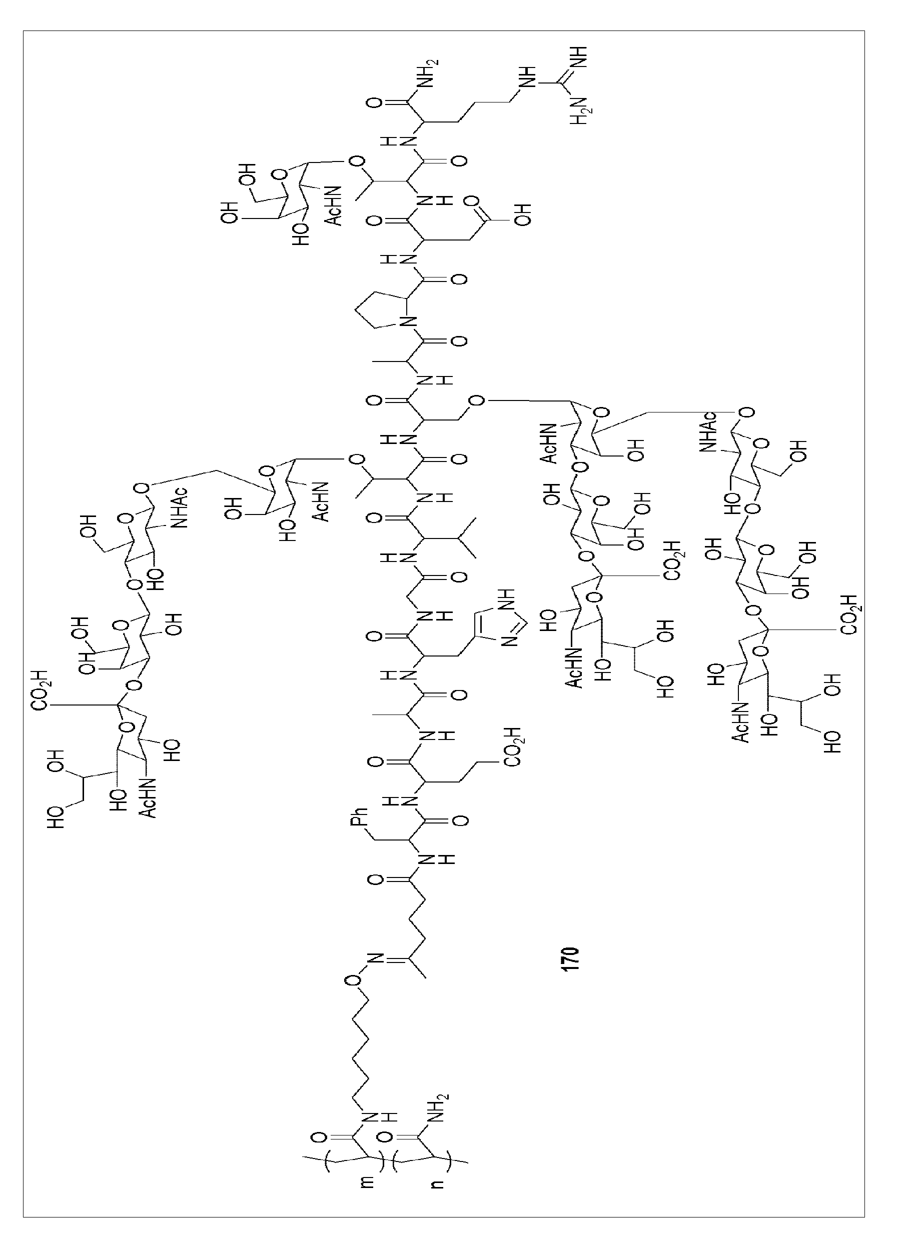

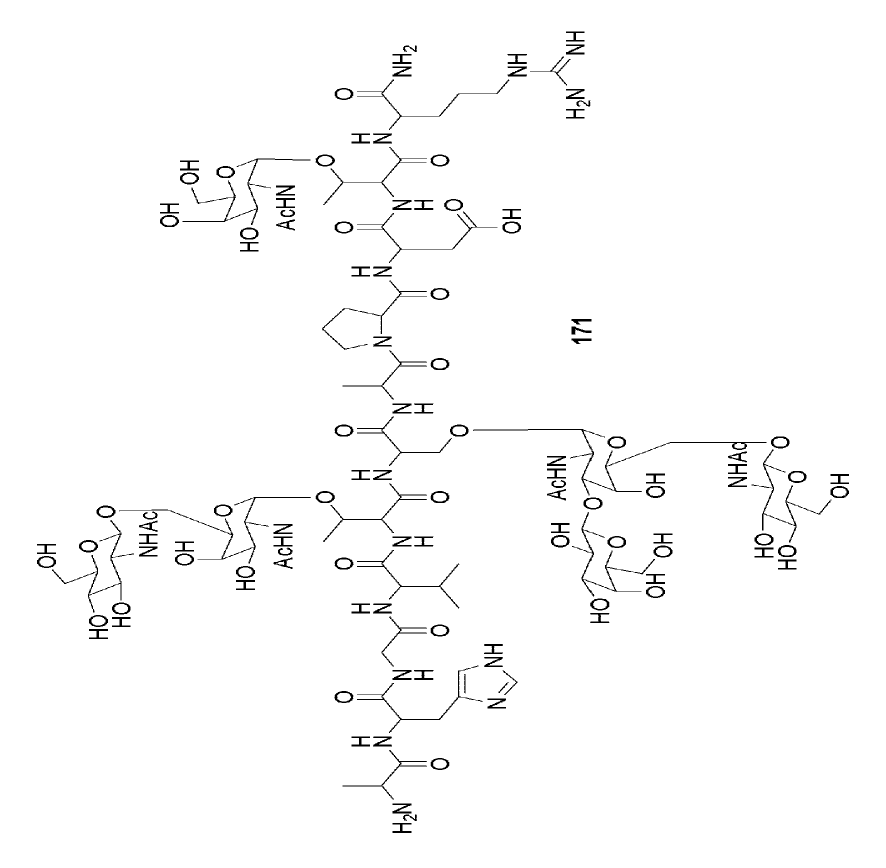

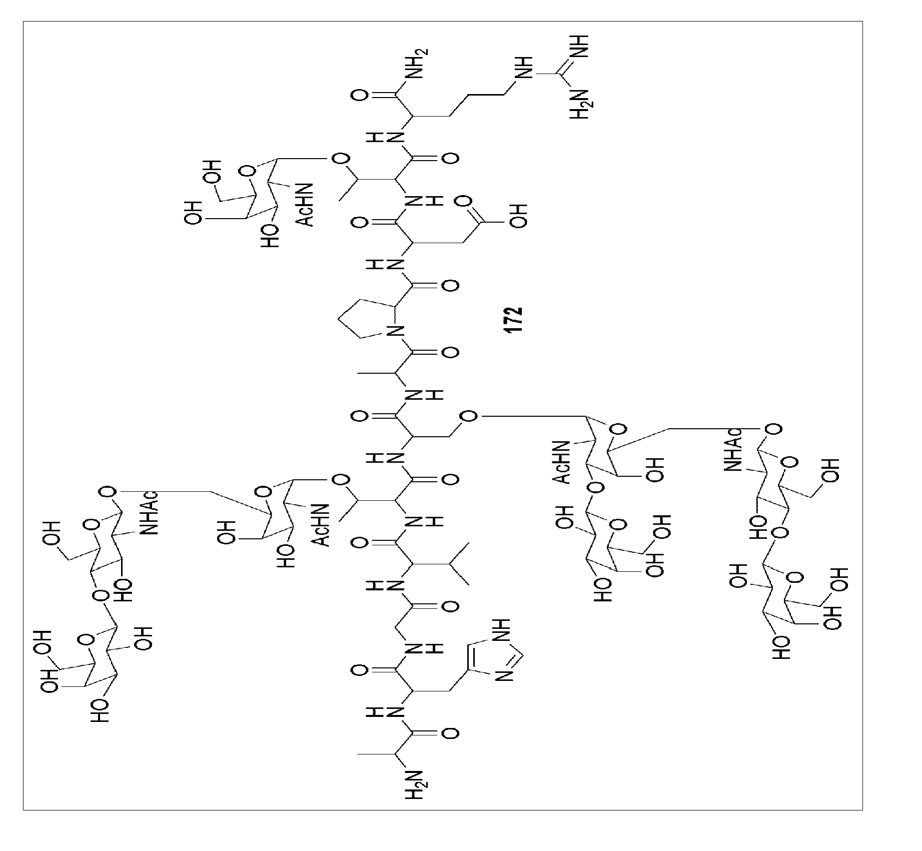

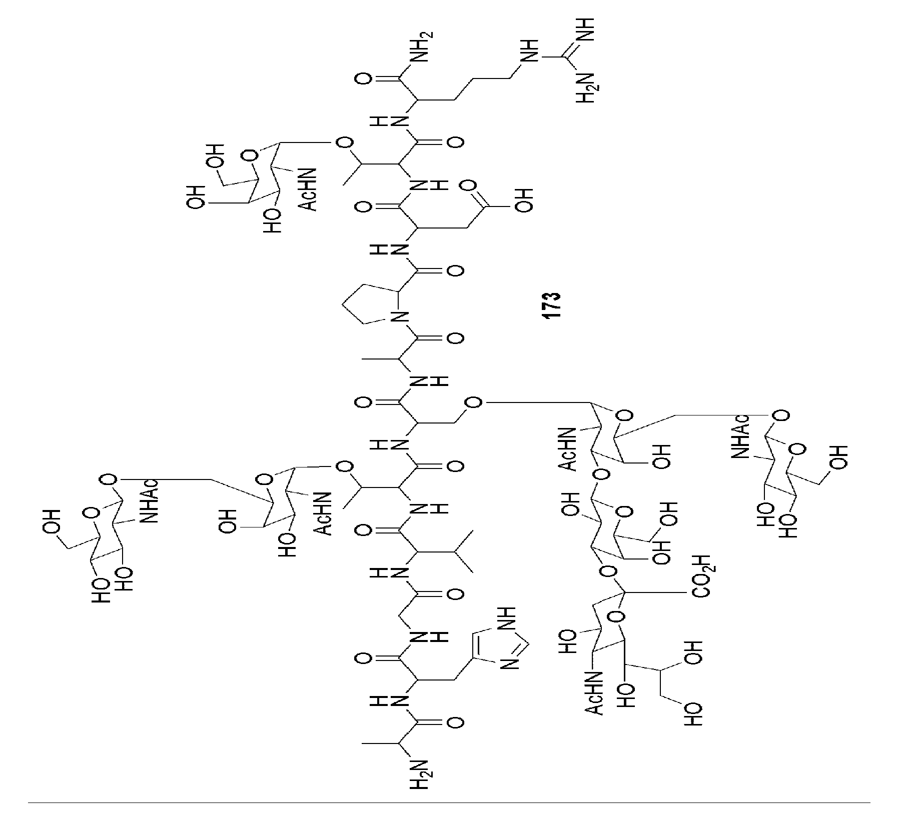

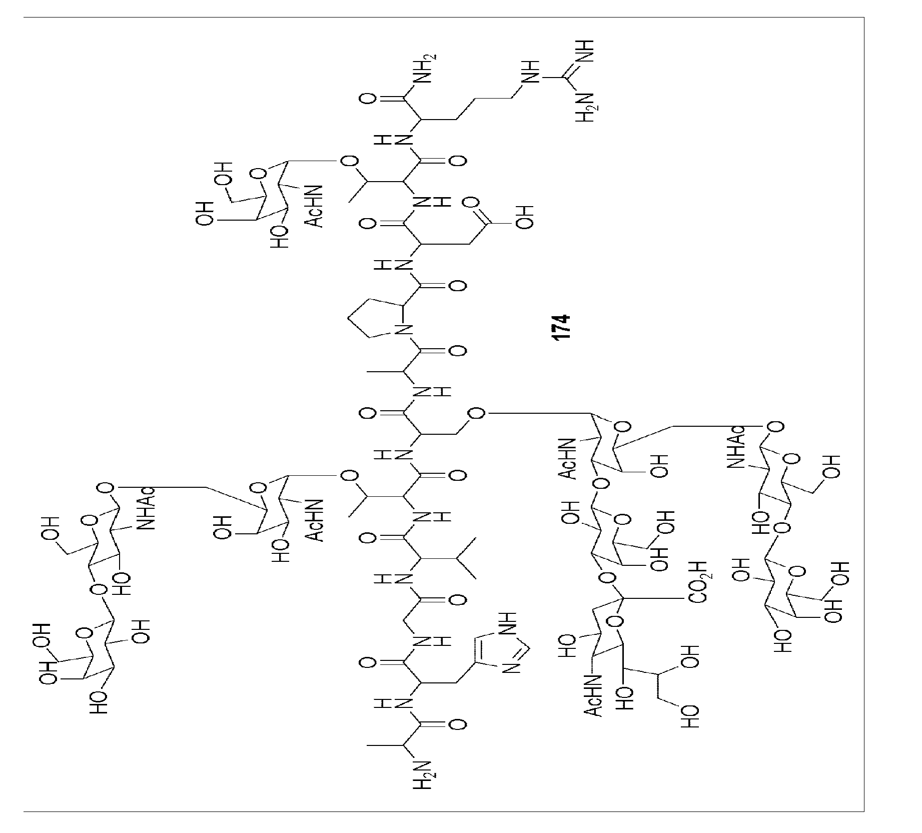

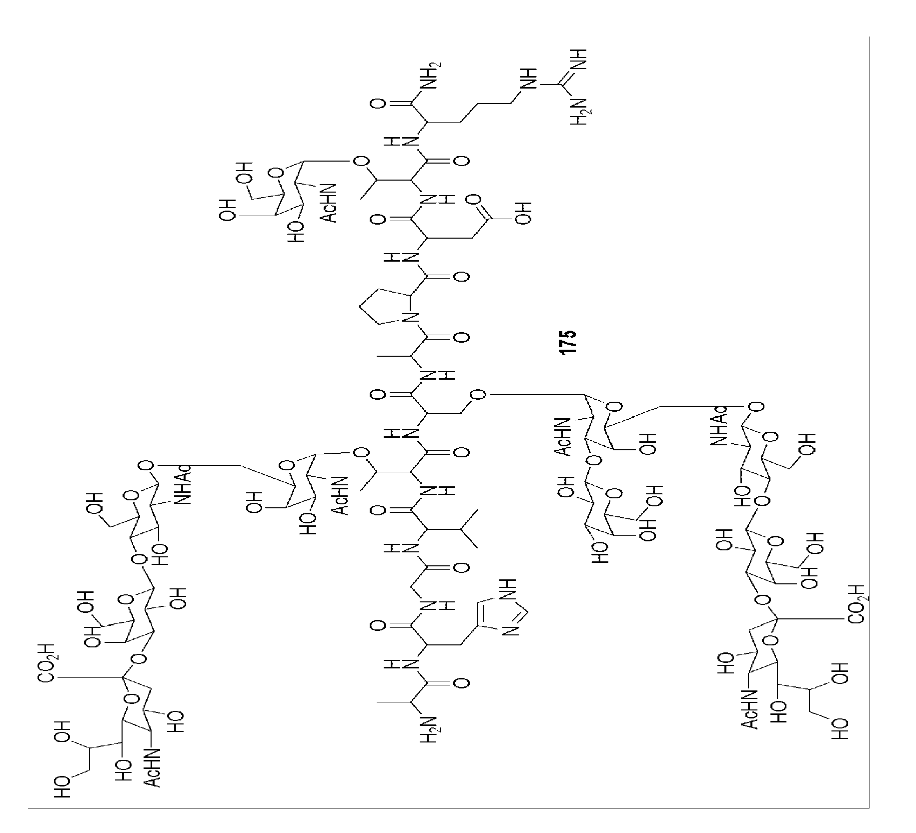

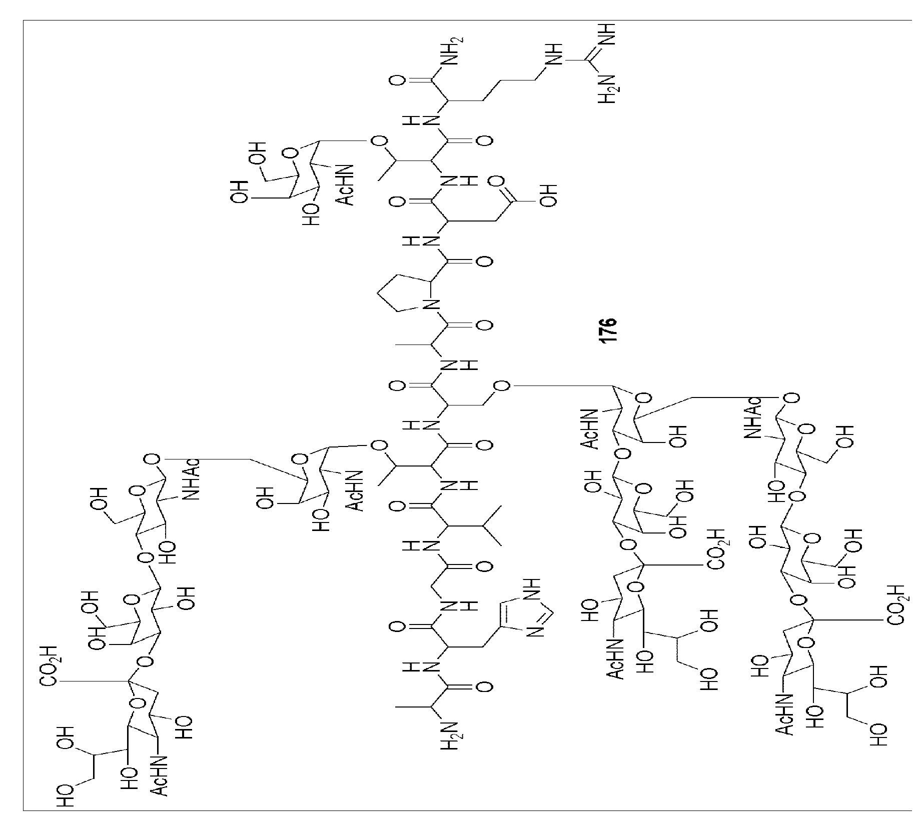

The following reaction solutions A) to E) were allowed to react for 24 hours at 25°C:
A) 150µl of reaction solution containing 50mMHEPES buffer solution (pH 7.0), 0.1U/ml human-derived β1,4-galactosyltransferase (available from TOYOBO CO., LTD.), 10mM manganese chloride, 0.1% BSA, 5mM uridine-5'-disodium diphosphogalactose (UDP-Gal) and glycopeptide derivative (165) (8mM at a theoretical content from solid-phase synthesis);
B) 150 µl of reaction solution containing 50m MHEPES buffer solution (pH 7.0), 0.0175U/ml recombinant rat α2,3-(O)-sialyltransferase (available from Calbiochem), 10mM manganese chloride, 0.1% BSA, 5mM cytidine-5'-sodium monophosphosialate (CMP-NANA) and glycopeptide derivative (165) (8mM at a theoretical content from solid-phase synthesis);
C) 150µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 10mM manganese chloride, 0.1% BSA, 2mM uridine-5'-disodium diphosphogalactose (UDP-Gal), 5mM cytidine-5'-sodium monophosphosialate (CMP-NANA) and glycopeptide derivative (165) (8mM at a theoretical content from solid-phase synthesis);
D) 150µl of reaction solution containing 50mM HEPES buffer solution (pH 7.0), 0.1U/ml human-derived β1,4-galactosyltransferase (available from TOYOBO CO., LTD.), 0.074U/ml recombinant rat α2,3-(N)-sialyltransferase (available from Calbiochem), 10mM manganese chloride, 0.1% BSA, 5mM uridine-5'-disodium diphosphogalactose (UDP-Gal), 5mM cytidine-5'-sodium monophosphosialate (CMP-NANA) and glycopeptide derivative (165) (8mM at a theoretical content from solid-phase synthesis);
E) 150µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 0.074U/ml recombinant rat α2,3-(N)-sialyltransferase (available from Calbiochem), 10mM manganese chloride, 0.1% BSA, 5mM uridine-5'-disodium diphosphogalactose (UDP-Gal), 5mM cytidine-5'-sodium monophosphosialate (CMP-NANA) and glycopeptide derivative (165) (8mM at a theoretical content from solid-phase synthesis).

After completion of the reaction, the respective reaction solutions were transferred to an ultrafiltration filter, ULTRAFRE-MC 10,000NMWL Filter Unit (available from Millipore) and were subjected to centrifugal concentration. Thereafter, 25mM ammonium acetate buffer solution (pH 6.5) was added thereto, and the solution was again subjected to concentration with a centrifugal separator, thereby washing the polymer. This manipulation was repeated three times, thereby respectively obtaining aqueous solutions of compounds (166) to (170). Thereafter, to 150µl of the solution retained at the filter containing compounds (166) to (170) and 150µl solution which was obtained by diluting 60µl of aqueous solution of (165) with 25mM ammonium acetate buffer solution (pH 6.5), 0.75µl of 1.74mg/ml solution of BLase (available from Shionogi & Co., Ltd.) was added, and the solution was allowed to react for two hours at 25°C. Thereafter, the solution was subjected to centrifugal filtration with an ultrafiltration filter, ULTRAFRE-MC 10,000NMWL Filter Unit (available from Millipore), thereby separating the glycopeptide of interest from the polymer. The obtained aqueous solution (filtrate) was lyophilized, thereby obtaining compounds (171) to (176). MALDI-TOF/MS:[M(average)+H]⁺ of compound (171)=2288.0 (theoretical value:[M(average)+H]⁺=2288.0); MALDI-TOF/MS:[M(average)+H]⁺ of compound (172)=2612.5 (theoretical value:[M(average)+H]⁺=2612.1); MALDI-TOF/MS:[M(average)+H]⁺ of compound (173)=2579.6 (theoretical value:[M(average)+H]⁺=2579.1); MALDI-TOF/MS:[M(average)+H]⁺ of compound (174)=2903.2 (theoretical value:[M(average)+H]⁺=2903.2); MALDI-TOF/MS:[M(average)+H]⁺ of compound (175)=3194.2 (theoretical value:[M(average)+H]⁺=3194.3); and MALDI-TOF/MS:[M(average)+H]⁺ of compound (176)=3485.2 (theoretical value:[M(average)+H]⁺=3485.4).

### (3.28 Combinatorial synthesis of (97) to (162) using a distributing apparatus)

The above compounds (97) to (162) could be automatically synthesized using a distributing apparatus.

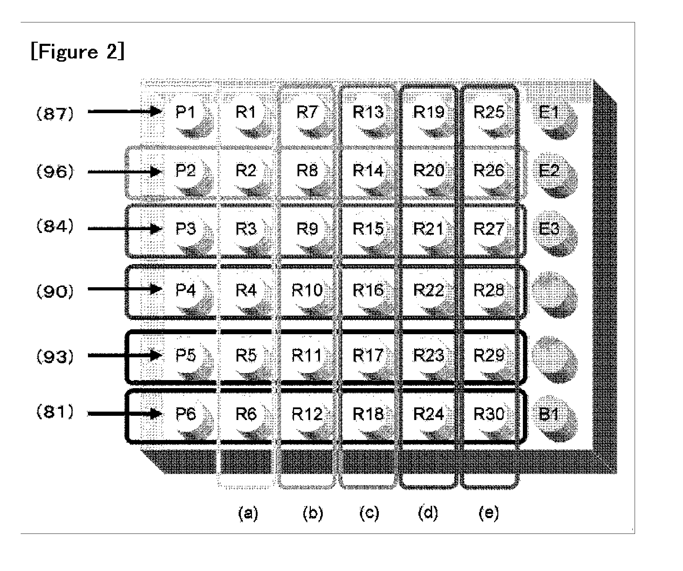

The following solutions P1-P6, E1-E3 and B1 were prepared, and were set in Hitachi Programmable Autosampler L-7250 where the temperature inside was set to 25°C, as shown in Figure 2.

P1: 50mM HEPES buffer solution (pH 7.0) containing 6.67mM compound (87) (theoretical concentration from solid-phase synthesis), 16.7mM MnCl₂ and 0.1% BSA
P2: 50mM HEPES buffer solution (pH 7.0) containing 6.67mM compound (96) (theoretical concentration from solid-phase synthesis), 16.7mM MnCl₂ and 0.1% BSA
P3: 50mM HEPES buffer solution (pH 7.0) containing 6.67mM compound (84) (theoretical concentration from solid-phase synthesis), 16.7mM MnCl₂ and 0.1% BSA
P4: 50mM HEPES buffer solution (pH 7.0) containing 6.67mM compound (90) (theoretical concentration from solid-phase synthesis), 16.7mM MnCl₂ and 0.1% BSA
P5: 50mM HEPES buffer solution (pH 7.0) containing 6.67mM compound (93) (theoretical concentration from solid-phase synthesis), 16.7mM MnCl₂ and 0.1% BSA
P6: 50mM HEPES buffer solution (pH 7.0) containing 6.67mM compound (81) (theoretical concentration from solid-phase synthesis), 16.7mM MnCl₂ and 0.1% BSA
E1: 50mM HEPES buffer solution (pH 7.0) containing 20mM uridine-5'-disodium diphosphogalactose (UDP-Gal), 1U/ml human-derived β1,4-galactosyltransferase (available from TOYOBO CO., LTD.) and 0.1% BSA
E2: 50mM HEPES buffer solution (pH 7.0) containing 20mM cytidine-5'-sodium monophosphosialate (CMP-NANA), 0.175U/ml recombinant rat α2,3-(O)-sialyltransferase (available from Calbiochem) and 0.1% BSA
E3: 50mM HEPES buffer solution (pH 7.0) containing 20mM cytidine-5'-sodium monophosphosialate (CMP-NANA), 0.185U/ml recombinant rat α2,3-(N)-sialyltransferase (available from Calbiochem) and 0.1% BSA
B1: 50mM HEPES buffer solution (pH 7.0) containing 0.1% BSA
In accordance with the program by Hitachi D-7000 HPLC system, P1-P6, E1-E3 and B1 were distributed for preparation of reaction to arrange R1-R30 so as to compose the following reactants (a) to (e):

(a) R1-R6: 250µl of reaction solution containing 50mM HEPES buffer solution (pH 7.0), 0.1U/ml human-derived β1,4-galactosyltransferase (available from TOYOBO CO., LTD.), 10mM manganese chloride, 0.1% BSA, 2mM uridine-5'-disodium diphosphogalactose (UDP-Gal) and glycopeptide derivative (R1: (87), R2: (96), R3: (84), R4: (90), R5:(93) or R6:(81)) (4mM at a theoretical content from solid-phase synthesis); (b) R7-R12: 250µl of reaction solution containing 50mM HEPES buffer solution (pH 7.0), 0.0175U/ml recombinant rat α2,3-(O)-sialyltransferase (available from Calbiochem), 10mM manganese chloride, 0.1% BSA, 2mM cytidine-5'-sodium monophosphosialate (CMP-NANA) and glycopeptide derivative (R7:(87), R8:(96), R9:(84), R10:(90), R11:(93) or R12:(81)) (4mM at a theoretical content from solid-phase synthesis) ; (c) R13-R18: 250µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2, 3- (O) -sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (R13:(87), R14:(96), R15:(84), R16:(90), R17:(93) or R18:(81))(4mM at a theoretical content from solid-phase synthesis); (d) R19-R24: 250µl of reaction solution containing 50mM HEPES buffer solution (pH 7.0), 0.1U/ml human-derived β1,4-galactosyltransferase (available from TOYOBO CO., LTD.), 0.0185U/ml recombinant rat α2,3-(N)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (R19:(87), R20:(96), R21:(84), R22:(90), R23:(93) or R24:(81)) (4mM at a theoretical content from solid-phase synthesis); and
(e) R25-R30: 250µl of reaction solution containing 50mM HEPES buffer solution (pH 7.0), 0.1U/ml human-derived β1,4-galactosyltransferase (available from TOYOBO CO., LTD.), 0.175U/ml recombinant rat α2,3-(O)-sialyltransferase (available from Calbiochem), 0.1875U/ml recombinant rat α2,3-(N)-sialyltransferase (available from Calbiochem) 10mM manganese chloride, 0.1% BSA, 2mM uridine-5'-disodium diphosphogalactose (UDP-Gal), 4mM cytidine-5'-sodium monophosphosialate (CMP-NANA) and glycopeptide derivative (R25:(87), R26:(96), R27:(84), R28:(90), R29:(93) or R30:(81)) (4mM at a theoretical content from solid-phase synthesis).

After distribution, the solutions were allowed to react for 24 hours at 25°C. After completion of the reaction, the respective reaction solution were transferred to an ultrafiltration filter, ULTRAFRE-MC 10,000NMWL Filter Unit (available from Millipore) and were subjected to centrifugal concentration. Thereafter, 25mM ammonium acetate buffer solution (pH 6.5) was added thereto, and the solution was again subjected to concentration with centrifugal separator, thereby washing the polymer. This manipulation was repeated three times, thereby respectively obtaining aqueous solutions (97) to (101), (108) to (112), (119) to (123), (130) to (134), (141) to (145) and (152) to (156). Thereafter, to 150µl of solution retained at the filter containing compounds (97) to (101), (108) to (112), (119) to (123), (130) to (134), (141) to (145) and (152) to (156), and 150µl of the respective aqueous solutions which was obtained by diluting aqueous solutions of (81), (84), (87), (90), (93) and (96) with 25mM ammonium buffer solution (pH 6.5), 1µl of 0.174mg/ml solution of BLase (available from Shionogi & Co., Ltd.) was added. The respective solutions were allowed to react for two hours at room temperature, and were subsequently subjected to centrifugal filtration with an ultrafiltration filter, ULTRAFRE-MC 10,000NMWL Filter Unit (available from Millipore), thereby separating the glycopeptide of interest from the polymer. The obtained aqueous solutions (filtrates) were lyophilized, thereby obtaining compounds (102) to (107), (113) to (118), (124) to (129), (135) to (140), (146) to (151) and (157) to (162).

(3.29 Synthesis of compounds (177) to (179) using a one-pot reaction)

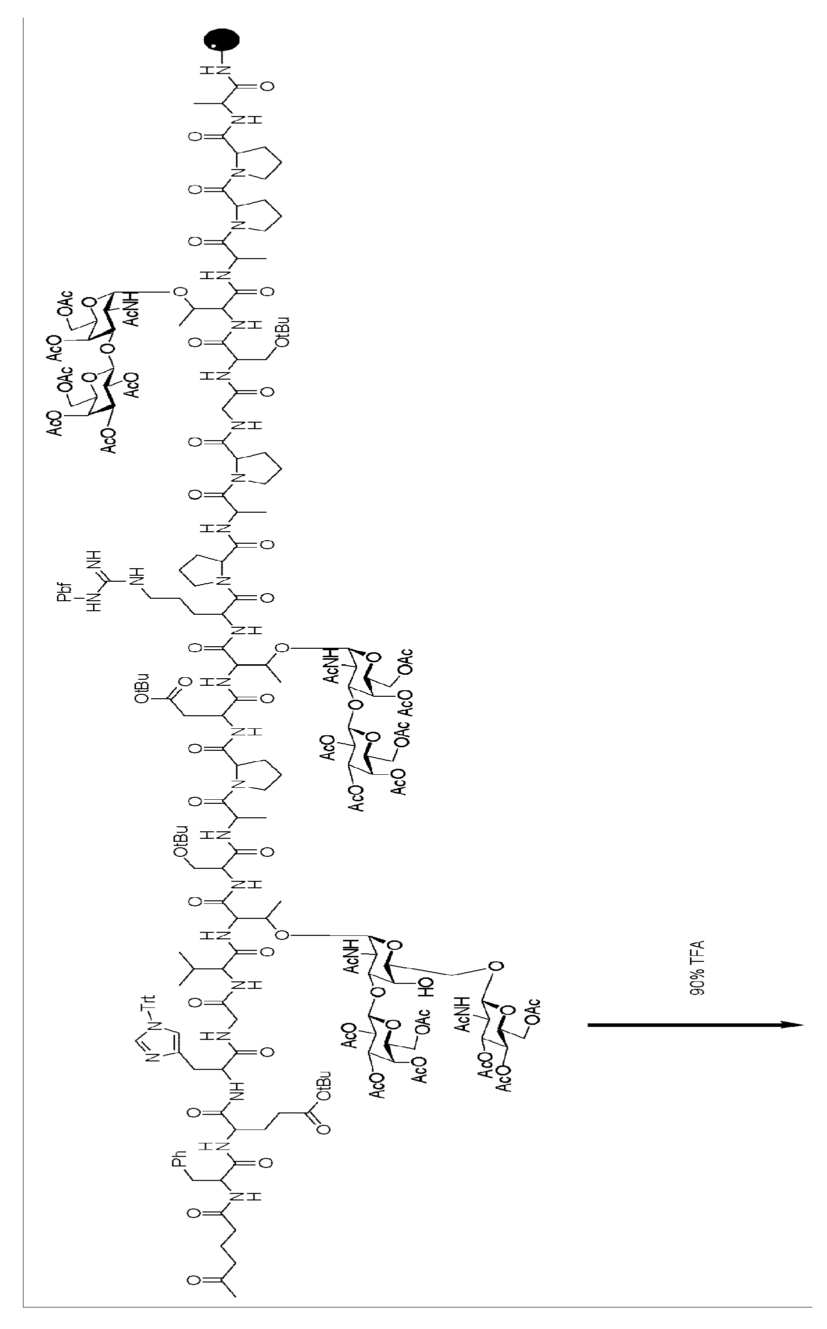

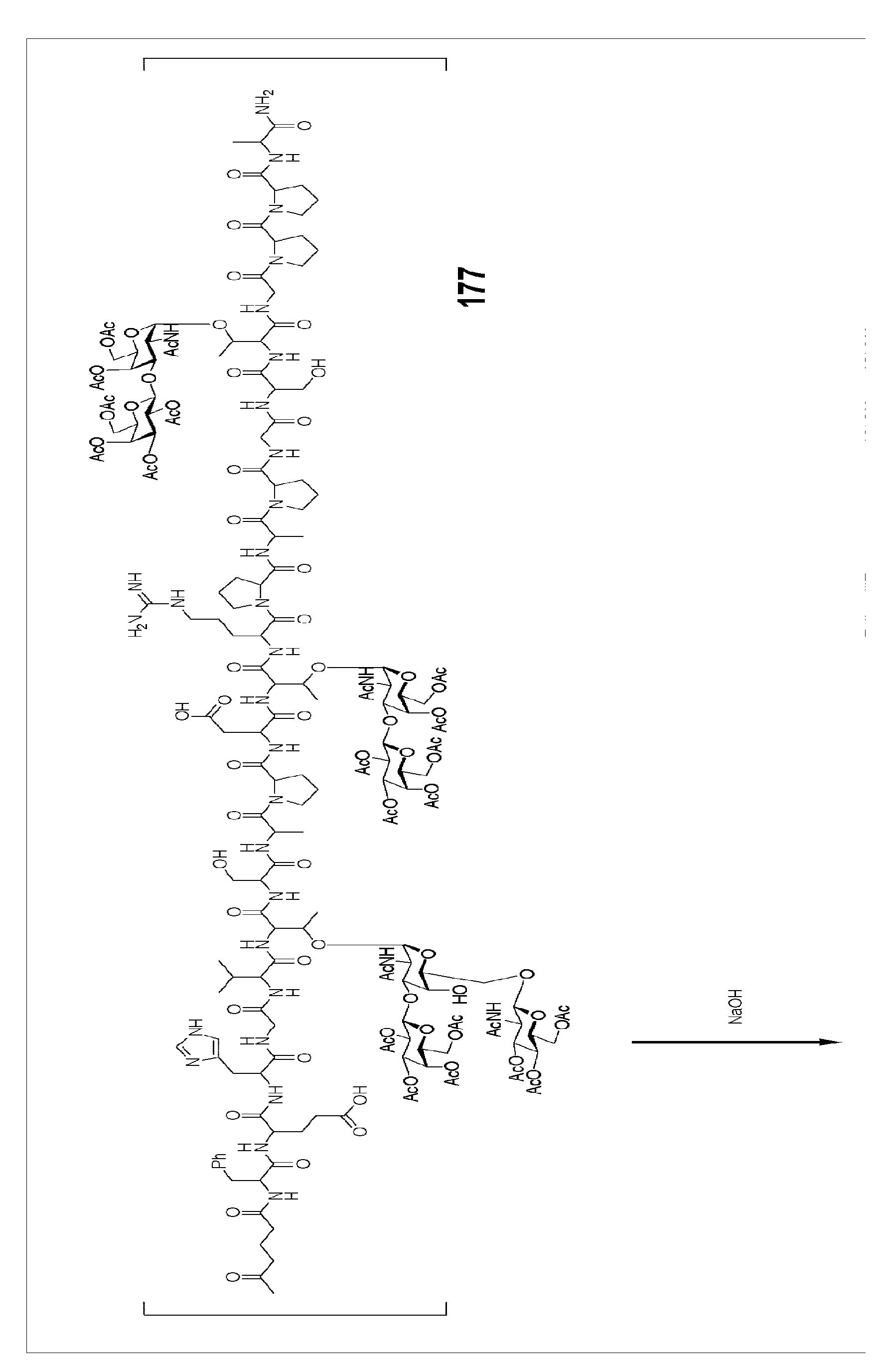

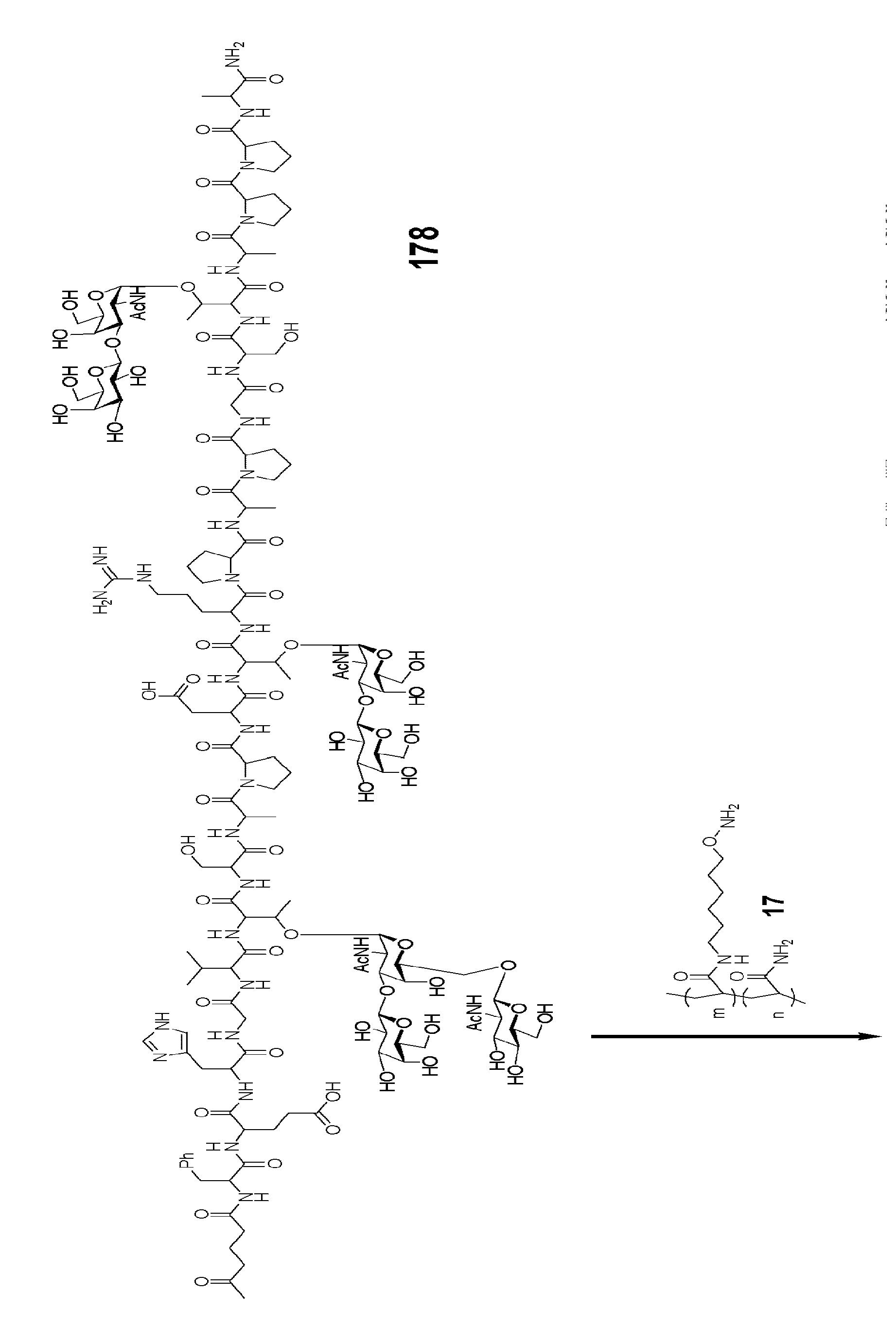

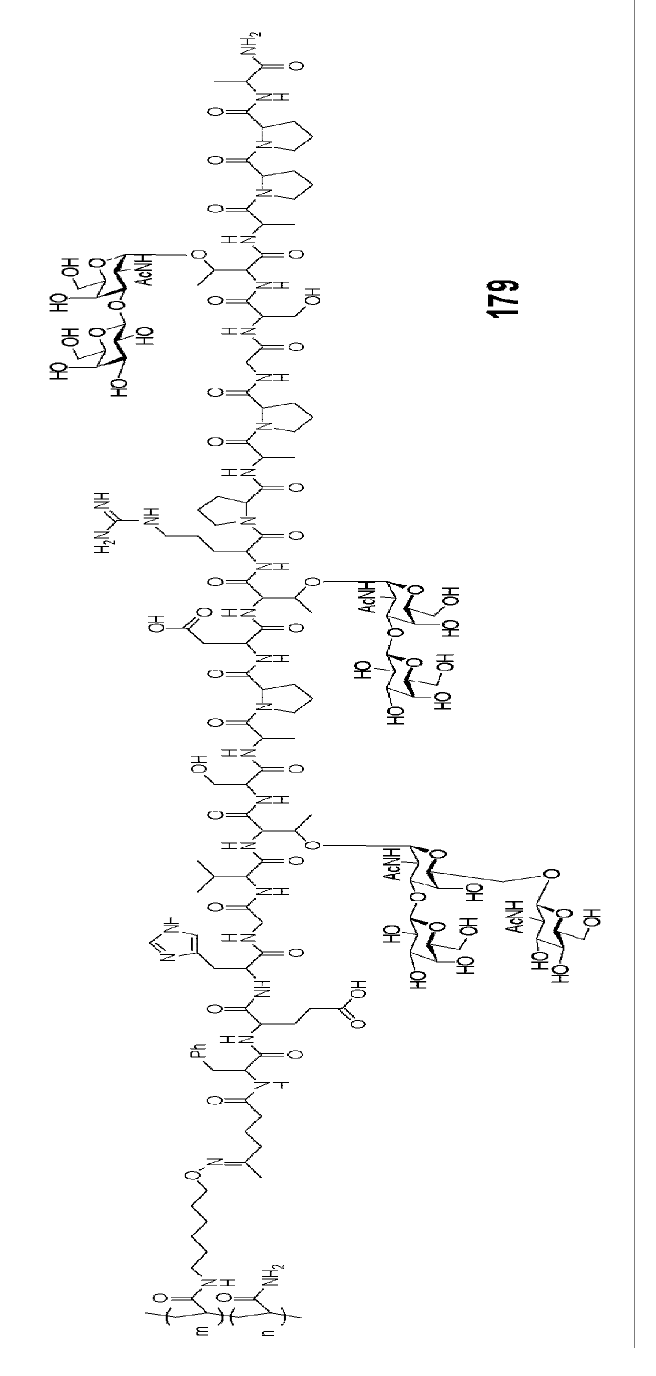

Using 71mg (0.02mmol) of Tentagel® S RAM resin (Hipep Laboratories, 0.28mmol/g) as a support, N-protected amino acids and keto acids as described below were sequentially condensed by Fmoc/HBTU/HOBt method, thereby synthesizing a glycopeptide derivative of interest: Fmoc-Ala-OH; Fmoc-Pro-OH; Fmoc-Pro-OH; Fmoc-Ala-OH; Fmoc-Thr(Ac6core1)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(Ac6corel)-OH; Fmoc-Asp(OtBu)-OH; Fmoc-Pro-OH; Fmoc-Ala-OH; Fmoc-Ser(tBu)-OH; Fmoc-Thr(Ac7core2)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained resin equivalent to 5µmol was allowed to react in 90% TFA aqueous solution for two hours at room temperature to eliminate a protective group on a peptide residue and concurrently release compound (177) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 1.5ml of methanol. 1N sodium hydroxide aqueous solution was added to this solution, and was adjusted to have a pH of 12-12.5. The solution was then stirred for 1.5 hours at room temperature, thereby performing Ac deprotection reaction. After the reaction, 1N acetic acid was added and the solution was neutralized. Thereafter, the resin was separated by filtration. The solvent in the filtrate was removed, and the residue was dissolved in 0.5ml of 50mM acetic acid/sodium acetate buffer solution (pH 5.5). 0.5m1 of 10mM (oxyamine residue calculation) water-soluble polymer (17) aqueous solution was added to the solution, and the solution was stirred for 18 hours at room temperature, thereby reacting compound (178) with compound (17). After completion of the reaction, the reaction solution was subjected to centrifugal concentration with an ultrafiltration filter 10K Apollo® 20ml (Orbital Biosciences, available from LIC). 25mM HEPES buffer solution (pH 7.0) was added thereto and the solution was again subjected to concentration, thereby washing the polymer. By adding water so that the final amount of the solution was 0.5ml, 10mM (theoretical content of glycopeptide) polymer (179) was obtained. Identification of the polymer (179) was performed based on that product (254) was obtained in the following section (3.53).

(3.30 Synthesis of compounds (180) to (182) using a one-pot reaction)

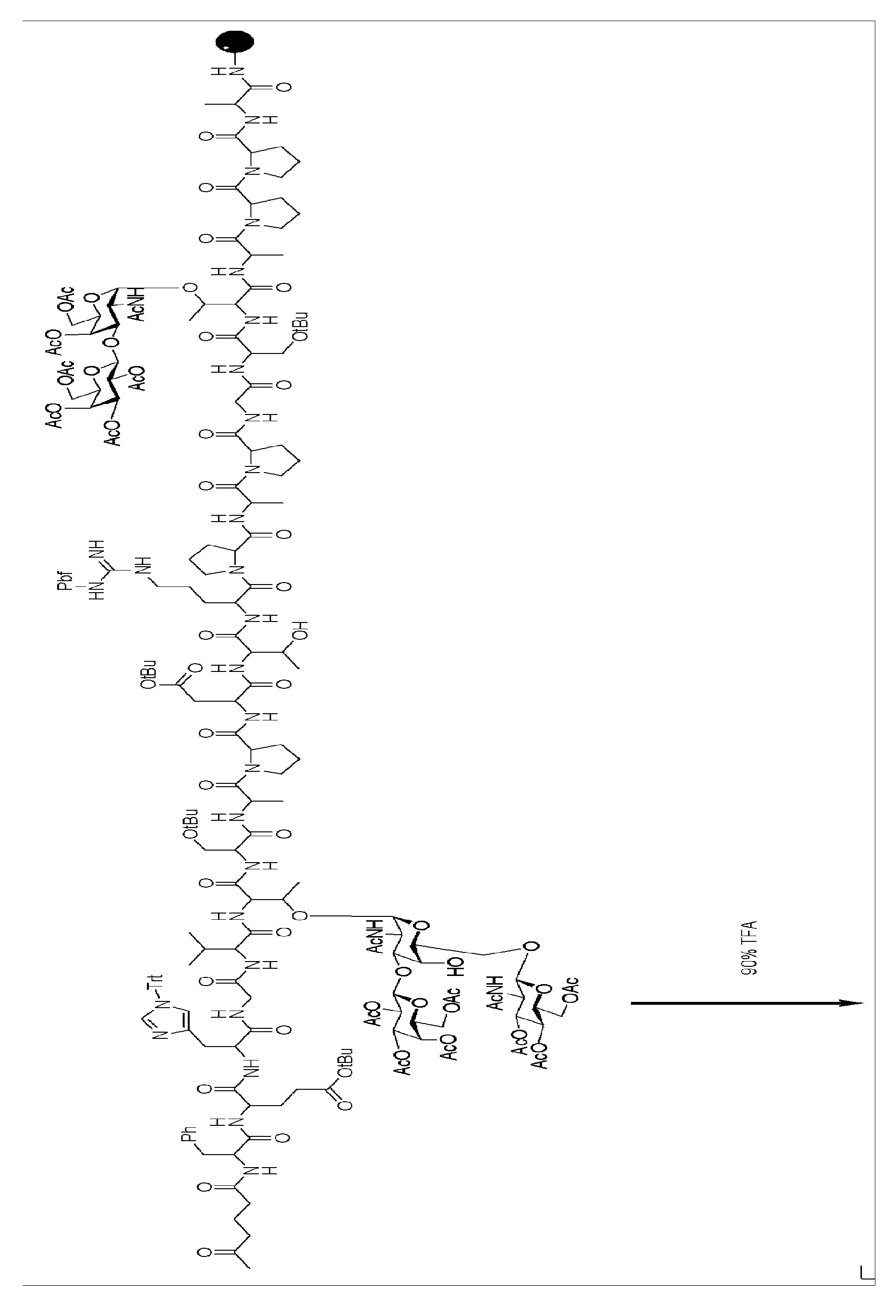

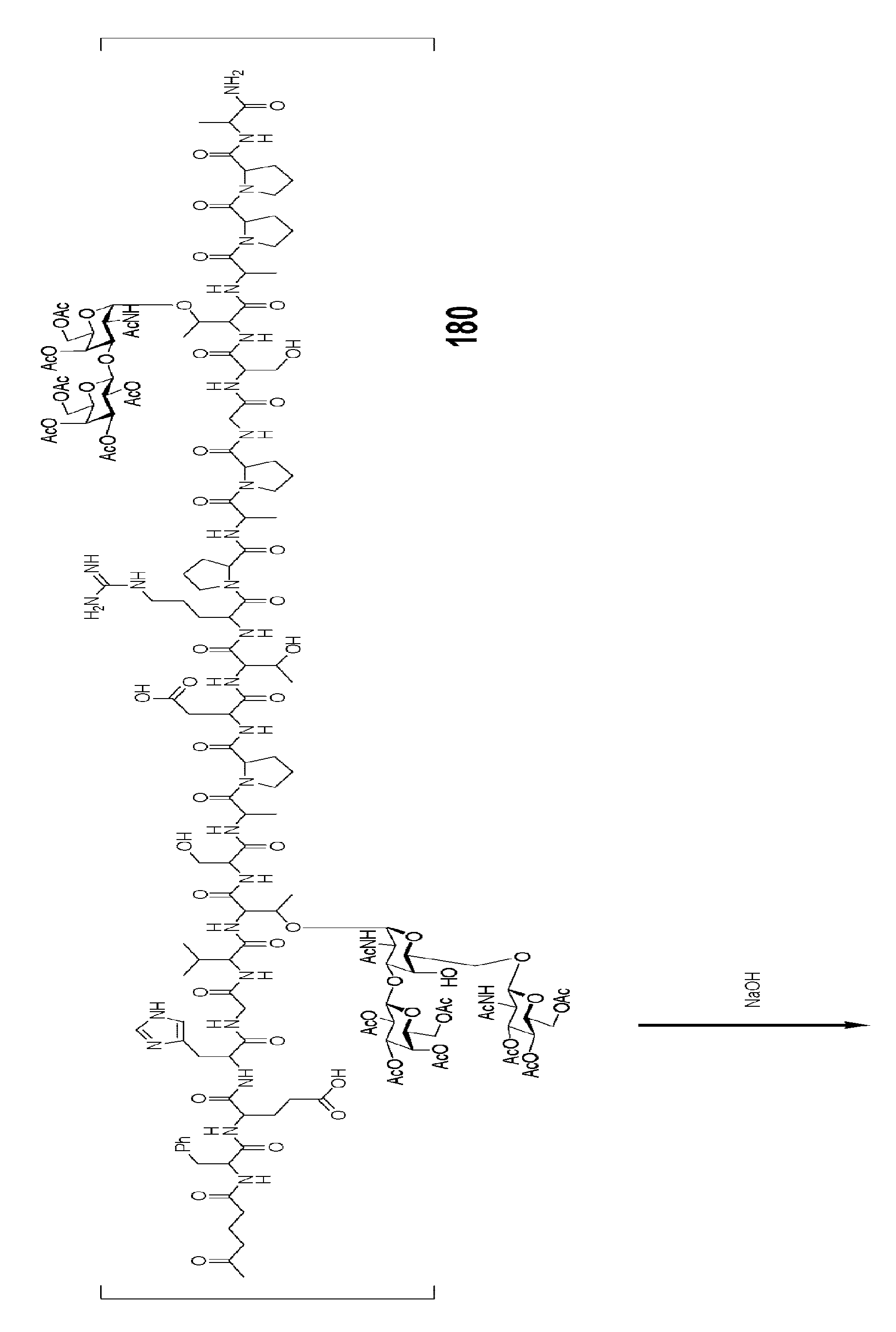

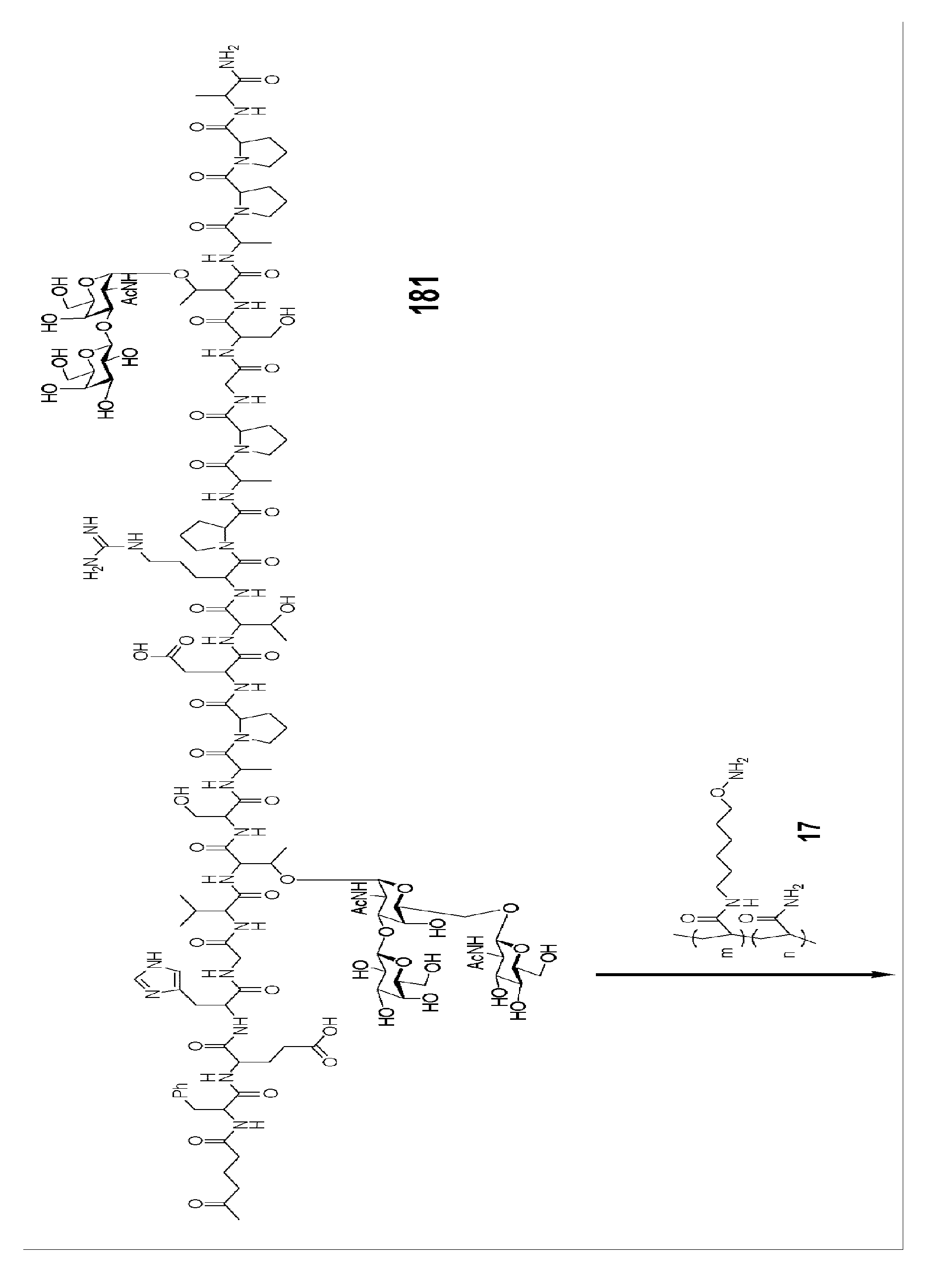

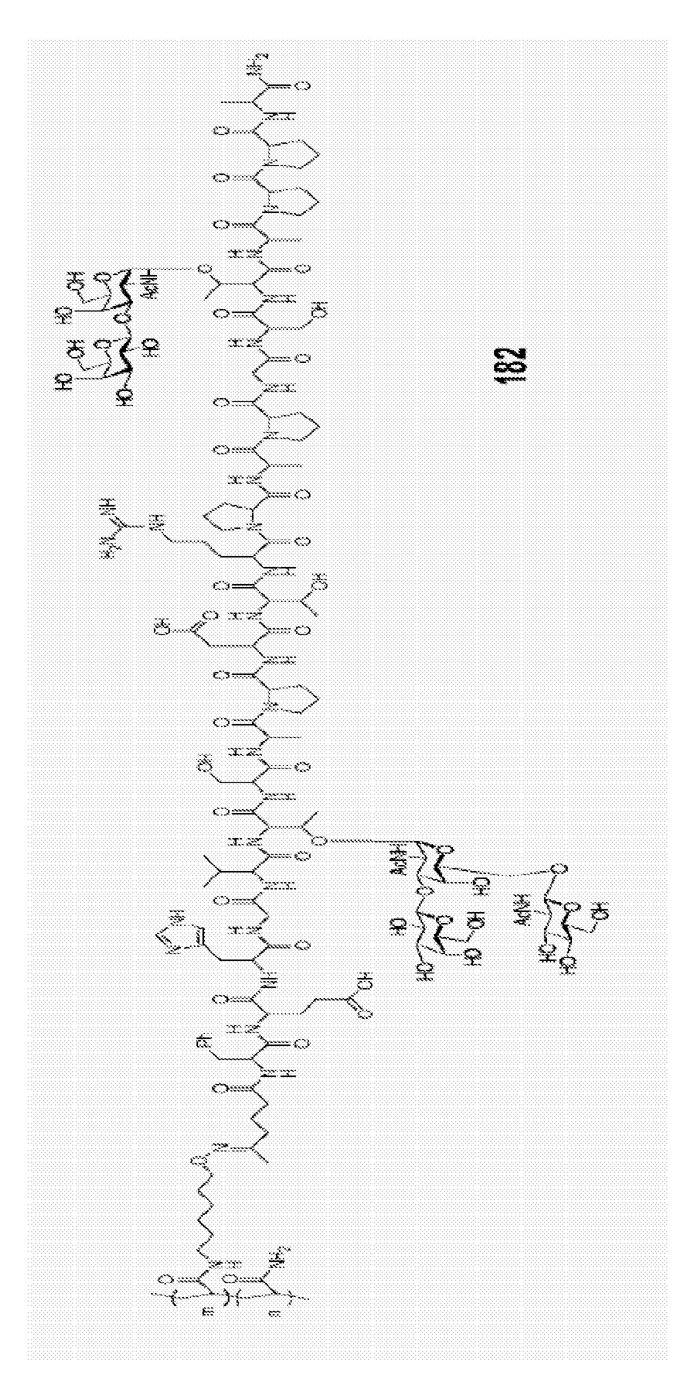

Using 71mg (0.02mmol) of Tentagel® S RAM resin (Hipep Laboratories, 0.28mmol/g) as a support, N-protected amino acids and keto acids as described below were sequentially condensed by Fmoc/HBTU/HOBt method, thereby synthesizing a glycopeptide derivative of interest: Fmoc-Ala-OH; Fmoc-Pro-OH; Fmoc-Pro-OH; Fmoc-Ala-OH; Fmoc-Thr(Ac6corel)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(tBu)-OH; Fmoc-Asp(OtBu)-OH; Fmoc-Pro-OH; Fmoc-Ala-OH; Fmoc-Ser(tBu)-OH; Fmoc-Thr(Ac7core2)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained resin equivalent to 5nmol was allowed to react in 90% TFA aqueous solution for two hours at room temperature to eliminate a protective group on a peptide residue and concurrently release compound (180) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 1.5ml of methanol. 1N sodium hydroxide aqueous solution was added to this solution, and was adjusted to have a pH of 12-12.5. The solution was then stirred for 1.5 hours at room temperature, thereby performing Ac deprotection reaction. After the reaction, 1N acetic acid was added and the solution was neutralized. Thereafter, the resin was separated by filtration. The solvent in the filtrate was removed, and the residue was dissolved in 0.5ml of 50mM acetic acid/sodium acetate buffer solution (pH 5.5). 0.5ml of 10mM (oxyamine residue calculation) water-soluble polymer (17) aqueous solution was added to the solution, and the solution was stirred for 18 hours at room temperature, thereby reacting compound (181) with compound (17). After completion of the reaction, the reaction solution was subjected to centrifugal concentration with an ultrafiltration filter 10K Apollo ® 20ml (Orbital Biosciences, available from LIC). 25mM HEPES buffer solution (pH 7.0) was added thereto and the solution was again subjected to concentration, thereby washing the polymer. By adding water so that the final amount of the solution was 0.5ml, 10mM (theoretical content of glycopeptide) polymer (182) was obtained. Identification of the polymer (182) was performed based on that product (265) was obtained in the following section (3.54).

(3.31 Synthesis of compounds (183) to (185) using a one-pot reaction)

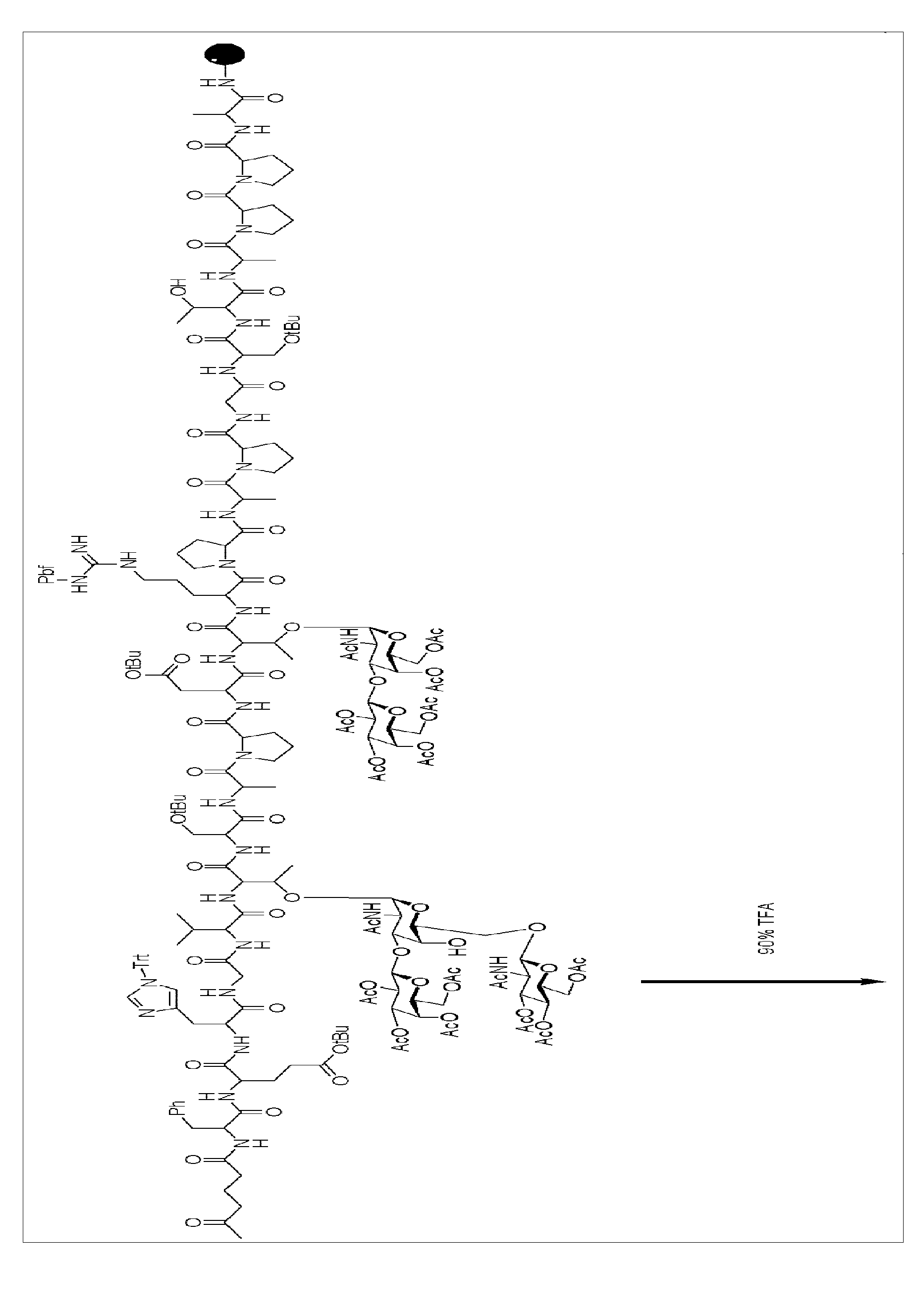

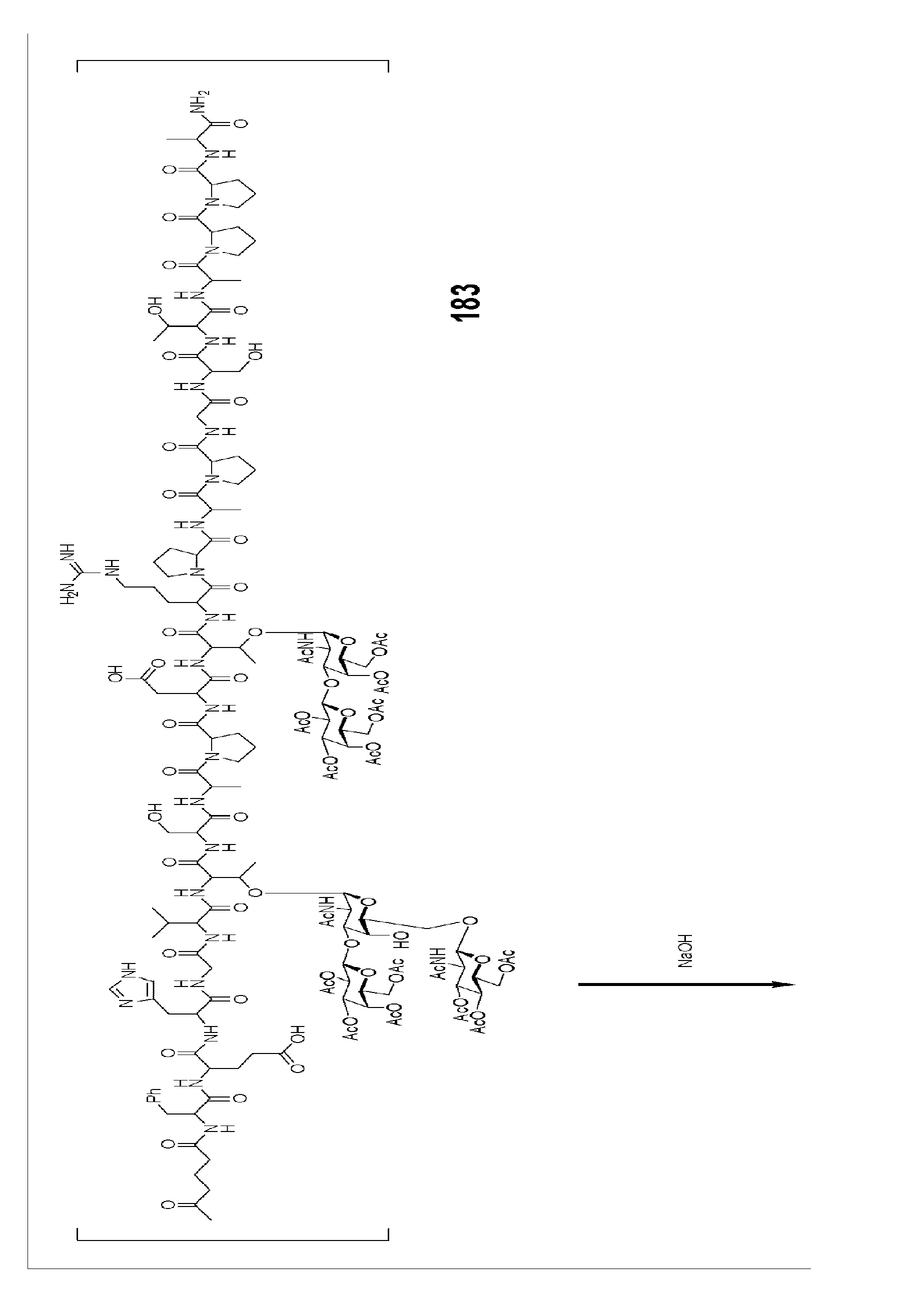

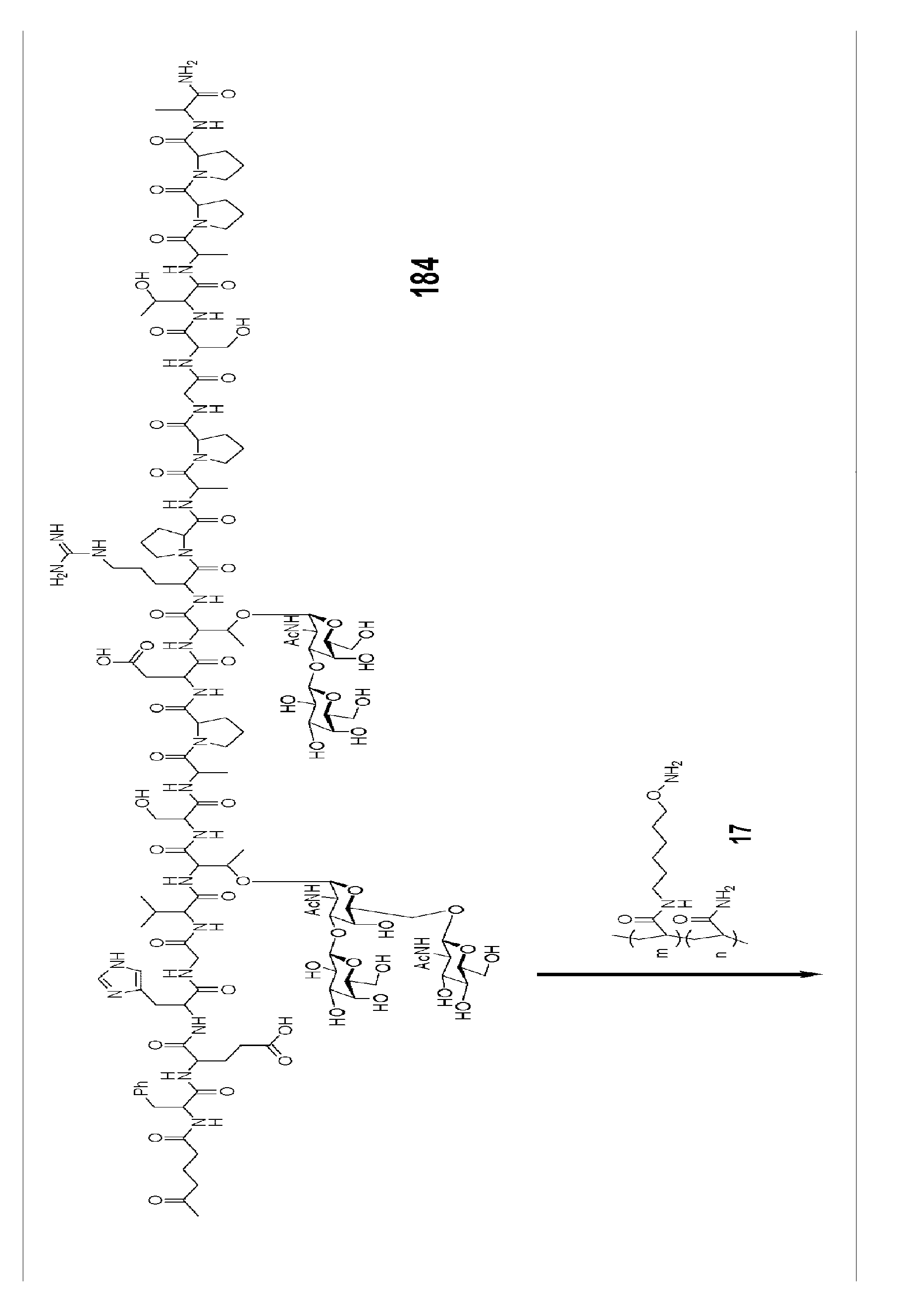

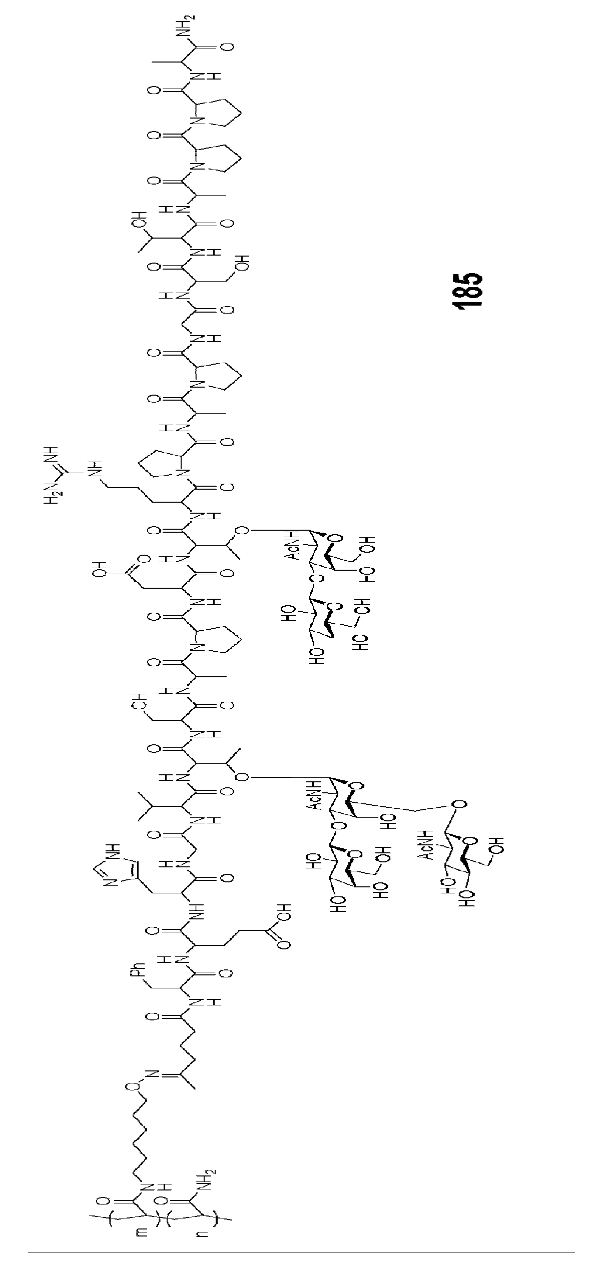

Using 71mg (0.02mmol) of Tentagel® S RAM resin (Hipep Laboratories, 0.28mmol/g) as a support, N-protected amino acids and keto acids as described below were sequentially condensed by Fmoc/HBTU/HOBt method, thereby synthesizing a glycopeptide derivative of interest: Fmoc-Ala-OH; Fmoc-Pro-OH; Fmoc-Pro-OH; Fmoc-Ala-OH; Fmoc-Thr(tBu)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(Ac6core1)-OH; Fmoc-Asp(OtBu)-OH; Fmoc-Pro-OH; Fmoc-Ala-OH; Fmoc-Ser(tBu)-OH; Fmoc-Thr(Ac7core2)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained resin equivalent to 5nmol was allowed to react in 90% TFA aqueous solution for two hours at room temperature to eliminate a protective group on a peptide residue and concurrently release compound (183) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether was again added, and the precipitate was washed. Centrifugal separation was again performed and the supernatant was removed. The obtained precipitant was dissolved in 1.5ml of methanol. 1N sodium hydroxide aqueous solution was added to this solution, and was adjusted to have a pH of 12-12.5. The solution was then stirred for 1.5 hours at room temperature, thereby performing Ac deprotection reaction. After the reaction, 1N acetic acid was added and the solution was neutralized. Thereafter, the resin was separated by filtration. The solvent in the filtrate was removed, and the residue was dissolved in 0.5ml of 50mM acetic acid/sodium acetate buffer solution (pH 5.5). 0.5ml of 10mM (oxyamine residue calculation) water-soluble polymer (17) aqueous solution was added to the solution, and the solution was stirred for 18 hours at room temperature, thereby reacting compound (184) with compound (17). After completion of the reaction, the reaction solution was subjected to centrifugal concentration with an ultrafiltration filter 10K Apollo ® 20ml (Orbital Biosciences, available from LIC). 25mM HEPES buffer solution (pH 7.0) was added thereto and the solution was again subjected to concentration, thereby washing the polymer. By adding water so that the final amount of the solution was 0.5ml, 10mM (theoretical content of glycopeptide) polymer (185) was obtained. Identification of the polymer (185) was performed based on that product (276) was obtained in the following section (3.55).

(3.32 Synthesis of compounds (186) to (188) using a one-pot reaction)

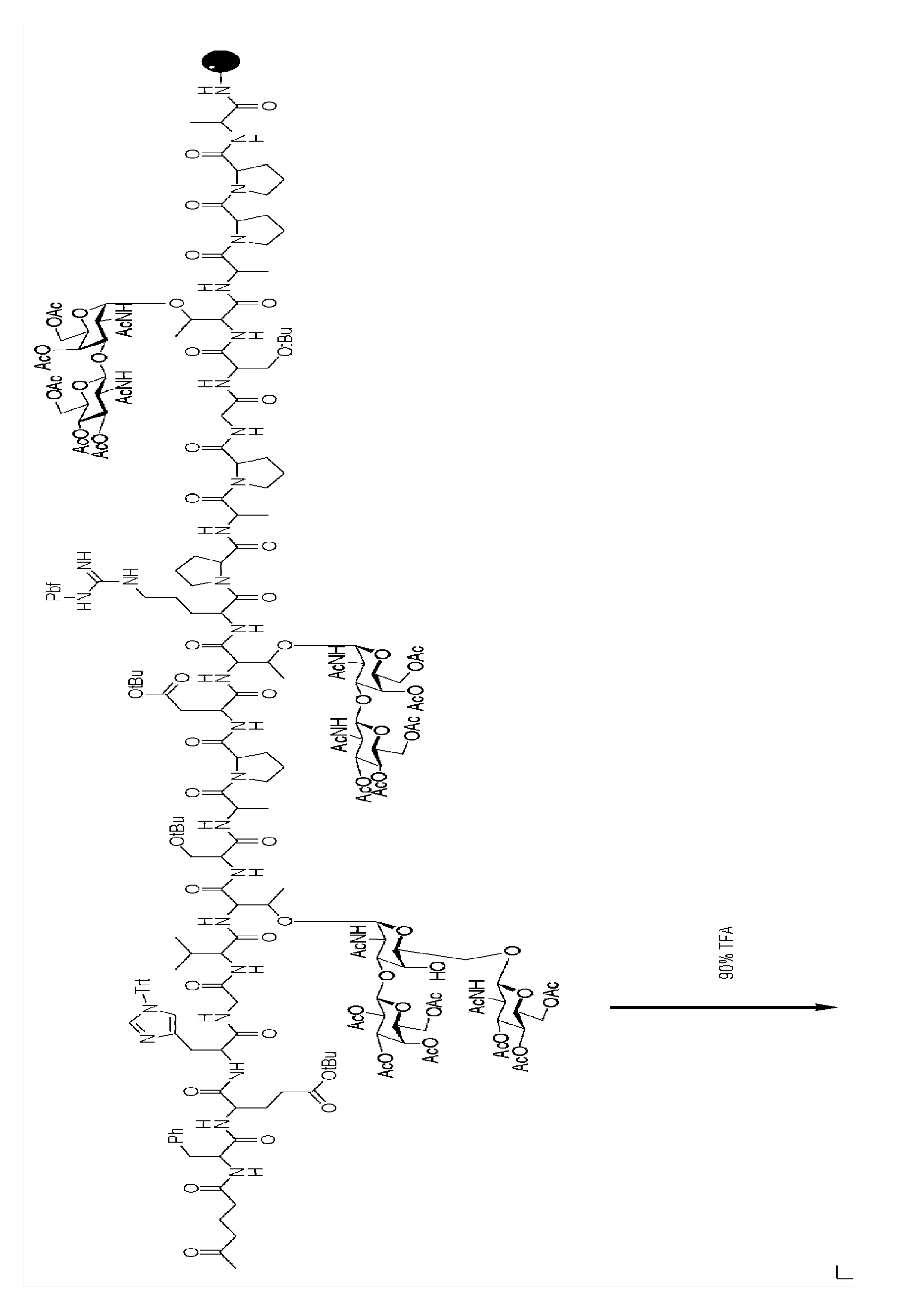

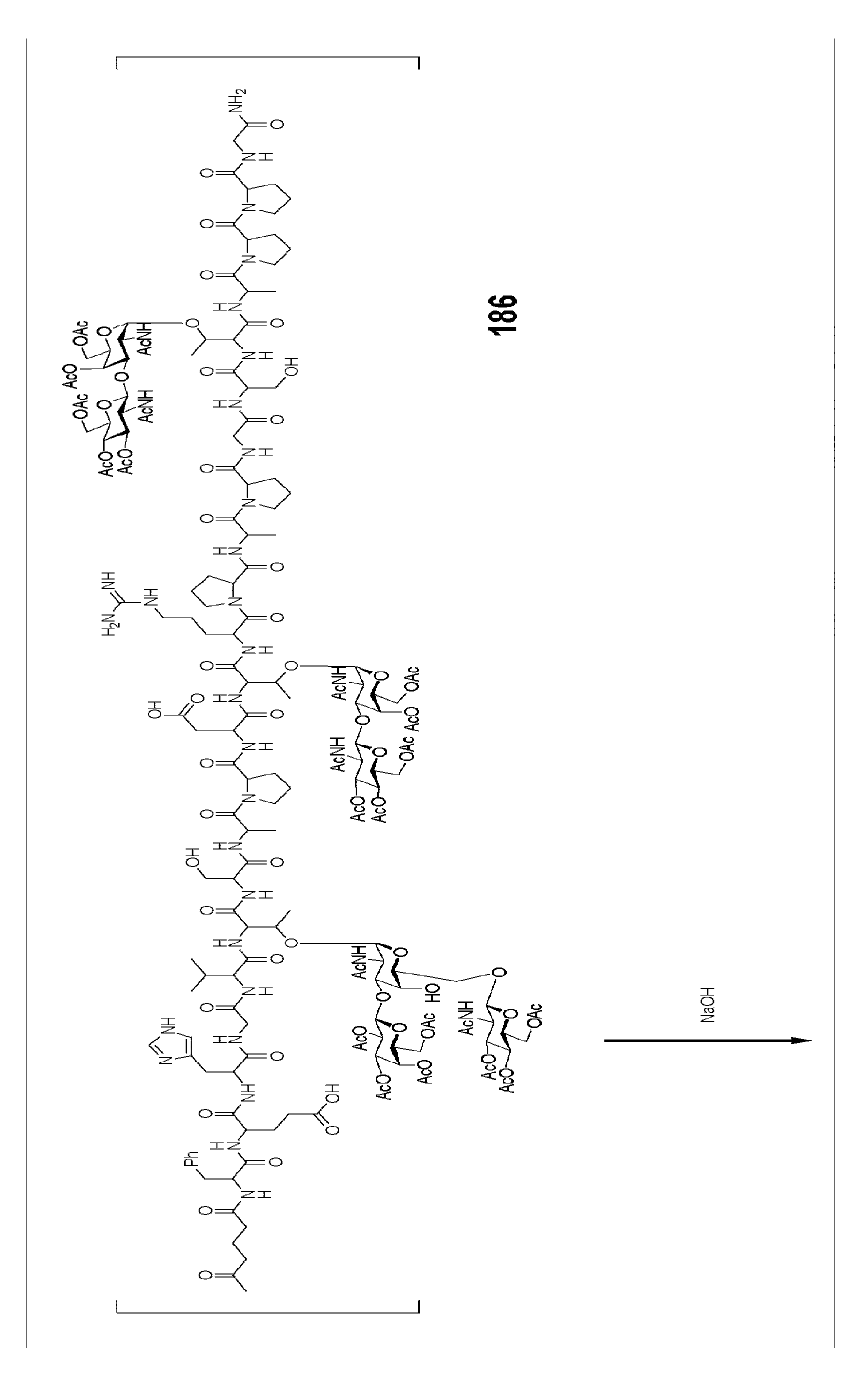

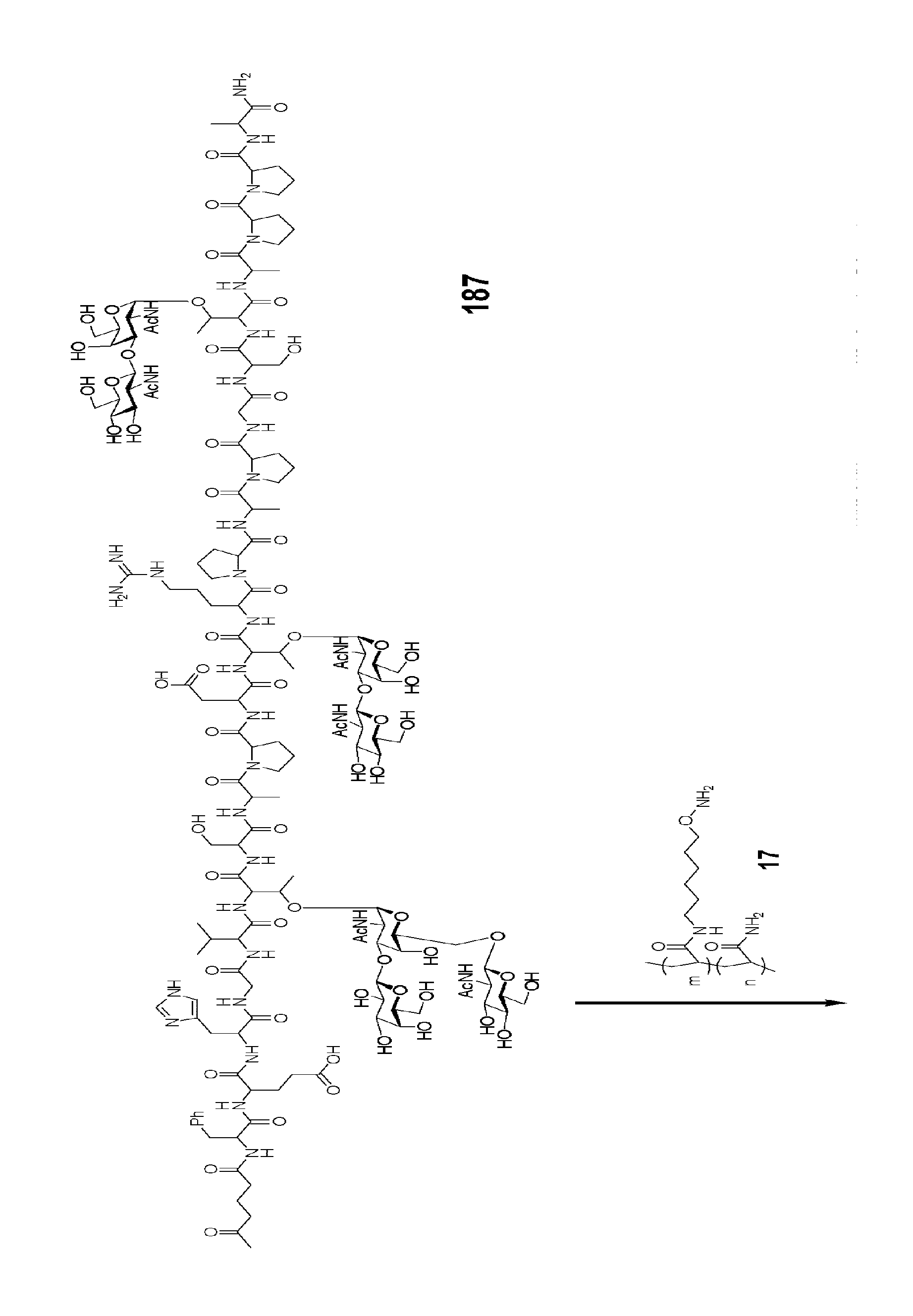

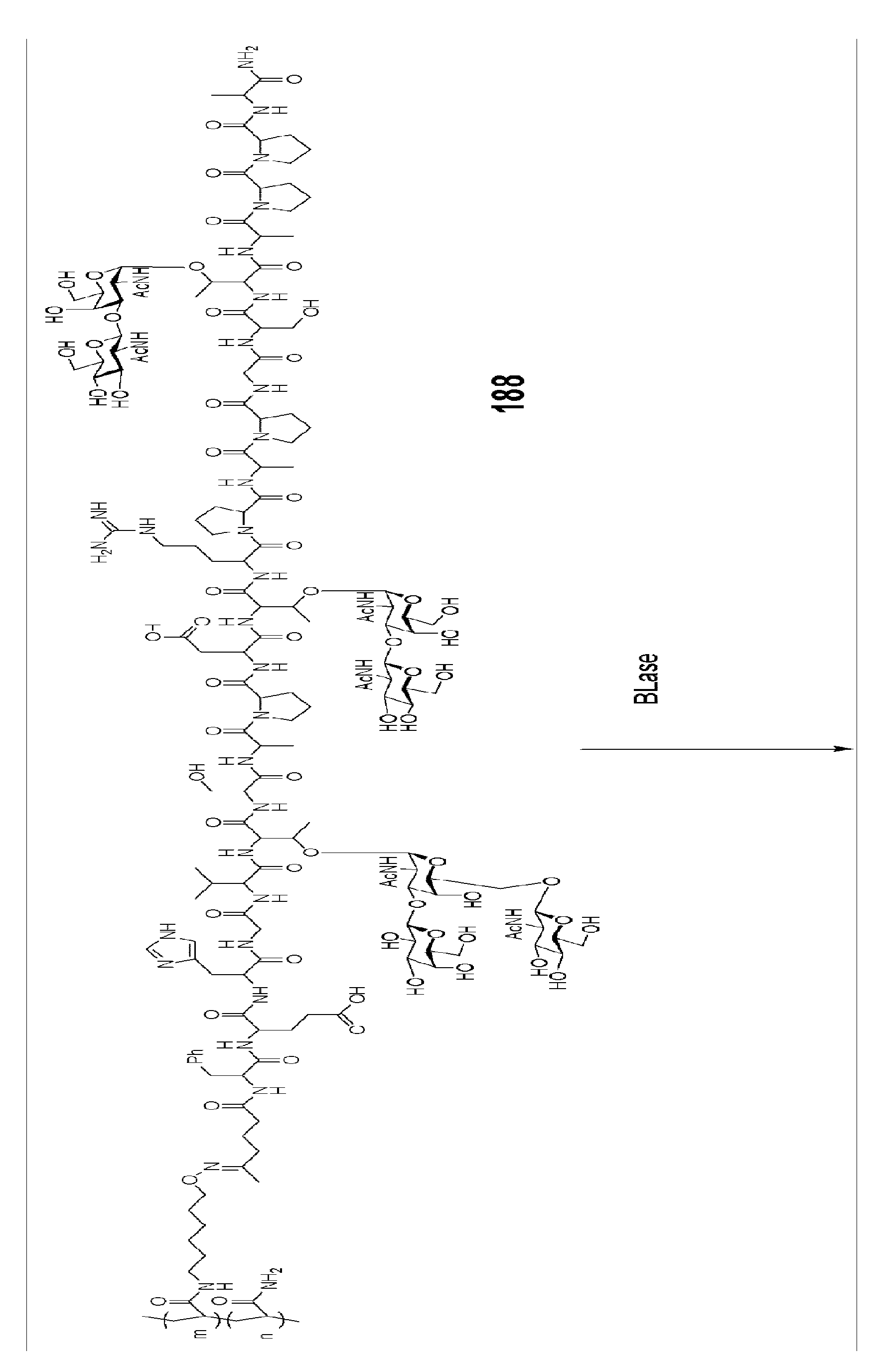

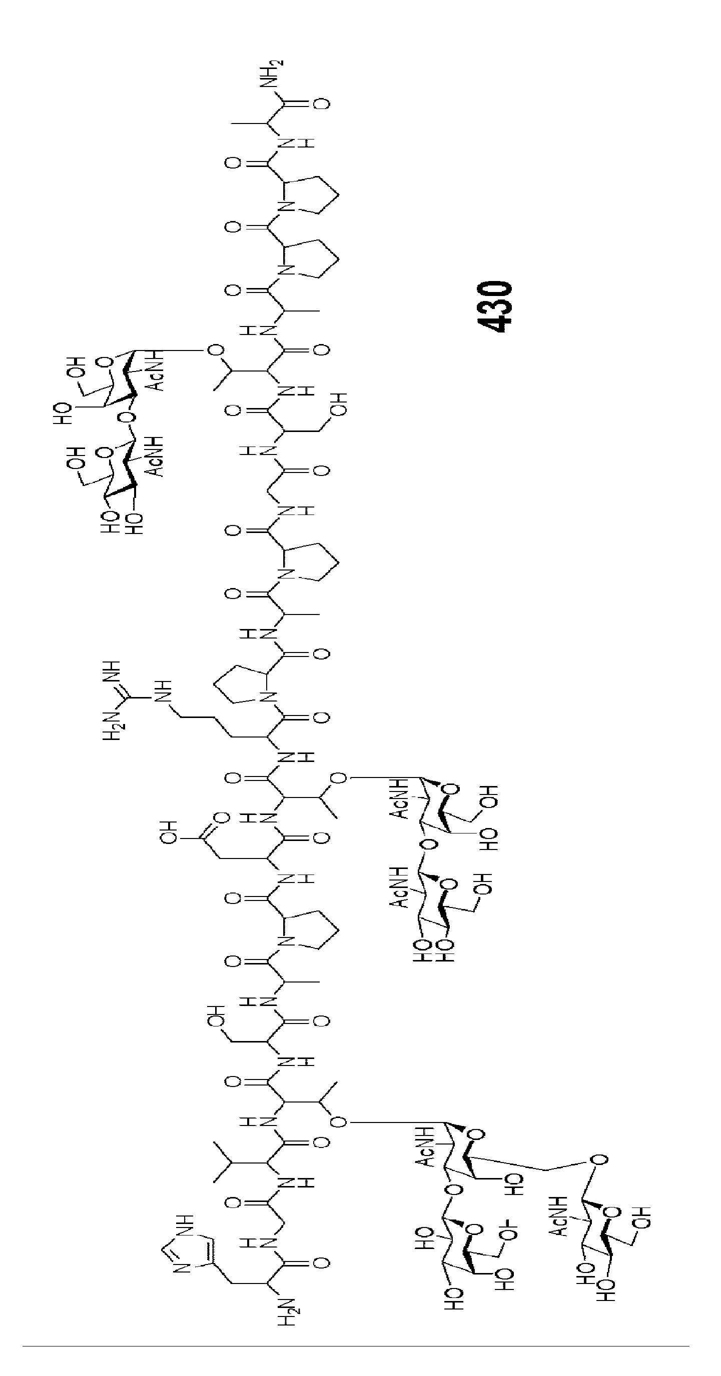

Using 71mg (0.02mmol) of Tentagel® S RAM resin (Hipep Laboratories, 0.28mmol/g) as a support, N-protected amino acids and keto acids as described below were sequentially condensed by Fmoc/HBTU/HOBt method, thereby synthesizing a glycopeptide derivative of interest: Fmoc-Ala-OH; Fmoc-Pro-OH; Fmoc-Pro-OH; Fmoc-Ala-OH; Fmoc-Thr(Ac5core3)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(Ac5core3)-OH; Fmoc-Asp(OtBu)-OH; Fmoc-Pro-OH; Fmoc-Ala-OH; Fmoc-Ser(tBu)-OH; Fmoc-Thr(Ac7core2)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained resin equivalent to 5nmol was allowed to react in 90% TFA aqueous solution for two hours at room temperature to eliminate a protective group on a peptide residue and concurrently release compound (186) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 1.5ml of methanol. 1N sodium hydroxide aqueous solution was added to this solution, and was adjusted to have a pH of 12-12.5. The solution was then stirred for 1.5 hours at room temperature, thereby performing Ac deprotection reaction. After the reaction, 1N acetic acid was added and the solution was neutralized. Thereafter, the resin was separated by filtration. The solvent in the filtrate was removed, and the residue was dissolved in 0.5ml of 50mM acetic acid/sodium acetate buffer solution (pH 5.5). 0.5ml of 10mM (oxyamine residue calculation) water-soluble polymer (17) aqueous solution was added to the solution, and the solution was stirred for 18 hours at room temperature, thereby reacting compound (187) with compound (17). After completion of the reaction, the reaction solution was subjected to centrifugal concentration with an ultrafiltration filter 10K Apollo ® 20ml (Orbital Biosciences, available from LIC). 25mM HEPES buffer solution (pH 7.0) was added thereto and the solution was again subjected to concentration, thereby washing the polymer. By adding water so that the final amount of the solution was 0.5ml, 10mM (theoretical content of glycopeptide) polymer (180) was obtained. Identification of the polymer (188) was performed by treating a part of polymer (188) with BLase to obtain product (430). MALDI-TOF/MS:[M(average)+H]⁺of compound (430)=3269.9 (theoretical value: [M(average)+H]⁺=3268.3).

(3.33 Synthesis of compounds (189) to (191) using a one-pot reaction)

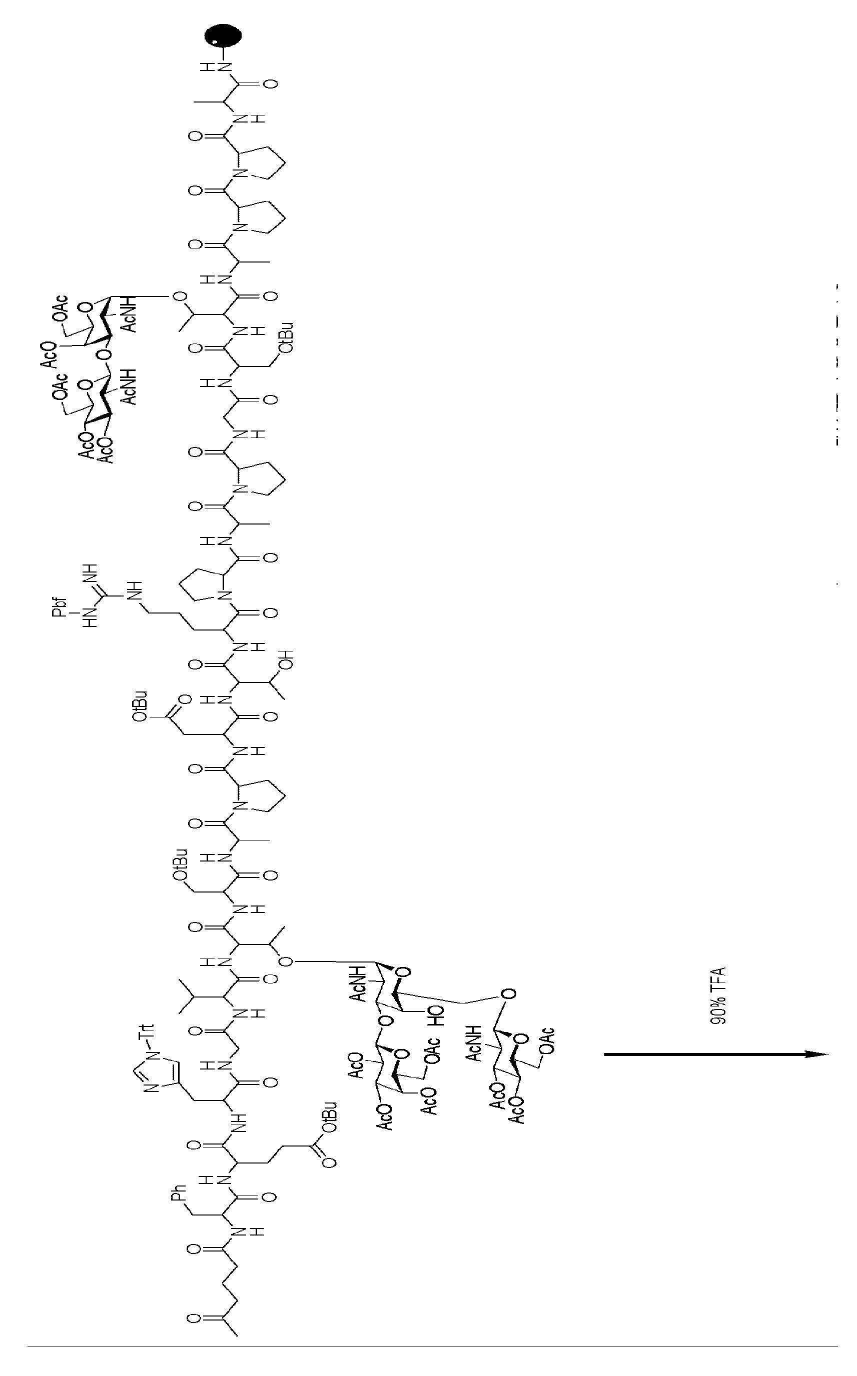

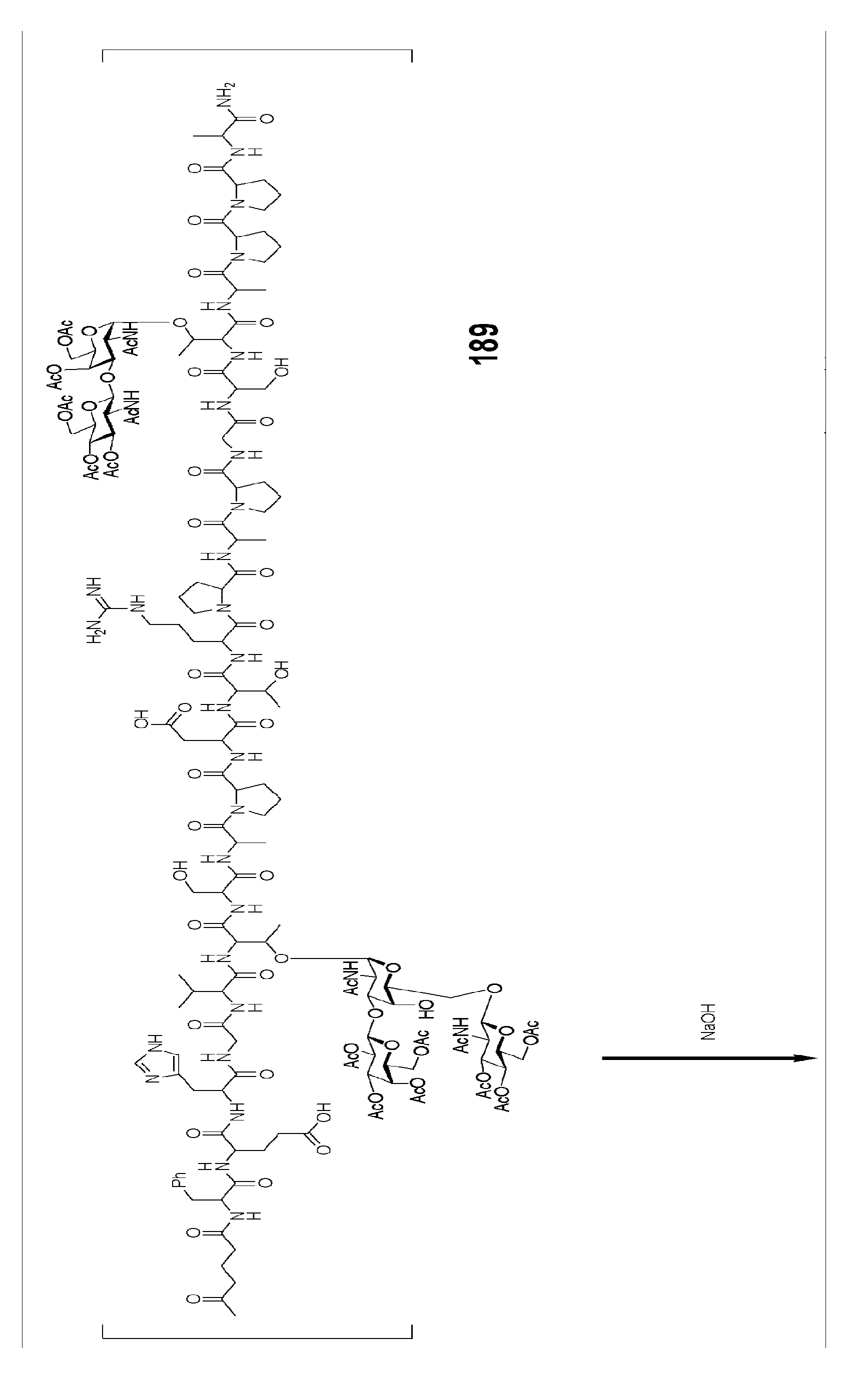

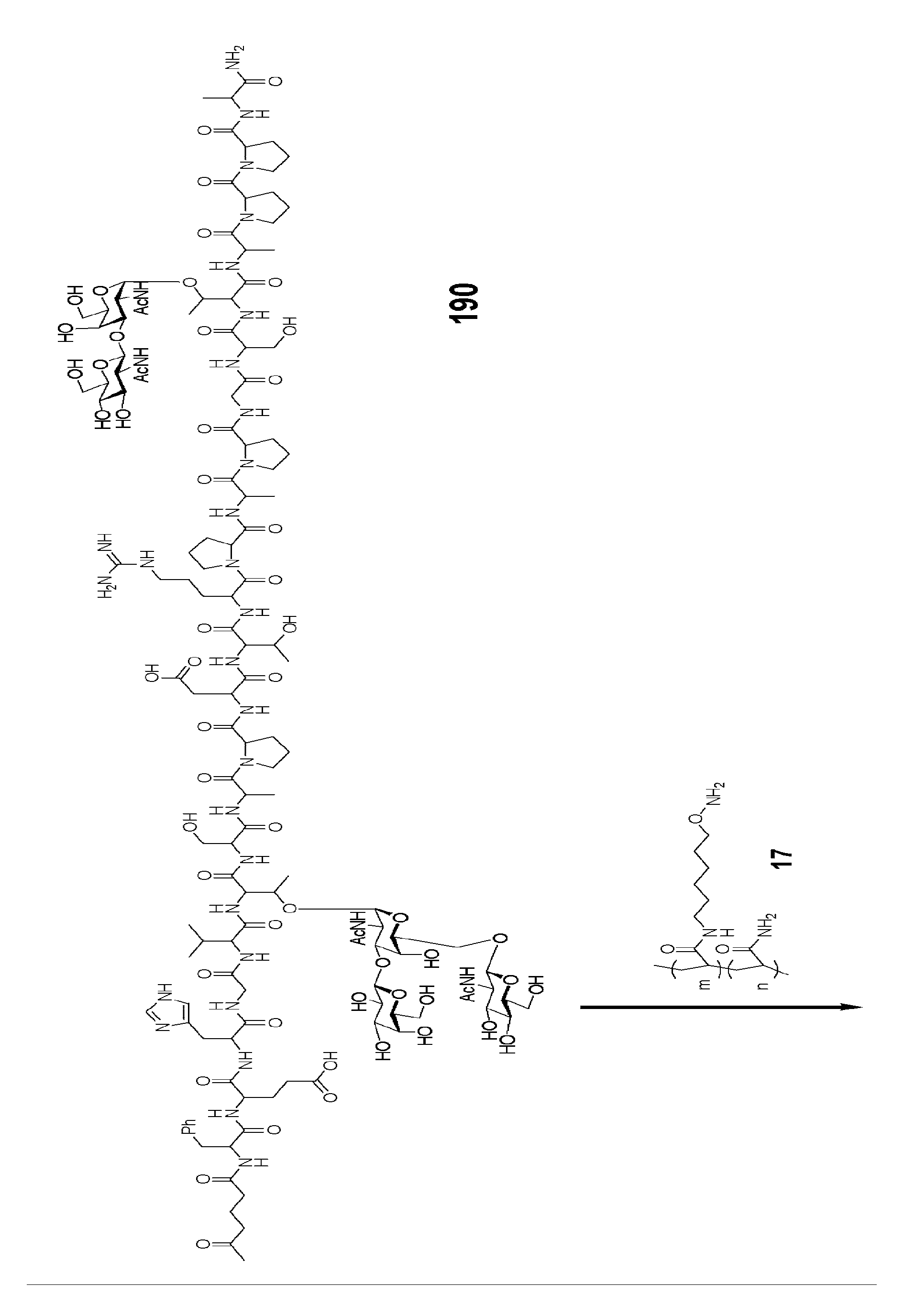

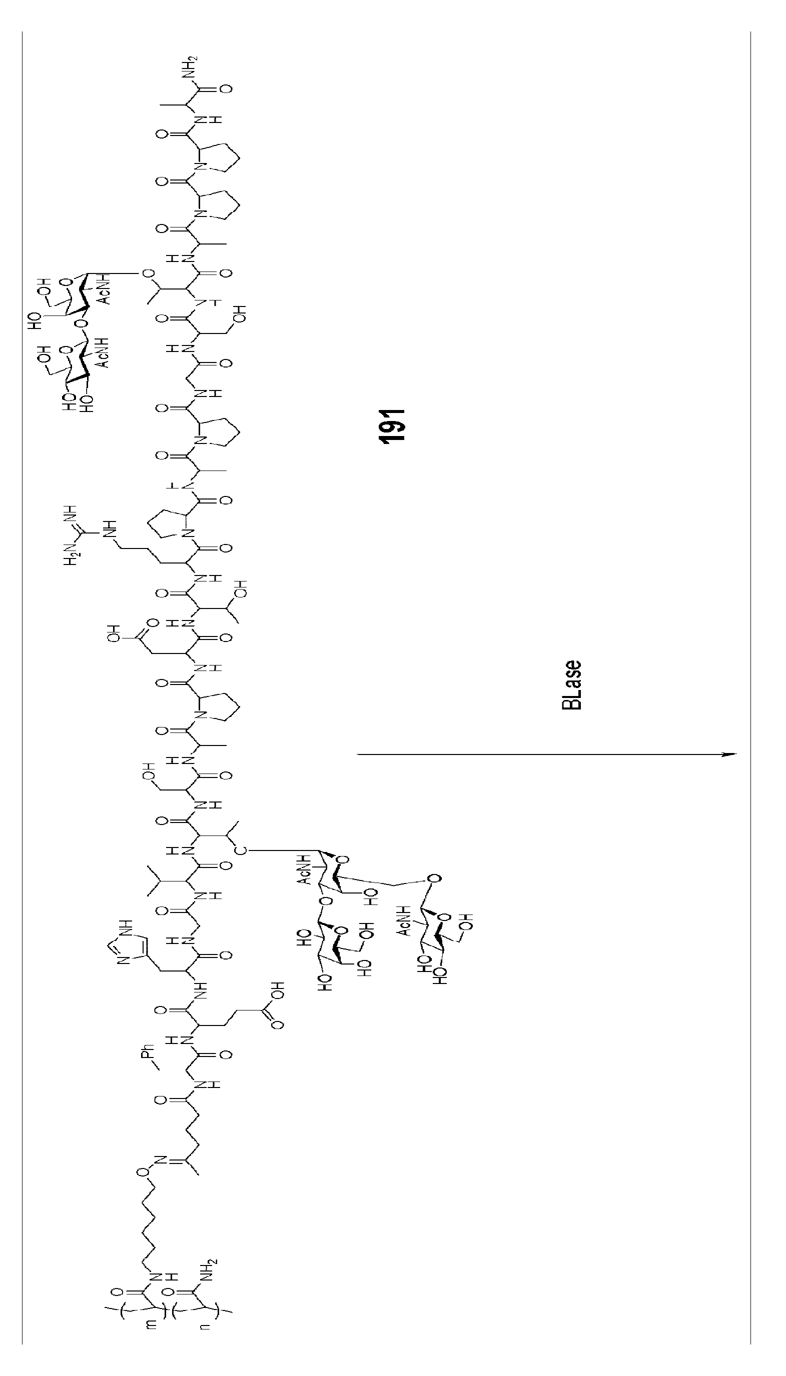

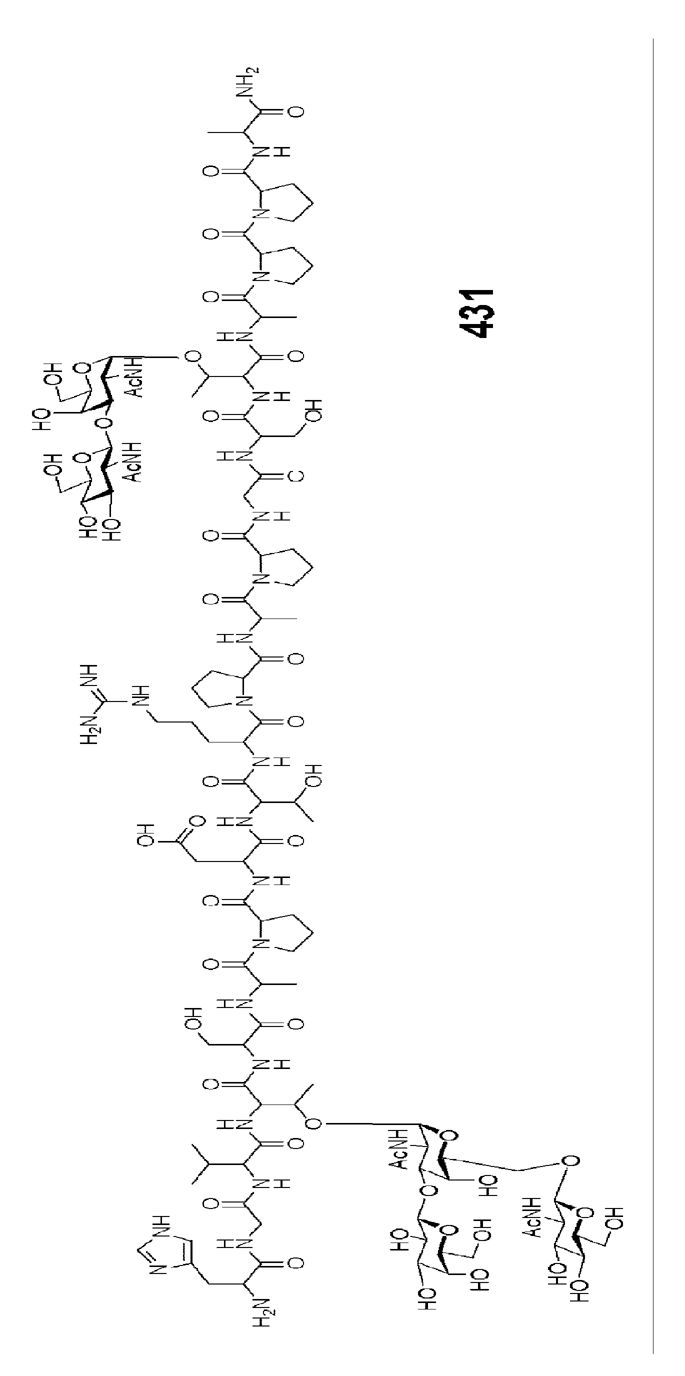

Using 71mg (0.02mmol) of Tentagel® S RAM resin (Hipep Laboratories, 0.28mmol/g) as a support, N-protected amino acids and keto acids as described below were sequentially condensed by Fmoc/HBTU/HOBt method, thereby synthesizing a glycopeptide derivative of interest: Fmoc-Ala-OH; Fmoc-Pro-OH; Fmoc-Pro-OH; Fmoc-Ala-OH; Fmoc-Thr(Ac5core3)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(tBu)-OH; Fmoc-Asp(OtBu)-OH; Fmoc-Pro-OH; Fmoc-Ala-OH; Fmoc-Ser(tBu)-OH; Fmoc-Thr(Ac7core2)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained resin equivalent to 5nmol was allowed to react in 90% TFA aqueous solution for two hours at room temperature to eliminate a protective group on a peptide residue and concurrently release compound (189) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 1.5ml of methanol. 1N sodium hydroxide aqueous solution was added to this solution, and was adjusted to have a pH of 12-12.5. The solution was then stirred for 1.5 hours at room temperature, thereby performing Ac deprotection reaction. After the reaction, 1N acetic acid was added and the solution was neutralized. Thereafter, the resin was separated by filtration. The solvent in the filtrate was removed, and the residue was dissolved in 0.5ml of 50mM acetic acid/sodium acetate buffer solution (pH 5.5). 0.5ml of 10mM (oxyamine residue calculation) water-soluble polymer (17) aqueous solution was added to the solution, and the solution was stirred for 18 hours at room temperature, thereby reacting compound (190) with compound (17). After completion of the reaction, the reaction solution was subjected to centrifugal concentration with an ultrafiltration filter 10K Apollo ® 20ml (Orbital Biosciences, available from LIC). 25mM HEPES buffer solution (pH 7.0) was added thereto and the solution was again subjected to concentration, thereby washing the polymer. By adding water so that the final amount of the solution was 0.5ml, 10mM (theoretical content of glycopeptide) polymer (191) was obtained. Identification of the polymer (191) was performed by treating a part of polymer (191) with BLase to obtain product (431). MALDI-TOF/MS:[M(average)+H]⁺ of compound (431)=2863.6 (theoretical value: [M(average)+H]+=2861.9).

(3.34 Synthesis of compounds (192) to (194) using a one-pot reaction)

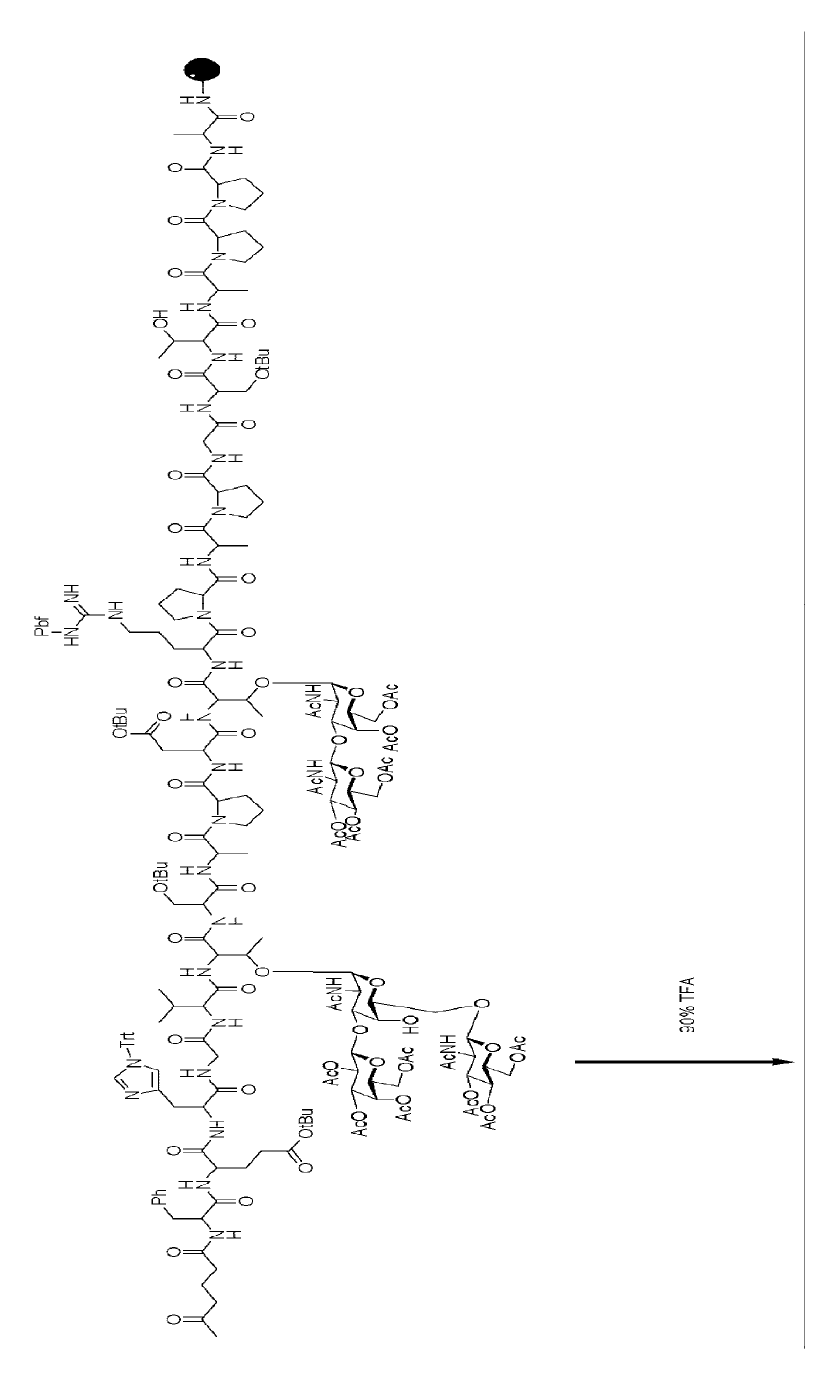

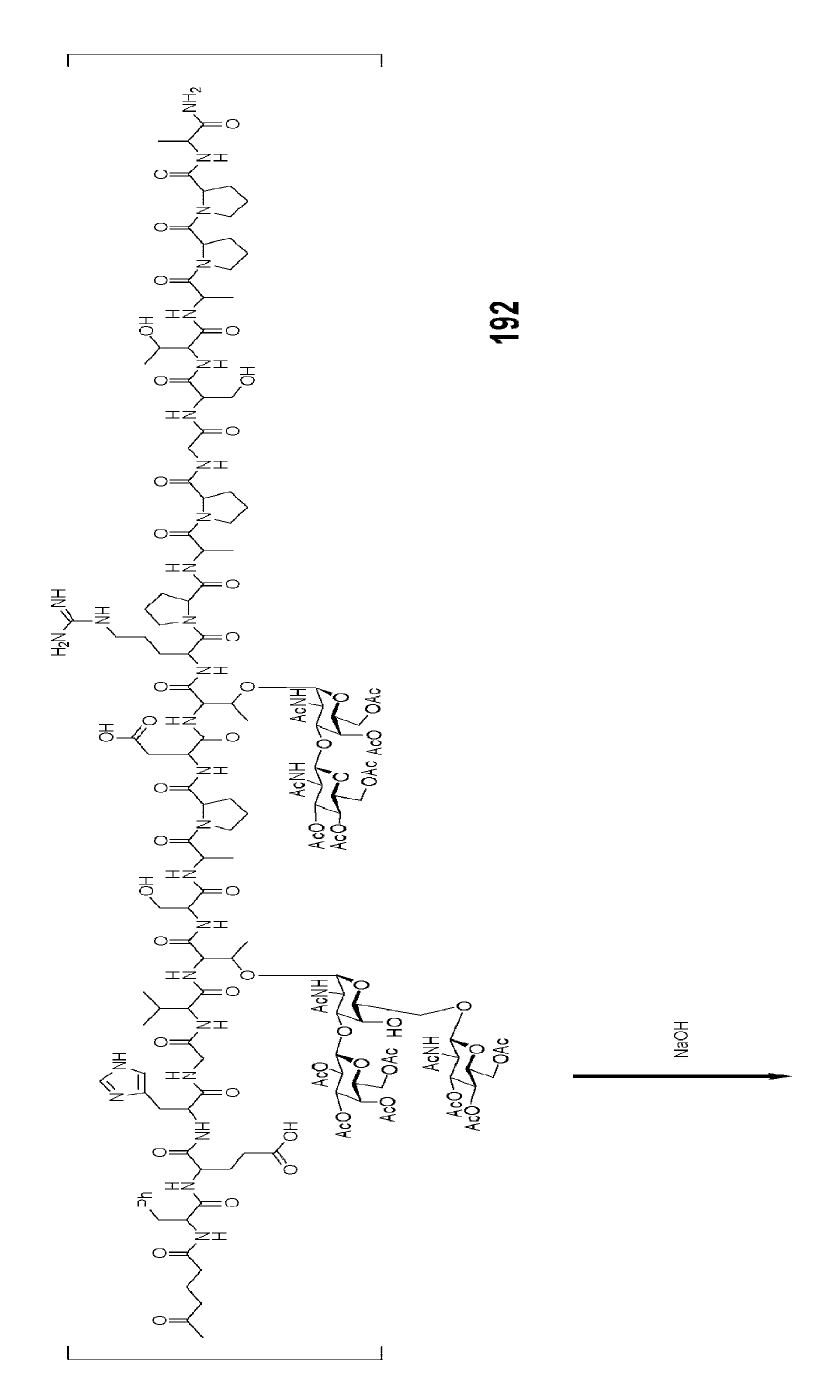

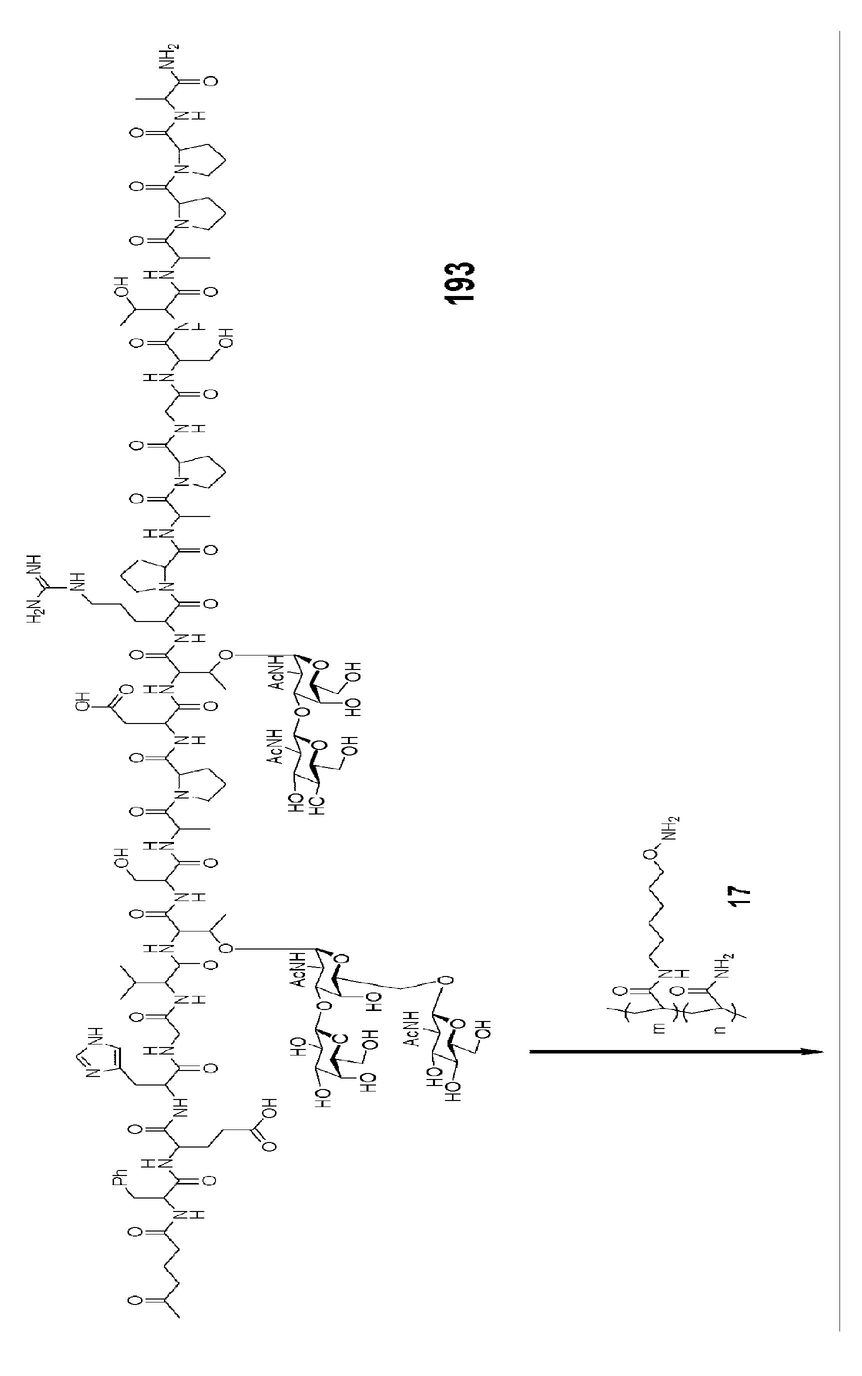

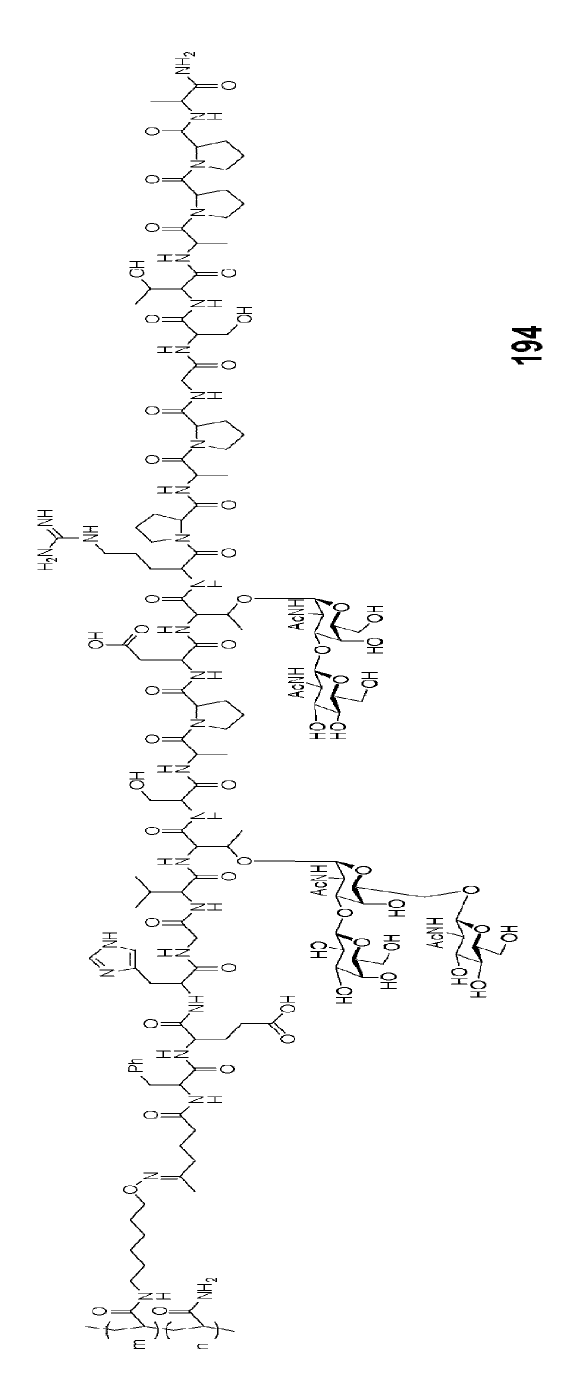

Using 71mg (0.02mmol) of Tentagel® S RAM resin (Hipep Laboratories, 0.28mmol/g) as a support, N-protected amino acids and keto acids as described below were sequentially condensed by Fmoc/HBTU/HOBt method, thereby synthesizing a glycopeptide derivative of interest: Fmoc-Ala-OH; Fmoc-Pro-OH; Fmoc-Pro-OH; Fmoc-Ala-OH; Fmoc-Thr(tBu)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(Ac5core3)-OH; Fmoc-Asp(OtBu)-OH; Fmoc-Pro-OH; Fmoc-Ala-OH; Fmoc-Ser(tBu)-OH; Fmoc-Thr(Ac7core2)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained resin equivalent to 5nmol was allowed to react in 90% TFA aqueous solution for two hours at room temperature to eliminate a protective group on a peptide residue and concurrently release compound (192) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether was again added, and the precipitate was washed. Centrifugal separation was again performed and the supernatant was removed. The obtained precipitant was dissolved in 1.5ml of methanol. 1N sodium hydroxide aqueous solution was added to this solution, and was adjusted to have a pH of 12-12.5. The solution was then stirred for 1.5 hours at room temperature, thereby performing Ac deprotection reaction. After the reaction, 1N acetic acid was added and the solution was neutralized. Thereafter, the resin was separated by filtration. The solvent in the filtrate was removed, and the residue was dissolved in 0.5ml of 50mM acetic acid/sodium acetate buffer solution (pH 5.5). 0.5ml of 10mM (oxyamine residue calculation) water-soluble polymer (17) aqueous solution was added to the solution, and the solution was stirred for 18 hours at room temperature, thereby reacting compound (193) with compound (17). After completion of the reaction, the reaction solution was subjected to centrifugal concentration with an ultrafiltration filter 10K Apollo ® 20ml (Orbital Biosciences, available from LIC). 25mM HEPES buffer solution (pH 7.0) was added thereto and the solution was again subjected to concentration, thereby washing the polymer. By adding water so that the final amount of the solution was 0.5ml, 10mM (theoretical content of glycopeptide) polymer (194) was obtained. Identification of the polymer (194) was performed based on that product (287) was obtained in the following section (3.56).

(3.35 Synthesis of compounds (195) to (197) using a one-pot reaction)

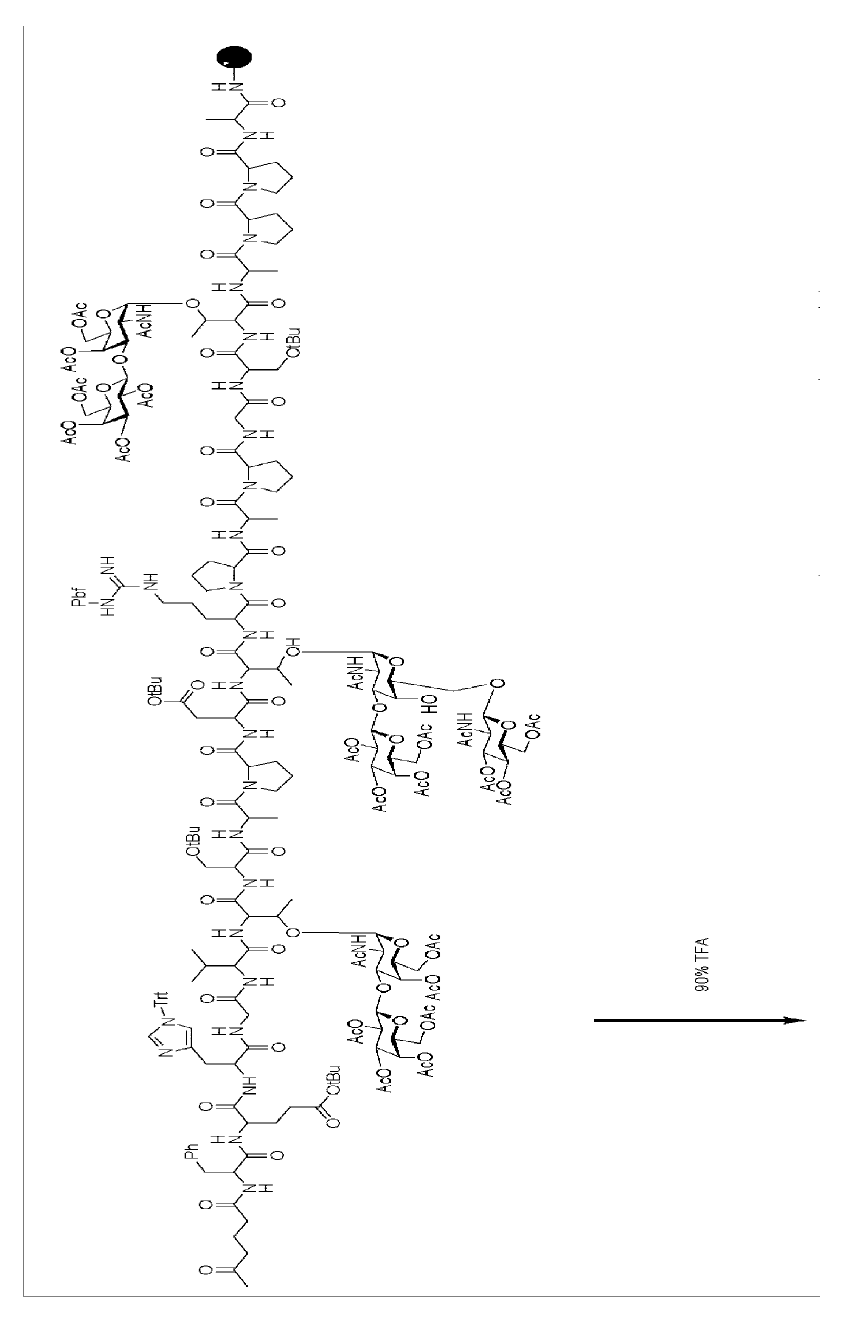

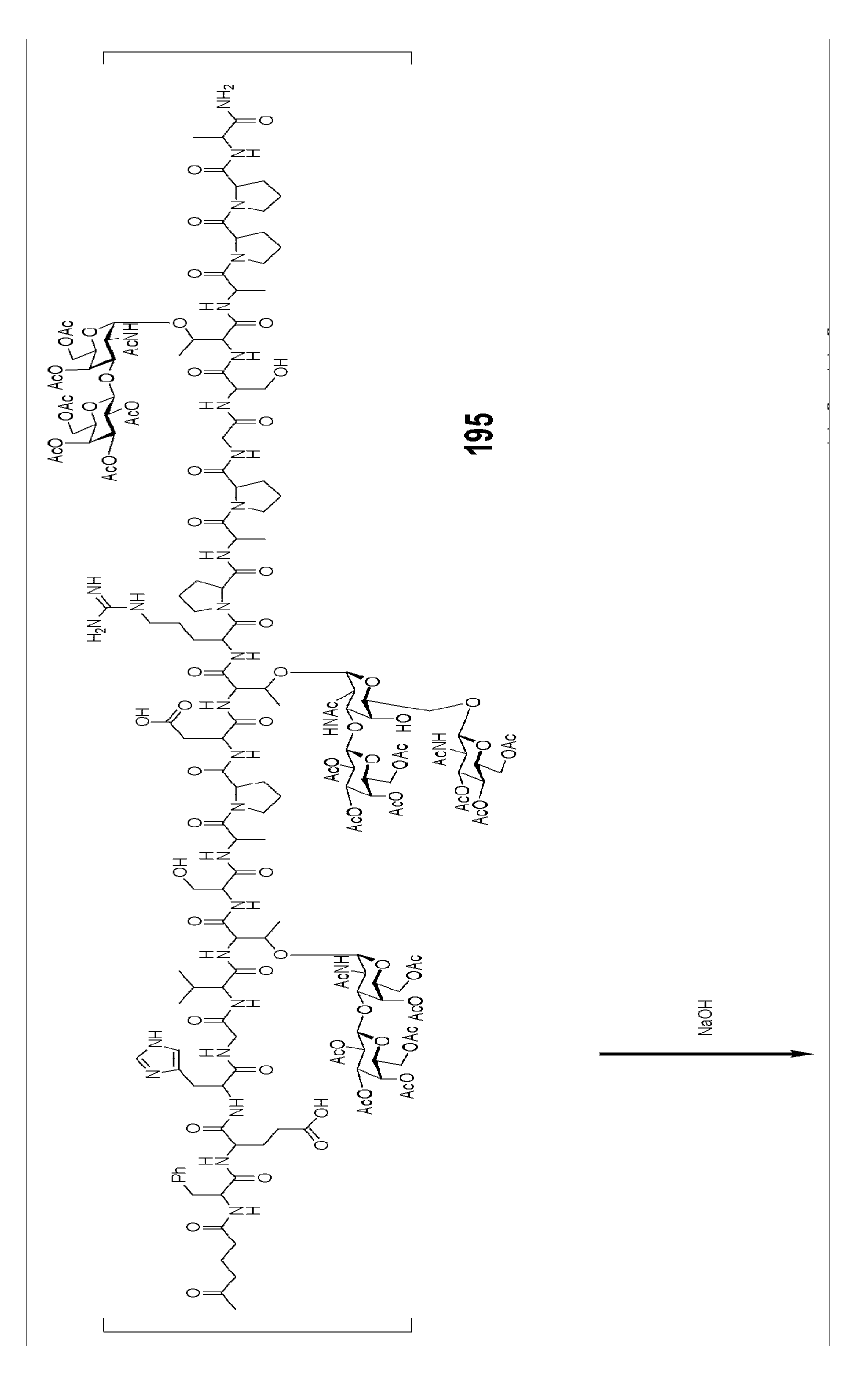

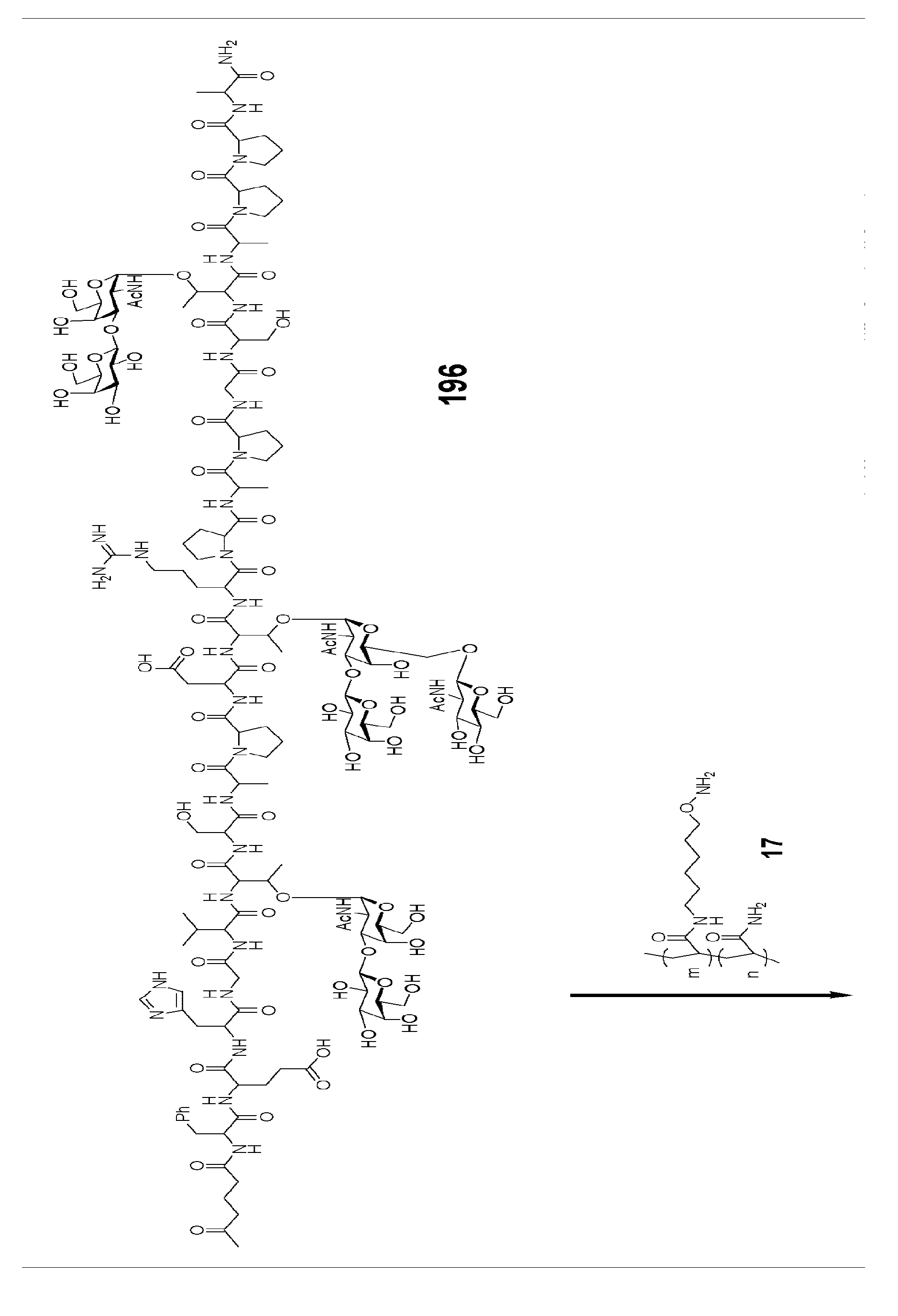

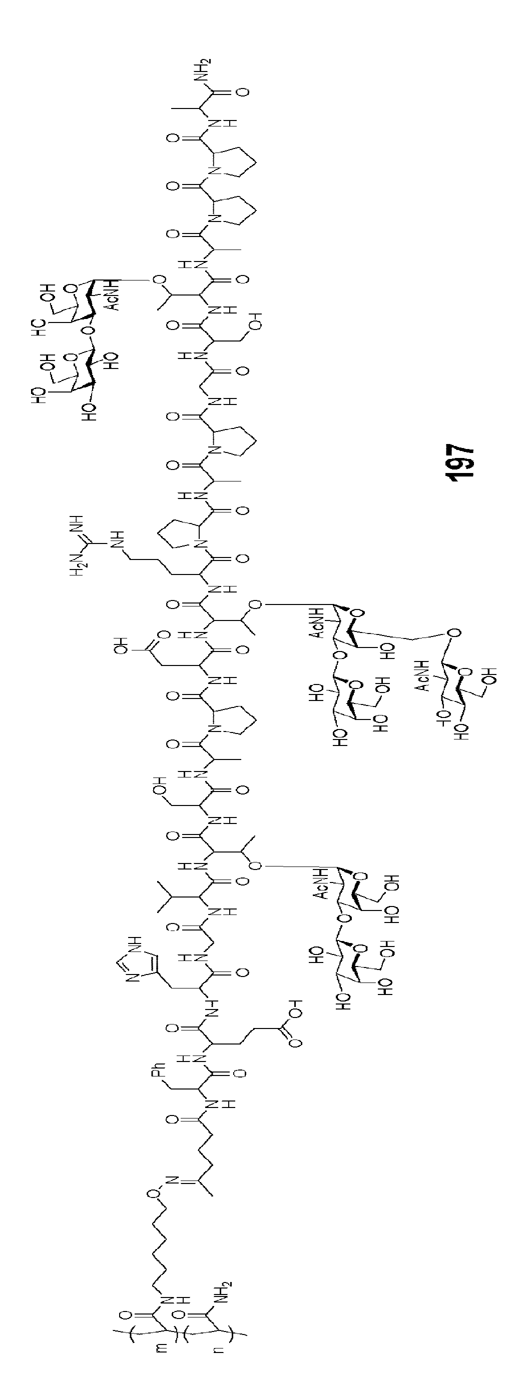

Using 71mg (0.02mmol) of Tentagel® S RAM resin (Hipep Laboratories, 0.28mmol/g) as a support, N-protected amino acids and keto acids as described below were sequentially condensed by Fmoc/HBTU/HOBt method, thereby synthesizing a glycopeptide derivative of interest: Fmoc-Ala-OH; Fmoc-Pro-OH; Fmoc-Pro-OH; Fmoc-Ala-OH; Fmoc-Thr(Ac6core1)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(Ac7core2)-OH; Fmoc-Asp(OtBu)-OH; Fmoc-Pro-OH; Fmoc-Ala-OH; Fmoc-Ser(tBu)-OH; Fmoc-Thr(Ac6core1)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained resin equivalent to 5nmol was allowed to react in 90% TFA aqueous solution for two hours at room temperature to eliminate a protective group on a peptide residue and concurrently release compound (195) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 1.5ml of methanol. 1N sodium hydroxide aqueous solution was added to this solution, and was adjusted to have a pH of 12-12.5. The solution was then stirred for 1.5 hours at room temperature, thereby performing Ac deprotection reaction. After the reaction, 1N acetic acid was added and the solution was neutralized. Thereafter, the resin was separated by filtration. The solvent in the filtrate was removed, and the residue was dissolved in 0.5ml of 50mM acetic acid/sodium acetate buffer solution (pH 5.5). 0.5ml of 10mM (oxyamine residue calculation) water-soluble polymer (17) aqueous solution was added to the solution, and the solution was stirred for 18 hours at room temperature, thereby reacting compound (196) with compound (17) . After completion of the reaction, the reaction solution was subjected to centrifugal concentration with an ultrafiltration filter 10K Apollo ® 20ml (Orbital Biosciences, available from LIC). 25mM HEPES buffer solution (pH 7.0) was added thereto and the solution was again subjected to concentration, thereby washing the polymer. By adding water so that the final amount of the solution was 0.5ml, 10mM (theoretical content of glycopeptide) polymer (197) was obtained. Identification of the polymer (197) was performed based on that product (298) was obtained in the following section (3.57).

(3.36 Synthesis of compounds (198) to (200) using a one-pot reaction)

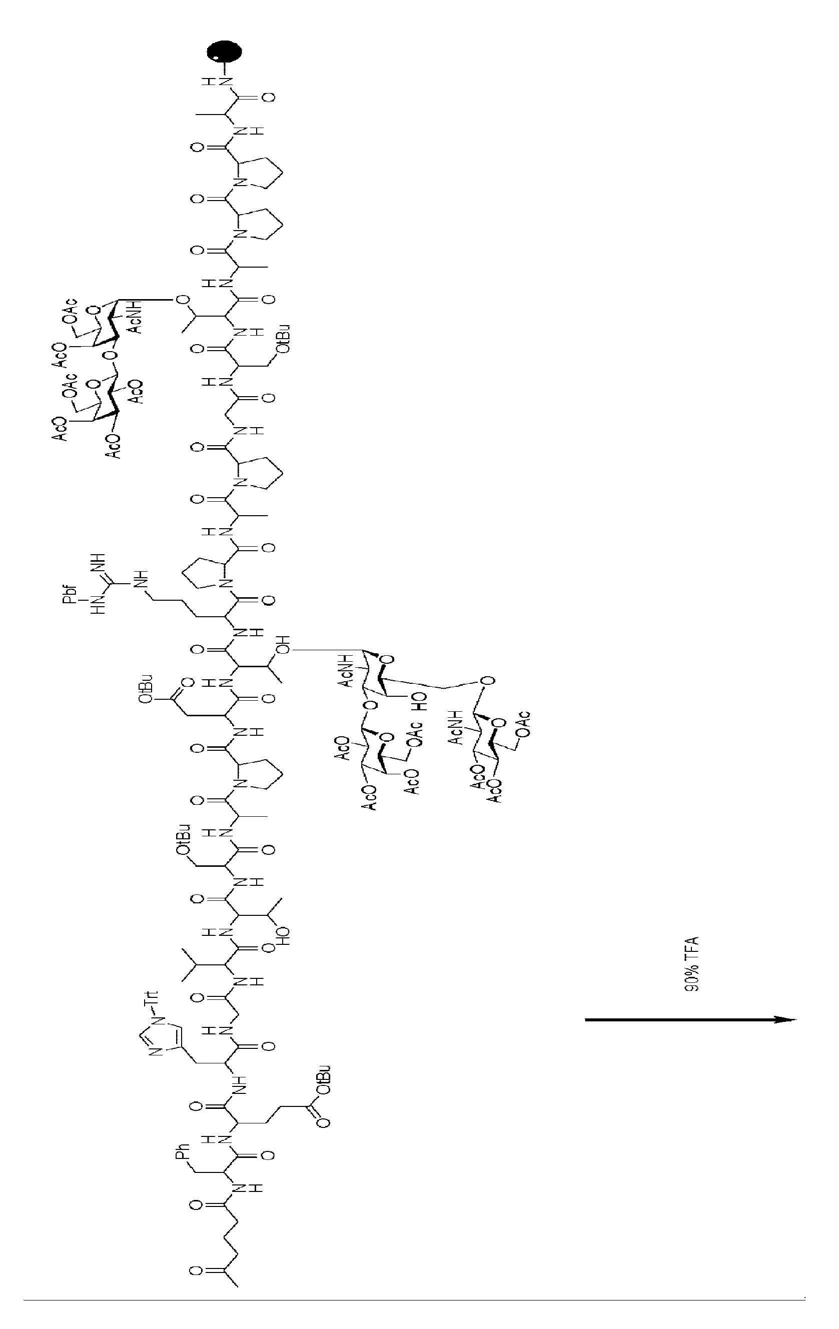

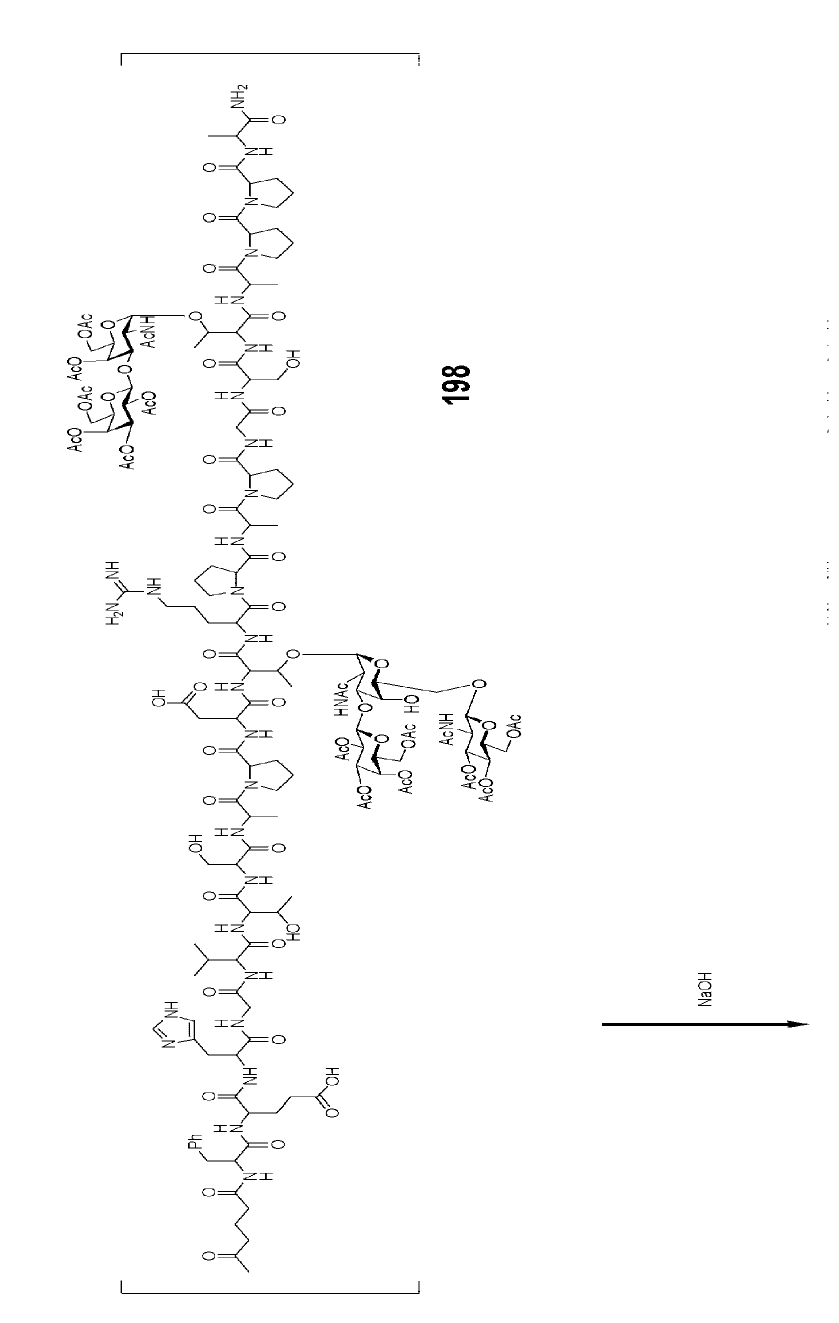

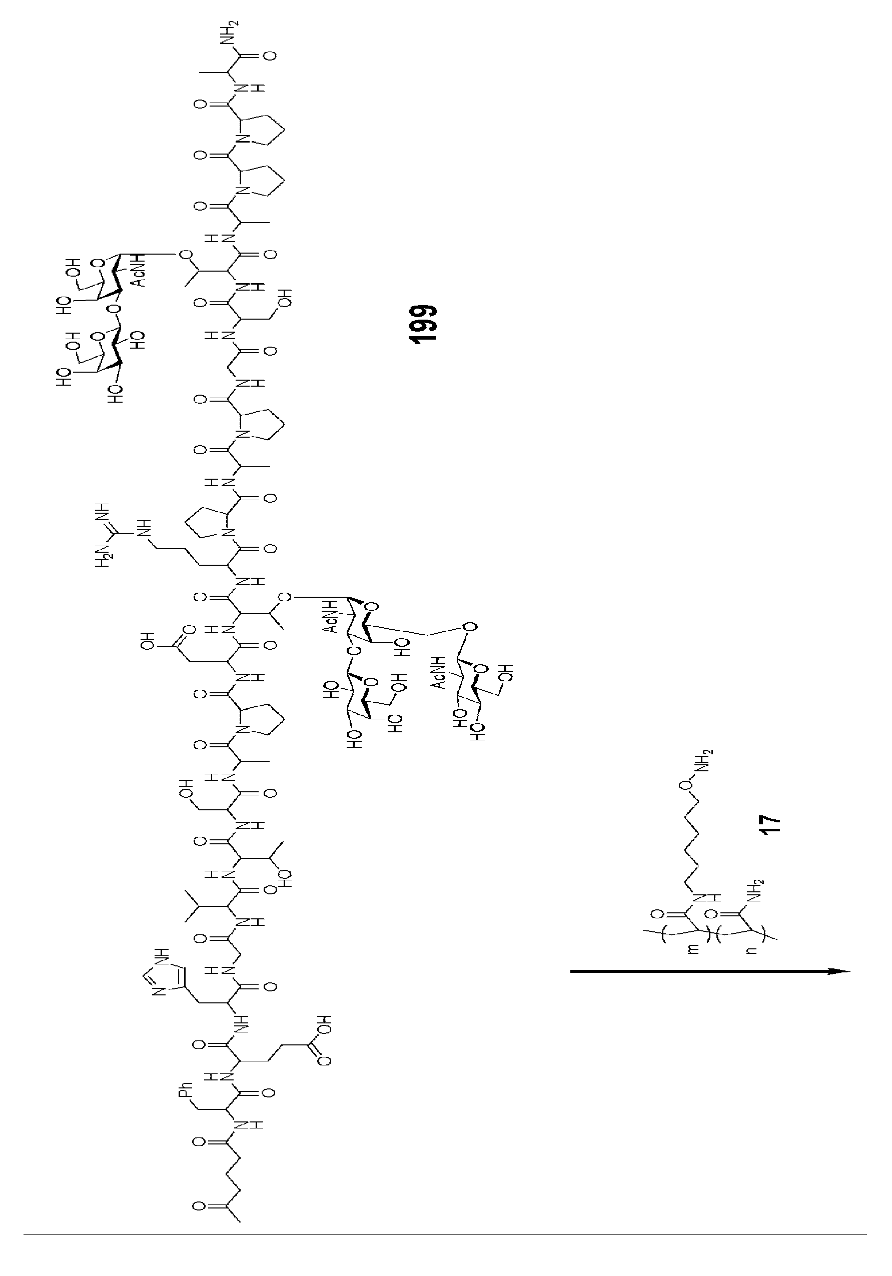

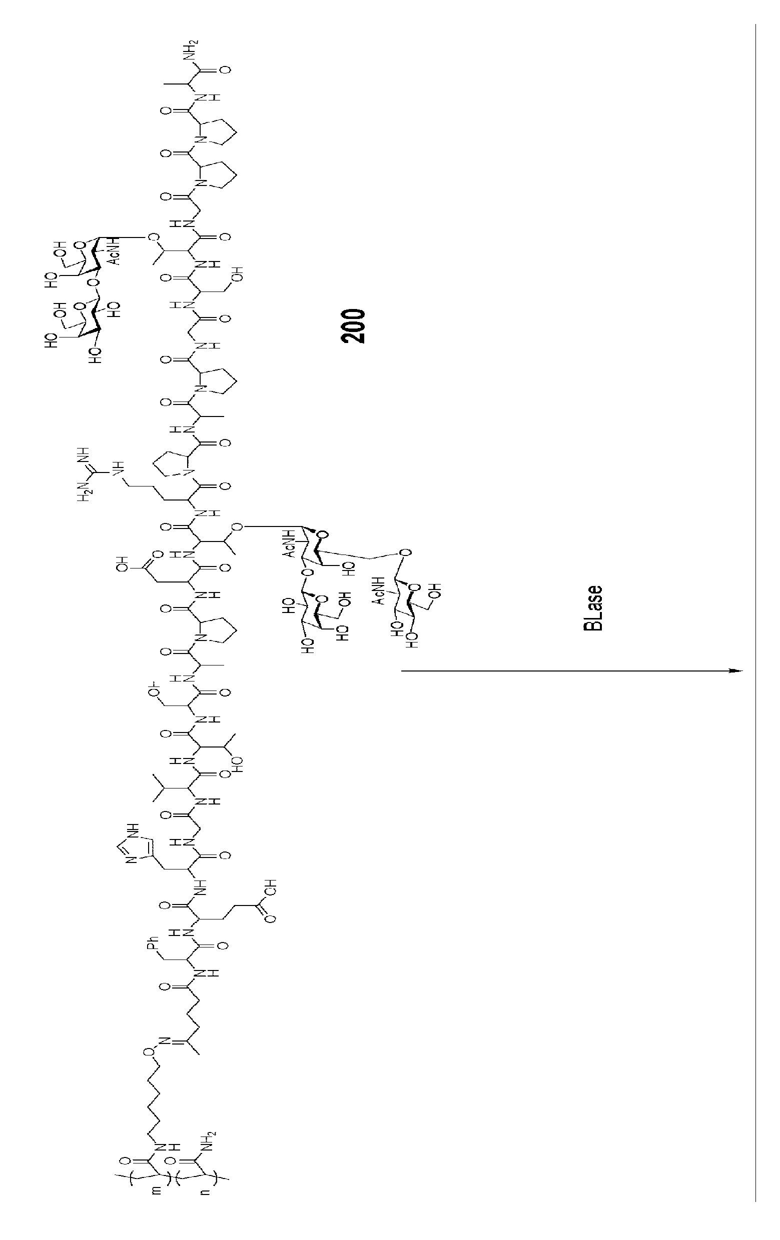

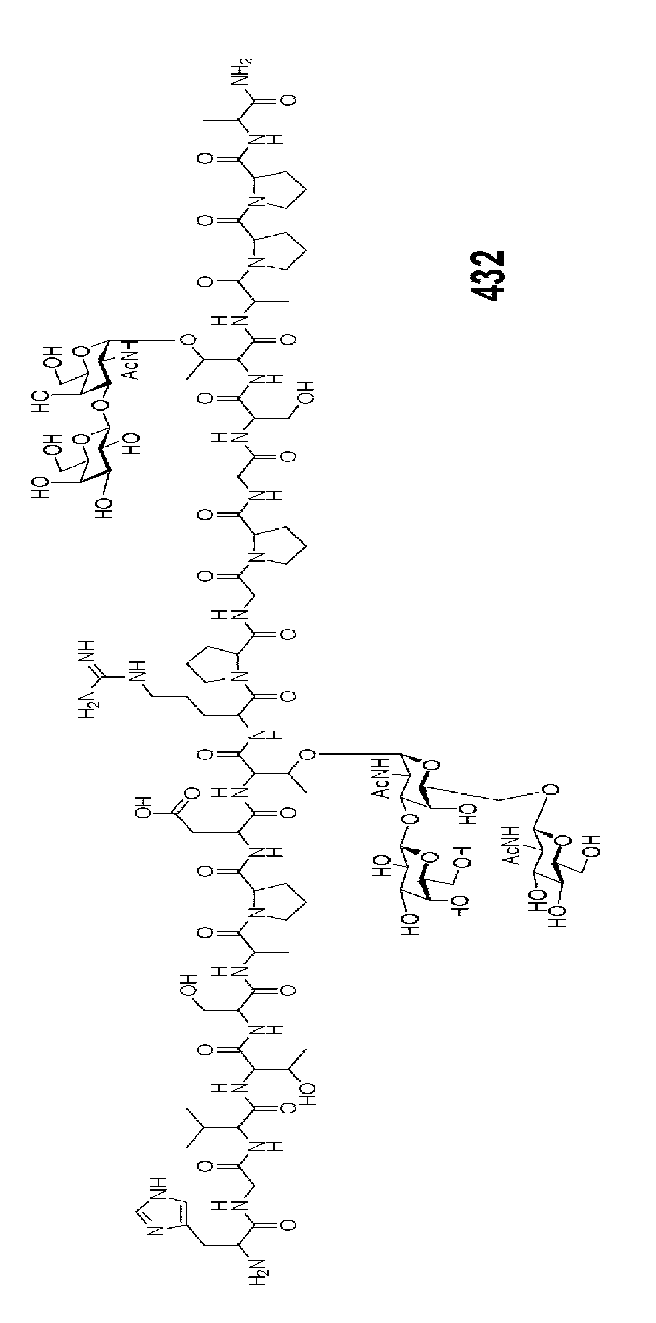

Using 71mg (0.02mmol) of Tentagel® S RAM resin (Hipep Laboratories, 0.28mmol/g) as a support, N-protected amino acids and keto acids as described below were sequentially condensed by Fmoc/HBTU/HOBt method, thereby synthesizing a glycopeptide derivative of interest: Fmoc-Ala-OH; Fmoc-Pro-OH; Fmoc-Pro-OH; Fmoc-Ala-OH; Fmoc-Thr(Ac6core1)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(Ac7core2)-OH; Fmoc-Asp(OtBu)-OH; Fmoc-Pro-OH; Fmoc-Ala-OH; Fmoc-Ser(tBu)-OH; Fmoc-Thr(tBu)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained resin equivalent to 5nmol was allowed to react in 90% TFA aqueous solution for two hours at room temperature to eliminate a protective group on a peptide residue and concurrently release compound (198) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 1.5ml of methanol. 1N sodium hydroxide aqueous solution was added to this solution, and was adjusted to have a pH of 12-12.5. The solution was then stirred for 1.5 hours at room temperature, thereby performing Ac deprotection reaction. After the reaction, 1N acetic acid was added and the solution was neutralized. Thereafter, the resin was separated by filtration. The solvent in the filtrate was removed, and the residue was dissolved in 0.5ml of 50mM acetic acid/sodium acetate buffer solution (pH 5.5). 0.5ml of 10mM (oxyamine residue calculation) water-soluble polymer (17) aqueous solution was added to the solution, and the solution was stirred for 18 hours at room temperature, thereby reacting compound (199) with compound (17). After completion of the reaction, the reaction solution was subjected to centrifugal concentration with an ultrafiltration filter 10K Apollo ® 20ml (Orbital Biosciences, available from LIC). 25mM HEPES buffer solution (pH 7.0) was added thereto and the solution was again subjected to concentration, thereby washing the polymers. By adding water so that the final amount of the solution was 0.5ml, 10mM (theoretical content of glycopeptide) polymer (200) was obtained. Identification of the polymer (200) was performed by treating a part of polymer (200) with BLase to obtain product (432). MALDI-TOF/MS:[M(average)+H]⁺ of compound (432)=2822.4 (theoretical value: [M(average)+H]⁺=2820.9).

(3.37 Synthesis of compounds (201) to (203) using a one-pot reaction)

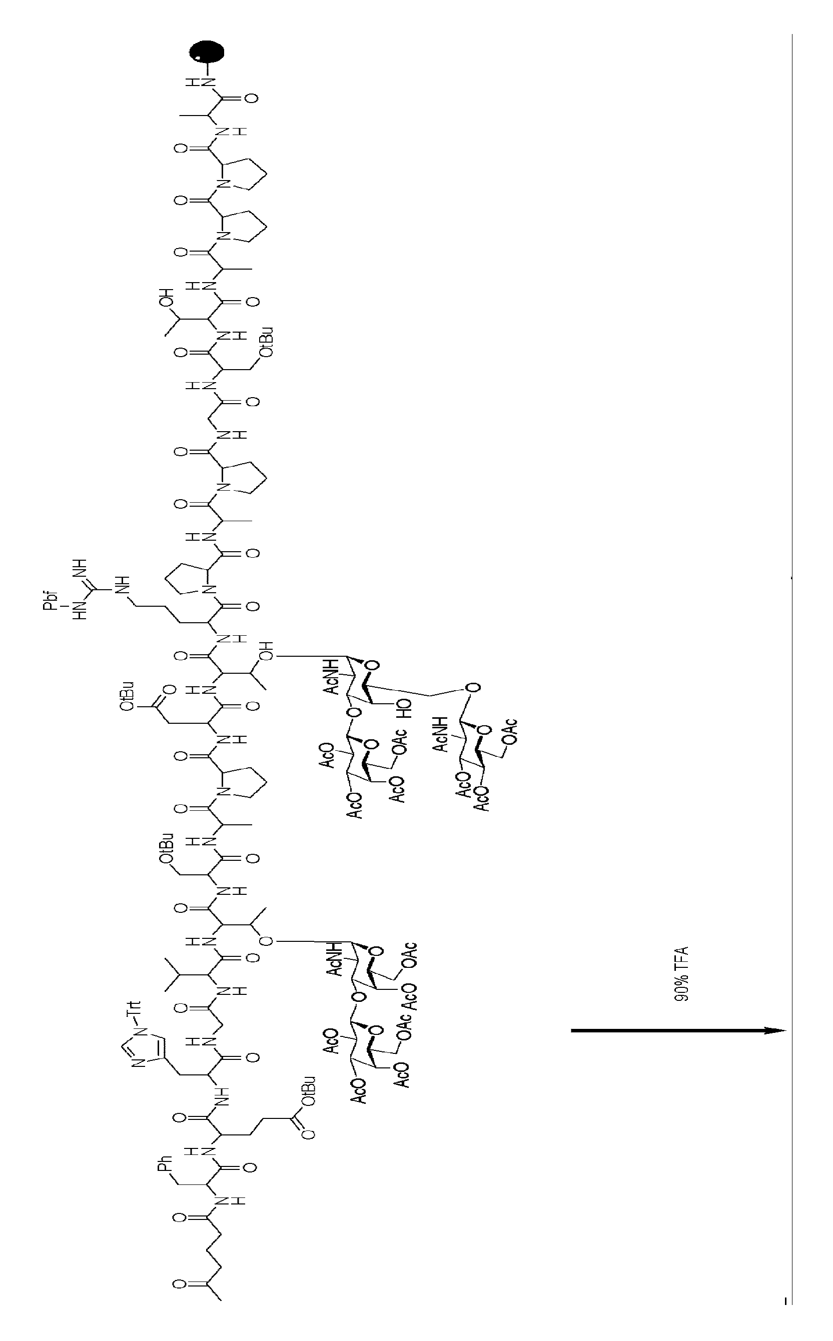

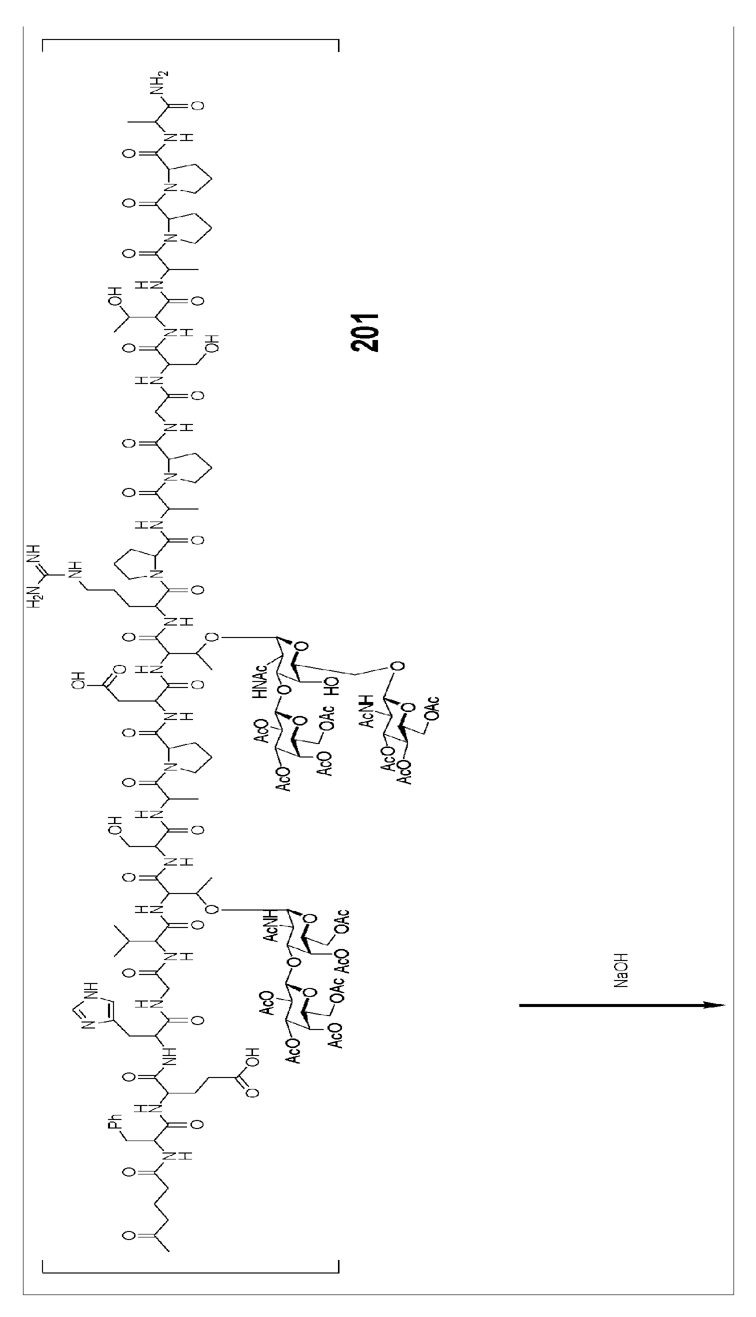

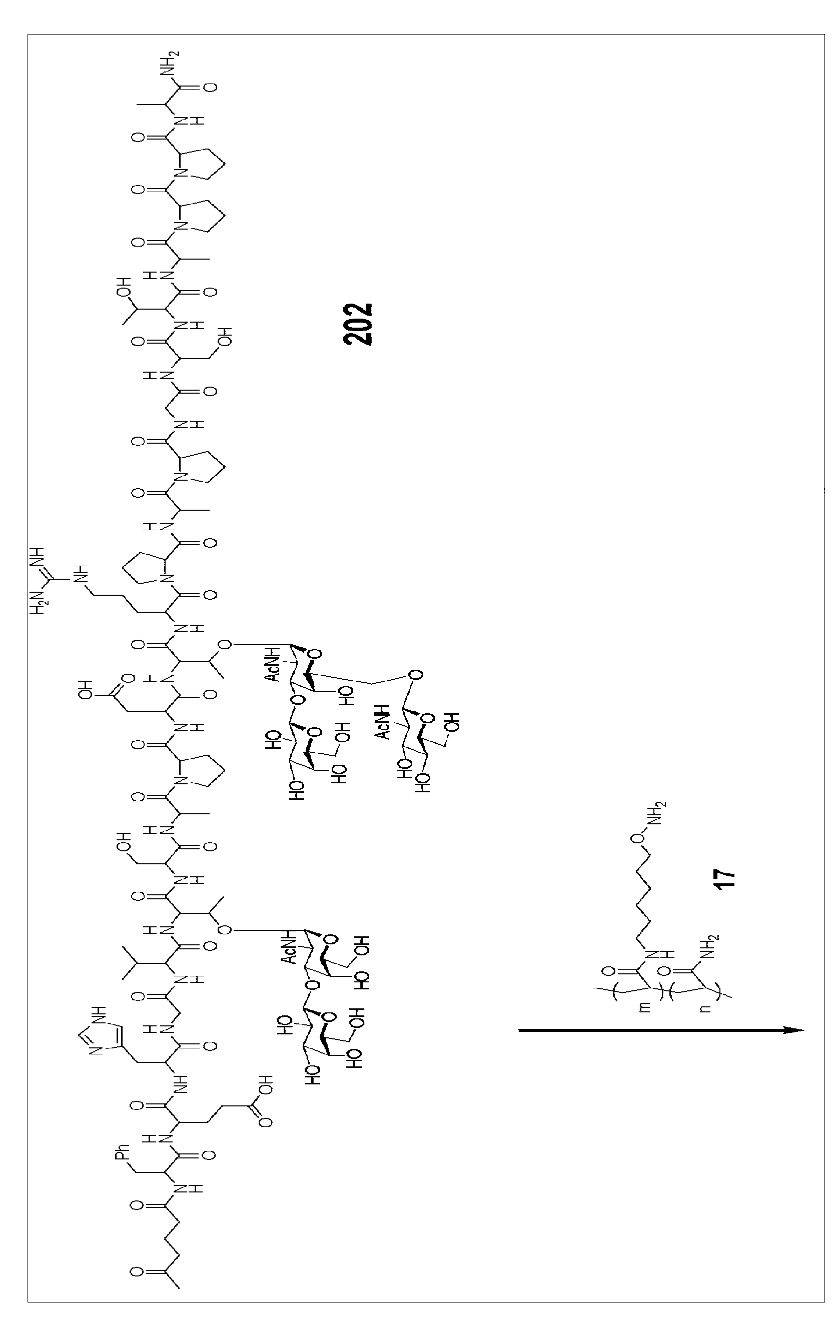

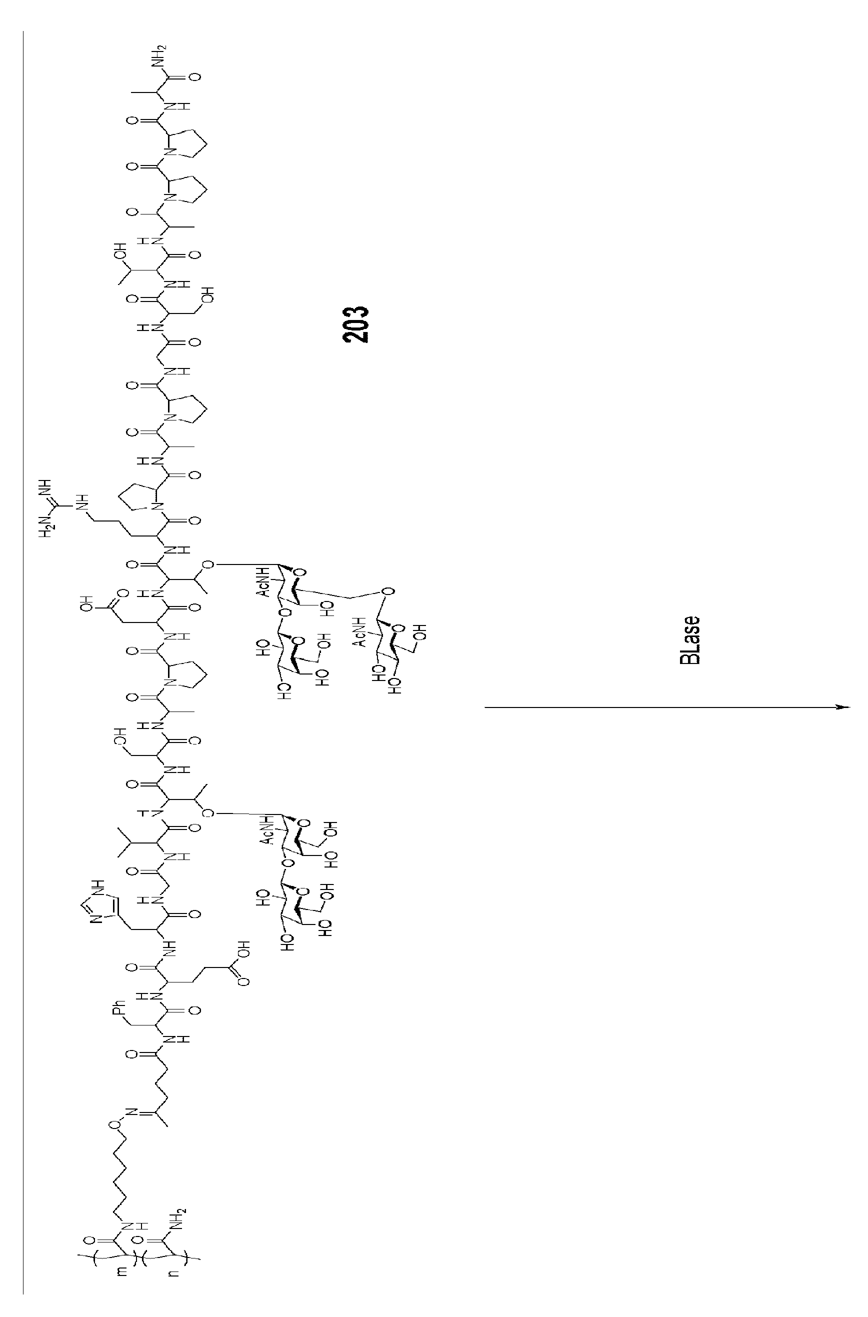

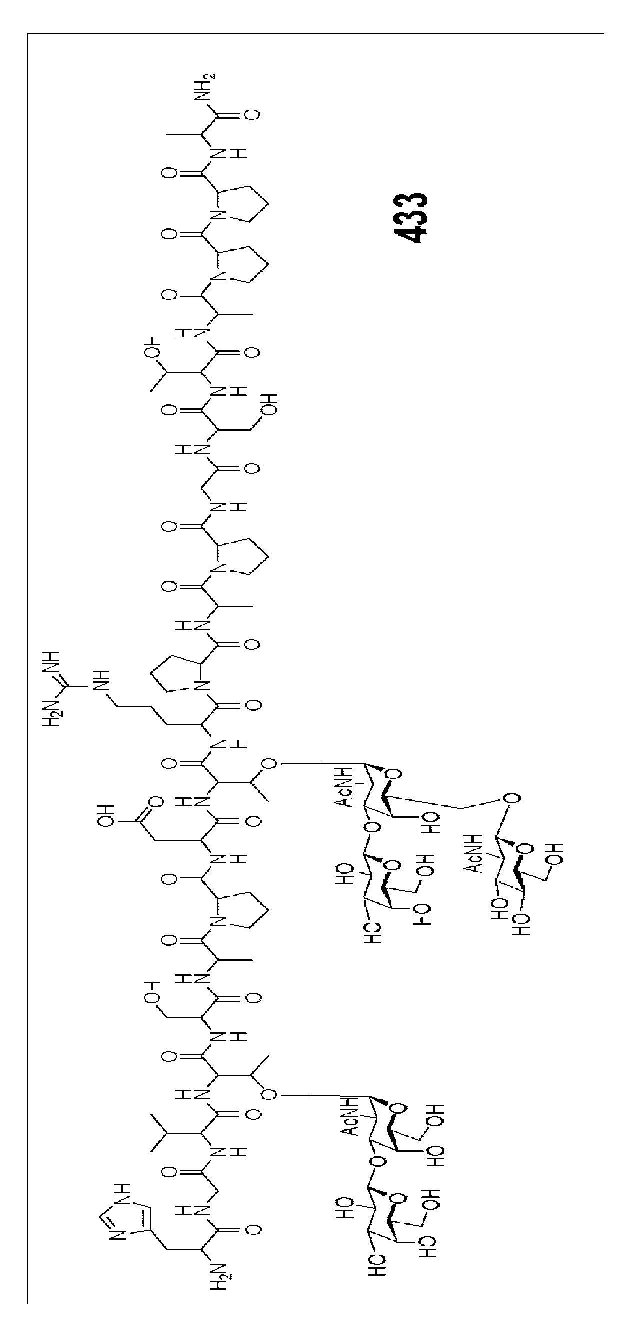

Using 71mg (0.02mmol) of Tentagel® S RAM resin (Hipep Laboratories, 0.28mmol/g) as a support, N-protected amino acids and keto acids as described below were sequentially condensed by Fmoc/HBTU/HOBt method, thereby synthesizing a glycopeptide derivative of interest: Fmoc-Ala-OH; Fmoc-Pro-OH; Fmoc-Pro-OH; Fmoc-Ala-OH; Fmoc-Thr(Ac5core3)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(Ac7core2)-OH; Fmoc-Asp(OtBu)-OH; Fmoc-Pro-OH; Fmoc-Ala-OH; Fmoc-Ser(tBu)-OH; Fmoc-Thr(Ac5core3)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained resin equivalent to 5nmol was allowed to react in 90% TFA aqueous solution for two hours at room temperature to eliminate a protective group on a peptide residue and concurrently release compound (204) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 1.5ml of methanol. 1N sodium hydroxide aqueous solution was added to this solution, and was adjusted to have a pH of 12-12.5. The solution was then stirred for 1.5 hours at room temperature, thereby performing Ac deprotection reaction. After the reaction, 1N acetic acid was added and the solution was neutralized. Thereafter, the resin was separated by filtration. The solvent in the filtrate was removed, and the residue was dissolved in 0.5ml of 50mM acetic acid/sodium acetate buffer solution (pH 5.5). 0.5ml of 10mM (oxyamine residue calculation) water-soluble polymer (17) aqueous solution was added to the solution, and the solution was stirred for 18 hours at room temperature, thereby reacting compound (202) with compound (17) . After completion of the reaction, the reaction solution was subjected to centrifugal concentration with an ultrafiltration filter 10K Apollo ® 20ml (Orbital Biosciences, available from LIC). 25mM HEPES buffer solution (pH 7.0) was added thereto and the solution was again subjected to concentration, thereby washing the polymers. By adding water so that the final amount of the solution was 0.5ml, 10mM (theoretical content of glycopeptide) polymer (203) was obtained. Identification of the polymer (203) was performed by treating a part of polymer (203) with BLase to obtain product (433). MALDI-TOF/MS:[M(average)+H]⁺of compound (433)=2822.3 (theoretical value: [M(average)+H]+=2820.9).

(3.38 Synthesis of compounds (204) to (206) using a one-pot reaction)

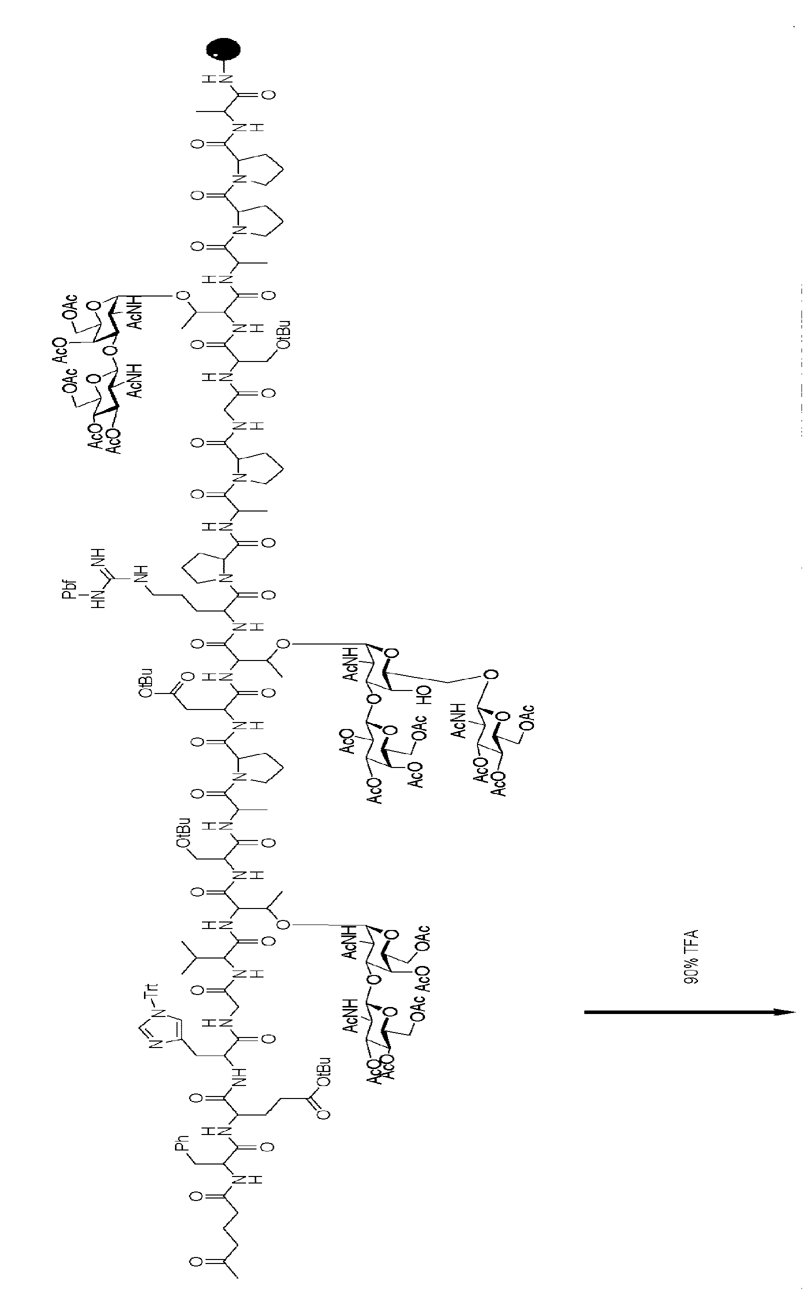

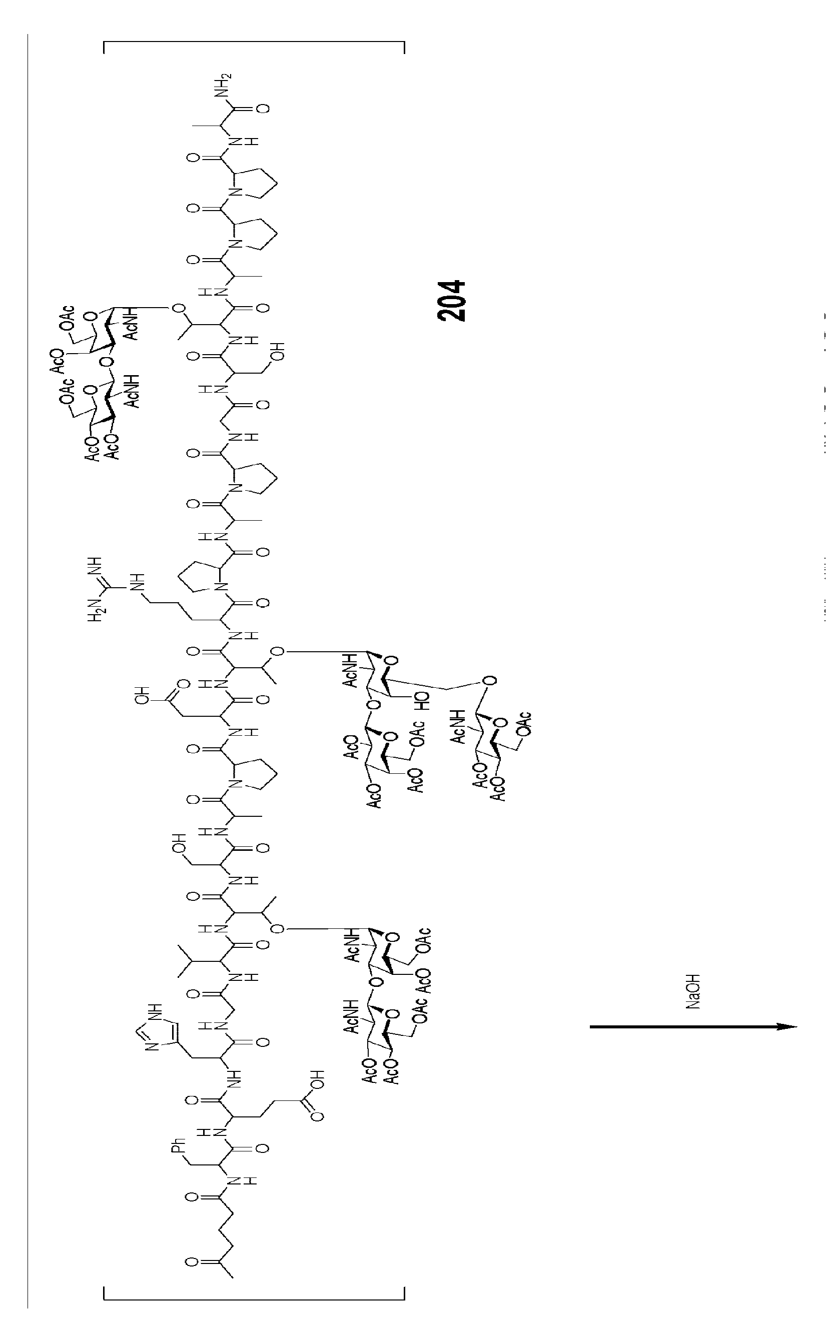

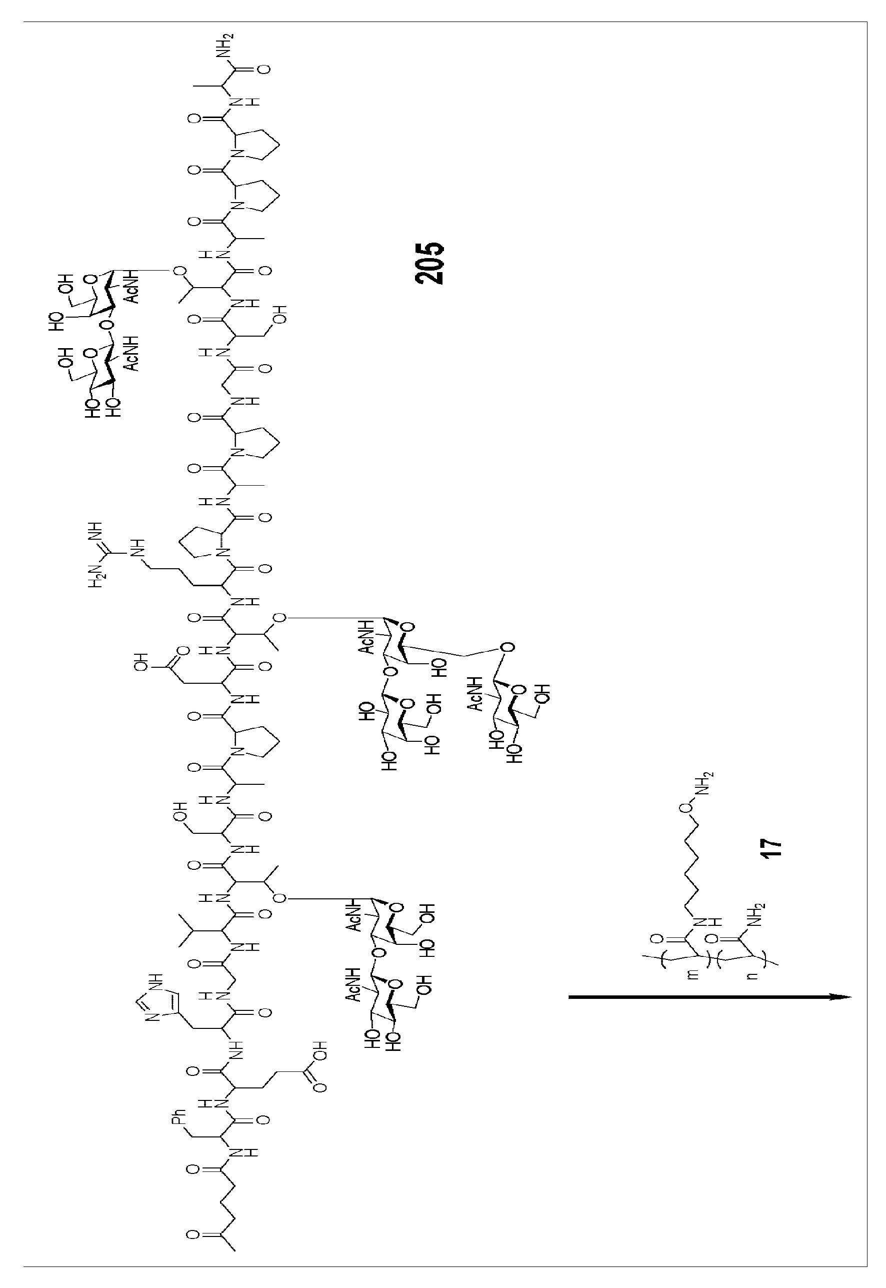

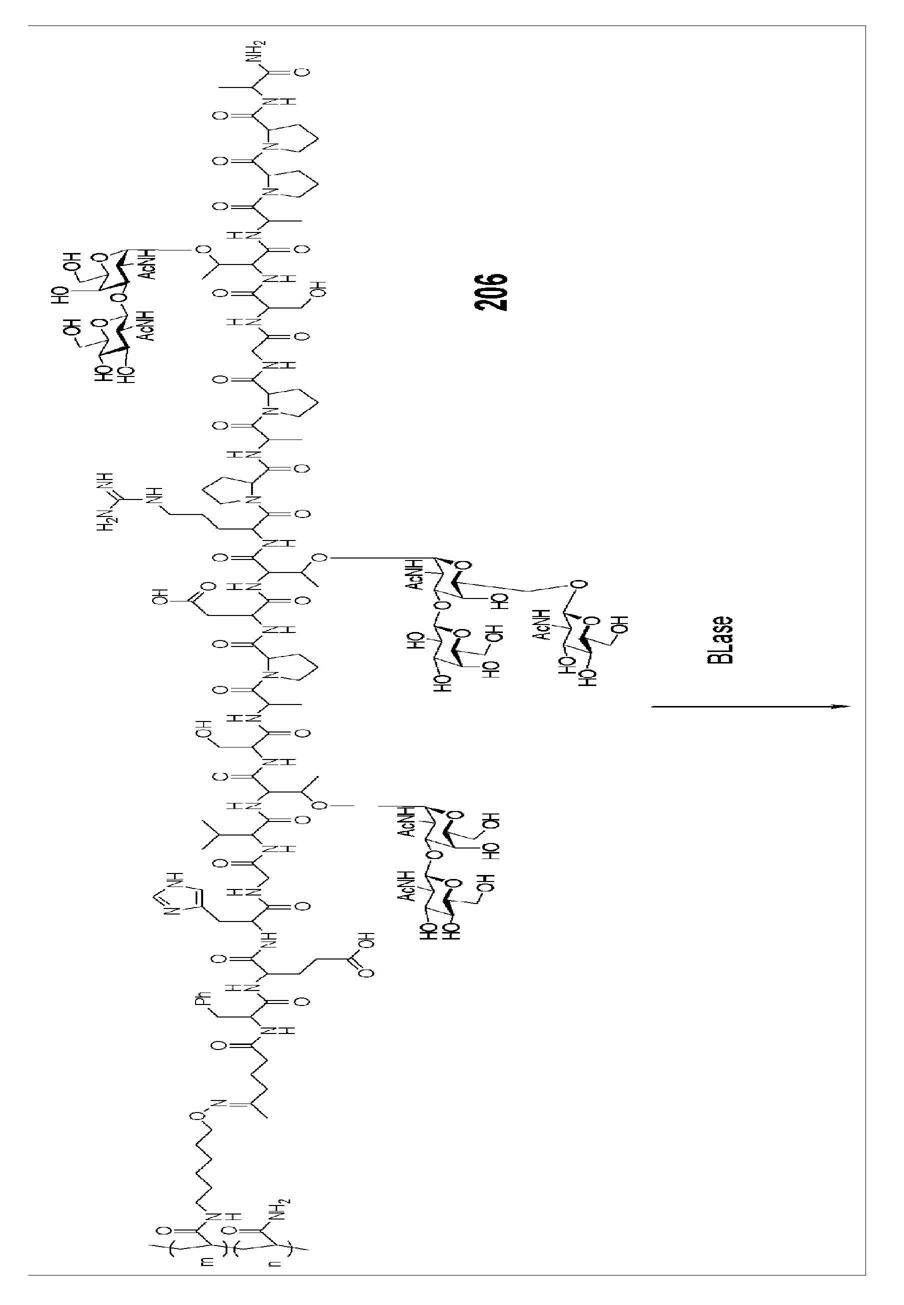

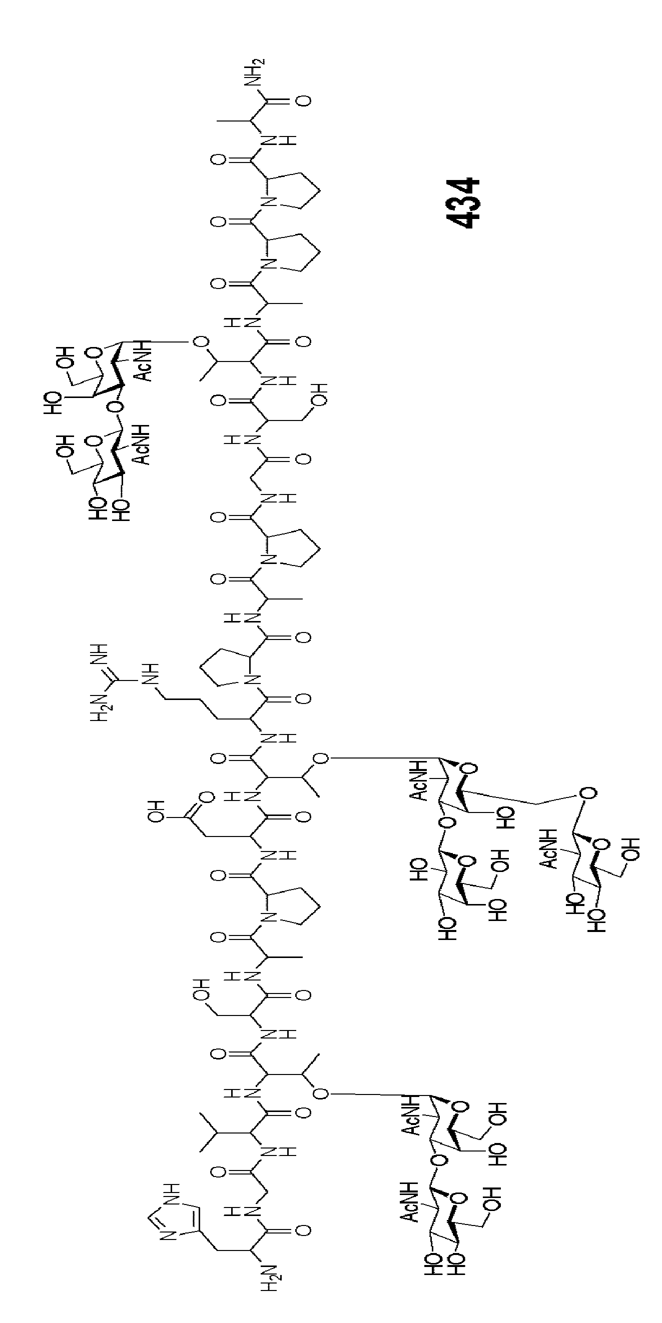

Using 71mg (0.02mmol of Tentagel® S RAM resin (Hipep Laboratories, 0.28mmol/g) as a support, N-protected amino acids and keto acids as described below were sequentially condensed by Fmoc/HBTU/HOBt method, thereby synthesizing a glycopeptide derivative of interest: Fmoc-Ala-OH; Fmoc-Pro-OH; Fmoc-Pro-OH; Fmoc-Ala-OH; Fmoc-Thr(Ac5core3)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(Ac7core2)-OH; Fmoc-Asp(OtBu)-OH; Fmoc-Pro-OH; Fmoc-Ala-OH; Fmoc-Ser(tBu)-OH; Fmoc-Thr(Ac5core3)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained resin equivalent to 5nmol was allowed to react in 90% TFA aqueous solution for two hours at room temperature to eliminate a protective group on a peptide residue and concurrently release compound (204) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 1.5ml of methanol. 1N sodium hydroxide aqueous solution was added to this solution, and was adjusted to have a pH of 12-12.5. The solution was then stirred for 1.5 hours at room temperature, thereby performing Ac deprotection reaction. After the reaction, 1N acetic acid was added and the solution was neutralized. Thereafter, the resin was separated by filtration. The solvent in the filtrate was removed, and the residue was dissolved in 0.5ml of 50mM acetic acid/sodium acetate buffer solution (pH 5.5). 0.5ml of 10mM (oxyamine residue calculation) water-soluble polymer (17) aqueous solution was added to the solution, and the solution was stirred for 18 hours at room temperature, thereby reacting compound (205) with compound (17). After completion of the reaction, the reaction solution was subjected to centrifugal concentration with an ultrafiltration filter 10K Apollo ® 20ml (Orbital Biosciences, available from LIC). 25mM HEPES buffer solution (pH 7.0) was added thereto and the solution was again subjected to concentration, thereby washing the polymers. By adding water so that the final amount of the solution was 0.5ml, 10mM (theoretical content of glycopeptide) polymer (206) was obtained. Identification of the polymer (206) was performed by treating a part of polymer (206) with BLase to obtain product (434). MALDI-TOF/MS:[M(average)+H]⁺ of compound (434)=3269.9 (theoretical value: [M(average)+H]+=3268.3).

(3.39 Synthesis of compounds (207) to (209) using a one-pot reaction)

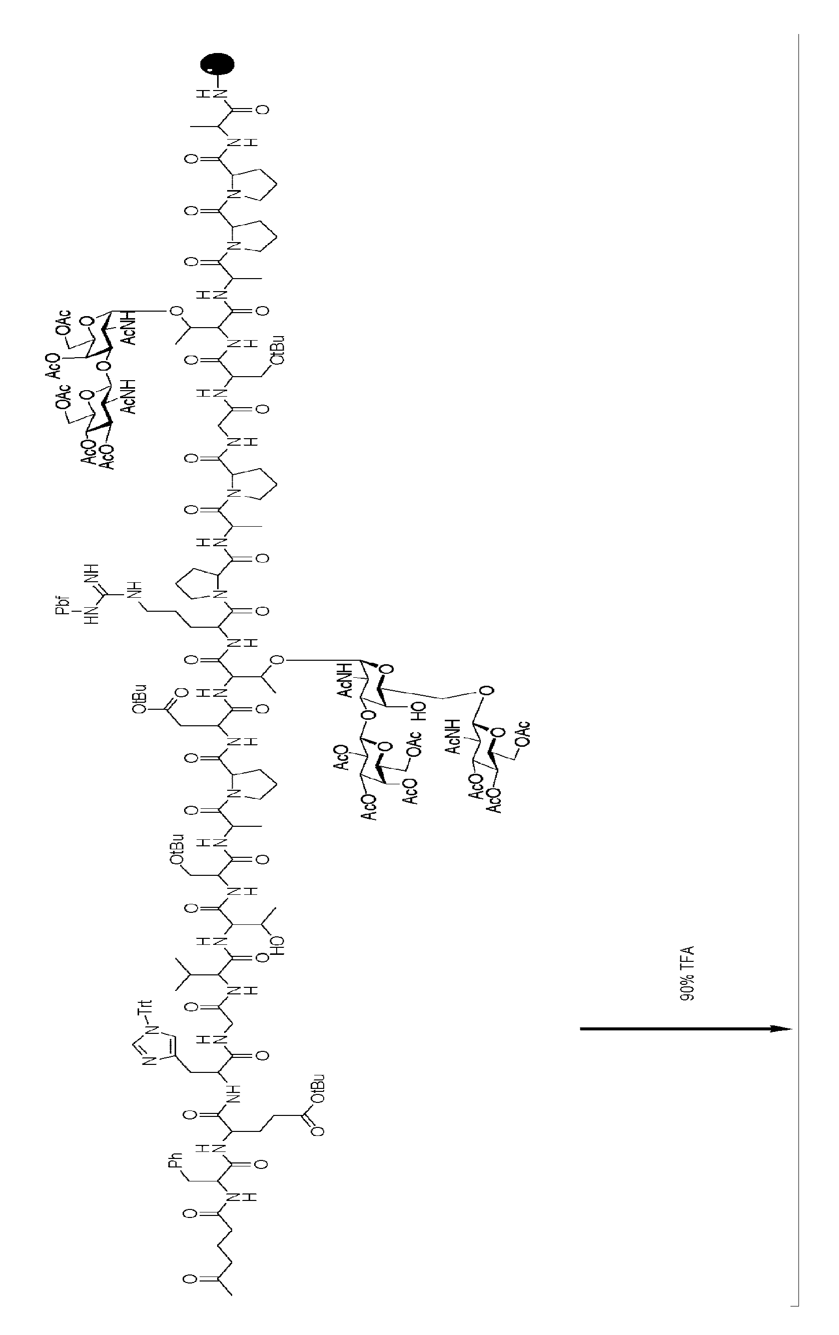

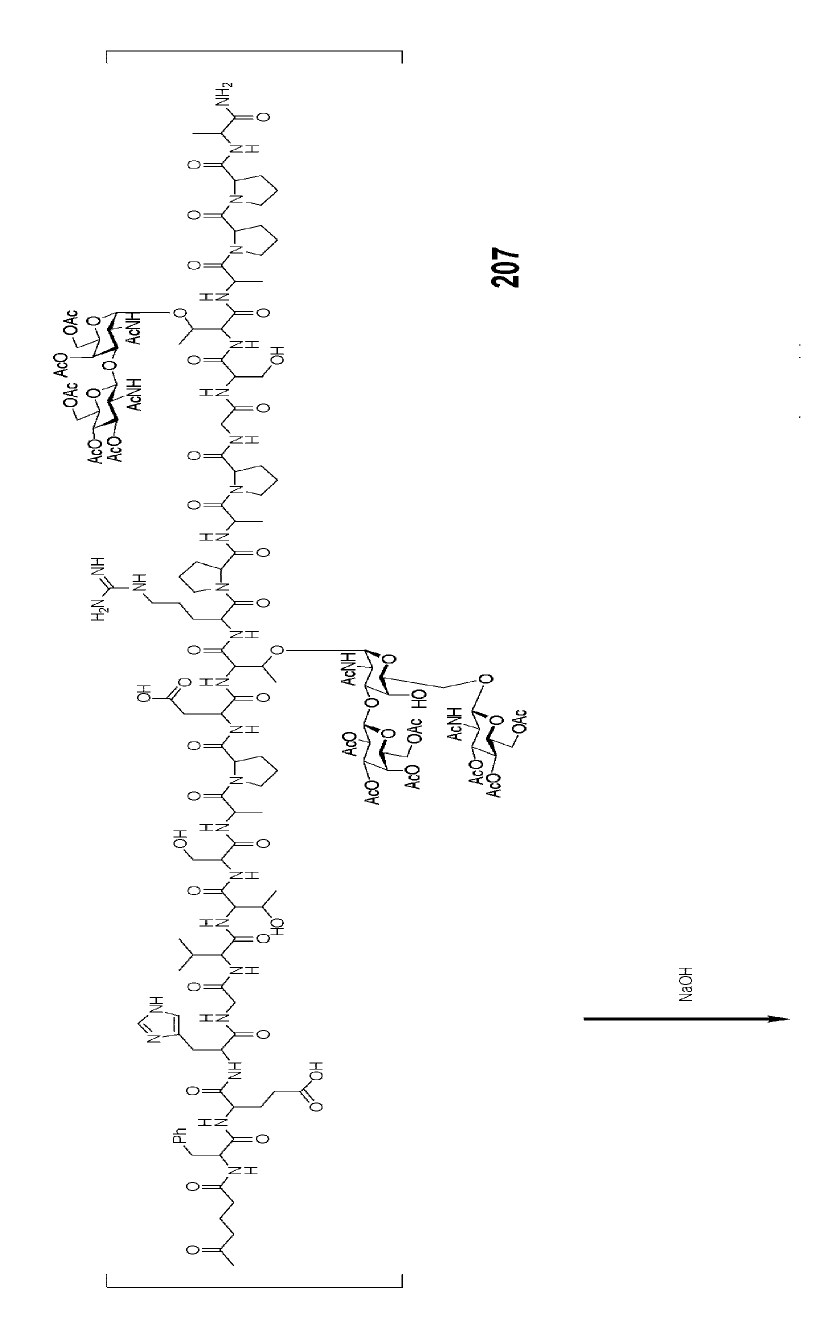

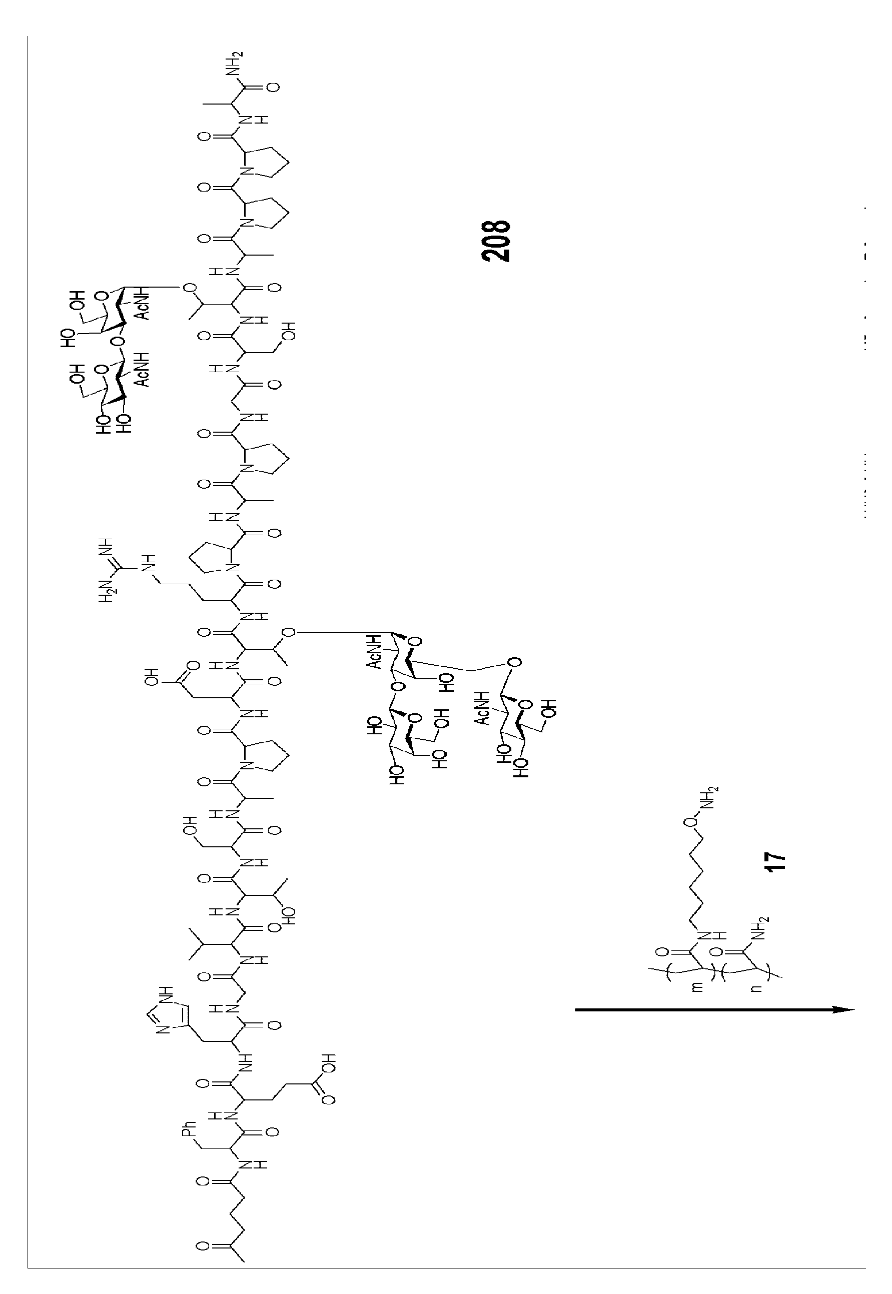

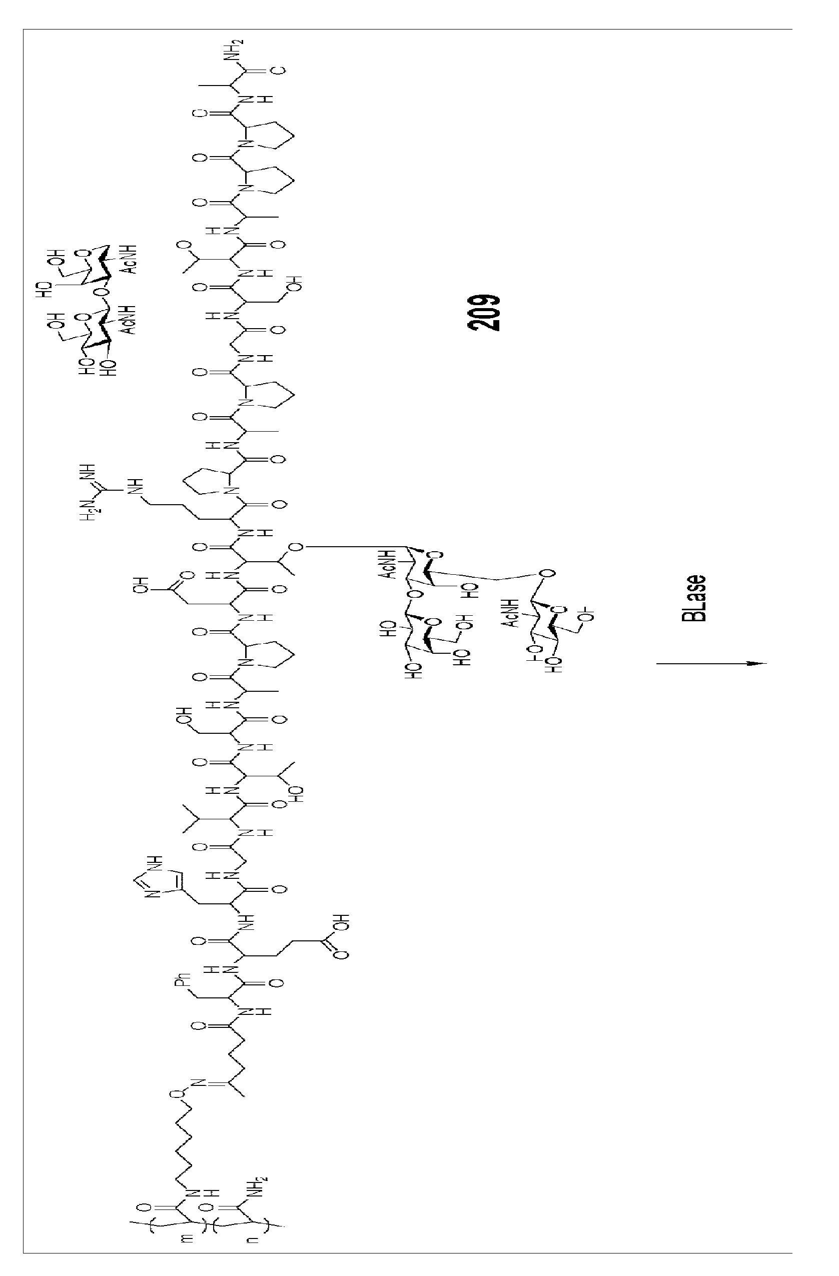

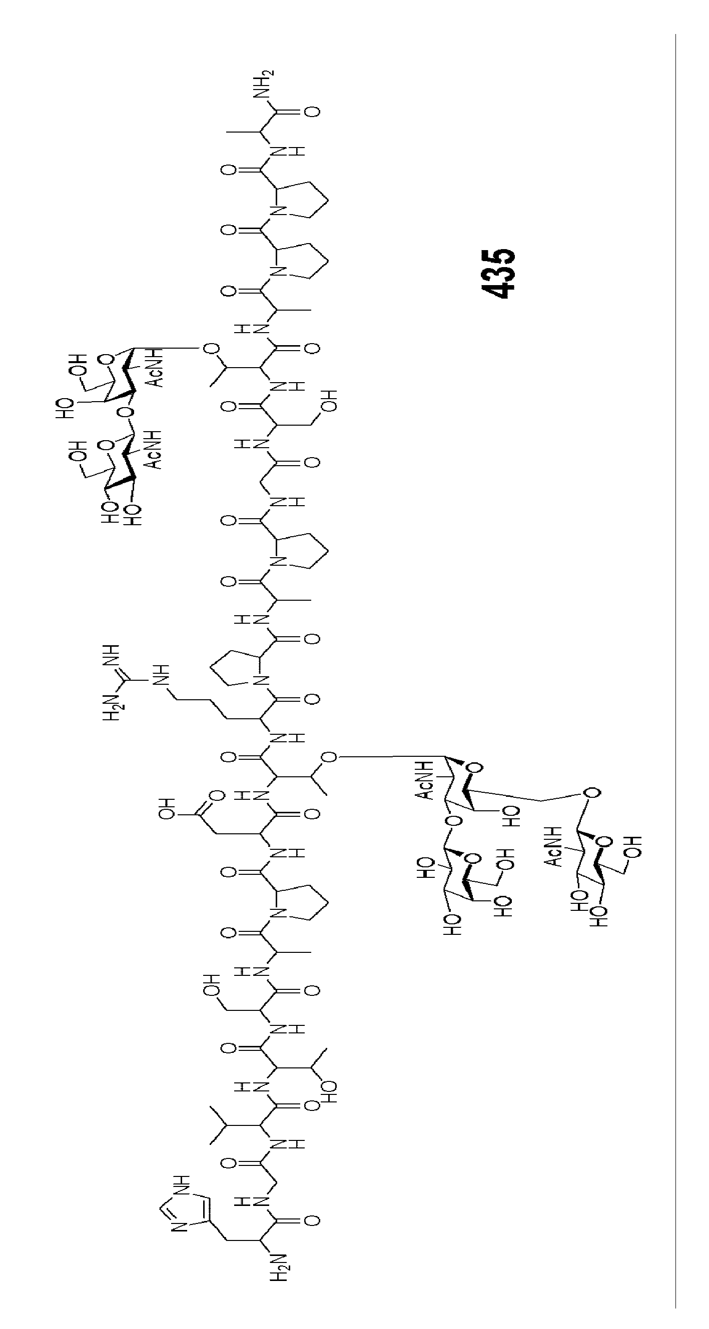

Using 71mg (0.02mmol) of Tentagel® S RAM resin (Hipep Laboratories, 0.28mmol/g) as a support, N-protected amino acids and keto acids as described below were sequentially condensed by Fmoc/HBTU/HOBt method, thereby synthesizing a glycopeptide derivative of interest: Fmoc-Ala-OH; Fmoc-Pro-OH; Fmoc-Pro-OH; Fmoc-Ala-OH; Fmoc-Thr(Ac5core3)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(Ac7core2)-OH; Fmoc-Asp(OtBu)-OH; Fmoc-Pro-OH; Fmoc-Ala-OH; Fmoc-Ser(tBu)-OH; Fmoc-Thr(tBu)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained resin equivalent to 5nmol was allowed to react in 90% TFA aqueous solution for two hours at room temperature to eliminate a protective group on a peptide residue and concurrently release compound (207) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 1.5ml of methanol. 1N sodium hydroxide aqueous solution was added to this solution, and was adjusted to have a pH of 12-12.5. The solution was then stirred for 1.5 hours at room temperature, thereby performing Ac deprotection reaction. After the reaction, 1N acetic acid was added and the solution was neutralized. Thereafter, resin was separated by filtration. The solvent in the filtrate was removed, and the residue was dissolved in 0.5ml of 50mM acetic acid/sodium acetate buffer solution (pH 5.5). 0.5ml of 10mM (oxyamine residue calculation) water-soluble polymer (17) aqueous solution was added to the solution, and the solution was stirred for 18 hours at room temperature, thereby reacting compound (208) with compound (17). After completion of the reaction, the reaction solution was subjected to centrifugal concentration with an ultrafiltration filter 10K Apollo ® 20ml (Orbital Biosciences, available from LIC). 25mM HEPES buffer solution (pH 7.0) was added thereto and the solution was again subjected to concentration, thereby washing the polymers. By adding water so that the final amount of the solution was 0.5ml, 10mM (theoretical content of glycopeptide) polymer (209) was obtained. Identification of the polymer (209) was performed by treating a part of polymer (209) with BLase to obtain product (435). MALDI-TOF/MS:[M(average)+H]⁺ of compound (435)=2863.6 (theoretical value: [M(average)+H]+=2861.9).

(3.40 Synthesis of compounds (210) to (212) using a one-pot reaction)

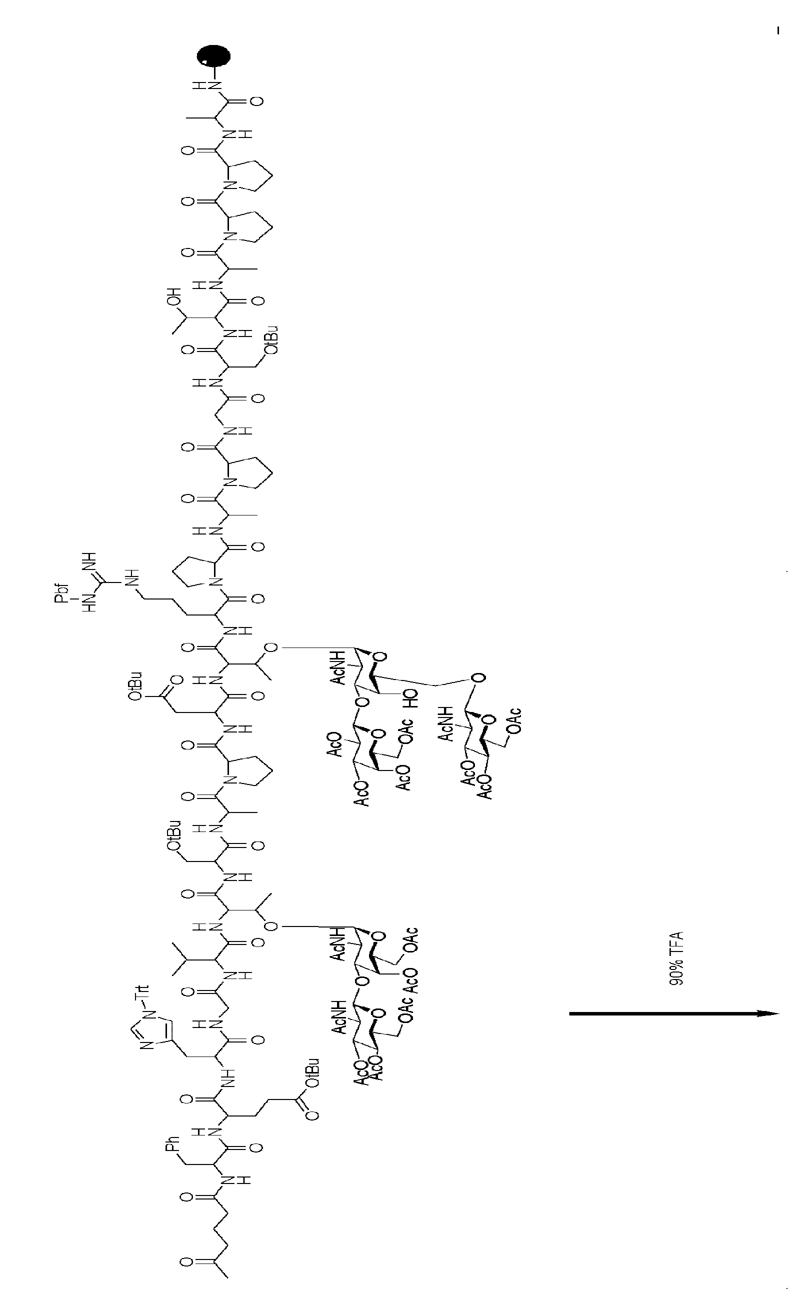

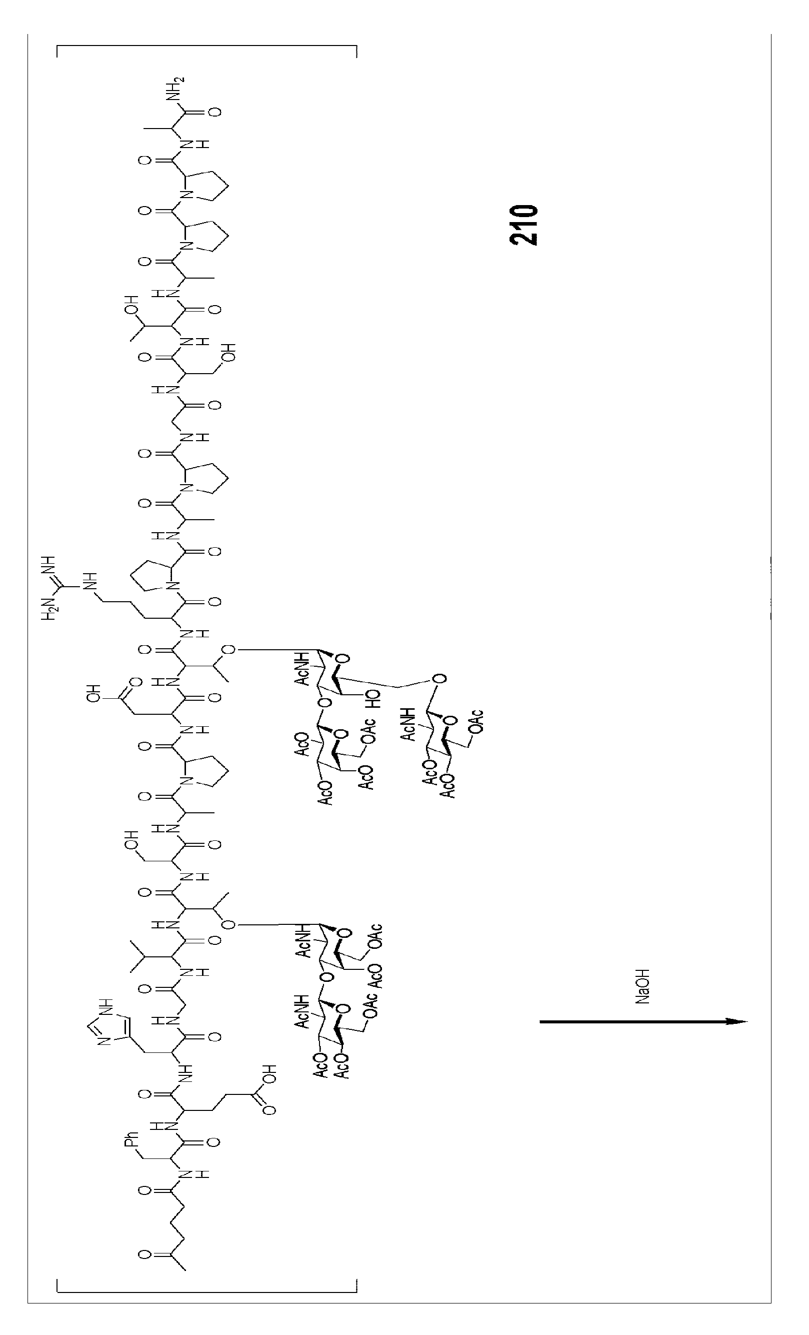

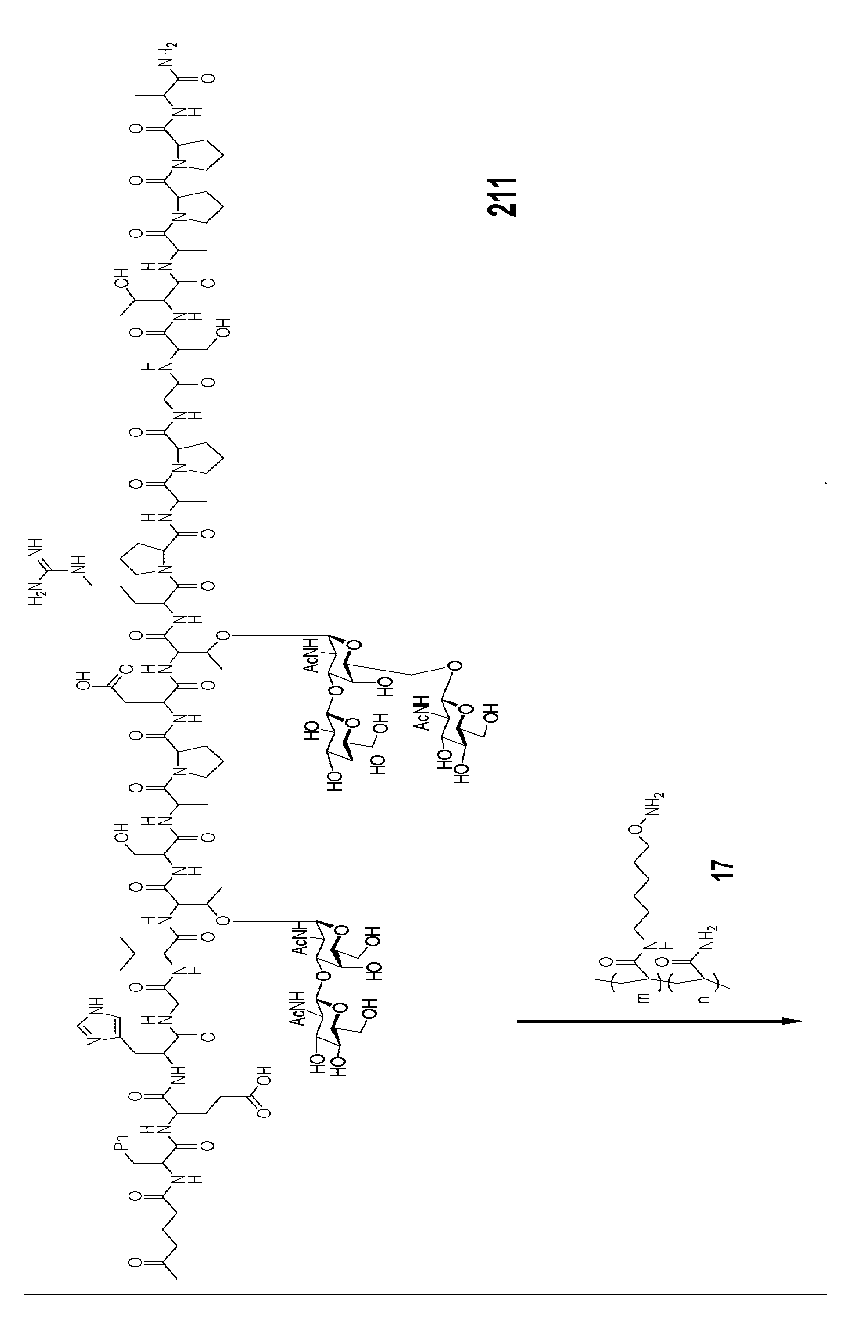

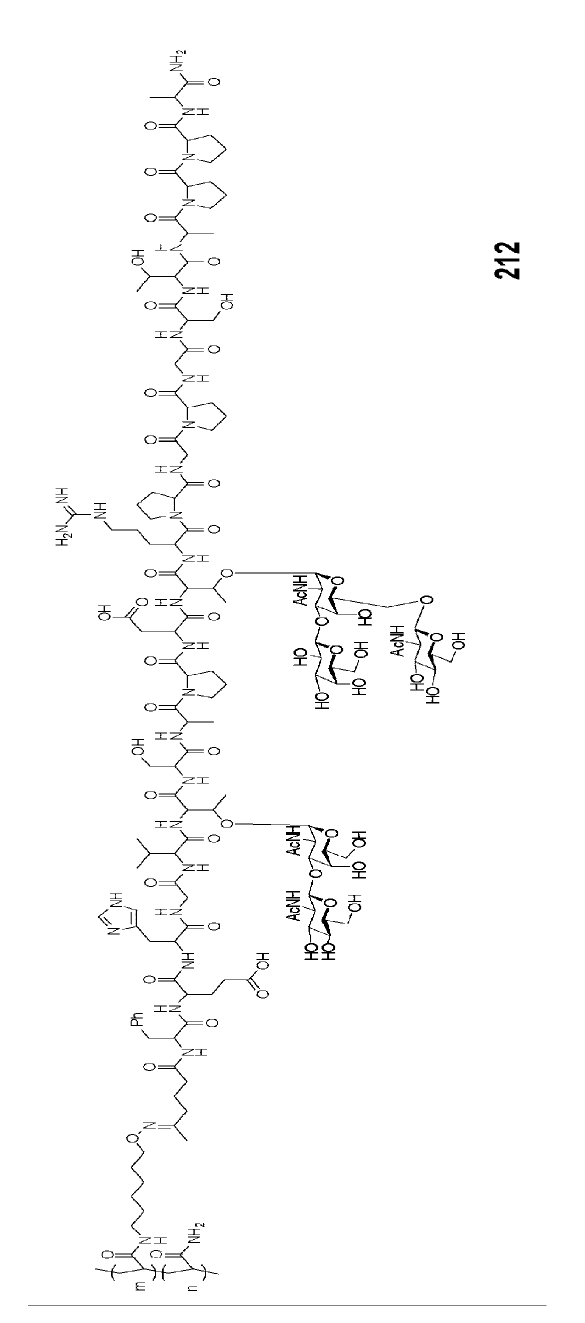

Using 71mg (0.02mmol) of Tentagel® S RAM resin (Hipep Laboratories, 0.28mmol/g) as a support, N-protected amino acids and keto acids as described below were sequentially condensed by Fmoc/HBTU/HOBt method, thereby synthesizing a glycopeptide derivative of interest: Fmoc-Ala-OH; Fmoc-Pro-OH; Fmoc-Pro-OH; Fmoc-Ala-OH; Fmoc-Thr(tBu)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(Ac7core2)-OH; Fmoc-Asp(OtBu)-OH; Fmoc-Pro-OH; Fmoc-Ala-OH; Fmoc-Ser(tBu)-OH; Fmoc-Thr(Ac5core3)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained resin equivalent to 5nmol was allowed to react in 90% TFA aqueous solution for two hours at room temperature to eliminate a protective group on a peptide residue and concurrently release compound (210) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether was again added, and the precipitate was washed. Centrifugal separation was again performed and the supernatant was removed. The obtained precipitant was dissolved in 1.5ml of methanol. 1N sodium hydroxide aqueous solution was added to this solution, and was adjusted to have a pH of 12-12.5. The solution was then stirred for 1.5 hours at room temperature, thereby performing Ac deprotection reaction. After the reaction, 1N acetic acid was added and the solution was neutralized. Thereafter, the resin was separated by filtration. The solvent in the filtrate was removed, and the residue was dissolved in 0.5ml of 50mM acetic acid/sodium acetate buffer solution (pH 5.5). 0.5ml of 10mM (oxyamine residue calculation) water-soluble polymer (17) aqueous solution was added to the solution, and the solution was stirred for 18 hours at room temperature, thereby reacting compound (211) with compound (17). After completion of the reaction, the reaction solution was subjected to centrifugal concentration with an ultrafiltration filter 10K Apollo ® 20ml (Orbital Biosciences, available from LIC). 25mM HEPES buffer solution (pH 7.0) was added thereto and the solution was again subjected to concentration, thereby washing the polymers. By adding water so that the final amount of the solution was 0.5ml, 10mM (theoretical content of glycopeptide) polymer (212) was obtained. Identification of the polymer (212) was performed based on that product (309) was obtained in the following section (3.58).

(3.41 Synthesis of compounds (213) to (215) using a one-pot reaction)

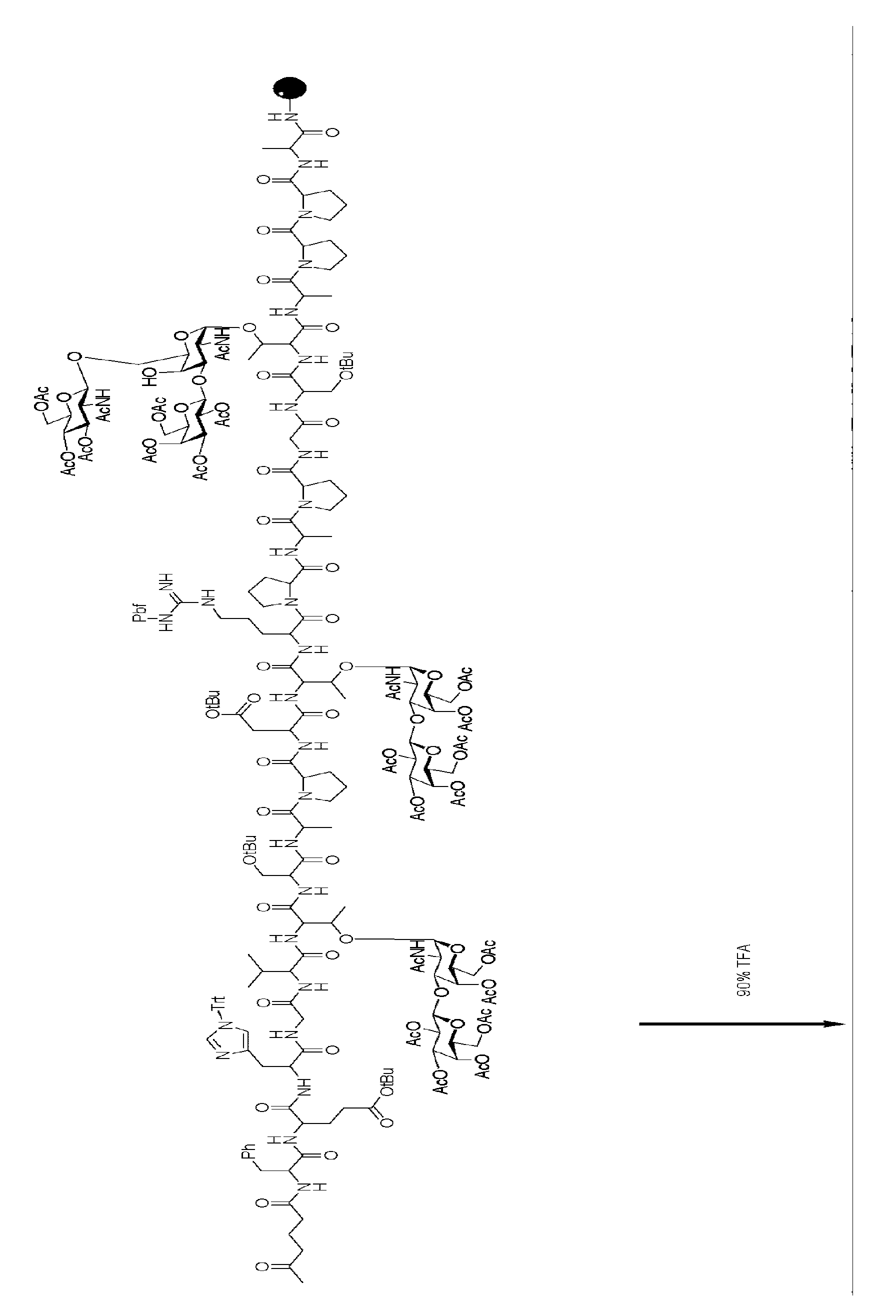

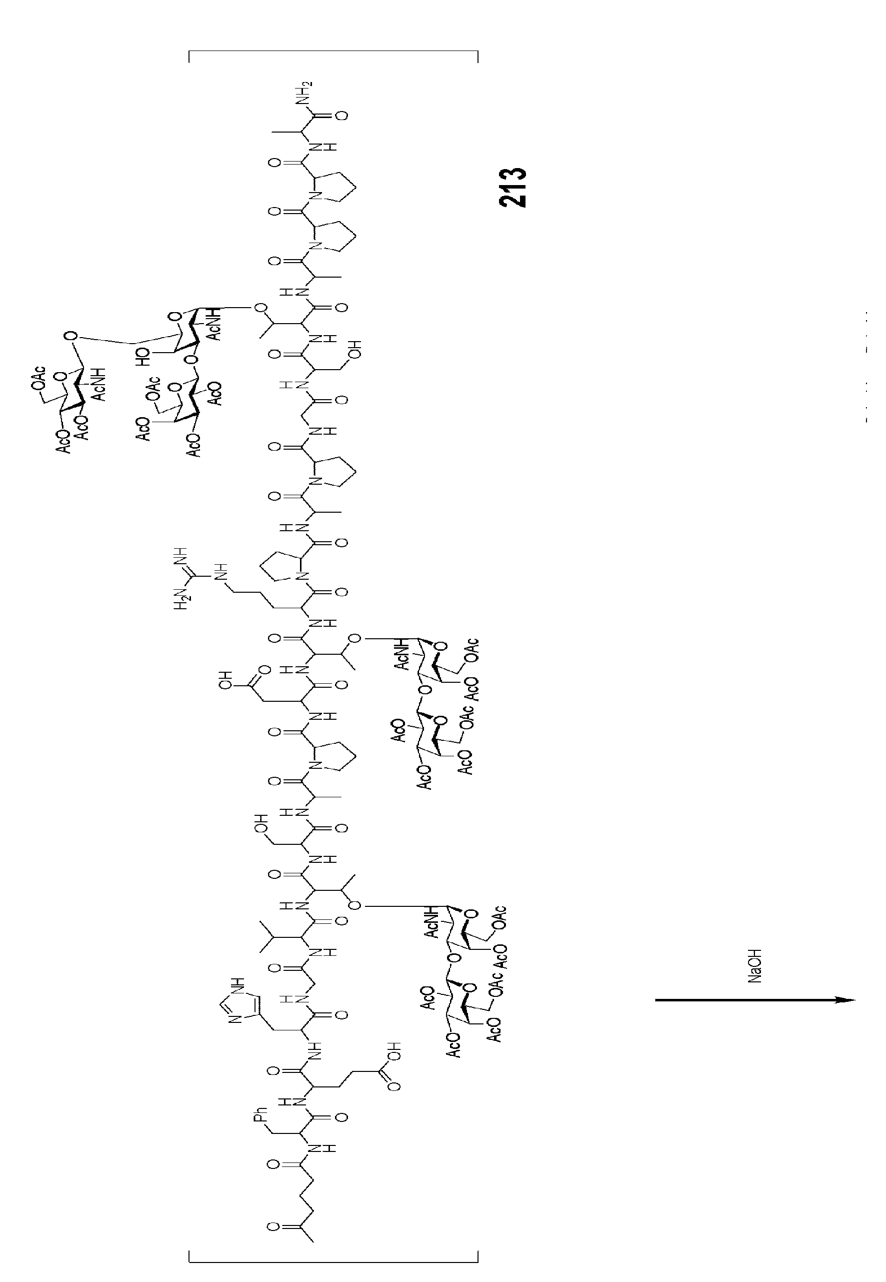

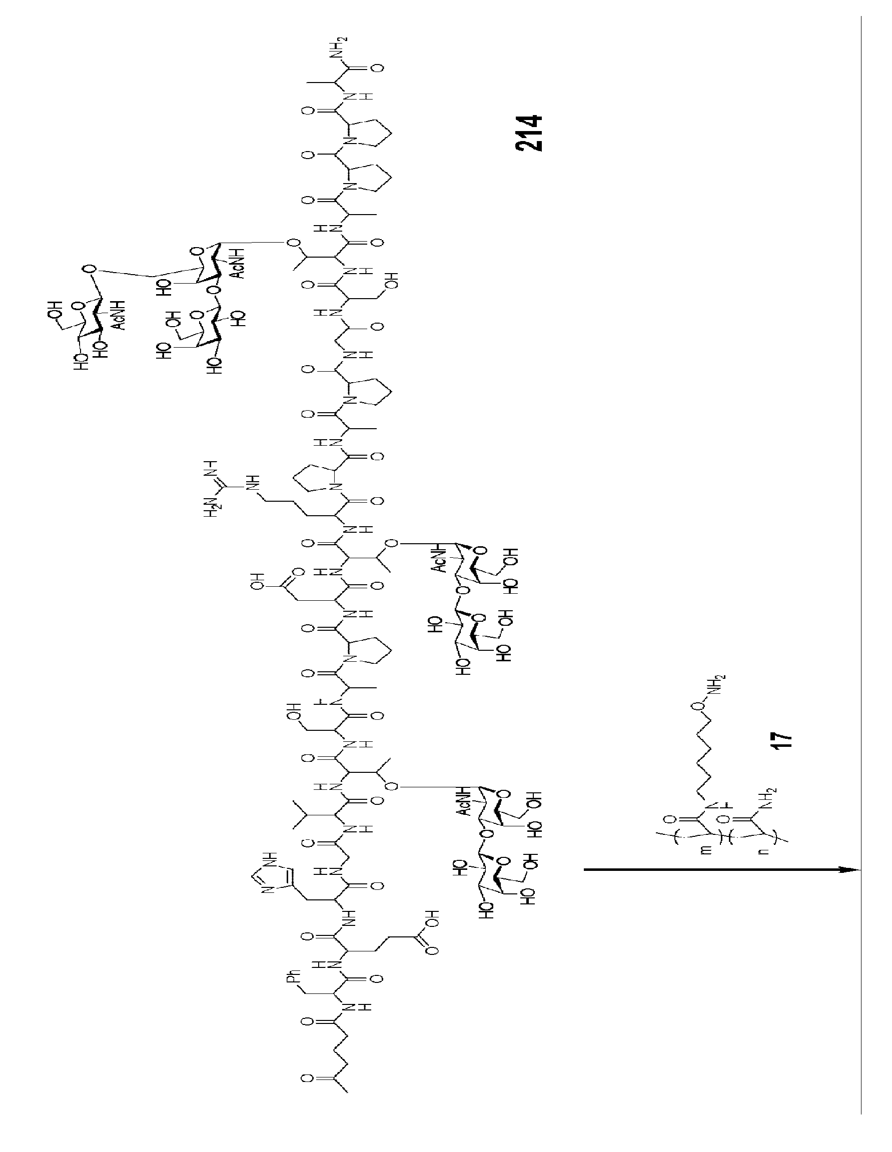

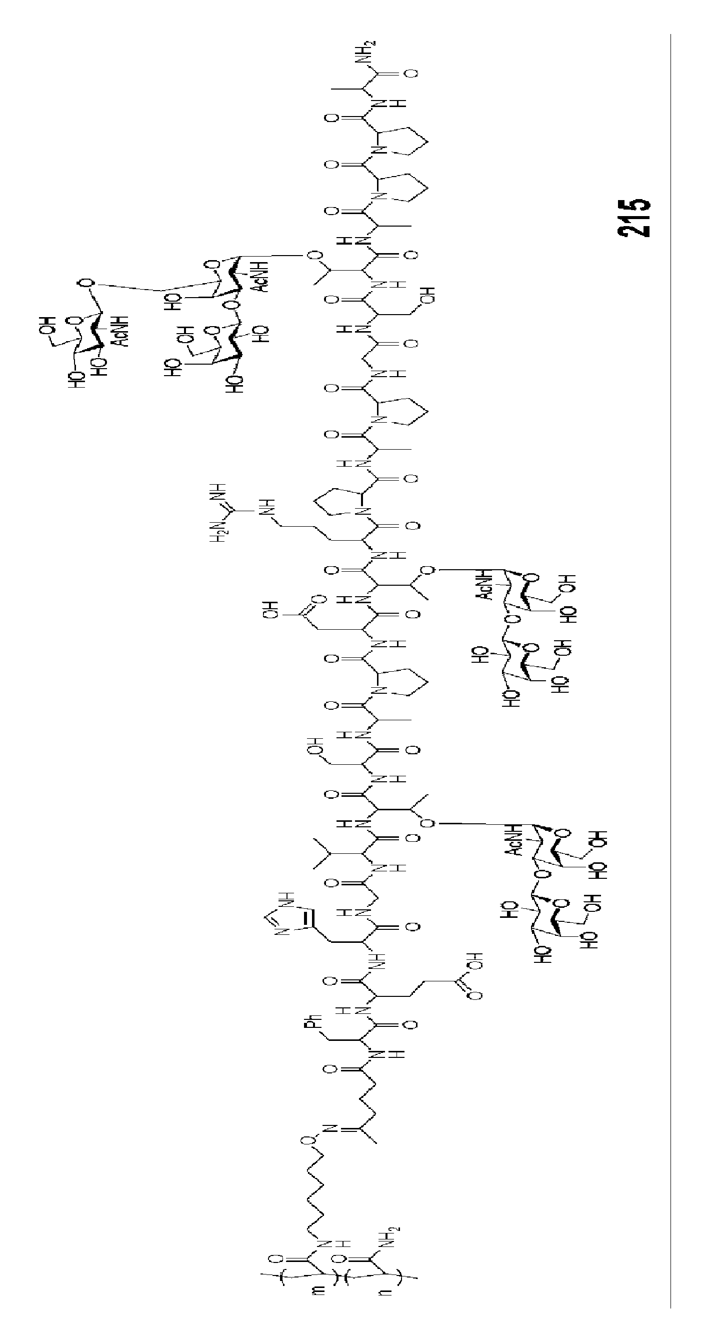

Using 71mg (0.02mmol) of Tentagel® S RAM resin (Hipep Laboratories, 0.28mmol/g) as a support, N-protected amino acids and keto acids as described below were sequentially condensed by Fmoc/HBTU/HOBt method, thereby synthesizing a glycopeptide derivative of interest: Fmoc-Ala-OH; Fmoc-Pro-OH; Fmoc-Pro-OH; Fmoc-Ala-OH; Fmoc-Thr(Ac7core2)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(Ac6corel)-OH; Fmoc-Asp(OtBu)-OH; Fmoc-Pro-OH; Fmoc-Ala-OH; Fmoc-Ser(tBu)-OH; Fmoc-Thr(Ac6corel)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained resin equivalent to 5nmol was allowed to react in 90% TFA aqueous solution for two hours at room temperature to eliminate a protective group on a peptide residue and concurrently release compound (213) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 1.5ml of methanol. 1N sodium hydroxide aqueous solution was added to this solution, and was adjusted to have a pH of 12-12.5. The solution was then stirred for 1.5 hours at room temperature, thereby performing Ac deprotection reaction. After the reaction, 1N acetic acid was added and the solution was neutralized. Thereafter, the resin was separated by filtration. The solvent in the filtrate was removed, and the residue was dissolved in 0.5ml of 50mM acetic acid/sodium acetate buffer solution (pH 5.5). 0.5ml of 10mM (oxyamine residue calculation) water-soluble polymer (17) aqueous solution was added to the solution, and the solution was stirred for 18 hours at room temperature, thereby reacting compound (214) with compound (17). After completion of the reaction, the reaction solution was subjected to centrifugal concentration with an ultrafiltration filter 10K Apollo ® 20ml (Orbital Biosciences, available from LIC) . 25mM HEPES buffer solution (pH 7.0) was added thereto and the solution was again subjected to concentration, thereby washing the polymers. By adding water so that the final amount of the solution was 0.5ml, 10mM (theoretical content of glycopeptide) polymer (215) was obtained. Identification of the polymer (215) was performed based on that compound (320) was obtained in the following section (3.59).

(3.42 Synthesis of compounds (216) to (218) using a one-pot reaction)

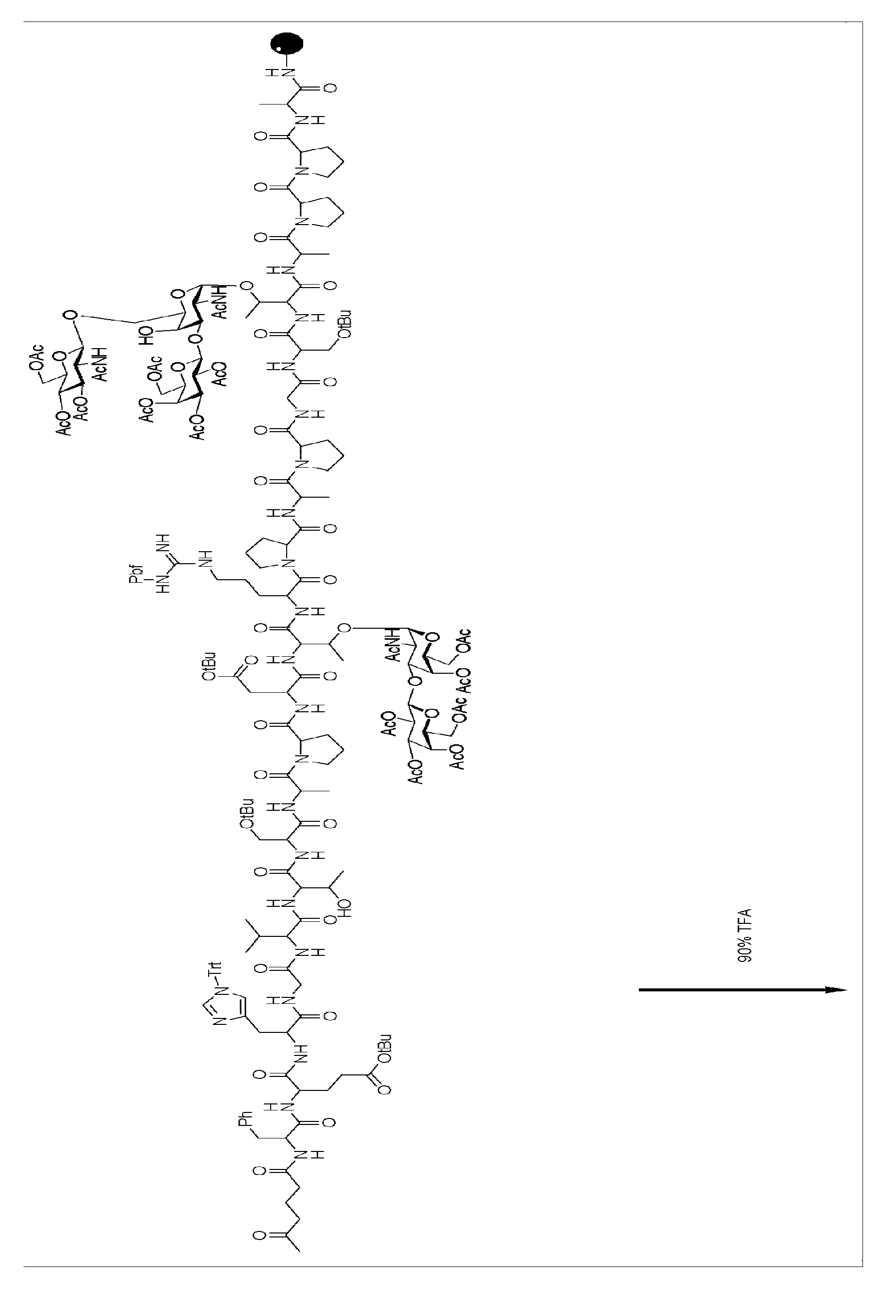

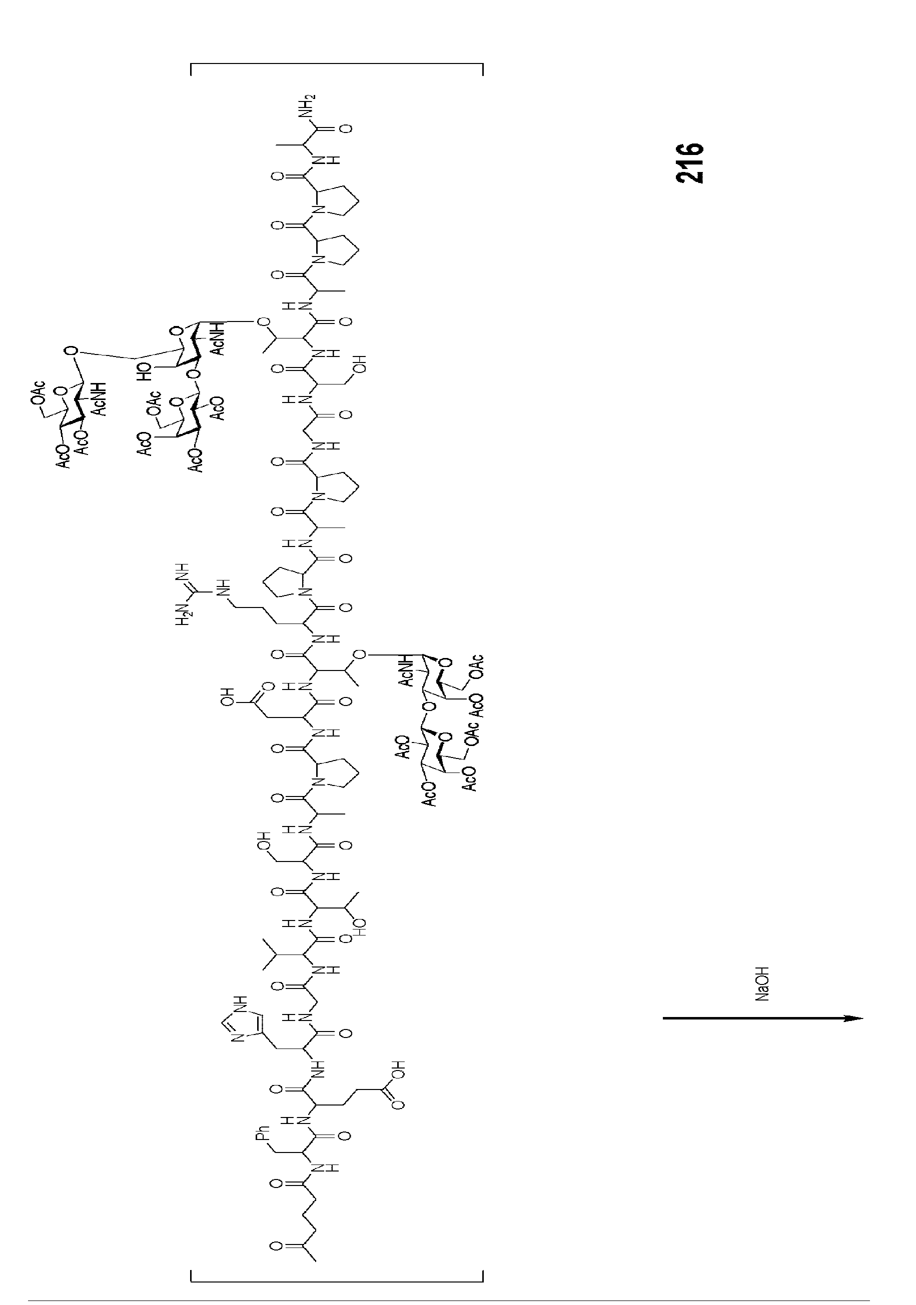

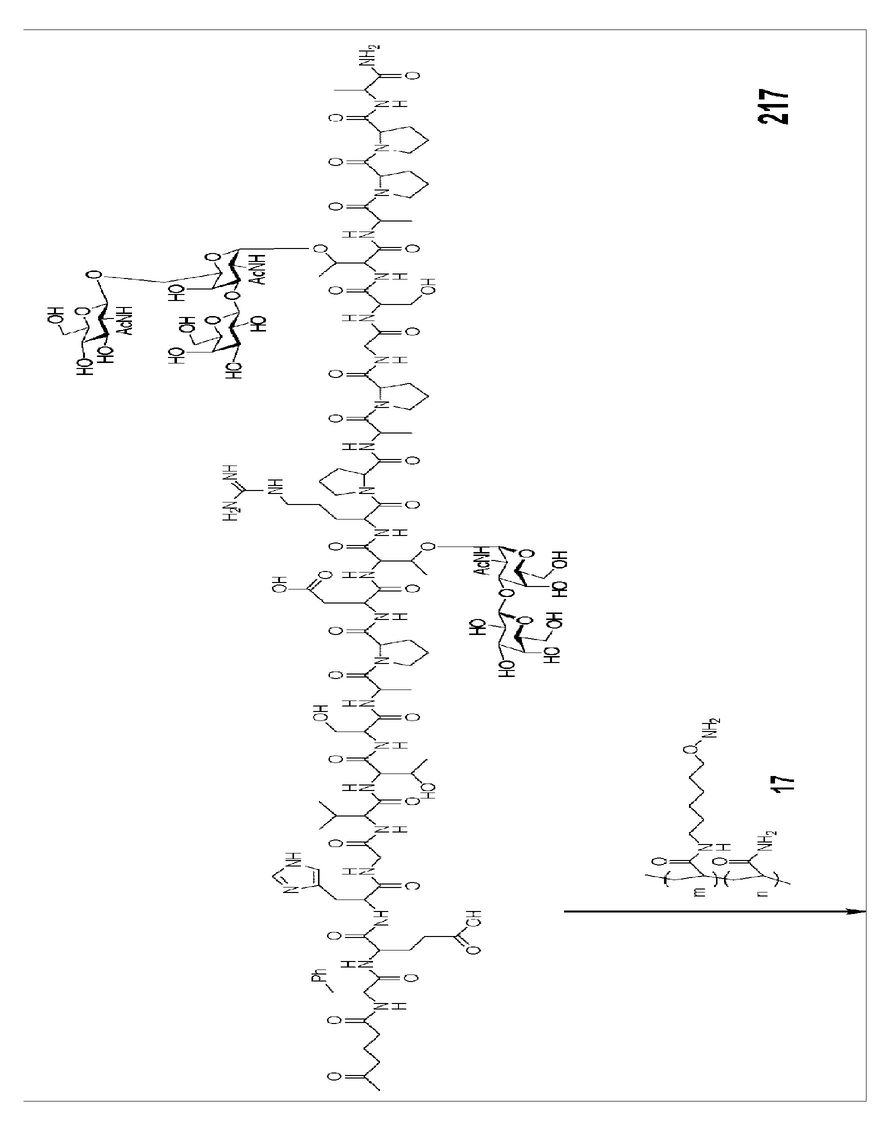

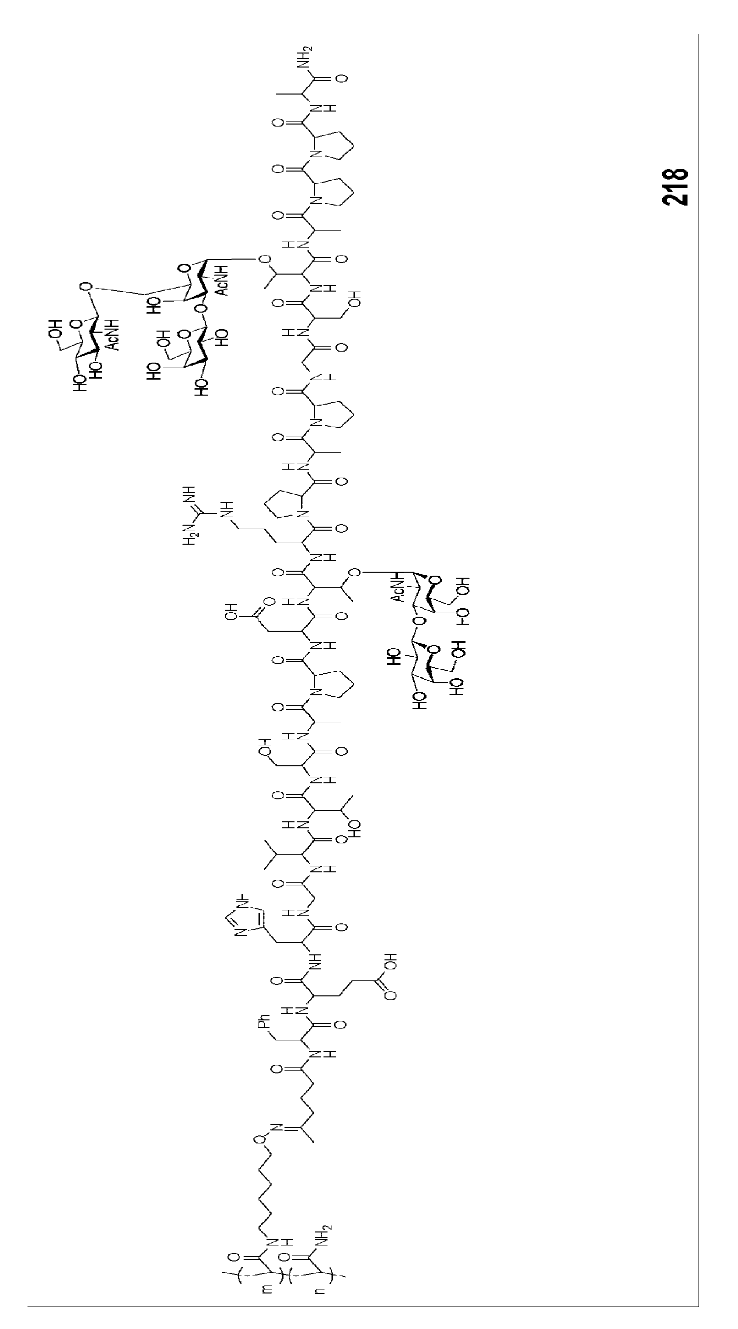

Using 71mg (0.02mmol) of Tentagel® S RAM resin (Hipep Laboratories, 0.28mmol/g) as a support, N-protected amino acids and keto acids as described below were sequentially condensed by Fmoc/HBTU/HOBt method, thereby synthesizing a glycopeptide derivative of interest: Fmoc-Ala-OH; Fmoc-Pro-OH; Fmoc-Pro-OH; Fmoc-Ala-OH; Fmoc-Thr(Ac7core2)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(Ac6core1)-OH; Fmoc-Asp(OtBu)-OH; Fmoc-Pro-OH; Fmoc-Ala-OH; Fmoc-Ser(tBu)-OH; Fmoc-Thr(tBu)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained resin equivalent to 5nmol was allowed to react in 90% TFA aqueous solution for two hours at room temperature to eliminate a protective group on a peptide residue and concurrently release compound (216) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 1.5ml of methanol. 1N sodium hydroxide aqueous solution was added to this solution, and was adjusted to have a pH of 12-12.5. The solution was then stirred for 1.5 hours at room temperature, thereby performing Ac deprotection reaction. After the reaction, 1N acetic acid was added and the solution was neutralized. Thereafter, the resin was separated by filtration. The solvent in the filtrate was removed, and the residue was dissolved in 0.5ml of 50mM acetic acid/sodium acetate buffer solution (pH 5.5). 0.5ml of 10mM (oxyamine residue calculation) water-soluble polymer (17) aqueous solution was added to the solution, and the solution was stirred for 18 hours at room temperature, thereby reacting compound (217) with compound (17). After completion of the reaction, the reaction solution was subjected to centrifugal concentration with an ultrafiltration filter 10K Apollo ® 20ml (Orbital Biosciences, available from LIC). 25mM HEPES buffer solution (pH 7.0) was added thereto and the solution was again subjected to concentration, thereby washing the polymers. By adding water so that the final amount of the solution was 0.5ml, 10mM (theoretical content of glycopeptide) polymer (218) was obtained. Identification of the polymer (218) was performed based on that product (331) was obtained in the following section (3.60).

(3.43 Synthesis of compounds (219) to (221) using a one-pot reaction)

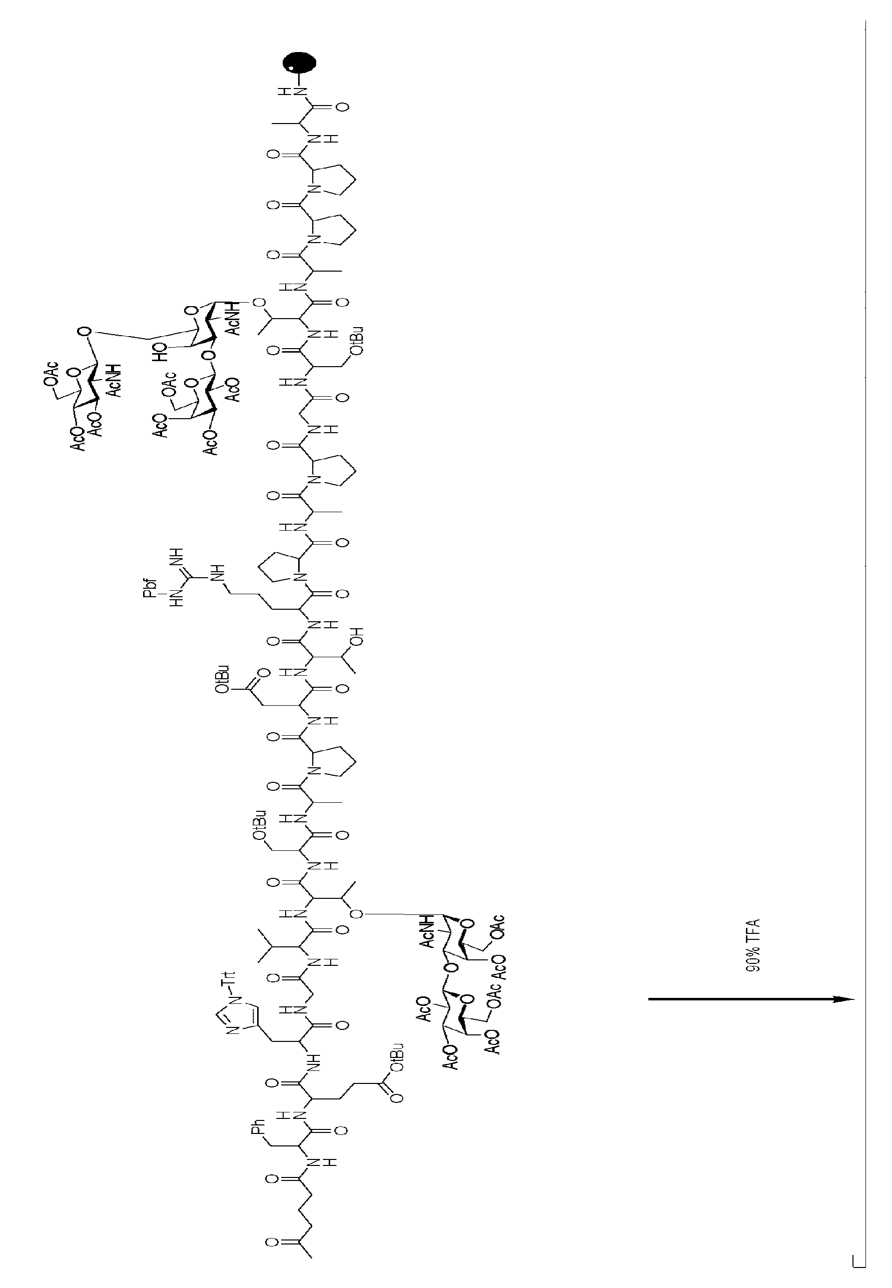

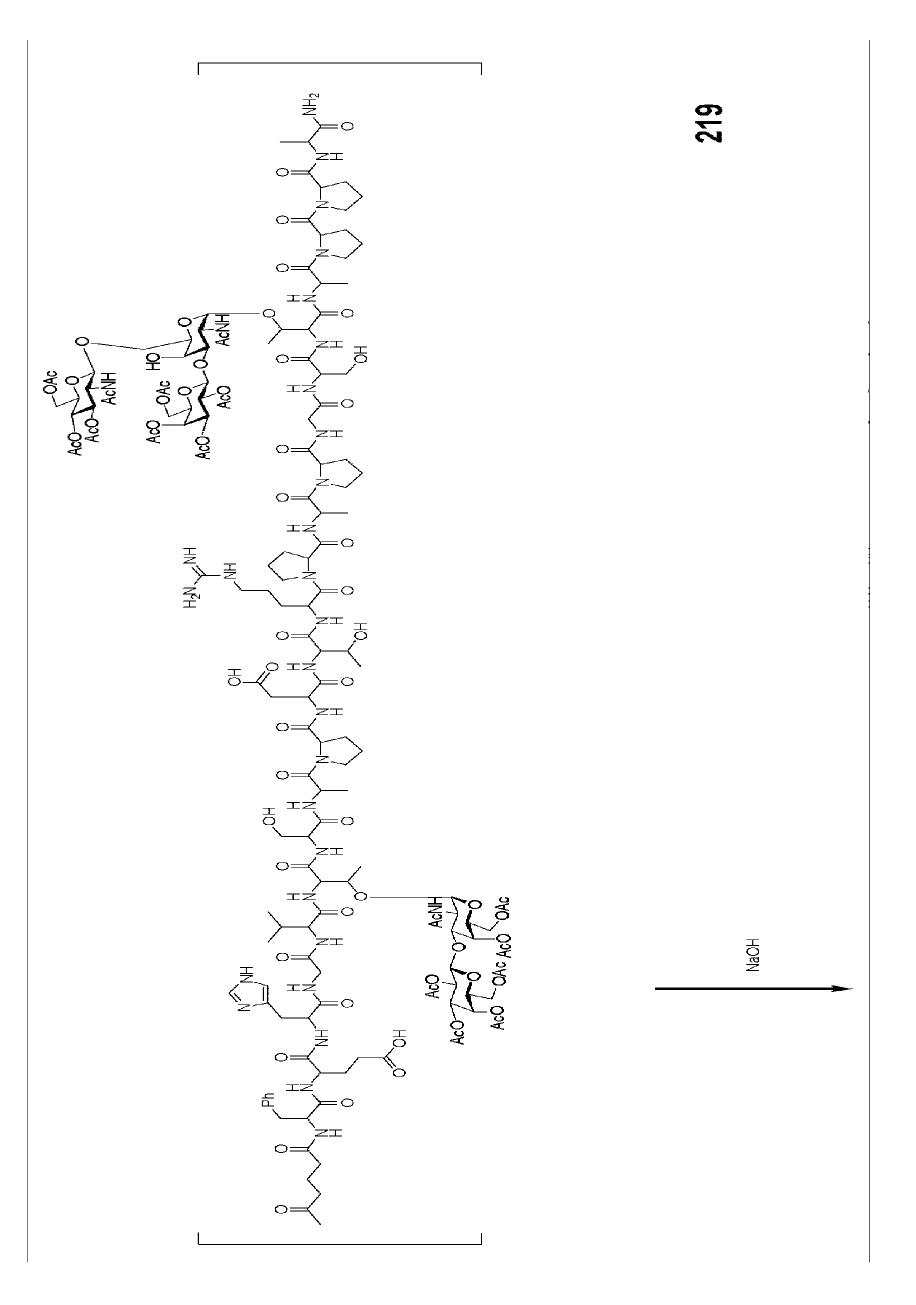

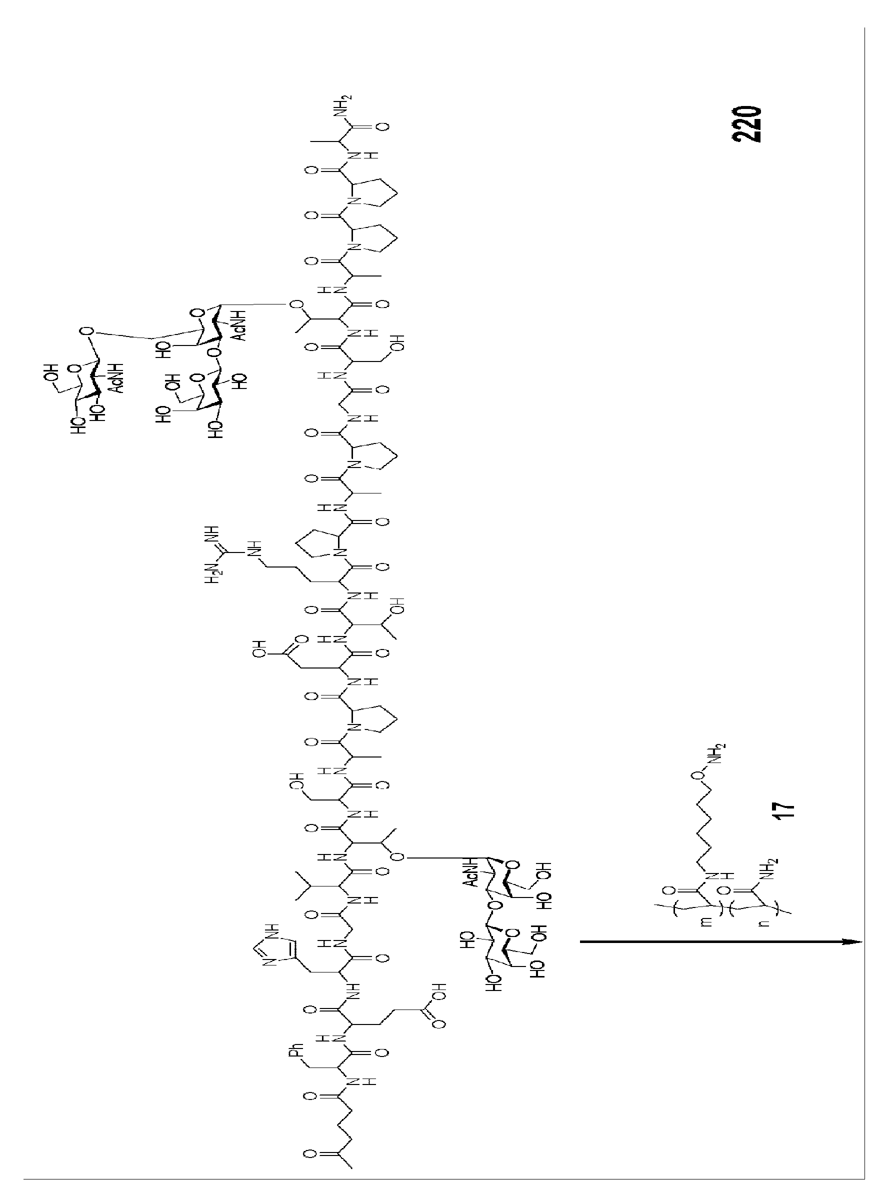

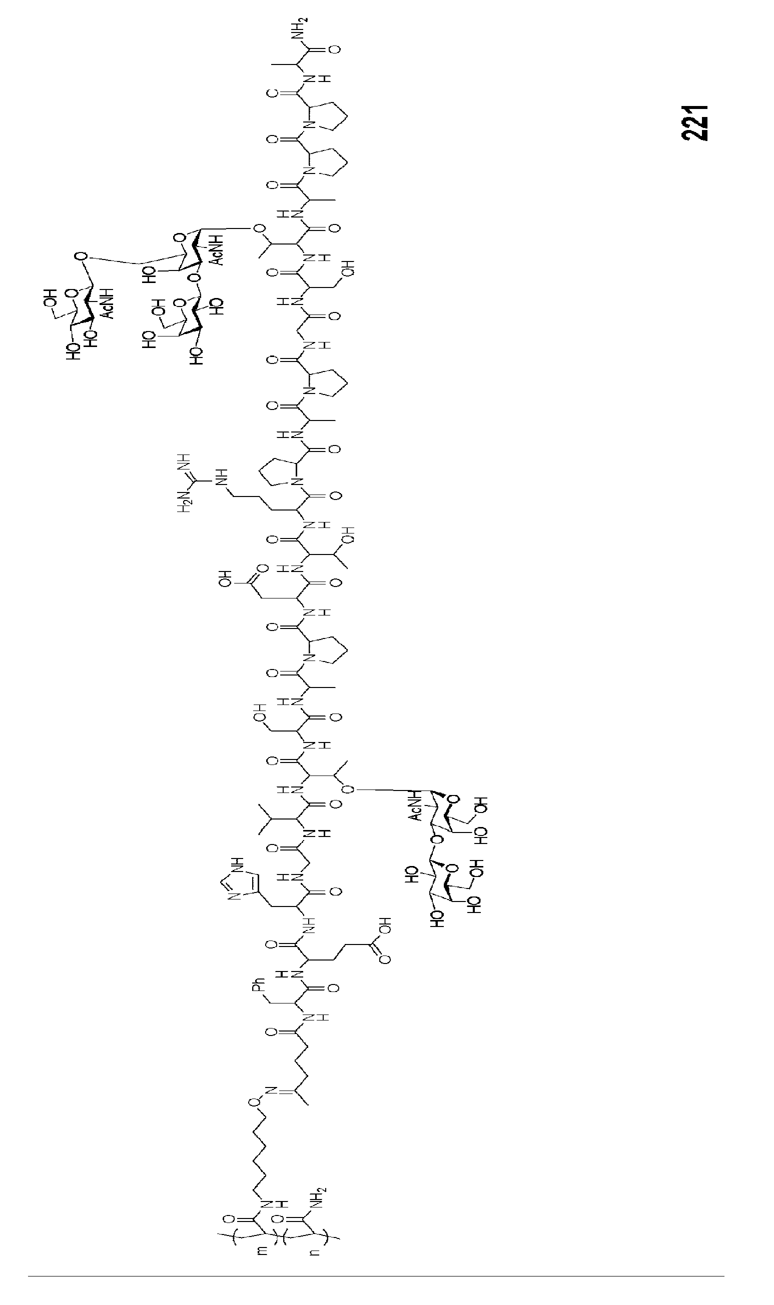

Using 71mg (0.02mmol) of Tentagel® S RAM resin (Hipep Laboratories, 0.28mmol/g) as a support, N-protected amino acids and keto acids as described below were sequentially condensed by Fmoc/HBTU/HOBt method, thereby synthesizing a glycopeptide derivative of interest: Fmoc-Ala-OH; Fmoc-Pro-OH; Fmoc-Pro-OH; Fmoc-Ala-OH; Fmoc-Thr(Ac7core2)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(tBu)-OH; Fmoc-Asp(OtBu)-OH; Fmoc-Pro-OH; Fmoc-Ala-OH; Fmoc-Ser(tBu)-OH; Fmoc-Thr(Ac6core1)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained resin equivalent to 5nmol was allowed to react in 90% TFA aqueous solution for two hours at room temperature to eliminate a protective group on a peptide residue and concurrently release compound (219) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 1.5ml of methanol. 1N sodium hydroxide aqueous solution was added to this solution, and was adjusted to have a pH of 12-12.5. The solution was then stirred for 1.5 hours at room temperature, thereby performing Ac deprotection reaction. After the reaction, 1N acetic acid was added and the solution was neutralized. Thereafter, the resin was separated by filtration. The solvent in the filtrate was removed, and the residue was dissolved in 0.5ml of 50mM acetic acid/sodium acetate buffer solution (pH 5.5). 0.5m1 of 10mM (oxyamine residue calculation) water-soluble polymer (17) aqueous solution was added to the solution, and the solution was stirred for 18 hours at room temperature, thereby reacting compound (220) with compound (17). After completion of the reaction, the reaction solution was subjected to centrifugal concentration with an ultrafiltration filter 10K Apollo ® 20ml (Orbital Biosciences, available from LIC). 25mM HEPES buffer solution (pH 7.0) was added thereto and the solution was again subjected to concentration, thereby washing the polymers. By adding water so that the final amount of the solution was 0.5ml, 10mM (theoretical content of glycopeptide) polymer (221) was obtained. Identification of the polymer (221) was performed based on that product (342) was obtained in the following section (3.61).

(3.44 Synthesis of compounds (222) to (224) using a one-pot reaction)

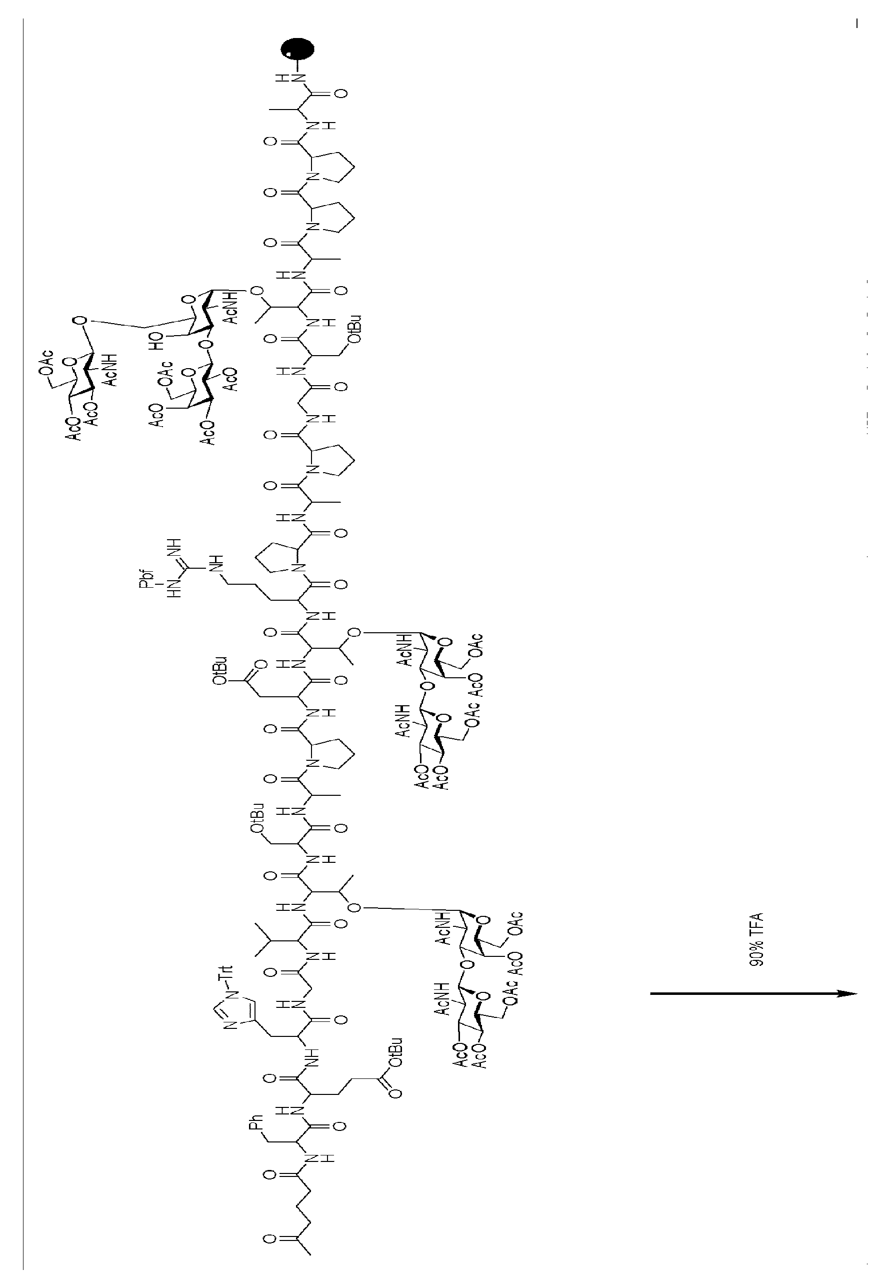

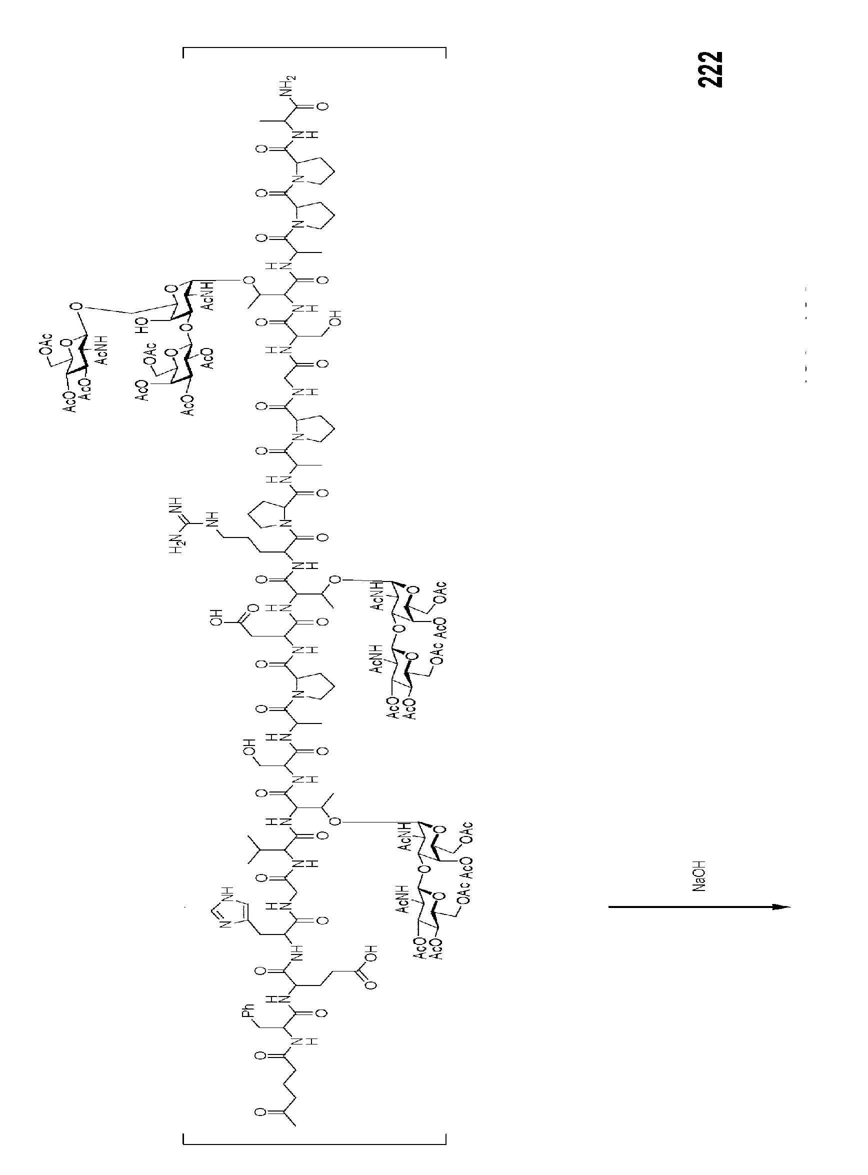

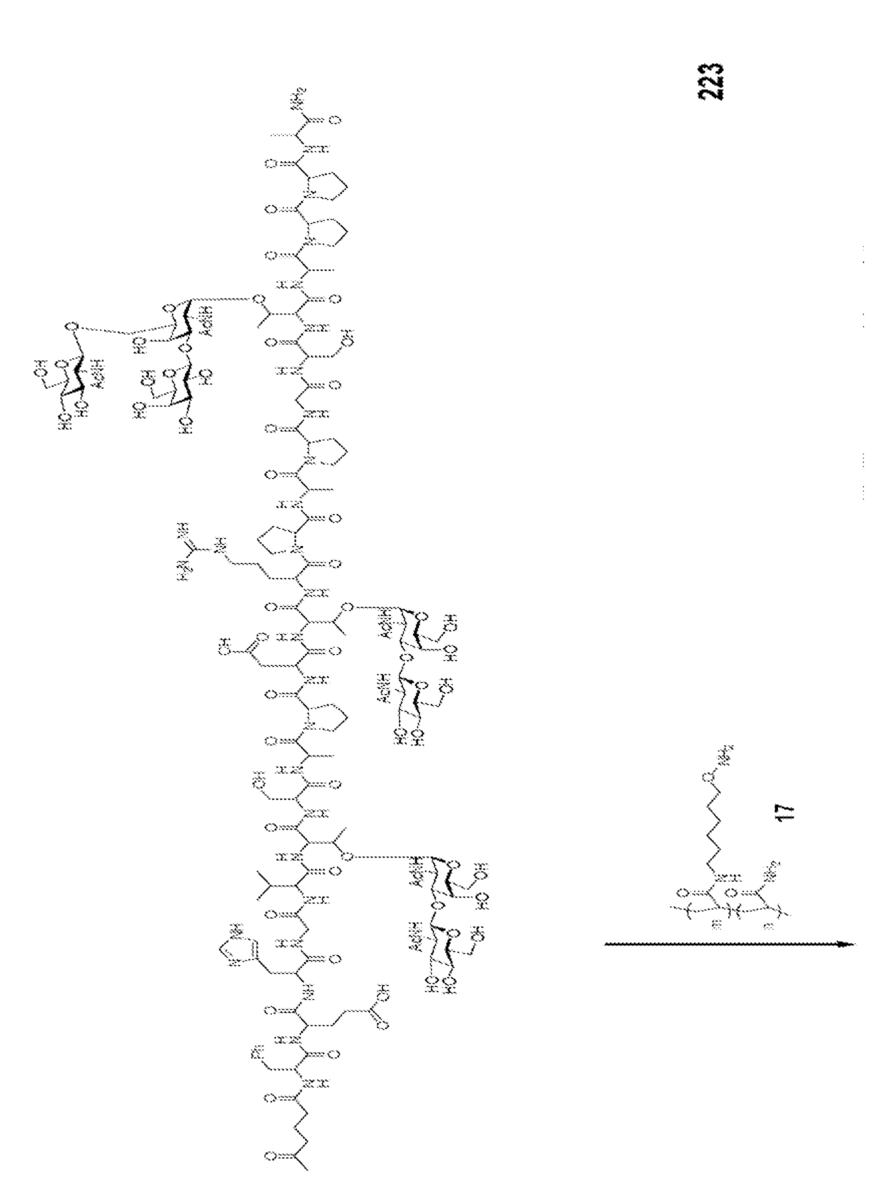

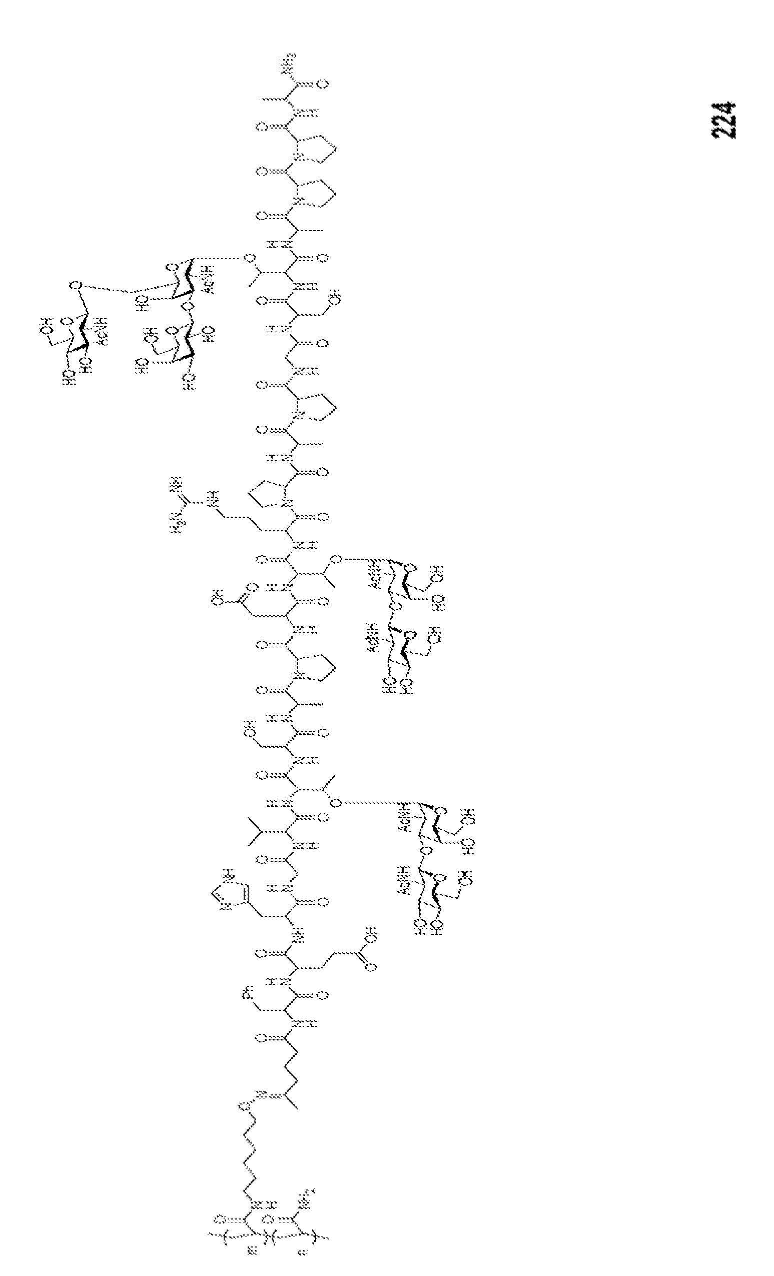

Using 71mg (0.02mmol) of Tentagel® S RAM resin (Hipep Laboratories, 0.28mmol/g) as a support, N-protected amino acids and keto acids as described below were sequentially condensed by Fmoc/HBTU/HOBt method, thereby synthesizing a glycopeptide derivative of interest: Fmoc-Ala-OH; Fmoc-Pro-OH; Fmoc-Pro-OH; Fmoc-Ala-OH; Fmoc-Thr(Ac7core2)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(Ac5core3)-OH; Fmoc-Asp(OtBu)-OH; Fmoc-Pro-OH; Fmoc-Ala-OH; Fmoc-Ser(tBu)-OH; Fmoc-Thr(Ac5core3)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained resin equivalent to 5nmol was allowed to react in 90% TFA aqueous solution for two hours at room temperature to eliminate a protective group on a peptide residue and concurrently release compound (222) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 1.5ml of methanol. 1N sodium hydroxide aqueous solution was added to this solution, and was adjusted to have a pH of 12-12.5. The solution was then stirred for 1.5 hours at room temperature, thereby performing Ac deprotection reaction. After the reaction, 1N acetic acid was added and the solution was neutralized. Thereafter, the resin was separated by filtration. The solvent in the filtrate was removed, and the residue was dissolved in 0.5ml of 50mM acetic acid/sodium acetate buffer solution (pH 5.5). 0.5m1 of 10mM (oxyamine residue calculation) water-soluble polymer (17) aqueous solution was added to the solution, and the solution was stirred for 18 hours at room temperature, thereby reacting compound (223) with compound (17) . After completion of the reaction, the reaction solution was subjected to centrifugal concentration with an ultrafiltration filter 10K Apollo ® 20ml (Orbital Biosciences, available from LIC). 25mM HEPES buffer solution (pH 7.0) was added thereto and the solution was again subjected to concentration, thereby washing the polymers. By adding water so that the final amount of the solution was 0.5ml, 10mM (theoretical content of glycopeptide) polymer (224) was obtained. Identification of the polymer (224) was performed based on that product (353) was obtained in the following section (3.62).

(3.45 Synthesis of compounds (225) to (227) using a one-pot reaction)

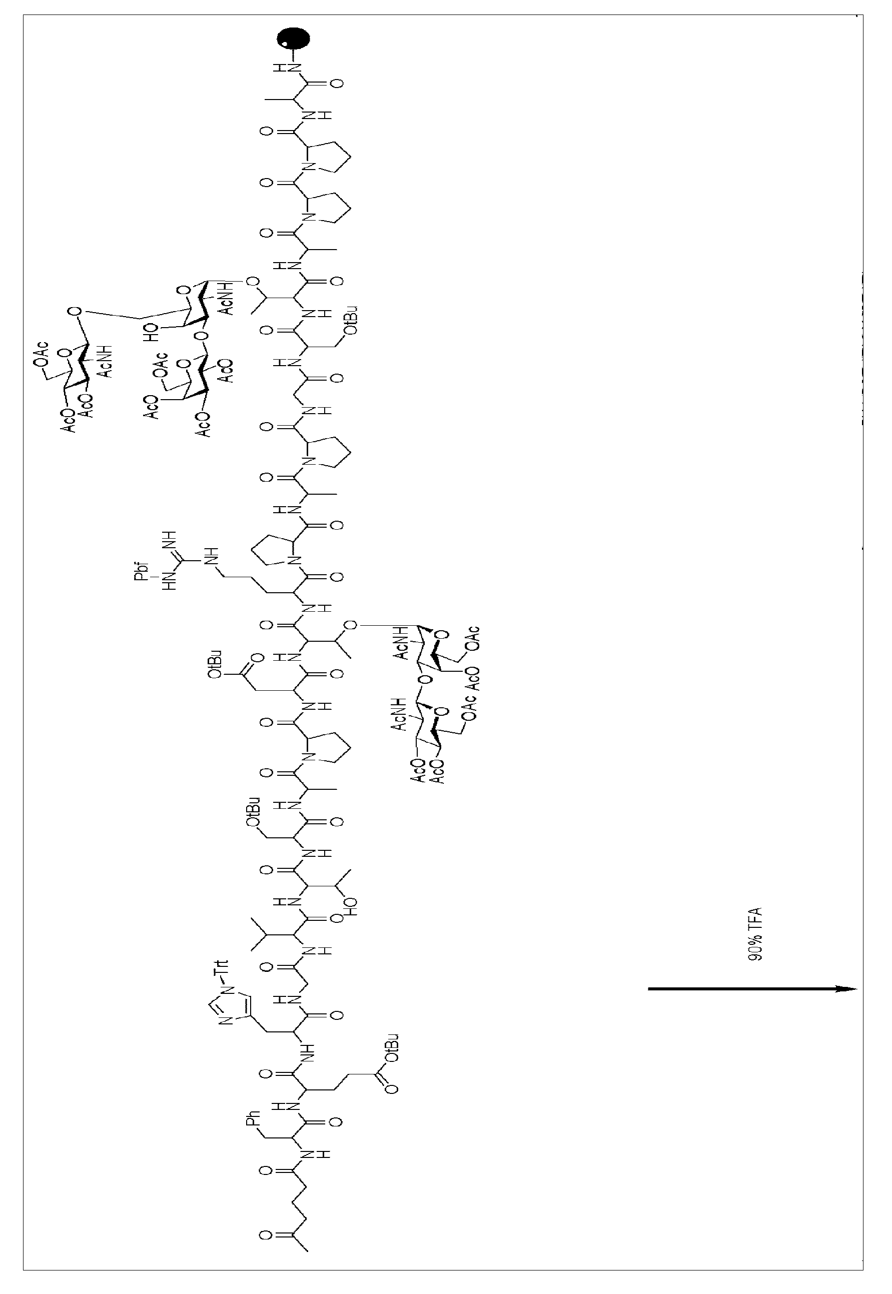

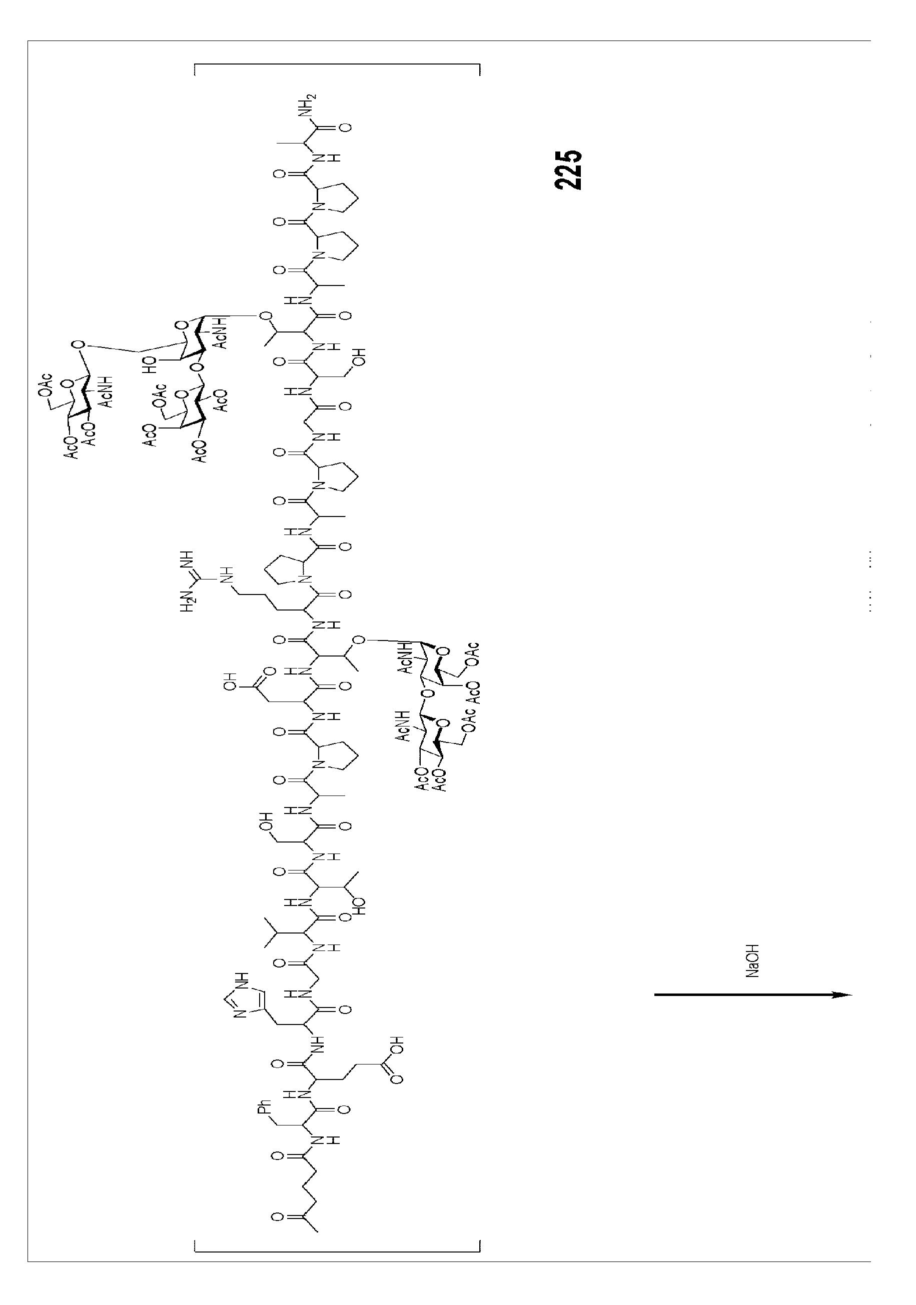

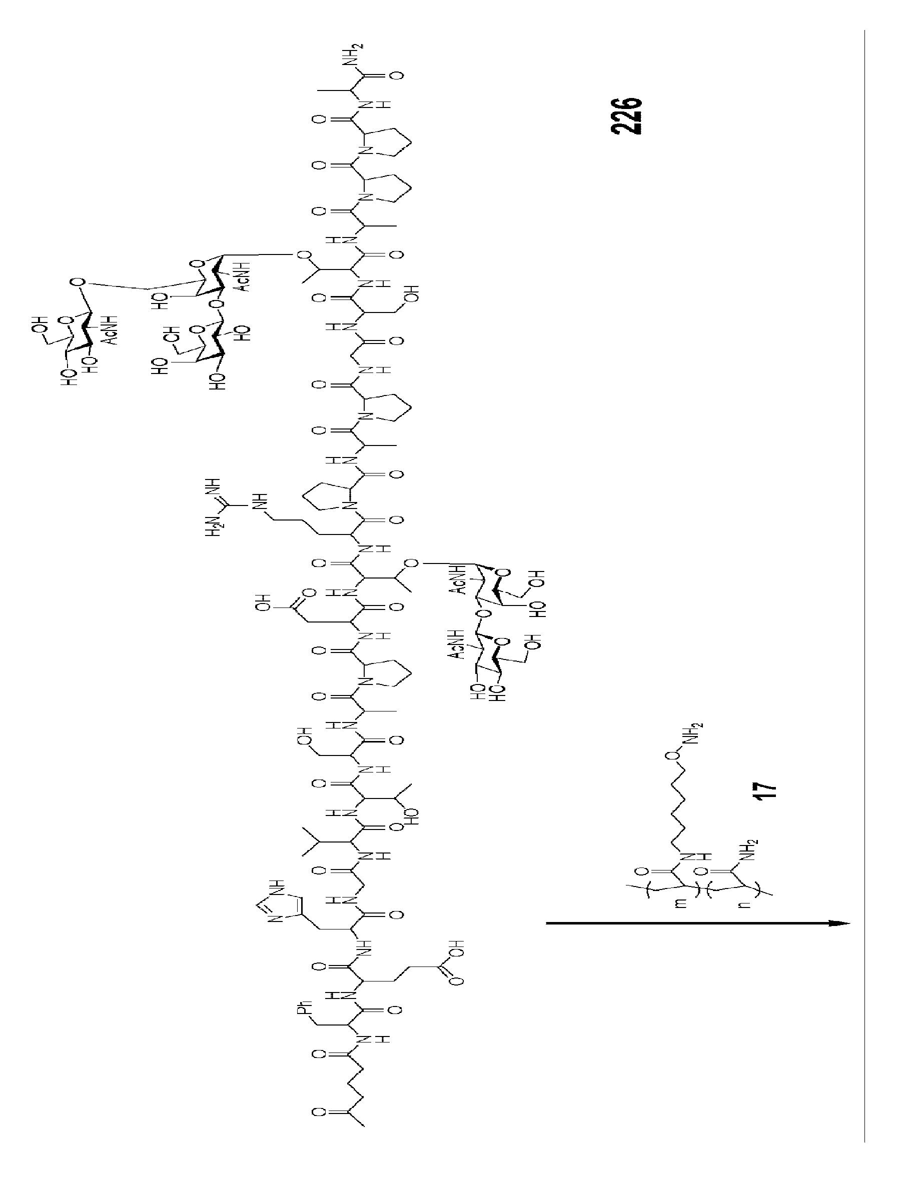

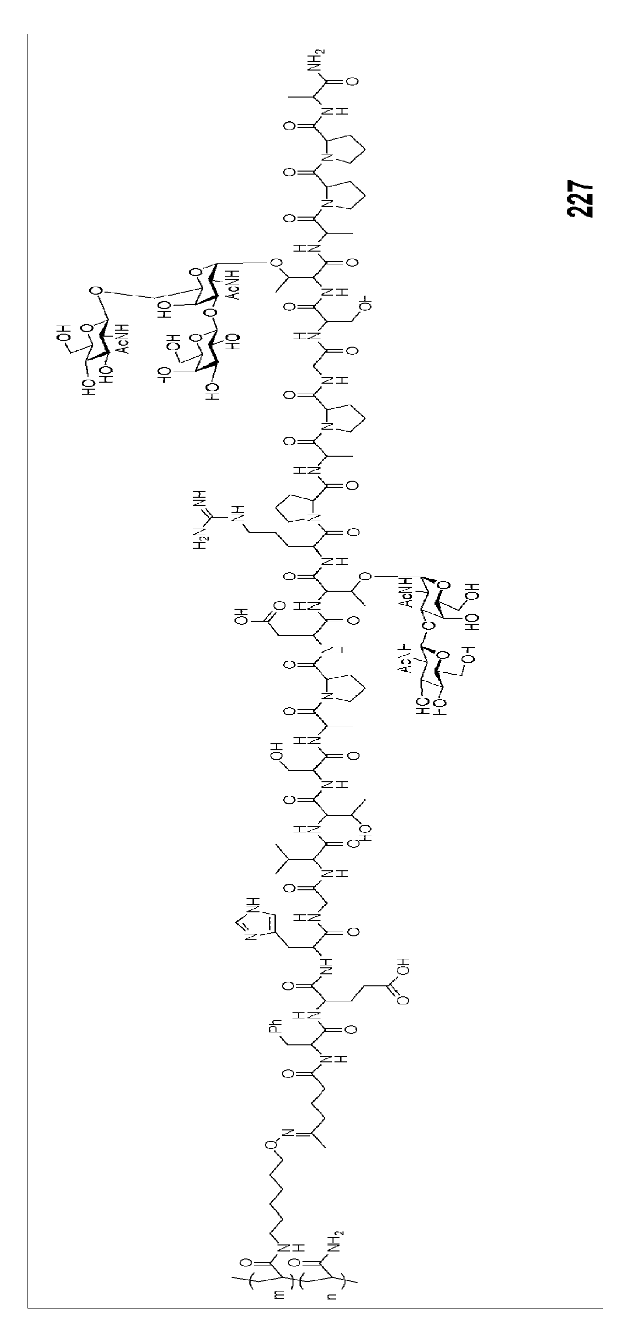

Using 71mg (0.02mmol) of Tentagel® S RAM resin (Hipep Laboratories, 0.28mmol/g) as a support, N-protected amino acids and keto acids as described below were sequentially condensed by Fmoc/HBTU/HOBt method, thereby synthesizing a glycopeptide derivative of interest: Fmoc-Ala-OH; Fmoc-Pro-OH; Fmoc-Pro-OH; Fmoc-Ala-OH; Fmoc-Thr(Ac7core2)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(Ac5core3)-OH; Fmoc-Asp(OtBu)-OH; Fmoc-Pro-OH; Fmoc-Ala-OH; Fmoc-Ser(tBu)-OH; Fmoc-Thr(tBu)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained resin equivalent to 5nmol was allowed to react in 90% TFA aqueous solution for two hours at room temperature to eliminate a protective group on a peptide residue and concurrently release compound (225) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 1.5ml of methanol. 1N sodium hydroxide aqueous solution was added to this solution, and was adjusted to have a pH of 12-12.5. The solution was then stirred for 1.5 hours at room temperature, thereby performing Ac deprotection reaction. After the reaction, 1N acetic acid was added and the solution was neutralized. Thereafter, the resin was separated by filtration. The solvent in the filtrate was removed, and the residue was dissolved in 0.5ml of 50mM acetic acid/sodium acetate buffer solution (pH 5.5). 0.5ml of 10mM (oxyamine residue calculation) water-soluble polymer (17) aqueous solution was added to the solution, and the solution was stirred for 18 hours at room temperature, thereby reacting compound (226) with compound (17) . After completion of the reaction, the reaction solution was subjected to centrifugal concentration with an ultrafiltration filter 10K Apollo ® 20ml (Orbital Biosciences, available from LIC). 25mM HEPES buffer solution (pH 7.0) was added thereto and the solution was again subjected to concentration, thereby washing the polymers. By adding water so that the final amount of the solution was 0.5ml, 10mM (theoretical content of glycopeptide) polymer (227) was obtained. Identification of the polymer (227) was performed based on that product (364) was obtained in the following section (3.63).

(3.46 Synthesis of compounds (228) to (230) using a one-pot reaction)

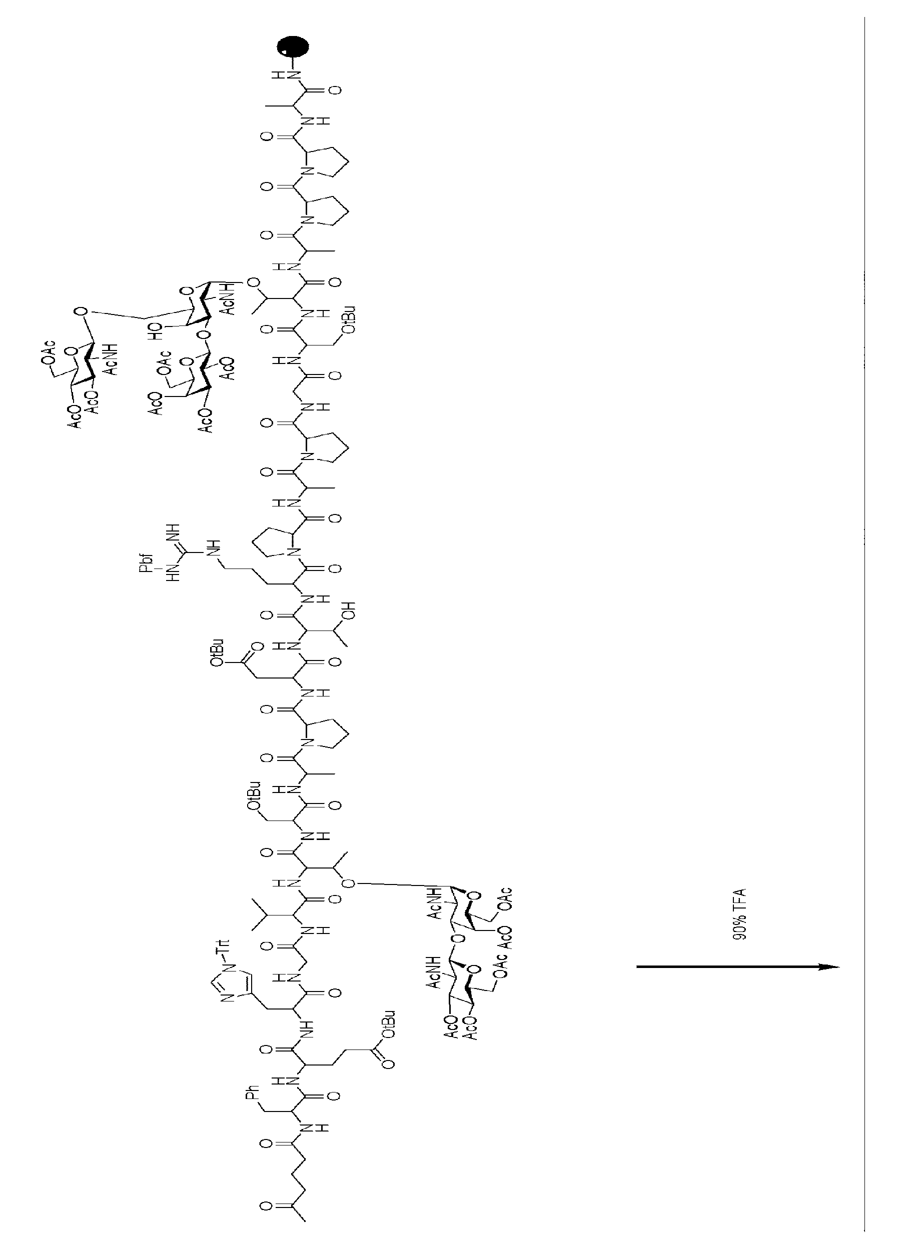

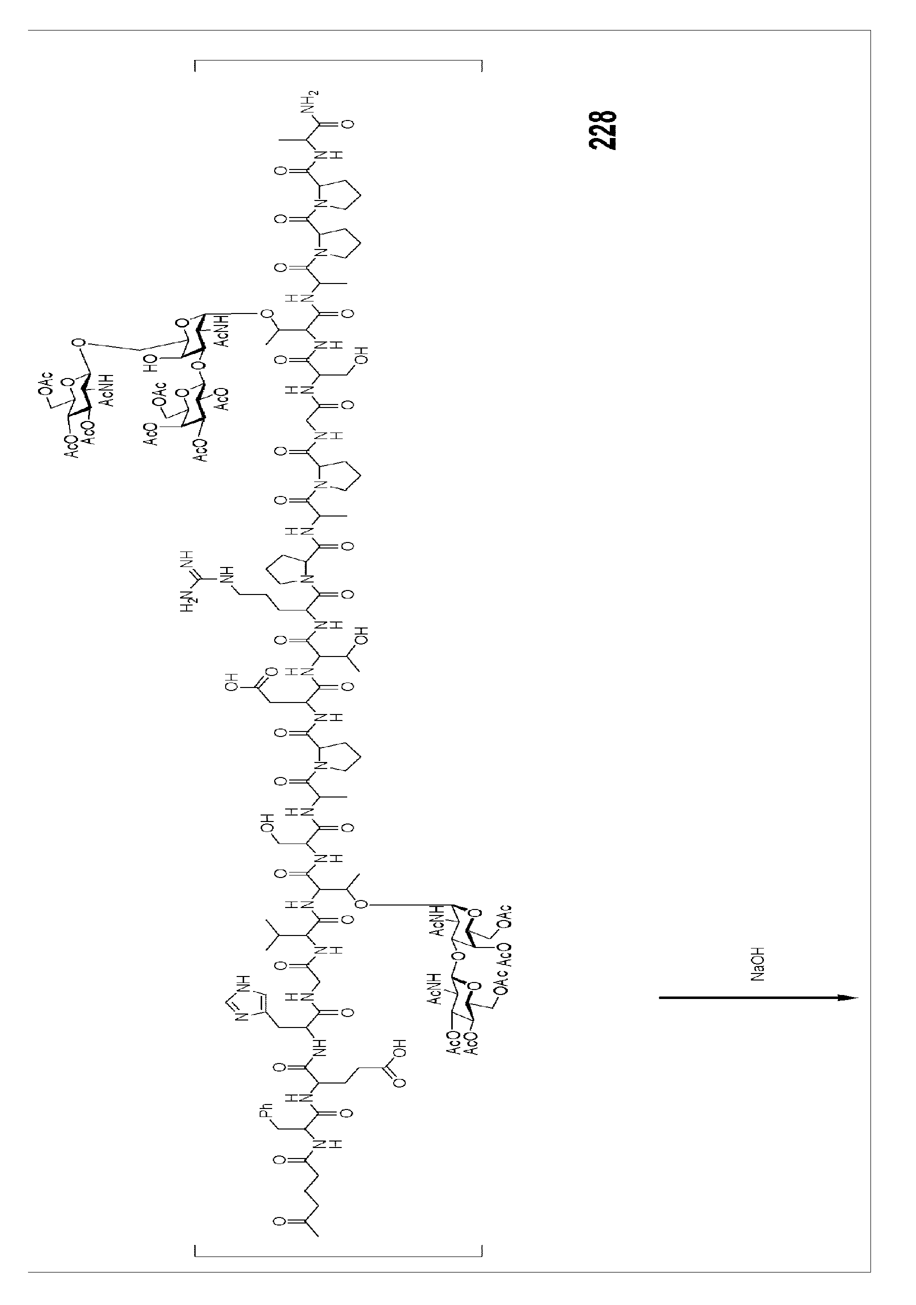

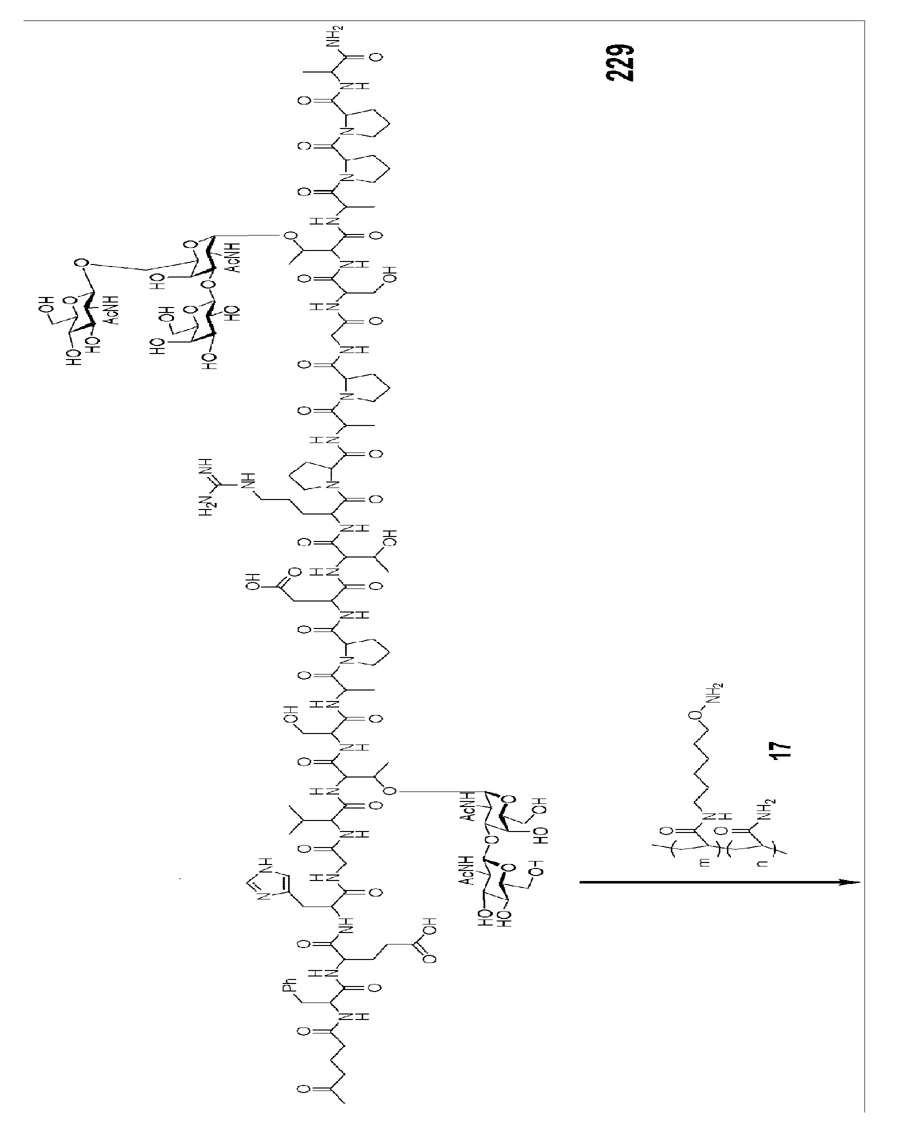

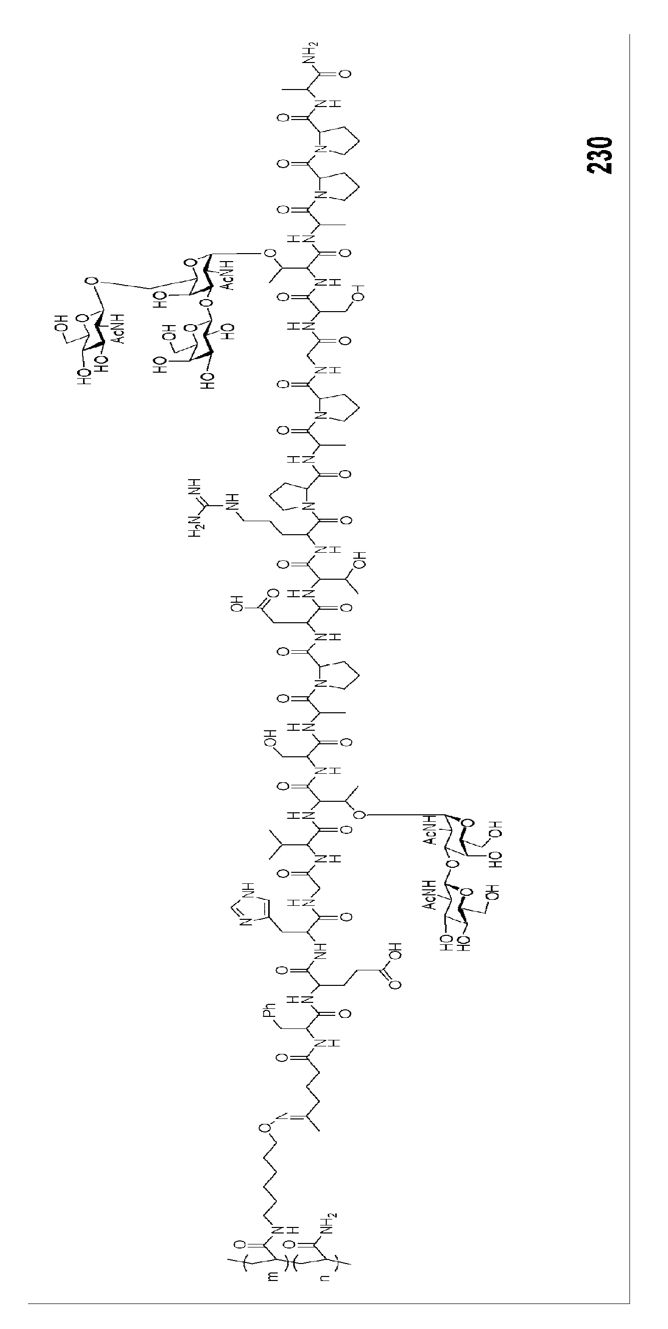

Using 71mg (0.02mmol) of Tentagel® S RAM resin (Hipep Laboratories, 0.28mmol/g) as a support, N-protected amino acids and keto acids as described below were sequentially condensed by Fmoc/HBTU/HOBt method, thereby synthesizing a glycopeptide derivative of interest: Fmoc-Ala-OH; Fmoc-Pro-OH; Fmoc-Pro-OH; Fmoc-Ala-OH; Fmoc-Thr(Ac7core2)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(tBu)-OH; Fmoc-Asp(OtBu)-OH; Fmoc-Pro-OH; Fmoc-Ala-OH; Fmoc-Ser(tBu)-OH; Fmoc-Thr(Ac5core3)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained resin equivalent to 5nmol was allowed to react in 90% TFA aqueous solution for two hours at room temperature to eliminate a protective group on a peptide residue and concurrently release compound (228) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 1.5ml of methanol. 1N sodium hydroxide aqueous solution was added to this solution, and was adjusted to have a pH of 12-12.5. The solution was then stirred for 1.5 hours at room temperature, thereby performing Ac deprotection reaction. After the reaction, 1N acetic acid was added and the solution was neutralized. Thereafter, the resin was separated by filtration. The solvent in the filtrate was removed, and the residue was dissolved in 0.5ml of 50mM acetic acid/sodium acetate buffer solution (pH 5.5). 0.5ml of 10mM (oxyamine residue calculation) water-soluble polymer (17) aqueous solution was added to the solution, and the solution was stirred for 18 hours at room temperature, thereby reacting compound (229) with compound (17). After completion of the reaction, the reaction solution was subjected to centrifugal concentration with an ultrafiltration filter 10K Apollo ® 20ml (Orbital Biosciences, available from LIC). 25mM HEPES buffer solution (pH 7.0) was added thereto and the solution was again subjected to concentration, thereby washing the polymers. By adding water so that the final amount of the solution was 0.5ml, 10mM (theoretical content of glycopeptide) polymer (230) was obtained. Identification of the polymer (230) was performed based on that product (375) was obtained in the following section (3.64).

(3.47 Synthesis of compounds (231) to (233) using a one-pot reaction)

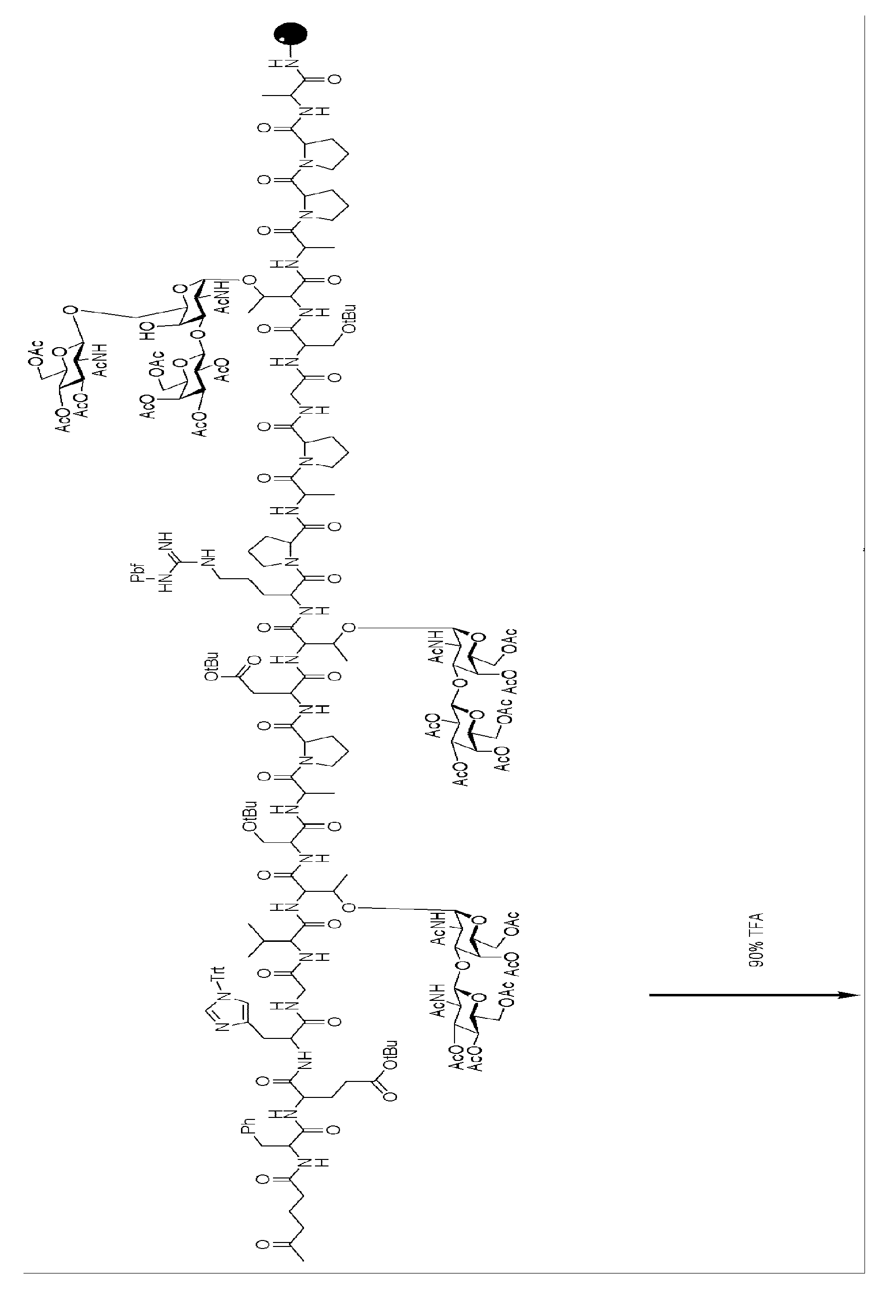

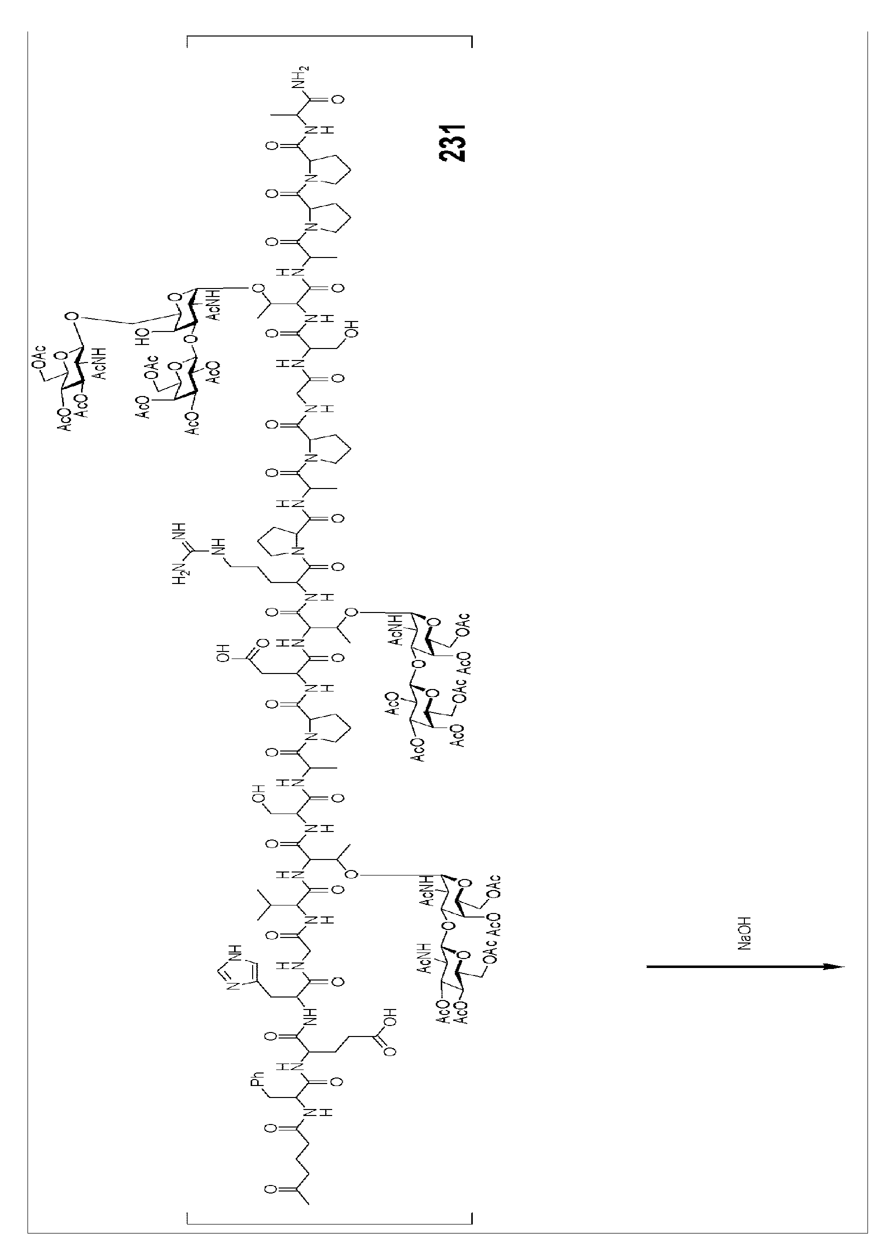

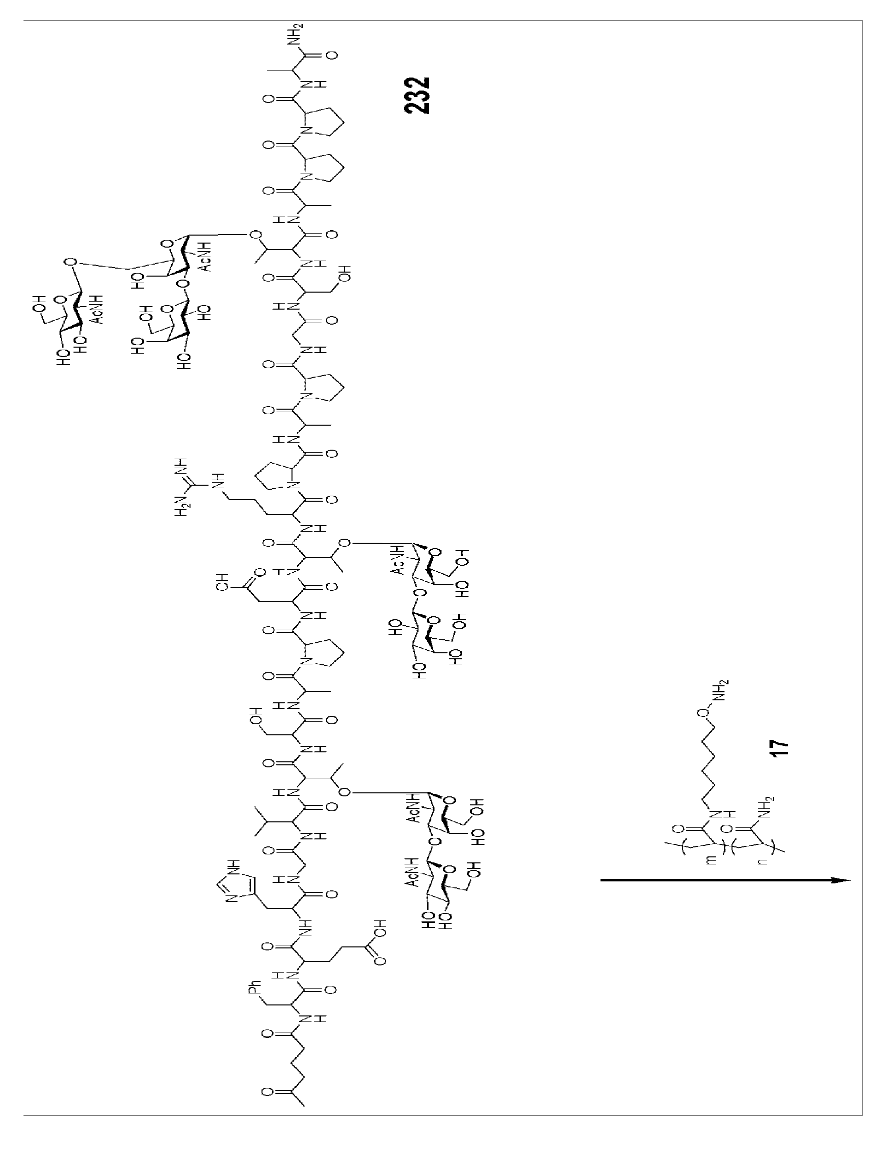

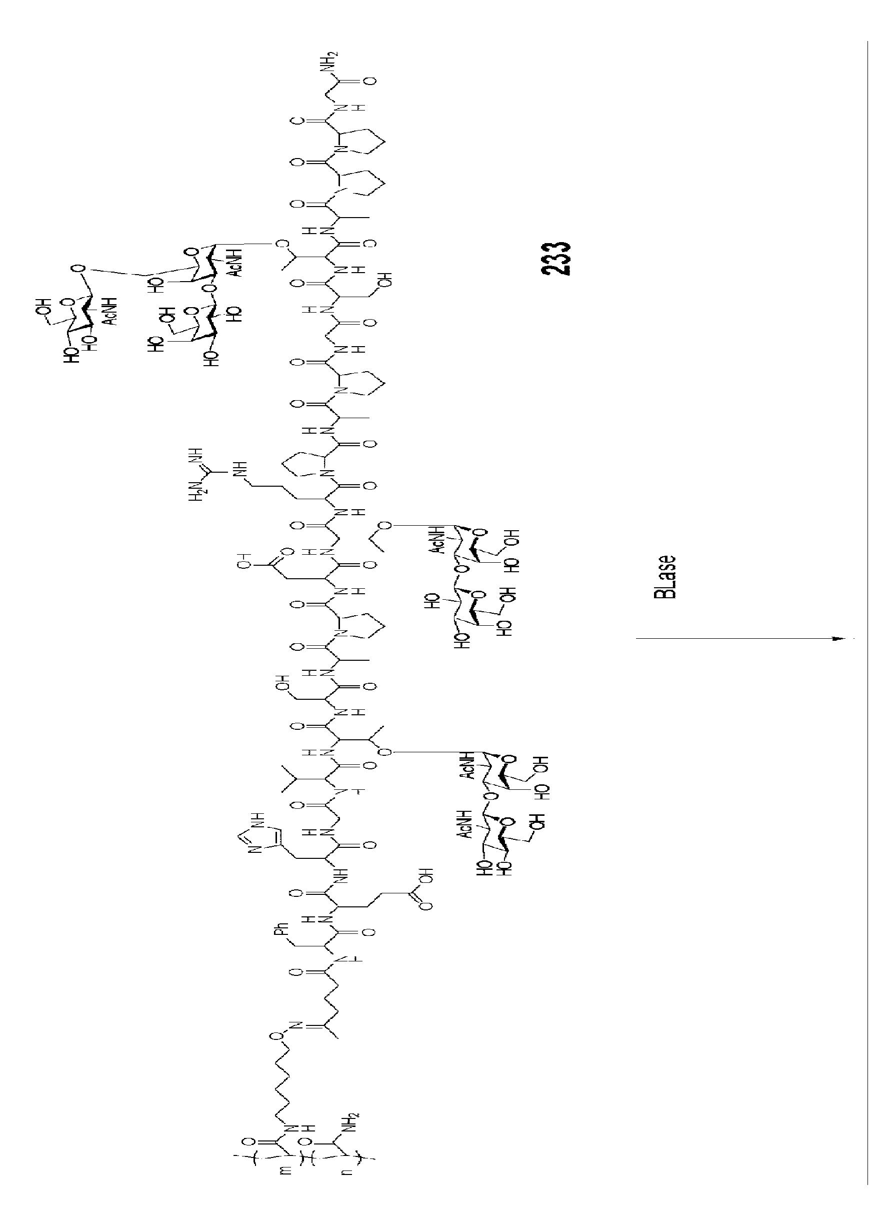

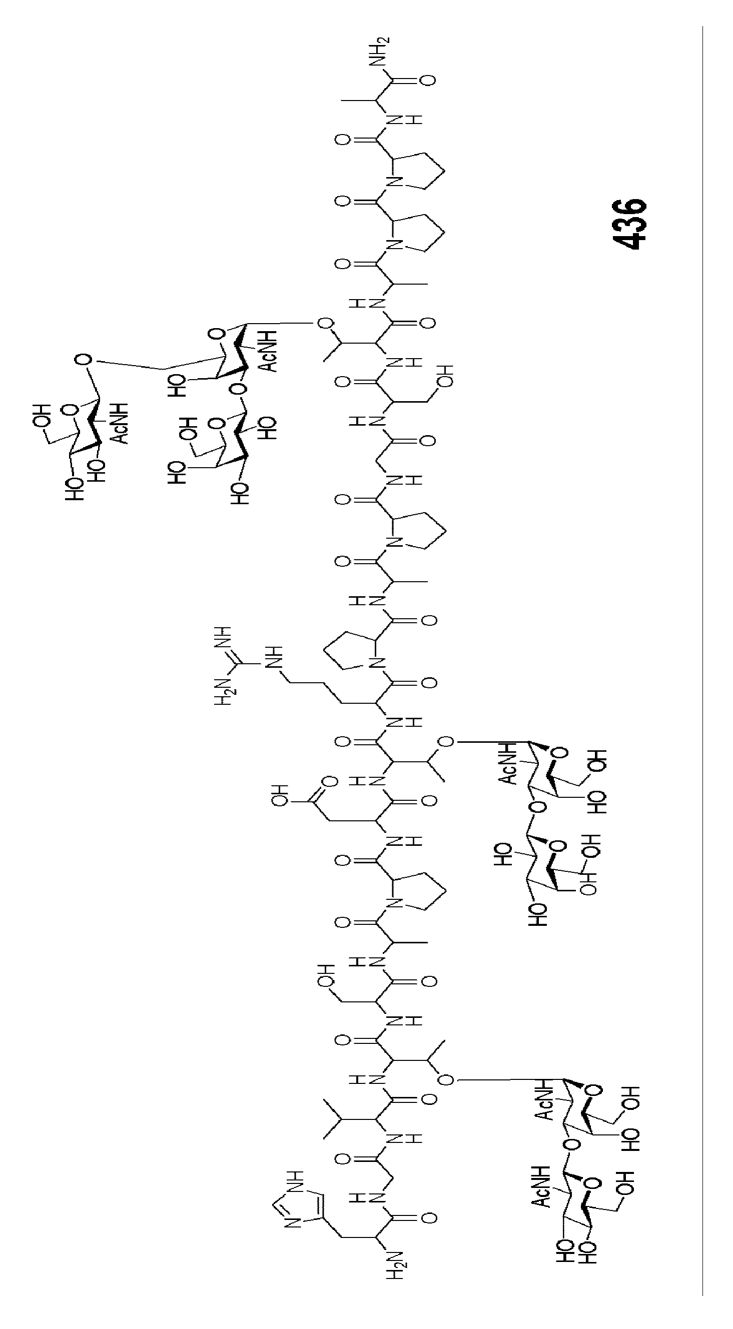

Using 71mg (0.02mmol) of Tentagel® S RAM resin (Hipep Laboratories, 0.28mmol/g) as a support, N-protected amino acids and keto acids as described below were sequentially condensed by Fmoc/HBTU/HOBt method, thereby synthesizing a glycopeptide derivative of interest: Fmoc-Ala-OH; Fmoc-Pro-OH; Fmoc-Pro-OH; Fmoc-Ala-OH; Fmoc-Thr(Ac7core2)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(Ac6corel)-OH; Fmoc-Asp(OtBu)-OH; Fmoc-Pro-OH; Fmoc-Ala-OH; Fmoc-Ser(tBu)-OH; Fmoc-Thr(Ac5core3)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained resin equivalent to 5nmol was allowed to react in 90% TFA aqueous solution for two hours at room temperature to eliminate a protective group on a peptide residue and concurrently release compound (231) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 1.5ml of methanol. 1N sodium hydroxide aqueous solution was added to this solution, and was adjusted to have a pH of 12-12.5. The solution was then stirred for 1.5 hours at room temperature, thereby performing Ac deprotection reaction. After the reaction, 1N acetic acid was added and the solution was neutralized. Thereafter, the resin was separated by filtration. The solvent in the filtrate was removed, and the residue was dissolved in 0.5ml of 50mM acetic acid/sodium acetate buffer solution (pH 5.5). 0.5ml of 10mM (oxyamine residue calculation) water-soluble polymer (17) aqueous solution was added to the solution, and the solution was stirred for 18 hours at room temperature, thereby reacting compound (232) with compound (17). After completion of the reaction, the reaction solution was subjected to centrifugal concentration with an ultrafiltration filter 10K Apollo ® 20ml (Orbital Biosciences, available from LIC). 25mM HEPES buffer solution (pH 7.0) was added thereto and the solution was again subjected to concentration, thereby washing the polymers. By adding water so that the final amount of the solution was 0.5ml, 10mM (theoretical content of glycopeptide) polymer (233) was obtained. Identification of the polymer (233) was performed by treating a part of polymer (233) with BLase to obtain product (436). MALDI-TOF/MS:[M(average)+H]⁺ of compound (436)=3228.1 (theoretical value: [M(average)+H]+=3227.3).

(3.48 Synthesis of compounds (234) to (236) using a one-pot reaction)

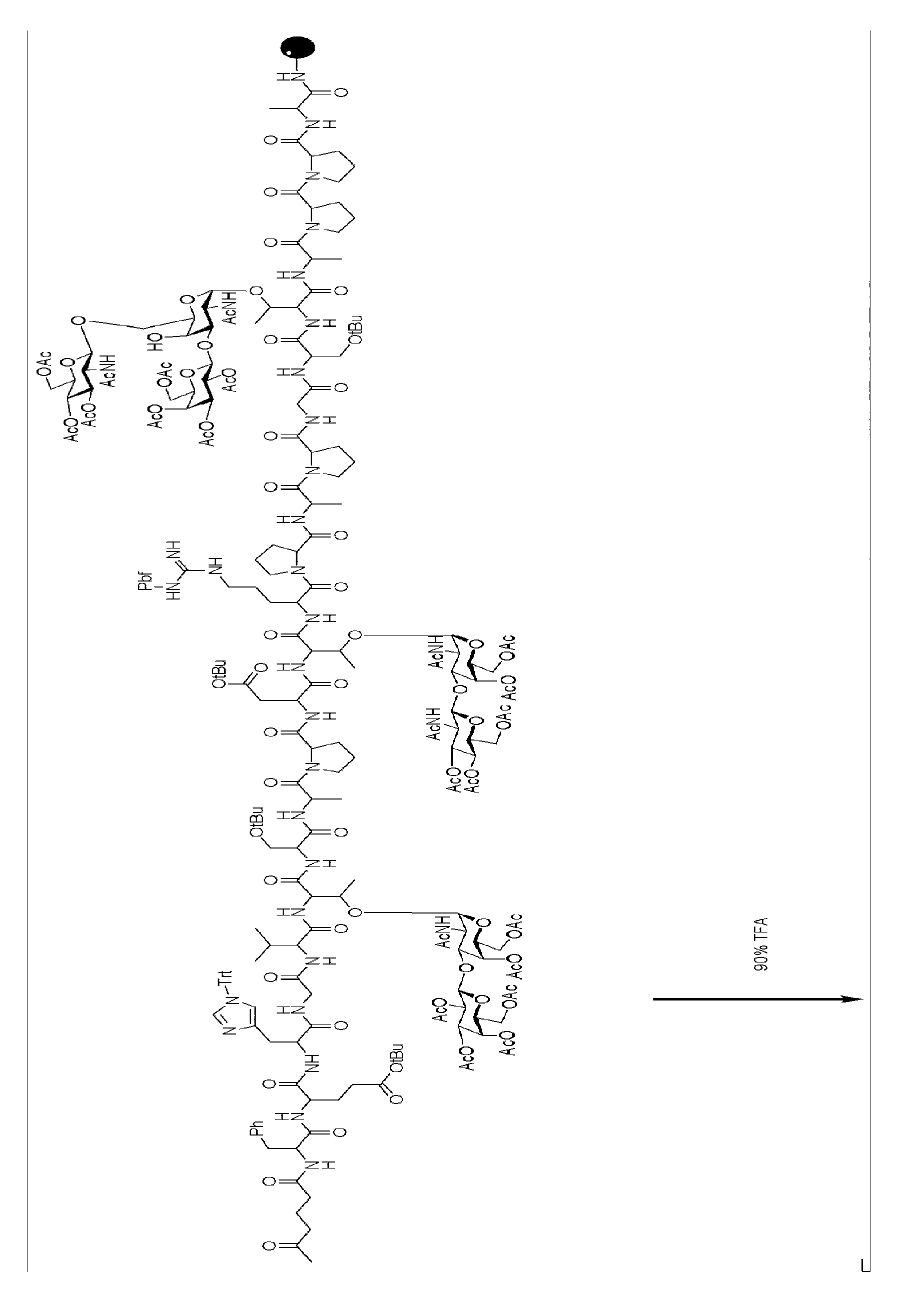

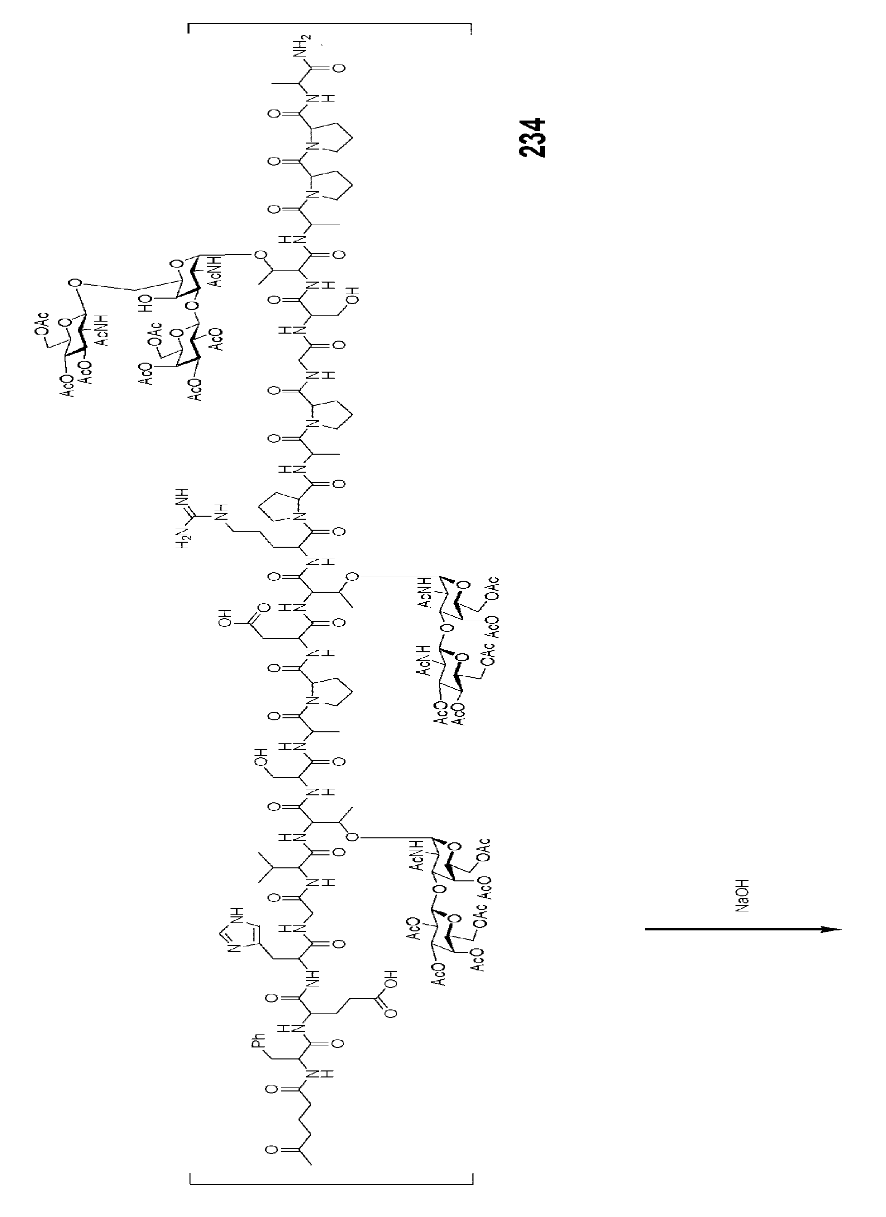

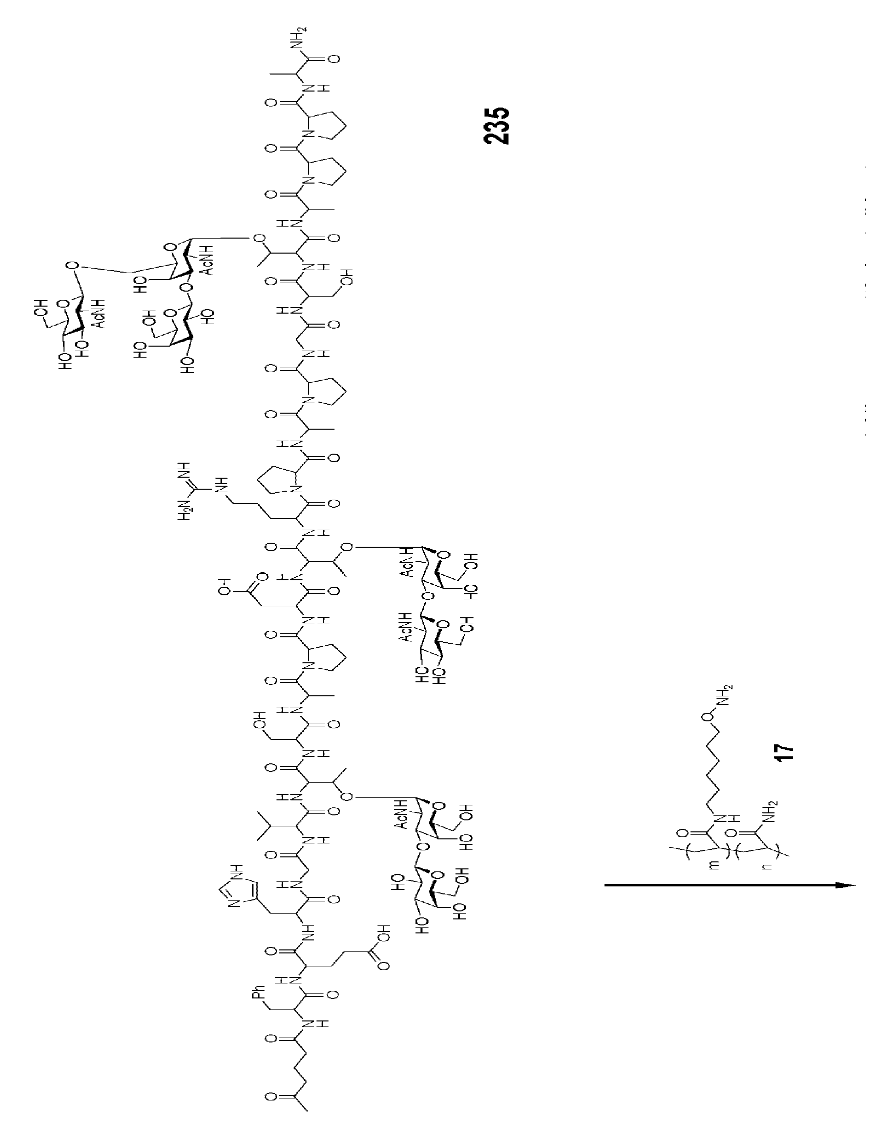

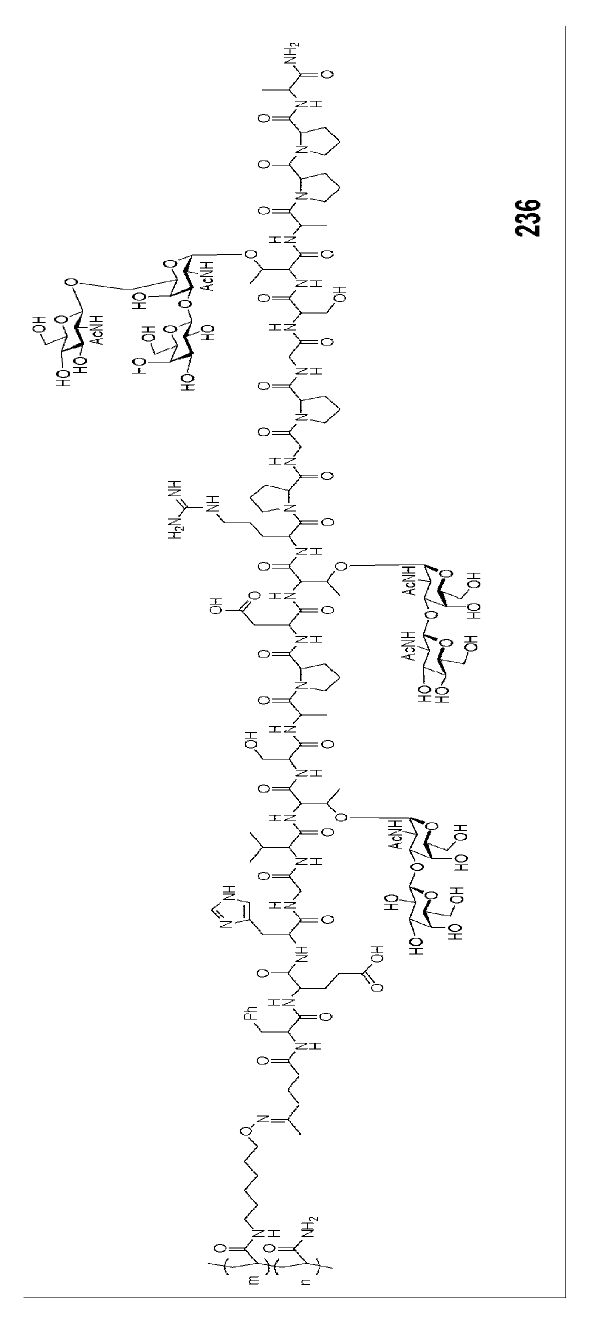

Using 71mg (0.02mmol) of Tentagel® S RAM resin (Hipep Laboratories, 0.28mmol/g) as a support, N-protected amino acids and keto acids as described below were sequentially condensed by Fmoc/HBTU/HOBt method, thereby synthesizing a glycopeptide derivative of interest: Fmoc-Ala-OH; Fmoc-Pro-OH; Fmoc-Pro-OH; Fmoc-Ala-OH; Fmoc-Thr(Ac7core2)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(Ac5core3)-OH; Fmoc-Asp(OtBu)-OH; Fmoc-Pro-OH; Fmoc-Ala-OH; Fmoc-Ser(tBu)-OH; Fmoc-Thr(Ac6core1)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained resin equivalent to 5nmol was allowed to react in 90% TFA aqueous solution for two hours at room temperature to eliminate a protective group on a peptide residue and concurrently release compound (234) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 1.5ml of methanol. 1N sodium hydroxide aqueous solution was added to this solution, and was adjusted to have a pH of 12-12.5. The solution was then stirred for 1.5 hours at room temperature, thereby performing Ac deprotection reaction. After the reaction, 1N acetic acid was added and the solution was neutralized. Thereafter, the resin was separated by filtration. The solvent in the filtrate was removed, and the residue was dissolved in 0.5ml of 50mM acetic acid/sodium acetate buffer solution (pH 5.5). 0.5ml of 10mM (oxyamine residue calculation) water-soluble polymer (17) aqueous solution was added to the solution, and the solution was stirred for 18 hours at room temperature, thereby reacting compound (235) with compound (17). After completion of the reaction, the reaction solution was subjected to centrifugal concentration with an ultrafiltration filter 10K Apollo ® 20ml (Orbital Biosciences, available from LIC). 25mM HEPES buffer solution (pH 7.0) was added thereto and the solution was again subjected to concentration, thereby washing the polymers. By adding water so that the final amount of the solution was 0.5ml, 10mM (theoretical content of glycopeptide) polymer (236) was obtained. Identification of the polymer (236) was performed based on that product (386) was obtained in the following section (3.65).

(3.49 Synthesis of compounds (237) to (239) using a one-pot reaction)

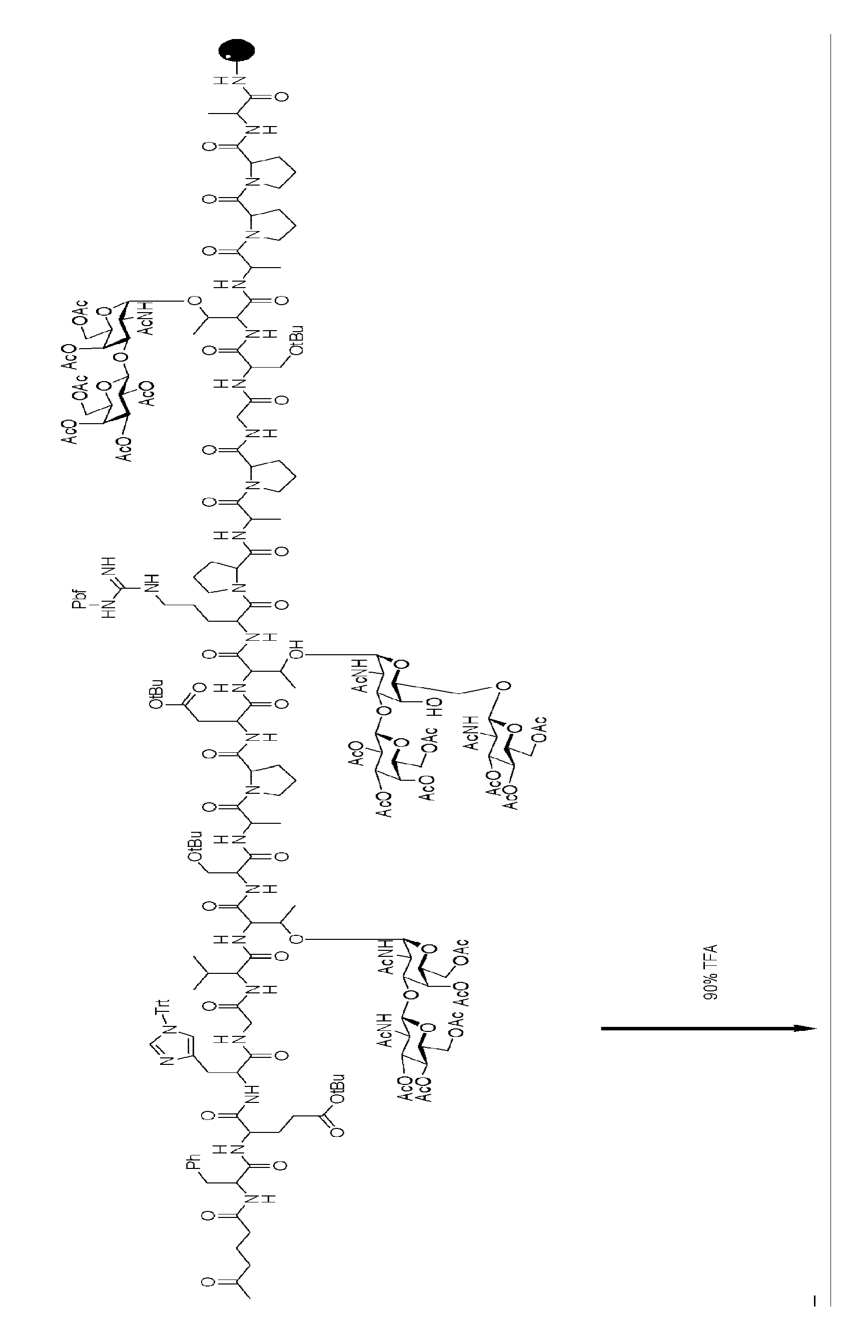

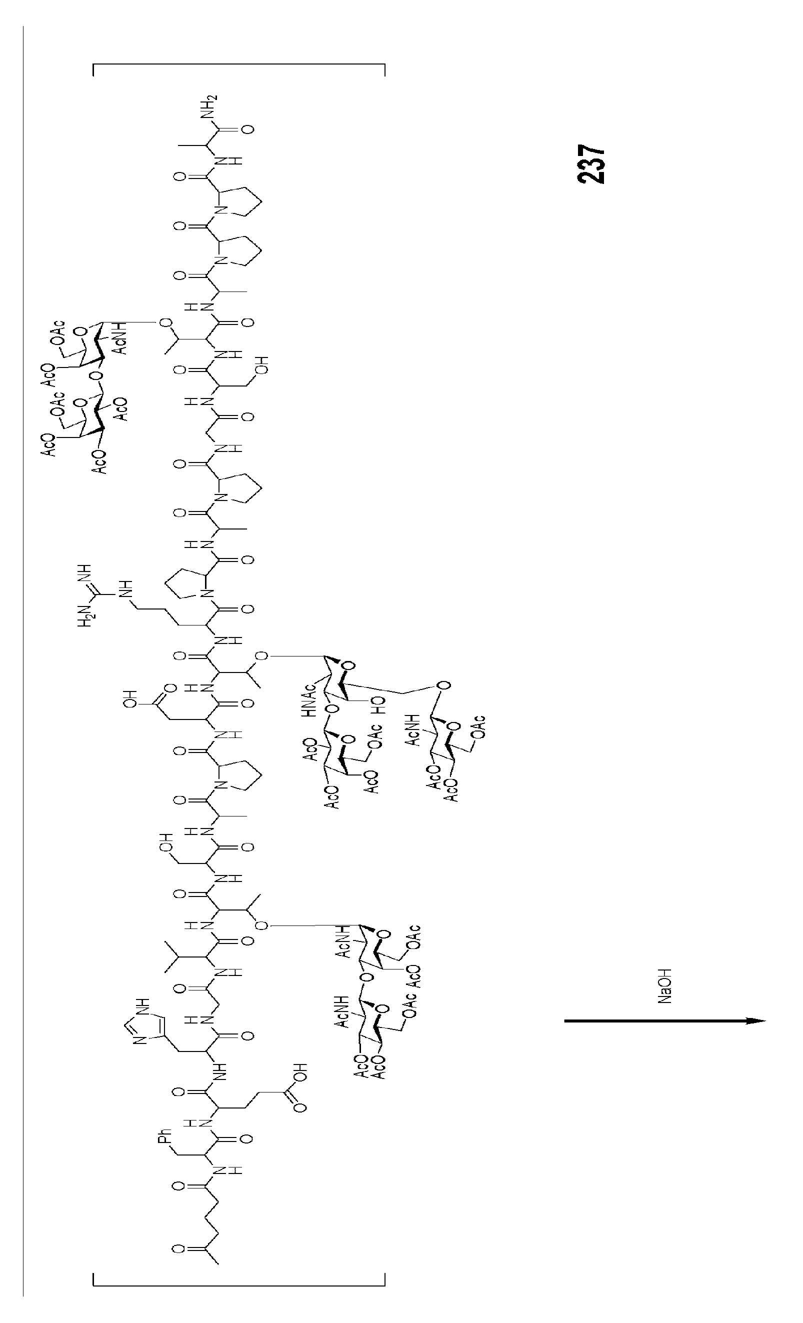

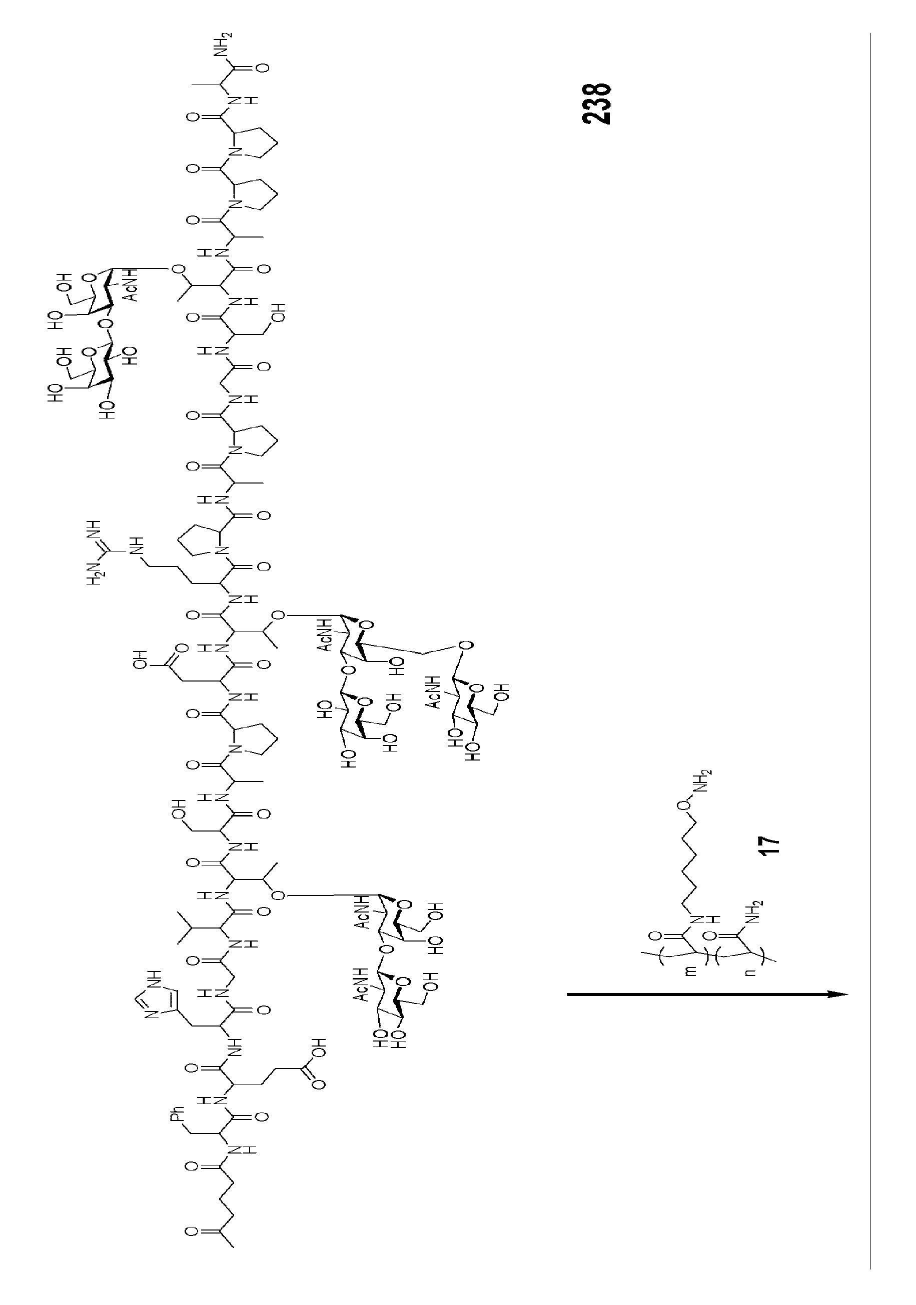

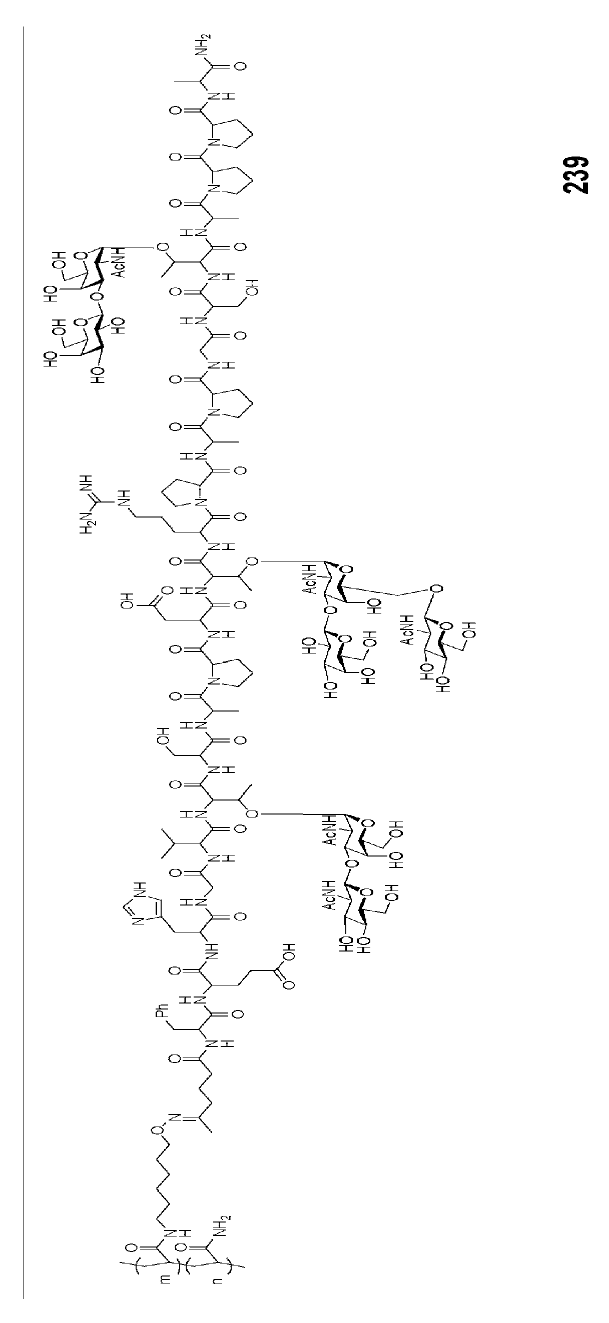

Using 71mg (0.02mmol) of Tentagel® S RAM resin (Hipep Laboratories, 0.28mmol/g) as a support, N-protected amino acids and keto acids as described below were sequentially condensed by Fmoc/HBTU/HOBt method, thereby synthesizing a glycopeptide derivative of interest: Fmoc-Ala-OH; Fmoc-Pro-OH; Fmoc-Pro-OH; Fmoc-Ala-OH; Fmoc-Thr(Ac6core1)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(Ac7core2)-OH; Fmoc-Asp(OtBu)-OH; Fmoc-Pro-OH; Fmoc-Ala-OH; Fmoc-Ser(tBu)-OH; Fmoc-Thr(Ac5core3)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained resin equivalent to 5nmol was allowed to react in 90% TFA aqueous solution for two hours at room temperature to eliminate a protective group on a peptide residue and concurrently release compound (237) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 1.5ml of methanol. 1N sodium hydroxide aqueous solution was added to this solution, and was adjusted to have a pH of 12-12.5. The solution was then stirred for 1.5 hours at room temperature, thereby performing Ac deprotection reaction. After the reaction, 1N acetic acid was added and the solution was neutralized. Thereafter, the resin was separated by filtration. The solvent in the filtrate was removed, and the residue was dissolved in 0.5ml of 50mM acetic acid/sodium acetate buffer solution (pH 5.5). 0.5ml of 10mM (oxyamine residue calculation) water-soluble polymer (17) aqueous solution was added to the solution, and the solution was stirred for 18 hours at room temperature, thereby reacting compound (238) with compound (17). After completion of the reaction, the reaction solution was subjected to centrifugal concentration with an ultrafiltration filter 10K Apollo ® 20ml (Orbital Biosciences, available from LIC). 25mM HEPES buffer solution (pH 7.0) was added thereto and the solution was again subjected to concentration, thereby washing the polymers. By adding water so that the final amount of the solution was 0.5ml, 10mM (theoretical content of glycopeptide) polymer (239) was obtained. Identification of the polymer (239) was performed based on that product (397) was obtained in the following section (3.66).

(3.50 Synthesis of compounds (240) to (242) using a one-pot reaction)

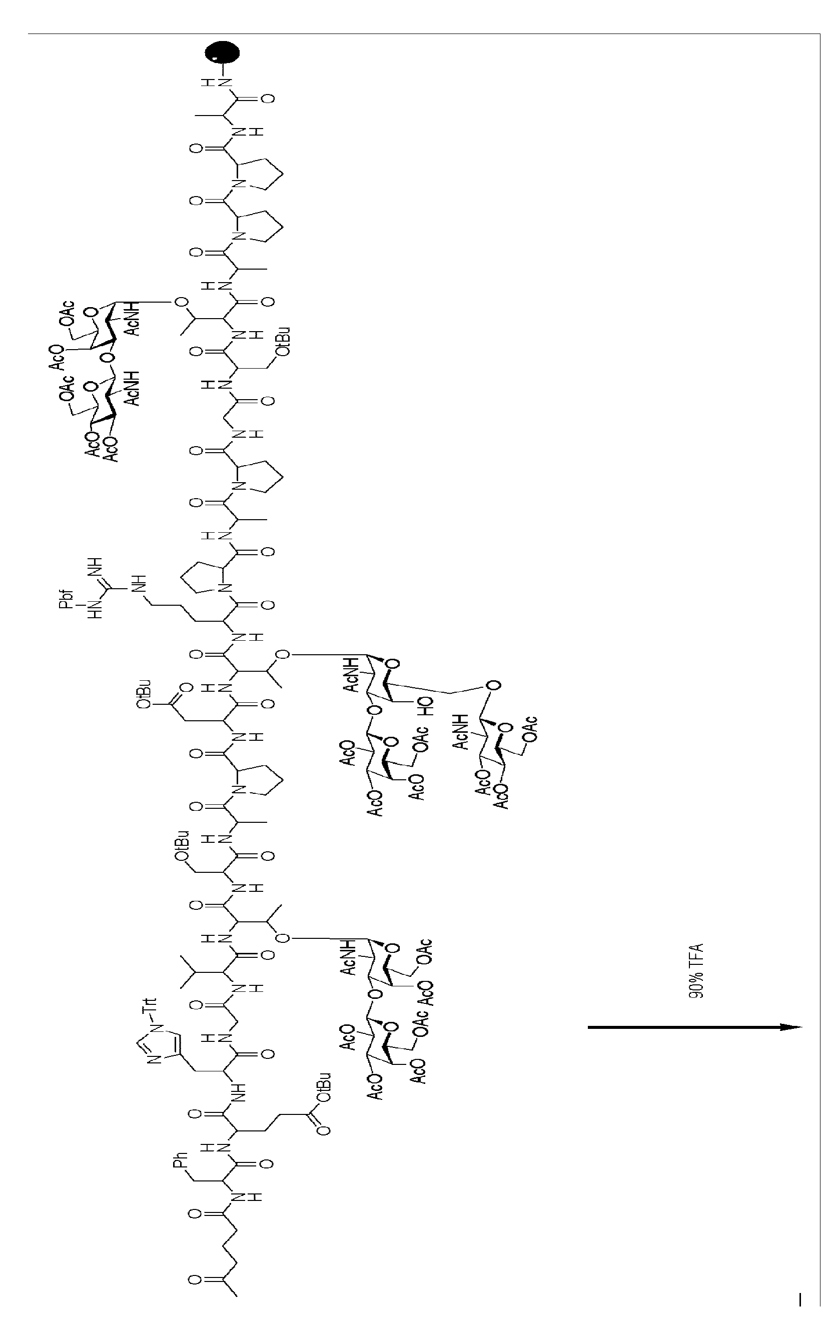

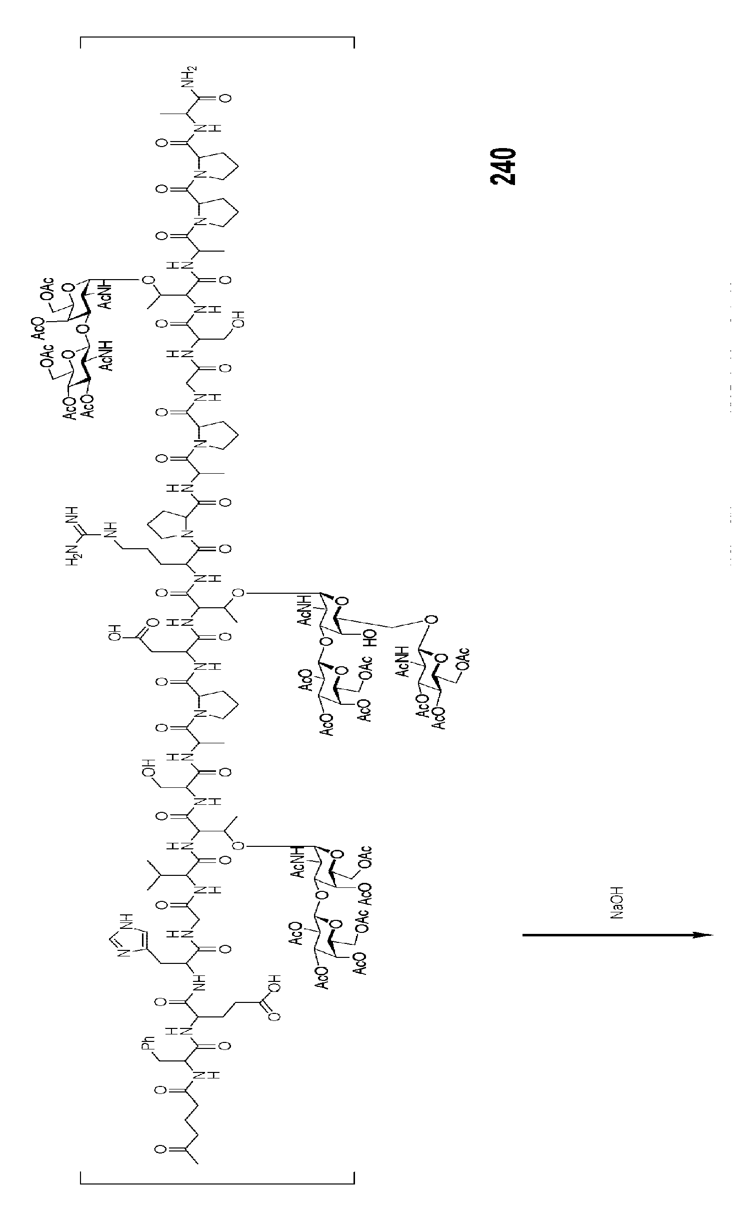

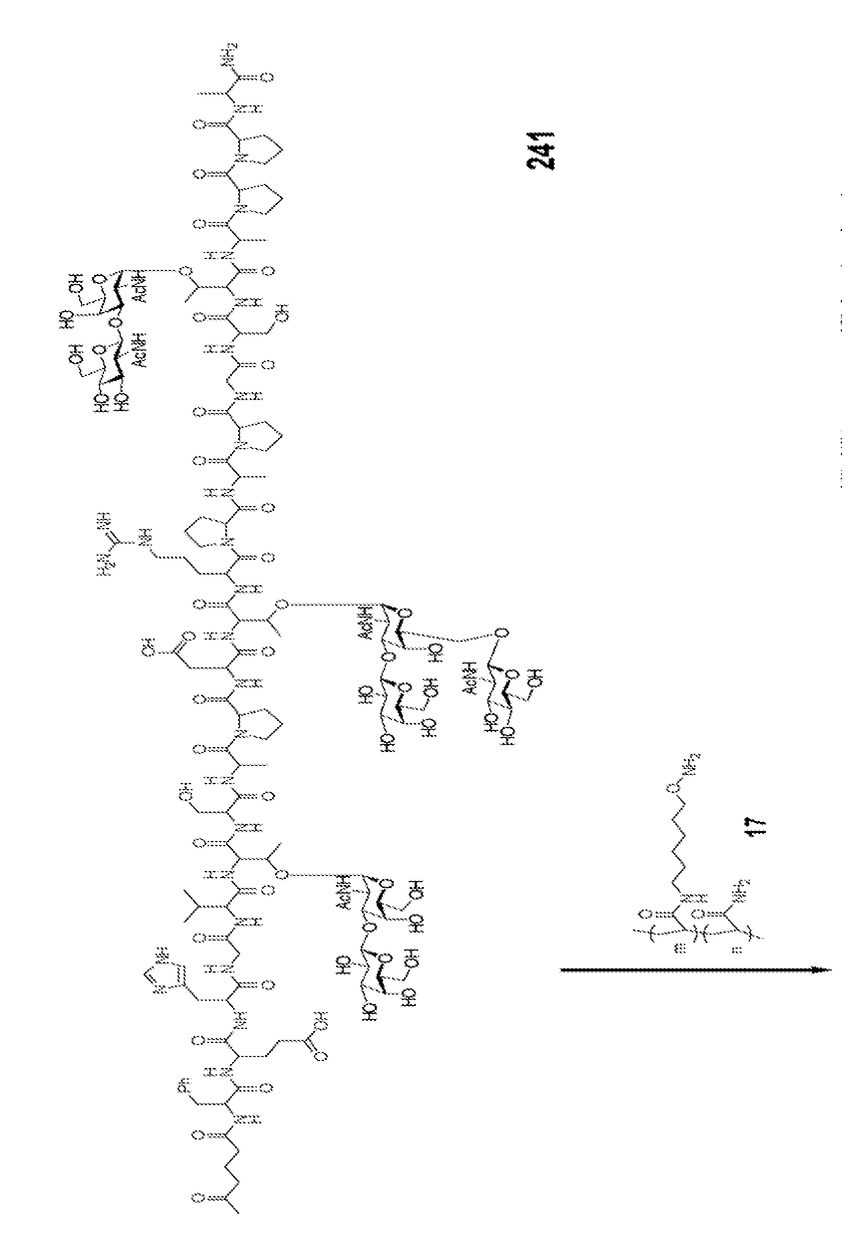

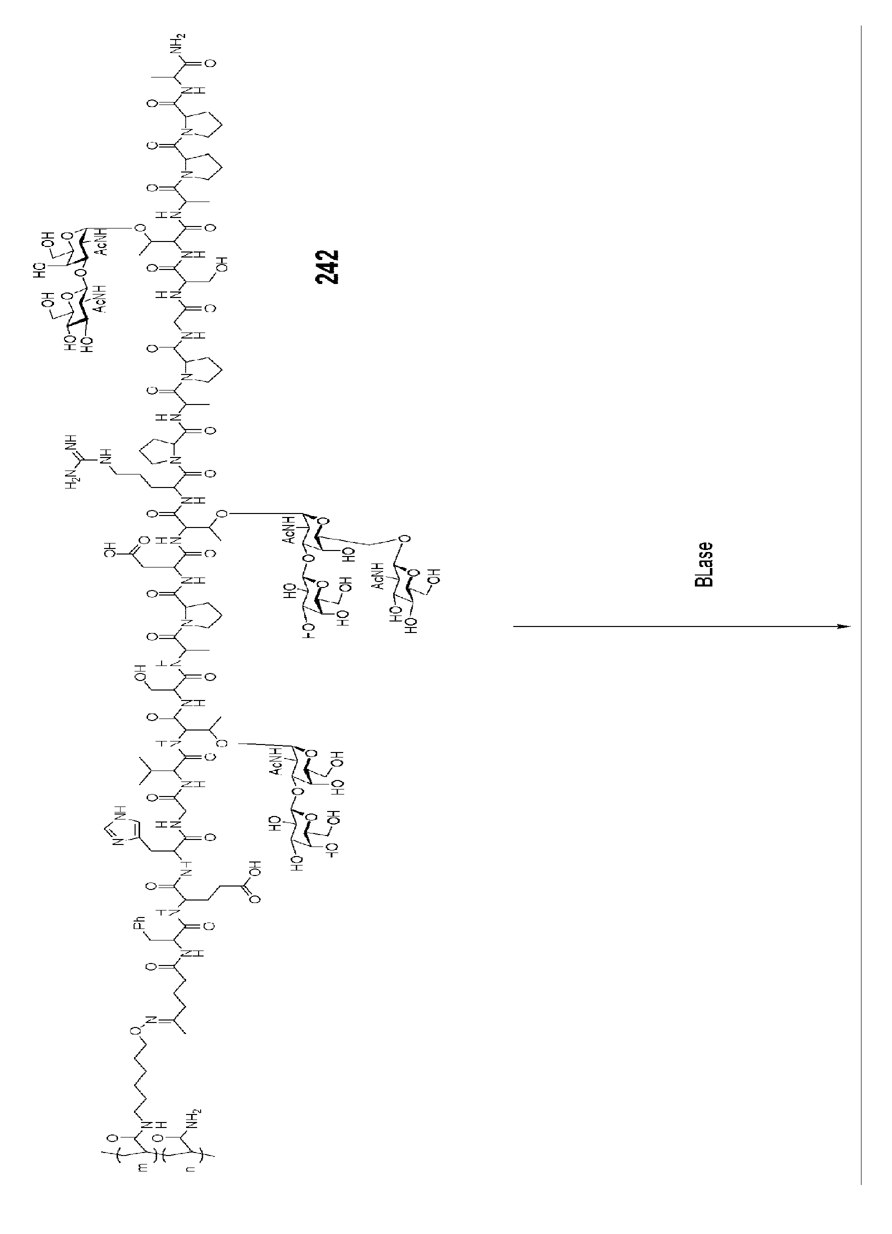

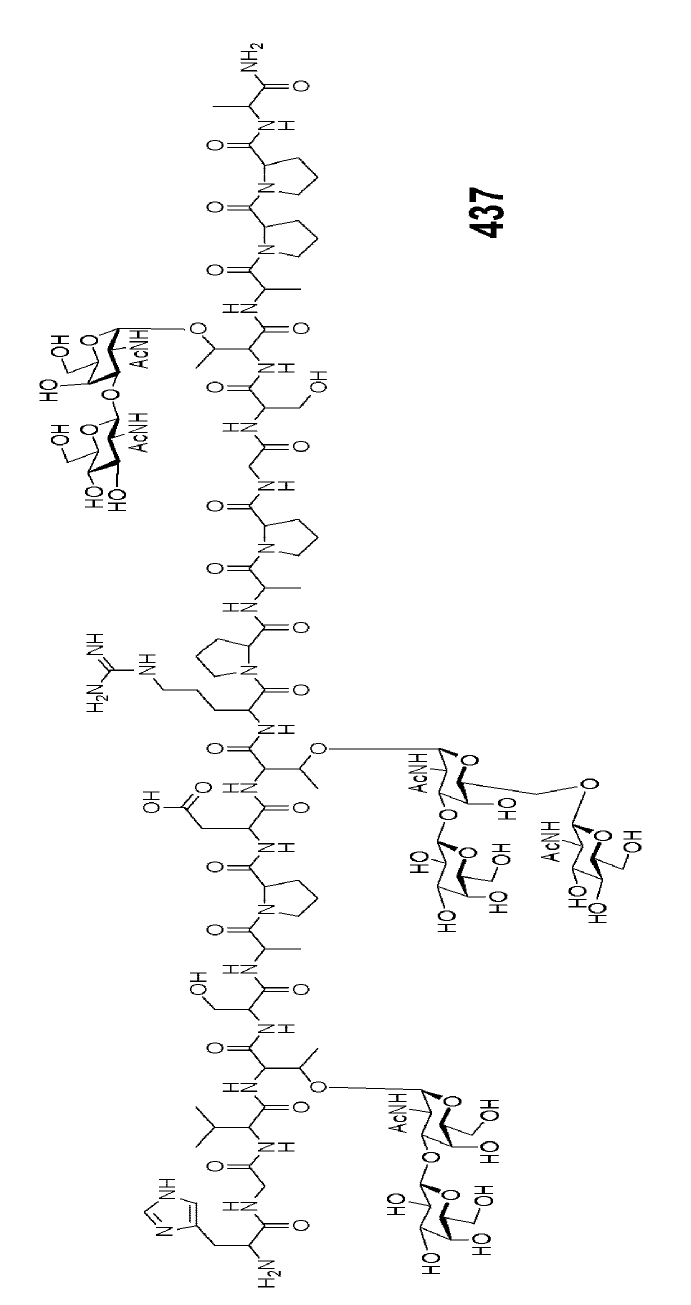

Using 71mg (0.02mmol) of Tentagel® S RAM resin (Hipep Laboratories, 0.28mmol/g) as a support, N-protected amino acids and keto acids as described below were sequentially condensed by Fmoc/HBTU/HOBt method, thereby synthesizing a glycopeptide derivative of interest: Fmoc-Ala-OH; Fmoc-Pro-OH; Fmoc-Pro-OH; Fmoc-Ala-OH; Fmoc-Thr(Ac5core3)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(Ac7core2)-OH; Fmoc-Asp(OtBu)-OH; Fmoc-Pro-OH; Fmoc-Ala-OH; Fmoc-Ser(tBu)-OH; Fmoc-Thr(Ac6corel)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained resin equivalent to 5nmol was allowed to react in 90% TFA aqueous solution for two hours at room temperature to eliminate a protective group on a peptide residue and concurrently release compound (240) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 1.5ml of methanol. 1N sodium hydroxide aqueous solution was added to this solution, and was adjusted to have a pH of 12-12.5. The solution was then stirred for 1.5 hours at room temperature, thereby performing Ac deprotection reaction. After the reaction, 1N acetic acid was added and the solution was neutralized. Thereafter, the resin was separated by filtration. The solvent in the filtrate was removed, and the residue was dissolved in 0.5ml of 50mM acetic acid/sodium acetate buffer solution (pH 5.5). 0.5ml of 10mM (oxyamine residue calculation) water-soluble polymer (17) aqueous solution was added to the solution, and the solution was stirred for 18 hours at room temperature, thereby reacting compound (241) with compound (17). After completion of the reaction, the reaction solution was subjected to centrifugal concentration with an ultrafiltration filter 10K Apollo 0 20ml (Orbital Biosciences, available from LIC). 25mM HEPES buffer solution (pH 7.0) was added thereto and the solution was again subjected to concentration, thereby washing the polymers. By adding water so that the final amount of the solution was 0.5ml, 10mM (theoretical content of glycopeptide) polymer (242) was obtained. Identification of the polymer (242) was performed by treating a part of polymer (242) with BLase to obtain product (437). MALDI-TOF/MS:[M(average)+H]⁺ of compound (437)=3228.0 (theoretical value: [M(average)+H]+=3227.3).

(3.51 Synthesis of compounds (243) to (245) using a one-pot reaction)

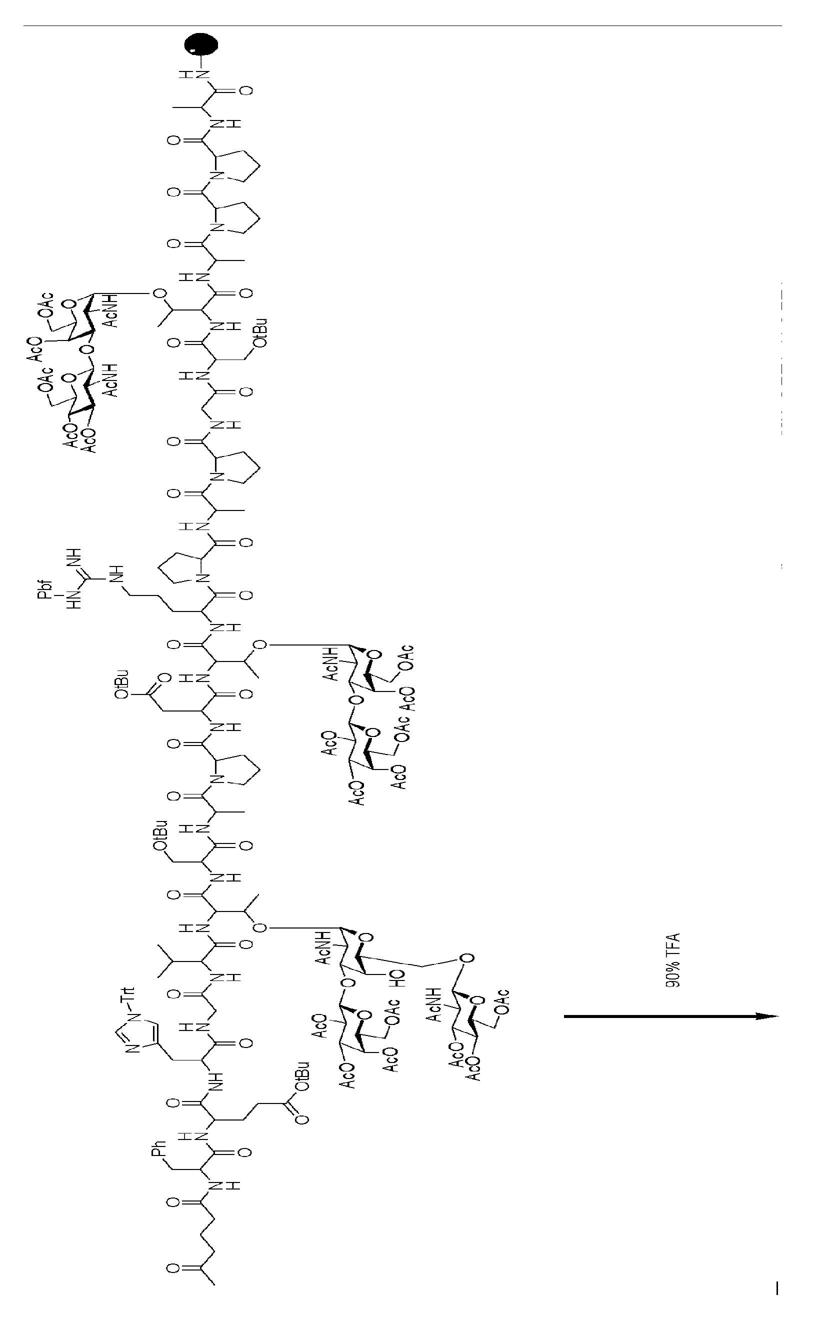

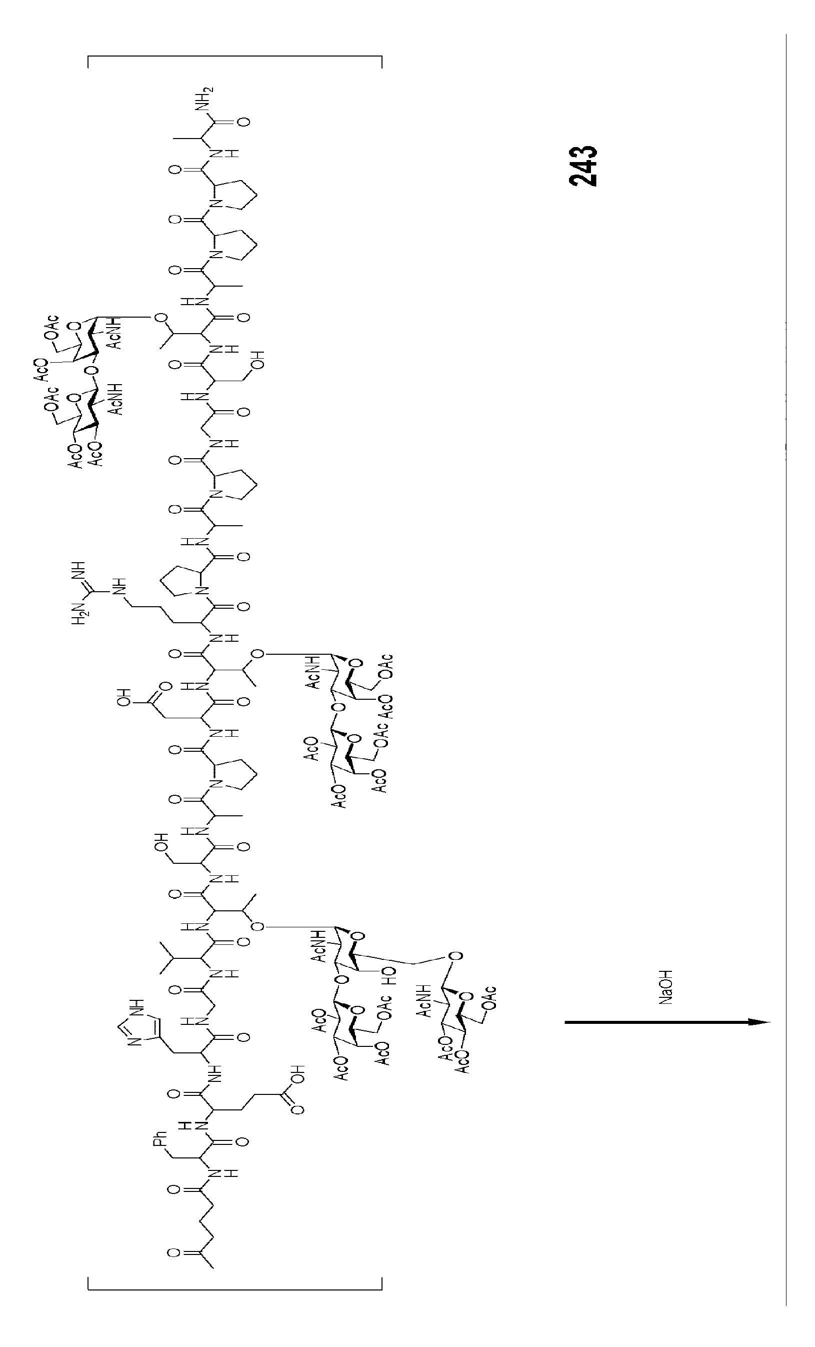

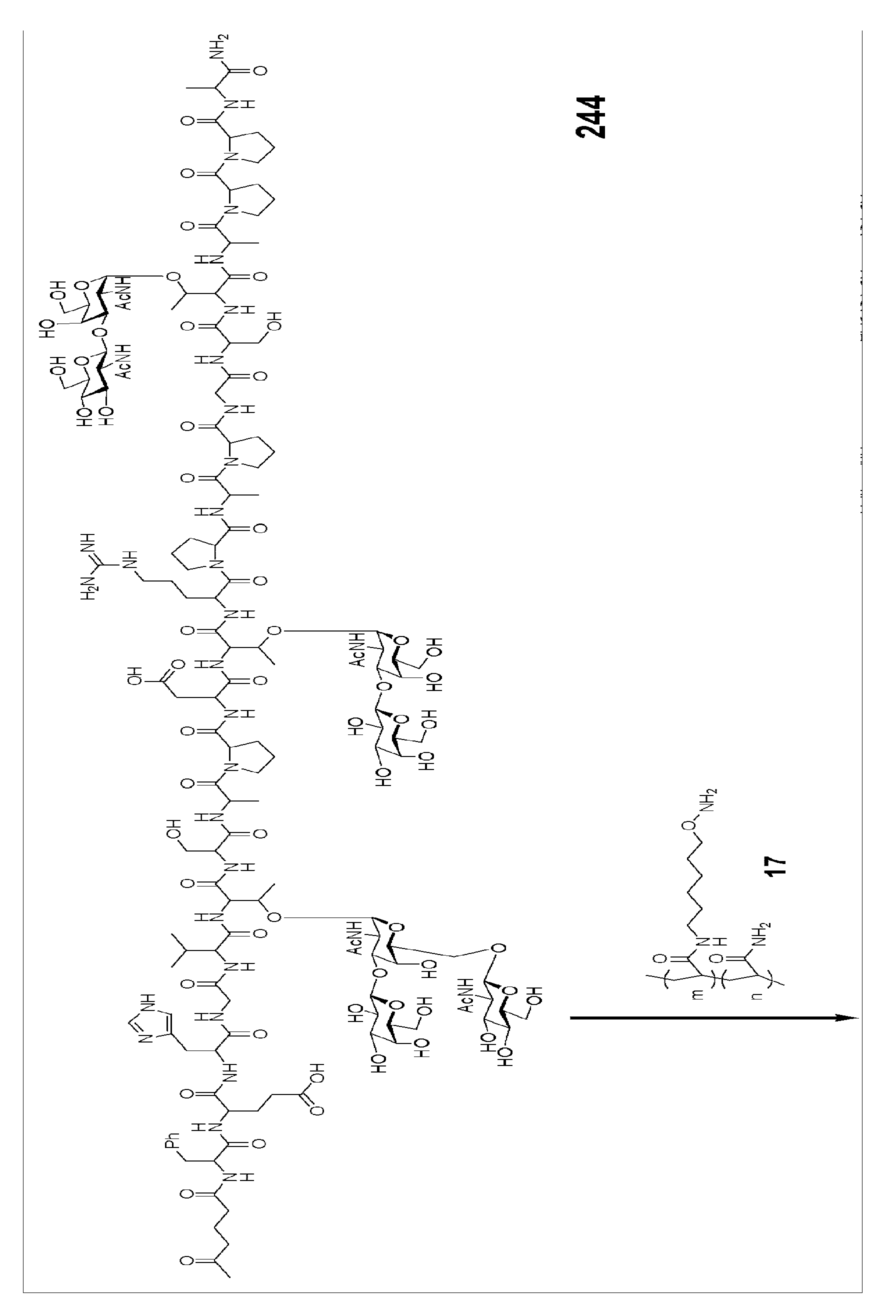

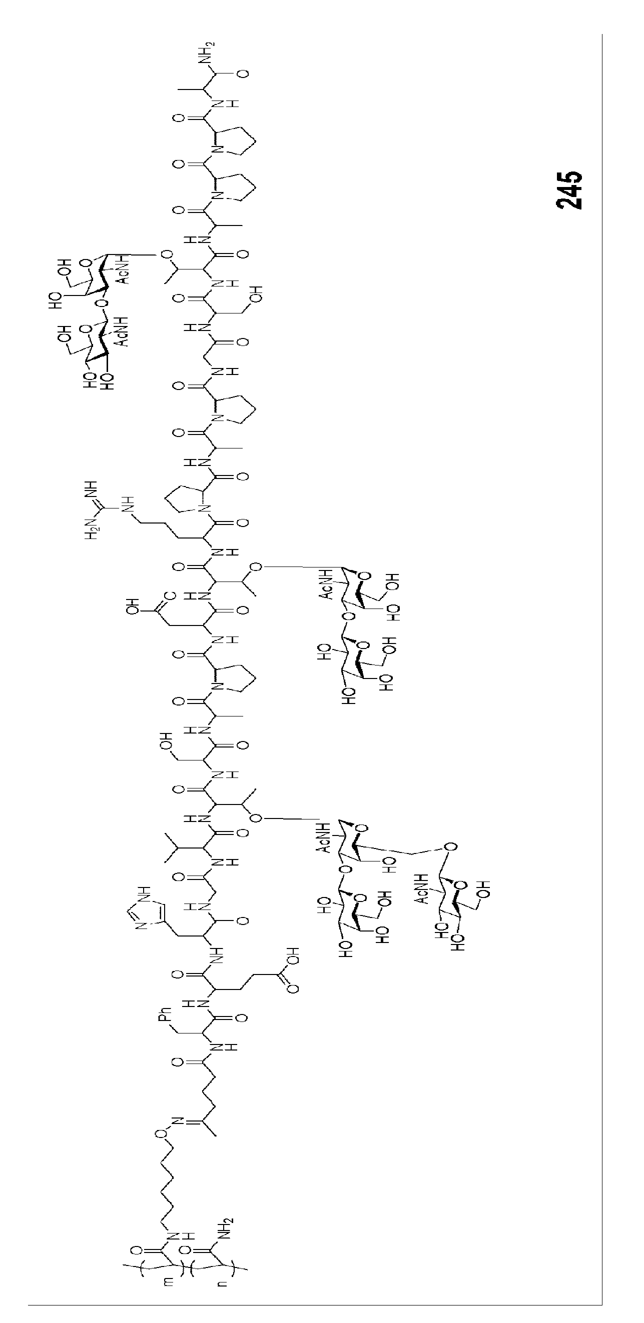

Using 71mg (0.02mmol) of Tentagel® S RAM resin (Hipep Laboratories, 0.28mmol/g) as a support, N-protected amino acids and keto acids as described below were sequentially condensed by Fmoc/HBTU/HOBt method, thereby synthesizing a glycopeptide derivative of interest: Fmoc-Ala-OH; Fmoc-Pro-OH; Fmoc-Pro-OH; Fmoc-Ala-OH; Fmoc-Thr(Ac5core3)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(Ac6corel)-OH; Fmoc-Asp(OtBu)-OH; Fmoc-Pro-OH; Fmoc-Ala-OH; Fmoc-Ser(tBu)-OH; Fmoc-Thr(Ac7core2)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained resin equivalent to 5nmol was allowed to react in 90% TFA aqueous solution for two hours at room temperature to eliminate a protective group on a peptide residue and concurrently release compound (243) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 1.5ml of methanol. 1N sodium hydroxide aqueous solution was added to this solution, and was adjusted to have a pH of 12-12.5. The solution was then stirred for 1.5 hours at room temperature, thereby performing Ac deprotection reaction. After the reaction, 1N acetic acid was added and the solution was neutralized. Thereafter, the resin was separated by filtration. The solvent in the filtrate was removed, and the residue was dissolved in 0.5ml of 50mM acetic acid/sodium acetate buffer solution (pH 5.5). 0.5ml of 10mM (oxyamine residue calculation) water-soluble polymer (17) aqueous solution was added to the solution, and the solution was stirred for 18 hours at room temperature, thereby reacting compound (244) with compound (17). After completion of the reaction, the reaction solution was subjected to centrifugal concentration with an ultrafiltration filter 10K Apollo ® 20ml (Orbital Biosciences, available from LIC). 25mM HEPES buffer solution (pH 7.0) was added thereto and the solution was again subjected to concentration, thereby washing the polymers. By adding water so that the final amount of the solution was 0.5ml, 10mM (theoretical content of glycopeptide) polymer (245) was obtained. Identification of the polymer (245) was performed based on that compound (408) in the following section (3.67).

(3.52 Synthesis of compounds (246) to (248) using a one-pot reaction)

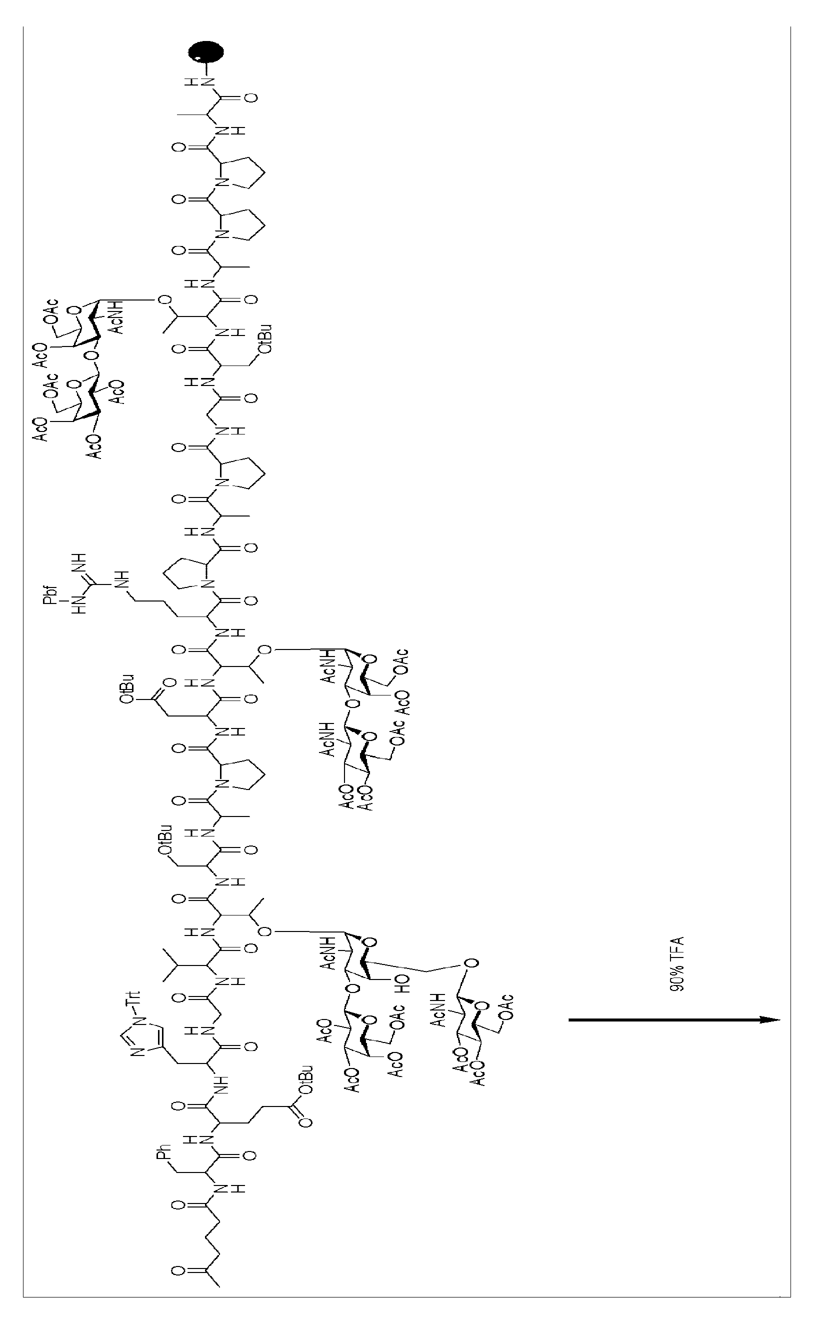

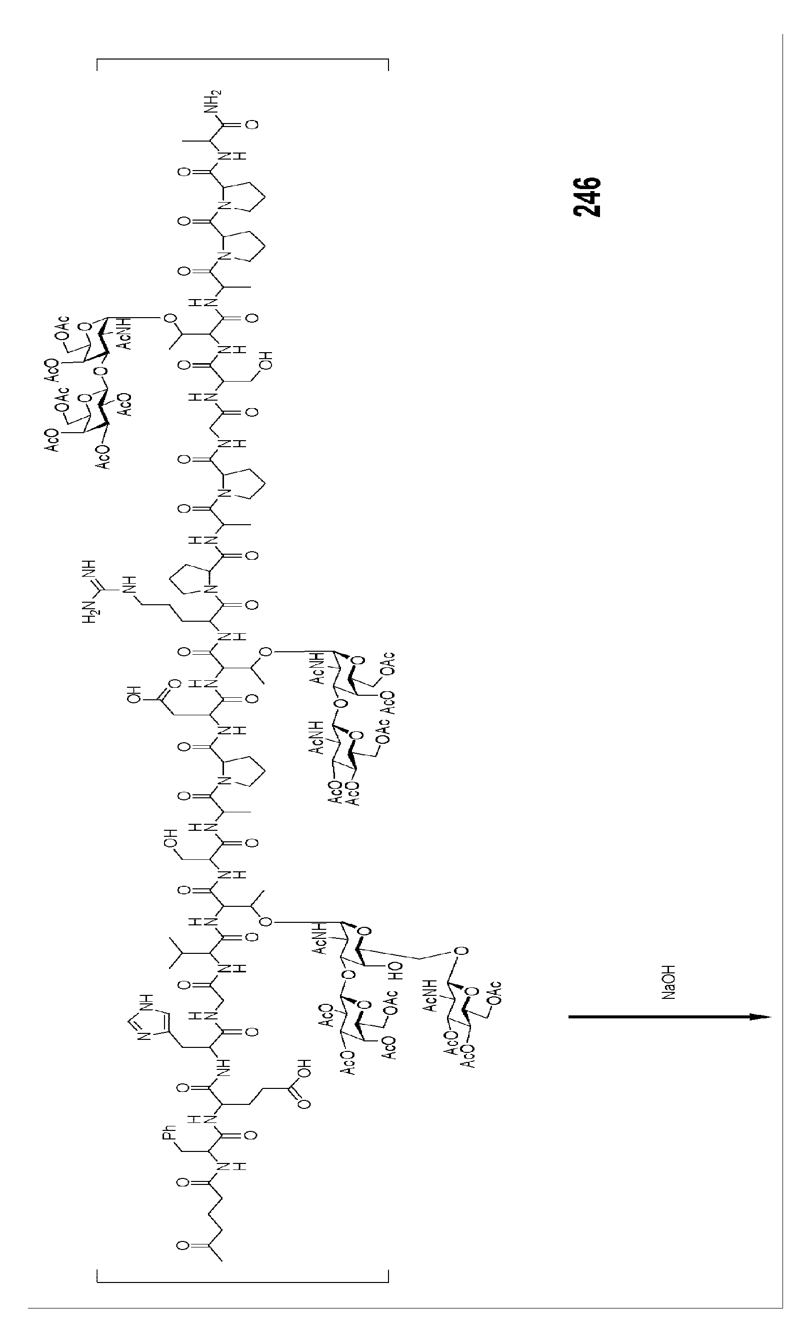

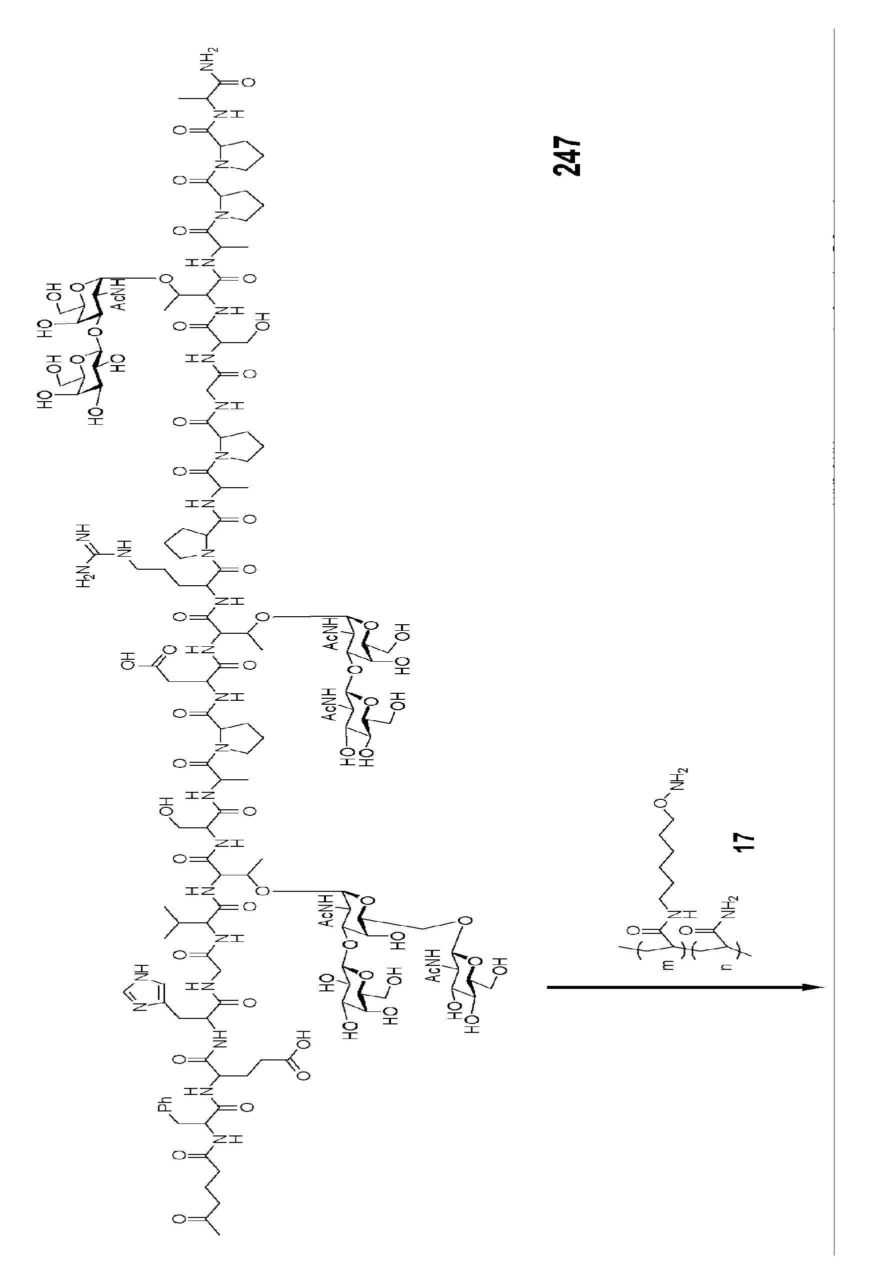

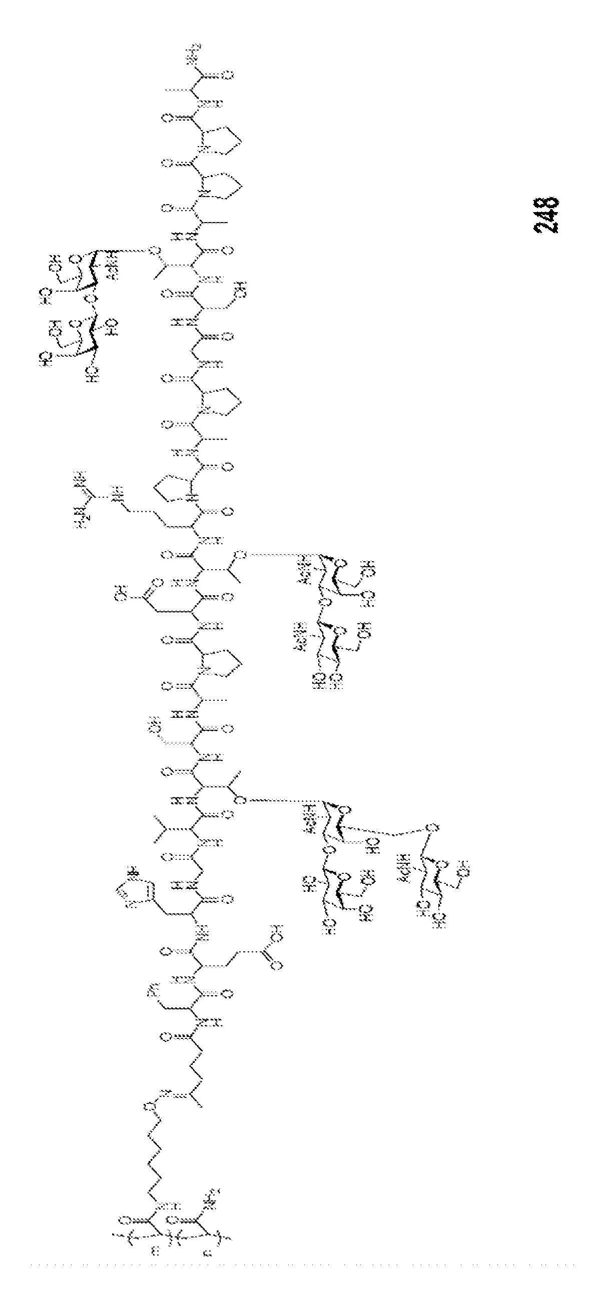

Using 71mg (0.02mmol) of Tentagel® S RAM resin (Hipep Laboratories, 0.28mmol/g) as a support, N-protected amino acids and keto acids as described below were sequentially condensed by Fmoc/HBTU/HOBt method, thereby synthesizing a glycopeptide derivative of interest: Fmoc-Ala-OH; Fmoc-Pro-OH; Fmoc-Pro-OH; Fmoc-Ala-OH; Fmoc-Thr(Ac6core1)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(Ac5core3)-OH; Fmoc-Asp(OtBu)-OH; Fmoc-Pro-OH; Fmoc-Ala-OH; Fmoc-Ser(tBu)-OH; Fmoc-Thr(Ac7core2)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained resin equivalent to 5nmol was allowed to react in 90% TFA aqueous solution for two hours at room temperature to eliminate a protective group on a peptide residue and concurrently release compound (246) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether wasagain added,andthe precipitate waswashed. Centrifugal separation was again performed and the supernatant was removed. The obtained precipitant was dissolved in 1.5ml of methanol. 1N sodium hydroxide aqueous solution was added to this solution, and was adjusted to have a pH of 12-12.5. The solution was then stirred for 1.5 hours at room temperature, thereby performing Ac deprotection reaction. After the reaction, 1N acetic acid was added and the solution was neutralized. Thereafter, the resin was separated by filtration. The solvent in the filtrate was removed, and the residue was dissolved in 0.5ml of 50mM acetic acid/sodium acetate buffer solution (pH 5.5). 0.5m1 of 10mM (oxyamine residue calculation) water-soluble polymer (17) aqueous solution was added to the solution, and the solution was stirred for 18 hours at room temperature, thereby reacting compound (247) with compound (17). After completion of the reaction, the reaction solution was subjected to centrifugal concentration with an ultrafiltration filter 10K Apollo ® 20ml (Orbital Biosciences, available from LIC). 25mM HEPES buffer solution (pH 7.0) was added thereto and the solution was again subjected to concentration, thereby washing the polymers. By adding water so that the final amount of the solution was 0.5ml, 10mM (theoretical content of glycopeptide) polymer (248) was obtained. Identification of the polymer (248) was performed based on that product (419) was obtained in the following section (3.68).
The following sections (3.53) to (3.68) were carried out in parallel using ultrafiltration-type AcroPrep®Multi-well Filter Plates (available from PALL) 96 well plates.

(3.53 Synthesis of compounds (249) to (259))

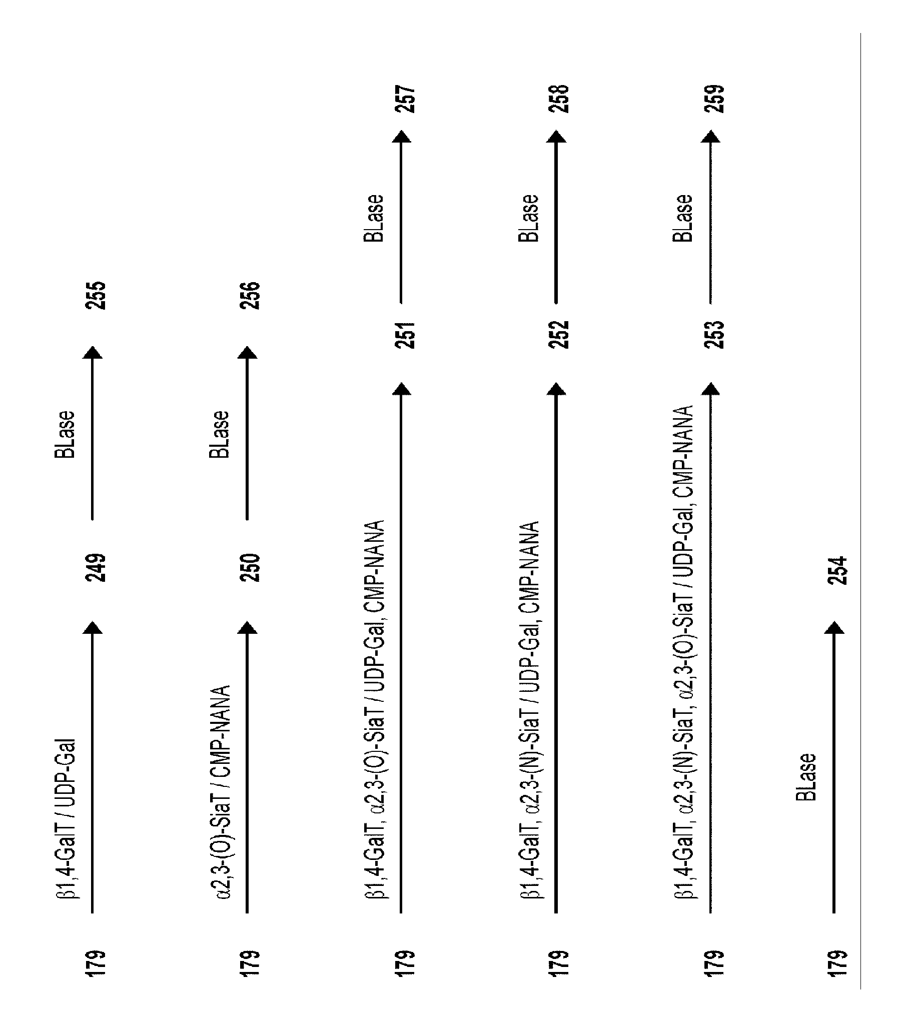

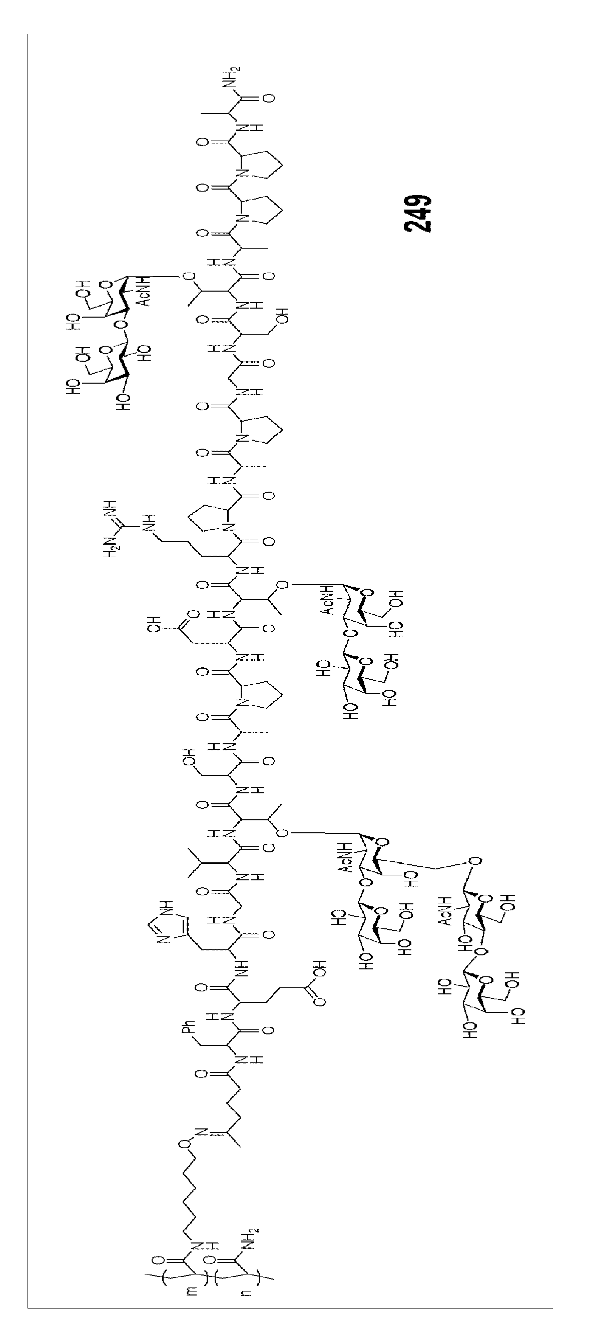

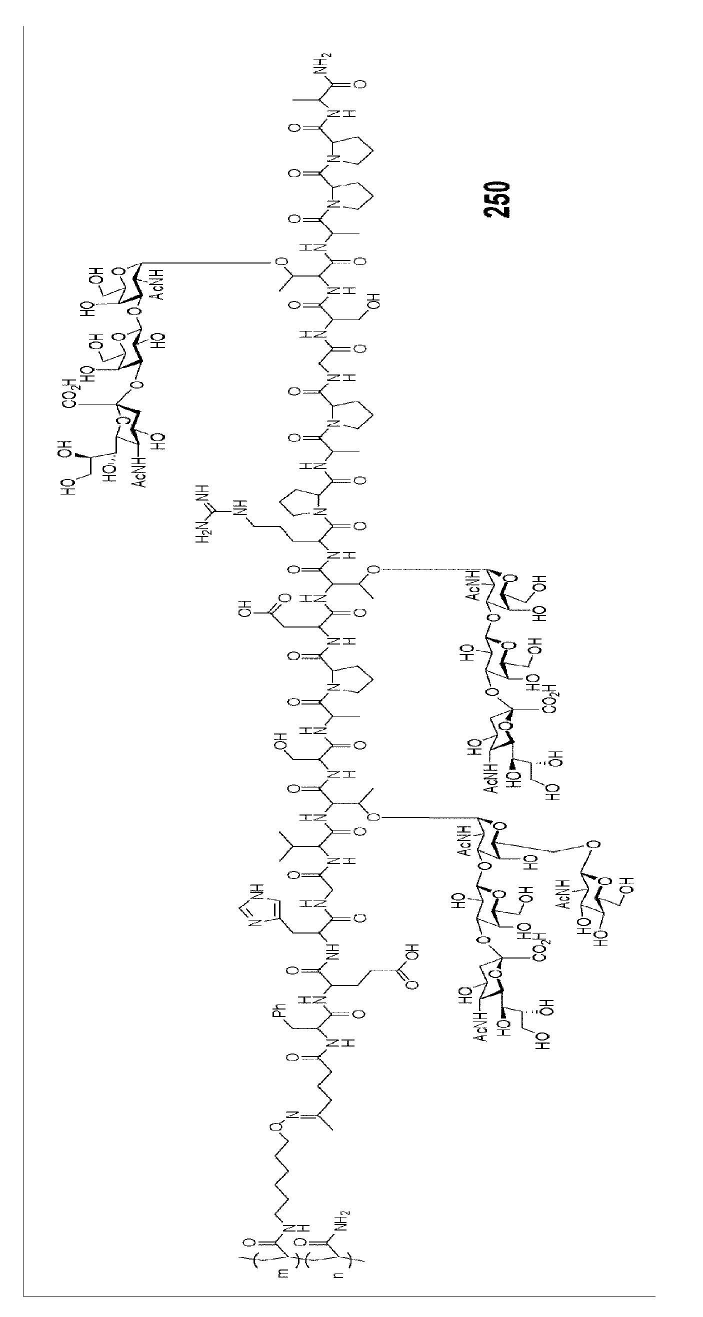

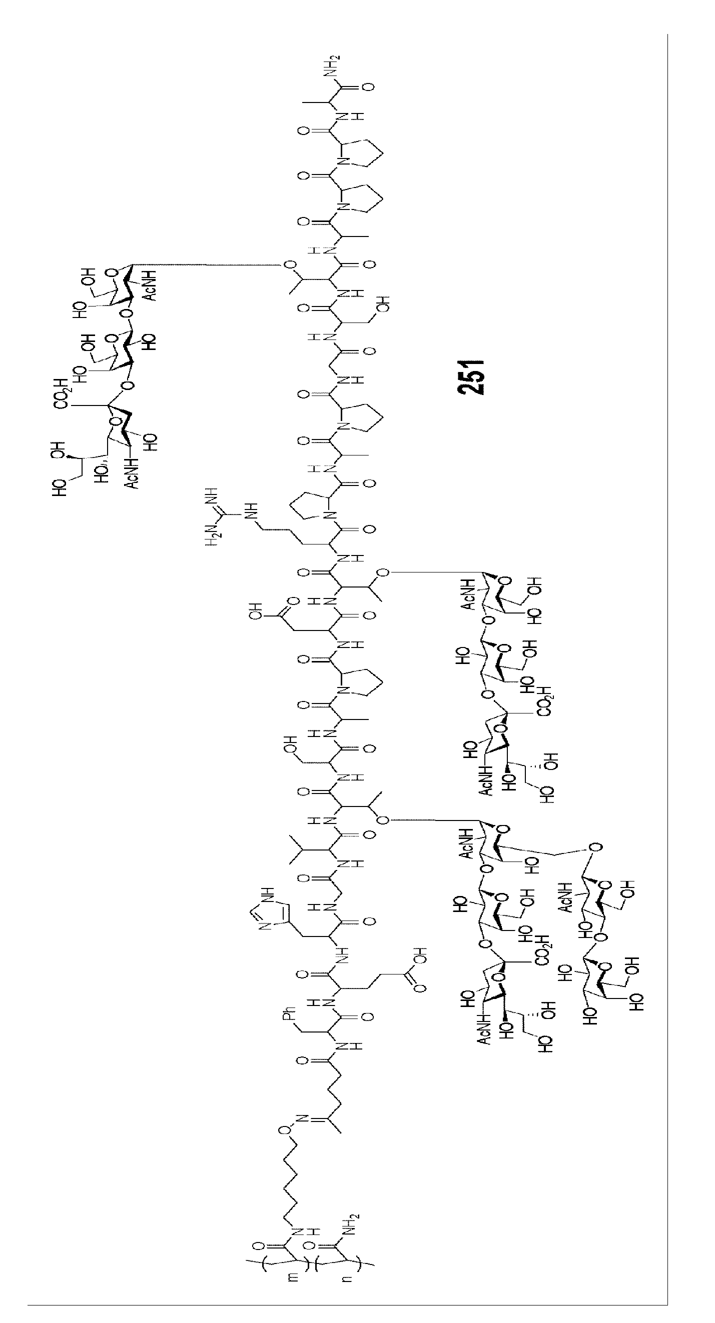

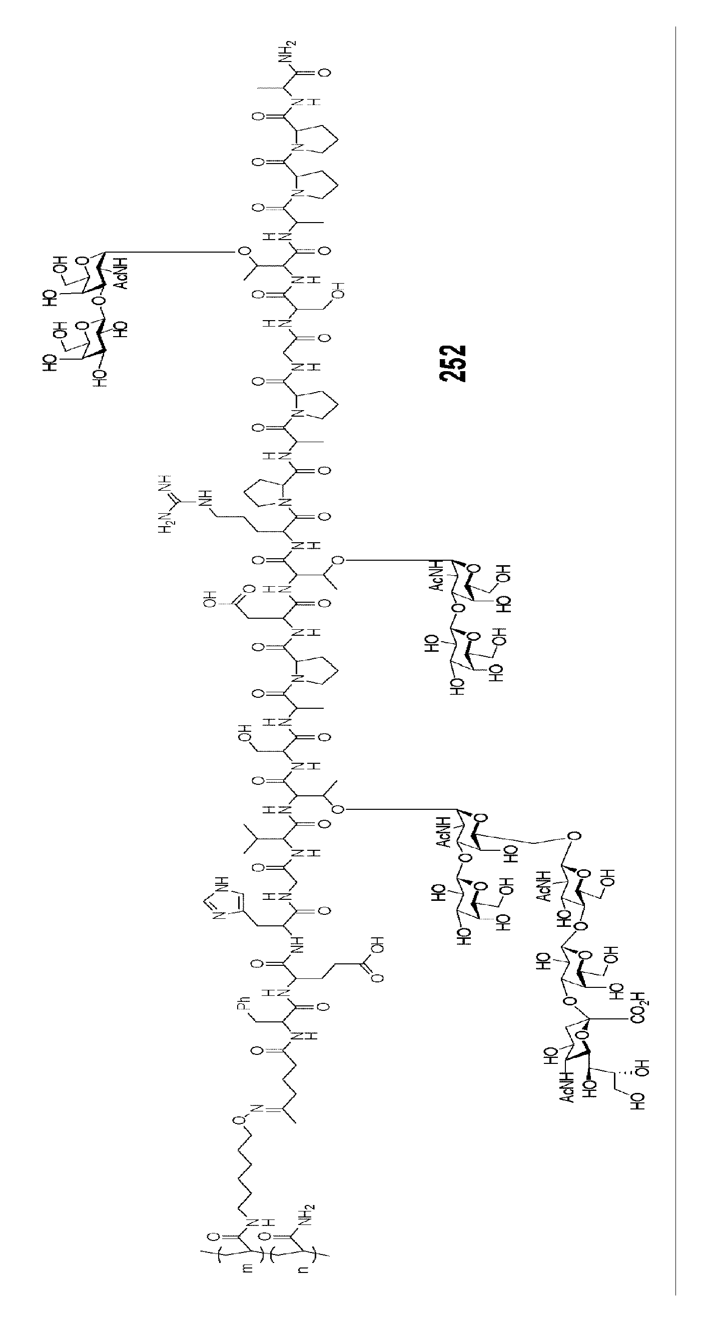

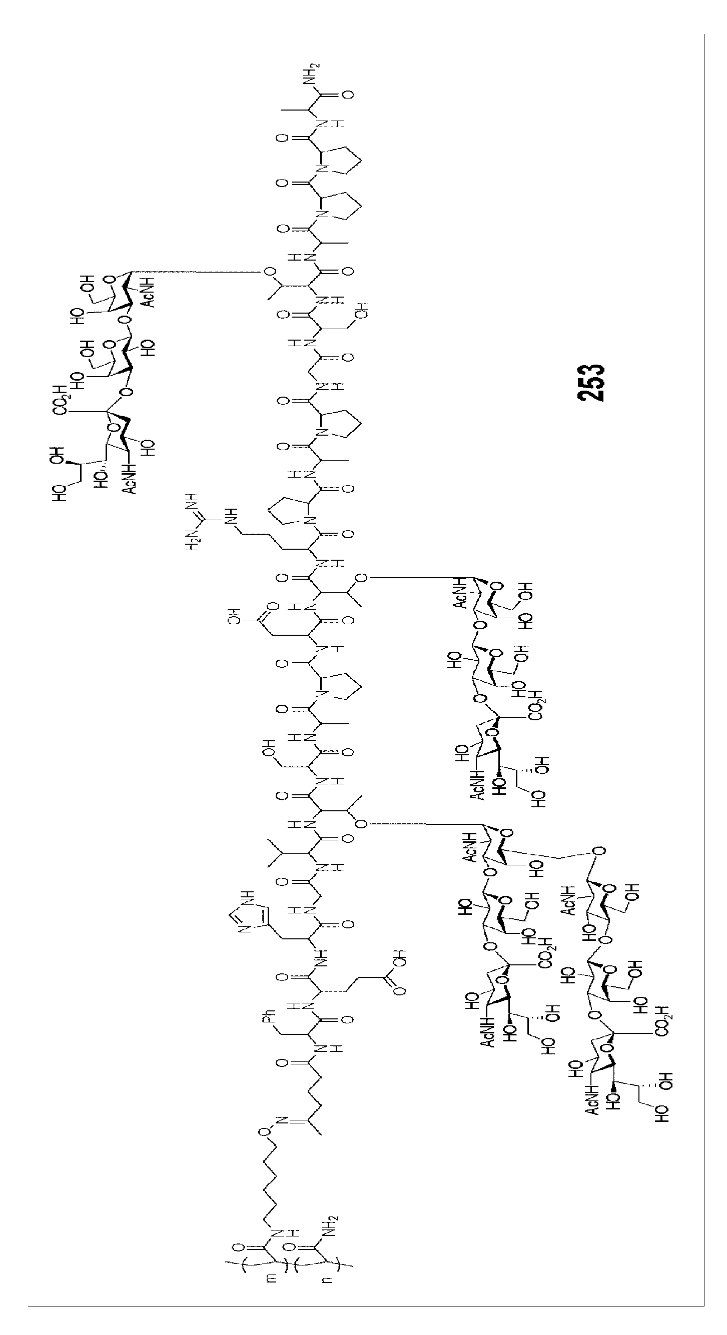

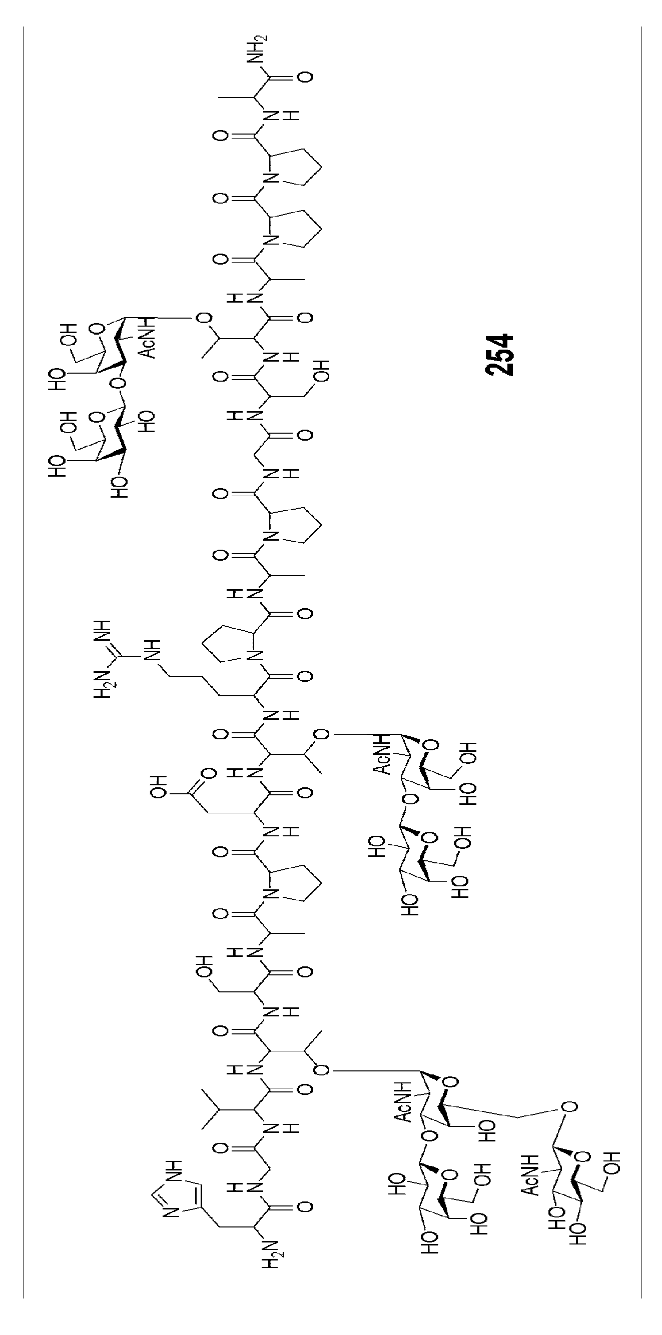

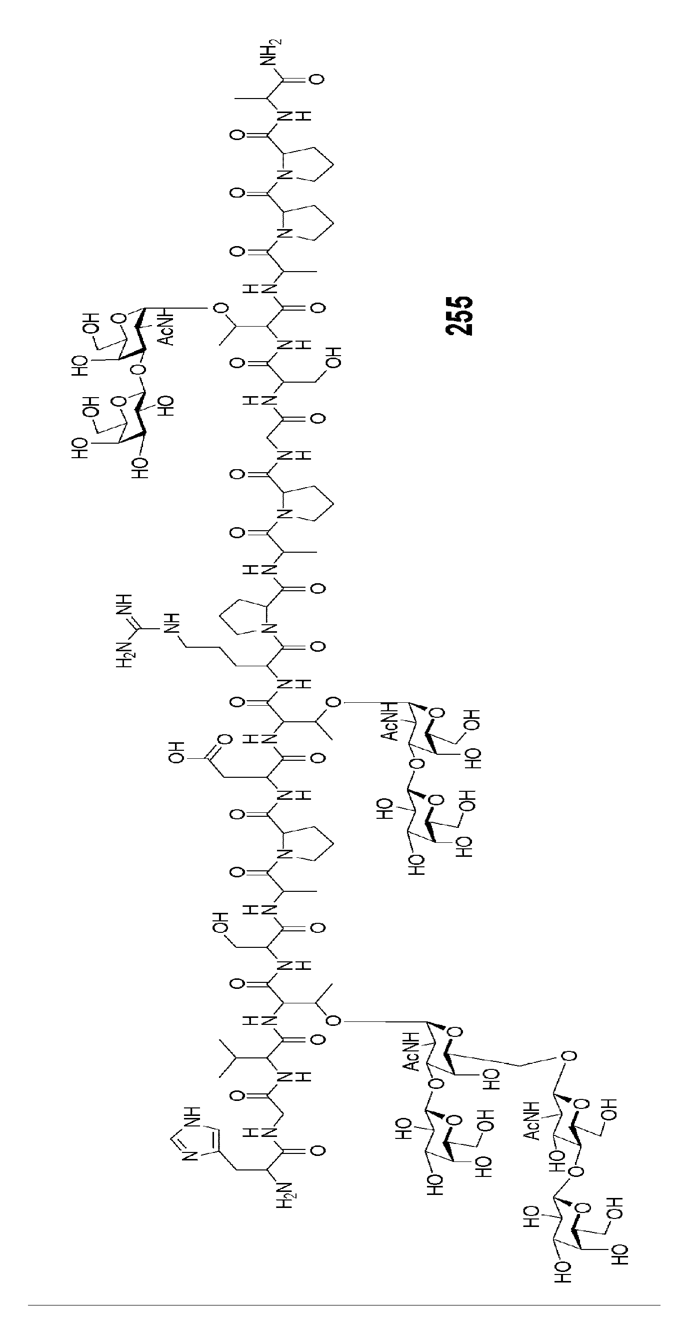

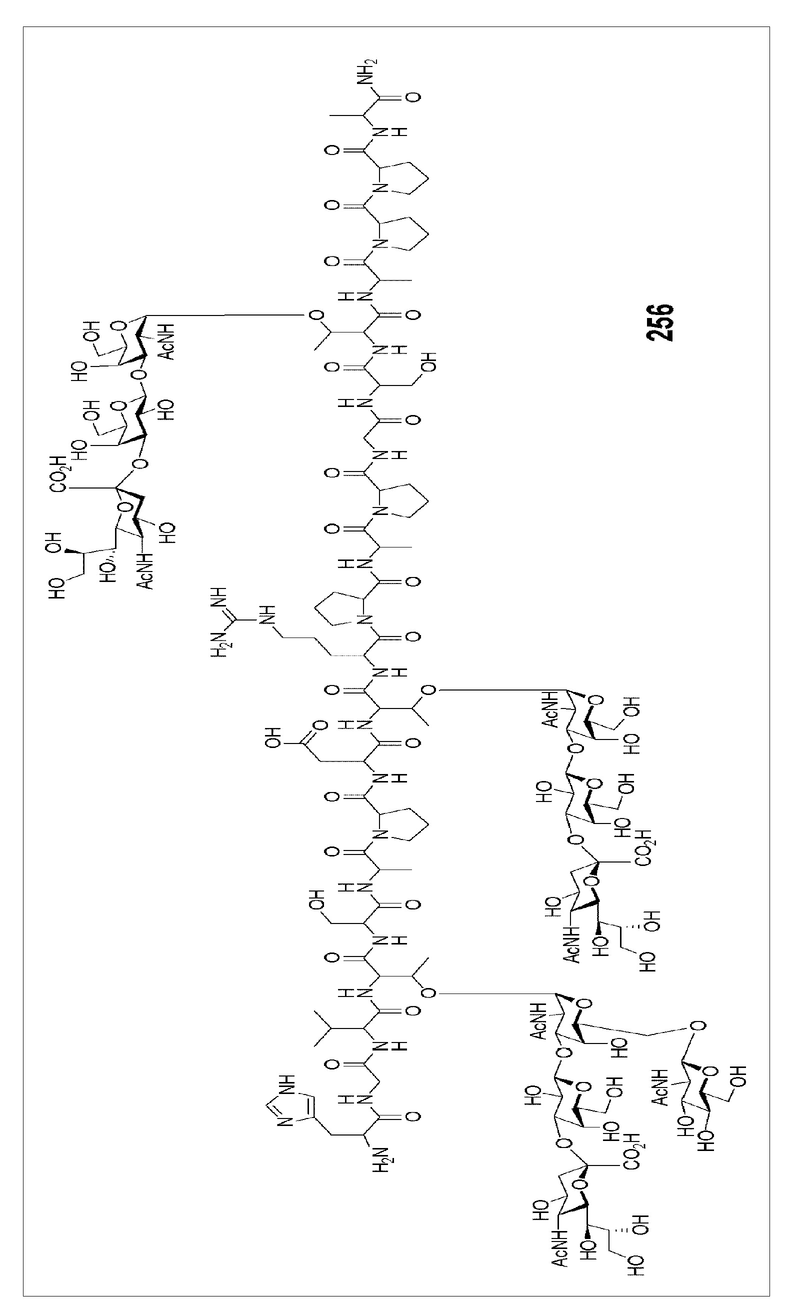

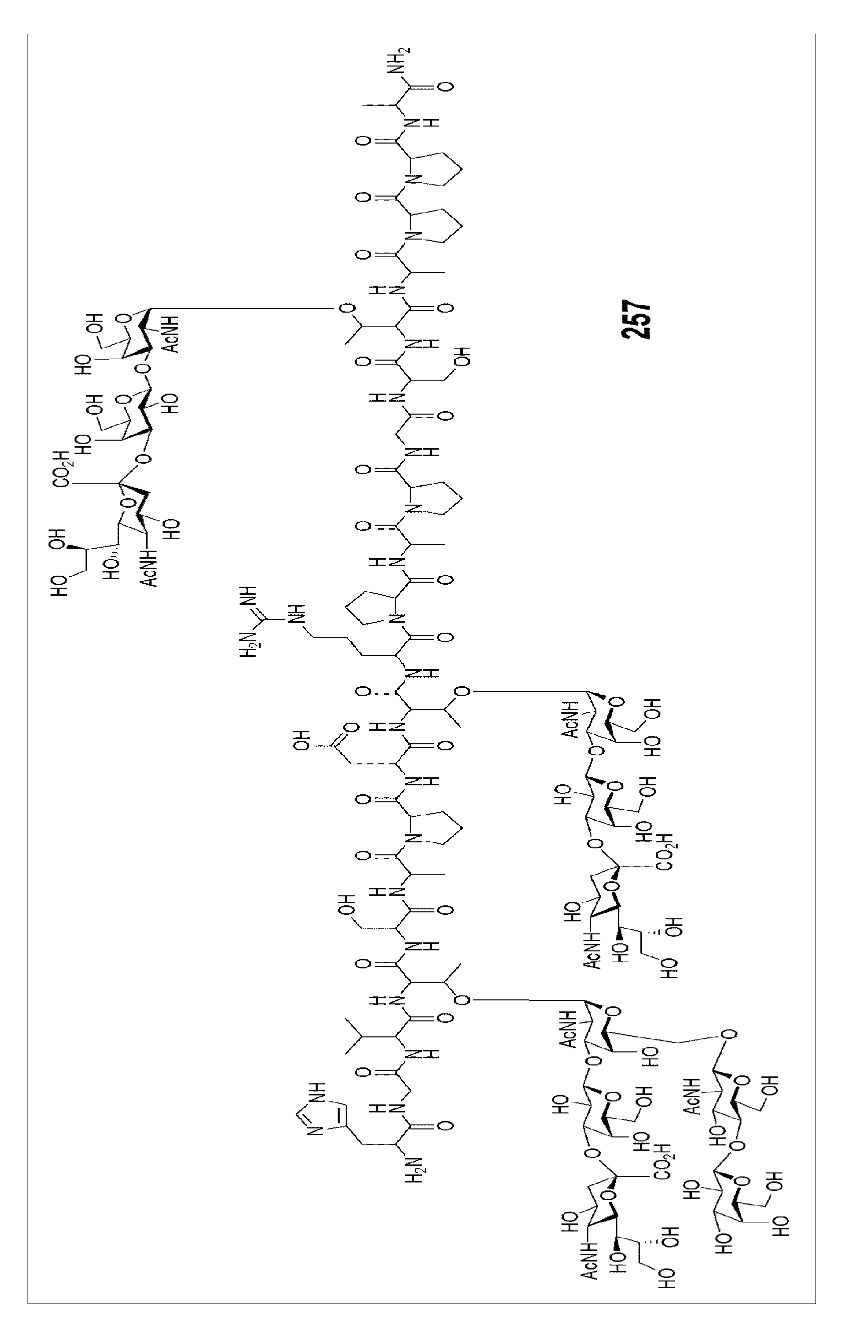

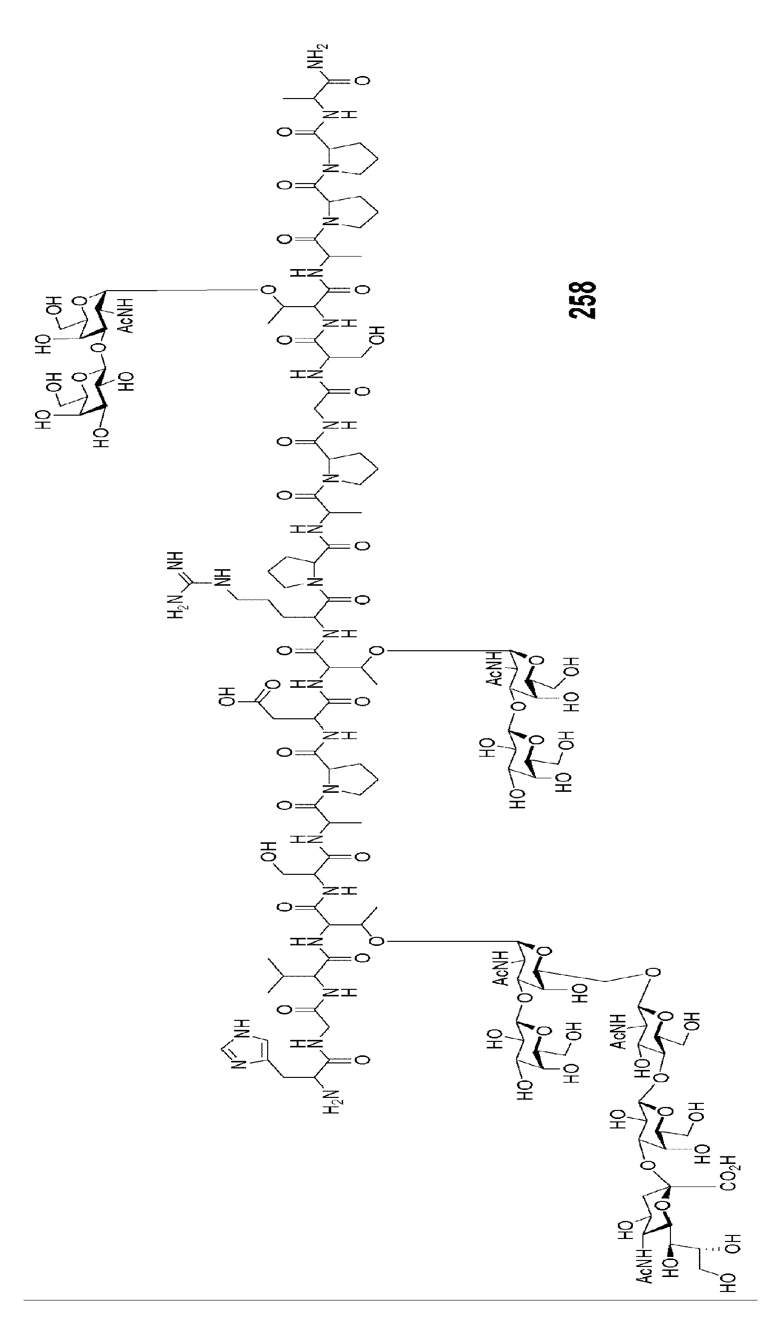

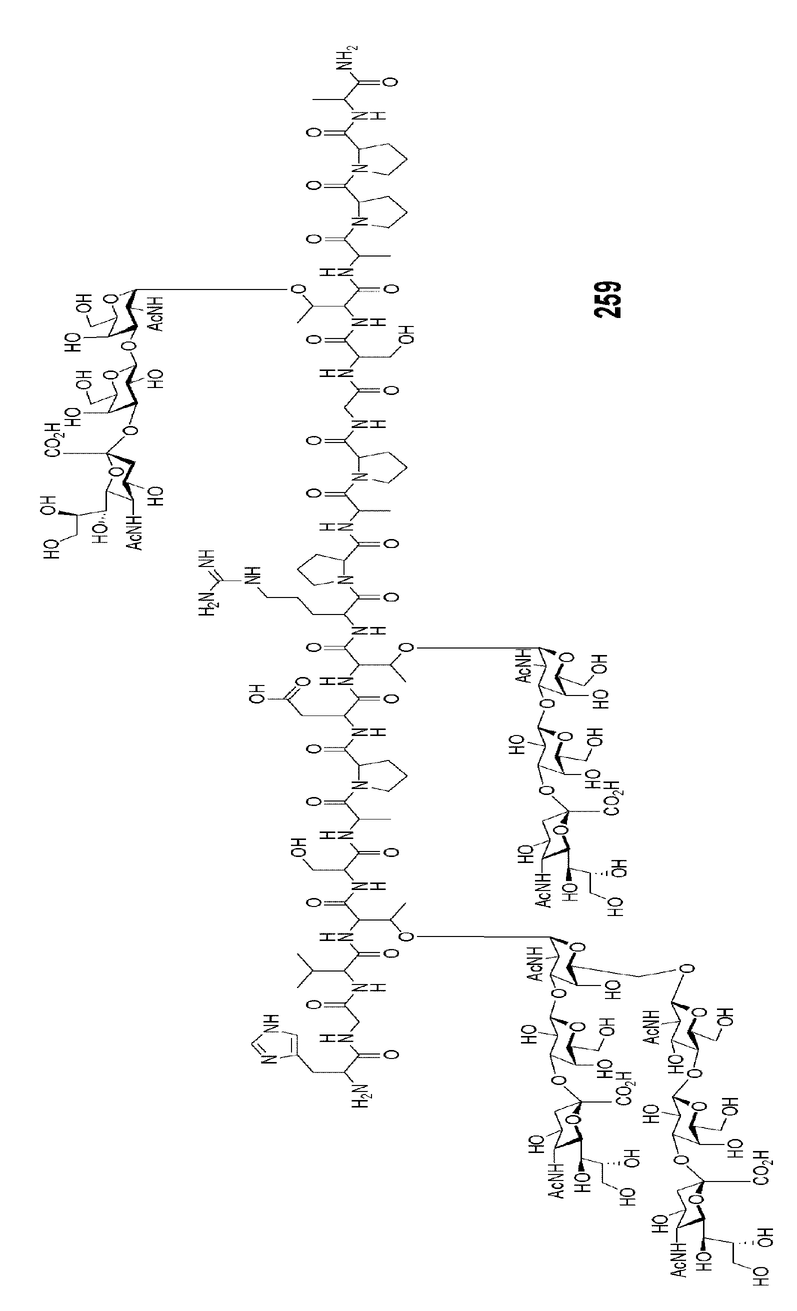

The following reaction solutions A) to E) were allowed to react for 24 hours at 25°C:
A) 100µl of reaction solution containing 50mMHEPES buffer solution (pH 7.0), 0.1U/ml human-derived β1,4-galactosyltransferase (available from TOYOBO CO., LTD.), 10mM manganese chloride, 0.1% BSA, 2mM uridine-5'-disodium diphosphogalactose (UDP-Gal) and glycopeptide derivative (179) (4mM at a theoretical content from solid-phase synthesis);
B) 100µl of reaction solution containing 50mM HEPES buffer solution (pH 7.0), 0.0175U/ml recombinant rat α2,3-(O)-sialyltransferase (available from Calbiochem), 10mM manganese chloride, 0.1% BSA, 2mM cytidine-5'-sodium monophosphosialate (CMP-NANA) and glycopeptide derivative (179) (4mM at a theoretical content from solid-phase synthesis);
C) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (179) (4mM at a theoretical content from solid-phase synthesis);
D) 100µl of reaction solution containing 50mM HEPES buffer solution (pH 7.0), 0.1U/ml human-derived β1,4-galactosyltransferase (available from TOYOBO CO., LTD.), 0.0185U/ml recombinant rat α2,3-(N)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (179) (4mM at a theoretical content from solid-phase synthesis);
E) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 0.0185U/ml recombinant rat α2,3-(N)-sialyltransferase (available from Calbiochem), 10mM manganese chloride, 0.1% BSA, 4mM uridine-5'-disodium diphosphogalactose (UDP-Gal), 2mM cytidine-5'-sodium monophosphosialate (CMP-NANA) and glycopeptide derivative (179) (4mM at a theoretical content from solid-phase synthesis).

After completion of the reaction, the respective reaction solutions were subjected to centrifugal concentration with an ultrafiltration filter. Thereafter, 25mM ammonium acetate buffer solution (pH 6.5) was added thereto, and the solution was again subjected to concentration with a centrifugal separator, thereby washing the polymer. This manipulation was repeated three times, thereby respectively obtaining aqueous solutions of compounds (249) to (253). Thereafter, to the solution retained at the filter containing compounds (249) to (253) and a solution which was obtained by diluting an aqueous solution of (179) with 25mM ammonium acetate buffer solution (pH 6.5), 1µl of 0.174mg/ml solution of BLase (available from Shionogi & Co., Ltd.) was added, and the solution was allowed to react for two hours at room temperature. Thereafter, the solution was subjected to centrifugal filtration with an ultrafiltration filter, thereby separating the glycopeptide of interest from the polymer. The obtained aqueous solution (filtrate) was lyophilized, thereby obtaining compounds (254) to (259).
MALDI TOF/MS:[M(average)+H]⁺ of compound (254)=3187.9 (theoretical value:[M(average)+H]⁺=3186.2); MALDI TOF/MS:[M(average)+H]⁺ of compound (255)=3349.5 (theoretical value:[M(average)+H]⁺=3348.4); MALDI TOF/MS:[M(average)+H]⁺ of compound (256)=4062.6 (theoretical value:[M(average)+H]⁺=4060.0); MALDI TOF/MS:[M(average)+H]⁺ of compound (257)=4220.4 (theoretical value:[M(average)+H]⁺=4222.1); MALDI TOF/MS:[M(average)+H]⁺ of compound (258)=3638.7 (theoretical value:[M(average)+H]⁺=3639.6); and MALDI TOF/MS:[M(average)+H]⁺ of compound (259)=4513.8 (theoretical value:[M(average)+H]⁺=4513.4).

(3.54 Synthesis of compounds (260) to (270))

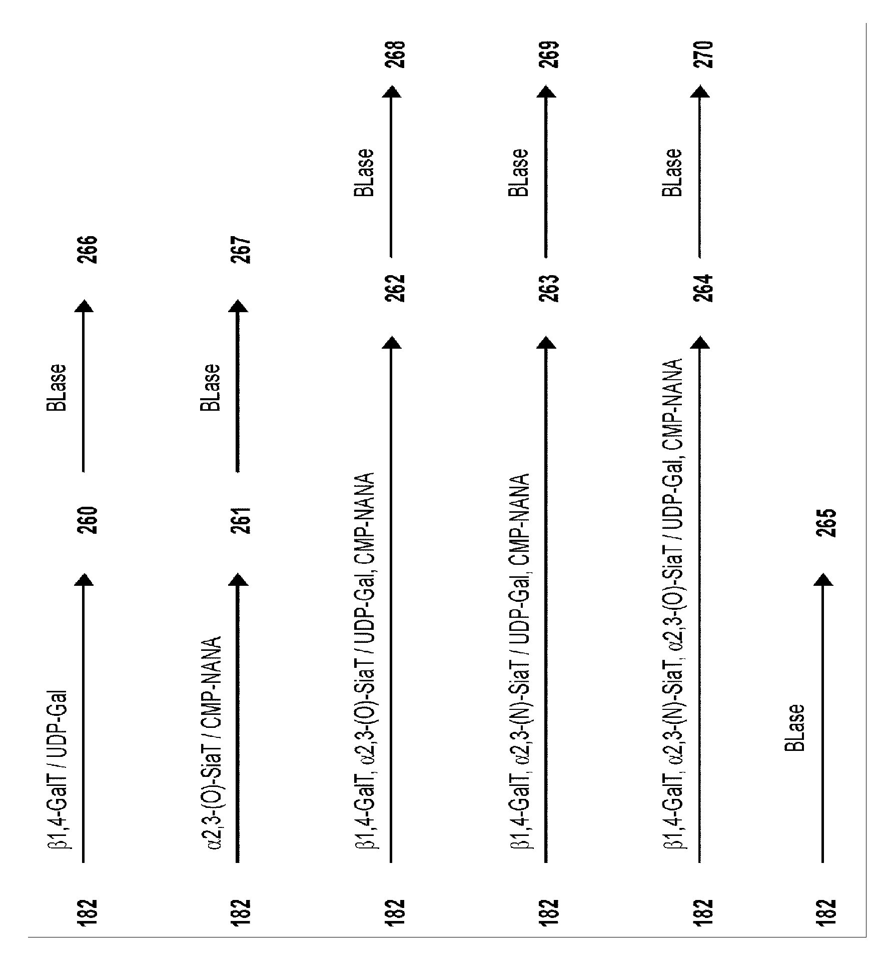

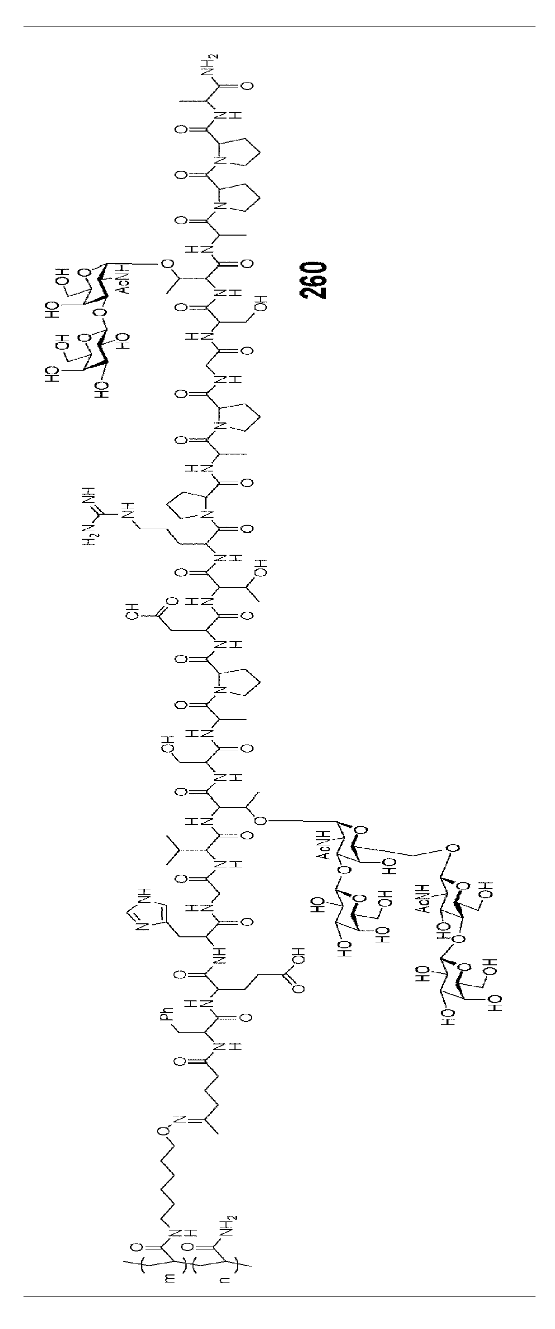

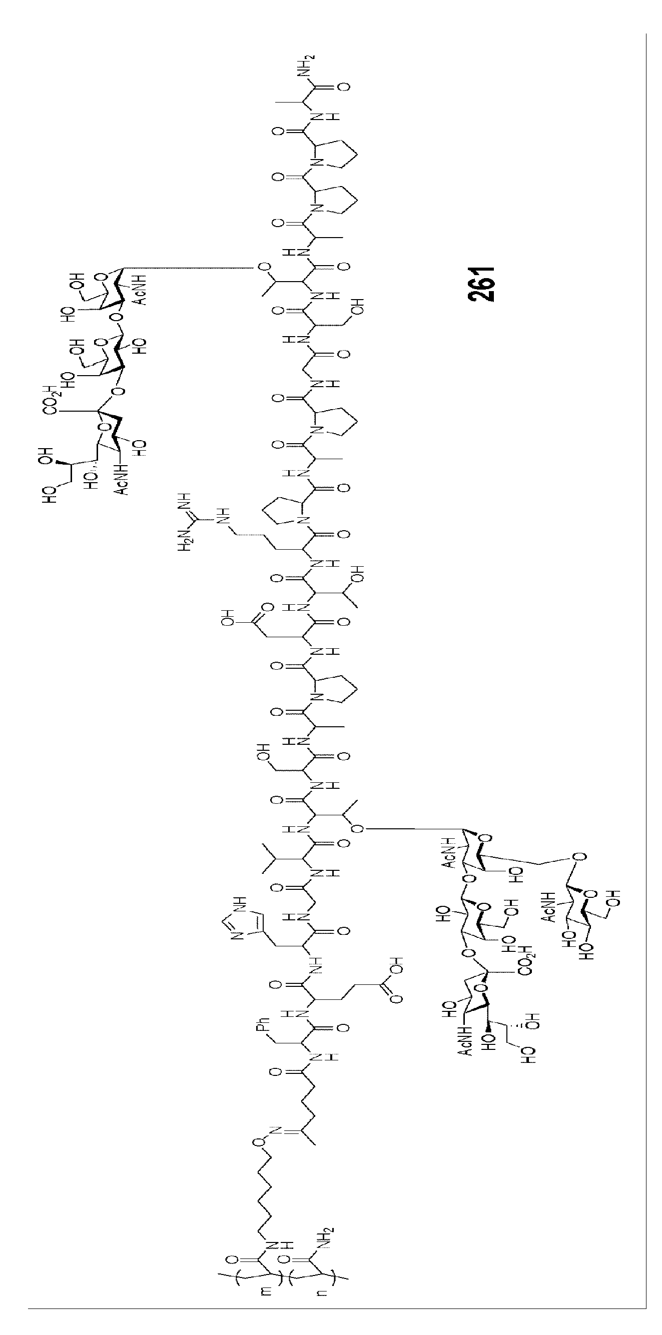

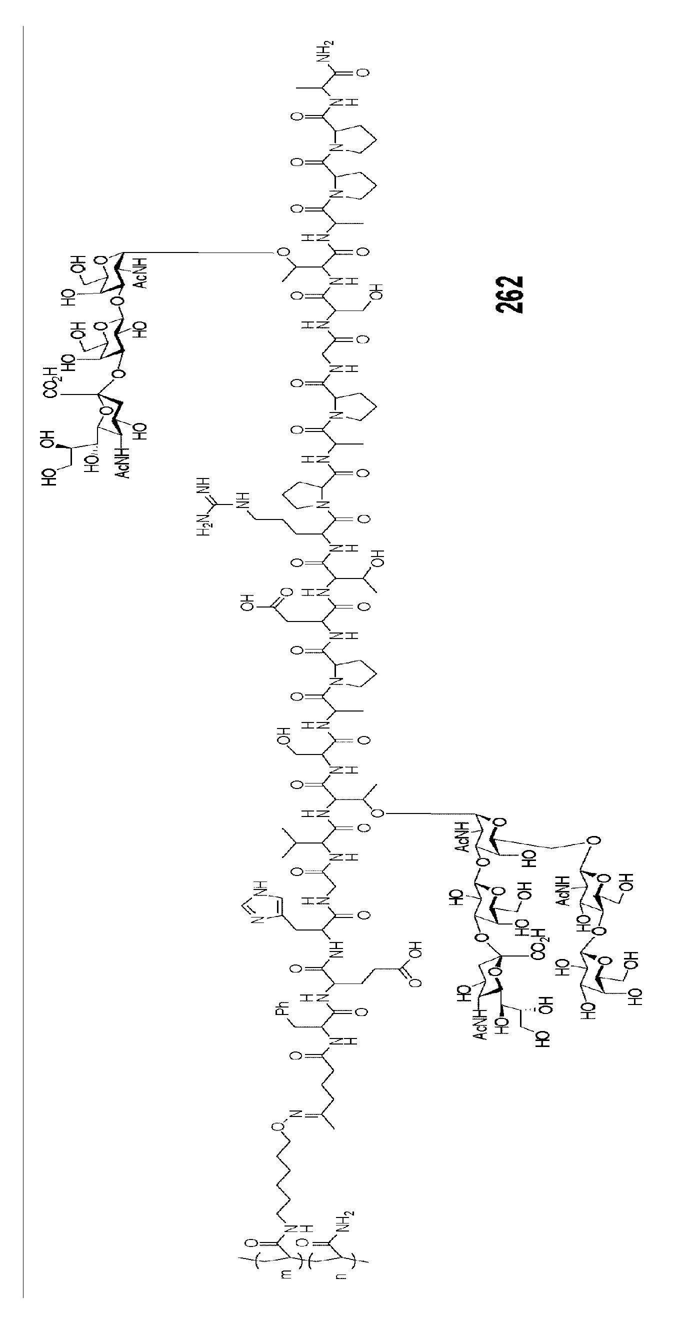

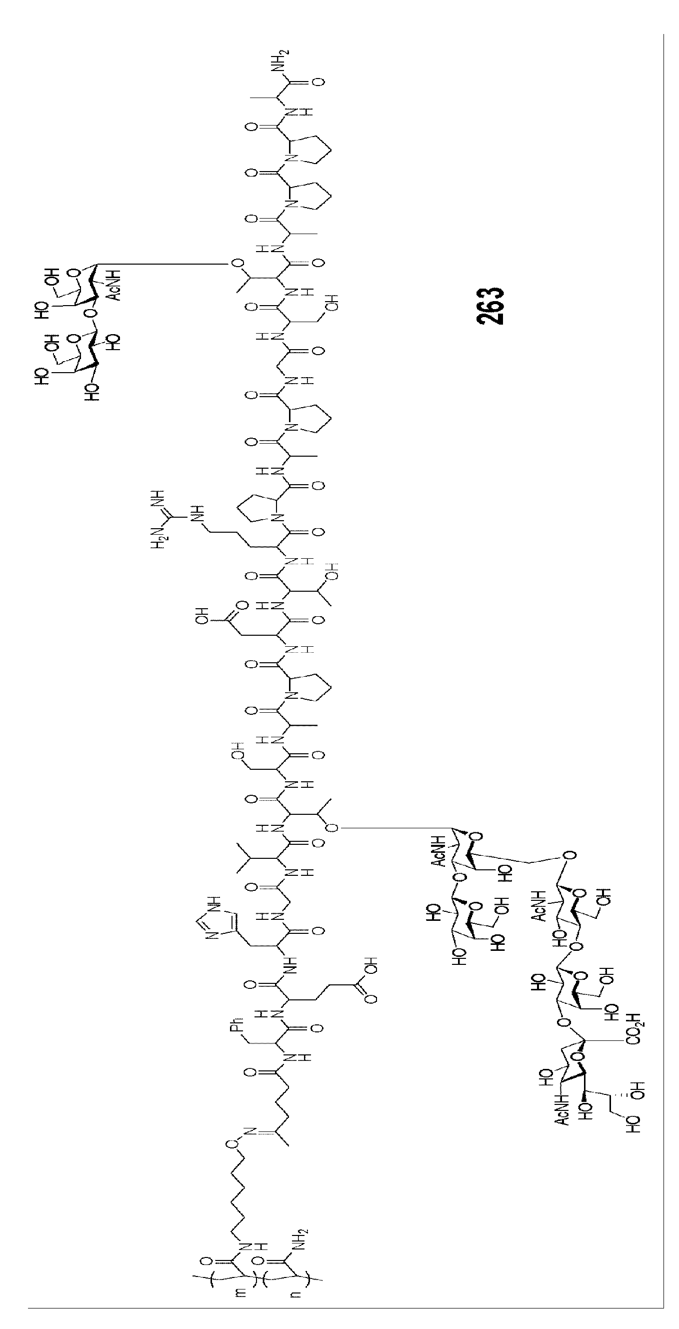

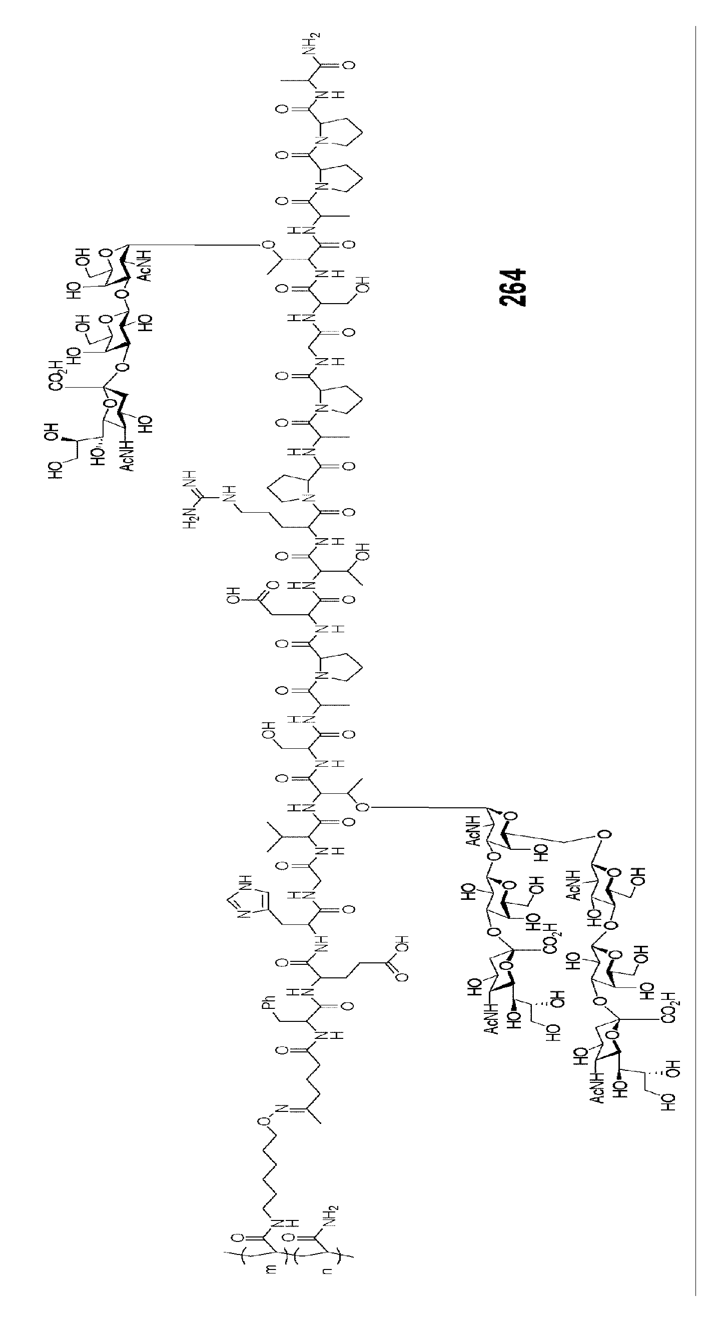

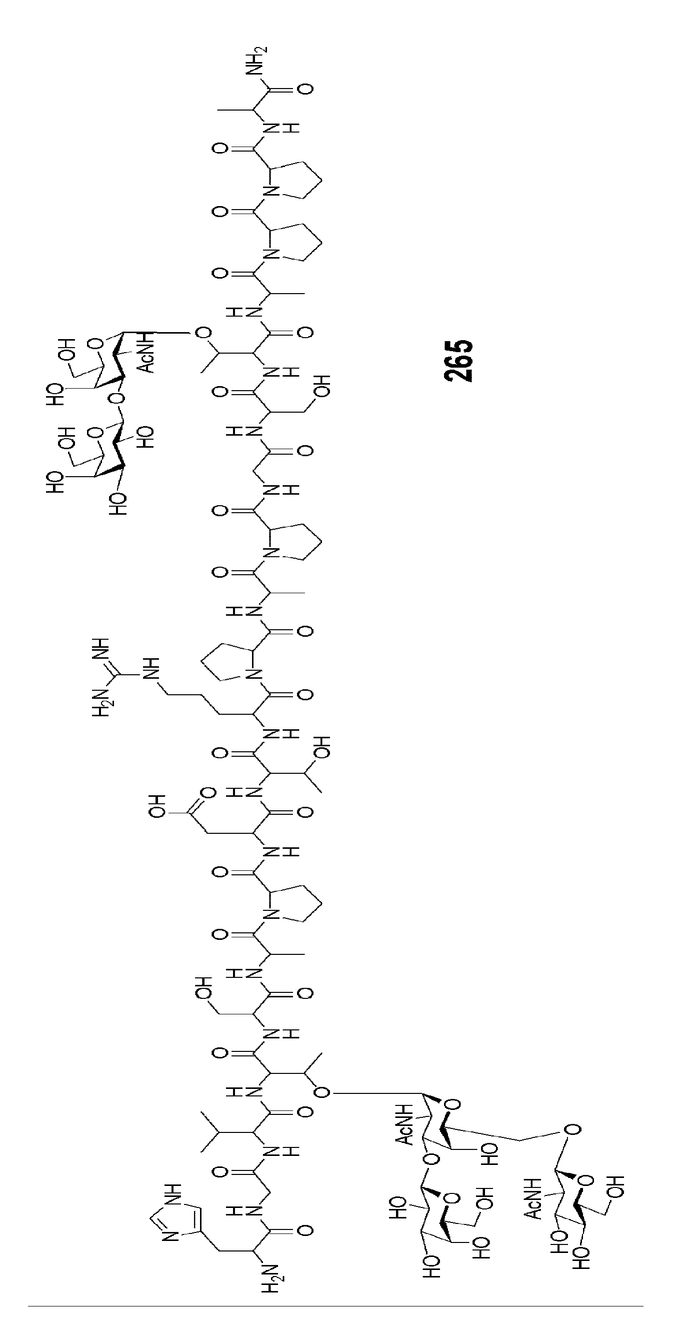

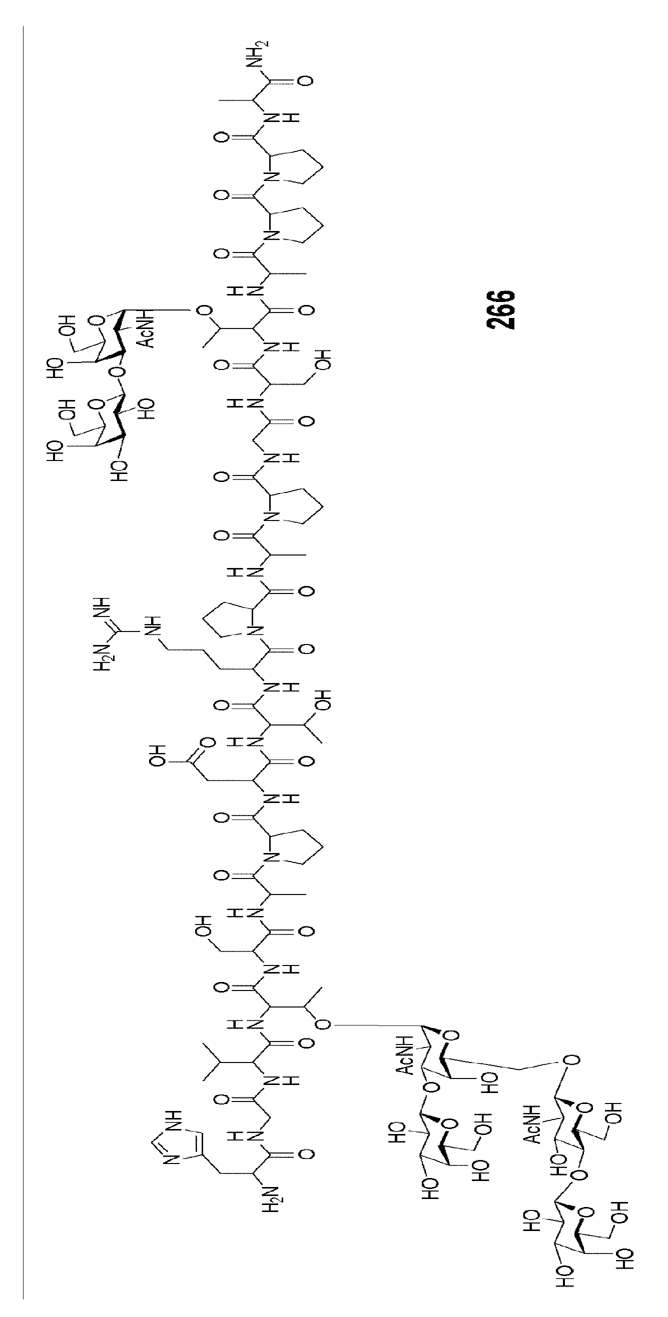

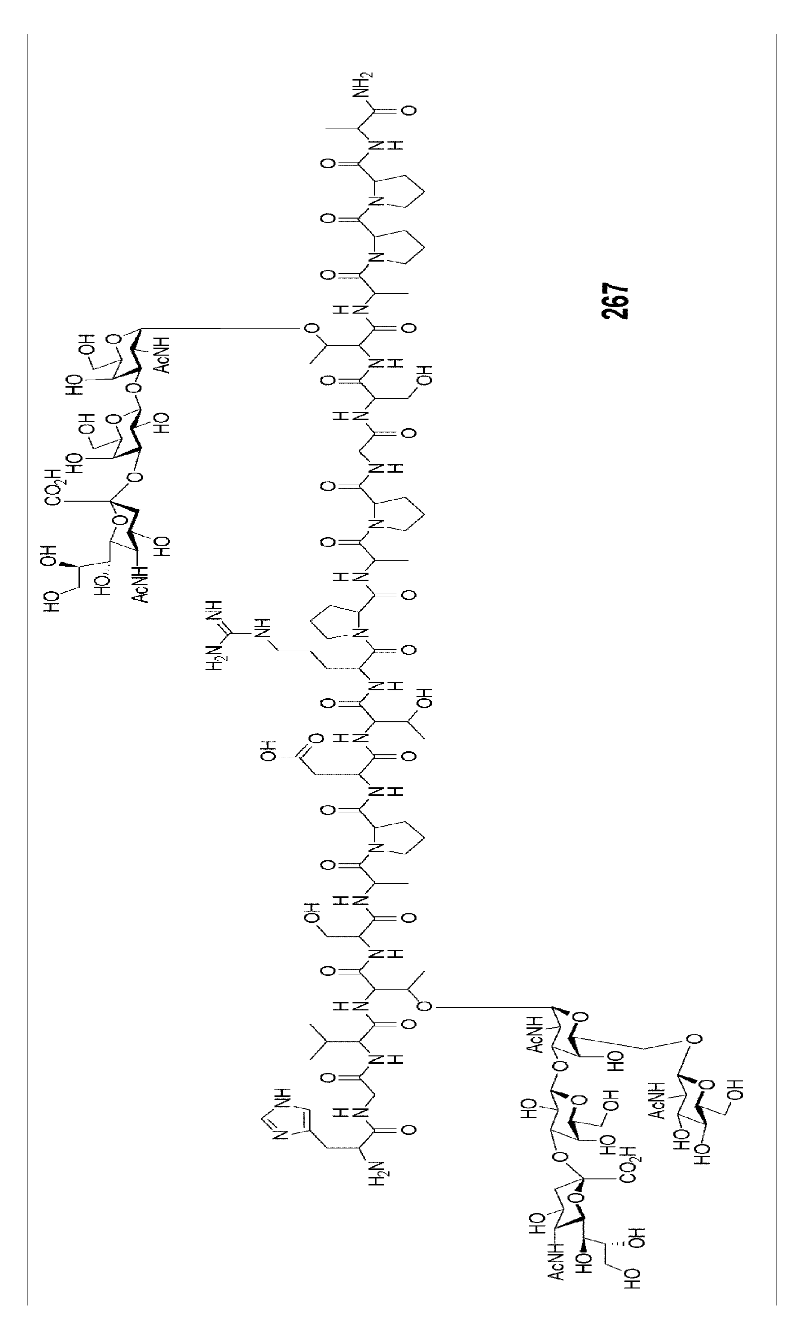

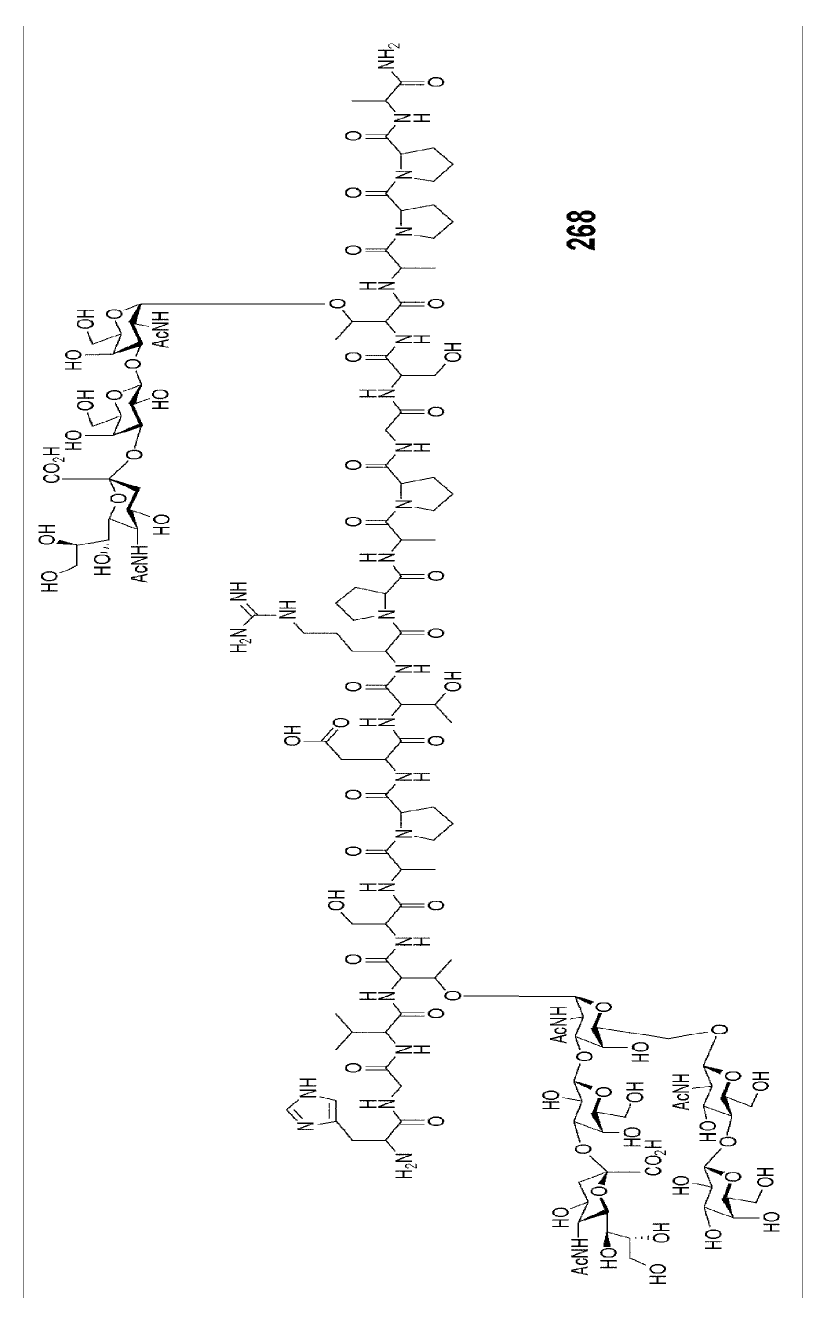

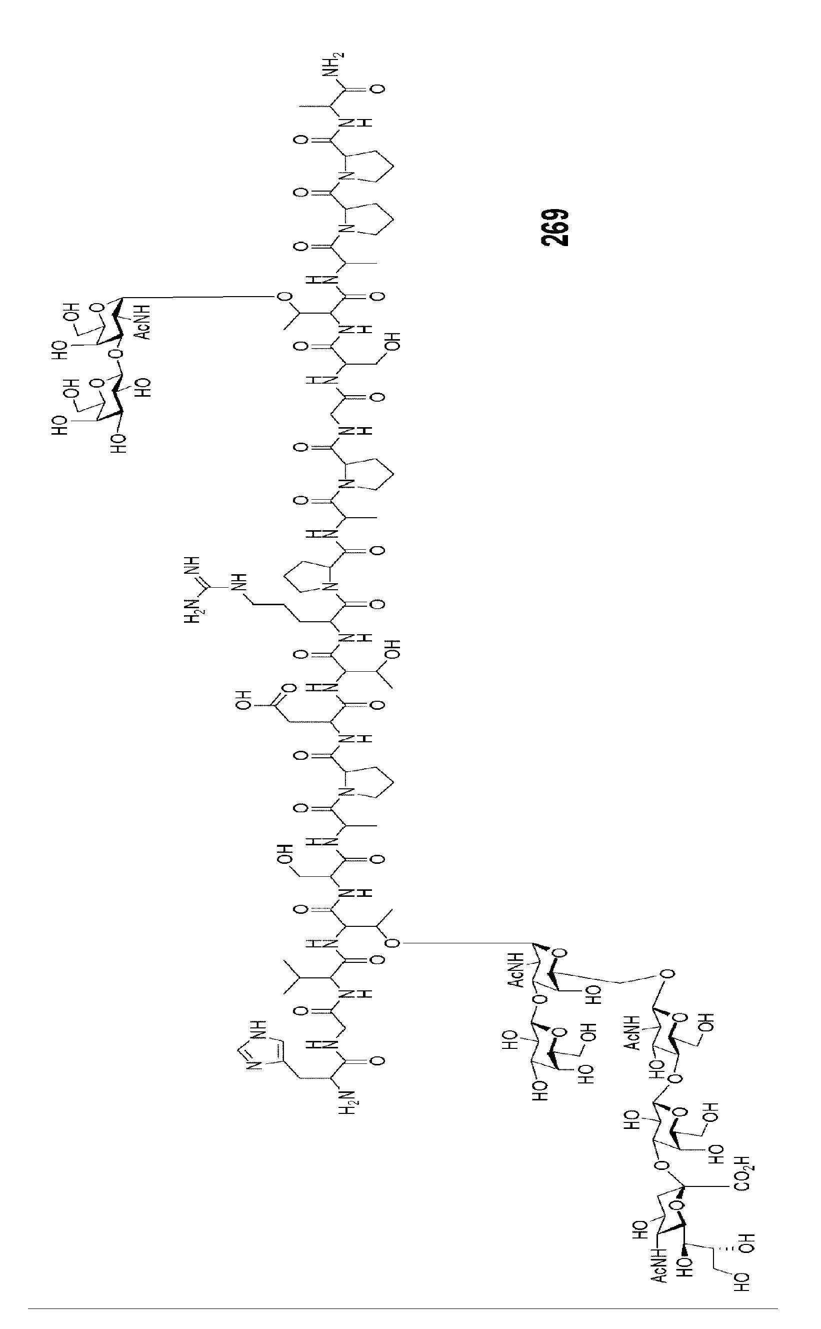

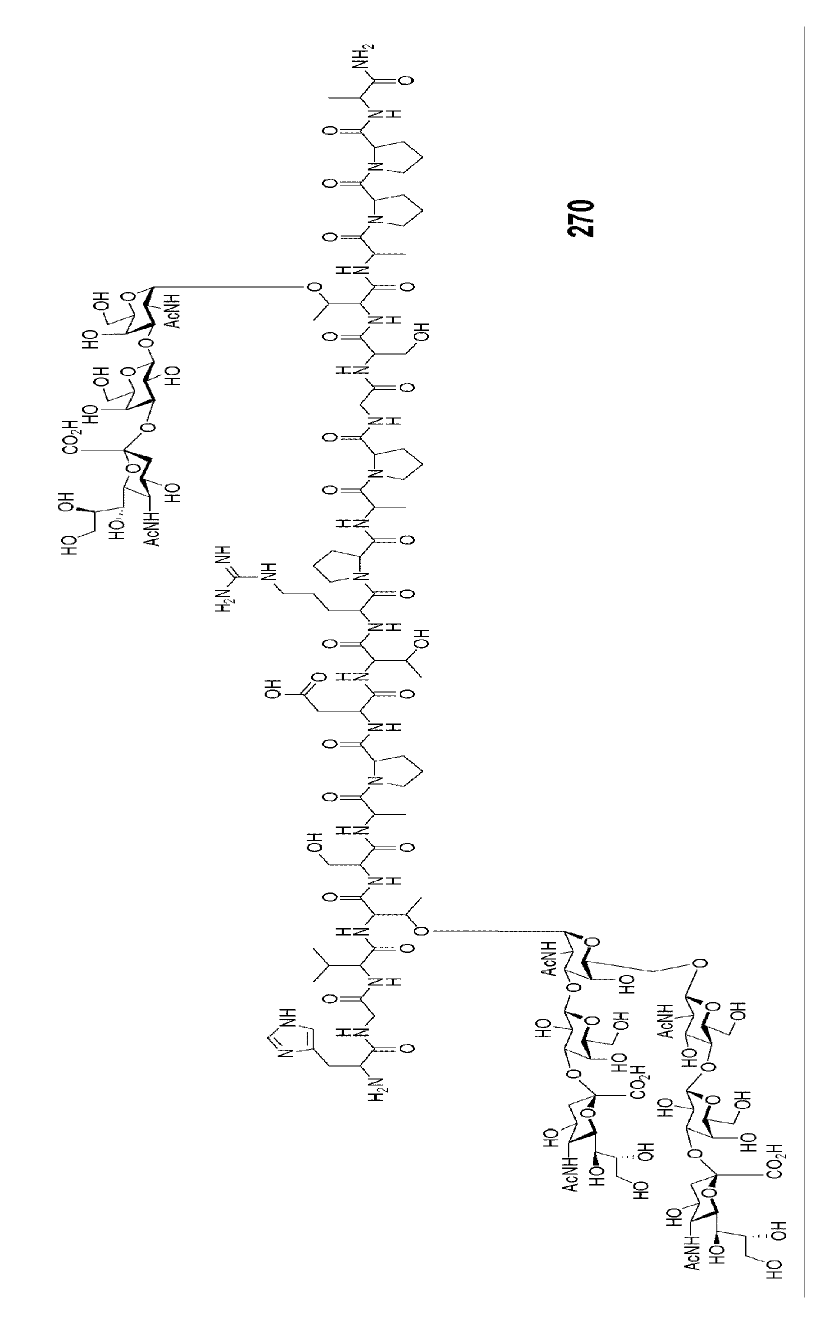

The following reaction solutions A) to E) were allowed to react for 24 hours at 25°C:
A) 100µl of reaction solution containing 50mMHEPES buffer solution (pH 7.0), 0.1U/ml human-derived β1,4-galactosyltransferase (available from TOYOBO CO., LTD.), 10mM manganese chloride, 0.1% BSA, 2mM uridine-5'-disodium diphosphogalactose (UDP-Gal) and glycopeptide derivative (182) (4mM at a theoretical content from solid-phase synthesis);
B) 100µl of reaction solution containing 50mM HEPES buffer solution (pH 7.0), 0.0175U/ml recombinant rat α2,3-(O)-sialyltransferase (available from Calbiochem), 10mM manganese chloride, 0.1% BSA, 2mM cytidine-5'-sodium monophosphosialate (CMP-NANA) and glycopeptide derivative (182) (4mM at a theoretical content from solid-phase synthesis);
C) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (182) (4mM at a theoretical content from solid-phase synthesis);
D) 100µl of reaction solution containing 50mM HEPES buffer solution (pH 7.0), 0.1U/ml human-derived β1,4-galactosyltransferase (available from TOYOBO CO., LTD.), 0.0185U/ml recombinant rat α2,3-(N)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (182) (4mM at a theoretical content from solid-phase synthesis);
E) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 0.0185U/ml recombinant rat α2,3-(N)-sialyltransferase (available from Calbiochem), 10mM manganese chloride, 0.1% BSA, 4mM uridine-5'-disodium diphosphogalactose (UDP-Gal), 2mM cytidine-5'-sodium monophosphosialate (CMP-NANA) and glycopeptide derivative (182) (4mM at a theoretical content from solid-phase synthesis).

After completion of the reaction, the respective reaction solutions were subjected to centrifugal concentration with an ultrafiltration filter. Thereafter, 25mM ammonium acetate buffer solution (pH 6.5) was added thereto, and the solution was again subjected to concentration with a centrifugal separator, thereby washing the polymer. This manipulation was repeated three times, thereby respectively obtaining aqueous solutions of compounds (260) to (270). Thereafter, to the solution retained at the filter containing compounds (260) to (270) and a solution which was obtained by diluting an aqueous solution of (182) with 25µM ammonium acetate buffer solution (pH 6.5), 1µl of 0.174mg/ml solution of BLase (available from Shionogi & Co., Ltd.) was added, and the solution was allowed to react for two hours at room temperature. Thereafter, the solution was subjected to centrifugal filtration with an ultrafiltration filter, thereby separating the glycopeptide of interest from the polymer. The obtained aqueous solution (filtrate) was lyophilized, thereby obtaining compounds (265) to (270).
MALDI TOF/MS:[M(average)+H]⁺ of compound (265)=2822.9 (theoretical value:[M(average)+H]⁺=2820.9); MALDI TOF/MS:[M(average)+H]⁺ of compound (266)=2983.7 (theoretical value:[M(average)+H]⁺=2983.0); MALDI TOF/MS:[M(average)+H]⁺ of compound (267)=3405.1 (theoretical value:[M(average)+H]⁺=3403.4); MALDI TOF/MS:[M(average)+H]⁺ of compound (268)=3567.1 (theoretical value:[M(average)+H]⁺=3565.5); MALDI TOF/MS:[M(average)+H]⁺ of compound (269)=3275.2 (theoretical value:[M(average)+H]⁺=3274.3); and MALDI TOF/MS:[M(average)+H]⁺ of compound (270)=3858.4 (theoretical value:[M(average)+H]⁺=3856.8).

(3.55 Synthesis of compounds (271) to (181))

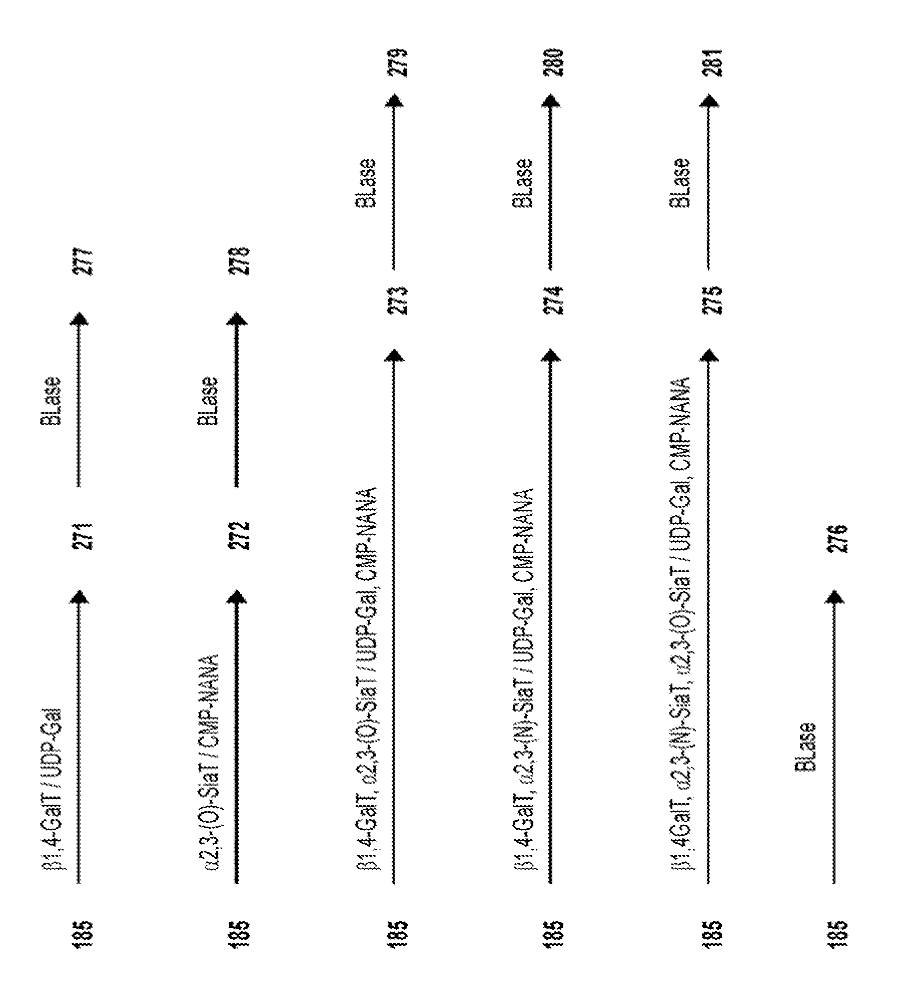

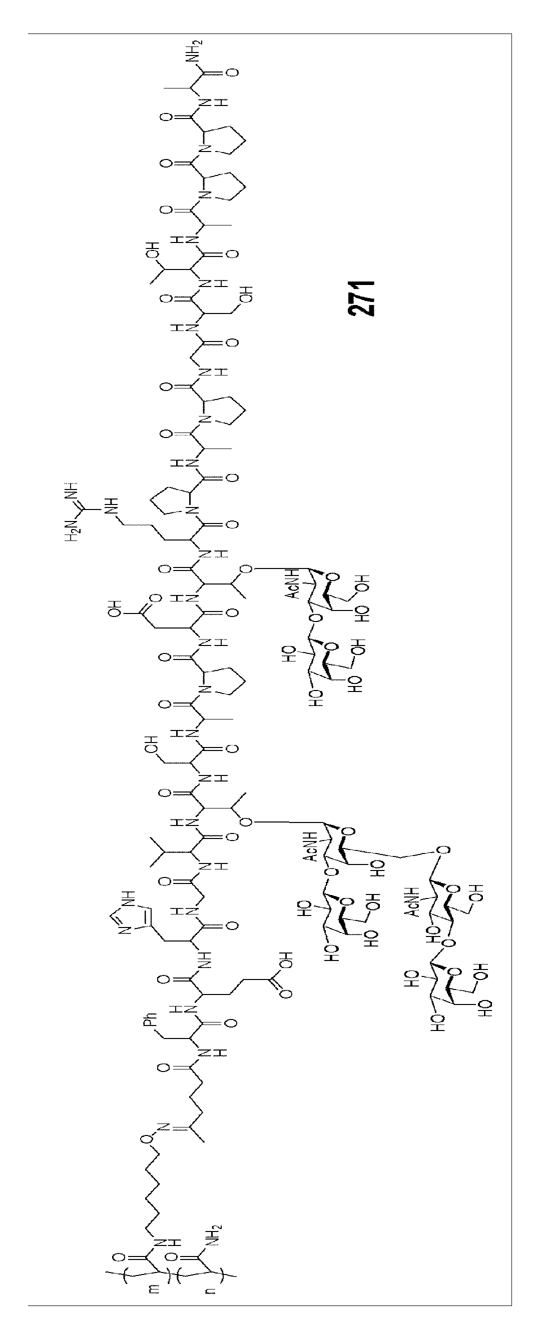

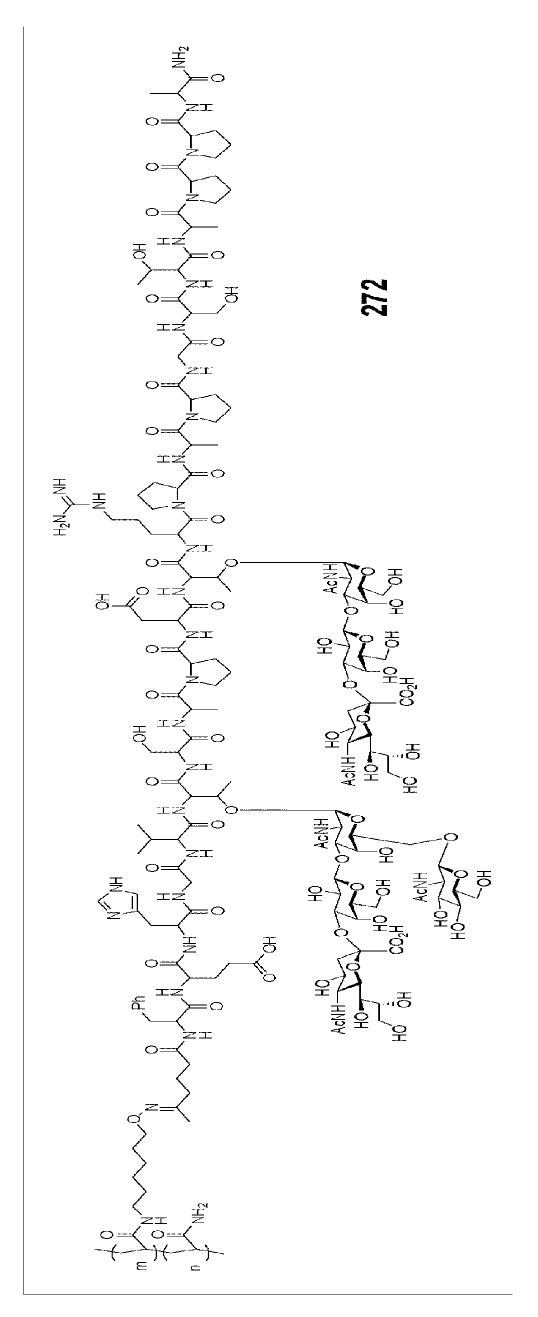

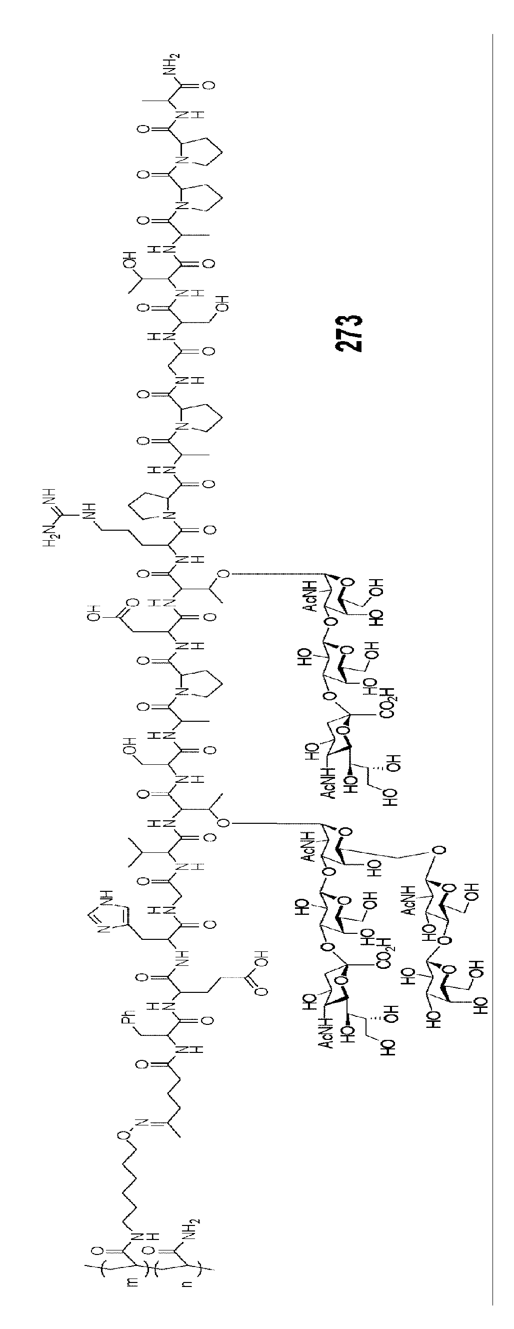

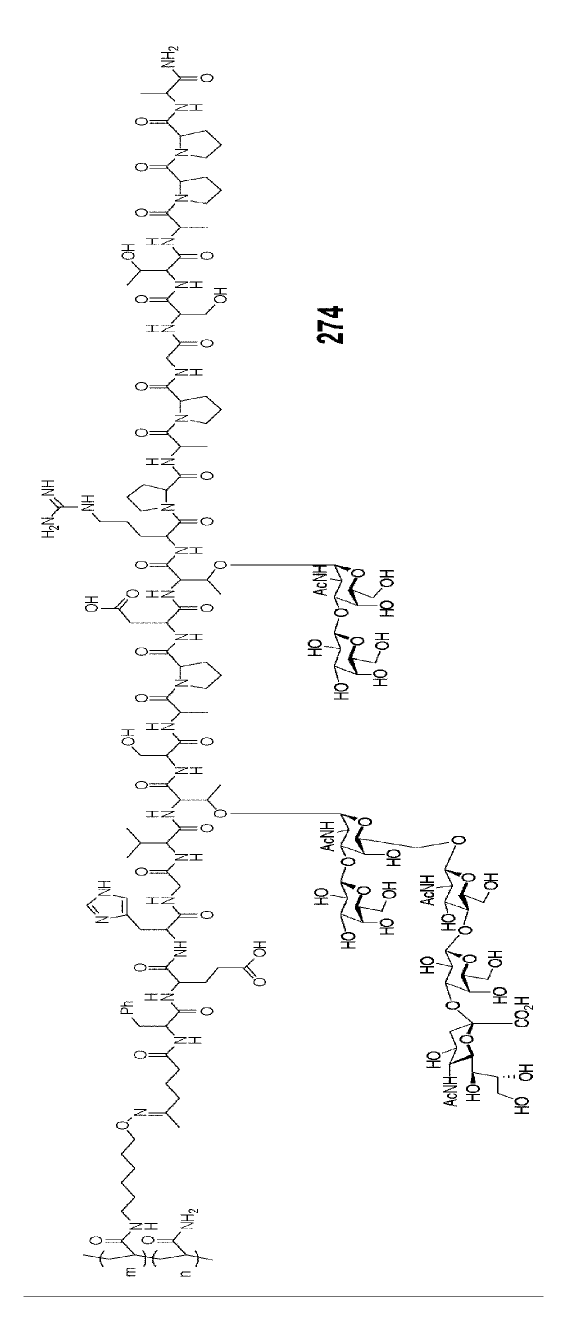

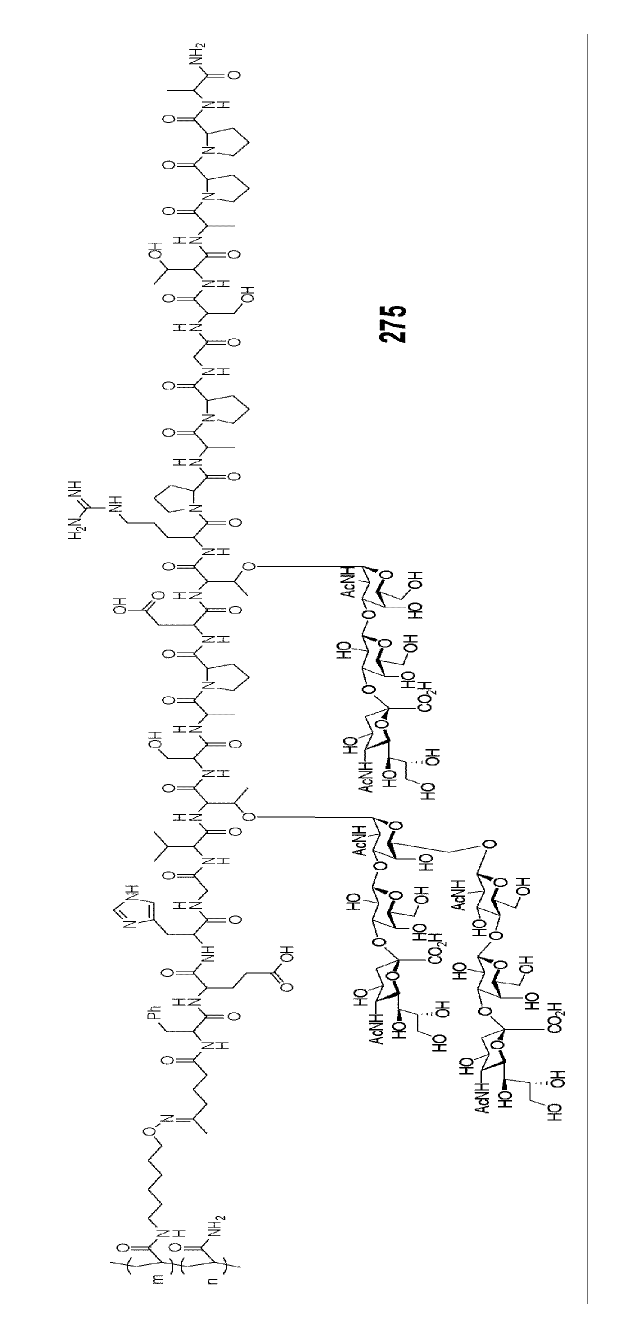

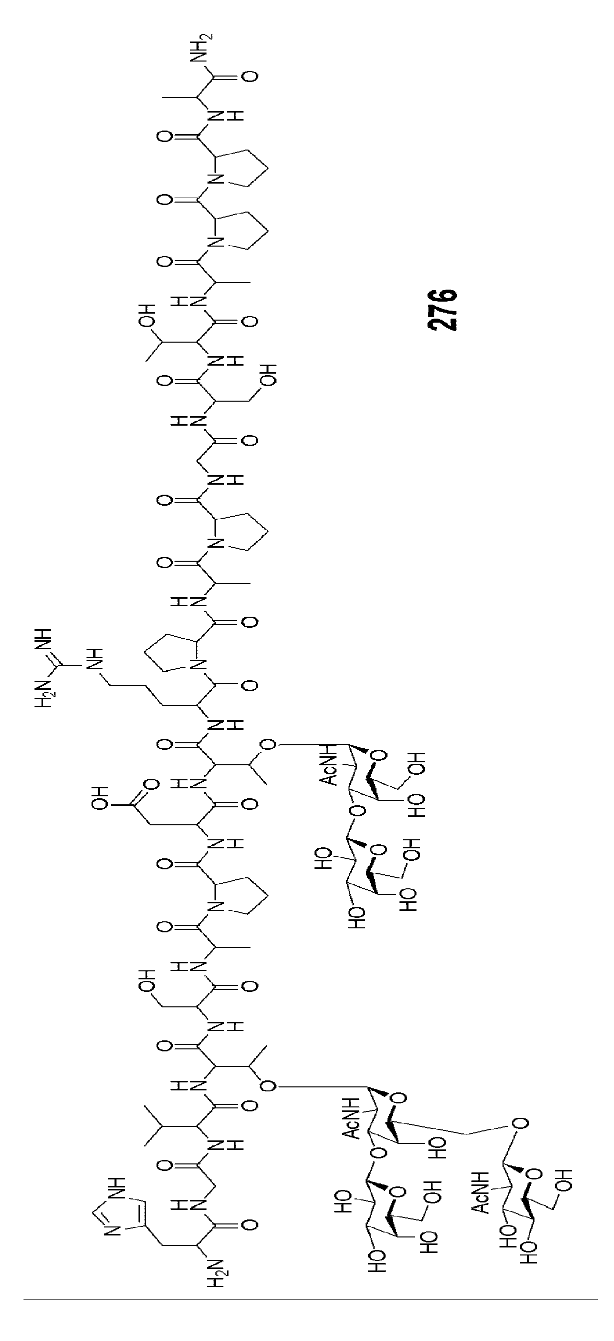

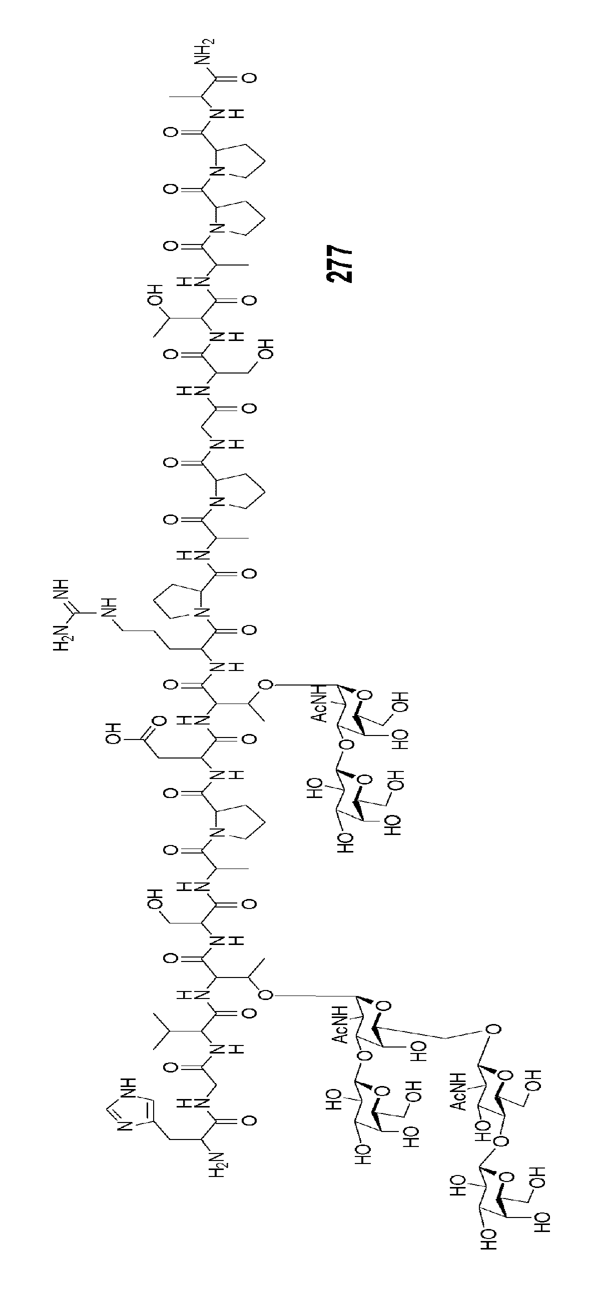

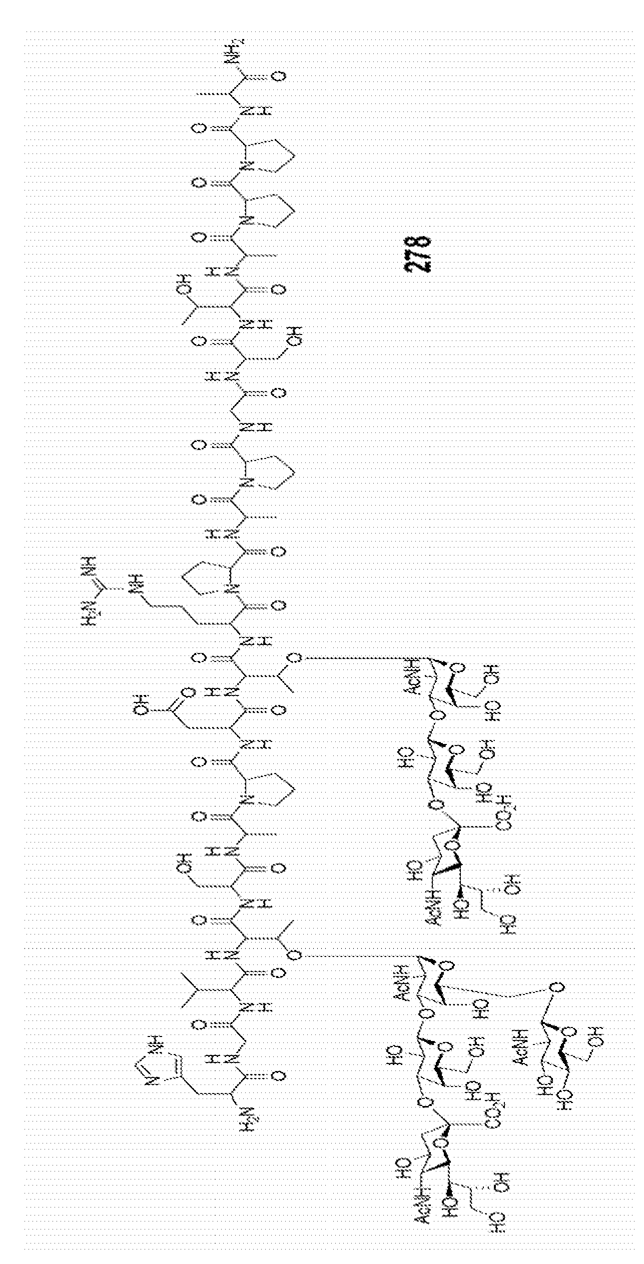

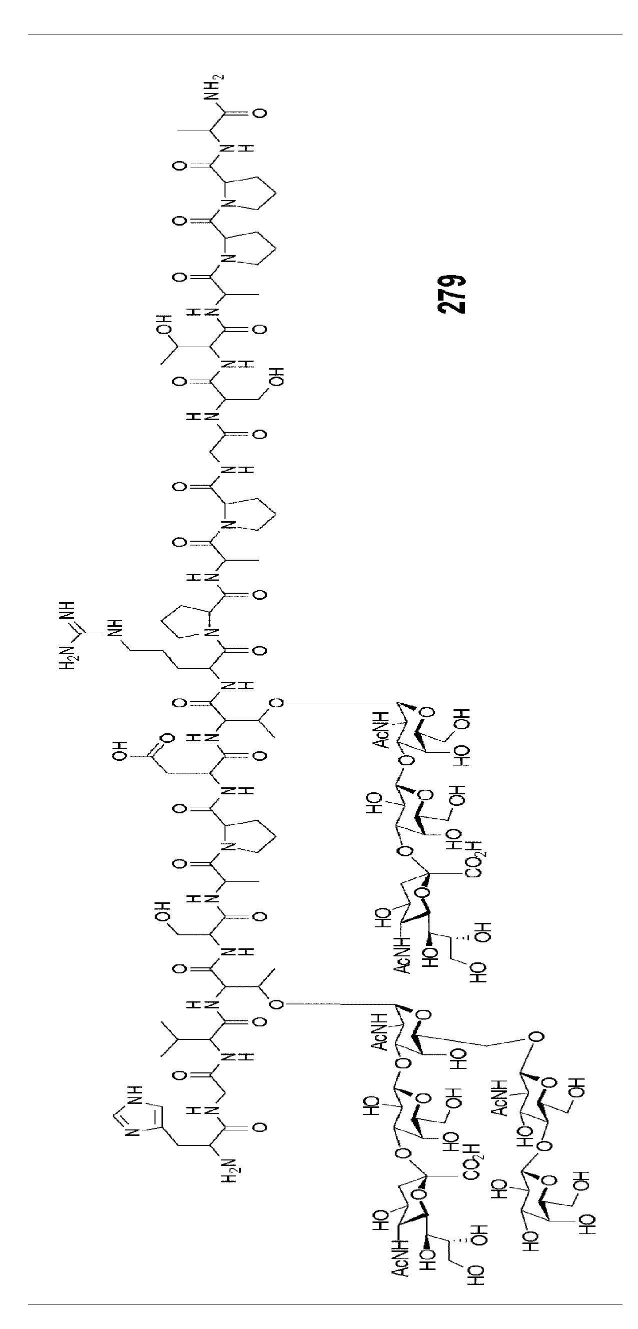

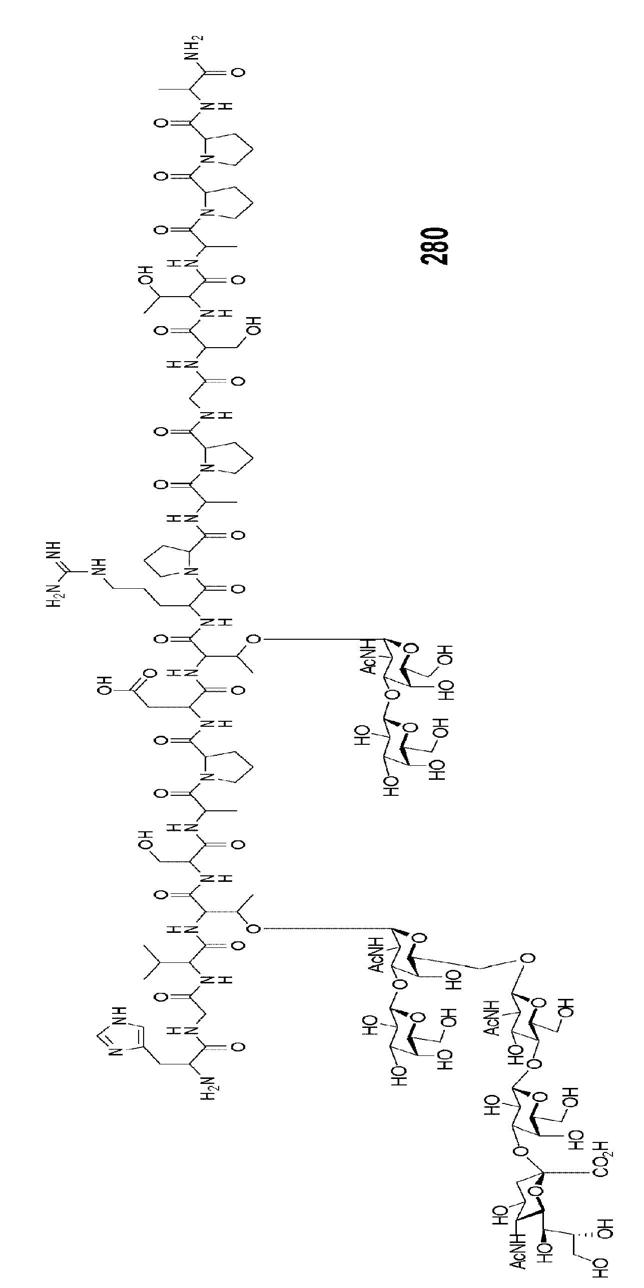

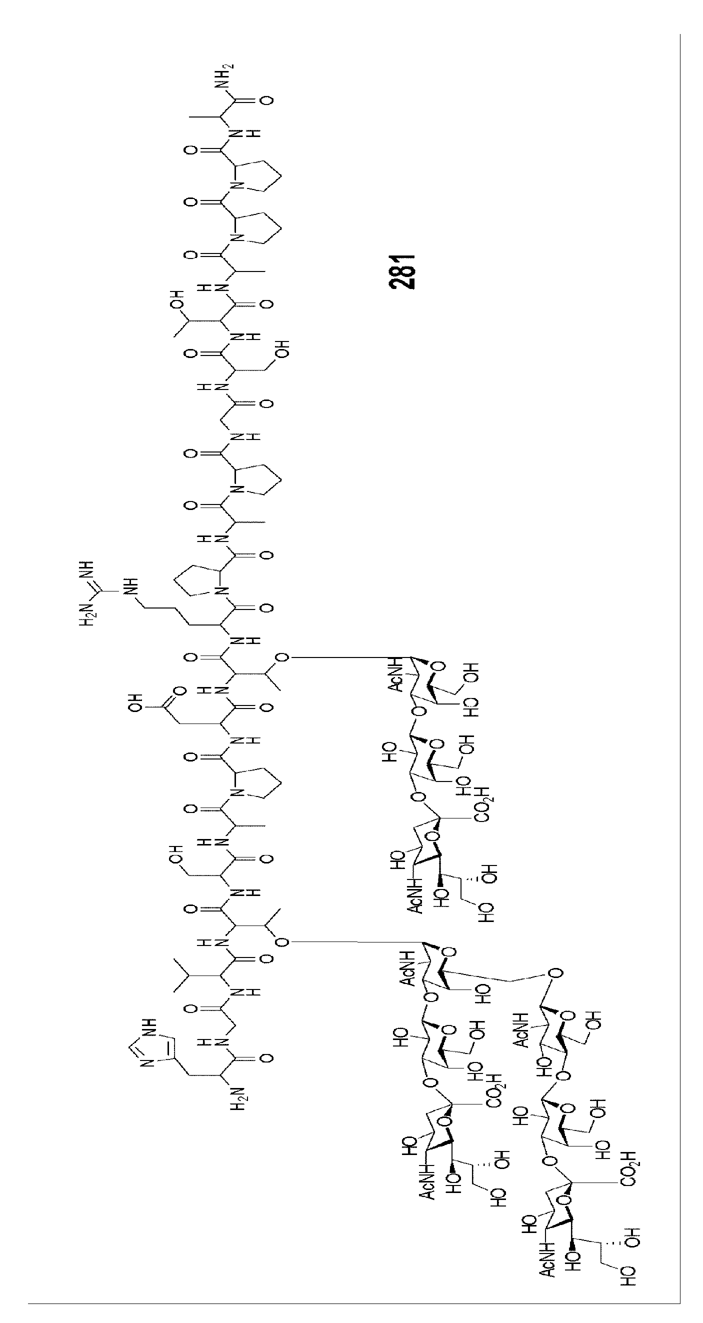

The following reaction solutions A) to E) were allowed to react for 24 hours at 25°C:
A) 100µl of reaction solution containing 50mMHEPES buffer solution (pH 7.0), 0.1U/ml human-derived β1,4-galactosyltransferase (available from TOYOBO CO., LTD.), 10mM manganese chloride, 0.1% BSA, 2mM uridine-5'-disodium diphosphogalactose (UDP-Gal) and glycopeptide derivative (185) (4mM at a theoretical content from solid-phase synthesis);
B) 100µl of reaction solution containing 50mM HEPES buffer solution (pH 7.0), 0.0175U/ml recombinant rat α2,3-(O)-sialyltransferase (available from Calbiochem), 10mM manganese chloride, 0.1% BSA, 2mM cytidine-5'-sodium monophosphosialate (CMP-NANA) and glycopeptide derivative (185) (4mM at a theoretical content from solid-phase synthesis);
C) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (185) (4mM at a theoretical content from solid-phase synthesis);
D) 100µl of reaction solution containing 50mM HEPES buffer solution (pH 7.0), 0.1U/ml human-derived β1,4-galactosyltransferase (available from TOYOBO CO., LTD.), 0.0185U/ml recombinant rat α2,3-(N)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (185) (4mM at a theoretical content from solid-phase synthesis);
E) 100µl of reaction solution containing 50mMH EPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 0.0185U/ml recombinant rat α2,3-(N)-sialyltransferase (available from Calbiochem), 10mM manganese chloride, 0.1% BSA, 4mM uridine-5'-disodium diphosphogalactose (UDP-Gal), 2mM cytidine-5'-sodium monophosphosialate (CMP-NANA) and glycopeptide derivative (185) (4mM at a theoretical content from solid-phase synthesis).

After completion of the reaction, the respective reaction solutions were subjected to centrifugal concentration with an ultrafiltration filter. Thereafter, 25mM ammonium acetate buffer solution (pH 6.5) was added thereto, and the solution was again subjected to concentration with a centrifugal separator, thereby washing the polymer. This manipulation was repeated three times, thereby respectively obtaining aqueous solutions of compounds (271) to (275). Thereafter, to the solution retained at the filter containing compounds (271) to (275) and a solution which was obtained by diluting an aqueous solution of (185) with 25mM ammonium acetate buffer solution (pH 6.5), 1µl of 0.174mg/ml solution of BLase (available from Shionogi & Co., Ltd.) was added, and the solution was allowed to react for two hours at room temperature. Thereafter, the solution was subjected to centrifugal filtration with an ultrafiltration filter, thereby separating the glycopeptide of interest from the polymer. The obtained aqueous solution (filtrate) was lyophilized, thereby obtaining compounds (276) to (281).
MALDI-TOF/MS:[M(average)+H]⁺ of compound (276)=2823.1 (theoretical value:[M(average)+H]⁺=2820.9); MALDI-TOF/MS:[M(average)+H]⁺ of compound (277)=2983.7 (theoretical value:[M(average)+H]⁺=2983.0); MALDI-TOF/MS:[M(average)+H]⁺ of compound (278)=3404.2 (theoretical value:[M(average)+H]⁺=3403.4); MALDI-TOF/MS:[M(average)+H]⁺ of compound (279)=3567.6 (theoretical value:[M(average)+H]⁺=3565.5); MALDI-TOF/MS:[M(average)+H]⁺ of compound (280)=3276.0 (theoretical value:[M(average)+H]⁺=3274.3); and MALDI-TOF/MS:[M(average)+H]⁺ of compound (281)=3659.3 (theoretical value:[M(average)+H]⁺=3656.8).

(3.56 Synthesis of compounds (282) to (292))

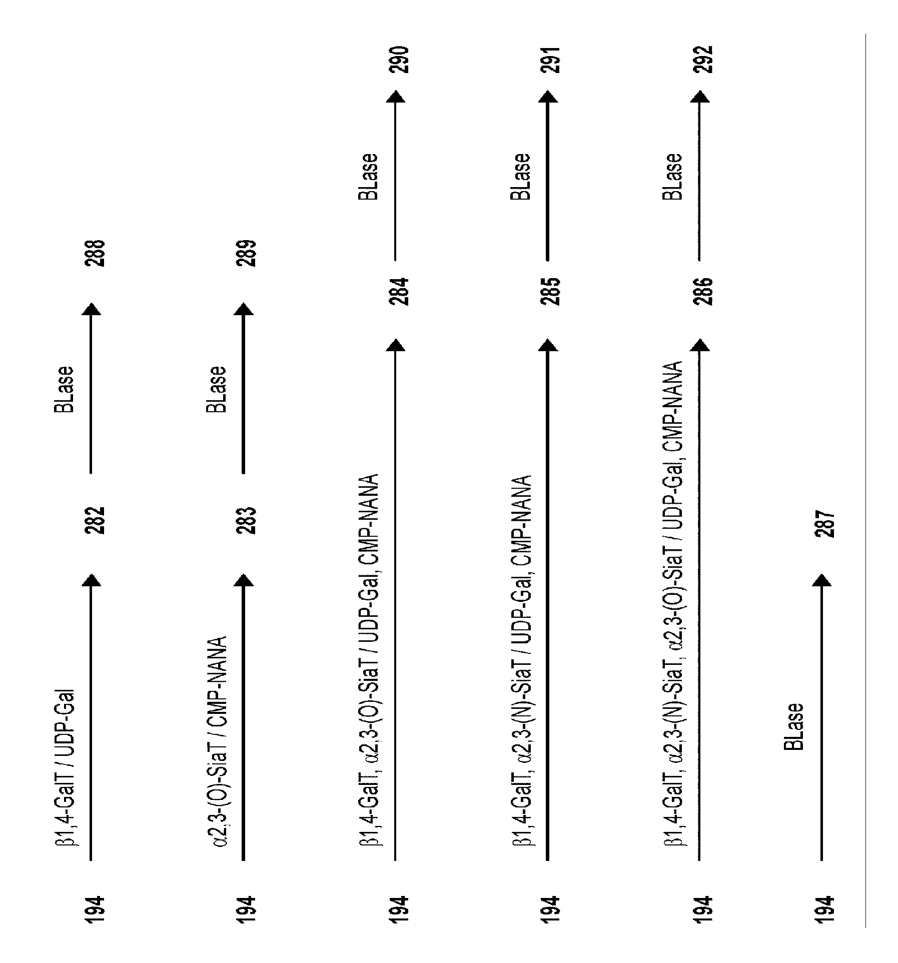

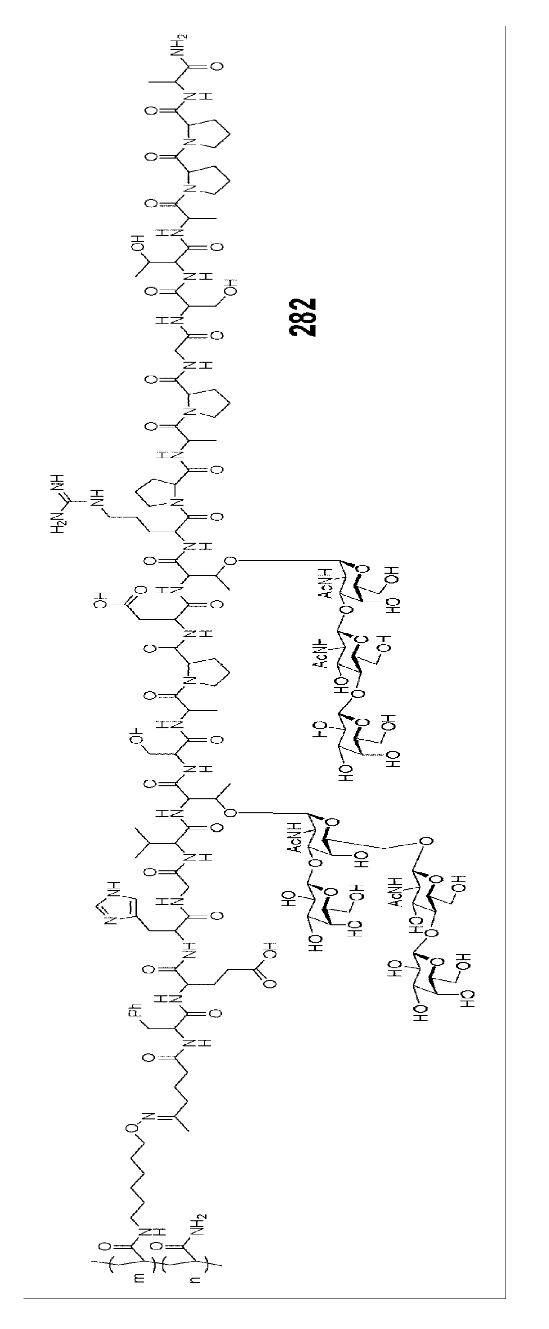

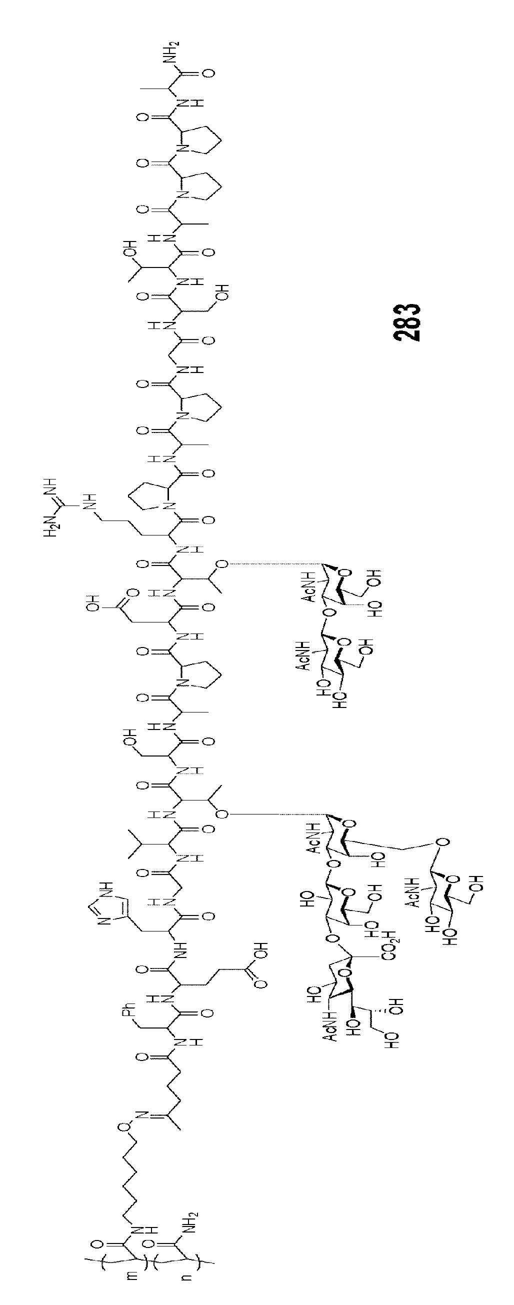

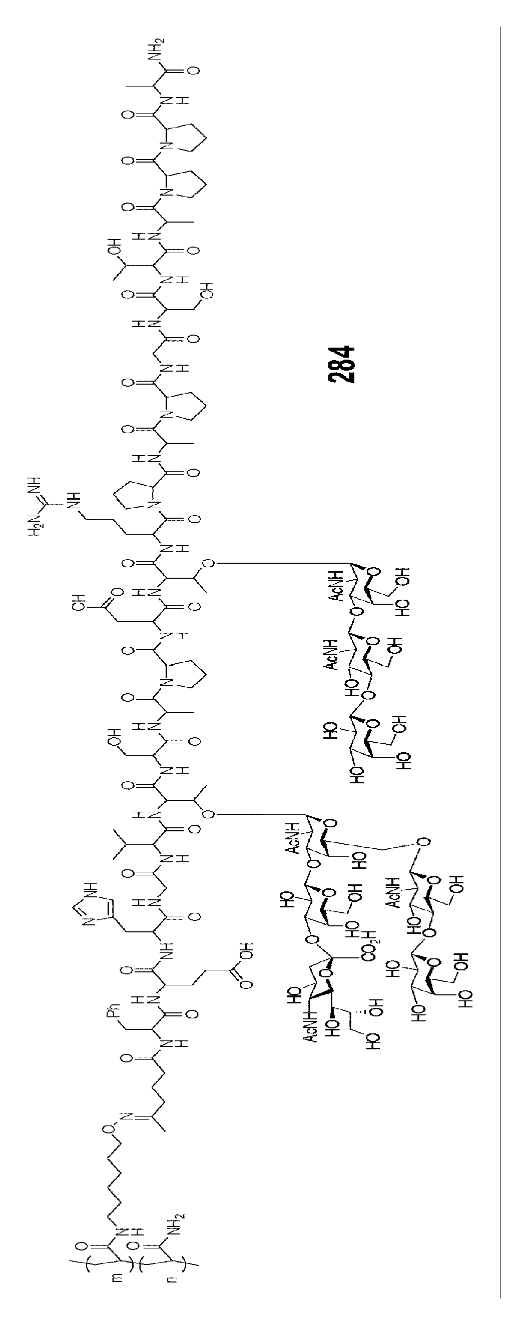

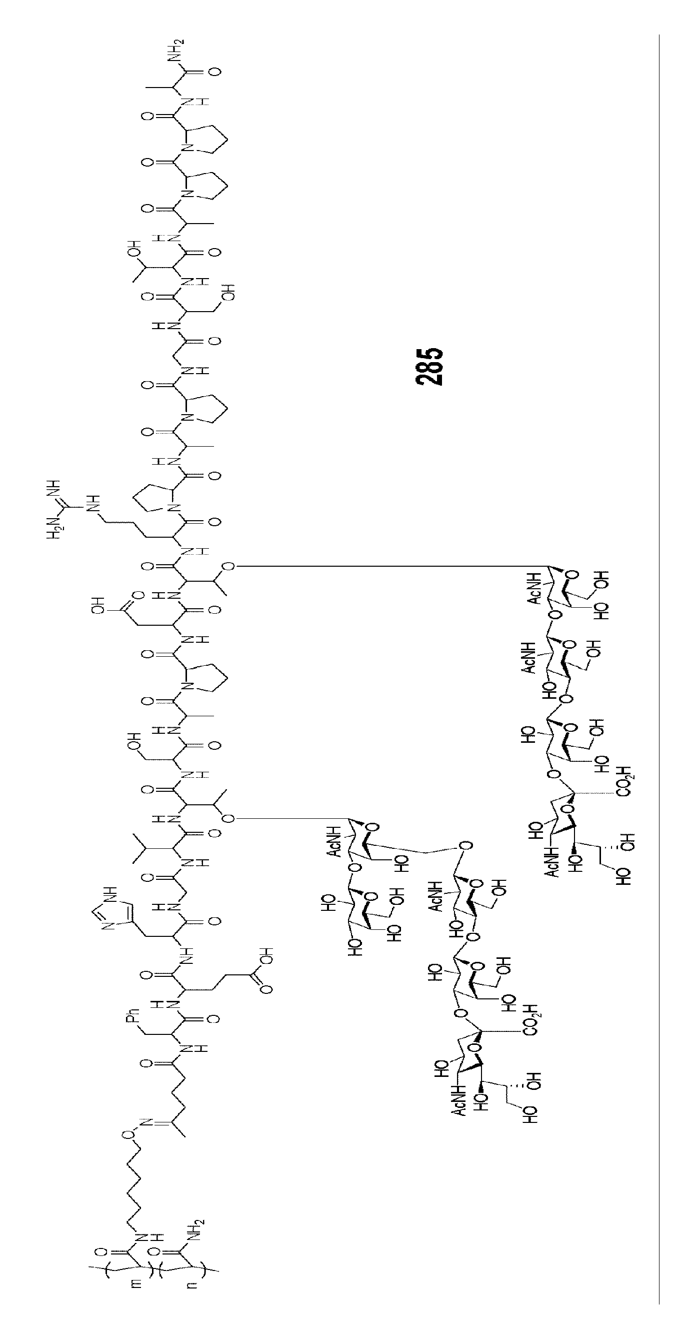

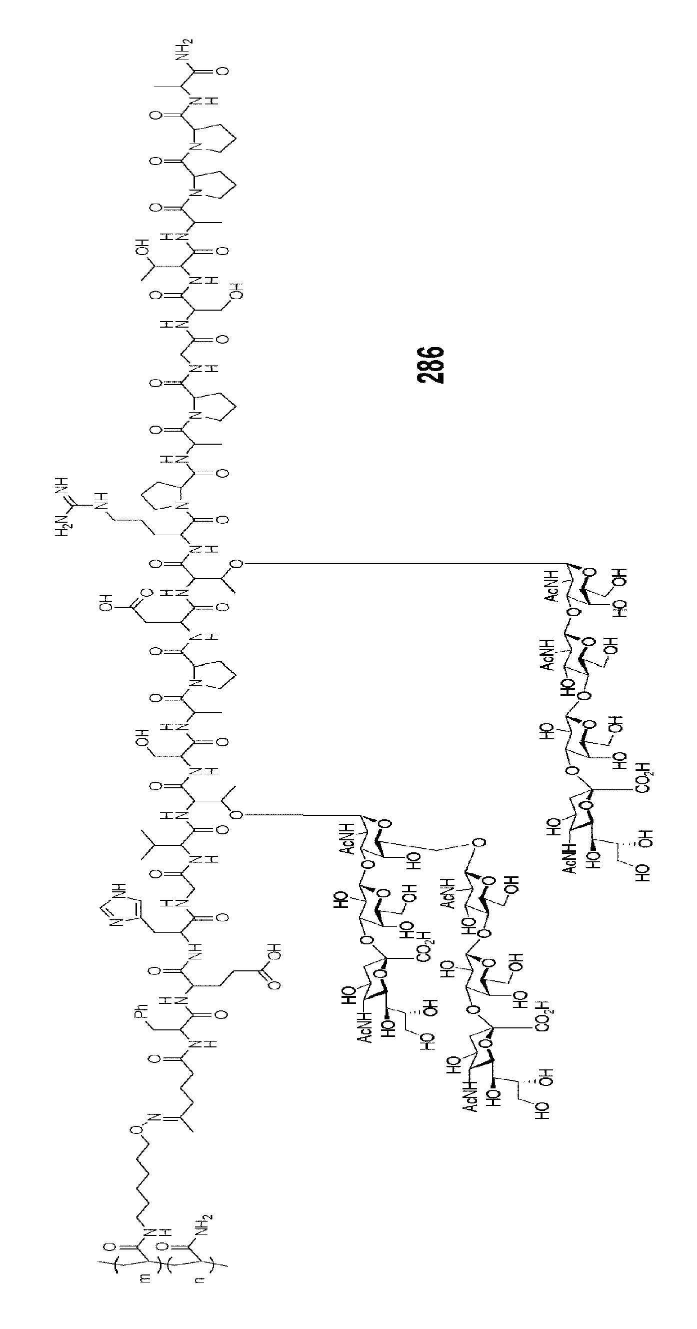

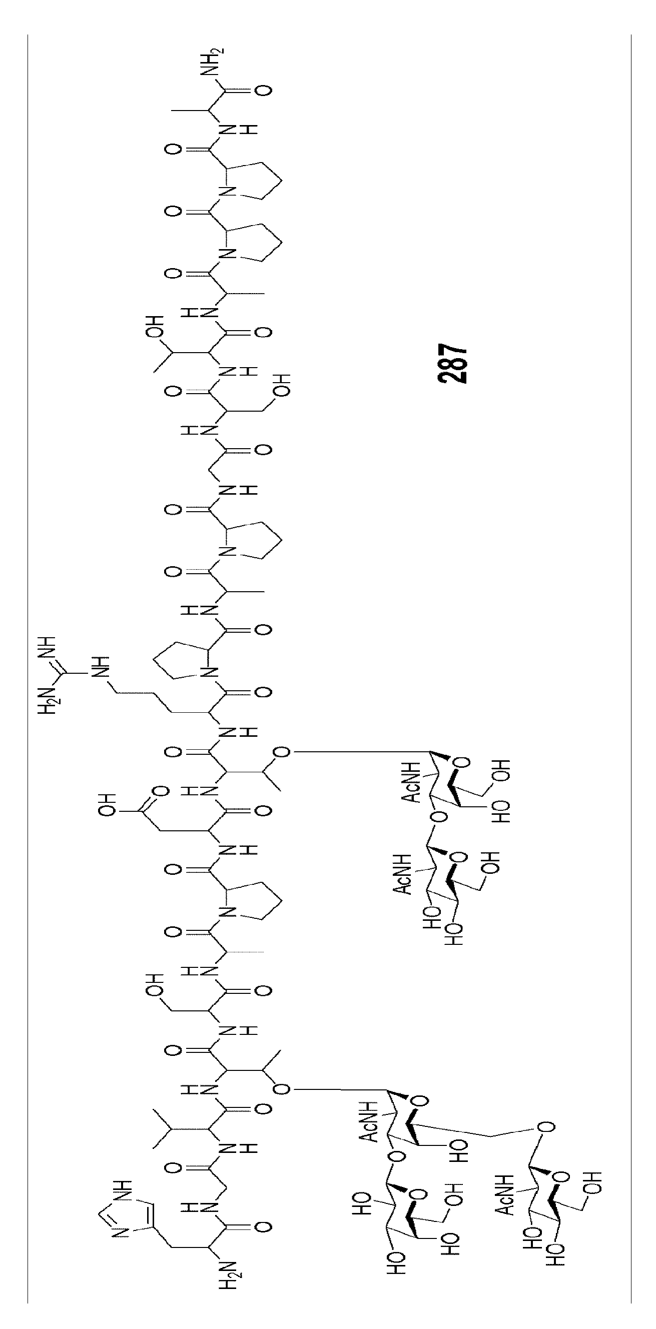

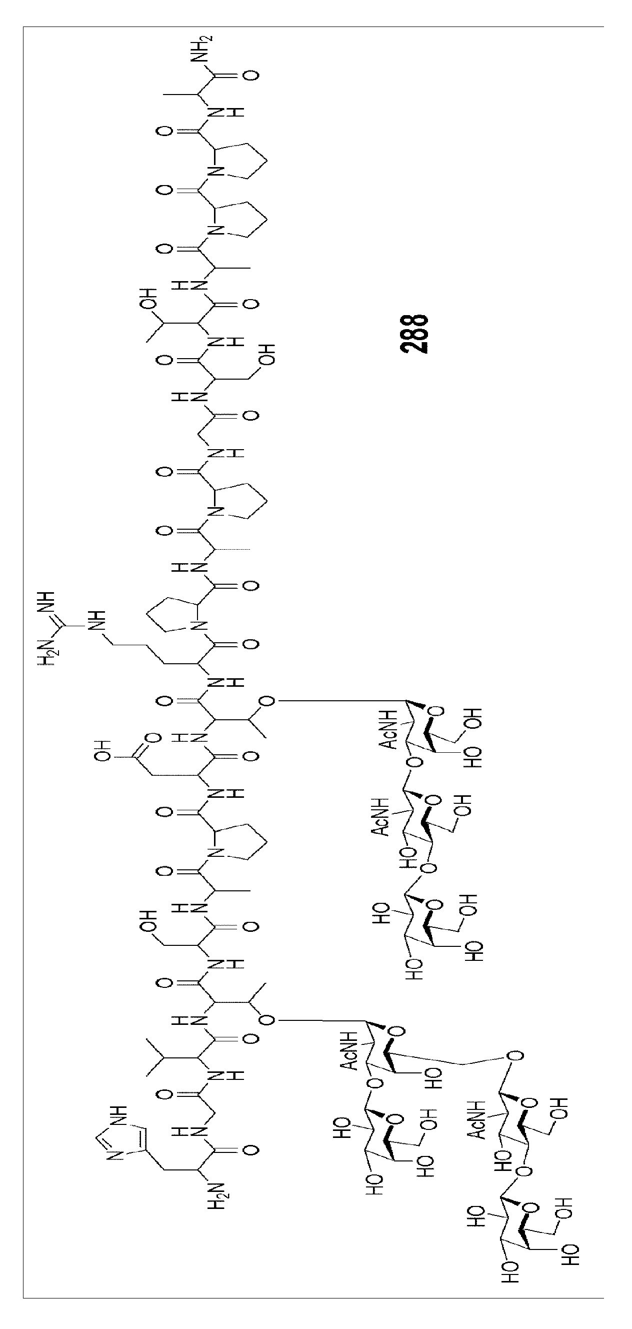

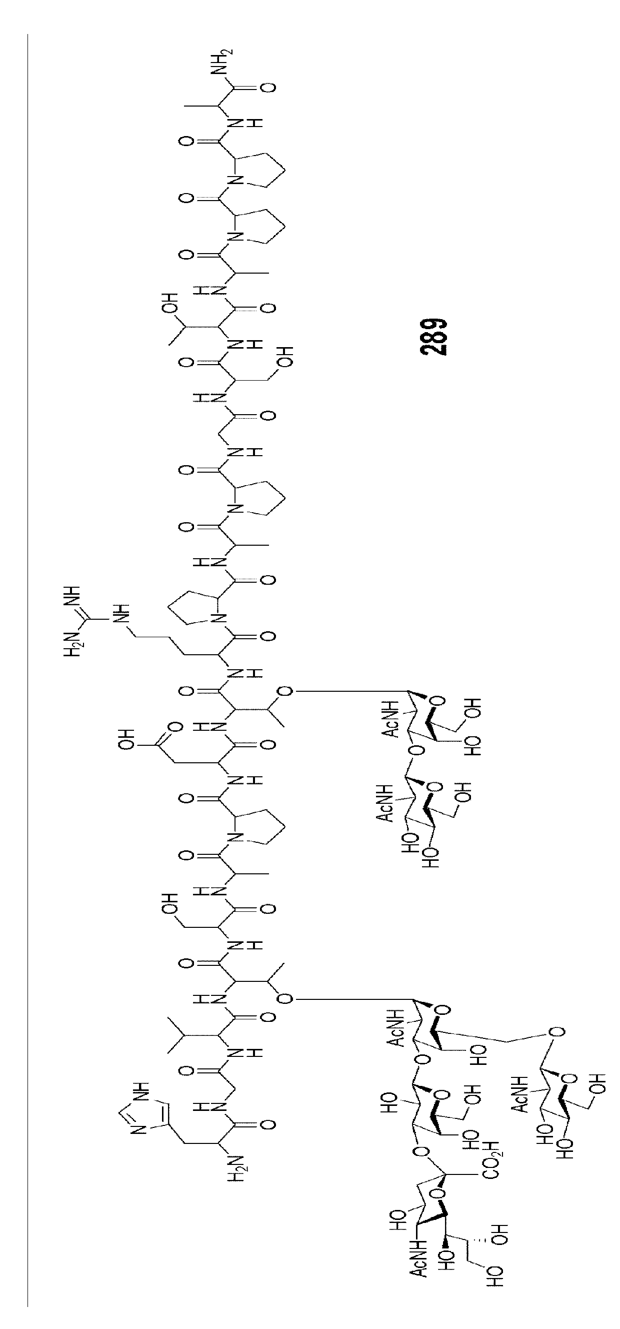

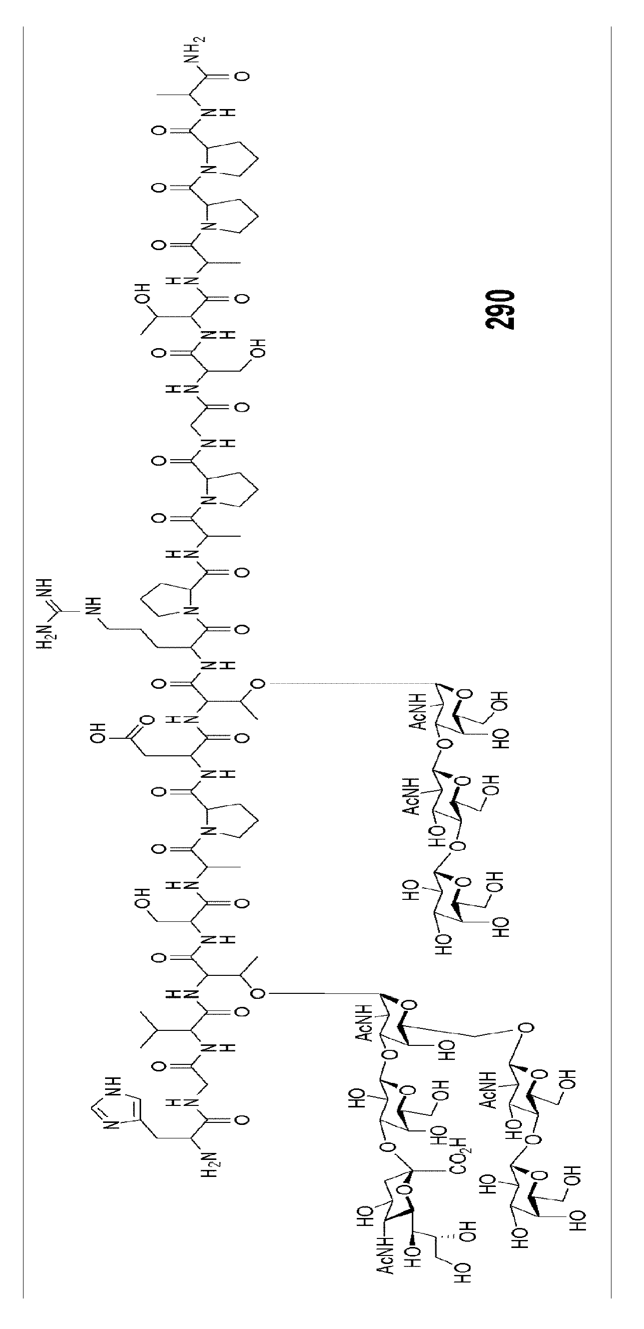

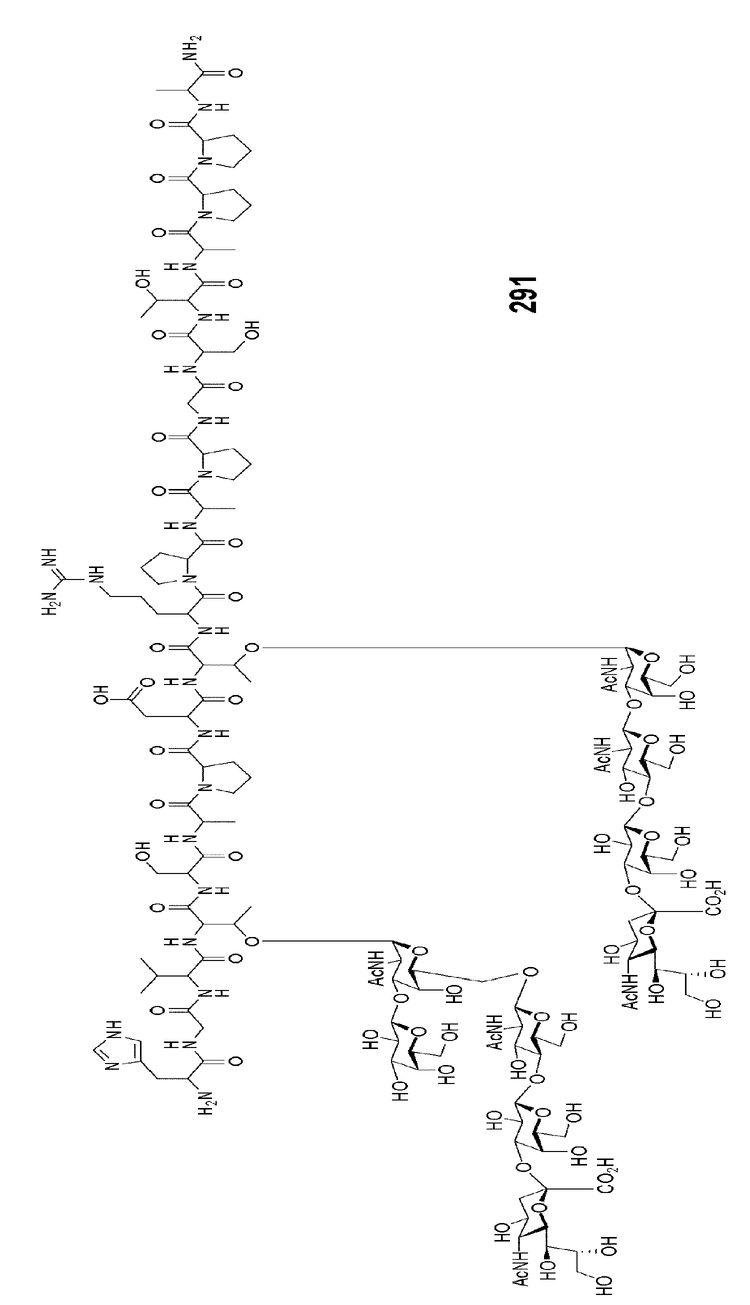

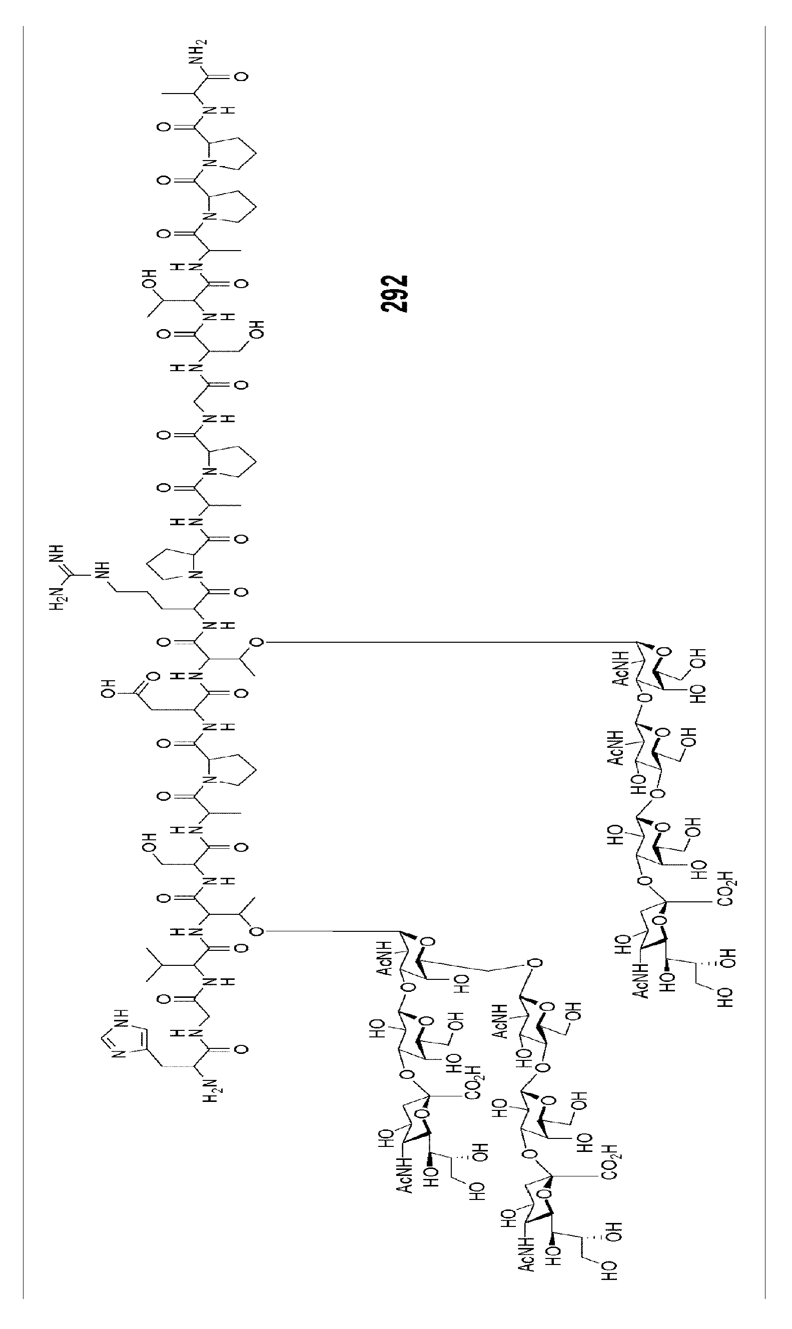

The following reaction solutions A) to E) were allowed to react for 24 hours at 25°C:
A) 100µl of reaction solution containing 50mMHEPES buffer solution (pH 7.0), 0.1U/ml human-derived β1,4-galactosyltransferase (available from TOYOBO CO., LTD.), 10mM manganese chloride, 0.1% BSA, 2mM uridine-5'-disodium diphosphogalactose (UDP-Gal) and glycopeptide derivative (194) (4mM at a theoretical content from solid-phase synthesis);
B) 100µl of reaction solution containing 50mM HEPES buffer solution (pH 7.0), 0.0175U/ml recombinant rat α2,3-(O)-sialyltransferase (available from Calbiochem), 10mM manganese chloride, 0.1% BSA, 2mM cytidine-5'-sodium monophosphosialate (CMP-NANA) and glycopeptide derivative (194) (4mM at a theoretical content from solid-phase synthesis);
C) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (194) (4mM at a theoretical content from solid-phase synthesis);
D) 100µl of reaction solution containing 50mM HEPES buffer solution (pH 7.0), 0.1U/ml human-derived β1,4-galactosyltransferase (available from TOYOBO CO., LTD.), 0.0185U/ml recombinant rat α2,3-(N)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (194) (4mM at a theoretical content from solid-phase synthesis);
E) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 0.0185U/ml recombinant rat α2,3-(N)-sialyltransferase (available from Calbiochem), 10mM manganese chloride, 0.1% BSA, 4mM uridine-5'-disodium diphosphogalactose (UDP-Gal), 2mM cytidine-5'-sodium monophosphosialate (CMP-NANA) and glycopeptide derivative (194) (4mM at a theoretical content from solid-phase synthesis).

After completion of the reaction, the respective reaction solutions were subjected to centrifugal concentration with an ultrafiltration filter. Thereafter, 25mM ammonium acetate buffer solution (pH 6.5) was added thereto, and the solution was again subjected to concentration with a centrifugal separator, thereby washing the polymer. This manipulation was repeated three times, thereby respectively obtaining aqueous solutions of compounds (282) to (286). Thereafter, to the solution retained at the filter containing compounds (282) to (286) and a solution which was obtained by diluting an aqueous solution of (194) with 25mM ammonium acetate buffer solution (pH 6.5), 1µl of 0.174mg/ml solution of BLase (available from Shionogi & Co., Ltd.) was added, and the solution was allowed to react for two hours at room temperature. Thereafter, the solution was subjected to centrifugal filtration with an ultrafiltration filter, thereby separating the glycopeptide of interest from the polymer. The obtained aqueous solution (filtrate) was lyophilized, thereby obtaining compounds (287) to (292).
MALDI-TOF/MS:[M(average)+H]⁺ of compound (287)=2864.1 (theoretical value:[M(average)+H]⁺=2861.9); MALDI-TOF/MS:[M(average)+H]⁺ of compound (288)=3187.8 (theoretical value:[M(average)+H]⁺=3186.2); MALDI-TOF/MS:[M(average)+H]⁺ of compound (289)=3154.9(theoretical value:[M(average)+H]⁺=3153.2); MALDI-TOF/MS:[M(average)+H]⁺ of compound (290)=3479.2 (theoretical value:[M(average)+H]⁺=3477.5); MALDI-TOF/MS:[M(average)+H]⁺ of compound (291)=3769.4 (theoretical value:[M(average)+H]⁺=3768.7); and MALDI-TOF/MS:[M(average)+H]⁺ of compound (292)=4062.4 (theoretical value:[M(average)+H]⁺=4060.0).

(3.57 Synthesis of compounds (293) to (303))

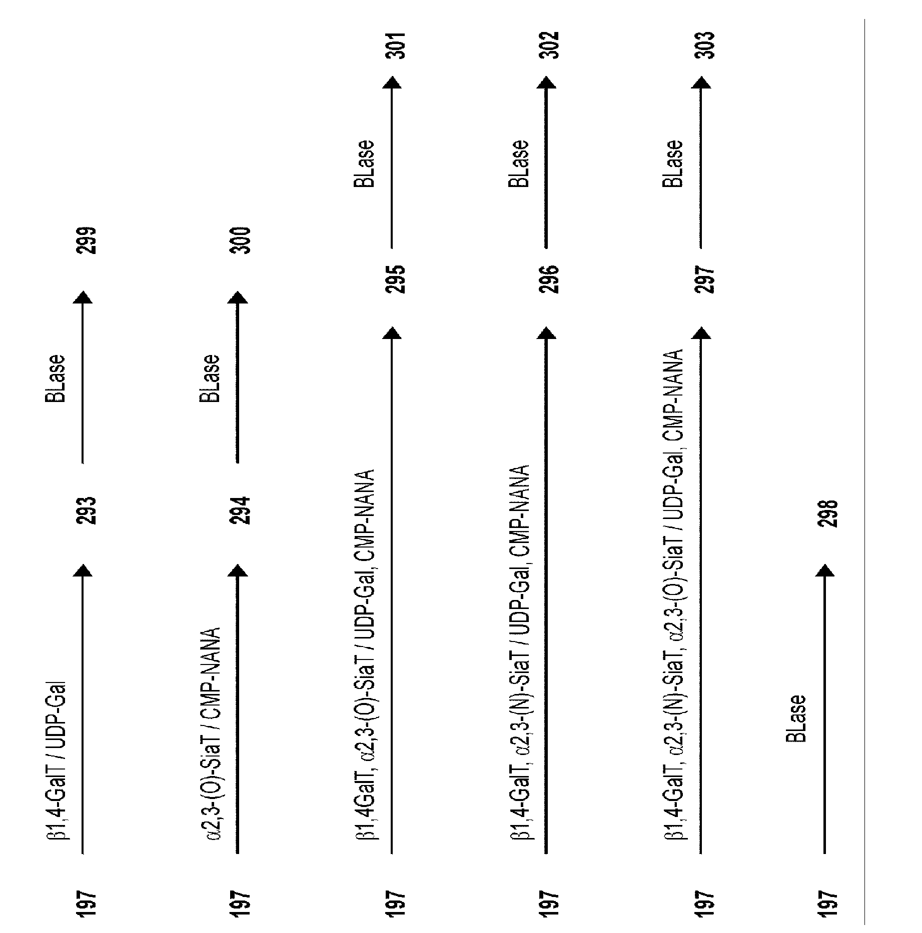

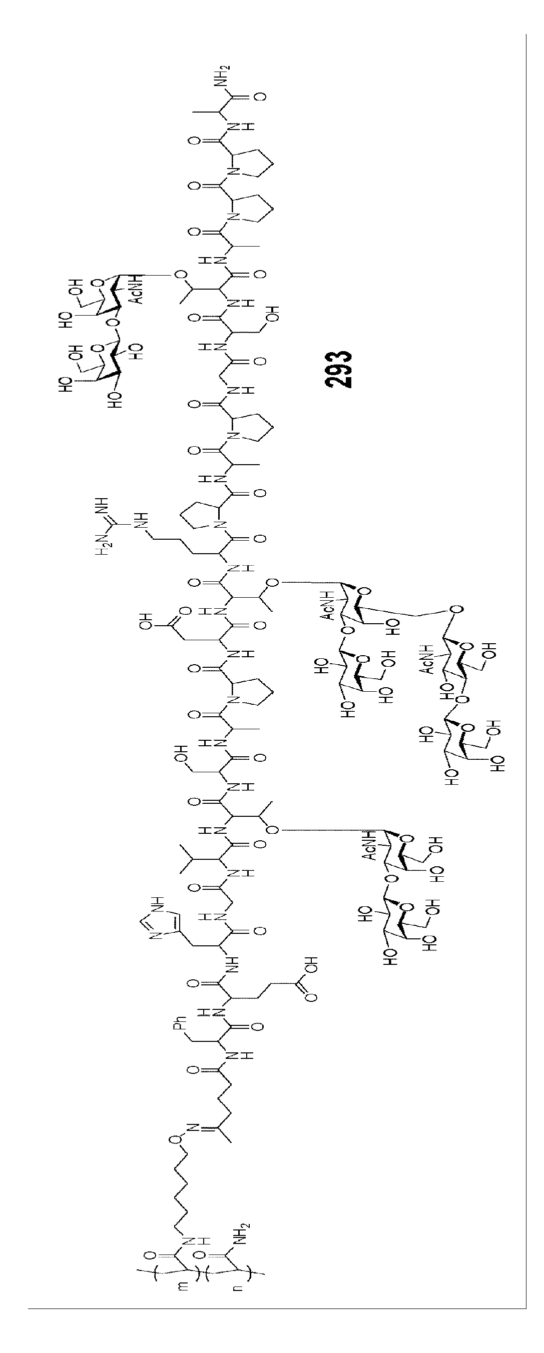

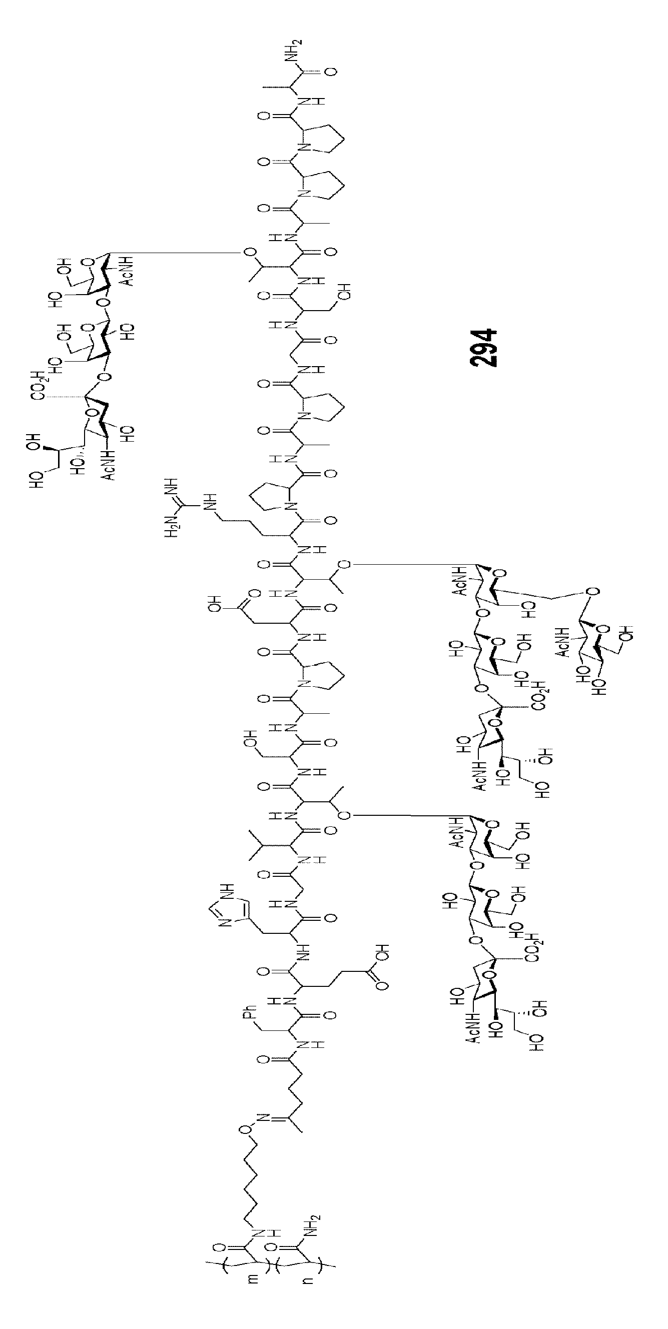

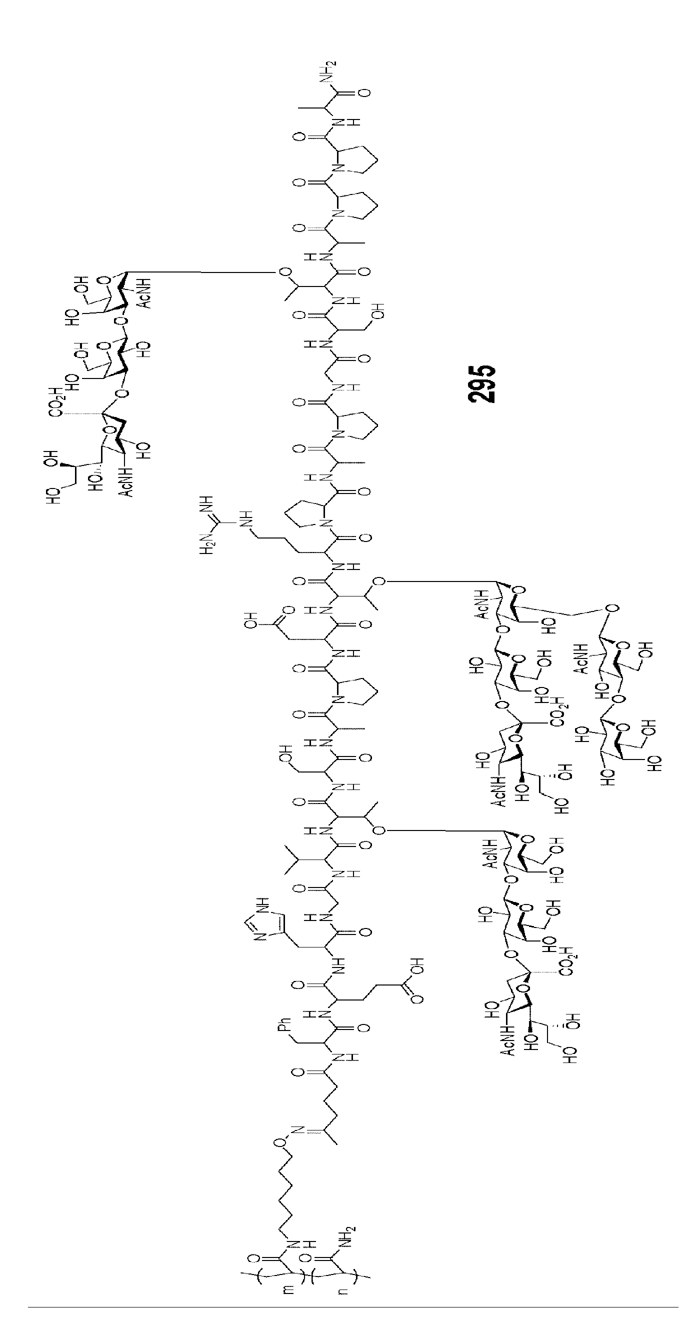

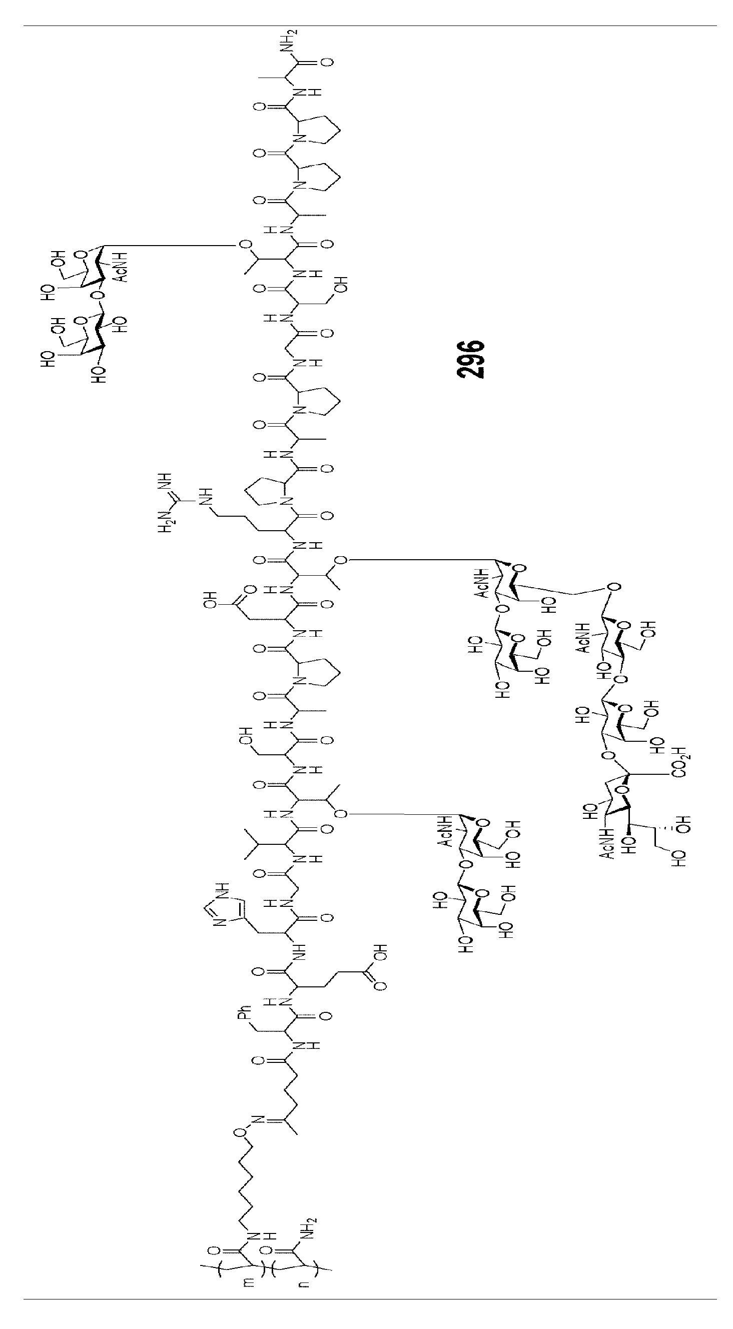

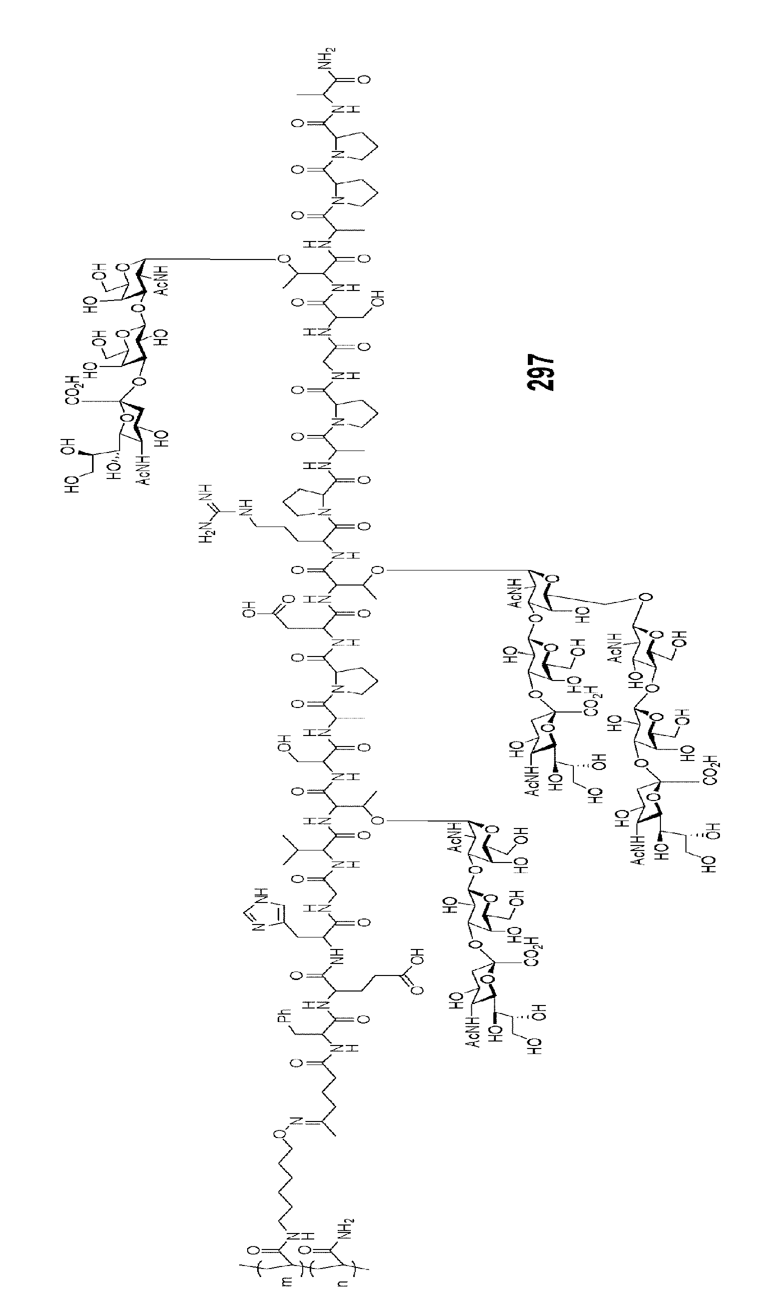

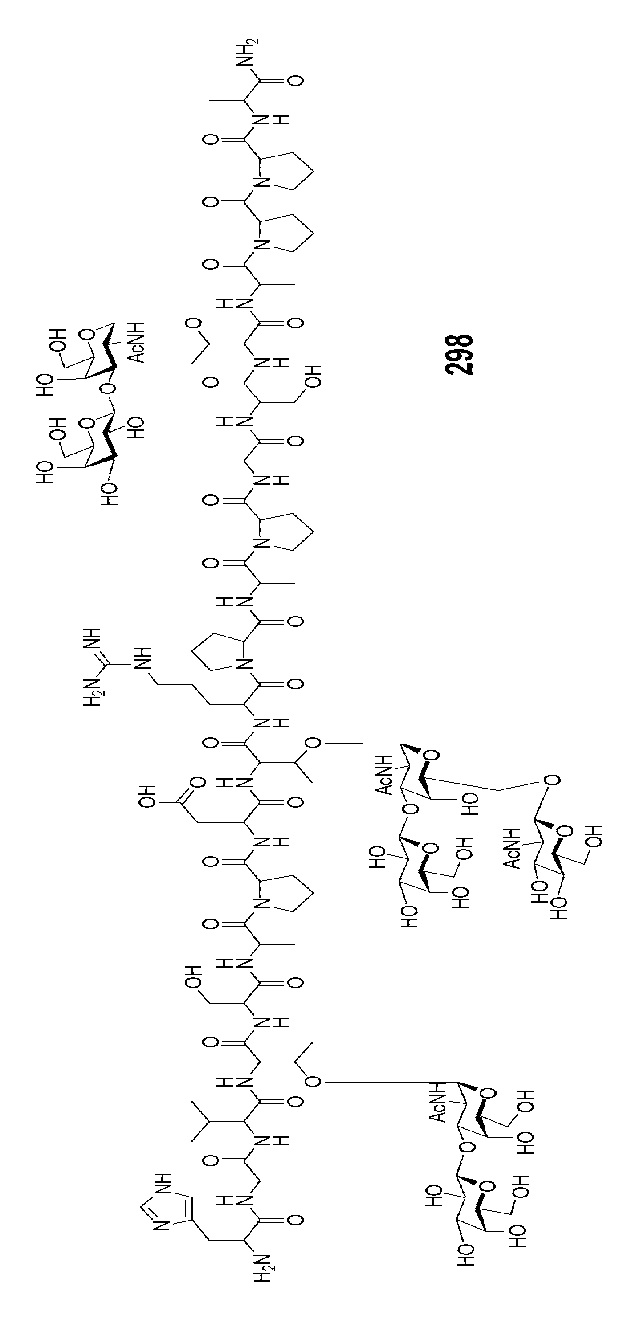

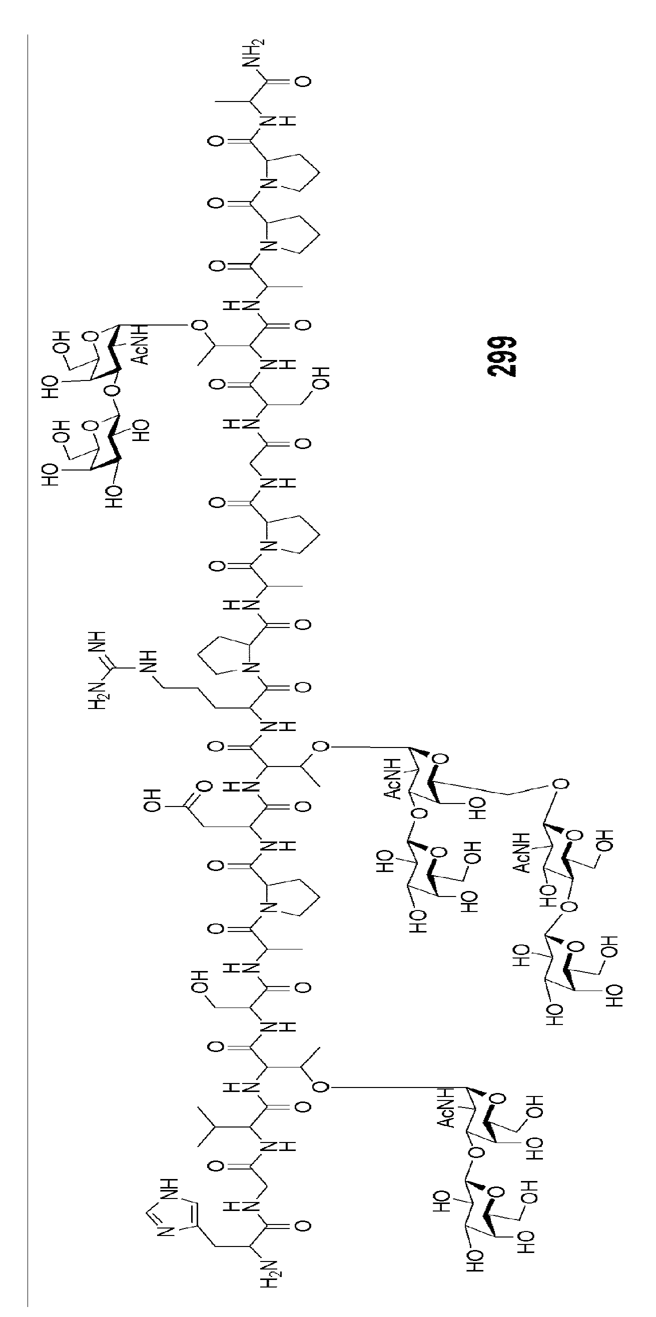

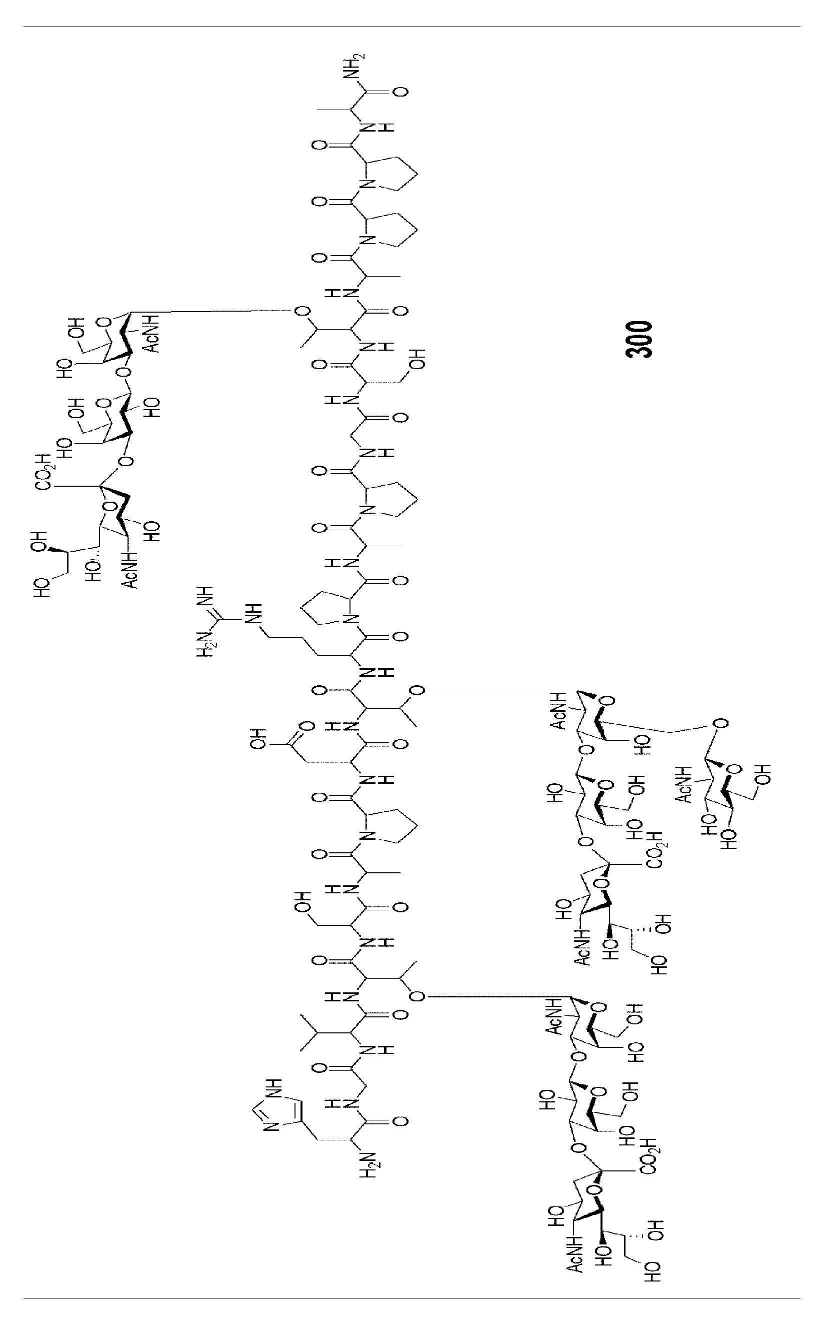

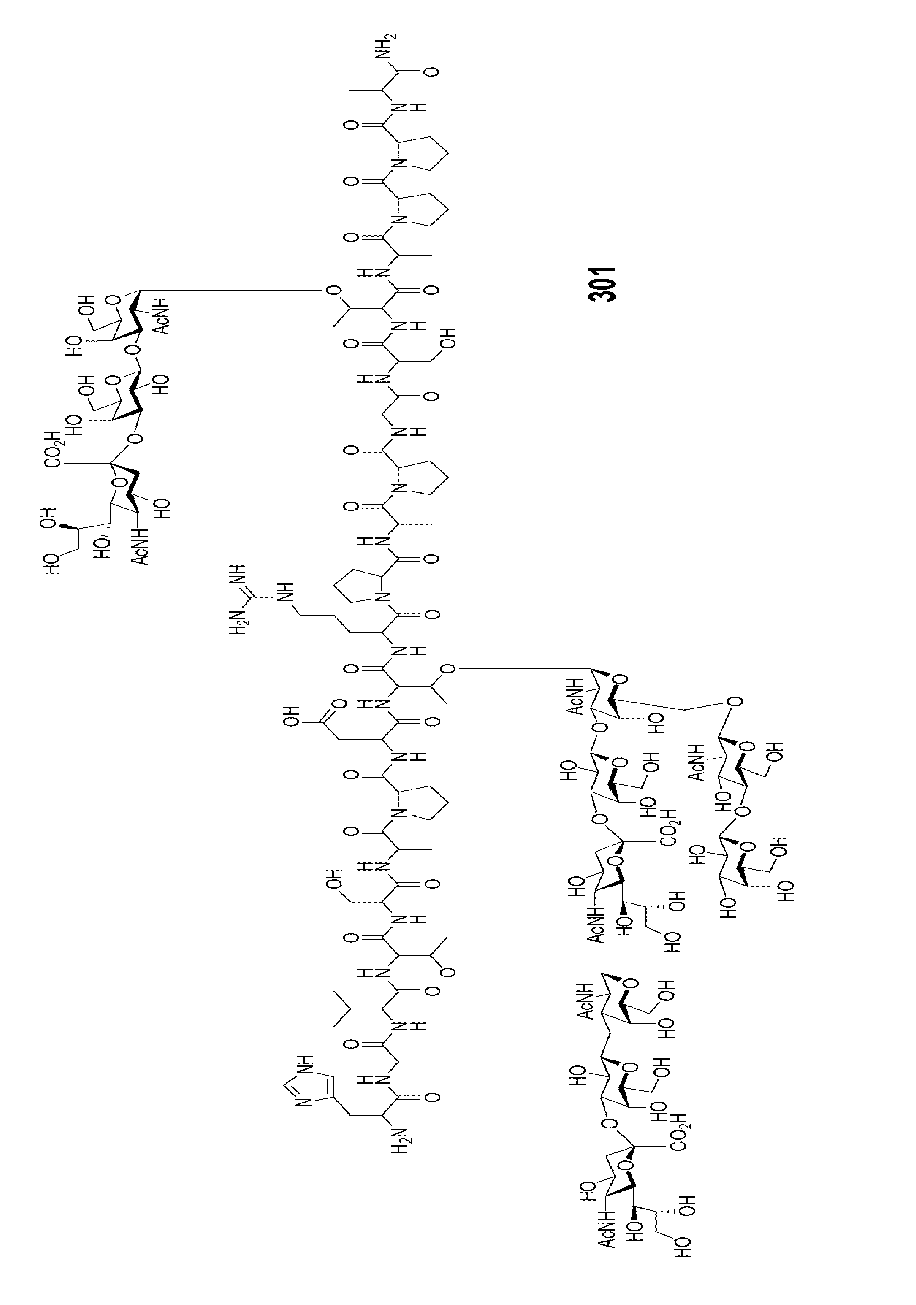

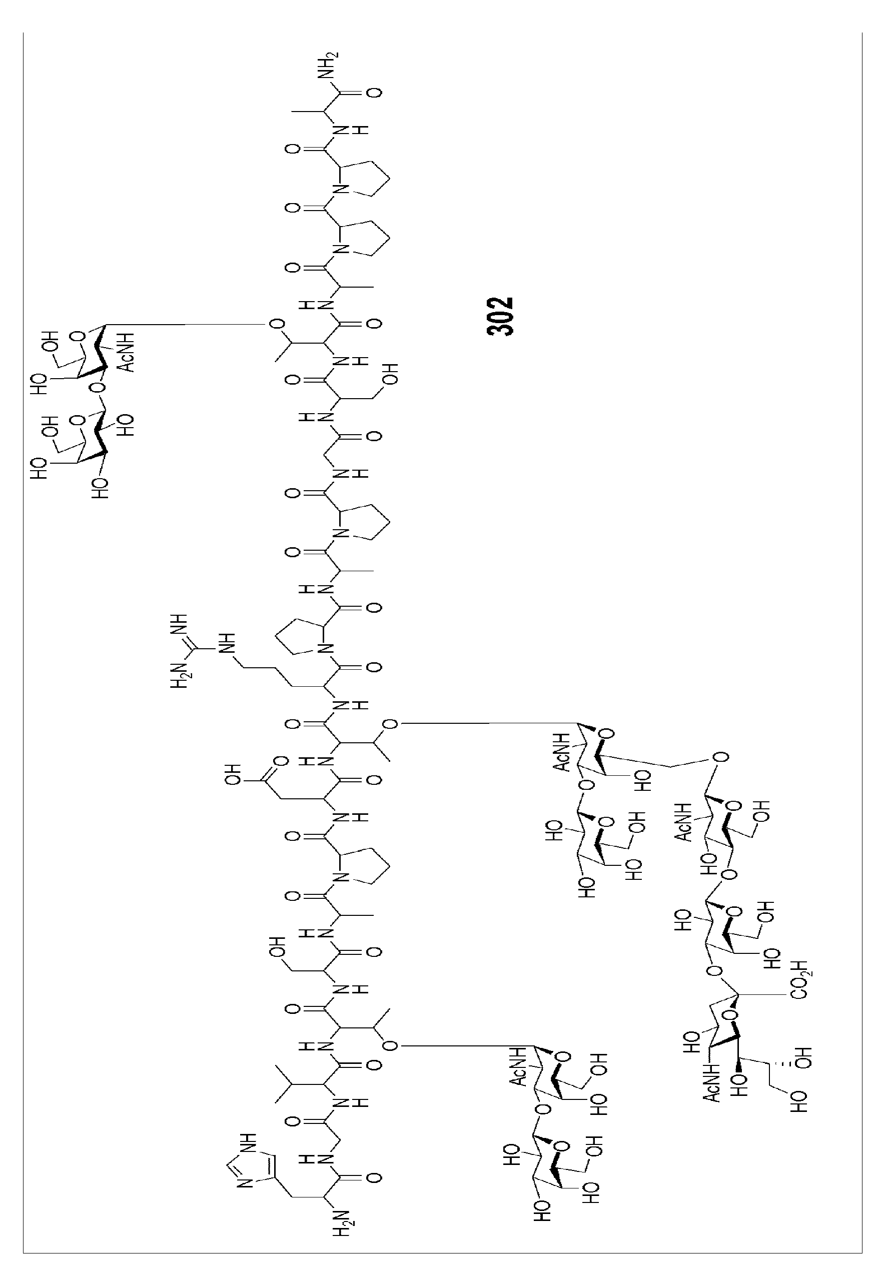

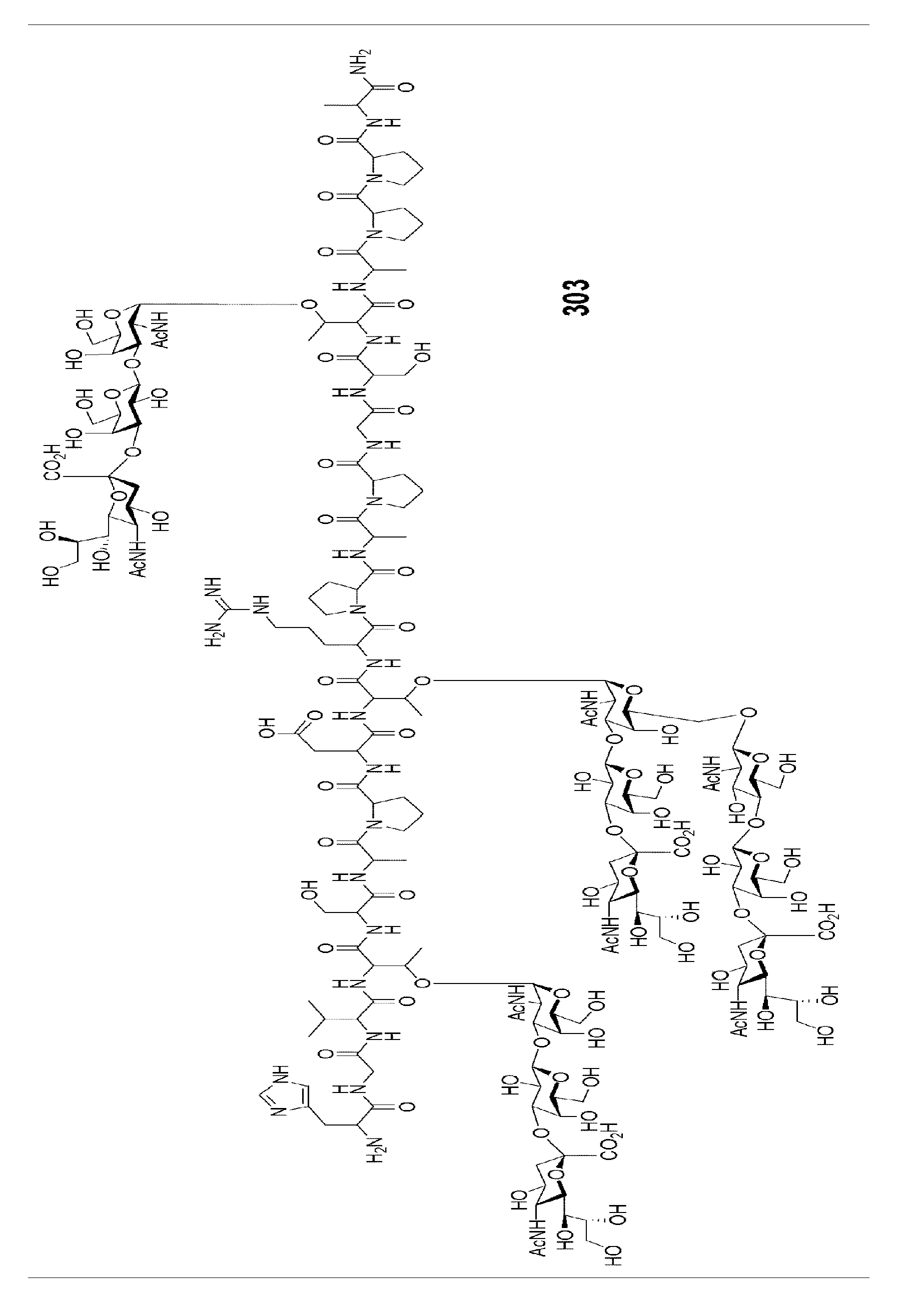

The following reaction solutions A) to E) were allowed to react for 24 hours at 25°C:
A) 100µl of reaction solution containing 50mMHEPES buffer solution (pH 7.0), 0.1U/ml human-derived β1,4-galactosyltransferase (available from TOYOBO CO., LTD.), 10mM manganese chloride, 0.1% BSA, 2mM uridine-5'-disodium diphosphogalactose (UDP-Gal) and glycopeptide derivative (197) (4mM at a theoretical content from solid-phase synthesis);
B) 100µl of reaction solution containing 50mM HEPES buffer solution (pH 7.0), 0.0175U/ml recombinant rat α2,3-(O)-sialyltransferase (available from Calbiochem), 10mM manganese chloride, 0.1% BSA, 2mM cytidine-5'-sodium monophosphosialate (CMP-NANA) and glycopeptide derivative (197) (4mM at a theoretical content from solid-phase synthesis);
C) 100 µl of reaction solution containing 50 mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (197) (4mM at a theoretical content from solid-phase synthesis);
D) 100µl of reaction solution containing 50mM HEPES buffer solution (pH 7.0), 0.1U/ml human-derived β1,4-galactosyltransferase (available from TOYOBO CO., LTD.), 0.0185U/ml recombinant rat α2,3-(N)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (197) (4mM at a theoretical content from solid-phase synthesis);
E) 100 µl of reaction solution containing 50 mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 0.0185U/ml recombinant rat α2,3-(N)-sialyltransferase (available from Calbiochem), 10mM manganese chloride, 0.1% BSA, 4mM uridine-5'-disodium diphosphogalactose (UDP-Gal), 2mM cytidine-5'-sodium monophosphosialate (CMP-NANA) and glycopeptide derivative (197) (4mM at a theoretical content from solid-phase synthesis).

After completion of the reaction, the respective reaction solutions were subjected to centrifugal concentration with an ultrafiltration filter. Thereafter, 25mM ammonium acetate buffer solution (pH 6.5) was added thereto, and the solution was again subjected to concentration with a centrifugal separator, thereby washing the polymer. This manipulation was repeated three times, thereby respectively obtaining aqueous solutions of compounds (293) to (297). Thereafter, to the solution retained at the filter containing compounds (293) to (297) and a solution which was obtained by diluting an aqueous solution of (197) with 25mM ammonium acetate buffer solution (pH 6.5), 1µl of 0.174mg/ml solution of BLase (available from Shionogi & Co., Ltd.) was added, and the solution was allowed to react for two hours at room temperature. Thereafter, the solution was subjected to centrifugal filtration with an ultrafiltration filter, thereby separating the glycopeptide of interest from the polymer. The obtained aqueous solution (filtrate) was lyophilized, thereby obtaining compounds (298) to (303).
MALDI-TOF/MS:[M(average)+H]⁺ of compound (298)=3188.1 (theoretical value:[M(average)+H]⁺=3186.2); MALDI-TOF/MS:[M(average)+H]⁺ of compound (299)=3349.5 (theoretical value:[M(average)+H]⁺=3348.4); MALDI-TOF/MS:[M(average)+H]⁺ of compound (300)=4061.0 (theoretical value:[M(average)+H]⁺=4060.0); MALDI-TOF/MS:[M(average)+H]⁺ of compound (301)=4223.9 (theoretical value:[M(average)+H]⁺=4220.2); MALDI-TOF/MS:[M(average)+H]⁺ of compound (302)=3641.5 (theoretical value:[M(average)+H]⁺=3639.6); and MALDI-TOF/MS:[M(average)+H]⁺ of compound (303)=4514.8 (theoretical value:[M(average)+H]⁺=4513.4).

(3.58 Synthesis of compounds (304) to (314))

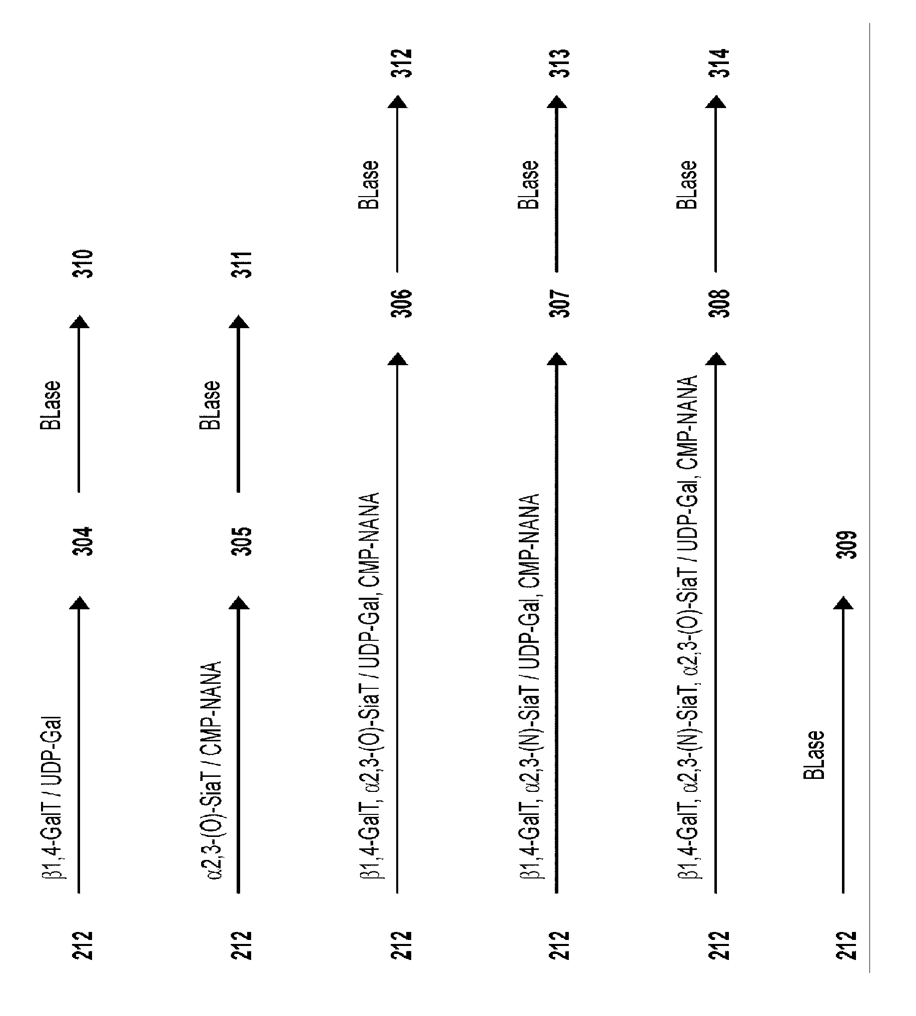

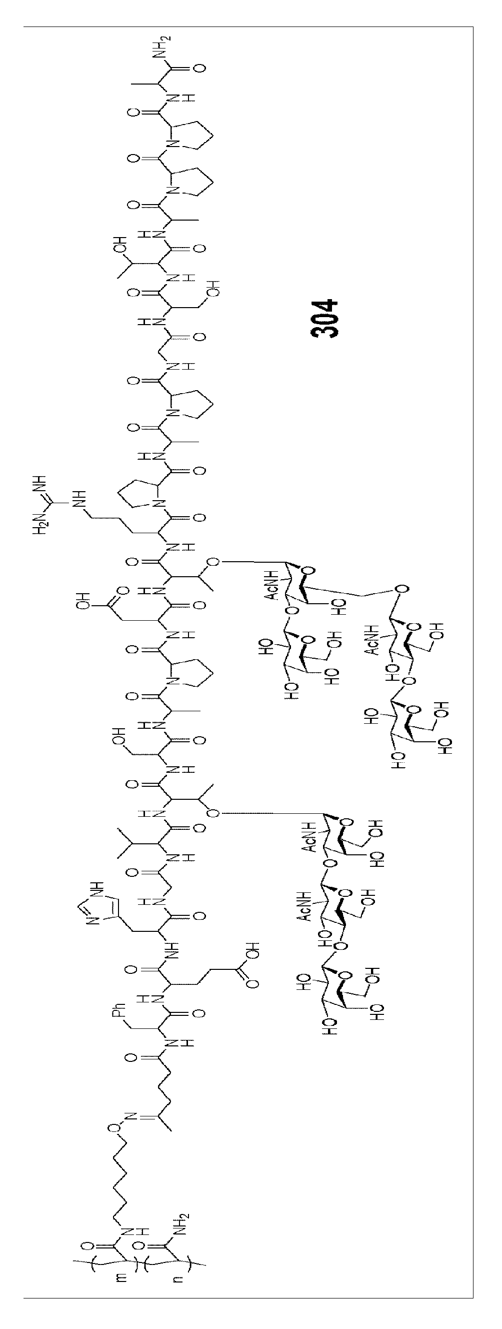

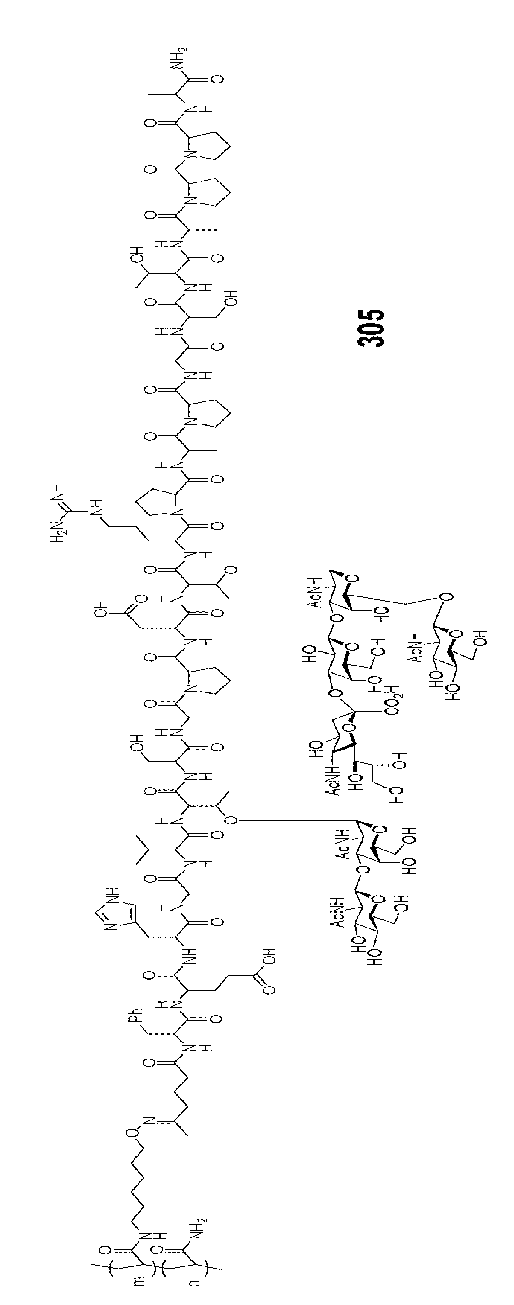

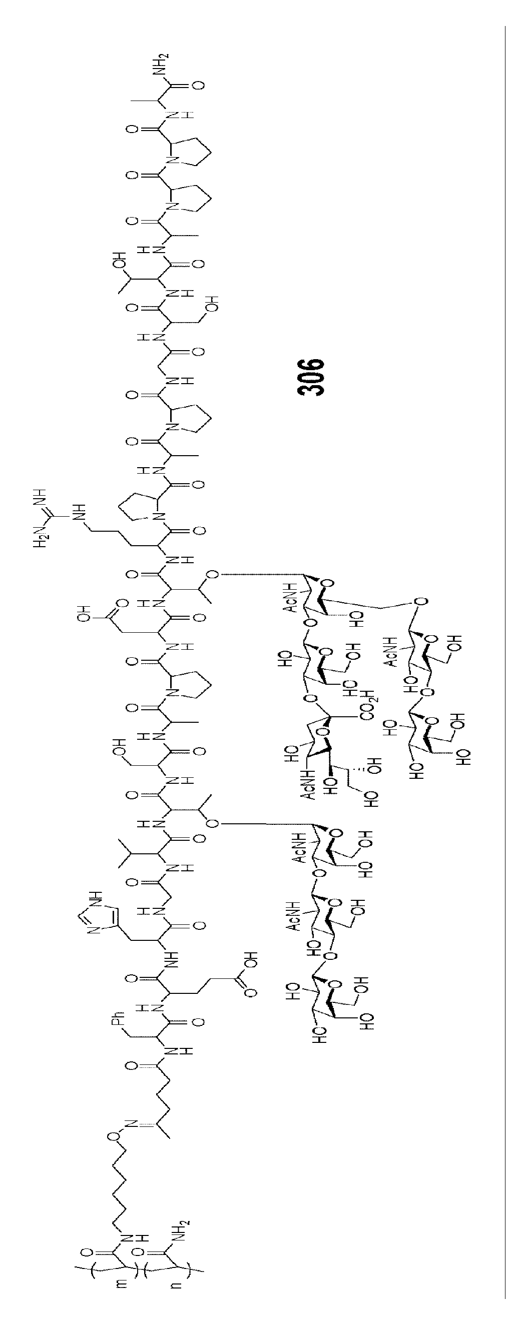

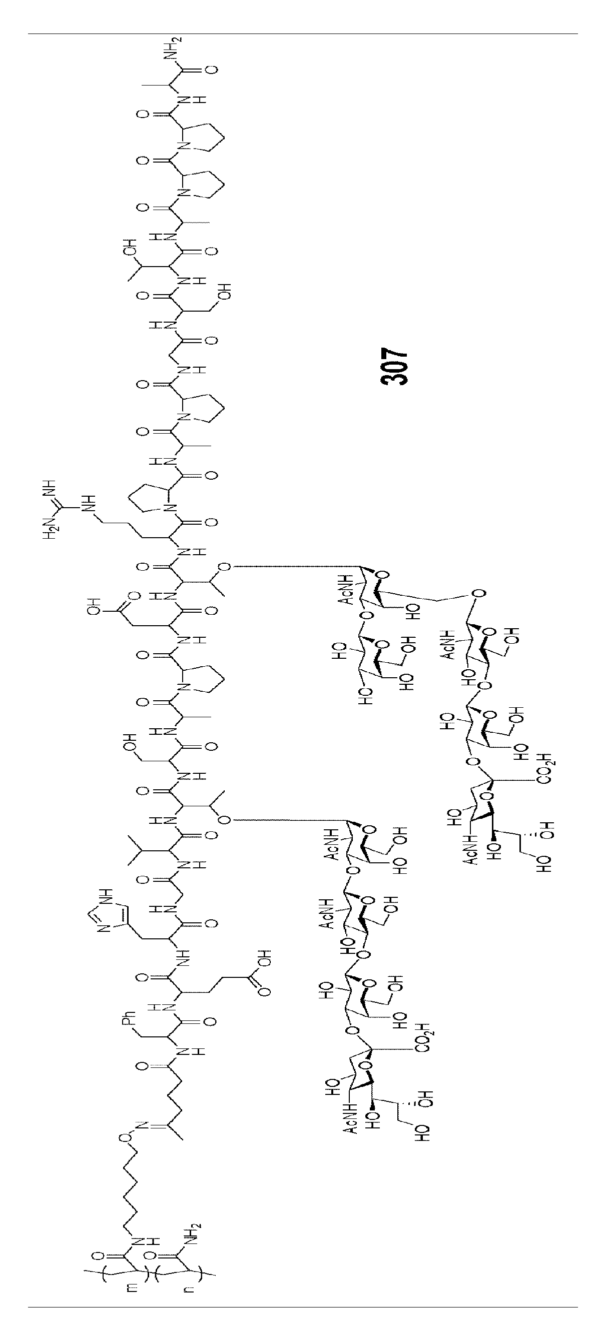

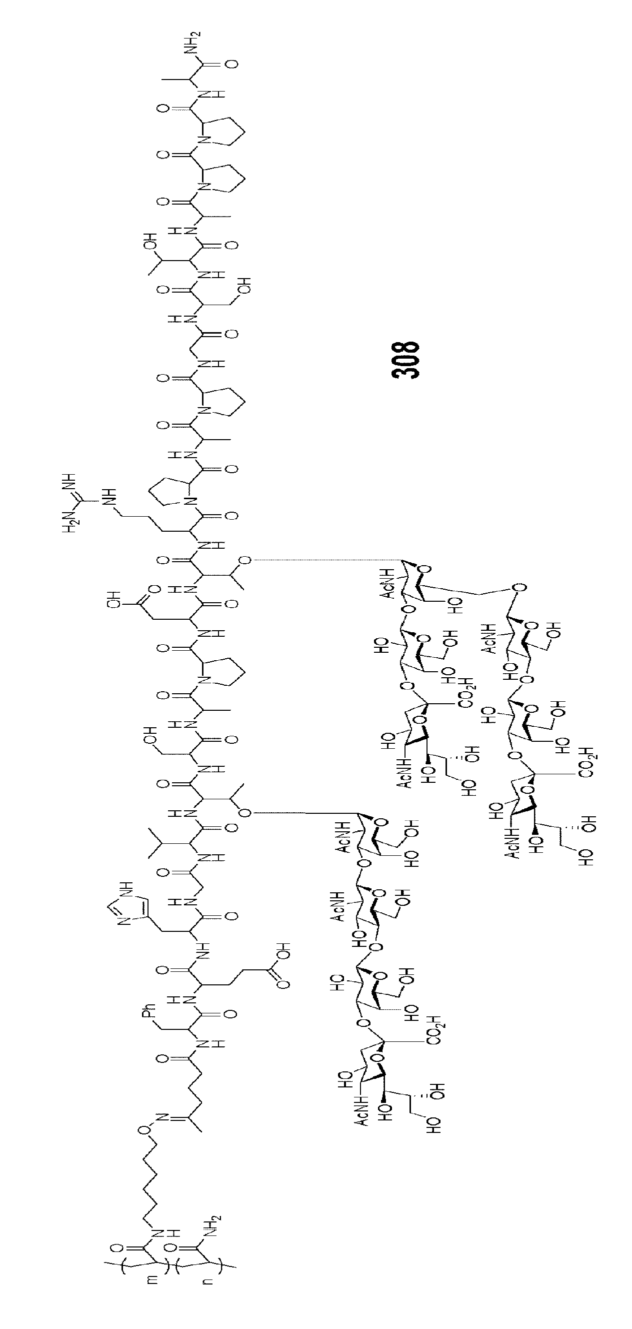

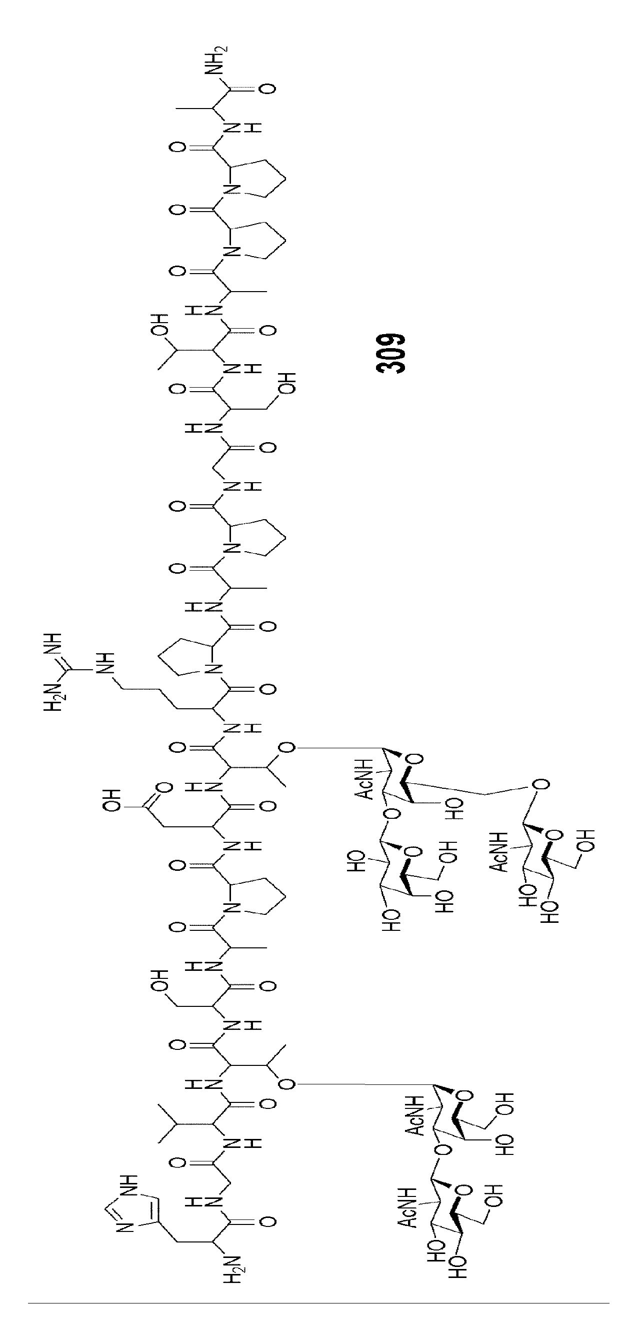

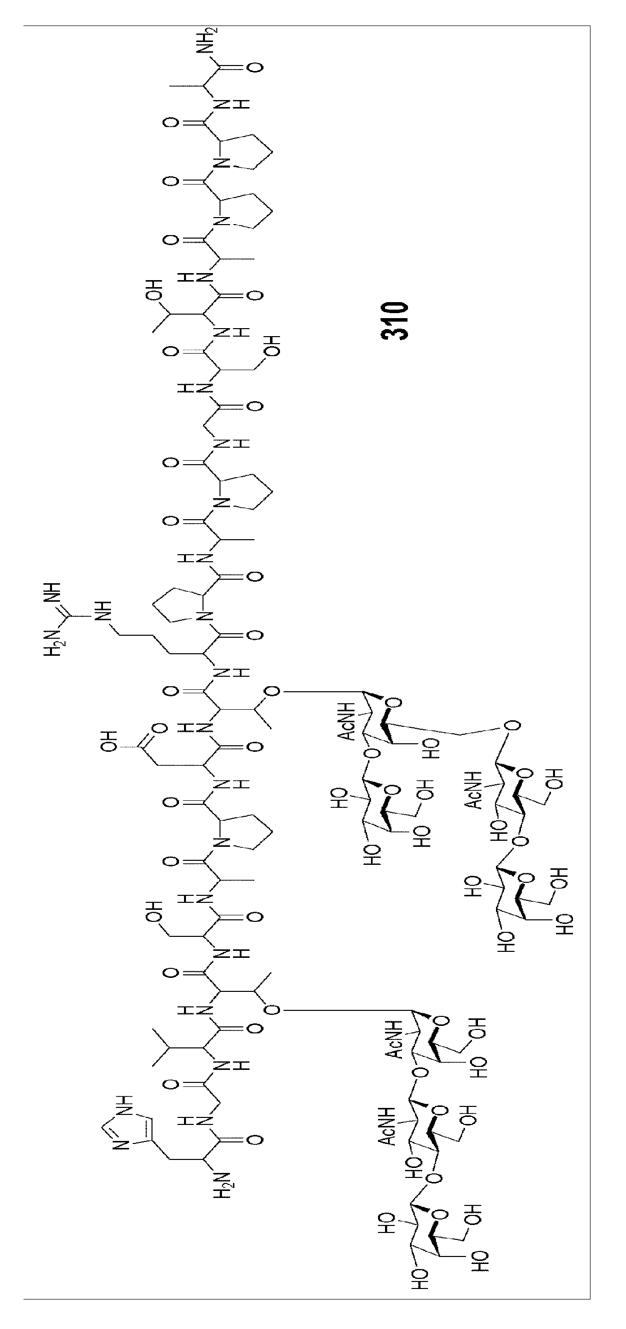

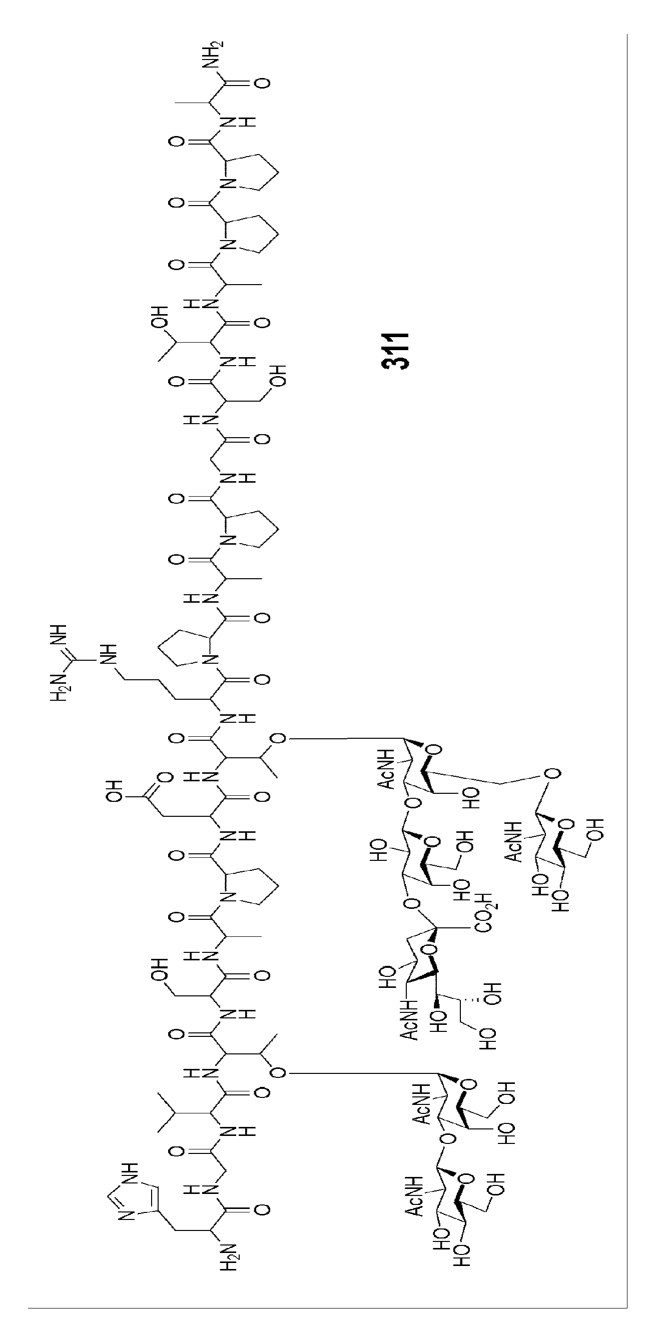

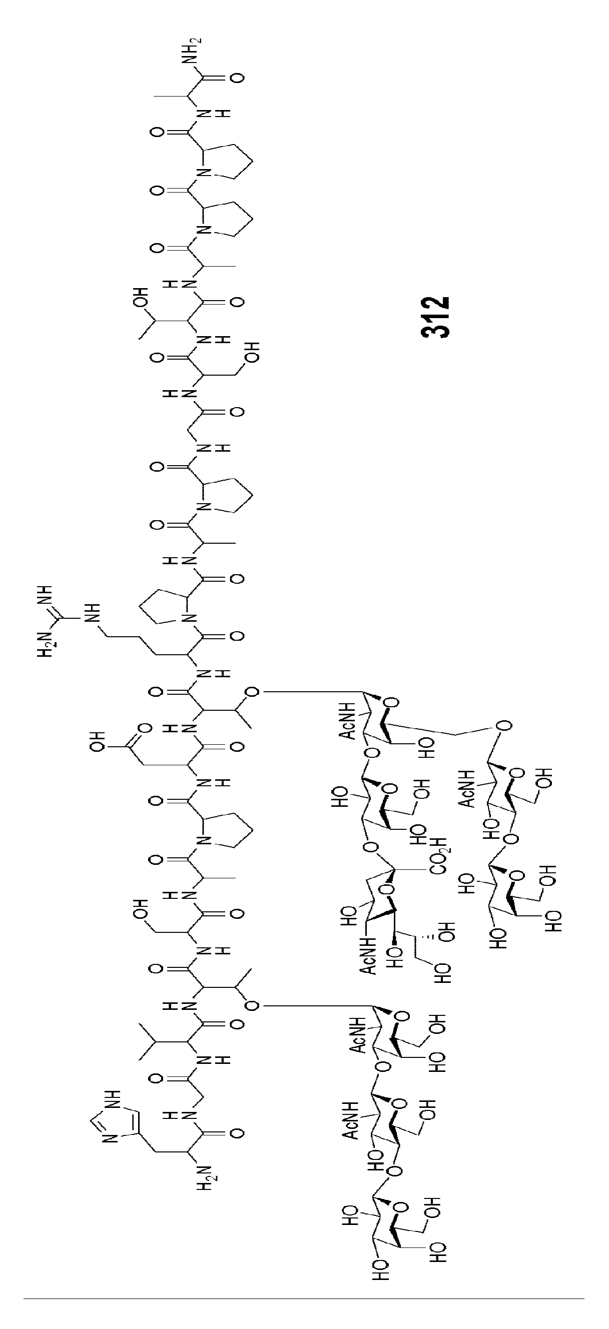

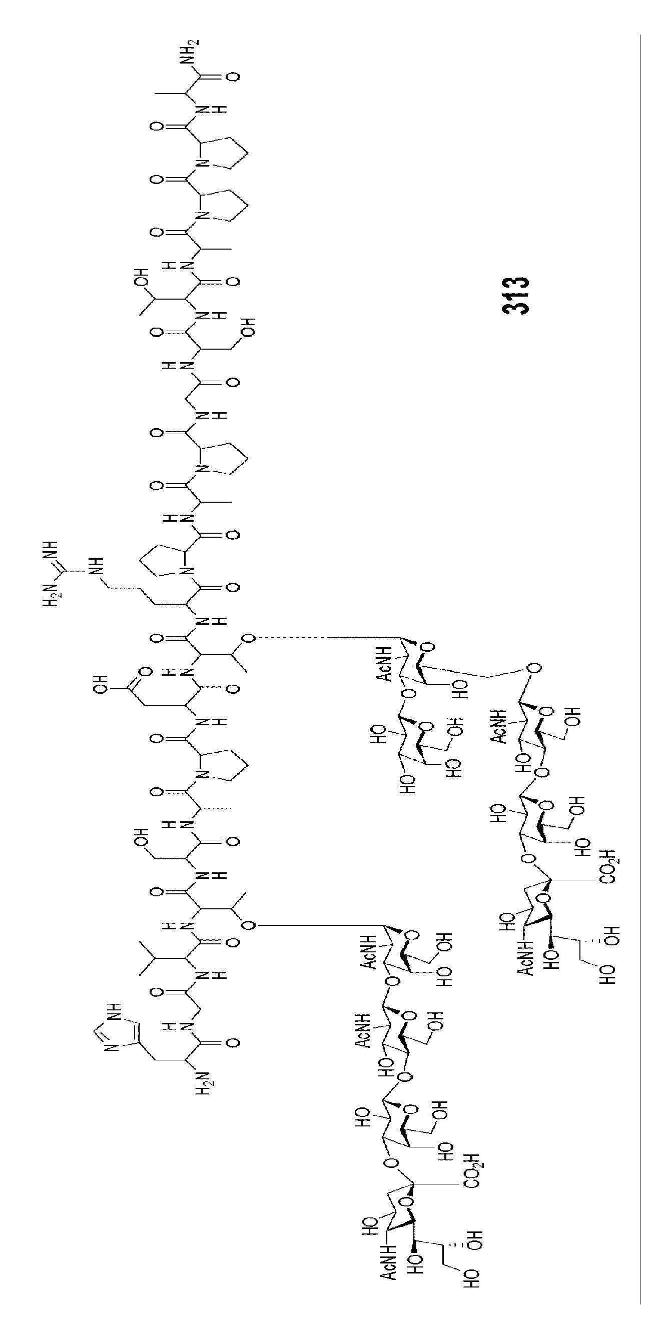

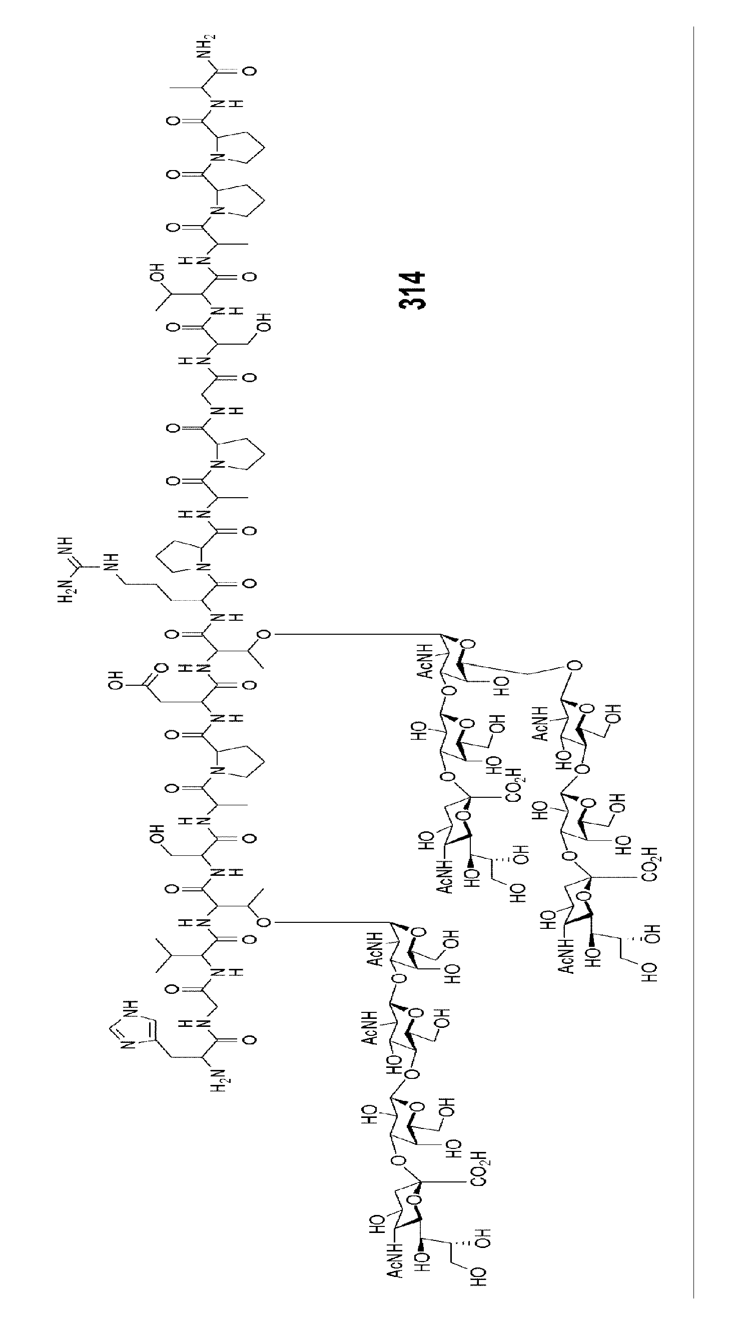

The following reaction solutions A) to E) were allowed to react for 24 hours at 25°C:
A) 100µl of reaction solution containing 50mMHEPES buffer solution (pH 7.0), 0.1U/ml human-derived β1,4-galactosyltransferase (available from TOYOBO CO., LTD.), 10mM manganese chloride, 0.1% BSA, 2mM uridine-5'-disodium diphosphogalactose (UDP-Gal) and glycopeptide derivative (212) (4mM at a theoretical content from solid-phase synthesis);
B) 100µl of reaction solution containing 50mM HEPES buffer solution (pH 7.0), 0.0175U/ml recombinant rat α2,3-(O)-sialyltransferase (available from Calbiochem), 10mM manganese chloride, 0.1% BSA, 2mM cytidine-5'-sodium monophosphosialate (CMP-NANA) and glycopeptide derivative (212) (4mM at a theoretical content from solid-phase synthesis);
C) 100 µl of reaction solution containing 50 mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (212) (4mM at a theoretical content from solid-phase synthesis);
D) 100µl of reaction solution containing 50mM HEPES buffer solution (pH 7.0), 0.1U/ml human-derived β1,4-galactosyltransferase (available from TOYOBO CO., LTD.), 0.0185U/ml recombinant rat α2,3-(N)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (212) (4mM at a theoretical content from solid-phase synthesis);
E) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 0.0185U/ml recombinant rat α2,3-(N)-sialyltransferase (available from Calbiochem), 10mM manganese chloride, 0.1% BSA, 4mM uridine-5'-disodium diphosphogalactose (UDP-Gal), 2mM cytidine-5'-sodium monophosphosialate (CMP-NANA) and glycopeptide derivative (212) (4mM at a theoretical content from solid-phase synthesis).

After completion of the reaction, the respective reaction solutions were subjected to centrifugal concentration with an ultrafiltration filter. Thereafter, 25mM ammonium acetate buffer solution (pH 6.5) was added thereto, and the solution was again subjected to concentration with a centrifugal separator, thereby washing the polymer. This manipulation was repeated three times, thereby respectively obtaining aqueous solutions of compounds (304) to (308). Thereafter, to the solution retained at the filter containing compounds (304) to (308) and a solution which was obtained by diluting an aqueous solution of (212) with 25mM ammonium acetate buffer solution (pH 6.5), 1µl of 0.174mg/ml solution of BLase (available from Shionogi & Co., Ltd.) was added, and the solution was allowed to react for two hours at room temperature. Thereafter, the solution was subjected to centrifugal filtration with an ultrafiltration filter, thereby separating the glycopeptide of interest from the polymer. The obtained aqueous solution (filtrate) was lyophilized, thereby obtaining compounds (309) to (314).
MALDI-TOF/MS:[M(average)+H]⁺ of compound (309)=2864.3 (theoretical value:[M(average)+H]⁺=2861.9); MALDI-TOF/MS:[M(average)+H]⁺ of compound (310)=3186.4 (theoretical value:[M(average)+H]⁺=3186.2); MALDI-TOF/MS:[M(average)+H]⁺ of compound (311)=3154.4 (theoretical value:[M(average)+H]⁺=3153.2); MALDI-TOF/MS:[M(average)+H]⁺ of compound (312)=3478.4 (theoretical value:[M(average)+H]⁺=3477.5); MALDI-TOF/MS:[M(average)+H]⁺ of compound (313)=3769.1 (theoretical value:[M(average)+H]⁺=3768.7); and MALDI-TOF/MS:[M(average)+H]⁺ of compound (314)=4064.4 (theoretical value:[M(average)+H]⁺=4060.0).

(3.59 Synthesis of compounds (315) to (325))

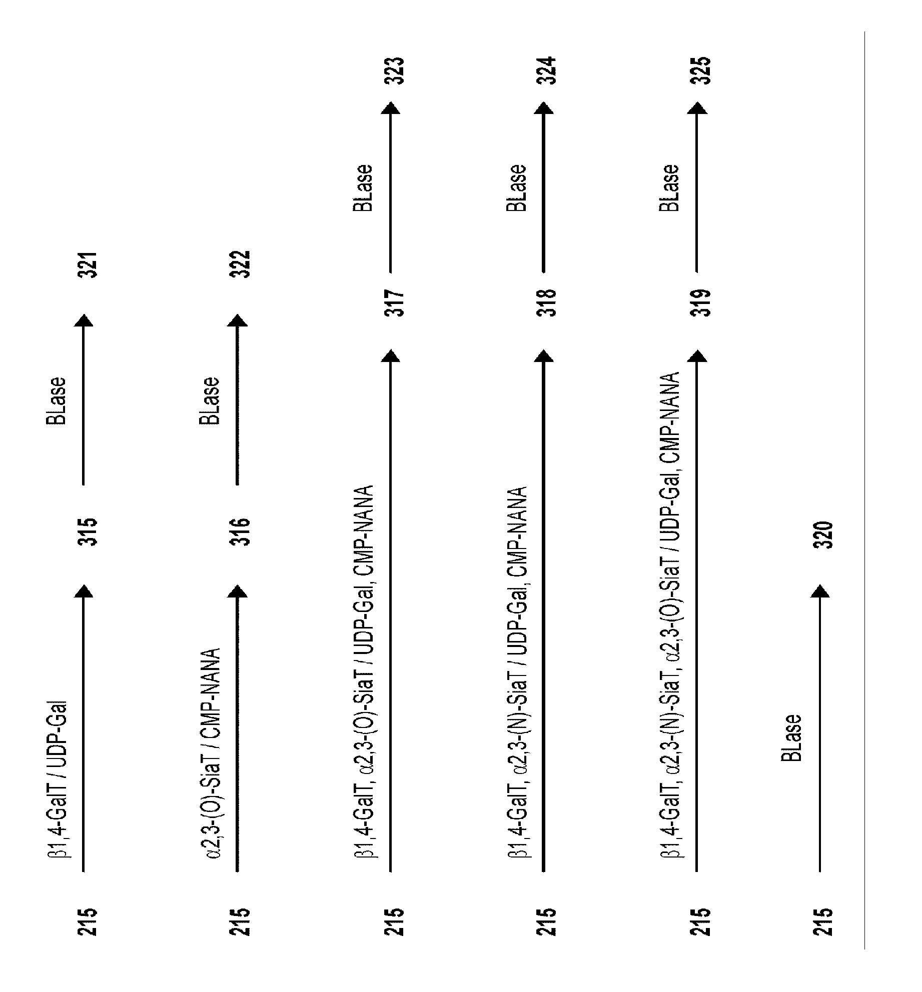

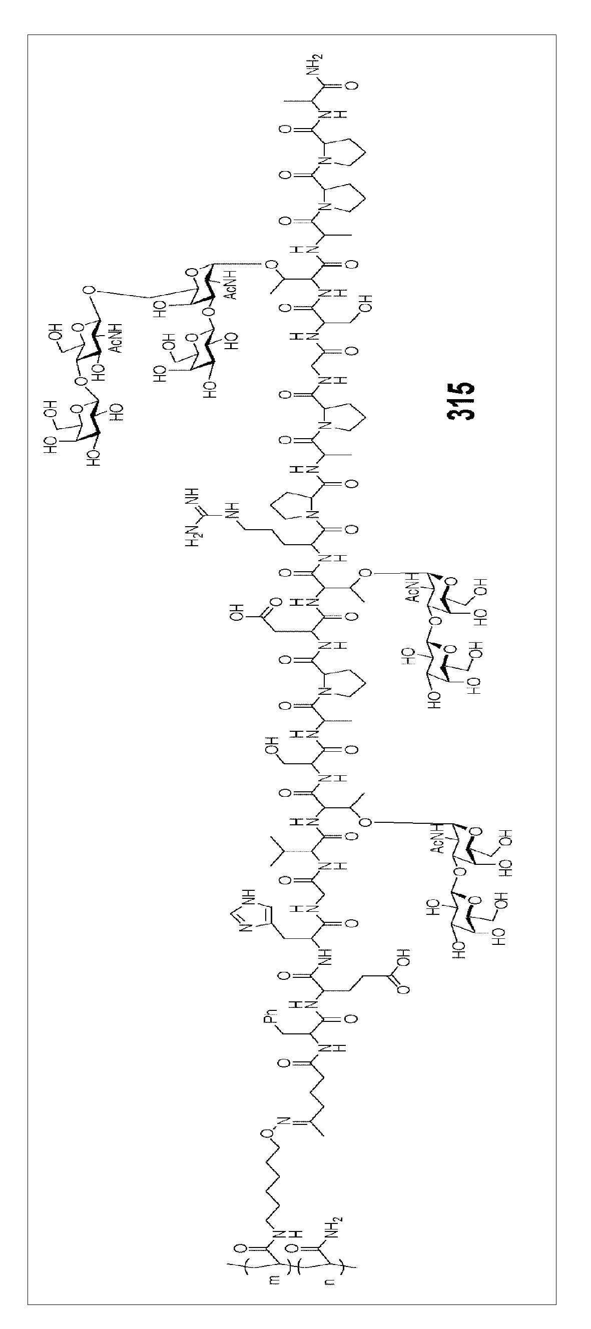

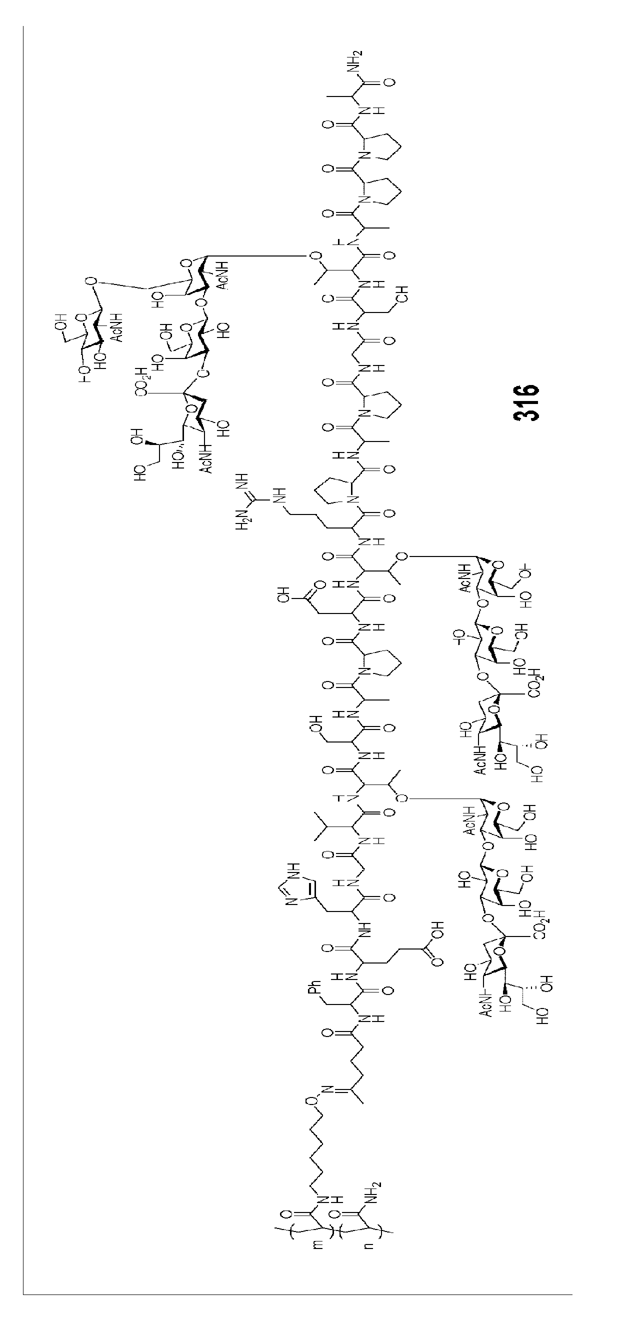

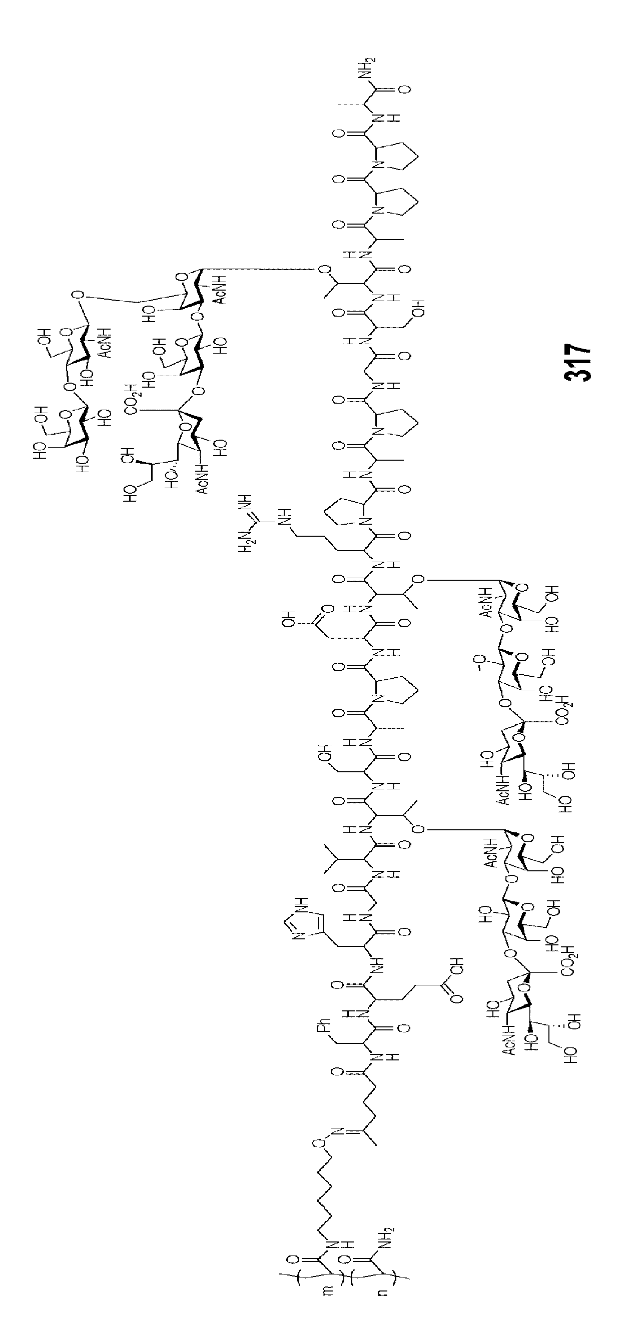

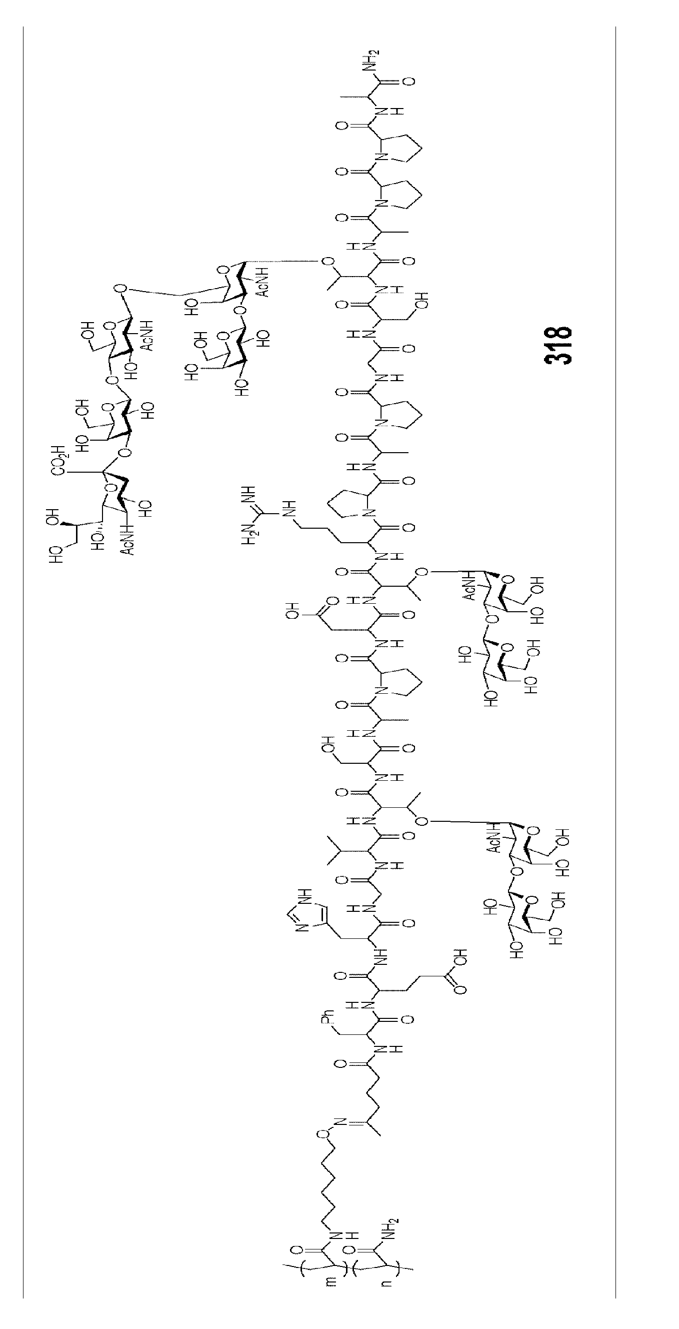

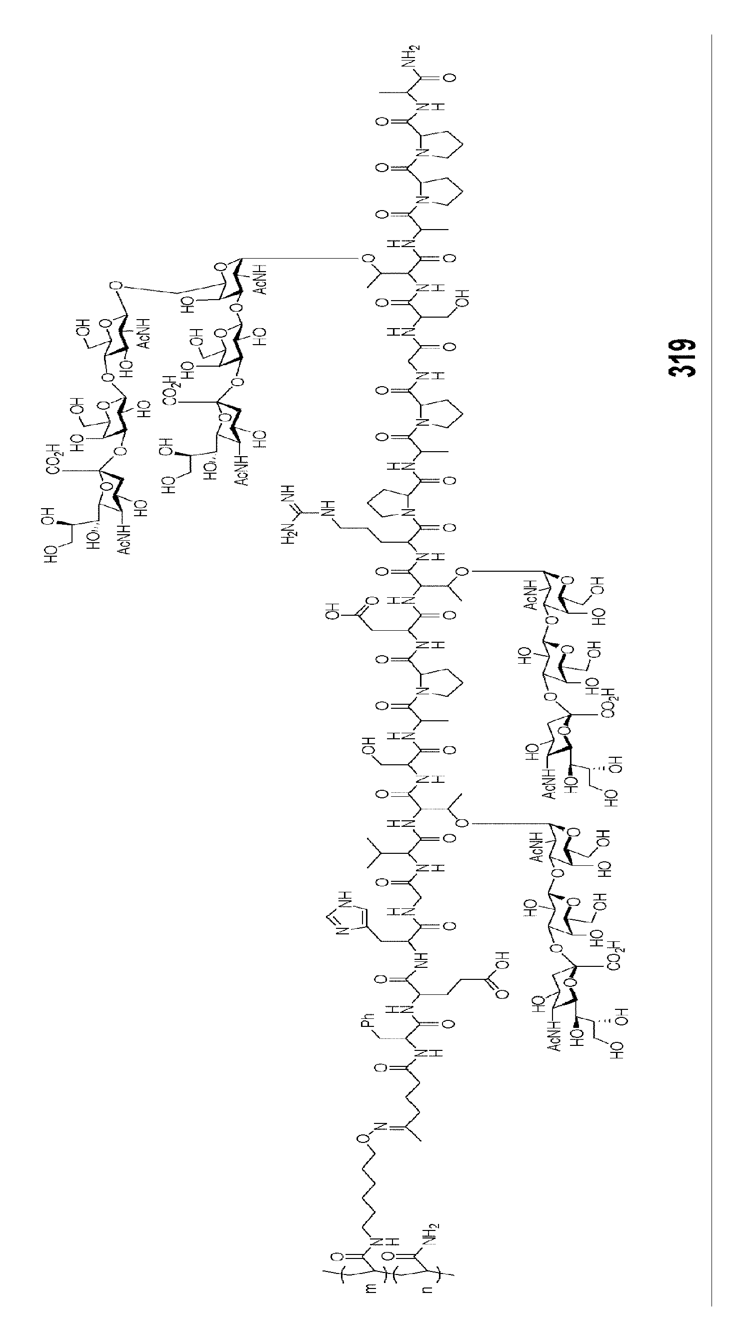

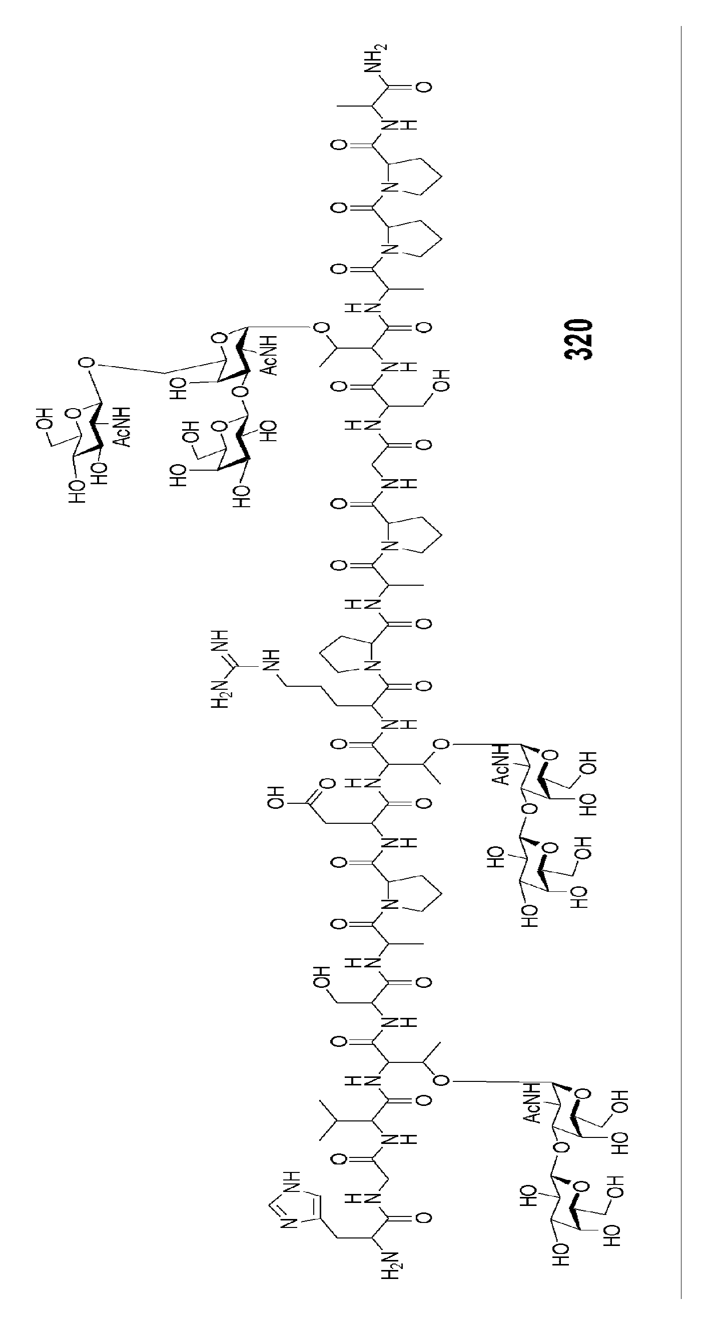

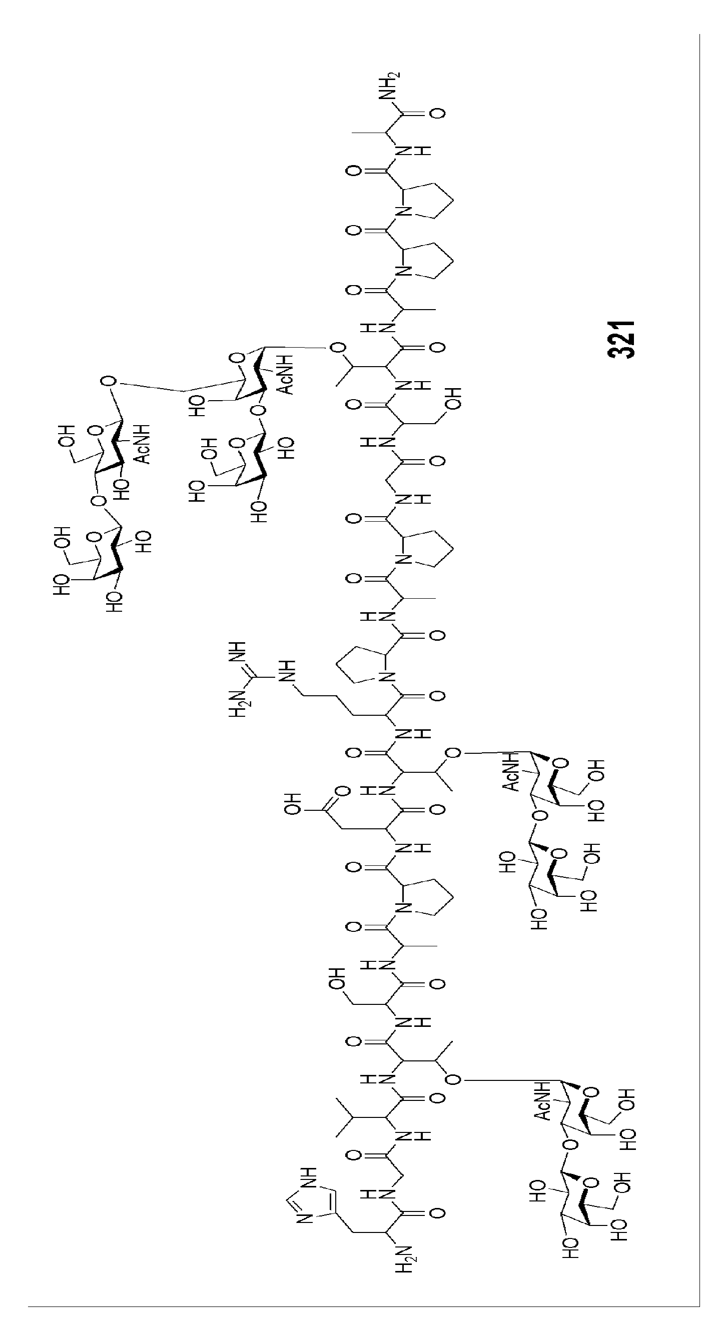

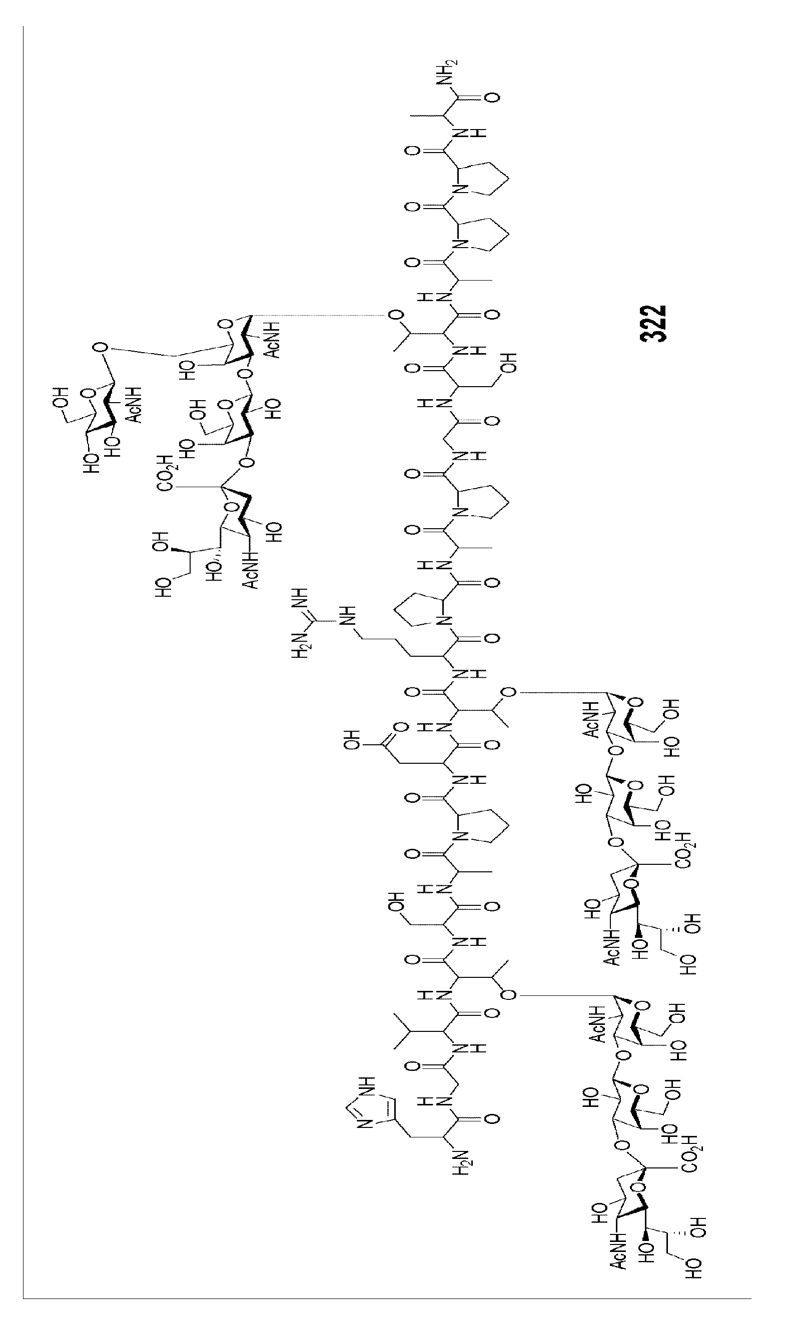

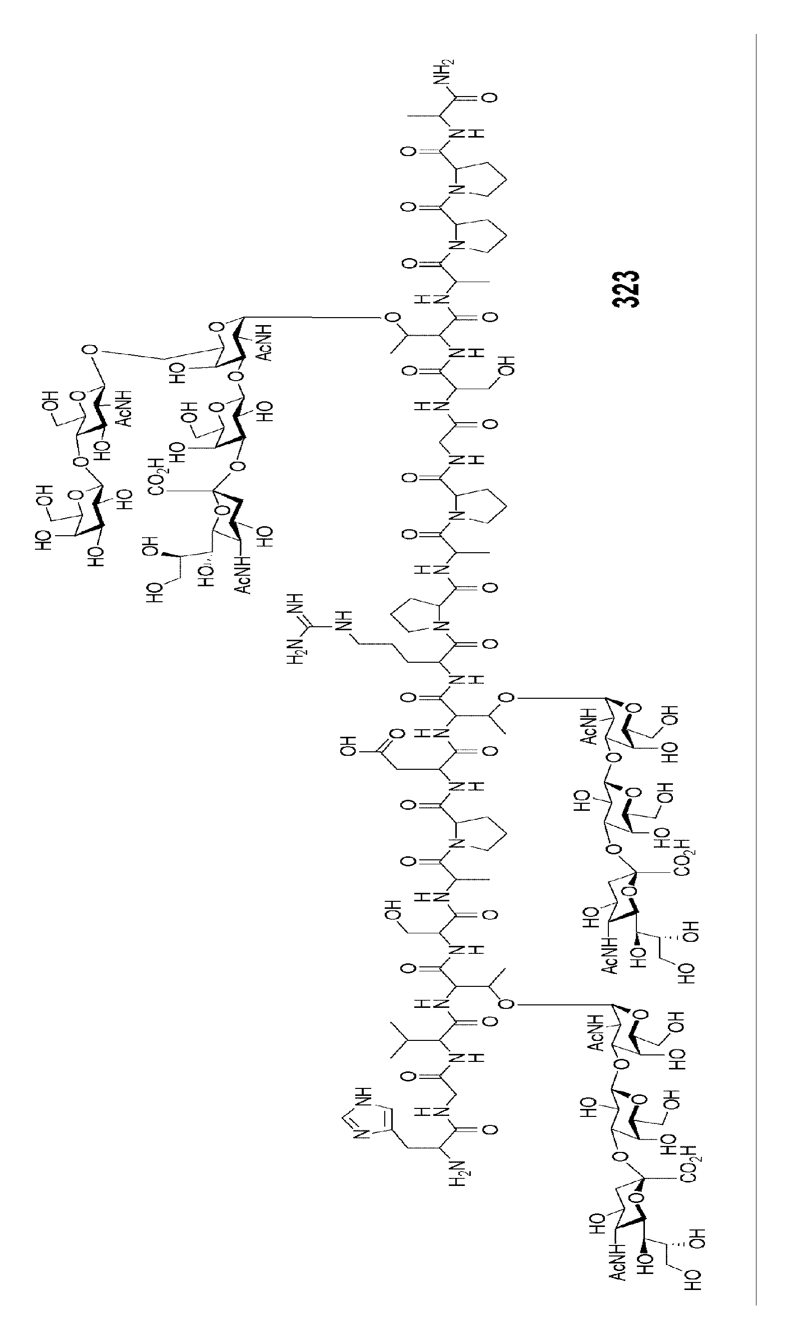

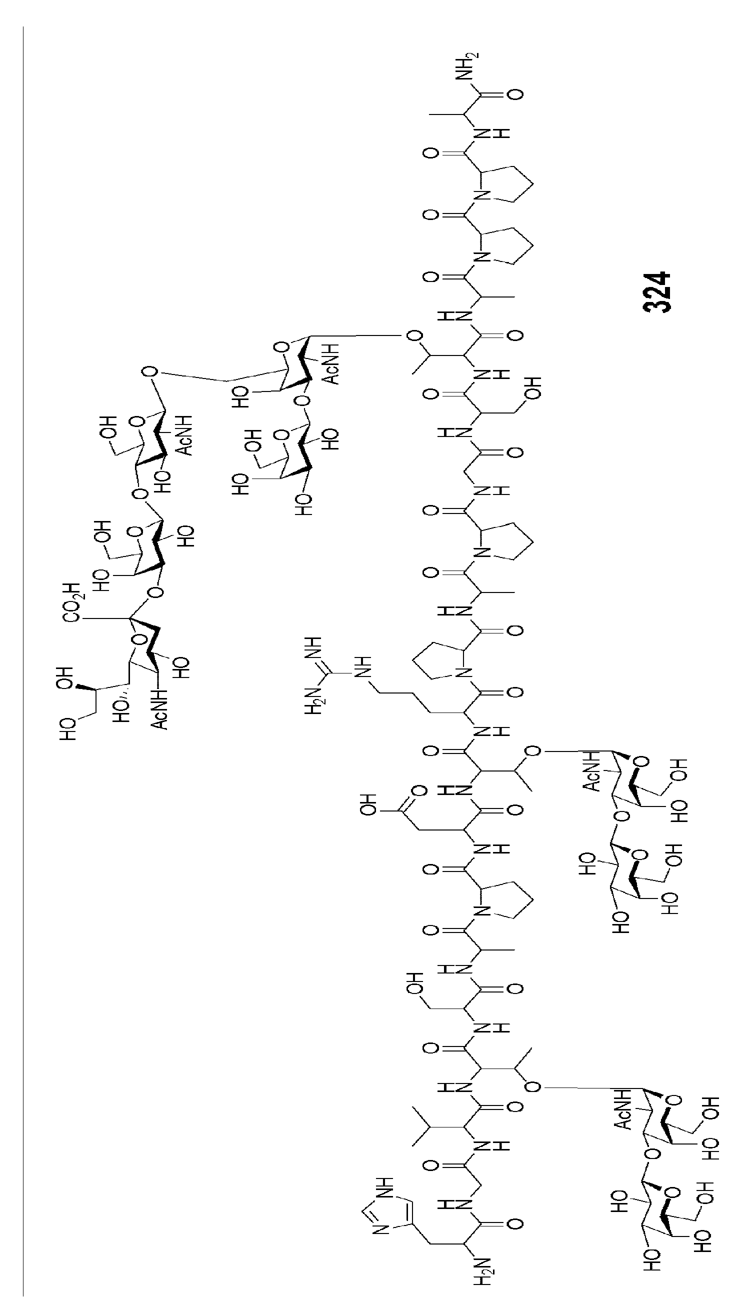

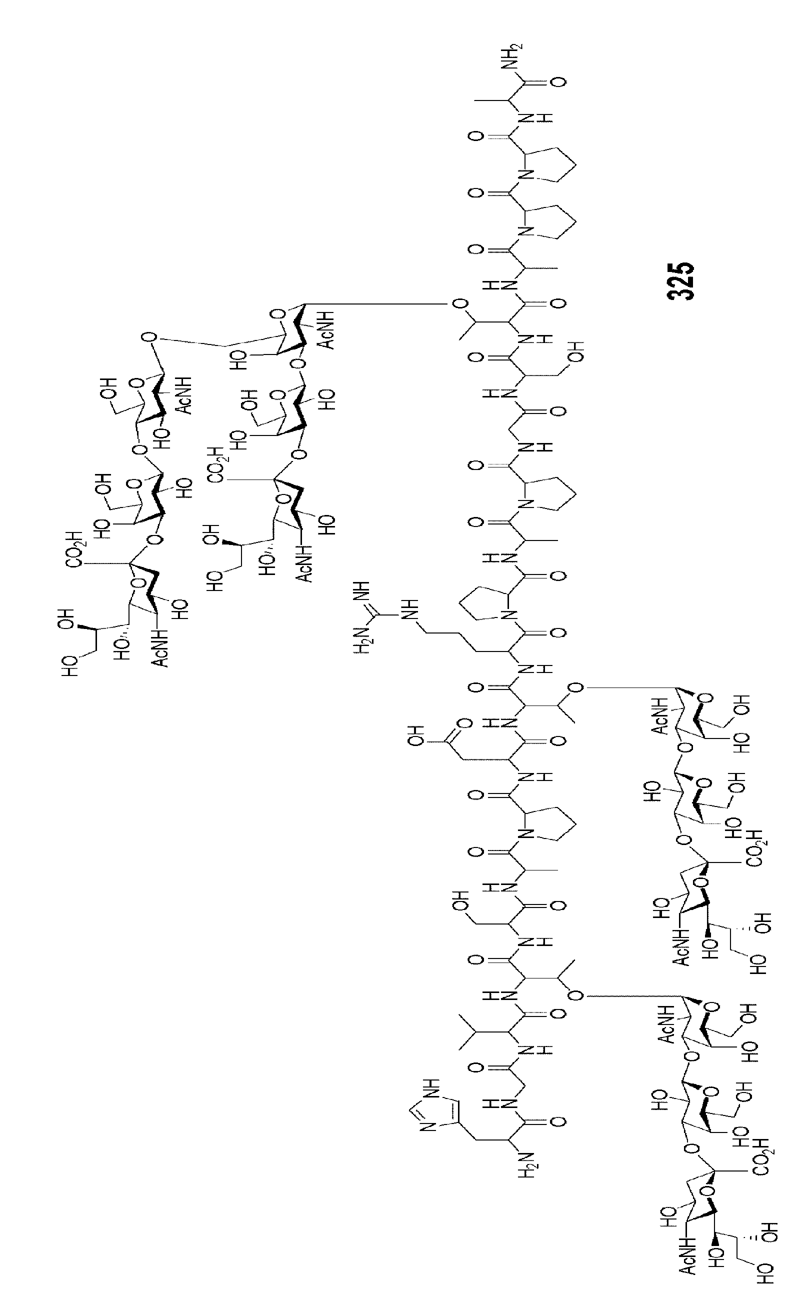

The following reaction solutions A) to E) were allowed to react for 24 hours at 25°C:
A) 100µl of reaction solution containing 50mMHEPES buffer solution (pH 7.0), 0.1U/ml human-derived β1,4-galactosyltransferase (available from TOYOBO CO., LTD.), 10mM manganese chloride, 0.1% BSA, 2mM uridine-5'-disodium diphosphogalactose (UDP-Gal) and glycopeptide derivative (215) (4mM at a theoretical content from solid-phase synthesis);
B) 100µl of reaction solution containing 50mM HEPES buffer solution (pH 7.0), 0.0175U/ml recombinant rat α2,3-(O)-sialyltransferase (available from Calbiochem), 10mM manganese chloride, 0.1% BSA, 2mM cytidine-5'-sodium monophosphosialate (CMP-NANA) and glycopeptide derivative (215) (4mM at a theoretical content from solid-phase synthesis);
C) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (215) (4mM at a theoretical content from solid-phase synthesis);
D) 100µl of reaction solution containing 50mM HEPES buffer solution (pH 7.0), 0.1U/ml human-derived β1,4-galactosyltransferase (available from TOYOBO CO., LTD.), 0.0185U/ml recombinant rat α2,3-(N)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (215) (4mM at a theoretical content from solid-phase synthesis);
E) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 0.0185U/ml recombinant rat α2,3-(N)-sialyltransferase (available from Calbiochem), 10mM manganese chloride, 0.1% BSA, 4mM uridine-5'-disodium diphosphogalactose (UDP-Gal), 2mM cytidine-5'-sodium monophosphosialate (CMP-NANA) and glycopeptide derivative (215) (4mM at a theoretical content from solid-phase synthesis).

After completion of the reaction, the respective reaction solutions were subjected to centrifugal concentration with an ultrafiltration filter. Thereafter, 25mM ammonium acetate buffer solution (pH 6.5) was added thereto, and the solution was again subjected to concentration with a centrifugal separator, thereby washing the polymer. This manipulation was repeated three times, thereby respectively obtaining aqueous solutions of compounds (315) to (319). Thereafter, to the solution retained at the filter containing compounds (315) to (319) and a solution which was obtained by diluting an aqueous solution of (215) with 25mM ammonium acetate buffer solution (pH 6.5), 1µl of 0.174mg/ml solution of BLase (available from Shionogi & Co., Ltd.) was added, and the solution was allowed to react for two hours at room temperature. Thereafter, the solution was subjected to centrifugal filtration with an ultrafiltration filter, thereby separating the glycopeptide of interest from the polymer. The obtained aqueous solution (filtrate) was lyophilized, thereby obtaining compounds (320) to (325).
MALDI-TOF/MS:[M(average)+H]⁺ of compound (320)=3187.7 (theoretical value:[M(average)+H]⁺=3186.2); MALDI-TOF/MS:[M(average)+H]⁺ of compound (321)=3348.4 (theoretical value:[M(average)+H]⁺=3348.4); MALDI-TOF/MS:[M(average)+H]⁺ of compound (322)=4059.7 (theoretical value:[M(average)+H]⁺=4060.0); MALDI-TOF/MS:[M(average)+H]⁺ of compound (323)=4223.3 (theoretical value:[M(average)+H]⁺=4222.1); MALDI-TOF/MS:[M(average)+H]⁺ of compound (324)=3642.0 (theoretical value:[M(average)+H]⁺=3639.6); and MALDI-TOF/MS:[M(average)+H]⁺ of compound (325)=4511.7 (theoretical value:[M(average)+H]⁺=4513.4).

(3.60 Synthesis of compounds (326) to (336))

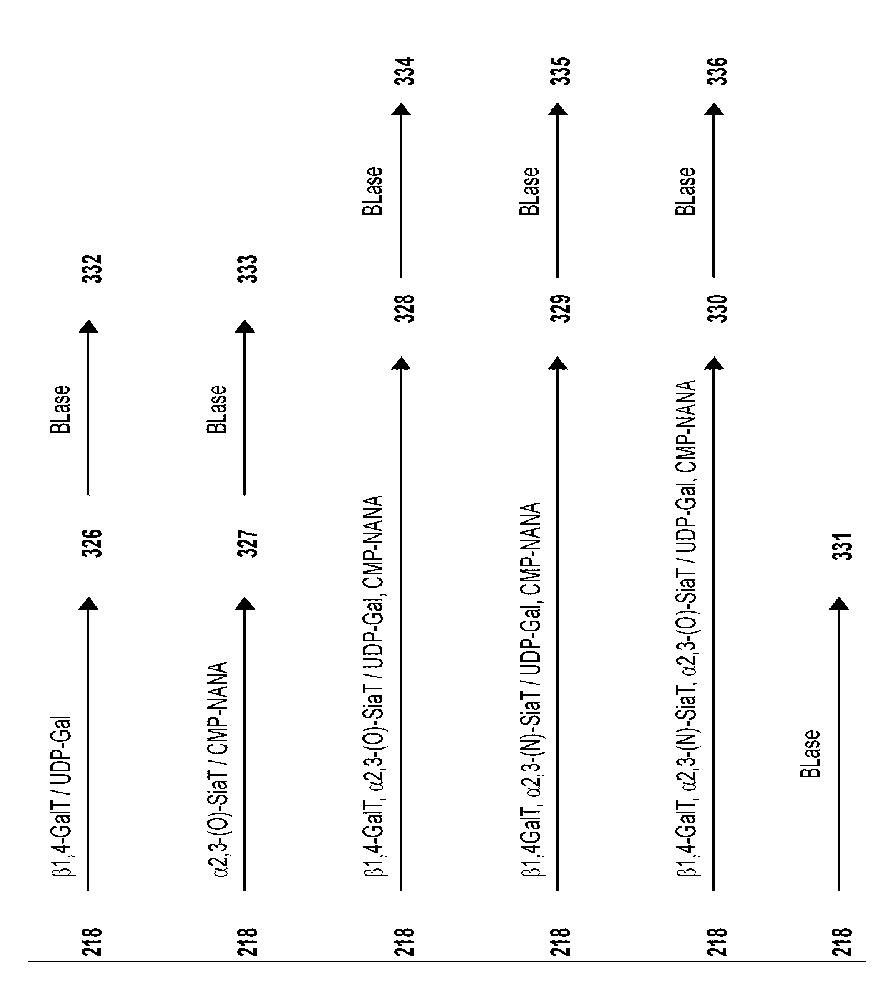

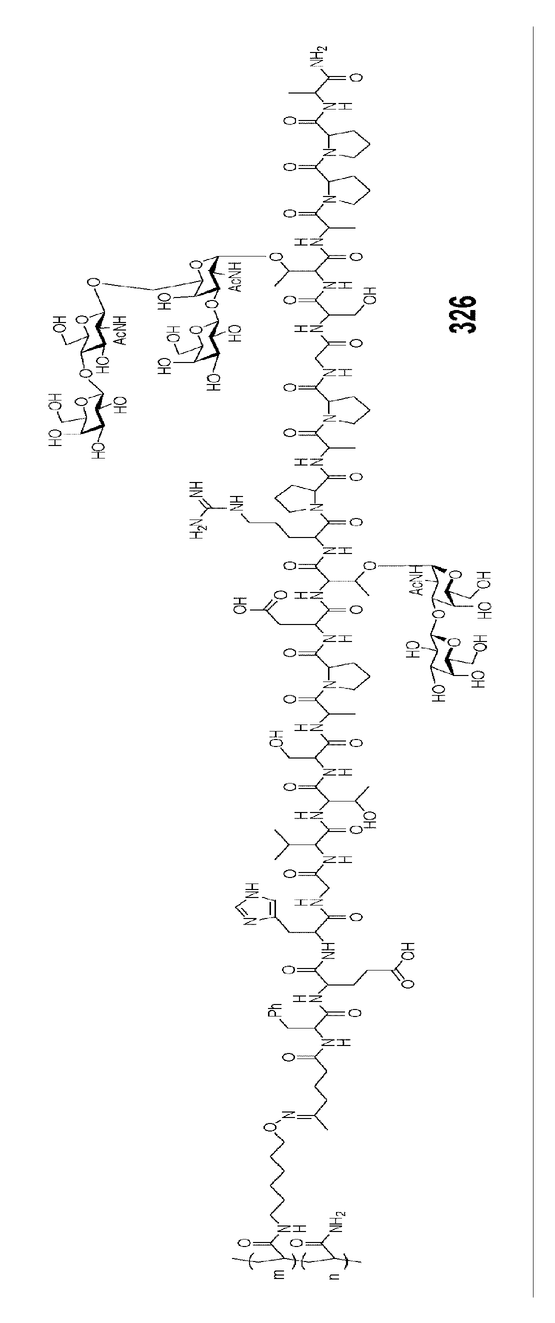

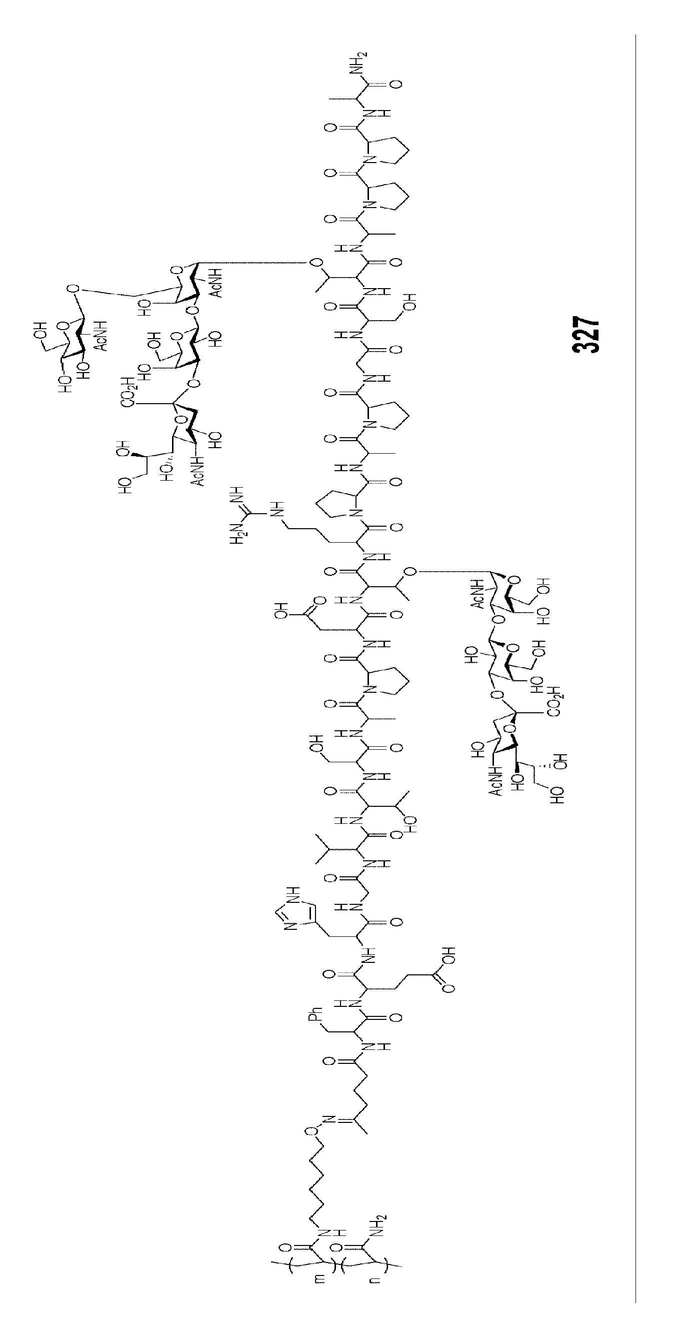

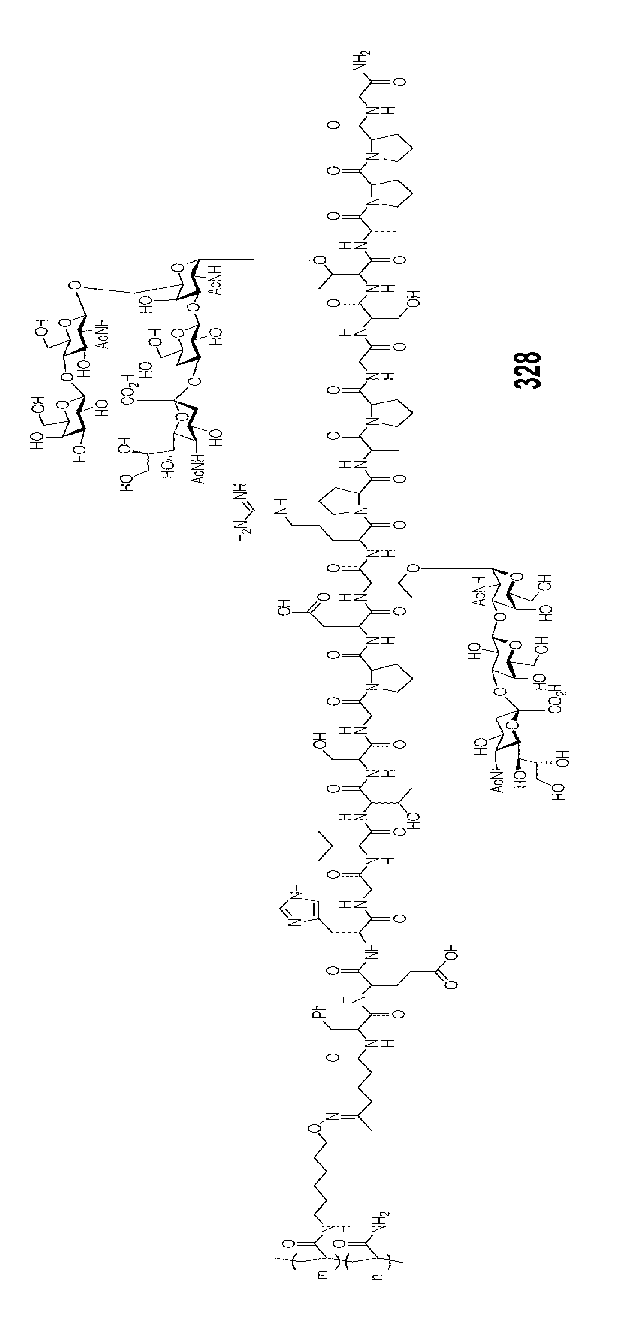

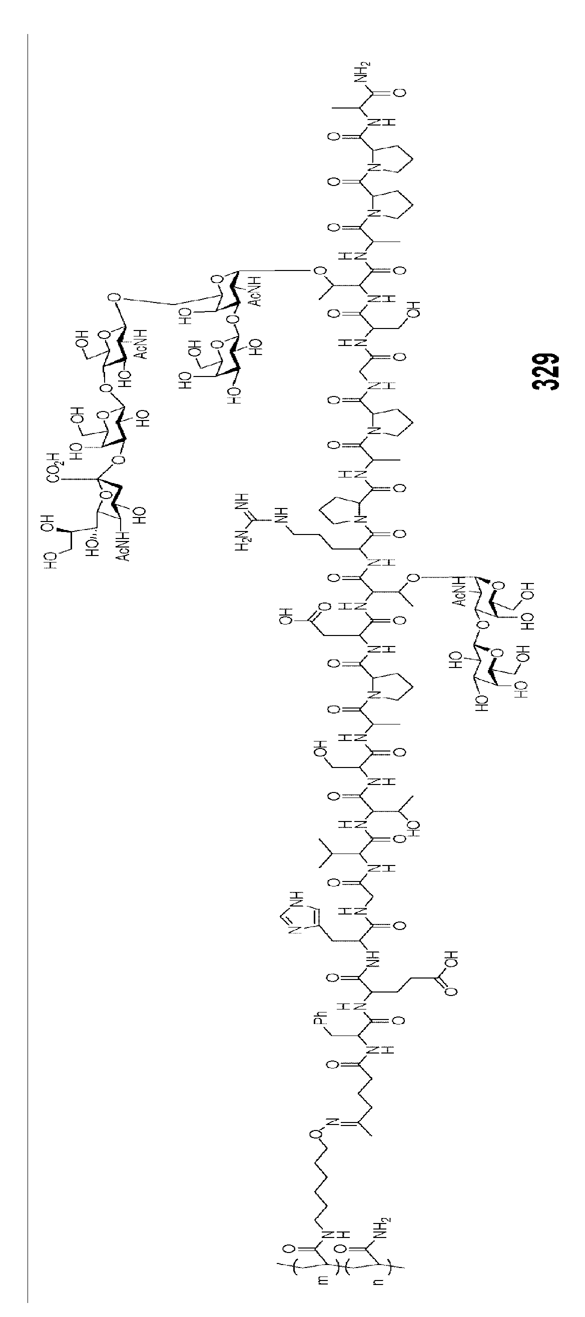

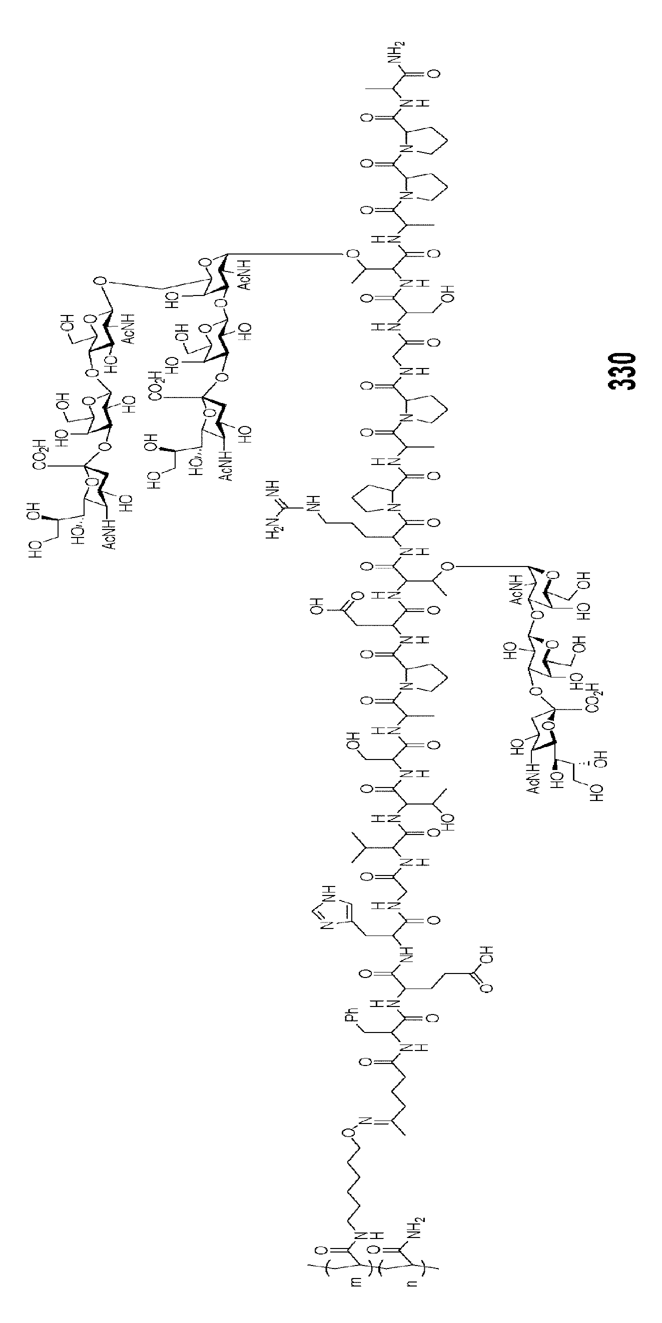

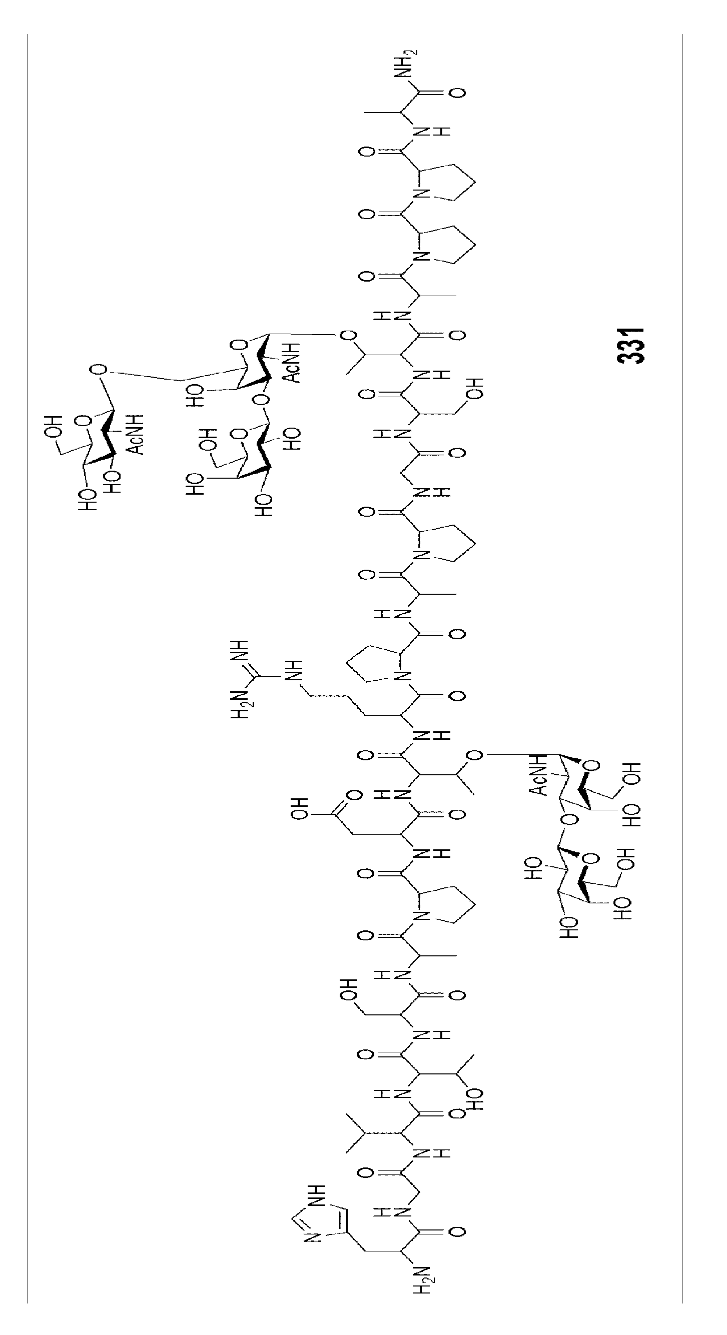

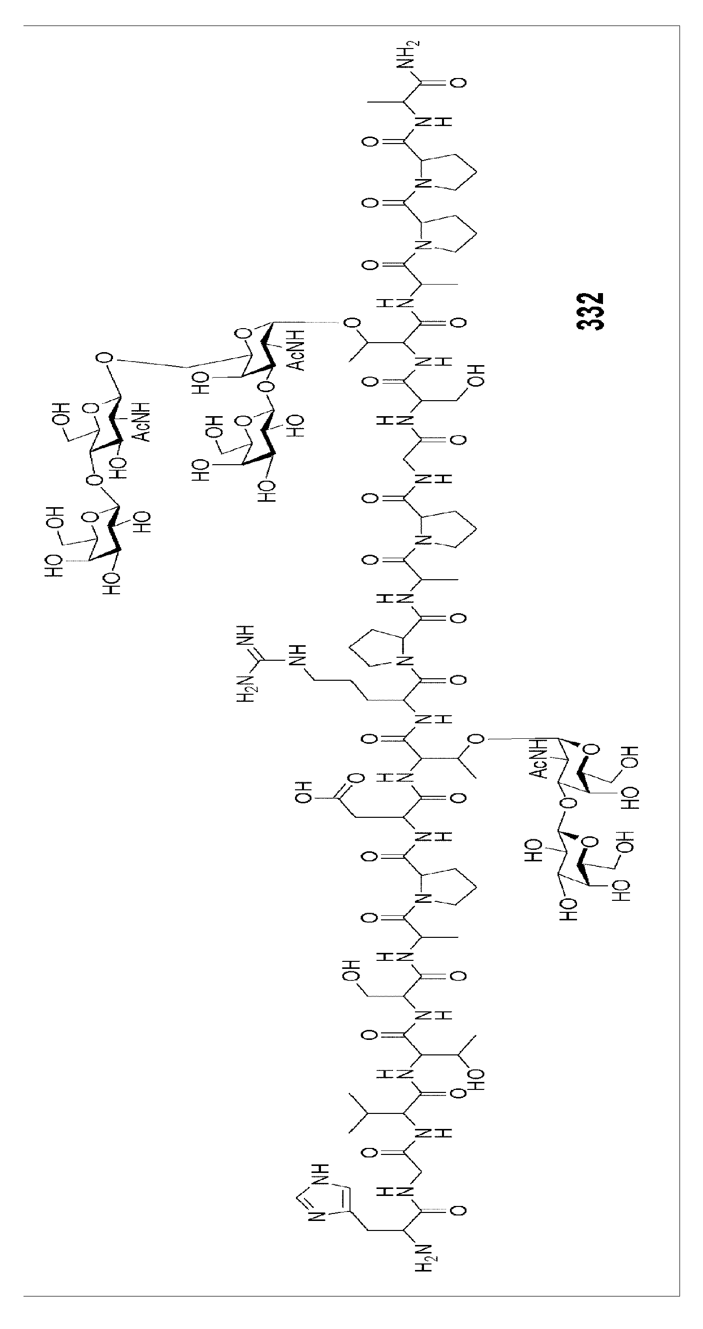

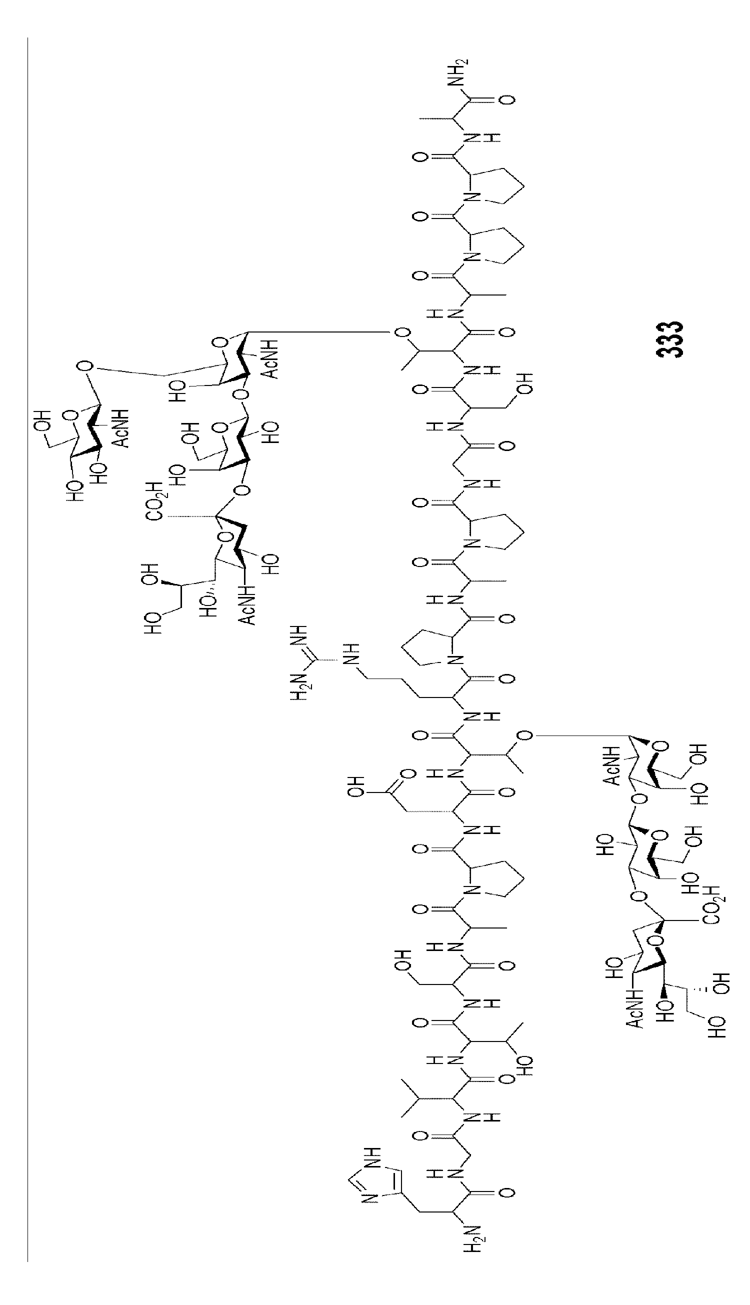

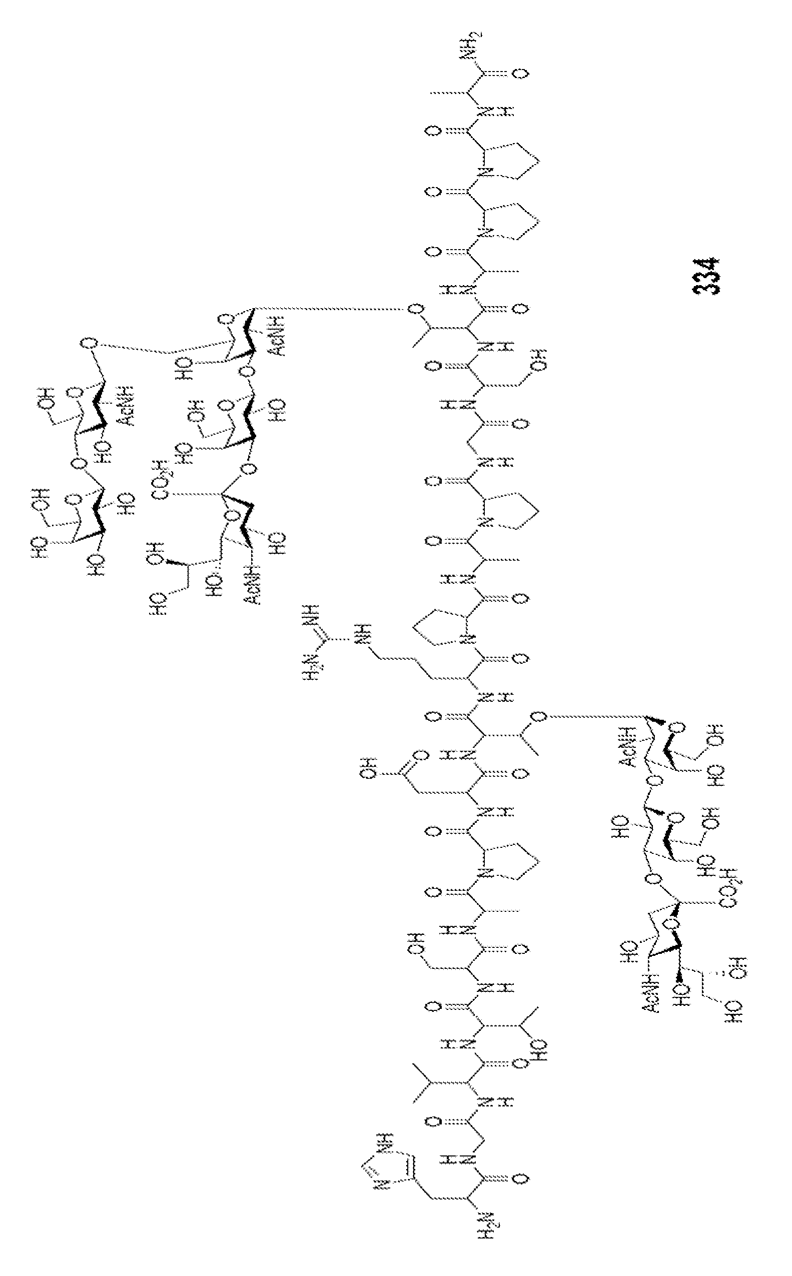

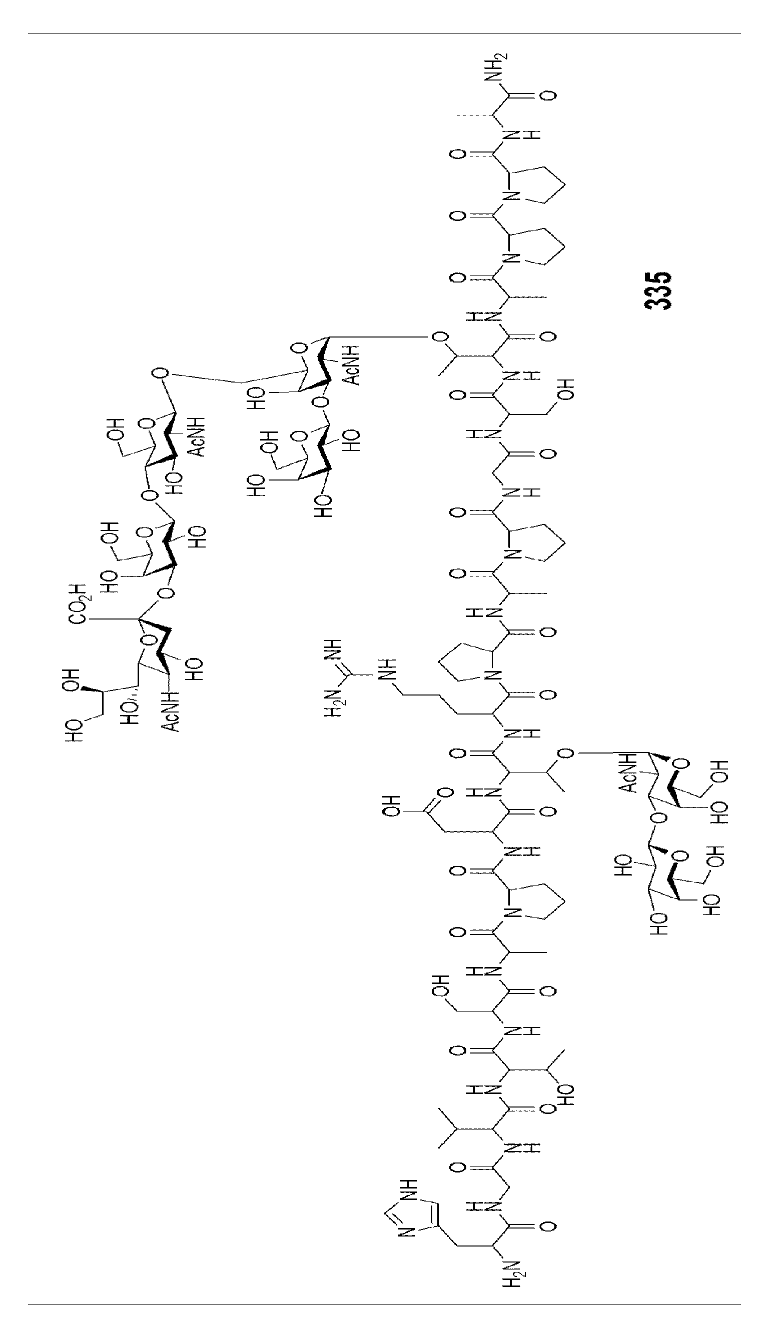

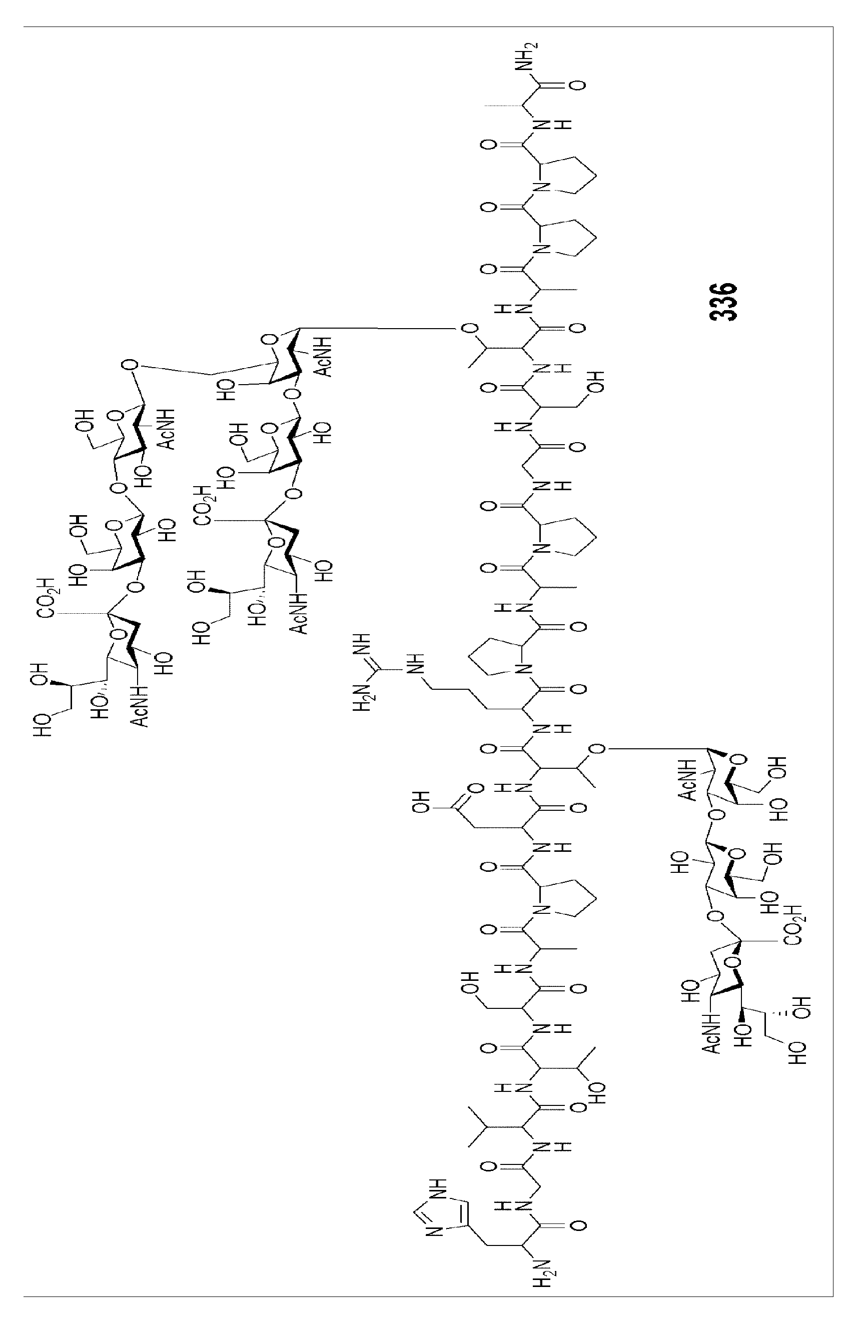

The following reaction solutions A) to E) were allowed to react for 24 hours at 25°C:
A) 100µl of reaction solution containing 50mMHEPES buffer solution (pH 7.0), 0.1U/ml human-derived β1,4-galactosyltransferase (available from TOYOBO CO., LTD.), 10mM manganese chloride, 0.1% BSA, 2mM uridine-5'-disodium diphosphogalactose (UDP-Gal) and glycopeptide derivative (218) (4mM at a theoretical content from solid-phase synthesis);
B) 100µl of reaction solution containing 50mM HEPES buffer solution (pH 7.0), 0.0175U/ml recombinant rat α2,3-(O)-sialyltransferase (available from Calbiochem), 10mM manganese chloride, 0.1% BSA, 2mM cytidine-5'-sodium monophosphosialate (CMP-NANA) and glycopeptide derivative (218) (4mM at a theoretical content from solid-phase synthesis);
C) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (218) (4mM at a theoretical content from solid-phase synthesis);
D) 100µl of reaction solution containing 50mM HEPES buffer solution (pH 7.0), 0.1U/ml human-derived β1,4-galactosyltransferase (available from TOYOBO CO., LTD.), 0.0185U/ml recombinant rat α2,3-(N)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (218) (4mM at a theoretical content from solid-phase synthesis);
E) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 0.0185U/ml recombinant rat α2,3-(N)-sialyltransferase (available from Calbiochem), 10mM manganese chloride, 0.1% BSA, 4mM uridine-5'-disodium diphosphogalactose (UDP-Gal), 2mM cytidine-5'-sodium monophosphosialate (CMP-NANA) and glycopeptide derivative (218) (4mM at a theoretical content from solid-phase synthesis).

After completion of the reaction, the respective reaction solutions were subjected to centrifugal concentration with an ultrafiltration filter. Thereafter, 25mM ammonium acetate buffer solution (pH 6.5) was added thereto, and the solution was again subjected to concentration with a centrifugal separator, thereby washing the polymer. This manipulation was repeated three times, thereby respectively obtaining aqueous solutions of compounds (326) to (330). Thereafter, to the solution retained at the filter containing compounds (326) to (330) and a solution which was obtained by diluting an aqueous solution of (218) with 25mM ammonium acetate buffer solution (pH 6.5), 1µl of 0.174mg/ml solution of BLase (available from Shionogi & Co., Ltd.) was added, and the solution was allowed to react for two hours at room temperature. Thereafter, the solution was subjected to centrifugal filtration with an ultrafiltration filter, thereby separating the glycopeptide of interest from the polymer. The obtained aqueous solution (filtrate) was lyophilized, thereby obtaining compounds (331) to (336).
MALDI-TOF/MS:[M(average)+H]⁺ of compound (331)=2821.7 (theoretical value:[M(average)+H]⁺=2820.9); MALDI-TOF/MS:[M(average)+H]⁺ of compound (332)=2983.3 (theoretical value:[M(average)+H]⁺=2983.0); MALDI-TOF/MS:[M(average)+H]⁺ of compound (333)=3404.6 (theoretical value:[M(average)+H]⁺=3403.4); MALDI-TOF/MS:[M(average)+H]⁺ of compound (334)=3566.4 (theoretical value:[M(average)+H]⁺=3565.5); MALDI-TOF/MS:[M(average)+H]⁺ of compound (335)=3276.4 (theoretical value:[M(average)+H]⁺=3274.3); and MALDI-TOF/MS:[M(average)+H]⁺ of compound (336)=3858.4 (theoretical value:[M(average)+H]⁺=3856.8).

(3.61 Synthesis of compounds (337) to (347))

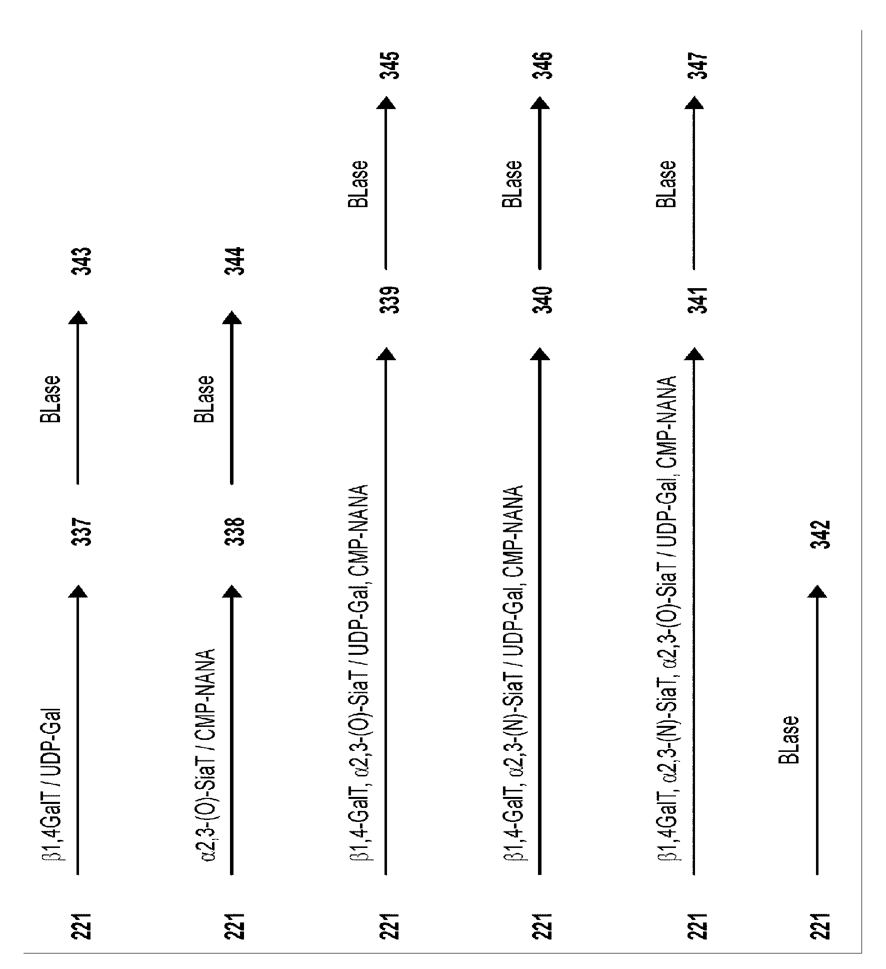

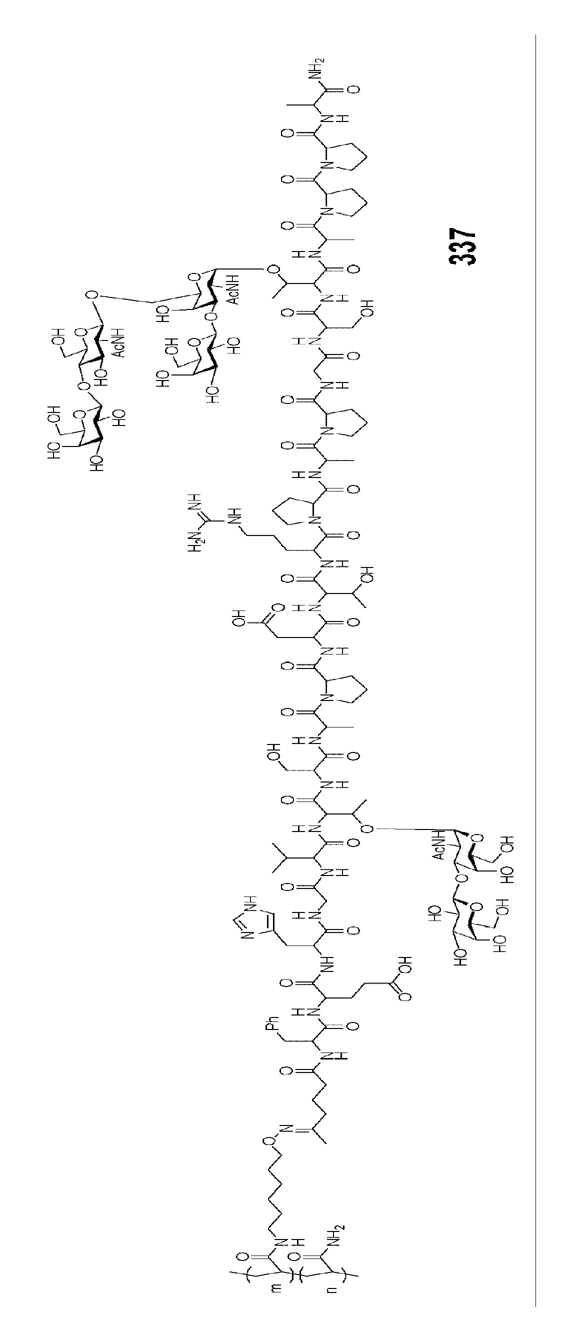

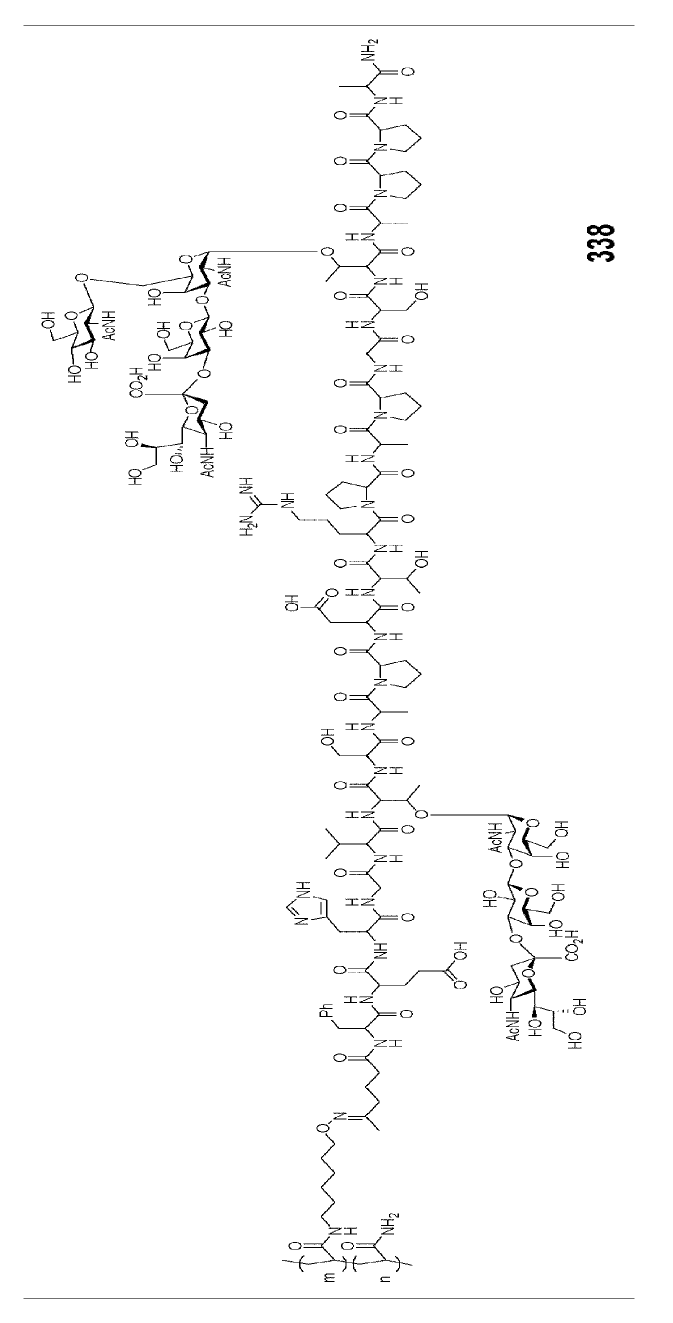

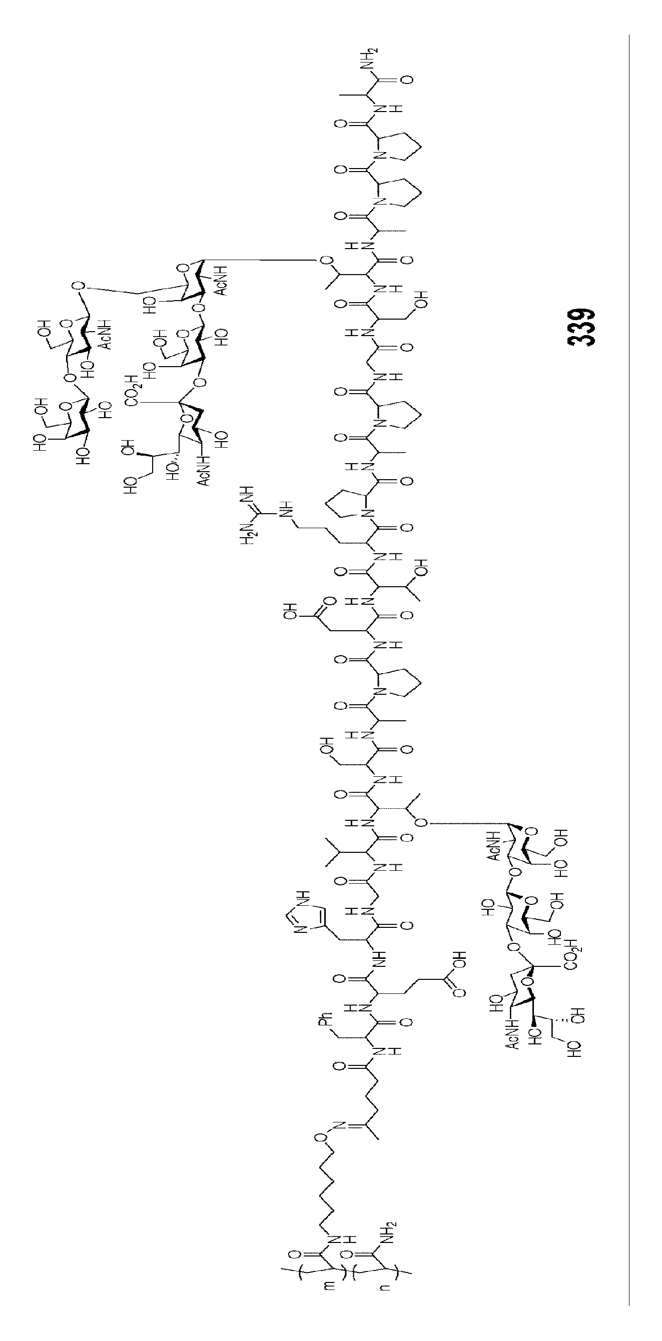

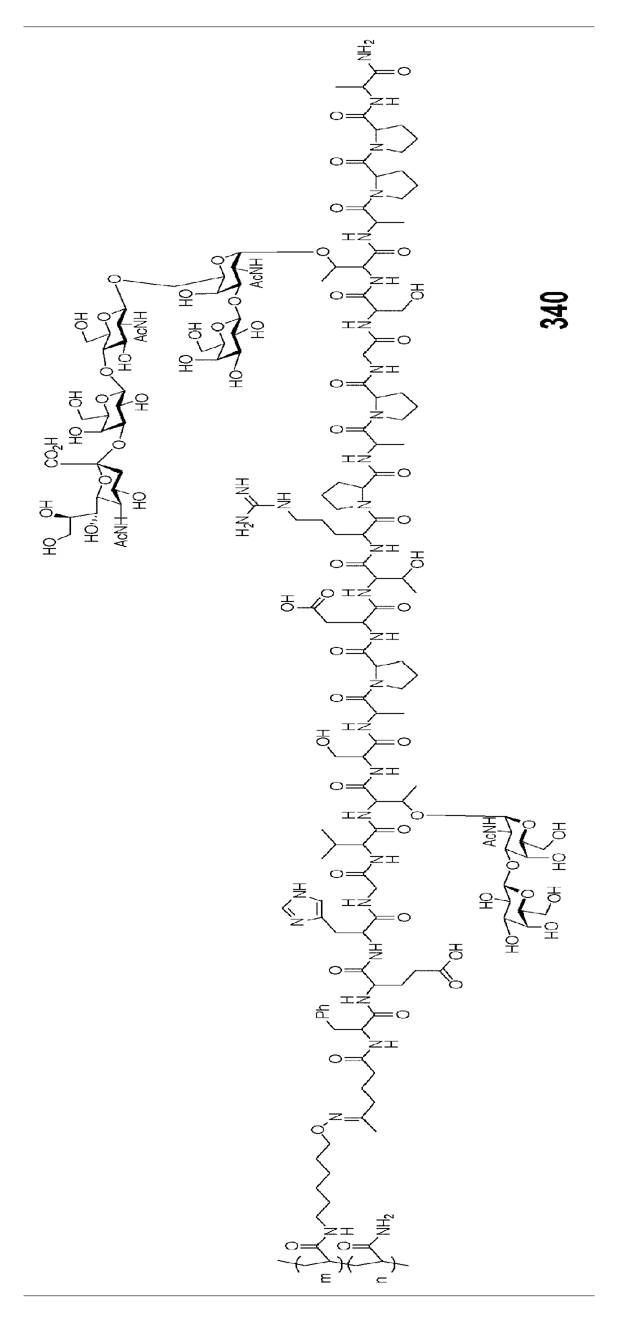

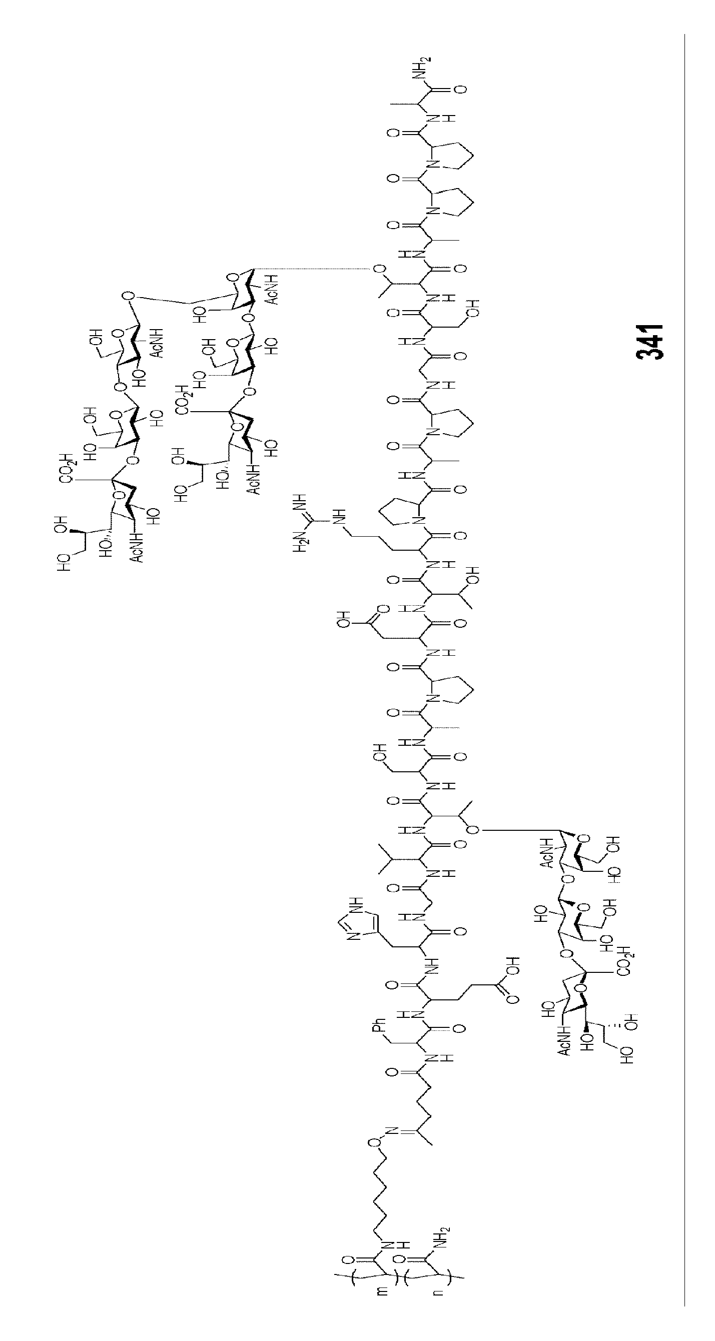

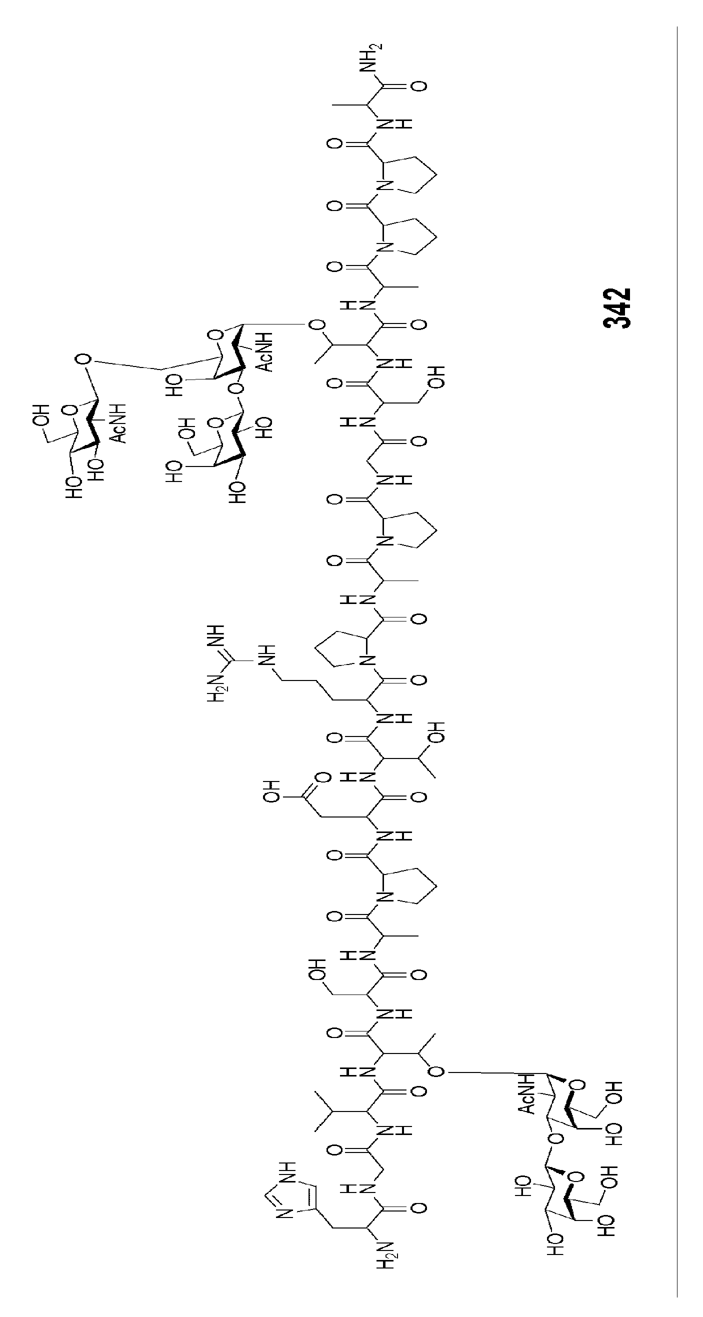

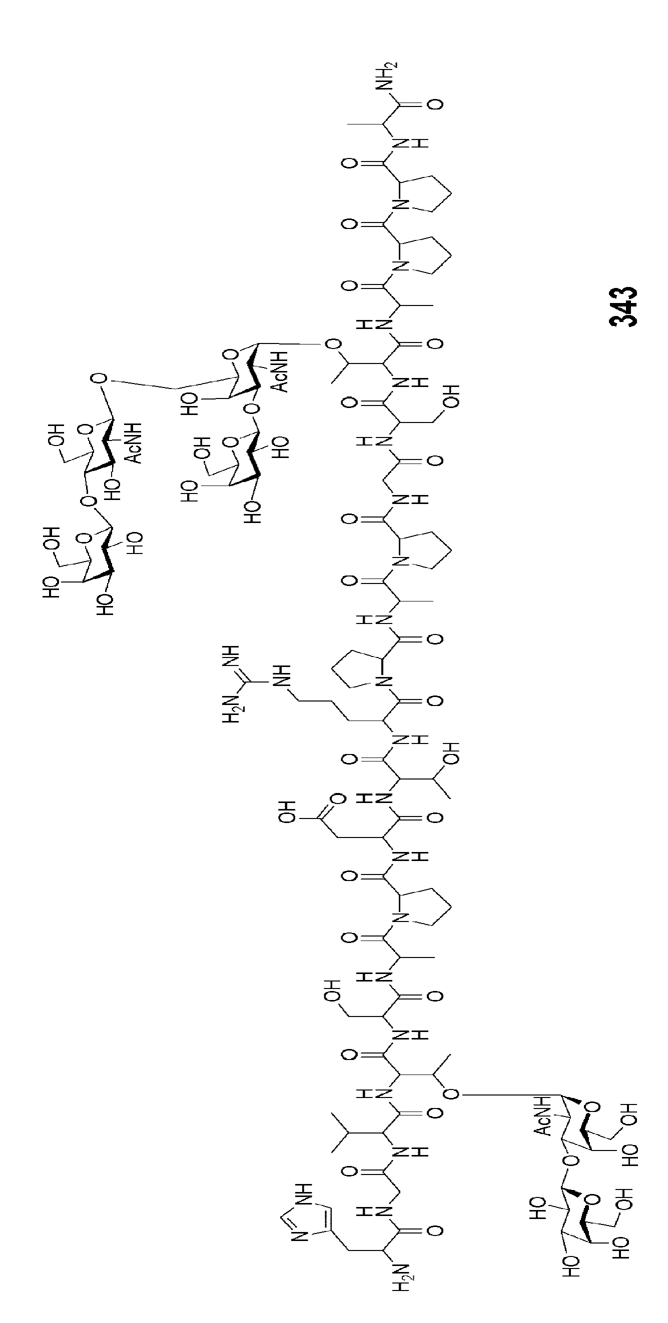

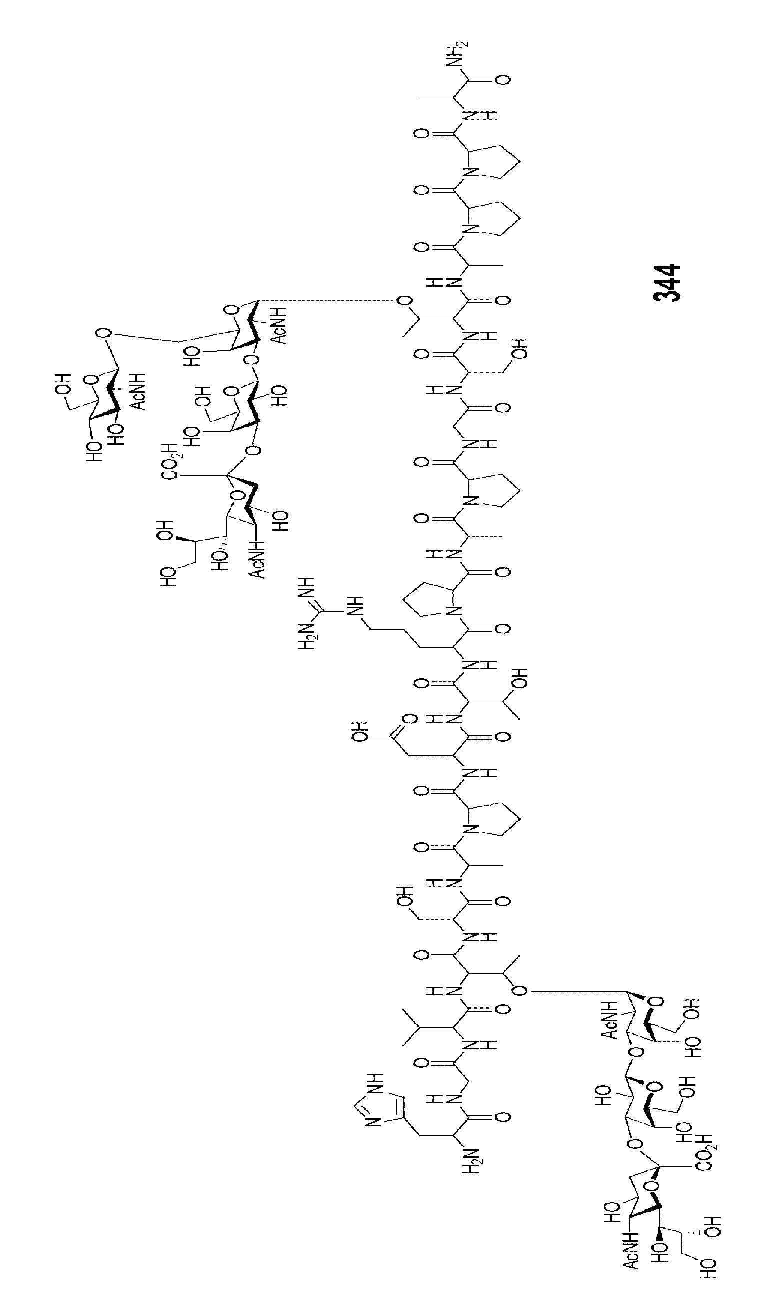

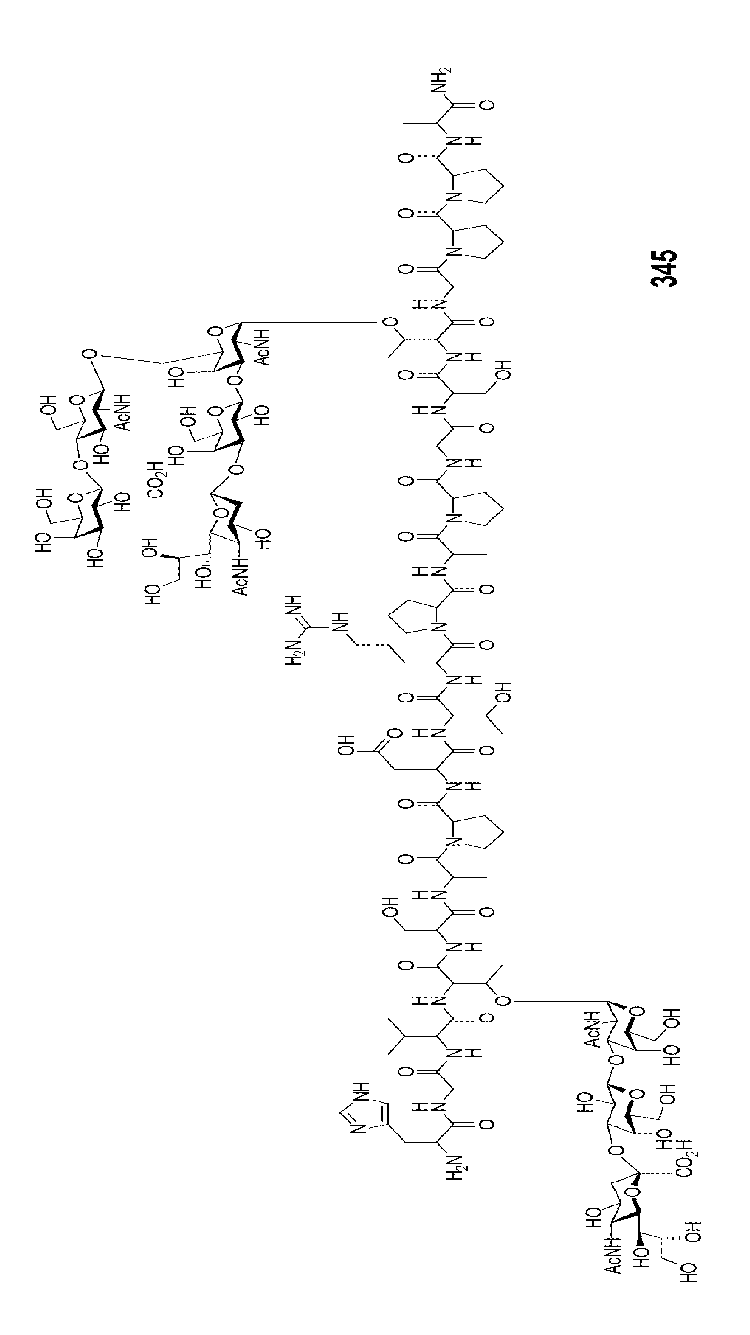

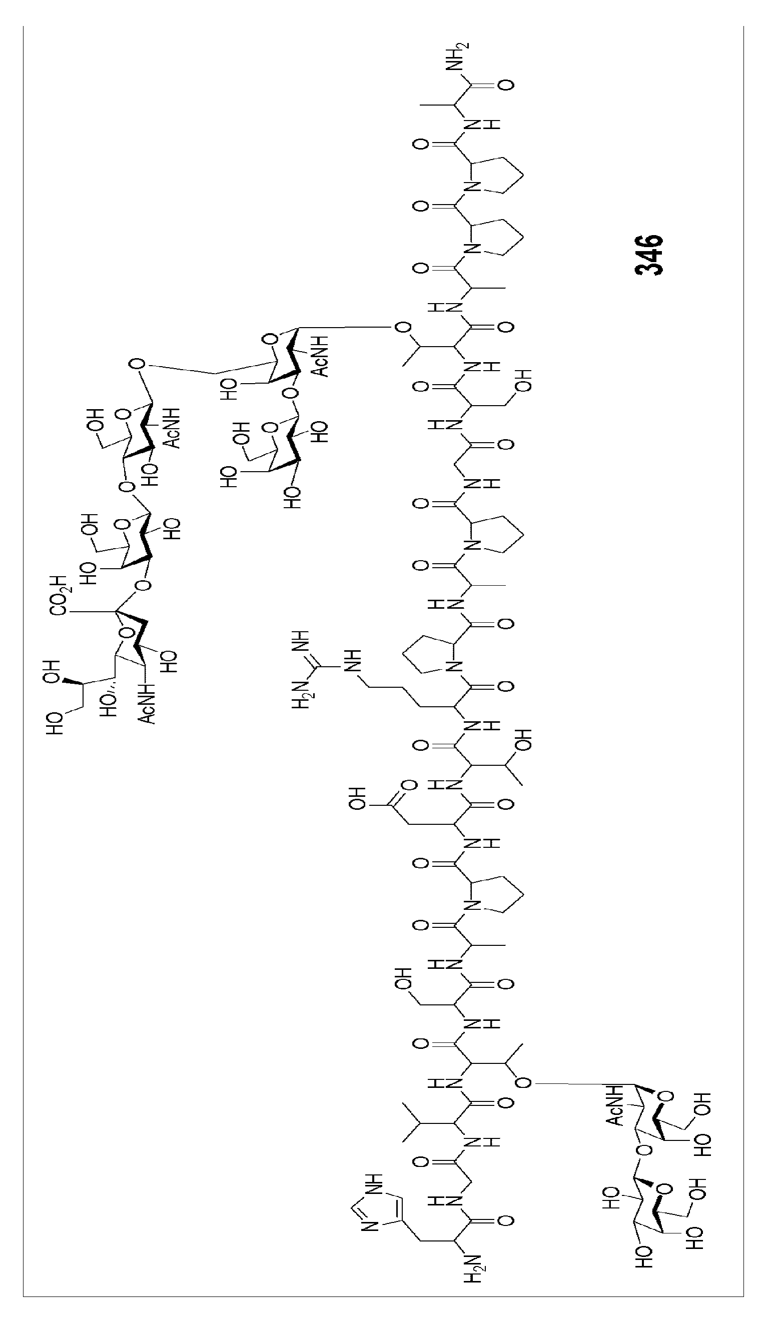

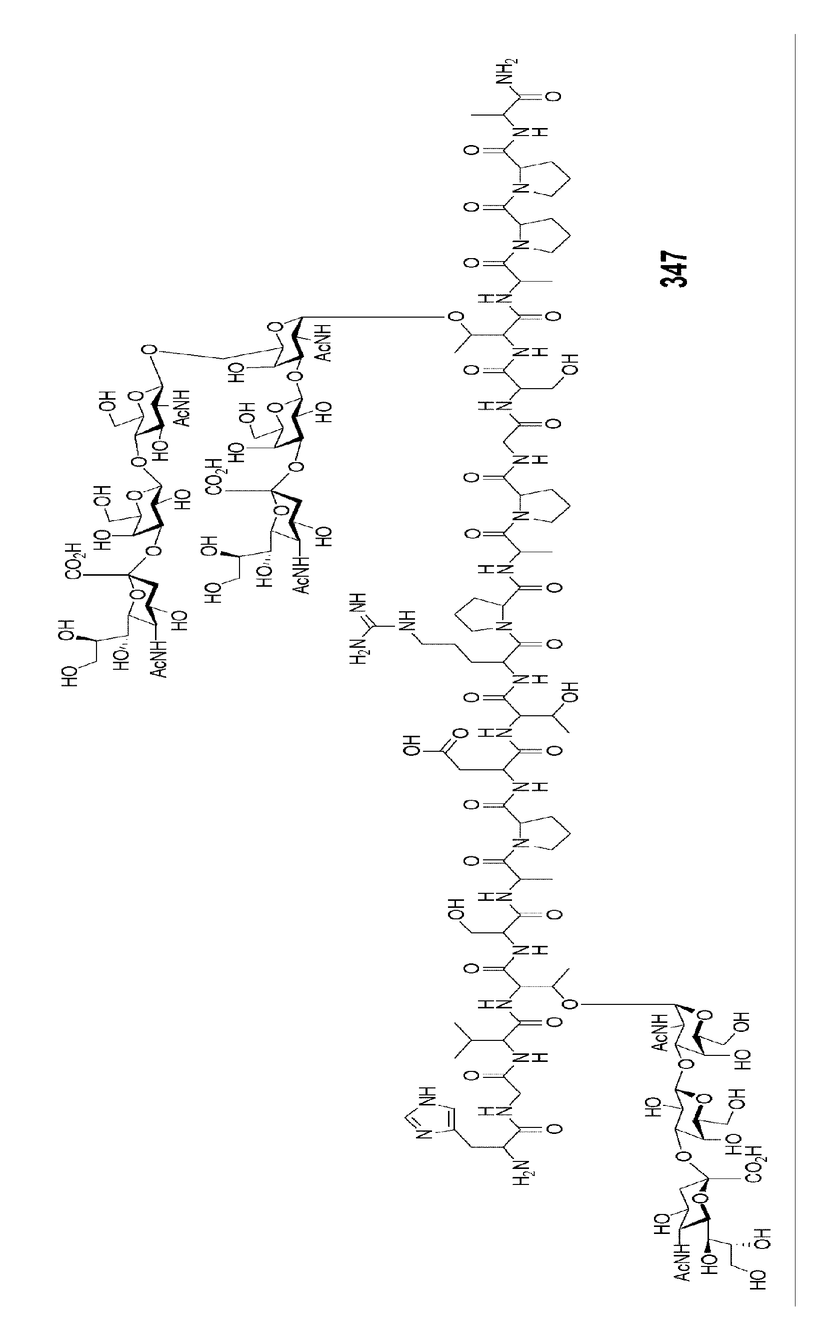

The following reaction solutions A) to E) were allowed to react for 24 hours at 25°C:
A) 100µl of reaction solution containing 50mMHEPES buffer solution (pH 7.0), 0.1U/ml human-derived β1,4-galactosyltransferase (available from TOYOBO CO., LTD.), 10mM manganese chloride, 0.1% BSA, 2mM uridine-5'-disodium diphosphogalactose (UDP-Gal) and glycopeptide derivative (221) (4mM at a theoretical content from solid-phase synthesis);
B) 100µl of reaction solution containing 50mM HEPES buffer solution (pH 7.0), 0.0175U/ml recombinant rat α2,3-(O)-sialyltransferase (available from Calbiochem), 10mM manganese chloride, 0.1% BSA, 2mM cytidine-5'-sodium monophosphosialate (CMP-NANA) and glycopeptide derivative (221) (4mM at a theoretical content from solid-phase synthesis);
C) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (221) (4mM at a theoretical content from solid-phase synthesis);
D) 100µl of reaction solution containing 50mM HEPES buffer solution (pH 7.0), 0.1U/ml human-derived β1,4-galactosyltransferase (available from TOYOBO CO., LTD.), 0.0185U/ml recombinant rat α2,3-(N)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (221) (4mM at a theoretical content from solid-phase synthesis);
E) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 0.0185U/ml recombinant rat α2,3-(N)-sialyltransferase (available from Calbiochem), 10mM manganese chloride, 0.1% BSA, 4mM uridine-5'-disodium diphosphogalactose (UDP-Gal), 2mM cytidine-5'-sodium monophosphosialate (CMP-NANA) and glycopeptide derivative (221) (4mM at a theoretical content from solid-phase synthesis).

After completion of the reaction, the respective reaction solutions were subjected to centrifugal concentration with an ultrafiltration filter. Thereafter, 25mM ammonium acetate buffer solution (pH 6.5) was added thereto, and the solution was again subjected to concentration with a centrifugal separator, thereby washing the polymer. This manipulation was repeated three times, thereby respectively obtaining aqueous solutions of compounds (337) to (341). Thereafter, to the solution retained at the filter containing compounds (337) to (341) and a solution which was obtained by diluting an aqueous solution of (221) with 25mM ammonium acetate buffer solution (pH 6.5), 1µl of 0.174mg/ml solution of BLase (available from Shionogi & Co., Ltd.) was added, and the solution was allowed to react for two hours at room temperature. Thereafter, the solution was subjected to centrifugal filtration with an ultrafiltration filter, thereby separating the glycopeptide of interest from the polymer. The obtained aqueous solution (filtrate) was lyophilized, thereby obtaining compounds (342) to (347).
MALDI-TOF/MS:[M(average)+H]⁺ of compound (342)=2822.2 (theoretical value:[M(average)+H]⁺=2820.9); MALDI-TOF/MS:[M(average)+H]⁺ of compound (343)=2984.6 (theoretical value:[M(average)+H]⁺=2983.0); MALDI-TOF/MS:[M(average)+H]⁺ of compound (344)=3405.3 (theoretical value:[M(average)+H]⁺=3403.4); MALDI-TOF/MS:[M(average)+H]⁺ of compound (345)=3567.4 (theoretical value:[M(average)+H]⁺=3565.5); MALDI-TOF/MS:[M(average)+H]⁺ of compound (346)=3276.1 (theoretical value:[M(average)+H]⁺=3274.3); and MALDI-TOF/MS:[M(average)+H]⁺ of compound (347)=3857.0 (theoretical value:[M(average)+H]⁺=3856.8).

(3.62 Synthesis of compounds (348) to (358))

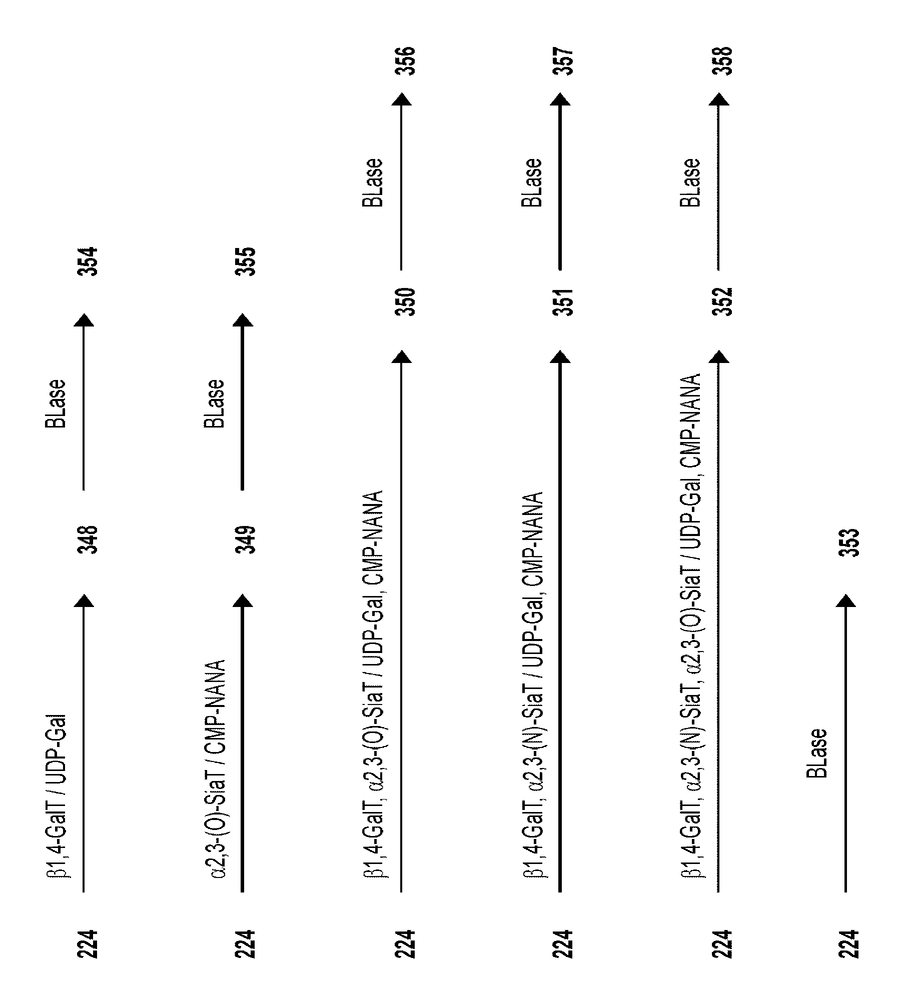

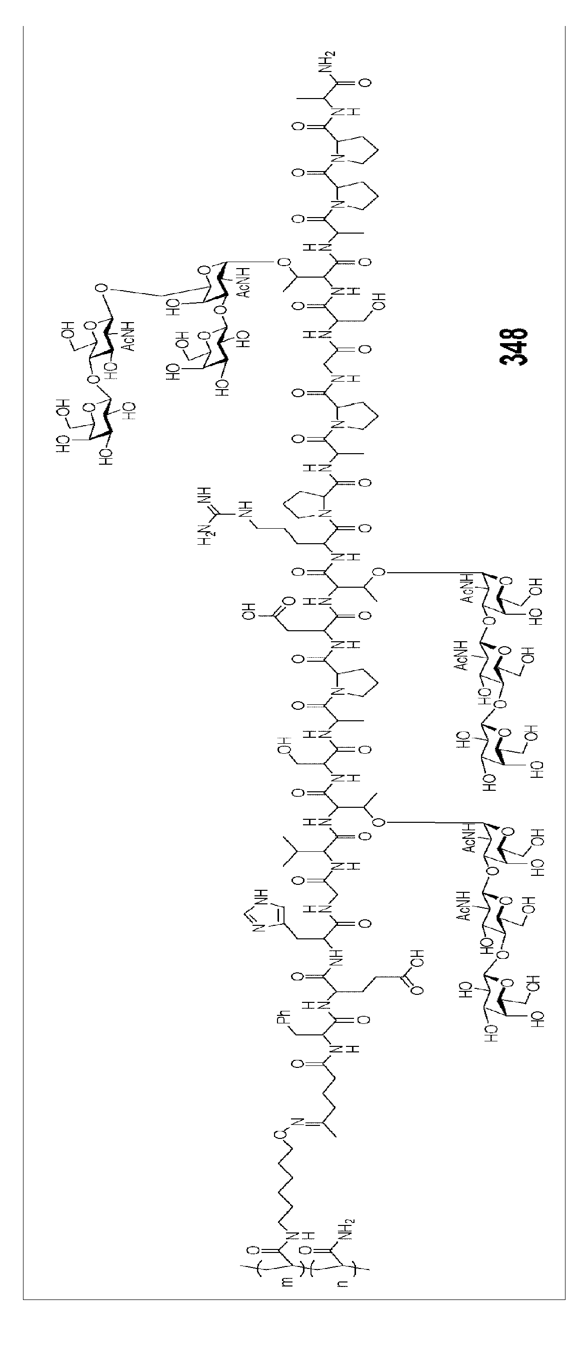

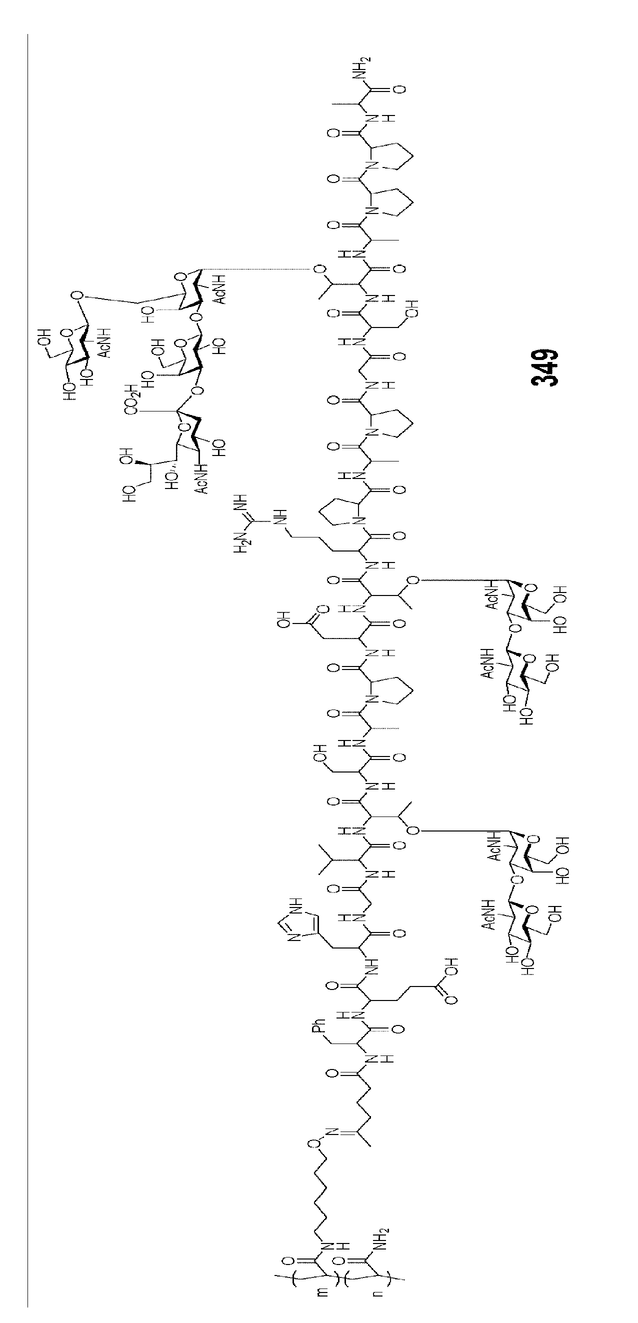

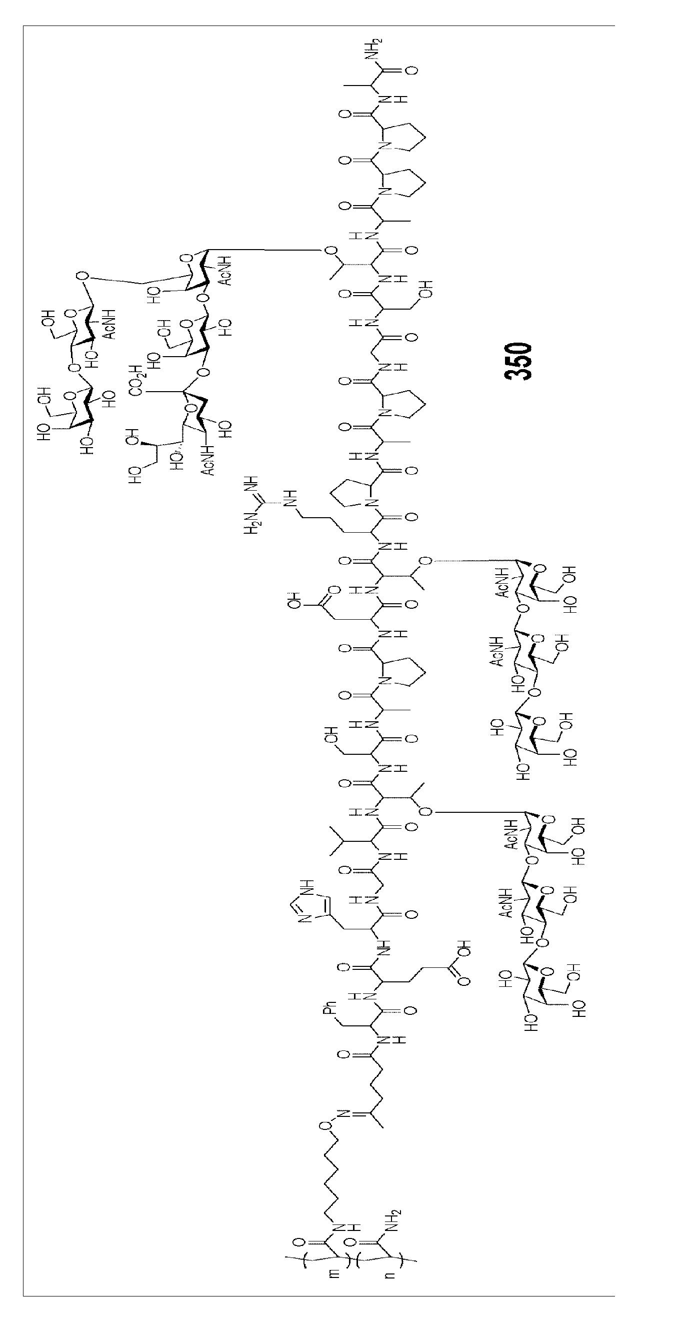

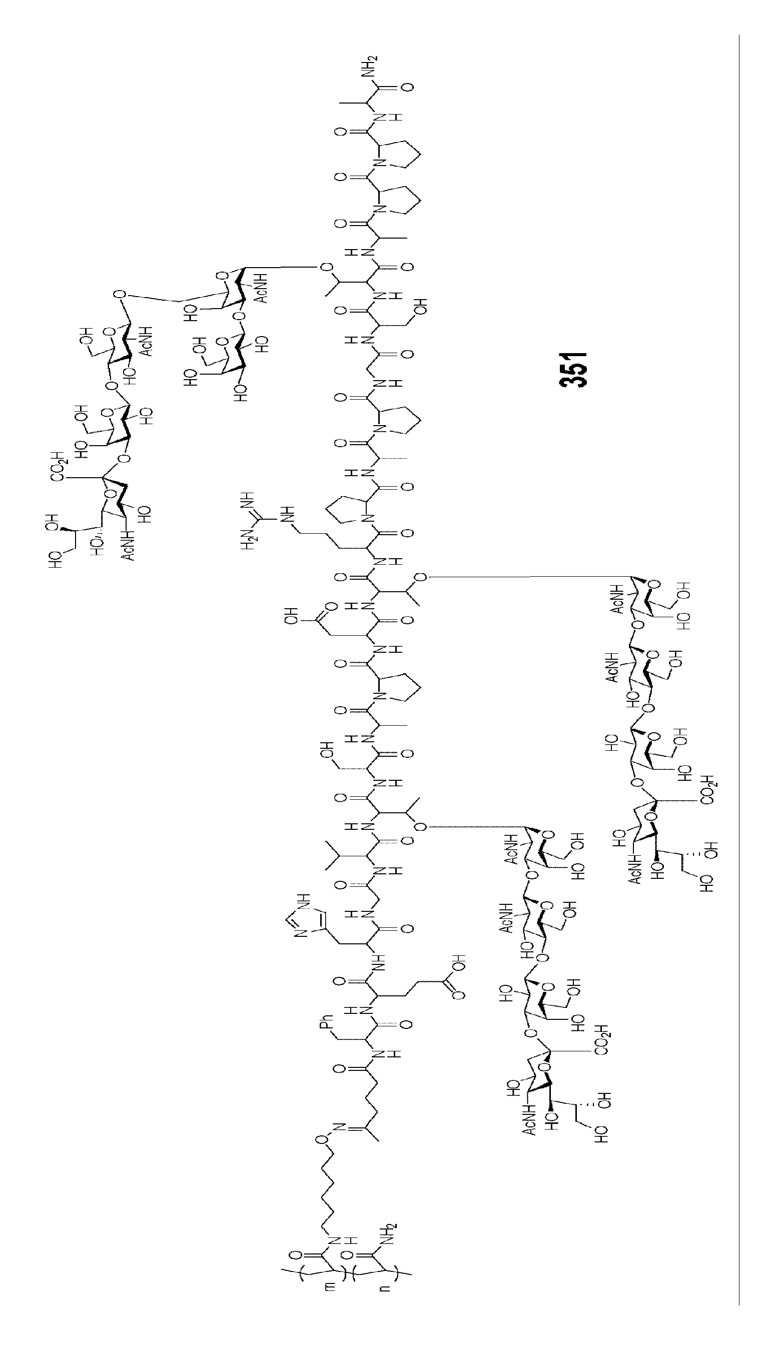

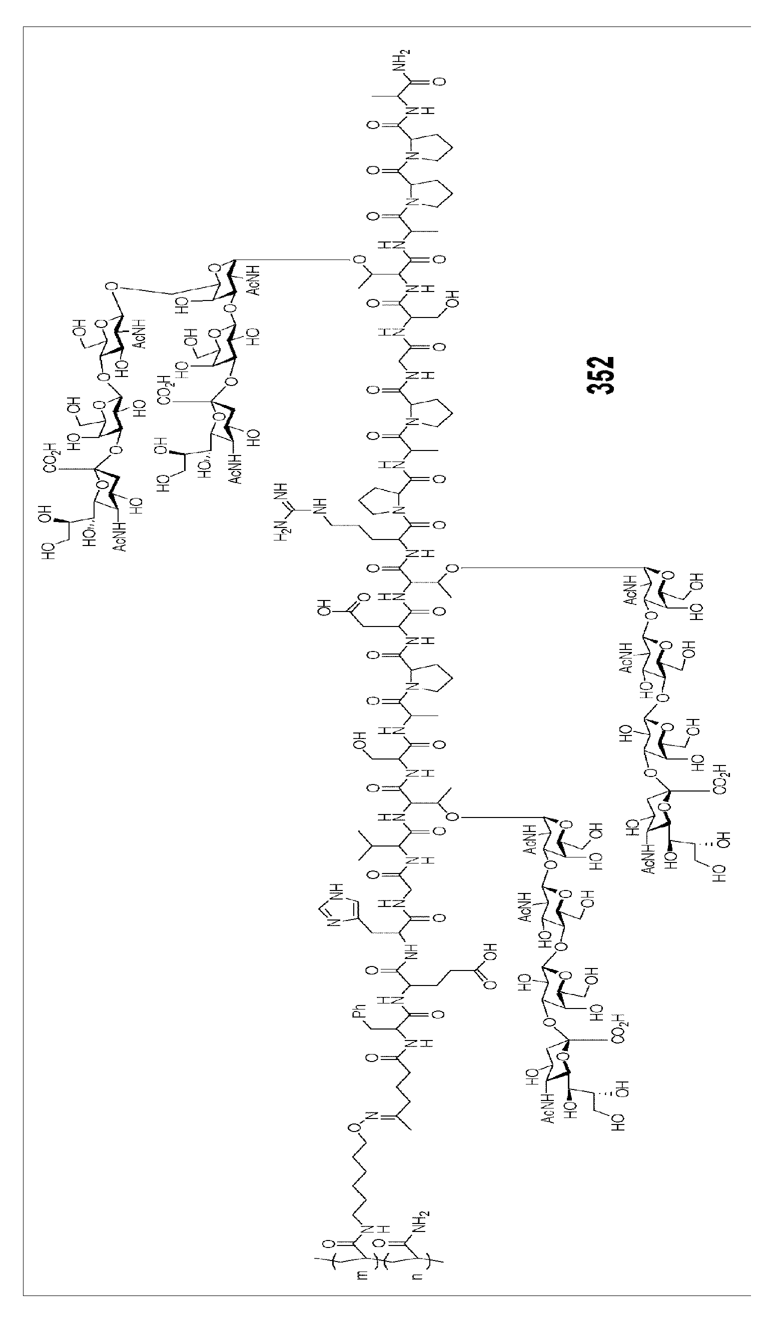

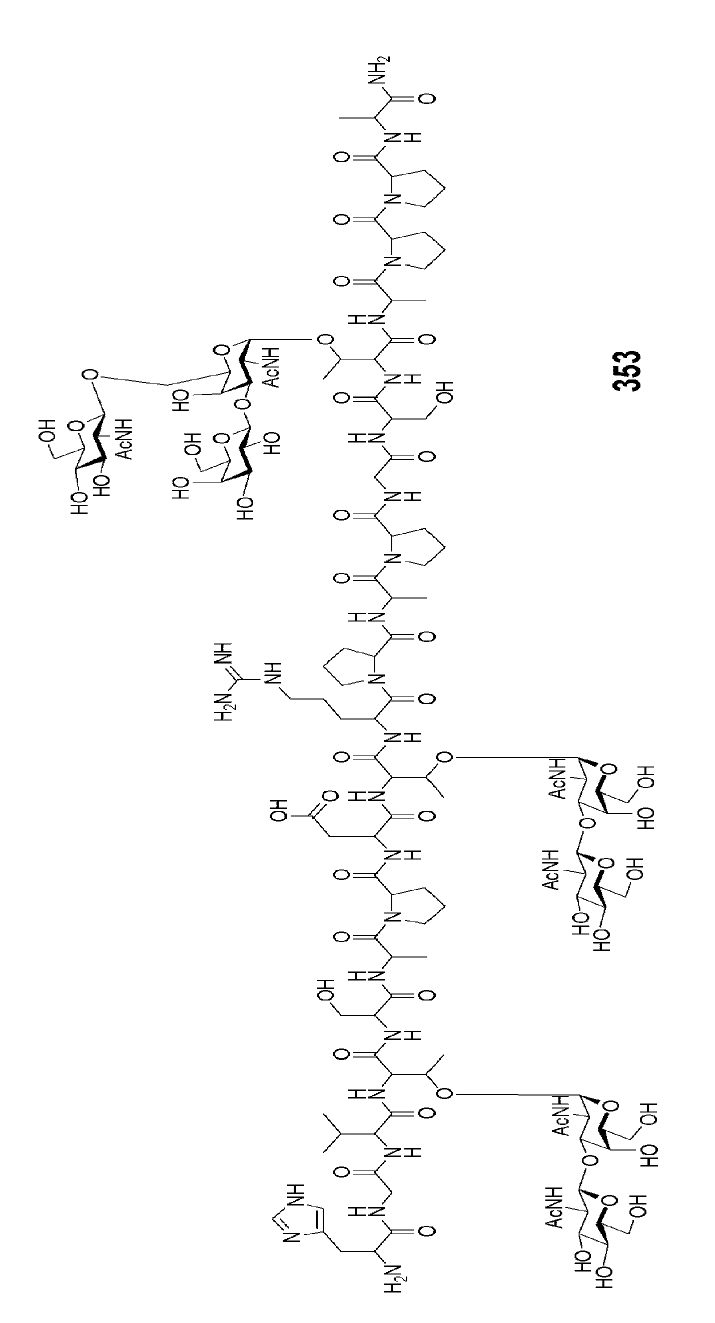

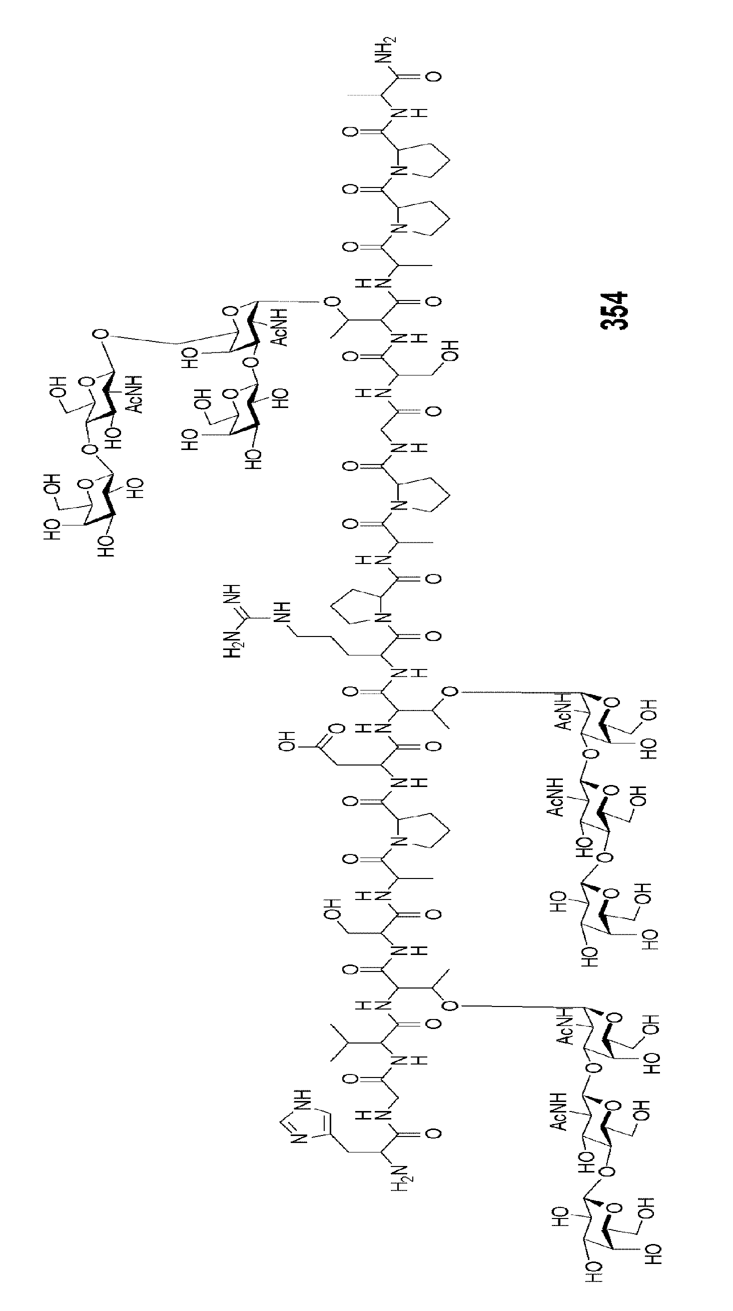

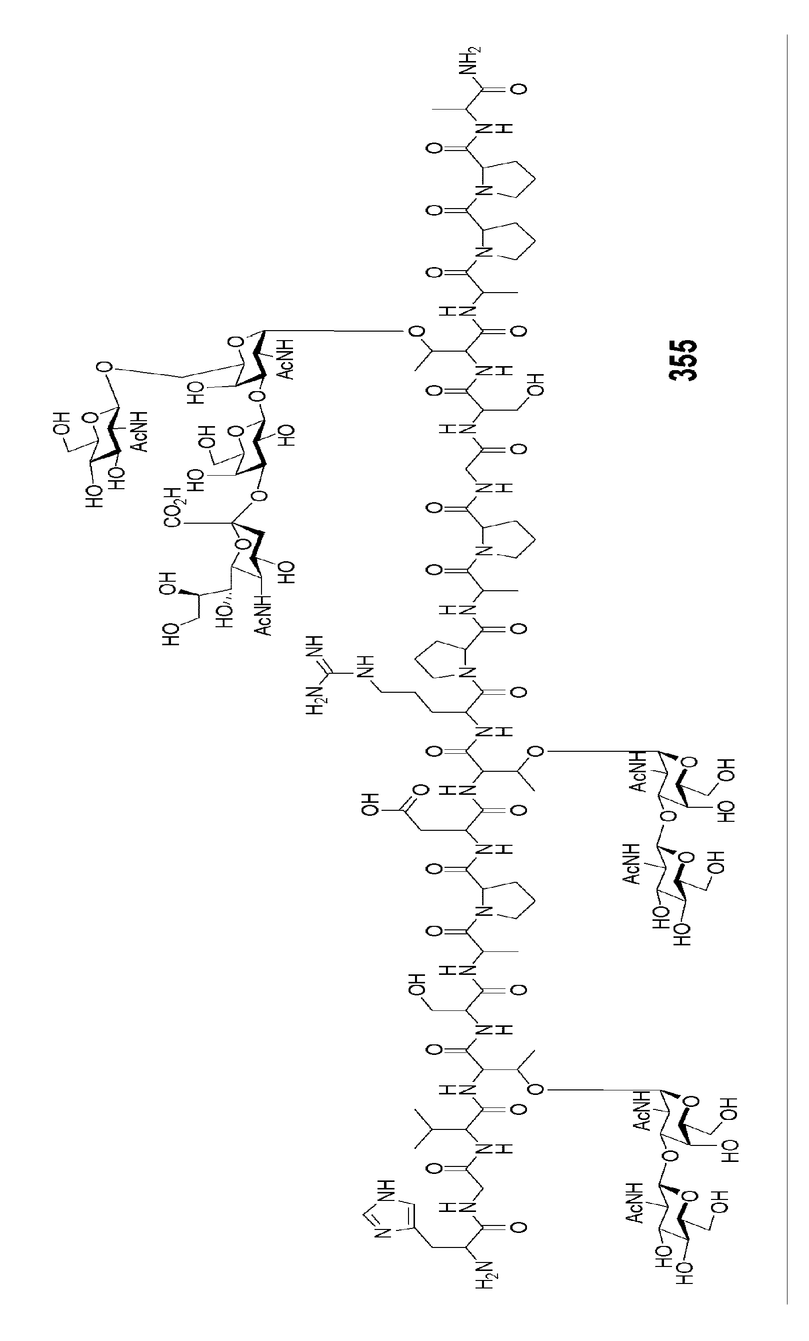

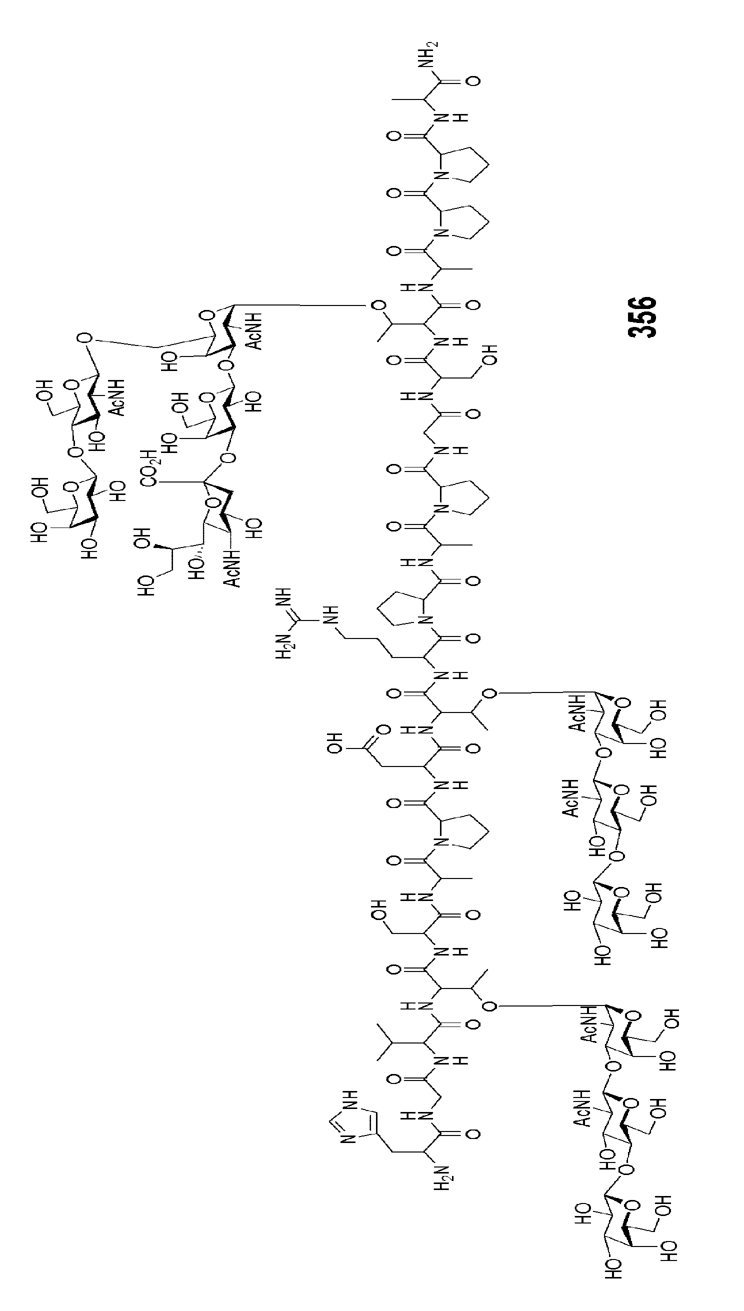

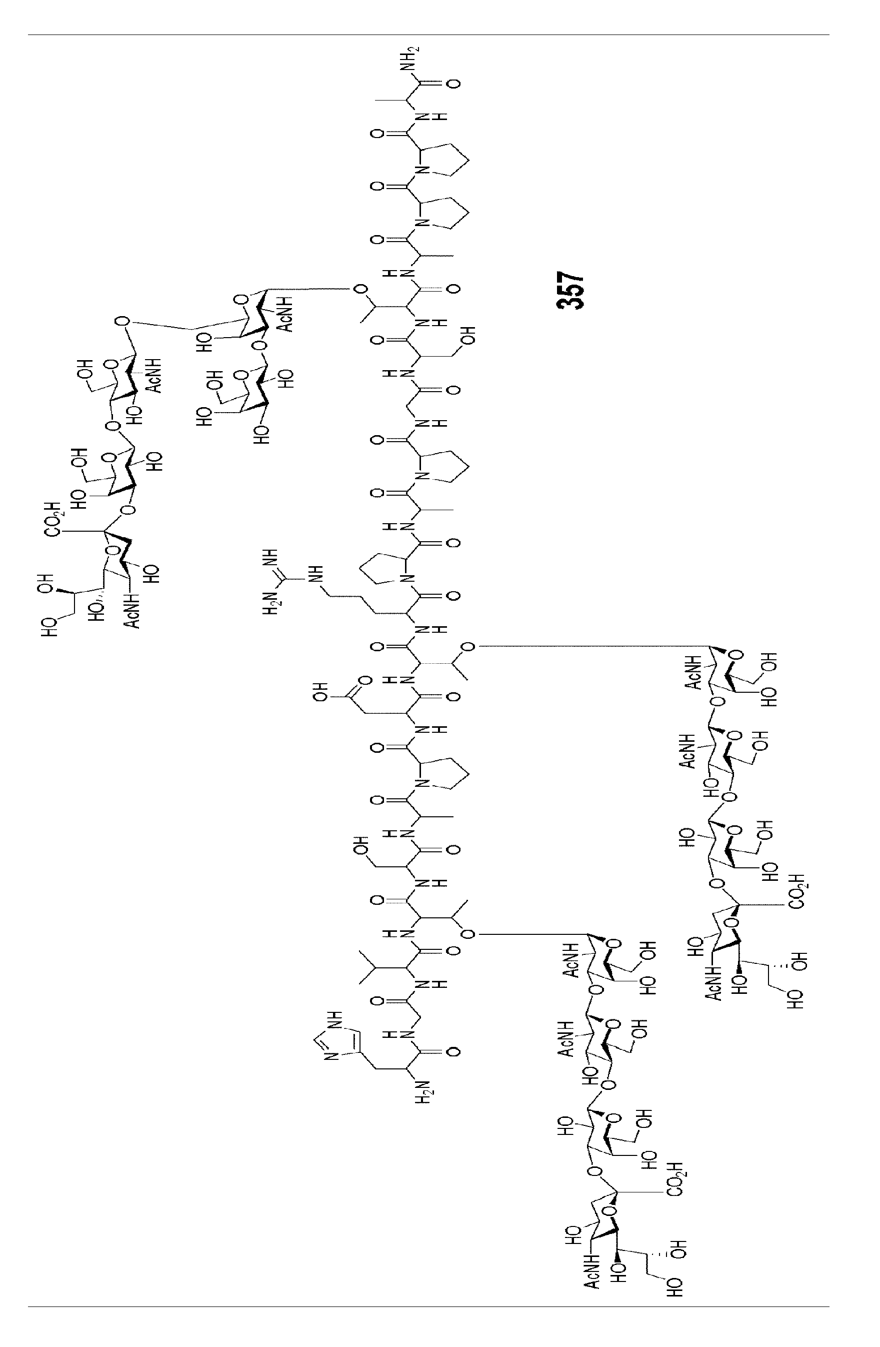

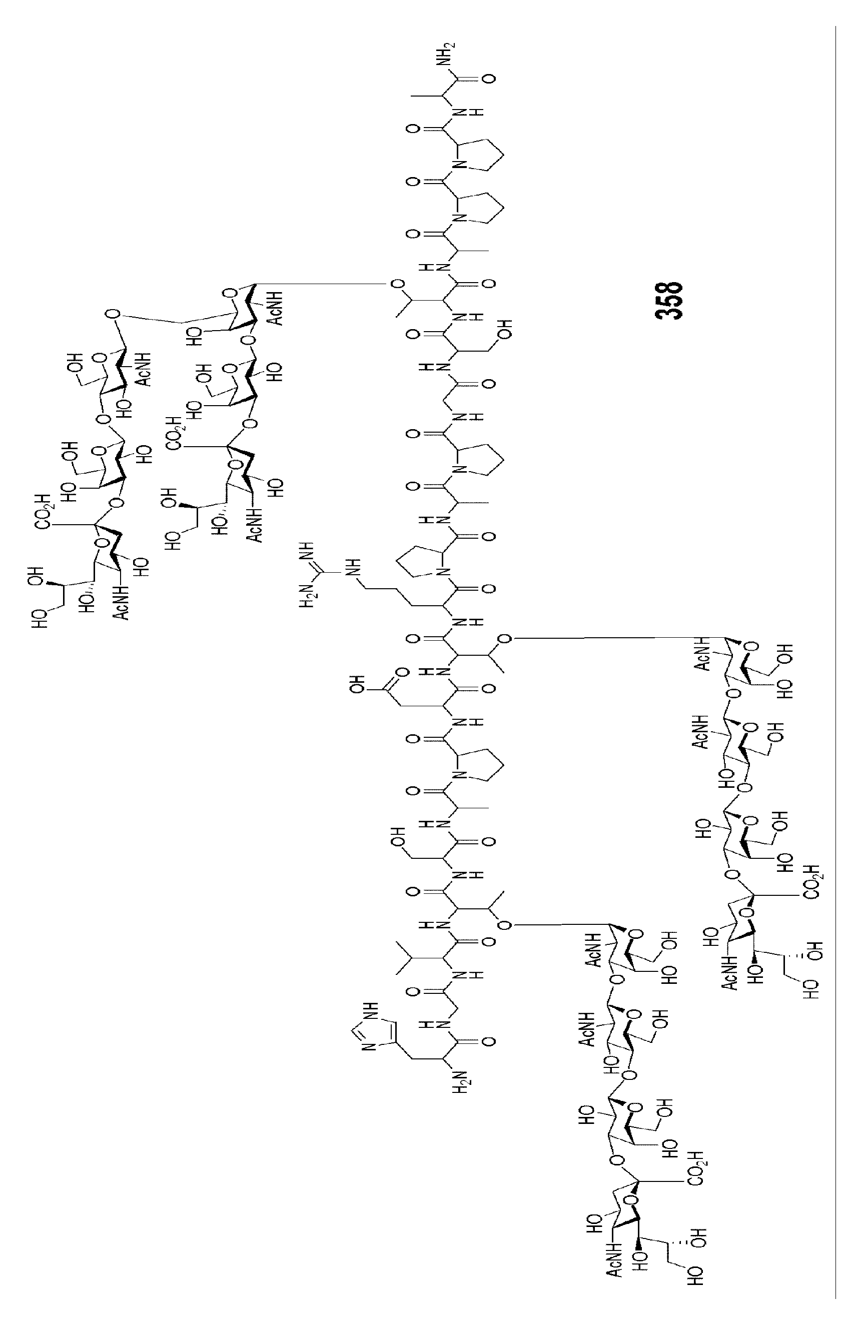

The following reaction solutions A) to E) were allowed to react for 24 hours at 25°C:
A) 100µl of reaction solution containing 50mMHEPES buffer solution (pH 7.0), 0.1U/ml human-derived β1,4-galactosyltransferase (available from TOYOBO CO., LTD.), 10mM manganese chloride, 0.1% BSA, 2mM uridine-5'-disodium diphosphogalactose (UDP-Gal) and glycopeptide derivative (224) (4mM at a theoretical content from solid-phase synthesis);
B) 100µl of reaction solution containing 50mM HEPES buffer solution (pH 7.0), 0.0175U/ml recombinant rat α2,3-(O)-sialyltransferase (available from Calbiochem), 10mM manganese chloride, 0.1% BSA, 2mM cytidine-5'-sodium monophosphosialate (CMP-NANA) and glycopeptide derivative (224) (4mM at a theoretical content from solid-phase synthesis);
C) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (224) (4mM at a theoretical content from solid-phase synthesis);
D) 100µl of reaction solution containing 50mM HEPES buffer solution (pH 7.0), 0.1U/ml human-derived β1,4-galactosyltransferase (available from TOYOBO CO., LTD.), 0.0185U/ml recombinant rat α2,3-(N)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (224) (4mM at a theoretical content from solid-phase synthesis);
E) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 0.0185U/ml recombinant rat α2,3-(N)-sialyltransferase (available from Calbiochem), 10mM manganese chloride, 0.1% BSA, 4mM uridine-5'-disodium diphosphogalactose (UDP-Gal), 2mM cytidine-5'-sodium monophosphosialate (CMP-NANA) and glycopeptide derivative (224) (4mM at a theoretical content from solid-phase synthesis).

After completion of the reaction, the respective reaction solutions were subjected to centrifugal concentration with an ultrafiltration filter. Thereafter, 25mM ammonium acetate buffer solution (pH 6.5) was added thereto, and the solution was again subjected to concentration with a centrifugal separator, thereby washing the polymer. This manipulation was repeated three times, thereby respectively obtaining aqueous solutions of compounds (348) to (352). Thereafter, to the solution retained at the filter containing compounds (348) to (352) and a solution which was obtained by diluting an aqueous solution of (224) with 25mM ammonium acetate buffer solution (pH 6.5), 1µl of 0.174mg/ml solution of BLase (available from Shionogi & Co., Ltd.) was added, and the solution was allowed to react for two hours at room temperature. Thereafter, the solution was subjected to centrifugal filtration with an ultrafiltration filter, thereby separating the glycopeptide of interest from the polymer. The obtained aqueous solution (filtrate) was lyophilized, thereby obtaining compounds (353) to (358).
MALDI-TOF/MS:[M(average)+H]⁺ of compound (353)=3269.5 (theoretical value:[M(average)+H]⁺=3268.3); MALDI-TOF/MS:[M(average)+H]⁺ of compound (354)=3757.0 (theoretical value:[M(average)+H]⁺=3754.8); MALDI-TOF/MS:[M(average)+H]⁺ of compound (355)=3561.7 (theoretical value:[M(average)+H]⁺=3559.6); MALDI-TOF/MS:[M(average)+H]⁺ of compound (356)=4047.7 (theoretical value:[M(average)+H]⁺=4046.0); MALDI-TOF/MS:[M(average)+H]⁺ of compound (357)=4625.4 (theoretical value:[M(average)+H]⁺=4628.5); and MALDI-TOF/MS:[M(average)+H]⁺ of compound (357)=4917.9 (theoretical value: [M (average) +H]⁺=4919.9).

(3.63 Synthesis of compounds (359) to (369))

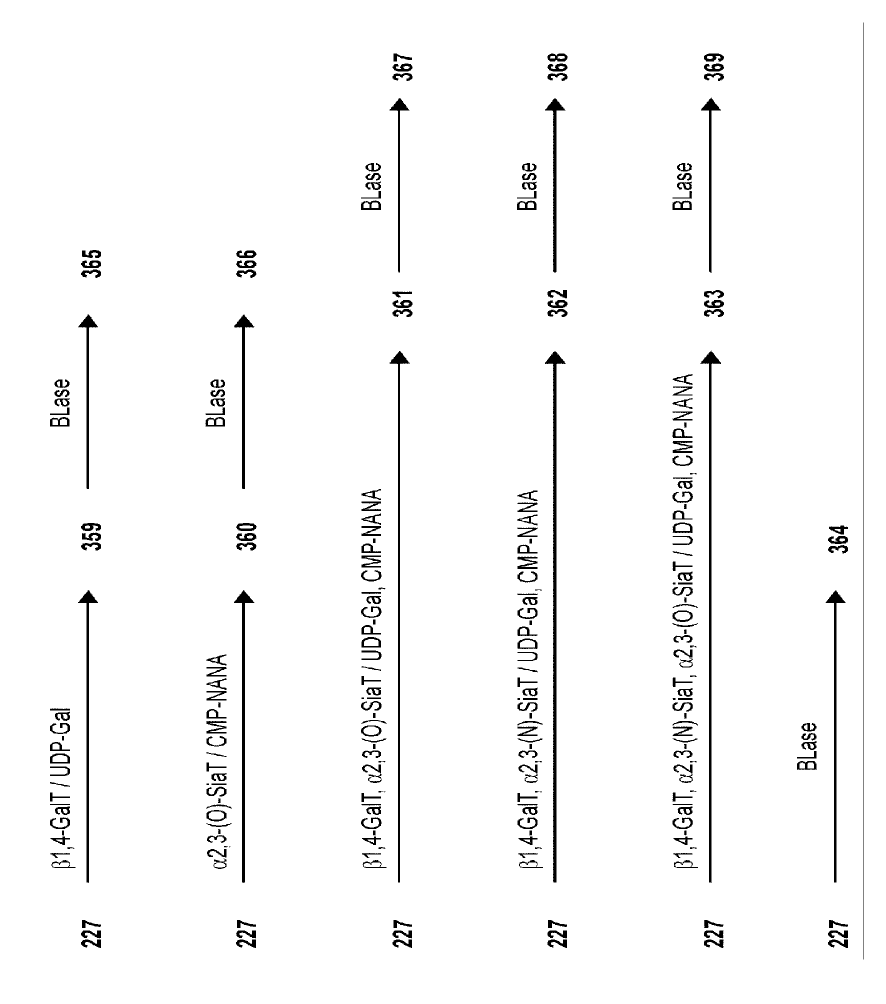

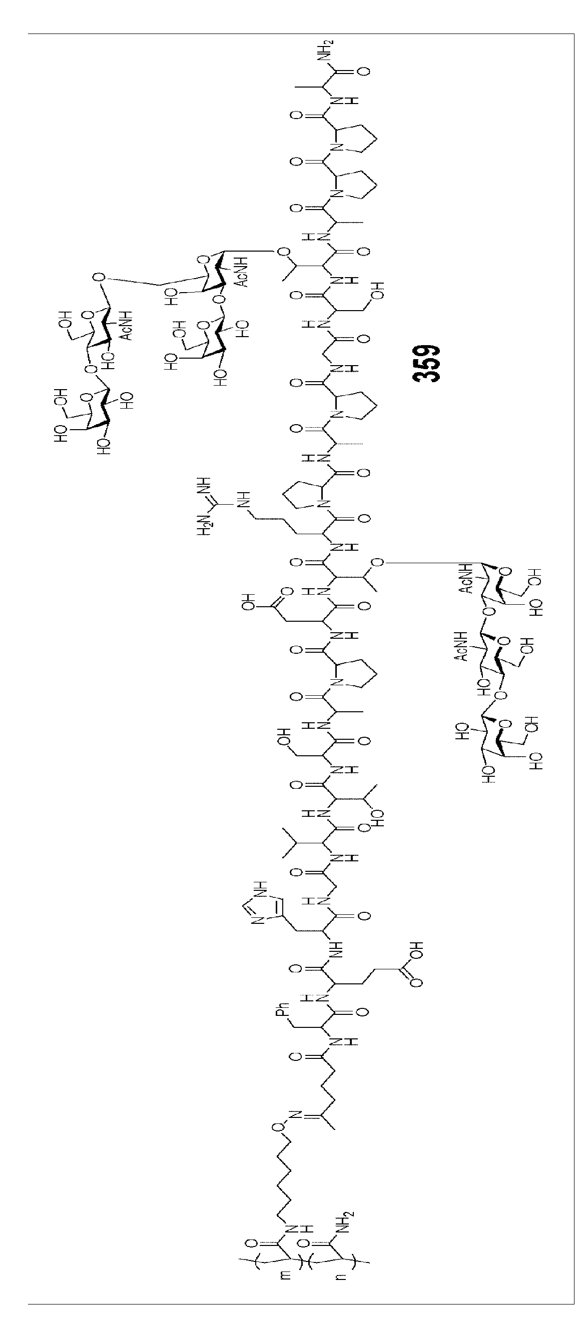

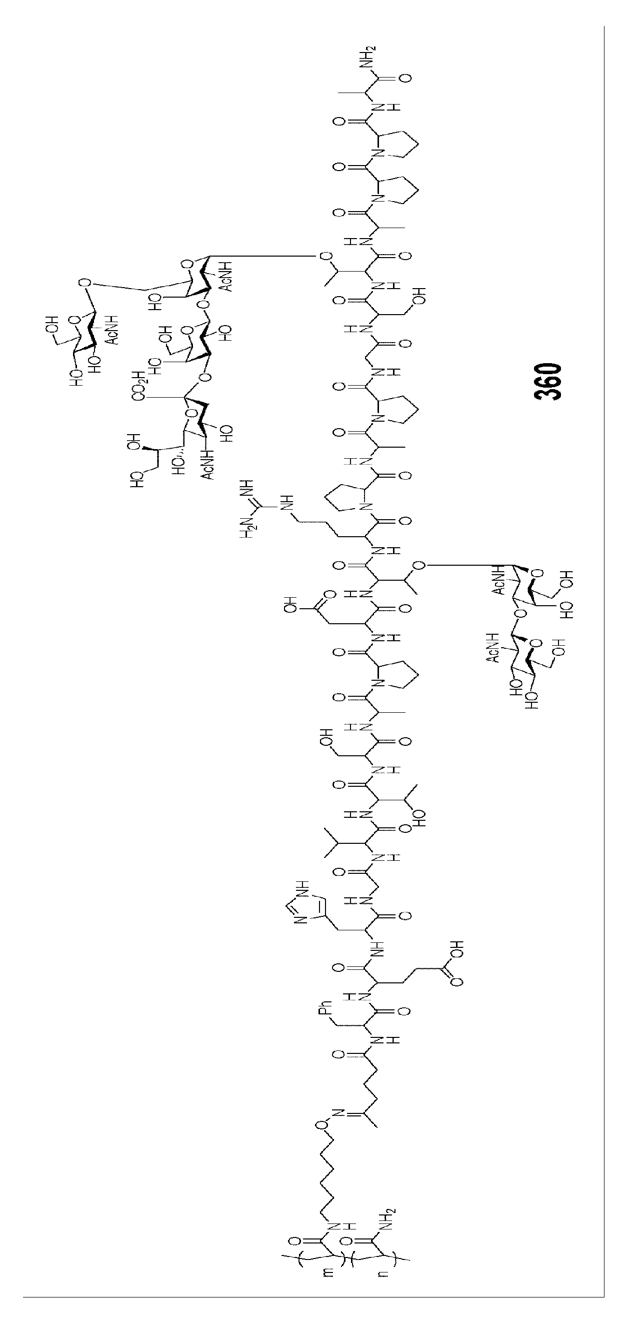

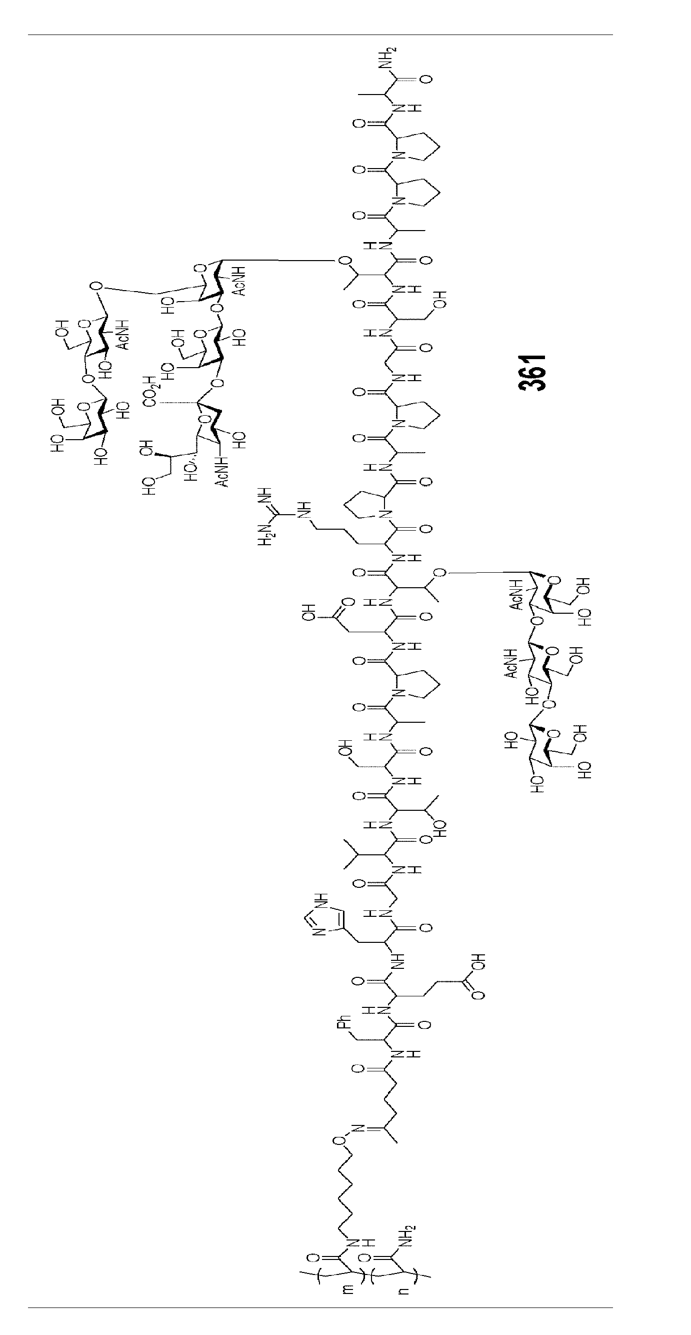

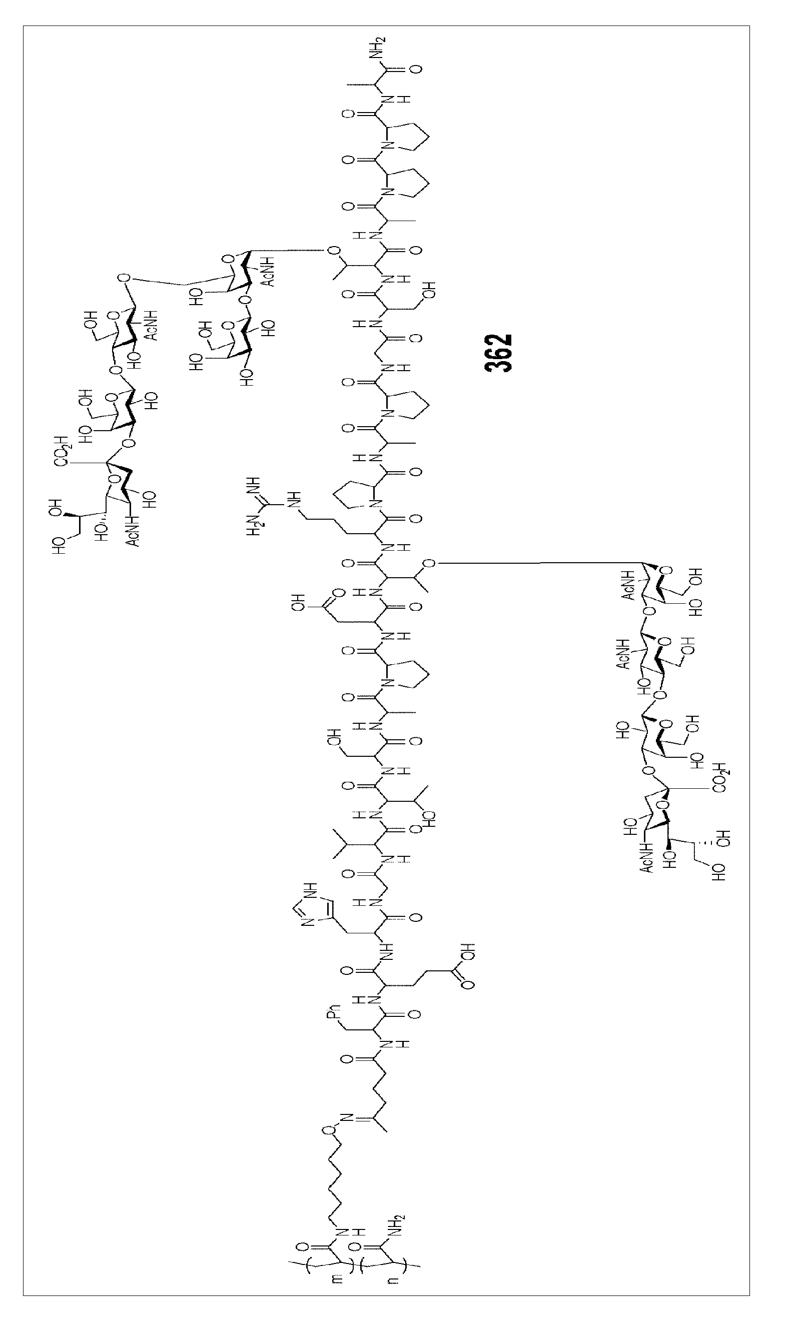

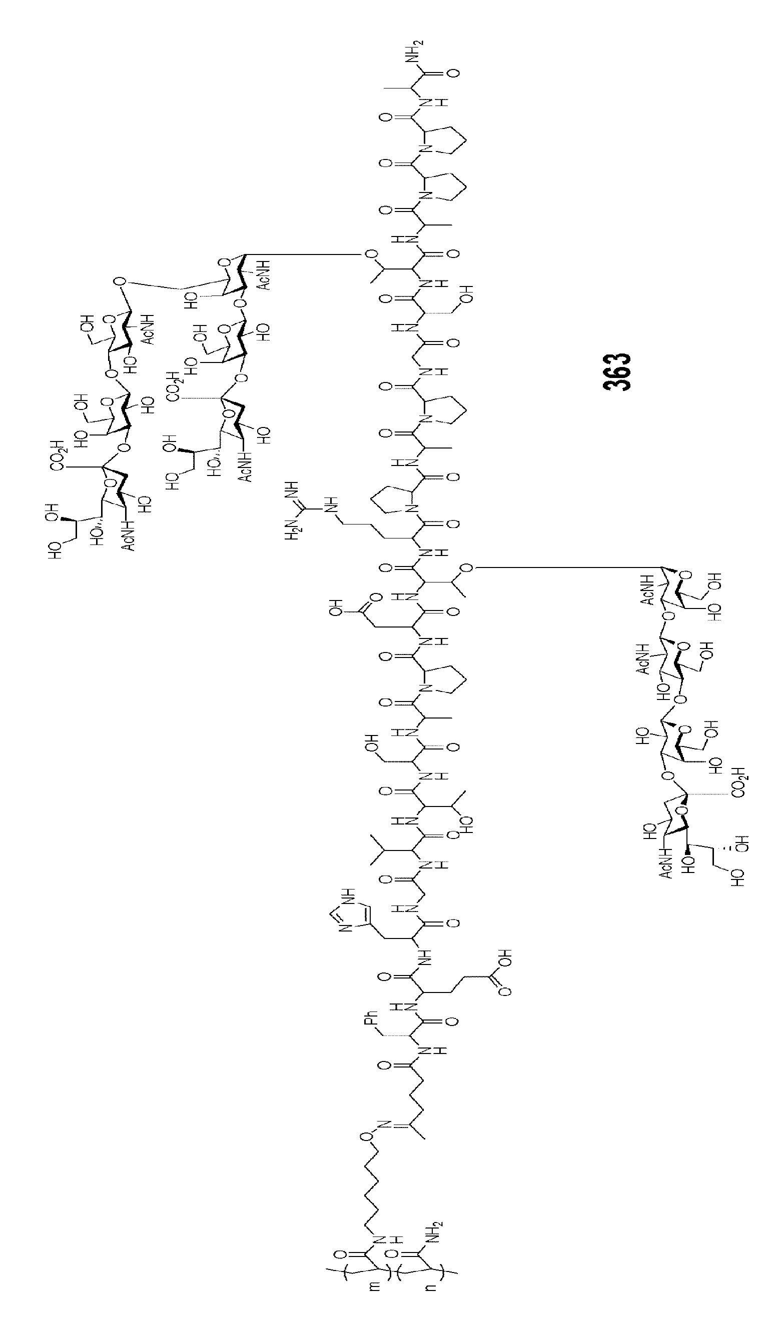

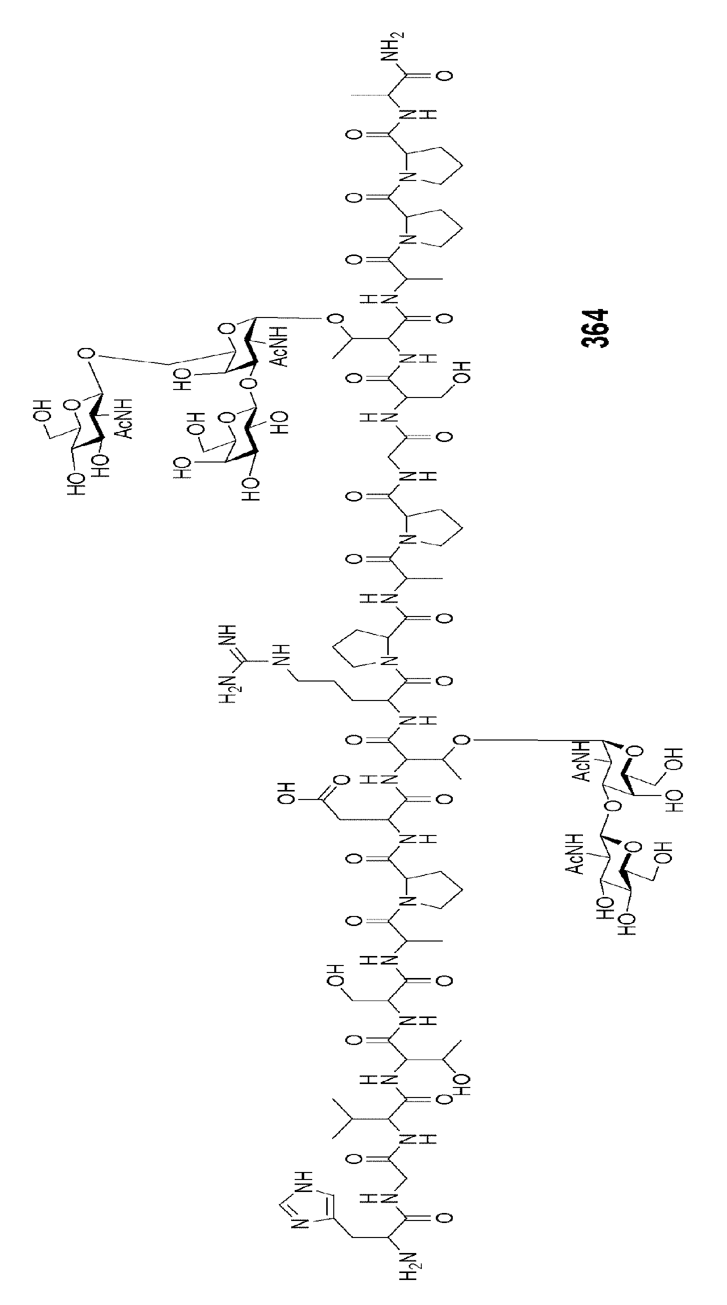

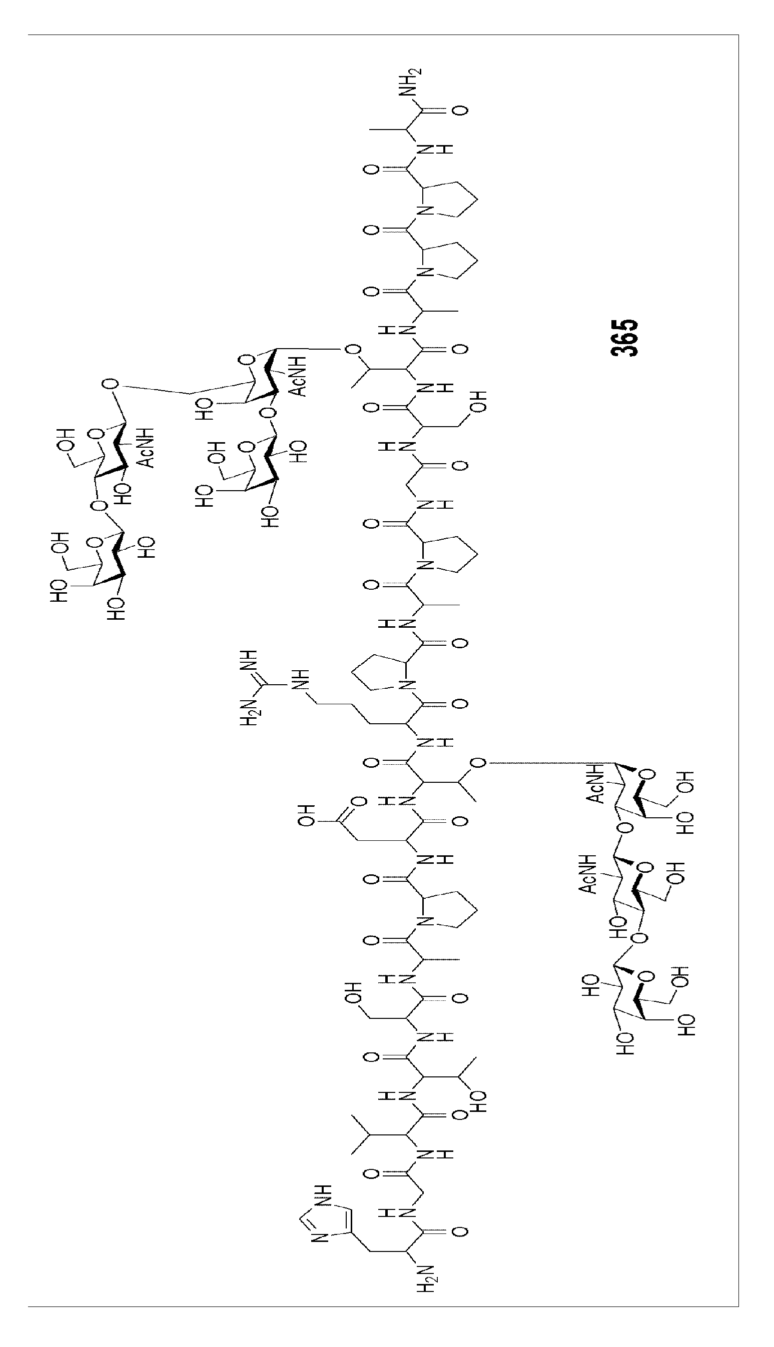

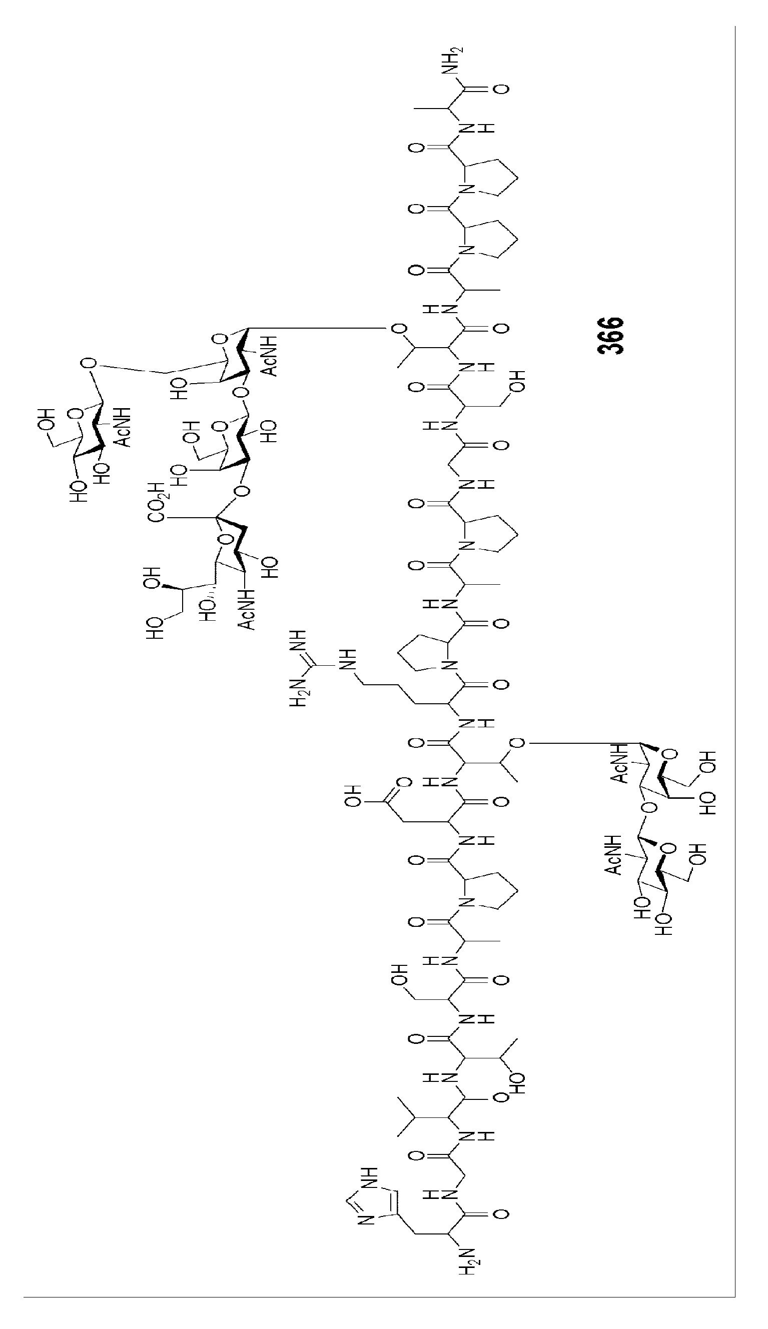

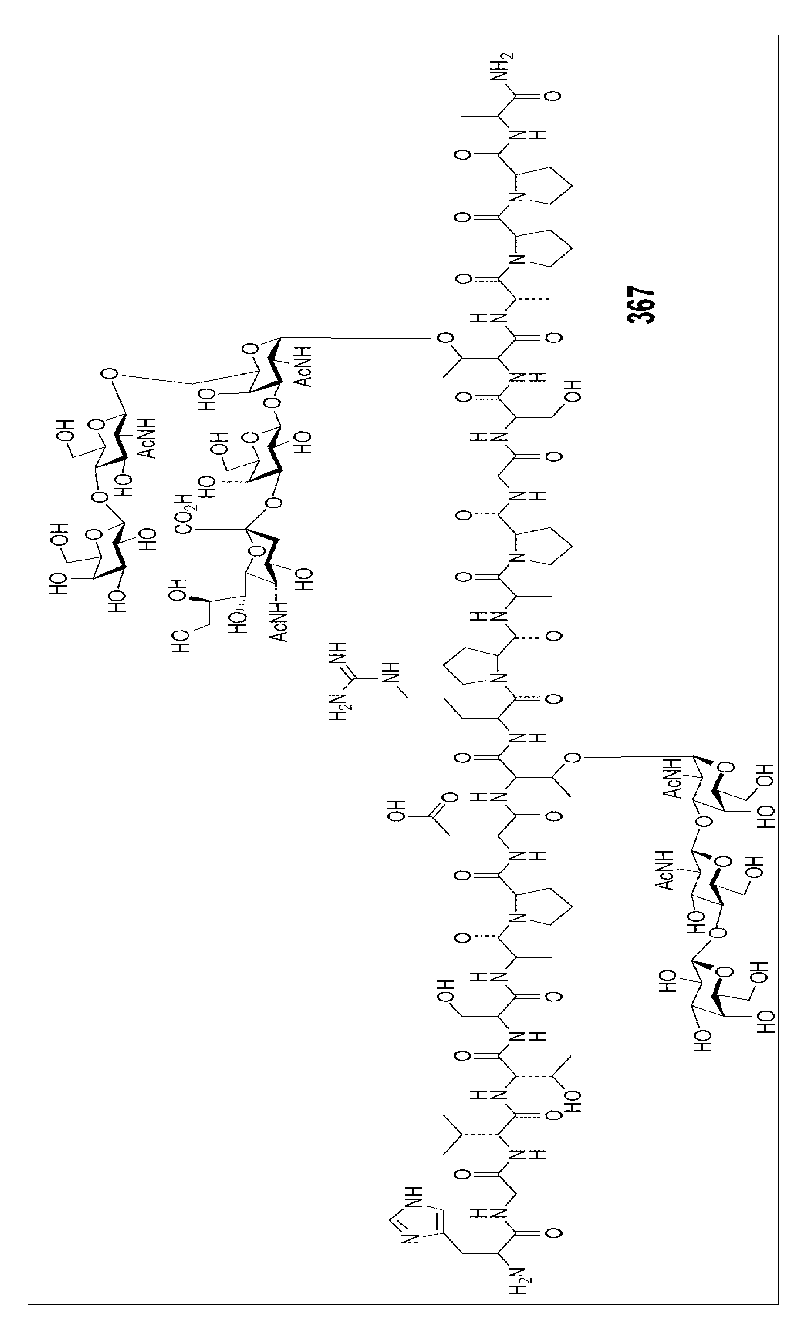

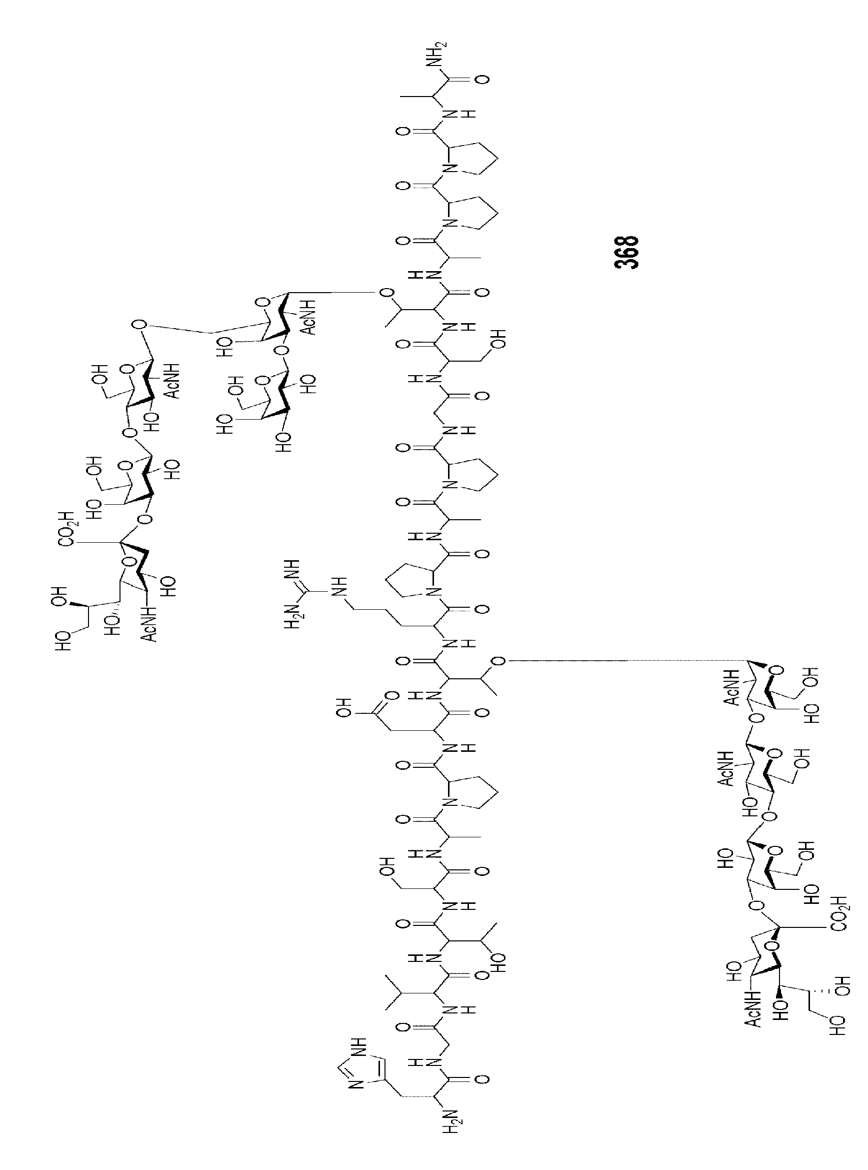

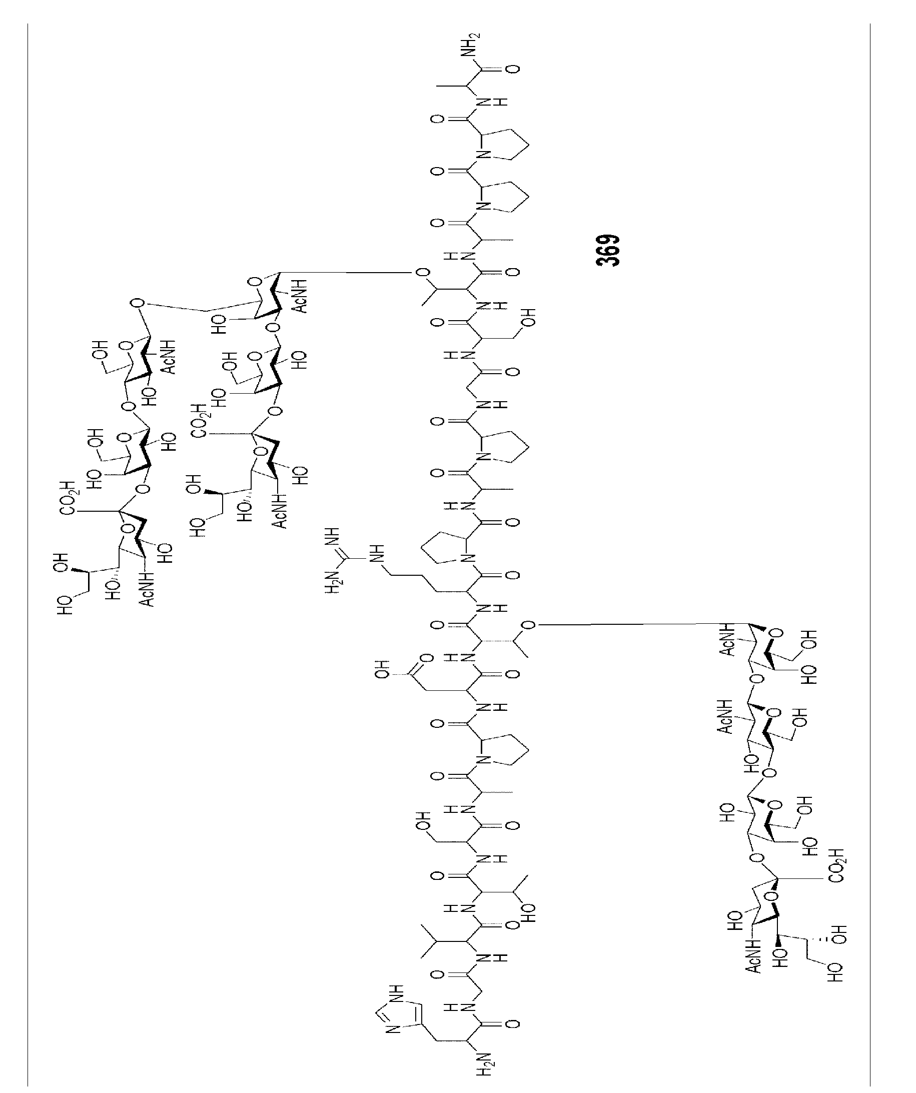

The following reaction solutions A) to E) were allowed to react for 24 hours at 25°C:
A) 100µl of reaction solution containing 50mMHEPES buffer solution (pH 7.0), 0.1U/ml human-derived β1,4-galactosyltransferase (available from TOYOBO CO., LTD.), 10mM manganese chloride, 0.1% BSA, 2mM uridine-5'-disodium diphosphogalactose (UDP-Gal) and glycopeptide derivative (230) (4mM at a theoretical content from solid-phase synthesis);
B) 100µl of reaction solution containing 50mM HEPES buffer solution (pH 7.0), 0.0175U/ml recombinant rat α2,3-(O)-sialyltransferase (available from Calbiochem), 10mM manganese chloride, 0.1% BSA, 2mM cytidine-5'-sodium monophosphosialate (CMP-NANA) and glycopeptide derivative (230) (4mM at a theoretical content from solid-phase synthesis);
C) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (230) (4mM at a theoretical content from solid-phase synthesis);
D) 100µl of reaction solution containing 50mM HEPES buffer solution (pH 7.0), 0.1U/ml human-derived β1,4-galactosyltransferase (available from TOYOBO CO., LTD.), 0.0185U/ml recombinant rat α2,3-(N)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (230) (4mM at a theoretical content from solid-phase synthesis);
E) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 0.0185U/ml recombinant rat α2,3-(N)-sialyltransferase (available from Calbiochem), 10mM manganese chloride, 0.1% BSA, 4mM uridine-5'-disodium diphosphogalactose (UDP-Gal), 2mM cytidine-5'-sodium monophosphosialate (CMP-NANA) and glycopeptide derivative (230) (4mM at a theoretical content from solid-phase synthesis).

After completion of the reaction, the respective reaction solutions were subjected to centrifugal concentration with an ultrafiltration filter. Thereafter, 25mM ammonium acetate buffer solution (pH 6.5) was added thereto, and the solution was again subjected to concentration with a centrifugal separator, thereby washing the polymer. This manipulation was repeated three times, thereby respectively obtaining aqueous solutions of compounds (370) to (374). Thereafter, to the solution retained at the filter containing compounds (370) to (374) and a solution which was obtained by diluting an aqueous solution of (230) with 25mM ammonium acetate buffer solution (pH 6.5), 1µl of 0.174mg/ml solution of BLase (available from Shionogi & Co., Ltd.) was added, and the solution was allowed to react for two hours at room temperature. Thereafter, the solution was subjected to centrifugal filtration with an ultrafiltration filter, thereby separating the glycopeptide of interest from the polymer. The obtained aqueous solution (filtrate) was lyophilized, thereby obtaining compounds (375) to (380).
MALDI-TOF/MS:[M(average)+H]⁺ of compound (375)=2863.6 (theoretical value:[M(average)+H]⁺=2861.9); MALDI-TOF/MS:[M(average)+H]⁺ of compound (376)=3186.5 (theoretical value:[M(average)+H]⁺=3186.2); MALDI-TOF/MS:[M(average)+H]⁺ of compound (377)=3155.3 (theoretical value:[M(average)+H]⁺=3153.2); MALDI-TOF/MS:[M(average)+H]⁺ of compound (378)=3478.3 (theoretical value:[M(average)+H]⁺=3477.5); MALDI-TOF/MS:[M(average)+H]⁺ of compound (379)=3769.5 (theoretical value:[M(average)+H]⁺=3768.7); and MALDI-TOF/MS:[M(average)+H]⁺ of compound (380)=4062.8 (theoretical value:[M(average)+H]⁺=4060.0).

(3.64 Synthesis of compounds (370) to (380))

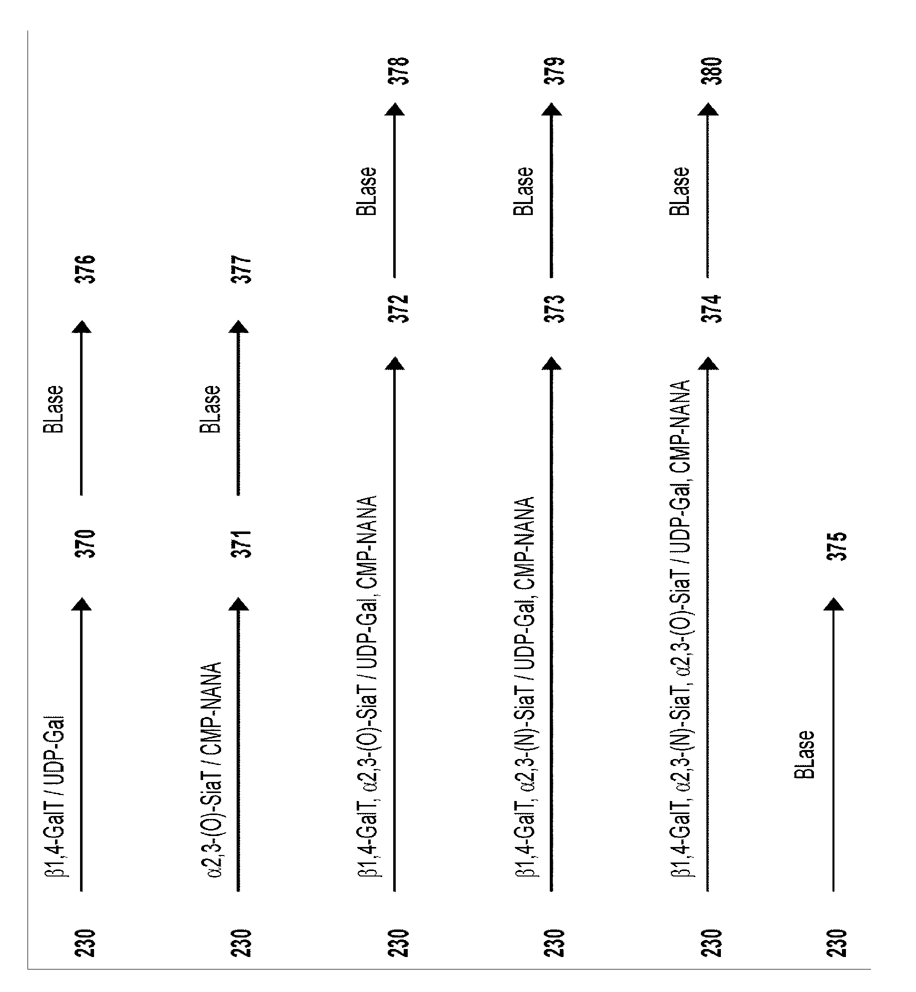

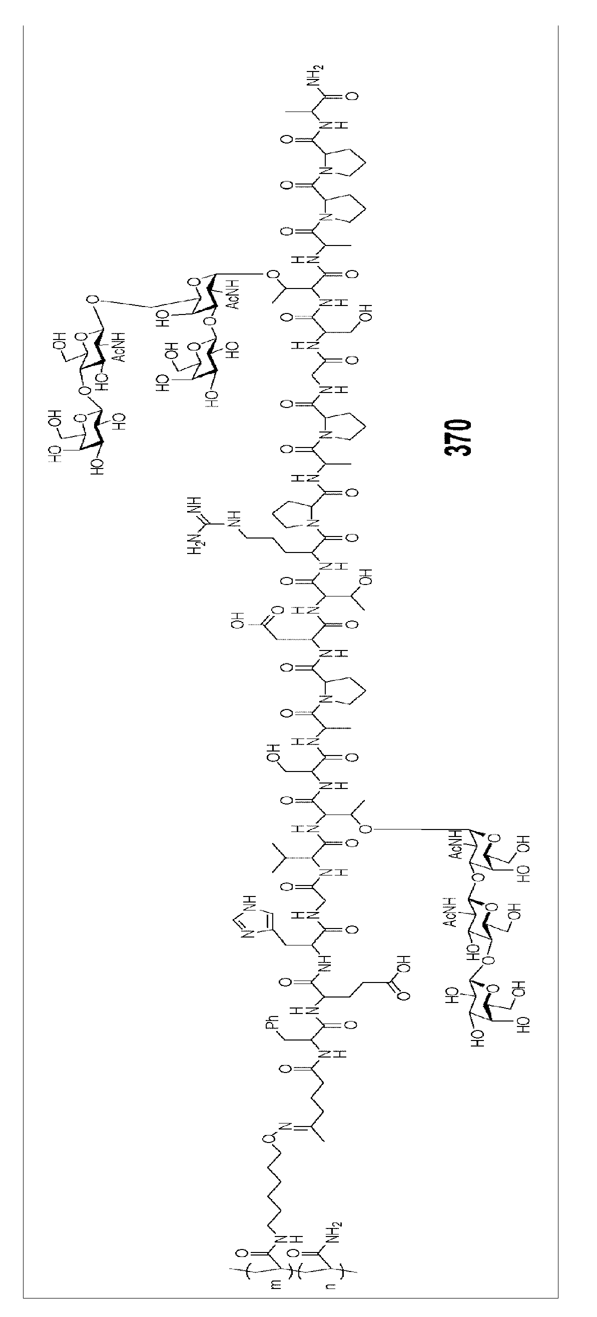

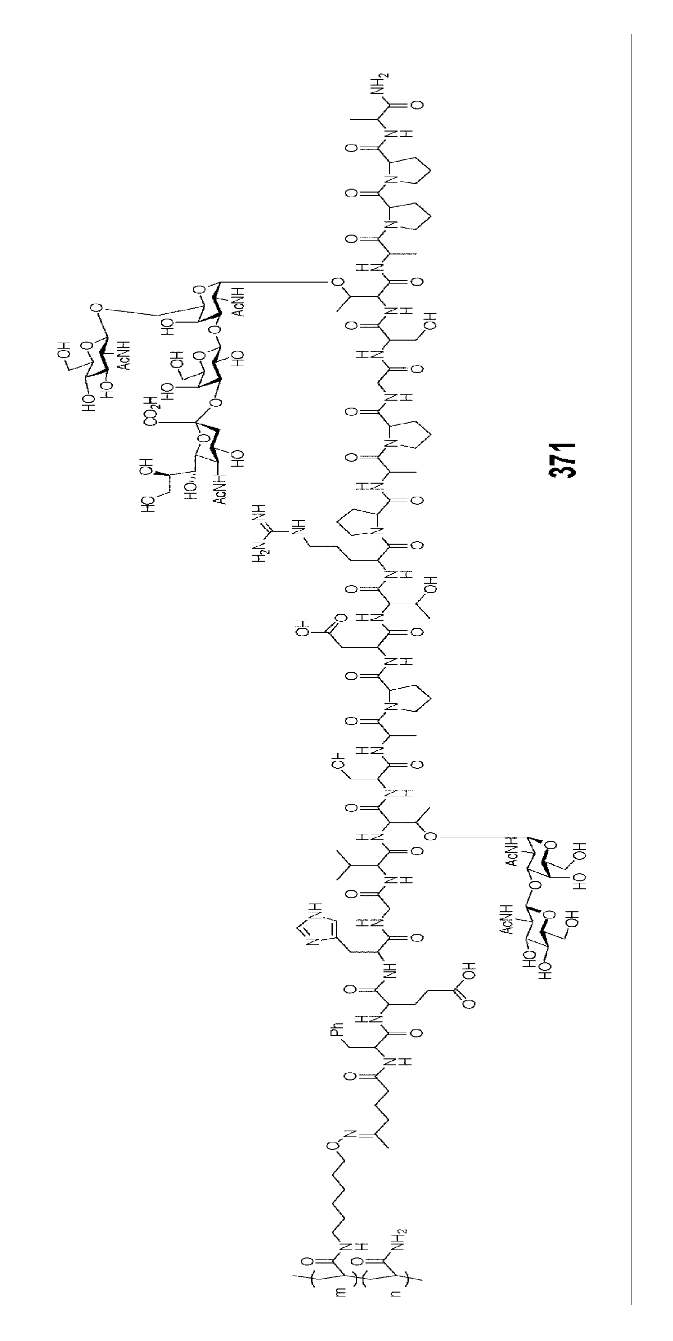

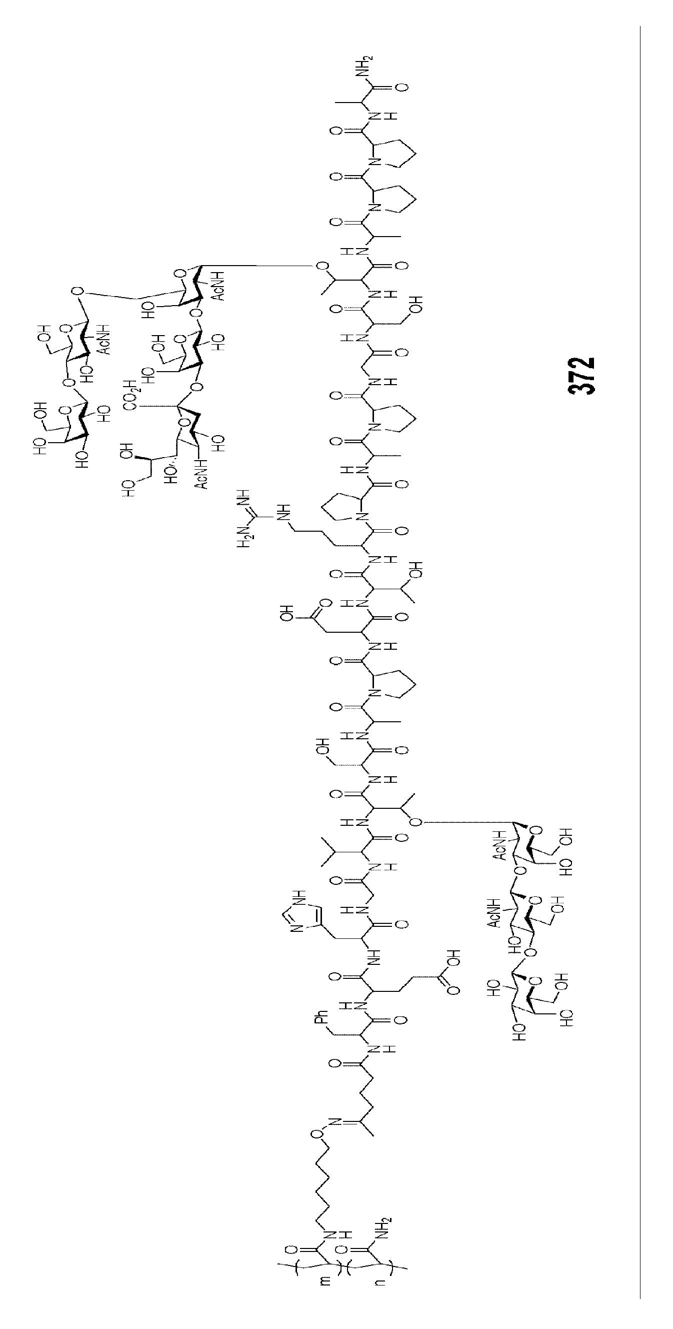

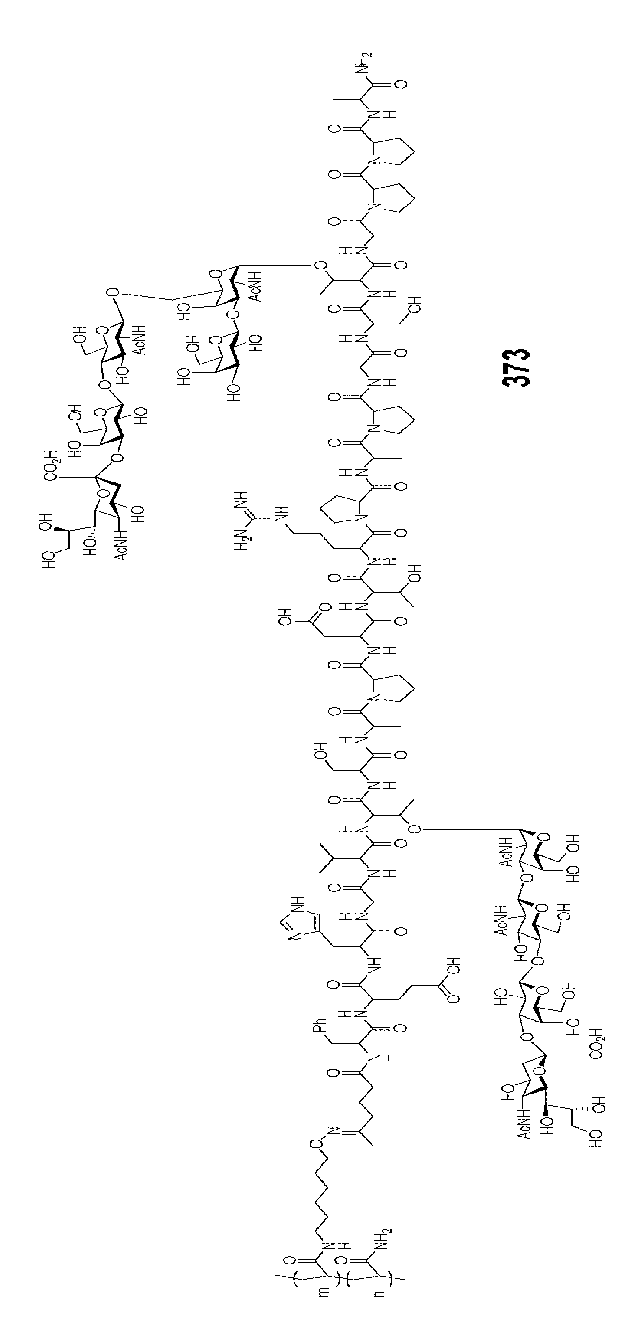

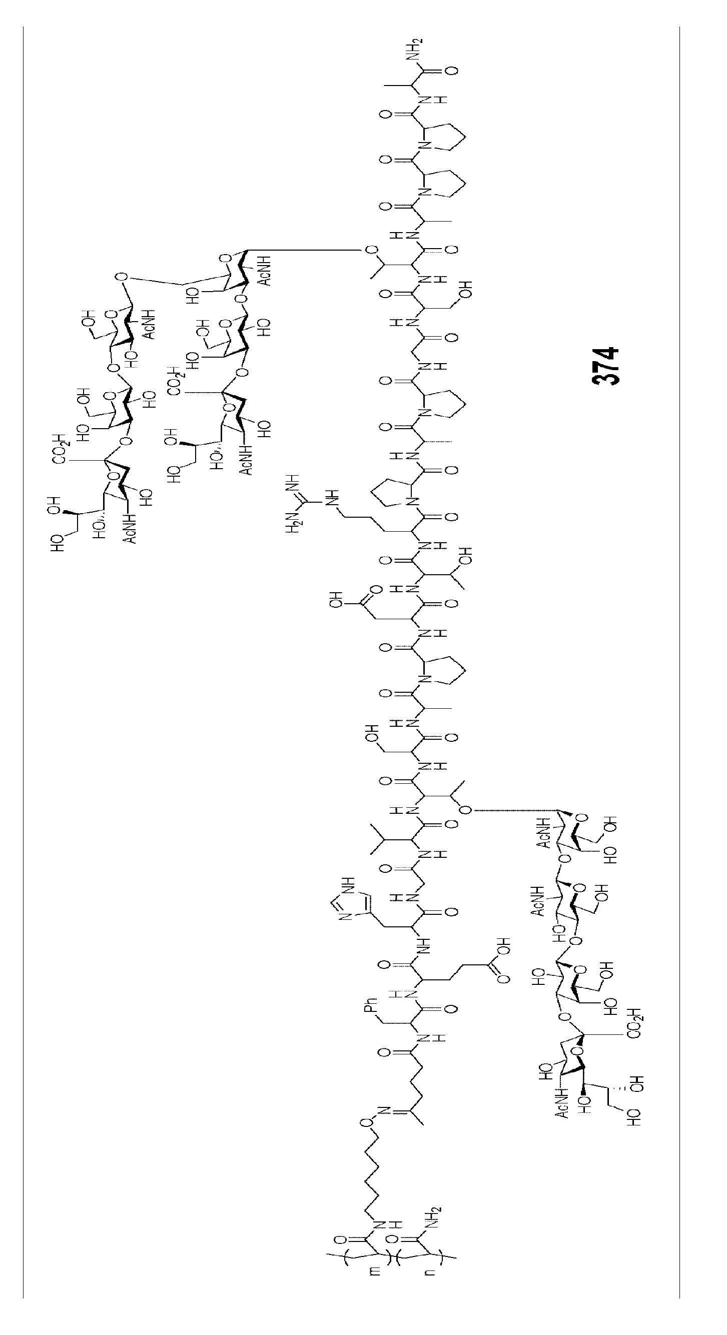

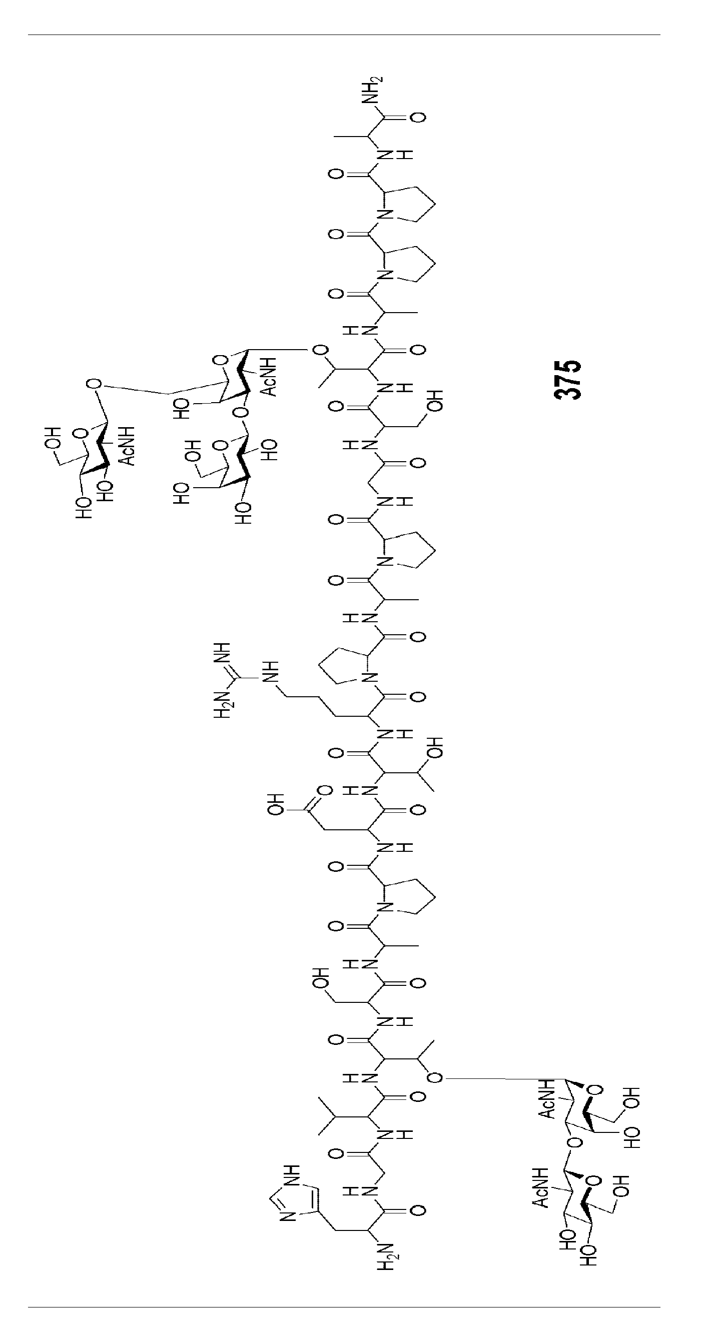

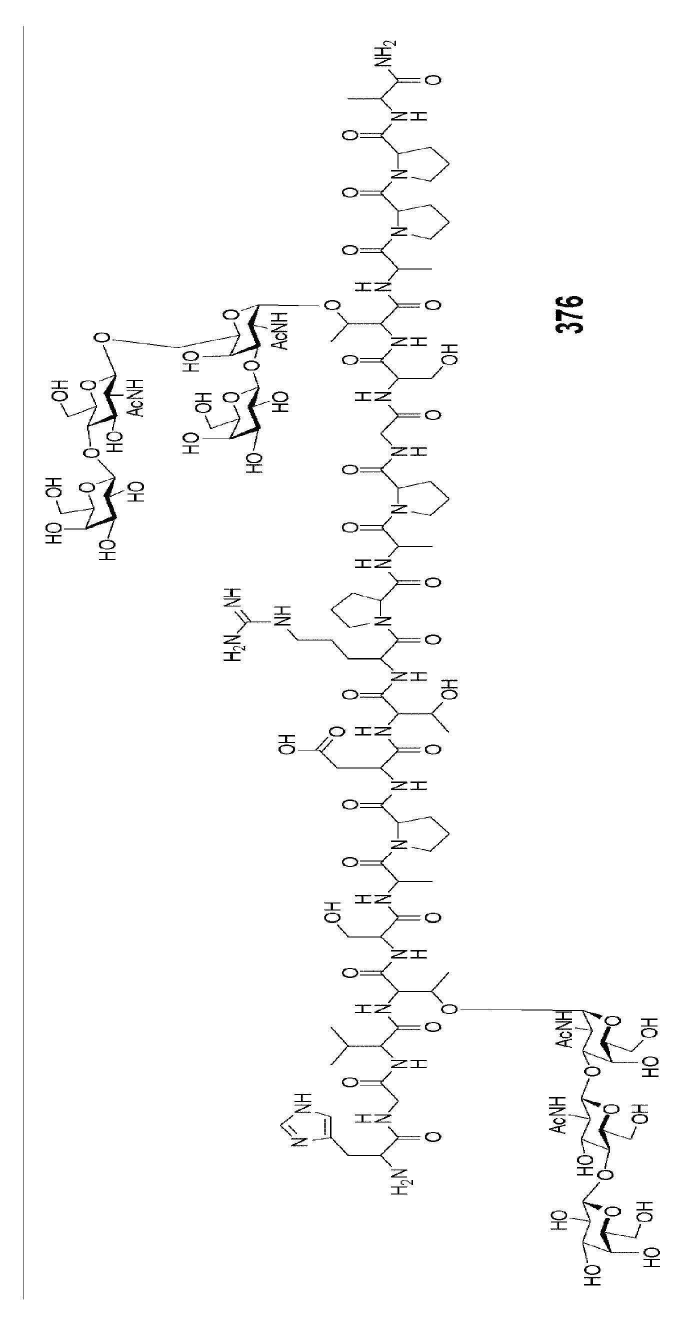

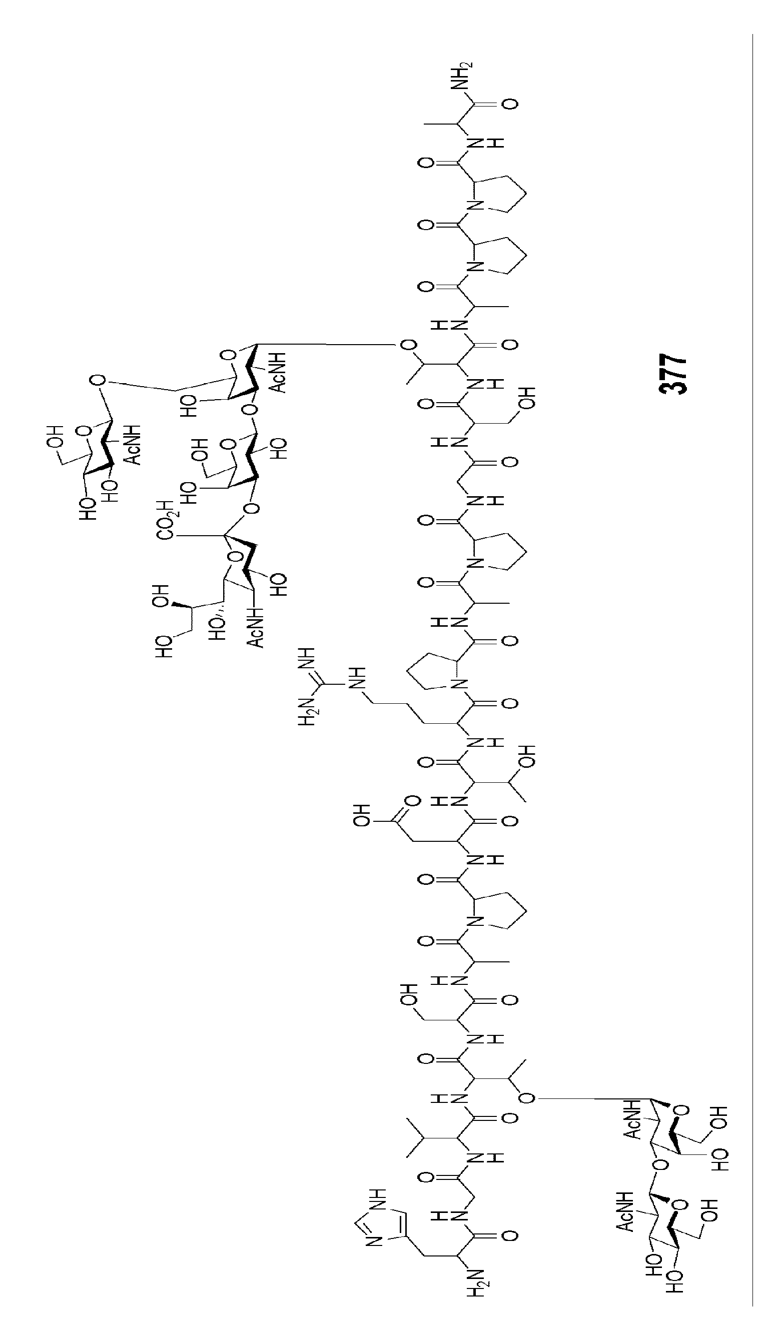

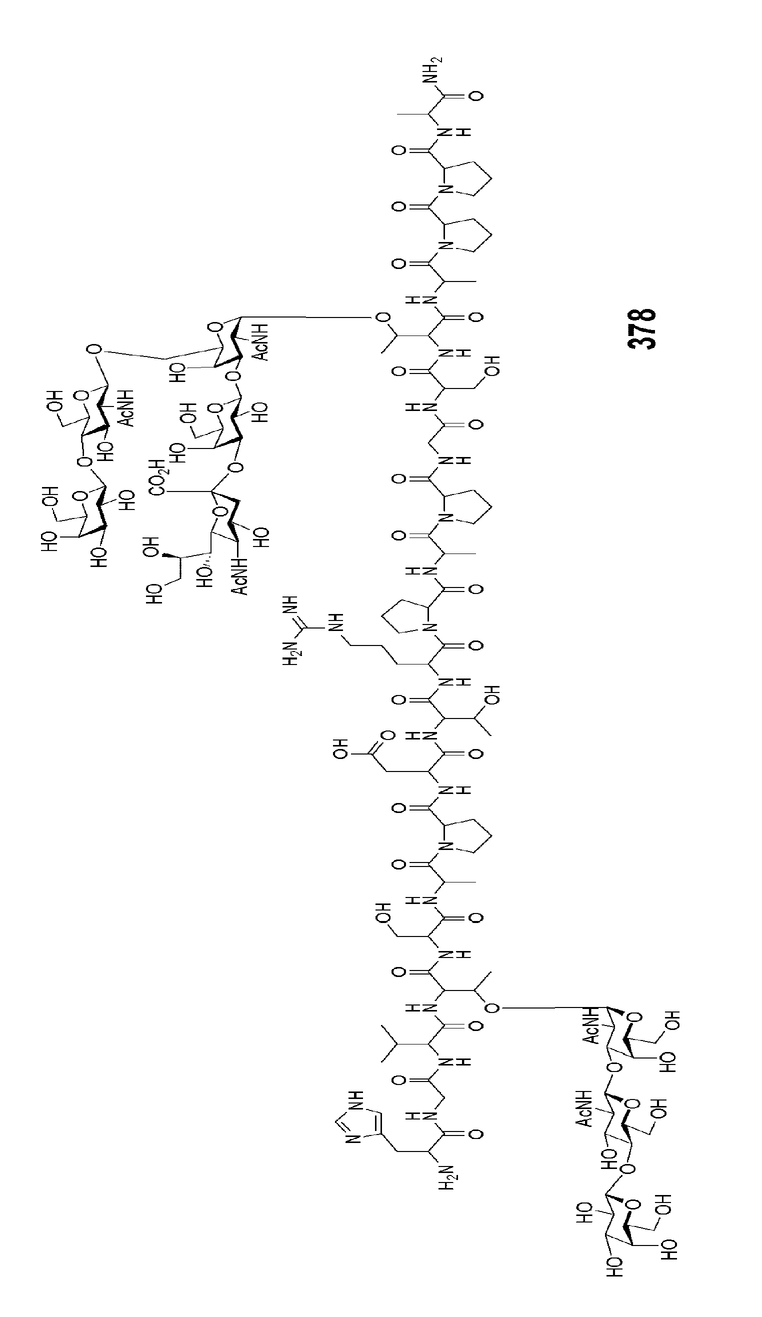

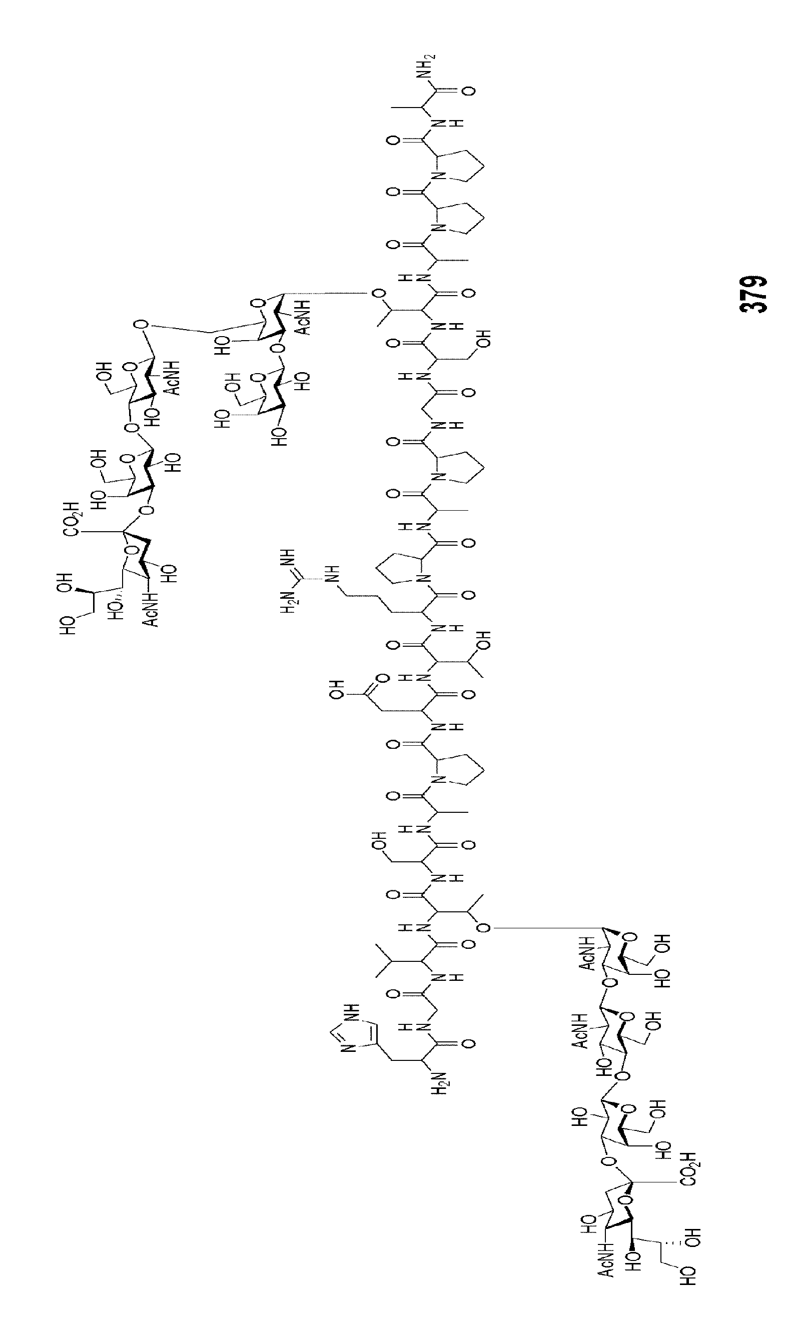

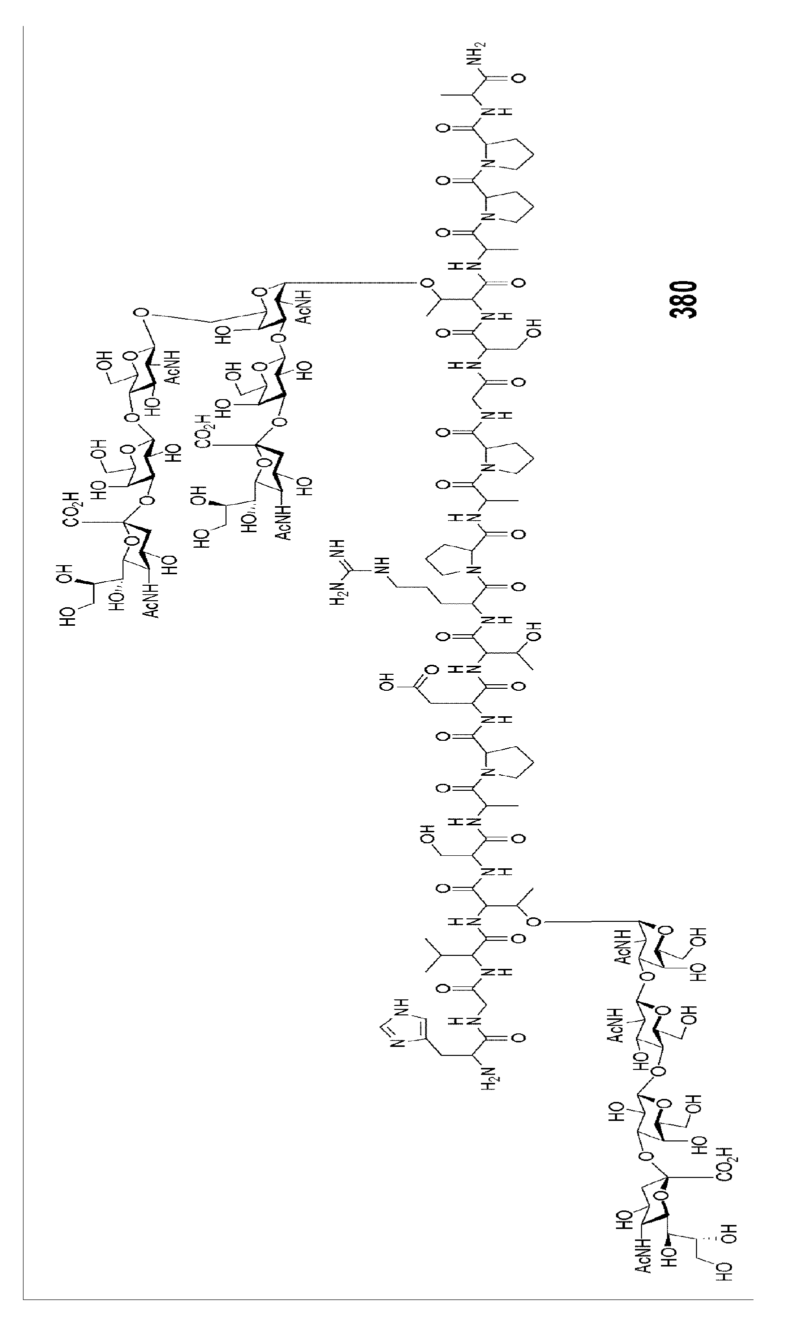

The following reaction solutions A) to E) were allowed to react for 24 hours at 25°C:
A) 100µl of reaction solution containing 50mMHEPES buffer solution (pH 7.0), 0.1U/ml human-derived β1,4-galactosyltransferase (available from TOYOBO CO., LTD.), 10mM manganese chloride, 0.1% BSA, 2mM uridine-5'-disodium diphosphogalactose (UDP-Gal) and glycopeptide derivative (230) (4mM at a theoretical content from solid-phase synthesis);
B) 100µl of reaction solution containing 50mM HEPES buffer solution (pH 7.0), 0.0175U/ml recombinant rat α2,3-(O)-sialyltransferase (available from Calbiochem), 10mM manganese chloride, 0.1% BSA, 2mM cytidine-5'-sodium monophosphosialate (CMP-NANA) and glycopeptide derivative (230) (4mM at a theoretical content from solid-phase synthesis);
C) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (230) (4mM at a theoretical content from solid-phase synthesis);
D) 100µl of reaction solution containing 50mM HEPES buffer solution (pH 7.0), 0.1U/ml human-derived β1,4-galactosyltransferase (available from TOYOBO CO., LTD.), 0.0185U/ml recombinant rat α2,3-(N)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (230) (4mM at a theoretical content from solid-phase synthesis);
E) 100µl of reaction solution containing 50 mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 0.0185U/ml recombinant rat α2,3-(N)-sialyltransferase (available from Calbiochem), 10mM manganese chloride, 0.1% BSA, 4mM uridine-5'-disodium diphosphogalactose (UDP-Gal), 2mM cytidine-5'-sodium monophosphosialate (CMP-NANA) and glycopeptide derivative (230) (4mM at a theoretical content from solid-phase synthesis).

After completion of the reaction, the respective reaction solutions were subjected to centrifugal concentration with an ultrafiltration filter. Thereafter, 25mM ammonium acetate buffer solution (pH 6.5) was added thereto, and the solution was again subjected to concentration with a centrifugal separator, thereby washing the polymer. This manipulation was repeated three times, thereby respectively obtaining aqueous solutions of compounds (370) to (374). Thereafter, to the solution retained at the filter containing compounds (370) to (374) and a solution which was obtained by diluting an aqueous solution of (230) with 25mM ammonium acetate buffer solution (pH 6.5), 1µl of 0.174mg/ml solution of BLase (available from Shionogi & Co., Ltd.) was added, and the solution was allowed to react for two hours at room temperature. Thereafter, the solution was subjected to centrifugal filtration with an ultrafiltration filter, thereby separating the glycopeptide of interest from the polymer. The obtained aqueous solution (filtrate) was lyophilized, thereby obtaining compounds (375) to (380).
MALDI-TOF/MS:[M(average)+H]⁺ of compound (375)=2863.6 (theoretical value:[M(average)+H]⁺=2861.9); MALDI-TOF/MS:[M(average)+H]⁺ of compound (376)=3186.5 (theoretical value:[M(average)+H]⁺=3186.2); MALDI-TOF/MS:[M(average)+H]⁺ of compound (377)=3155.3 (theoretical value:[M(average)+H]⁺=3153.2); MALDI-TOF/MS:[M(average)+H]⁺ of compound (378)=3478.3 (theoretical value:[M(average)+H]⁺=3477.5); MALDI-TOF/MS:[M(average)+H]⁺ of compound (379)=3769.5 (theoretical value:[M(average)+H]⁺=3768.7); and MALDI-TOF/MS:[M(average)+H]⁺ of compound (380)=4062.8 (theoretical value:[M(average)+H]⁺=4060.0).

(3.65 Synthesis of compounds (381) to (391))

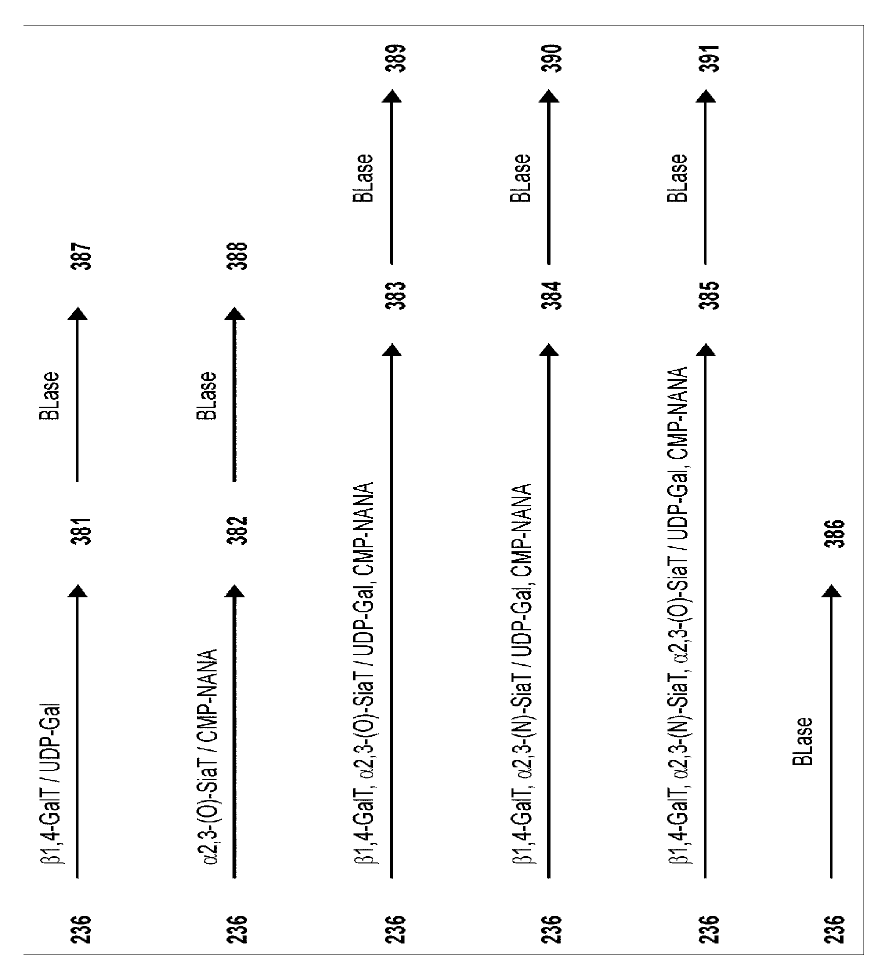

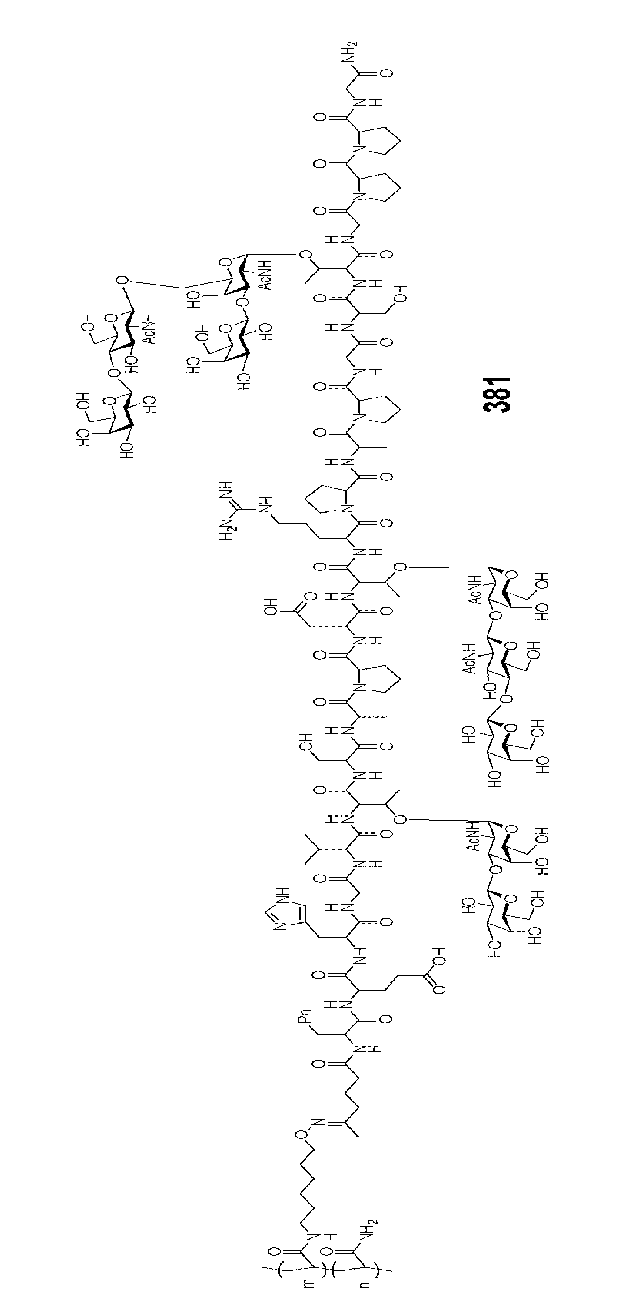

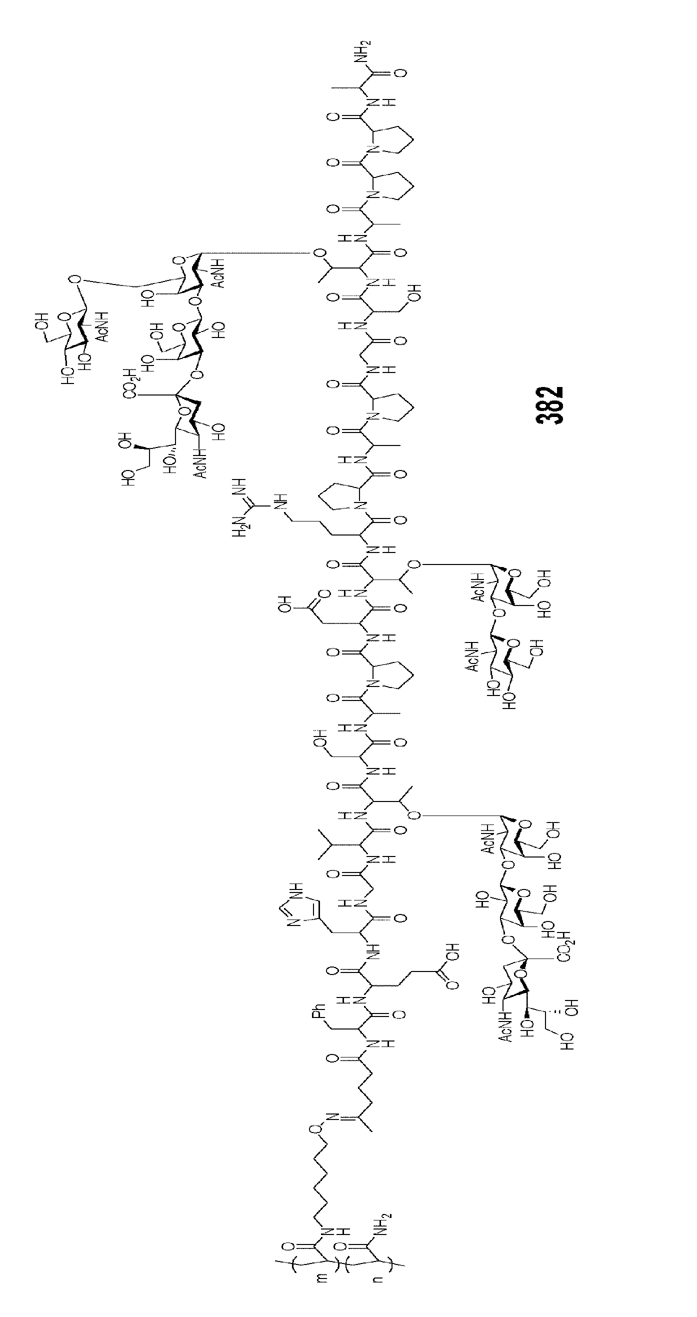

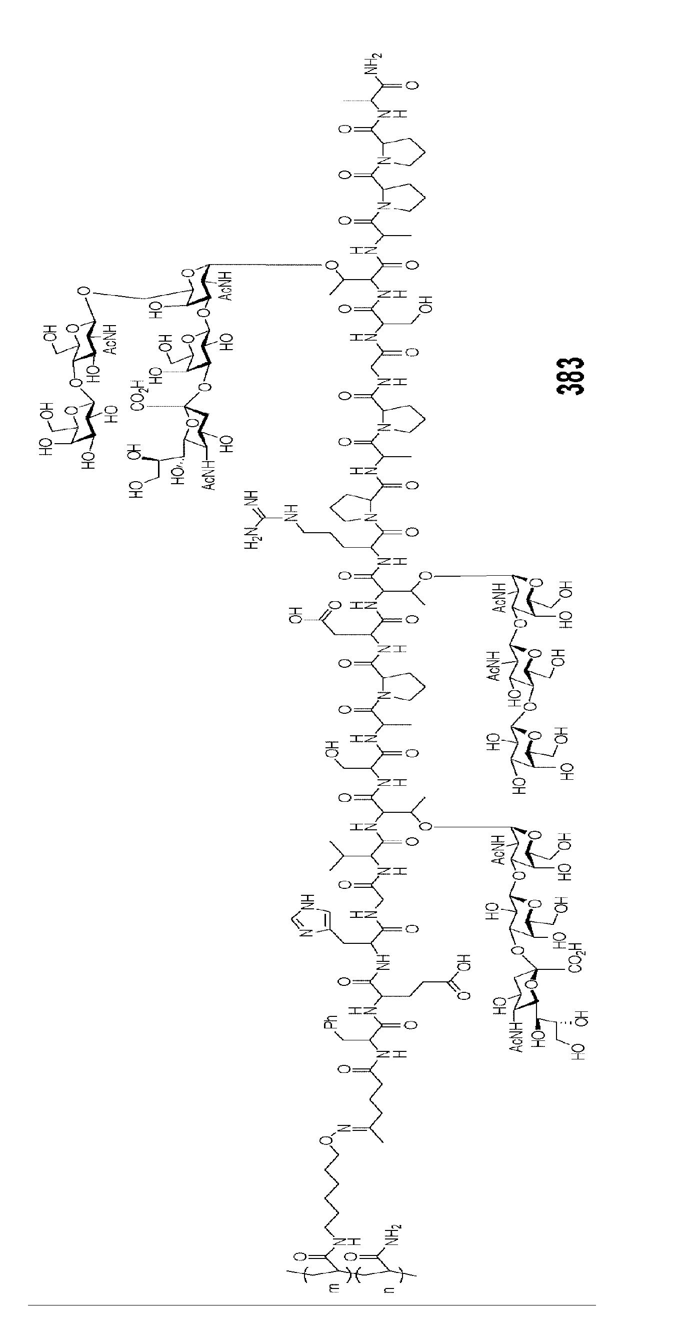

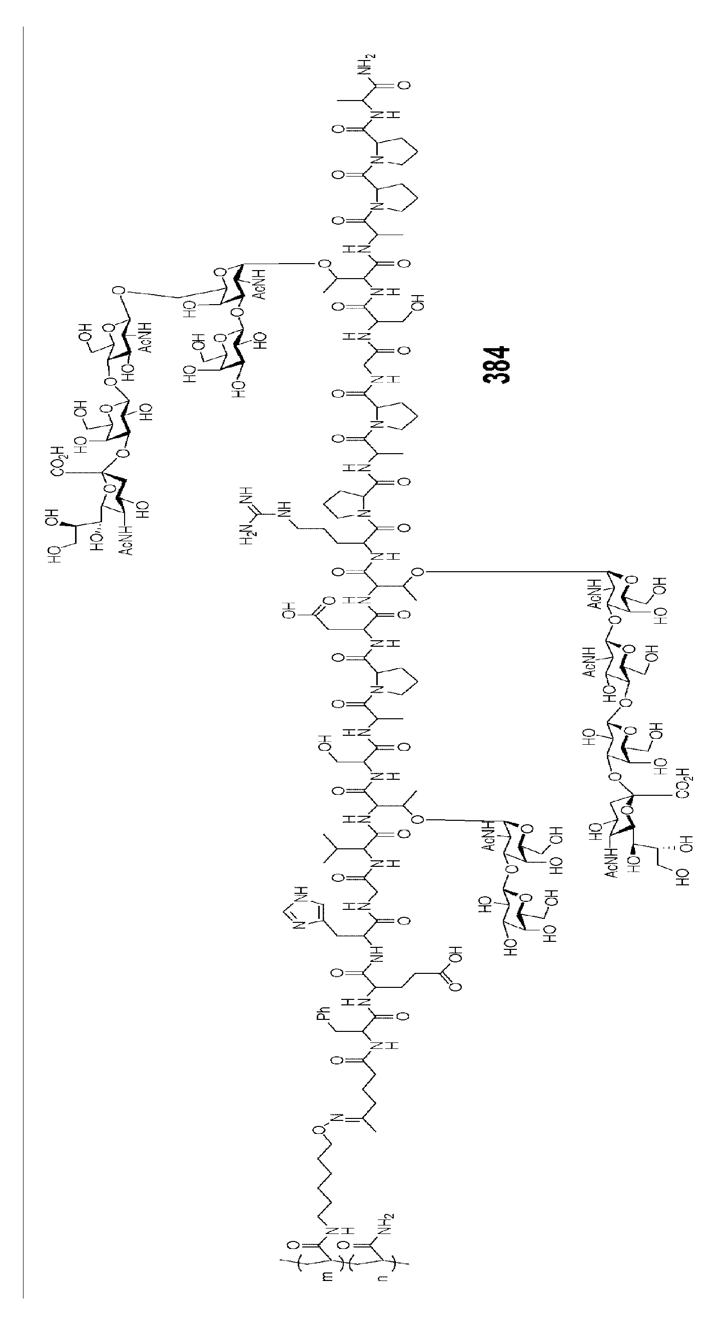

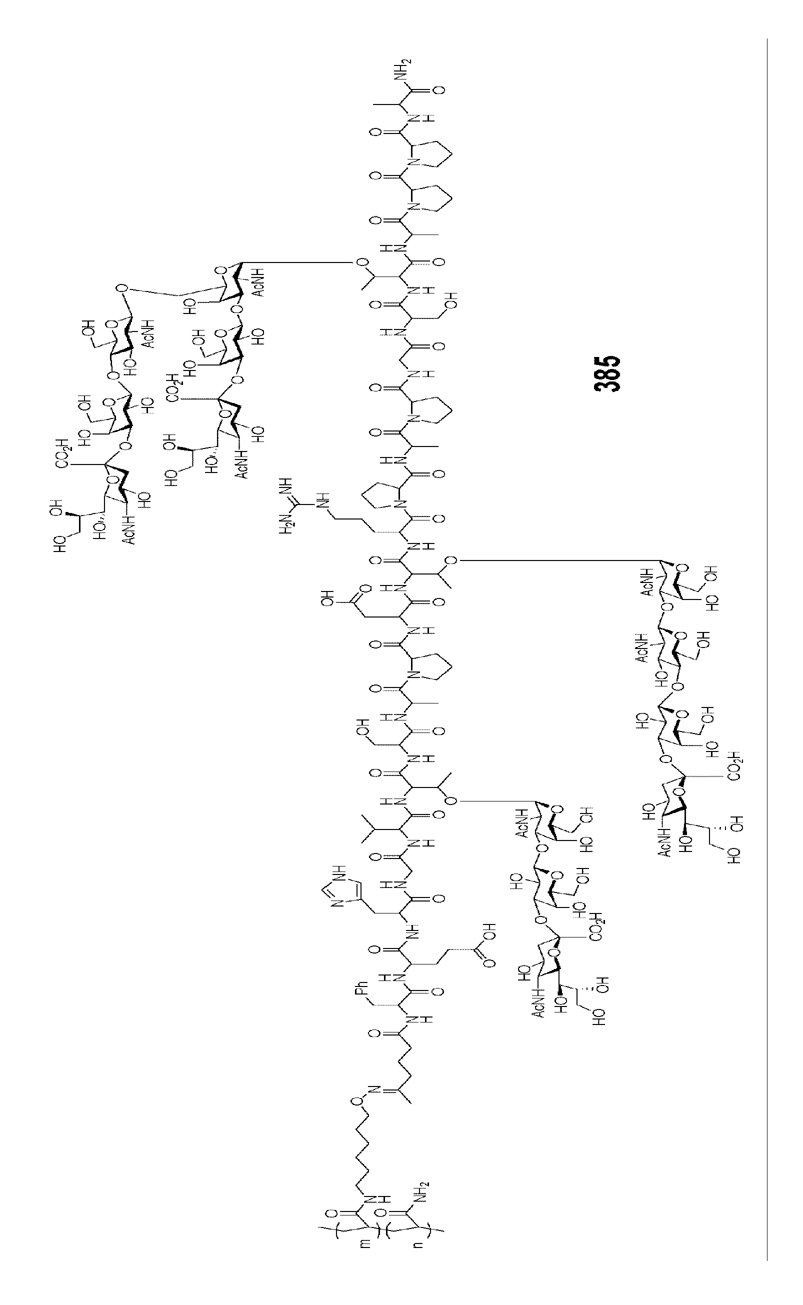

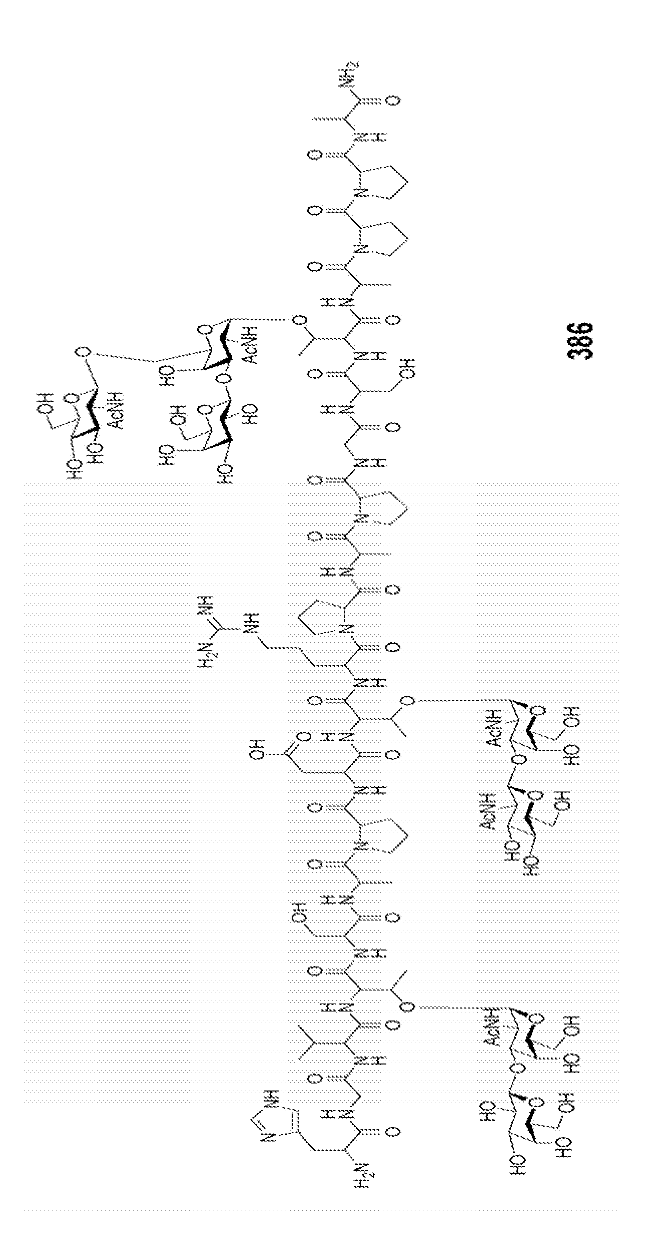

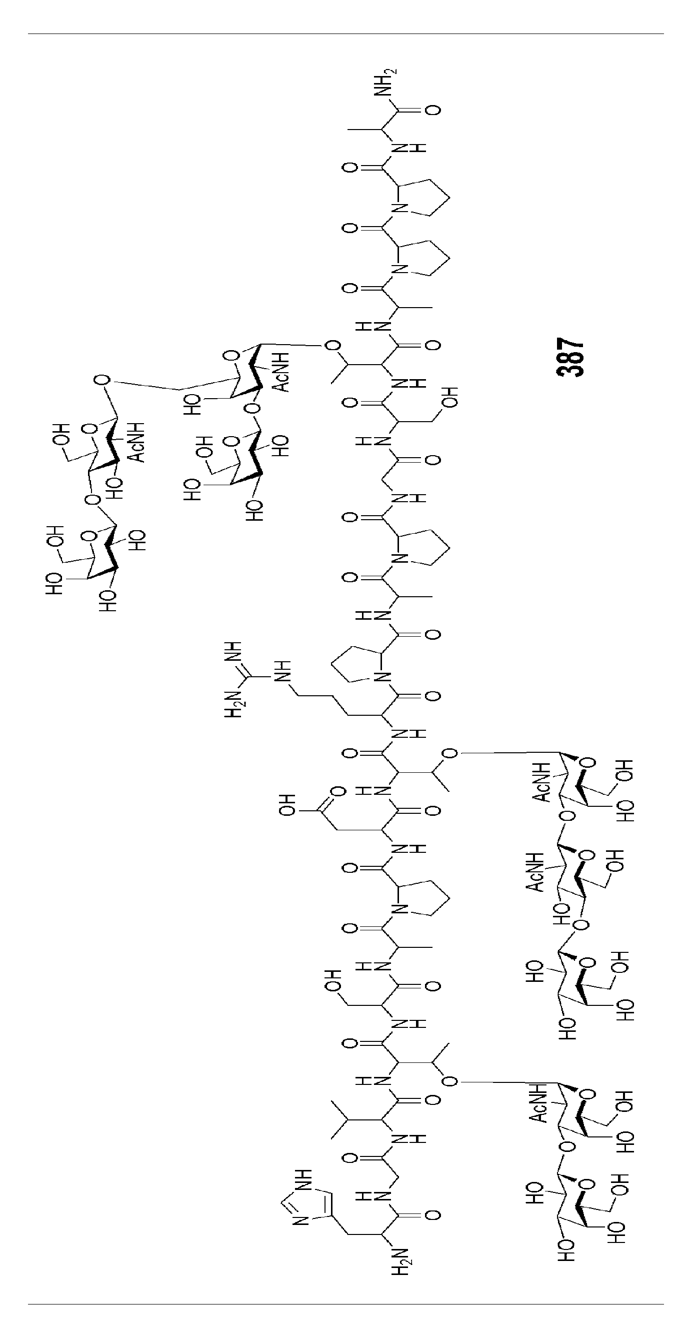

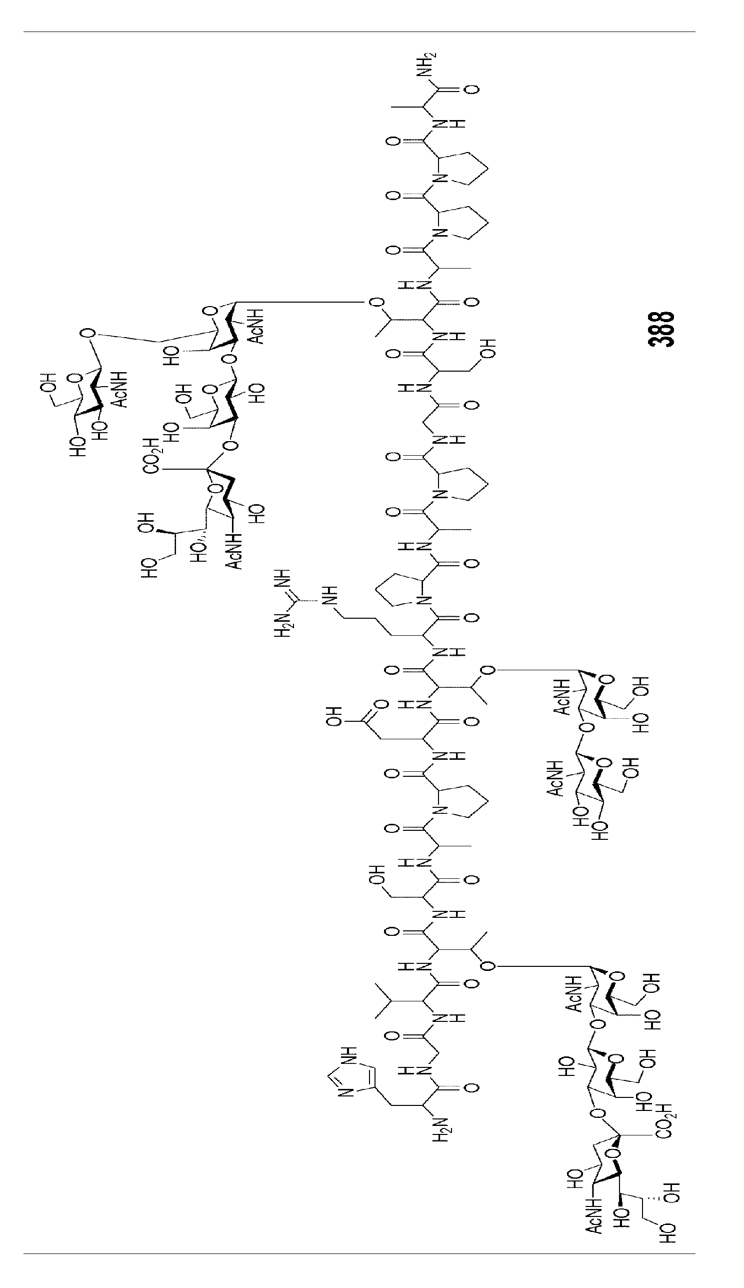

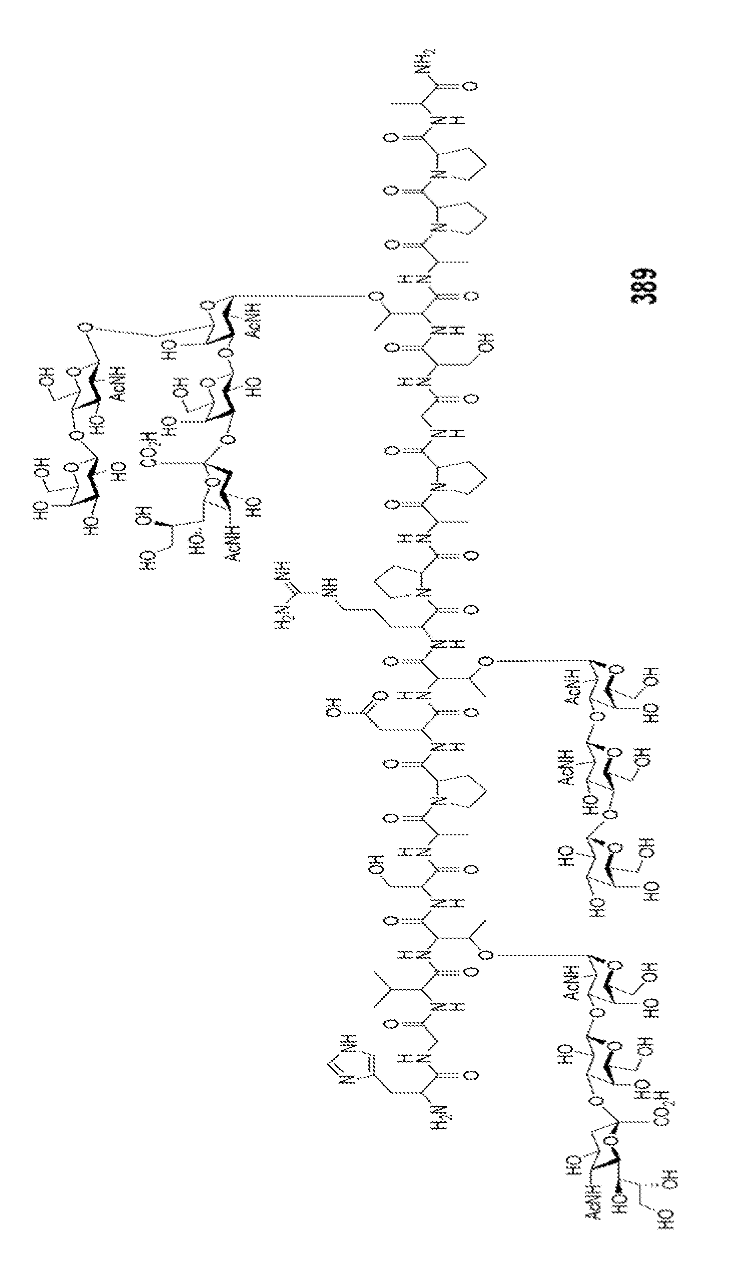

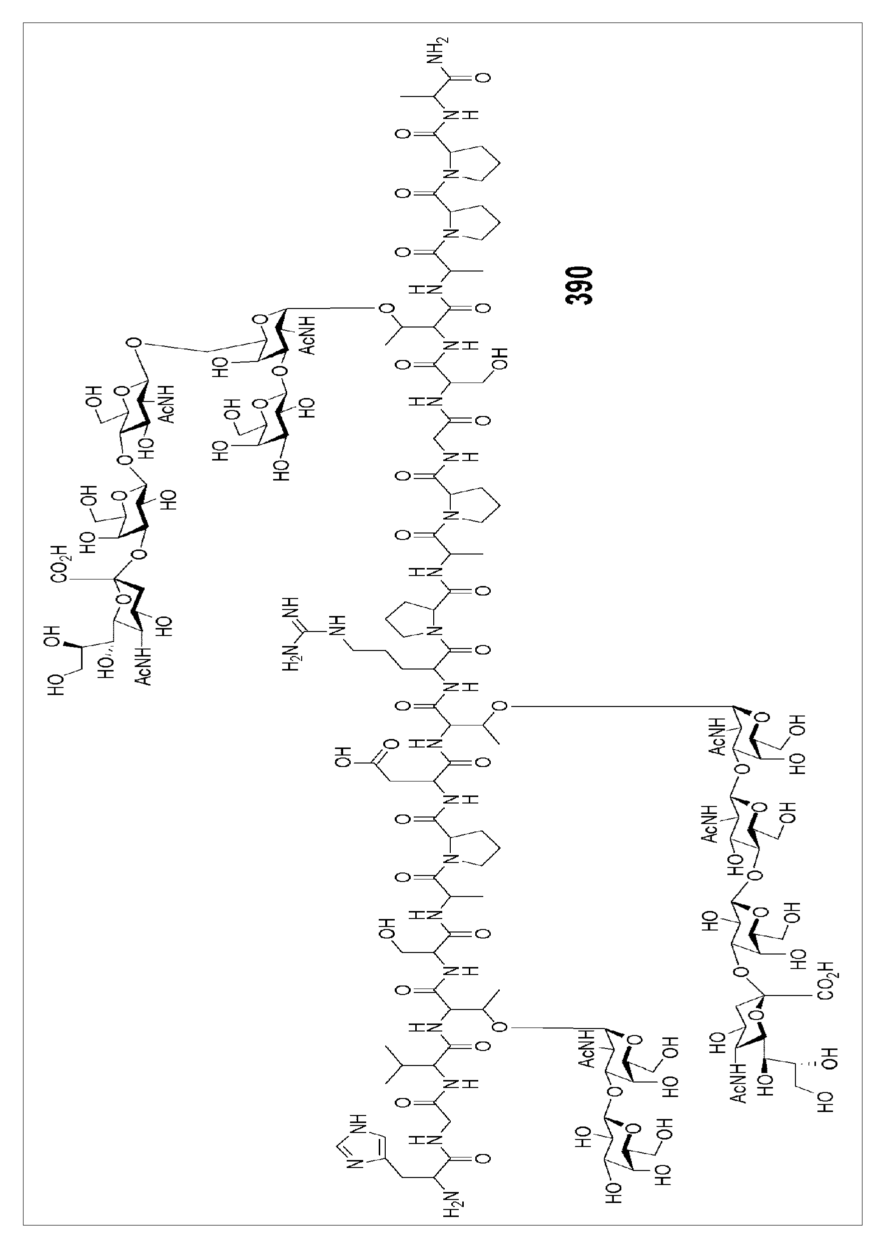

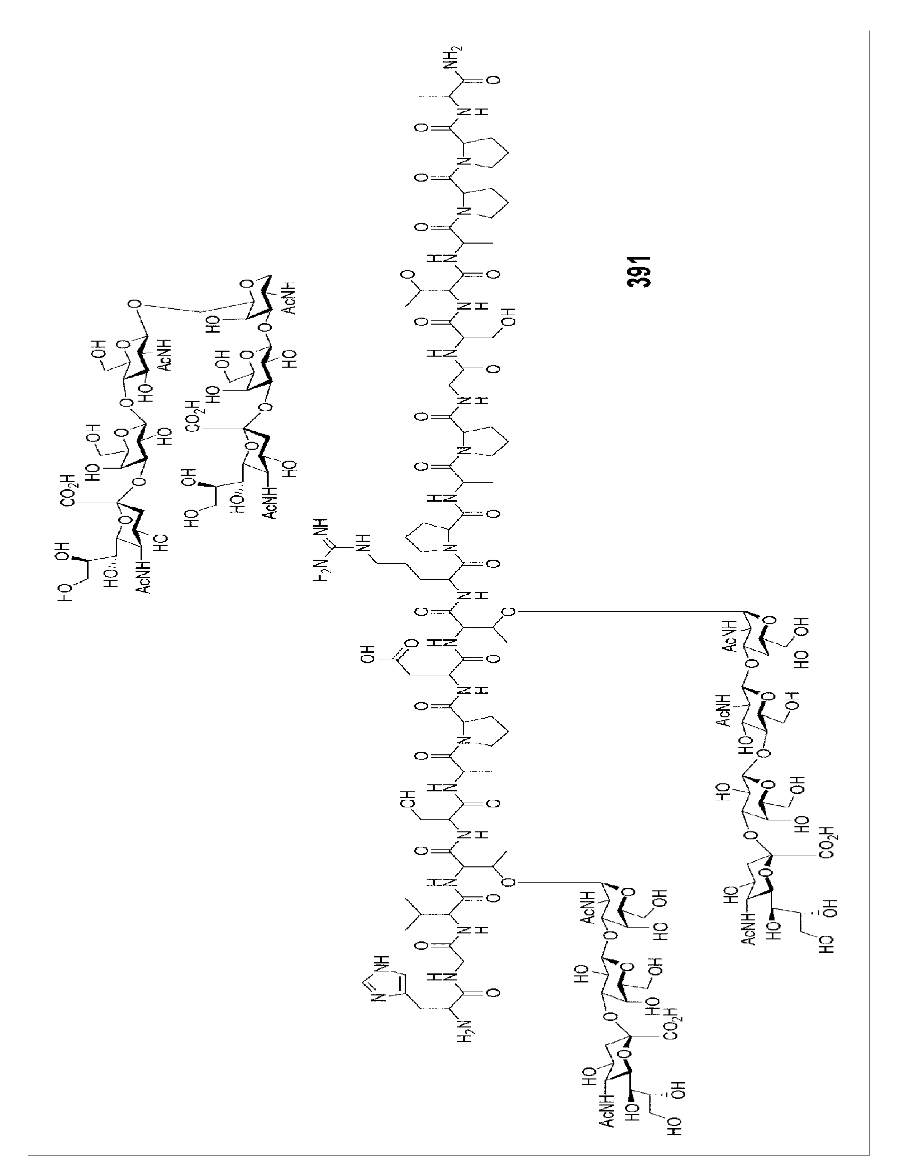

The following reaction solutions A) to E) were allowed to react for 24 hours at 25°C:
A) 100µl of reaction solution containing 50mM HEPES buffer solution (pH 7.0), 0.1U/ml human-derived β1,4-galactosyltransferase (available from TOYOBO CO., LTD.), 10mM manganese chloride, 0.1% BSA, 2mM uridine-5'-disodium diphosphogalactose (UDP-Gal) and glycopeptide derivative (236) (4mM at a theoretical content from solid-phase synthesis);
B) 100 µl of reaction solution containing 50 mM HEPES buffer solution (pH 7.0), 0.0175U/ml recombinant rat α2,3-(O)-sialyltransferase (available from Calbiochem), 10mM manganese chloride, 0.1% BSA, 2mM cytidine-5'-sodium monophosphosialate (CMP-NANA) and glycopeptide derivative (236) (4mM at a theoretical content from solid-phase synthesis);
C) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (236) (4mM at a theoretical content from solid-phase synthesis);
D) 100µl of reaction solution containing 50mM HEPES buffer solution (pH 7.0), 0.1U/ml human-derived β1,4-galactosyltransferase (available from TOYOBO CO., LTD.), 0.0185U/ml recombinant rat α2,3-(N)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (236) (4mM at a theoretical content from solid-phase synthesis);
E) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 0.0185U/ml recombinant rat α2,3-(N)-sialyltransferase (available from Calbiochem), 10mM manganese chloride, 0.1% BSA, 4mM uridine-5'-disodium diphosphogalactose (UDP-Gal), 2mM cytidine-5'-sodium monophosphosialate (CMP-NANA) and glycopeptide derivative (236) (4mM at a theoretical content from solid-phase synthesis).

After completion of the reaction, the respective reaction solutions were subjected to centrifugal concentration with an ultrafiltration filter. Thereafter, 25mM ammonium acetate buffer solution (pH 6.5) was added thereto, and the solution was again subjected to concentration with a centrifugal separator, thereby washing the polymer. This manipulation was repeated three times, thereby respectively obtaining aqueous solutions of compounds (381) to (385). Thereafter, to the solution retained at the filter containing compounds (381) to (385) and a solution which was obtained by diluting an aqueous solution of (236) with 25mM ammonium acetate buffer solution (pH 6.5), 1µl of 0.174mg/ml solution of BLase (available from Shionogi & Co., Ltd.) was added, and the solution was allowed to react for two hours at room temperature. Thereafter, the solution was subjected to centrifugal filtration with an ultrafiltration filter, thereby separating the glycopeptide of interest from the polymer. The obtained aqueous solution (filtrate) was lyophilized, thereby obtaining compounds (386) to (391).
MALDI-TOF/MS:[M(average)+H]⁺ of compound (386)=3229.0 (theoretical value:[M(average)+H]⁺=3227.3); MALDI-TOF/MS:[M(average)+H]⁺ of compound (387)=3551.4 (theoretical value:[M(average)+H]⁺=3551.6); MALDI-TOF/MS:[M(average)+H]⁺ of compound (388)=3811.9 (theoretical value:[M(average)+H]⁺=3809.8); MALDI-TOF/MS:[M(average)+H]⁺ of compound (389)=4136.1 (theoretical value:[M(average)+H]⁺=4134.1); MALDI-TOF/MS:[M(average)+H]⁺ of compound (390)=4133.4 (theoretical value:[M(average)+H]⁺=4134.1); and MALDI-TOF/MS:[M(average)+H]⁺ of compound (391)=4713.0 (theoretical value:[M(average)+H]⁺=4716.6).

(3.66 Synthesis of compounds (392) to (402))

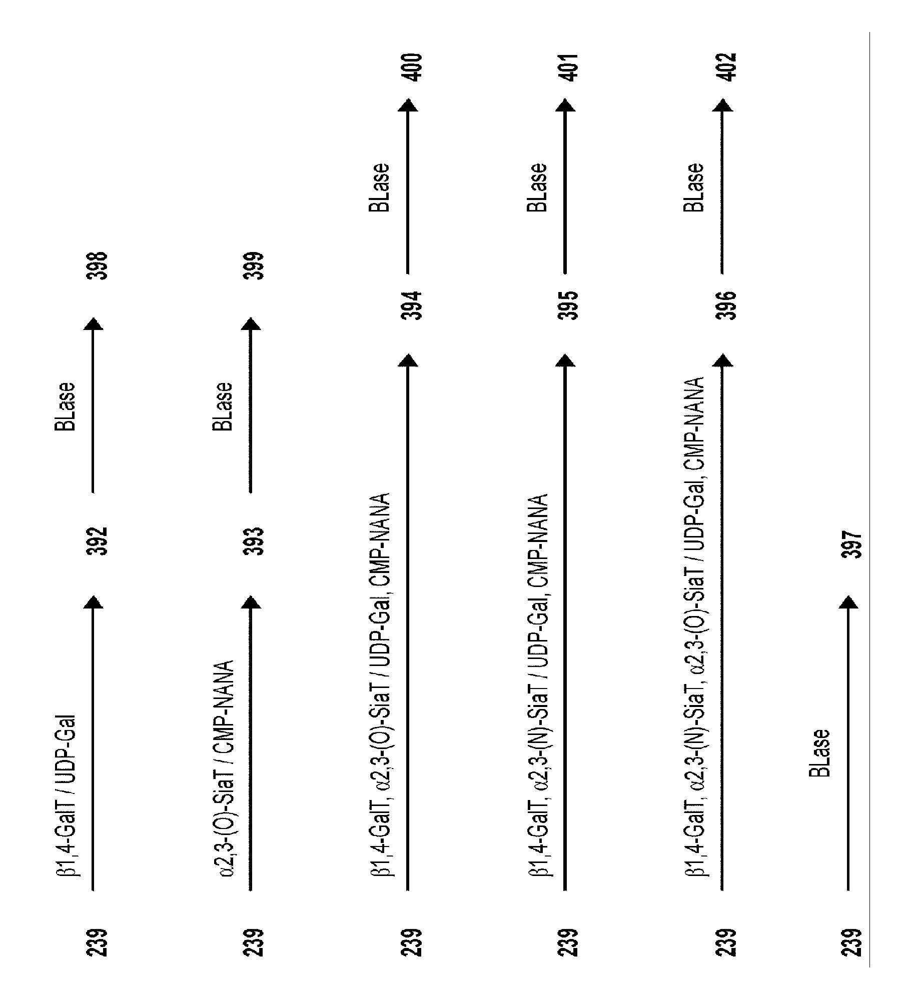

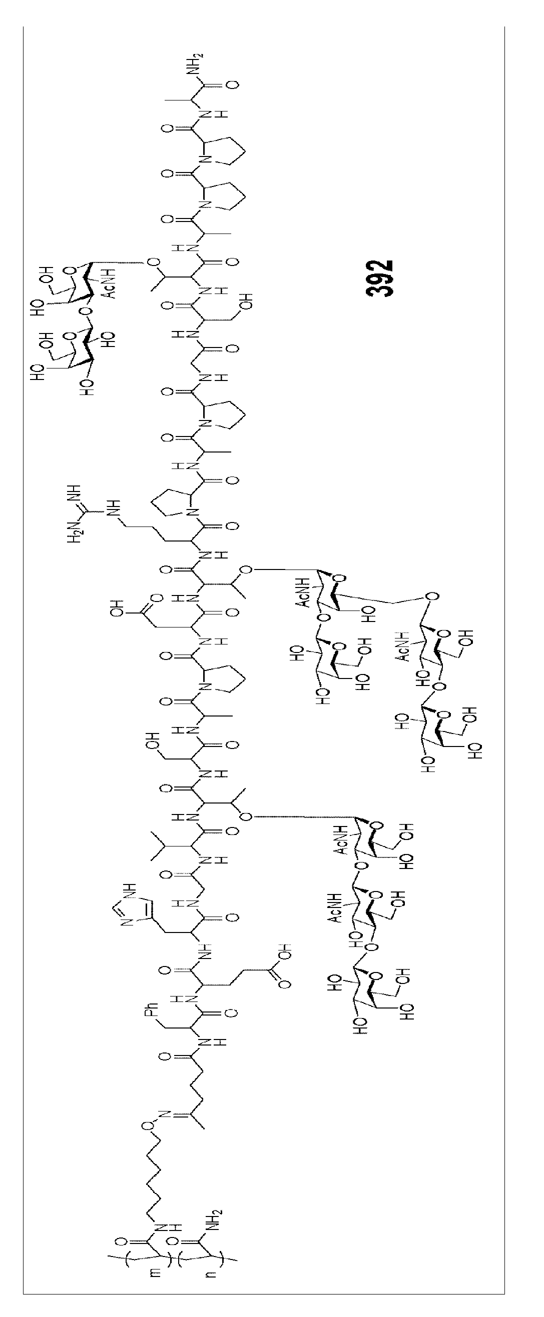

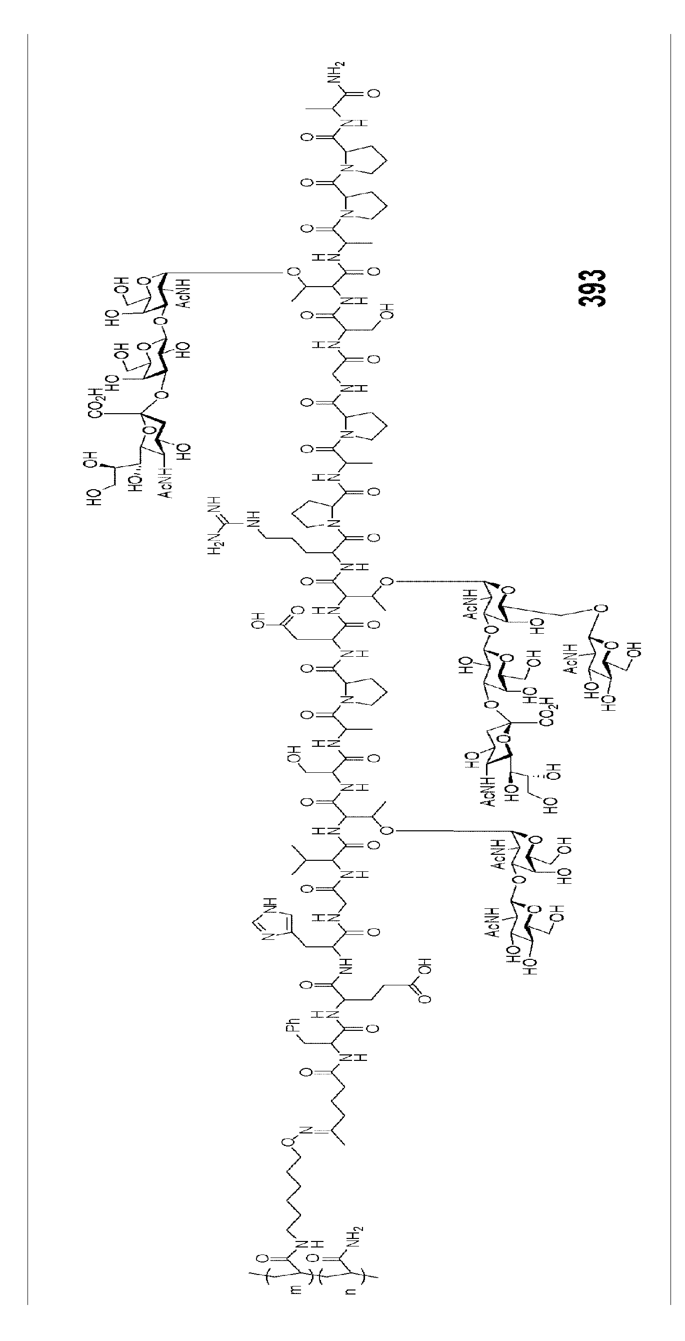

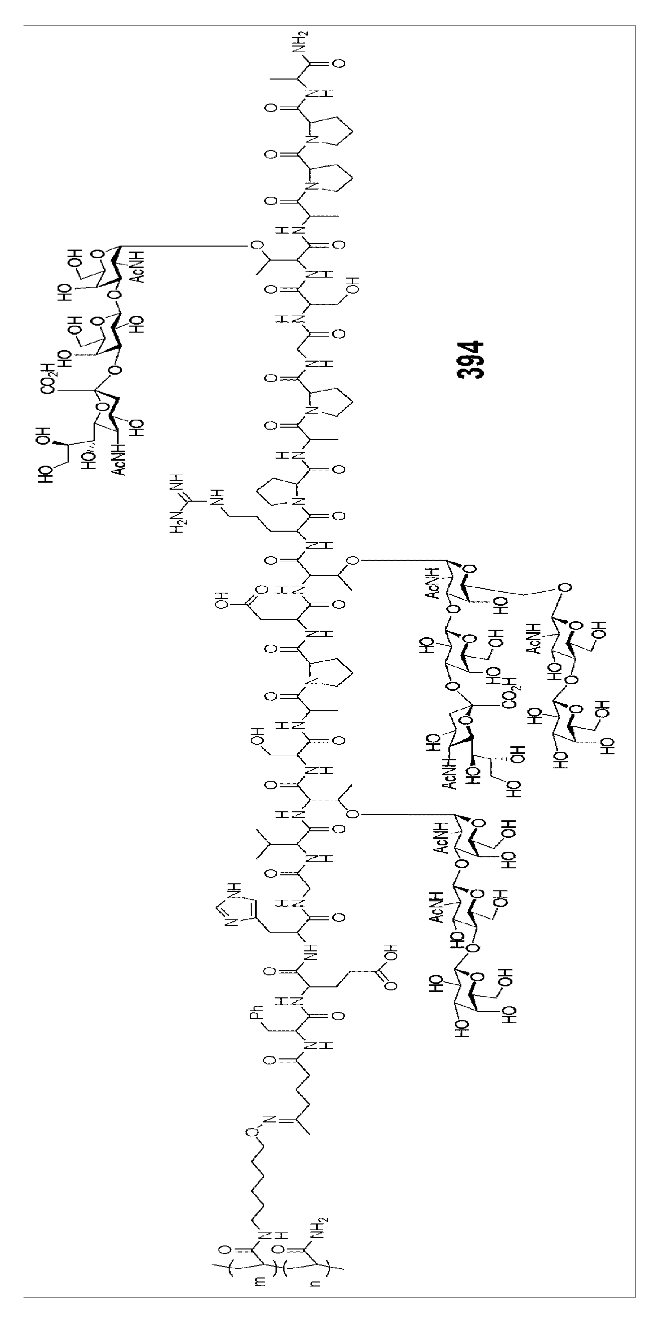

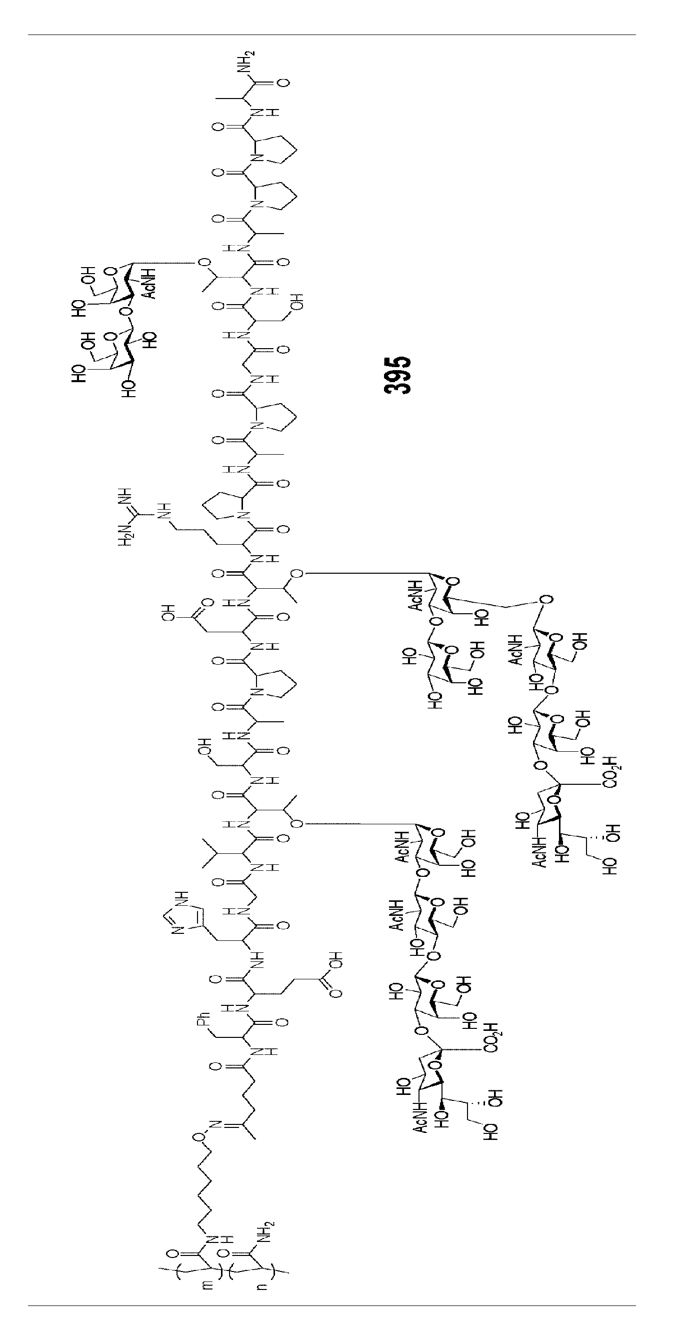

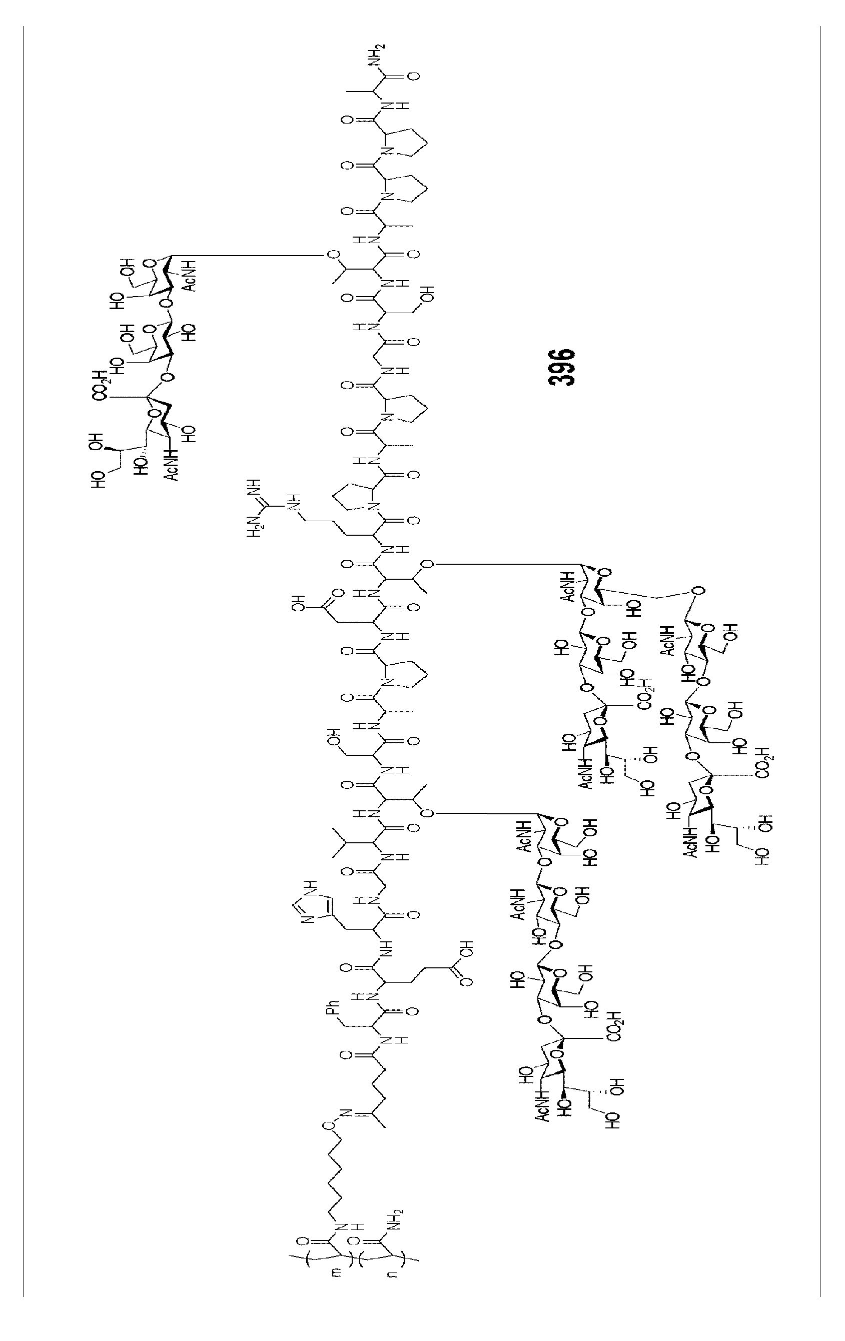

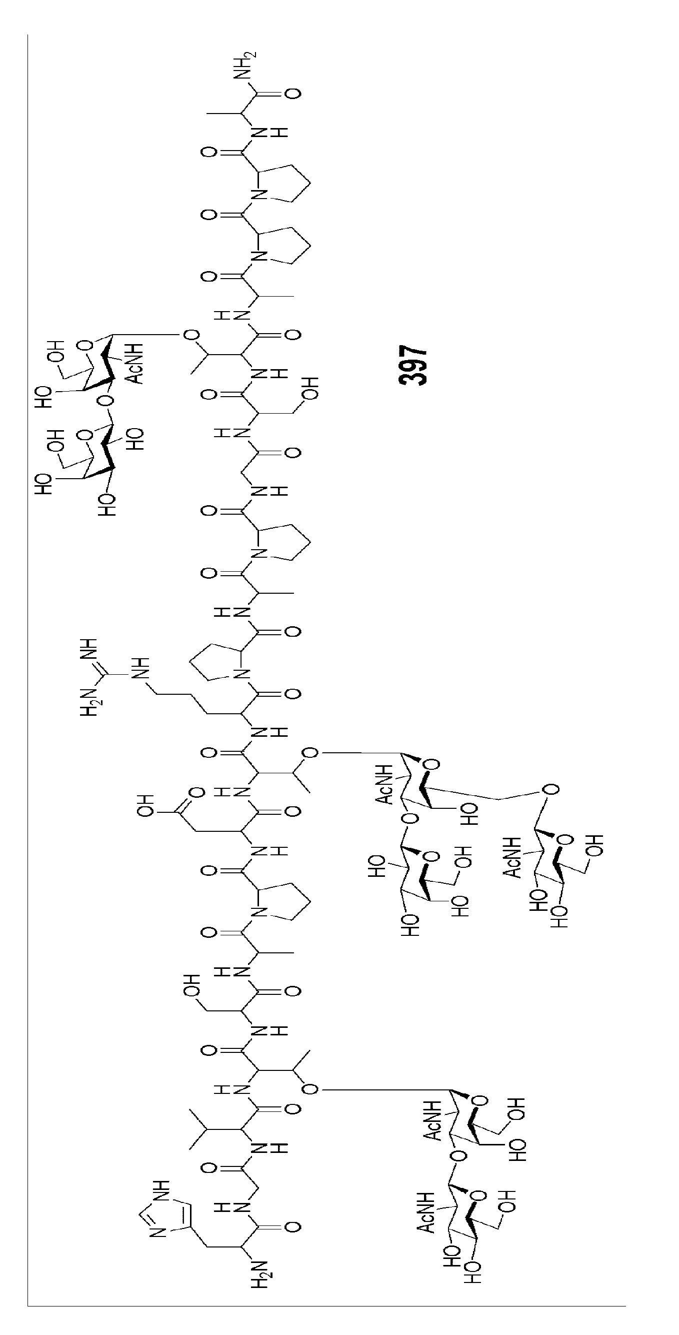

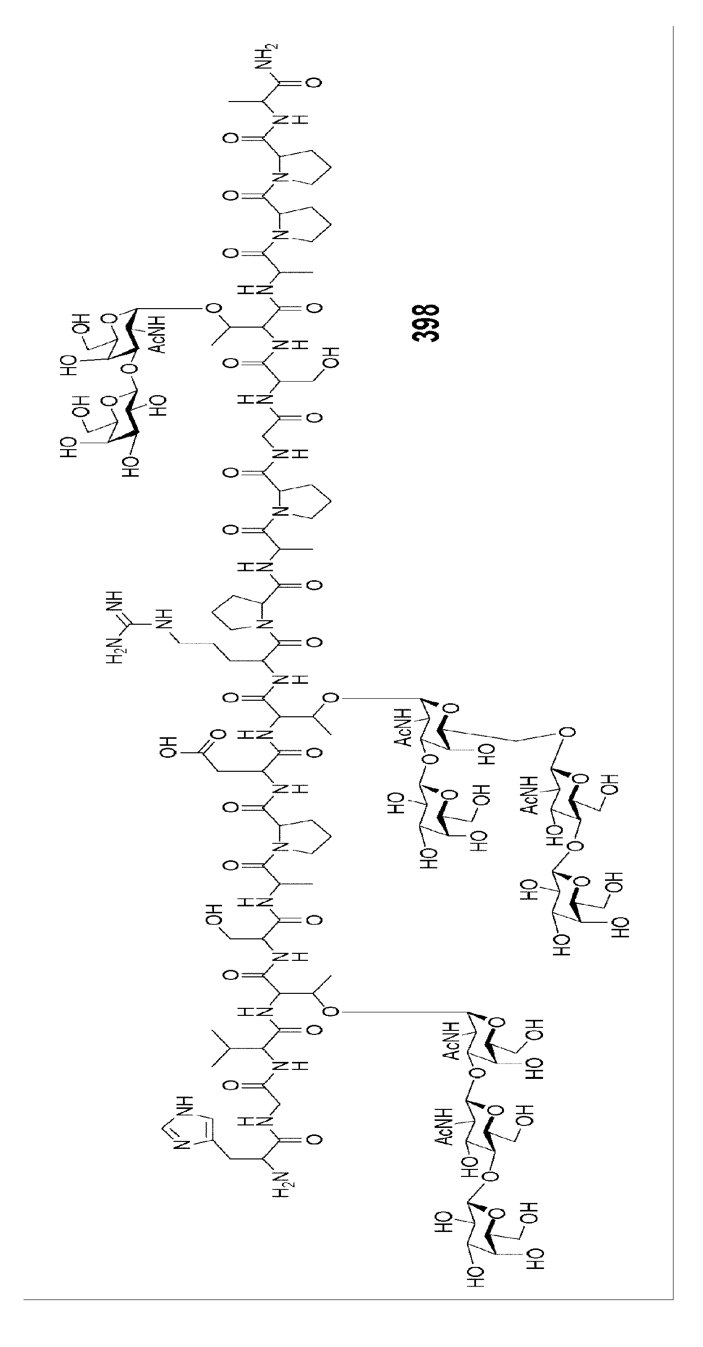

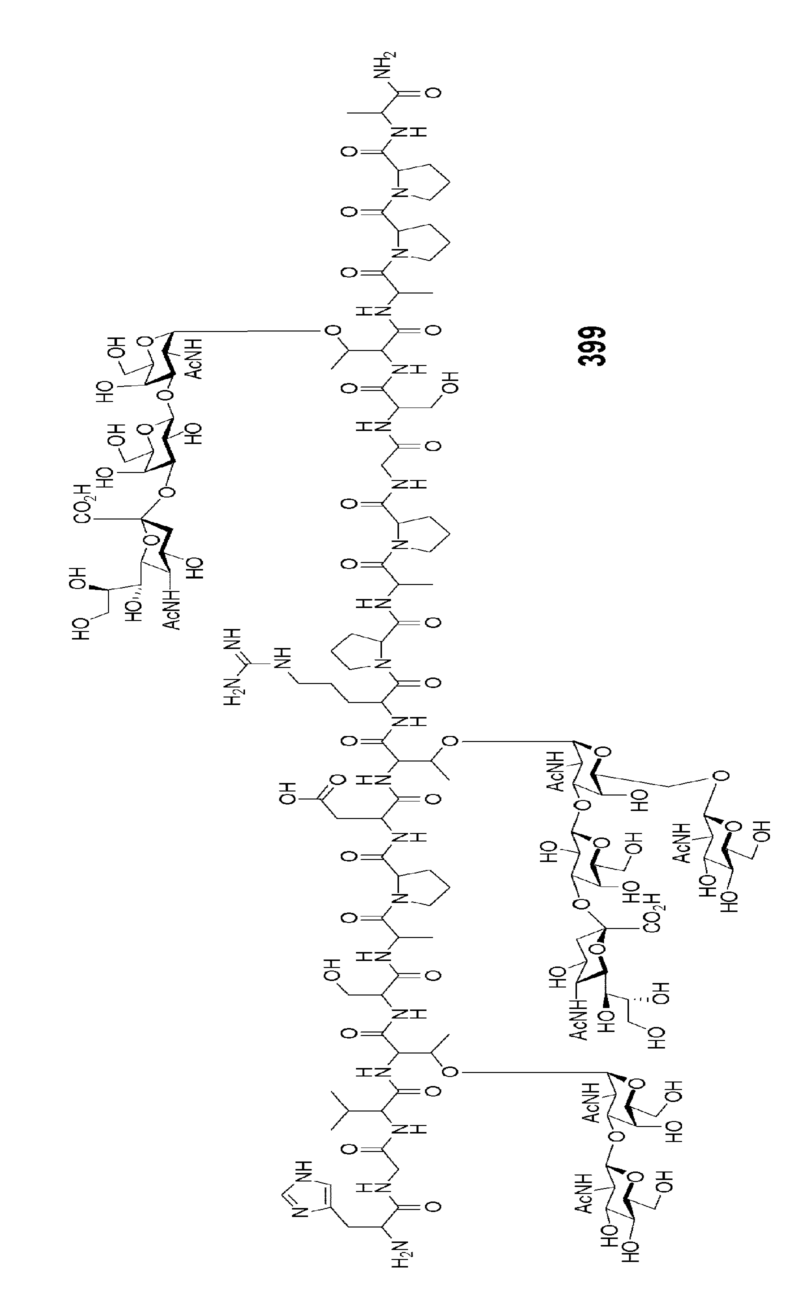

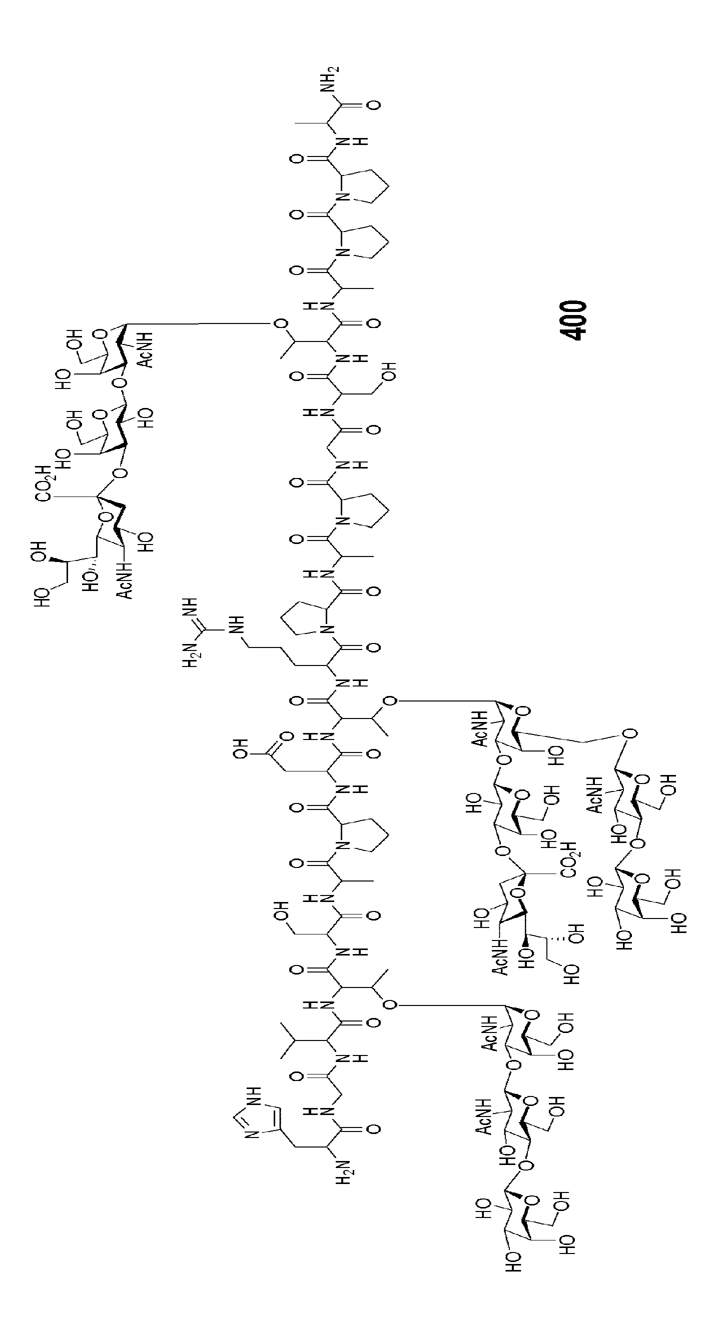

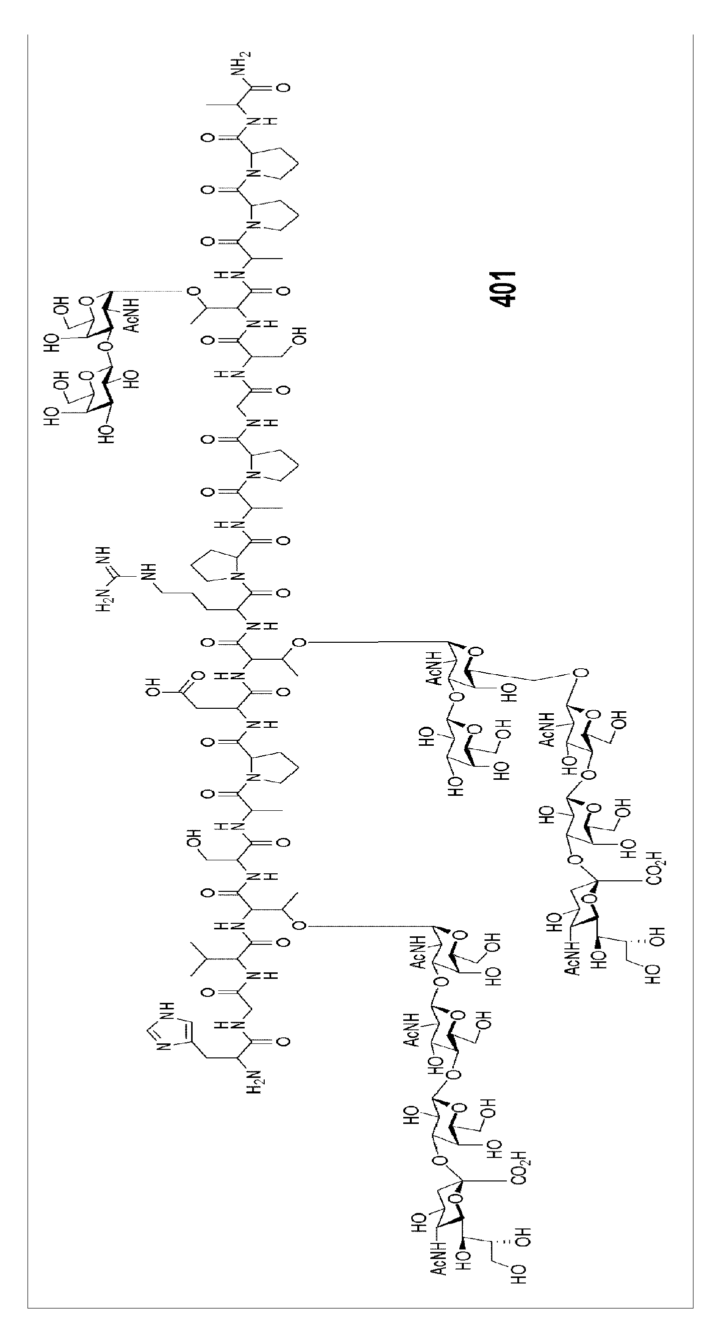

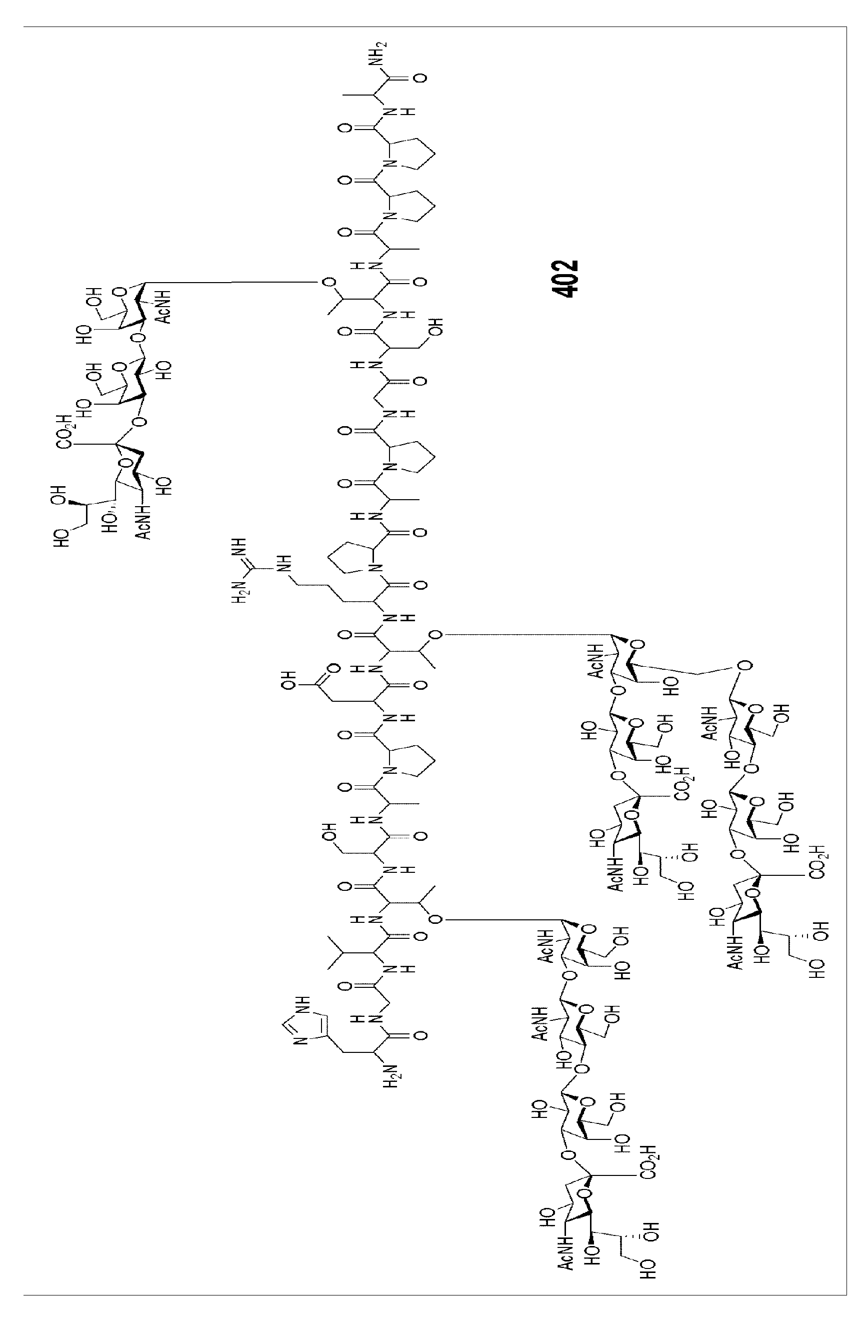

The following reaction solutions A) to E) were allowed to react for 24 hours at 25°C:
A) 100µl of reaction solution containing 50mMHEPES buffer solution (pH 7.0), 0.1U/ml human-derived β1,4-galactosyltransferase (available from TOYOBO CO., LTD.), 10mM manganese chloride, 0.1% BSA, 2mM uridine-5'-disodium diphosphogalactose (UDP-Gal) and glycopeptide derivative (239) (4mM at a theoretical content from solid-phase synthesis);
B) 100µl of reaction solution containing 50mM HEPES buffer solution (pH 7.0), 0.0175U/ml recombinant rat α2,3-(O)-sialyltransferase (available from Calbiochem), 10mM manganese chloride, 0.1% BSA, 2mM cytidine-5'-sodium monophosphosialate (CMP-NANA) and glycopeptide derivative (239) (4mM at a theoretical content from solid-phase synthesis);
C) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (239) (4mM at a theoretical content from solid-phase synthesis);
D) 100 µl of reaction solution containing 50 mM HEPES buffer solution (pH 7.0), 0.1U/ml human-derived β1,4-galactosyltransferase (available from TOYOBO CO., LTD.), 0.0185U/ml recombinant rat α2,3-(N)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (239) (4mM at a theoretical content from solid-phase synthesis);
E) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 0.0185U/ml recombinant rat α2,3-(N)-sialyltransferase (available from Calbiochem), 10mM manganese chloride, 0.1% BSA, 4mM uridine-5'-disodium diphosphogalactose (UDP-Gal), 2mM cytidine-5'-sodium monophosphosialate (CMP-NANA) and glycopeptide derivative (239) (4mM at a theoretical content from solid-phase synthesis).

After completion of the reaction, the respective reaction solutions were subjected to centrifugal concentration with an ultrafiltration filter. Thereafter, 25mM ammonium acetate buffer solution (pH 6.5) was added thereto, and the solution was again subjected to concentration with a centrifugal separator, thereby washing the polymer. This manipulation was repeated three times, thereby respectively obtaining aqueous solutions of compounds (392) to (396). Thereafter, to the solution retained at the filter containing compounds (392) to (396) and a solution which was obtained by diluting an aqueous solution of (239) with 25mM ammonium acetate buffer solution (pH 6.5), 1µl of 0.174mg/ml solution of BLase (available from Shionogi & Co., Ltd.) was added, and the solution was allowed to react for two hours at room temperature. Thereafter, the solution was subjected to centrifugal filtration with an ultrafiltration filter, thereby separating the glycopeptide of interest from the polymer. The obtained aqueous solution (filtrate) was lyophilized, thereby obtaining compounds (397) to (402).
MALDI-TOF/MS:[M(average)+H]⁺ of compound (397)=3228.9 (theoretical value:[M(average)+H]⁺=3227.3); MALDI-TOF/MS:[M(average)+H]⁺ of compound (398)=3552.9 (theoretical value:[M(average)+H]⁺=3551.6); MALDI-TOF/MS:[M(average)+H]⁺ of compound (399)=3811.0 (theoretical value:[M(average)+H]⁺=3809.8); MALDI-TOF/MS:[M(average)+H]⁺ of compound (400)=4135.2 (theoretical value:[M(average)+H]⁺=4134.1); MALDI-TOF/MS:[M(average)+H]⁺ of compound (401)=4135.5 (theoretical value:[M(average)+H]⁺=4134.1); and MALDI-TOF/MS:[M(average)+H]⁺ of compound (402)=4715.8 (theoretical value: [M(average)+H]⁺=4716.6).

(3.67 Synthesis of compounds (403) to (413))

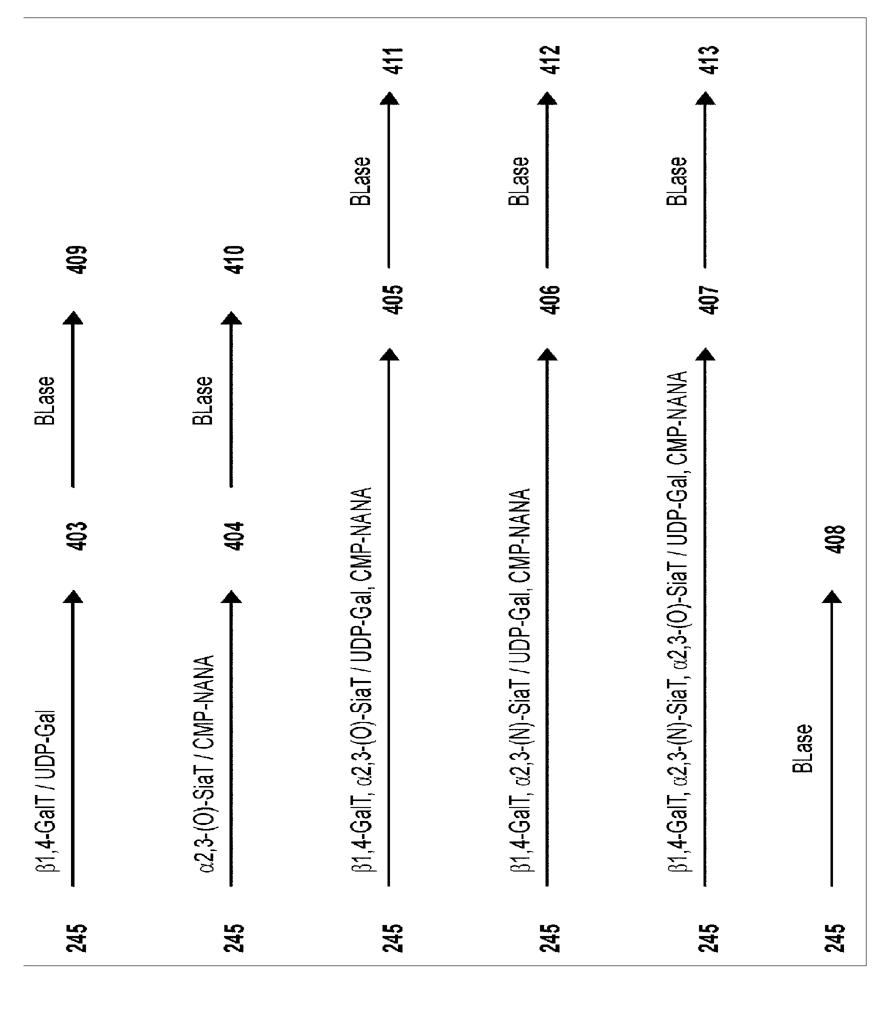

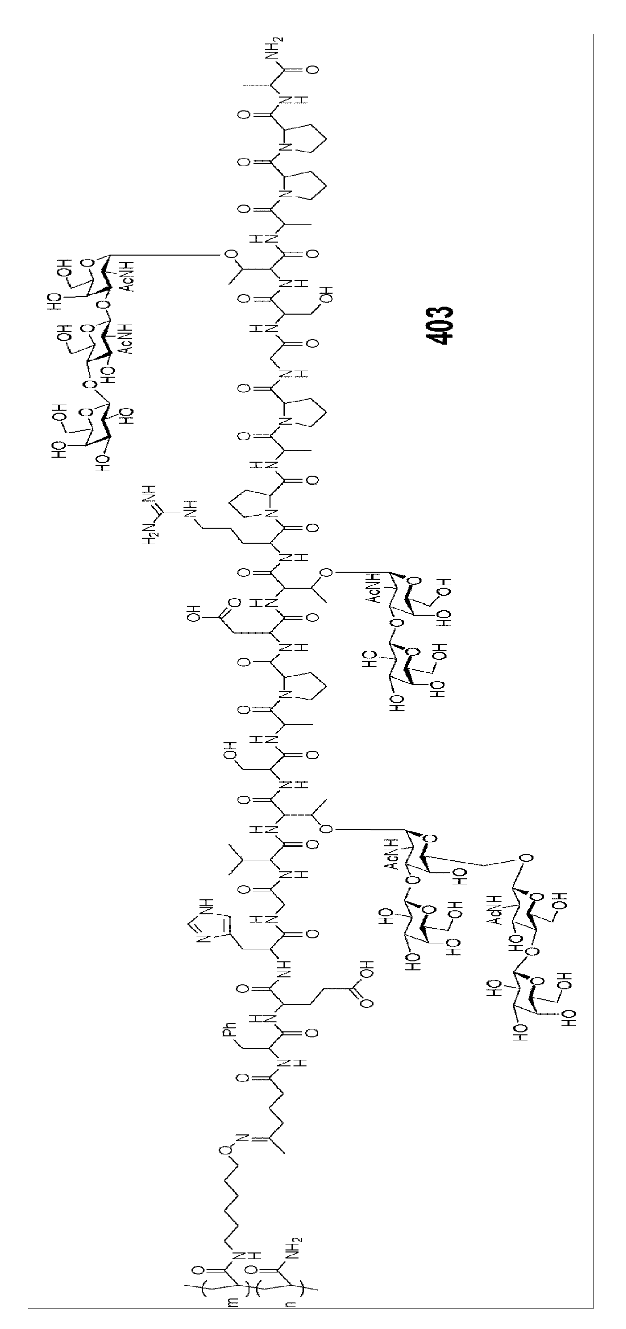

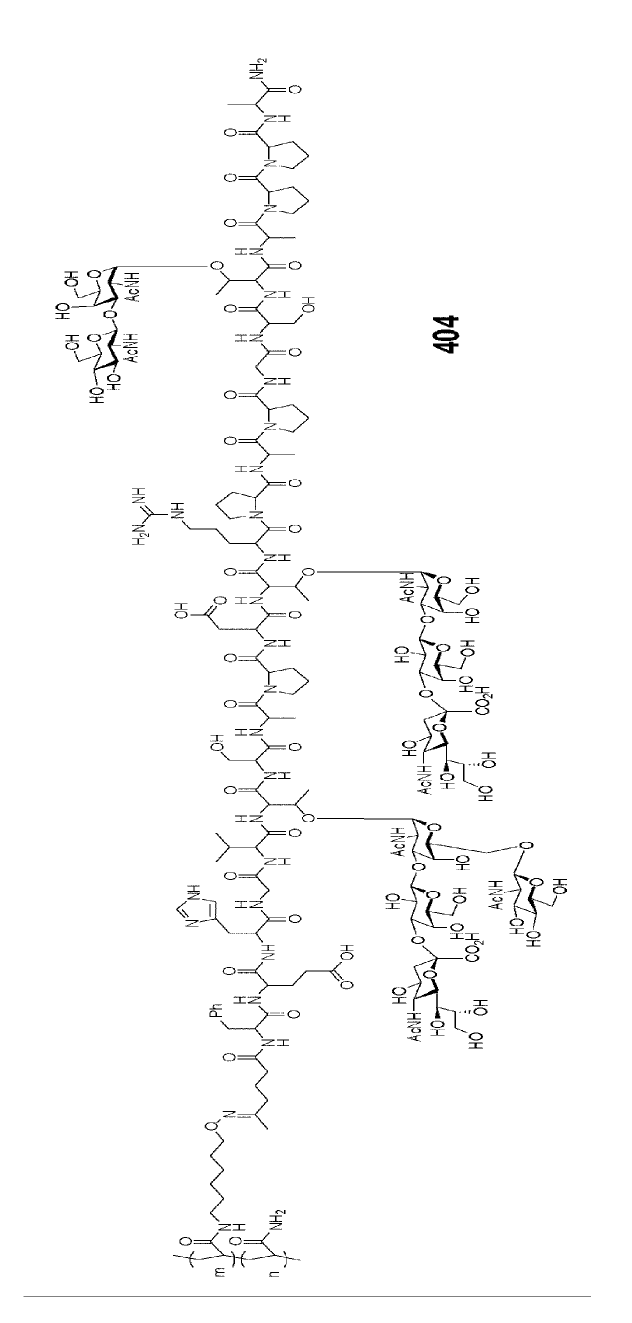

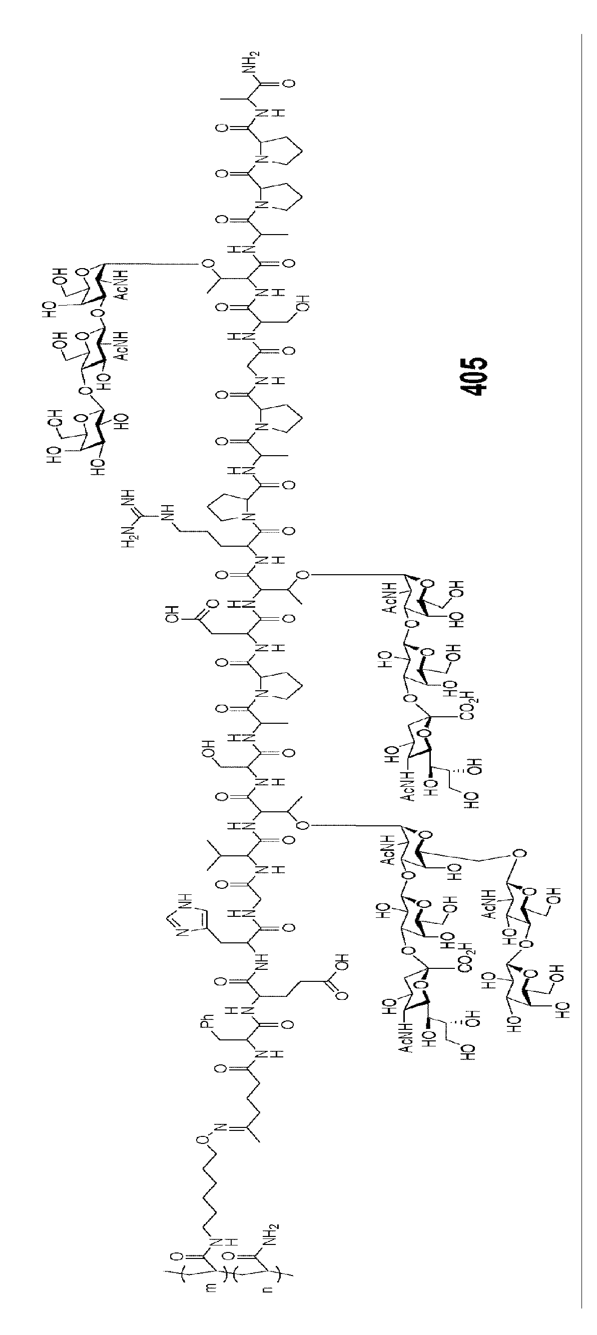

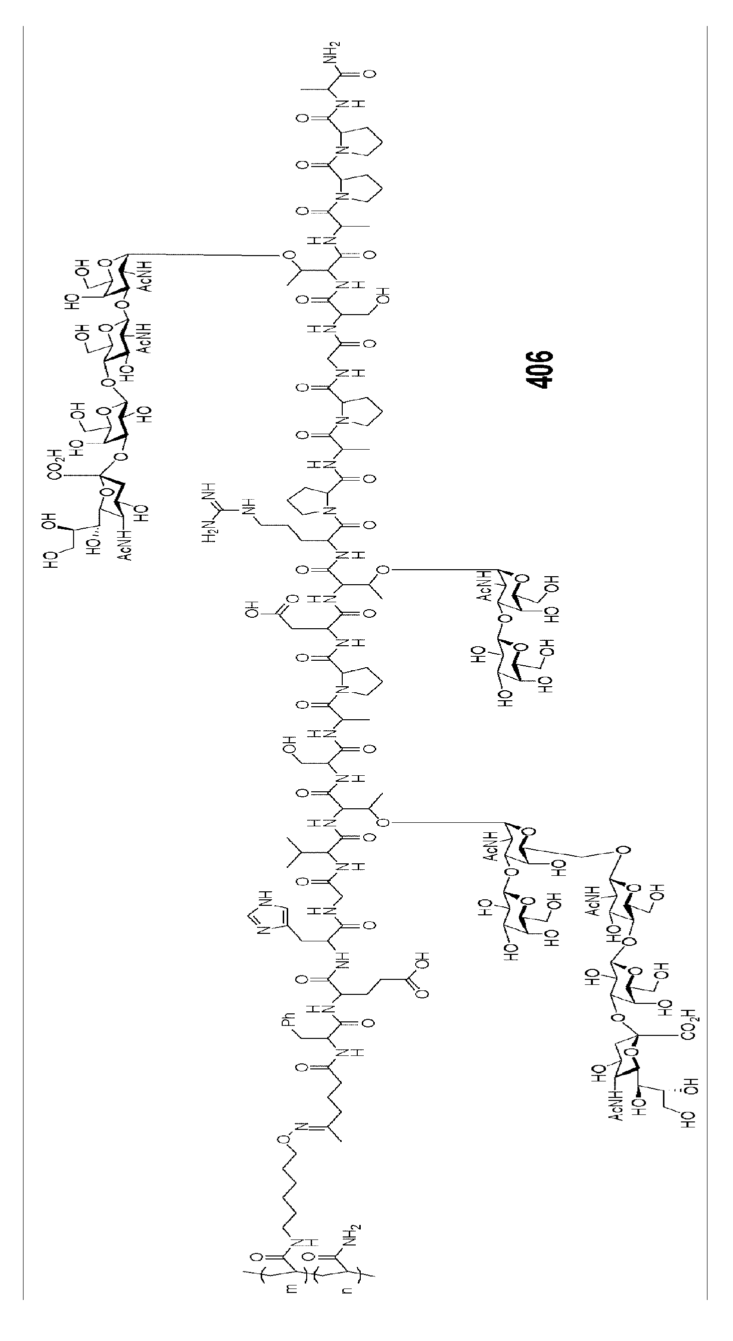

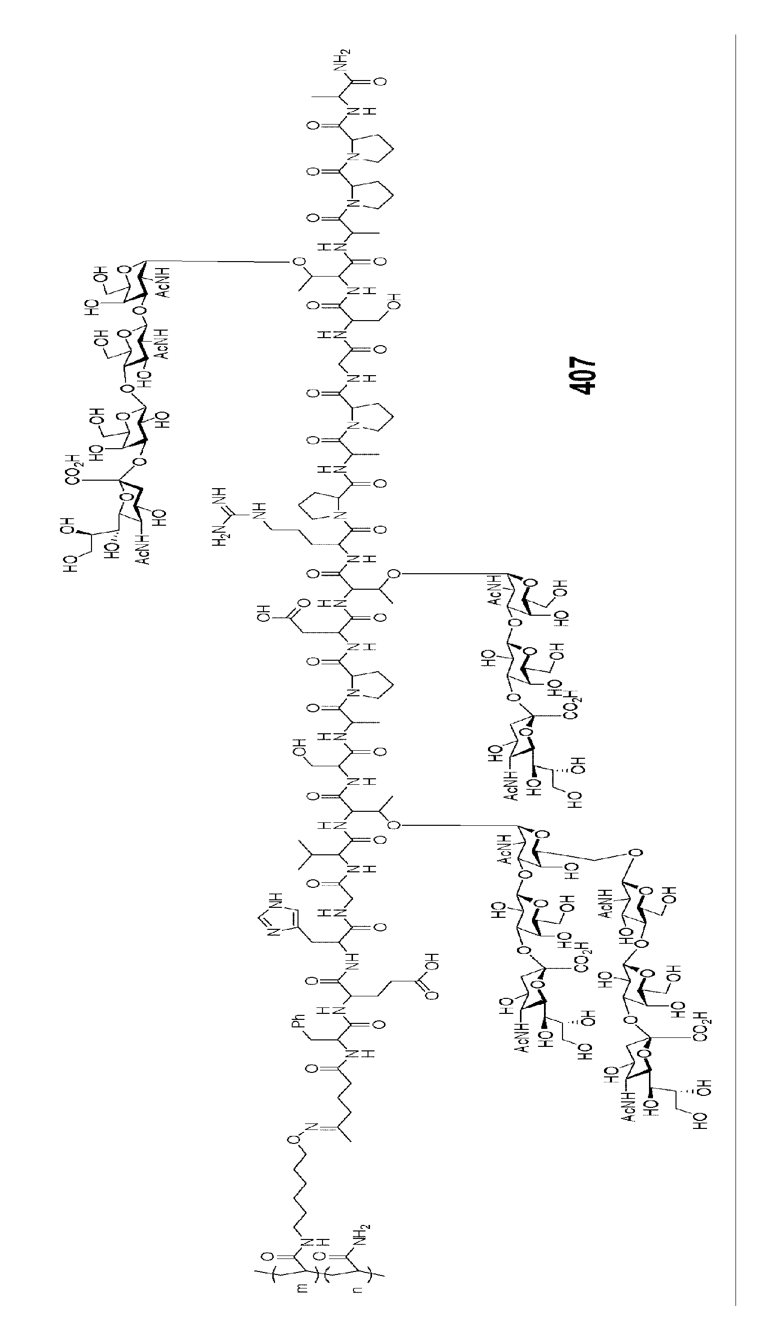

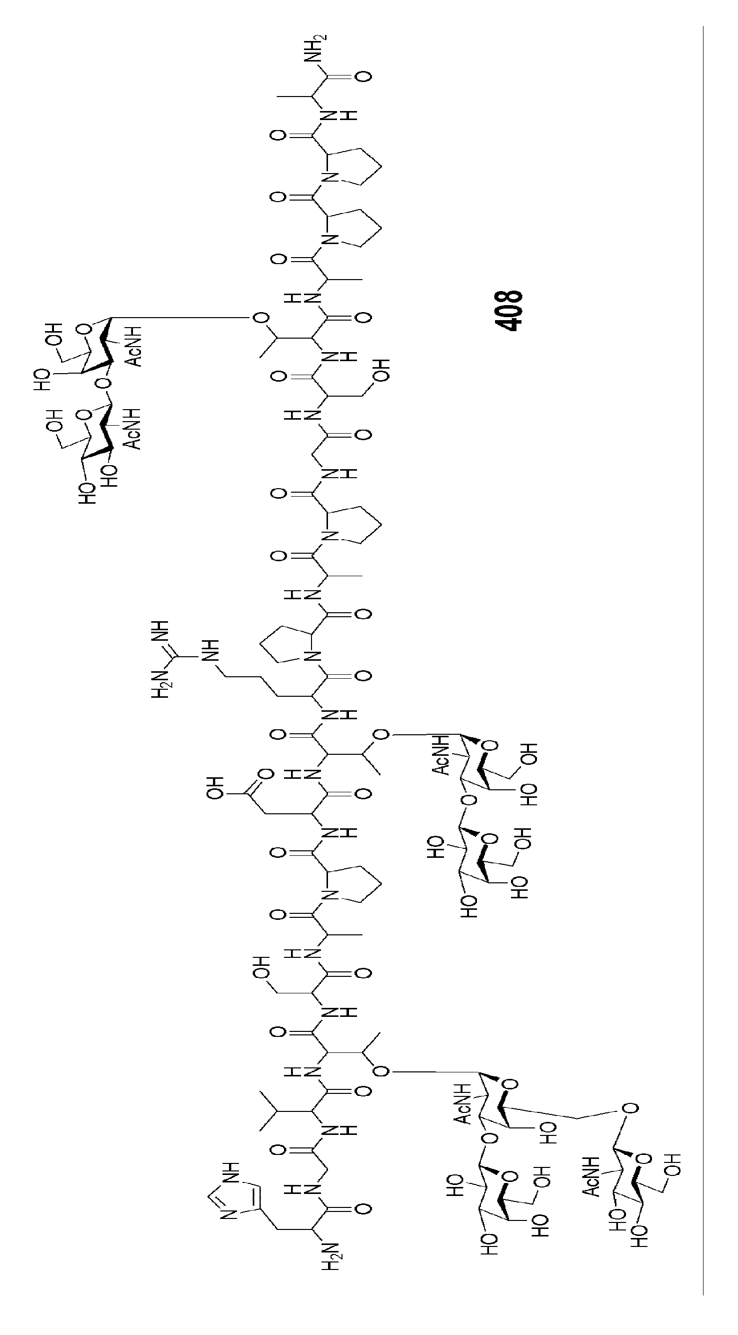

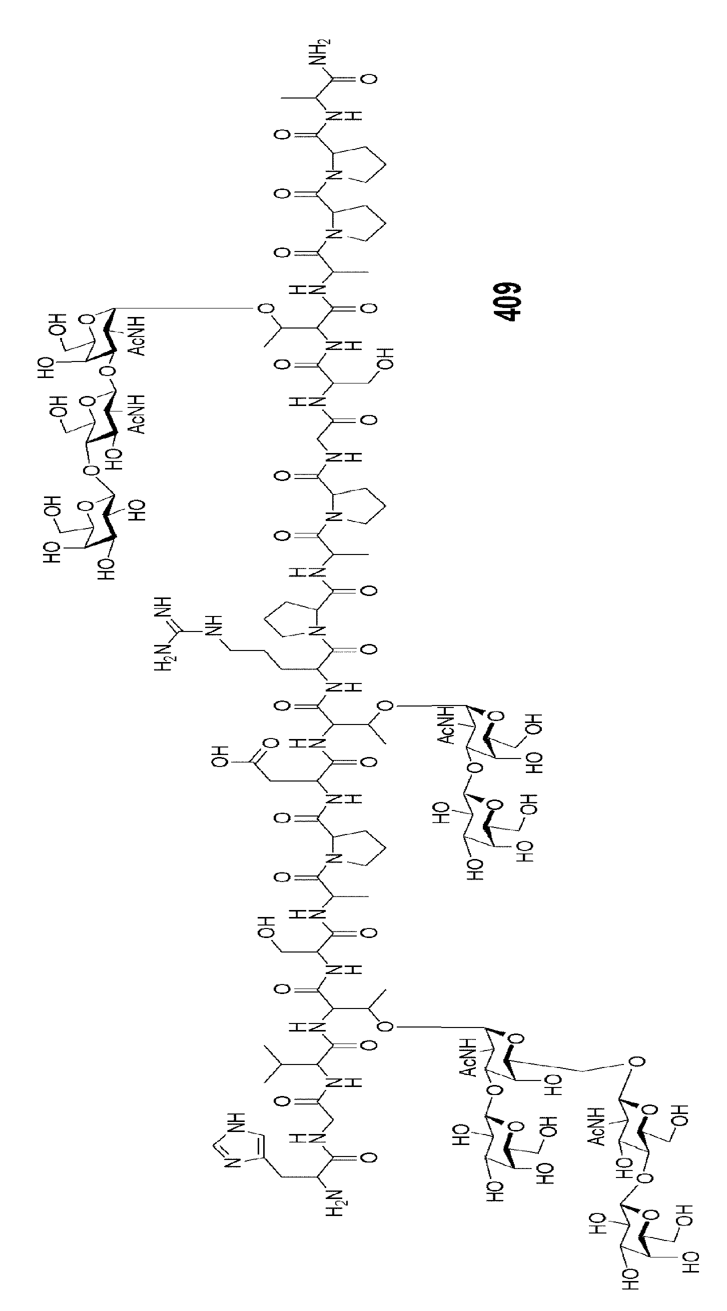

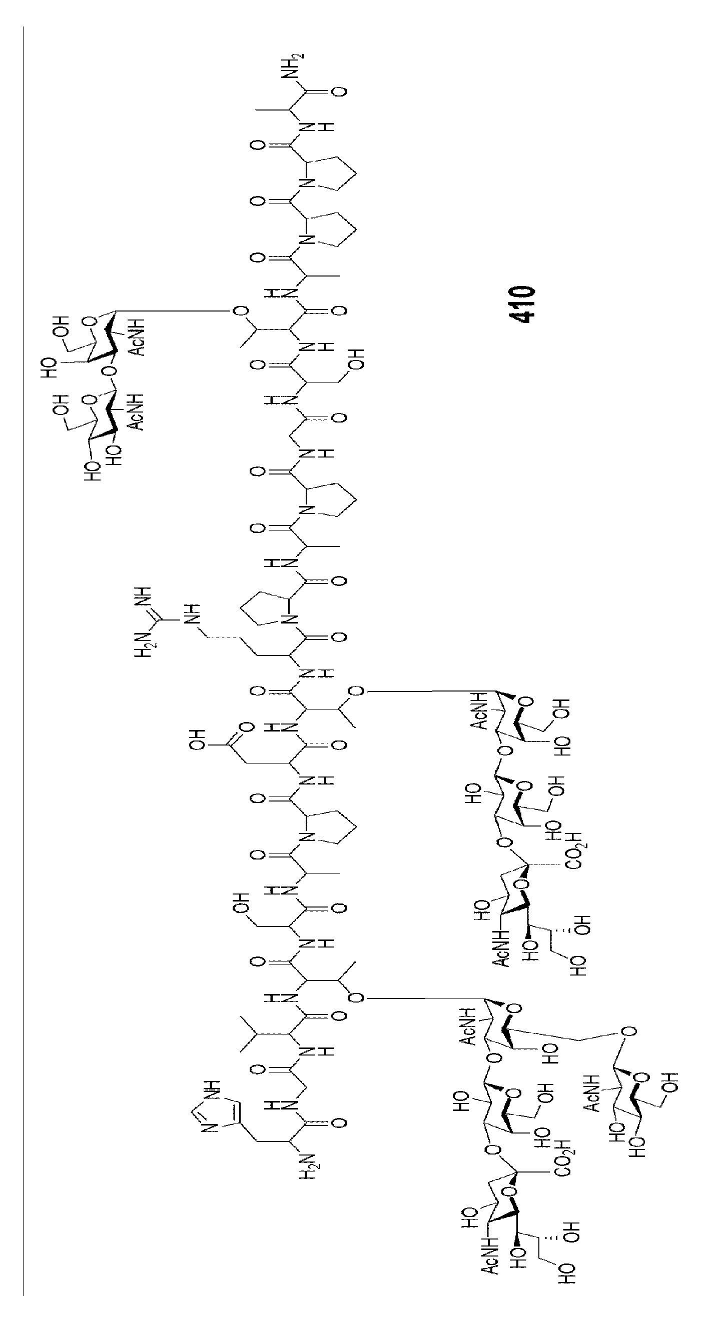

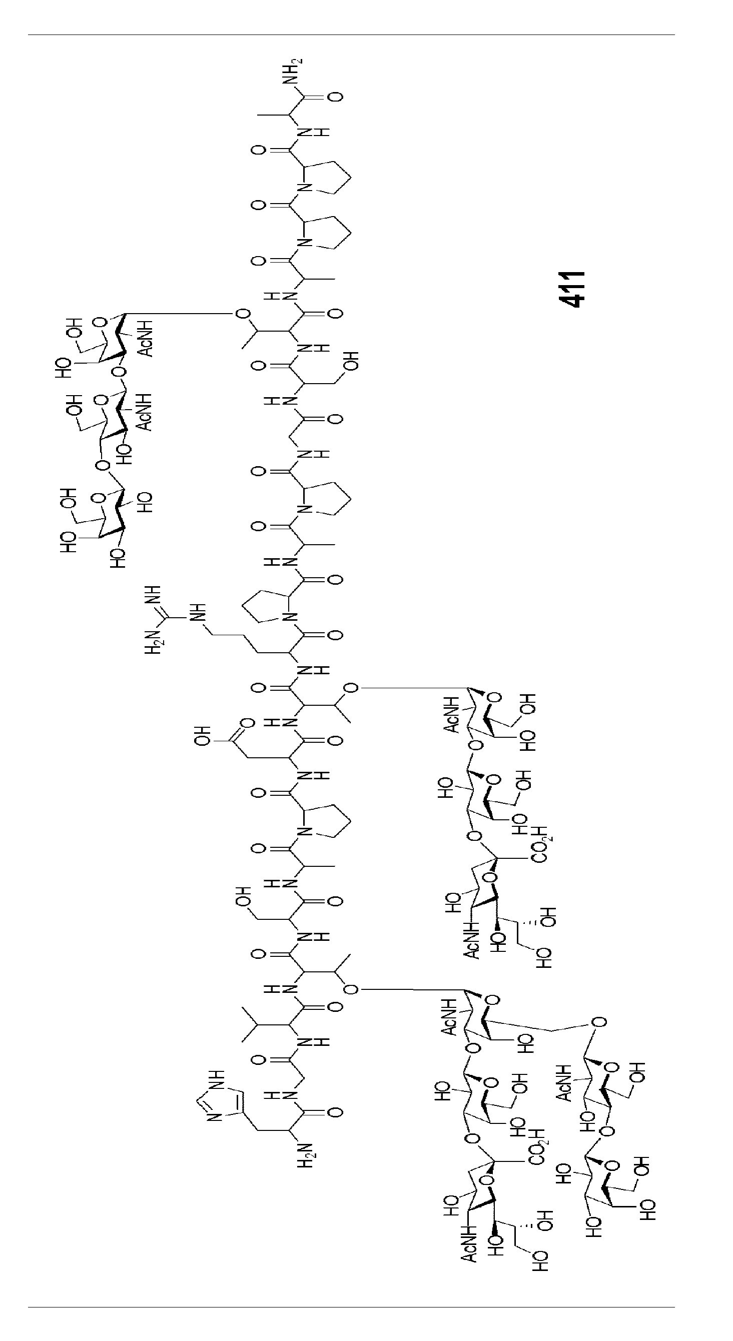

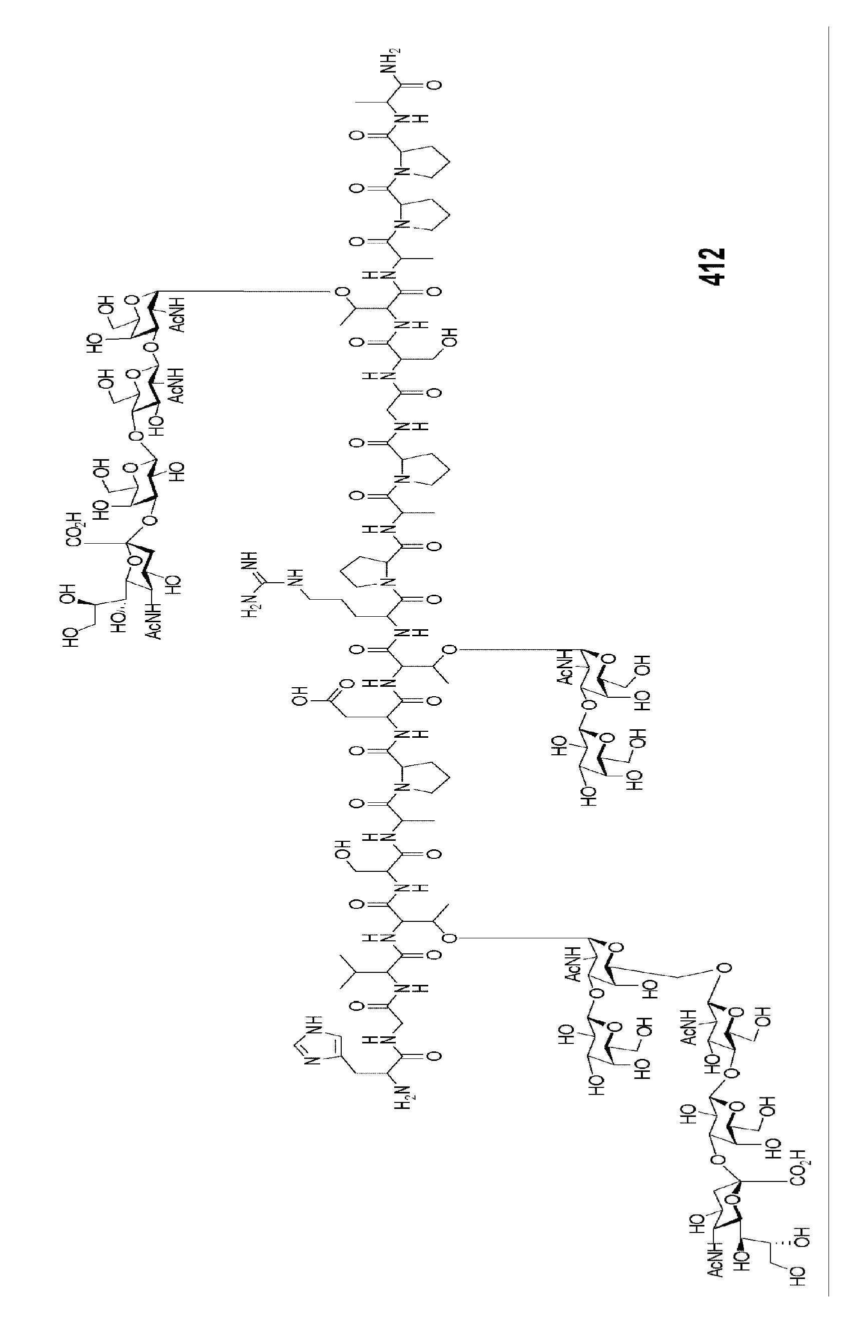

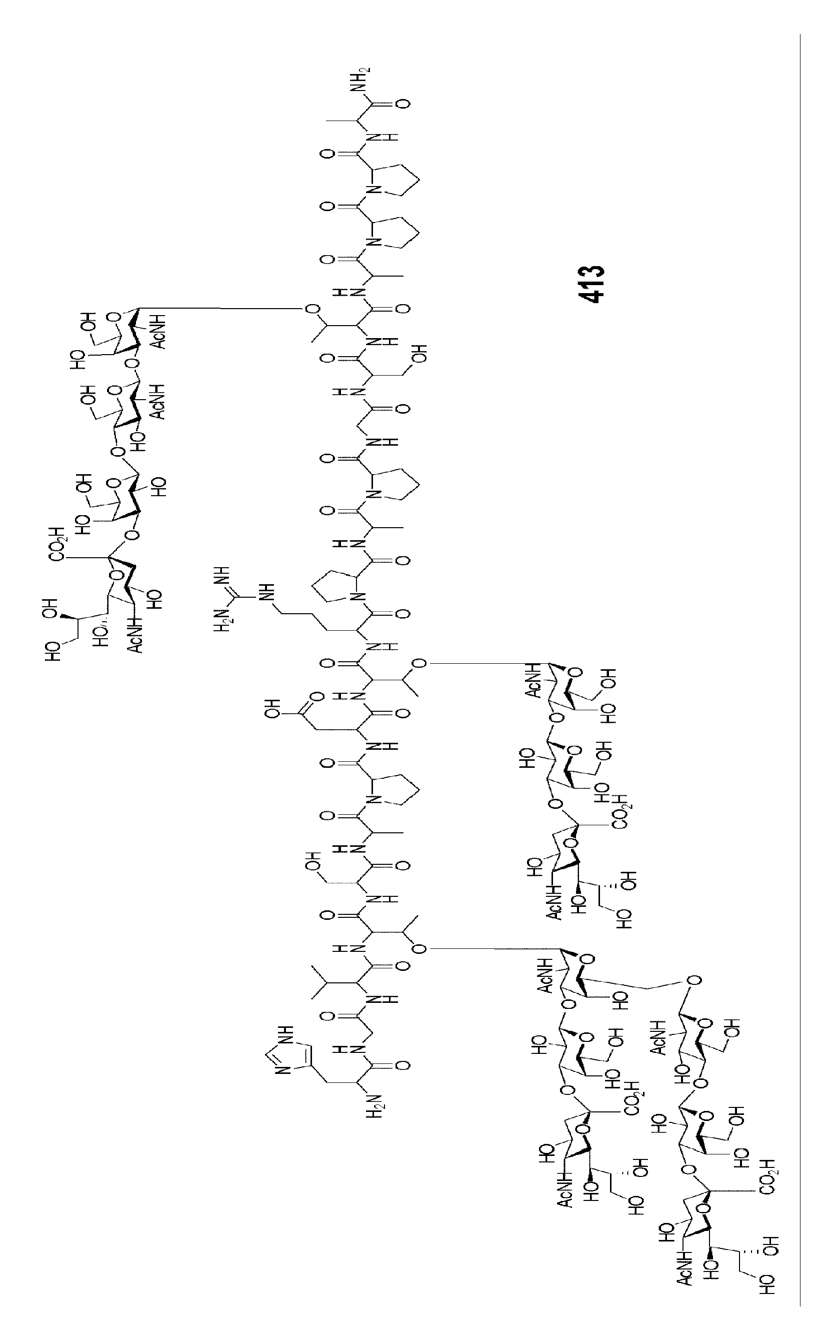

The following reaction solutions A) to E) were allowed to react for 24 hours at 25°C:
A) 100µl of reaction solution containing 50mMHEPES buffer solution (pH 7.0), 0.1U/ml human-derived β1,4-galactosyltransferase (available from TOYOBO CO., LTD.), 10mM manganese chloride, 0.1% BSA, 2mM uridine-5'-disodium diphosphogalactose (UDP-Gal) and glycopeptide derivative (245) (4mM at a theoretical content from solid-phase synthesis);
B) 100µl of reaction solution containing 50mM HEPES buffer solution (pH 7.0), 0.0175U/ml recombinant rat α2,3-(O)-sialyltransferase (available from Calbiochem), 10mM manganese chloride, 0.1% BSA, 2mM cytidine-5'-sodium monophosphosialate (CMP-NANA) and glycopeptide derivative (245) (4mM at a theoretical content from solid-phase synthesis);
C) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (245) (4mM at a theoretical content from solid-phase synthesis);
D) 100µl of reaction solution containing 50mM HEPES buffer solution (pH 7.0), 0.1U/ml human-derived β1,4-galactosyltransferase (available from TOYOBO CO., LTD.), 0.0185U/ml recombinant rat α2,3-(N)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (245) (4mM at a theoretical content from solid-phase synthesis);
E) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 0.0185U/ml recombinant rat α2,3-(N)-sialyltransferase (available from Calbiochem), 10mM manganese chloride, 0.1% BSA, 4mM uridine-5'-disodium diphosphogalactose (UDP-Gal), 2mM cytidine-5'-sodium monophosphosialate (CMP-NANA) and glycopeptide derivative (245) (4mM at a theoretical content from solid-phase synthesis).

After completion of the reaction, the respective reaction solutions were subjected to centrifugal concentration with an ultrafiltration filter. Thereafter, 25mM ammonium acetate buffer solution (pH 6.5) was added thereto, and the solution was again subjected to concentration with a centrifugal separator, thereby washing the polymer. This manipulation was repeated three times, thereby respectively obtaining aqueous solutions of compounds (403) to (407). Thereafter, to the solution retained at the filter containing compounds (403) to (407) and a solution which was obtained by diluting an aqueous solution of (245) with 25mM ammonium acetate buffer solution (pH 6.5), 1µl of 0.174mg/ml solution of BLase (available from Shionogi & Co., Ltd.) was added, and the solution was allowed to react for two hours at room temperature. Thereafter, the solution was subjected to centrifugal filtration with an ultrafiltration filter, thereby separating the glycopeptide of interest from the polymer. The obtained aqueous solution (filtrate) was lyophilized, thereby obtaining compounds (408) to (413).
MALDI-TOF/MS:[M(average)+H]⁺ of compound (408)=3228.5 (theoretical value:[M(average)+H]⁺=3227.3); MALDI-TOF/MS:[M(average)+H]⁺ of compound (409)=3552.6 (theoretical value:[M(average)+H]⁺=3551.6); MALDI-TOF/MS:[M(average)+H]⁺ of compound (410)=3810.9 (theoretical value:[M(average)+H]⁺=3809.8); MALDI-TOF/MS:[M(average)+H]⁺ of compound (411)=4135.3 (theoretical value:[M(average)+H]⁺=4134.1); MALDI-TOF/MS:[M(average)+H]⁺ of compound (412)=4135.7 (theoretical value:[M(average)+H]⁺=4134.1); and MALDI-TOF/MS:[M(average)+H]⁺ of compound (413)=4716.9 (theoretical value:[M(average)+H]⁺=4716.6).

(3.68 Synthesis of compounds (414) to (424))

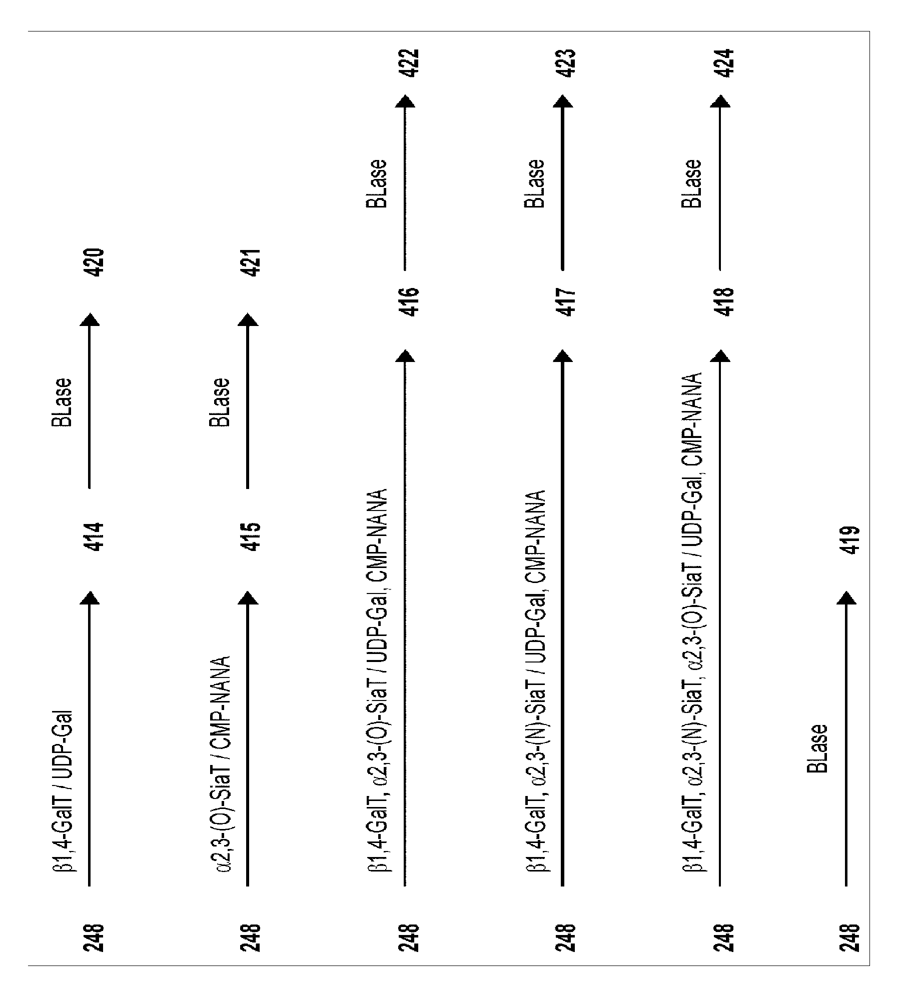

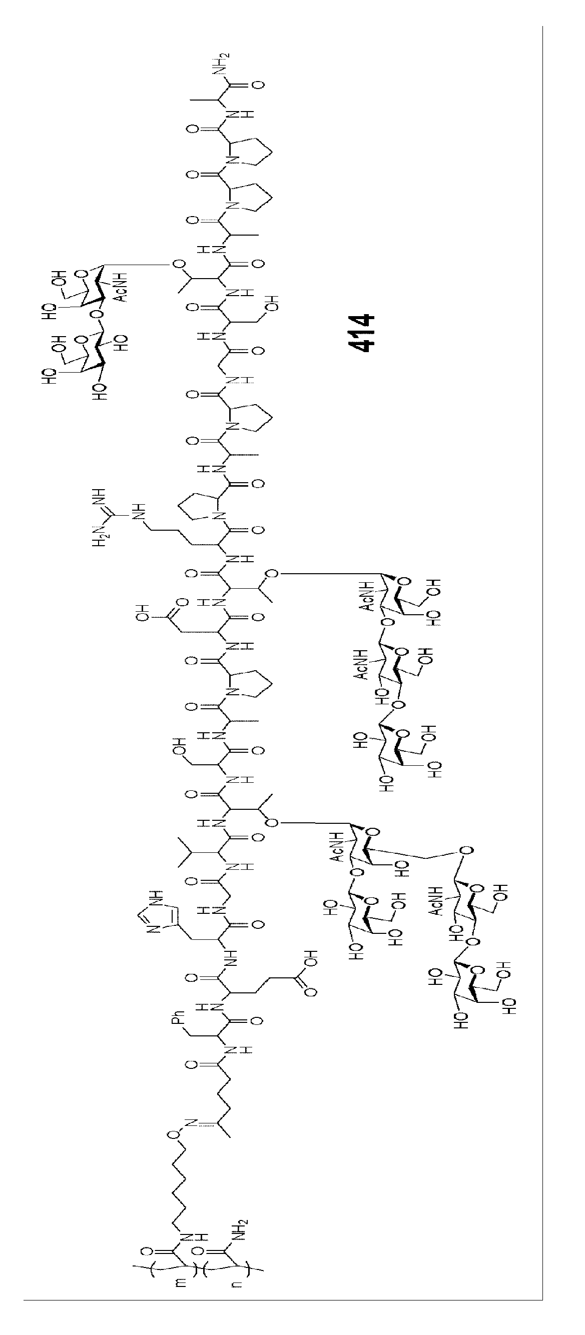

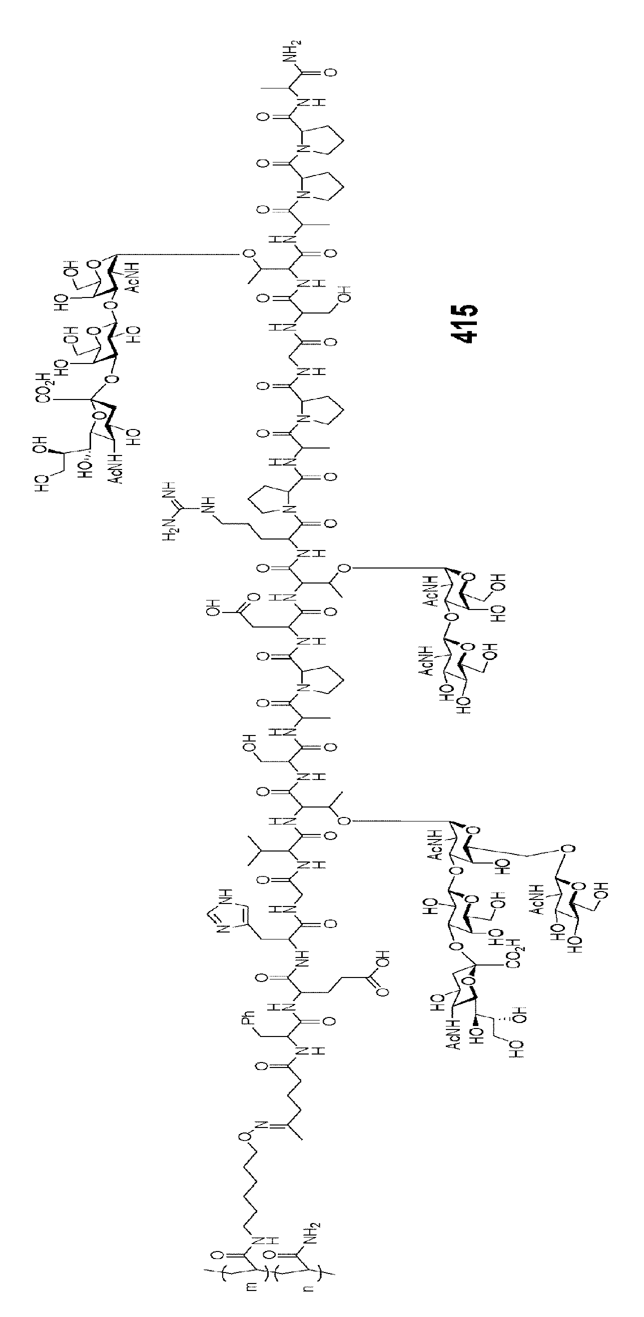

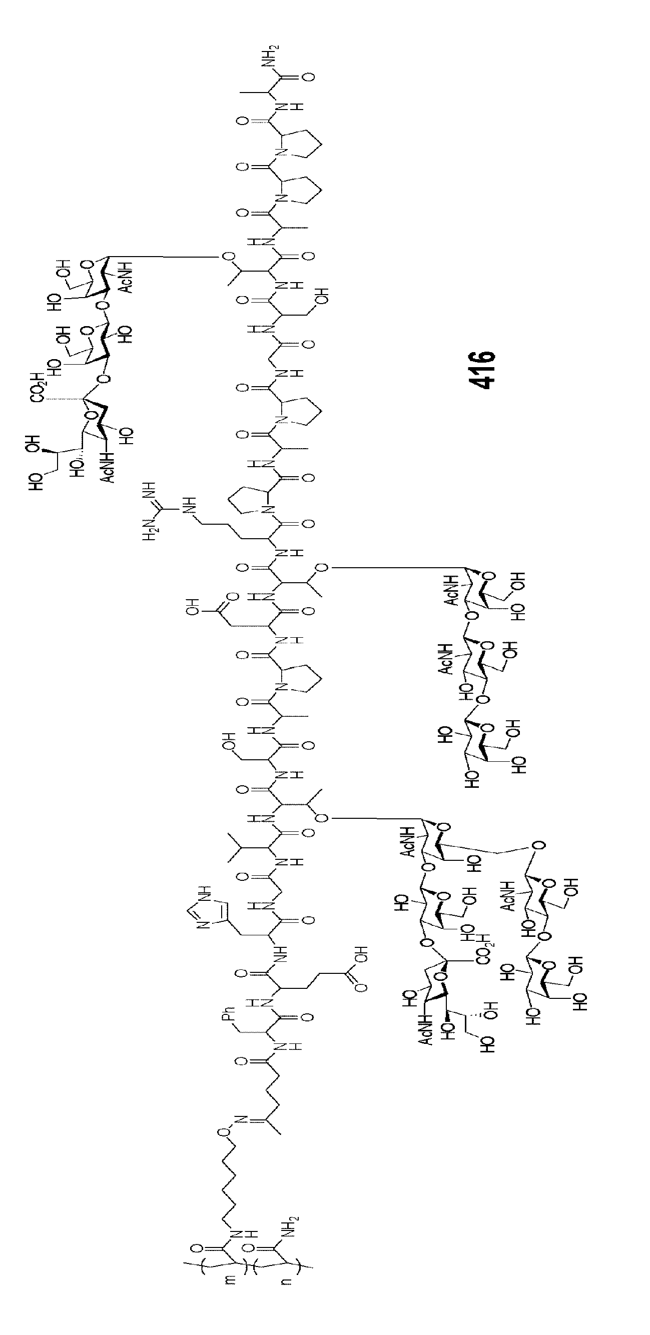

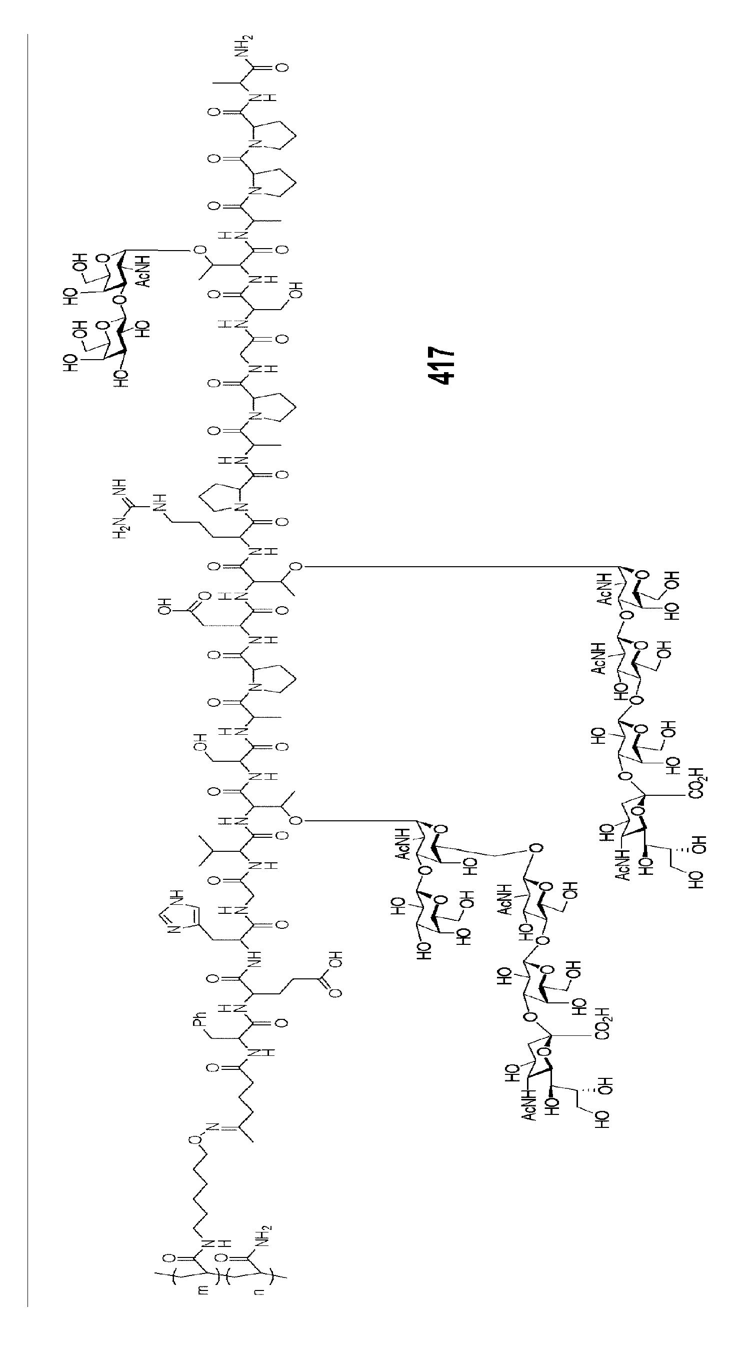

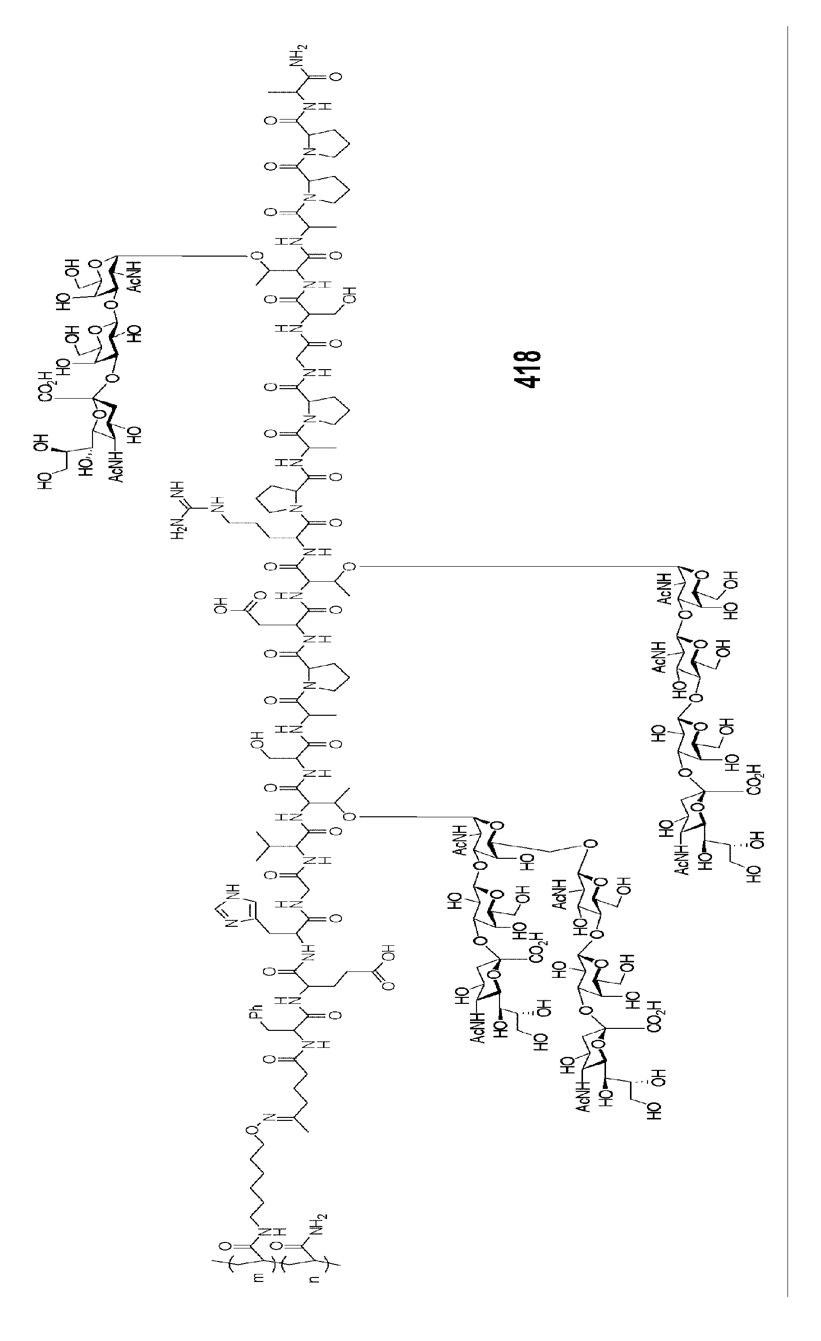

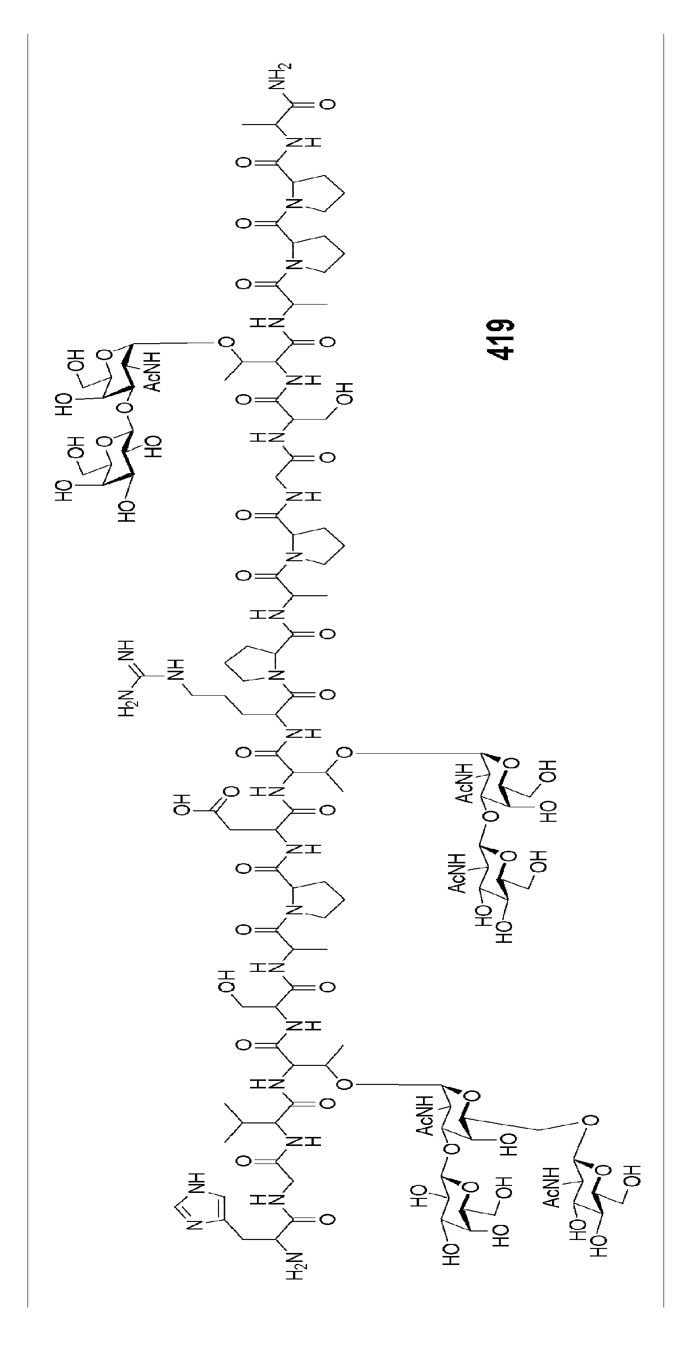

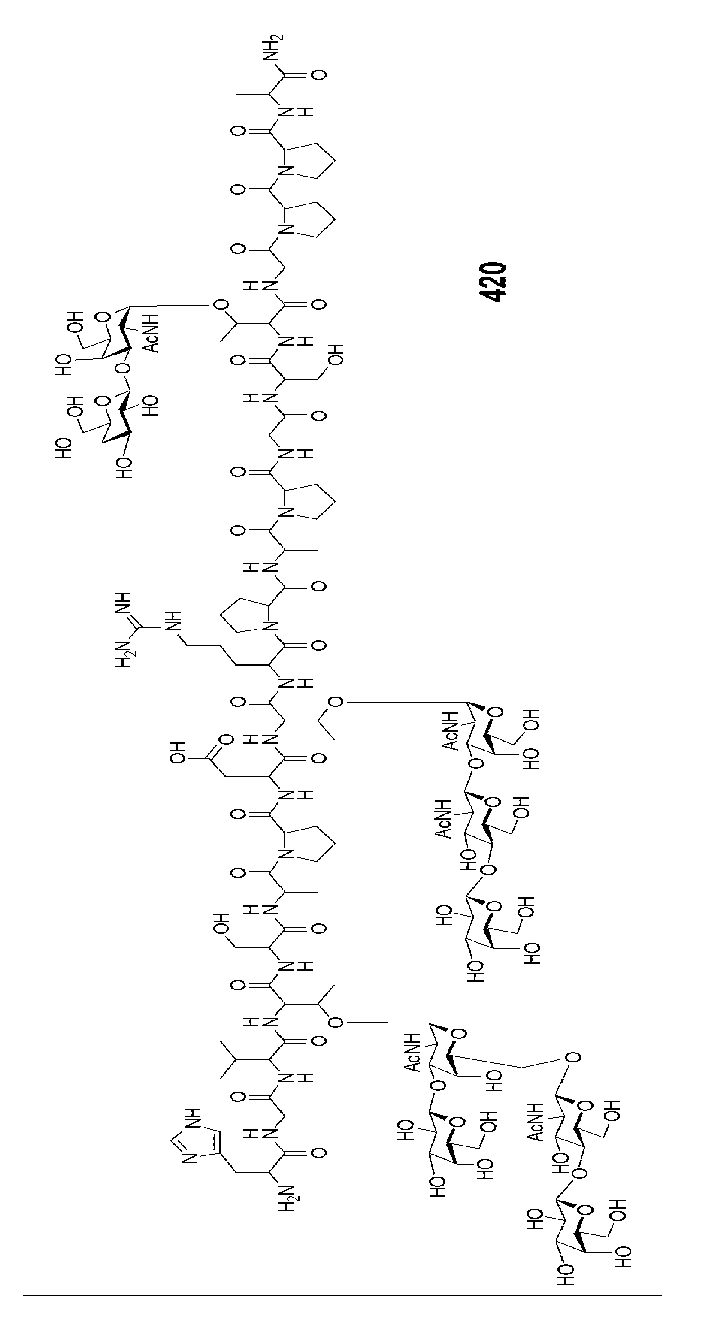

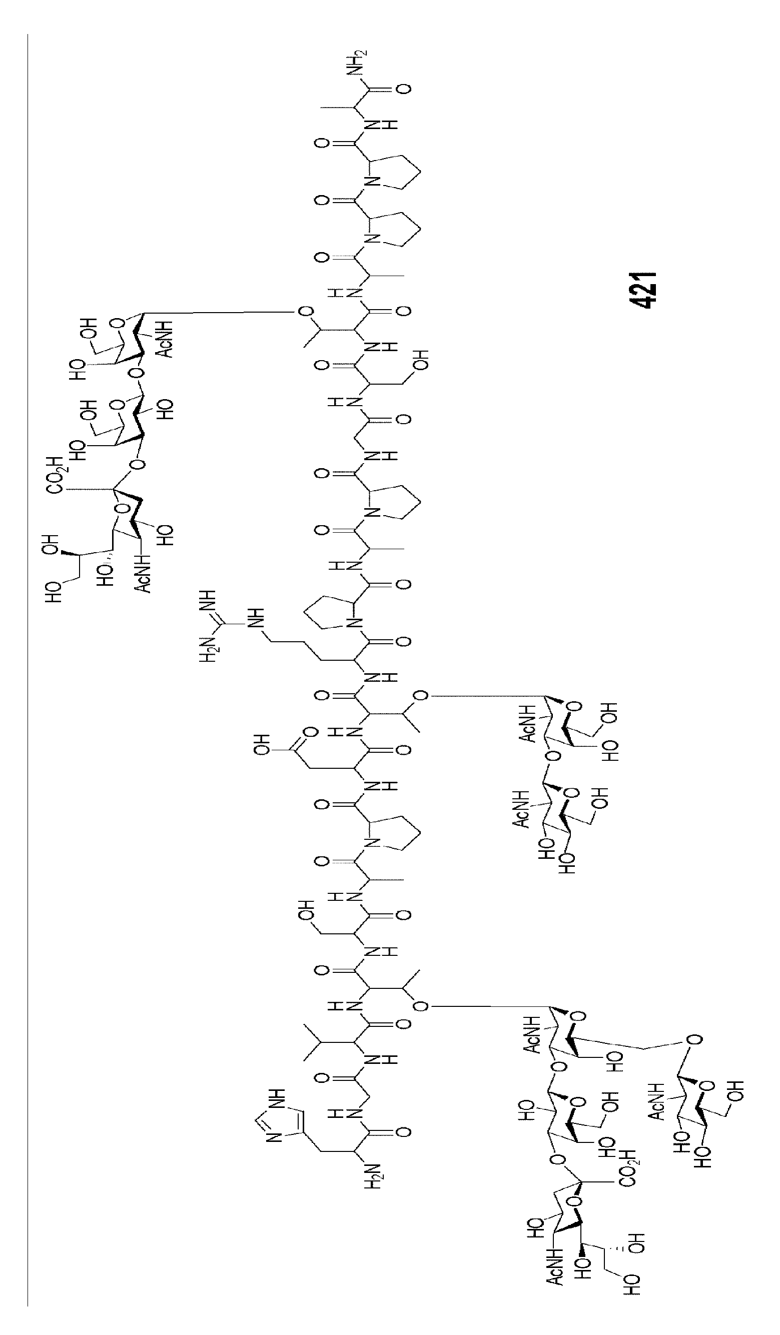

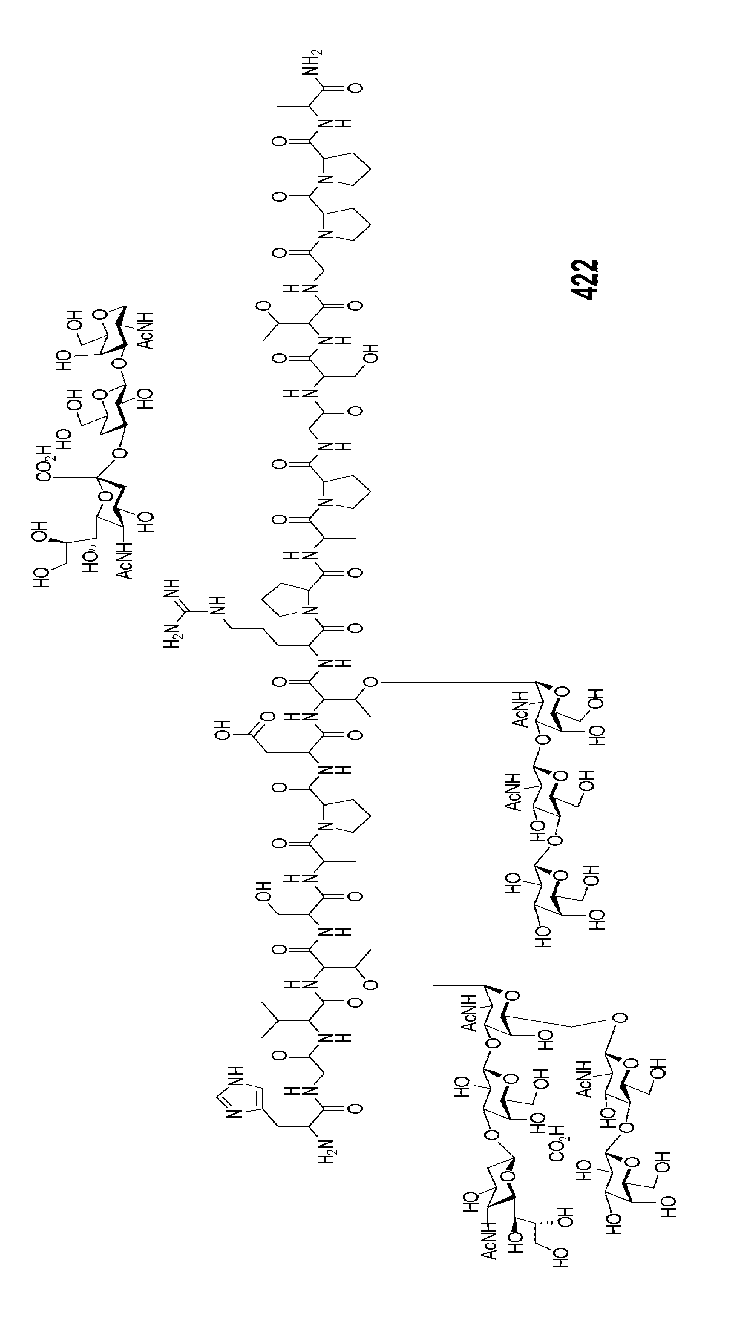

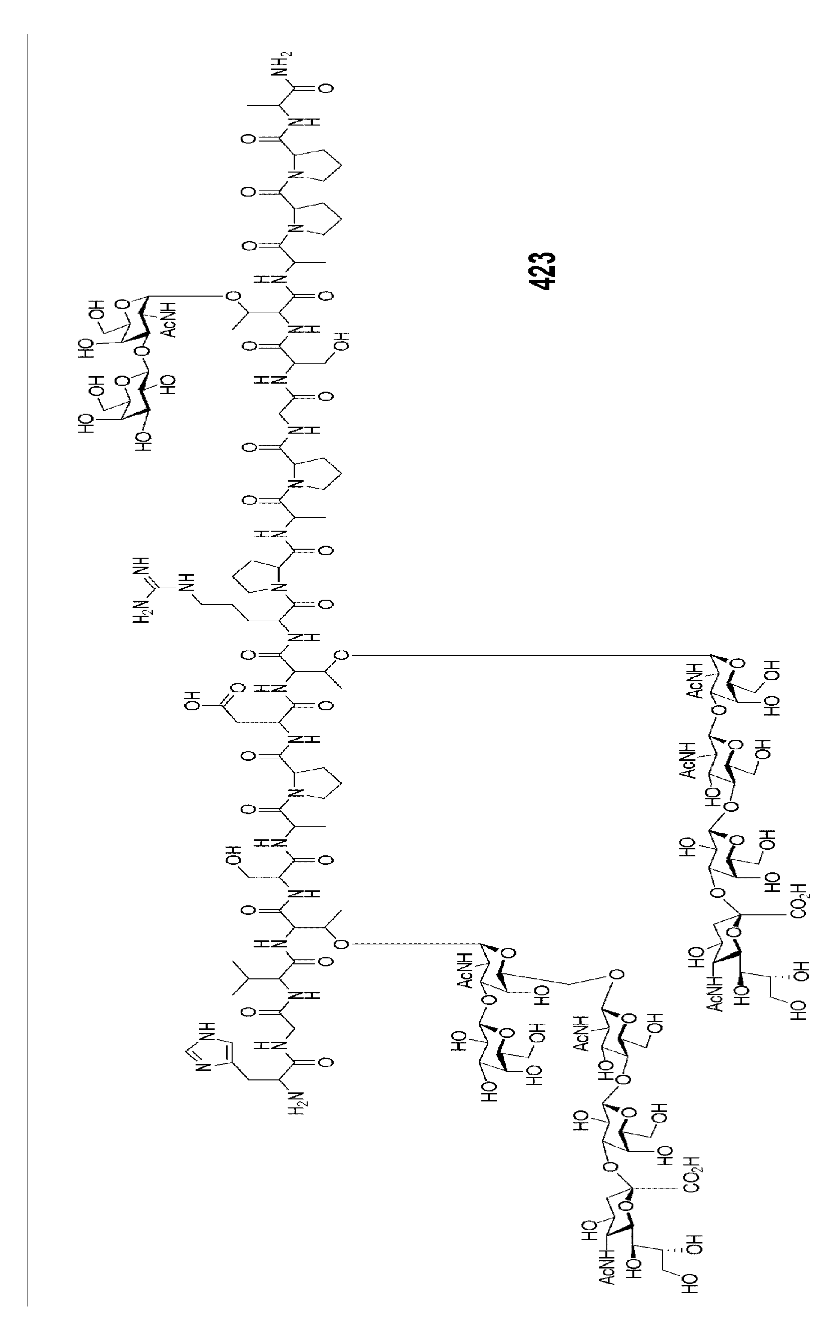

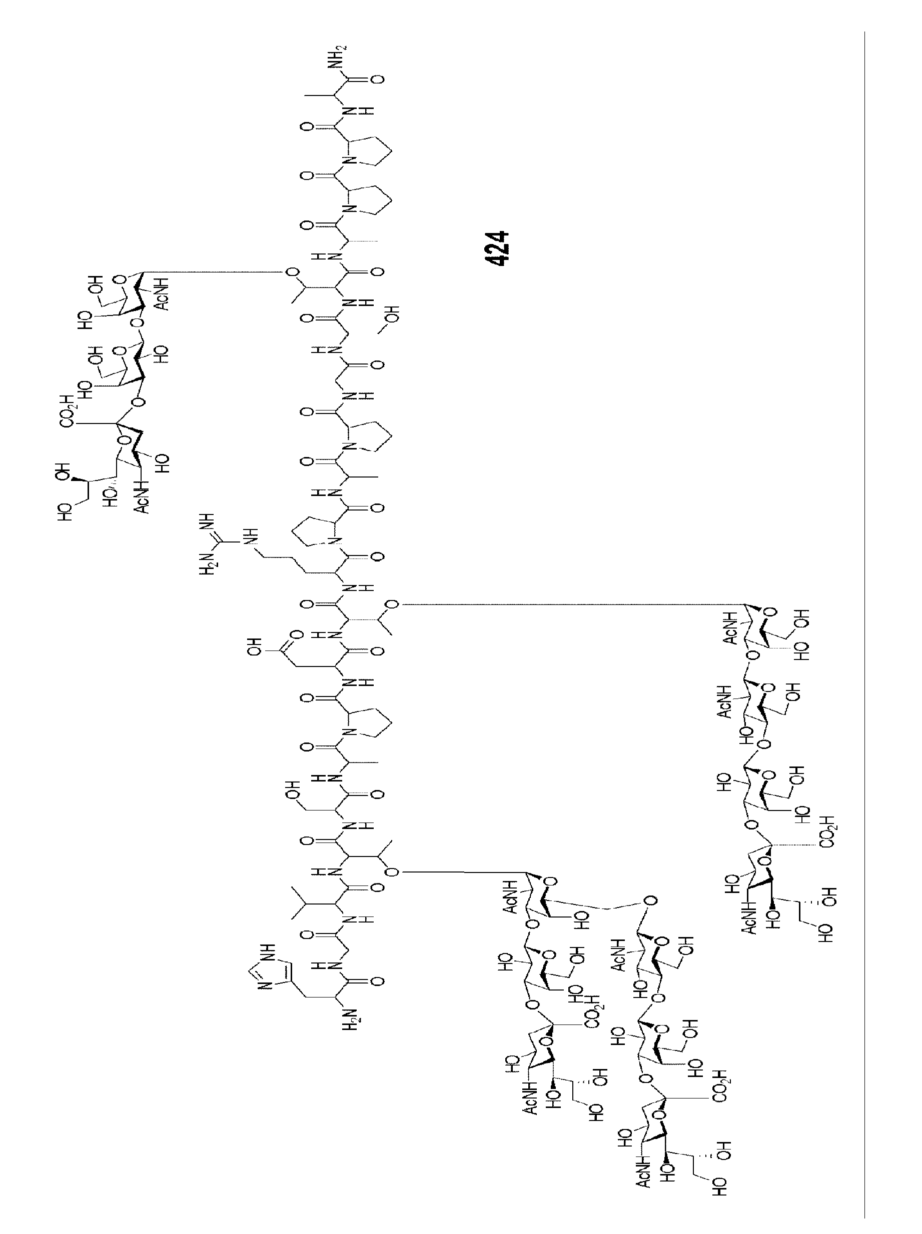

The following reaction solutions A) to E) were allowed to react for 24 hours at 25°C:
A) 100µl of reaction solution containing 50mMHEPES buffer solution (pH 7.0), 0.1U/ml human-derived β1,4-galactosyltransferase (available from TOYOBO CO., LTD.), 10mM manganese chloride, 0.1% BSA, 2mM uridine-5'-disodium diphosphogalactose (UDP-Gal) and glycopeptide derivative (248) (4mM at a theoretical content from solid-phase synthesis);
B) 100µl of reaction solution containing 50mM HEPES buffer solution (pH 7.0), 0.0175U/ml recombinant rat α2,3-(O)-sialyltransferase (available from Calbiochem), 10mM manganese chloride, 0.1% BSA, 2mM cytidine-5'-sodium monophosphosialate (CMP-NANA) and glycopeptide derivative (248) (4mM at a theoretical content from solid-phase synthesis);
C) 100µl of reaction solution containing 50mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (248) (4mM at a theoretical content from solid-phase synthesis);
D) 100 µl of reaction solution containing 50 mM HEPES buffer solution (pH 7.0), 0.1U/ml human-derived β1,4-galactosyltransferase (available from TOYOBO CO., LTD.), 0.0185U/ml recombinant rat α2,3-(N)-sialyltransferase (available from Calbiochem), 10mM manganese chloride, 0.1% BSA, 2mM uridine-5'-disodium diphosphogalactose (UDP-Gal), 2mM cytidine-5'-sodium monophosphosialate (CMP-NANA) and glycopeptide derivative (248) (4mM at a theoretical content from solid-phase synthesis);
E) 100µl of reaction solution containing 50 mM HEPES buffer solution (pH 7.0), 0.1U/ml human-derived β1,4-galactosyltransferase (available from TOYOBO CO., LTD.), 0.0175U/ml recombinant rat α2,3-(O)-sialyltransferase (available from Calbiochem), 0.0185U/ml recombinant rat α2,3-(N)-sialyltransferase (available from Calbiochem), 10mM manganese chloride, 0.1% BSA, 4mM uridine-5'-disodium diphosphogalactose (UDP-Gal), 2mM cytidine-5'-sodium monophosphosialate (CMP-NANA) and glycopeptide derivative (248) (4mM at a theoretical content from solid-phase synthesis).

After completion of the reaction, the respective reaction solutions were subjected to centrifugal concentration with an ultrafiltration filter. Thereafter, 25mM ammonium acetate buffer solution (pH 6.5) was added thereto, and the solution was again subjected to concentration with a centrifugal separator, thereby washing the polymer. This manipulation was repeated three times, thereby respectively obtaining aqueous solutions of compounds (414) to (418). Thereafter, to the solution retained at the filter containing compounds (414) to (418) and a solution which was obtained by diluting an aqueous solution of (248) with 25mM ammonium acetate buffer solution (pH 6.5), 1µl of 0.174mg/ml solution of BLase (available from Shionogi & Co., Ltd.) was added, and the solution was allowed to react for two hours at room temperature. Thereafter, the solution was subjected to centrifugal filtration with an ultrafiltration filter, thereby separating the glycopeptide of interest from the polymer. The obtained aqueous solution (filtrate) was lyophilized, thereby obtaining compounds (419) to (424).
MALDI-TOF/MS:[M(average)+H]⁺ of compound (419)=3227.8 (theoretical value:[M(average)+H]⁺=3227.3); MALDI-TOF/MS:[M(average)+H]⁺ of compound (420)=3552.3 (theoretical value:[M(average)+H]⁺=3551.6); MALDI-TOF/MS:[M(average)+H]⁺ of compound (421)=3810.1 (theoretical value:[M(average)+H]⁺=3809.8); MALDI-TOF/MS:[M(average)+H]⁺ of compound (422)=4131.5 (theoretical value:[M(average)+H]⁺=4134.1); MALDI-TOF/MS:[M(average)+H]⁺ of compound (423)=4133.5 (theoretical value:[M(average)+H]⁺=4134.1); and MALDI-TOF/MS:[M(average)+H]⁺ of compound (424)=4716.7 (theoretical value: [M(average)+H]⁺=4716.6).

(3. 69 Synthesis of compounds (425) to (427) using one-pot reaction)

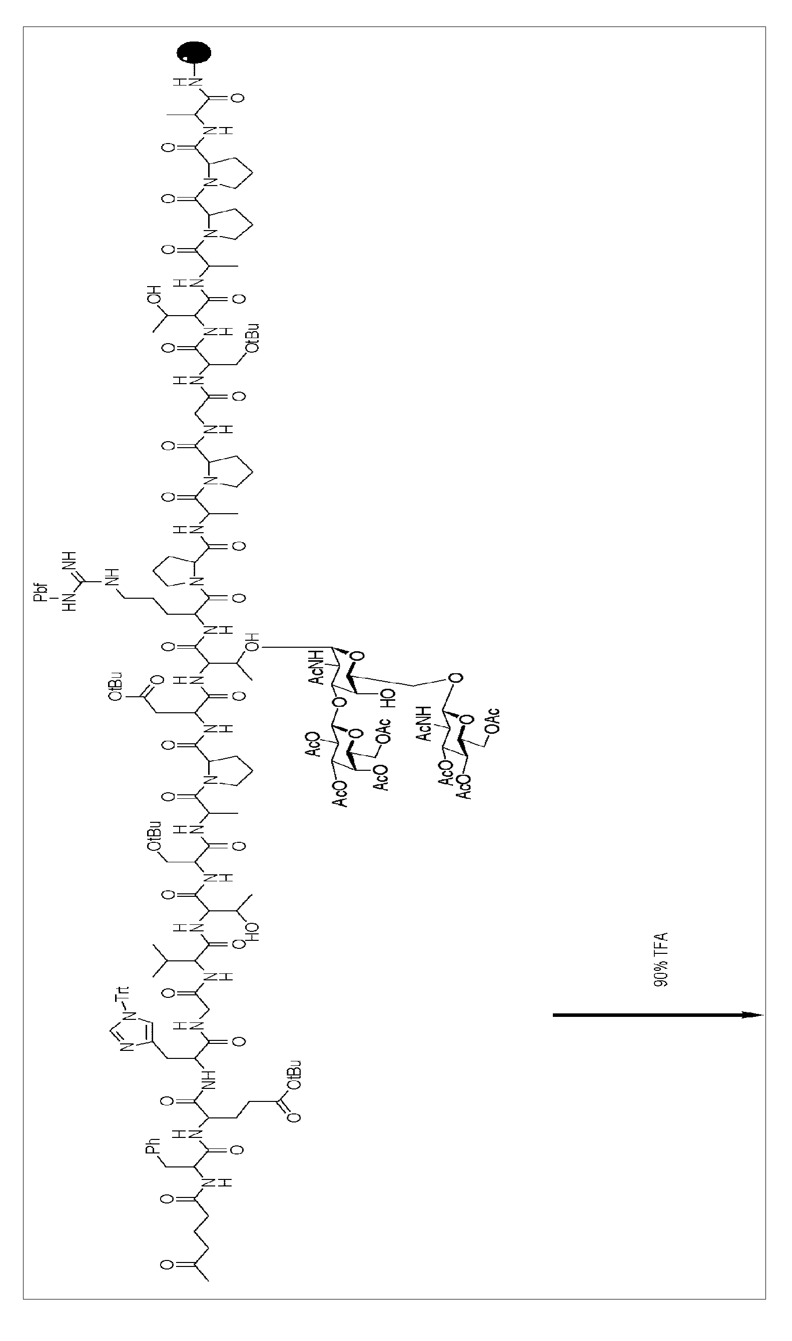

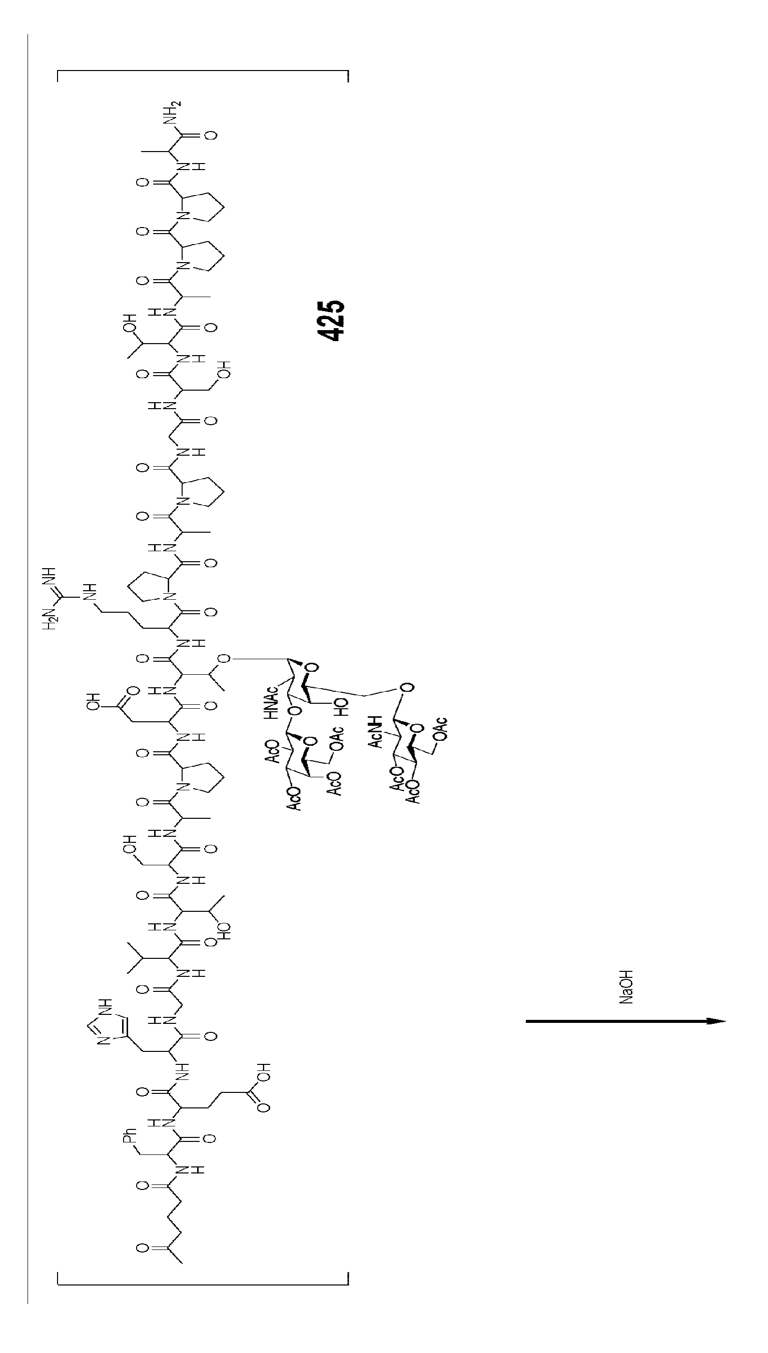

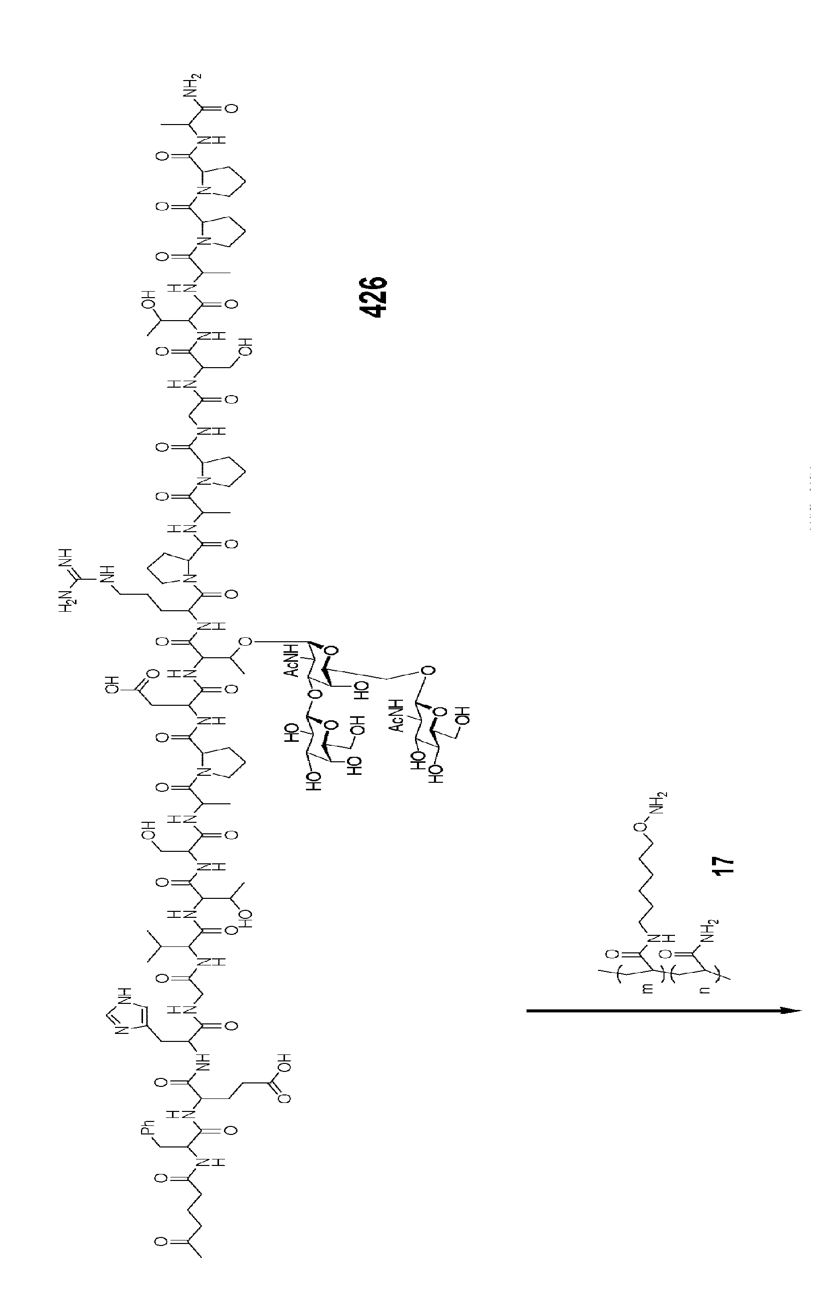

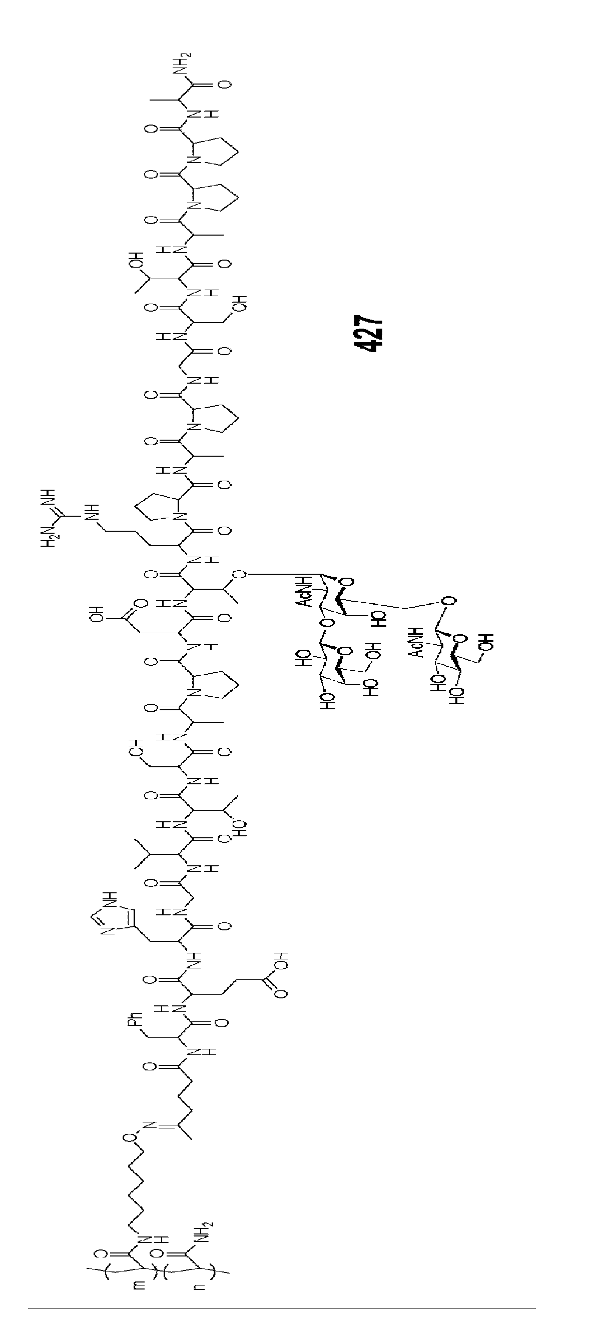

Using 610.1mg (wet, 30.5imol) of Rink Amide PEGA resin (available from Novabiochem, pre-swolled in Methanol, wet:0.05mmol/g, dry:0.24mmol/g) as a support, N-protected amino acids and keto acid as describedbelowwere sequentially condensed by Fmoc/HBTU/HOBt method, thereby synthesizing glycopeptide derivative of interest: Fmoc-Ala-OH; Fmoc-Pro-OH; Fmoc-Pro-OH; Fmoc-Ala-OH; Fmoc-Thr(tBu)-OH; Fmoc-Ser(tBu)-OH; Fmoc-Gly-OH; Fmoc-Pro-OH; Fmoc-Ala-OH; Fmoc-Pro-OH; Fmoc-Arg(Pbf)-OH; Fmoc-Thr(Ac7core2)-OH; Fmoc-Asp(OtBu)-OH; Fmoc-Pro-OH; Fmoc-Ala-OH; Fmoc-Ser(tBu)-OH; Fmoc-Thr(tBu)-OH; Fmoc-Val-OH; Fmoc-Gly-OH; Fmoc-His(Trt)-OH; Fmoc-Glu(OtBu)-OH; Fmoc-Phe-OH; and 5-ketohexane acid. After peptide elongation reaction, the obtained substance was allowed to react in 90% TFA aqueous solution for 1.5 hours at room temperature to eliminate a protective group on peptide residue and concurrently release compound (425) from the solid-phase support. The resin was separated by filtration, and TFA was removed by volatilization. Thereafter, diethyl ether was added to the filtrate, and a product was allowed to precipitate. The obtained slurry was subjected to centrifugal separation, and thereafter, the supernatant was removed. Diethyl ether was again added, and the precipitate was washed. Centrifugal separation was again performed and the supernatant was removed. The obtained precipitant was dissolved in 9.0ml of methanol. 1N sodium hydroxide aqueous solution was added to this solution, and was adjusted to have pH of 12-12.5. The solution was then stirred for 1.0 hour at room temperature, thereby performing Ac deprotection reaction. After the reaction, 1N acetic acid was added and the solution was neutralized. Thereafter, the resin was separated by filtration. The solvent in the filtrate was removed, and the residue was dissolved in 3.0ml of 50mM acetic acid/sodium acetate buffer solution (pH 5.5). 0.5ml of 10mM (oxyamine residue calculation) water-soluble polymer (17) aqueous solution was added to the solution, and the solution was stirred for 18 hours at room temperature, thereby reacting compound (426) with compound (17). After completion of the reaction, the reaction solution was subjected to centrifugal concentration with an ultrafiltration filter 10K Apollp ® 20ml (Orbital Biosciences, available from LIC). 25mM HEPES buffer solution (pH 7.0) was added thereto and the solution was again subjected to concentration, thereby washing the polymer. By adding water so that the final amount of the solution was 1.5ml, 10mM (theoretical content of glycopeptid) polymer (427) was obtained.

(3.70 Synthesis of compounds (428) to (429)

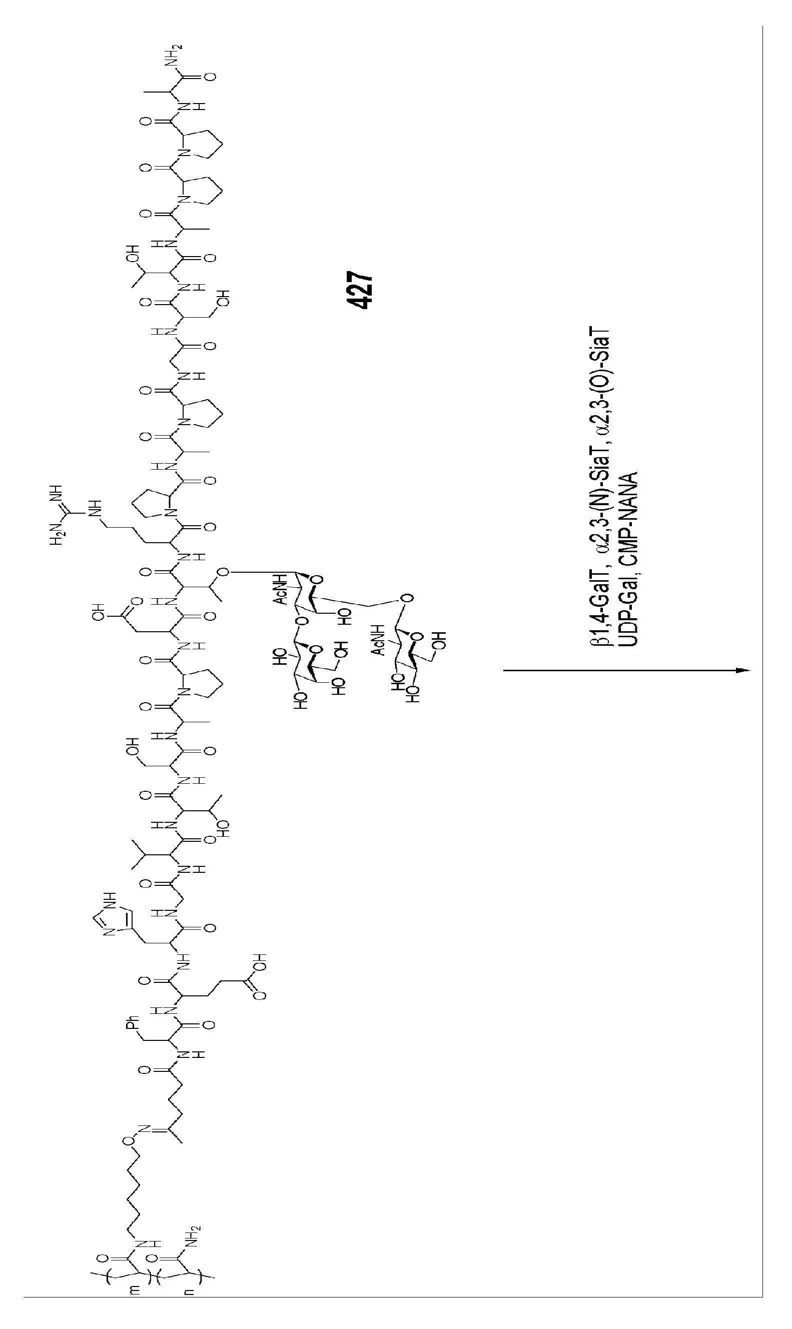

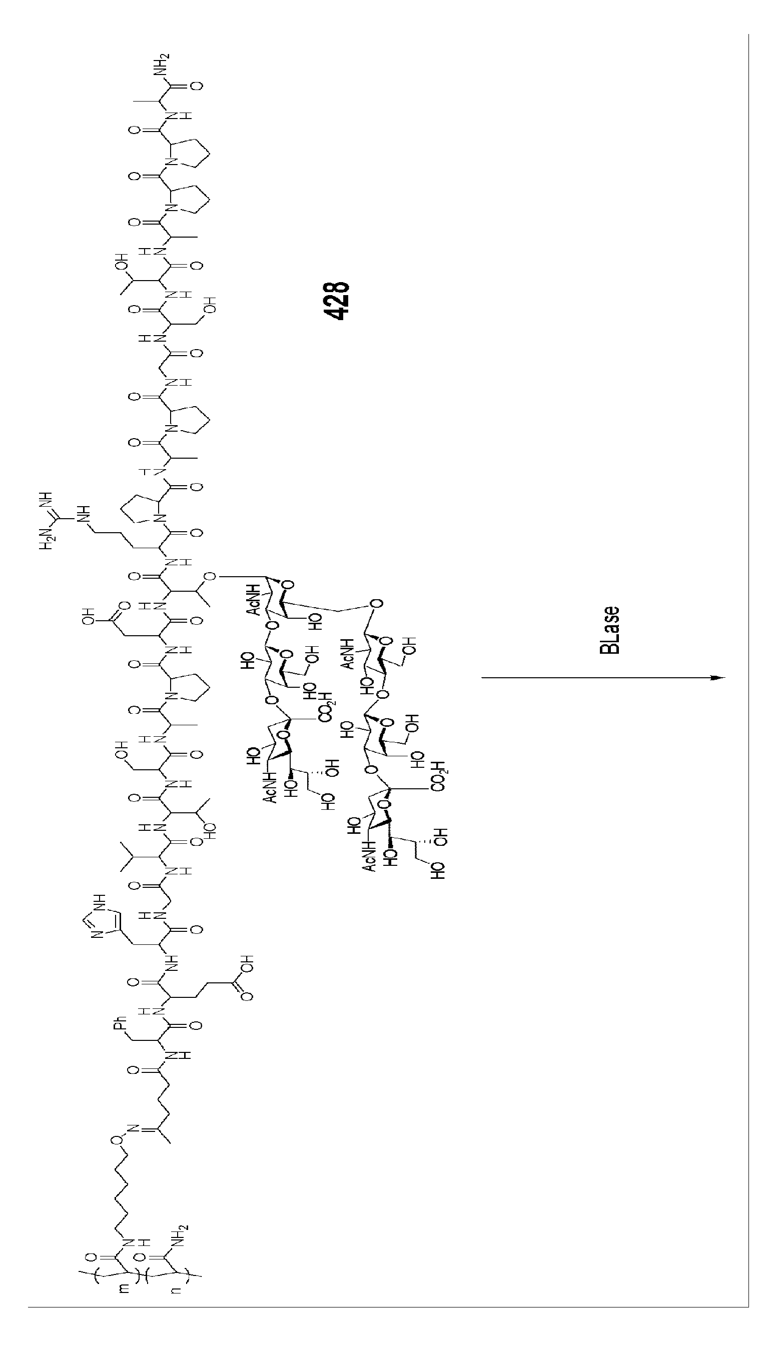

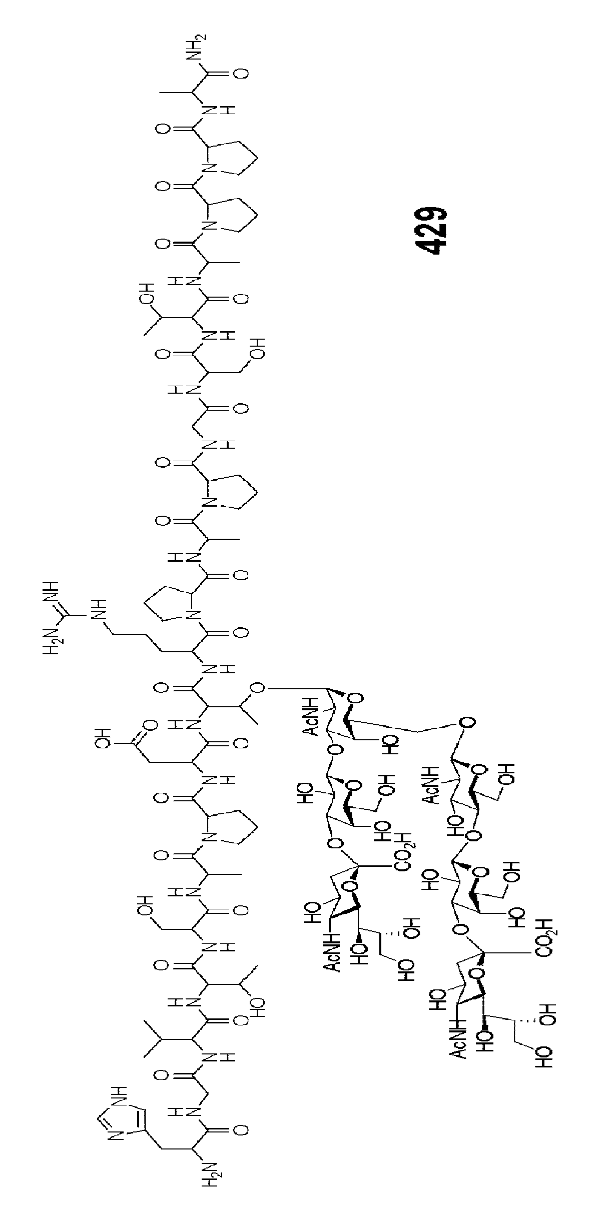

7ml of reaction solution containing 50mM HEPES buffer solution (pH 7.0), 0.05U/ml human-derived β1,4-galactosyltransferase (available from TOYOBO CO., LTD.), 0.0175U/ml recombinant rat α2,3-(O)-sialyltransferase (available from Calbiochem), 0.0185U/ml recombinant rat α2,3-(N)-sialyltransferase (available from Calbiochem), 10mM manganese chloride, 0.1% BSA, 10mM uridine-5'-disodium diphosphogalactose (UDP-Gal), 10mM cytidine-5'-sodium monophosphosialate (CMP-NANA) and glycopeptide derivative (427) (4mM at a theoretical content from solid-phase synthesis) was allowed to react for 24 hours at 25°C.

After completion of the reaction, the respective reaction solutions were subjected to centrifugal concentration with an ultrafiltration filter, 10K Apollo® 20ml (Orbital Biosciences, available from LIC). Thereafter, 25mM ammonium acetate buffer solution (pH 6.5) was added thereto, and the solution was again subjected to concentration with a centrifugal separator, thereby washing the polymer. This manipulation was repeated three times, thereby obtaining aqueous solution of compounds (428). Thereafter, to 4.2ml of the solution retained at the filter containing compound (428), 5µl of 1.74mg/ml solution of BLase (available from Shionogi & Co., Ltd.) was added, and the solution was allowed to react for two hours at room temperature. Thereafter, the solution was subjected to centrifugal filtration with an ultrafiltration filter, 30K Apollo® 20,1 (Orbital Biosciences, available from LIC), thereby separating the glycopeptide of interest from the polymer. The obtained aqueous solution (filtrate) was lyophilized, thereby obtaining 11.4mg of compound (429).
MALDI-TOF/MS:[M(average)+H]⁺ of compound (429)=3200.0 (theoretical value:[M(average)+H]⁺=3200.2).

### INDUSTRIAL APPLICABILITY

The present invention enables preparation of a glycopeptide library exhaustively having from simple sugar chain structures to complicated sugar chain structures, which has been extremely difficult by using conventional techniques. For example, the present invention enables synthesis of mucin-type glycopeptides which are useful in a wide field including materials for biochemical research, drugs and food and which have been conventionally difficult to produce.

The obtained glycopeptide library can be used as a standard sample for structural analysis and biochemical tests. Further, it is enabled to arrange this glycopeptide library on a chip to exhaustively perform detection of glycopeptide-recognizingproteins, pathological diagnosis, search for a cell adhesion sequence, sequence analysis related to cellular growth, apotopsis and the like.

## Claims

1. A compound represented by the following formula:
X-C (=O) - (CH₂)ₙ-A₁-A₂-A₃ (I)
wherein X represents a hydrogen atom, C₁-C₃₀ alkyl, C₆-C₃₀ aryl or a chromophore;
n represents an integer from 0 to 20;
A₁ represents - (CH₂)₀₋₂₀-C(=O)-, -(CH₂CH₂O)₁₋₁₀-, oligoacrylamide or polyacrylamide having a degree of polymerization of 1 to 10, oligopeptide or polypeptide having a degree of polymerization of 1 to 10, an oxygen atom or NH;
A₂ represents an amino acid residue which can be cleaved by a protease; and
A₃ represents a glycoamino acid residue substantially free of a site which can be cleaved by a protease, or a glycopeptide residue free of a site which can be cleaved by a protease and comprising a glycoamino acid.

2. The compound according to claim 1, wherein A₂ is a glutamic acid residue or cysteine residue which can be cleaved by a protease derived from *Bacillus Licheniformis.*

3. The compound according to claim 1, wherein at least a part of A₃ has an amino acid sequence selected from the group consisting of the amino acid sequences as set forth in SEQ ID NOS: 1-60 derived from mucin-type glycoprotein MUC1.

4. A compound which is obtained by reacting the compound according to claim 1 and a support comprising a functional group selected from the group consisting of: a protected or unprotected aminooxy group; a protected or unprotected N-alkylaminooxy group; a protected or unprotected hydrazid group; a protected or unprotected azide group; a protected or unprotected thiosemicarbazide group; a protected or unprotected 1,2-dithiol group; and a protected or unprotected cysteine residue.

5. The compound according to claim 4, wherein the support is selected from the group consisting of:
a) a polymer or copolymer of a vinyl-type monomer having a protected or unprotected amiooxy group or protected or unprotected hydrazide group, or polyethers having a protected or unprotected aminooxy group or protected or unprotected hydrazide group;
b) a silica support, a resin support, magnetic beads or a metalic support, having a protected or unprotected aminooxy group or protected or unprotected hydrazide group; and
c) a compound represented by the following formula:
[(NH₂OCH₂C(=O))₂-Lys]₂-Lys-NHCH₂CH₂C(=O)-R³,
[(NH₂OCH₂C(=O))₂-Lys]₂-Lys-NHCH(CH₂SH)C(=O)-R³,
[(NH₂OCH₂C(=O))₂-Lys]₂-Lys-Cys-NHCH₂CH₂C(=O)-R³(SEQ ID NO: 61);
{[(NH₂OCH₂C(=O))₂-Lys]₂-Lys-NHCH[C(=O)-R³]CH₂-S}₂,
{[(NH₂OCH₂C(=O))₂-Lys]₂-Lys-NHCH[C(=O)NHCH₂CH₂C(=O)-R³ ]CH₂-S}₂,
{[(NH₂OCH₂C(=O))₂-Lys]₂-Lys}₂-Lys-NHCH₂CH₂C(=O)-R³ (SEQ ID NO: 62);,
{[(NH₂OCH₂C(=O))₂-Lys]₂-Lys}₂-Lys-NHCH(CH₂SH)C(=O)-R³ (SEQ ID NO: 63);,
{[(NH₂OCH₂C(=O))₂-Lys]₂-Lys}₂-Lys-Cys-N
HCH₂CH₂C(=O)-R³ (SEQ ID NO:64);,
[[[(NH₂OCH₂C(=O))₂-Lys]₂-Lys)₂-Lys-NHCH[C(=O)-R³]CH₂-S ]₂ (SEQ ID NO: 65);
[[[(NH₂OCH₂C(=O))₂-Lys]₂-Lys]₂-Lys-NHCH[C(=O)NHCH₂CH₂C 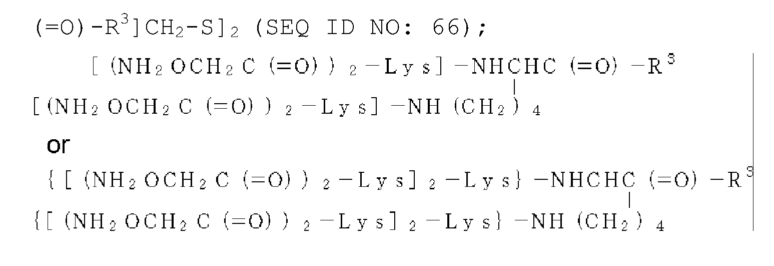
wherein R₃ represents a hydroxyl group or amino group, Lys represents lysine and Cys represents cysteine; 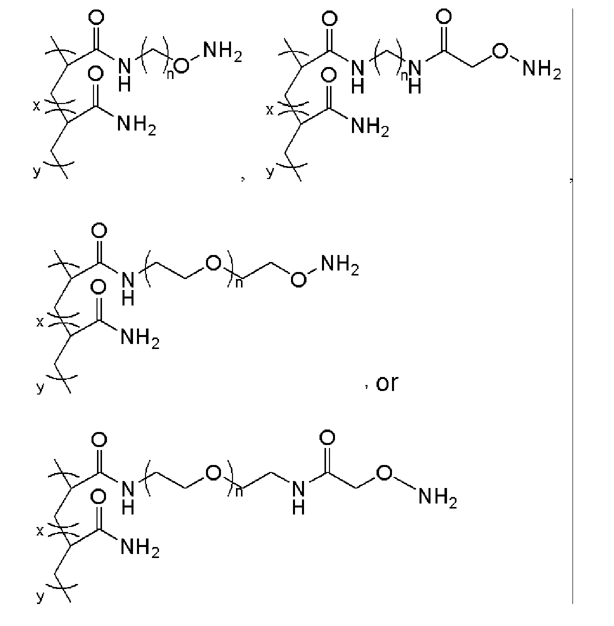 wherein n is an integer from 1 to 15 and x:y is 1:0 to 1:1000.

6. A compound represented by the following formula:
A₄-N=C(-X) - (CH₂)ₙ-A₁-A₂-A₃ (II)
wherein X represents a hydrogen atom, C₁-C₃₀ alkyl, C₆-C₃₀ aryl or a chromophore;
n represents an integer from 0 to 20;
A₁ represents -(CH₂)₀₋₂₀-C(=O)-, -(CH₂CH₂O)₁₋₁₀-, oligoacrylamide or polyacrylamide having a degree of polymerization of 1 to 10, oligopeptide or polypeptide having a degree of polymerization of 1 to 10, an oxygen atom or NH;
A₂ represents a glutamic acid residue or cysteine residue which can be cleaved by a protease derived from *Bacillus Licheniformis;*
A₃ represents a glycoamino acid residue substantially free of a site which can be cleaved by a protease, or a glycopeptide residue free of a site which can be cleaved by a protease and comprising a glycoamino acid;
A₄ is a group represented by the following formula: 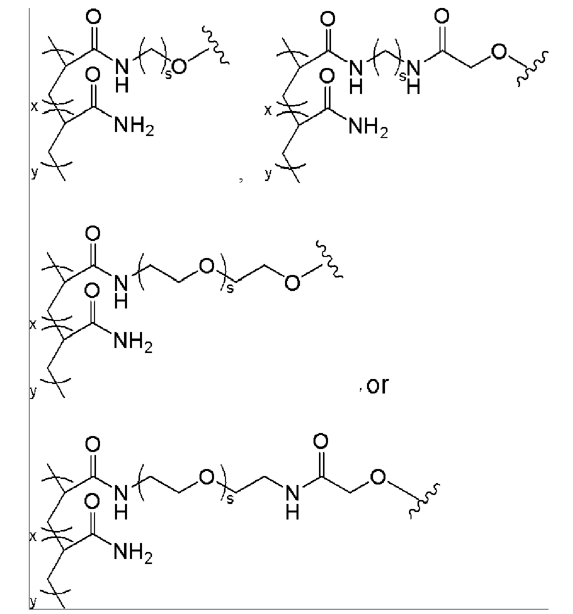 wherein s is an integer of 1 to 15 and x:y is 1:0 to 1:1000.

7. The compound according to claim 6, wherein at least a part of A₃ has an amino acid sequence selected from the group consisting of the amino acid sequences as set forth in SEQ ID NOS: 1-60 derived from mucin-type glycoprotein MUC1.

8. A method for producing a glycopeptide, the method comprising the steps of:
(A) reacting the compound according to any one of claims 1 to 3 with a support comprising a functional group selected from the group consisting of: a protected or unprotected aminooxy group; a protected or unprotected N-alkylaminooxy group; a protected or unprotected hydrazid group; a protected or unprotected azide group; a protected or unprotected thiosemicarbazide group; a protected or unprotected 1,2-dithiol group; and a protected or unprotected cysteine residue, the functional group being capable of specifically reacting with a ketone residue or aldehyde residue;
(B) allowing glycosyltransferase to act on the compound obtainedfromthestep (A) in the presence of a sugar nucleotide so as to cause a sugar residue to transfer from the sugar nucleotide to the compound, thereby obtaining a compound having an elongated sugar chain;
(C) optionally removing unreacted sugar nucleotides and by-product nucleotides; and
(D) allowing a protease to act on the compound having an elongated sugar chain as a result of transfer of the sugar residue.

9. A method for producing a glycopeptide, the method comprising the steps of:
(A) allowing glycosyltransferase to act on the compound according to any one of claims 4 to 7 in the presence of a sugar nucleotide so as to cause a sugar residue to transfer from the sugar nucleotide to the compound, thereby obtaining a compound having an elongated sugar chain;
(B) optionally removing unreacted sugar nucleotides and by-product nucleotides; and
(C) allowing a protease to act on the compound having an elongated sugar chain as a result of transfer of the sugar residue.

10. A method for producing a glycopeptide, the method comprising the steps of:
(A) allowing glycosyltransferase to act on the compound according to any one of claims 4 to 7 in the presence of a sugar nucleotide so as to cause a sugar residue to transfer from the sugar nucleotide to the compound, thereby obtaining a compound having an elongated sugar chain;
(B) repeating the step (A) for one or more times to elongate a sugar chain;
(C) optionally removing unreacted sugar nucleotides and by-product nucleotides; and
(D) allowing a protease to act on the compound having an elongated sugar chain as a result of transfer of a plurality of sugar residues.

11. A method for producing a glycopeptide, the method comprising the steps of:
(A) performing solid phase peptide synthesis using an amino acid, glycoamino acid and keto acid or aldehydic acid, which can be cleaved by a protease, as raw materials, thereby obtaining the compound according to any one of claims 1 to 3;
(B) reacting the compound obtained from the step (A) with a support comprising a functional group selected from the group consisting of: a protected or unprotected aminooxy group; a protected or unprotected N-alkylaminooxy group; a protected or unprotected hydrazid group; a protected or unprotected azide group; a protected or unprotected thiosemicarbazide group; a protected or unprotected 1,2-dithiol group; and a protected or unprotected cysteine residue, the functional group being capable of specifically reacting with a ketone residue or aldehyde residue;
(C) allowing glycosyltransferase to act on the compound obtained from the step (B) in the presence of a sugar nucleotide so as to cause a sugar residue to transfer from the sugar nucleotide to the compound, thereby obtaining a compound having an elongated sugar chain;
(D) optionally removing unreacted sugar nucleotides and by-product nucleotides; and
(E) allowing a protease to act on the compound having an elongated sugar chain as a result of transfer of the sugar residue.

12. A method for producing a glycopeptide, the method comprising the steps of:
(A) performing solid phase peptide synthesis using an amino acid, glycoamino acid and keto acid or aldehydic acid, which can be cleaved by a protease, as raw materials, thereby obtaining the compound according to any one of claims 1 to 3;
(B) reacting the compound obtained from the step (A) with a support comprising a functional group selected from the group consisting of: a protected or unprotected aminooxy group; a protected or unprotected N-alkylaminooxy group; a protected or unprotected hydrazid group; a protected or unprotected azide group; a protected or unprotected thiosemicarbazide group; a protected or unprotected 1,2-dithiol group; and a protected or unprotected cysteine residue, the functional group being capable of specifically reacting with a ketone residue or aldehyde residue, and simultaneously removing unreacted substances in the step (A);
(C) allowing glycosyltransferase to act on the compound bound to the support, which has been obtained from the step (B), in the presence of a sugar nucleotide so as to cause a sugar residue to transfer from the sugar nucleotide to the compound, thereby obtaining a compound having an elongated sugar chain;
(D) repeating the step (C) for one or more times to elongate a sugar chain;
(E) optionally removing unreacted sugar nucleotides and by-product nucleotides; and
(F) allowing a protease to act on the compound having an elongated sugar chain as a result of transfer of a plurality of sugar residues.

13. The method according to claim 11 or 12, wherein the keto acid or aldehydic acid in the step (A) is a compound represented by the following formula:
X-C(=O)-(CH₂)ₙ-A₁-COOH (III)
wherein X represents a hydrogen atom, C₁-C₃₀ alkyl, C₆-C₃₀ aryl or a chromophore;
n represents an integer from 0 to 20; and
A₁ represents a linker having a length of 1 to 20 methylene groups.

14. A method for producing a glycopeptide, the method comprising the steps of:
(A) allowing glycosyltransferase to act on the compound according to any one of claims 1 to 3 in the presence of a sugar nucleotide so as to cause a sugar residue to transfer from the sugar nucleotide to the compound, thereby obtaining a compound having an elongated sugar chain;
(B) optionally repeating the step (A) for one or more times to elongate a sugar chain;
(C) reacting the compound having an elongated sugar chain as a result of transfer of the sugar residue with a support comprising a functional group selected from the group consisting of: a protected or unprotected aminooxy group; a protected or unprotected N-alkylaminooxy group; a protected or unprotected hydrazid group; a protected or unprotected azide group; a protected or unprotected thiosemicarbazide group; a protected or unprotected 1,2-dithiol group; and protected or unprotected cysteine residue, the functional group being capable of specifically reacting with a ketone residue or aldehyde residue; and
(D) optionally removing unreacted sugar nucleotides and by-product nucleotides.

15. A method for producing a glycopeptide, the method comprising the steps of:
(A) allowing glycosyltransferase to act on the compound according to any one of claims 1 to 3 in the presence of a sugar nucleotide so as to cause a sugar residue to transfer from the sugar nucleotide to the compound, thereby obtaining a compound having an elongated sugar chain;
(B) optionally repeating the step (A) for one or more times to elongate a sugar chain;
(C) reacting the compound having an elongated sugar chain as a result of transfer of the sugar residue with a support comprising a functional group selected from the group consisting of: a protected or unprotected aminooxy group; a protected or unprotected N-alkylaminooxy group; a protected or unprotected hydrazid group; a protected or unprotected azide group; a protected or unprotected thiosemicarbazide group; a protected or unprotected 1,2-dithiol group; and protected or unprotected cysteine residue, the functional group being capable of specifically reacting with a ketone residue or aldehyde residue; and
(D) optionally removing unreacted sugar nucleotides and by-product nucleotides; and
(E) allowing a protease to act on the compound having an elongated sugar chain as a result of transfer of the sugar residue.

16. The method according to any one of claims 8 to 15, wherein the glycopeptide is represented by the following formula: 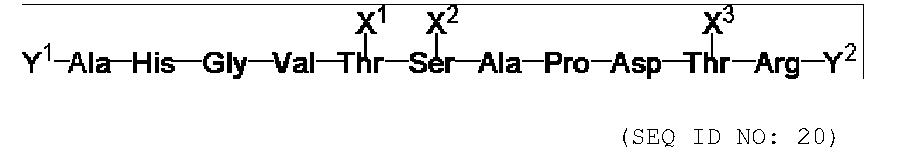 wherein X¹-X³ independently represent a hydrogen atom or a group represented by the following formula: 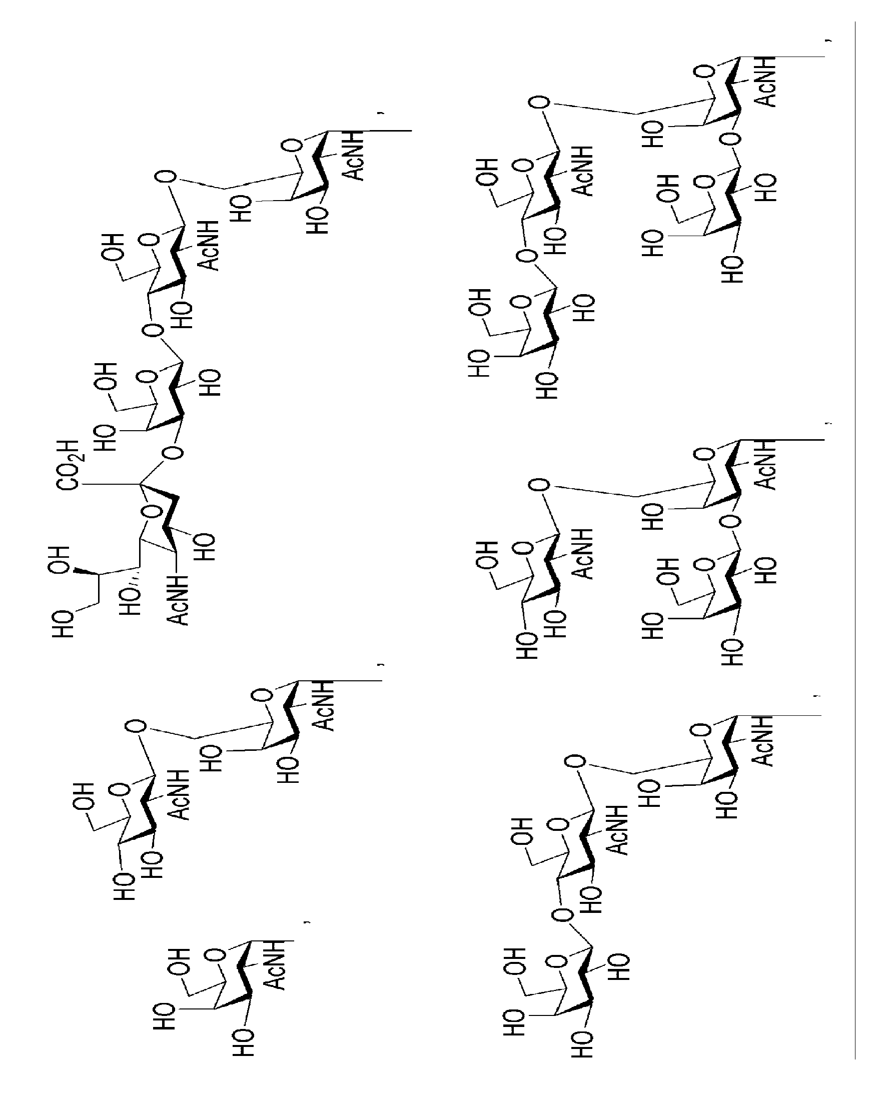 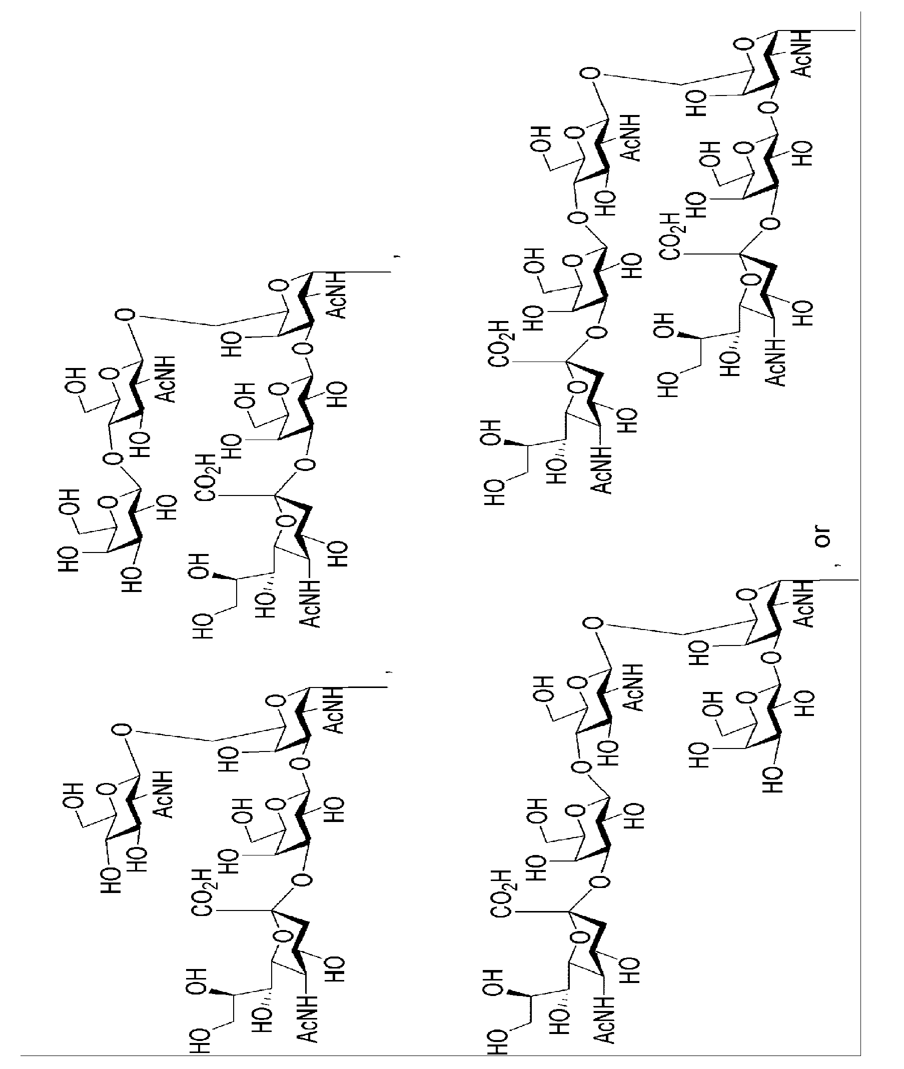 wherein Ac represents acetyl;
Y¹ represents a hydrogen atom, acetyl, acyl, alkyl or aryl;
Y² represents hydroxyl group, NH₂, alkyl or aryl, except the case where all of X¹-X³ are hydrogen atoms.

17. The method according to any one of claims 8 to 15, wherein the glycopeptide is represented by the following formula: 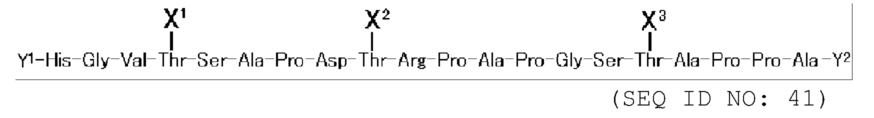 wherein X¹-X³ independently represent a hydrogen atom or a group represented by the following formula: 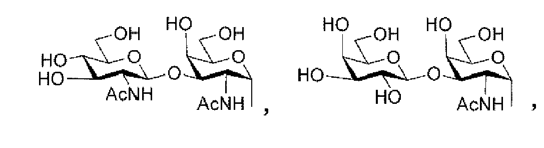 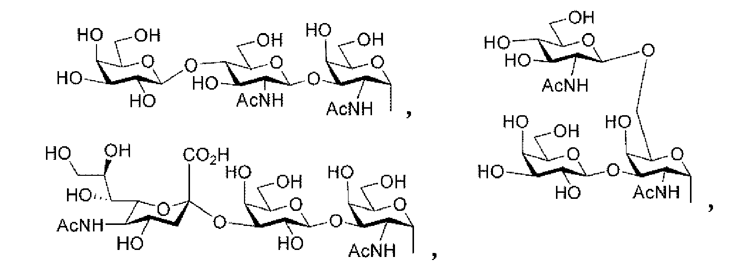 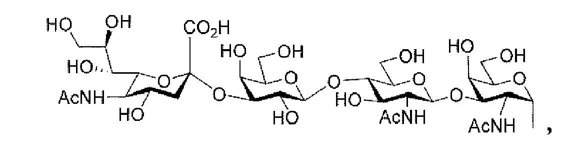 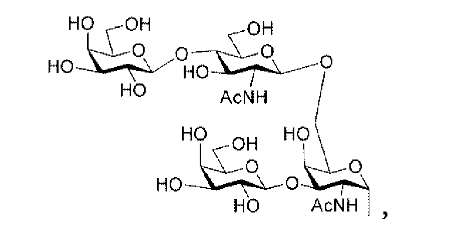 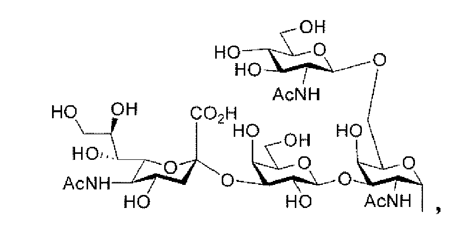 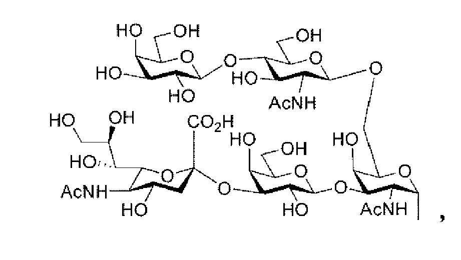 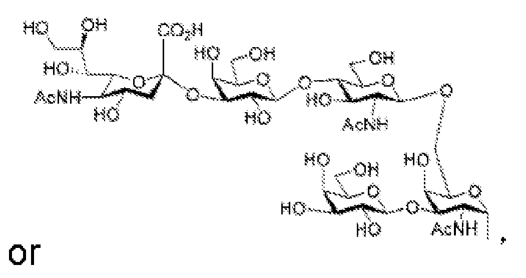 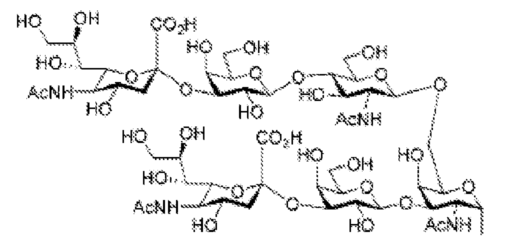 wherein Ac represents acetyl;
Y¹ represents a hydrogen atom, acetyl, acyl, alkyl or aryl;
Y² represents hydroxyl group, NH₂, alkyl or aryl, except the case where all of X¹-X³ are hydrogen atoms.

18. A glycopeptide represented by the following formula: 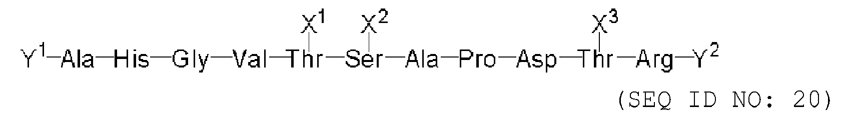 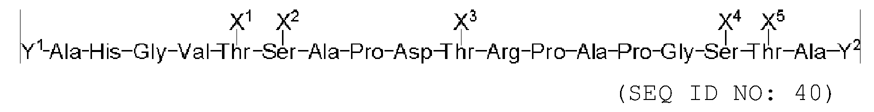 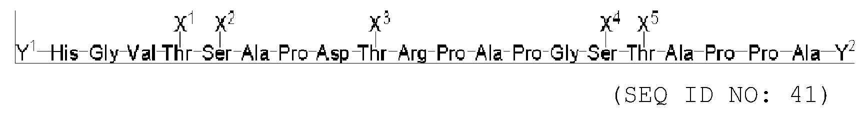 wherein X¹-X⁵ independently represent a hydrogen atom or a group represented by the following formula: 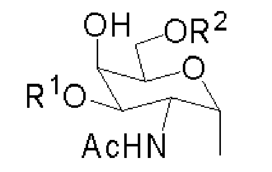 wherein R¹ and R² independently represent a hydrogen atom, monosaccharide or sugar chain, and Ac representes acetyl;
Y¹ represents a hydrogen atom, acetyl, acyl, alkyl or aryl;
Y² represents a hydroxyl group, NH₂, alkyl or aryl.

19. The glycopeptide according to claim 18, represented by the formula: 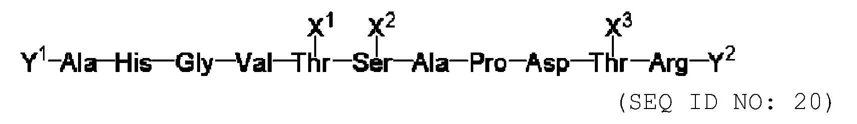 wherein X¹-X³ independently represent a hydrogen atom or a group represented by the following formula: 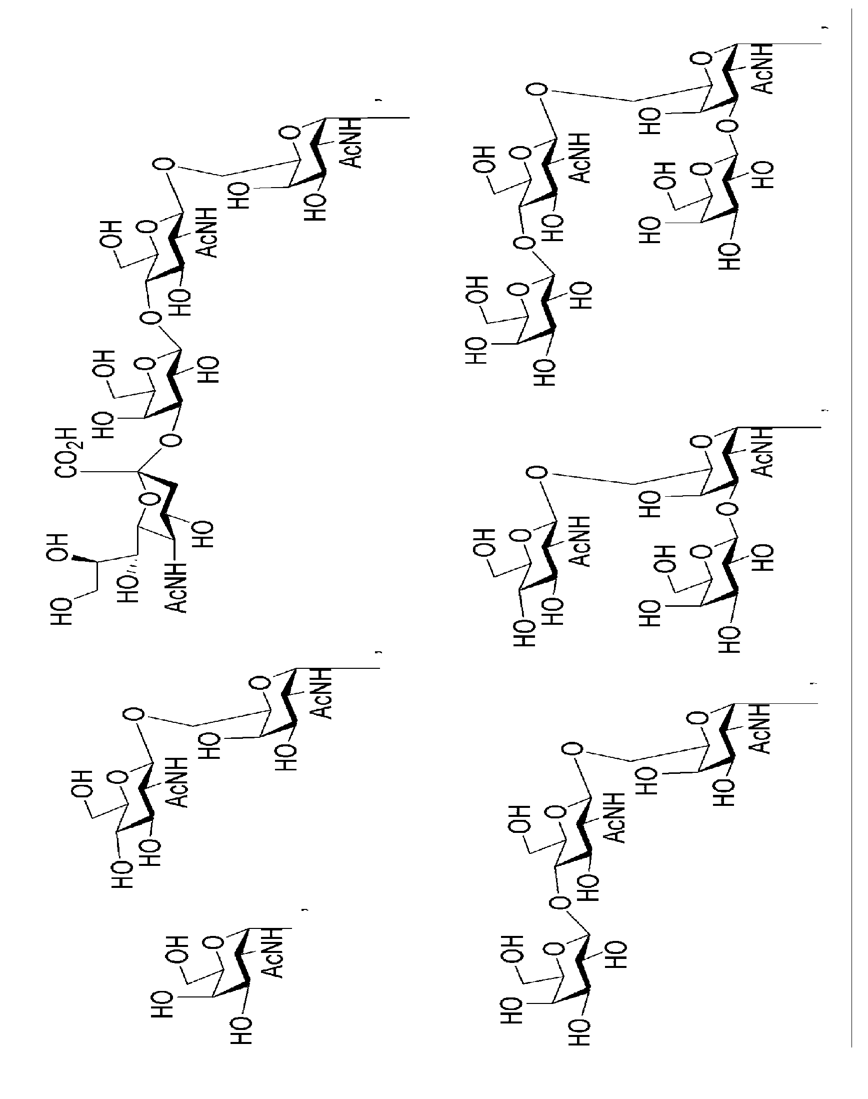 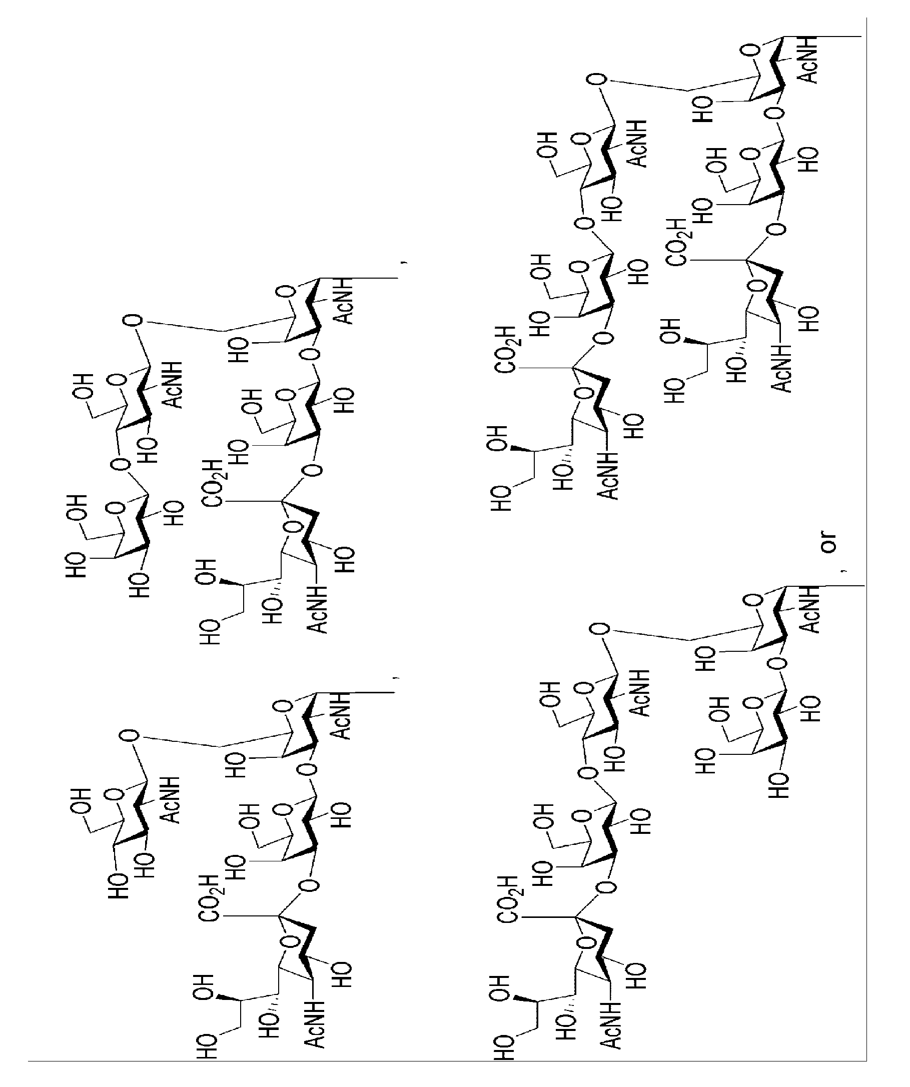 wherein Ac represents acetyl;
Y¹ represents a hydrogen atom, acetyl, acyl, alkyl or aryl;
Y² represents hydroxyl group, NH₂, alkyl or aryl, except the case where all of X¹-X³ are hydrogen atoms.

20. The glycopeptide according to claim 18, represented by the formula: 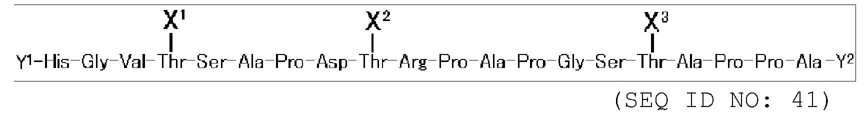 wherein X¹-X³ independently represent a hydrogen atom or a group represented by the following formula: 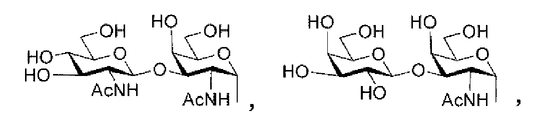 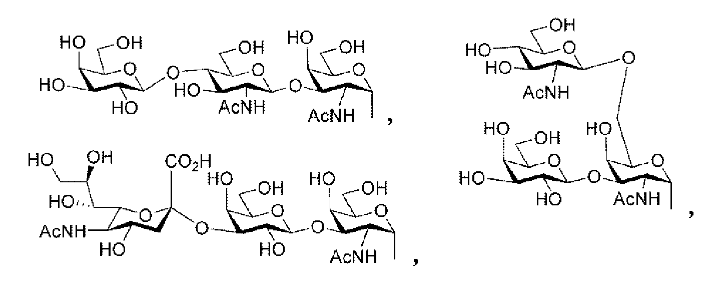 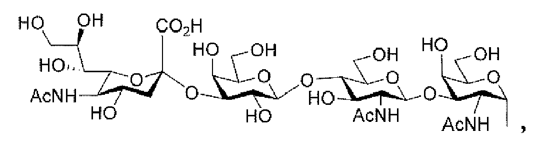 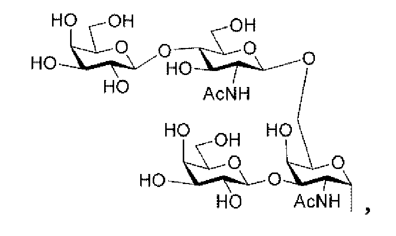 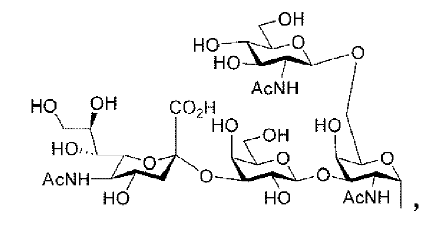 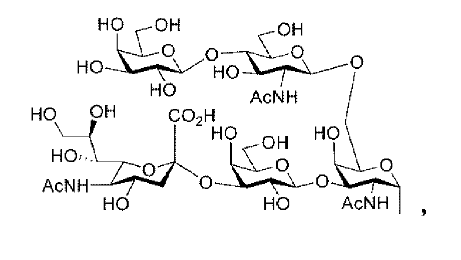 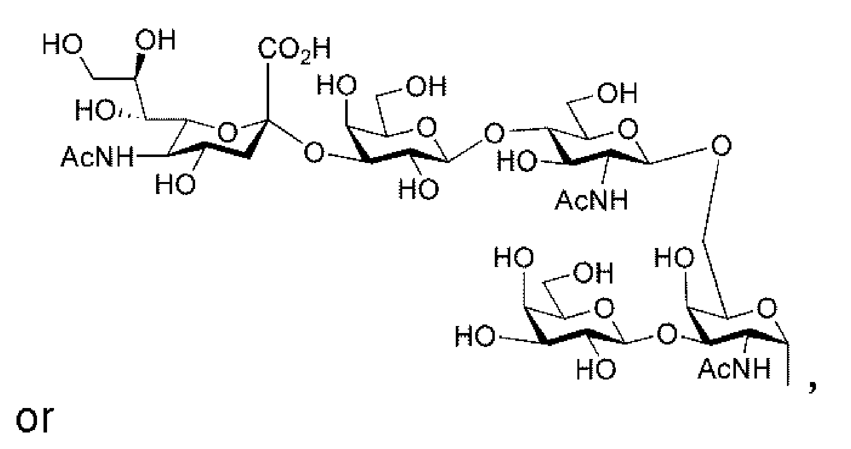 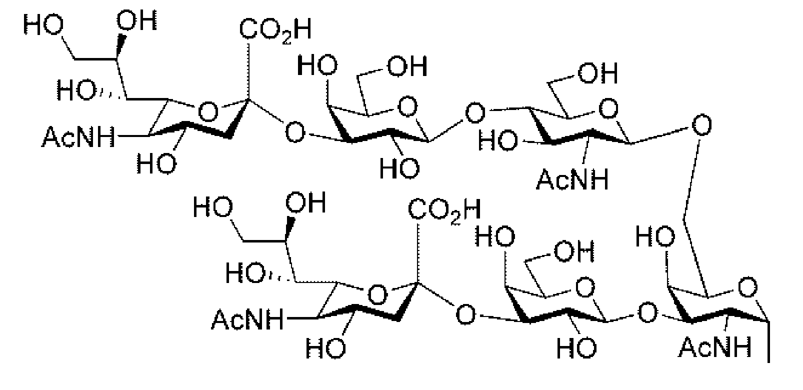 wherein Ac represents acetyl;
Y¹ represents a hydrogen atom, acetyl, acyl, alkyl or aryl;
Y² represents hydroxyl group, NH₂, alkyl or aryl, except the case where all of X¹-X³ are hydrogen atoms.
